# EUROPEAN PATENT APPLICATION

(11) **EP 2 039 762 A2**
(43) Date of publication of application: **25.03.2009**
(21) Application number: 08075753.7
(22) Date of filing: 15.05.2002
(51) Int. Cl.: C12N 9/00, C12N 9/78, C12N 1/20, C12N 15/00, C07H 21/02, C12P 1/00, C12P 7/00, C12P 7/62, C07K 1/00

(54) **Nitralases**

(30) Priority: 21.06.2001 US 300189 P; 30.07.2001 US 309006 P; 22.01.2002 US 351336 P
(62) Divisional of application: 02780877.3
(71) Applicant: Verenium Corporation, San Diego, CA 92191-0550 (US)
(72) Inventor: Desantis, Grace, San Diego, CA 92129 (US); Chaplin, Jennifer, Ann, San Diego, CA 92130 (US); Weiner, David, Paul, Del Mar, CA 92104 (US); Milan, Aileen, San Diego, CA 92126 (US); Chi, Ellen, Del Mar, CA 92014 (US); Short, Jay, M., Rancho Santa Fe, CA 92067-7214 (US); Madden, Mark, deceased (US); Burk, Mark, San Diego, CA 92130 (US); Robertson, Dan, Belmont, MA 02478 (US)
(74) Representative: Wakerley, Helen Rachael

(57) **Abstract**

The invention relates to nitrilases and to nucleic acids encoding the nitrilases. In addition, methods of designing new nitrilases and methods of use thereof are also provided. The nitrilases have increased activity and stability at increased pH and temperature.

## Description

### CROSS-REFERENCES TO RELATED APPLICATIONS

This application claims the benefit of priority to U.S. Provisional Application No. 60/351,336, filed January 22, 2002, U.S. Provisional Application Serial No. 60/309, 006, filed July 30, 2001, and U.S. Provisional Application Serial No. 60/300,189, filed June 21, 2001; and is a continuation-in-part ofU.S. Ser. No. 09/751,299, filed December 28, 2000, which claims the benefit of priority to each of U.S. Provisional Application Serial No. 60/254,414, filed December 7, 2000, and U.S. Provisional Application Serial No. 60/173,609, filed December 29, 1999. These applications are hereby incorporated by reference into the subject application in their entireties for all purposes.

### COPYRIGHT NOTIFICATION

Pursuant to 37 C.F.R. §1.71(e), a portion of this patent document contains material which is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

### FIELD OF THE INVENTION

The invention relates generally to the field of molecular biology, biochemistry and chemistry, and particularly to enzymatic proteins having nitrilase activity. The invention also relates to polynucleotides encoding the enzymes, and to uses of such polynucleotides and enzymes.

### BACKGROUND OF THE INVENTION

There are naturally occurring enzymes which have great potential for use in industrial chemical processes for the conversion of nitriles to a wide range of useful products and intermediates. Such enzymes include nitrilases which are capable of converting nitriles directly to carboxylic acids. Nitrilase enzymes are found in a wide range of mesophilic micro-organisms, including species of *Bacillus, Norcardia, Bacteridium, Rhodococcus, Micrococcus, Brevibacterium, Alcaligenes, Acinetobacter, Corynebacterium, Fusarium* and *Klebsiella.* Additionally, there are thermophilic nitrilases which exist in bacteria.

There are two major routes from a nitrile to an analogous acid: (1) a nitrilase catalyzes the direct hydrolysis of a nitrile to a carboxylic acid with the concomitant release of ammonia; or (2) a nitrile hydratase adds a molecule of water across the carbon-nitrogen bonding system to give the corresponding amide, which can then act as a substrate for an amidase enzyme which hydrolyzes the carbon-nitrogen bond to give the carboxylic acid product with the concomitant release of ammonia. The nitrilase enzyme therefore provides the more direct route to the acid.

A nitrile group offers many advantages in devising synthetic routes in that it is often easily introduced into a molecular structure and can be carried through many processes as a masked acid or amide group. This is only of use, however, if the nitrile can be unmasked at the relevant step in the synthesis. Cyanide represents a widely applicable C₁-synthon (cyanide is one of the few water-stable carbanions) which can be employed for the synthesis of a carbon framework. However, further transformations of the nitrile thus obtained are impeded due to the harsh reaction conditions required for its hydrolysis using normal chemical synthesis procedures. The use of enzymes to catalyze the reactions of nitriles is attractive because nitrilase enzymes are able to effect reactions with fewer environmentally hazardous reagents and by-products than in many traditional chemical methods. Indeed, the chemoselective biocatalytic hydrolysis of nitriles represents a valuable alternative because it occurs at ambient temperature and near physiological pH.

The importance of asymmetric organic synthesis in drug design and discovery has fueled the search for new synthetic methods and chiral precursors which can be utilized in developing complex molecules of biological interest. One important class of chiral molecules are the α-substituted carboxylic acids, which include the α-amino acids. These molecules have long been recognized as important chiral precursors to a wide variety of complex biologically active molecules, and a great deal of research effort has been dedicated to the development of methods for the synthesis of enantiomerically pure α-amino acids and chiral medicines.

Of particular use to synthetic chemists who make chiral medicines would be an enzyme system which is useful under non-sterile conditions, which is useful in non-biological laboratories, which is available in a form convenient for storage and use; which has broad substrate specificity, which acts on poorly water soluble substrates; which has predictable product structure; which provides a choice of acid or amide product; and which is capable of chiral differentiation. Accordingly, there is a need for efficient, inexpensive, high-yield synthetic methods for producing enantiomerically pure α-substituted carboxylic acids, such as, for example, α-amino acids and α-hydroxy acids.

Throughout this application, various publications are referenced by author and date. The disclosures of these publications in their entireties are hereby incorporated by reference into this application in order to more fully describe the state of the art as known to those skilled therein as of the date of the invention described and claimed herein.

### SUMMARY OF THE INVENTION

The present invention is directed to an isolated nucleic acid comprising consecutive nucleotides having a sequence at least 50% identical to SEQ ID NO: 1, 3, 5, 7, 9, 11, 13, 15, 17, 19, 21, 23, 25, 27, 29, 31, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, or 385 wherein the nucleic acid encodes a polypeptide having nitrilase activity. In an embodiment of the invention, the nucleic acid comprises consecutive nucleotides having a sequence at least 60 %, 70%, 80%, 85%, 90%, 95%, 98% or 100% identical to the SEQ ID NO. In an embodiment of the invention, the nucleic acid comprises consecutive nucleotides having a sequence substantially identical to the SEQ ID NO. In another embodiment, the invention provides for an isolated nucleic acid comprising consecutive nucleotides having a sequence at least 79 % identical to SEQ ID NO: 33, wherein the nucleic acid encodes a polypeptide having nitrilase activity. The invention provides for a fragment of the nucleic acid, wherein the fragment encodes a polypeptide having nitrilase activity. The invention also provides for an isolated nucleic acid complementary to any of the nucleic acids. The invention also provides for an isolated nucleic acid that hybridizes to any one of the nucleic acids under stringent conditions. In one embodiment, the stringent conditions comprise at least 50% formamide, and about 37°C to about 42°C.

The invention provides for a nucleic acid probe comprising from about 15 nucleotides to about 50 nucleotides, wherein at least 15 consecutive nucleotides are at least 50% complementary to a nucleic acid target region within a nucleic acid sequence of SEQ ID NO: 1, 3, 5, 7, 9, 11, 13, 15, 17, 19, 21, 23, 25, 27, 29, 31, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, or 385. In one embodiment, the nucleic acid probe comprises consecutive nucleotides which are at least 55% complementary to the nucleic acid target region. In one embodiment, the invention provides for a nucleic acid probe, wherein the consecutive nucleotides are at least 60%, 65%, 70%, 75%, 80%, 85%, 90%, 95%, 97%, 98% or 100% complementary to the nucleic acid target region. In another embodiment, the nucleic acid consists essentially of from about 20 to about 50 nucleotides.

The invention provides for a nucleic acid vector capable of replication in a host cell, wherein the vector comprises the nucleic acid of the invention. The invention also provides for a host cell comprising the nucleic acid. The invention also provides for a host organism comprising the host cell. In one embodiment, the host organism comprises a gram negative bacterium, a gram positive bacterium or a eukaryotic organism. In another embodiment, the gram negative bacterium comprises *Escherichia coli,* or *Pseudomonas fluorescens*. In a further embodiment, the gram positive bacterium comprises *Streptomyces diversa, Lactobacillus gasseri, Lactococcus lactis, Lactococcus cremoris,* or *Bacillus subtilis.* In a further embodiment, the eukaryotic organism comprises *Saccharomyces cerevisiae, Schizosaccharomyces pombe, Pichia pastoris, Kluyveromyces lactis, Hansenula plymorpha,* or *Aspergillus niger.*

The invention provides for an isolated nucleic acid encoding a polypeptide comprising consecutive amino acids having a sequence at least 50% identical to SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384 or 386 wherein the polypeptide has nitrilase activity. In one embodiment, the polypeptide comprises consecutive amino acids having at least 60%, 70%, 80%, 85%, 90%, 95%, 98% or 100% identity to the SEQ ID NO.

The invention also provides for an isolated nucleic acid encoding a polypeptide comprising at least 10 consecutive amino acids having a sequence identical to a portion of an amino acid sequence of SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384 or 386.

An isolated polypeptide comprising consecutive amino acids having a sequence at least 50% identical to SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, or 386 wherein the polypeptide has nitrilase activity. In one embodiment of the invention, the polypeptide comprises consecutive amino acids having a sequence at least 60%, 70%, 80%, 85%, 90%, 95%, 98% or 100% identical to the SEQ ID NO.

The invention provides an isolated nucleic acid comprising consecutive nucleotides having a sequence as set forth in any one of the following SEQ ID NOS:1, 3, 5, 7, 9, 11, 13, 15, 17, 19, 21, 23, 25, 27, 29, 31, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, or 385 (hereinafter referred to as "Group A nucleic acids"). The invention is also directed to nucleic acids having specified minimum percentages of sequence identity to any of the Group A nucleic acids sequences.

In another aspect, the invention provides a purified polypeptide comprising consecutive amino acid residues having a sequence as set forth in any one of the following SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384 and 386 (hereinafter referred to as "Group B amino acid sequences"). The invention is also directed to purified polypeptides having specified minimum percentages of sequence identity to any of the Group B amino acid sequences.

The invention provides for a fragment of the polypeptide which is at least 50 amino acids in length, and wherein the fragment has nitrilase activity. Furthermore, the invention provides for a peptidomimetic of the polypeptide or a fragment thereof having nitrilase activity. The invention provides for a codon-optimized polypeptide or a fragment thereof, having nitrilase activity, wherein the codon usage is optimized for a particular organism or cell. Narum et al. Infect. Immun. 2001 Dec, 69(12):7250-3 describes codon-optimzation in the mouse system. Outchkourov et al. Protein Expr. Purif. 2002 Feb; 24(1):18-24 describes codon-optimization in the yeast system. Feng et al. Biochemistry 2000 Dec 19, 39(50):15399-409 describes codon-optimization in *E. coli.* Humphreys et al. Protein Expr. Purif. 2000 Nov, 20(2):252-64 describes how codon usage affects secretion in *E. coli.*

In one embodiment, the organism or cell comprises a gram negative bacterium, a gram positive bacterium or a eukaryotic organism. In another embodiment of the invention, the gram negative bacterium comprises *Escherichia coli,* or *Pseudomonas fluorescens*. In another embodiment of the invention, the gram positive bacterium comprise *Streptomyces diversa, Lactobacillus gasseri, Lactococcus lactis, Lactococcus cremoris,* or *Bacillus subtilis.* In another embodiment of the invention, the eukaryotic organism comprises *Saccharomyces cerevisiae, Schizosaccharomyces pombe, Pichia pastoris, Kluyveromyces lactis, Hansenula plymorpha*, or *Aspergillus niger.*

In another aspect, the invention provides for a purified antibody that specifically binds to the polypeptide of the invention or a fragment thereof, having nitrilase activity. In one embodiment, the invention provides for a fragment of the antibody that specifically binds to a polypeptide having nitrilase activity.

The invention provides for an enzyme preparation which comprises at least one of the polypeptides of the invention, wherein the preparation is liquid or dry. The enzyme preparation includes a buffer, cofactor, or second or additional protein. In one embodiment the preparation is affixed to a solid support. In one embodiment of the invention, the solid support can be a gel, a resin, a polymer, a ceramic, a glass, a microelectrode and any combination thereof. In another embodiment, the preparation can be encapsulated in a gel or a bead.

The invention further provides for a composition which comprises at least one nucleic acid of the invention which comprises at least one polypeptide of the invention or a fragment thereof, or a peptidomimetic thereof, having nitrilase activity, or any combination thereof.

The invention provides for a method for hydrolyzing a nitrile to a carboxylic acid comprising contacting the molecule with at least one polypeptide of the invention or a fragment thereof, or a peptidomimetic thereof, having nitrilase activity, under conditions suitable for nitrilase activity. In one embodiment, the conditions comprise aqueous conditions. In another embodiment, the conditions comprise a pH of about 8.0 and/or a temperature from about 37° C to about 45° C.

The invention provides for a method for hydrolyzing a cyanohydrin moiety or an aminonitrile moiety of a molecule, the method comprising contacting the molecule with at least one polypeptide of the invention, or a fragment thereof, or a peptidomimetic thereof, having nitrilase activity, under conditions suitable for nitrilase activity.

The invention provides for a method for making a chiral α-hydroxy acid molecule, a chiral amino acid molecule, a chiral β-hydroxy acid molecule, or a chiral gamma-hydroxy acid molecule, the method comprising admixing a molecule having a cyanohydrin moiety or an aminonitrile moiety with at least one polypeptide having an amino acid sequence at least 50% identical to any one of the Group B amino acid sequences or a fragment thereof, or a peptidomimetic thereof, having enantio-selective nitrilase activity. In one embodiment, the chiral molecule is an *(R)*-enantiomer. In another embodiment, the chiral molecule is an *(S)*-enantiomer. In one embodiment of the invention, one particular enzyme can have R-specificity for one particular substrate and the same enzyme can have S-specificity for a different particular substrate.

The invention also provides for a method for making a composition or an intermediate thereof, the method comprising admixing a precursor of the composition or intermediate, wherein the precursor comprises a cyanohydrin moiety or an aminonitrile moiety, with at least one polypeptide of the invention or a fragment or peptidomimetic thereof having nitrilase activity, hydrolyzing the cyanohydrin or the aminonitrile moiety in the precursor thereby making the composition or the intermediate thereof. In one embodiment, the composition or intermediate thereof comprises *(S)*-2-amino-4-phenyl butanoic acid. In a further embodiment, the composition or intermediate thereof comprises an L-amino acid. In a further embodiment, the composition comprises a food additive or a pharmaceutical drug.

The invention provides for a method for making an (R)-ethyl 4-cyano-3-hydroxybutyric acid, the method comprising contacting a hydroxyglutaryl nitrile with at least one polypeptide having an amino acid sequence of the Group B amino acid sequences, or a fragment or peptidomimetic thereof having nitrilase activity that selectively produces an *(R)*-enantiomer, so as to make *(R)*-ethyl 4-cyano-3-hydroxybutyric acid. In one embodiment, the *ee* is at least 95% or at least 99%. In another embodiment, the hydroxyglutaryl nitrile comprises 1,3-di-cyano-2-hydroxypropane or 3-hydroxyglutaronitrile. In a further embodiment, the polypeptide has an amino acid sequence of any one of the Group B amino acid sequences, or a fragment or peptidomimetic thereof having nitrilase activity.

The invention also provides a method for making an *(S)*-ethyl 4-cyano-3-hydroxybutyric acid, the method comprising contacting a hydroxyglutaryl nitrile with at least one polypeptide having an amino acid sequence of the Group B amino acid sequences, or a fragment or peptidomimetic thereof having nitrilase activity that selectively produces an *(S)*-enantiomer, so as to make *(S)*-ethyl 4-cyano-3-hydroxybutyric acid.

The invention provides a method for making an *(R)*-mandelic acid, the method comprising admixing a mandelonitrile with at least one polypeptide having an amino acid sequence of any one of the Group B amino acid sequences or any fragment or peptidomimetic thereof having appropriate nitrilase activity. In one embodiment, the *(R)*-mandelic acid comprises *(R)*-2-chloromandelic acid. In another embodiment, the *(R)*-mandelic acid comprises an aromatic ring substitution in the *ortho-, meta-*, or *para-* positions; a 1-naphthyl derivative of *(R)*-mandelic acid, a pyridyl derivative of *(R)*-mandelic acid or a thienyl derivative of *(R)*-mandelic acid or a combination thereof.

The invention provides a method for making an *(S)*-mandelic acid, the method comprising admixing a mandelonitrile with at least one polypeptide having an amino acid sequence of Group B sequences or any fragment or peptidomimetic thereof having nitrilase activity. In one embodiment, the *(S)*-mandelic acid comprises *(S)-*methyl benzyl cyanide and the mandelonitrile comprises *(S)*-methoxy-benzyl cyanide. In one embodiment, the *(S)*-mandelic acid comprises an aromatic ring substitution in the *ortho-, meta-,* or *para-* positions; a 1-naphthyl derivative of *(S)*-mandelic acid, a pyridyl derivative of *(S)*-mandelic acid or a thienyl derivative of *(S)*-mandelic acid or a combination thereof.

The invention also provides a method for making an *(S)*-phenyl lactic acid derivative or an *(R)*-phenyllactic acid derivative, the method comprising admixing a phenyllactonitrile with at least one polypeptide selected from the group of the Group B amino acid sequences or any active fragment or peptidomimetic thereof that selectively produces an *(S)*-enantiomer or an *(R)*-enantiomer, thereby producing an *(S)*-phenyl lactic acid derivative or an *(R)*-phenyl lactic acid derivative.

The invention provides for a method for making the polypeptide of the invention or a fragment thereof, the method comprising (a) introducing a nucleic acid encoding the polypeptide into a host cell under conditions that permit production of the polypeptide by the host cell, and (b) recovering the polypeptide so produced.

The invention provides for a method for generating a nucleic acid variant encoding a polypeptide having nitrilase activity, wherein the variant has an altered biological activity from that which naturally occurs, the method comprising (a) modifying the nucleic acid by (i) substituting one or more nucleotides for a different nucleotide, wherein the nucleotide comprises a natural or non-natural nucleotide; (ii) deleting one or more nucleotides, (iii) adding one or more nucleotides, or (iv) any combination thereof. In one embodiment, the non-natural nucleotide comprises inosine. In another embodiment, the method further comprises assaying the polypeptides encoded by the modified nucleic acids for altered nitrilase activity, thereby identifying the modified nucleic acid(s) encoding a polypeptide having altered nitrilase activity. In one embodiment, the modifications of step (a) are made by PCR, error-prone PCR, shuffling, oligonucleotide-directed mutagenesis, assembly PCR, sexual PCR mutagenesis, in vivo mutagenesis, cassette mutagenesis, recursive ensemble mutagenesis, exponential ensemble mutagenesis, site-specific mutagenesis, gene reassembly, gene site saturated mutagenesis, ligase chain reaction, *in vitro* mutagenesis, ligase chain reaction, oligonuclteotide synthesis, any DNA-generating technique and any combination thereof. In another embodiment, the method further comprises at least one repetition of the modification step (a).

The invention further provides a method for making a polynucleotide from two or more nucleic acids, the method comprising: (a) identifying regions of identity and regions of diversity between two or more nucleic acids, wherein at least one of the nucleic acids comprises a nucleic acid of the invention; (b) providing a set of oligonucleotides which correspond in sequence to at least two of the two or more nucleic acids; and, (c) extending the oligonucleotides with a polymerase, thereby making the polynucleotide.

The invention further provides a screening assay for identifying a nitrilase, the assay comprising: (a) providing a plurality of nucleic acids or polypeptides comprising at least one of the nucleic acids of the invention, or at least one of the polypeptides of the invention; (b) obtaining polypeptide candidates to be tested for nitrilase activity from the plurality; (c) testing the candidates for nitrilase activity; and (d) identifying those polypeptide candidates which are nitrilases. In one embodiment, the method further comprises modifying at least one of the nucleic acids or polypeptides prior to testing the candidates for nitrilase activity. In another embodiment, the testing of step (c) further comprises testing for improved expression of the polypeptide in a host cell or host organism. In a further embodiment, the testing of step (c) further comprises testing for nitrilase activity within a pH range from about pH 3 to about pH 12. In a further embodiment, the testing of step (c) further comprises testing for nitrilase activity within a pH range from about pH 5 to about pH 10. In another embodiment, the testing of step (c) further comprises testing for nitrilase activity within a temperature range from about 4 °C to about 80 °C. In another embodiment, the testing of step (c) further comprises testing for nitrilase activity within a temperature range from about 4 °C to about 55 °C. In another embodiment, the testing of step (c) further comprises testing for nitrilase activity which results in an enantioselective reaction product. In another embodiment, the testing of step (c ) further testing for nitrilase activity which results in a regio-selective reaction product.

The invention provides for use of the nucleic acids of the invention, or a fragment or peptidomimetic thereof having nitrilase activity, in a process designed to optimize an aspect of the gene or an aspect of the polypeptide encoded by the gene. In one embodiment, the process comprises introducing modifications into the nucleotide sequence of the nucleic acid. In another embodiment, the modifications are introduced by PCR, error-prone PCR, shuffling, oligonucleotide-directed mutagenesis, assembly PCR, sexual PCR mutagenesis, in vivo mutagenesis, cassette mutagenesis, recursive ensemble mutagenesis, exponential ensemble mutagenesis, site-specific mutagenesis, gene reassembly, gene site saturated mutagenesis, ligase chain reaction, *in nitro* mutagenesis, ligase chain reaction, oligonucleotide synthesis, any other DNA-generating technique or any combination thereof. In a further embodiment, the process is repeated.

The invention provides for use of the polypeptide of the invention, or a fragment or peptidomimetic thereof having nitrilase activity, in an industrial process. In one embodiment, the process is for production of a pharmaceutical composition, the process is for production of a chemical, the process is for production of a food additive, the process is catalyzing the breakdown of waste, or the process is production of a drug intermediate. In a further embodiment, the process comprises use of the polypeptide to hydrolyze a hydroxyglutarylnitrile substrate. In a further embodiment, the process is for production of LIPITOR™. In another embodiment, the polypeptide used comprises a polypeptide having consecutive amino acids of the sequence SEQ ID NO:44, 196, 208, 210, or 238 or a fragment thereof having nitrilase activity. In another embodiment, the process is production of a detergent. In another embodiment, the process is production of a food product.

The invention provides for use of a nucleic acid of the invention, or a fragment thereof encoding a polypeptide having nitrilase activity, in the preparation of a transgenic organism.

The invention provides for a kit comprising (a) the nucleic acid of the inventions, or a fragment thereof encoding a polypeptide having nitrilase activity, or (b) the polypeptide of the invention, or a fragment or a peptidomimetic thereof having nitrilase activity, or a combination thereof; and (c) a buffer.

The invention provides for a method for modifying a molecule comprising: (a) mixing a polypeptide of the invention or a fragment or peptidomimetic thereof having nitrilase activity, with a starting molecule to produce a reaction mixture; (b) reacting the starting molecule with the polypeptide to produce the modified molecule.

The invention provides for a method for identifying a modified compound comprising: (a) admixing a polypeptide of the invention, or a fragment or peptidomimetic thereof having nitrilase activity, with a starting compound to produce a reaction mixture and thereafter a library ofmodified starting compounds; (b) testing the library to determine whether a modified starting compound is present within the library which exhibits a desired activity; (c) identifying the modified compound exhibiting the desired activity.

The invention provides for a computer readable medium having stored thereon at least one nucleotide sequence selected from the group consisting of: SEQ ID NO: 1, 3, 5, 7, 9, 11, 13, 15, 17, 19, 21, 23, 25, 27, 29, 31, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 35, 137, 139,141, 143, 45, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, and 385 and/or at least one amino acid sequence selected from the group consisting of SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384 and 386.

The invention provides for a computer system comprising a processor and a data storage device, wherein the data storage device has stored thereon at least one nucleotide sequence selected from the group consisting of SEQ ID NO: 1, 3, 5, 7, 9, 11, 13, 15, 17, 19, 21, 23, 25, 27, 29, 31, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383 and 385 and/or at least one amino acid sequence selected from the group consisting of: SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384 and 386. In one embodiment, the computer system further comprises a sequence comparison algorithm and a data storage device having at least one reference sequence stored thereon. In another embodiment, the sequence comparison algorithm comprises a computer program that identifies polymorphisms.

The invention provides for a method for identifying a feature in a sequence which comprises: (a) inputting the sequence into a computer; (b) running a sequence feature identification program on the computer so as to identify a feature within the sequence; and (c) identifying the feature in the sequence, wherein the sequence comprises at least one of SEQ ID NOS:1-386 or any combination thereof.

The invention provides for an assay for identifying a functional fragment of a polypeptide which comprises: (a) obtaining a fragment of at least one polypeptide of the invention; (b) contacting at least one fragment from step (a) with a substrate having a cyanohydrin moiety or an aminonitrile moiety under reaction conditions suitable for nitrilase activity; (c) measuring the amount of reaction product produced by each at least one fragment from step (b); and (d) identifying the at least one fragment which is capable of producing a nitrilase reaction product; thereby identifying a functional fragment of the polypeptide. In one embodiment, the fragment of step (a) is obtained by synthesizing the fragment. In another embodiment, the fragment of step (a) is obtained by fragmenting the polypeptides. The invention provides for an assay for identifying a functional variant of a polypeptide which comprises: (a) obtaining at least one variant of at least one polypeptide of the invention; (b) contacting at least one variant from step (a) with a substrate having a cyanohydrin moiety or an aminonitrile moiety under reaction conditions suitable for nitrilase activity; (c) measuring the amount of reaction product produced by each at least one variant from step (b); and (d) identifying the at least one variant which is capable of producing a nitrilase reaction product; thereby identifying a functional variant of the polypeptide.

### BRIEF DESCRIPTION OF THE DRAWINGS

**Figure 1** shows chemical reaction schemes wherein stereoselective nitrilases hydrolyze a cyanohydrin or an amino nitrile to produce a chiral α-hydroxy acid or α-amino acid.

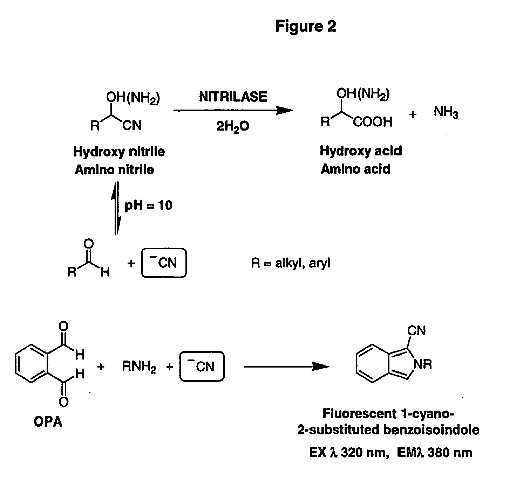

**Figure 2** illustrates an OPA based cyanide detection assay used for identifying the presence of nitrilase activity.
**Figure 3** is an illustration of a spectroscopic system for the detection and quantification of α-hydroxy acids based on stereoselective lactate dehydrogenases.
**Figure 4** is an illustration of a spectroscopic system for the detection and quantification of α-amino acids based on stereoselective amino acid oxidase.
**Figure 5** is a flow diagram illustrating the steps of a nitrilase screening method.
**Figures 6A-6E** are chromatograms characteristic of the substrate and product combination for D-phenylglycine showing a blank sample (Fig. 6A), an enzymatic reaction sample (Fig. 6B); a negative control consisting of cell lysate in buffer (Fig. 6C); a chiral analysis of phenylglycine (Fig. 6D); and coelution of the nitrile peak with the D-enantiomer (Fig. 6E).
**Figures 7A-7E** illustrate chromatograms which are characteristic of substrate and product combinations for (R)-2-chloromandelic acid. Fig. 7A shows only 2-chloromandelonitrile in buffer; Fig. 7B shows a cloromandelic acid standard. The chromatogram in Fig. 7C shows the appearance of product and the reduction of substrate peaks.
**Figures 8A-8B** illustrate chromatograms characteristic of substrate and product combinations for (*S*)-phenyllactic acid.
**Figures 9A-9B** illustrate chromatograms characteristic of substrate and product combinations for L-2-methylphenylglycine.
**Figures 10A-10C** illustrate chromatograms characteristic of substrate and product combinations for L-*tert*-leucine.
**Figures 11A-11C** illustrate chromatograms characteristic of substrate and product combinations for (S)-2-amino-6-hydroxy hexanoic acid.
**Figures 12A-12D** illustrate chromatograms characteristic of substrate and product combinations for 4-methyl-D-leucine and 4-methyl-L-leucine.
**Figures 13A-13B** illustrate chromatograms characteristic of substrate and product combinations for (*S*)-cyclohexylmandelic acid.

### DETAILED DESCRIPTION OF THE INVENTION

The present invention relates to nitrilases, nucleic acids encoding nitrilases, and uses therefor. As used herein, the term "nitrilase" encompasses any polypeptide having nitrilase activity, *i.e*., the ability to hydrolyze nitriles into their corresponding carboxylic acids and ammonia. Nitrilases have commercial utility as biocatalysts for use in the synthesis of enantioselective aromatic and aliphatic amino acids or hydroxy acids.

Nitrilase chemistry is as follows:

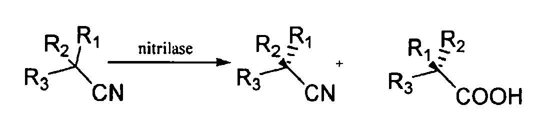

A nitrilase reaction for the preparation of hydroxy acids is as follows:

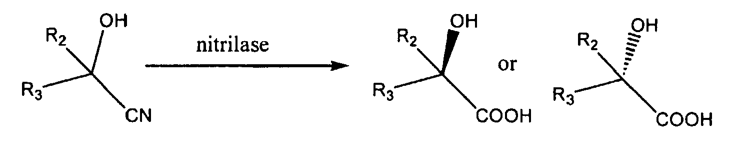

A nitrilase reaction for the preparation of amino acids is as follows:

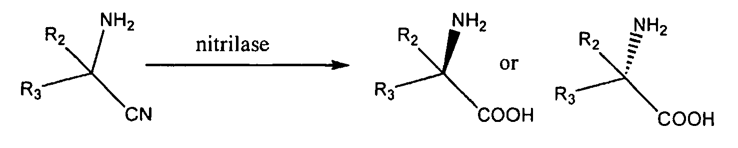

In addition, in each of the foregoing hydrolysis reactions, two water molecules are consumed and one ammonia molecule is released.

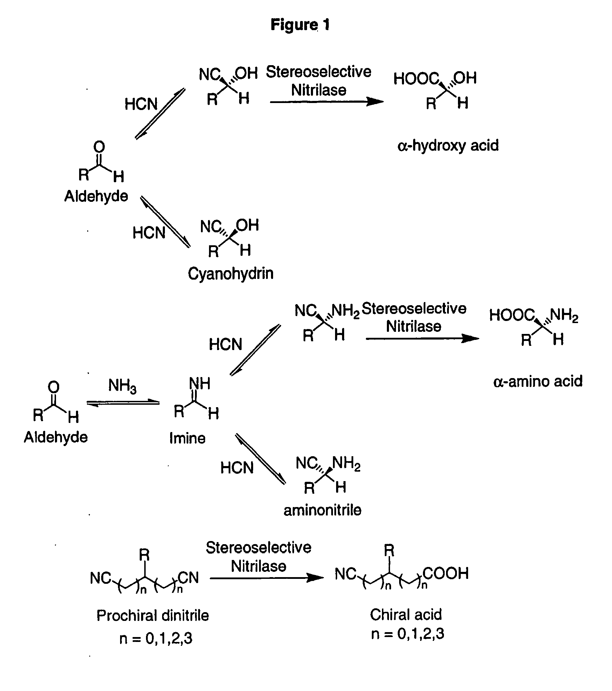

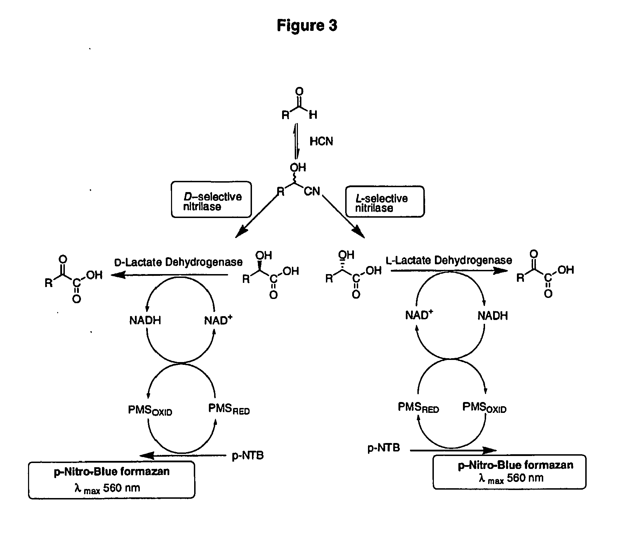

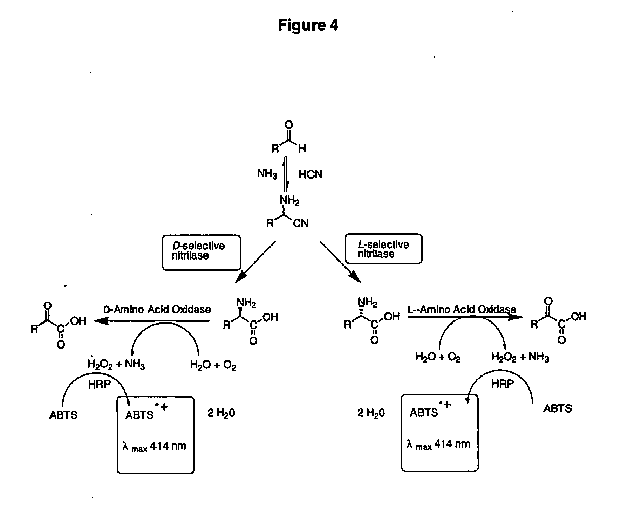

There are several different types of assays which can be performed to test for the presence of nitrilase activity in a sample or to test whether a particular polypeptide exhibits nitrilase activity. For example, assays can detect the presence or absence of products or by-products from a chemical reaction catalyzed by a nitrilase. For example, the presence of nitrilase activity can be detected by the production of α-hydroxy acids or α-amino acids from, respectively, cyanohydrins or aminonitriles, and the level of nitrilase activity can be quantified by measuring the relative quantities of the reaction products produced. Figure 1 shows chemical reaction schemes using stereoselective nitrilases to create chiral α-hydroxy acids or α-amino acids in high yield. The starting material is an aldehyde or an imine which is produced from an aldehyde by reaction with ammonia. Reaction of the aldehyde or imine with hydrogen cyanide results in the production of enantiomeric mixtures of the corresponding cyanohydrins and aminonitriles. A stereoselective nitrilase can then be used to stereoselectively convert one enantiomer into the corresponding α-hydroxy acid or α-amino acid. Figure 3 illustrates schematically the stereoselective nitrilase-dependent production and spectrophotometric detection of α-hydroxy acids based on lactate dehydrogenase conversion of the α-hydroxy acids to the corresponding α-keto acids and concomitant oxidation-reduction of a detectable dye. Figure 4 illustrates schematically the stereoselective nitrilase-dependent production and spectrophotometric detection of α-amino acids based on amino acid oxidase conversion of the α-amino acids to the corresponding α-keto acids and concomitant oxidation-reduction of a detectable dye.

Nitrilases contemplated for use in the practice of the present invention include those which stereoselectively hydrolyze nitriles or cyanohydrins into their corresponding acids and ammonia. Nitrilases include, for example, those set forth in the Group B amino acid sequences). Some nitrilases which stereoselectively hydrolyze their substrates are set forth in the Tables hereinbelow.

The nitrilases of the invention share the following additional characteristics: (1) full-length amino acid sequences from about 333 amino acids to about 366 amino acids, (2) aggregation and activity as homo-multimers of about 2 subunits to about 16 subunits, (3) presence of a catalytic triad of the consecutive amino acids Glu-Lys-Cys, (4) pH optima from about pH 5 to about pH 9, and (5) temperature optima from about 0°C to about 100°C, or from about 40°C to about 50°C.

### Consensus Sequences Among New Nitrilases

The nitrilases disclosed herein were studied using bioinformatics and sequence comparison programs and the following consensus information was collected. Three regions of conserved motifs were identified within the nitrilase polypeptides. These correspond to the catalytic triad (E-K-C) present in nitrilase enzymes. (H. Pace and C. Brenner (Jan. 15, 2001) "The Nitrilase Superfamily: classification, structure and function" Genome Biology Vol. 2, No. 1, pp 1-9.)

The abbreviations used herein are conventional one letter codes for the amino acids: A, alanine; B, asparagine or aspartic acid; C, cysteine; D aspartic acid; E, glutamate, glutamic acid; F, phenylalanine; G, glycine; H histidine; I isoleucine; K, lysine; L, leucine; M, methionine; N, asparagine; P, proline; Q, glutamine; R, arginine; S, serine; T, threonine; V, valine; W, tryptophan; Y, tyrosine; Z, glutamine or glutamic acid. See L. Stryer, Biochemistry, 1988, W. H. Freeman and Company, New York.

The computer sequence comparisons made among the nitrilase polypeptide sequences of the invention resulted in the identification of these motifs within each amino acid sequence:

| | | | | | | | | | | | | | | | | | | | | | | |
|---|---|---|---|---|---|---|---|---|---|---|---|---|---|---|---|---|---|---|---|---|---|---|
| F | P | E | t | *f* | | r | R | K | L | . | P | T | | L | . | C | W | E | h | . | . | P |

The following residues (those that are underlined) are completely conserved among all of the identified nitrilases: the third amino acid in the first motif or region (E, glutamate); the second residue in the second motif (R, arginine); the third residue in the second motif (K, lysine); the third residue in the third motif (C, cysteine); and the fifth residue in the third motif (E, glutamate).

In the boxes, upper case letters indicate 90% or greater consensus among the nitrilases of the invention, while lower case letters indicate 50% or greater consensus. An italicized letter indicates 30% or greater consensus among the nitrilases of the invention. A dot in a box indicates a residue which is not conserved.

The sequences of nitrilases in the nitrilase branch of the nitrilase superfamily were described as having a catalytic triad in the Pace and Brenner article (Genome Biology, 2001, Vol. 2, No. 1, pp. 1-9). However, the catalytic triad regions of the nitrilases of this invention differ from those previously identified in the Pace and Brenner reference in the following ways:
Differences in the first motif: The F in the first box of the first motif is conserved in 90% of the nitrilases of the invention, rather than in only 50% of those previously identified. The fourth residue of the first motif is a "t", threonine in the nitrilases of this invention, and it is found at 50% or greater consensus. However, that residue was identified by Pace and Brenner as "a" (alanine). The last residue of the first motif was identified as "f" (phenylalanine) and was indicated to occur at 50% or greater consensus. However, the nitrilases of this invention only show "f" (phenylalanine occurring at 30% consensus.
Differences in the second motif: There is an "r" (arginine) in the first box of the second motif of the nitrilases of this invention. However, the Pace and Brenner consensus shows an "h" (histidine) in that position. The "R" (arginine) in the second box is completely conserved in the nitrilases of the present invention, however that residue only appears at 90% consensus in the Pace and Brenner reference. The "L" (leucine) in the fourth box of the second motif is conserved in 90% or more of the nitrilases of this invention. However, the Pace and Brenner nitrilases only showed conservation of that residue in 50% of the sequences. Similarly, the "P" (proline) at the sixth box of the second motif is conserved in 90% or more of the nitrilases of this invention. However, the Pace and Brenner nitrilases only showed conservation of that residue in 50% of the sequences.
Differences in the third motif: The "L in the first box is conserved at 90% or greater in the nitrilases of the invention. However, the Pace and Brenner reference only shows that residue appearing 50% of the time. Finally, the sixth box in the third motif in the nitrilases of the invention show a histidine 50% of the time or more. However, the Pace and Brenner reference indicates that that position shows an asparagine ("n") 50% of the time.

The invention provides for an isolated polypeptide having nitrilase activity which polypeptide comprises three regions, wherein the first region comprises five amino acids and wherein the first amino acid of the first region is F and the fourth amino acid of the first region is T. The invention also provides for an isolated polypeptide having nitrilase activity which polypeptide comprises three regions, wherein the second region comprises seven amino acids and wherein the first amino acid of the second region is R, wherein the second amino acid of the second region is R, and wherein the sixth amino acid of the second region is P. The invention also provides for an isolated polypeptide having nitrilase activity which polypeptide comprises three regions, wherein the third region comprises nine amino acids and wherein the first amino acid of the third region is L and the sixth amino acid of the third region is H.

The invention also provides for an isolated polypeptide having nitrilase activity which polypeptide comprises three consenus subsequences, wherein the first consensus subsequence is FPETF, wherein the second consensus subsequence is RRKLXPT, and wherein the third consensus subsequence is LXCWEHXXP.

The invention also provides for an isolated polypeptide having nitrilase activity which polypeptide comprises three consenus subsequences, wherein the first consensus subsequence is FPEXX, wherein the second consensus subsequence is XRKLXPT, and wherein the third consensus subsequence is LXCWEXXXP.

In accordance with the present invention, methods are provided for producing enantiomerically pure α-substituted carboxylic acids. The enantiomerically pure α-substituted carboxylic acids produced by the methods of the present invention have the following structure: 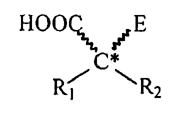 wherein:
R₁ ≠ R₂ and R₁ and R₂ are otherwise independently -H, substituted or unsubstituted alkyl, alkenyl, alkynyl, aryl, heteroaryl, cycloalkyl, or heterocyclic, wherein said substituents are lower alkyl, hydroxy, alkoxy, amino, mercapto, cycloalkyl, heterocyclic, aryl, heteroaryl, aryloxy, or halogen or optionally R₁ and R₂ are directly or indirectly covalently joined to form a functional cyclic moiety, and E is -N(Rₓ)₂ or - OH, wherein each Rₓ is independently -H or lower alkyl.

As used herein, the term "alkyl" refers to straight or branched chain or cyclic hydrocarbon groups of from 1 to 24 carbon atoms, including methyl, ethyl, n-propyl, isopropyl, n-butyl, isobutyl, tert-butyl, n-pentyl, n-hexyl, and the like. The term "lower alkyl" refers to monovalent straight or branched chain or cyclic radicals of from one to about six carbon atoms.

As used herein, "alkenyl" refers to straight or branched chain or cyclic hydrocarbon groups having one or more carbon-carbon double bonds, and having in the range of about 2 to about 24 carbon atoms.

As used herein, "alkynyl" refers to straight or branched chain or cyclic hydrocarbon groups having at least one carbon-carbon triple bond, and having in the range of about 2 to about 24 carbon atoms.

As used herein, "cycloalkyl" refers to cyclic hydrocarbon groups containing in the range of about 3 to about 14 carbon atoms.

As used herein, "heterocyclic" refers to cyclic groups containing one or more heteroatoms (e.g., N, O, S, P, Se, B, etc.) as part of the ring structure, and having in the range of about 3 to about 14 carbon atoms.

As used herein, "aryl" refers to aromatic groups (*i.e*., cyclic groups with conjugated double-bond systems) having in the range of about 6 to about 14 carbon atoms.

As used herein with respect to a chemical group or moiety, the term "substituted" refers to such a group or moiety further bearing one or more non-hydrogen substituents. Examples of such substituents include, without limitation, oxy (e.g., in a ketone, aldehyde, ether, or ester), hydroxy, alkoxy (of a lower alkyl group), amino, thio, mercapto (of a lower alkyl group), cycloalkyl, substituted cycloalkyl, heterocyclic, substituted heterocyclic, aryl, substituted aryl, heteroaryl, substituted heteroaryl, aryloxy, substituted aryloxy, halogen, trifluoromethyl, cyano, nitro, nitrone, amino, amido, -C(O)H, acyl, oxyacyl, carboxyl, carbamate, sulfonyl, sulfonamide, sulfuryl, and the like.

In preferred embodiments, the enantiomerically pure α-substituted carboxylic acid produced by the methods of the present invention is an α-amino acid or α-hydroxy acid. In some embodiments, the enantiomerically pure α-amino acid is D-phenylalanine, D-phenylglycine, L-methylphenylglycine, L-*tert*-leucine, D-alanine, or D-hydroxynorleucine ((S)-2-amino-6-hydroxy hexanoic acid), R-pantolactone, 2-chloromandelic acid, or (*S*)- or *(R)*-mandelic acid and the enantiomerically pure α-hydroxy acid is (*S*)-cyclohexylmandelic acid. As used herein, a "small molecule" encompasses any molecule having a molecular weight from at least 25 Daltons.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent up or down (higher or lower).

As used herein, the word "or" means any one member of a particular list and also includes any combination of members of that list.

The phrase "nucleic acid" as used herein refers to a naturally occurring or synthetic oligonucleotide or polynucleotide, whether DNA or RNA or DNA-RNA hybrid, single-stranded or double-stranded, sense or antisense, which is capable of hybridization to a complementary nucleic acid by Watson-Crick base-pairing. Nucleic acids of the invention can also include nucleotide analogs (*e.g*., BrdU), and non-phosphodiester internucleoside linkages (*e.g*., peptide nucleic acid (PNA) or thiodiester linkages). In particular, nucleic acids can include, without limitation, DNA, RNA, cDNA, gDNA, ssDNA or dsDNA or any combination thereof. In some embodiments, a "nucleic acid" of the invention includes, for example, a nucleic acid encoding a polypeptide as set forth in the Group B amino acid sequences, and variants thereof. The phrase "a nucleic acid sequence" as used herein refers to a consecutive list of abbreviations, letters, characters or words, which represent nucleotides. In one embodiment, a nucleic acid can be a "probe" which is a relatively short nucleic acid, usually less than 100 nucleotides in length. Often a nucleic acid probe is from about 50 nucleotides in length to about 10 nucleotides in length. A "target region" of a nucleic acid is a portion of a nucleic acid that is identified to be of interest.

A "coding region" of a nucleic acid is the portion of the nucleic acid which is transcribed and translated in a sequence-specific manner to produce into a particular polypeptide or protein when placed under the control of appropriate regulatory sequences. The coding region is said to encode such a polypeptide or protein.

The term "gene" refers to a coding region operably joined to appropriate regulatory sequences capable of regulating the expression of the polypeptide in some manner. A gene includes untranslated regulatory regions of DNA (e.g., promoters, enhancers, repressors, etc.) preceding (upstream) and following (downstream) the coding region (open reading frame, ORF) as well as, where applicable, intervening sequences (*i*.*e*., introns) between individual coding regions (*i*.*e*., exons).

"Polypeptide" as used herein refers to any peptide, oligopeptide, polypeptide, gene product, expression product, or protein. A polypeptide is comprised of consecutive amino acids. The term "polypeptide" encompasses naturally occurring or synthetic molecules.

In addition, as used herein, the term "polypeptide" refers to amino acids joined to each other by peptide bonds or modified peptide bonds, e.g., peptide isosteres, and may contain modified amino acids other than the 20 gene-encoded amino acids. The polypeptides can be modified by either natural processes, such as post-translational processing, or by chemical modification techniques which are well known in the art. Modifications can occur anywhere in the polypeptide, including the peptide backbone, the amino acid side-chains and the amino or carboxyl termini. It will be appreciated that the same type of modification can be present in the same or varying degrees at several sites in a given polypeptide. Also a given polypeptide can have many types of modifications. Modifications include, without limitation, acetylation, acylation, ADP-ribosylation, amidation, covalent cross-linking or cyclization, covalent attachment of flavin, covalent attachment of a heme moiety, covalent attachment of a nucleotide or nucleotide derivative, covalent attachment of a lipid or lipid derivative, covalent attachment of a phosphytidylinositol, disulfide bond formation, demethylation, formation of cysteine or pyroglutamate, formylation, gamma-carboxylation, glycosylation, GPI anchor formation, hydroxylation, iodination, methylation, myristolyation, oxidation, pergylation, proteolytic processing, phosphorylation, prenylation, racemization, selenoylation, sulfation, and transfer-RNA mediated addition of amino acids to protein such as arginylation. (See Proteins - Structure and Molecular Properties 2nd Ed., T.E. Creighton, W.H. Freeman and Company, New York (1993); Posttranslational Covalent Modification of Proteins, B.C. Johnson, Ed., Academic Press, New York, pp. 1-12 (1983)).

As used herein, the term "amino acid sequence" refers to a list of abbreviations, letters, characters or words representing amino acid residues.

As used herein, the term "isolated" means that a material has been removed from its original environment. For example, a naturally-occurring polynucleotide or polypeptide present in a living animal is not isolated, but the same polynucleotide or polypeptide, separated from some or all of the coexisting materials in the natural system, is isolated. Such polynucleotides can be part of a vector and/or such polynucleotides or polypeptides could be part of a composition, and would be isolated in that such a vector or composition is not part of its original environment.

As used herein with respect to nucleic acids, the term "recombinant" means that the nucleic acid is covalently joined and adjacent to a nucleic acid to which it is not adjacent in its natural environment. Additionally, as used herein with respect to a particular nucleic acid in a population of nucleic acids, the term "enriched" means that the nucleic acid represents 5% or more of the number of nucleic acids in the population of molecules. Typically, the enriched nucleic acids represent 15% or more of the number of nucleic acids in the population of molecules. More typically, the enriched nucleic acids represent 50%, 90% or more of the number of nucleic acids in the population molecules.

"Recombinant" polypeptides or proteins refer to polypeptides or proteins produced by recombinant DNA techniques, *i.e*., produced from cells transformed by an exogenous recombinant DNA construct encoding the desired polypeptide or protein. "Synthetic" polypeptides or proteins are those prepared by chemical synthesis (*e.g*., solid-phase peptide synthesis). Chemical peptide synthesis is well known in the art (see, *e.g*., Merrifield (1963), Am. Chem. Soc. 85:2149-2154; Geysen et al. (1984), Proc. Natl. Acad. Sci., USA 81:3998) and synthesis kits and automated peptide synthesizer are commercially available (*e.g*., Cambridge Research Biochemicals, Cleveland, United Kingdom; Model 431A synthesizer from Applied Biosystems, Inc., Foster City, CA). Such equipment provides ready access to the peptides of the invention, either by direct synthesis or by synthesis of a series of fragments that can be coupled using other known techniques.

As used herein with respect to pairs of nucleic acid or amino acid sequences, "identity" refers to the extent to which the two sequences are invariant at positions within the sequence which can be aligned. The percent identity between two given sequences can be calculated using an algorithm such as BLAST (Altschul et al. (1990), J. Mol. Biol. 215:403-410). See www.ncbi.nlm.nih.gov/Education/BLASTinfo. When using the BLAST algorithm for sequences no longer than 250 nucleotides or about 80 amino acids ("short queries"), the search parameters can be as follows: the filter is off, the scoring matrix is PAM30, the word size is 3 or 2, the E value is 1000 or more, and the gap costs are 11, 1. For sequences longer than 250 nucleotides or 80 amino acid residues, the default search parameters can be used. The BLAST website provides advice for special circumstances which is to be followed in such circumstances.

As used herein, "homology" has the same meaning as "identity" in the context of nucleotide sequences. However, with respect to amino acid sequences, "homology" includes the percentage of identical and conservative amino acid substitutions. Percentages of homology can be calculated according to the algorithms of Smith and Waterman (1981), Adv. Appl. Math. 2:482.

As used herein in the context of two or more nucleic acid sequences, two sequences are "substantially identical" when they have at least 99.5% nucleotide identity, when compared and aligned for maximum correspondence, as measured using the known sequence comparison algorithms described above. In addition, for purposes of determining whether sequences are substantially identical, synonymous codons in a coding region may be treated as identical to account for the degeneracy of the genetic code. Typically, the region for determination of substantial identity must span at least about 20 residues, and most commonly the sequences are substantially identical over at least about 25-200 residues.

As used herein in the context of two or more amino acid sequences, two sequences are "substantially identical" when they have at least 99.5% identity, when compared and aligned for maximum correspondence, as measured using the known sequence comparison algorithms described above. In addition, for purposes of determining whether sequences are substantially identical, conservative amino acid substitutions may be treated as identical if the polypeptide substantially retains its biological function.

"Hybridization" refers to the process by which a nucleic acid strand joins with a complementary strand through hydrogen bonding at complementary bases. Hybridization assays can be sensitive and selective so that a particular sequence of interest can be identified even in samples in which it is present at low concentrations. Stringent conditions are defined by concentrations of salt or formamide in the prehybridization and hybridization solutions, or by the hybridization temperature, and are well known in the art. Stringency can be increased by reducing the concentration of salt, increasing the concentration of formamide, or raising the hybridization temperature. In particular, as used herein, "stringent hybridization conditions" include 42°C in 50% formamide, 5X SSPE, 0.3% SDS, and 200 ng/ml sheared and denatured salmon sperm DNA, and equivalents thereof Variations on the above ranges and conditions are well known in the art.

The term "variant" refers to polynucleotides or polypeptides of the invention modified at one or more nucleotides or amino acid residues (respectively) and wherein the encoded polypeptide or polypeptide retains nitrilase activity. Variants can be produced by any number of means including, for example, error-prone PCR, shuffling, oligonucleotide-directed mutagenesis, assembly PCR, sexual PCR mutagenesis, *in vivo* mutagenesis, cassette mutagenesis, recursive ensemble mutagenesis, exponential ensemble mutagenesis, site-specific mutagenesis, gene reassembly, gene site-saturated mutagenesis or any combination thereof.

Methods of making peptidomimetics based upon a known sequence is described, for example, in U.S. Patent Nos. 5,631,280; 5,612,895; and 5,579,250. Use of peptidomimetics can involve the incorporation of a non-amino acid residue with non-amide linkages at a given position. One embodiment of the present invention is a peptidomimetic wherein the compound has a bond, a peptide backbone or an amino acid component replaced with a suitable mimic. Examples of unnatural amino acids which may be suitable amino acid mimics include β-alanine, L-α-amino butyric acid, L-γ-amino butyric acid, L-α-amino isobutyric acid, L-ε-amino caproic acid, 7-amino heptanoic acid, L-aspartic acid, L-glutamic acid, N-ε-Boc-N-α-CBZ-L-lysine, N-ε-Boc-N-α-Fmoc-L-lysine, L-methionine sulfone, L-norleucine, L-norvaline, N-α-Boc-N-δCBZ-L-ornithine, N-δ-Boc-N-α-CBZ-L-ornithine, Boc-p-nitro-L-phenylalanine, Boc-hydroxyproline, Boc-L-thioproline.

As used herein, "small molecule" encompasses a molecule having a molecular weight from about 20 Daltons to about 1.5 kiloDaltons.

The molecular biological techniques, such as subcloning, were performed using routine methods which would be well known to one of skill in the art. (Sambrook, J. Fritsch, EF, Maniatis, T. (1989) Molecular Cloning: A Laboratory Manual (2nd ed.), Cold Spring Harbor Laboratory Press, Plainview NY.).

**Table 2 includes the SEQ ID NOS, the Closest Hit (BLAST) Organism, the Closest Hit (BLAST) percentage amino acid identity and the percent nucleotide identity for the nitrilases of the present invention.**

| **SEQ ID NO:** | **Top Public Hit** | **Public E-Value** | **Public Definition** | **Public Organism** | **Public EC Number** | **Public Protein % Identity** | **Public Nucleotide % Identity** | **Length of SEQ ID NO. (# of Amino Acids)** | **Public Sequence Length** |
|---|---|---|---|---|---|---|---|---|---|
| 1, 2 | gi\|15229 | 0.00000 | nitrilase 1 | Arabidopsis | N/A | 20 | N/A | 312 | 346 |
| | 934\|ref\|NP_190 017.1\| | 006 | [Arabidopsisthaliana]. | thaliana Eukaryota | | | | | |
| 3, 4 | gi\|14211 | 8E-56 | nitrilase-like | Brassicanapus | N/A | 41 | 51 | 326 | 350 |
| | 396\|gb\| AAK57 | | protein | Eukaryota | | | | | |
| | 436.1\| | | [Brassicanapus]. | | | | | | |
| 5, 6 | gi\|15143 | e-113 | unnamed | unidentified | N/A | 62 | 67 | 334 | 346 |
| | 035\|emb | | protein | | | | | | |
| | \|CAC50 | | product | unclassified. | | | | | |
| | 776.1\| | | [unidentified]. | | | | | | |
| 7, 8 | gi\|74359 | 9E-63 | nitrilase | Nicotiana | 3.5.5.1 | 41 | 50 | 332 | 348 |
| | 80\|pir\|\|T | | (EC 3.5.5.1) | tabacum | | | | | |
| | 03739 | | 4B - common | Eukaryota | | | | | |
| | | | tobacco. | | | | | | |
| 9, 10 | gi\|74359 | 1E-55 | nitrilase | Nicotiana tabacum | 3.5.5.1 | 39 | 48 | 314 | 348 |
| | 80\|pir\|\|T 03739 | | (EC 3.5.5.1) | Eukaryota | | | | | |
| | | | 4B - common | | | | | | |
| | | | tobacco. | | | | | | |
| 11, 12 | gi\|15795 | 5E-42 | unnamed | Rhodococcus | N/A | 32 | 46 | 321 | 366 |
| | 659\|emb | | protein | | | | | | |
| | \|CAC88 | | product | rhodochrous | | | | | |
| | 237.1\| | | [Rhodococcus rhodochrous]. | Bacteria | | | | | |
| 13, 14 | gi\|15143 | e-109 | unnamed | unidentified | N/A | 62 | 66 | 337 | 346 |
| | 035\|emb | | protein | | | | | | |
| | \|CAC50 | | product | unclassifi ed. | | | | | |
| | 776.1\| | | [unidentified]. | | | | | | |
| 15, 16 | gi\|15143 | e-131 | unnamed | unidentified | N/A | 65 | 64 | 348 | 346 |
| | 035\|emb | | protein | | | | | | |
| | \|CAC50 | | product | unclassified. | | | | | |
| | 776.1\| | | [unidentified]. | | | | | | |
| 17, 18 | gi\|74359 | e-100 | nitrilase | Synechocystis sp. | 3.5.5.1 | 52 | 58 | 330 | 346 |
| | 78\|pir\|\|S 77025 | | (EC 3.5.5.1) - | Bacteria | | | | | |
| | | | Synechocystis sp. | | | | | | |
| | | | (strain PCC 6803). | | | | | | |
| 19, 20 | gi\|15143 | e-107 | unnamed | unidentified | N/A | 55 | 60 | 349 | 346 |
| | 035\|emb | | protein | | | | | | |
| | \|CAC50 | | product | unclassified. | | | | | |
| | 776.1\| | | [unidentified]. | | | | | | |
| 21, 22 | gi\|15143 | e-111 | unnamed | unidentified | N/A | 59 | 63 | 354 | 346 |
| | 035\|emb | | protein | | | | | | |
| | \|CAC50 | | product | | | | | | |
| | 776.1\| | | [unidentified]. | | | | | | |
| 23, 24 | gi\|74359 | e-101 | nitrilase | Synechocystis sp. | 3.5.5.1 | 55 | 57 | 334 | 346 |
| | 78\|pir\|\|S 77025 | | (EC 3.5.5.1)- | Bacteria | | | | | |
| | | | Synechocystis sp. | | | | | | |
| | | | (strain PCC 6803). | | | | | | |
| 25, 26 | gi\|48355 | 2E-50 | nitrilase-like | Oryzasativa Eukaryota | N/A | 38 | 48 | 312 | 362 |
| | 88\|dbj\|B AA7767 9.1\| | | protein | | | | | | |
| | | | [Oryzasativa]. | | | | | | |
| 27, 28 | gi\|15242 | 4E-62 | Nitrilase 4 | Arabidopsis | N/A | 38 | N/A | 351 | 355 |
| | 205\|ref\| NP_197 | | (sp P46011) | thaliana | | | | | |
| | 622.1\| | | [Arabidopsis | Eukaryota | | | | | |
| | | | thaliana]. | | | | | | |
| 29, 30 | gi\|15143 | e-106 | unnamed | unidentified | N/A | 61 | 66 | 338 | 346 |
| | 035\|emb | | protein | | | | | | |
| | \|CAC50 | | product | unclassified. | | | | | |
| | 776.1\| | | [unidentified]. | | | | | | |
| 31, 32 | gi\|14211 | 3E-51 | nitrilase-like | Brassicanapus | N/A | 38 | 49 | 310 | 350 |
| | 396\|gb\| AAK57 | | protein | Eukaryota | | | | | |
| | 436.1\| | | [Brassicanapus]. | | | | | | |
| 33, 34 | gi\|15143 | e-150 | unnamed | unidentified | N/A | 80 | 78 | 341 | 346 |
| | 035\|emb | | protein | | | | | | |
| | \|CAC50 | | product | unclassified. | | | | | |
| | 776.1\| | | [unidentified]. | | | | | | |
| 35, 36 | gi\|15902 | 0.00000 | Beta- | Streptococcus | N/A | 21 | 41 | 313 | 291 |
| | 867\|ref\| | 002 | alanine | | | | | | |
| | NP_358 | | synthase | pneumoniae | | | | | |
| | 417.1\| | | or beta- | R6 | | | | | |
| | | | ureidopro | Bacteria | | | | | |
| | | | pionase | | | | | | |
| | | | [Streptococcus | | | | | | |
| | | | pneumoniae | | | | | | |
| | | | R6]. | | | | | | |
| 37, 38 | gi\|15143 | 2E-56 | unnamed | unidentified | N/A | 39 | 47 | 330 | 337 |
| | 037\|emb | | protein | | | | | | |
| | \|CAC50 | | product | unclassified. | | | | | |
| | 777.1\| | | [unidentified]. | | | | | | |
| 39, 40 | gi\|74359 | 1E-97 | nitrilase | Synechocystis sp. | 3.5.5.1 | 50 | 58 | 335 | 346 |
| | 78\|pir\|\|S 77025 | | (EC 3.5.5.1)- | Bacteria | | | | | |
| | | | Synechoc ystis sp. | | | | | | |
| | | | (strain PCC 6803). | | | | | | |
| 41, 42 | gi\|66248 | 2E-60 | nitrilase 2 | Arabidopsis | N/A | 41 | 52 | 321 | 339 |
| | 86\|emb\| | | [Arabidopsis | | | | | | |
| | CAA68 | | | thaliana | | | | | |
| | 934.3\| | | thaliana]. | Eukaryota | | | | | |
| 43, 44 | gi\|74359 | 8E-96 | nitrilase | Synechoc | 3.5.5.1 | 51 | 58 | 330 | 346 |
| | 78\|pir\|\|S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain PCC 6803). | | | | | | |
| 45, 46 | gi\|48355 | 6E-54 | nitrilase-like | Oryzasativa | N/A | 39 | 51 | 331 | 362 |
| | 88\|dbj\|B AA7767 | | protein | Eukaryota | | | | | |
| | 9.1\| | | [Oryzasativa]. | | | | | | |
| 47, 48 | gi\|15143 | e-146 | unnamed | unidentified | N/A | 74 | 68 | 337 | 337 |
| | 037\|emb | | protein | | | | | | |
| | \|CAC50 | | product | unclassified. | | | | | |
| | 777.1\| | | [unidentified]. | | | | | | |
| 49, 50 | gi\|74359 | e-106 | nitrilase | Synechocystis sp. | 3.5.5.1 | 59 | 58 | 345 | 346 |
| | 78\|pir\|\|S 77025 | | (EC 3.5.5.1) - | Bacteria | | | | | |
| | | | Synechocystis sp. | | | | | | |
| | | | (strain PCC 6803). | | | | | | |
| 51, 52 | gi\|75109 | 5E-47 | probable | Caenorhabditis | 3.5.5.1 | 36 | 48 | 298 | 305 |
| | 01\|pir\|\|T | | nitrilase | | | | | | |
| | 27679 | | (EC 3.5.5.1) | elegans | | | | | |
| | | | ZK1058.6 | Eukaryota | | | | | |
| | | | Caenorhabditis elegans. | | | | | | |
| 53, 54 | gi\|74359 | 5E-61 | nitrilase | Nicotiana tabacum | 3.5.5.1 | 41 | 51 | 317 | 348 |
| | 80\|pir\|\|T03739 | | (EC .5.5.1) | Eukaryota | | | | | |
| | | | 4B - common tobacco. | | | | | | |
| 55, 56 | gi\|15143 | e-111 | unnamed | unidentified | N/A | 60 | 67 | 338 | 346 |
| | 035\|emb | | protein | | | | | | |
| | \|CAC50 | | product | unclassified. | | | | | |
| | 776.1\| | | [unidentified]. | | | | | | |
| 57,58 | gi\|15143 | e-146 | unnamed | unidentified | N/A | 74 | 68 | 337 | 337 |
| | 037\|emb | | protein | | | | | | |
| | \|CAC50 777.1\| | | product | unclassified. | | | | | |
| | | | [unidentified]. | | | | | | |
| 59, 60 | gi\|22940 | 9E-50 | unnamed | unidentified | N/A | 35 | 49 | 328 | 354 |
| | 01\|emb\| | | protein | | | | | | |
| | CAA02 | | product | unclassified. | | | | | |
| | 248.1\| | | [unidentified]. | | | | | | |
| 61, 62 | gi\|15795 | 2E-43 | unnamed | Rhodococcus | N/A | 33 | 46 | 321 | 366 |
| | 659\|emb | | protein | | | | | | |
| | \|CAC88 | | product | rhodochrous | | | | | |
| | 237.1\| | | [Rhodococcus | Bacteria | | | | | |
| | | | rhodochrous]. | | | | | | |
| 63, 64 | gi\|48355 | 6E-67 | nitrilase-like | Oryzasativa | N/A | 44 | 51 | 325 | 362 |
| | 88\|dbj\|B AA7767 | | protein | Eukaryota | | | | | |
| | 9.1\| | | [Oryzasativa]. | | | | | | |
| 65, 66 | gi\|39141 | 5E-63 | NITRILASE 4. | Nicotiana | N/A | 43 | 49 | 333 | 349 |
| | 63\|sp\|Q4 | | | tabacum | | | | | |
| | 2965\|N | | | Eukaryota | | | | | |
| | RL4_T | | | | | | | | |
| | OBAC | | | | | | | | |
| 67, 68 | gi\|15242 | 1E-50 | Nitrilase 4 | Arabidopsis | N/A | 39 | N/A | 311 | 355 |
| | 205\|ref\| | | | | | | | | |
| | NP_197 | | (sp P46011) | thaliana | | | | | |
| | 622.1\| | | [Arabidopsis thaliana]. | Eukaryota | | | | | |
| 69, 70 | gi\|74359 | 2E-55 | nitrilase | Nicotiana tabacum | 3.5.5.1 | 43 | 48 | 312 | 348 |
| | 80\|pir\|\|T 03739 | | (EC 3.5.5.1) | Eukaryota | | | | | |
| | | | 4B - common tobacco. | | | | | | |
| 71, 72 | gi\|74359 | 2E-58 | nitrilase | Nicotiana | 3.5.5.1 | 39 | 52 | 321 | 348 |
| | 80\|pir\|\|T | | | tabacum | | | | | |
| | 03739 | | (EC 3.5.5.1) | Eukaryota | | | | | |
| | | | 4B - | | | | | | |
| | | | common | | | | | | |
| | | | tobacco. | | | | | | |
| 73, 74 | gi\|15229 | 2E-53 | nitrilase 1 | Arabidopsis | N/A | 34 | N/A | 344 | 346 |
| | 934\|ref\| | | [Arabidopsis | | | | | | |
| | NP_190 | | | thaliana | | | | | |
| | 017.1\| | | thaliana]. | Eukaryota | | | | | |
| 75, 76 | gi\|74359 | 4E-98 | nitrilase | Synechoc | 3.5.5.1 | 48 | 50 | 374 | 346 |
| | 78\|pir\|\|S | | | ystis sp. | | | | | |
| | 77025 | | (EC 3.5.5.1)- | Bacteria | | | | | |
| | | | Synechoc | | | | | | |
| | | | ystis sp. | | | | | | |
| | | | (strain PCC 6803). | | | | | | |
| 77, 78 | gi\|15242 | 8E-62 | Nitrilase 4 | Arabidop | N/A | 38 | N/A | 351 | 355 |
| | 205\|ref\| | | (sp | sis | | | | | |
| | NP_197 | | P46011) | thaliana | | | | | |
| | 622.1\| | | [Arabidopsisthaliana]. | Eukaryota | | | | | |
| 79, 80 | gi\|74359 | 5E-54 | nitrilase | Nicotiana | 3.5.5.1 | 38 | 46 | 329 | 348 |
| | 80\|pir\|\|T | | | tabacum | | | | | |
| | 03739 | | (EC 3.5.5.1) | Eukaryota | | | | | |
| | | | 4B - | | | | | | |
| | | | common | | | | | | |
| | | | tobacco. | | | | | | |
| 81, 82 | gi\|74359 | 3E-94 | nitrilase | Synechoc | 3.5.5.1 | 51 | 56 | 330 | 346 |
| | 78\|pir\|\|S | | | ystis sp. | | | | | |
| | 77025 | | (EC 3.5.5.1) - | Bacteria | | | | | |
| | | | Synechocystis sp. | | | | | | |
| | | | (strain PCC 6803). | | | | | | |
| 83, 84 | gi\|74359 | e-114 | nitrilase | Synechocystis sp. | 3.5.5.1 | 61 | 59 | 356 | 346 |
| | 78\|pir\|\|S | | (EC 3.5.5.1) - | | | | | | |
| | 77025 | | Synechocystis sp. | Bacteria | | | | | |
| | | | (strain PCC 6803). | | | | | | |
| 85, 86 | gi\|15143 | e-115 | unnamed | unidentified | N/A | 63 | 66 | 337 | 346 |
| | 035\|emb | | protein | | | | | | |
| | \|CAC50 | | product | unclassified. | | | | | |
| | 776.1\| | | [unidentified]. | | | | | | |
| 87, 88 | gi\|14699 12\|gb\|A | 3E-54 | nitrilase 2. | Arabidopsis | N/A | 36 | 46 | 353 | 339 |
| | AB0522 | | | thaliana | | | | | |
| | 0.1\| | | | Eukaryota | | | | | |
| 89,90 | gi\|15242 | 5E-62 | Nitrilase 4 | Arabidopsis | N/A | 41 | N/A | 305 | 355 |
| | 205\|ref\|NP_197 | | (sp P46011) | thaliana | | | | | |
| | 622.1\| | | [Arabidopsis thaliana]. | Eukaryota | | | | | |
| 91, 92 | gi\|14211 | 6E-58 | nitrilase- | Brassica | N/A | 35 | 50 | 312 | 350 |
| | 396\|gb\| | | like | napus | | | | | |
| | AAK57 | | protein | Eukaryota | | | | | |
| | 436.1\| | | [Brassicanapus]. | | | | | | |
| 93, 94 | gi\|74359 | e-112 | nitrilase | Synechocystis sp. | 3.5.5.1 | 60 | 61 | 325 | 346 |
| | 78\|pir\|\|S77025 | | (EC 3.5.5.1) - | Bacteria | | | | | |
| | | | Synechocystis sp. | | | | | | |
| | | | (strain | | | | | | |
| | | | PCC | | | | | | |
| | | | 6803). | | | | | | |
| 95, 96 | gi\|66248 | 1E-60 | nitrilase 2 | Arabidopsis | N/A | 42 | 53 | 321 | 339 |
| | 86\|emb\|CAA68934.3\| | | [Arabidopsis thaliana]. | thaliana Eukaryota | | | | | |
| 97, 98 | gi\|15143 | e-109 | unnamed | unidentified | N/A | 62 | 67 | 338 | 346 |
| | 035\|emb | | protein | | | | | | |
| | \|CAC50 | | product | unclassified. | | | | | |
| | 776.1\| | | [unidentified]. | | | | | | |
| 99, | gi\|74359 | e-123 | nitrilase | Synechocystis sp. | 3.5.5.1 | 62 | 61 | 337 | 346 |
| 100 | 78\|pir\|\|S77025 | | (EC 3.5.5.1) - | Bacteria | | | | | |
| | | | Synechocystis sp. | | | | | | |
| | | | (strain | | | | | | |
| | | | PCC | | | | | | |
| | | | 6803). | | | | | | |
| 101, | gi\|15143 | e-121 | unnamed | unidentified | N/A | 62 | 66 | 354 | 346 |
| 102 | 035\|emb | | protein | | | | | | |
| | \|CAC50 | | product | unclassified. | | | | | |
| | 776.1\| | | [unidentified]. | | | | | | |
| 103, | gi\|15143 | 2E-41 | unnamed | unidentified | N/A | 34 | 49 | 314 | 337 |
| 104 | 037\|emb | | protein | | | | | | |
| | \|CAC50 | | product | unclassified. | | | | | |
| | 777.1\| | | [unidentified]. | | | | | | |
| 105, | gi\|74359 | 1E-97 | nitrilase | Synechocystis sp. | 3.5.5.1 | 54 | 58 | 324 | 346 |
| 106 | 78\|pir\|\|S | | | | | | | | |
| | 77025 | | (EC 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 107, | gi\|74359 | e-113 | nitrilase | Synechoc | 3.5.5.1 | 61 | 61 | 326 | 346 |
| 108 | 78\|pir\|\|S | | (EC | ystis sp. | | | | | |
| | 77025 | | 3.5.5.1)- | Bacteria | | | | | |
| | | | Synechocystis sp. | | | | | | |
| | | | (strain | | | | | | |
| | | | PCC | | | | | | |
| | | | 6803). | | | | | | |
| 109, | gi\|74359 | e-113 | nitrilase | Synechoc | 3.5.5.1 | 55 | 57 | 363 | 346 |
| 110 | 78\|pir\|\|S | | (EC | ystis sp. | | | | | |
| | 77025 | | 3.5.5.1) - | Bacteria | | | | | |
| | | | Synechocystis sp. | | | | | | |
| | | | (strain | | | | | | |
| | | | PCC | | | | | | |
| | | | 6803). | | | | | | |
| 111, | gi\|74359 | e-121 | nitrilase | Synechocystis sp. | 3.5.5.1 | 64 | 61 | 329 | 346 |
| 112 | 78\|pir\|\|S | | (EC | | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 113, | gi\|15162 | 4E-09 | AGR_pA | Plasmid | N/A | 26 | 47 | 330 | 600 |
| 114 | 360\|gb\| | | T_799p | Agrobact | | | | | |
| | AAK90 | | [Agrobact | erium | | | | | |
| | 913.1\| | | erium | tumefaciens | | | | | |
| | | | tumefaciens]. | Bacteria | | | | | |
| 115, | gi\|74359 | e-115 | nitrilase | Synechoc | 3.5.5.1 | 59 | 61 | 330 | 346 |
| 116 | 78\|pir\|\|S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 117, | gi\|74359 | e-106 | nitrilase | Synechoc | 3.5.5.1 | 64 | 64 | 318 | 346 |
| 118 | 78\|pir\|\|S | | | ystis sp. | | | | | |
| | 77025 | | (EC 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 119, | gi\|15795 | 1E-40 | unnamed | Rhodococcus | N/A | 30 | 49 | 327 | 366 |
| 120 | 659\|emb | | protein | | | | | | |
| | \|CAC88 | | product | rhodochrous | | | | | |
| | 237.1\| | | [Rhodococcus | Bacteria | | | | | |
| | | | rhodochrous]. | | | | | | |
| 121, | gi\|15229 | 1E-45 | nitrilase 2 | Arabidopsis | N/A | 30 | N/A | 385 | 339 |
| 122 | 932\|ref\| | | [Arabidopsis | thaliana | | | | | |
| | NP_190 016.1\| | | thaliana]. | Eukaryota | | | | | |
| 123, | gi\|14211 | 9E-52 | nitrilase- | Brassica | N/A | 35 | 51 | 329 | 350 |
| 124 | 396\|gb\| | | like | napus | | | | | |
| | AAK57 | | protein | Eukaryota | | | | | |
| | 436.1\| | | [Brassicanapus]. | | | | | | |
| 125, | gi\|74359 | e-125 | nitrilase | Synechoc | 3.5.5.1 | 63 | 61 | 349 | 346 |
| 126 | 78\|pir∥S | | | ystis sp. | | | | | |
| | 77025 | | (EC 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 127, | gi\|74359 | e-117 | nitrilase | Synechocystis sp. | 3.5.5.1 | 61 | 60 | 334 | 346 |
| 128 | 78\|pir∥S | | | | | | | | |
| | 77025 | | (EC 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 129, | gi\|74359 | e-125 | nitrilase | Synechoc | 3.5.5.1 | 64 | 63 | 336 | 346 |
| 130 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 131, | gi\|74359 | e-125 | nitrilase | Synechoc | 3.5.5.1 | 63 | 63 | 336 | 346 |
| 132 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 133, | gi\|74359 | e-120 | nitrilase | Synechoc | 3.5.5.1 | 63 | 60 | 341 | 346 |
| 134 | 78\|pir∥S | | | ystis sp. | | | | | |
| | 77025 | | (EC 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 135, | gi\|74359 | e-124 | nitrilase | Synechoc | 3.5.5.1 | 64 | 63 | 336 | 346 |
| 136 | 78\|pir∥S | | | ystis sp. | | | | | |
| | 77025 | | (EC 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 137, | gi\|74359 | e-102 | nitrilase | Synechoc | 3.5.5.1 | 54 | 56 | 325 | 346 |
| 138 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 139, | gi\|15143 | e-123 | unnamed | unidentified | N/A | 65 | 63 | 332 | 337 |
| 140 | 037\|emb | | protein | | | | | | |
| | \|CAC50 | | product | unclassified. | | | | | |
| | 777.1\| | | [unidentified]. | | | | | | |
| 141, | gi\|15143 | 3E-49 | unnamed | unidentified | N/A | 32 | 47 | 341 | 337 |
| 142 | 037\|emb | | protein | | | | | | |
| | \|CAC50 | | product | unclassified. | | | | | |
| | 777.1\| | | [unidentified]. | | | | | | |
| 143, | gi\|15143 | 8E-55 | unnamed | unidentified | N/A | 30 | 44 | 373 | 337 |
| 144 | 037\|emb | | protein | | | | | | |
| | \|CAC50 | | product | unclassified. | | | | | |
| | 777.1\| | | [unidentified]. | | | | | | |
| 145, | gil\|15143 | e-110 | unnamed | unidentified | N/A | 58 | 63 | 337 | 346 |
| 146 | 035\|emb | | protein | | | | | | |
| | \|CAC50 | | product | unclassified. | | | | | |
| | 776.1\| | | [unidentified]. | | | | | | |
| 147, | gi\|15143 | 2E-52 | unnamed | unidentified | N/A | 33 | 42 | 365 | 337 |
| 148 | 037\|emb | | protein | | | | | | |
| | \|CAC50 | | product | unclassified. | | | | | |
| | 777.1\| | | [unidentified]. | | | | | | |
| 149, | gi\|15229 | 5E-63 | nitrilase 2 | Arabidopsis | N/A | 43 | N/A | 313 | 339 |
| 150 | 932\|ref\| | | [Arabidopsis | | | | | | |
| | NP_190 | | | thaliana | | | | | |
| | 016.1\| | | thaliana]. | Eukaryota | | | | | |
| 151, | gi\|74359 | 6E-96 | nitrilase | Synechoc | 3.5.5.1 | 51 | 58 | 330 | 346 |
| 152 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 153, | gi\|74359 | 7E-46 | nitrilase | Synechoc | 3.5.5.1 | 31 | 44 | 357 | 346 |
| 154 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 155, | gi\|15143 | e-101 | unnamed | unidentified | N/A | 60 | 66 | 346 | 346 |
| 156 | 035\|emb | | protein | | | | | | |
| | \|CAC50 | | product | unclassified. | | | | | |
| | 776.1\| | | [unidentified]. | | | | | | |
| 157, | gi\|74359 | 3E-99 | nitrilase | Synechoc | 3.5.5.1 | 52 | 55 | 336 | 346 |
| 158 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 159, | gi\|74359 | 7E-52 | nitrilase | Nicotiana | 3.5.5.1 | 41 | 52 | 309 | 348 |
| 160 | 80\|pir∥T | | (EC | tabacum | | | | | |
| | 03739 | | 3.5.5.1) | Eukaryota | | | | | |
| | | | 4B - common | | | | | | |
| | | | tobacco. | | | | | | |
| 161, | gi\|74359 | e-111 | nitrilase | Synechoc | 3.5.5.1 | 58 | 60 | 335 | 346 |
| 162. | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 163, | gi\|74359 | e-101 | nitrilase | Synechoc | 3.5.5.1 | 57 | 58 | 325 | 346 |
| 164 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 165, | gi\|15143 | e-108 | unnamed | unidentified | N/A | 63 | 65 | 335 | 346 |
| 166 | 035\|emb | | protein | | | | | | |
| | \|CAC50 | | product | unclassified. | | | | | |
| | 776.1\| | | [unidentified]. | | | | | | |
| 167, | gi\|15143 | e-111 | unnamed | unidentified | N/A | 61 | 66 | 338 | 346 |
| 168 | 035\|emb | | protein | | | | | | |
| | \|CAC50 | | product | unclassified. | | | | | |
| | 776.1\| | | [unidentified]. | | | | | | |
| 169, | gi\|15143 | e-122 | unnamed | unidentified | N/A | 60 | 65 | 358 | 346 |
| 170 | 035\|emb | | protein | | | | | | |
| | \|CAC50 | | product | unclassified. | | | | | |
| | 776.1\| | | [unidentified]. | | | | | | |
| 171, | gi\|66248 | 9E-61 | nitrilase 2 | Arabidopsis | N/A | 38 | 48 | 336 | 339 |
| 172 | 86\|emb\| | | [Arabidopsis | | | | | | |
| | CAA68 | | | thaliana | | | | | |
| | 934.31 | | thaliana]. | Eukaryota | | | | | |
| 173, | gi\|74359 | e-101 | nitrilase | Synechoc | 3.5.5.1 | 54 | 56 | 330 | 346 |
| 174 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 175, | gi\|15143 | 2E-52 | unnamed | unidentified | N/A | 39 | 49 | 314 | 337 |
| 176 | 037\|emb | | protein | | | | | | |
| | \|CAC50 | | product | unclassified. | | | | | |
| | 777.1\| | | [unidentified]. | | | | | | |
| 177, | gi\|15795 | 9E-41 | unnamed | Rhodococcus | N/A | 32 | 50 | 315 | 366 |
| 178 | 659\|emb | | protein | | | | | | |
| | \|CAC88 | | product | rhodochrous | | | | | |
| | 237.1\| | | [Rhodococcus | Bacteria | | | | | |
| | | | rhodochrous]. | | | | | | |
| 179, | gi\|74359 | 3E-53 | nitrilase | Nicotiana | 3.5.5.1 | 38 | 46 | 304 | 348 |
| 180 | 80\|pir∥T | | (EC | tabacum | | | | | |
| | 03739 | | 3.5.5.1) | Eukaryota | | | | | |
| | | | 4B - common tobacco. | | | | | | |
| 181, | gi\|74359 | e-121 | nitrilase | Synechoc | 3.5.5.1 | 64 | 61 | 329 | 346 |
| 182 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 183, | gi\|74359 | e-118 | nitrilase | Synechoc | 3.5.5.1 | 63 | 63 | 333 | 346 |
| 184 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 185, | gi\|15143 | e-110 | unnamed | unidentified | N/A | 60 | 67 | 338 | 346 |
| 186 | 035\|emb | | protein | | | | | | |
| | \|CAC50 | | product | unclassified. | | | | | |
| | 7761.\| | | [unidentified]. | | | | | | |
| 187, | gi\|15143 | e-113 | unnamed | unidentified | N/A | 59 | 62 | 352 | 346 |
| 188 | 035\|emb | | protein | | | | | | |
| | \|CAC50 | | product | unclassified. | | | | | |
| | 776.1\| | | [unidentified]. | | | | | | |
| 189, | gi\|15143 | e-101 | unnamed | unidentified | N/A | 56 | 61 | 334 | 346 |
| 190 | 035\|emb | | protein | | | | | | |
| | \|CAC50 | | product | unclassified. | | | | | |
| | 776.1\| | | [unidentified]. | | | | | | |
| 191, | gi\|15242 | 6E-56 | Nitrilase 4 | Arabidopsis | N/A | 37 | N/A | 314 | 355 |
| 192 | 205\|ref\| | | | | | | | | |
| | NP_197 | | (sp P46011) | thaliana | | | | | |
| | 622.1\| | | [Arabidopsis | Eukaryota | | | | | |
| | | | thaliana]. | | | | | | |
| 193, | gi\|74359 | 3E-58 | nitrilase | Nicotiana | 3.5.5.1 | 39 | 51 | 321 | 348 |
| 194 | 80\|pir∥T | | (EC | tabacum | | | | | |
| | 03739 | | 3.5.5.1) | Eukaryota | | | | | |
| | | | 4B - common | | | | | | |
| | | | tobacco. | | | | | | |
| 195, | gi\|74359 | 5E-89 | nitrilase | Synechoc | 3.5.5.1 | 50 | 57 | 330 | 346 |
| 196 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 197, | gi\|15143 | e-110 | unnamed | unidentified | N/A | 65 | 68 | 338 | 346 |
| 198 | 035\|emb | | protein | | | | | | |
| | \|CAC50 | | product | unclassified. | | | | | |
| | 776.1\| | | [unidentified]. | | | | | | |
| 199, | gi\|11266 | 1E-58 | nitrilase 1 | Arabidopsis | N/A | 38 | N/A | 330 | 346 |
| 200 | 289\|pir∥ | | - | | | | | | |
| | T49147 | | Arabidops | thaliana | | | | | |
| | | | is thaliana. | Eukaryota | | | | | |
| 201, | gi\|48355 | 5E-50 | nitrilase- | Oryzasativa | N/A | 39 | 49 | 309 | 362 |
| 202 | 88\|dbj\|B | | like | | | | | | |
| | AA7767 | | protein | Eukaryota | | | | | |
| | 9.1\| | | [Oryzasativa]. | | | | | | |
| 203, | gi\|74359 | 4E-57 | nitrilase | Nicotiana | 3.5.5.1 | 39 | 50 | 321 | 348 |
| 204 | 80\|pir∥T | | (EC | tabacum | | | | | |
| | 03739 | | 3.5.5.1) | Eukaryota | | | | | |
| | | | 4B - common | | | | | | |
| | | | tobacco. | | | | | | |
| 205, | gi\|74359 | 1E-99 | nitrilase | Synechoc | 3.5.5.1 | 55 | 56 | 322 | 346 |
| 206 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 207, | gi\|74359 | 5E-59 | nitrilase | Nicotiana | 3.5.5.1 | 40 | 51 | 321 | 348 |
| 208 | 80\|pir∥T | | (EC | tabacum | | | | | |
| | 03739 | | 3.5.5.1) | Eukaryota | | | | | |
| | | | 4B - common | | | | | | |
| | | | tobacco. | | | | | | |
| 209, | gi\|74359 | 2E-95 | nitrilase | Synechoc | 3.5.5.1 | 53 | 55 | 330 | 346 |
| 210 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 211, | gi\|15143 | e-117 | unnamed | unidentified | N/A | 64 | 67 | 353 | 346 |
| | 035\|emb | | protein | | | | | | |
| 212 | \|CAC50 | | product | unclassified. | | | | | |
| | 776.1\| | | [unidentified]. | | | | | | |
| 213, | gi\|74359 | e-101 | nitrilase | Synechoc | 3.5.5.1 | 53 | 57 | 330 | 346 |
| 214 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 215, | gi\|15143 | e-114 | unnamed | unidentified | N/A | 62 | 61 | 335 | 346 |
| 216 | 035\|emb | | protein | | | | | | |
| | \|CAC50 | | product | unclassified. | | | | | |
| | 776.1\| | | [unidentified]. | | | | | | |
| 217, | gi\|15143 | e-115 | unnamed | unidentified | N/A | 58 | 62 | 336 | 346 |
| 218 | 035\|emb | | protein | | | | | | |
| | \|CAC50 | | product | unclassified. | | | | | |
| | 776.1\| | | [unidentified]. | | | | | | |
| 219, | gi\|74359 | c-101 | nitrilase | Synechoc | 3.5.5.1 | 53 | 59 | 331 | 346 |
| 220 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 221, | gi\|41738 | e-122 | ALIPHAT | Rhodococcus | N/A | 58 | 62 | 381 | 383 |
| 222 | 2\|sp\|Q02 | | IC | | | | | | |
| | 068\|NR | | N1TRILASE. | rhodochrous | | | | | |
| | L1_RH ORH | | | Bacteria | | | | | |
| 223, | gi\|74359 | 3E-94 | nitrilase | Synechoc | 3.5.5.1 | 51 | 56 | 331 | 346 |
| 224 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC 6803). | | | | | | |
| 225, | gi\|74359 | 6E-99 | nitrilase | Synechoc | 3.5.5.1 | 53 | 57 | 316 | 346 |
| 226 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 227, | gi\|74359 | e-121 | nitrilase | Synechoc | 3.5.5.1 | 62 | 60 | 344 | 346 |
| 228 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 229, | gi\|75109 | 3E-48 | probable | Caenorha | 3.5.5.1 | 36 | 48 | 324 | 305 |
| 230 | 01\|pir∥T | | nitrilase | bditis | | | | | |
| | 27679 | | (EC | elegans | | | | | |
| | | | 3.5.5.1) | Eukaryota | | | | | |
| | | | ZK1058.6 | | | | | | |
| | | | Caenorhabditis | | | | | | |
| | | | elegans. | | | | | | |
| 231, | gi\|15143 | e-125 | unnamed | unidentified | N/A | 63 | 65 | 353 | 346 |
| 232 | 035\|emb | | protein | | | | | | |
| | \|CAC50 | | product | unclassified. | | | | | |
| | 776.1\| | | [unidentified]. | | | | | | |
| 233, | gi\|74359 | e-101 | nitrilase | Synechoc | 3.5.5.1 | 54 | 55 | 333 | 346 |
| 234 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 235, | gi\|74359 | 1E-90 | nitrilase | Synechoc | 3.5.5.1 | 51 | 57 | 330 | 346 |
| 236 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 237, | gi\|74359 | 7E-97 | nitrilase | Synechoc | 3.5.5.1 | 53 | 56 | 330 | 346 |
| 238 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 239, | gi\|74359 | 8E-59 | nitrilase | Nicotiana | 3.5.5.1 | 40 | 50 | 322 | 348 |
| 240 | 80\|pir∥T | | (EC | tabacum | | | | | |
| | 03739 | | 3.5.5.1) | Eukaryota | | | | | |
| | | | 4B - common | | | | | | |
| | | | tobacco. | | | | | | |
| 241, | gi\|66248 | 8E-59 | nitrilase 2 | Arabidopsis | N/A | 40 | 50 | 323 | 339 |
| 242 | 86\|emb\| | | [Arabidopsis | | | | | | |
| | CAA68 | | | thaliana | | | | | |
| | 934.3\| | | thaliana]. | Eukaryota | | | | | |
| 243, | gi\|15143 | 2E-51 | unnamed | unidentified | N/A | 38 | 47 | 332 | 337 |
| 244 | 037\|emb | | protein | | | | | | |
| | \|CAC50 | | product | unclassified. | | | | | |
| | 777.1\| | | [unidentified]. | | | | | | |
| 245, | gi\|66248 | 5E-56 | nitrilase 2 | Arabidopsis | N/A | 38 | 50 | 332 | 339 |
| 246 | 86\|emb\| | | [Arabidopsis | | | | | | |
| | CAA68 | | | thaliana | | | | | |
| | 934.3\| | | thaliana]. | Eukaryota | | | | | |
| 247, | gi\|74359 | 9E-61 | nitrilase | Nicotiana | 3.5.5.1 | 42 | 49 | 329 | 348 |
| 248 | 80\|pir∥T | | (EC | tabacum | | | | | |
| | 03739 | | 3.5.5.1) | Eukaryota | | | | | |
| | | | 4B - common tobacco. | | | | | | |
| 249, | gi\|15143 | e-113 | unnamed | unidentified | N/A | 62 | 67 | 338 | 346 |
| 250 | 035\|emb | | protein | | | | | | |
| | \|CAC50 | | product | unclassified. | | | | | |
| | 776.1\| | | [unidentified]. | | | | | | |
| 251, | gi\|74359 | e-100 | nitrilase | Synechoc | 3.5.5.1 | 55 | 57 | 325 | 346 |
| 252 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 253, | gi\|74359 | 1E-56 | nitrilase | Nicotiana | 3.5.5.1 | 40 | 51 | 307 | 348 |
| 254 | 80\|pir∥T | | (EC | tabacum | | | | | |
| | 03739 | | 3.5.5.1) | Eukaryota | | | | | |
| | | | 4B - common tobacco. | | | | | | |
| 255, | gi\|15229 | 4E-63 | nitrilase 3 | Arabidopsis | N/A | 40 | N/A | 334 | 346 |
| 256 | 936\|ref\| | | [Arabidopsis | | | | | | |
| | NP_190 | | | thaliana | | | | | |
| | 018.1\| | | thaliana]. | Eukaryota | | | | | |
| 257, | gi\|48355 | 6E-51 | nitrilase- | Oryza | N/A | 38 | 48 | 313 | 362 |
| 258 | 88\|dbj\|B | | like | sativa | | | | | |
| | AA7767 | | protein | Eukaryota | | | | | |
| | 9.1\| | | [Oryzasativa]. | | | | | | |
| 259, | gi\|74359 | e-113 | nitrilase | Synechoc | 3.5.5.1 | 60 | 61 | 326 | 346 |
| 260 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 261, | gi\|15143 | e-114 | unnamed | unidentified | N/A | 62 | 67 | 337 | 346 |
| | 035\|emb | | protein | | | | | | |
| 262 | \|CAC50 | | product | unclassified. | | | | | |
| | 776.1\| | | [unidentified]. | | | | | | |
| 263, | gi\|74359 | 9E-98 | nitrilase | Synechoc | 3.5.5.1 | 53 | 54 | 337 | 346 |
| 264 | 78\|pir∥S | | (EC | ystis sp. | | | | | |
| | 77025 | | 3.5.5.1) - | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 265, | gi\|14590 | 8E-17 | hypothetic | Pyrococcus | N/A | 21 | 34 | 332 | 262 |
| 266 | 532\|ref\| | | al protein | | | | | | |
| | NP_142 | | [Pyrococcus | horikoshii | | | | | |
| | 600.1\| | | | Archaea | | | | | |
| | | | horikoshii]. | | | | | | |
| 267, | gi\|15143 | e-102 | unnamed | unidentified | N/A | 53 | 64 | 345 | 346 |
| 268 | 035\|emb | | protein | | | | | | |
| | \|CAC50 | | product | unclassified. | | | | | |
| | 776.1\| | | [unidentified]. | | | | | | |
| 269, | gi\|15143 | 8E-97 | unnamed | unidentified | N/A | 57 | 61 | 337 | 346 |
| 270 | 035\|emb | | protein | | | | | | |
| | \|CAC50 | | product | unclassified. | | | | | |
| | 776.1\| | | [unidentified]. | | | | | | |
| 271, | gi\|15229 | 3E-59 | nitrilase 1 | Arabidopsis | N/A | 41 | N/A | 321 | 346 |
| 272 | 934\|ref\| | | [Arabidop | | | | | | |
| | NP_190 | | sis | thaliana | | | | | |
| | 017.1\| | | thaliana]. | Eukaryota | | | | | |
| 273, | gi\|74359 | e-141 | nitrilase | Synechoc | 3.5.5.1 | 69 | 67 | 340 | 346 |
| 274 | 78\|pir∥S | | (EC | ystis sp. | | | | | |
| | 77025 | | 3.5.5.1)- | Bacteria | | | | | |
| | | | Synechoc | | | | | | |
| | | | ystis sp. | | | | | | |
| | | | (strain | | | | | | |
| | | | PCC | | | | | | |
| | | | 6803). | | | | | | |
| 275, | gi\|74359 | 8E-42 | nitrilase | Nicotiana | 3.5.5.1 | 35 | 48 | 282 | 348 |
| 276 | 80\|pir∥T | | (EC | tabacum | | | | | |
| | 03739 | | 3.5.5.1) | Eukaryota | | | | | |
| | | | 4B - common tobacco. | | | | | | |
| 277, | gi\|15242 | 4E-62 | Nitrilase 4 | Arabidopsis | N/A | 38 | N/A | 351 | 355 |
| 278 | 205\|ref\| | | (sp | | | | | | |
| | NP_197 | | P46011) | thaliana | | | | | |
| | 622.1\| | | [Arabidopsis | Eukaryota | | | | | |
| | | | thaliana]. | | | | | | |
| 279, | gi\|15143 | e-102 | unnamed | unidentified | N/A | 57 | 63 | 337 | 346 |
| 280 | 035\|emb | | protein | | | | | | |
| | \|CAC50 | | product | unclassified. | | | | | |
| | 776.1\| | | [unidentified]. | | | | | | |
| 281, | gi\|75109 | 5E-55 | probable | Caenorha | 3.5.5.1 | 39 | 46 | 311 | 305 |
| 282 | 01\|pir∥T | | nitrilase | bditis | | | | | |
| | 27679 | | (EC | elegans | | | | | |
| | | | 3.5.5.1) | Eukaryota | | | | | |
| | | | ZK1058.6 | | | | | | |
| | | | Caenorhabditis | | | | | | |
| | | | elegans. | | | | | | |
| 283, | gi\|15143 | e-111 | unnamed | unidentified | N/A | 61 | 66 | 338 | 346 |
| 284 | 035\|emb | | protein | | | | | | |
| | \|CAC50 | | product | unclassified. | | | | | |
| | 776.1\| | | [unidentified]. | | | | | | |
| 285, | gi\|15242 | 1E-64 | Nitrilase 4 | Arabidop | N/A | 44 | N/A | 305 | 355 |
| 286 | 205\|ref\| | | (sp | sis | | | | | |
| | NP_197 | | P46011) | thaliana | | | | | |
| | 622.1\| | | [Arabidopsis | Eukaryota | | | | | |
| | | | thaliana]. | | | | | | |
| 287, | gi\|66248 | 2E-55 | nitrilase 2 | Arabidop | N/A | 39 | 51 | 311 | 339 |
| 288 | 86\|emb\| | | [Arabidop | sis | | | | | |
| | CAA68 | | sis | thaliana | | | | | |
| | 934.3\| | | thaliana]. | Eukaryota | | | | | |
| 289, | gi\|48355 | 3E-58 | nitrilase- | Oryza | N/A | 43 | 51 | 306 | 362 |
| 290 | 88\|dbj\|B | | like | sativa | | | | | |
| | AA77679.1\| | | protein | Eukaryota | | | | | |
| | | | [Oryzasativa]. | | | | | | |
| 291, | gi\|15143 | e-123 | unnamed | unidentified | N/A | 64 | 63 | 333 | 337 |
| 292 | 037\|emb | | protein | | | | | | |
| | \|CAC50 | | product | unclassified. | | | | | |
| | 777.1\| | | [unidentified]. | | | | | | |
| 293, | gi\|16331 | 1E-97 | nitrilase | Synechoc | N/A | 50 | N/A | 335 | 346 |
| 294 | 918\|ref\| | | [Synechoc | ystis sp. | | | | | |
| | NP_442 | | ystis sp. | PCC | | | | | |
| | 646.1\| | | PCC | 6803 | | | | | |
| | | | 6803]. | Bacteria | | | | | |
| 295, | gi\|15229 | 6E-35 | nitrilase 2 | Arabidop | N/A | 27 | N/A | 377 | 339 |
| 296 | 932\|ref\| | | [Arabidop | sis | | | | | |
| | NP_190 | | sis | thaliana | | | | | |
| | 016.1\| | | thaliana]. | Eukaryota | | | | | |
| 297, | gi\|15143 | 3E-44 | unnamed | unidentified | N/A | 34 | 47 | 352 | 337 |
| 298 | 037\|emb | | protein | | | | | | |
| | \|CAC50 | | product | unclassified. | | | | | |
| | 777.1\| | | [unidentified]. | | | | | | |
| 299, | gi\|16331 | e-103 | nitrilase | Synechoc | N/A | 56 | N/A | 328 | 346 |
| 300 | 918\|ref\| | | [Synechoc | ystis sp. | | | | | |
| | NP_442 | | ystis sp. | PCC | | | | | |
| | 646.1\| | | PCC | 6803 | | | | | |
| | | | 6803]. | Bacteria | | | | | |
| 301, | gi\|74359 | 6E-57 | nitrilase | Nicotiana | 3.5.5.1 | 37 | 46 | 335 | 348 |
| 302 | 80\|pir∥T | | (EC | tabacum | | | | | |
| | 03739 | | 3.5.5.1) | Eukaryota | | | | | |
| | | | 4B - common | | | | | | |
| | | | tobacco. | | | | | | |
| 303, | gi\|15143 | e-112 | unnamed | unidentified | N/A | 60 | 59 | 336 | 346 |
| | 035\|emb | | protein | | | | | | |
| 304 | \|CAC50 | | product | unclassified. | | | | | |
| | 776.1\| | | [unidentified]. | | | | | | |
| 305, | gi\|15143 | e-121 | unnamed | unidentified | N/A | 59 | N/A | 355 | 346 |
| 306 | 035\|emb | | protein | | | | | | |
| | \|CAC50 | | product | unclassified. | | | | | |
| | 776.1\| | | [unidentified]. | | | | | | |
| 307, | gi\|74359 | 5E-61 | nitrilase | Nicotiana | 3.5.5.1 | 43 | 50 | 313 | 348 |
| 308 | 80\|pir∥T | | (EC | tabacum | | | | | |
| | 03739 | | 3.5.5.1) | Eukaryota | | | | | |
| | | | 4B - common | | | | | | |
| | | | tobacco. | | | | | | |
| 309, | gi\|41738 | 1E-43 | ALIPHAT | Rhodoco | N/A | 34 | 53 | 316 | 383 |
| 310 | 2\|sp\|Q02 | | IC | ccus | | | | | |
| | 068\|NR | | NITRILA | rhodochr | | | | | |
| | L1_RH | | SE. | ous | | | | | |
| | ORH | | | Bacteria | | | | | |
| 311, | gi\|16331 | e-126 | nitrilase | Synechoc | N/A | 64 | N/A | 336 | 346 |
| 312 | 918\|ref\| | | [Synechoc | ystis sp. | | | | | |
| | NP_442 | | ystis sp. | PCC | | | | | |
| | 646.1\| | | PCC | 6803 | | | | | |
| | | | 6803]. | Bacteria | | | | | |
| 313, | gi\|15143 | 1E-49 | unnamed | unidentified | N/A | 36 | 50 | 328 | 346 |
| 314 | 035\|emb | | protein | | | | | | |
| | \|CAC50 | | product | unclassified. | | | | | |
| | 776.1\| | | [unidentified]. | | | | | | |
| 315, | gi\|15229 | 4E-64 | nitrilase 3 | Arabidopsis | N/A | 42 | N/A | 319 | 346 |
| 316 | 936\|ref\| | | [Arabidopsis | | | | | | |
| | NP_190 | | | thaliana | | | | | |
| | 018.1\| | | thaliana]. | Eukaryota | | | | | |
| 317, | gi\|16331 | e-102 | nitrilase | Synechoc | N/A | 54 | N/A | 330 | 346 |
| 318 | 918\|ref\| | | [Synechoc | ystis sp. | | | | | |
| | NP_442 | | ystis sp. | PCC | | | | | |
| | 646.1\| | | PCC | 6803 | | | | | |
| | | | 6803]. | Bacteria | | | | | |
| 319, | gi\|16331 | e-118 | nitrilase | Synechoc | N/A | 62 | N/A | 338 | 346 |
| 320 | 918\|ref\| | | [Synechoc | ystis sp. | | | | | |
| | NP_442 | | ystis sp. | PCC | | | | | |
| | 646.1\| | | PCC | 6803 | | | | | |
| | | | 6803]. | Bacteria | | | | | |
| 321, | gi\|16331 | e-100 | nitrilase | Synechoc | N/A | 53 | N/A | 330 | 346 |
| 322 | 918\|ref\| | | [Synechoc | ystis sp. | | | | | |
| | NP_442 | | ystis sp. | PCC | | | | | |
| | 646.1\| | | PCC | 6803 | | | | | |
| | | | 6803]. | Bacteria | | | | | |
| 323, | gi\|15229 | 3E-52 | nitrilase 3 | Arabidop | N/A | 42 | N/A | 316 | 346 |
| 324 | 936\|ref\| | | [Arabidop | sis | | | | | |
| | NP_190 | | sis | thaliana | | | | | |
| | 018.1\| | | thaliana]. | Eukaryota | | | | | |
| 325, | gi\|16331 | e-121 | nitrilase | Synechoc | N/A | 64 | N/A | 358 | 346 |
| 326 | 918\|ref\| | | [Synechoc | ystis sp. | | | | | |
| | NP_442 | | ystis sp. | PCC | | | | | |
| | 646.1\| | | PCC | 6803 | | | | | |
| | | | 6803]. | Bacteria | | | | | |
| 327, | gi\|75109 | 5E-40 | probable | Caenorha | 3.5.5.1 | 30 | 46 | 324 | 305 |
| 328 | 01\|pir∥T | | nitrilase | bditis | | | | | |
| | 27679 | | (EC | elegans | | | | | |
| | | | 3.5.5.1) ZK1058.6 | Eukaryota | | | | | |
| | | | Caenorhabditis | | | | | | |
| | | | elegans. | | | | | | |
| 329, | gi\|16331 | e-113 | nitrilase | Synechoc | N/A | 60 | N/A | 340 | 346 |
| 330 | 918\|ref\| | | [Synechoc | ystis sp. | | | | | |
| | NP_442 | | ystis sp. | PCC | | | | | |
| | 646.1\| | | PCC | 6803 | | | | | |
| | | | 6803]. | Bacteria | | | | | |
| 331, | gi\|15143 | 2E-98 | unnamed | unidentified | N/A | 53 | 60 | 346 | 346 |
| 332 | 035\|emb | | protein | | | | | | |
| | \|CAC50 | | product | unclassified. | | | | | |
| | 776.1\| | | [unidentified]. | | | | | | |
| 333, | gi\|15143 | e-111 | unnamed | unidentified | N/A | 61 | 63 | 345 | 346 |
| | 035\|emb | | protein | | | | | | |
| 334 | \|CAC50 | | product | unclassified. | | | | | |
| | 776.1\| | | [unidentified]. | | | | | | |
| 335, | gi\|16331 | e-120 | nitrilase | Synechoc | N/A | 62 | N/A | 350 | 346 |
| 336 | 918\|ref\| | | [Synechoc | ystis sp. | | | | | |
| | NP_442 | | ystis sp. | PCC | | | | | |
| | 646.1\| | | PCC | 6803 | | | | | |
| | | | 6803]. | Bacteria | | | | | |
| 337, | gi\|17557 | 3E-22 | Nitrilase | Caenorha | N/A | 28 | N/A | 313 | 305 |
| 338 | 111\|ref\| | | [Caenorha | bditis | | | | | |
| | NP_497 | | bditis | elegans | | | | | |
| | 791.1\| | | elegans]. | Eukaryota | | | | | |
| 339, | gi\|15143 | 5E-25 | unnamed | unidentified | N/A | 27 | N/A | 329 | 346 |
| 340 | 035\|emb | | protein | | | | | | |
| | \|CAC50 | | product | unclassified. | | | | | |
| | 776.1\| | | [unidentified]. | | | | | | |
| 341, | gi\|15143 | 2E-11 | unnamed | unidentified | N/A | 26 | N/A | 342 | 346 |
| 342 | 035\|emb | | protein | | | | | | |
| | \|CAC50 | | product | unclassified. | | | | | |
| | 776.1\| | | [unidentified]. | | | | | | |
| 343, | gi\|15229 | 8E-18 | nitrilase 2 | Arabidopsis | N/A | 28 | N/A | 303 | 339 |
| 344 | 932\|ref\| | | [Arabidopsis | | | | | | |
| | NP_190 | | | thaliana | | | | | |
| | 016.1\| | | thaliana]. | Eukaryota | | | | | |
| 345, | gi\|15143 | 3E-50 | unnamed | unidentified | N/A | 40 | N/A | 329 | 346 |
| 346 | 035\|emb | | protein | | | | | | |
| | \|CAC50 | | product | unclassified. | | | | | |
| | 776.1\| | | [unidentified]. | | | | | | |
| 347, | gi\|15143 | 2E-11 | unnamed | unidentified | N/A | 22 | N/A | 297 | 337 |
| 348 | 037\|emb | | protein | | | | | | |
| | \|CAC50 | | product | unclassified. | | | | | |
| | 777.1\| | | [unidentified]. | | | | | | |
| 349, | gi\|41738 | 5.00E- | Aliphatic | Rhodococcus | N/A | 43.3 | 53.7 | 333 | 366 |
| | 41\|sp\|Q03 | | | | | | | | |
| 350 | 217\| | 86 | Nitrilase | rhodochrous | | | | | |
| 351, | gi\|15229 | 1E-11 | nitrilase 3 | Arabidopsis | N/A | 22 | N/A | 301 | 346 |
| 352 | 936\|ref\| | | [Arabidopsis | | | | | | |
| | NP_190 | | | thaliana | | | | | |
| | 018.1\| | | thaliana]. | Eukaryota | | | | | |
| 353, | gi\|15143 | 2E-10 | unnamed | unidentified | N/A | 29 | N/A | 312 | 346 |
| 354 | 035\|emb | | protein | | | | | | |
| | \|CAC50 | | product | unclassified. | | | | | |
| | 776.1\| | | [unidentified]. | | | | | | |
| 355, | gi\|17231 | 0.2 | heterocyst | Nostoc | N/A | 25 | N/A | 325 | 779 |
| 356 | 038\|ref\| | | differentia | sp. PCC | | | | | |
| | NP_487 | | tion | 7120 | | | | | |
| | 586.1\| | | protein | Bacteria | | | | | |
| | | | [Nostoc | | | | | | |
| | | | sp. PCC | | | | | | |
| | | | 7120]. | | | | | | |
| 357, | gi\|15242 | 1E-15 | Nitrilase 4 | Arabidopsis | N/A | 23 | N/A | 300 | 355 |
| 358 | 205\|ref\| | | (sp | | | | | | |
| | NP_197 | | P46011) | thaliana | | | | | |
| | 622.1\| | | [Arabidopsisthaliana]. | Eukaryota | | | | | |
| 359, | gi\|16331 | 1E-58 | nitrilase | Synechoc | N/A | 40 | N/A | 335 | 346 |
| 360 | 918\|ref\| | | [Synechoc | ystis sp. | | | | | |
| | NP_442 | | ystis sp. | PCC | | | | | |
| | 646.1\| | | PCC | 6803 | | | | | |
| | | | 6803]. | Bacteria | | | | | |
| 361, | gi\|15795 | 0.007 | unnamed | Rhodoco | N/A | 24 | N/A | 302 | 366 |
| 362 | 659\|emb | | protein | ccus | | | | | |
| | \|CAC88 | | product | rhodochrous | | | | | |
| | 237.1\| | | [Rhodococcus | | | | | | |
| | | | rhodochrous]. | Bacteria | | | | | |
| 363, | gi\|16331 | 1E-19 | nitrilase | Synechoc | N/A | 22 | N/A | 335 | 346 |
| 364 | 918\|ref\| | | [Synechoc | ystis sp. | | | | | |
| | NP_442 | | ystis sp. | PCC | | | | | |
| | | | PCC | 6803 | | | | | |
| | 646.1\| | | 6803]. | Bacteria | | | | | |
| 365, | gi\|48355 | 0.004 | nitrilase- | Oryza | N/A | 17 | N/A | 315 | 362 |
| 366 | 88\|dbj\|B | | like | sativa | | | | | |
| | AA7767 | | protein | Eukaryota | | | | | |
| | 9.1\| | | [Oryzasativa]. | | | | | | |
| 367, | gi\|41738 | 0.023 | ALIPHAT | Rhodococcus | N/A | 18 | N/A | 317 | 383 |
| 368 | 2\|sp\|Q02 | | IC | | | | | | |
| | 068\|NR | | NITRILA | rhodochrous | | | | | |
| | L1_RH | | SE. | | | | | | |
| | ORH | | | Bacteria | | | | | |
| 369, | gi\|17546 | 0.00000 | PROBAB | Ralstonia | N/A | 19 | N/A | 346 | 343 |
| 370 | 542\|ref\| | 001 | LE | solanacea | | | | | |
| | NP_519 | | NITRILA | rum | | | | | |
| | 944.1\| | | SE | Bacteria | | | | | |
| | | | PROTEIN | | | | | | |
| | | | [Ralstonia | | | | | | |
| | | | solanacearum]. | | | | | | |
| 371, | gi\|5143 | 9E-23 | unnamed | unidentified | N/A | 24 | N/A | 327 | 337 |
| 372 | 037\|emb | | protein | | | | | | |
| | \|CAC50 | | product | unclassified. | | | | | |
| | 777.1\| | | [unidentified]. | | | | | | |
| 373, | gi\|21206 | 7E-47 | nitrilase | Comamonas | 3.5.5.1 | 32 | N/A | 351 | 354 |
| 374 | 06\|pir∥J | | (EC | | | | | | |
| | C4212 | | 3.5.5.1) - | testosteroni | | | | | |
| | | | Comamonas | Bacteria | | | | | |
| | | | testosteroni. | | | | | | |
| 375, | gi\|14211 | 5E-59 | nitrilase- | Brassica | N/A | 36 | N/A | 312 | 350 |
| 376 | 396\|gb\| | | like | napus | | | | | |
| | AAK57 | | protein | Eukaryota | | | | | |
| | 436.1\| | | [Brassicanapus]. | | | | | | |
| 377, | gi\|59539 | 1.00E- | Sequence | N/A | N/A | N/A | N/A | 349 | N/A |
| 378 | 61\| | 102 | 1 from | | | | | | |
| | | | patent US 5872000. | | | | | | |
| 379, | gi\|14211 | 6E-58 | nitrilase- | Brassica | N/A | 38 | N/A | 311 | 350 |
| 380 | 396\|gb\| | | like | napus | | | | | |
| | AAK57 | | protein | Eukaryota | | | | | |
| | 436.1\| | | [Brassicanapus]. | | | | | | |
| 381, | gi\|17546 | 2E-40 | PROBAB | Ralstonia | N/A | 33 | N/A | 314 | 343 |
| 382 | 542\|ref\| | | LE | solanacea | | | | | |
| | NP_519 | | NITRILA | rum | | | | | |
| | 944.1\| | | SE | Bacteria | | | | | |
| | | | PROTEIN | | | | | | |
| | | | [Ralstonia | | | | | | |
| | | | solanacearum]. | | | | | | |
| 383, | gi\|15143 | e-108 | unnamed | unidentified | N/A | 55 | 55 | 345 | 337 |
| 384 | 037\|emb | | protein | | | | | | |
| | \|CAC50 | | product | unclassified. | | | | | |
| | 777.1\| | | [unidentified]. | | | | | | |
| 385, | gi\|15143 | e-107 | unnamed | unidentified | N/A | 57 | 57 | 337 | 346 |
| 386 | 035\|emb | | protein | | | | | | |
| | \|CAC50 | | product | unclassified. | | | | | |
| | 776.1\| | | [unidentified]. | | | | | | |

### Computer Systems

In one aspect of the invention, any nucleic acid sequence and/or polypeptide sequence of the invention can be stored, recorded, and manipulated on any medium which can be read and accessed by a computer. As used herein, the words "recorded" and "stored" refer to a process for storing information on a computer medium. Another aspect of the invention is a computer readable medium having recorded thereon at least 2, 5, 10, 15 or 20 nucleic acid sequences as set forth in SEQ ID NOS: 1-386, and sequences substantially identical thereto. In a further embodiment, another aspect is the "comparison among and between nucleic acid sequences or polypeptide sequences of the invention and the comparison between sequences of the invention and other sequences by a computer. Computer readable media include magnetically readable media, optically readable media, electronically readable media and magnetic/optical media. For example, the computer readable media may be a hard disk, a floppy disk, a magnetic tape, CD-ROM, Digital Versatile Disk (DVD), Random Access Memory (RAM), or Read Only Memory (ROM) as well as other types of other media known to those skilled in the art.

Embodiments of the invention include systems (*e.g*., internet based systems), particularly computer systems which store and manipulate the sequence information described herein. As used herein, "a computer system" refers to the hardware components, software components, and data storage components used to analyze a sequence (either nucleic acid or polypeptide) as set forth in at least any one of SEQ ID NOS: 1-386 and sequences substantially identical thereto. The computer system typically includes a processor for processing, accessing and manipulating the sequence data. The processor can be any well-known type of central processing unit, such as, for example, the Pentium III from Intel Corporation, or similar processor from Sun, Motorola, Compaq, AMD or International Business Machines.

Typically the computer system is a general purpose system that comprises the processor and one or more internal data storage components for storing data, and one or more data retrieving devices for retrieving the data stored on the data storage components.

In one particular embodiment, the computer system includes a processor connected to a bus which is connected to a main memory (preferably implemented as RAM) and one or more internal data storage devices, such as a hard drive and/or other computer readable media having data recorded thereon. In some embodiments, the computer system further includes one or more data retrieving device for reading the data stored on the internal data storage devices.

The data retrieving device may represent, for example, a floppy disk drive, a compact disk drive, a magnetic tape drive, or a modem capable of connection to a remote data storage system (e.g., via the internet) etc. In some embodiments, the internal data storage device is a removable computer readable medium such as a floppy disk, a compact disk, a magnetic tape, etc. containing control logic and/or data recorded thereon. The computer system may advantageously include or be programmed by appropriate software for reading the control logic and/or the data from the data storage component once inserted in the data retrieving device.

The computer system includes a display which is used to display output to a computer user. It should also be noted that the computer system can be linked to other computer systems in a network or wide area network to provide centralized access to the computer system. In some embodiments, the computer system may further comprise a sequence comparison algorithm. A "sequence comparison algorithm" refers to one or more programs which are implemented (locally or remotely) on the computer system to compare a nucleotide sequence with other nucleotide sequences and/or compounds stored within a data storage means.

### Uses of Nitrilases

Nitrilases have been identified as key enzymes for the production of chiral α-hydroxy acids, which are valuable intermediates in the fine chemicals industry, and as pharmaceutical intermediates. The nitrilase enzymes of the invention are useful to catalyze the stereoselective hydrolysis of cyanohydrins and aminonitriles, producing chiral α-hydroxy- and α-amino acids, respectively.

Stereoselective enzymes provide a key advantage over chemical resolution methods as they do not require harsh conditions and are more environmentally compatible. The use of nitrilases is of particular interest for the production of chiral amino acids and α-hydroxy acids. Using a stereoselective nitrilase, dynamic resolution conditions can be established, due to the racemisation of the substrate under aqueous conditions. Thus 100% theoretical yields are achievable.

This invention is directed to the nitrilases which have been discovered and isolated from naturally occurring sources. This invention is also directed to evolving novel genes and gene pathways from diverse and extreme environmental sources. In an effort to develop the most extensive assortment of enzymes available, DNA was extracted directly from samples that have been collected from varying habitats around the globe. From these efforts, the largest collection of environmental genetic libraries in the world was developed. Through extensive high-throughput screening of these libraries, 192 new sequence-unique nitrilase enzymes have been discovered to date. Previous to this invention, fewer than 20 microbial-derived nitrilases had been reported in the literature and public databases.

Biocatalysts, such as nitrilases, play an important role in catalyzing metabolic reactions in living organisms. In addition, biocatalysts have found applications in the chemical industry, where they can perform many different reactions. Some examples of the advantages of the use of nitrilases is that they provide: high enantio-, chemo-and regio- selectivity; they function under mild reaction conditions; they provide direct access to products - with minimal protection; they have high catalytic efficiencies; they produce reduced waste compared with the chemical alternatives; they are easily immobilized as enzymes or cells; they are recoverable, recyclable and are capable of being manipulated via molecular biological techniques; they can be regenerated in whole cell processes; they are tolerant to organic solvents; and importantly, they can be evolved or optimized. Optimized nitrilases are presented herein as working examples of the invention.

Nitrilases catalyze the hydrolysis of nitrile moieties generating the corresponding carboxylic acid. Conventional chemical hydrolysis of nitriles requires strong acid or base and high temperature. However, one advantage of the invention is that nitrilases are provided which perform this reaction under mild conditions. Wide ranges of nitrile substrates can be transformed by nitrilases with high enantio-, chemo-and regio- selectivity.

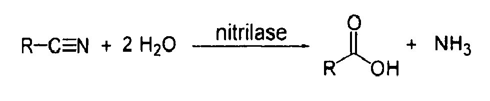

**Table 3 - Some characteristics of Nitrilases of the Invention**

| **Previously Discovered Nitrilases** | **New Nitrilases** | |
|---|---|---|
| **Limitations** | **New Features** | **Benefits** |
| < 20 reported | >180 newly discovered | Access to a wider substrate range |
| Homologous | Unique nitrilases, many with little homology to previously known nitrilases | |
| Narrow substrate activity spectrum | Broad substrate activity spectrum | |
| Very few shown to be enantioselective enantiomers | Enantioselective; both accessible | Product with high enantiomeric excess and minimal waste production |
| Limited stability profile | Stable in a variety of conditions | Potential use in a wide range of process conditions |
| Inconsistent supply | Consistent supply | Reliable source of product |
| Not applicable | Amenable to optimization | Good source material leads to better product |

Dynamic Kinetic Resolution: The use of the nitrilases allows discrimination between two rapidly equilibrating enantiomers to give a single product in 100% theoretical yield. Nitrilases are utilized for dynamic resolution of key cyanohydrins and aminonitriles to produce enantiomerically pure α-carboxylic and α-amino acids. Newly discovered nitrilases disclosed herein yield products with >95% enantiomeric excess (ee) with and >95% yield. The nitrilases perform this transformation efficiently under mild conditions in aqueous solution or in the presence of organic solvent.

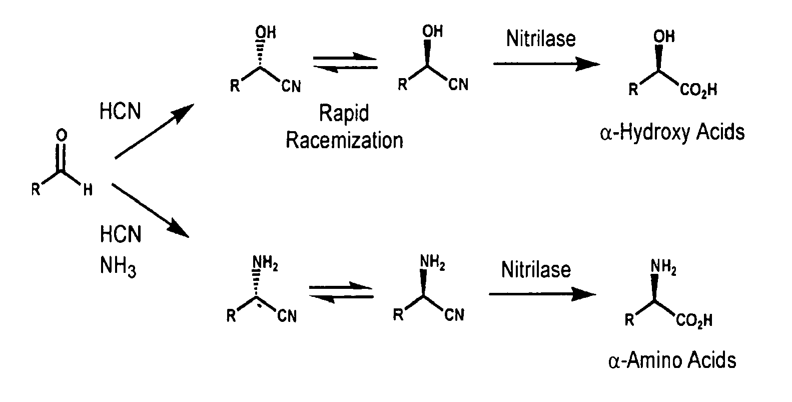

These products shown above also include the opposite enanatiomers, although they are not shown. In one aspect, the invention provides an isolated nucleic acid having a sequence as set forth in any one of the Group A nucleic acid sequences, having a sequence substantially identical thereto, or having a sequence complementary thereto.

In another aspect, the invention provides an isolated nucleic acid including at least 20 consecutive nucleotides identical to a portion of a nucleotide sequence as set forth in the Group A nucleic acid sequences, having a sequence substantially identical thereto, or having a sequence complementary thereto.

In another aspect, the invention provides an isolated nucleic acid encoding a polypeptide having a sequence as set forth in the Group B amino acid sequences, or having a sequence substantially identical thereto.

In another aspect, the invention provides an isolated nucleic acid encoding a polypeptide having at least 10 consecutive amino acids identical to a portion of a sequence as set fourth in the Group B amino acid sequences, or having a sequence substantially identical thereto.

In yet another aspect, the invention provides a substantially purified polypeptide comprising consecutive amino acid residues having a sequence as set forth in the Group B amino acid sequences, or having a sequence substantially identical thereto.

In another aspect, the invention provides an isolated antibody that specifically binds to a polypeptide of the invention. The invention also provides for a fragment of the antibody which retains the ability to specifically bind the polypeptide.

In another aspect, the invention provides a method of producing a polypeptide having a sequence as set forth in the Group B amino acid sequences, and sequences substantially identical thereto. The method includes introducing a nucleic acid encoding the polypeptide into a host cell, wherein the nucleic acid is operably joined to a promoter, and culturing the host cell under conditions that allow expression of the nucleic acid.

In another aspect, the invention provides a method of producing a polypeptide having at least 10 consecutive amino acids from a sequence as set forth in the Group B amino acid sequences, and sequences substantially identical thereto. The method includes introducing a nucleic acid encoding the polypeptide into a host cell, wherein the nucleic acid is operably joined to a promoter, and culturing the host cell under conditions that allow expression of the nucleic acid, thereby producing the polypeptide.

In another aspect, the invention provides a method of generating a variant of a nitrilase, including choosing a nucleic acid sequence as set forth in the Group A nucleic acid sequences, and changing one or more nucleotides in the sequence to another nucleotide, deleting one or more nucleotides in the sequence, or adding one or more nucleotides to the sequence.

In another aspect, the invention provides assays for identifying functional variants of the Group B amino acid sequences that retain the enzymatic function of the polypeptides of the Group B amino acid sequences. The assays include contacting a polypeptide comprising consecutive amino acid residues having a sequence identical to a sequence of the Group B amino acid sequences or a portion thereof, having a sequence substantially identical to a sequence of the Group B amino acid sequences or a portion thereof, or having a sequence which is a variant of a sequence of the Group B amino acid sequences that retains nitrilase activity, with a substrate molecule under conditions which allow the polypeptide to function, and detecting either a decrease in the level of substrate or an increase in the level of a specific reaction product of the reaction between the polypeptide and the substrate, thereby identifying a functional variant of such sequences.

### Modification of Polypeptides of the Invention

Enzymes are highly selective catalysts. Their hallmark is the ability to catalyze reactions with exquisite stereo-selectivity, regio-selectivity, and chemo-selectivity that is unparalleled in conventional synthetic chemistry. Moreover, enzymes are remarkably versatile. They can be tailored to function in organic solvents, operate at extreme pHs (for example, acidic or basic conditions) extreme temperatures (for example, high temperatures and low temperatures), extreme salinity levels (for example, high salinity and low salinity), and catalyze reactions with compounds that can be structurally unrelated to their natural, physiological substrates except for the enzymatic active site.

The invention provides methods for modifying polypeptides having nitrilase activity or polynucleotides encoding such polypeptides in order to obtain new polypeptides which retain nitrilase activity but which are improved with respect to some desired characteristic. Such improvements can include the ability to function (*i.e*., exhibit nitrilase activity) in organic solvents, operate at extreme or uncharacteristic pHs, operate at extreme or uncharacteristic temperatures, operate at extreme or uncharacteristic salinity levels, catalyze reactions with different substrates, etc.

The present invention directed to methods of using nitrilases so as to exploit the unique catalytic properties of these enzymes. Whereas the use of biocatalysts (*i.e*., purified or crude enzymes) in chemical transformations normally requires the identification of a particular biocatalyst that reacts with a specific starting compound, the present invention uses selected biocatalysts and reaction conditions that are specific for functional groups that are present in many starting compounds. Each biocatalyst is specific for one functional group, or several related functional groups, and can react with many starting compounds containing this functional group.

Enzymes react at specific sites within a starting compound without affecting the rest of the molecule, a process which is very difficult to achieve using traditional chemical methods. This high degree of specificity provides the means to identify a single active compound within a library of compounds. The library is characterized by the series of biocatalytic reactions used to produce it, a so-called "biosynthetic history." Screening the library for biological activities and tracing the biosynthetic history identifies the specific reaction sequence producing the active compound. The reaction sequence is repeated and the structure of the synthesized compound determined. This mode of identification, unlike other synthesis and screening approaches, does not require immobilization technologies, and compounds can be synthesized and tested free in solution using virtually any type of screening assay. It is important to note, that the high degree of specificity of enzyme reactions on functional groups allows for the "tracking" of specific enzymatic reactions that make up the biocatalytically produced library. (For further teachings on modification of molecules, including small molecules, see PCT Application No. PCT/US94/09174, herein incorporated by reference in its entirety).

In one exemplification, the invention provides for the chimerization of a family of related nitrilase genes and their encoded family of related products. Thus according to this aspect of the invention, the sequences of a plurality of nitrilase nucleic acids (*e*.*g*., the Group A nucleic acids) serve as nitrilase "templates" which are aligned using a sequence comparison algorithm such as those described above. One or more demarcation points are then identified in the aligned template sequences, which are located at one or more areas of homology. The demarcation points can be used to delineate the boundaries of nucleic acid building blocks, which are used to generate chimeric nitrilases. Thus, the demarcation points identified and selected in the nitrilase template molecules serve as potential chimerization points in the assembly of the chimeric nitrilase molecules.

Typically, a useful demarcation point is an area of local identity between at least two progenitor templates, but preferably the demarcation point is an area of identity that is shared by at least half of the templates, at least two thirds of the templates, at least three fourths of the templates, or at nearly all of the templates.

The building blocks, which are defined by the demarcation points, can then be mixed (either literally, in solution, or theoretically, on paper or in a computer) and reassembled to form chimeric nitrilase genes. In one embodiment, the gene reassembly process is performed exhaustively in order to generate an exhaustive library of all possible combinations. In other words, all possible ordered combinations of the nucleic acid building blocks are represented in the set of finalized chimeric nucleic acid molecules. At the same time, however, the order of assembly of each building block in the 5' to 3' direction in each combination is designed to reflect the order in the templates, and to reduce the production of unwanted, inoperative products.

In some embodiments, the gene reassembly process is performed systematically, in order to generate a compartmentalized library with compartments that can be screened systematically, *e*.*g*., one by one. In other words, the invention provides that, through the selective and judicious use of specific nucleic acid building blocks, coupled with the selective and judicious use of sequentially stepped assembly reactions, an experimental design can be achieved where specific sets of chimeric products are made in each of several reaction vessels. This allows a systematic examination and screening procedure to be performed. Thus, it allows a potentially very large number of chimeric molecules to be examined systematically in smaller groups.

In some embodiments, the synthetic nature of the step in which the building blocks are generated or reassembled allows the design and introduction of sequences of nucleotides (*e*.*g*., codons or introns or regulatory sequences) that can later be optionally removed in an *in vitro* process (*e*.*g*., by mutagenesis) or in an *in vivo* process (*e*.*g*., by utilizing the gene splicing ability of a host organism). The introduction of these nucleotides may be desirable for many reasons, including the potential benefit of creating a useful demarcation point.

The synthetic gene reassembly method of the invention utilizes a plurality of nucleic acid building blocks, each of which has two ligatable ends. Some examples of the two ligatable ends on each nucleic acid building block includes, but are not limited to, two blunt ends, or one blunt end and one overhang, or two overhangs. In a further, non-limiting example, the overhang can include one base pair, 2 base pairs, 3 base pairs, 4 base pairs or more.

A double-stranded nucleic acid building block can be of variable size. Preferred sizes for building blocks range from about 1 base pair (bp) (not including any overhangs) to about 100,000 base pairs (not including any overhangs). Other preferred size ranges are also provided, which have lower limits of from about 1 bp to about 10,000 bp (including every integer value in between), and upper limits of from about 2 bp to about 100,000 bp (including every integer value in between).

According to one embodiment, a double-stranded nucleic acid building block is generated by first generating two single stranded nucleic acids and allowing them to anneal to form a double-stranded nucleic acid building block. The two strands of a double-stranded nucleic acid building block may be complementary at every nucleotide apart from any that form an overhang; thus containing no mismatches, apart from any overhang(s). Alternatively, the two strands of a double-stranded nucleic acid building block can be complementary at fewer than every nucleotide, apart from any overhang(s). In particular, mismatches between the strands can be used to introduce codon degeneracy using methods such as the site-saturation mutagenesis described herein.

*In vivo* shuffling of molecules is also useful in providing variants and can be performed utilizing the natural property of cells to recombine multimers. While recombination *in vivo* has provided the major natural route to molecular diversity, genetic recombination remains a relatively complex process that involves (1) the recognition of homologies; (2) strand cleavage, strand invasion, and metabolic steps leading to the production of recombinant chiasma; and finally (3) the resolution of chiasma into discrete recombined molecules. The formation of the chiasma requires the recognition of homologous sequences.

Thus, the invention includes a method for producing a chimeric or recombinant polynucleotide from at least a first polynucleotide and a second polynucleotide *in vivo.* The invention can be used to produce a recombinant polynucleotide by introducing at least a first polynucleotide and a second polynucleotide which share at least one region of partial sequence homology (*e*.*g*., the Group A nucleic acid sequences, and combinations thereof) into a suitable host cell. The regions of partial sequence homology promote processes which result in sequence reorganization producing a recombinant polynucleotide. Such hybrid polynucleotides can result from intermolecular recombination events which promote sequence integration between DNA molecules. In addition, such hybrid polynucleotides can result from intramolecular reductive reassortment processes which utilize repeated sequences to alter a nucleotide sequence within a DNA molecule.

The invention provides a means for generating recombinant polynucleotides which encode biologically active variant polypeptides (*e*.*g*., a nitrilase variant). For example, a polynucleotide may encode a particular enzyme from one microorganism. An enzyme encoded by a first polynucleotide from one organism can, for example, function effectively under a particular environmental condition, *e*.*g*., high salinity. An enzyme encoded by a second polynucleotide from a different organism can function effectively under a different environmental condition, such as extremely high temperature. A recombined polynucleotide containing sequences from the first and second original polynucleotides encodes a variant enzyme which exhibits characteristics of both enzymes encoded by the original polynucleotides. Thus, the enzyme encoded by the recombined polynucleotide can function effectively under environmental conditions shared by each of the enzymes encoded by the first and second polynucleotides, *e*.*g*., high salinity and extreme temperatures.

A variant polypeptide can exhibit specialized enzyme activity not displayed in the original enzymes. For example, following recombination and/or reductive reassortment of polynucleotides encoding nitrilase activity, the resulting variant polypeptide encoded by a recombined polynucleotide can be screened for specialized nitrilase activity obtained from each of the original enzymes, *i*.*e*.*,* the temperature or pH at which the nitrilase functions. Sources of the original polynucleotides may be isolated from individual organisms ("isolates"), collections of organisms that have been grown in defined media ("enrichment cultures"), or, uncultivated organisms ("environmental samples"). The use of a culture-independent approach to derive polynucleotides encoding novel bioactivities from environmental samples is most preferable since it allows one to access untapped resources of biodiversity. The microorganisms from which the polynucleotide may be prepared include prokaryotic microorganisms, such as *Xanthobacter*, *Eubacteria* and *Archaebacteria,* and lower eukaryotic microorganisms such as fungi, some algae and protozoa. Polynucleotides may be isolated from environmental samples in which case the nucleic acid may be recovered without culturing of an organism or recovered from one or more cultured organisms. In one aspect, such microorganisms may be extremophiles, such as hyperthermophiles, psychrophiles, psychrotrophs, halophiles, barophiles and acidophiles. Polynucleotides encoding enzymes isolated from extremophilic microorganisms are particularly preferred. Such enzymes may function at temperatures above 100°C in terrestrial hot springs and deep sea thermal vents, at temperatures below 0°C in arctic waters, in the saturated salt environment of the Dead Sea, at pH values around 0 in coal deposits and geothermal sulfur-rich springs, or at pH values greater than 11 in sewage sludge.

Examples of mammalian expression systems that can be employed to express recombinant proteins include the COS-7, C127, 3T3, CHO, HeLa and BHK cell lines. Mammalian expression vectors comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice and polyadenylation sites may be used to provide the required nontranscribed genetic elements. U.S. Patent No. 6,054,267 is hereby incorporated by reference in its entirety.

Host cells containing the polynucleotides of interest can be cultured in conventional nutrient media modified as appropriate for activating promoters, selecting transformants or amplifying genes. The culture conditions, such as temperature, pH and the like, are those previously used with the host cell selected for expression, and will be apparent to the ordinarily skilled artisan. Clones, which are identified as having a desired enzyme activity or other property may then be sequenced to identify the recombinant polynucleotide sequence encoding the enzyme having the desired activity or property.

In one embodiment, the invention provides for the isolated nitrilases as either an isolated nucleic acid or an isolated polypeptide wherein the nucleic acid or the polypeptide was prepared by recovering DNA from a DNA population derived from at least one uncultivated microorganism, and transforming a host with recovered DNA to produce a library of clones which is screened for the specified protein, *e*.*g*. nitrilase activity. U.S. Patent No. 6,280,926, Short, provides descriptions of such methods and is hereby incorporated by reference in its entirety for all purposes.

Therefore, in a one embodiment, the invention relates to a method for producing a biologically active recombinant nitrilase polypeptide and screening such a polypeptide for desired activity or property by:
1) introducing at least a first nitrilase polynucleotide and a second nitrilase polynucleotide, said at least first nitrilase polynucleotide and second nitrilase polynucleotide sharing at least one region of sequence homology, into a suitable host cell;
2) growing the host cell under conditions which promote sequence reorganization resulting in a recombinant nitrilase polynucleotide;
3) expressing a recombinant nitrilase polypeptide encoded by the recombinant nitrilase polynucleotide;
4) screening the recombinant nitrilase polypeptide for the desired activity or property; and
5) isolating the recombinant nitrilase polynucleotide encoding the recombinant nitrilase polypeptide.

Examples of vectors which may be used include viral particles, baculovirus, phage, plasmids, phagemids, cosmids, fosmids, bacterial artificial chromosomes, viral DNA (*e*.*g*., vaccinia, adenovirus, fowlpox virus, pseudorabies and derivatives of SV40), P1-based artificial chromosomes, yeast plasmids, yeast artificial chromosomes, and any other vectors specific for the hosts of interest (*e*.*g*., *Bacillus*, *Aspergillus* and yeast). Large numbers of suitable vectors are known to those of skill in the art, and are commercially available. Examples of bacterial vectors include pQE vectors (Qiagen, Valencia, CA); pBluescript plasmids, pNH vectors, and lambda-ZAP vectors (Stratagene, La Jolla, CA); and pTRC99a, pKK223-3, pDR540, and pRIT2T vectors (Pharmacia, Peapack, NJ). Examples of eukaryotic vectors include pXT1 and pSG5 vectors (Stratagene, La Jolla, CA); and pSVK3, pBPV, pMSG, and pSVLSV40 vectors (Pharmacia, Peapack, NJ). However, any other plasmid or other vector may be used so long as they are replicable and viable in the host.

A preferred type of vector for use in the present invention contains an f-factor (or fertility factor) origin of replication. The f-factor in *E*. *coli* is a plasmid which effects high frequency transfer of itself during conjugation and less frequent transfer of the bacterial chromosome itself. A particularly preferred embodiment is to use cloning vectors referred to as "fosmids" or bacterial artificial chromosome (BAC) vectors. These are derived from *E*. *coli* f-factor which is able to stably integrate large segments of genomic DNA.

The DNA sequence in the expression vector is operably joined to appropriate expression control sequences, including a promoter, to direct RNA synthesis. Useful bacterial promoters include lacI, lacZ, T3, T7, gpt, lambda P_{R}, P_{L} and trp. Useful eukaryotic promoters include CMV immediate early, HSV thymidine kinase, early and late SV40, LTRs from retrovirus, and mouse metallothionein-I. Selection of the appropriate vector and promoter is well within the level of ordinary skill in the art. The expression vector also contains a ribosome binding site for translation initiation and a transcription terminator. The vector may also include appropriate sequences for amplifying expression. Promoter regions can be selected from any desired gene using CAT (chloramphenicol transferase) vectors or other vectors with selectable markers.

In addition, the expression vectors can contain one or more selectable marker genes to provide a phenotypic trait for selection of transformed host cells. Useful selectable markers include dihydrofolate reductase or neomycin resistance for eukaryotic cell culture, or tetracycline or ampicillin resistance in *E*. *coli*.

The vector may be introduced into the host cells using any of a variety of techniques, including transformation, transfection, transduction, viral infection, gene guns, or Ti-mediated gene transfer. Particular methods include calcium phosphate transfection, DEAE-Dextran mediated transfection, lipofection, or electroporation

Reductive Reassortment - In another aspect, variant nitrilase polynucleotides can be generated by the process of reductive reassortment. Whereas recombination is an "inter-molecular" process which, in bacteria, is generally viewed as a "recA-dependent" phenomenon, the process of "reductive reassortment" occurs by an "intra-molecular", recA-independent process. In this aspect, the invention can rely on the ability of cells to mediate reductive processes to decrease the complexity of quasi-repeated sequences in the cell by deletion. The method involves the generation of constructs containing consecutive repeated or quasi-repeated sequences (original encoding sequences), the insertion of these sequences into an appropriate vector, and the subsequent introduction of the vector into an appropriate host cell. The reassortment of the individual molecular identities occurs by combinatorial processes between the consecutive sequences in the construct possessing regions of homology, or between quasi-repeated units. The reassortment process recombines and/or reduces the complexity and extent of the repeated sequences, and results in the production of novel molecular species. Various treatments may be applied to enhance the rate of reassortment, such as ultra-violet light or DNA damaging chemicals. In addition, host cell lines displaying enhanced levels of "genetic instability" can be used.

Repeated Sequences - Repeated or "quasi-repeated" sequences play a role in genetic instability. In the present invention, "quasi-repeats" are repeats that are not identical in structure but, rather, represent an array of consecutive sequences which have a high degree of similarity or identity sequences. The reductive reassortment or deletion process in the cell reduces the complexity of the resulting construct by deleting sequences between positions within quasi-repeated sequences. Because the deletion (and potentially insertion) events can occur virtually anywhere within the quasi-repetitive units, these sequences provide a large repertoire of potential variants.

When the quasi-repeated sequences are all ligated in the same orientation, for instance head-to-tail or vice versa, the endpoints of a deletion are, for the most part, equally likely to occur anywhere within the quasi-repeated sequences. In contrast, when the units are presented head-to-head or tail-to-tail, the inverted quasi-repeated sequences can form a duplex which delineates the endpoints of the adjacent units and thereby favors deletion of discrete units. Therefore, it is preferable in the present invention that the quasi-repeated sequences are joined in the same orientation because random orientation of quasi-repeated sequences will result in the loss of reassortment efficiency, while consistent orientation of the sequences will offer the highest efficiency. Nonetheless, although having fewer of the contiguous sequences in the same orientation decreases the efficiency or reductive reassortment, it may still provide sufficient variation for the effective recovery of novel molecules.

Sequences can be assembled in a head-to-tail orientation using any of a variety of methods, including the following:
a) Primers can be utilized that include a poly-A head and poly-T tail which, when made single-stranded, would provide orientation. This is accomplished by having the first few bases of the primers made from RNA and hence easily removed by RNAse H.
b) Primers can be utilized that include unique restriction cleavage sites. Multiple sites, a battery of unique sequences, and repeated synthesis and ligation steps would be required.
c) The inner few bases of the primer can be thiolated and an exonuclease used to produce properly tailed molecules.

The recovery of the reassorted sequences relies on the identification of cloning vectors with a reduced repetitive index (RI). The reassorted coding sequences can then be recovered by amplification. The products are recloned and expressed. The recovery of cloning vectors with reduced R1 can be effected by:
1) The use of vectors only stably maintained when the construct is reduced in complexity.
2) The physical recovery of shortened vectors by physical procedures. In this case, the cloning vector would be recovered using standard plasmid isolation procedures and then size-fractionated using standard procedures (*e*.*g*., agarose gel or column with a low molecular weight cut off).
3) The recovery of vectors containing interrupted genes can be selected when insert size decreases.
4) The use of direct selection techniques wherein an expression vector is used and the appropriate selection is carried out.

Coding sequences from related organisms may demonstrate a high degree of homology but nonetheless encode quite diverse protein products. These types of sequences are particularly useful in the present invention as quasi-repeats. However, while the examples illustrated below demonstrate the reassortment of coding sequences with a high degree of identity (quasi-repeats), this process is not limited to nearly identical repeats.

The following example demonstrates a method of the invention. Quasi-repetitive coding sequences derived from three different species are obtained. Each sequence encodes a protein with a distinct set of properties. Each of the sequences differs by one or more base pairs at unique positions in the sequences which are designated "A", "B" and "C". The quasi-repeated sequences are separately or collectively amplified and ligated into random assemblies such that all possible permutations and combinations are available in the population of ligated molecules. The number of quasi-repeat units can be controlled by the assembly conditions. The average number of quasi-repeated units in a construct is defined as the repetitive index (RI).

Once formed, the constructs can be size-fractionated on an agarose gel according to published protocols, inserted into a cloning vector, and transfected into an appropriate host cell. The cells can then be propagated to allow reductive reassortment to occur. The rate of the reductive reassortment process may be stimulated by the introduction of DNA damage if desired. Whether the reduction in RI is mediated by deletion formation between repeated sequences by an "intra-molecular" mechanism, or mediated by recombination-like events through "inter-molecular" mechanisms is immaterial. The end result is a reassortment of the molecules into all possible combinations.

In another aspect, prior to or during recombination or reassortment, polynucleotides of the invention or polynucleotides generated by the methods described herein can be subjected to agents or processes which promote the introduction of mutations into the original polynucleotides. The introduction of such mutations would increase the diversity of resulting hybrid polynucleotides and polypeptides encoded therefrom. The agents or processes which promote mutagenesis include, but are not limited to: (+)-CC-1065, or a synthetic analog such as (+)-CC-1065-(N3-adenine) (Sun et al. (1992), Biochemistry 31(10):2822-9); an N-acetylated or deacetylated 4'-fluoro-4-aminobiphenyl adduct capable of inhibiting DNA synthesis (see, for example, van de Poll et al. (1992), Carcinogenesis 13(5):751-8); or a N-acetylated or deacetylated 4-aminobiphenyl adduct capable of inhibiting DNA synthesis (see also, Van de Poll et al. (1992), *supra*); trivalent chromium, a trivalent chromium salt, a polycyclic aromatic hydrocarbon ("PAH") DNA adduct capable of inhibiting DNA replication, such as 7-bromomethyl-benz[a]anthracene ("BMA"), tris(2,3-dibromopropyl)phosphate ("Tris-BP"), 1,2-dibromo-3-chloropropane ("DBCP"), 2-bromoacrolein (2BA), benzo[a]pyrene-7,8-dihydrodiol-9-10-epoxide ("BPDE"), a platinum(II) halogen salt, N-hydroxy-2-amino-3-methylimidazo[4,5-f]-quinoline ("N-hydroxy-IQ"), and N-hydroxy-2-amino-1-methyl-6-phenylimidazo[4,5-f]-pyridine ("N-hydroxy-PhIP"). Especially preferred means for slowing or halting PCR amplification consist of UV light (+)-CC-1065 and (+)-CC-1065-(N3-Adenine). Particularly encompassed means are DNA adducts or polynucleotides comprising the DNA adducts from the polynucleotides or polynucleotides pool, which can be released or removed by a process including heating the solution comprising the polynucleotides prior to further processing.

GSSM^{™} - The invention also provides for the use of codon primers containing a degenerate N,N,G/T sequence to introduce point mutations into a polynucleotide, so as to generate a set of progeny polypeptides in which a full range of single amino acid substitutions is represented at each amino acid position, a method referred to as gene site-saturated mutagenesis (GSSM^{™}). The oligos used are comprised contiguously of a first homologous sequence, a degenerate N,N,G/T sequence, and possibly a second homologous sequence. The progeny translational products from the use of such oligos include all possible amino acid changes at each amino acid site along the polypeptide, because the degeneracy of the N,N,G/T sequence includes codons for all 20 amino acids.

In one aspect, one such degenerate oligo (comprising one degenerate N,N,G/T cassette) is used for subjecting each original codon in a parental polynucleotide template to a full range of codon substitutions. In another aspect, at least two degenerate N,N,G/T cassettes are used - either in the same oligo or not, for subjecting at least two original codons in a parental polynucleotide template to a full range of codon substitutions. Thus, more than one N,N,G/T sequence can be contained in one oligo to introduce amino acid mutations at more than one site. This plurality of N,N,G/T sequences can be directly contiguous, or separated by one or more additional nucleotide sequences. In another aspect, oligos serviceable for introducing additions and deletions can be used either alone or in combination with the codons containing an N,N,G/T sequence, to introduce any combination or permutation of amino acid additions, deletions, and/or substitutions.

In a particular exemplification, it is possible to simultaneously mutagenize two or more contiguous amino acid positions using an oligo that contains contiguous N,N,G/T triplets, *i*.*e*., a degenerate (N,N,G/T)ₙ sequence.

In another aspect, the present invention provides for the use of degenerate cassettes having less degeneracy than the N,N,G/T sequence. For example, it may be desirable in some instances to use a degenerate triplet sequence comprised of only one N, where said N can be in the first second or third position of the triplet. Any other bases including any combinations and permutations thereof can be used in the remaining two positions of the triplet. Alternatively, it may be desirable in some instances to use a degenerate N,N,N triplet sequence, or an N,N, G/C triplet sequence.

It is appreciated, however, that the use of a degenerate triplet (such as N,N,G/T or an N,N, G/C triplet sequence) as disclosed in the instant invention is advantageous for several reasons. In one aspect, this invention provides a means to systematically and fairly easily generate the substitution of the full range of the 20 possible amino acids into each and every amino acid position in a polypeptide. Thus, for a 100 amino acid polypeptide, the invention provides a way to systematically and fairly easily generate 2000 distinct species (*i*.*e*., 20 possible amino acids per position times 100 amino acid positions). It is appreciated that there is provided, through the use of an oligo containing a degenerate N,N,G/T or an N,N, G/C triplet sequence, 32 individual sequences that code for the 20 possible amino acids. Thus, in a reaction vessel in which a parental polynucleotide sequence is subjected to saturation mutagenesis using one such oligo, there are generated 32 distinct progeny polynucleotides encoding 20 distinct polypeptides. In contrast, the use of a nondegenerate oligo in site-directed mutagenesis leads to only one progeny polypeptide product per reaction vessel.

This invention also provides for the use of nondegenerate oligonucleotides, which can optionally be used in combination with degenerate primers disclosed. It is appreciated that in some situations, it is advantageous to use nondegenerate oligos to generate specific point mutations in a working polynucleotide. This provides a means to generate specific silent point mutations, point mutations leading to corresponding amino acid changes, and point mutations that cause the generation of stop codons and the corresponding expression of polypeptide fragments.

Thus, in one embodiment, each saturation mutagenesis reaction vessel contains polynucleotides encoding at least 20 progeny polypeptide molecules such that all 20 amino acids are represented at the one specific amino acid position corresponding to the codon position mutagenized in the parental polynucleotide. The 32-fold degenerate progeny polypeptides generated from each saturation mutagenesis reaction vessel can be subjected to clonal amplification (*e*.*g*., cloned into a suitable *E*. *coli* host using an expression vector) and subjected to expression screening. When an individual progeny polypeptide is identified by screening to display a favorable change in property (when compared to the parental polypeptide), it can be sequenced to identify the correspondingly favorable amino acid substitution contained therein.

It is appreciated that upon mutagenizing each and every amino acid position in a parental polypeptide using saturation mutagenesis as disclosed herein, favorable amino acid changes may be identified at more than one amino acid position. One or more new progeny molecules can be generated that contain a combination of all or part of these favorable amino acid substitutions. For example, if 2 specific favorable amino acid changes are identified in each of 3 amino acid positions in a polypeptide, the permutations include 3 possibilities at each position (no change from the original amino acid, and each of two favorable changes) and 3 positions. Thus, there are 3 x 3 x 3 or 27 total possibilities, including 7 that were previously examined - 6 single point mutations (*i*.*e*., 2 at each of three positions) and no change at any position.

In yet another aspect, site-saturation mutagenesis can be used together with shuffling, chimerization, recombination and other mutagenizing processes, along with screening. This invention provides for the use of any mutagenizing process(es), including saturation mutagenesis, in an iterative manner. In one exemplification, the iterative use of any mutagenizing process(es) is used in combination with screening.

Thus, in a non-limiting exemplification, polynucleotides and polypeptides of the invention can be derived by saturation mutagenesis in combination with additional mutagenization processes, such as process where two or more related polynucleotides are introduced into a suitable host cell such that a hybrid polynucleotide is generated by recombination and reductive reassortment.

In addition to performing mutagenesis along the entire sequence of a gene, mutagenesis can be used to replace each of any number of bases in a polynucleotide sequence, wherein the number of bases to be mutagenized can be each integer from about 15 to about 100,000. Thus, instead of mutagenizing every position along a molecule, one can subject every or a discrete number of bases (*e*.*g*., a subset totaling from about 15 to about 100,000) to mutagenesis. In one embodiment, a separate nucleotide is used for mutagenizing each position or group of positions along a polynucleotide sequence. A group of 3 positions to be mutagenized can be a codon. In one embodiment, the mutations are introduced using a mutagenic primer, containing a heterologous cassette, also referred to as a mutagenic cassette. For example, cassettes can have from about 1 to about 500 bases. Each nucleotide position in such heterologous cassettes can be N, A, C, G, T, A/C, A/G, A/T, C/G, C/T, G/T, C/G/T, A/G/T, A/C/T, A/C/G, or E, where E is any base that is not A, C, G, or T.

In a general sense, saturation mutagenesis comprises mutagenizing a complete set of mutagenic cassettes (for example, each cassette is about 1-500 bases in length) in a defined polynucleotide sequence to be mutagenized (for example, the sequence to be mutagenized is from about 15 to about 100,000 bases in length). Thus, a group of mutations (ranging from about 1 to about 100 mutations) is introduced into each cassette to be mutagenized. A grouping of mutations to be introduced into one cassette can be different or the same from a second grouping of mutations to be introduced into a second cassette during the application of one round of saturation mutagenesis. Such groupings are exemplified by deletions, additions, groupings of particular codons, and groupings of particular nucleotide cassettes.

Defined sequences to be mutagenized include a whole gene, pathway, cDNA, entire open reading frame (ORF), promoter, enhancer, repressor/transactivator, origin of replication, intron, operator, or any polynucleotide functional group. Generally, a "defined sequence" for this purpose may be any polynucleotide that a 15 base-polynucleotide sequence, and polynucleotide sequences of lengths between about 15 bases and about 15,000 bases (this invention specifically names every integer in between). Considerations in choosing groupings of codons include types of amino acids encoded by a degenerate mutagenic cassette.

In a particularly preferred exemplification a grouping of mutations that can be introduced into a mutagenic cassette, this invention specifically provides for degenerate codon substitutions (using degenerate oligos) that code for 2, 3, 4, 5, 6, 7, 8, 9, 10, 11, 12, 13, 14, 15, 16, 17, 18, 19, and 20 amino acids at each position, and a library of polypeptides encoded thereby.

One aspect of the invention is an isolated nucleic acid comprising one of the sequences of the Group A nucleic acid sequences, sequences substantially identical thereto, sequences complementary thereto, or a fragment comprising at least 10, 15, 20, 25, 30, 35, 40, 50, 75, 100, 150, 200, 300, 400, or 500 consecutive bases of one of the sequences of the Group A nucleic acid sequences. The isolated nucleic acids may comprise DNA, including cDNA, genomic DNA, and synthetic DNA. The DNA may be double-stranded or single-stranded, and if single stranded may be the coding strand or non-coding (anti-sense) strand. Alternatively, the isolated nucleic acids may comprise RNA.

As discussed in more detail below, the isolated nucleic acid sequences of the invention may be used to prepare one of the polypeptides of the Group B amino acid sequences, and sequences substantially identical thereto, or fragments comprising at least 5, 10, 15, 20, 25, 30, 35, 40, 50, 75, 100, or 150 consecutive amino acids of one of the polypeptides of the Group B amino acid sequences, and sequences substantially identical thereto.

Alternatively, the nucleic acid sequences of the invention may be mutagenized using conventional techniques, such as site directed mutagenesis, or other techniques familiar to those skilled in the art, to introduce silent changes into the polynucleotides of the Group A nucleic acid sequences, and sequences substantially identical thereto. As used herein, "silent changes" include, for example, changes which do not alter the amino acid sequence encoded by the polynucleotide. Such changes may be desirable in order to increase the level of the polypeptide produced by host cells containing a vector encoding the polypeptide by introducing codons or codon pairs which occur frequently in the host organism.

The invention also relates to polynucleotides which have nucleotide changes which result in amino acid substitutions, additions, deletions, fusions and truncations in the polypeptides of the invention (*e*.*g*., the Group B amino acid sequences). Such nucleotide changes may be introduced using techniques such as site-directed mutagenesis, random chemical mutagenesis, exonuclease III deletion, and other recombinant DNA techniques. Alternatively, such nucleotide changes may be naturally occurring allelic variants which are isolated by identifying nucleic acid sequences which specifically hybridize to probes comprising at least 10, 15, 20, 25, 30, 35, 40, 50, 75, 100, 150, 200, 300, 400, or 500 consecutive bases of one of the sequences of the Group A nucleic acid sequences, and sequences substantially identical thereto (or the sequences complementary thereto) under conditions of high, moderate, or low stringency as provided herein.

### Immobilized Enzyme Solid Supports

The enzymes, fragments thereof and nucleic acids which encode the enzymes and fragments can be affixed to a solid support. This is often economical and efficient in the use of the enzymes in industrial processes. For example, a consortium or cocktail of enzymes (or active fragments thereof), which are used in a specific chemical reaction, can be attached to a solid support and dunked into a process vat. The enzymatic reaction can occur. Then, the solid support can be taken out of the vat, along with the enzymes affixed thereto, for repeated use. In one embodiment of the invention, the isolated nucleic acid is affixed to a solid support. In another embodiment of the invention, the solid support is selected from the group of a gel, a resin, a polymer, a ceramic, a glass, a microelectrode and any combination thereof.

For example, solid supports useful in this invention include gels. Some examples of gels include sepharose, gelatin, glutaraldehyde, chitosan-treated glutaraldehyde, albumin-glutaraldehyde, chitosan-Xanthan, toyopearl gel (polymer gel), alginate, alginate-polylysine, carrageenan, agarose, glyoxyl agarose, magnetic agarose, dextran-agarose, poly(Carbamoyl Sulfonate) hydrogel, BSA-PEG hydrogel, phosphorylated polyvinyl alcohol (PVA), monoaminoethyl-N-aminoethyl (MANA), amino, or any combination thereof.

Another solid support useful in the present invention are resins or polymers. Some examples of resins or polymers include cellulose, acrylamide, nylon, rayon, polyester, anion-exchange resin, AMBERLITE™ XAD-7, AMBERLITE™ XAD-8, AMBERLITE™ IRA-94, AMBERLITE™ IRC-50, polyvinyl, polyacrylic, polymethacrylate, or any combination thereof. Another type of solid support useful in the present invention is ceramic. Some examples include non-porous ceramic, porous ceramic, SiO₂, Al₂O₃. Another type of solid support useful in the present invention is glass. Some examples include non-porous glass, porus glass, aminopropyl glass or any combination thereof. Another type of solid support which can be used is a mcroelectrode. An example is a polyethyleneimine-coated magnetite. Graphitic particles can be used as a solid support. Another example of a solid support is a cell, such as a red blood cell.

### Methods of immobilization

There are many methods which would be known to one of skill in the art for immobilizing enzymes or fragments thereof, or nucleic acids, onto a solid support. Some examples of such methods include electrostatic droplet generation, electrochemical means, via adsorption, via covalent binding, via cross-linking, via a chemical reaction or process, via encapsulation, via entrapment, via calcium alginate, or via poly (2-hydroxyethyl methacrylate). Like methods are described in Methods in Enzymology, Immobilized Enzymes and Cells, Part C. 1987. Academic Press. Edited by S. P. Colowick and N. O. Kaplan. Volume 136; and Immobilization of Enzymes and Cells. 1997. Humana Press. Edited by G. F. Bickerstaff. Series: Methods in Biotechnology, Edited by J. M. Walker.

Probes - The isolated nucleic acids of the Group A nucleic acid sequences, sequences substantially identical thereto, complementary sequences, or a fragment comprising at least 10, 15, 20, 25, 30, 35, 40, 50, 75, 100, 150, 200, 300, 400, or 500 consecutive bases of one of the foregoing sequences may also be used as probes to determine whether a biological sample, such as a soil sample, contains an organism having a nucleic acid sequence of the invention or an organism from which the nucleic acid was obtained. In such procedures, a biological sample potentially harboring the organism from which the nucleic acid was isolated is obtained and nucleic acids are obtained from the sample. The nucleic acids are contacted with the probe under conditions which permit the probe to specifically hybridize to any complementary sequences which are present therein.

Where necessary, conditions which permit the probe to specifically hybridize to complementary sequences may be determined by placing the probe in contact with complementary sequences from samples known to contain the complementary sequence as well as control sequences which do not contain the complementary sequence. Hybridization conditions, such as the salt concentration of the hybridization buffer, the formamide concentration of the hybridization buffer, or the hybridization temperature, can be varied to identify conditions which allow the probe to hybridize specifically to complementary nucleic acids. Stringent hybridization conditions are recited herein.

Hybridization may be detected by labeling the probe with a detectable agent such as a radioactive isotope, a fluorescent dye or an enzyme capable of catalyzing the formation of a detectable product. Many methods for using the labeled probes to detect the presence of complementary nucleic acids in a sample are familiar to those skilled in the art. These include Southern Blots, Northern Blots, colony hybridization procedures, and dot blots. Protocols for each of these procedures are provided in Ausubel et al. (1997), Current Protocols in Molecular Biology, John Wiley & Sons, Inc., and Sambrook et al. (1989), Molecular Cloning: A Laboratory Manual 2d Ed., Cold Spring Harbor Laboratory Press, the entire disclosures of which are incorporated herein by reference.

In one example, a probe DNA is "labeled" with one partner of a specific binding pair *(i*.*e*., a ligand) and the other partner of the pair is bound to a solid matrix to provide ease of separation of target from its source. For example, the ligand and specific binding partner can be selected from, in either orientation, the following: (1) an antigen or hapten and an antibody or specific binding fragment thereof; (2) biotin or iminobiotin and avidin or streptavidin; (3) a sugar and a lectin specific therefor; (4) an enzyme and an inhibitor therefor; (5) an apoenzyme and cofactor; (6) complementary homopolymeric oligonucleotides; and (7) a hormone and a receptor therefor. In one example, the solid phase is selected from: (1) a glass or polymeric surface; (2) a packed column of polymeric beads; and (3) magnetic or paramagnetic particles.

Alternatively, more than one probe (at least one of which is capable of specifically hybridizing to any complementary sequences which are present in the nucleic acid sample), may be used in an amplification reaction to determine whether the sample contains an organism containing a nucleic acid sequence of the invention (*e*.*g*., an organism from which the nucleic acid was isolated). Typically, the probes comprise oligonucleotides. In one embodiment, the amplification reaction may comprise a PCR reaction. PCR protocols are described in Ausubel et al. (1997), *supra*, and Sambrook et al. (1989), *supra*. Alternatively, the amplification may comprise a ligase chain reaction, 3SR, or strand displacement reaction. (See Barany (1991), PCR Methods and Applications 1:5-16; Fahy et al. (1991), PCR Methods and Applications 1:25-33; and Walker et al. (1992), Nucleic Acid Research 20:1691-1696, the disclosures of which are incorporated herein by reference in their entireties).

Probes derived from sequences near the ends of a sequence as set forth in the Group A nucleic acid sequences, and sequences substantially identical thereto, may also be used in chromosome walking procedures to identify clones containing genomic sequences located adjacent to the nucleic acid sequences as set forth above. Such methods allow the isolation of genes which encode additional proteins from the host organism.

An isolated nucleic acid sequence as set forth in the Group A nucleic acid sequences, sequences substantially identical thereto, sequences complementary thereto, or a fragment comprising at least 10, 15, 20, 25, 30, 35, 40, 50, 75, 100, 150, 200, 300, 400, or 500 consecutive bases of one of the foregoing sequences may be used as probes to identify and isolate related nucleic acids. In some embodiments, the related nucleic acids may be cDNAs or genomic DNAs from organisms other than the one from which the nucleic acid was isolated. For example, the other organisms may be related organisms. In such procedures, a nucleic acid sample is contacted with the probe under conditions which permit the probe to specifically hybridize to related sequences. Hybridization of the probe to nucleic acids from the related organism is then detected using any of the methods described above.

In nucleic acid hybridization reactions, the conditions used to achieve a particular level of stringency will vary, depending on the nature of the nucleic acids being hybridized. For example, the length of the nucleic acids, the amount of complementarity between the nucleic acids, the nucleotide sequence composition (*e*.*g*., G-C rich v. A-T rich content), and the nucleic acid type (*e*.*g*., RNA v. DNA) can be considered in selecting hybridization conditions. Stringency may be varied by conducting the hybridization at varying temperatures below the melting temperatures of the probes. The melting temperature, Tm, is the temperature (under defined ionic strength and pH) at which 50% of the target sequence hybridizes to a perfectly complementary probe. Stringent conditions are selected to be equal to or about 5°C lower than the Tm for a particular probe. The melting temperature of the probe may be calculated using the following formulas:

For probes between 14 and 70 nucleotides in length the melting temperature (Tm) is calculated using the formula: Tm=81.5+16.6(log [Na⁺])+0.41(fraction G+C)-(600/N) where N is the length of the probe.

If the hybridization is carried out in a solution containing formamide, the melting temperature may be calculated using the equation: Tm=81.5+16.6(log [Na⁺])+0.41(fraction G+C)-(0.63% formamide)-(600/N) where N is the length of the probe.

Expression Libraries - Expression libraries can be created using the polynucleotides of the invention in combination with expression vectors and appropriate host cells. The library allows for the *in vivo* expression of the polypeptides which are encoded by the polynucleotides of the invention. After such expression libraries have been generated one can include the additional step of "biopanning" such libraries prior to screening by cell sorting. The "biopanning" procedure refers to a process for identifying clones having a specified biological activity by screening for sequence identity in a library of clones prepared by (i) selectively isolating target DNA derived from at least one microorganism by use of at least one probe DNA comprising at least a portion of a DNA sequence encoding a polypeptide having a specified biological activity (*e*.*g*., nitrilase activity); and (ii) optionally transforming a host with the isolated target DNA to produce a library of clones which are screened for the specified biological activity.

The probe DNA used for selectively isolating the target DNA of interest from the DNA derived from at least one microorganism can be a full-length coding region sequence or a partial coding region sequence of DNA for an enzyme of known activity. The original DNA library can be probed using mixtures of probes comprising at least a portion of DNA sequences encoding enzymes having the specified enzyme activity. These probes or probe libraries are single-stranded and the microbial DNA which is probed has been converted into single-stranded form. The probes that are particularly suitable are those derived from DNA encoding enzymes having an activity similar or identical to the specified enzyme activity that is to be screened.

Having prepared a multiplicity of clones from DNA selectively isolated from an organism, such clones are screened for a specific enzyme activity and to identify the clones having the specified enzyme characteristics.

The screening for enzyme activity may be affected on individual expression clones or may be initially affected on a mixture of expression clones to ascertain whether or not the mixture has one or more specified enzyme activities. If the mixture has a specified enzyme activity, then the individual clones may be rescreened for such enzyme activity or for a more specific activity. Thus, for example, if a clone mixture has nitrilase activity, then the individual clones may be recovered and screened to determine which of such clones has nitrilase activity.

As described with respect to one of the above aspects, the invention provides a process for enzyme activity screening of clones containing selected DNA derived from a microorganism which process includes: screening a library for specified enzyme activity, said library including a plurality of clones, said clones having been prepared by recovering from genomic DNA of a microorganism selected DNA, which DNA is selected by hybridization to at least one DNA sequence which is all or a portion of a DNA sequence encoding an enzyme having the specified activity; and transforming a host with the selected DNA to produce clones which are screened for the specified enzyme activity.

In one embodiment, a DNA library derived from a microorganism is subjected to a selection procedure to select therefrom DNA which hybridizes to one or more probe DNA sequences which is all or a portion of a DNA sequence encoding an enzyme having the specified enzyme activity by:
(a) contacting the single-stranded DNA population from the DNA library with the DNA probe bound to a ligand under stringent hybridization conditions so as to produce a duplex between the probe and a member of the DNA library;
(b) contacting the duplex with a solid phase specific binding partner for the ligand so as to produce a solid phase complex;
(c) separating the solid phase complex from the non-duplexed members of the DNA library;
(d) denaturing the duplex to release the member of the DNA library;
(e) creating a complementary DNA strand of the member from step (d) so as to make the member a double-stranded DNA;
(f) introducing the double-stranded DNA into a suitable host so as to express a polypeptide which is encoded by the member DNA; and
(g) determining whether the polypeptide expressed exhibits the specified enzymatic activity.

In another aspect, the process includes a preselection to recover DNA including signal or secretion sequences. In this manner it is possible to select from the genomic DNA population by hybridization as hereinabove described only DNA which includes a signal or secretion sequence. The following paragraphs describe the protocol for this embodiment of the invention, the nature and function of secretion signal sequences in general and a specific exemplary application of such sequences to an assay or selection process.

A particularly embodiment of this aspect further comprises, after (a) but before (b) above, the steps of:
(i) contacting the single-stranded DNA population of (a) with a ligand-bound oligonucleotide probe that is complementary to a secretion signal sequence unique to a given class of proteins under hybridization conditions to form a double-stranded DNA duplex;
(ii) contacting the duplex of (i) with a solid phase specific binding partner for said ligand so as to produce a solid phase complex;
(iii) separating the solid phase complex from the single-stranded DNA population of (a);
(iv) denaturing the duplex so as to release single-stranded DNA members of the genomic population; and
(v) separating the single-stranded DNA members from the solid phase bound probe.

The DNA which has been selected and isolated to include a signal sequence is then subjected to the selection procedure hereinabove described to select and isolate therefrom DNA which binds to one or more probe DNA sequences derived from DNA encoding an enzyme(s) having the specified enzyme activity. This procedure is described and exemplified in U.S. Pat. No. 6,054,267, incorporated herein by reference in its entirety.

*In vivo* biopanning may be performed utilizing a (fluorescence activated cell sorter) FACS-based machine. Complex gene libraries are constructed with vectors which contain elements which stabilize transcribed RNA. For example, the inclusion of sequences which result in secondary structures such as hairpins which are designed to flank the transcribed regions of the RNA would serve to enhance their stability, thus increasing their half life within the cell. The probe molecules used in the biopanning process consist of oligonucleotides labeled with reporter molecules that only fluoresce upon binding of the probe to a target molecule. These probes are introduced into the recombinant cells from the library using one of several transformation methods. The probe molecules bind to the transcribed target mRNA resulting in DNA/RNA heteroduplex molecules. Binding of the probe to a target will yield a fluorescent signal that is detected and sorted by the FACS machine during the screening process.

In some embodiments, the nucleic acid encoding one of the polypeptides of the Group B amino acid sequences, sequences substantially identical thereto, or fragments comprising at least about 5, 10, 15, 20, 25, 30, 35, 40, 50, 75, 100, or 150 consecutive amino acids thereof is assembled in appropriate phase with a leader sequence capable of directing secretion of the translated polypeptide or fragment thereof. Optionally, the nucleic acid can encode a fusion polypeptide in which one of the polypeptides of the Group B amino acid sequences, sequences substantially identical thereto, or fragments comprising at least 5, 10, 15, 20, 25, 30, 35, 40, 50, 75, 100, or 150 consecutive amino acids thereof is fused to heterologous peptides or polypeptides, such as N-terminal identification peptides which impart desired characteristics, such as increased stability or simplified purification.

The host cell may be any of the host cells familiar to those skilled in the art, including prokaryotic cells, eukaryotic cells, mammalian cells, insect cells, or plant cells. As representative examples of appropriate hosts, there may be mentioned: bacterial cells, such as *E*. *coli, Streptomyces, Bacillus subtilis, Salmonella typhimurium* and various species within the genera *Pseudomonas, Streptomyces,* and *Staphylococcus,* fungal cells, such as yeast, insect cells such as *Drosophila* S2 and *Spodoptera* Sf9, animal cells such as CHO, COS or Bowes melanoma, and adenoviruses. The selection of an appropriate host is within the abilities of those skilled in the art.

Where appropriate, the engineered host cells can be cultured in conventional nutrient media modified as appropriate for activating promoters, selecting transformants or amplifying the genes of the invention. Following transformation of a suitable host strain and growth of the host strain to an appropriate cell density, the selected promoter may be induced by appropriate means (*e*.*g*., temperature shift or chemical induction) and the cells may be cultured for an additional period to allow them to produce the desired polypeptide or fragment thereof.

Cells are typically harvested by centrifugation, disrupted by physical or chemical means, and the resulting crude extract is retained for further purification. Microbial cells employed for expression of proteins can be disrupted by any convenient method, including freeze-thaw cycling, sonication, mechanical disruption, or use of cell lysing agents. Such methods are well known to those skilled in the art. The expressed polypeptide or fragment thereof can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the polypeptide. If desired, high performance liquid chromatography (HPLC) can be employed for final purification steps.

Various mammalian cell culture systems can also be employed to express recombinant protein. Examples of mammalian expression systems include the COS-7 lines of monkey kidney fibroblasts (described by Gluzman (1981), Cell 23:175,), and other cell lines capable of expressing proteins from a compatible vector, such as the C127, 3T3, CHO, HeLa and BHK cell lines.

The invention also relates to variants of the polypeptides of the Group B amino acid sequences, sequences substantially identical thereto, or fragments comprising at least 5, 10, 15, 20, 25, 30, 35, 40, 50, 75, 100, or 150 consecutive amino acids thereof. In particular, the variants may differ in amino acid sequence from the polypeptides of the Group B amino acid sequences, and sequences substantially identical thereto, by one or more substitutions, additions, deletions, fusions and truncations, which may be present in any combination.

The variants may be naturally occurring or created *in vitro.* In particular, such variants may be created using genetic engineering techniques such as site directed mutagenesis, random chemical mutagenesis, Exonuclease III deletion procedures, and standard cloning techniques. Alternatively, such variants, fragments, analogs, or derivatives may be created using chemical synthesis or modification procedures.

Other methods of making variants are also familiar to those skilled in the art. These include procedures in which nucleic acid sequences obtained from natural isolates are modified to generate nucleic acids which encode polypeptides having characteristics which enhance their value in industrial or laboratory applications. In such procedures, a large number of variant sequences having one or more nucleotide differences with respect to the sequence obtained from the natural isolate are generated and characterized. Typically, these nucleotide differences result in amino acid changes with respect to the polypeptides encoded by the nucleic acids from the natural isolates.

### Error Prone PCR

For example, variants may be created using error prone PCR. In error prone PCR, PCR is performed under conditions where the copying fidelity of the DNA polymerase is low, such that a high rate of point mutations is obtained along the entire length of the PCR product. Error prone PCR is described in Leung et al. (1989), Technique 1:11-15 and Caldwell et al. (1992), PCR Methods Applic. 2:28-33, the disclosures of which are incorporated herein by reference in their entirety. Briefly, in such procedures, nucleic acids to be mutagenized are mixed with PCR primers and reagents (e.g., reaction buffer, MgCl₂, MnCl₂, Taq polymerase and an appropriate concentration of dNTPs) for achieving a high rate of point mutation along the entire length of the PCR product. For example, the reaction may be performed using 20 fmoles of nucleic acid to be mutagenized, 30 pmoles of each PCR primer, a reaction buffer comprising 50mM KCl, 10mM Tris HCl (pH 8.3) and 0.01% gelatin, 7mM MgCl₂, 0.5mM MnCl₂, 5 units ofTaq polymerase, 0.2mM dGTP, 0.2mM dATP, 1mM dCTP, and 1mM dTTP. PCR may be performed for 30 cycles of 94°C for 1 min, 45°C for 1 min, and 72°C for 1 min. However, it will be appreciated that these parameters may be varied as appropriate. The mutagenized nucleic acids are cloned into an appropriate vector and the activities of the polypeptides encoded by the mutagenized nucleic acids are evaluated.

Variants also may be created using oligonucleotide directed mutagenesis to generate site-specific mutations in any cloned DNA of interest. Oligonucleotide mutagenesis is described in Reidhaar-Olson et al. (1988), Science, 241:53-57, the disclosure of which is incorporated herein by reference in its entirety. Briefly, in such procedures a plurality of double stranded oligonucleotides bearing one or more mutations to be introduced into the cloned DNA are synthesized and inserted into the cloned DNA to be mutagenized. Clones containing the mutagenized DNA are recovered and the activities of the polypeptides they encode are assessed.

### Assembly PCR

Another method for generating variants is assembly PCR. Assembly PCR involves the assembly of a PCR product from a mixture of small DNA fragments. A large number of different PCR reactions occur in parallel in the same vial, with the products of one reaction priming the products of another reaction. Assembly PCR is described in U.S. Pat. No. 5,965,408, the disclosure of which is incorporated herein by reference in its entirety.

### Sexual PCR mutagenesis

Still another method of generating variants is sexual PCR mutagenesis. In sexual PCR mutagenesis, forced homologous recombination occurs between DNA molecules of different but highly related DNA *sequence in vitro*, as a result of random fragmentation of the DNA molecule based on sequence homology, followed by fixation of the crossover by primer extension in a PCR reaction. Sexual PCR mutagenesis is described in Stemmer (1994), Proc. Natl. Acad. Sci. USA 91:10747-10751, the disclosure of which is incorporated herein by reference in its entirety. Briefly, in such procedures a plurality of nucleic acids to be recombined are digested with DNAse to generate fragments having an average size of about 50-200 nucleotides. Fragments of the desired average size are purified and resuspended in a PCR mixture. PCR is conducted under conditions which facilitate recombination between the nucleic acid fragments. For example, PCR may be performed by resuspending the purified fragments at a concentration of 10-30 ng/µl in a solution of 0.2mM of each dNTP, 2.2mM MgCl₂, 50mM KCl, 10mM Tris HCl, pH 9.0, and 0.1% Triton X-100. 2.5 units of Taq polymerase per 100µl of reaction mixture is added and PCR is performed using the following regime: 94°C for 60 seconds, 94°C for 30 seconds, 50-55°C for 30 seconds, 72°C for 30 seconds (30-45 times) and 72°C for 5 minutes. However, it will be appreciated that these parameters may be varied as appropriate. In some embodiments, oligonucleotides may be included in the PCR reactions. In other embodiments, the Klenow fragment of DNA polymerase I may be used in a first set of PCR reactions and Taq polymerase may be used in a subsequent set of PCR reactions. Recombinant sequences are isolated and the activities of the polypeptides they encode are assessed.

### In vivo Mutagenesis

Variants may also be created by *in vivo* mutagenesis. In some embodiments, random mutations in a sequence of interest are generated by propagating the sequence of interest in a bacterial strain, such as an *E*. *coli* strain, which carries mutations in one or more of the DNA repair pathways. Such "mutator" strains have a higher random mutation rate than that of a wild-type parent. Propagating the DNA in one of these strains will eventually generate random mutations within the DNA. Mutator strains suitable for use for in vivo mutagenesis are described in PCT Publication No. WO 91/16427 the disclosure of which is incorporated herein by reference in its entirety.

### Cassette Mutagenesis

Variants may also be generated using cassette mutagenesis. In cassette mutagenesis a small region of a double stranded DNA molecule is replaced with a synthetic oligonucleotide "cassette" that differs from the native sequence. The oligonucleotide often contains completely and/or partially randomized native sequence.

### Recursive Ensemble Mutagenesis

Recursive ensemble mutagenesis may also be used to generate variants. Recursive ensemble mutagenesis is an algorithm for protein engineering (protein mutagenesis) developed to produce diverse populations of phenotypically related mutants whose members differ in amino acid sequence. This method uses a feedback mechanism to control successive rounds of combinatorial cassette mutagenesis. Recursive ensemble mutagenesis is described in Arkin et al. (1992), Proc. Natl. Acad. Sci. USA, 89:7811-7815, the disclosure of which is incorporated herein by reference in its entirety.

### Exponential Ensemble Mutagenesis

In some embodiments, variants are created using exponential ensemble mutagenesis. Exponential ensemble mutagenesis is a process for generating combinatorial libraries with a high percentage of unique and functional mutants, wherein small groups of residues are randomized in parallel to identify, at each altered position, amino acids which lead to functional proteins. Exponential ensemble mutagenesis is described in Delegrave et al. (1993), Biotechnology Research 11:1548-1552, the disclosure of which incorporated herein by reference in its entirety.

### Random and site-directed mutagenesis

Random and site-directed mutagenesis is described in Arnold (1993), Current Opinions in Biotechnology 4:450-455, the disclosure of which is incorporated herein by reference in its entirety.

### Shuffling Procedures

In some embodiments, the variants are created using shuffling procedures wherein portions of a plurality of nucleic acids which encode distinct polypeptides are fused together to create chimeric nucleic acid sequences which encode chimeric polypeptides as described in U.S. Patent. Nos. 5,965,408 and 5,939,250, each of which is hereby incorporated by reference in their entireties.

The variants of the polypeptides of the Group B amino acid sequences may be variants in which one or more of the amino acid residues of the polypeptides of the Group B amino acid sequences are substituted with a conserved or non-conserved amino acid residue (*e*.*g*, a conserved amino acid residue) and such substituted amino acid residue may or may not be one encoded by the genetic code.

Conservative substitutions are those that substitute a given amino acid in a polypeptide by another amino acid of like characteristics. Typically seen as conservative substitutions are the following replacements: replacements of an aliphatic amino acid such as Alanine, Valine, Leucine and Isoleucine with another aliphatic amino acid; replacement of a Serine with a Threonine or vice versa; replacement of an acidic residue such as Aspartic acid and Glutamic acid with another acidic residue; replacement of a residue bearing an amide group, such as Asparagine and Glutamine, with another residue bearing an amide group; exchange of a basic residue such as Lysine and Arginine with another basic residue; and replacement of an aromatic residue such as Phenylalanine, Tyrosine with another aromatic residue.

Other variants are those in which one or more of the amino acid residues of the polypeptides of the Group B amino acid sequences includes a substituent group.

Still other variants are those in which the polypeptide is associated with another compound, such as a compound to increase the half-life of the polypeptide (for example, polyethylene glycol).

Additional variants are those in which additional amino acids are fused to the polypeptide, such as a leader sequence, a secretory sequence, a proprotein sequence or a sequence which facilitates purification, enrichment, or stabilization of the polypeptide.

In some embodiments, the fragments, derivatives and analogs retain the same biological function or activity as the polypeptides of the Group B amino acid sequences, and sequences substantially identical thereto. In other embodiments, the fragment, derivative, or analog includes a proprotein, such that the fragment, derivative, or analog can be activated by cleavage of the proprotein portion to produce an active polypeptide.

Another aspect of the invention is polypeptides or fragments thereof which have at least about 85%, at least about 90%, at least about 95%, or more than about 95% homology to one of the polypeptides of the Group B amino acid sequences, sequences substantially identical thereto, or a fragment comprising at least 5, 10, 15, 20, 25, 30, 35, 40, 50, 75, 100, or 150 consecutive amino acids thereof. Percent identity may be determined using any of the programs described above which aligns the polypeptides or fragments being compared and determines the extent of amino acid homology or similarity between them. It will be appreciated that amino acid "homology" includes conservative amino acid substitutions such as those described above. In one embodiment of the invention, the fragments can be used to generate antibodies. These antibodies can be used to immobilize nitrilases can be used in industrial processes. Polynucleotides encoding the nitrilases of the present invention can be used in a similar way.

Alternatively, the homologous polypeptides or fragments may be obtained through biochemical enrichment or purification procedures. The sequence of potentially homologous polypeptides or fragments may be determined by proteolytic digestion, gel electrophoresis and/or microsequencing. The sequence of the prospective homologous polypeptide or fragment can be compared to one of the polypeptides of the Group B amino acid sequences, sequences substantially identical thereto, or a fragment comprising at least about 5, 10, 15, 20, 25, 30, 35, 40, 50, 75, 100, or 150 consecutive amino acids thereof using any of the programs described herein.

Another aspect of the invention is an assay for identifying fragments or variants of the Group B amino acid sequences, or sequences substantially identical thereto, which retain the enzymatic function of the polypeptides of the Group B amino acid sequences, and sequences substantially identical thereto. For example, the fragments or variants of the polypeptides, may be used to catalyze biochemical reactions, which indicate that said fragment or variant retains the enzymatic activity of the polypeptides in Group B amino acid sequences.

The assay for determining if fragments of variants retain the enzymatic activity of the polypeptides of the Group B amino acid sequences, and sequences substantially identical thereto includes the steps of: contacting the polypeptide fragment or variant with a substrate molecule under conditions which allow the polypeptide fragment or variant to function, and detecting either a decrease in the level of substrate or an increase in the level of the specific reaction product of the reaction between the polypeptide and substrate.

The polypeptides of the Group B amino acid sequences, sequences substantially identical thereto or fragments comprising at least 5, 10, 15, 20, 25, 30, 35, 40, 50, 75, 100, or 150 consecutive amino acids thereof may be used in a variety of applications. For example, the polypeptides or fragments thereof may be used to catalyze biochemical reactions. In accordance with one aspect of the invention, there is provided a process for utilizing a polypeptide of the Group B amino acid sequences, and sequences substantially identical thereto or polynucleotides encoding such polypeptides for hydrolyzing aminonitriles. In such procedures, a substance containing a haloalkane compound is contacted with one of the polypeptides of the Group B amino acid sequences, and sequences substantially identical thereto under conditions which facilitate the hydrolysis of the compound.

Antibodies - The polypeptides of Group B amino acid sequences, sequences substantially identical thereto or fragments comprising at least 5, 10, 15, 20, 25, 30, 35, 40, 50, 75, 100, or 150 consecutive amino acids thereof, may also be used to generate antibodies which bind specifically to the enzyme polypeptides or fragments. The resulting antibodies may be used in immunoaffinity chromatography procedures to isolate or purify the polypeptide or to determine whether the polypeptide is present in a biological sample. In such procedures, a protein preparation, such as an extract, or a biological sample is contacted with an antibody capable of specifically binding to one of the polypeptides of the Group B amino acid sequences, sequences substantially identical thereto, or fragments of the foregoing sequences.

In immunoaffinity procedures, the antibody is attached to a solid support, such as a bead or column matrix. The protein preparation is placed in contact with the antibody under conditions under which the antibody specifically binds to one of the polypeptides of the Group B amino acid sequences, sequences substantially identical thereto, or fragments thereof. After a wash to remove non-specifically bound proteins, the specifically bound polypeptides are eluted.

The ability of proteins in a biological sample to bind to the antibody may be determined using any of a variety of procedures familiar to those skilled in the art. For example, binding may be determined by labeling the antibody with a detectable label such as a fluorescent agent, an enzymatic label, or a radioisotope. Alternatively, binding of the antibody to the sample may be detected using a secondary antibody having such a detectable label thereon. Particular assays include ELISA assays, sandwich assays, radioimmunoassays, and Western Blots.

The antibodies of the invention can be attached to solid supports and used to immobilize nitrilases of the present invention. Such immobilized nitrilases can be used, as described above, in industrial chemical processes for the conversion of nitriles to a wide range of useful products and intermediates.

Polyclonal antibodies generated against the polypeptides of the Group B amino acid sequences, and sequences substantially identical thereto, or fragments comprising at least 5, 10, 15, 20, 25, 30, 35, 40, 50, 75, 100, or 150 consecutive amino acids thereof can be obtained by direct injection of the polypeptides into an animal or by administering the polypeptides to an animal. The antibody so obtained will then bind the polypeptide itself. In this manner, even a sequence encoding only a fragment of the polypeptide can be used to generate antibodies which may bind to the whole native polypeptide. Such antibodies can then be used to isolate the polypeptide from cells expressing that polypeptide.

For preparation of monoclonal antibodies, any technique which provides antibodies produced by continuous cell line cultures can be used. Examples include the hybridoma technique (Kohler and Milstein (1975), Nature, 256:495-497, the disclosure of which is incorporated herein by reference), the trioma technique, the human B-cell hybridoma technique (Kozbor et al. (1983), Immunology Today 4:72, the disclosure of which is incorporated herein by reference), and the EBV-hybridoma technique (Cole et al. (1985), in Monoclonal Antibodies and Cancer Therapy, Alan R. Liss, Inc., pp. 77-96, the disclosure of which is incorporated herein by reference in its entirety).

Techniques described for the production of single chain antibodies (U.S. Pat. No. 4,946,778, the disclosure of which is incorporated herein by reference in its entirety) can be adapted to produce single chain antibodies to the polypeptides of, for example, the Group B amino acid sequences, or fragments thereof. Alternatively, transgenic mice may be used to express humanized antibodies to these polypeptides or fragments.

Antibodies generated against a polypeptide of the Group B amino acid sequences, sequences substantially identical thereto, or fragments comprising at least 5, 10, 15, 20, 25, 30, 35, 40, 50, 75, 100, or 150 consecutive amino acids thereof may be used in screening for similar polypeptides from other organisms and samples. In such techniques, polypeptides from the organism are contacted with the antibody and those polypeptides which specifically bind to the antibody are detected. Any of the procedures described above may be used to detect antibody binding. One such screening assay is described in "Methods for Measuring Cellulase Activities", Methods in Enzymology, 160:87-116, which is hereby incorporated by reference in its entirety.

### Use of Whole Cells Comprising A Nucleic Acid

The invention provides for the use of whole cells which have been transformed with nucleic acid (or an active fragment thereof) encoding one or more of the nitrilases of the invention. The invention also provides for the use of such a whole cell in performing a nitrilase reaction on a substrate. Therefore, this invention provides for methods of hydrolyzing a cyanohydrin or aminonitrile linkage using a whole cell comprising at least one nucleic acid or polypeptide disclosed herein (SEQ ID NOS:1-386). For example, a whole cell which is stably transfected (the invention also encompasses transiently transfected or transformed whole cells) with a nucleic acid encoding a nitrilase is an embodiment of the invention. Such a cell is useful as a reagent in a reaction mixture to act on a substrate and exhibit nitrilase activity.

### Sequence Analysis Software

Percent identity or homology between two or more sequences is typically measured using sequence analysis software (*e*.*g*., Sequence Analysis Software Package of the Genetics Computer Group, University of Wisconsin Biotechnology Center, Madison, WI). Such software matches similar sequences by assigning a percent identity or homology to various deletions, substitutions and other modifications. The term "percent identity," in the context of two or more nucleic acids or polypeptide sequences, refers to the percentage of nucleotides or amino acid residues that are the same when compared after being aligned for maximum correspondence over a designated region or comparison "window." Under some algorithms, a conservative amino acid substitution can be considered "identical" and a change at a wobble site of a codon can be considered "identical."

"Alignment" refers to the process of lining up two or more sequences to achieve maximal correspondence for the purpose of assessing the degree of identity or homology, as defined within the context of the relevant alignment algorithm.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated for a particular algorithm. Default program parameters can be used, or alternative parameters can be designated. The sequence comparison algorithm then calculates the percent identity or homology for the test sequences relative to the reference sequence, based on the program parameters.

A "comparison window", as used herein, is a segment of the contiguous positions in a nucleic acid or an amino acid sequence consisting of from 20 to 600, usually about 50 to about 200, more usually about 100 to about 150 nucleotides or residues, which may be compared to a reference sequence of the same or different number of contiguous positions after the two sequences are optimally aligned. Methods of alignment of sequences for comparison are well-known in the art. Optimal alignment of sequences for comparison can be conducted, *e*.*g*., by the local homology algorithm of Smith and Waterman (1981), Adv. Appl. Math. 2:482, by the homology alignment algorithm of Needleman and Wunsch (1970), J Mol. Biol 48:443, by the search for similarity method of Pearson and Lipman (1988), Proc. Natl. Acad. Sci. USA 85:2444-2448, by computerized implementations of these algorithms, or by manual alignment and visual inspection. Other algorithms for determining homology or identity include, for example, the BLAST program (Basic Local Alignment Search Tool, National Center for Biological Information), BESTFIT, FASTA, and TFASTA (Wisconsin Genetics Software Package, Genetics Computer Group, Madison, WI), ALIGN, AMAS (Analysis of Multiply Aligned Sequences), AMPS (Alignment of Multiple Protein Sequence), ASSET (Aligned Segment Statistical Evaluation Tool), BANDS, BESTSCOR, BIOSCAN (Biological Sequence Comparative Analysis Node), BLIMPS (BLocks IMProved Searcher), Intervals and Points, BMB, CLUSTAL V, CLUSTAL W, CONSENSUS, LCONSENSUS, WCONSENSUS, Smith-Waterman algorithm, DARWIN, Las Vegas algorithm, FNAT (Forced Nucleotide Alignment Tool), Framealign, Framesearch, DYNAMIC, FILTER, FSAP (Fristensky Sequence Analysis Package), GAP (Global Alignment Program), GENAL, GIBBS, GenQuest, ISSC (Sensitive Sequence Comparison), LALIGN (Local Sequence Alignment), LCP (Local Content Program), MACAW (Multiple Alignment Construction and Analysis Workbench), MAP (Multiple Alignment Program), MBLKP, MBLKN, PIMA (Pattern-Induced Multi-sequence Alignment), SAGA (Sequence Alignment by Genetic Algorithm) and WHAT-IF. Such alignment programs can also be used to screen genome databases to identify polynucleotide sequences having substantially identical sequences. A number of genome databases are available, for example, a substantial portion of the human genome is available as part of the Human Genome Sequencing Project (J. Roach, http://weber.u.Washington.edu/∼roach/human_ genome_ progress 2.html) (Gibbs, 1995). At least twenty-one other genomes have already been sequenced, including, for example, *M*. *genitalium* (Fraser et al., 1995), *M. jannaschii* (Bult et al., 1996), *H*. *influenzae* (Fleischmann et al., 1995), *E*. *coli* (Blattner et al., 1997), and yeast (*S*. *cerevisiae*) (Mewes et al., 1997), and *D*. *melanogaster* (Adams et al., 2000). Significant progress has also been made in sequencing the genomes of model organism, such as mouse, *C*. *elegans*, and *Arabadopsis sp*. Several databases containing genomic information annotated with some functional information are maintained by different organizations, and are accessible via the internet, for example, http://wwwtigr.org/tdb; http://www.genetics.wisc.edu; http://genome-www.stanford.edu/∼ball; http://hiv-web.lanl.gov; http://www.ncbi.nlm.nih.gov; http://www.ebi.ac.uk; http://Pasteur.fr/other/biology; and http://www.genome.wi.mit.edu.

Examples of useful algorithms are the BLAST and the BLAST 2.0 algorithms, which are described in Altschul et al. (1977), Nuc. Acids Res. 25:3389-3402, and Altschul et al. (1990), J. Mol. Biol. 215:403-410, respectively. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra*). These initial neighborhood word hits act as seeds for initiating searches to find longer HSPs containing them. The word hits are extended in both directions along each sequence for as far as the cumulative alignment score can be increased. Cumulative scores are calculated using the parameter M (reward score for a pair of matching residues; always >0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. For nucleotide sequences, the BLASTN program uses as defaults a wordlength (W) of 11, an expectation (E) of 10, M=5, N=-4 and a comparison of both strands. For amino acid sequences, the BLASTP program uses as defaults a wordlength (W) of 3, an expectation (E) of 10, and the BLOSUM62 scoring matrix (see Henikoff and Henikoff (1989), Proc. Natl. Acad. Sci. USA 89:10915).

The BLAST algorithm also performs a statistical analysis of the similarity between two sequences (see, *e*.*g*., Karlin and Altschul (1993), Proc. Natl. Acad. Sci. USA 90:5873). One measure of similarity provided by BLAST algorithm is the smallest sum probability (P(N)), which provides an indication of the probability by which a match between two nucleotide or amino acid sequences would occur by chance. For example, a nucleic acid is considered similar to a references sequence if the smallest sum probability in a comparison of the test nucleic acid to the reference nucleic acid is less than about 0.2, less than about 0.01, or less than about 0.001.

In one embodiment, protein and nucleic acid sequence homologies are evaluated using the Basic Local Alignment Search Tool ("BLAST"). In particular, five specific BLAST programs are used to perform the following task:
(1) BLASTP and BLAST3 compare an amino acid query sequence against a protein sequence database;
(2) BLASTN compares a nucleotide query sequence against a nucleotide sequence database;
(3) BLASTX compares the six-frame conceptual translation products of a query nucleotide sequence (both strands) against a protein sequence database;
(4) TBLASTN compares a query protein sequence against a nucleotide sequence database translated in all six reading frames (both strands); and
(5) TBLASTX compares the six-frame translations of a nucleotide query sequence against the six-frame translations of a nucleotide sequence database.

The BLAST programs identify homologous sequences by identifying similar segments, which are referred to herein as "high-scoring segment pairs," between a query amino or nucleic acid sequence and a test sequence which may be obtained from a protein or nucleic acid sequence database. High-scoring segment pairs are identified (*i*.*e*., aligned) by means of a scoring matrix, many of which are known in the art. In one example, the scoring matrix used is the BLOSUM62 matrix (Gonnet et al. (1992), Science 256:1443-1445; Henikoff and Henikoff (1993), Proteins 17:49-61). In another example, the PAM or PAM250 matrices may also be used (see, *e*.*g*., Schwartz and Dayhoff, eds. (1978), Matrices for Detecting Distance Relationships: Atlas of Protein Sequence and Structure, Washington: National Biomedical Research Foundation). BLAST programs are accessible through the U.S. National Library of Medicine, *e*.*g*., at www.ncbi.nlm.nih.gov.

The parameters used with the above algorithms may be adapted depending on the sequence length and degree of homology studied. In some embodiments, the parameters may be the default parameters used by the algorithms in the absence of instructions from the user.

In a particular embodiment, the invention provides a method for modifying small molecules, comprising contacting a polypeptide encoded by a polynucleotide described herein or enzymatically active fragments thereof with a small molecule to produce a modified small molecule. A library of modified small molecules is tested to determine if a modified small molecule is present within the library which exhibits a desired activity. A specific biocatalytic reaction which produces the modified small molecule of desired activity is identified by systematically eliminating each of the biocatalytic reactions used to produce a portion of the library, and then testing the small molecules produced in the portion of the library for the presence or absence of the modified small molecule with the desired activity. The specific biocatalytic reactions, which produce the modified small molecule of, desired activity is optionally repeated. The biocatalytic reactions are conducted with a group of biocatalysts that react with distinct structural moieties found within the structure of a small molecule, each biocatalyst is specific for one structural moiety or a group of related structural moieties; and each biocatalyst reacts with many different small molecules which contain the distinct structural moiety.

Some embodiments of the use of the nitrilases are:

α-hydroxy acid - Nitrilases produce α-hydroxy acids through hydrolysis of cyanohydrins. Production of mandelic acid and derivatives thereof is an example of this. A significant application of this type involves commercial production of *(R)-*mandelic acid in both high yield and high enantioselectivity from mandelonitrile. Mandelic acid and derivatives have found broad application as intermediates and resolving agents for the production of many chiral pharmaceutical and agricultural products. Previous attempts to employ the few known nitrilases in processes using analogous substrates have been plagued by significantly lower activity, productivity, and selectivity.

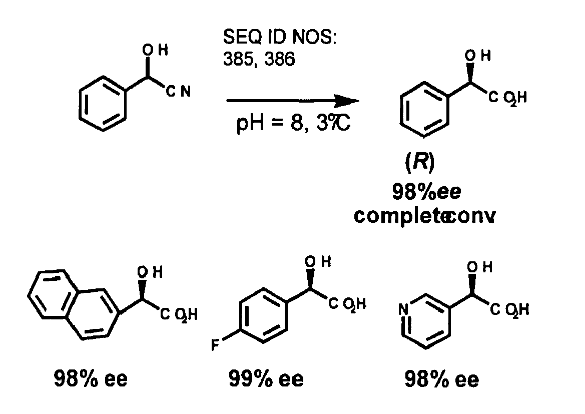

### Phenyllactic acid derivatives

An additional application is in the production of *(S)*-phenyl lactic acid derivatives in both high yield and high enantioselectivity. Phenyl lactic acid derivatives have found broad application in the production of many chiral pharmaceutical and agricultural products.

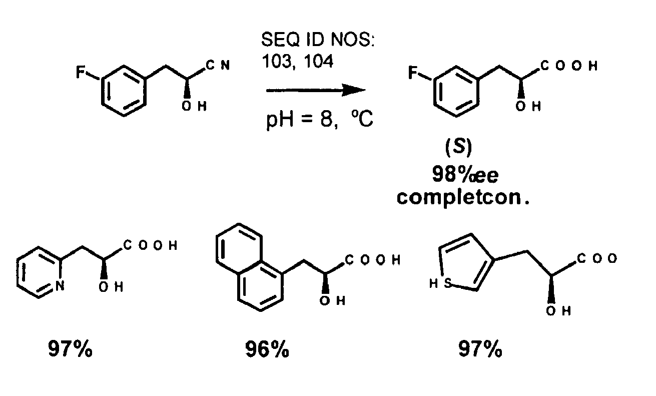

### β-hydroxy acid

With important commercial considerations, nitrilases are provided produce either enantiomer of 4-cyano-3-hydroxybutyric acid, the *(R)*-enanatiomer of which is a key intermediate in the synthesis of the blockbuster drug LIPITOR™. 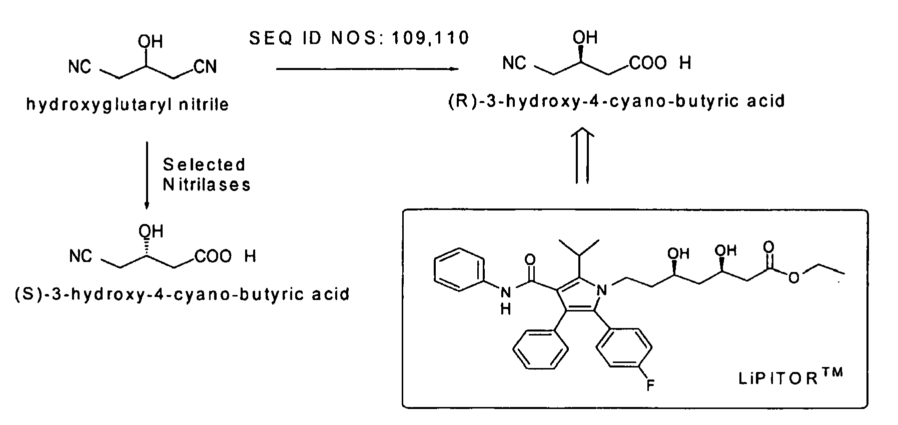 The following nitrilases are more examples of nitrilases useful in converting hydroxyglutarylnitrile to (R)-3-hydroxy-4-cyano-butyric acid: SEQ ID NOS:205, 206, SEQ ID NOS:207, 208, SEQ ID NOS:195, 196, SEQ ID NOS:43, 44, SEQ ID NOS:321, 322, and SEQ ID NOS:237, 238. The above schematic indicates that "selected nitrilases" can be used to convert hydroxyglutarylnitrile to (S)-3-hydroxy-4-cyano-butyric acid: SEQ ID NOS:107, 108, SEQ ID NOS:109, 110, SEQ ID NOS:111, 112, SEQ ID NOS:127, 128, SEQ ID NOS:129, 130, SEQ ID NOS:133, 134, SEQ ID NOS:113, 114, SEQ ID NOS:145, 146, SEQ ID NOS:101, 102, SEQ ID NOS:179, 180, SEQ ID NOS:201, 202, SEQ ID NOS:159, 160, SEQ ID NOS:177, 178, SEQ ID NOS:181, 182, SEQ ID NOS:183, 184, SEQ ID NOS:185, 186, SEQ ID NOS:57, 58, SEQ ID NOS:197, 198, SEQ ID NOS:59, 60, SEQ ID NOS:67, 68, and SEQ ID NOS:359, 360.

The invention will be further described with reference to the following examples; however, it is to be understood that the invention is not limited to such examples. Rather, in view of the present disclosure which describes the current best mode for practicing the invention, many modifications and variations would present themselves to those of skill in the art without departing from the scope and spirit of this invention. All changes, modifications, and variations coming within the meaning and range of equivalency of the claims are to be considered within their scope.

### Example 1

### Phagemid infections

For each library to be screened for nitrilases, an infection was set up as follows: 5ml of an OD₆₀₀nm=1 resuspension of SEL700 cells and 1ml of the phagemid library to be screened were combined. The combination was incubated in a 37°C waterbath for 45 min.

Using the infection, serial dilutions were made in 10mM MgSO₄, using 10µl aliquots of the infection.

| titer of library | dilutions to make |
|---|---|
| ∼10⁵ cfu/ml | 10⁻¹ dilution |
| ∼10⁶ cfu/ml | 10⁻¹, 10⁻² dilution |
| ∼10⁷ cfu/ml | 10⁻¹, 10⁻², 10⁻³ dilution |

60µl of each of the following dilutions were deposited onto a small LB-kan⁵⁰ plate:

| titer of library | dilutions to make |
|---|---|
| ∼10⁵ cfu/ml | undiluted infection, 10⁻¹ dilution |
| ∼10⁶ cfu/ml | 10⁻¹, 10⁻² dilutions |
| ∼10⁷ cfu/ml | 10⁻², 10⁻³ dilutions |

The cells in the infection were centrifuged in a tabletop centrifuge at 4°C, 4.6k rpm, 10 min to form pellets. The supernatant was decanted from the resulting pellets. The cells were resuspended in residual liquid. All of the resuspended cells were deposited onto a single large LB-kar⁵⁰ plate. All plates were incubated at 30°C overnight.

### Example 2

### Selection Screenings

The cells of each infection plate were resuspended with ∼4mls 10mM MgSO₄. The resuspensions were placed in a tube. The remaining cells on each plate were resuspended with ∼3mls 10mM MgSO₄ and combined with the first resuspension from the same plate. The volume of each tube was brought to 12ml with 10mM MgSO₄, The tubes were vortexed vigorously. The tubes were centrifuged in a tabletop centrifuge at 4°C and 4.6k for 10min to form pellets. The supernatant was decanted from each resuspension. The washed cells in each tube were resuspended with 10ml 10mM MgSO₄. The resuspensions from each library were stored at 4°C until the selection cultures were ready to be set up.

For each resuspension, selection cultures were set up using the following process:
1) The nitrilase selection medium was prepared, using: 1XM9 medium with 0.2% glucose, no nitrogen and 50µg/ml kanamycin (for pBK phagemid libraries only; use ampicillin for pBS libraries).
2) 5ml of the medium was aliquoted into a 50ml screw top conical tube.
3) 25µl of the stored resuspension was added to the tube.
4) 5µl of adiponitrile was added to the tube, to bring the final concentration to 8.8mM. Additional nitrile substrates may be used, in place of adiponitrile.
5) The resulting combination was cultured at 30°C.

Steps 1-5 were repeated for each nitrile substrate.

### Example 3

### Isolation of a positive nitrilase clone from selection cultures

Ten (10) µl of selection culture with growth was streaked out onto a small LB-kan⁵⁰ plate and allowed to grow for 2 nights at 30°C. Five isolated cfu were picked and each was grown in 2ml nitrilase selection medium at 30°C. Each culture was monitored (where growth indicates positive cfu was picked), and was removed when monitoring indicated that it was in a stationary phase of growth. One (1) ml of culture was used to do a plasmid preparation and was eluted with 40µl elution buffer. Five to eight (5-8) µl DNA was cut with Pst I/Xho I or Sac I/Kpn 1 restriction enzymes to remove insert from vector. A restriction fragment length polymorphism (RFLP) determination was carried out to identify the size of the insert. The insert was sequenced.

### Example 4

### Screening and Characterization of Nitrilases

Nitrilases of the invention were screened against target substrates. Of those showing hydrolytic activity in a primary screen, enzymes with enantioselectivities above 20% enantiomeric excess (*ee*) were selected for further characterization. Those enzymes were selected based on: 1) having activity against one of the substrates of interest and 2) exhibition of greater than 35% *ee* (enantiomeric excess). The results of this screening process are set forth in Table 1 above. The products used for screening were: D-Phenylglycine, L-Phenyllactic acid, *(R)* 2-chloromandelic acid, (*S*)-Cyclohexylmandelic acid, L-2-methylphenylglycine, (S)-2-amino-6-hydroxy hexanoic acid, and 4-methyl-L-leucine.

### Screening of nitrilases against target substrate D-Phenylglycine

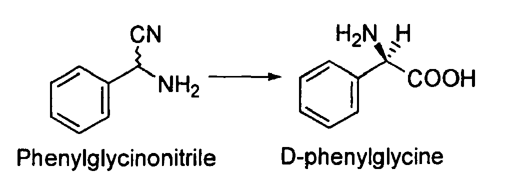

The hydrolysis of phenylglycinonitrile was performed. Some of these enzymes showed an *ee* higher than 20% and those were selected for preliminary characterization.

Based on the preliminary characterization experiments, a number of putative hits were identified on phenylglycinonitrile and a large amount of data was accumulated on these enzymes. The data revealed many common properties: the majority of the enzymes had pH optima for activity at pH 7 and, in general, the enantioselectivity was enhanced at the lower pH values. The enzymes were found to be more active at higher temperature, particularly 38°C, although this temperature often resulted in lower enantioselectivities. The use of water-miscible co-solvents in the reaction was shown to be a practical option. The inclusion of 10-25% methanol (v/v) in the enzyme reactions did not substantially affect enzyme activity and in many cases, led to an increase in enantioselectivity. The use of biphasic systems has also shown some promise, with the enzymes maintaining their level of activity with the addition of up to 70% (v/v) of hexane and, in some cases, toluene. The use of ethyl acetate in the biphasic systems, however, led to lower activity.

Of the enzymes identified active on phenylglycinonitrile, the enantioselectivity of several enzymes was shown to remain above the success criterion of 35% *ee.* The preliminary characterization data indicated that some of the enzymes exhibited high enantioselectivities for D-phenylglycine, with corresponding conversion to product of 40-60%. Further investigation suggested that the rate of activity of some of these enzymes was faster than the rate of racemization of the substrate. Reducing the concentration of enzyme led to improved enantioselectivity; therefore, it appears that some benefit could be gained by control of the relative rates of the chemical racemization and the enzyme activity.

### Screening of nitrilases against target substrate (R)-2-chloromandelic acid

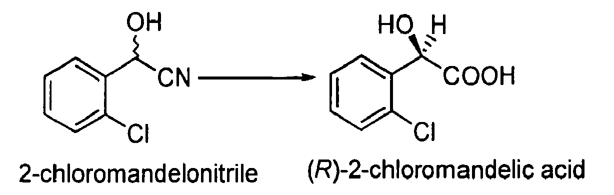

Enzymes were identified which showed activity on 2-chloromandelonitrile. A high degree of overlap existed between the enzymes which were active on 2-chloromandelonitrile and phenylglycinonitrile. Many of these enzymes also formed a distinct sequence family.

Higher temperatures and neutral pH appeared to lead to the highest activity for the active enzymes. For the majority of the nitrilases, the enantioselectivity also increased at higher temperatures, particularly 38°C. The enzymes retained their activity in the presence of up to 25% methanol or 10% isopropanol; in many of these cases, the enantioselectivity was also enhanced. Activity in biphasic systems was largely comparable to aqueous conditions, particularly with hexane as the non-aqueous phase; varying tolerances to toluene were observed between the different nitrilases.

**Table 4. Examples of enzymes for enantioselective hydrolysis of 2-chloromandelonitrile.**

| **SEQ ID NOS:** | **Conversion To Product** | **Time For Highest Conversion (h)** | **ee (%)** | **Turnover (g product/kg Nitrilase/h)** | **Specific Activity (µmol Product/mg Nitrilase/h)** | **Conditions** |
|---|---|---|---|---|---|---|
| 385,386 | 22% | 8 | 92 | 1014 | 88 | pH 7, 38°C; 20% MeOH |
| 169, 170 | 36% | 1 | 92 | 29278 | 39 | pH 7, 38°C; 20% MeOH |
| 185, 186 | 44% | 1 | 79 | 14559 | 99 | pH 7, 38°C |
| 47,48 | 43% | 5 | 65 | 2475 | 20 | pH 7, 38°C; 10% MeOH |
| 197, 198 | >95% | 1 | 87 | 45564 | 149 | pH 6, 38°C; 10% MeOH |
| 187, 188 | 53% | 1 | 82 | 14100 | 80 | pH 7, 38°C; 10% MeOH |
| 217, 218 | >95% | 2 | 85 | 19773 | 185 | pH 7, 38°C; 10% MeOH |
| 55, 56 | >95% | 1 | 93 | 52951 | 329 | pH 8, 38°C; 10% MeOH |
| 167, 168 | >95% | 2 | 88 | 14895 | 100 | pH 8, 38°C; 10% MeOH |
| 15, 16 | 55% | 1 | 81 | 25825 | 308 | pH 5, 38°C; 10% MeOH |

**Table 4A. Summary of optimal conditions determined from characterization experiments for enantioselective hydrolysis of 2-chloromandelonitrile.**

| **SEQ ID NOS:** | **Optimum pH** | **Optimum Temp** °**C** | **Solvent Tolerance** |
|---|---|---|---|
| 385,386 | 7 | 38 | 25% MeOH |
| 169, 170 | 5 | 38 | 25% MeOH, 10% IPA |
| 185, 186 | 7 | 38 | 25% MeOH, 10% IPA |
| 47,48 | 7 | 38 | 10% MeOH |
| 197, 198 | 6 | 55 | 25% MeOH, 10% IPA |
| 187, 188 | 7 | 38 | 10% MeOH; 40% IPA |
| 217, 218 | 7 | 38 | 25% MeOH, 10% IPA, 70% hexane, 40% tolucne |
| 55, 56 | 7 | 38 | 10% MeOH, IPA, 70% hexane |
| 167, 168 | 9 | 38 | 10% MeOH, IPA, 70% hexane |
| 15, 16 | 7 | 38 | 25% MeOH, 10% IPA, 70% hexane, 40% toluene |

### Screening of nitrilases against target substrate (S)-phenyllactic acid:

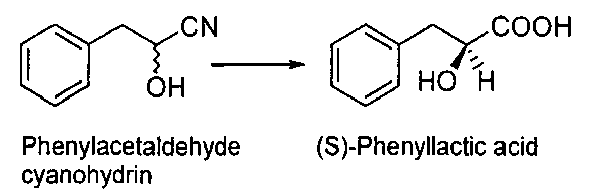

Many of the nitrilases tested were active on phenaylacetaldehyde cyanohydrin. Many of these enzymes were part of two related sequence families and were distinct from those enzymes that were active on phenylglycinonitrile and chloromandelonitrile.

The pH optima of the enzymes was generally above pH 7 (*i.e.* pH 8 or 9), with higher enantioselectivities being exhibited at these levels. Most of the enzymes showed superior activity at higher temperature, particularly 38°C. The effect of temperature on the enantioselectivities of the enzymes varied; in most cases, this property was slightly lower at higher temperatures. While the enzymes were tolerant towards the addition of co-solvents, particularly 10% (v/v) methanol, no advantage in activity or enantioselectivity was gained by such additions. The use of a biphasic system was again shown to be feasible.

**Table 5. Summary of optimal conditions determined from characterization experiments for enantioselective hydrolysis of phenylacetaldehyde cyanohydrin**

| **SEQ ID NOS:** | **Optimum pH** | **Optimum Temp °C** | **Solvent Tolerance** |
|---|---|---|---|
| 103, 104 | 7 | 55 | 10% MeOH, IPA |
| 99, 100 | 8 | 38 | 10% MeOH, 70% hexane, toluene |
| 183, 184 | 9 | 38 | 10% MeOH, IPA, 70% toluene, hexane |
| 173, 174 | 5 | 38-55 | 25% MeOH, IPA, 70% hexane, toluene |
| 213, 214 | 7 | 38 | 10% MeOH, 25% IPA, 70% hexane, toluene |
| 61, 62 | 7 | 38 | 10% MeOH, 70% hexane, toluene |
| 205, 206 | 8 | 38-55 | 10% MeOH, IPA, 40% hexane, toluene |
| 207,208 | 8 | 38 | 10% MeOH, 70% hexane |
| 309, 210 | 8 | 38 | 10% MeOH, 40% hexane, toluene |
| 195, 196 | 8 | 38 | 10% MeOH, 40% hexane, toluene |
| 43,44 | 9 | 38 | 10% MeOH, 40% hexane |
| 161, 162 | 9 | 38 | 25% MeOH, IPA, 10% hexane, toluene |
| 175, 176 | 6 | 38-55 | 10% MeOH, I PA, 40% hexane |
| 293,294 | 6 | 38 | 10% MeOH, IPA, 40% hexane |

**Table 6. Summary of hit enzymes for enantioselective hydrolysis of phenylacetaldehyde cyanohydrin**

| **SEQ ID NOS:** | **Conversion To Product (%)** | **Time For Highest Conversion (h)** | **ee (%)** | **Turnover (g Product/kg Nitrilase/h)** | **Specific Activity (**µ**mol Product/mg Nitrilase/h)** | **Experimental Conditions¹** |
|---|---|---|---|---|---|---|
| 103, 104 | 77 | 3 | 94 | 2339* | 14* | pH 7, 38 °C |
| 99, 100 | 90 | 4 | 82 | 19787 | 119 | pH 8, 38°C |
| 183, 184 | 49 | 1 | 57 | 28153 | 169 | pH 9, 38°C |
| 173, 174 | 20 | 2 | 83 | 3200 | 27 | pH 5, 38 °C |
| 213, 214 | 70 | 6 | 86 | 4372 | 26 | pH 7, 38°C |
| 61, 62 | 56 | 5 | 80 | 5500 | 33 | pH 7, 38°C |
| 205, 206 | 90 | 6 | 73 | 4458 | 27 | pH 8, 38°C |
| 207,208 | 78 | 4 | 81 | 9190 | 55 | pH 8, 38°C |
| 209, 210 | 98 | 4 | 75 | 8343 | 50 | pH 8, 38°C |
| 195, 196 | 89 | 4 | 89 | 6874 | 41 | pH 8, 38°C |
| 43, 44 | 40 | 5 | 84 | 3879 | 23 | pH 9, 38°C |
| 161, 162 | >95 | 2 | 39 | 16430 | 98 | pH 9, 38°C |
| 175, 176 | 87 | 8 | 45 | 5065 | 30 | pH 6, 38°C |
| 293, 294 | >95 | 8 | 65 | 7725 | 46 | pH 6, 38°C |

| | | | | | | |
|---|---|---|---|---|---|---|
| ¹. Experiments were carried out using 25 mM substrate, at the pH and temperature conditions noted. ^{*}. Turnover in (g product/kg protein/h) and specific activity (µmol product/mg protein/h) | | | | | | |

### Screening of nitrilases against target substrate L-2-methylphenylglycine

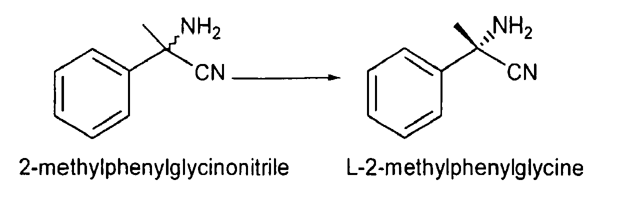

Nitrilases have shown activity on this substrate and preferentially yielded the D-2-methylphenylglycine, rather than the required L-2-methylphenylglcyine.

**Table 7. Summary of activity and enantioselectivity for SEQ ID NOS:189, 190 on 2-methylphenylglycinonitrile**

| **SEQ ID NOS:** | **Conversion To Product (%)** | **Time For Highest Conversion (h)** | **ee (%) for D-enatiomer** | **Turnover (g Product/kg Nitrilase/h)** | **Specific Activity (µmol Product/mg Nitrilase/h)** | **Experimental Conditions¹** |
|---|---|---|---|---|---|---|
| 189, 190 | 50% | 4.5 h | 45 | 2426 | 13 | pH 7 38°C, 10% MeOH |

### Screening of nitrilases against target substrate L-hydroxynorleucine ((S)-2-amino-6-hydroxy hexanoic acid)

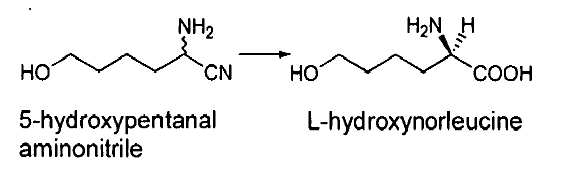

A number of nitrilases, which showed activity on 2-amino-6-hydroxy hexanenitrile, were isolated. All of these enzymes showed enantioselectivity towards the L-isomer of the product.

As shown in Table 8, the enzymes all showed higher enantioselectivities at higher pH and appeared to more susceptible to the addition of solvents than the other nitrilases tested. Although activity was detected in the presence of organic solvents, it was generally lower than that of the aqueous control. Once again, the activity of the enzymes was negatively affected by the acid product and aldehyde starting material.

**Table 8. Summary of optimal conditions determined from characterization experiments for enantioselective hydrolysis of 2-amino-6-hydroxy hexanenitrile.**

| **SEQ ID NOS:** | **Optimum pH** | **Optimum Temp °C** | **Solvent** |
|---|---|---|---|
| 217, 218 | 9 | 38 | 10% MeOH |
| 55, 56 | 9 | 38 | None |
| 187, 188 | 9 | 38 | 10% MeOH |
| 167, 168 | 9 | 38 | None |
| 221, 222 | 9 | 38 | |

A range of hydrolytic activities was observed among the confirmed hit enzymes for 2-amino-6-hydroxy hexanenitrile.

**Table 9. Summary of hit enzymes for enantioselective hydrolysis of 2-amino-6-hydroxy hexanenitrile.**

| **SEQ ID NOS:** | **Conversion To Product (%)** | **Time For Highest Conversion (h)** | **ee (%)** | **Turnover (g Product/kg Nitrilase/h)** | **Specific Activity (**µ**mol Product/mg Nitrilase/h))** | **Experimental Conditions¹** |
|---|---|---|---|---|---|---|
| 217, 218 | >95 | 1.5 | 52 | 33712 | 229 | pH 9, 38 °C |
| 55, 56 | 80 | 3 | 55 | 11221 | 76 | pH 9, 38 °C |
| 187, 188 | 50 | 6 | 60 | 1238 | 4 | pH 9, 38 °C, 10% MeOH |
| 167, 168 | 35 | 6 | 54 | 1684 | 11 | pH 9, 38 °C |
| 221, 222 | 80 | 3 | 55 | 9901 | 148 | pH 9, 38 °C |

### Screening of nitrilases against target substrate 4-methyl-D-leucine and 4-methyl-L-leucine

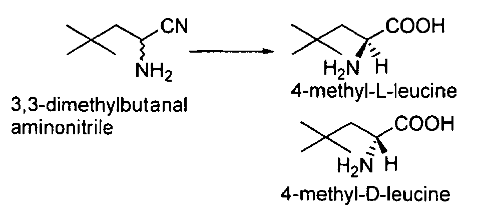

Hydrolysis of 2-amino-4,4-dimethyl pentanenitrile was performed by several of the nitrilases. Of these, some were shown to hydrolyse the nitrile to the L-isomer of the corresponding acid and were selected for further characterization.

**Table 10. Summary of hit enzymes for enantioselective hydrolysis of 2-amino-4,4-dimethyl pentanenitrile**

| **SEQ ID NOS:** | **Conversion To Product (%)** | **Time For Highest Conversion (h)** | **ee(%)** | **Turnover (g Product/kg Nitrilase/h)** | **Specific Activity (µmol Product/mg Nitrilase/h)** | **Conditions¹** |
|---|---|---|---|---|---|---|
| 103, 104 | 30 | 0.5 | 91 | 12489 | 36 | pH 7, room temp |
| 59, 60 | 30 | 0.5 | >99 | 33806 | 233 | pH 7, room temp |
| 221, 222 | 32 | 7.5 | 79 | 1098 | 7 | pH 6, 38°C |

**Table 11. Summary of optimal conditions determined from characterization experiments for enantioselective hydrolysis of 2-amino-4,4-dimethyl pentanenitrile**

| **NOS:** | **SEQ ID Optimum pH** | **Optimum Temp °C** | **Solvent Tolerance** |
|---|---|---|---|
| 103, 104 | 7 | 23 | 25% MeOH, 10% IPA |
| 59, 60 | 8 | 23 | 25% MeOH |
| 221, 222 | 6 | 38 | 25% MeOH, 10% IPA |

### Screening of nitrilases against target substrate (S)-cyclohexylmandelic acid

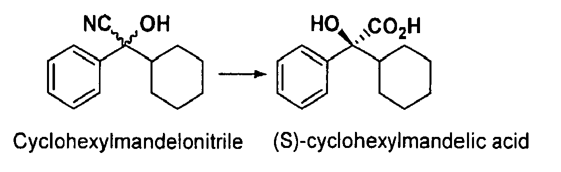

### Screening of nitrilases against target substrate Mandelonitrile

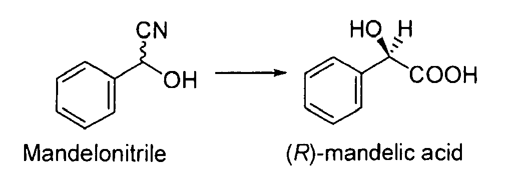

The nitrilase collection was also screened on mandelonitrile. The nitrilases actively hydrolyzed both phenylglycinonitrile and chloromandelonitrile.

### Enzymatic assay for determination of enantioselectivity

In the design of a spectroscopic system for determination of the chiral α-hydroxy acids and α-amino acids, an enzyme based assay which permits the detection of product formation and enantioselectivity was developed and used.

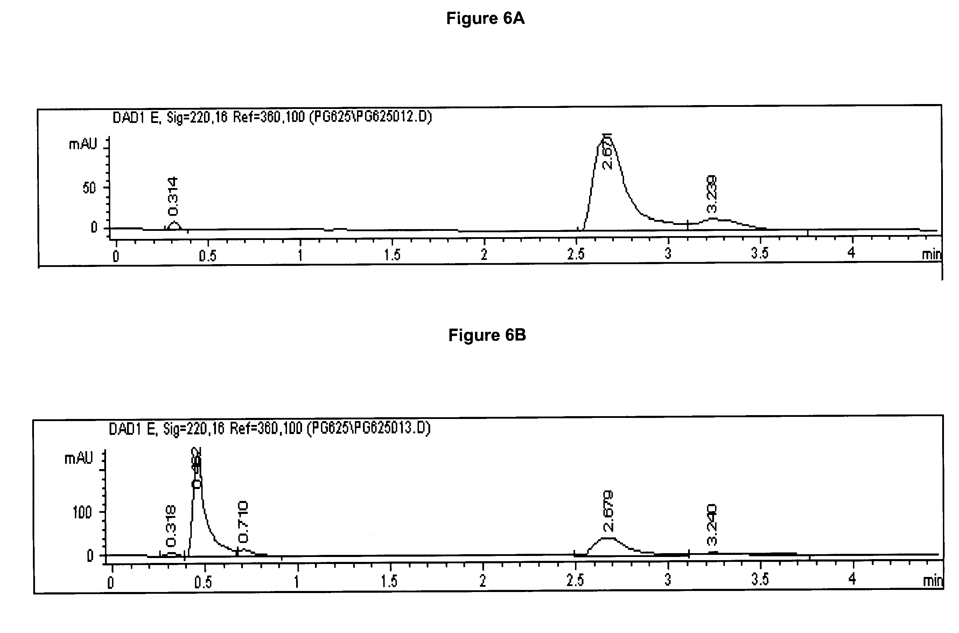

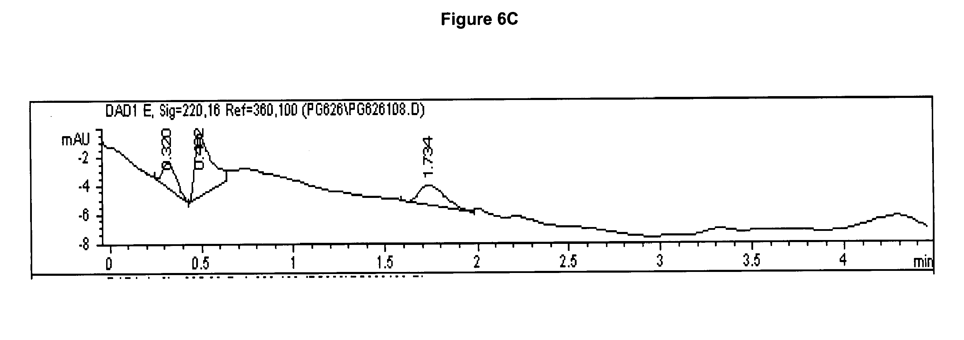

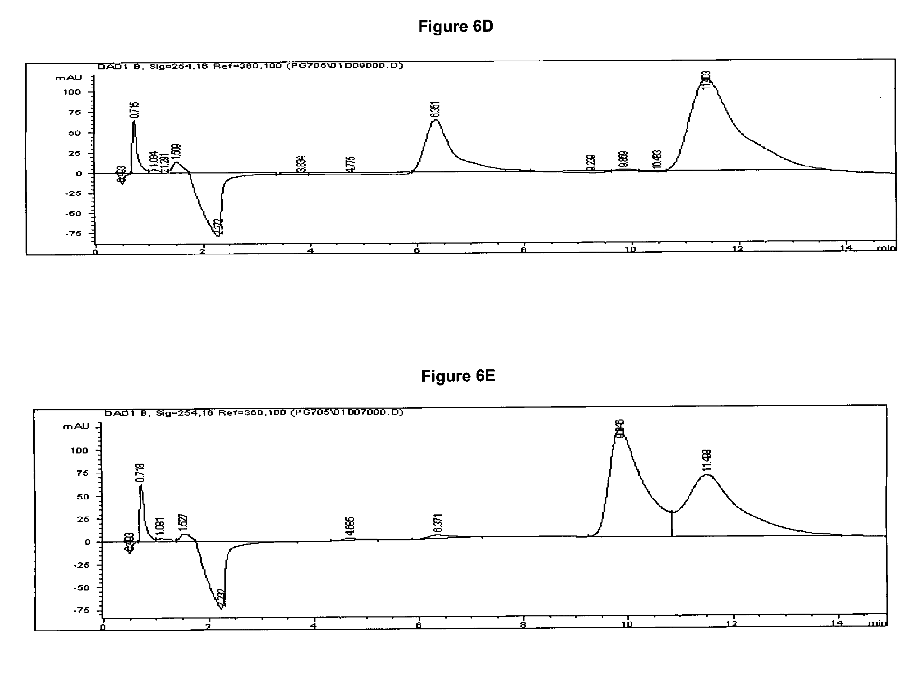

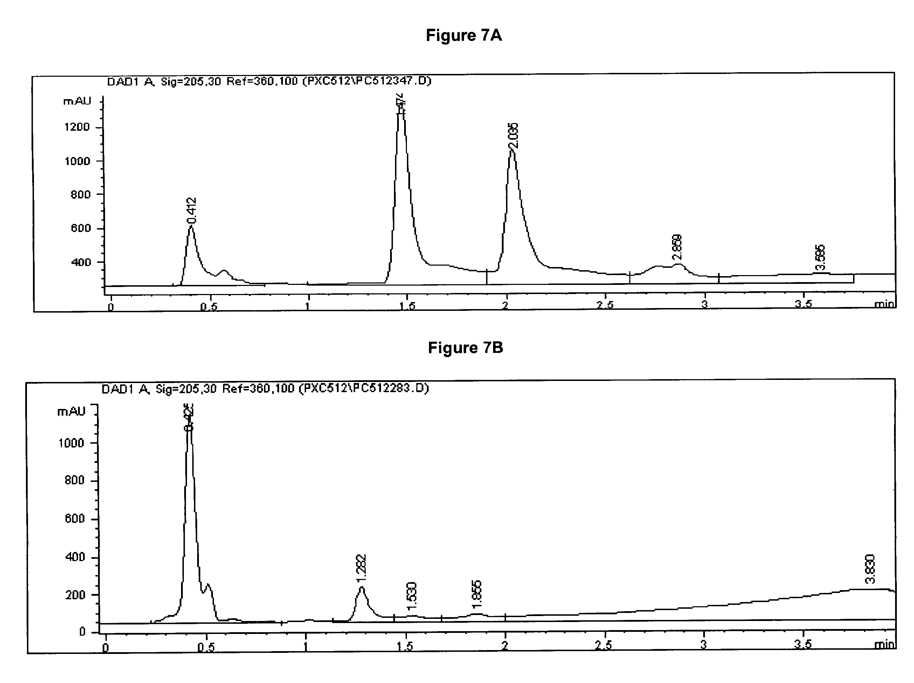

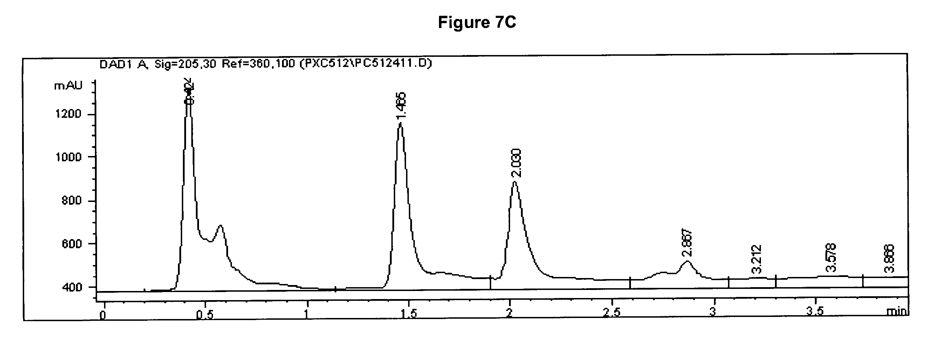

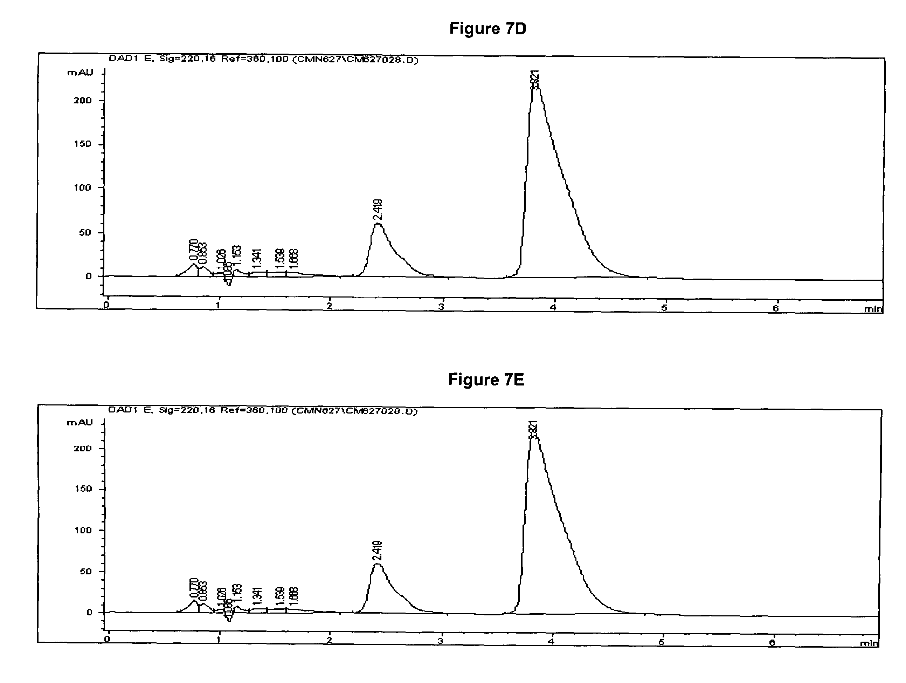

Spectroscopic systems for the detection of α-hydroxy- and for α-amino- acids based on lactate dehydrogenase (L-LDH & D-LDH) and on amino acid oxidase (L-AA Oxid & D-AA Oxid) are described in Figures 6 and 7. These enzymes were chosen because they are reported to have reasonably broad substrate ranges while still retaining near absolute enantiospecificity.

The overall feasibility of this system has been established (Table 12). Neither the parent hydroxynitrile nor the aminonitrile is metabolized by the secondary or detection enzyme and thus starting material does not interfere. Cell lysate which is not heat treated results in background activity for the LDH system; however, heat inactivation eliminates the background activity. Cell lysate does not appear to interfere in the AA Oxidase assay. One concern is the inactivation of the AA Oxidase, which utilizes a FMN co-factor, by residual cyanide. However, the control studies indicated that at 2 mM PGN (which could release up to 2 mM HCN) inactivation is not a problem. This assay is suitable for automation of 384 well (or possibly greater density) microtiter plates.

**Table 12: Summary of Identification of Secondary Enzyme to Chiral Detection of Acid Product.**

| **SUBSTRATE** | **ENZYME WITH SUITABLE ACTIVITY FOUND FROM COMMERCIAL SOURCE** |
|---|---|
| **Hydroxy Acid Products:** | |
| L-lactic acid | YES |
| D-lactic acid | YES |
| L-phenyl lactic acid | YES |
| D-phenyl lactic acid | YES |
| S-cyclohexylmandelic acid¹ | *Not applicable* |
| R-cyclohexylmandelic acid¹ | *Not applicable* |

| **Amino Acid Products:** | |
|---|---|
| 4-methyl-L-leucine | YES |
| 4-methyl-L/D-leucine | YES (D-unknown) |
| D-phenylalanine | YES |
| R-phenylglycine | YES |
| L-homophenyllactic acid | YES |
| D-homophenyllactic acid | YES |
| L-homophenylalanine | YES |
| D-homophenylalaninc | YES |
| (S)-2-amino-6-hydroxy hexanoic acid | YES |
| (R/S)-2-amino-6-hydroxy hexanoic acid | YES (D-unknown) |
| L-methylphenylglycine¹ | 1. ***Not Applicable*** |
| D-methylphenylglycine¹ | *Not Applicable* |

| | |
|---|---|
| ¹: The assay will not be applicable to cyclohexylmandelic acid and 2-methylphenylglycine, as tertiary alcohols are not amenable to this particular oxidation | |

### Example 5

### Standard assay conditions

The following solutions were prepared:
- Substrate stock solution: 50 mM of the aminonitrile substrate in 0.1 M phosphate buffer (pH 7) or 50 mM of the cyanohydrin substrate in 0.1 M Na Acetate buffer (pH 5)
- Enzyme stock solution: 3.33 ml of 0.1 M phosphate buffer (pH 7) to each vial of 20 mg of lyophilized cell lysate (final concentration 6 mg protein/ml)

Procedure:
- Add 100 µl of the 50 mM substrate solution to the appropriate number of wells of a 96-well plate
- Add 80 µl of buffer to each well
- Add 20 µl of enzyme solution to each well
- Blank controls were set up by substitution of 20 µl of buffer for the enzyme solution
- Negative controls consisting of 20 µl of enzyme solution in 180 µl of buffer were also included in many of the experiments. Once it had been established that the cell lysate did not interfere with the detection of the products, these controls were not included.

Sampling of reactions:
- The reactions were sampled by removing an aliquot from each well (15-50 µl) and diluting the samples as follows:
- Samples for non-chiral HPLC analysis:
- Phenylglycine, 2-chloromandelic acid and phenyllactic acid: initially, the samples were diluted 2-fold with water and a further 2-fold with methanol or acetonitrile (final dilution: 4-fold). It was found that an 8-fold dilution of these samples led to improved chromatographic separation
- (S)-2-amino-6-hydroxy hexanoic acid, 4-methylleucine, *t*-leucine, 2-methylphenylglycine and cyclohexylmandelic acid: samples were diluted 1:1 with methanol or acetonitrile. The choice of solvent was based on the solvent used in the HPLC analysis method.
- Samples for chiral HPLC analysis:
- Phenylglycine, 2-chloromandelic acid and phenyllactic acid: as described above for the non-chiral analyses, the samples for chiral analyses were initially diluted 2-fold and in the later stages of the project, at 4-fold.
- (S)-2-amino-6-hydroxy hexanoic acid, 4-methylleucine, *t*-leucine, 2-methylphenylglycine: samples were diluted 1:1 with methanol or acetonitrile
- For each experiment, a standard curve of the product was included in the HPLC run. The curve was plotted on an X-Y axis and the concentration of product in the samples calculated from the slope of these curves.
- For the preliminary characterization experiments, samples were taken such that the activity of the enzymes was in the linear phase; this was performed so that differences in the effects of the parameters on the rate of reaction, rather than the complete conversion, could be determined. The sampling times are denoted in the tables included in the text.
- The samples were analyzed by HPLC, using the methods outlined in Table 20 and 21.

### Example 6

### Determination of the Effect of pH on enzyme activity and enantioselectivity

The effect of pH on the enzyme activity and enantioselectivity was studied by performance of the standard assay in a range of different buffers:
0.1 M Citrate Phosphate pH 5
0.1 M Citrate Phosphate pH 6
0.1 M Sodium Phosphate pH 7
0.1 M Tris-HCl pH 8
M Tris-HCl pH 9

The samples were analyzed by non-chiral and chiral HPLC methods and examples of the results are presented in Tables 5, 8 and 11 herein.

### Example 7

### Determination of the Effect of temperature on enzyme activity and enantioselectivity

The effect of temperature on the activity and enantioselectivity was investigated by performing the standard assay at room temperature, 38°C and 55°C. The samples were analyzed by non-chiral and chiral HPLC methods and examples of the results are given in Tables 5, 8 and 11 herein.

### Example 8

### Determination of the Effect of solvents on enzyme activity and enantioselectivity

The enzyme reactions were performed in the presence of cosolvents and as biphasic systems, in order to investigate the effect of water-miscible and water-immiscible solvents on the enzymes. In the presence of cosolvents, the reactions were run under standard conditions, with substitution of the buffer with methanol or isopropanol. The final concentrations of solvent in the reactions was 0, 10, 25 and 40% (v/v).

The biphasic reactions were also carried out under standard conditions, with a layer of water-immiscible organic solvent forming the nonaqueous phase. The solvent was added at the following levels: 0%, 10%, 40% and 70% (v/v) of the aqueous phase. The samples from these reactions were evaporated by centrifugation under vacuum and redissolved in a 50:50 mixture of methanol or acetonitrile and water. The samples were analyzed by non-chiral and chiral HPLC methods.

### Example 9

### Determination of the Effect of process components on enzyme activity and enantioselectivity

### Activity

The effect of the process components on the activity of the enzymes was established by addition of the individual components to the enzymatic reaction. These components included the starting materials for the nitrile synthesis, aldehyde, cyanide and ammonium, as well as triethylamine, which is added in catalytic amounts to the nitrile synthesis reaction. The concentrations of the reactants were selected with possible process conditions in mind and were adapted to the levels of reactants used in the enzyme assays. In some cases, the solubility of the aldehydes and products was relatively low; in these cases, the highest level of solubility was added to the reactions as the highest level and 10% of this level as the lower value.

The enzymatic reactions were carried out under standard conditions, with addition of one or more of the following components: benzaldehyde, phenylglycine, phenylacetaldehyde, phenyllactic acid, 2-chlorobenzaldehyde, 2-chloromandelic acid, 5-hydroxypentanal, (S)-2-amino-6-hydroxy hexanoic acid, 4-methylleucuie, KCN, Triethylamine, NH₄Cl. Control reactions were performed under standard conditions, with no additive. The samples were analyzed by non-chiral HPLC.

### Stability

The stability of the enzymes to process conditions was monitored by incubation of the enzymes in the presence of the individual reaction components for predetermined time periods, prior to assay of the enzyme activity under standard conditions. In these experiments, the enzymes were incubated at a concentration of 1.2 mg protein/ml in the presence of each of the following reaction components: methanol, benzaldehyde, phenylglycine, phenylacetaldehyde, phenyllactic acid, 2-chlorobenzaldehyde, 2-chloromandelic acid, 5-hydroxypentanal, (S)-2-amino-6-hydroxy hexanoic acid, KCN, NH₄Cl.

### Assay conditions:

At 0, 2, 6 and 24 hours of incubation in the particular additive, 50 µl of the enzyme solution was removed, 50 µl of a 50 mM substrate stock solution added and the enzyme activity assayed under standard conditions. After substrate addition, the reactions were sampled at the following times: Phenylglycinonitrile: 10 mins; Phenylacetaldehyde cyanohydrin: 1 hour; 2-chloromandelonitrile: 2 hours. Control reactions were performed by incubation of the enzyme in buffer only. The samples were analyzed using non-chiral HPLC methods.

### Example 10

### Confirmation of putative hit enzymes

Following the preliminary characterization experiments, the enzymes which were identified as putative hits were assayed under the optimal conditions determined, in order to evaluate their performance, especially in terms of enantioselectivity, when higher conversions were attained. The enzymes were assayed with 25 mM substrate, under the conditions of pH and temperature noted in the tables included in the text. A standard concentration of 0.6 mg/ml protein was used for each of the enzymes, unless otherwise stated.

### Example 11

### Selected examples of chromatograms from enzyme reactions

In this section, representative examples of chromatograms for each substrate and product combination will be shown, together with a discussion of some of the challenges encountered with the methods and how they were addressed.

### D-phenylglycine

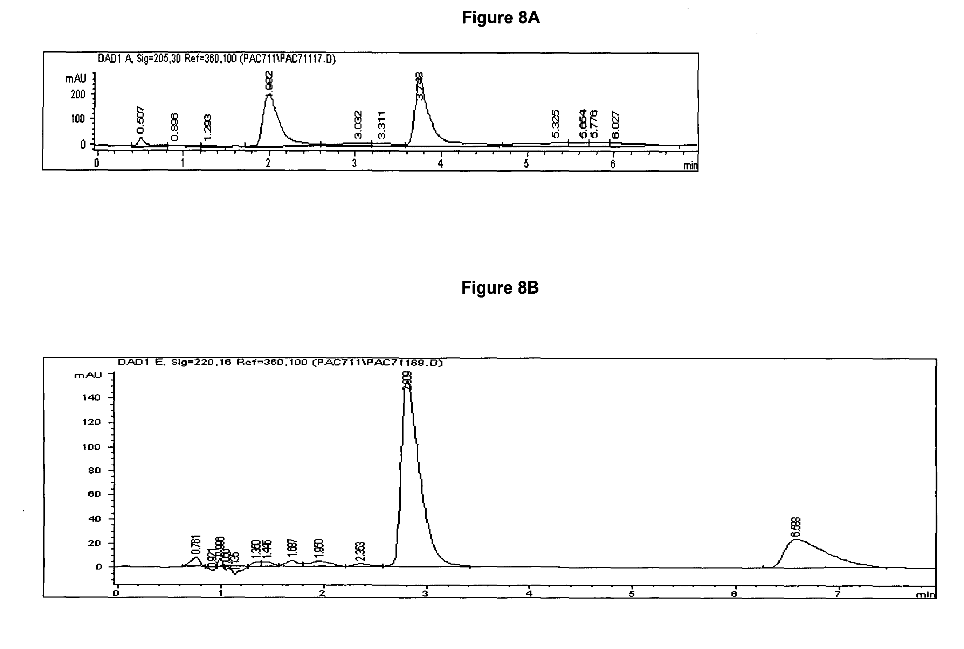

Non-chiral analysis showing the substrate peak eluting at 2.6 min and 3.2 min. See Figures 8A-8E. The two peaks were present in all samples containing higher concentrations of the nitrile; the second peak is thought to be a product associated with the nitrile; it decreased with time and was no longer present once complete conversion to the product had taken place. The chromatogram shown in Figure 8A is a blank control, containing only nitrile and buffer; the samples were all diluted with water and solvent as explained in section 1 above. This was repeated for all samples discussed below. An enzymatic reaction sample is shown in the chromatogram in Figure 8B , with the product eluting at 0.4 min.

Of note in these chromatograms is the small solvent front peak eluting at 0.3 min. Further representation of this peak is given in the chromatogram shown in Figure 8C, in which a negative control consisting of cell lysate in buffer, was run. A very small peak coeluted with the product at 0.4 min. In the initial phase of the project, this peak was regarded as problematic, although the appropriate controls were run with each experiment for in order to maintain accuracy. In these experiments, the peak area resulting from the cell lysate, although it was relatively small, was subtracted from the peak areas of the product in the enzymatic reactions. Improvement of this analysis was obtained by further dilution of the samples and the use of lower injection volumes on the HPLC. Following the implementation of these improvements, interference by this peak was shown to be minimal, as shown in the chromatogram illustrated in Fig. 6C.

The chiral analysis of phenylglycine is shown in chromatogram in Fig. 6D with the L-enantiomer eluting at 6 min and the D-enantiomer at 11 min. Good resolution between the two isomers was obtained. However, the column used was very sensitive and the characteristics of the column appeared to change over time, resulting in changes in the elution times of the acids. While this was easily detected by the use of the proper controls and standards, a greater problem existed in the coelution of the nitrile peak with the D-enantiomer (chromatogram shown in Fig. 6E). The cause of this coelution was unclear; however, it was easily detected by the use of appropriate standards; in addition, the UV spectrum of the acid was very distinctive, making the use of this tool effective in detecting the coelution. The problem was also easily resolved by adjusting the methanol content in the mobile phase.

### (R)-2-chloromandelic acid

The HPLC analysis of chloromandelic acid and chloromandelonitrile offered many of the challenges associated with the analysis of the phenylglycine samples. From the chromatogram shown in Fig. 7A, which contains only chloromandelonitrile in buffer, it is evident that a peak eluted at the same time as the product in the chromatogram shown in Fig. 7B, which represents a chloromandelic acid standard. The contribution of the cell lysate to this peak was found to be small; it would appear that the greatest contribution to this peak was from the chloromandelonitrile, either from a breakdown product or a contaminant in the nitrile preparation. The peak area remained constant throughout each experiment and, using the appropriate controls, it was found that subtraction of the peak area from that of the product yielded sufficient accuracy. Many attempts were made to change the HPLC conditions so that the product peak eluted at a later time; however, these attempts were not successful. Chromatogram shown in Fig. 7C illustrates the appearance of product and the reduction of the substrate peaks.

The chiral analysis of chloromandelic acid was almost problem-free. The elution of a small peak at the same time as the (*S*)-enantiomer presented some concern (the peak at 2.4 min in chromatogram shown in Fig. 7D). However, once it was established that this peak was present in all the samples at the same level, including the blank control, and that it had a different UV spectrum to that of the chloromandelic acid peak, it was not regarded as a problem. Consequently, it was subtracted from the peak eluting at 2.4 min in each sample. The (R)-enantiomer eluted at 3 minutes.

### (S)-phenyllactic acid

The analysis of phenyllactic acid was initially plagued with the same problems discussed for phenylglycine and 2-chloromandelic acid. However, in this case, adjustment of the solvent concentration in the nonchiral HPLC method led to a shift in the retention time of the acid, so that it no longer coeluted with the cell lysate peak. Following this, no problems were encountered with either the nonchiral or chiral methods. Representative nonchiral chromatograms of the product (1.9 min) and cyanohydrin substrate (3.7 min) are shown in Fig. 8A, while the chiral analysis of the acid is shown in Fig. 8B, with the L-enantiomer eluting at 2 min and the opposite enantiomer at 6 min.

### L-2-methylphenylglycine

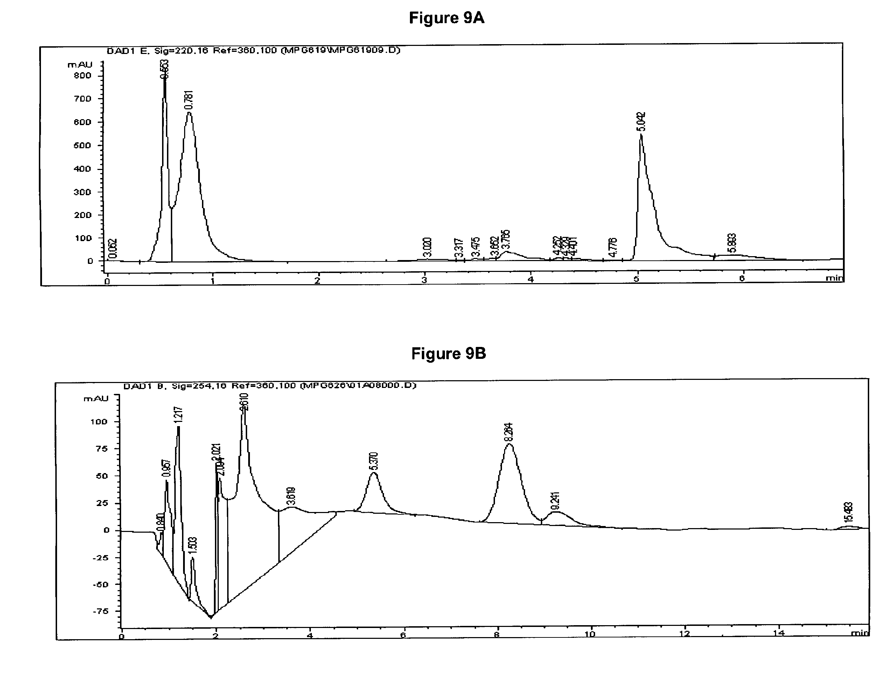

The analysis of methylphenylglycine was unproblematic, although the nonchiral method did not provide baseline separation between a cell lysate peak and the product peak, as shown in the chromatogram illustrated in Fig. 9A. The amino acid standard for this method was provided in the final stages of the project, thus minimizing the time for method development. In the chromatogram shown in Fig. 9A the amino acid elutes at 0.7 min and the aminonitrile at 5.0 min. Sufficient separation between the two initial peaks was obtained to allow the calculation of approximate conversion to product.

The chiral analysis of this compound provided good separation between the two enantiomers, as shown in the chromatogram illustrated in Fig. 9B. The L-enantiomer elutes at 5 min and the D-enantiomer at 8 min.

### L-tert-leucine

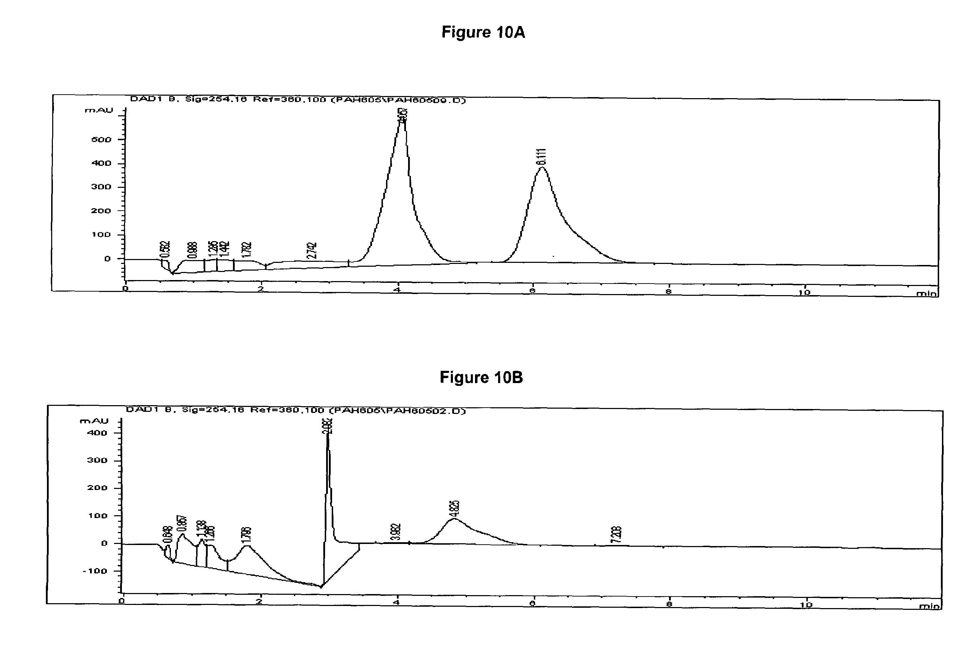

For the nonchiral analysis of *t*-leucine, the cell lysate presented the most serious problem amongst the group of products for this project. This was compounded by the low spectroscopic properties of the amino acid, leading to difficulty in differentiating the product peak from the cell lysate. Good separation of the individual product enantiomers was obtained by chiral analysis as shown in Fig. 10A. During the primary screen, a small peak eluted at the same time as the L-amino acid standard in certain samples (see Fig. 10B) and was thought to be the amino acid. However, further development of the method and the use of the appropriate controls established that this peak was actually a cell lysate peak.

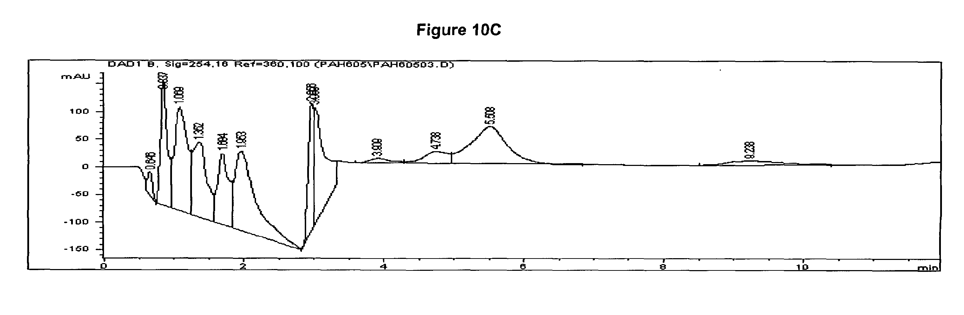

The aminonitrile eluted between the two *t*-leucine peaks, as shown in Fig. 10C; this chromatogram also shows the cell lysate peak at 4.8 min. The UV spectrum of the nitrile was distinct from that of the amino acid, making it easier to differentiate from the acid peaks.

### L-hydroxynorleucine ((S)-2-amino-6-hydroxy hexanoic acid)

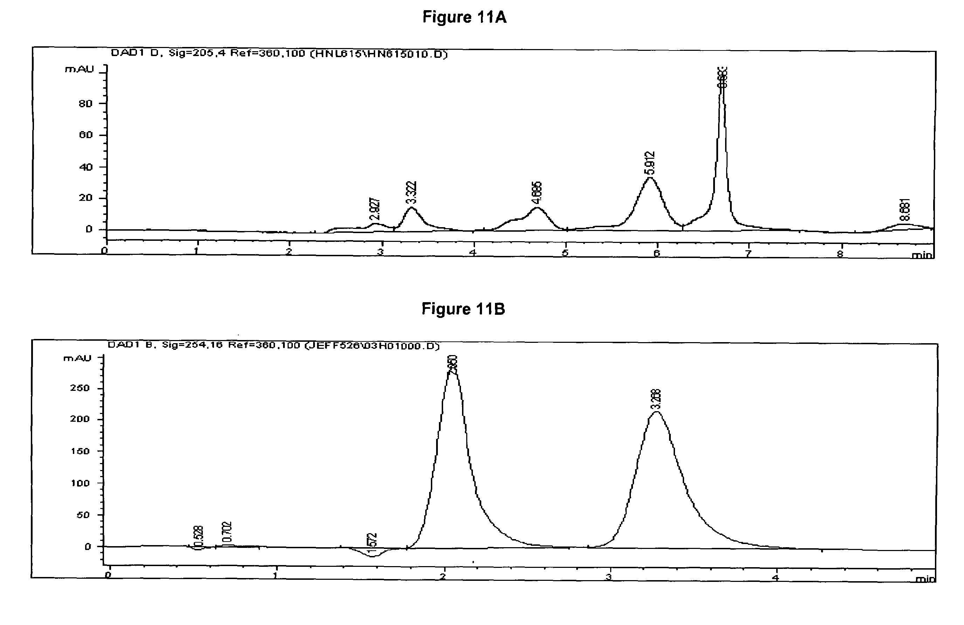

The chiral analysis of (S)-2-amino-6-hydroxy hexanoic acid was consistent and reliable. By contrast, the nonchiral method presented many problems, primarily as a result of non-separation between the nitrile and the acid peaks. Towards the latter half of the project, a method was developed and used successfully for the confirmation of activities. Prior to this, most of the analysis was performed using the chiral method; standard curves of the products were run in order to quantify the reactions. A representative chromatogram of (S)-2-amino-6-hydroxy hexanoic acid is shown in Figure 11A, with (S)-2-amino-6-hydroxy hexanoic acid eluting at 6 min. The aminonitrile was not detected by this method.

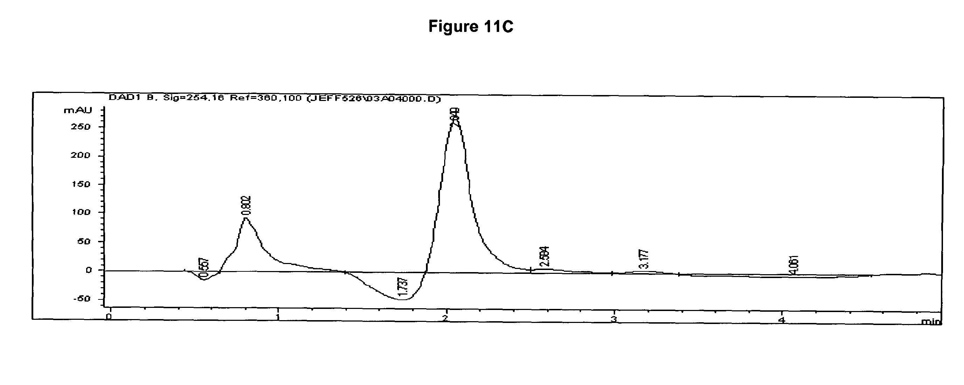

Separation of the individual 2-amino-6-hydroxy hexanoic acid enantiomers is shown in Fig. 11B. The L-enantiomer elutes first, at 2 min, followed by the D-enantiomer at 3 min. In Fig. 11C, an enzymatic sample is represented; the only area of slight concern is the negative peak preceding the elution of the L-enantiomer. However, it did not appear to interfere significantly with the elution of this enantiomer; method development did not eliminate the negative peak.

### 4-methyl-D-leucine and 4-methyl-L-leucine

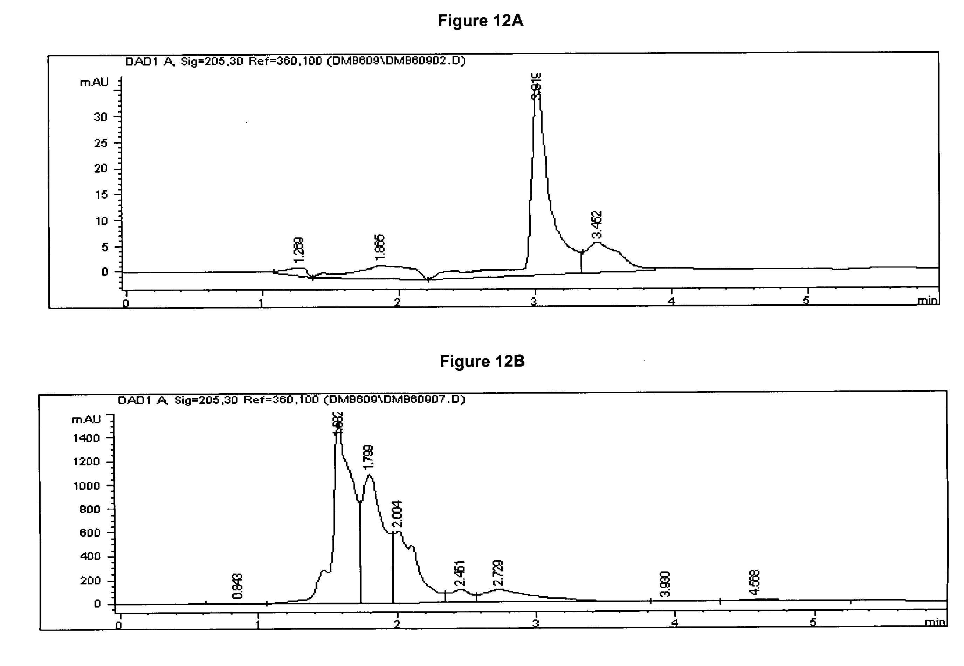

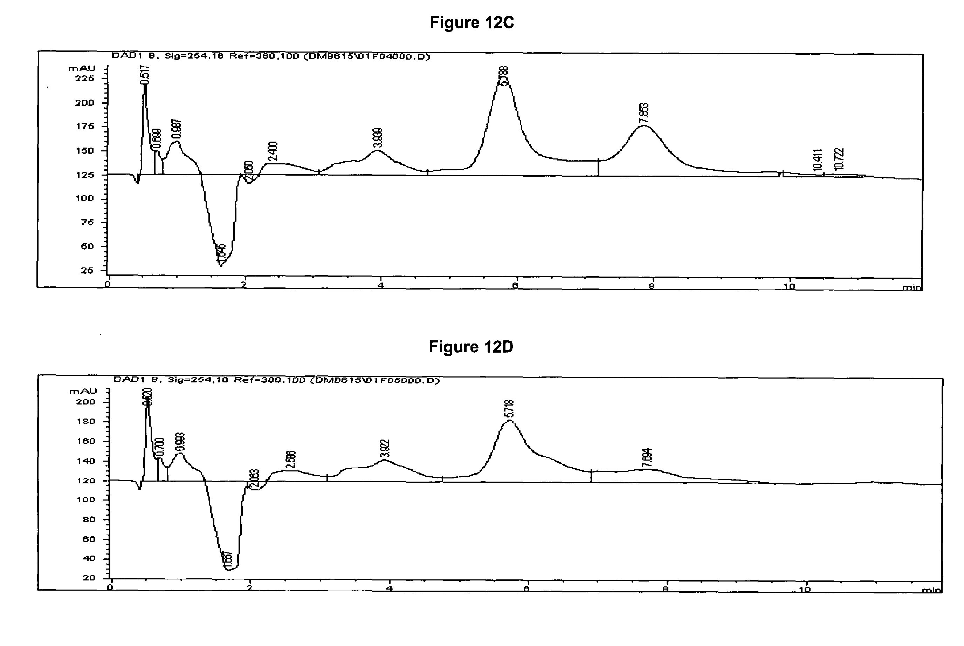

For the detection of 4-methylleucine, the chiral HPLC method again proved more reliable. The combination of low activities, together with the low sensitivity of the method to the compound led to difficulties in detection using nonchiral HPLC. A 2.5 mM standard of the amino acid is shown in Fig. 12A, with a peak height of approximately 40 mAU; this was substantially lower than those detected for the aromatic compounds. Chromatogram in Fig. 12B shows an enzymatic sample, in which conversion was detected using the chiral HPLC method; while it is not clear, it would appear that the 4-methylleucine peak elutes at 2.7 min and is extremely low in both peak height and area. This peak did not appear in samples which were negative by chiral HPLC analysis.

The chiral analysis of 4-methyl-L-leucine and 4-methyl-D-leucine did not present any problems. The L-enantiomer eluted at 5 min and the D-enantiomer at 7 min, although some peak shift did occur, as a result of the sensitivity to the column, described in section (i) for phenylglycine. In chromatograms shown in Figs. 14C-14D, the separation of these amino acids is shown; the first sample represents an enzyme which produced both enantiomers and in the second sample, the enzyme preferentially hydrolyzed the L-enantiomer, with a small amount D-amino acid forming.

### (S)-cyclohexylmandelic acid

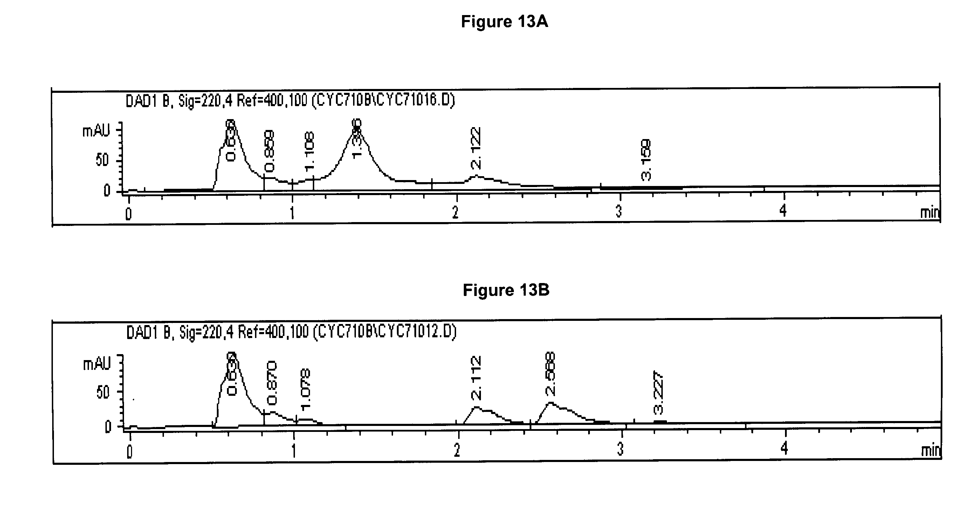

Chromatograms of the standards for cyclohexylmandelic acid (Fig. 13A) and the corresponding nitrile (Fig. 13B) are shown. The acid eluted at 1.3 min, while the cyanohydrin was observed at 2.5 min. The peak eluting at 2.1 min is thought to be the cyclohexylphenylketone, as shown by the elution of a ketone standard at this point.

### Example 12

### An Enzyme Library Approach to Biocatalysis: Development of a Nitrilase Platform for Enantioselective Production of Carboxylic Acid Derivatives

Biocatalytic processes can offer unique advantages in transformations that are challenging to accomplish through conventional chemical methods (Wong, C.-H.; Whitesides, G.M. Enzymes in Synthetic Organic Chemistry; Pergamon, New York, 1994; Drauz, K.; Waldmann, H., Roberts, S.M. Eds. Enzyme Catalysis in Organic Synthesis; VCH: Weinheim, Germany, 2nd ed., 2002). Nitrilases (EC 3.5.5.1) promote the mild hydrolytic conversion of organonitriles directly to the corresponding carboxylic acids (Kobayashi, M.; Shimizu, S. FEMS Microbiol. Lett. 1994, 120, 217; Bunch, A.W. In Biotechnology; Rehm, H.-J.; Reed, G.; Puhler, A.; Stadler, P., Eds.; Wiley-VCH: Weinheim, Germany, Vol. 8a, Chapter 6, pp 277-324; Wieser, M.; Nagasawa, T. In Stereoselective Biocatalysis; Patel, R.N., Ed.; Marcel Dekker: New York, 2000, Chapter 17, pp 461-486.) Fewer than fifteen microbially-derived nitrilases have been characterized and reported to date. (Harper, D.B. Int. J. Biochem. 1985, 17, 677; Levy-Schil, S.; Soubrier, F.; Crutz-Le Coq, A.M.; Faucher, D.; Crouzet, J.; Petre, D. Gene 1995, 161, 15; Yu, F. 1999, US Patent 5872000; Ress-Loschke, M.; Friedrich, T.; Hauer, B.; Mattes, R.; Engels, D. PCT Appl. WO 00/23577, **April 2000**.). Several nitrilases previously have been explored for the preparation of single-enantiomer carboxylic acids, although little progress has been made in the development of nitrilases as viable synthetic tools. This application describes the discovery of a large and diverse set of nitrilases and herein demonstrate the utility of this nitrilase library for identifying enzymes that catalyze efficient enantioselective production of valuable hydroxy carboxylic acid derivatives.

In an effort to access the most diversified range of enzymes that can be found in Nature, we create large genomic libraries by extracting DNA directly from environmental samples that have been collected from varying global habitats. (For a description of these methods, see: Short, J.M. Nature Biotech. 1997, 15, 1322; Handelsman, J.; Rondon, M.J.; Brady, S.F.; Clardy, J.; Goodman, R.M. Chem. Biol. 1998, 5, R245; Henne, A.; Daniel, R.; Schmitz, R.A.; Gottschalk, G. Appl. Environ. Microbiol. 1999, 65, 3901.). We have established a variety of methods for identifying novel activities through screening mixed populations of uncultured DNA. (Robertson, D.E.; Mathur, E.J.; Swanson, R.V.; Marrs, B.L.; Short, J.M. SIM News 1996, 46, 3; Short, J.M. US Patent 5,958,672, 1999**;** Short J.M. US Patent 6,030,779, 2000.) Through this approach, nearly 200 new nitrilases have been discovered and characterized. (For a concise description of the studies, see Materials and Methods section below.) All nitrilases were defined as unique at the sequence level and were shown to possess the conserved catalytic triad Glu-Lys-Cys which is characteristic for this enzyme class. (Pace, H.; Brenner, C. Genome Biology 2001, 2, 0001.1-0001.9.) Each nitrilase in our library was overexpressed and stored as a lyophilized cell lysate in order to facilitate rapid evaluation of the library for particular biocatalytic functions.

The initial investigations focused upon the efficacy of nitrilases for production of α-hydroxy acids **2** formed through hydrolysis of cyanohydrins **1**. Cyanohydrins are well-documented to racemize readily under basic conditions through reversible loss of HCN. (Inagaki, M.; Hiratake, J.; Nishioka, T.; Oda, J.; J. Org. Chem 1992, 57, 5643. (b) van Eikeren, P. US Patent 5,241,087, 1993.) Thus, a dynamic kinetic resolution process is possible whereby an enzyme selectively hydrolyzes only one enantiomer of **1**, affording **2** in 100% theoretical yield and with high levels of enantiomeric purity.

One important application of this type involves commercial production of (*R*)-mandelic acid from mandelonitrile. (Ress-Loschke, M.; Friedrich, T.; Hauer, B.; Mattes, R.; Engels, D. PCT Appl. WO 00/23577, April 2000; Yamamoto, K.; Oishi, K.; Fujimatsu, I.; Komatsu, K. Appl. Environ. Microbiol. 1991, 57, 3028; Endo, T.; Tamura, K. US Patent 5,296,373, March 1994.) Mandelic acid and derivatives find broad use as intermediates and resolving agents for production of many pharmaceutical and agricultural products. (Coppola, G.M.; Schuster, H.F. Chiral α-Hydroxy Acids in Enantioselective Synthesis; Wiley-VCH: Weinheim, Germany: 1997.) However, the few known nitrilases derived from cultured organisms have not been found useful for efficient and selective hydrolysis of analogous substrates.

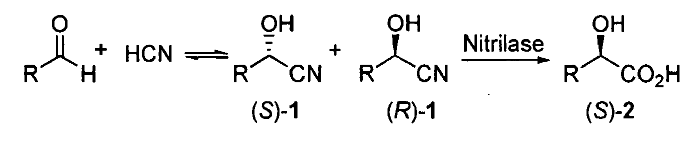

The nitrilase library was screened for activity and enantioselectivity in the hydrolysis of mandelonitrile (3a, Ar = phenyl) to mandelic acid. Preliminary results revealed that 27 enzymes afforded mandelic acid in >90% ee. One enzyme, SEQ ID NOS:385, 386, was studied in greater detail and was found to be very active for hydrolysis of mandelonitrile. Under standard conditions using 25 mM 3a and 0.12 mg/mL enzyme in 10% MeOH (v/v) 0.1 M phosphate buffer at 37oC and pH 8, (*R*)-mandelic acid was formed quantitatively within 10 min and with 98% ee. To confirm synthetic utility, the reaction was performed using 1.0 g 3a (50 mM) and 9 mg nitrilase (0.06 mg/mL nitrilase I); after 3 h (R)-mandelic acid was isolated in high yield (0.93 g, 86%) and again with 98% *ee.*

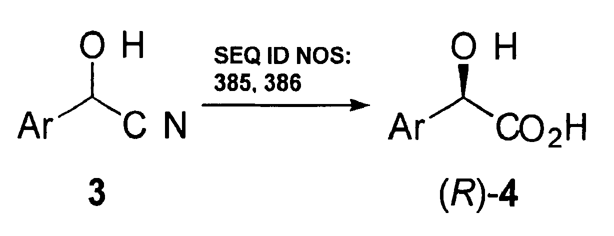

**Table 13. SEQ ID NOS:385, 386-catalyzed production of mandelic acid derivatives and analogues 4^{a}**

| Entry | Ar in 4 | Spec. Act.^{b} | TOF^{c} | % ee^{d} |
|---|---|---|---|---|
| 1 | C₆H₅ | 50 | 28 | 98 |
| 2 | 2-Cl-C₆H₅ | 3 | 1.7 | 97 |
| 3 | 2-Br-C₆H₅ | 10 | 5.6 | 96 |
| 4 | 2-Me-C₆H₅ | 9 | 5.1 | 95 |
| 5 | 3-Cl-C₆H₅ | 6 | 3.4 | 98 |
| 6 | 3-Br-C₆H₅ | 3 | 1.7 | 99 |
| 7 | 4-F-C₆H₅ | 21 | 11.8 | 99 |
| 8 | 1-naphthyl | 5 | 2.8 | 95 |
| 9 | 2-naphthyl | 5 | 2.8 | 98 |
| 10 | 3-pyridyl | 33 | 18.6 | 97 |
| 11 | 3-thienyl | 30 | 16.8 | 95 |

| | | | | |
|---|---|---|---|---|
| (a) Reactions were conducted under standard conditions (see text). Reaction time for complete conversion to 4 was 1-3 h. Entries 8-9 were conducted at pH 9 and 5 mM substrate concentration. (b) Specific activities were measured at 5 min transformation timepoints and are expressed as µmol mg⁻¹ min⁻¹. (c) TOF = turnover frequency, mol product/mol catalyst/sec. (d) Enantioselectivites were determined by chiral HPLC analysis. Hydroxy acids were isolated and absolute configurations were determined to be (R) in all cases. | | | | |

The substrate scope of SEQ ID NOS:385, 386 was next explored. As shown in Table 13, a broad range of mandelic acid derivatives as well as aromatic and heteroaromatic analogues (**4**) may be prepared through this method. SEQ ID NOS:385, 386 tolerates aromatic ring substituents in the *ortho-, meta-*, and *para-*positions of mandelonitrile derivatives and products of type 4 were produced with high enantioselectivities. Other larger aromatic groups such as 1-naphthyl and 2-naphthyl also are accommodated within the active site, again affording the acids **4** with high selectivity (Table 13, entries 8-9). Finally, 3-pyridyl and 3-thienyl analogues of mandelic acid were prepared readily using this process (Table 13, entries 10-11). This is the first reported demonstration of a nitrilase that affords a range of mandelic acid derivatives and heteroaromatic analogues of type **4**. High activity on the more sterically encumbered *ortho*-substituted and 1-naphthyl derivatives is particularly noteworthy.

We next examined the preparation of aryllactic acid derivatives **6** through hydrolysis of the corresponding cyanohydrins **5.** Phenyllactic acid and derivatives serve as versatile building blocks for the preparation of numerous biologically active compounds. (Coppola, G.M.; Schuster, H.F. Chiral α-Hydroxy Acids in Enantioselective Synthesis; Wiley-VCH: Weinheim, Germany: 1997.) Upon screening our nitrilase library against the parent cyanohydrin **5a** (Ar = phenyl), we found several enzymes that provided **6a** with high enantiomeric excess. One enzyme, SEQ ID NOS: 103, 104, was further characterized. After optimization, SEQ ID NOS:103, 104, was shown to provide (*S*)-phenyllactic acid (**6a**) with complete conversion (50 mM) and very high enantioselectivity (98% ee) over 6 h. The highest enantioselectivity 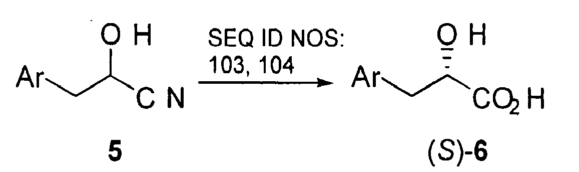 previously reported for biocatalytic
conversion of **5** to **6** was 75% ee achieved through a whole cell transformation using a *Pseudomonas* strain. (Hashimoto, Y.; Kobayashi, E.; Endo, T.; Nishiyama, M.; Horinouchi, S. Biosci. Biotech. Biochem. 1996, 60, 1279.)

**Table 14. Nitrilase II-catalyzed production of aryllactic acid derivatives and analogues 6^{a}**

| Entry | Ar in 6 | Spec. Act.*^{b}* | TOF*^{c}* | % ee*^{d}* |
|---|---|---|---|---|
| 1 | C₆H₅ | 25 | 16 | 99 |
| 2 | 2-Me-C₆H₅ | 160 | 100 | 95 |
| 3 | 2-Br-C₆H₅ | 121 | 76 | 95 |
| 4 | 2-F-C₆H₅ | 155 | 97 | 91 |
| 5 | 3-Me-C₆H₅ | 21 | 13 | 95 |
| 6 | 3-F-C₆H₅ | 22 | 14 | 99 |
| 7 | 1-naphthyl | 64 | 40 | 96 |
| 8 | 2-pyridyl | 10.5 | 6.6 | 99 |
| 9 | 3-pyridyl | 11.6 | 7.2 | 97 |
| 10 | 2-thienyl | 3.4 | 2.1 | 96 |
| 11 | 3-thienyl | 2.3 | 1.4 | 97 |

| | | | | |
|---|---|---|---|---|
| (a) Reaction conditions as in Table 13, except 0.016 mg/mL nitrilase was used. Full conversion to **6** was observed within 6 h. (b)-(d) See Table 13. The absolute configuration was determined to be (*S*) for phenyllactic acid and entries 2-11 were assigned (*S*) based upon identical chiral HPLC peak elution order. | | | | |

*Ortho* and *meta* substituents appear to be tolerated well by nitrilase II, with *ortho* substituted derivatives surprisingly being converted with higher rates relative to the parent substrate **5a**. Novel heteroaromatic derivatives, such as 2-pyridyl-, 3-pyridyl, 2-thienyl- and 3-thienyllactic acids, were prepared with high conversions and enantioselectivities (entries 8-11). Unexpectedly, *para* substituents greatly lowered the rates of these reactions, with full conversion taking over two weeks under these conditions.

The final transformation that we examined was desymmetrization of the readily available prochiral substrate 3-hydroxyglutarylnitrile (7) (Johnson, F.; Panella, J.P.; Carlson, A.A J. Org. Chem. 1962, 27, 2241) to afford hydroxy acid (*R*)-**8** which, once esterified to (*R*)-**9**, is an intermediate used in the manufacture of the cholesterol-lowering drug LIPITOR™. Previously reported attempts to use enzymes for this process were unsuccessful and **8** was produced with low selectivity (highest: 22% ee) and the undesired (*S*)-configuration. (Crosby, J.A.; Parratt, J.S.; Turner, N.J. Tetrahedron: Asymmetry 1992, 3, 1547; Beard, T.; Cohen, M.A.; Parratt, J.S.; Turner, N.J. Tetrahedron: Asymmetry 1993, 4, 1085; Kakeya, H.; Sakai, N.; Sano, A.; Yokoyama, M.; Sugai, T.; Ohta, H. Chem. Lett. 1991, 1823.)

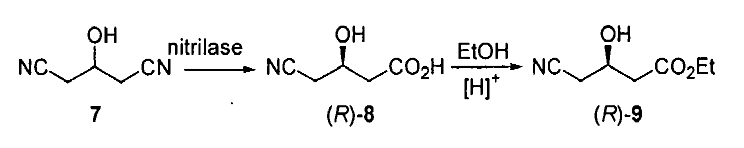

The nitrilase library was screened and unique enzymes were discovered and isolated that provided the required product (*R*)-**8** with high conversion (>95%) and >90% ee. Using one of the (*R*)-specific nitrilases, this process was operated on a 1.0 g scale (240 mM 7, 30 mg enzyme, 22°C, pH 7) and after 22 h, (*R*)-**8** was isolated in 98% yield and 95% ee. Interestingly, the same screening program also identified nitrilases that afford the opposite enantiomer (*S*)-**8** with 90-98% ee. Thus, the extensive screen of biodiversity has uncovered enzymes that provide ready access to either enantiomer of the intermediate **8** with high enantioselectivities. Our discovery of the first enzymes that furnish (*R*)-**8** underscores the advantage of having access to a large and diverse library of nitrilases.

| **SUMMARY for Nitrilases Activity on HydroxyGlutarylnitrile (Primary Data)** | | | |
|---|---|---|---|
| SEQ ID NO: | **%ee** | **% Conversion** | **LIPITOR™** |
| 107, 108 | 100 | 79 | S |
| 109, 110 | 100 | 79 | S |
| 111, 112 | 91 | 32 | S |
| 127, 128 | 92 | 106 | S |
| 129, 130 | 100 | 22 | S |
| 133, 134 | 86 | 14 | S |
| 113, 114 | 100 | 108 | S |
| 145, 146 | 100 | 100 | S |
| 101, 102 | 100 | 61 | S |
| 179, 180 | 100 | 75 | S |
| 201, 202 | 100 | 100 | S |
| 159, 160 | 100 | 71 | S |
| 177, 178 | 100 | 11 | S |
| 181, 182 | 100 | 58 | S |
| 183, 184 | 100 | 19 | S |
| 185, 186 | 100 | 78 | S |
| 191, 192 | 100 | 67 | S |
| 57, 58 | 100 | 73 | S |
| 197, 198 | 100 | 64 | S |
| 41, 42 | 100 | 16 | R |
| 59, 60 | 100 | 100 | S |
| 207, 208 | 100 | 111 | R |
| 209, 210 | 92 | 100 | R |
| 73, 74 | 100 | 3 | R |
| 153, 154 | 35 | 39 | |
| 171, 172 | 27 | 33 | |
| 195, 196 | 100 | 87 | R |
| 43, 44 | 100 | 111 | R |
| 67, 68 | 100 | 35 | S |
| 359, 360 | 100 | 87 | S |

By plumbing our environmental genomic libraries created from uncultured DNA, we have discovered a large array of novel nitrilases. This study has revealed specific nitrilases that furnish mandelic and aryllactic acid derivatives, as well as either enantiomer of 4-cyano-3-hydroxybutyric acid in high yield and enantiomeric excess.

### Procedures and Analytical Data:

Hydroxyglutarylnitrile was purchased from TCI America and used as received. Amino acids used for the preparation of aryl lactic acid standards were purchased from PepTech (Cambridge, MA). (*R*)-3-hydroxy-4-cyanobutyric acid was obtained from Gateway Chemical Technology (St. Louis, MO). Both (*R*)- and (*S*)- mandelic acid and (*R*)- and (*S*)- phenyl lactic acid standards were purchased from Sigma Aldrich. All other reagents were purchased from Sigma Aldrich and utilized without further purification. Silica Gel, 70-230 mesh, 60 Å, purchased from Aldrich, was used for chromatographic purifications. All ¹H NMRs and ¹³C NMRs were run on Bruker model AM-500 machines, set at room temperature, 500 MHz and 125MHz respectively for ¹H and ¹³C. Mass analyses and unit mass resolution was achieved by flow injection analysis (FlA) using a Perkin-Elmer Sciex API-4000 TURBOION™ Spray LC/MS/MS system. The LC flow was provided by Schimadzu LC-10Advp pumps, with 0.05% acetic acid and MeOH. Injections were accomplished via a Valco injector valve. The HPLC analysis was done on an Agilent 1100 HPLC with Astec's Chirobiotic R column (100 x 4.6 mm, cat no. 13022 or 150 x 4.6 mm, cat no. 13023) or Daicel's Chiralcel OD column (50 x 4.6 mm, cat no. 14022) and the DAD detector set at 210, 220, 230, and 250 nm. For specific rotations, a Perkin Elmer Model 341 Polarimeter was used, set at 589 nm, Na lamp, at room temperature, with a 100 mm path length cell. Concentrations for specific rotation are reported in grams per 100 mL of solvent. Microbiology techniques were executed in accordance to published protocols. (Sambrook, J. Fritsch, EF, Maniatis, T. (1989) Molecular Cloning: A Laboratory Manual (2nd ed.), Cold Spring Harbor Laboratory Press, Plainview NY.) Glycolic acid products were isolated and absolute configurations were determined to be (R) in all cases by comparison with literature optical rotation data on configurationally defined compounds except for (-)-3-pyridylglycolic acid, which to our knowledge is not known as a single enantiomer. (For mandelic, 2-chloromandelic, 2-methyl mandelic, 3-chloromandelic, 3-bromomandelic and 4-fluoromandelic acid see Hoover, J.R. E.; Dunn, G. L.; Jakas, D.R.; Lam, L.L.; Taggart, J. J.; Guarini, J.R.; Phillips, L. J. Med. Chem. 1974, 17(1), 34-41; For 2-bromo mandelic acid see Collet, A.; Jacques, J.; Bull. Soc. Chem. Fr. 1973, 12, 3330-3331; For 1- and 2-napthylglycolic acid see Takahashi, I; Y. Aoyagi, I. Nakamura, Kitagawa, A., Matsumoto, K., Kitajima, H. Isa, K. Odashima, K. Koga, K. Heterocycles 1999, 51(6), 1371-88; For 3-thienylglycolic acid Gronowitz, S. Ark. Kemi, 1957, 11, 519-525.)

For the aryl lactic acid products, absolute configuration was established to be (*S*) for phenyl lactic acid by comparison with literature optical rotation and for all other phenyl lactic acid products, absolute configurations were predicted based upon elution order using chiral HPLC. Absolute configuration for 3-hydroxy-4-cyano-butanoic acid was established by derivatization to (R)-(-)-Methyl (3-O-[benzoyl]-4-cyano)-butanoate and comparison to literature optical rotation data on configurationally defined compound. (3. Beard, T. Cohen, M. A. Parratt, J.S. Turner, N. J. Tetrahedron:Asymm. 4(6), 1993, 1085-1104.)

### Nitrilase Discovery and Characterization Methods:

### 1. Nitrilase Selection.

An *Escherichia coli* screening host strain, SEL700, was optimized for nitrilase selections on a nitrile substrate. An Abs₆₀₀ₙₘ = 1, resuspension of SEL700 screening host in 10 mM MgSO₄ was infected with kanamycin-resistant environmental DNA library for 45 minutes at 37°C, such that complete screening coverage of the library was achieved. Infected cells, now denoted by kanamycin resistance, were plated on kanamycin LB plates and allowed to grow overnight at 30°C. Titer plates were also made to determine infection efficiency. Cells were pooled, washed, and resuspended the next morning with 10 mM MgSO₄. Transformed clones were inoculated into M9 media (without nitrogen) with 10 mM of nitrile substrate. M9 media consisted of 1X M9 salts (NH₄Cl omitted), 0.1 mM CaCl₂, 1 mM MgSO₄, 0.2 % glucose, and approximately 10 mM of a nitrile selection substrate. The selection cultures were then incubated at 30°C, shaking at 200 rpm, for up to five weeks. Positive nitrilase cultures were identified by growth, due to positive clone's ability to hydrolyze nitrile substrate. Positive clones were isolated by streaking out a selection culture with growth and subsequent secondary culturing of isolated colonies in the same defined media. The DNA from any positive secondary cultures exhibiting re-growth was then isolated and sequenced to confirm discovery of a nitrilase gene and to establish the unique nature of that gene.

### 2. Nitrilase Biopanning.

Traditional filter lift hybridization screening protocols are limited to libraries with approximately 10⁶ to 10⁷ members. Attempting to screen one library would require approximately 5,000 filter lifts. Therefore, solution phase and other biopanning formats have been developed for ultra high throughput sequence based screening permitting rapid screening of up to 10⁸ member environmental libraries In the solution format, the DNA from a large number of library clones is mixed with tagged molecules of interest under conditions which promote hybridization. The tagged clones and hybridized DNA are then removed from solution and washed at some level of stringency to remove clones which do not have sequence identity with the probe. The hybridized DNA is then eluted and recovered. Clones of interest are sequenced and cloned to provide enzyme activities of interest. This method has been demonstrated to achieve up to 1,000-fold enrichment per round for sequences of interest.

### 3. High Throughput Nitrilase Activity Assay.

Activity assays were conducted using 25 mM (∼3 mg/mL) substrate, 0.1 mg/mL nitrilase in 0.25 mL of assay solution. Assay solutions consisted of 0-10% (v/v) MeOH in 0.1 M sodium phosphate buffer solution at pH 7 to 9 and temperatures 37°C or 22°C. Specific activities were measured at 5 min transformation time point, unless otherwise noted, and are expressed in units µmol mg⁻¹ min⁻¹. Enantiomeric excess and conversion rates were determined by high throughput HPLC analysis comparing enzyme product concentration to standard curves of racemic acid products. Analytical conditions for the products are tabulated below.

### Analytical Methods:

| | **Acid Product** | **Column** | **Liquid Chromatography Method** | **Retention Times of enantiomers (min)** |
|---|---|---|---|---|
| 1.1 | mandelic acid | Chirabiotic R 100 x 4.6 mm | 20%[0.5% AcOH], 80% CH₃CN 1 ml/min | 2.4 (*S*); 2.9 (*R*) |
| 1.2 | 2-Cl-mandelic acid | Chirabiotic R 100 x 4.6 mm | 20%[0.5% AcOH], 80% CH₃CN 1 ml/min | 2.3 (*S*); 2.9 (*R*) |
| 1.3 | 2-Br-mandelic acid | Chirabiotic R 100 x 4.6 mm | 20%[0.5% AcOH], 80% CH₃CN 1 ml/min | 2.8; 4.0 |
| 1.4 | 2-CH₃-inandelic acid | Chirabiotic R 100 x 4.6 mm | 20%[0.5% AcOH], 80% CH₃CN 1 ml/min | 3.1; 3.8 |
| 1.5 | 3-Cl-mandelic | Chirabiotic R 100 x 4.6 mm | 10%[0.5% AcOH], 90% CH₃CN I ml/min | 3.1; 3.8 |
| 1.6 | 3-Br-mandelic | Chirabiotic R 100 x 4.6 mm | 10%[0.5% AcOH], 90% CH₃CN 1 ml/min | 3.3; 3.9 |
| 1.7 | 4-F-mandelic | Chirabiotic R 150 x 4.6 mm | 20%[0.5% AcOH], 80% CH₃CN 1 ml/min | 3.7; 4.8 |
| 1.8 | 1-napthylglycolic acid | Chirabiotic R 100 x 4.6 mm | 4%[0.5% AcOH], 96% CH₃CN I ml/min | 3.1; 3.7 |
| 1.9 | 2-napthylglycolic acid | Chirabiotic R 100 x 4.6 mm | 4%[0.5% AcOH], 96% CH₃CN 1 ml/min | 3.7; 4.7 |
| 1.10 | 3-pyridylglycolic acid | Chirabiotic R 100 x 4.6 mm | 5% [0.5% AcOH], 65% H₂O, 30% CH₃CN, 2 ml/min | 4.4; 5.5 |
| 1.11 | 3-thienylglycolic acid | Chirabiotic R 100 x 4.6 mm | 20%[0.5% AcOH], 80% CH₃CN 2 ml/min | 1.4; 2.5 |
| 2.1 | phenyl lactic acid | Chirabiotic R 150 x 4.6 mm | 20%[0.5% AcOH], 80% CH₃CN 1 ml/min | 2.8 (*S*); 4.0 (*R*) |
| 2.2 | 2-methylphenyl lactic acid | Chirabiotic R 150 x 4.6 mm | 20%[0.5% AcOH], 80% CH₃CN 1 ml/min | 2.5; 2.8 |
| 2.3 | 2-bromophenyl lactic acid | Chirabiotic R 150 x 4.6 mm | 20%[0.5% AcOH], 80% CH₃CN 1 ml/min | 2.8; 3.2 |
| 2.4 | 2-tluorophenyl lactic acid | Chirabiotic R 150 x 4.6 mm | 20%[0.5% AcOH], 80% CH₃CN 1 ml/min | 2.6; 2.9 |
| 2.5 | 3-methylphenyl lactic acid | Chirabiotic R 150 x 4.6 mm | 20%[0.5% AcOH], 80% CH₃CN 1 ml/min | 2.4; 3.2 |
| 2.6 | 3-fluorophenyl lactic acid | Chirabiotic R 150 x 4.6mm | 20%[0.5% AcOH], 80% CH₃CN 1 ml/min | 2.8; 3.6 |
| 2.7 | 1-napthyllactic acid | Chirabiotic R 150 x 4.6mm | 20%[0.5% AcOH], 80% CH₃CN 1 ml/min | 2.7; 3.1 |
| 2.8 | 2-pyridyllactic acid | Chirabiotic R 150 x 4.6mm | 20%[0.5% AcOH], 80% CH₃CN 1 ml/min | 2.5; 2.9 |
| 2.9 | 3-pyridyllactic acid | Chirabiotic R 150 x 4.6mm | 20%[0.5% AcOH], 80% CH₃CN 1 ml/min | 2.9; 3.6 |
| 2.10 | 2-thienyllactic acid | Chirabiotic R 150 x 4.6mm | 20%[0.5% AcOH], 80% CH₃CN 1 ml/min | 3.6; 4.6 |
| 2.11 | 3-thienyllactic acid | Chirabiotic R 150 x 4.6mm | 20%[0.5% AcOH], 80% CH₃CN 1 ml/min | 3.5; 4.6 |
| | Methyl(3-O [benzoyl]-4-cyano)-butanoate | Daicel OD 50 x 4.6 mm | 5% isopropanol, 95% hexane I ml/min | 4.5 (*R*); 5.4(*S*) |

### Cyanohydrin (Substrate) Synthesis:

**Mandelonitrile Synthesis Method A:** Acetone cyanohydrin (685 µL, 7.5 mmol), aldehyde (5 mmol), and catalytic DIEA (13 µL, 0.075 mmol) were mixed at 0 °C. The reactions were stirred on ice for 45 minutes. To drive the equilibrium toward the product, acetone was removed *in vacuo.* Subsequently, crude reactions were acidified with H₂SO₄ (3 µL) and stored at -20°C. TLC was used to monitor reaction progress (3:1 hexane/ethylacetate (EtOAc).

**Mandelonitrile Synthesis Method B:** To a solution of KCN (358 mg, 5.5 mmol) in MeOH (1 mL) at 0°C was added aldehyde (5 mmol) and acetic acid (315 µL, 5.5 mmol). After stirring for one hour on ice, MeOH was removed *in vacuo,* and the crude mixture was partitioned using EtOAc and H₂O. The organic fraction was retained and concentrated *in vacuo.* TLC analysis was used to monitor reaction progress (3:1 Hexanes/EtOAc).

**Aryl Acetaldehyde Cyanohydrin:**, Arylacetic acid (50 mmol) was dissolved in 50 ml anhydrous tetrahydrofuran (THF) in a two-neck 500 ml round-bottom flask under N₂(g) atmosphere. To this solution cooled to 0 °C, under vigorous mixing, was added slowly 105 mmol ofthexylchloroborane-dimethyl sulfide (2.55 M in methylene chloride). The reaction was allowed to proceed overnight. Excess acetic acid (10 ml) was added to quench and acidify the reaction followed by the addition 10 ml water. After stirring at room temperature for 1 hour, solvent was removed *in vacuo* and the residue was dissolved in 100 ml water and extracted with 200 ml EtOAc. The EtOAc layer was dried over sodium sulfate, filtered and then concentrated *in vacuo.* Subsequently, 60 mmol of KCN, followed by 100 ml methanol was added to the residue. The solution was then cooled to 0 °C and acetic acid (60 mmol) added. The reaction was stirred for 1-2 hours after all KCN dissolved. Solvents were removed *in vacuo* and residue was dissolved in 100 ml water and 200 ml EtOAc. The aqueous layer was extracted with EtOAc one more time. Combined EtOAc extracts were washed with saturated brine and dried over sodium sulfate, filtered and then concentrated *in vacuo* to obtain crude cyanohydrin product. The cyanohydrin was purified by silica-gel column (hexane/EtOAc), as necessary.

**2-chloro mandelonitrile:** ¹H NMR (CDCl₃, 500 MHz) δ 7.69 (m, 1H), 7.41 (m, 1H), 7.36 (m, 2H), 5.84 (s, 1H), 3.07 (br, 1H). ¹³C NMR (CDCl₃, 125 MHz) δ 132.89, 132.73, 131.22, 130.19, 128.48, 127.84, 118.24, 60.87. MS calc'd for [C₈H₆ClNO] 167.01 found 167.9 (LC-MS +).

**2-bromomandelonitrile: ¹H** NMR (CDCl_{3,} 500 MHz) δ 7.72 (d, 1H, *J*= 6.58), 7.62 (d, 1H, *J*= 8.35), 7.43 (t, 1H, *J*= 8.42), 7.30 (t, 1H, *J*= 7.00), 5.85 (s, 1H). ¹³C NMR (CDCl₃, 125 MHz) δ 134.550, 133.584, 131.564, 128.819, 128.535, 122.565, 118.153, 63.379.

**2-methylmandelonitrile:** ¹H NMR (CDCl₃, 500 MHz) δ: 7.60 (d, 1H, *J* = 7.4), 7.23-7.35 (m, 3H), 5.66 (s, 1H), 2.44 (s, 3H). ¹³C NMR (CDCl₃, 298 K, 125 MHz) δ: 136.425, 133.415, 131.450, 130.147,127.204, 126.894, 118.952, 18.916. MS calc'd for [C₉H₉NO] 147.07, found 147.2 (ESI +).

**3-chloromandelonitrilc:** ¹H NMR (CDCl₃, 500 MHz) δ 7.55 (s, 1H), 7.43-7.37 (m, 3H), 5.54 (s, 1H). ¹³C NMR (CDCl₃, 125 MHz) δ 137.183, 135.480, 130.718, 130.303,127.047, 124.891, 118.395, 63.156. MS calc'd for [C₈H₆ClNO] 167.01 found 167.9 (LC-MS +).

**3-bromomandelonitrile:** ¹H NMR (CDCl₃, 500 MHz) δ 7.69 (s, 1H), 7.56 (d, *J*= 6.2 Hz, 1H), 7.45 (d, *J*= 5.5Hz, 1H), 7.32 (t, *J*= 6.4. Hz, 1H), 5.53 (s, 1H). ¹³C NMR (CDCl₃, 125 MHz) δ 137.376, 133.201, 130.934, 129.208,125.359, 123.380, 118.458, 63.006. MS calc'd for [C₈H₆BrNO] 212.0 found 211.9 (LC-MS +).

**4-fluoromandelonitrile:** ¹H NMR (CDCl₃, 500 MHz) δ 5.54 (s, 1H), 7.13 (m, 2H), 7.51-7.53 (m, 2H). ¹³C NMR (CDCl₃, 125 MHz) δ 63.02, 116.44, 118.97, 128.90, 131.54, 132.51, 162.575.

**4-chloromandelonitrile:** ¹H NMR (CDCl₃, 500 MHz) δ 7.47 (d, *J*= 7.0 Hz, 2H), 7.42 (d, *J*= 7.0 Hz , 2H), 5.53 (s , 1H). ¹³C NMR (CDCl₃, 125 MHz) δ 136.209, 133.845, 129.647, 128.232, 118.630, 63.154. MS calc'd for [C₈H₆ClNO] 167.01 found 167.9 (LC-MS +)

**1-naphthyl cyanohydrin :** ¹H NMR (CDCl₃, 500 MHz) δ 8.14 (d, 1H, *J* = 8.5), 7.92 (t, 2H, *J* = 6.1), 7.82 (d, 1H, *J* = 5.7), 7.62 (t, 1H, *J* = 6.1), 7.56 (t, 1H, *J* = 6.1), 7.50 (t, 1H, *J* = 6.1), 6.18 (s, 1H); ¹³C NMR (CDCl₃, 125 MHz) δ 137.0, 135.7, 134.2, 131.1, 129.2, 127.5, 126.7, 125.8, 125.3, 123.1, 119.0, 62.4; MS calc'd for [C₁₂H₉O] 183.21, found 183.2 (ESI +).

**2-naphthyl cyanohydrin:** ¹H NMR (CDCl₃, 500 MHz) δ 8.03 (s, 1H), 7.92 (d, 1H, *J* = 8.6), 7.87-7.91 (m, 2H), 7.61 (dd, 1H, *J* = 6.7, 1.2), 7.55-7.60 (m, 2H), 5.72 (s, 1H); ¹³C NMR (CDCl₃, 125 MHz) δ 134.9, 133.9, 132.7, 129.6, 128.6, 128.0, 127.4, 127.2, 126.4, 123.9, 118.9, 64.1; MS calc'd for [C₁₂H₉O] 183.21, found 183.2 (ESI +).

**3-pyridyl cyanohydrin:** ¹H NMR (CDCl₃, 500 MHz) δ: 8.62 (d, 1H, *J* = 1.8), 8.57 (d, 1H, *J* = 5.1), 7.94 (d, 1H, *J* = 8.1), 7.41 (dd, 1H, *J* = 8.1, 5.1), 5.64 (s, 1H); ¹³C NMR (CDCl₃, 125 MHz) δ 149.921, 147.355, 135.412, 133.044, 124.443, 118.980, 61.085. MS calc'd for [C₇H₆N₂O] 134.05, found 135.2 (ESI +).

**3-thienyl cyanohydrin:** ¹H NMR (CDCl₃, 500 MHz) δ 7.45 (d, *J* = 2.2 Hz 1H), 7.56 (dd, *J* = 6.2 Hz, 1H), 7.45 (d, *J*= 5.5Hz, 1H), 7.32 (t, *J*= 6.4. Hz, 1H), 5.53 (s, 1H). ¹³C NMR (CDCl₃, 125 MHz) δ 137.376, 133.201, 130.934, 129.208,125.359, 123.380, 118.458, 63.006. MS calc'd for [C₆H₅NOS] 139.01 found 139.9 (LC-MS +). **phenyl acetaldehyde cyanohydrin:** ¹H NMR (CDCl₃, 500 MHz) δ □7.34 (m, 5H), 4.64 (t, *J* = 6.75 Hz, 1H), 3.11 (d, *J* = 6.75 Hz, 2H), 2.75 (br, 1H). □¹³C NMR (CDCl₃, 125 MHz) δ 133.96, 129.91, 129.16, 128.08, 119.47, 62.33, 41.55.

**2-methylphenyl acetaldehyde cyanohydrin:** ¹H NMR (CDCl₃, 500 MHz) δ □7.11 (m, 4H), 4.61 (t, *J* = 6.62 Hz, 1H), 3.12 (d, *J* = 6.62 Hz, 2H), 2.14(s, 3H). □¹³C NMR (CDCl₃, 125 MHz) δ 136.94, 136.47, 132.57, 130.48, 127.61, 125.75, 120.11, 62.95, 44.73 MS calc'd for [C₁₀H₁₁NO]: 161.08, found 162.2 (M+Na, ESI +)

**2-bromophenyl acetaldehyde cyanohydrin:** ¹H NMR (CDCl₃, 500 MHz) δ □□7.20 (m, 4H), 4.78 (t, *J* = 6.5 Hz, 1H), 3.26 (d, *J* = 6.5 Hz, 2H). ¹³C NMR (CDCl₃, 100 MHz) δ 133.93, 132.82, 131.72, 129.21, 128.12, 124.86, 119.41, 63.02, 44.89.

**2-fluorophenyl acetaldehyde cyanohydrin:** ¹H NMR (CDCl₃, 500 MHz) δ 7.2 (m, 2H), 7.02 (m, 2H), 4.50 (dd, *J* = 4.62 Hz, *J* = 7.88 Hz, 1H), 3.23(dd, *J* = 4.62 Hz, 1 *J* = 14.12 Hz, 1H), 2.97 (dd, 7.88 Hz, 14.12 Hz, 1H). ¹³CNMR (CDCl₃, 125 MHz) δ 132.18, 131.52, 129.66, 129.03, 128.07, 124.05, 115.8, 63.02, 44.79 MS calc'd for [C₉H₈FNO] 165.06, found 164.2 (ESI +).

**3-methylphenyl acetaldehyde cyanohydrin:** ¹H NMR (CDCl₃, 500 MHz) δ 7.18 (m, 1H), 7.02 (m, 3H), 4.54 (dd, *J* = 4.62 Hz, *J* = 8 Hz, 1H), 3.06 (dd, *J* = 4.62 Hz, *J* = 14.38 Hz, 1H), 2.83(dd, *J* = 8 Hz, *J* = 14.38 Hz, 1H), 2.36 (s, 3H) ¹³C NMR (CDCl₃, 125 MHz) δ 176.25, 138.18, 136.0, 130.97, 128.93, 127.68, 126.58, 76.42, 34.29, 37.69 MS calc'd for [C₁₀H₁₂O₃] 180.08, found 180.0 (ESI +).

**3-fluorophenyl acetaldehyde cyanohydrin:** ¹H NMR (CDCl₃, 500 MHz) δ 7.18 (m, 2H), 6.95 (m, 2H), 4.44 (dd, 1H), 3.11 (dd, 1H). ¹³C NMR (CDCl₃, 125 MHz) δ 130.40, 125.53, 124.85, 116.92, 114.87, 114.50, 119.77, 61.97, 41.27.

**1-napthyl acetaldehyde cyanohydrin:** ¹H NMR (CDCl₃, 500 MHz) δ 8.07(m, 1H), 7.86(m, 1H), 7.74(m, 1H), 7.41(m, 4H),4.20 (t, *J* = 7 Hz, 1H), 3.33 (d, *J* = 6.8 Hz, 2H) ¹³C NMR (CDCl₃, 125 MHz) δ 177.7, 140.31, 129.74, 129.24, 128.92, 128.26, 127.84, 125.63, 124.53, 124.05, 123.42, 70.58, 38.0 MS calc'd for [C₁₃H₁₁NO] 197.08, found 197.1 (ESI +).

**2-pyridyl acetaldehyde cyanohydrin:** ¹H NMR (CDCl₃, 500 MHz) δ 8.50 (m, 1H), 7.85 (m, 1H), 7.48 (m, 1H), 7.34 (m, 1H), 4.42 (m, 1H), 3.19 (dd, *J* = 3.5 Hz, *J* = 13.7 Hz, 2H). ¹³C NMR (CDCl₃, 125 MHz) δ 157.44, 145.69, 140.24, 126.96, 126.16, 122.99, 60.30, 42.60 MS calc'd for [C₈H₈N₂O] 148.06, found 149.1 (ESI +).

**3-pyridyl acetaldehyde cyanohydrin:** ¹H NMR (CDCl₃, 500 MHz) δ 8.62 (d, 1H, *J* = 1.8), 8.57 (d, 1H, *J* = 5.1), 7.94 (d, 1H, *J* = 8.1), 7.41 (dd, 1H, *J* = 8.1, 5.1), 5.64 (s, 1H). ¹³C NMR (CDCl₃, 125 MHz) δ: 149.921, 147.355, 135.412, 133.044, 124.443, 118.980, 61.085. Exact Mass calculated for [C₇H₆N₂O]: 134.05, found: 135.2 (ESI +).

**2-thienyl acetaldehyde cyanohydrin:** ¹H NMR (CDCl₃, 500 MHz) δ 7.1 (m, 1H), 6.9 (m, 1H), 6.8 (m, 1H), 4.11 (t, *J* = 7.0Hz, 1H), 2.86 (d, *J* = 7.0Hz, 2H). ¹³C NMR (CDCl₃, 125 MHz) δ 127.68, 127.41, 125.58, 124.60, 118.70, 63.25, 44.84.

**3-thienyl acetaldehyde cyanohydrin:** ¹H NMR (CDCl₃, 500 MHz) δ 7.09 (m, 3H), 4.60 (t, *J* = 6.25Hz, 1 H), 3.12 (d, *J* = 6.25Hz, 2H). ¹³C NMR (CDCl₃, 125 MHz) δ 129.05, 127.16, 125.27, 122.65, 119.87, 61.58, 44.90.

**Preparation of racemic mandelic acids standards from corresponding cyanohydrins:** (Stoughton, R.W. J. Am. Chem. Soc. 1941, 63, 2376) 2-bromomandelonitrile (230 mg, 1.08 mmol) was dissolved in cone. HCl (1 mL) and stirred at room temperature for 18 h and then at 70 °C for 24 h. After cooling, the reaction mixture was extracted with diethyl ether (4 x 2 mL). Organic extracts were, combined, dried over MgSO₄, filtered and concentrated *in vacuo.* 2-bromomandelic acid was isolated as a colorless powder (180 mg, 0.78 mmol, 70 % yield).

**Preparation of racemic aryllactic acids standards from corresponding amino acids:** Phenylalanine (10 mmol, 1.65g) was dissolved in 30 ml 2N H₂SO₄ at room temperature under N₂ (g) atmosphere. Sodium nitrite (1.4 g in 3 ml aqueous solution, 2 eq) solution was added slowly to the reaction mixture over a period of 3-4 hours with vigorous stirring at room temperature under N₂ (g) atmosphere. The reaction mixture was stirred overnight and the phenyllactic acid product was then extracted into diethylether (3 x 30 ml). Combined ether extracts were dried over MgSO₄ and then filtered and concentrated *in vacuo.* (Kenji, I.; Susumu, A.; Masaru, M.; Yasuyoshi, U.; Koki, Y.; Koichi, K. Patent Number, WO0155074, Publication date: 2001-08-02.)

### General Method for Enzymatic Preparation of α-hydroxy acids:

**(*R*)-(-)-Mandelic Acid** To a solution of mandelonitrile (1.005 g, 7.56 mmol) in 150 mL of sodium phosphate (100 mM) buffer at pH 8 with 10% v/v methanol, that had been N₂ (g) sparged, at 37 °C, was added 9 mg of nitrilase 1 (normalized for nitrilase content). The reaction was conducted under N₂ (g) atmosphere on a rotating platform shaker. Reaction progress was monitored by withdrawing aliquots for HPLC analysis. After 3 h incubation, the reaction mixture was acidified to pH 2 with 1 N HCl and extracted with diethyl ether (4 x 50 ml). Organic fractions were concentrated *in vacuo* and then the residue was taken up in 10% sodium bicarbonate solution. This aqueous solutions was then washed with diethyl ether (3 x 50 ml) and then acidified to pH 2 with 1 N HCl and extracted with diethyl ether (3 x 50 ml). Organic fractions were combined, washed with brine, dried over MgSO₄, filtered and then concentrated *in vacuo*. (*R*)-(-)-Mandelic acid (933 mg, 6.22 mmol) was isolated as a colorless powder in 86 % yield. ¹H NMR (DMSO-d₆, 500 MHz) δ 12.6 (br, s, 1H) 7.41 (m, 2H), 7.34 (m, 2H), 7.28 (m, 1H), 5.01 (s, 1H). ¹³C NMR DMSO-d₆, 125 MHz) δ 174.083, 140.216, 128.113, 127.628, 126.628, 72.359. MS calc'd for [C₈H₈O₃] 150.07, found 150.9 (ESI +); *ee* = 98 % [HPLC]. [α]²⁰₅₉₈ = -134.6 (c = 0.5, methanol).

**(-)-2-chloromandelic acid** ¹H NMR (DMSO-d₆, 500 MHz) δ 7.75 (m, 1H), 7.44 (m, 1H), 7.34 (m, 2H), 5.34 (s, 1H). ¹³C NMR (DMSO, 298K, 125MHz) δ 173.070, 137.985, 132.105, 129.399, 129.158, 128.705, 127.235. MS calc'd for [C₈H₇ClO₃] 186.0, found 185.0 (LC-MS -). *ee* = 96 % [HPLC]. 92 % yield. [α]²⁰₅₉₈ = -137.6 (c= 0.5, ethanol).

**(-)-2-bromomandelic acid** ¹H NMR (DMSO-d₆, 500 MHz) δ 7.60 (d, *J* = 7.93, 1H), 7.48 (m, 1H), 7.40 (m, 1H), 7.25 (m, 1H), 5.30 (s, 1H). ¹³C NMR DMSO-d₆, 125 MHz) δ 172.994, 139.61, 132.355, 129.652, 128.753, 127.752, 122.681, 71.644. MS calc'd for [C₈H₇BrO₃] 230.0, found 230.9. *ee* = 96% [HPLC]. 92% yield. [α]²⁰₅₉₈ = - 116.4 (c= 0.5, ethanol).

**(-)-2-methylmandelic acid** ¹H NMR (DMSO-d_{b}, 500 MHz) δ 11.78 (bs, 1H) 7.38 (m, 1H), 7.16-7.38 (m, 3H), 5.18 (s, 1H), 2.35 (s, 3H). ¹³C NMR DMSO-d₆, 125 MHz) δ 174.229, 138.623, 135.649, 130.129, 127.491, 126.990, 125.698, 125.698, 69.733, 18.899. MS calc'd for [C₉H₁₀O₃] 166.1, found 165.2. *ee* = 91 % [HPLC]. 86 % yield. [α]²⁰₅₉₈ = -164.4 (c = 0.5, ethanol).

**(-)-3-chloromandelic acid** ¹H NMR (DMSO-d₆, 500 MHz) δ 7.46 (s, 1H), 7.36 (m, 3H), 5.07 (s, 1H). ¹³C NMR (DMSO, 298K, 125MHz) δ 173.554, 142.685, 132.813, 130.069, 127.568, 126.355, 125.289,71.659. MS calc'd for [C₈H₇ClO₃] 186.0, found 185.34 (MALDI TOF -). *ee* = 98 % [HPLC]. 70 % yield. [α]²⁰₅₉₈ = -120.4 8 (c= 0.5, methanol).

**(-)-3-bromomandelic acid** ¹H NMR (DMSO-d₆, 500 MHz) δ 7.60 (s, 1H), 7.49 (m, 1H), 7.42 (m, 1H), 7.31 (m, 1H), 5.06 (s, 1H). ¹³C NMR (DMSO, 298K, 125MHz) δ 173.551, 142.917, 130.468, 130.379, 129.237, 125.687, 121.404, 71.605. MS calc'd for [C₈H₇BrO₃] 229.98, found 229.1 (LC-MS). *ee* = 98 % [HPLC]. 82 % yield. [α]²⁰₅₉₈ = -84.8 (c= 0.5, ethanol).

**(-)-4-fluoromandelic acid** ¹H NMR (DMSO, 298K, 500MHz) δ 12.65 (s, 1H), 7.44 (m, 2H), 7.17 (m, 2H), 5.91 (s, 1H), 5.03 (s, 1H) ¹³C NMR (DMSO, 298K, 125MHz) δ 173.93, 162.57, 136.47, 128.61, 128.55, 114.96, 114.80, 71.61. MS calc'd for [C₈H₇FO₃] 170.0, found 168.8. *ee* = 99% [HPLC]. 81% yield. [α]²⁰₅₉₈ = -152.8 (c= 0.5, methanol).

**(-)-1-naphthylglycolic acid** ¹H NMR (DMSO-d₆, 500 MHz) δ 8.28-8.26 (m, 1H), 7.87-7.93 (m, 2H), 7.47-7.58 (m, 4H), 5.66 (s, 1H). ¹³C NMR DMSO-d₆, 125 MHz) δ 174.288, 136.284, 133.423, 130.654, 128.353, 128.192, 125.926, 125.694, 125.613, 125.266, 124.558, 70.940. MS calc'd for [C₁₂H₁₀O₃]: 202.21 found 201.37 (MALDI TOF -). *ee* = 95% [HPLC]. 90 % yield [α]²⁰₅₉₈ = -115.4 (c = 0.5, ethanol).

**(-)-2-naphthylglycolic acid** ¹H NMR (DMSO-d₆, 500 MHz) δ 12.6 (bm, 1H), 7.88-7.93 (m, 4H), 7.48-7.56 (m, 3H), 5.20 (s, 1H). ¹³C NMR DMSO-d₆, 125 MHz) δ 174.005,137.760,132.644,132.498, 127.811, 127.658, 127.506, 127.209, 125.993, 125.334, 124.761, 72.472. MS calc'd for [C₁₂H₁₀O₃] 202.21, found 201.37 (MALDI TOF). *ee* = 98% [HPLC]. 68% yield. [α]²⁰₅₉₈ = -15.4 (c = 0.5, ethanol).

**(-)-3-pyridylglycolic acid** This Reaction was performed in 100 mM ammonium formate buffer at pH 8. To isolate the product, the reaction mixture was filtered through a 10,000 MWCO membrane to remove enzyme and then concentrated *in vacuo*. ¹H NMR (DMSO-d₆, 500 MHz) δ 8.56 (s, 1H), 8.36 (d, *J* = 4.57 Hz, 1H), 8.25 (s, 1H), 7.71 (m, 1H), 7.25 (dd, *J* = 4.98, 4.80 Hz 1H), 5.45 (s, 1H). ¹³C NMR DMSO-d₆, 125 MHz) δ 165.911, 147.862, 147.251, 139.118, 133.381, 122.746, 71.508. MS calc'd for [C₇H₇NO₃] 153.04, found 154.0 ((MALDI TOF). *ee* = 92% [HPLC], 84% yield, [α]²⁰₅₉₈ = -65.2 (c = 0.5, H₂O).

**(-)-3-thienylglycolic acid** ¹H NMR (DMSO-d₆, 500 MHz) δ 7.48 (m, 1H), 7.45 (d, *J*= 2.81, 1H,), 7.10 (m, 1H), 5.09 (s, 1H), 3.33 (s, 1H) ¹³C NMR (DMSO, 298K, 125MHz) δ 173.704, 141.109, 126.446, 126.042, 122.247, 68.915 MS calc'd for [C₆H₆O₃S] 158.00, found 157.224 (MALDI TOF). *ee* = 95 % [HPLC]. 70 % yield. [α]²⁰₅₉₈ = -123.2 8 (*c*= 0.5, methanol).

**(S)-(-)-phenyllactic acid** ¹H NMR (DMSO-d₆, 500 MHz) δ 7.28(m, 5H), 4.17(dd, *J* = 4.5 Hz, *J* = 8.3 Hz, 1H), 2.98(dd, *J* = 4.5 Hz, *J* = 13.7 Hz, 1H), 2.79 (dd, *J* = 8.3 Hz, *J* = 13.7 Hz, 1H). ¹³C NMR (DMSO, 298K, 125MHz) δ 178.16, 133.4, 129.27, 128.6, 127.3, 70.45, 44.12. *ee* = 97 % [HPLC], 84 % yield. [α]²⁰₅₉₈ = -17.8 (c= 0.5, methanol).

**(-)-2-methylphenyllactic acid** ¹H NMR (DMSO-d₆, 500 MHz) δ 7.16 (m, 4H), 4.47 (dd, *J* = 3.9 Hz, *J* = 8.8 Hz, 1H), 3.25(dd, *J* = 3.9Hz, 14.3 Hz, 1H), 2.94 (dd, *J* = 8.8 Hz, *J* = 14.3Hz), 2.35(s, 3H). ¹³C NMR (DMSO, 298K, 125MHz) δ 178.61, 137.08, 134.74, 130.80, 130.25, 127.44, 126.34, 70.93, 37.67, 19.79. MS calc'd [C₁₀H₁₂O₃] 180.08, found 180.0 (ESI +). 86 % yield. *ee* = 95 % [HPLC]. [α]²⁰₅₉₈ = -13.2 (c= 0.5, methanol).

**(-)-2-bromophenyllactic acid** ¹H NMR (DMSO-d₆, 500 MHz) δ 7.28 (m, 4H), 4.60(dd, *J* = 4.0 Hz, *J* = 9.1 Hz, 1H), 3.45(dd, *J* = 4.0 Hz, *J* = 14.1 Hz, 1H), 3.04(dd, *J* = 8.0 Hz, *J* = 14.1 Hz, 1H). ¹³C NMR (DMSO, 298K, 125MHz) δ 178.70, 136.05, 133.21, 132.10, 128.99, 127.72, 125.0, 70.04, 40.76. MS calc'd for [C₉H₉BrO₃] 243.9, found 243.3 (ESI +). 91 % yield. *ee* = 93 % [HPLC], [α] ²⁰₅₉₈ = -17.6 (c= 0.5, methanol)

**(-)-2-fluorophenyllactic acid** ¹H NMR (DMSO-d₆, 500 MHz) δ 7.10 (m, 4H), 4.64 (t, *J* = 6.8 Hz, 1H), 3.11(d, *J* = 6.8 Hz, 2H). ¹³C NMR (DMSO, 298K, 125MHz) δ 132.18, 131.52, 129.66, 129.03, 128.07, 124.05, 115.8, 63.02, 44.79. MS calc'd for [C₉H₈FNO]: 165.06, found 164.2 (ESI +). 91 % yield. *ee* = 88 % [HPLC]. [α]²⁰₅₉₈ = -14.0 (c= 0.5, methanol).

**(-)-3-methylphenyllactic acid** ¹H NMR (DMSO-d₆, 500 MHz) δ 7.18 (m, 1H), 7.02 (m, 3H), 4.54 (dd, *J* = 4.6 Hz, *J* = 8.0 Hz, 1H), 3.06(dd, *J* = 4.54 Hz, *J* = 14.4 Hz, 1H), 2.83(dd, *J* = 8.0 Hz, *J* = 14.4 Hz, 1H), 2.36 (s, 3H). ¹³C NMR (DMSO, 298K, 125MHz) δ 175.88, 163.80, 130.33, 130.09, 125.7, 116.68, 113.75, 71.31, 34.28. MS calc'd for [C₁₀H₁₁NO] 161.08, found 162.2 (ESI +). 80 % yield. *ee* = 98 % [HPLC]. [α]²⁰₅₉₈ = -2.4 (c= 0.5, methanol).

**(-)-3-fluorophenyllactic acid** ¹H NMR (DMSO-d₆, 500 MHz) δ 7.2 (m, 1H), 6.9 (m, 3H), 4.56 (dd, 4.5 Hz, *J* = 7.9 Hz, 1H), 3.09(dd, *J* = 4.5 Hz, *J* = 14.1 Hz, 1H), 2.86 (dd, *J* = 7.9 Hz, *J* = 14.1 Hz, 1H). ¹³C NMR (DMSO, 298K, 125MHz) δ 175.88, 163.80, 130.33, 130.09, 125.7, 116.68, 113.75, 71.31, 34.28. MS calc'd for [C₉H₉O₃F] 184.05, found 184.1 (ESI +). 82 % yield. *ee* = 97 % [HPLC]. [α]²⁰₅₉₈ =-5.2 (c= 0.5, methanol).

**(-)-1-napthyllactic acid** ¹H NMR (DMSO-d₆, 500 MHz) δ 8.57 (m, 1H), 8.21(m, 1H), 8.08 (m, 1H), 7.61 (m, 4H), 4.64 (dd, 3.5 Hz, 8.5 Hz, 1H), 3.84 (dd, *J* = 3.5 Hz, *J* = 14.5 Hz, 1H), 3.38 (dd, *J* = 8.5 Hz, *J* = 14.5 Hz, 1H) ¹³C NMR (DMSO, 298K, 125MHz) δ 177.7, 140.31, 129.74, 129.24, 128.92, 128.26, 127.84, 125.63, 124.53, 124.05, 123.42, 70.58, 38.0. MS calc'd for [C₁₃H₁₁NO] 197.08, found 197.1(ESI +). 87 % yield. *ee* = 94 % [HPLC]. [α]²⁰₅₉₈ = -16.2 (c= 0.5, methanol).

**(-)-2-pyridyllactic acid** ¹H NMR (DMSO-d₆, 500 MHz) δ 8.49 (m, 1H), 7.62 (m, 1H), 7.21 (m, 2H), 4.50 (t, *J* = 5.0 Hz, 1H), 3.01 (d, *J* = 5.0 Hz, 2H). ¹³C NMR (DMSO, 298K, 125 MHz) δ 178.8, 159.79, 148.84, 136.89, 124.35, 121.75, 71.14, 44.09. MS calc'd for [C₈H₉NO3]: 167.06, found 167.0. (ESI +). 62 % yield. *ee* = 94 % [HPLC], [α]²⁰₅₉₈ = -3.6 (c= 0.5, methanol).

**(-)-3-pyridyllactic acid** ¹H NMR (DMSO-d₆, 500 MHz) δ 8.43(m, 2H), 7.62(m, 1H), 7.28(m, 1H), 4.57(t, 5.37Hz, 1H), 2.85(d, 5.37Hz, 2H). ¹³C NMR (DMSO, 298K, 125 MHz) δ 176.6, 150.03, 147.12, 136.41, 129.45, 123.26, 61.56, 31.46 MS calc'd for [C₈H₉NO₃] 167.06, found 167.0 (ESI +). 59 % yield. *ee* = 94 % [HPLC]. [α]²⁰₅₉₈ = -4.0 (c = 0.5, methanol).

**(-)-2-thienyllactic acid** ¹H NMR (DMSO-d₆, 500 MHz) δ 7.18(m, 1H), 6.94(m, 1H), 6.90 (m, 1H), 4.49 (dd, *J* = 4.1 Hz, *J* = 6.25 Hz, 1H), 3.36 (dd, *J* = 4.1 Hz, *J* = 15.0 Hz, 1H), 3.26(dd, *J* = 6.25 Hz, *J* = 15.0 Hz, 1H). ¹³C NMR (DMSO, 298K, 125MHz) δ 127.68, 127.41, 125.58, 124.60, 118.70, 63.25, 44.84. MS calc'd for [C₇H₇NOS] 153.02, found 153.0 (ESI +). 85 % yield. *ee* = 95 % [HPLC]. [α]²⁰₅₉₈ = - 13.0 (c= 0.5, methanol).

**(-)-3-thienyllactic acid** ¹H NMR (DMSO-d₆, 500 MHz) δ 7.30(m, 1H), 7.13(m, 1H), 7.01(m, 1H), 4.50 (dd, *J* = 4.25 Hz, *J* = 6.5 Hz, 1H), 3.21(dd, *J* = 4.25 Hz, *J* = 15.0 Hz, 1H), 3.10 (dd, *J* = 6.5 Hz, *J* = 15.0 Hz, 1H). ¹³C NMR (DMSO, 298K, 125MHz) δ 127.50, 136.09, 128.83, 126.24, 123.32, 70.65, 34.84. MS calc'd for [C₇H₈O₃S] 172.02, found 172.1 (ESI +). 81 % yield. *ee* = 96 % [HPLC]. [α]²⁰₅₉₈ = - 18.8 (c= 0.5, methanol).

### Enzymic Hydrolysis of 3-Hydroxyglutarylnitrile:

3-Hydroxyglutarylnitrile (1.0 g, 9.0 mmol, 240 mM) was suspended in N₂ (g) sparged sodium phosphate buffer (37.5 mL, pH 7, 100 mM) at room temperature. Cell lysate (30 mg, normalized for nitrilase content) was added to bring the concentration to 0.8 mg/ml enzyme and the reaction was at shaken at 100 rpm, room temperature. Reaction progress was monitored by TLC (1:1 EtOAc:Hexanes, R_{f}=0.32, nitrile; R_{f}=0.0, acid) After 22 h, the reaction was acidified with 1M HCl. The reaction mixture was continuously extracted with diethyl ether. The acid product was isolated as a yellow oil (1.15 g, 98 % yield). ¹H NMR (DMSO, 298K, 500MHz) δ 12.32 (s, 1H), 5.52 (s, 1H), 4.10(m, 1H), 2.70 (dd, 1H, J = 16.8, 4.1 Hz), 2.61 (dd, 1H, J = 16.9, 6.3 Hz), 2.44 (dd, 1H, J = 15.4, 5.3 Hz), 2.37 (dd, 1H, J = 15.6, 7.8 Hz). ¹³C NMR (DMSO, 298K, 125 MHz) δ 171.9, 118.7, 63.4, 41.2, 25.2 MS calc'd for [C₅H₇NO₃]: 129.0, found 130.0 [M+H⁺], (ESI +).

### Preparation of (R)-(-)-Methyl (3-O-[benzoyl]-4-cyano)-butanoate

Benzoyl chloride (0.068 ml, 0.752 mmol) was added to a stirred solution of (*R*)-methyl-(3-hydroxy-4-cyano)-butanoate (71.7 mg, 0.501mmol) in pyridine (2.0 ml), at room temperature. After 19 hours, add an additional 0.5 equivalent of benzoyl chloride (0.023ml, 0.251mmol). Reaction was complete at 23 h, as determined by TLC. Add 1ml H₂O, extract with ether (3 x 10ml). Wash with brine (2 x 10ml). Dry combined aqueous extracts with MgSO₄. Filter off drying agent and remove solvent by rotary evaporation. Purify by column chromatography (hexane:ethyl acetate [2:1]. Rotary evaporation of fractions yielded the product as a yellow oil (46 mg, 0.186 mmol, 37%). ¹H NMR (DMSO, 298K, 500MHz) δ 7.96 (d, 2H, J = 7.8), 7.70 (t, 1H, J = 7.25), 7.56 (t, 2H, J = 7.8), 5.55 (m, 1H), 3.59 (s, 3H), 3.13 (m, 2H), 2.90 (m, 2H). ¹³C NMR (DMSO, 298K, 125MHz) δ 169.6, 164.5, 133.8, 129.3, 128.9, 128.5, 117.3, 66.0, 51.8, 37.5, 22.2 MS calc'd for [C₁₃H₁₃NO₄]: 247.25, found 270.3 [M+Na⁺] *ee* = 95% [HPLC]. [α]²⁰ ₅₉₈ -32.4 (c = 0.5, CHCl₃).

### Synthesis of (R)-Ethyl-(3-hydroxy-4cyano)-butanoate

A 0.2 M solution of (*R*)-3-hydroxy-4-cyano-butanoic acid (50 mg, 0.387 mmol) in anhydrous ethanol (1.94 mL) was prepared. The ethanol solution was added dropwise to 1.0 ml of a 50:50 (v/v) mixture of anhydrous 1 M HCl ethereal solution and anhydrous ethanol over sieves. The reaction was stirred overnight at room temperature under N₂ (g) atmosphere. The reaction was monitored by TLC, (1:1 EtOAc:Hexanes, R_{f} = 0.45, ester; R_{f} = 0.0, acid, stained with p-anisaldehyde). After 30 hrs, solvent was removed by rotary evaporation. The crude product was taken up in 25 mL ether, washed with 5 mL saturated bicarbonate and then 5 mL brine. The organic extract was dried over MgSO₄, filtered and then concentrated *in vacuo,* yielding the product as a clear oil. ¹H NMR (DMSO, 298K, 500MHz) δ 5.60 (d, 1H, *J* = 5.58 Hz), 4.12 (m, 1H), 4.07 (q, 2H, *J* = 7.1), 2.66 (m, 2H), 2.47 (m, 2H), 1.87 (t, 3H, *J* = 7.0). ¹³C NMR (DMSO, 298K, 125 MHz) δ 170.21, 118.60, 63.40, 59.98, 41.10, 25.14, 14.02. MS calc'd for [C₇H₁₁NO₃]: 157.1, found 158.2. [M+H⁺]

### Example 13 - Optimization Of Nitrilases For The Enantioselective Production Of (R)-2-Chloromandelic Acid

Chloromandelic acid has the structure:

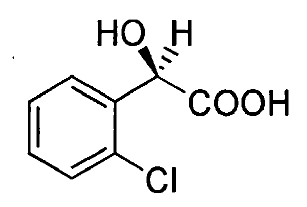

Nitrilases were identified which selectively produced *(R)*-2-chloromandelic acid from *(R,S)*-2-chloromandelonitrile. Nitrilases were identified which were useful to improve the enantioselectivity of the enzymes and establishing the effects of process conditions on the enzymes. An examination of the reaction conditions for the enzymatic nitrile hydrolysis was carried out in order to improve the enantiomeric excess of the product. Additionally, further investigation into the effects of process conditions on the enzyme was performed.

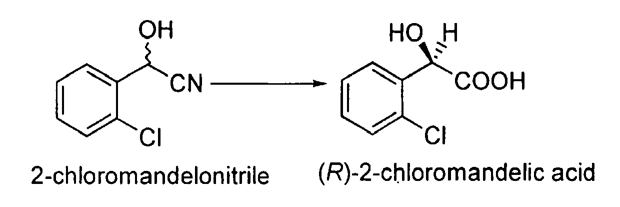

In this embodiment, the enantioselective production of *(R)*-2-chloromandelic acid was the target. One enzyme, SEQ ID NOS:385, 386, was selected for further confirmation of its enantioselectivity on 2-chloromandelonitrile. SEQ ID NOS:385, 386 was shown to be stable to process components, with a half-life of 8 hours. The enzyme was inhibited by 2-chlorobenzaldehyde and a contaminant in the cyanohydrin substrate, 2-chlorobenzoic acid. The enzymatic reaction was scaled up to a substrate concentration of 45 mM 2-chloromandelonitrile. Over 90% conversion was obtained, with *ee* of 97%. The chiral HPLC method was improved, to remove a contaminating peak that was present in the substrate. Improved accuracy in the determination of enantioselectivity was obtained using this method.

Nitrilases were screened against 2-chloromandelonitrile, with 31 nitrilases exhibiting activity on this substrate. High enantioselectivities were shown by 9 enzymes. The optimization of 5 of these enzymes was undertaken and one of them was identified as a candidate for the next stage of development.

In an effort to improve the enantioselectivity of the selected enzymes for *(R)*-2-chloromandelic acid, a number of factors that are known to affect this property, together with the activity of the enzymes, were investigated. These included pH, temperature, buffer strength and addition of solvents to the reaction. Initially, 5 nitrilases were selected for these studies, based on the high enantioselectivities obtained by these enzymes. These enzymes were: SEQ ID NOS:385, 386, SEQ ID NOS:197, 198, SEQ ID NOS:217, 218, SEQ ID NOS:55, 56, and SEQ ID NOS:167, 168.

### Effect of pH

The enzymatic reactions were run at a range of pH values, from pH 5 to pH 9. An increase in both activity and enantioselectivity with increasing pH was observed for all of the enzymes. With the exception of SEQ ID NOS:385, 386, pH 9 (0.1 M Tris-HCl buffer) was determined as the optimum for activity and enantioselectivity. The optimum pH for SEQ ID NOS:385, 386 was pH 8 (0.1 M sodium phosphate buffer).

### Effect of temperature

The enzymes exhibited similar temperature profiles, with the highest activities being measured at 37°C and 45°C. Although the latter temperature resulted in higher conversions, the enantioselectivity of most of the enzymes showed a clear preference for the lower temperatures, with ee values being 10-20% lower when the temperature was raised above 37°C. In the case of SEQ ID NOS:385, 386 a slight optimum for enantioselectivity was evident at 37°C. Therefore, this temperature was established as the optimum for hydrolysis of 2-chloromandelonitrile by these enzymes.

### Effect of enzyme concentration

During the concurrent investigation into the enantioselective hydrolysis of phenylacetaldehyde cyanohydrin to L-phenyllactic acid, the concentration of the enzyme in the reaction was found to have a significant effect on the enantioselectivity of the reaction. This provided an indication that the enzymatic hydrolysis rate was faster than the rate of racemization of the remaining cyanohydrin in the reaction. On this basis, the effect of enzyme concentration on the enantioselectivity of the enzymes towards *(R)*-2-chloromandelonitrile was investigated. Enzymatic reactions were performed with the standard concentration of enzyme (0.6 mg protein/ml), half the standard concentration and one-tenth of the standard concentration.

The following Table indicates the highest conversions achieved for the reactions, with the corresponding ee. With the exception of SEQ ID NOS:385, 386, it appears that very little, if any, increased enantioselectivity is observed. Therefore, it appears that the rate of racemization of the remaining chloromandelonitrile is not a limiting factor to obtaining higher enantioselectivities.
Effect of enzyme concentration on the activity and enantioselectivity of nitrilases for the production of *(R)*-2-chloromandelic acid.

| | **Enzyme conc (mg protein/ml)** | **Conversion to product (%)** | **Time for highest conversion (h)** | **ee (%)** |
|---|---|---|---|---|
| SEO ID NOS:385, 386 | 0.06 | 67 | 3 | 92 |
| | 0.3 | 80 | 1 | 82 |
| | 0.6 | 81 | 1 | 82 |
| SEQ ID NOS: 197, 198 | 0.06 | 3 | 5 | 100 |
| | 0.3 | 46 | 4 | 88 |
| | 0.6 | 75 | 3 | 86 |
| SEQ ID NOS:189, 190 | 0.06 | 82 | 4 | 88 |
| | 0.3 | 80 | 1 | 86 |
| | 0.6 | 76 | 0.5 | 82 |
| SEQ ID NOS:217, 218 | 0.06 | 22 | 5 | ND |
| | 0.3 | 73 | 4 | 82 |
| | 0.6 | 72 | 1.5 | 81 |
| SEQ ID NOS:55, 56 | 0.06 | 81 | 5 | 84 |
| | 0.3 | 73 | 2 | 79 |
| | 0.6 | 67 | 0.5 | 76 |
| SEQ ID NOS:167, 168 | 0.06 | 61 | 2 | 81 |
| | 0.3 | 74 | 1.5 | 80 |
| | 0.6 | 74 | 2 | 78 |

### Investigation of other positive enzymes

In addition to the enzymes in the above Table, a number of other nitrilases were screened for their enantioselectivities on 2-chloromandelonitrile. Some of these enzymes were newly discovered enzymes. Some were reinvestigated under conditions that have since been found to be optimal for these enzymes (pH 8 and 37°C). The results of this screening are shown below in the Table.

### Summary of enzymes screened for activity and enantioselectivity on 2-chloromandelonitrile

| **Enzyme** | **Conversion to product (%)** | **Time for highest conversion (h)** | **ee (%)** |
|---|---|---|---|
| SEQ ID NOS:383, 384 | 61 | 6 | 78 |
| SEQ ID NOS:101, 102 | 58 | 3 | 53 |
| SEQ ID NOS:97, 98 | 46 | 3 | 87 |
| SEQ ID NOS:13, 14 | 70 | 3 | 71 |
| SEQ ID NOS:5, 6 | >95 | 3 | 67 |
| SEQ ID NOS:85, 86 | 50 | 4 | 52 |
| SEQ ID NOS:279, 280 | >95 | 1 | 37 |
| SEQ ID NOS:33, 34 | >95 | 3 | 60 |
| SEQ ID NOS:261, 262 | >95 | 3 | 70 |

### Effect of co-solvent concentration

The addition of methanol as a cosolvent in the enzymatic reactions was shown to enhance the ee. In order to establish the lowest level of methanol that could be added to the reactions, the enzyme reactions were performed at varying concentrations of methanol, ranging from 0-20% (v/v). No significant differences in enantioselectivity were evident between the various methanol concentrations. However, the ee in these reactions was 97-98%, while that of the control reaction, with no added methanol was 95-96%. While this difference in ee is small, the effect of the methanol was shown in more than one set of experiments during the course of this investigation and is therefore regarded as significant.

### Effect of reaction components on activity of SEQ ID NOS:385, 386

A vital part of an investigation into process optimization of an enzyme involves the determination of the effects of any compounds which could be present in the enzymatic reaction. For SEQ ID NOS:385, 386, these components were established as the starting material and equilibrium product of the cyanohydrin, 2-chlorobenzaldehyde; the product, 2-chloromandelic acid and the contaminant detected in the substrate, 2-chlorobenzoic acid. The addition of cyanide to the reaction was found to have no effect on the enzyme activity. The presence of trace amounts of triethylamine was also found to be tolerable to the enzyme.

The effect of the various reaction components on the activity of SEQ ID NOS:385, 386 was assessed by addition of various levels of possible inhibitors to the enzyme reaction. From these experiments, it appeared that both the aldehyde and its oxidation product, 2-chlorobenzoic acid were detrimental to enzyme activity. Approximately 70% and 40% of the activity of SEQ ID NOS:385, 386 was lost upon addition of 5 mM 2-chlorobenzaldehyde or 5 mM 2-chlorobenzoic acid to the reaction, respectively.

### Scale-up hydrolysis of 2-chloromandelonitrile

In order to confirm the conversion and enantioselectivity obtained by SEQ ID NOS:385, 386 for the production of (R)-2-chloromandelic acid, a larger scale reaction was performed and the product isolated from the aqueous mixture. The reaction was performed in a 20 ml reaction volume, with a substrate concentration of 45 mM 2-chloromandelonitrile. Complete conversion of the cyanohydrin was obtained, with 30 mM product formed. The ee of the product was 97% and the specific activity of the enzyme was 0.13 mmol product/mg nitrilase/h.

It is evident from this experiment, together with the other experiments performed, that the formation of product does not account for the complete loss of substrate. In all experiments, a nitrile-containing control sample was run, in order to determine the extent of breakdown of the cyanohydrin. Overall, it appears that approximately 50% of the substrate is lost over a period of 4 hours at 37°C. It is expected that this breakdown would be to its equilibrium products, cyanide and 2-chlorobenzaldehyde, which could undergo further oxidation. A larger scale reaction was also run at a substrate concentration of 90 mM 2-chloromandelonitrile. However, no product was detected in this reaction. At higher substrate concentrations, it is expected that the concentration of the equilibrium product, 2-chlorobenzaldehyde and the contaminant, 2-chlorobenzoic acid will be present in higher amounts. Based on the results above, it is possible that the enzyme will be completely inhibited under such conditions.

### Reactions under biphasic conditions

The use of biphasic systems can facilitate product recovery following the enzymatic reaction step. These systems can be also be used for the removal of products or by-products which are inhibitory to the enzyme. The nitrilases were shown to be active under biphasic conditions using a variety of solvents. Following the low conversions obtained at the higher substrate concentration above, further investigation of a biphasic system was performed with the hit enzyme, SEQ ID NOS:385, 386. It was important to ascertain whether any inhibitory factors could be removed by the solvent phase and whether any process advantages could be gained by the use of a biphasic system.

Promising results were obtained with hexane as the organic phase. Therefore, further investigations involved the use of this solvent at two different levels: 100% and 70% of the volume of the aqueous phase, with increasing substrate concentrations, up to 90 mM. The substrate was dissolved in the organic phase. The level of hexane did not appear to affect the level of product formation, particularly at the higher concentrations of 2-chloromandelonitrile.

Once again, high conversion was observed in a biphasic system, with a 76% yield of product being observed after 5 hours. The rate of product formation appeared to be slightly lower than in the corresponding monophasic system, where the reaction is complete within I hour. Lower enantioselectivity was observed in the biphasic system. Some possibilities which may account for these results are (i) the mass transfer rate is lower than the rate of enzyme activity or (ii) the non-polar solvent directly affects the enzyme.

At a higher substrate concentration, a very low conversion was observed, with 7 mM 2-chloromandelic acid being formed from 90 mM 2-chloromandelonitrile. This level of conversion, albeit low, was higher than that observed in the monophasic system with the same substrate concentration. These results suggest that some of the inhibitory 2-chlorobenzaldehyde or 2-chlorobenzoic acid is retained in the non-polar organic solvent.

### Standard assay conditions:

The following solutions were prepared:
- Substrate stock solution: 50 mM of the cyanohydrin substrate in 0.1 M phosphate buffer (pH 8).
- Enzyme stock solution: 3.33 ml of 0.1 M phosphate buffer (pH 8) to each vial of 20 mg of lyophilized cell lysate (final concentration 6 mg protein/ml)

The reaction volumes varied between the different experiments, depending on the number of time points taken. Unless otherwise noted, all reactions consisted of 25 mM 2-chloromandelonitrile and 10% (v/v) of the enzyme stock solution (final concentration 0.6 mg protein/ml). The reactions were run at 37°C, unless otherwise stated. Controls to monitor the nitrile degradation were run with every experiment. These consisted of 25 mM 2-chloromandelonitrile in 0.1 M phosphate buffer (pH 8).

Sampling of reactions: The reactions were sampled by removing an aliquot from each reaction and diluting these samples by a factor of 8. Duplicate samples were taken for analysis by chiral and achiral HPLC methods. The reactions were sampled at 0.5, 1, 1.5, 2, 3, and 4 hours, unless otherwise shown in the figures above.

### HPLC methods

The achiral HPLC method was run on a SYNERGI-RP™ column (4 µm; 50 x 2 mm) with a mobile phase of 10 mM Na phosphate buffer (pH 2.5). A gradient of methanol was introduced at 3.5 min and increased to 50% over 1.5 min, following which the methanol was decreased to 0%. Elution times for 2-chloromandelic acid and 2-chloromandelonitrile were 2.5 and 6.1 minutes, with another peak appearing with the nitrile at 5.9 minutes.

As described above, the chiral HPLC method was optimized during the course of the investigation, to improve the separation between 2-chlorobenzoic acid and (S)-2-chloromandelic acid. The optimized method was used during the latter half of the investigation and was run on a CHIROBIOTIC-R™ column. The mobile phase was 80% Acetonitrile:20% of 0.5% (v/v) acetic acid. Elution times for (S)-2-chloromandelic acid and (R)-2-chloromandelic acid were 2.4 and 3.5 minutes respectively. A peak for 2-chlorobenzoic acid eluted at 1.9 minutes. For each experiment, a standard curve of the product was included in the HPLC run. The concentration of product in the samples was calculated from the slope of these curves.

### Effect of pH

The effect of pH on the enzyme activity and enantioselectivity was studied by performance of the standard assay in a range of different buffers: 0.1 M Citrate Phosphate pH 5; 0.1 M Citrate Phosphate pH 6; 0.1 M Sodium Phosphate pH 6; 0.1 M Sodium Phosphate pH 7; 0.1 M Sodium Phosphate pH 8; 0.1 M Tris-HCl pH 8; and 0.1 M Tris-HCl pH 9. The standard enzyme concentration was used for all enzymes, with the exception of SEQ ID NOS:385, 386, where half the standard concentration was used (5% v/v of the enzyme stock solution).

### Effect of temperature

The effect of temperature on the activity and enantioselectivity was investigated by performing the standard assay at a range of different temperatures: room temperature, 37°C, 45°C, 50°C and 60°C. The standard enzyme concentration was used for all enzymes, with the exception of SEQ ID NOS:385, 386, where half the standard concentration was used (5% v/v of the enzyme stock solution).

### Effect of enzyme concentration

Reactions were run under standard conditions, with varying enzyme concentrations: 1%, 5% and 10% (v/v) of the enzyme stock solution. The reaction volume was normalized with the appropriate buffer.

### Addition of solvents

The enzyme reactions were performed in the presence of methanol as a cosolvent. Methanol was added to the standard reaction mixture at the following levels: 0, 5, 10, 15 and 20% (v/v).

Biphasic reactions with hexane were also investigated. The aqueous phase contained 10% (v/v) of the enzyme stock solution in 0.1 M phosphate buffer (pH 8). The cyanohydrin was dissolved in the hexane, prior to addition to the reaction. Two levels of organic phase were used: I equivalent and 0.7 equivalents of the aqueous phase volume. In addition, a range of nitrile concentrations was investigated: 25, 45 and 90 mM. These reactions were run at room temperature.

Samples from these reactions were taken both from the aqueous and the solvent phase. The hexane was evaporated by centrifugation under vacuum and redissolved in a 50:50 mixture of methanol and water, so that the samples were at the same dilution as the aqueous samples. Analysis of the samples was performed by non-chiral and chiral HPLC.

### Effect of process components

(i) Activity: The effect of the process components on the activity of the enzymes was established by addition of the individual components, 2-chlorobenzaldehyde, 2-chlorobenzoic acid or 2-chloromandelic acid, to the enzymatic reaction. The enzymatic reactions were carried out under standard conditions, in the presence of one of the 2 possible inhibitors as follows: 5, 10, 20 and 25 mM 2-chlorobenzaldehyde; 1.5 and 5 mM 2-chlorobenzoic acid; and 10, 20, 40 and 80 mM 2-chloromandelic acid. Control reactions were performed under standard conditions, with no additive. At each of the sampling times, the samples were diluted to a level of 1 in 10. Control samples containing the reaction components without enzyme were used and diluted to the same level. The samples were analysed by non-chiral HPLC.
(ii) Stability: The stability of the enzymes to process conditions was monitored by incubation of the enzymes in the presence of the reaction components, 2-chlorobenzaldehyde and 2-chloromandelic acid for predetermined time periods, prior to assay of the enzyme activity under standard conditions. In these experiments, the enzymes were incubated at a concentration of 3 mg protein/ml in the presence of each of the following reaction components: 5, 10, 20 and 25 mM 2-chlorobenzaldehyde; and 10, 20, 40 and 80 mM 2-chloromandelic acid. Control reactions were performed by incubation of the enzyme in buffer only.

Assay conditions: At 0, 4, 8 and 24 hours of incubation in the particular additive, 20 µl of the enzyme solution was removed and added to 60 µl of a 41.6 mM substrate stock solution and 20 µl buffer. The enzyme activity was thus assayed under standard conditions. The reactions were sampled 90 minutes after substrate addition and analyzed using the non-chiral HPLC method.

### Scale-up of enzymatic reaction

The enzymatic reactions were run at two difference concentrations: 45 mM and 90 mM substrate. The reactions were run under standard conditions, i.e. pH 8 (0.1 M sodium phosphate buffer), 37°C and 10% (v/v) of the enzyme stock solution. The substrate was dissolved in 10% (v/v/) methanol prior to addition of the buffer. The final reaction volume was 20 ml and the reactions were performed with magnetic stirring.

### Example 14 - Optimization Of Nitrilases For The Enantioselective Production Of L-2-amino-6,6-dimethoxyhexanoic acid

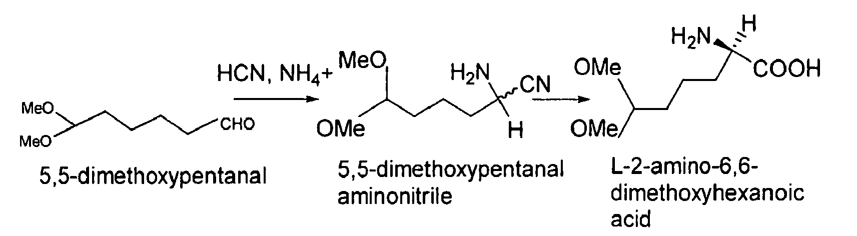

Four of the isolated enzymes were shown to hydrolyze 2-amino-6-hydroxy hexanenitrile to (S)-2-amino-6-hydroxy hexanoic acid, with selectivity towards the L-enantiomer. A new target, with a similar structure to (S)-2-amino-6-hydroxy hexanoic acid was identified. A panel of the isolated nitrilases are screened against the target, 5,5-dimethoxypentanal aminonitrile. The positive enzymes are characterized on this substrate. Laboratory evolution techniques can be used to optimize these nitrilases for improved enantiospecificity towards the specified target. A primary screen is used to identify putative up-mutants, which is confirmed using HPLC.

Optimization of enzymes: GSSM™ and GeneReassembly™ can be performed on selected nitrilases, in order to improve the enantioselectivity and activity of the enzymes for the production of L-2-amino-6,6-dimethoxyhexanoic acid. Four enzymes were identified that can hydrolyze enantioselectively 2-amino-6-hydroxy hexanenitrile to L-(S)-2-amino-6-hydroxy hexanoic acid. However, a slight structural difference is present in the new target molecule, L-2-amino-6,6-dimethoxyhexanoic acid. In order to determine whether this difference affects the activity and enantioselectivity of the enzymes, the complete spectrum of nitrilases is screened against the new target.

An enzyme exhibiting the highest combination of activity and enantioselectivity for the production of L-2-amino-6,6-dimethoxyhexanoic is selected for GSSM™. Following the mutation of the target enzyme, the resulting mutants will be screened on 5,5-dimethoxypentanal aminonitrile, using high throughput screening technology. Following confirmation of the up-mutants by HPLC analysis, the individual up-mutants will be combined in order to further enhance the properties of the mutant enzymes.

In parallel to GSSM™, a GeneReassembly™ can be performed on a combination of parent enzymes, at least one of which can be selected for activity and enantioselectivity on L-2-amino-6,6-dimethoxyhexanoic acid. At least two other nitrilases, with a high degree of homology, can be reassembled with the former enzyme(s); these enzymes will be selected in order to provide diversity to the reassembled sequences.

Crucial to the success of this evolution effort is the development of a high throughput assay for enantioselectivity. Such an assay is a novel enzyme-based enantioselectivity assay that allows for the screening of >30,000 mutants in a significantly shorter time period than the traditionally used method of HPLC.

In one aspect, a non-stochastic method, termed synthetic ligation reassembly, that is related to stochastic shuffling, except that the nucleic acid building blocks are not shuffled or concatenated or chimerized randomly, but rather are assembled non-stochastically, can be used to create variants. This method does not require the presence of high homology between nucleic acids to be shuffled. The ligation reassembly method can be used to non-stochastically generate libraries (or sets) of progeny molecules having at least 10¹⁰⁰ or at least 10¹⁰⁰⁰ different chimeras. The ligation reassembly method provides a non-stochastic method of producing a set of finalized chimeric nucleic acids that have an overall assembly order that is chosen by design, which method is comprised of the steps of generating by design a plurality of specific nucleic acid building blocks having serviceable mutally compatible ligatable ends, as assembling these nucleic acid building blocks, such that a designed overall assembly order is achieved.

The mutually compatible ligatable ends of the nucleic acid building blocks to be assembled are considered to be "serviceable" for this type of ordered assembly if they enable the building blocks to be coupled in predetermined orders. Thus, in one aspect, the overall assembly order in which the nucleic acid building blocks can be coupled is specified by the design of the ligatable ends and, if more than one assembly step is to be used, then the overall assembly order in which the nucleic acid building blocks can be coupled is also specified by the sequential order of the assembly step(s). In a one embodiment of the invention, the annealed building pieces are treated with an enzyme, such as a ligase (e.g., T4 DNA ligase) to achieve covalent bonding of the building pieces.

In a another embodiment, the design of nucleic acid building blocks is obtained upon analysis of the sequences of a set of progenitor nucleic acid templates that serve as a basis for producing a progeny set of finalized chimeric nucleic acid molecules. These progenitor nucleic acid templates thus serve as a source of sequence information that aids in the design of the nucleic acid building blocks that are to be mutagenized, *i.e.* chimerized, recombined or shuffled.

In one exemplification, the invention provides for the chimerization of a family of related genes and their encoded family of related products. In a particular exemplification, the encoded products are nitrilase enzymes. Nucleic acids encoding the nitrilases of the invention can be mutagenized in accordance with the methods described herein.

Thus, according to one aspect of the invention, the sequences of a plurality of progenitor nucleic acid templates encoding nitrilases are aligned in order to select one or more demarcation points, which demarcation points can be located at an area of homology. The demarcation points can be used to delineate the boundaries of nucleic acid building blocks to be generated. Thus, the demarcation points identified and selected in the progenitor molecules serve as potential chimerization points in the assembly of the progeny molecules.

Typically a serviceable demarcation point is an area of homology (comprised of at least one homologous nucleotide base) shared by at least two progenitor templates, but the demarcation point can be an area of homology that is shared by at least half of the progenitor templates, at least two thirds of the progenitor templates, at least three fourths of the progenitor templates, and preferably at almost all of the progenitor templates. Even more preferably still a serviceable demarcation point is an area of homology that is shared by all of the progenitor templates.

In a one embodiment, the ligation reassembly process is performed exhaustively in order to generate an exhaustive library. In other words, all possible ordered combinations of the nucleic acid building blocks are represented in the set of finalized chimeric nucleic acid molecules. At the same time, the assembly order (*i*.*e*., the order of assembly of each building block in the 5' to 3' sequence of each finalized chimeric nucleic acid) in each combination is by design (or non-stochastic, non-random). Because of the non-stochastic nature of the method, the possibility of unwanted side products is greatly reduced.

In another embodiment, the method provides that, the ligation reassembly process is performed systematically, for example in order to generate a systematically compartmentalized library, with compartments that can be screened systematically, e.g., one by one. Each compartment (or portion) holds chimeras or recombinants with known characteristics. In other words the invention provides that, through the selective and judicious use of specific nucleic acid building blocks, coupled with the selective and judicious use of sequentially stepped assembly reactions, an experimental design can be achieved where specific sets of progeny products are made in each of several reaction vessels. This allows a systematic examination and screening procedure to be performed. Thus, it allows a potentially very large number of progeny molecules to be examined systematically in smaller groups.

Because of its ability to perform chimerizations in a manner that is highly flexible, yet exhaustive and systematic, particularly when there is a low level of homology among the progenitor molecules, the invention described herein provides for the generation of a library (or set) comprised of a large number of progeny molecules. Because of the non-stochastic nature of the ligation reassembly method, the progeny molecules generated preferably comprise a library of finalized chimeric nucleic acid molecules having an overall assembly order that is chosen by design. In a particularly embodiment, such a generated library is comprised of greater than 10³ to greater than 10¹⁰⁰⁰ different progeny molecular species.

In another exemplification, the synthetic nature of the step in which the building blocks are generated allows the design and introduction of nucleotides (e.g., one or more nucleotides, which may be, for example, codons or introns or regulatory sequences) that can later be optionally removed in an *in vitro* process (*e*.*g*., by mutageneis) or in an *in vivo* process (*e*.*g*., by utilizing the gene splicing ability of a host organism). It is appreciated that in many instances the introduction of these nucleotides may also be desirable for many other reasons in addition to the potential benefit of creating a serviceable demarcation point.

The synthetic ligation reassembly method of the invention utilizes a plurality of nucleic acid building blocks, each of which preferably has two ligatable ends. The two ligatable ends on each nucleic acid building block may be two blunt ends (i.e. each having an overhang of zero nucleotides), or preferably one blunt end and one overhang, or more preferably still two overhangs. On a double-stranded nucleic acid, a useful overhang can be a 3' overhang, or a 5' overhang. A nucleic acid building block can have a 3' overhang, a 5' overhang, two 3' overhangs, or two 5' overhangs. The overall order in which the nucleic acid building blocks are assembled to form a finalized chimeric nucleic acid molecule is determined by purposeful experimental design (*e*.*g*., by designing sticky ends between building block nucleic acids based on the sequence of the 5' and 3' overhangs) and is not random.

According to one preferred embodiment, a nucleic acid building block is generated by chemical synthesis of two single-stranded nucleic acids (also referred to as single- stranded oligos) and contacting them together under hybridization conditions so as to allow them to anneal to form a double-stranded nucleic acid building block. A double-stranded nucleic acid building block can be of variable size. The sizes of these building blocks can be small or large. Preferred sizes for building block range from 1 base pair (not including any overhangs) to 100,000 base pairs (not including any overhangs). Other preferred size ranges are also provided, which have lower limits of from 1 bp to 10,000 bp (including every integer value in between), and upper limits of from 2 bp to 100, 000 bp (including every integer value in between).

According to one embodiment, a double-stranded nucleic acid building block is generated by first generating two single stranded nucleic acids and allowing them to anneal to form a double-stranded nucleic acid building block. The two strands of a double-stranded nucleic acid building block may be complementary at every nucleotide apart from any that form an overhang; thus containing no mismatches, apart from any overhang(s). According to another embodiment, the two strands of a double-stranded nucleic acid building block are complementary at fewer than every nucleotide apart from any that form an overhang. Thus, according to this embodiment, a double-stranded nucleic acid building block can be used to introduce codon degeneracy. Preferably the codon degeneracy is introduced using the site-saturation mutagenesis described herein, using one or more N,N,GIT cassettes or alternatively using one or more N,N,N cassettes.

### Example 15 - Assays for Evaluation of Nitrilase Activity and Enantioselectivity

An assay method amenable to high throughput automation to increase the screening throughput both of the discovery and evolution efforts for nitrilases is described. The ideal assay is one that permits quantification of both product formation or substrate conversion and also enantiomeric excess. Two achiral and two chiral colorimetric assays that are amenable to high throughput screening were developed.

### Achiral Colorimetic Assays Developed:

OPA assay for residual substrate. The OPA assay is Applicable to α-amino or α-hydroxy nitrile substrates. The lysis of whole cells is not necessary. These results were corroborated by HPLC for 2-chloromandelonitrile and phenyl acetaldehyde cyanohydrin. The assay works best with aromatic nitriles. Aliphatic compounds exhibit a linear standard curve, fluorescence is reduced, reducing the efficacy of the assay.

### LDH Assay for quantification and ee determination of hydroxyacid formed.

The LDH assay is applicable to phenyl lactic acid but not to 2-chloromandelic acid. Use of a resazurin detection system increases sensitivity and reduces background. Background fluorescence of whole cells was overcome either by centrifugation or heat inactivation prior to performing assay.

AAO Assay for quantification and *ee* determination of aminoacid formed. The AAO assay is applicable to phenylalanine and (S)-2-amino-6-hydroxy hexanoic acid. The use of the Amplex Red detection system increases sensitivity. Cell lysis was shown not be necessary. Cells are grown in defined media in order to prevent background fluorescence.

### OPA Assay

The o-phthalaldehyde (OPA) fluorescence based nitrilase assay is used to quantify the amount of α-hydroxynitrile substrate remaining. OPA reacts with the cyanide released from the pH controlled decomposition of α-hydroxynitriles to the corresponding aldehyde and cyanide to yield a fluorescent, quantifiable product. OPA reacts with the cyanide released from the pH controlled decomposition of α-hydroxynitriles to the corresponding aldehyde and cyanide to yield the fluorescent 1-cyano-2-R benzoisoindole.

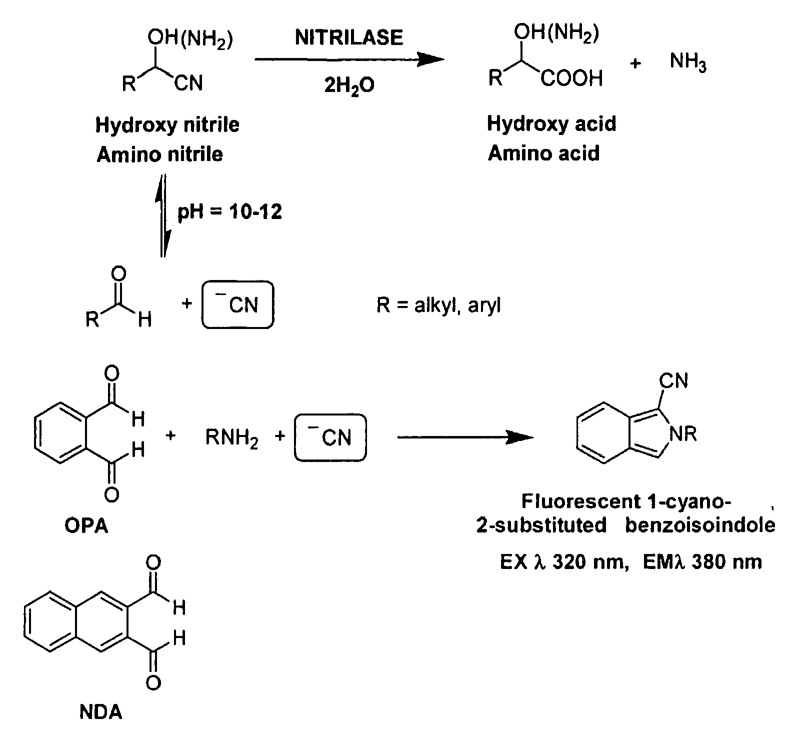

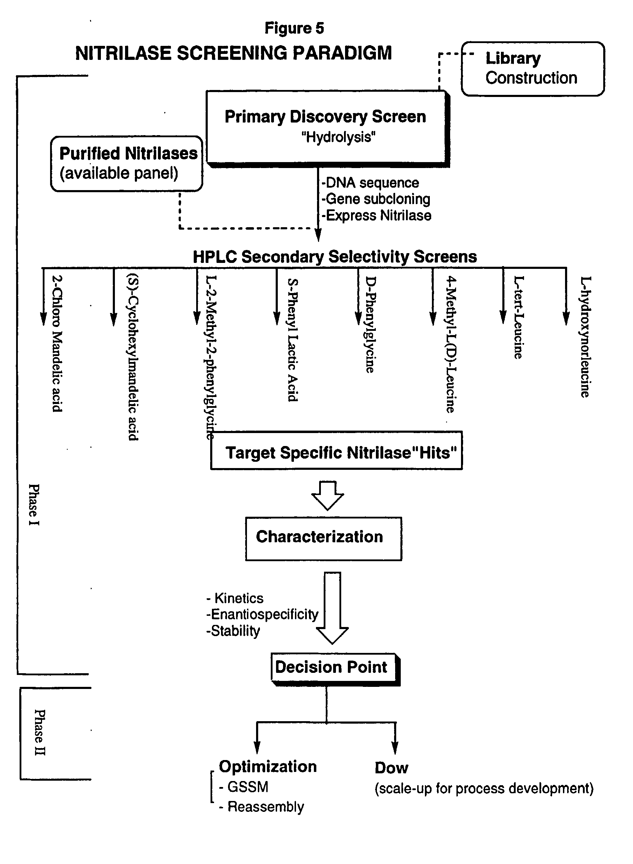

Standard curves were established for the following substrates: 2-Chloromandelonitrile (CMN, 0.998), Cyclohexylmandelonitrile (CHMN, 0.99), Acetophenone aminonitrile (APA, 0.99), and Phenylacetaldehyde cyanohydrin (PAC, 0.97), (Figure 5), (R² values in parentheses). A standard curve for Phenylglycine (PGN, 0.93) was also established. Three of the substrates tested, Dimethylbutanal aminonitrile (DMB) (2-amino-4,4-dimethyl pentanenitrile), Hydroxypivaldehyde aminonitrile (HPA) and Pivaldehyde aminonitrile (PAH), gave very low fluorescence readings and unreliable results under the original assay conditions. For these compounds a number of parameters where adjusted, however the fluorescent signal strength of these compounds was not increased by these manipulations.

In an attempt to increase the fluorescent signal of these three compounds, naphthalene dicarboxaldehyde (NDA) was substituted for OPA. Standard curves for PAH, HPA and DMB with either OPA or NDA were constructed. To determine sensitivity and background fluorescence, a lyophilized nitrilase lysate (SEQ ID NOS:189, 190) with suspected catalytic activity on each of the substrates was added. Hydrolysis was detected in three out of four of the compounds. NDA sharply boosted the signal, often by an order of magnitude, though this reduced linearity is presumably due to signal saturation.

NDA was established as an alternative detection reagent for the aliphatic compounds. However, it is desirable for the assay to utilize the same detection system for all of the substrates since this would facilitate the automated evaluation of multiple nitrilase substrates. The current OPA based assay is effective for the analysis of PAC, CMN, CHMN, APA, MN and PGN. While standard curves have been developed for the aliphatic compounds PAH, HPA, and DMB.

### Whole cell optimization

The effect of addition of lyophilized nitrilase lysate to the assay components, either untreated or heat inactivated, was evaluated. Interfering background fluorescence was not observed in either case. The OPA assay was next evaluated and optimized for nitrilase activity detection in a whole cell format. Both nitrilase expressing whole cells and in-situ lysed cells were evaluated. Lyophilized cell lysates were evaluated alongside their respective whole cell clones as controls. For this optimization study, mandelonitrile (MN) was chosen as a model substrate.

The lyophilized cell lysate of SEQ ID NOS:187, 188 was evaluated alongside whole cells expressing SEQ ID NOS:187, 188 and in *situ lysed* cells expressing SEQ ID NOS:187, 188 The addition of whole cells did not affect fluorescence nor result in fluorescence quenching. Addition of any of the three cell lysis solutions improved permeability (and therefore conversion) of mandelonitrile in the whole cell systems. Three cell lysing solutions were evaluated: B-PER (Pierce), BugBuster (Novagen) and CelLytic B-II (Sigma) and were found not to have a deleterious affect on the OPA assay. The addition of product α-hydroxyacid or α-aminoacid did not affect detection by the OPA assay.

The assay was modified from its original format, which required several liquid transfer steps, into a one plate process, where cell growth, nitrile hydrolysis and OPA assay reaction occurred in the same microtiter plate. Mandelonitrile was tested using this single well format. In this case, the *E. coli*. Gene site-saturation mutagenesis (GSSM^{™}) cell host was evaluated. Three clones were tested: SEQ ID NOS:101, 102, SEQ ID NOS:187, 188, and an empty vector, which was used as a control. Hydrolysis was evaluated at four timepoints, at 10 and 20 mM, and also with a 0 mM control. In an earlier experiment, clone SEQ ID NOS:187, 188 was evaluated against the phenylacetaldehyde cyanohydrin substrate (for which this enzyme does not exhibit activity), and no activity was observed.

The OPA assay was found to detect the presence of both α-hydroxy and α-amino nitrile substrate. Aromatic compounds were readily detectable with the assay, while aliphatic compounds posed some detection challenges. No background issues were evident when using lyophilized cell lysates, *in-situ* lysed whole cells or unlysed whole cells. The assay is amenable to one-plate analysis, where cells are grown, incubated with the substrate, and assayed on the same plate: no liquid transfers are required, easing automation. While all nitriles tested produced a linear response, aliphatic compounds gave a low fluorescent response.

### Chiral LDH Assay

A spectroscopic system based on lactate dehydrogenase (L-LDH) was developed for the analysis of the chiral α-hydroxy acids which are generated by the nitrilase catalyzed hydrolysis of cyanohydrins. The hydroxynitrile substrate is not metabolized by the secondary or detection enzyme and thus starting material does not interfere. Cell lysate which is not heat treated results in background activity for the LDH system; however, heat inactivation or pelleting of the cell lysates eliminates the background activity. (See Figure 4.)

The activity and enantiomeric specificity of commercially available D- and L-lactate dehydrogenases against the nitrilases disclosed herein was evaluated. An LDH was identified which is suitable to both D- and L-phenyl lactic acid analysis. An enzyme suitable for 2-Chloromandelic acid analysis was not found. The chosen LDH enzymes exhibited virtually absolute stereoselectivity. The viability of the assay to detect D- and L-LDH produced from PAC using lyophilized cell lysate was established.

Originally, three colorimetric dyes were evaluated, all of which are tetrazolium salts: NBT (3,3'-dimethoxy-4,4'-biphenylene)bis[2,(4-nitrophenyl) -5-phenyl-2H]-, chloride) MTT (3-(4,5-dimethylthiazol-2-yl)-2,5-diphenyltetrazolium bromide) INT (2-(4-Iodophenyl)-3-(4-nitrophenyl)-5-phenyl- 2H-tetrazolium chloride). The insolubility of the product of these detection system posed an analytical challenge. To address this, another tetrazolium salt with a reportedly soluble product, XTT (2,3-Bis-(2-methoxy-4-nitro-5-sulfophenyl) -2*H*-tetrazolium-5-carboxanilide, was evaluated. While XTT yielded a soluble bright red product, the substrate was insoluble which thus effected the same analytical challenges. As an alternative to the tetrazolium family of dyes, the dual colorimetric/fluorometric dye resazurin was evaluated. Oxidation of resazurin produces resourfin. Both substrate and product are soluble, and the color change can be quantified colorimetrically or fluorimetrically, increasing accuracy. Due to the sensitivity of resazurin, 0.05 mM of lactic acid can be quantified. Optimal results were obtained when using the dye in the same range as the substrate, e.g. 0.5 mM resazurin can quantify a range of lactic (and analogs) from 0.05 to 0.5, though the best linearity is at the lower end of this scale. Resourfin was stable over 28 hours, and had a linear fluorescent response.

In the presence of the LDH assay components, lyophilized enzyme gave background fluorescence/absorption. To address this problem the lysate was boiled for 10 minutes and then centrifuged. This resulted in a 90% decrease in background signal. Interestingly, both centrifugation alone (5 minutes @ 14.1 rcf) or boiling followed by centrifugation (5 minutes @ 100°C) reduced the fluorescence to background levels. In a high-throughput format such as 1536 well plates, spinning would be preferable to boiling, as boiling would increase evaporation (8 µl well size) and potentially volatize the nitrile substrates. No background signal resulting from growth media (LB and TB and M9) or cell lytic solutions (B-PER, CelLytic and BugBuster) was noted.

### Chiral AAO Assay

A spectroscopic system based on amino acid oxidase (AAO) was developed for the analysis of the chiral α-amino acids which are generated by the nitrilase catalyzed hydrolysis of amino nitriles.

### Assay Development and Validation

The initial assay validation utilized the 2,2'-azino-di-{3-ethylbenzothiazoline-6-sulfonic acid (ABTS) detection system as outlined above. However, since the color was not stable further investigations utilized the phenol amino antipyrine (PAAP) detection system which is analyzed at λ max 510nm. Enzymes with suitable activity were found for each enantiomer of 4-methyl- leucine, phenylalanine, (S)-2-amino-6-hydroxy hexanoic acid, and *tert*-leucine. The assay is not applicable to methylphenylglycine and does not work well with phenylglycine.

Standard curves were generated for phenylalanine from 0-15 mM. The curve is much more linear when the concentrations remained below 1 mM. The color remains stable for several days as long as it is kept in the dark. Three cell lysing solutions Bug Buster (BB), Bacterial Protein Extracting Reagent (BPER), and Cell Lytic Reagent (CLR) were added to the standard curve and shown to have no affect on color development. The addition of cell lysate (cl) did not exhibit background color formation. Addition of the phenylacetaldehyde aminonitrile sulfate (PAS) starting material also showed no effect on color formation.

The AAO system exhibits greater linearity at up to 1 mM substrate. The concentration of the AAO enzymes and of the acid substrate were adjusted to try to move the intersection of the L-AAO and D-AAO curves closer to the middle of the graph. Premixing the PAAP, the HRP, and the AAO was demonstrated to be effective and caused no change in observed activity establishing that the assay components may be added to the assay in a cocktail format.

A high level of background was observed for the AAO assay of whole cells and this was attributed to the L-amino acids present in the TB and LB growth media. Washing and resuspension of the cells in M9 media eliminated background. For all future experiments cells were grown in M9 media with 0.2% glucose. The lysed cells gave only a slightly better response that unlysed cells. Therefore, cell lysis is not necessary. SEQ ID NOS:187, 188 demonstrated activity on HPA in primary screening based on HPLC analysis.

The use of a fluorescent detection system which would permits implementation of the assay in ultra high throughput fashion such as 1536 well or gigamatrix format was investigated. The fluorescent reagent most applicable to our system is Amplex Red from Molecular Probes which produces the highly fluorescent resorufin (λₑₓ 545 nm; λₑₘ 590nm) Standard curves for phenylalanine and (S)-2-amino-6-hydroxy hexanoic acid were established (0-100 µM).

In preparation for assay automation, nitrilase expressing cells were added into microtiter plate containing M9 0.2% glucose,0.25 mM IPTG media by florescence activated cell sorting (FACS). Three nitrilase expressing subclones, and the empty vector control were evaluated: SEQ ID NOS:101, 102, SEQ ID NOS:187, 188, SEQ ID NOS:29, 30 and the empty vector. The viability of the cells following cell sorting proved to be inconsistent. Thus colony picking is currently being evaluated as an alternative method to add cells into microtiter plates. The evaporative loss from an uncovered 1536-well microtiter plate is approximately 30% per day in the robot incubator (incubator conditions: 37°C at 85% relative humidity (RH)). Incubation in the 95% RH incubator reduced evaporative loss to 1% per day.

The ability of the three subclones to grow in the presence of up to 3.5 mM of nitrile was established using HPA nitrile. Growth rates were only slightly retarded (less that 30%). Subclones grown in the presence of HPA were shown to express a nitrilase that catalyzes the formation of hydroxy norleucine (HNL) as established using the Amplex Red detection system. Only S was evaluated as the enzymes are S-selective. The reaction plate was read at 10 minute intervals, with 40 minutes showing the best linearity. While cell growth is significantly inhibited above 5 mM of HPA when the cells were grown at pH 7, growth was inhibited above 0.1 mM HPA for cells grown at pH 8.

In order to verify the AAO results by HPLC, a reaction was performed using high concentrations of HPA, up to 40 mM (due to HPLC detection challenges for (S)-2-amino-6-hydroxy hexanoic acid) and lyophilized cell lysate SEQ ID NOS:187, 188.

| Comparison AAO and HPLC data for HNL | | | | |
|---|---|---|---|---|
| | %ee | | %conversion | |
| [HNL] mM | AAO | HPLC | AAO | HPLC |
| 40 | 89% | 100% | 17% | 18% |
| 30 | 89% | 97% | 29% | 36% |
| 20 | 86% | 97% | 21% | 34% |
| 10 | 78% | 98% | 13% | 35% |

In order to determine if conducting the screen at a lower concentration introduces a bias in the results compared to the 20 mM substrate range that was used for HPLC based screens, an experiment was performed with SEQ ID NOS:187, 188 using three concentration ranges. Each experiment was done in triplicate in order to remove any nonsystematic error.

SEQ ID NOS:187, 188: Observed conversion and *ee* at multiple concentrations of HPA.

| | **Enantiomeric Excess (% *ee*)** | | | | | | | | |
|---|---|---|---|---|---|---|---|---|---|
| **[HPA] mM** | **10** | **20** | **30** | **1** | **2** | **3** | **0.1** | **0.2** | **0.3** |
| **Trial #1** | 60 | 54 | 72 | 61 | 64 | 63 | 60 | 63 | 60 |
| **Trial #2** | 57 | 62 | 69 | 58 | 57 | 53 | 32 | 51 | 37 |
| **Trial #3** | 53 | 62 | 69 | 60 | 55 | 52 | 79 | 103 | 72 |
| **AVG** | 57% | 59% | 70% | 59% | 59% | 56% | 57% | 72% | 56% |
| | **% Conversion** | | | | | | | | |

| **[HPA] mM** | **10** | **20** | **30** | **1** | **2** | **3** | **0.1** | **0.2** | **0.3** |
|---|---|---|---|---|---|---|---|---|---|
| **Trial #1** | 27% | 27% | 37% | 34% | 29% | 43% | 37% | 29% | 42% |
| **Trial #2** | 42% | 43% | 49% | 48% | 42% | 56% | 46% | 44% | 54% |
| **Trial #3** | 31% | 32% | 43% | 22% | 29% | 27% | 46% | 27% | 35% |
| **AVG** | 33% | 34% | 43% | 35% | 34% | 42% | 43% | 33% | 44% |

The AAO assay can be run on 384 or 1536 well format with cells sorted into an M9 0.2% glucose, 0.25 mM IPTG media. Cells can be grown in the presence of nitrile (in this case HPA), or the cells can be allowed to reach a certain density and the nitrile can then be added. Though cell lytic reagents do not interfere with the assay, when HPA was assayed, addition of the lytic reagents was found to be unnecessary. Either pre- or post- nitrile addition, the mother plate will have to be split into daughter plates, which are then assayed for the respective L- and D- enantiomer content. Incubation times with the AAO/Amplex Red reagents can be adjusted so that the D- and L- plate are read at separate times.

### Example 16 - Identification, Development and Production of Robust, Novel Enzymes Targeted for a Series of High-Value Enantioselective Bioprocesses

The invention provides for the development of nitrilases, through directed evolution, which provide significant technical and commercial advantages for the process manufacturing of the following chemical target:
L-2-amino-6,6-dimethoxyhexanoic acid

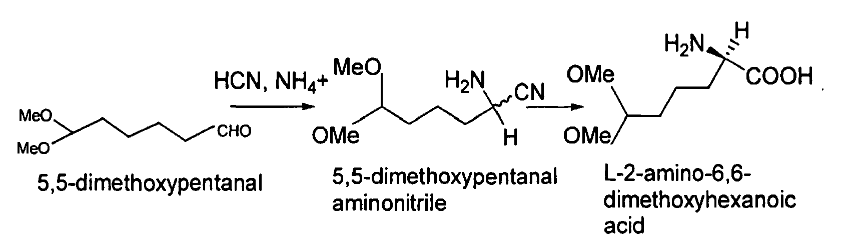

Nitrilase enzymes were shown to hydrolyze 2-amino-6-hydroxy hexanenitrile to (S)-2-amino-6-hydroxy hexanoic acid, with selectivity towards the L-enantiomer. The panel of nitrilases was screened against the target, 5,5-dimethoxypentanal aminonitrile. The positive enzymes were characterized on this substrate. A primary screen is used to identify putative up-mutants, which is then confirmed using HPLC.

GSSM™ and GeneReassembly^{™} are performed on selected nitrilases, in order to improve the enantioselectivity and activity of the enzymes for the production of L-2-amino-6,6-dimethoxyhexanoic acid. Nitrilases were identified for the enantioselective hydrolysis of 2-amino-6-hydroxy hexanenitrile to L-(S)-2-amino-6-hydroxy hexanoic acid. However, a slight structural difference is presented by the new target molecule, L-2-amino-6,6-dimethoxyhexanoic acid. In order to determine whether this difference affects the activity and enantioselectivity of the enzymes, the complete spectrum of nitrilases was screened against the new target.

First, identification of the correct target gene for GSSM through more detailed characterization of the hit enzymes for the production of L-2-amino-6,6-dimethoxyhexanoic acid was carried out. This effort involves a more extensive investigation of the effects of pH and temperature on activity and enantioselectivity and a more in-depth analysis of the stability of the enzyme to process conditions. Prior to initiation of the screening, the synthesis of a single enantiomer of an alkyl aminonitrile is done; the racemization of this nitrile is studied, in an effort to understand the relationship between this factor and enantioselectivity of the enzymes.

An enzyme exhibiting the highest combination of activity and enantioselectivity for the production of L-2-amino-6,6-dimethoxyhexanoic acid is selected for GSSM. Following the mutation of the target enzyme, the resulting mutants are screened on 5,5-dimethoxypentanal aminonitrile, using high throughput screening technology. Following confirmation of the up-mutants by HPLC analysis a decision point is reached, in order to evaluate the results of the GSSM on the target.

In parallel to GSSM™, a GeneReassembly™ is performed on a combination of parent enzymes, at least one of which is selected for activity and enantioselectivity on L-2-amino-6,6-dimethoxyhexanoic acid. At least two other nitrilases are reassembled with the former enzyme(s); these enzymes are selected in order to provide diversity to the reassembled sequences.

The present invention provides for development of racemization conditions for the original substrate aminonitriles. In addition, the present invention provides for the identification of enzymes capable of the conversion of these aminonitriles to the target α-amino acids by dynamic kinetic resolution. The present invention also provides for screening and development of a nitrilase-catalyzed kinetic resolution process for (R)-2-amino-6,6-dimethoxy hexanoic acid (allysine) production. (S)-2-amino-6-hydroxy hexanoic acid will be used as a model substrate for development of the kinetic resolution.
The target α-amino acid products are shown below:
(i) D-4-Fluorophenylglycine 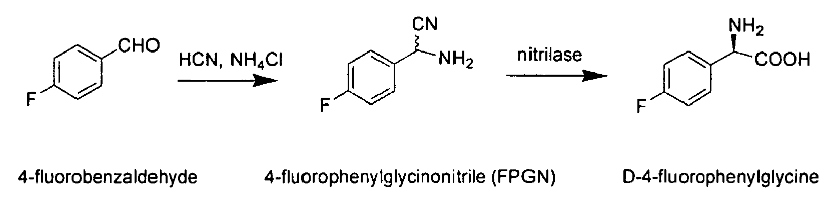
(ii) L-2-Amino-6,6-dimethoxyhexanoic acid (Allysine)

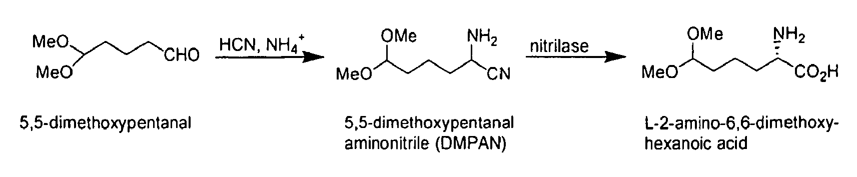

Conditions are developed for the racemization of the aminonitrile substrates for the nitrilase-catalyzed production of D-4-fluorophenylglycine and 2-amino-4,4-dimethyl pentanenitrile (allysine). Two model substrates, phenylglycinonitrile and pentanal aminonitrile are used initially, and racemization is studied in the absence of the enzyme. Concurrently determination of the performance of one or more available nitrilases under a variety of possible racemization conditions is carried out. In addition, the nitrilases are screened against hydroxypentanal aminonitrile for the production of (S)-2-amino-6-hydroxy hexanoic acid, and the promising enzymes are optimized. Once racemization conditions are established, the nitrilases are screened for activity. Further optimization for a kinetic resolution of the product is performed.

A number of enantioselective nitrilases were identified for the hydrolysis of α-aminonitriles to α-amino acids. While these enzymes were shown to have a preference for the required enantiomer of certain aminonitriles, a limiting factor in the further screening, development and comparison of candidate nitrilases is the rate of racemization of the aminonitrile substrates under the reaction conditions.

### Aromatic aminonitrile racemization

The first step is to establish conditions under which aromatic aminonitrile racemization occurs, using the model substrate, phenylglycinonitrile. Racemization strategies include, but are not limited to the list below. Options are roughly prioritized according to their commercial applicability.
(1) Manipulation of the pH of the reaction. Since it has been shown that racemization is rapid at high pH, this approach requires the discovery and optimization of nitrilases which are active and selective at pH> 10.
(2) Addition of known chemical racemizing agents, such as aldehydes, ketones, weak bases, resins, metal ions, Lewis acids etc., which can enhance racemization at lower pH.
(3) Synthesis of N-acylated aminonitrile derivatives, e.g. N-acetyl phenylglycinonitrile, which may be more easily racemized. In the case of N-acetyl phenylglycinonitrile, a selective D-acylase which removes the acetyl group would enhance the optical purity of the nitrilase product.
(4) Use of a biphasic system in which base-catalyzed racemization occurs in the hydrophobic organic phase and enzymatic hydrolysis in the aqueous phase.
(5) Use of a 2-enzyme system comprised of a nitrilase and an aminonitrile racemase. One amino acid racemase is commercially available at present, and will be tested for activity against phenyl- and fluorophenylglycinonitrile. Gene libraries will be searched for genes showing homology to known amino acid amide racemases, hydantoin racemases or any other racemases which can be identified.

Once conditions for this racemization have been established, they provide the basis for development of conditions for racemization of the target aromatic substrate, 4-fluorophenylglycinonitrile (FPGN). The FPGN is expected to be less stable than the model substrate; thus, it may racemize more quickly, but degradation reactions may be faster as well. The ability of sample enzyme(s) to tolerate and/or function well under them is evaluated. Final optimization of screening methods include the target substrates, sample nitrilases, and substrate racemization conditions.

Investigations carried out have shown that phenylglycinonitrile is easily racemized at pH 10.8. However, it does not appear that any of the existing enzymes can tolerate such harsh conditions of pH. Samples from highly alkaline environments are screened for the presence of nitrilases which are tolerant to such conditions. Once discovered, the enzymes are sequenced and subcloned, and the enzymes are produced as lyophilized cell lysates ready for screening.

### Aliphatic aminonitrile racemization

A model aliphatic aminonitrile, pentanal aminonitrile, is synthesized in its racemic form. However optically enriched samples are prepared using one the following approaches: (i) preparative chiral HPLC; (ii) diastereomeric salt resolution; (iii) diastereomeric derivatization or column chromatography; (iv) synthesis from L-*N-*BOC norleucine. An HPLC assay is used for the detection of these compounds.

### HPLC Assay

An HPLC assay for the detection of the (S)-2-amino-6-hydroxy hexanoic acid is used. An assay involving pre-column derivatization is used.

### Screening/Characterization:

Nitrilases are screened against 2-amino-6-hydroxy hexanenitrile. For enzymes capable of performing well at greater than 25 mM substrate, scale up reactions are performed. The substrate/product tolerance and stability profiles of the other enzymes are investigated.

The nitrilases are screened, and hits are characterized, focusing on pH and temperature optimum, enantioselectivity and stability under the reaction conditions.

### Enzyme Evolution

A target enzyme exhibiting the desired properties is selected for GSSM™. Following the mutation of the target enzyme, the resulting mutants are screened on the substrate using high throughput screening technology. Once the up-mutants have been confirmed by HPLC analysis, the individual mutations responsible for increased performance may be combined and evaluated for possible additive or synergistic effects.

In addition, a GeneReassembly™ will be performed on a combination of lead enzymes, which are selected for their desirable characteristics, including activity, enantioselectivity and stability in the reaction.

### Example 17

### Optimization of Nitrilases for the Enantioselective

### Production of (S)-Phenyllactic Acid

Nitrilases were identified for the enantioselective hydrolysis of 5 different nitrile substrates. These nitrilases were isolated and optimized for selected targets. The optimization involves process optimization and directed evolution. In particular, enzymes specific for the production of (S)-phenyllactic acid were characterized and optimized. This was aimed primarily at improving the activity of the enzymes, while maintaining a high enantioselectivity. An investigation into the effects of process conditions on the enzymes was also performed.

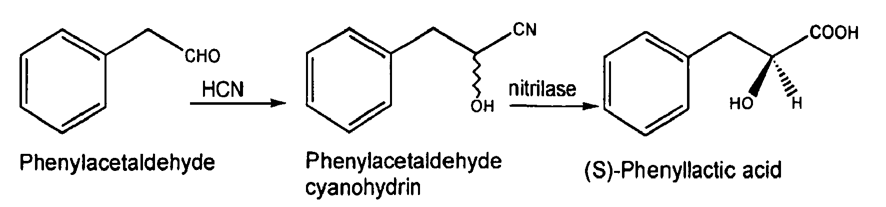

The development of high throughput assays for screening of mutants from potential directed evolution efforts was accomplished. Two achiral and two chiral colorimetric assays that are amenable to high throughput screening were developed and used for nitrilase directed evolution.

SEQ ID NOS:103, 104 was identified as a highly enantioselective nitrilase for the production of(S)- phenyllactic acid. Characterization of SEQ ID NOS:103, 104 shows the optimum reaction pH and temperature to be pH 8 and 37°C, respectively; the reaction starting material, phenylacetaldehyde, and the product, phenyllactic acid showed no effect on the enzyme activity up to levels of 5 mM and 30 mM, respectively. The scaled-up enzymatic reaction with an enantiomeric excess (*ee*) of 95%.

### Summary of enzymes screened for activity and enantioselectivity on Phenylacetaldehyde cyanohydrin

| **SEQ ID NOS:** | **Enzyme concentration** **(mg protein/ml)** | **Conversion (%)** | **Time to highest conversion (h)** | ***ee* (%)** |
|---|---|---|---|---|
| 109, 110 | 0.15 | 70% | 2 | 71% |
| 115, 116 | 0.15 | 70% | 3 | 72% |
| 127, 128 | 0.15 | 66% | 8 | 70% |
| 133, 134 | 0.6 | 60% | 3 | 84% |
| 135, 136 | 0.15 | 63% | 7 | 87% |
| 125, 126 | 0.6 | 63% | 3 | 83% |

### Example 18 - Directed Evolution of a nucleic acid encoding a nitrilase enzyme.

The *nitB* gene (GenBank Accession No. AX025996, from Alcaligenes faecalis) was subjected to Gene Site Saturated Mutagenesis™ or GSSM™ to generate a library of single amino acid substitution mutants covering the entire enzyme. The sequence of the "parental" *nitB* gene used in the directed evolution is SEQ ID NO: 103, 104. A nitB mutant library was generated from carrying out GSSM™. This *nitB* mutant library was then screened for clones with increased whole cell hydroxymethylthiobutryonitrile (HMTBN, which is a nitrilase substrate) activity. The product of the nitrilase reaction on that substrate is hydroxymethylthiobutyric acid (HMTBA).

Assays were run at 35°C with 100mM HMTBN and 100nM K₃PO₄, pH 7 to approximately 30-40% conversion. Two methods were used to quantitate HMTBN conversion, one being direct measurement of HMTBS produced by HPLC analysis and the other being indirect detection of residual HMTBN using the fluorescent cyanide assay, which has previously been described.

Putative *nitB* up mutants were subjected to a secondary assay to confirm the increased activity. In the secondary assay, up mutants and the wild type control were induced in expression medium in shake flasks. Shake flask cultures are then washed with 100mM K₃PO₄, pH7 and resuspended to the same optical density at 660nm. Kinetic assays were then performed with the normalized cell resuspensions under the same conditions used in the initial assays. Putative up mutants confirmed to have increased HMTBN activity were sequenced and tested for increased activity after transformation back into the same expression strain to ensure that increases in activity are not due to host mutations.

A confirmed *nitB* GSSM™ up-mutant is *nitB* G46P, which contains a glycine (GGT) to proline (CCG) substitution at amino acid 46. The whole cell HMTBN activity of this mutant is approximately 50% greater than that of wild type NitB at both 25°C and 35°C. Upon identification of the beneficial G46P mutation, GSSM™ was used again to generate a pool of double mutants using the *nitB* G46P template. These mutants all contain the G46P mutation and an additional single amino acid substitution at a random site. The double mutants were assayed for HMTBN activity greater than that of *nitB* G46P. Double, triple and quadruple mutants were created in order to speed up the mutation process and identify beneficial mutations more quickly. After the first few beneficial mutations were identified and isolated, they were combined to generate double mutants, the best of which was DM18. DM18 was used as a template to generate triple mutants. The most active triple mutant was TM3 and that was used as a template to generate quadruple mutants. The most active quadruple mutant was QM2. The table summarizes these mutations.

| **mutant** | **mutation 1** | **mutation 2** | **mutation 3** | **mutation 4** |
|---|---|---|---|---|
| DM18 | R (gcg) 29 C(tgt) | Y(tac) 207 M (atg) | | |
| TM3 | R (gcg) 29 C(tgt) | Y(tac) 207 M (atg) | L(ctt) 170 T(act) | |
| QM2 | R (gcg) 29 C(tgt) | Y(tac) 207 M (atg) | L(ctt) 170 T(act) | A(gcg) 197 N9(aat) |

The mutants were characterized first by studying their whole cell HMTBN activity. At 100 mM HMTBN, the HMTBS production rate of QM2 is 1.2 times greater than that of the parental gene. However, at 200 mM HMTBN, the rate of QM2 is 3.6 times that of the parental gene. The productivity of these mutants is increased considerably when the HMTBN concentration is raised from 100 mM to 300 mM. As to conversion rates, TM3 completely converted the substrate after 270 minutes and both DM18 and SM show greater than 75% conversion after this time. To further address the issue of HMTBN concentration effects on activity/productivity ofNitB, several mutants were assayed at both 400 mM and 528 mM HMTBN. NitB is essentially inactive at these substrate concentrations, however the mutants retain significant activity at these concentrations. In particular, the activity at these high concentrations were essentially the same as their activity at 200 mM substrate. Therefore, the mutants can be used over a wide substrate concentration range and provide much more flexibility in utility than the NitB parental gene.

The mutants were shown to have higher expression levels than the parental gene and it also appeared tha the QM2 and TM3 mutants contained a greater proportion of soluble enzyme than the wild type as seen in SDS-PAGE analysis. As to stability, all of the enzymes showed essentially the same stability pattern at both 25°C and 35°C.

Finally, the mutants were subjected to codon-optimization. The approach was to optimize the codons and therefore increase the expression levels in the particular host cell. That would, in turn, increase the activity per cell of the enzyme. This resulted in increased whole cell activity in the codon-optimized mutants as compared to controls. The increase in activity was approximately double the activity. An *E*. *coli* expression system was used.

### Example 20 - Selected Examples of Compounds Produced From a Nitrilase-Catalyzed Reaction

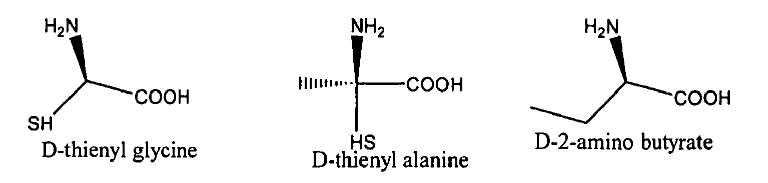

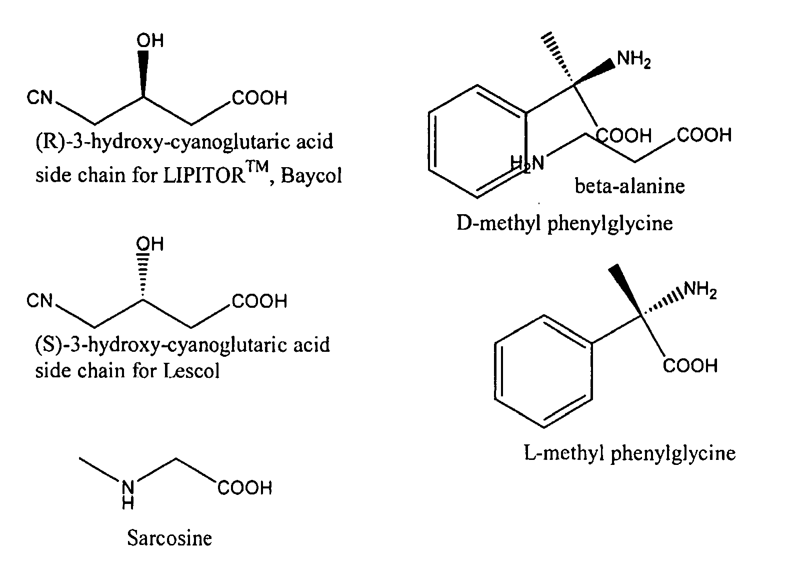

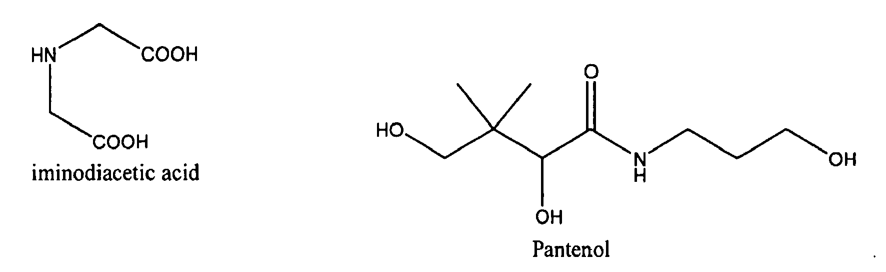

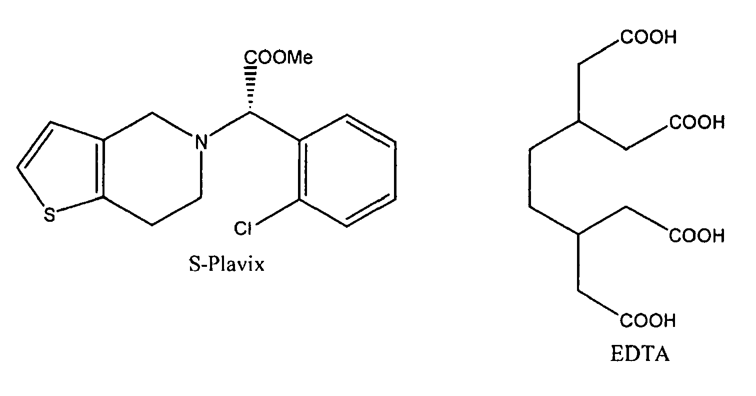

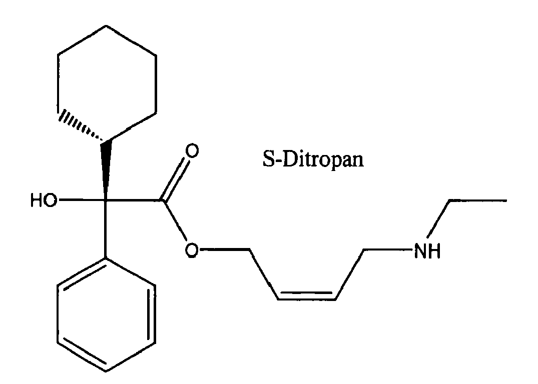

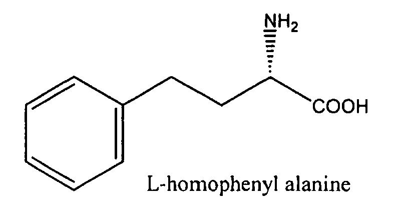

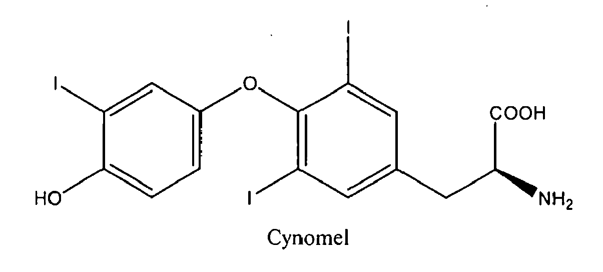

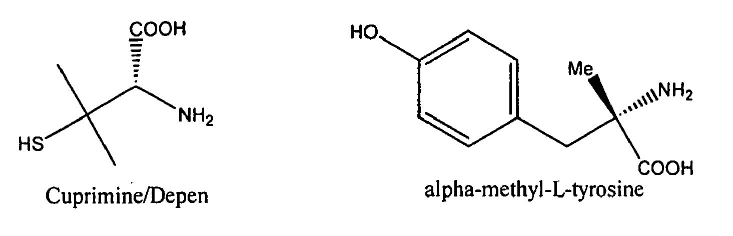

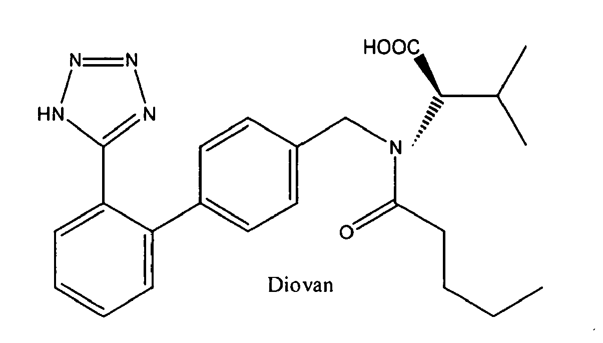

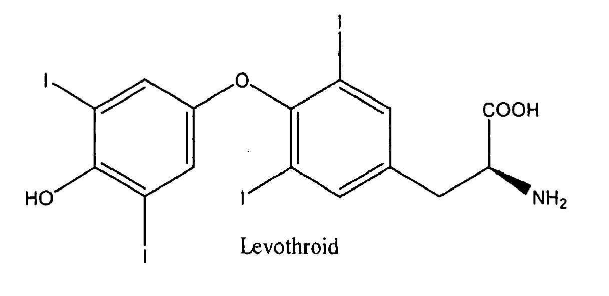

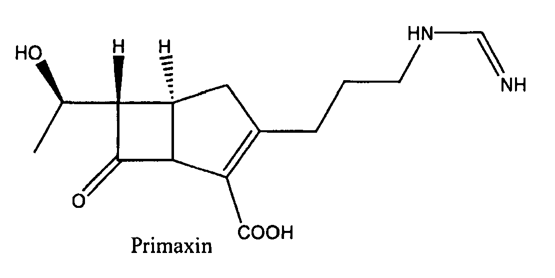

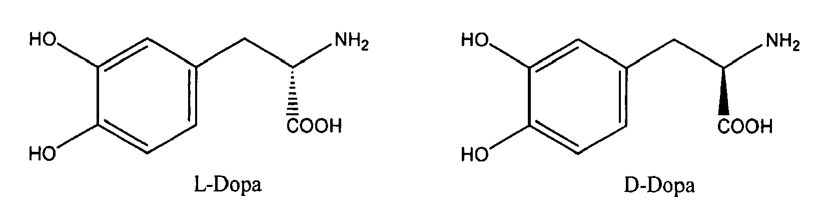

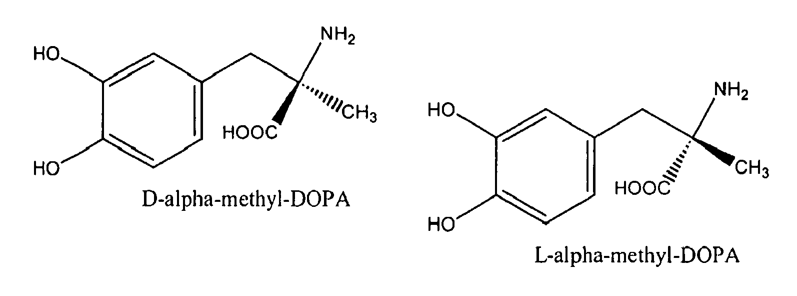

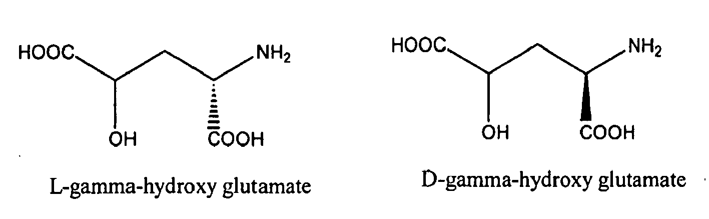

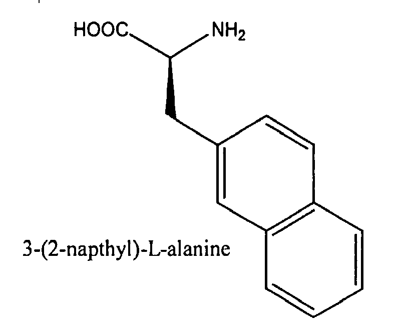

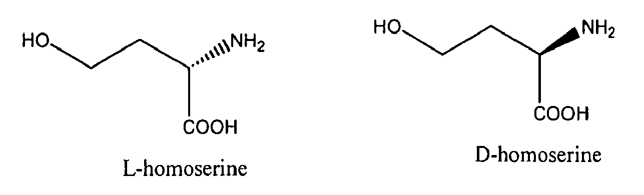

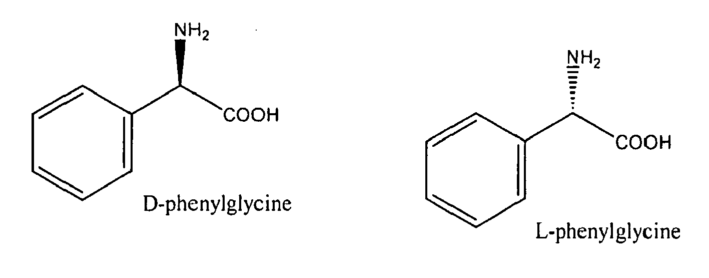

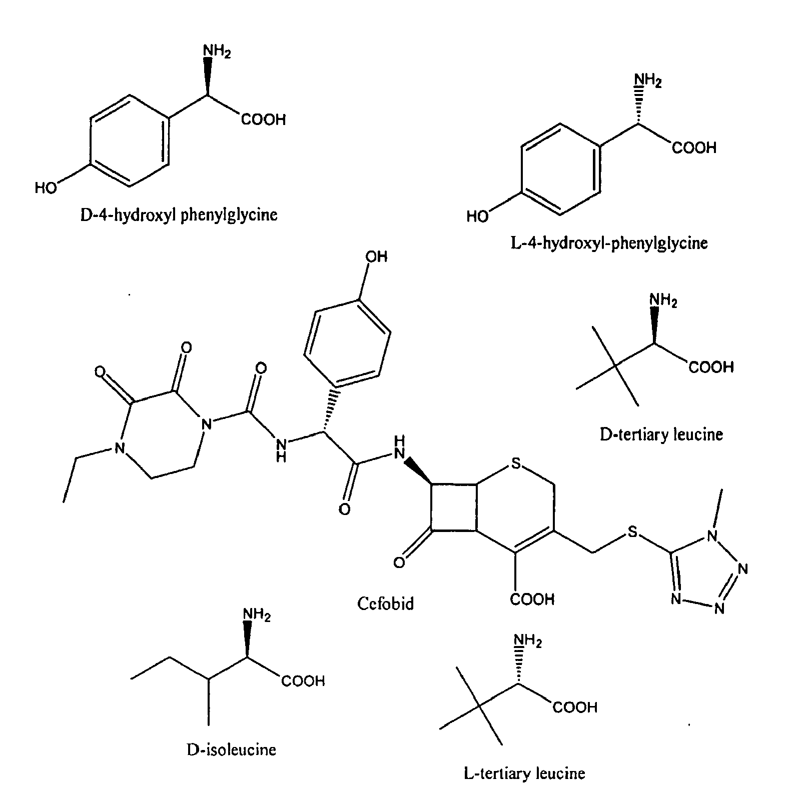

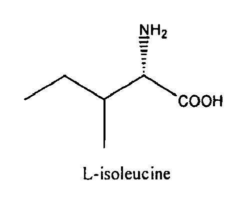

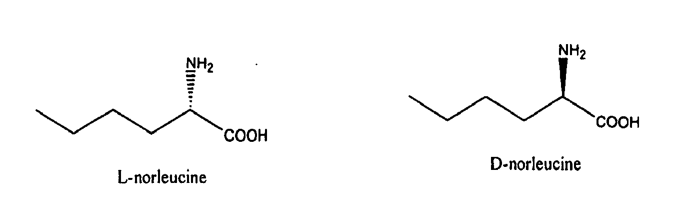

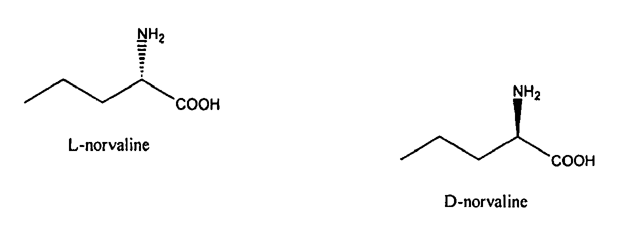

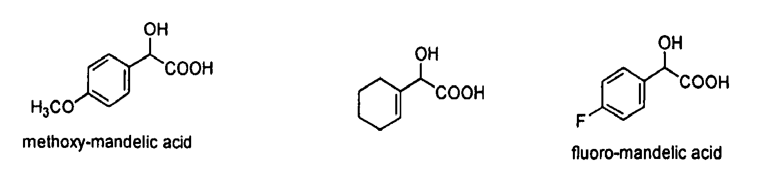

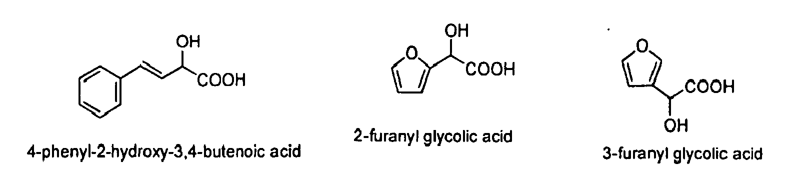

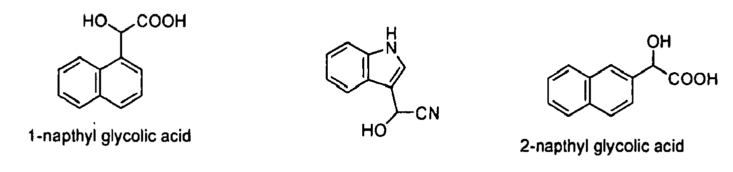

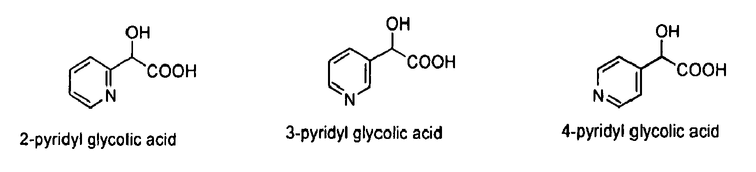

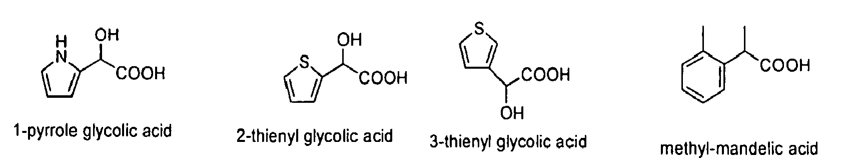

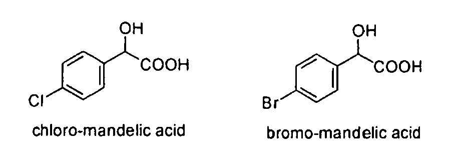

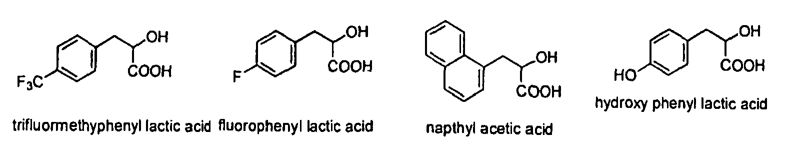

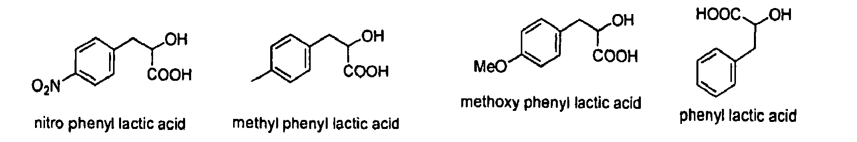

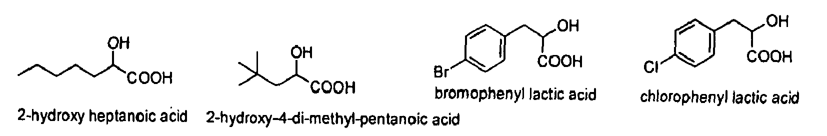

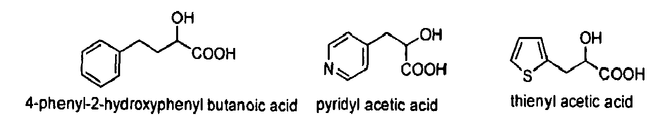

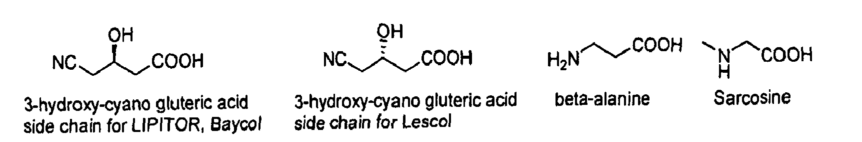

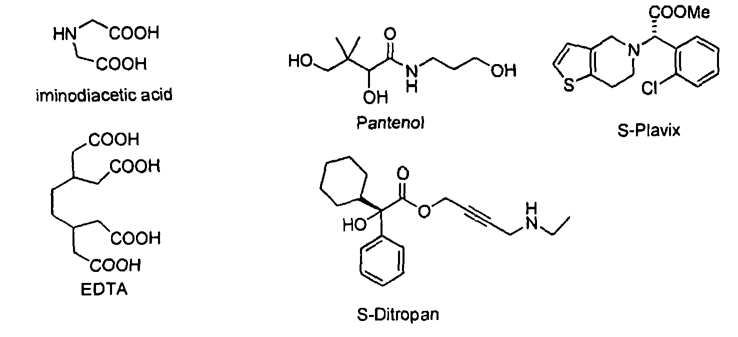

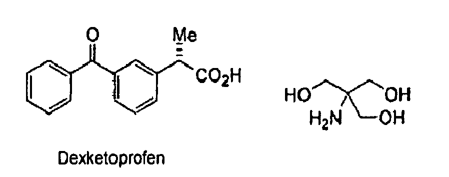

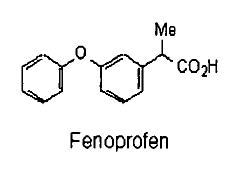

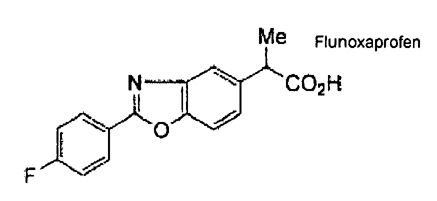

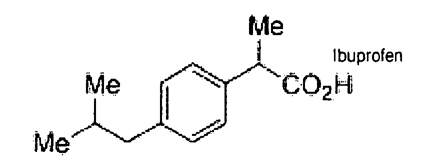

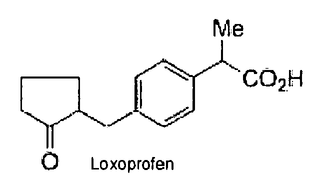

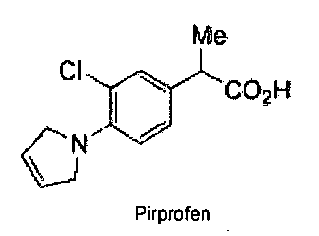

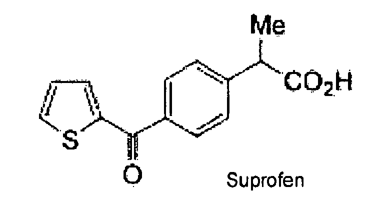

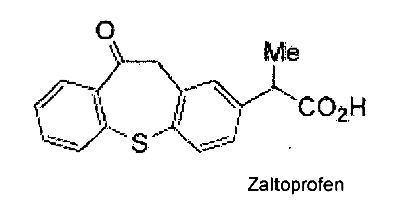

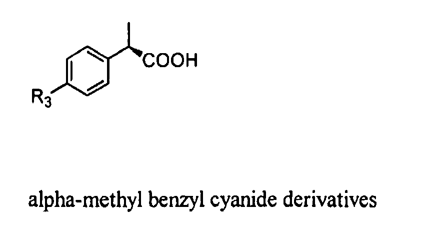

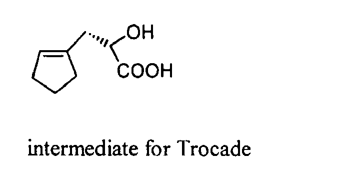

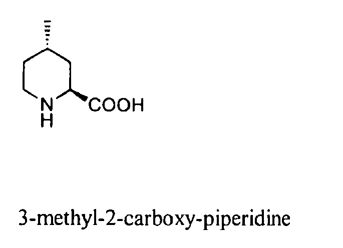

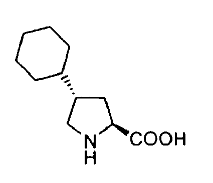

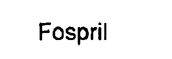

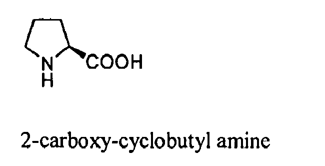

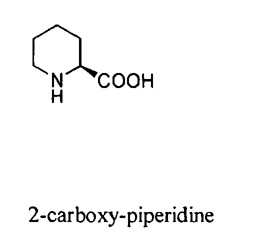

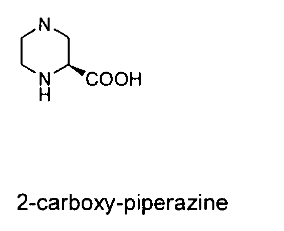

In addition, the following are potential products which can be made via the nitrilase Strecker format. More than 100 amino acids and many new drugs can be produced from their respective aldehydes or ketones utilizing the nitrilase enzymes of the invention. For example, large market drugs which can be synthesized using nitrilases of the invention include homophenylalanine, VASOTEC™, VASOTERIC™, TECZEM™, PRINIVIL™, PRINZIDE™, ZESTRIL™, ZESTORETIC™, RAMACE™, TARKA™, MAVIK™, TRANDOAPRIL™, TRANDOLAPRILAT™, ALTACE™, ODRIK™, UNIRETIC™, LOTENSIN™, LOTREL™, CAPOTEN™, MONOPRIL™, TANATRIL™, ACECOL™, LONGES™, SPIRAPRIL™, QUINAPRIL™, and CILAZAPRIL™. Other chiral drugs include DEMSER™ (alpha-methyl-L-Tyrosine), ALDOCHLOR™, LEVOTHROID™, SYNTHROID™, CYTOMEL™, THYOLAR™, HYCODAN™, CUPRIMINE™, DEPEN™, PRIMAXIN™, MIGRANOL™, D.H.E.-45, DIOVAN™, CEFOBID™, L-DOPA, D-DOPA, D-alpha-methyl-DOPA, L-alpha-methyl-DOPA, L-gamma-hydroxyglutamate, D-gamma-hydroxyglutamate, 3-(2-naphthyl)-L-alanine, D-homoserine, and L-homoserine.

Furthermore, the nitrilase enzymes of the invention can be useful for synthesizing the following amino acids. Many of these amino acids have pharmaceutical applications. D-phenylglycine, L-phenylglycine, D-hydroxyphenylglycine, L-hydroxyphenylglycine, L-tertiary leucine, D-tertiary leucine, D-isoleucine, L-isoleucine, D-norleucine, L-norleucine, D-norvaline, L-norvaline, D-2-thienylglycine, L-2-thienylglycine, L-2-aminobutyrate, D-2-aminobutyrate, D-cycloleucine, L-cycloleucine, D-2-methylphenylglycine, L-2-methylphenylglycine, L-thienylalanine, and D-thienylalanine.

The enzymes of the nitrilase enzymes of the invention can be useful for the synthesis of the following natural amino acids: glycine, L-alanine, L-valine, L-leucine, L-isoleucine, L-phenylalanine, L-tyrosine, L-tryptophan, L-cysteine, L-methionine, L-serine, D-serine, L-threonine, L-lysine, L-arginine, L-histidine, L-aspartate, L-glutamate, L-asparagine, L-glutamine, and L-proline. The following are examples of unnatural amino acids which can be produced using the nitrilase enzymes of the invention. D-alanine, D-valine, D-leucine, D-isoleucine, D-phenylalanine, D-tyrosine, D-tryptophan, D-cysteine, D-methionine, D-threonine, D-lysine, D-arginine, D-histidine, D-aspartate, D-glutamate, D-asparagine, D-glutamine, and D-proline.

Furthermore, nitrilase enzymes of the invention can be used in non-Strecker chemical reactions including the synthesis of more chiral drugs such as TAXOTERE™ as well as chiral drugs containing 3-hydroxy-glutaronitrile (a $5.5B market); LIPITOR™, BAYCOL™, and LESCOL™. Chiral product targets that are not drugs include PANTENOL™, L-phosphinothricin, D-phosphinothricin, D-fluorophenylalanine, and L-fluorophenylalanine. Finally, nitrilase can be used to produce unnatural amino acid compounds lacking a chiral center such as sarcosine, iminodiacetic acid, EDTA, alpha-aminobutyrate, and beta-alanine.

The following section includes examples of substrates and products produced by the nitrilases of the invention. The chemical structures of the substrates and of the products are shown. Activities, yield and the specific nitrilase shown to be useful in the chemical reactions are included in Tables following the reactions. The chemical reactions shown here are non-limiting examples of activities of the nitrilases of the invention.

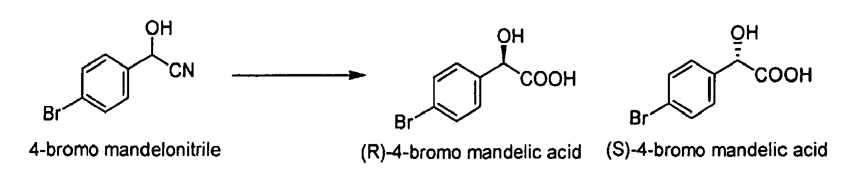

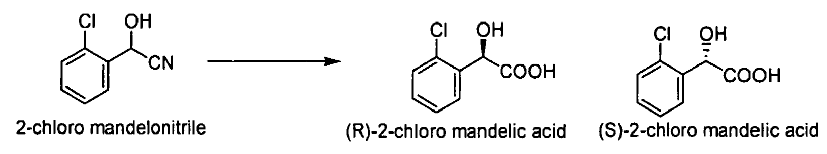

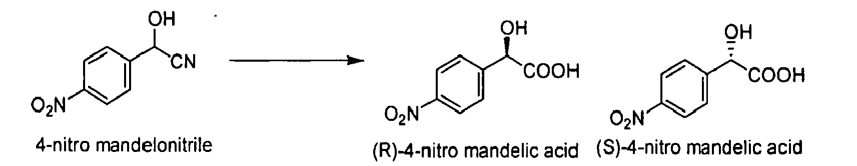

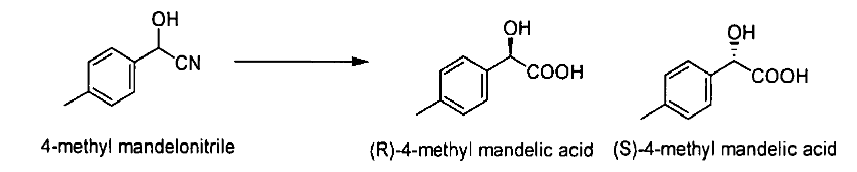

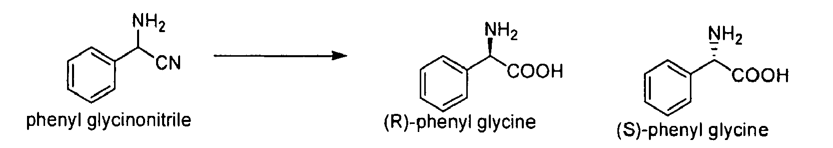

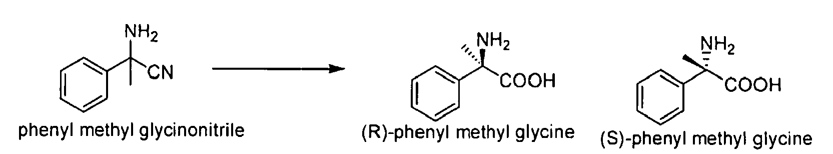

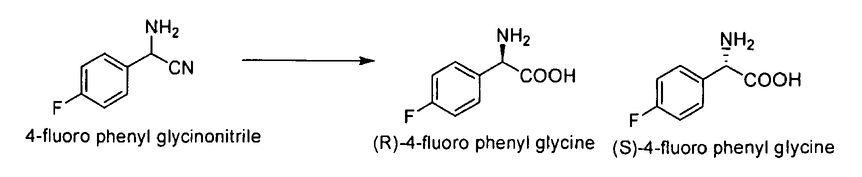

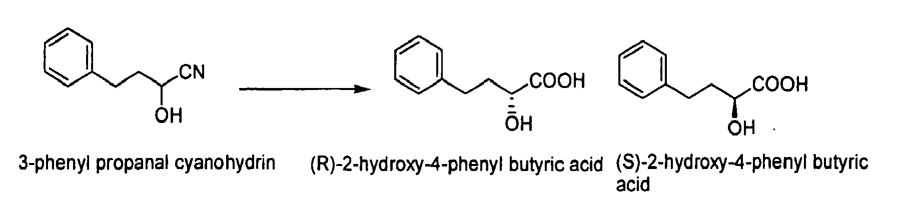

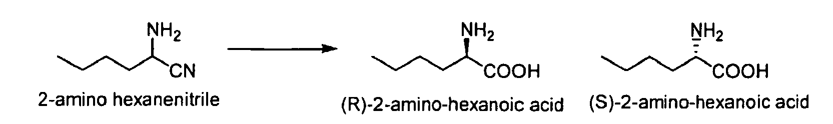

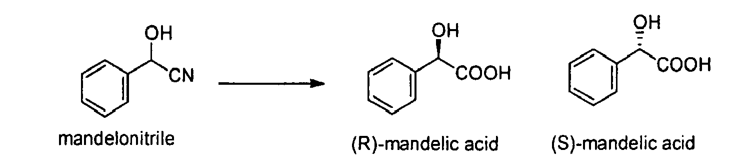

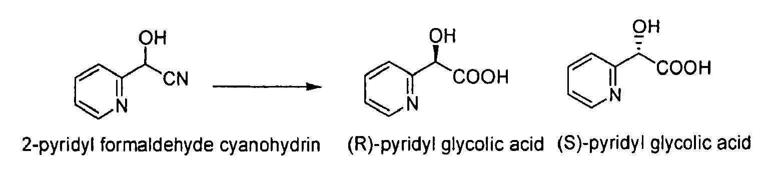

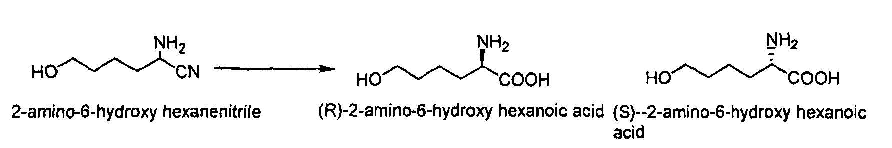

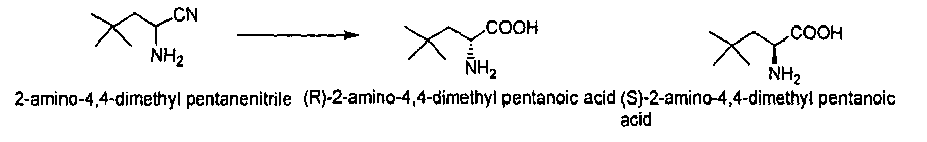

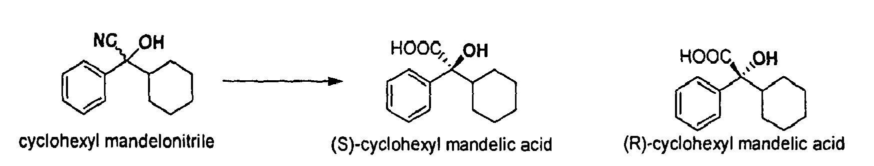

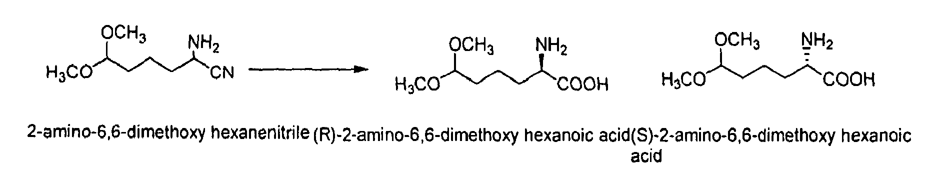

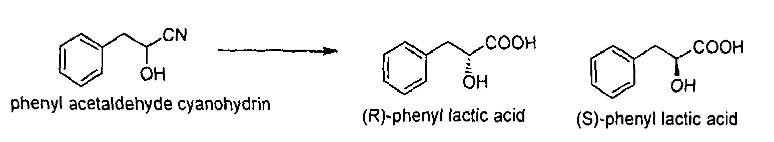

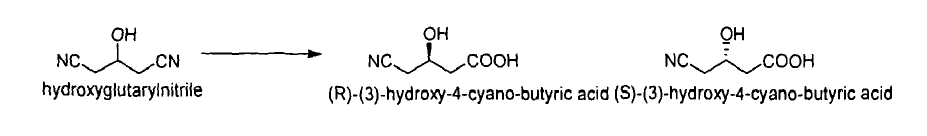

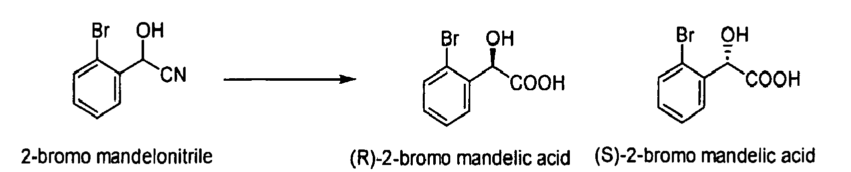

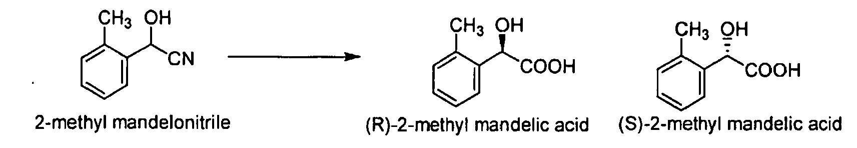

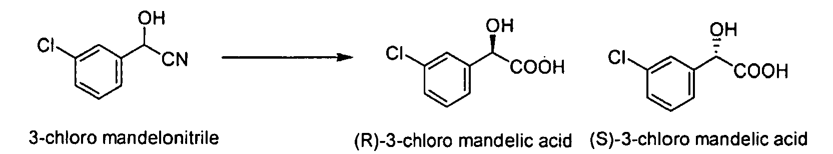

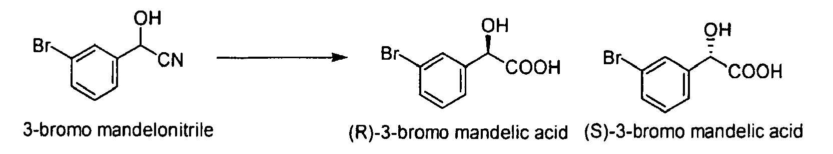

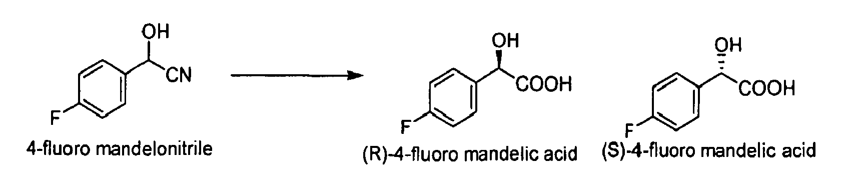

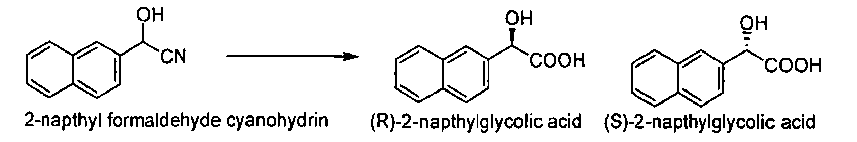

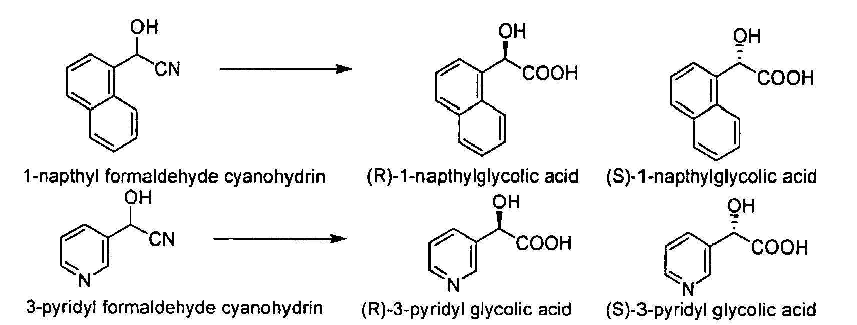

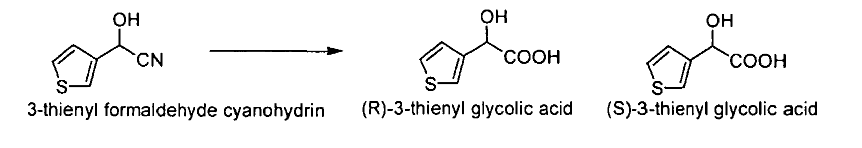

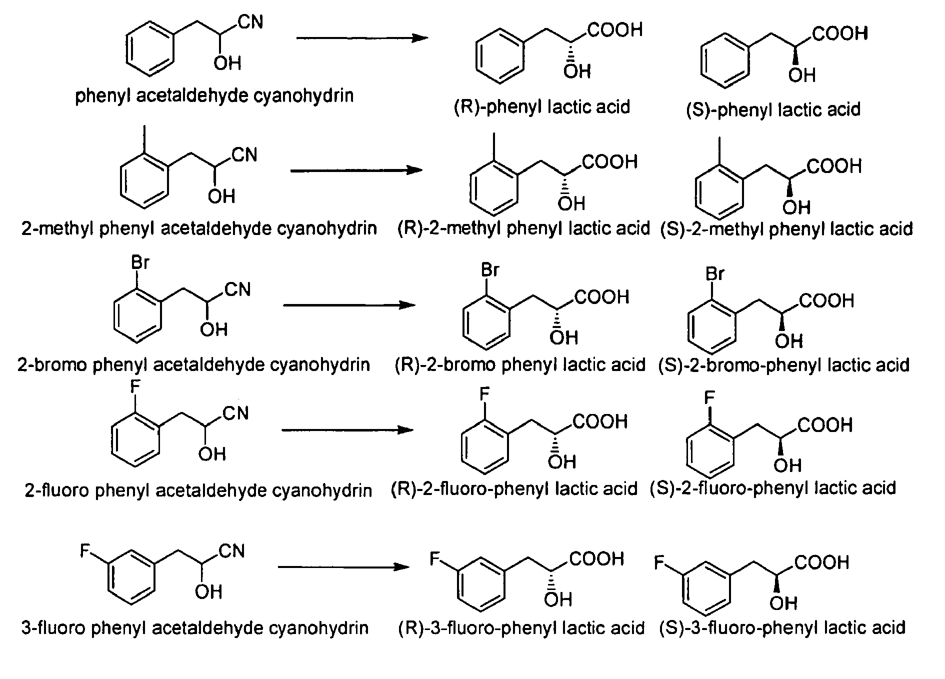

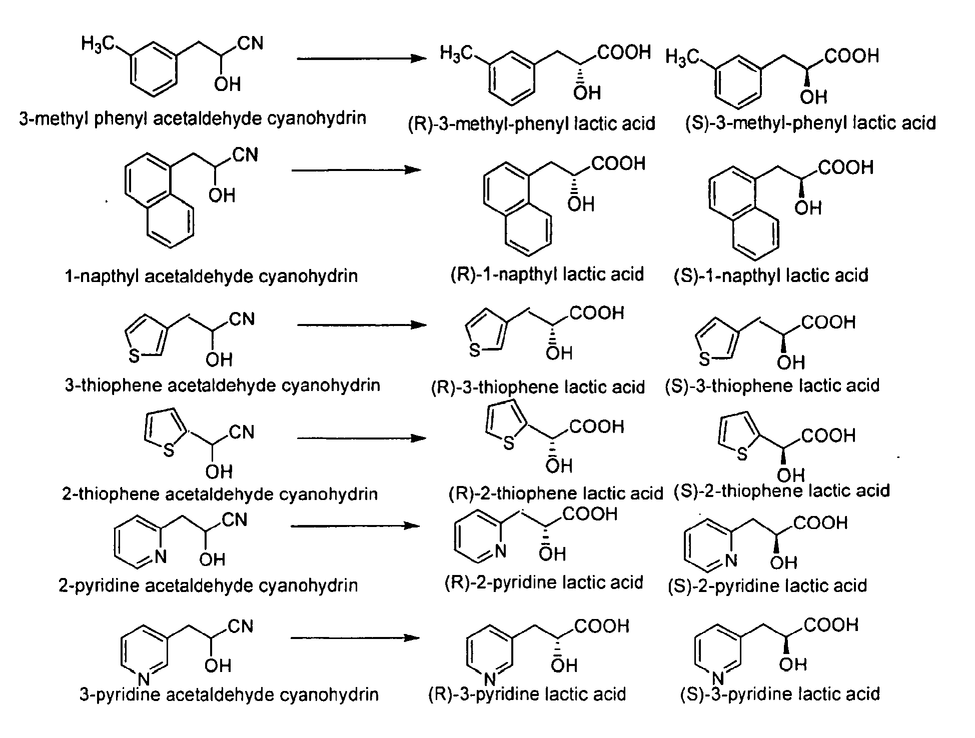

| **Seq ID DNA** | **Seq ID Protein** | | **2-chloromandelic acid** |
|---|---|---|---|
| | | **Yield %** | **ee %** **(enantiomer)** |
| 5 | 6 | 100% | 68% R |
| 13 | 14 | 100% | 79% R |
| 15 | 16 | 55% | 81% R |
| 33 | 34 | 95% | 61% R |
| 47 | 48 | 43% | 65% R |
| 55 | 56 | 100% | 93% R |
| 85 | 86 | 50% | 52% R |
| 261 | 262 | 100% | 60% R |
| 97 | 98 | 46% | 85% R |
| 279 | 280 | >95% | 40% R |
| 283 | 284 | 70% | 76% R |
| 331 | 332 | 36% | 79% R |
| 291 | 292 | 54% | 74% R |
| 101 | 102 | 53% | 62% R |
| 383 | 384 | 60% | 80% R |
| 385 | 386 | 80% | 97% R |
| 303 | 304 | >95% | 51% R |
| 139 | 140 | 60% | 85% R |
| 167 | 168 | 100% | 88% R |
| 169 | 170 | 72% | 85% R |
| 185 | 186 | 44% | 79% R |
| 187 | 188 | 60% | 78% R |
| 197 | 198 | 100% | 87% R |
| 217 | 218 | 72% | 82% R |

| **Seq ID DNA** | **Seq ID Protein** | | **4-bromo mandelic acid** |
|---|---|---|---|
| | | **Yield %** | **ee %** **(enantiomer)** |
| 3 | 4 | 12% | 92% R |
| 5 | 6 | 54% | 78% R |
| 333 | 334 | 48% | 93% R |
| 13 | 14 | 55% | 89% R |
| 15 | 16 | 57% | 92% R |
| 19 | 20 | 60% | 92% R |
| 29 | 30 | 58% | 93% R |
| 33 | 34 | 52% | 49% R |
| 47 | 48 | 60% | 92% R |
| 55 | 56 | 56% | 94% R |
| 57 | 58 | 60% | 81% R |
| 85 | 86 | 41% | 84% R |
| 261 | 262 | 48% | 94% R |
| 267 | 268 | 43% | 94% R |
| 97 | 98 | 53% | 91% R |
| 275 | 276 | 37% | 42% R |
| 279 | 280 | 59% | 2% R |
| 283 | 284 | 49% | 94% R |
| 331 | 332 | 42% | 89% R |
| 291 | 292 | 47% | 94% R |
| 101 | 102 | 68% | 77% R |
| 383 | 384 | 67% | 65% R |
| 385 | 386 | 66% | 99% R |
| 303 | 304 | 44% | 6% R |
| 139 | 140 | 61% | 95% R |
| 145 | 146 | 65% | 89% R |
| 167 | 168 | 54% | 92% R |
| 169 | 170 | 53% | 64% R |
| 185 | 186 | 56% | 94% R |
| 187 | 188 | 56% | 59% R |
| 189 | 190 | 59% | 6% R |
| 197 | 198 | 56% | 85% R |
| 215 | 216 | 44% | 65% R |
| 217 | 218 | 57% | 96% R |
| 221 | 222 | 56% | 55% R |
| 223 | 224 | 91% | 1% S |
| 231 | 232 | 8% | 94% R |
| 249 | 250 | 55% | 89% R |

| **Seq ID DNA** | **Seq ID Protein** | | **phenyl glycine** |
|---|---|---|---|
| | | **Yield %** | **ee % (enantiomer)** |
| 5 | 6 | 80% | 10% R |
| 15 | 16 | >90% | no specificity |
| 29 | 30 | 80-100% | < 20% R |
| 47 | 48 | 40-50% | <25% R |
| 55 | 56 | >90% | <20%R |
| 57 | 58 | 60% | 40% R |
| 85 | 86 | 70% | 32% R |
| 101 | 102 | 100% | <20% R |
| 383 | 384 | 75% | 62% R |
| 385 | 386 | 71% | 45% R |
| 139 | 140 | 100% | <20% R |
| 167 | 168 | >90% | <20% R |
| 169 | 170 | 89-90% | <20% R |
| 185 | 186 | ∼80% | ∼25% R |
| 187 | 188 | 84% | racemic |
| 189 | 190 | ∼90% | <10% R |
| 197 | 198 | >90% | 88% R |
| 215 | 216 | 10-20% | < 20% R |
| 217 | 218 | 52% | 86% R |
| 221 | 222 | 20% | R-selective |
| 231 | 232 | 20% | 80% R |

| **Seq ID DNA** | **Seq ID Protein** | | **phenyl methyl glycine** |
|---|---|---|---|
| | | **Yield %** | **ee % (enantiomer)** |
| 189 | 190 | 52% | 45% R |

| **Seq ID DNA** | **Seq ID Protein** | | **4-fluorophenylglycine** |
|---|---|---|---|
| | | **Yield %** | **ee % (enantiomer)** |
| 5 | 6 | 54% | 3% R |
| 333 | 334 | 53% | 2.5% R |
| 13 | 14 | 56% | 2.1% R |
| 33 | 34 | 52.30% | 6.9% R |
| 261 | 262 | 54% | 4.5% R |
| 267 | 268 | 44% | 22% R |
| 97 | 98 | 55% | 2.1% R |
| 279 | 280 | 55% | 7.5% S |
| 283 | 284 | 45% | 0.2% R |
| 291 | 292 | 54% | 0.3% R |
| 303 | 304 | 55% | 3% S |

| **Seq ID DNA** | **Seq ID Protein** | | **2-amino-6-hydroxy-hexanoic acid** |
|---|---|---|---|
| | | **Yield %** | **ee %** **(enantiomer)** |
| 5 | 6 | 24% | 86% S |
| 333 | 334 | 21% | 85% S |
| 13 | 14 | 16% | 90% S |
| 15 | 16 | <10% | activity observed |
| 29 | 30 | 8% | 78% S |
| 55 | 56 | 80% | 55% S |
| 261 | 262 | 11% | 79% S |
| 361 | 362 | 6% | 72% R |
| 267 | 267 | 25% | 90% S |
| 97 | 98 | 14% | 88% S |
| 279 | 280 | 35% | 93% S |
| 283 | 284 | 14% | 85% S |
| 343 | 344 | 19% | 67% R |
| 101 | 102 | 9% | 83% S |
| 103 | 104 | 30% | 91% S |
| 303 | 304 | 34% | 91% S |
| 145 | 146 | 20% | 85% S |
| 167 | 168 | 35% | 54% S |
| 185 | 186 | 13% | 86% S |
| 187 | 188 | 50% | 60% S |
| 189 | 190 | 5% | 62% S |
| 197 | 198 | 12% | 88% S |
| 217 | 218 | 100% | 52% S |
| 221 | 222 | 32% | 79% S |
| 249 | 250 | 8% | 87% S |

| **Seq ID DNA** | **Seq ID Protein** | | **2-amino-4,4-dimethyl pentanoic acid** |
|---|---|---|---|
| | | **Yield %** | **ee %** **(enantiomer)** |
| 55 | 56 | ∼40% | <20% S |
| 59 | 60 | 30% | >95% S |
| 267 | 268 | 25% | 90% S |
| 103 | 104 | 30% | 91% S |
| 167 | 168 | ∼40% | <20% S |
| 221 | 222 | 32% | 79% S |

| **Seq ID DNA** | **Seq ID Protein** | | **phenyl lactic acid** |
|---|---|---|---|
| | | **Yield %** | **ee % (enantiomer )** |
| 321 | 322 | 33% | 56% S |
| 23 | 24 | 20% | 5% S |
| 31 | 32 | 36% | 68% R |
| 39 | 40 | 17% | 5% S |
| 293 | 294 | 100% | 65% S |
| 41 | 42 | 35% | 45% R |
| 43 | 44 | 40% | 85% S |
| 49 | 50 | 75% | 66% S |
| 61 | 62 | 56% | 80% S |
| 73 | 74 | 100% | 5% R |
| 259 | 260 | 95% | 33% S |
| 335 | 336 | 96% | 62%S |
| 83 | 84 | 100% | 49% S |
| 93 | 94 | 80% | 50% S |
| 95 | 96 | 57% | 60% R |
| 271 | 272 | 75% | 60% R |
| 273 | 274 | 100% | 45% S |
| 275 | 276 | 20% | 3% S |
| 99 | 100 | 90% | 82% S |
| 107 | 108 | 80% | 40% S |
| 109 | 110 | 80% | 60% S |
| 115 | 116 | 60% | 63% S |
| 117 | 118 | 20% | 4% S |
| 125 | 126 | 20% | 6% S |
| 127 | 128 | 20% | 8% S |
| 129 | 130 | 20% | 9% S |
| 133 | 134 | 30% | 8% S |
| 135 | 136 | 30% | 7% S |
| 113 | 114 | 20% | 20% S |
| 161 | 162 | 70% | 66% S |
| 171 | 172 | 52% | 60% R |
| 173 | 174 | 20% | 83% S |
| 175 | 176 | 87% | 45% S |
| 183 | 184 | 50% | 57% S |
| 189 | 190 | 20% | 8% S |
| 195 | 196 | 89% | 89% S |
| 205 | 206 | 90% | 73% S |
| 207 | 208 | 76% | 85% S |
| 209 | 210 | 98% | 75% S |
| 213 | 214 | 70% | 86% S |
| 227 | 228 | 99% | 31% S |
| 239 | 240 | 22% | 100% R |
| 241 | 242 | 40% | 62% R |

| **Seq ID DNA** | **Seq ID Protein** | | **Cyclohexylmandelic acid** |
|---|---|---|---|
| | | **Yield %** | **ee % (enantiomer)** |
| 17 | 18 | 60% | Not determined |
| 321 | 322 | 70% | Not determined |
| 49 | 50 | 70% | Not determined |
| 61 | 62 | 70% | Not determined |
| 105 | 106 | >90% | Not determined |
| 115 | 116 | 70% | Not determined |
| 195 | 196 | 55% | Not determined |
| 213 | 214 | 65% | Not determined |
| 237 | 238 | 60% | Not determined |

| **Seq ID DNA** | **Seq ID Protein** | | **mandelic acid** |
|---|---|---|---|
| | | **EE % (enantiomer)** | **YIELD %** |
| 3 | 4 | 1 | 22 |
| 5 | 6 | 97 R | 100 |
| 333 | 334 | 93 R | 100 |
| 9 | 10 | 8 R | 21 |
| 13 | 14 | 98 R | 100 |
| 15 | 16 | 96 R | 100 |
| 29 | 30 | 99 R | 100 |
| 33 | 34 | 95 R | 100 |
| 35 | 36 | 3 S | 15 |
| 39 | 40 | 35 S | 14 |
| 47 | 48 | 97 R | 100 |
| 55 | 56 | 99 R | 100 |
| 57 | 58 | 96 R | 100 |
| 75 | 76 | 52 R | 56 |
| 257 | 258 | 26 S | 12 |
| 81 | 82 | 16 S | 23 |
| 259 | 260 | 47 S | 15 |
| 83 | 84 | 46 S | 14 |
| 85 | 86 | 83 R | 20 |
| 261 | 262 | 88 R | 100 |
| 361 | 362 | 14 S | 22 |
| 267 | 268 | 96 R | 100 |
| 97 | 98 | 99 R | 100 |
| 277 | 278 | 41 R | 13 |
| 279 | 280 | 28 R | 100 |
| 283 | 284 | 94 R | 100 |
| 331 | 332 | 87 S | 100 |
| 299 | 300 | 68 S | 12 |
| 351 | 352 | 89 R | 100 |
| 317 | 318 | 55 S | 14 |
| 343 | 344 | 21 S | 49 |
| 291 | 292 | 81 R | 100 |
| 287 | 288 | 8 S | 22 |
| 383 | 384 | 80 R | 100 |
| 119 | 120 | 31 R | 14 |
| 385 | 386 | 99 R | 100 |
| 303 | 304 | 45 R | 100 |
| 139 | 140 | 97 R | 100 |
| 145 | 146 | 95 R | 100 |
| 167 | 168 | 99 R | 100 |
| 169 | 170 | 99 R | 100 |
| 185 | 186 | 99 R | 100 |
| 187 | 188 | 99 R | 100 |
| 189 | 190 | 9 R | 100 |
| 197 | 198 | 99 R | 100 |
| 209 | 210 | 15 S | 9 |
| 215 | 216 | 88 R | 100 |
| 217 | 218 | 98 R | 100 |
| 221 | 222 | 85 R | 58 |
| 225 | 226 | 36 S | 14 |
| 231 | 232 | 91 R | 100 |
| 235 | 236 | 87 S | 22 |
| 237 | 238 | 18 S | 8 |
| 239 | 240 | 35 S | 16 |
| 249 | 250 | 98 R | 100 |

| NOTES | **Seq ID DNA** | **Seq ID Protein** | | **3-hydroxy-4-cyanobutyric acid** |
|---|---|---|---|---|
| | | | **EE % (enantiomer)** | **YIELD %** |
| | 1 | 2 | not determined | activity observed |
| | 3 | 4 | 4 S | activity observed |
| | 7 | 8 | 6 S | activity observed |
| | 333 | 334 | 6 S | activity observed |
| | 9 | 10 | 16 S | activity observed |
| | 11 | 12 | 8 R | 104 |
| | 15 | 16 | not determined | activity observed |
| | 17 | 18 | 16 R | activity observed |
| | 19 | 20 | 7 R | activity observed |
| | 21 | 22 | 6 S | activity observed |
| | 321 | 322 | 51 R | activity observed |
| | 25 | 26 | 19 S | activity observed |
| | 27 | 28 | 6 S | activity observed |
| | 27 | 28 | 100 S | 111 |
| | 29 | 30 | 16 R | 100 |
| | 31 | 32 | 54 R | 127 |
| | 33 | 34 | 12 S | activity observed |
| | 35 | 36 | 100 S | 35 |
| | 37 | 38 | 100 S | 87 |
| | 39 | 40 | 24 S | activity observed |
| | 293 | 294 | 12 S | 91 |
| | 255 | 256 | 65 S | activity observed |
| | 41 | 42 | 100 R | 16 |
| | 43 | 44 | not determined | activity observed |
| | 47 | 48 | 38 S | 96 |
| | 49 | 50 | 52 S | activity observed |
| | 55 | 56 | 92 R | 122 |
| | 57 | 58 | 100 S | 73 |
| | 59 | 60 | 100 S | 100 |
| | 61 | 62 | 18 R | activity observed |
| | 63 | 64 | 10 R | 9 |
| | 69 | 70 | 2 S | activity observed |
| | 71 | 72 | not determined | activity observed |
| | 73 | 74 | 100 S | 3 |
| | 325 | 326 | 73 R | 4 |
| | 77 | 78 | 100 S | activity observed |
| | 257 | 258 | 3 R | activity observed |
| | 259 | 260 | 55 S | activity observed |
| | 83 | 84 | 34 S | activity observed |
| | 261 | 262 | 22 R | activity observed |
| | 361 | 362 | 4 S | activity observed |
| | 89 | 90 | 31 R | 116 |
| | 297 | 298 | 24 R | activity observed |
| | 91 | 92 | 6 S | activity observed |
| | 267 | 268 | not determined | activity observed |
| | 93 | 94 | 58 S | activity observed |
| | 95 | 96 | 21 R | activity observed |
| | 97 | 98 | 17 R | activity observed |
| | 275 | 276 | 80 S | activity observed |
| | 279 | 280 | 9 S | activity observed |
| | 281 | 282 | not determined | activity observed |
| | 283 | 284 | 23 R | activity observed |
| | 313 | 314 | 12 S | activity observed |
| | 351 | 352 | 11 S | activity observed |
| | 309 | 310 | 28 S | activity observed |
| | 291 | 292 | 13 S | activity observed |
| | 287 | 288 | 100 S | activity observed |
| | 99 | 100 | 100 S | activity observed |
| | 101 | 102 | 80 S | 61 |
| | 383 | 384 | 8 R | activity observed |
| | 103 | 104 | 100 R | 98 |
| | 105 | 106 | 13 S | activity observed |
| | 107 | 108 | 100 S | 79 |
| | 109 | 110 | 1 00 S | 79 |
| | 111 | 112 | 91 S | 32 |
| | 113 | 114 | 100 S | activity observed |
| | 115 | 116 | 66 S | activity observed |
| | 117 | 118 | 22 R | activity observed |
| Different subclone | 119 | 120 | 13 S | activity observed |
| Different subclone | 119 | 120 | 100 S | activity observed |
| | 123 | 124 | 20 S | activity observed |
| | 385 | 386 | 13 S | activity observed |
| | 125 | 126 | 25 S | activity observed |
| | 127 | 128 | 92 S | 106 |
| | 129 | 130 | 100 S | 22 |
| | 131 | 132 | 100 S | activity observed |
| | 133 | 134 | 86 S | 14 |
| | 135 | 136 | 22 S | activity observed |
| | 139 | 140 | 16 S | activity observed |
| | 113 | 114 | 100 S | 108 |
| | 143 | 144 | 100 S | activity observed |
| | 145 | 146 | 100 S | 100 |
| | 149 | 150 | 3.2 S | activity observed |
| | 151 | 152 | 8 R | activity observed |
| | 153 | 154 | 35 S | 39 |
| | 155 | 156 | 100 S | activity observed |
| | 157 | 158 | 26 R | activity observed |
| | 159 | 160 | 100 S | 71 |
| | 161 | 162 | 64 R | 122 |
| | 163 | 164 | 10 S | activity observed |
| | 167 | 168 | 10 R | 106 |
| | 169 | 170 | 14 R | 38 |
| | 171 | 172 | 27 R | 33 |
| | 173 | 174 | 20 R | activity observed |
| | 175 | 176 | 31 S | 58 |
| | 177 | 178 | 100 S | 11 |
| | 179 | 180 | 100 S | 75 |
| | 181 | 182 | 100 S | 58 |
| | 183 | 184 | 100 S | 19 |
| | 185 | 186 | 100 S | 78 |
| | 187 | 188 | 7 S | activity observed |
| | 189 | 190 | 5 S | 104 |
| | 193 | 194 | 7 S | activity observed |
| | 195 | 196 | 95 R | 100 |
| | 197 | 198 | 100 S | 64 |
| | 201 | 202 | 100 S | 132 |
| | 253 | 254 | 4 S | activity observed |
| | 203 | 204 | not determined | activity observed |
| | 205 | 206 | 64 R | activity observed |
| | 207 | 208 | 95 R | 100 |
| | 209 | 210 | 95 R | 100 |
| | 213 | 214 | 25 R | activity observed |
| | 215 | 216 | 100 S | activity observed |
| | 217 | 218 | 11 S | 109 |
| | 219 | 220 | not determined | activity observed |
| | 221 | 222 | 26 R | 100 |
| | 223 | 224 | 5 S | activity observed |
| | 227 | 228 | 52 S | activity observed |
| Clone | 229 | 230 | 31 S | activity observed |
| Subclone | 229 | 230 | 100 S | activity observed |
| | 231 | 232 | 100 S | activity observed |
| | 233 | 234 | 35 S | activity observed |
| | 235 | 236 | 6 S | activity observed |
| | 237 | 238 | 95 R | 100 |
| | 239 | 240 | not determined | activity observed |
| | 241 | 242 | 9 R | activity observed |
| | 243 | 244 | 100 S | activity observed |
| | 245 | 246 | 5 S | activity observed |
| | 247 | 248 | not determined | activity observed |
| | 249 | 250 | 21 S | 98 |
| | 251 | 252 | 5 S | activity observed |

The indications of subclones and clones refer to subclones of the originally isolated nucleic acid of the respective SEQ ID NO.

| **Seq ID DNA** | **Seq ID Protein** | | **2-bromo mandelic acid** |
|---|---|---|---|
| | | **EE % (enantiomer)** | **YIELD %** |
| 5 | 6 | 82 R | 63 |
| 97 | 98 | 95 R | 100 |
| 101 | 102 | 92 R | 92 |
| 385 | 386 | 96 R | 100 |
| 185 | 186 | 92 R | 90 |
| 187 | 188 | 90 R | 100 |
| 197 | 198 | 93 R | 74 |
| 217 | 218 | 90 R | 100 |
| 235 | 236 | 91 R | 30 |

| **Seq ID DNA** | **Seq ID Protein** | | **2-methyl mandelic acid** |
|---|---|---|---|
| | | **EE % (enantiomer)** | **YIELD %** |
| 5 | 6 | 85 R | 100 |
| 55 | 56 | 90 R | 100 |
| 97 | 98 | 93 R | 100 |
| 383 | 384 | 97 R | 100 |
| 385 | 386 | 97 R | 100 |
| 139 | 140 | 88 R | 100 |
| 145 | 146 | 93 R | 100 |
| 167 | 168 | 85 R | 100 |
| 185 | 186 | 93 R | 95 |
| 187 | 188 | 96 R | 100 |
| 197 | 198 | 86 R | 100 |
| 217 | 218 | 80 R | 100 |

| **Seq ID Protein** | | **3-chloro mandelic acid** |
|---|---|---|
| | **EE %** **(enantiomer)** | **YIELD %** |
| 386 | 98 R | 100 |

| **Seq ID DNA** | **Seq ID Protein** | | **3-bromo mandelic acid** |
|---|---|---|---|
| | | **EE %** **(enantiomer)** | **YIELD %** |
| 385 | 386 | 99 R | 100 |

| **Seq ID DNA** | **Seq ID Protein** | | **4-fluoromandelic acid** |
|---|---|---|---|
| | | **EE %** **(enantiomer)** | **YIELD %** |
| 5 | 6 | 82 R | 50 |
| 15 | 16 | 92 R | 45 |
| 55 | 56 | 97 R | 45 |
| 85 | 86 | 97 R | 40 |
| 97 | 98 | 98 R | 45 |
| 101 | 102 | 95 R | 50 |
| 385 | 386 | 99 R | 100 |
| 167 | 168 | 97 R | 50 |
| 185 | 186 | 97 R | 50 |
| 187 | 188 | 95 R | 50 |
| 197 | 198 | 79 R | 45 |
| 215 | 216 | 89 R | 40 |
| 217 | 218 | 98 R | 50 |
| 221 | 222 | 68 R | 45 |

| **Seq ID DNA** | **Seq ID Protein** | | **2-napthyl glycolic acid** |
|---|---|---|---|
| | | **EE %** **(enantiomer)** | **YIELD %** |
| 13 | 14 | 95 R | 85 |
| 97 | 98 | 93 R | 40 |
| 101 | 102 | 96 R | 100 |
| 383 | 384 | 98 R | 100 |
| 385 | 386 | 98 R | 100 |
| 125 | 126 | 95 R | 20 |
| 127 | 128 | 75 R | 4 |
| 133 | 134 | 97 R | 20 |
| 145 | 146 | 96 R | 100 |
| 169 | 170 | 97 R | 100 |
| 187 | 188 | 95 R | 100 |
| 201 | 202 | 98 R | 9 |

| **Seq ID DNA** | **Seq ID Protein** | | **1-napthyl glycolic acid** |
|---|---|---|---|
| | | **EE %** **(enantiomer)** | **YIELD %** |
| 13 | 14 | 82 R | 100 |
| 97 | 98 | 84 R | 100 |
| 101 | 102 | 69 R | 100 |
| 383 | 384 | 46 R | 100 |
| 385 | 386 | 95 R | 100 |
| 125 | 126 | 83 R | 16 |
| 127 | 128 | 33 R | 13 |
| 133 | 134 | 42 R | 16 |
| 145 | 146 | 69 R | 100 |
| 169 | 170 | 62 R | 100 |
| 187 | 188 | 55 R | 100 |
| 201 | 202 | 59 R | 15 |

| **Seq ID DNA** | **Seq ID Protein** | | **3-pyridyl glycolic acid** |
|---|---|---|---|
| | | **EE %** **(enantiomer)** | **YIELD %** |
| 5 | 6 | 94 R | 100 |
| 13 | 14 | 94 R | 100 |
| 29 | 30 | 95 R | 95 |
| 47 | 48 | 95 R | 100 |
| 55 | 56 | 92 R | 85 |
| 57 | 58 | 95 R | 100 |
| 97 | 98 | 96 R | 100 |
| 383 | 384 | 95 R | 100 |
| 385 | 386 | 96 R | 100 |
| 139 | 140 | 90 R | 100 |
| 145 | 146 | 89 R | 100 |
| 167 | 168 | 94 R | 100 |
| 169 | 170 | 91 R | 70 |
| 185 | 186 | 93 R | 90 |
| 187 | 188 | 95 R | 100 |
| 197 | 198 | 93 R | 100 |
| 217 | 218 | 94 R | 100 |
| 249 | 250 | 94 R | 90 |

| **Seq ID DNA** | **Seq ID Protein** | | **3-thienylglycolic acid** |
|---|---|---|---|
| | | **EE %** **(enantiomer)** | **YIELD %** |
| 385 | 386 | 95 R | 100 |

| **Seq ID DNA** | **Seq ID Protein** | | **2-pyridyl glycolic acid** |
|---|---|---|---|
| | | **EE %** **(enantiomer)** | **YIELD %** |
| 385 | 386 | 95 R | 100 |

| NOTES | **Seq ID DNA** | **Seq ID Protein** | | **2-hydroxy-4-phenyl butyric acid** |
|---|---|---|---|---|
| | | | **EE % (enantiomer)** | **YIELD %** |
| | 1 | 2 | 66 S | 92 |
| | 3 | 4 | 82 S | 12 |
| | 5 | 6 | 85 S | 100 |
| | 7 | 8 | 81 S | 13 |
| | 333 | 334 | 85 S | 100 |
| | 9 | 10 | 88 S | 13 |
| | 13 | 14 | 82 S | 100 |
| | 15 | 16 | 72 S | 22 |
| | 17 | 18 | 67 S | 100 |
| | 19 | 20 | 90 S | 81 |
| | 21 | 22 | 69 S | 32 |
| | 321 | 322 | 66 S | 100 |
| | 23 | 24 | 71 S | 27 |
| | 25 | 26 | 69 S | 70 |
| | 27 | 28 | 72 S | 20 |
| | 29 | 30 | 91 S | 100 |
| | 31 | 32 | 13 S | 100 |
| | 33 | 34 | 83 S | 38 |
| | 35 | 36 | 69 S | 26 |
| | 37 | 38 | 66 S | 76 |
| | 39 | 40 | 3 S | 110 |
| | 293 | 294 | not determined | 100 |
| | 307 | 308 | 56 S | 54 |
| | 255 | 256 | 74 S | 27 |
| | 41 | 42 | 82 S | 100 |
| | 43 | 44 | 41 S | 100 |
| | 45 | 46 | 85 S | 17 |
| | 47 | 48 | 87 S | 76 |
| | 49 | 50 | 73 S | 17 |
| | 51 | 52 | 70 S | 100 |
| | 53 | 54 | 84 S | 12 |
| | 55 | 56 | 84 S | 100 |
| | 57 | 58 | 91 S | 100 |
| | 59 | 60 | 56 S | 100 |
| | 61 | 62 | 72 S | 65 |
| | 63 | 64 | 87 S | 20 |
| | 359 | 360 | 63 S | 79 |
| | 67 | 68 | 82 S | 19 |
| | 69 | 70 | 66 S | 72 |
| | 71 | 72 | 83 S | 13 |
| | 73 | 74 | 75 S | 26 |
| | 325 | 326 | 87 S | 12 |
| | 75 | 76 | 39 S | 42 |
| | 77 | 78 | 85 S | 23 |
| | 79 | 80 | 85 S | 14 |
| | 257 | 258 | 51 S | 71 |
| | 81 | 82 | 73 S | 51 |
| | 259 | 260 | 51 S | 46 |
| | 335 | 336 | 65 S | 69 |
| | 83 | 84 | 86 S | 10 |
| | 85 | 86 | 66 S | 20 |
| | 261 | 262 | 77 S | 100 |
| | 87 | 88 | 90 S | 16 |
| | 361 | 362 | 67 S | 76 |
| | 89 | 90 | 44 S | 47 |
| | 297 | 298 | 69 S | 100 |
| | 91 | 92 | 65 S | 81 |
| | 267 | 268 | 72 S | 100 |
| | 93 | 94 | 90 S | 17 |
| | 95 | 96 | 66 S | 22 |
| | 271 | 272 | 53 S | 38 |
| | 97 | 98 | 93 S | 100 |
| | 273 | 274 | 84 S | 22 |
| | 275 | 276 | 5 S | 100 |
| | 277 | 278 | 84 S | 20 |
| | 279 | 280 | 75 S | 100 |
| | 281 | 282 | 65 S | 91 |
| | 283 | 284 | 88 S | 100 |
| | 331 | 332 | 21 S | 100 |
| | 311 | 312 | 50 S | 32 |
| | 313 | 314 | 88 S | 100 |
| | 323 | 324 | 67 S | 31 |
| | 329 | 330 | 62 S | 77 |
| | 289 | 290 | 58 S | 70 |
| | 299 | 300 | 71 S | 33 |
| | 351 | 352 | 54 S | 80 |
| | 317 | 318 | 57 S | 18 |
| | 309 | 310 | 58 S | 100 |
| | 343 | 344 | 20 S | 100 |
| | 291 | 292 | 73 S | 27 |
| | 287 | 288 | 41 S | 52 |
| | 99 | 100 | 87 S | 14 |
| | 101 | 102 | 86 S | 100 |
| | 383 | 384 | 70 S | 69 |
| | 103 | 104 | 79 S | 100 |
| | 105 | 106 | 92 S | 74 |
| | 107 | 108 | 87 S | 14 |
| | 109 | 110 | 64 S | 76 |
| | 111 | 112 | 67 S | 81 |
| | 113 | 114 | 63 S | 79 |
| | 115 | 116 | 86 S | 34 |
| | 117 | 118 | 80 S | 21 |
| Different subclone | 119 | 120 | 87 S | 9 |
| Different subclone | 119 | 120 | 7 S | 100 |
| | 121 | 122 | 74 S | 21 |
| | 123 | 124 | 86 S | 12 |
| | 385 | 386 | 63 S | 58 |
| | 125 | 126 | 86 S | 23 |
| | 303 | 304 | 68 S | 100 |
| | 127 | 128 | 73 S | 25 |
| | 129 | 130 | 75 S | 24 |
| | 131 | 132 | 76 S | 22 |
| | 133 | 134 | 88 S | 18 |
| | 135 | 136 | 65 S | 91 |
| | 113 | 114 | 87 S | 20 |
| | 143 | 144 | 60 S | 47 |
| | 145 | 146 | 74 S | 34 |
| | 149 | 150 | 75 S | 100 |
| | 151 | 152 | 74 S | 31 |
| | 153 | 154 | 69 S | 100 |
| | 155 | 156 | 69 S | 74 |
| | 157 | 158 | 62 S | 100 |
| | 159 | 160 | 9 S | 100 |
| | 163 | 164 | 87 S | 21 |
| Different subclone | 165 | 166 | 76 S | 31 |
| Different subclone | 165 | 166 | 59 S | 100 |
| | 167 | 168 | 80 S | 100 |
| | 169 | 170 | 84 S | 23 |
| | 171 | 172 | 8 S | 100 |
| | 173 | 174 | 78 S | 67 |
| | 175 | 176 | 84 S | 23 |
| | 177 | 178 | 100 S | 88 |
| | 179 | 180 | 16 S | 100 |
| | 183 | 184 | 76 S | 100 |
| | 185 | 186 | 87 S | 100 |
| | 187 | 188 | 80 S | 100 |
| | 193 | 194 | 71 S | 9 |
| | 195 | 196 | 51 S | 100 |
| | 197 | 198 | 73 S | 100 |
| | 201 | 202 | 57 S | 100 |
| | 253 | 254 | 70 S | 32 |
| | 205 | 206 | 83 S | 21 |
| | 209 | 210 | 72 S | 70 |
| | 215 | 216 | 84 S | 19 |
| | 217 | 218 | 91 S | 100 |
| | 219 | 220 | 65 S | 93 |
| | 221 | 222 | 54 S | 100 |
| | 223 | 224 | 69 S | 37 |
| | 225 | 226 | 82 S | 15 |
| | 227 | 228 | 83 S | 23 |
| Clone | 229 | 230 | 78 S | 20 |
| Subclone | 229 | 230 | 74 S | 100 |
| | 231 | 232 | 81 S | 21 |
| | 233 | 234 | 84 S | 19 |
| | 235 | 236 | 61 S | 82 |
| | 237 | 238 | 51 S | 90 |
| | 239 | 240 | 89 S | 16 |
| | 241 | 242 | 24 S | 66 |
| | 243 | 244 | 88 S | 16 |
| | 245 | 246 | 74 S | 23 |
| | 247 | 248 | 72 S | 78 |
| | 249 | 250 | 93 S | 100 |
| | 251 | 252 | 71 S | 17 |

| **Seq ID DNA** | **Seq ID Protei n** | **2-amino hexanoic acid** | |
|---|---|---|---|
| | | **Yield** | **EE %** **(enantiomer)** |
| 5 | 6 | 27% | 11% R |
| 7 | 8 | 9% | 45% S |
| 333 | 334 | 36% | 21% R |
| 11 | 12 | 9% | 58% S |
| 13 | 14 | 23% | 3% S |
| 293 | 294 | 28% | 9% R |
| 47 | 48 | 20% | 15%S |
| 55 | 56 | 27% | 14% R |
| 61 | 62 | 9% | 46% S |
| 261 | 262 | 17% | 39% S |
| 267 | 268 | 11% | 83% S |
| 97 | 98 | 21% | 15%S |
| 283 | 284 | 29% | 20% R |
| 343 | 344 | 21% | 79% R |
| 101 | 102 | 26% | 1 % R |
| 103 | 104 | 8% | 91% S |
| 385 | 386 | 20% | 24% R |
| 303 | 304 | 24% | 13% S |
| 145 | 146 | 29% | 10% R |
| 159 | 160 | 14% | 40% S |
| 167 | 168 | 21% | 16% S |
| 169 | 170 | 12% | 53% S |
| 185 | 186 | 37% | 29% S |
| 187 | 188 | 25% | 13% R |
| 189 | 190 | 14% | 68% S |
| 197 | 198 | 28% | 21% R |
| 217 | 218 | 22% | 13% R |
| 221 | 222 | 17% | 54% S |
| 249 | 250 | 20% | 29% S |

While the invention has been described in detail with reference to certain preferred embodiments thereof, it will be understood that modifications and variations are within the spirit and scope of that which is described and claimed.

## Claims

1. An isolated, synthetic or recombinant nucleic acid comprising
(a) a nucleic acid sequence having a sequence at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or 100% sequence identity to SEQ ID NO:385, SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO:55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 123, SEQ ID NO: 125, SEQ ID NO: 127, SEQ ID NO:129, SEQ ID NO: 131, SEQ ID NO: 133, SEQ ID NO: 135, SEQ ID NO: 137, SEQ ID NO: 139, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, SEQ ID NO: 163, SEQ ID NO: 165, SEQ ID NO: 167, SEQ ID NO: 169, SEQ ID NO: 171, SEQ ID NO: 173, SEQ ID NO: 175, SEQ ID NO: 177, SEQ ID NO: 179, SEQ ID NO: 181, SEQ ID NO: 183, SEQ ID NO: 185, SEQ ID NO: 187, SEQ ID NO: 189, SEQ ID NO: 191, SEQ ID NO: 193, SEQ ID NO: 195, SEQ ID NO: 197, SEQ ID NO: 199, SEQ ID NO: 201, SEQ ID NO: 203, SEQ ID NO: 205, SEQ ID NO: 207, SEQ ID NO: 209, SEQ ID NO: 211, SEQ ID NO: 213, SEQ ID NO: 215, SEQ ID NO: 217, SEQ ID NO: 219, SEQ ID NO: 221, SEQ ID NO: 223, SEQ ID NO: 225, SEQ ID NO: 227, SEQ ID NO: 229, SEQ ID NO: 231, SEQ ID NO: 233, SEQ ID NO: 235, SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 265, SEQ ID NO: 267, SEQ ID NO: 269, SEQ ID NO: 271, SEQ ID NO: 273, SEQ ID NO: 275, SEQ ID NO: 277, SEQ ID NO: 279, SEQ ID NO: 281, SEQ ID NO: 283, SEQ ID NO: 285, SEQ ID NO: 287, SEQ ID NO: 289, SEQ ID NO: 291, SEQ ID NO: 293, SEQ ID NO: 295, SEQ ID NO: 297, SEQ ID NO: 299, SEQ ID NO: 301, SEQ ID NO: 303, SEQ ID NO: 305, SEQ ID NO: 307, SEQ ID NO: 309, SEQ ID NO: 311, SEQ ID NO: 313, SEQ ID NO: 315, SEQ ID NO: 317, SEQ ID NO: 319, SEQ ID NO: 321, SEQ ID NO: 323, SEQ ID NO: 325, SEQ ID NO: 327, SEQ ID NO: 329, SEQ ID NO: 331, SEQ ID NO: 333, SEQ ID NO: 335, SEQ ID NO: 337, SEQ ID NO: 339, SEQ ID NO: 341, SEQ ID NO: 343, SEQ ID NO: 345, SEQ ID NO: 347, SEQ ID NO: 349, SEQ ID NO: 351, SEQ ID NO: 353, SEQ ID NO:355, SEQ ID NO: 357, SEQ ID NO:359, SEQ ID NO:361, SEQ ID NO:363, SEQ ID NO: 365, SEQ ID NO:367, SEQ ID NO:369, SEQ ID NO:371, SEQ ID NO:373, SEQ ID NO:375, SEQ ID NO: 377, SEQ ID NO: 379, SEQ ID NO: 381, or SEQ ID NO: 383, wherein the nucleic acid encodes at least one polypeptide having nitrilase activity,
and optionally the sequence identities are determined by analysis with a sequence comparison algorithm or by a visual inspection, wherein the sequence comparison algorithm is a BLAST version 2.2.2 algorithm where a filtering setting is set to blastall -p blastp -d "nr pataa" -F F, and all other options are set to default;
(b) a nucleic acid sequence comprises a sequence as set forth in SEQ ID NO:385, SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO:55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 123, SEQ ID NO: 125, SEQ ID NO: 127, SEQ ID NO:129, SEQ ID NO: 131, SEQ ID NO: 133, SEQ ID NO: 135, SEQ ID NO: 137, SEQ ID NO: 139, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, SEQ ID NO: 163, SEQ ID NO: 165, SEQ ID NO: 167, SEQ ID NO: 169, SEQ ID NO: 171, SEQ ID NO: 173, SEQ ID NO: 175, SEQ ID NO: 177, SEQ ID NO: 179, SEQ ID NO: 181, SEQ ID NO: 183, SEQ ID NO: 185, SEQ ID NO: 187, SEQ ID NO: 189, SEQ ID NO: 191, SEQ ID NO: 193, SEQ ID NO: 195, SEQ ID NO: 197, SEQ ID NO: 199, SEQ ID NO: 201, SEQ ID NO: 203, SEQ ID NO: 205, SEQ ID NO: 207, SEQ ID NO: 209, SEQ ID NO: 211, SEQ ID NO: 213, SEQ ID NO: 215, SEQ ID NO: 217, SEQ ID NO: 219, SEQ ID NO: 221, SEQ ID NO: 223, SEQ ID NO: 225, SEQ ID NO: 227, SEQ ID NO: 229, SEQ ID NO: 231, SEQ ID NO: 233, SEQ ID NO: 235, SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 265, SEQ ID NO: 267, SEQ ID NO: 269, SEQ ID NO: 271, SEQ ID NO: 273, SEQ ID NO: 275, SEQ ID NO: 277, SEQ ID NO: 279, SEQ ID NO: 281, SEQ ID NO: 283, SEQ ID NO: 285, SEQ ID NO: 287, SEQ ID NO: 289, SEQ ID NO: 291, SEQ ID NO: 293, SEQ ID NO: 295, SEQ ID NO: 297, SEQ ID NO: 299, SEQ ID NO: 301, SEQ ID NO: 303, SEQ ID NO: 305, SEQ ID NO: 307, SEQ ID NO: 309, SEQ ID NO: 311, SEQ ID NO: 313, SEQ ID NO: 315, SEQ ID NO: 317, SEQ ID NO: 319, SEQ ID NO: 321, SEQ ID NO: 323, SEQ ID NO: 325, SEQ ID NO: 327, SEQ ID NO: 329, SEQ ID NO: 331, SEQ ID NO: 333, SEQ ID NO: 335, SEQ ID NO: 337, SEQ ID NO: 339, SEQ ID NO: 341, SEQ ID NO: 343, SEQ ID NO: 345, SEQ ID NO: 347, SEQ ID NO: 349, SEQ ID NO: 351, SEQ ID NO: 353, SEQ ID NO:355, SEQ ID NO: 357, SEQ ID NO:359, SEQ ID NO:361, SEQ ID NO:363, SEQ ID NO: 365, SEQ ID NO:367, SEQ ID NO:369, SEQ ID NO:371, SEQ ID NO:373, SEQ ID NO:375, SEQ ID NO: 377, SEQ ID NO: 379, SEQ ID NO: 381, or SEQ ID NO: 383;
(c) a nucleic acid sequence encodes a polypeptide having a nitrilase activity, or encodes a polypeptide or peptide capable of generating an antibody that specifically binds to SEQ ID NO:386, SEQ ID NO:2, SEQ ID NO:4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO:24, SEQ ID NO: 26, SEQ ID NO: 28, SEQ ID NO: 30, SEQ ID NO: 32, SEQ ID NO: 34, SEQ ID NO:36, SEQ ID NO: 38, SEQ ID NO:40, SEQ ID NO: 42, SEQ ID NO: 44, SEQ ID NO:46, SEQ ID NO:48, SEQ ID NO:50, SEQ ID NO:52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ 1D NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, SEQ ID NO: 114, SEQ ID NO: 116, SEQ ID NO:118, SEQ ID NO:120, SEQ ID NO:122, SEQ ID NO:124, SEQ ID NO:126, SEQ ID NO:128, SEQ ID NO:130, SEQ ID NO: 132, SEQ ID NO: 134, SEQ ID NO: 136, SEQ ID NO: 138, SEQ ID NO: 140, SEQ ID NO: 142, SEQ ID NO:144, SEQ ID NO: 146, SEQ ID NO: 148, SEQ ID NO:150, SEQ ID NO: 152, SEQ ID NO: 154, SEQ ID NO: 156, SEQ ID NO: 158, SEQ ID NO: 160, SEQ ID NO: 162, SEQ ID NO: 164, SEQ ID NO: 166, SEQ ID NO: 168, SEQ ID NO: 170, SEQ ID NO: 172, SEQ ID NO: 174, SEQ ID NO: 176, SEQ ID NO: 178, SEQ ID NO: 180, SEQ ID NO: 182, SEQ ID NO: 184, SEQ ID NO: 186, SEQ ID NO: 188, SEQ ID NO: 190, SEQ ID NO: 192, SEQ ID NO: 194, SEQ ID NO: 196, SEQ ID NO: 198, SEQ ID NO:200, SEQ ID NO:202, SEQ ID NO:204, SEQ ID NO:206, SEQ ID NO:208, SEQ ID NO:210, SEQ ID NO:212, SEQ ID NO:214, SEQ ID NO:216, SEQ ID NO:218, SEQ ID NO:220, SEQ ID NO:222, SEQ ID NO:224, SEQ ID NO:226, SEQ ID NO:228, SEQ ID NO:230, SEQ ID NO:232, SEQ ID NO:234, SEQ ID NO:236, SEQ ID NO:238, SEQ ID NO:240, SEQ ID NO:242, SEQ ID NO:244, SEQ ID NO:246, SEQ ID NO:248, SEQ ID NO:250, SEQ ID NO:252, SEQ ID NO:254, SEQ ID NO:256, SEQ ID NO:258, SEQ ID NO:260, SEQ ID NO:262, SEQ ID NO:264, SEQ ID NO:266, SEQ ID NO:268, SEQ ID NO:270, SEQ ID NO:272, SEQ ID NO:274, SEQ ID NO: 276, SEQ ID NO:278, SEQ ID NO:280, SEQ ID NO:282, SEQ ID NO:284, SEQ ID NO:286, SEQ ID NO:288, SEQ ID NO:290, SEQ ID NO:292, SEQ ID NO:294, SEQ ID NO:296, SEQ ID NO:298, SEQ ID NO:300, SEQ ID NO:302, SEQ ID NO:304, SEQ ID NO:306, SEQ ID NO:308, SEQ ID NO:310, SEQ ID NO:312, SEQ ID NO:314, SEQ ID NO:316, SEQ ID NO: 318, SEQ ID NO: 320, SEQ ID NO:322, SEQ ID NO:324, SEQ ID NO:326, SEQ ID NO:328, SEQ ID NO:330, SEQ ID NO: 332, SEQ ID NO:334, SEQ ID NO:336, SEQ ID NO:338, SEQ ID NO:340, SEQ ID NO:342, SEQ ID NO:344, SEQ ID NO:346, SEQ ID NO:348, SEQ ID NO:350, SEQ ID NO:352, SEQ ID NO: 354, SEQ ID NO:356, SEQ ID NO:358, SEQ ID NO:360, SEQ ID NO:362, SEQ ID NO:364, SEQ ID NO:366, SEQ ID NO:368, SEQ ID NO:370, SEQ ID NO:372, SEQ ID NO:374, SEQ ID NO:376, SEQ ID NO:378, SEQ ID NO:380, SEQ ID NO:382, or SEQ ID NO:384, or enzymatically active fragments thereof;
(d) a sequence that hybridizes under stringent conditions to a nucleic acid comprising SEQ ID NO:385, SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO:55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 123, SEQ ID NO: 125, SEQ ID NO: 127, SEQ ID NO:129, SEQ ID NO: 131, SEQ ID NO: 133, SEQ ID NO: 135, SEQ ID NO: 137, SEQ ID NO: 139, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, SEQ ID NO: 163, SEQ ID NO: 165, SEQ ID NO: 167, SEQ ID NO: 169, SEQ ID NO: 171, SEQ ID NO: 173, SEQ ID NO: 175, SEQ ID NO: 177, SEQ ID NO: 179, SEQ ID NO: 181, SEQ ID NO: 183, SEQ ID NO: 185, SEQ ID NO: 187, SEQ ID NO: 189, SEQ ID NO: 191, SEQ ID NO: 193, SEQ ID NO: 195, SEQ ID NO: 197, SEQ ID NO: 199, SEQ ID NO: 201, SEQ ID NO: 203, SEQ ID NO: 205, SEQ ID NO: 207, SEQ ID NO: 209, SEQ ID NO: 211, SEQ ID NO: 213, SEQ ID NO: 215, SEQ ID NO: 217, SEQ ID NO: 219, SEQ ID NO: 221, SEQ ID NO: 223, SEQ ID NO: 225, SEQ ID NO: 227, SEQ ID NO: 229, SEQ ID NO: 231, SEQ ID NO: 233, SEQ ID NO: 235, SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 265, SEQ ID NO: 267, SEQ ID NO: 269, SEQ ID NO: 271, SEQ ID NO: 273, SEQ ID NO: 275, SEQ ID NO: 277, SEQ ID NO: 279, SEQ ID NO: 281, SEQ ID NO: 283, SEQ ID NO: 285, SEQ ID NO: 287, SEQ ID NO: 289, SEQ ID NO: 291, SEQ ID NO: 293, SEQ ID NO: 295, SEQ ID NO: 297, SEQ ID NO: 299, SEQ ID NO: 301, SEQ ID NO: 303, SEQ ID NO: 305, SEQ ID NO: 307, SEQ ID NO: 309, SEQ ID NO: 311, SEQ ID NO: 313, SEQ ID NO: 315, SEQ ID NO: 317, SEQ ID NO: 319, SEQ ID NO: 321, SEQ ID NO: 323, SEQ ID NO: 325, SEQ ID NO: 327, SEQ ID NO: 329, SEQ ID NO: 331, SEQ ID NO: 333, SEQ ID NO: 335, SEQ ID NO: 337, SEQ ID NO: 339, SEQ ID NO: 341, SEQ ID NO: 343, SEQ ID NO: 345, SEQ ID NO: 347, SEQ ID NO: 349, SEQ ID NO: 351, SEQ ID NO: 353, SEQ ID NO:355, SEQ ID NO: 357, SEQ ID NO:359, SEQ ID NO:361, SEQ ID NO:363, SEQ ID NO: 365, SEQ ID NO:367, SEQ ID NO:369, SEQ ID NO:371, SEQ ID NO:373, SEQ ID NO:375, SEQ ID NO: 377, SEQ ID NO: 379, SEQ ID NO: 381, or SEQ ID NO: 383, wherein the nucleic acid encodes a polypeptide having a nitrilase activity;
wherein the stringent conditions include a wash step comprising a wash in 0.2X SSC at a temperature of about 65°C for about 15 minutes;
(e) the nucleic acid of (a), (b), (c) or (d) encoding a nitrilase but lacking a signal sequence;
(f) the nucleic acid of (a), (b), (c), (d) or (e) encoding a nitrilase but having a heterologous sequence, wherein optionally the heterologous sequence comprises a heterologous signal sequence, and optionally the heterologous signal sequence is derived from another nitrilase or a non-nitrilase enzyme; or
(g) a sequence complementary to (a), (b), (c), or (d), (e), or (f).

2. An expression cassette, a vector or a cloning vehicle comprising a nucleic acid comprising a sequence as set forth in claim 1.

3. A transformed cell comprising a nucleic acid comprising a sequence as set forth in claim 1 or an expression cassette, a vector or a cloning vehicle as set forth in claim 2.

4. A transgenic organism comprising a sequence as set forth in claim 1 or an expression cassette, a vector or a cloning vehicle as set forth in claim 2.

5. An isolated, synthetic or recombinant polypeptide having nitrilase activity, wherein the polypeptide comprises three consensus subsequences, and the first consensus subsequence is FPEXX (SEQ ID NO:390), the second consensus subsequence is XRKLXPT (SEQ ID NO: 391), and the third consensus subsequence is LXCWEXXXP (SEQ ID NO: 392), wherein the polypeptide comprises:
(a) a sequence having at least comprising consecutive amino acids having least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or has 100% sequence identity to SEQ ID NO:386, SEQ ID NO:2, SEQ ID NO:4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO:24, SEQ ID NO: 26, SEQ ID NO: 28, SEQ ID NO: 30, SEQ ID NO: 32, SEQ ID NO: 34, SEQ ID NO:36, SEQ ID NO: 38, SEQ ID NO:40, SEQ ID NO: 42, SEQ ID NO: 44, SEQ ID NO:46, SEQ ID NO:48, SEQ ID NO:50, SEQ ID NO:52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, SEQ ID NO: 114, SEQ ID NO: 116, SEQ ID NO:118, SEQ ID NO: 120, SEQ ID NO:122, SEQ ID NO:124, SEQ ID NO:126, SEQ ID NO:128, SEQ ID NO:130, SEQ ID NO:132, SEQ ID NO: 134, SEQ ID NO: 136, SEQ ID NO: 138, SEQ ID NO:140, SEQ ID NO: 142, SEQ ID NO:144, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO:162, SEQ ID NO:164, SEQ ID NO:166, SEQ ID NO:168, SEQ ID NO:170, SEQ ID NO:172, SEQ ID NO:174, SEQ ID NO:176, SEQ ID NO:178, SEQ ID NO:180, SEQ ID NO:182, SEQ ID NO:184, SEQ ID NO:186, SEQ ID NO:188, SEQ ID NO: 190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:210, SEQ ID NO:212, SEQ ID NO:214, SEQ ID NO:216, SEQ ID NO:218, SEQ ID NO:220, SEQ ID NO:222, SEQ ID NO:224, SEQ ID NO:226, SEQ ID NO:228, SEQ ID NO:230, SEQ ID NO:232, SEQ ID NO:234, SEQ ID NO:236, SEQ ID NO:238, SEQ ID NO:240, SEQ ID NO:242, SEQ ID NO:244, SEQ ID NO:246, SEQ ID NO:248, SEQ ID NO:250, SEQ ID NO:252, SEQ ID NO:254, SEQ ID NO:256, SEQ ID NO:258, SEQ ID NO:260, SEQ ID NO:262, SEQ ID NO:264, SEQ ID NO:266, SEQ ID NO:268, SEQ ID NO:270, SEQ ID NO:272, SEQ ID NO:274, SEQ ID NO: 276, SEQ ID NO:278, SEQ ID NO:280, SEQ ID NO:282, SEQ ID NO:284, SEQ ID NO:286, SEQ ID NO:288, SEQ ID NO:290, SEQ ID NO:292, SEQ ID NO:294, SEQ ID NO:296, SEQ ID NO:298, SEQ ID NO:300, SEQ ID NO:302, SEQ ID NO:304, SEQ ID NO:306, SEQ ID NO:308, SEQ ID NO:310, SEQ ID NO:312, SEQ ID NO:314, SEQ ID NO:316, SEQ ID NO: 318, SEQ ID NO: 320, SEQ ID NO:322, SEQ ID NO:324, SEQ ID NO:326, SEQ ID NO:328, SEQ ID NO:330, SEQ ID NO: 332, SEQ ID NO:334, SEQ ID NO:336, SEQ ID NO:338, SEQ ID NO:340, SEQ ID NO:342, SEQ ID NO:344, SEQ ID NO:346, SEQ ID NO:348, SEQ ID NO:350, SEQ ID NO:352, SEQ ID NO: 354, SEQ ID NO:356, SEQ ID NO:358, SEQ ID NO:360, SEQ ID NO:362, SEQ ID NO:364, SEQ ID NO:366, SEQ ID NO:368, SEQ ID NO:370, SEQ ID NO:372, SEQ ID NO:374, SEQ ID NO:376, SEQ ID NO:378, SEQ ID NO:380, SEQ ID NO:382, or SEQ ID NO:384, or an enzymatically active fragment thereof, wherein the polypeptide has nitrilase activity;
(b) a polypeptide encoded by a nucleic acid having a sequence as set forth in claim 1;
(c) the polypeptide of (a) or (b), but lacking a signal sequence;
(d) the polypeptide of (a), (b) or(c), but having a heterologous sequence wherein optionally the heterologous sequence comprises a heterologous signal sequence, and optionally the heterologous signal sequence is derived from another nitrilase or a non-nitrilase enzyme.

6. The isolated, synthetic or recombinant polypeptide of claim 5, wherein the second consensus subsequences is replaced by the sequence RRKLXPT (SEQ ID NO: 388), and the third consensus subsequence is replaced by the sequence LXCWEHXXP (SEQ ID NO:389).

7. An enzyme preparation comprising a polypeptide as set forth in claim 5, wherein the preparation is a liquid or a dry preparation.

8. The isolated, synthetic or recombinant polypeptide of claim 5, wherein the polypeptide is made by a method comprising introducing a nucleic acid encoding the polypeptide into a host cell, wherein the host cell produces the polypeptide and optionally the method further comprises recovering the polypeptide.

9. A method of generating a variant of a nucleic acid encoding a polypeptide with a nitrilase activity comprising the steps of:
(a) providing a template nucleic acid comprising a sequence as set forth in claim 1; and
(b) modifying, deleting or adding one or more nucleotides in the template sequence, or a combination thereof, to generate a variant of the template nucleic acid,
optionally further comprising expressing the variant nucleic acid to generate a variant nitrilase polypeptide,
optionally wherein the modifications, additions or deletions are introduced by a method comprising error-prone PCR, shuffling, oligonucleotide-directed mutagenesis, assembly PCR, sexual PCR mutagenesis, *in vivo* mutagenesis, cassette mutagenesis, recursive ensemble mutagenesis, exponential ensemble mutagenesis, site-specific mutagenesis, gene reassembly, gene site saturation mutagenesis (GSSM™), synthetic ligation reassembly (SLR) and a combination thereof,
optionally wherein the modifications, additions or deletions are introduced by a method comprising recombination, recursive sequence recombination, phosphothioate-modified DNA mutagenesis, uracil-containing template mutagenesis, gapped duplex mutagenesis, point mismatch repair mutagenesis, repair-deficient host strain mutagenesis, chemical mutagenesis, radiogenic mutagenesis, deletion mutagenesis, restriction-selection mutagenesis, restriction-purification mutagenesis, artificial gene synthesis, ensemble mutagenesis, chimeric nucleic acid multimer creation and a combination thereof,
optionally wherein the method is iteratively repeated until a nitrilase having an altered or different activity or an altered or different stability from that of a polypeptide encoded by the template nucleic acid is produced,
optionally wherein the variant nitrilase polypeptide is thermotolerant, and retains some activity after being exposed to an elevated temperature,
optionally wherein the variant nitrilase polypeptide has increased glycosylation as compared to the nitrilase encoded by a template nucleic acid,
optionally wherein the variant nitrilase polypeptide has a nitrilase activity under a high temperature, wherein the nitrilase encoded by the template nucleic acid is not active under the high temperature,
optionally wherein the method is iteratively repeated until a nitrilase coding sequence having an altered codon usage from that of the template nucleic acid is produced,
optionally wherein the method is iteratively repeated until a nitrilase gene having higher or lower level of message expression or stability from that of the template nucleic acid is produced.

10. An industrial process comprising using the isolated, synthetic or recombinant polypeptide of claim 5 in the hydrolysis of a substrate having a cyanohydrin moiety or an aminonitrile moiety to generate a composition comprising an alpha-hydroxy acid or an amino acid,
wherein optionally the industrial process further comprises the production of a pharmaceutical composition comprising the generated alpha-hydroxy acid or amino acid,
wherein optionally the industrial process further comprises the production of a chemical comprising the generated alpha-hydroxy acid or amino acid,
wherein optionally the industrial process further comprises catalyzing the breakdown of a waste, wherein the breakdown comprises hydrolysis of a substrate having a cyanohydrin moiety or an aminonitrile moiety,
wherein optionally the industrial process further comprises the production of a drug intermediate, wherein the drug intermediate comprises the generated alpha-hydroxy acid or amino acid, and wherein optionally the polypeptide is used to hydrolyze a hydroxygultarylnitrile substrate and wherein optionally the industrial process is used for the production of LIPITOR^{™},
wherein optionally the industrial process further comprises the production of a detergent, or
wherein optionally the industrial process further comprises the production of a food product.

11. A method for modifying a molecule comprising:
(a) mixing a polypeptide of claim 5 with a starting molecule to produce a reaction mixture; and
(b) reacting the starting molecule with the polypeptide to produce the modified molecule.

12. A method for identifying a modified compound comprising:
(a) admixing a polypeptide of claim 5 with a starting compound to produce a reaction mixture and thereafter a library of modified starting compounds;
(b) testing the library to determine whether a modified starting compound is present within the library which exhibits a desired activity; and
(c) identifying the modified compound exhibiting the desired activity.

13. An assay for identifying a functional fragment of a polypeptide which comprises:
(a) obtaining a fragment of at least one polypeptide of claim 5;
(b) contacting at least one fragment from step (a) with a substrate having a cyanohydrin moiety or an aminonitrile moiety under reaction conditions suitable for nitrilase activity;
(c) measuring the amount of reaction product produced by each at least one fragment from step (b); and
(d) identifying the at least one fragment which is capable of producing a nitrilase reaction product; thereby identifying a functional fragment of the polypeptide,
wherein optionally the fragment of step (a) is obtained by synthesizing a fragment, or
wherein optionally the fragment of step (a) is obtained by fragmenting the polypeptides.

14. An assay for identifying a functional variant of a polypeptide which comprises:
(a) obtaining at least one variant of at least one polypeptide of claim 5;
(b) contacting at least one variant from step (a) with a substrate having a cyanohydrin moiety or an aminonitrile moiety under reaction conditions suitable for nitrilase activity;
(c) measuring the amount of reaction product produced by each at least one variant from step (b); and
(d) identifying the at least one variant which is capable of producing a nitrilase reaction product; thereby identifying a functional variant of the polypeptide.

15. The isolated, synthetic or recombinant polypeptide of claim 5 encoded by a nucleic acid expressed in a host cell, wherein the host cell comprises a gram negative bacteria, a gram positive bacteria or a eukaryotic organism;
wherein optionally the gram negative bacteria comprises *Escherichia coli*;
wherein optionally the gram positive bacteria comprise *Streptomyces diversa, Lactobacillus gasseri, Lactococcus lactis, Lactococcus cremoris*, or *Bacillus subtilis*; or
wherein optionally the eukaryotic organism comprises *Saccharomyces cerevisiae, Schizosaccharomyces pombe, Pichia pastoris, Kluyveromyces lactis, Hansenula polymorpha*, or *Aspergillus niger.*

16. A screening assay for identifying a nitrilase, the assay comprising:
(a) providing a plurality of nucleic acids or polypeptides comprising at least one of the nucleic acids of claim 1, or at least one of the polypeptides of claim 5;
(b) obtaining polypeptide candidates to be tested for nitrilase activity from the plurality;
(c) testing the candidates for nitrilase activity; and identifying those polypeptide candidates which are nitrilases.

17. The isolated, synthetic or recombinant polypeptide of claim 5, wherein the nitrilase activity comprises:
(a) hydrolyzing a nitrile to a carboxylic acid;
(b) hydrolyzing a cyanhydrin moiety or an aminonitrile moiety of a molecule,
wherein optionally the molecule comprises (*S*)-2-amino-40 phenyl bunatoic acid,
wherein optionally the molecule comprises an L-amino acid, or
wherein optionally the molecule comprises a food additive or a pharmaceutical drug;
(c) an enantio-selective nitrilase activity wherein the enantio-selective activity comprises generating a chiral alpha-hydroxy acid molecule or a chiral amino acid molecule by catalyzing the hydrolysis of a molecule having a cyanohydrin moiety or an aminonitrile moiety,
wherein optionally the generated chiral molecule is an (*R*)-enantiomer or an (*S*)-enantiomer;
(d) hydrolyzing a hydroxyglutaryl nitrile to generate an (*R*)-ethyl 4-cyano-3-hydroxybutyric acid,
wherein optionally the polypeptide enantio-selectively hydrolyzes the hydroxyglutaryl nitrile to generate an (*R*)-enantiomer (*R*)-ehtyl 4-cyano-3-hydroxybutyric acid,
wherein optionally the polypeptide produces a product having an enantiomeric purity comprising an ee of at least 95%, 96%, 97%, 98% or 99%, and
wherein optionally the hydroxyglutaryl nitrile comprises 1,3-di-cyano-2-hydroxypropane or a hydoxyglutaronitile, or
wherein optionally the hydroxyglutaronitrile comprises a 3-hydroxyglutaronitrile;
(e) hydrolyzing a hydroxyglutaryl nitrile to generate an ethyl 4-cyano-3-hydroxybutyric acid,
wherein optionally the polypeptide enantio-selectively hydrolyzes the hydroxyglutaryl nitrile to generate an (*S*)-enantiomer (*S*)-ethyl 4-cyano-3-hydroxybutyric acid, and
wherein optionally the polypeptide produces a product having an enantiomeric purity comprising an ee of 95%, 96%, 97%, 98% or 99%;
(f) hydrolyzing a mandelonitrile to generate a mandelic acid or mandelic acid derivative,
wherein optionally the mandelic acid comprises (*R*)-2-chloromandelic acid,
wherein optionally the mandelic acid comprises an aromatic ring substitution in the *ortho-, meta-* or *para-* positions; a 1-paphthyl derivative of (*R*)-mandelic acid, a pyridyl derivative of (*R*)-mandelic acid or thienyl derivative of (*R*)-mandelic acid or a combination thereof,
wherein optionally the mandelic acid comprises (*S*)-methyl benzyl cyanide and the mandelonitrile comprises (*S*)-methoxy-benzyl cyanide, or
wherein optionally the mandelic acid comprises an aromatic ring substitution in the *ortho-, meta-* or *para-* positions; a 1-paphthyl derivative of (*S*)-mandelic acid, a pyridyl derivative of (*S*)-mandelic acid or thienyl derivative of (*S*)-mandelic acid or a combination thereof; or
(g) hydrolyzing a phenyllactocyanonitrile to generate an (*S*)-phenyl lactic acid derivative or an (*R*)-phenyl lactic acid derivative.

18. A method for making a carboxylic acid comprising hydrolyzing a nitrile using the isolated, synthetic or recombinant polypeptide of claim 17, under aqueous conditions.


SEQUENCE LISTING

<110> Short, Jay
      Weiner, David
      Chaplin, Jennifer
      Chi, Ellen
      Milan, Aileen
      DeSantis, Grace
      Madden, Mark
      Burk, Mark

<120> Nitrilases

<130> Docket No. DIV-013US

<140> Unknown
<141> 2002-05-15

<150> US 60/309,006
<151> 2001-07-30

<150> US 60/351,336
<151> 2002-01-22

<150> US 60/300,189
<151> 2001-06-21

<150> US 09/751,299
<151> 2000-12-28

<150> US 60/254,414
<151> 2000-12-07

<150> US 60/173,609
<151> 1999-12-29

<160> 386

<170> FastSEQ for Windows Version 4.0

<210> 1
<211> 939
<212> DNA
<213> Unknown

<220>
<223> Obtained from an environmental sample

<400> 1
   atggaaaagt atattaaagt cgccgcaatt cagatagcta caaaaatagc agattcaccc 60
   gtgaatatag aaaattgcga acgtttggca ttatcggcgg tcaatgaggg tgcgcgttgg 120
   attgctttgc cggagttctt caatacgggc gttagttgga acaaaaaaat tgccttggct 180
   attcagacgc ctgacggcaa ggctgcgatg ttcttacgcg acttttctgc aagacatcat 240
   gtattgatag gaggctcatt tctgtgcagg ttgccggatg gcagtgtgcg caaccgctat 300
   atgtgttatg ccaacggcgc tctcgtgggc aaacatgaca aagacctacc cacgatgtgg 360
   gaaaatgctt tttatgaagg tggggattcc agcgatattg gggtgctggg aacatttgaa 420
   aatacgcgcg ttggtgcagc cgtctgttgg gagttcatgc ggacgatgac tgcccggcgt 480
   cttcgcaatc aggtggatgt catcatgggt ggttcctgct ggtggagcat accgaccaat 540
   ttccccggtt ttgtgcaaaa gctgtgggaa cctggaaata gccgcaacgc gcttgctgcc 600
   atacaggata atgcgcgtct cattggcgtg ccggttgttc atgccgctca ttgcggtgaa 660
   attgagtgtc cgatgccagg attgccgata ggttacaggg ggttctttga gggtaacgcg 720
   gccattgtga atgcagaagg tcaggtgctt gcgcatcggg gtgctggcga gggcgaagga 780
   attgtttgcg cggagatttt accggtagcc aaatcaaaca ggtcggaaat tcccaatcgt 840
   tactggttgc gctgcagagg ctttctacct atttttgcct ggcatcagca acgttggttg 900
   ggaaggcatt ggtatttgcg caatgtgcgc aggacttaa 939

<210> 2
<211> 312
<212> PRT
<213> Unknown

<220>
<223> Obtained from an environmental sample

<400> 2
Met Glu Lys Tyr Ile Lys Val Ala Ala Ile Gln Ile Ala Thr Lys Ile
1               5                   10                  15
Ala Asp Ser Pro Val Asn Ile Glu Asn Cys Glu Arg Leu Ala Leu Ser
            20                  25                  30
Ala Val Asn Glu Gly Ala Arg Trp Ile Ala Leu Pro Glu Phe Phe Asn
        35                  40                  45
Thr Gly Val Ser Trp Asn Lys Lys Ile Ala Leu Ala Ile Gln Thr Pro
    50                  55                  60
Asp Gly Lys Ala Ala Met Phe Leu Arg Asp Phe Ser Ala Arg His His
65                  70                  75                  80
Val Leu Ile Gly Gly Ser Phe Leu Cys Arg Leu Pro Asp Gly Ser Val
                85                  90                  95
Arg Asn Arg Tyr Met Cys Tyr Ala Asn Gly Ala Leu Val Gly Lys His
            100                 105                 110
Asp Lys Asp Leu Pro Thr Met Trp Glu Asn Ala Phe Tyr Glu Gly Gly
        115                 120                 125
Asp Ser Ser Asp Ile Gly Val Leu Gly Thr Phe Glu Asn Thr Arg Val
    130                 135                 140
Gly Ala Ala Val Cys Trp Glu Phe Met Arg Thr Met Thr Ala Arg Arg
145                 150                 155                 160
Leu Arg Asn Gln Val Asp Val Ile Met Gly Gly Ser Cys Trp Trp Ser
                165                 170                 175
Ile Pro Thr Asn Phe Pro Gly Phe Val Gln Lys Leu Trp Glu Pro Gly
            180                 185                 190
Asn Ser Arg Asn Ala Leu Ala Ala Ile Gln Asp Asn Ala Arg Leu Ile
        195                 200                 205
Gly Val Pro Val Val His Ala Ala His Cys Gly Glu Ile Glu Cys Pro
    210                 215                 220
Met Pro Gly Leu Pro Ile Gly Tyr Arg Gly Phe Phe Glu Gly Asn Ala
225                 230                 235                 240
Ala Ile Val Asn Ala Glu Gly Gln Val Leu Ala His Arg Gly Ala Gly
                245                 250                 255
Glu Gly Glu Gly Ile Val Cys Ala Glu Ile Leu Pro Val Ala Lys Ser
            260                 265                 270
Asn Arg Ser Glu Ile Pro Asn Arg Tyr Trp Leu Arg Cys Arg Gly Phe
        275                 280                 285

Leu Pro Ile Phe Ala Trp His Gln Gln Arg Trp Leu Gly Arg His Trp
    290                 295                 300
Tyr Leu Arg Asn Val Arg Arg Thr
305                 310

<210> 3
<211> 981
<212> DNA
<213> Unknown

<220>
<223> Obtained from an environmental sample

<400> 3
atggggaatt cgttcaagat cgcggtggta caagcctgtc cggtctttct ggatcgtggc      60
gcgacagtcg ccaaggcatg ccgcctgatc gcggaggcag ccgcggcggg cgccagcctg     120
gtggtctttc cggaggcgtt tgtgcccgga tacccactgt gggtctggtt cattccggca     180
gggcattcgc aaccactgcg ggagttatac gccgaactgg tggggaacgc cgtggcggta     240
ccgggcgatg ccaccgatcg gctttgcgcg gcagccagag aagccggcgt ggtagtggcg     300
atcggcatca atgaagtgaa cagcgaagcc agcggcacga cgatttacaa tacgctgctg     360
tacatcggag cggacggcgc gattctgggc aaacaccgca aagtaatgcc gacgggcgga     420
gagcgcctgg tctgggcgct tggcgatggg agcgacctgg aggtctacga cctgcctttc     480
ggccgattgg gtggcctgtt gtgctgggag aactacatgc ccctggcccg gtacgcgatg     540
tcggcatggg gaaccgagat ctacgtggct ccaacttggg atcgcggaga accgtggctg     600
tccacaatgc ggcatatcgc gaaagaaggg cgatgctacg tagtgggatg ctgcagttgc     660
atgaaaattg acgatgtacc cgaccggctg gcgttcaaag ggaagtatct gtcgacggcc     720
gagggctggc tcaaccccgg cgatagcgta atcgtcgatc cggacggcaa gctgatcgcg     780
ggcccggcaa gcgagcagga gacgattctg tatgccgatg ccgaccggtc taagatcacc     840
gggcccaggt ggcagttgga tgtggccggc cactacgcgc ggccggatat cttcgaactg     900
atcgtgcacc gcgaacctaa gcgatttttg acgatagctc cgcggacgaa ggaggagcgg     960
gagcctgggc cggaggcctg a                                               981

<210> 4
<211> 326
<212> PRT
<213> Unknown

<220>
<223> Obtained from an environmental sample

<400> 4
Met Gly Asn Ser Phe Lys Ile Ala Val Val Gln Ala Cys Pro Val Phe
 1               5                  10                  15
Leu Asp Arg Gly Ala Thr Val Ala Lys Ala Cys Arg Leu Ile Ala Glu
            20                  25                  30
Ala Ala Ala Ala Gly Ala Ser Leu Val Val Phe Pro Glu Ala Phe Val
        35                  40                  45
Pro Gly Tyr Pro Leu Trp Val Trp Phe Ile Pro Ala Gly His Ser Gln
    50                  55                  60
Pro Leu Arg Glu Leu Tyr Ala Glu Leu Val Gly Asn Ala Val Ala Val
65                  70                  75                  80
Pro Gly Asp Ala Thr Asp Arg Leu Cys Ala Ala Ala Arg Glu Ala Gly
                85                  90                  95
Val Val Val Ala Ile Gly Ile Asn Glu Val Asn Ser Glu Ala Ser Gly
            100                 105                 110

Thr Thr Ile Tyr Asn Thr Leu Leu Tyr Ile Gly Ala Asp Gly Ala Ile
        115                 120                 125
Leu Gly Lys His Arg Lys Val Met Pro Thr Gly Gly Glu Arg Leu Val
    130                 135                 140
Trp Ala Leu Gly Asp Gly Ser Asp Leu Glu Val Tyr Asp Leu Pro Phe
145                 150                 155                 160
Gly Arg Leu Gly Gly Leu Leu Cys Trp Glu Asn Tyr Met Pro Leu Ala
                165                 170                 175
Arg Tyr Ala Met Ser Ala Trp Gly Thr Glu Ile Tyr Val Ala Pro Thr
            180                 185                 190
Trp Asp Arg Gly Glu Pro Trp Leu Ser Thr Met Arg His Ile Ala Lys
        195                 200                 205
Glu Gly Arg Cys Tyr Val Val Gly Cys Cys Ser Cys Met Lys Ile Asp
    210                 215                 220
Asp Val Pro Asp Arg Leu Ala Phe Lys Gly Lys Tyr Leu Ser Thr Ala
225                 230                 235                 240
Glu Gly Trp Leu Asn Pro Gly Asp Ser Val Ile Val Asp Pro Asp Gly
                245                 250                 255
Lys Leu Ile Ala Gly Pro Ala Ser Glu Gln Glu Thr Ile Leu Tyr Ala
            260                 265                 270
Asp Ala Asp Arg Ser Lys Ile Thr Gly Pro Arg Trp Gln Leu Asp Val
        275                 280                 285
Ala Gly His Tyr Ala Arg Pro Asp Ile Phe Glu Leu Ile Val His Arg
    290                 295                 300
Glu Pro Lys Arg Phe Leu Thr Ile Ala Pro Arg Thr Lys Glu Glu Arg
305                 310                 315                 320
Glu Pro Gly Pro Glu Ala
                325

<210> 5
<211> 1005
<212> DNA
<213> Unknown

<220>
<223> Obtained from an environmental sample


<400> 5
atgggtacca agcacccggc cttcaaggcc gcagtggtcc aggccgcgcc ggaatggctc      60
gatctcgacc gcaccgtcga caagaccatc gcgctgatcg aggaggccgc cggcgccggc     120
gcgaagctca ttgcgttccc ggaaacctgg attcccggct atccgtggca catctgggtc     180
ggcacgccgg cgtgggcgat cagccgcggc ttcgtgcagc gctacttcga caattcactg     240
gcctacgaca gcccgcaggc ccagcgcatc gcggacgccg cgaagaagaa caagatcacc     300
gtggtgctcg gcctgtcgga gcgcgagggt ggcagccttt atatctcgca gtggctgatt     360
gggccggacg gcgagaccat tgccaagcgc cgcaaactgc gccccaccca cgtcgagcgc     420
accgtgttcg gcgatggcga cggcagccac atcgcggtgc acgagcgtgc tgacatcggc     480
cgcctcggcg cgctgtgctg ctgggagcac atccagccgc tgaccaaata cgccatgtat     540
gcccagaacg agcaggtgca cgtcgccgcc tggccgagct tctcgatgta cgagccgttc     600
gcccacgcgc tcggctggga agtcaacaat gcggcgagca agatctacgc cgtcgaaggc     660
tcgtgtttcg tgctcggcgc atgcgcggtg atctcgcagg cgatggtcga cgaaatgtgc     720
gacaccgagg acaagcgggc gctggtccat gccggcggcg gccacgcggt gatcttcggg     780
ccggacggca gatcgctggc ggacaagatt ccggagaccc aggaaggcct gctctatgcc     840
gacatcgacc tcggcgcaat tggcgtggcc aagaacgcgg ccgatccggc ggggcactac     900
tcgcgcccgg acgtgacgcg gctcctgttc aacaacaagc cggcgcgccg ggtcgagtat     960
ttctcgctgc cggtcgacgc ggtcgagacg ccgccgcagc cctga                    1005

<210> 6
<211> 334
<212> PRT
<213> Unknown

<220>
<223> Obtained from an environmental sample


<400> 6
Met Gly Thr Lys His Pro Ala Phe Lys Ala Ala Val Val Gln Ala Ala
1               5                   10                  15
Pro Glu Trp Leu Asp Leu Asp Arg Thr Val Asp Lys Thr Ile Ala Leu
            20                  25                  30
Ile Glu Glu Ala Ala Gly Ala Gly Ala Lys Leu Ile Ala Phe Pro Glu
        35                  40                  45
Thr Trp Ile Pro Gly Tyr Pro Trp His Ile Trp Val Gly Thr Pro Ala
    50                  55                  60
Trp Ala Ile Ser Arg Gly Phe Val Gln Arg Tyr Phe Asp Asn Ser Leu
65                  70                  75                  80
Ala Tyr Asp Ser Pro Gln Ala Gln Arg Ile Ala Asp Ala Ala Lys Lys
                85                  90                  95
Asn Lys Ile Thr Val Val Leu Gly Leu Ser Glu Arg Glu Gly Gly Ser
            100                 105                 110
Leu Tyr Ile Ser Gln Trp Leu Ile Gly Pro Asp Gly Glu Thr Ile Ala
        115                 120                 125
Lys Arg Arg Lys Leu Arg Pro Thr His Val Glu Arg Thr Val Phe Gly
    130                 135                 140
Asp Gly Asp Gly Ser His Ile Ala Val His Glu Arg Ala Asp Ile Gly
145                 150                 155                 160
Arg Leu Gly Ala Leu Cys Cys Trp Glu His Ile Gln Pro Leu Thr Lys
                165                 170                 175
Tyr Ala Met Tyr Ala Gln Asn Glu Gln Val His Val Ala Ala Trp Pro
            180                 185                 190
Ser Phe Ser Met Tyr Glu Pro Phe Ala His Ala Leu Gly Trp Glu Val
        195                 200                 205
Asn Asn Ala Ala Ser Lys Ile Tyr Ala Val Glu Gly Ser Cys Phe Val
    210                 215                 220
Leu Gly Ala Cys Ala Val Ile Ser Gln Ala Met Val Asp Glu Met Cys
225                 230                 235                 240
Asp Thr Glu Asp Lys Arg Ala Leu Val His Ala Gly Gly Gly His Ala
                245                 250                 255
Val Ile Phe Gly Pro Asp Gly Arg Ser Leu Ala Asp Lys Ile Pro Glu
            260                 265                 270
Thr Gln Glu Gly Leu Leu Tyr Ala Asp Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Phe Asn Asn Lys Pro Ala Arg Arg Val Glu Tyr
305                 310                 315                 320
Phe Ser Leu Pro Val Asp Ala Val Glu Thr Pro Pro Gln Pro
                325                 330

<210> 7
<211> 999
<212> DNA
<213> Unknown

<220>
<223> Obtained from an environmental sample

<400> 7
atgccgagtg attatcatgc tccattcaaa gtagcagttg tccaggcaac tcccgtcttt 60
ctcgatcgca gcgcgacgat tgagaaggca tgtgagctaa ttgcctgtgc tggacgtgag 120
ggcgcacgtc tgatcgtgtt tcctgaagcg ttcattccca cctatcccga ttgggtctgg 180
accattccac ctggggagat gcggctgctt ggcgaactct acacagagtt gcttgccaat 240
gcggtcacga tccccagtaa tgcaacggat aggctctgcc aggctgcgaa acgagctgct 300
gcgtatgtgg tcatgggaat gaacgaacgc aatatcgagg cgagtggaag gagtctctat 360
aacaccctgt tatacatcga tgctcagggc cagatcatgg gcaaacaccg caagttgata 420
cccacagccg gtgagcggct catatgggcg caaggagatg ggagtacatt ccaggtctac 480
gatactcctc tgggcaaact gggagggctc atctgctggg aaaactacat gcctctggct 540
cgctatgcga tgtatgcctg gggcacgcag atttatgtcg ccccgacatg ggatcgtggc 600
aacctctggc tctctactct gcggcatatc gctaaggagg gaggcgtcta tgttcttggt 660
tgtagtatgg tcatgcgcaa gaatgacatt cccgatcact ttgctttcaa agagcagttt 720
tatgctactg tggacgaatg gatcaacgtt ggtgacagcg ccattgtcca tcccgagggg 780
aactttcttg cgggaccggt gcgccacaaa gaagagattc tctatgcaga acttgatcca 840
cgccaatcgt gcggtccggg atggatgctc gatgtggctg ggcactatgc acgccctgat 900
gtgtttgaat tgattgtcca cacagagatg cgacccatga tgaagcaaga agaggtagga 960
ggagaaaata catctgaggg aggtgtacga ttcttgtaa 999

<210> 8
<211> 332
<212> PRT
<213> Unknown

<220>
<223> Obtained from an environmental sample

<400> 8
Met Pro Ser Asp Tyr His Ala Pro Phe Lys Val Ala Val Val Gln Ala
1               5                   10                  15
Thr Pro Val Phe Leu Asp Arg Ser Ala Thr Ile Glu Lys Ala Cys Glu
            20                  25                  30
Leu Ile Ala Cys Ala Gly Arg Glu Gly Ala Arg Leu Ile Val Phe Pro
        35                  40                  45
Glu Ala Phe Ile Pro Thr Tyr Pro Asp Trp Val Trp Thr Ile Pro Pro
    50                  55                  60
Gly Glu Met Arg Leu Leu Gly Glu Leu Tyr Thr Glu Leu Leu Ala Asn
65                  70                  75                  80
Ala Val Thr Ile Pro Ser Asn Ala Thr Asp Arg Leu Cys Gln Ala Ala
                85                  90                  95
Lys Arg Ala Ala Ala Tyr Val Val Met Gly Met Asn Glu Arg Asn Ile
            100                 105                 110
Glu Ala Ser Gly Arg Ser Leu Tyr Asn Thr Leu Leu Tyr Ile Asp Ala
        115                 120                 125
Gln Gly Gln Ile Met Gly Lys His Arg Lys Leu Ile Pro Thr Ala Gly
    130                 135                 140
Glu Arg Leu Ile Trp Ala Gln Gly Asp Gly Ser Thr Phe Gln Val Tyr
145                 150                 155                 160
Asp Thr Pro Leu Gly Lys Leu Gly Gly Leu Ile Cys Trp Glu Asn Tyr
                165                 170                 175
Met Pro Leu Ala Arg Tyr Ala Met Tyr Ala Trp Gly Thr Gln Ile Tyr

180                 185                 190
Val Ala Pro Thr Trp Asp Arg Gly Asn Leu Trp Leu Ser Thr Leu Arg
        195                 200                 205
His Ile Ala Lys Glu Gly Gly Val Tyr Val Leu Gly Cys Ser Met Val
    210                 215                 220
Met Arg Lys Asn Asp Ile Pro Asp His Phe Ala Phe Lys Glu Gln Phe
225                 230                 235                 240
Tyr Ala Thr Val Asp Glu Trp Ile Asn Val Gly Asp Ser Ala Ile Val
                245                 250                 255
His Pro Glu Gly Asn Phe Leu Ala Gly Pro Val Arg His Lys Glu Glu
            260                 265                 270
Ile Leu Tyr Ala Glu Leu Asp Pro Arg Gln Ser Cys Gly Pro Gly Trp
        275                 280                 285
Met Leu Asp Val Ala Gly His Tyr Ala Arg Pro Asp Val Phe Glu Leu
    290                 295                 300
Ile Val His Thr Glu Met Arg Pro Met Met Lys Gln Glu Glu Val Gly
305                 310                 315                 320
Gly Glu Asn Thr Ser Glu Gly Gly Val Arg Phe Leu
                325                 330

<210> 9
<211> 945
<212> DNA
<213> Unknown

<220>
<223> Obtained from an environmental sample


<400> 9
atggcggctc acaagatcgc ggtggttcag gcgcccagcg ttctcctcga tcgcgagggc      60
tcggtcgcgc gcgcggtcac gctgctcgac gaggcggcgg cggccggcgc ccgcctggtc     120
gtgtttccgg aggcctacat ccccggctac ccggactgga tctggcgcct gcgcccctac     180
ccggacgtca agctggccgc cgagctgcac gaacggctgc tcgccaacgc ggtggatctc     240
tccaccgacg tgctggcgcc ggtgctggcg gcggcggcgc gtcacgggct caccgtggtc     300
atgtgcgtgc aggagcgcga cgccggattc agccgcgcca cactttacaa caccgcgctg     360
gtcatcgacg ccgccggcaa gatcgcgaac cggcaccgca agctcatgcc caccaacccc     420
gagcgaatgg tgtggggatt cggtgacgcc tcggggctgc gggtggtgag cacgcccgtc     480
gggcgggtgg gcacgctcct gtgctgggag agctacatgc ccctggcgcg ctgcgcgctc     540
tacgccgagg gggtcgagat ctacgtgacc ccgacctggg actacggcga aggctggcgc     600
gccagcatgc agcacatcgc ccgcgagggg cgctgctggg tggtgaccgc ttgcatgtgc     660
gtgcaggcgc gcgacgtgcc ggccgacttc cccgggcgcg cccagctcta ccccgacgag     720
gaggagtggt tgaaccccgg cgattcgctg gtggtcgacc ccggcggcaa gatcgtggcc     780
ggtccgatgt cgcgcgagaa ggggatcttg tacgcggaga tcgatccgga tcgcgtggcg     840
gggcgcaccg gctcgttcga cgtcgtgggc cactactcgc gtcccgacgt gttccggctg     900
gaggtcgatc ggacaccggc ggcgccggtg agcttcaaaa aatga                     945

<210> 10
<211> 314
<212> PRT
<213> Unknown

<220>
<223> Obtained from an environmental sample


<400> 10

Met Ala Ala His Lys Ile Ala Val Val Gln Ala Pro Ser Val Leu Leu
1               5                   10                  15
Asp Arg Glu Gly Ser Val Ala Arg Ala Val Thr Leu Leu Asp Glu Ala
            20                  25                  30
Ala Ala Ala Gly Ala Arg Leu Val Val Phe Pro Glu Ala Tyr Ile Pro
        35                  40                  45
Gly Tyr Pro Asp Trp Ile Trp Arg Leu Arg Pro Tyr Pro Asp Val Lys
    50                  55                  60
Leu Ala Ala Glu Leu His Glu Arg Leu Leu Ala Asn Ala Val Asp Leu
65                  70                  75                  80
Ser Thr Asp Val Leu Ala Pro Val Leu Ala Ala Ala Ala Arg His Gly
                85                  90                  95
Leu Thr Val Val Met Cys Val Gln Glu Arg Asp Ala Gly Phe Ser Arg
            100                 105                 110
Ala Thr Leu Tyr Asn Thr Ala Leu Val Ile Asp Ala Ala Gly Lys Ile
        115                 120                 125
Ala Asn Arg His Arg Lys Leu Met Pro Thr Asn Pro Glu Arg Met Val
    130                 135                 140
Trp Gly Phe Gly Asp Ala Ser Gly Leu Arg Val Val Ser Thr Pro Val
145                 150                 155                 160
Gly Arg Val Gly Thr Leu Leu Cys Trp Glu Ser Tyr Met Pro Leu Ala
                165                 170                 175
Arg Cys Ala Leu Tyr Ala Glu Gly Val Glu Ile Tyr Val Thr Pro Thr
            180                 185                 190
Trp Asp Tyr Gly Glu Gly Trp Arg Ala Ser Met Gln His Ile Ala Arg
        195                 200                 205
Glu Gly Arg Cys Trp Val Val Thr Ala Cys Met Cys Val Gln Ala Arg
    210                 215                 220
Asp Val Pro Ala Asp Phe Pro Gly Arg Ala Gln Leu Tyr Pro Asp Glu
225                 230                 235                 240
Glu Glu Trp Leu Asn Pro Gly Asp Ser Leu Val Val Asp Pro Gly Gly
                245                 250                 255
Lys Ile Val Ala Gly Pro Met Ser Arg Glu Lys Gly Ile Leu Tyr Ala
            260                 265                 270
Glu Ile Asp Pro Asp Arg Val Ala Gly Ala His Arg Ser Phe Asp Val
        275                 280                 285
Val Gly His Tyr Ser Arg Pro Asp Val Phe Arg Leu Glu Val Asp Arg
    290                 295                 300
Thr Pro Ala Ala Pro Val Ser Phe Lys Lys
305                 310

<210> 11
<211> 966
<212> DNA
<213> Unknown

<220>
<223> Obtained from an environmental sample

<400> 11
atgactggat cttatcctaa agacacactg atcgttgggc tagctcaaat cgctcctgtc      60
tggctggatc gggcggggac actgtcaaag atactggctc aagtccatgc ggcaaatcaa     120
gcgggttgtc atctcgtagc atttggcgaa gggctgcttc ctggatatcc gtttggattt     180
gagcgaacaa atggcgcgct gttcaactcg actgtacaaa aggaaatcca cgcgcattat     240
atggatcagg cggtgcagat cgaagccggt catctcgatc cgctttgtgc aacagccaag     300
aaatttggaa tcaccgttgt actcggatgc atcgaacgcc cactcgatcg gggcggtcac      360

agcttgtatg caagtctggt atatattgat tccgagggca gcattcaatc cgtgcatcgc 420
aaactaatgc caacctacga agaacgactt acctggtcgt caggcgatgg gcacggttta 480
cgagtgcata ccttaggtgc gtttacggtg ggtggtctca actgttggga aaattggatg 540
cccttggcgc gcgcagcgat gtatggtcag ggtgaagatt tacatgttgc gatctggcca 600
ggcggttctc atctcacgca ggatattacc cgctttattg cgctcgaatc acgttcgtac 660
gtattatctg tctccggtct gatgcgcgca accgattttc caaaagatac tccccatctt 720
gcctccatcc tagctaaagg tgaagagatt cttgcgaatg gtggttcttg tattgcaggt 780
cctgacggca agtgggtcgt tgggcctctt gtaggagaag agaagttaat tgtcgcaacc 840
attgatcact gccgcgtgcg cgaagaacgt cagaatttcg atccttccgg gcattacagc 900
cggcccgatg tactgcaatt aaaaatcaac agggaacgcc agagcacaat ttcatttagc 960
gagtag 966

<210> 12
<211> 321
<212> PRT
<213> Unknown

<220>
<223> Obtained from an environmental sample

<400> 12
Met Thr Gly Ser Tyr Pro Lys Asp Thr Leu Ile Val Gly Leu Ala Gln
1               5                   10                  15
Ile Ala Pro Val Trp Leu Asp Arg Ala Gly Thr Leu Ser Lys Ile Leu
            20                  25                  30
Ala Gln Val His Ala Ala Asn Gln Ala Gly Cys His Leu Val Ala Phe
        35                  40                  45
Gly Glu Gly Leu Leu Pro Gly Tyr Pro Phe Trp Ile Glu Arg Thr Asn
    50                  55                  60
Gly Ala Leu Phe Asn Ser Thr Val Gln Lys Glu Ile His Ala His Tyr
65                  70                  75                  80
Met Asp Gln Ala Val Gln Ile Glu Ala Gly His Leu Asp Pro Leu Cys
                85                  90                  95
Ala Thr Ala Lys Lys Phe Gly Ile Thr Val Val Leu Gly Cys Ile Glu
            100                 105                 110
Arg Pro Leu Asp Arg Gly Gly His Ser Leu Tyr Ala Ser Leu Val Tyr
        115                 120                 125
Ile Asp Ser Glu Gly Ser Ile Gln Ser Val His Arg Lys Leu Met Pro
    130                 135                 140
Thr Tyr Glu Glu Arg Leu Thr Trp Ser Ser Gly Asp Gly His Gly Leu
145                 150                 155                 160
Arg Val His Thr Leu Gly Ala Phe Thr Val Gly Gly Leu Asn Cys Trp
                165                 170                 175
Glu Asn Trp Met Pro Leu Ala Arg Ala Ala Met Tyr Gly Gln Gly Glu
            180                 185                 190
Asp Leu His Val Ala Ile Trp Pro Gly Gly Ser His Leu Thr Gln Asp
        195                 200                 205
Ile Thr Arg Phe Ile Ala Leu Glu Ser Arg Ser Tyr Val Leu Ser Val
    210                 215                 220
Ser Gly Leu Met Arg Ala Thr Asp Phe Pro Lys Asp Thr Pro His Leu
225                 230                 235                 240
Ala Ser Ile Leu Ala Lys Gly Glu Glu Ile Leu Ala Asn Gly Gly Ser
                245                 250                 255
Cys Ile Ala Gly Pro Asp Gly Lys Trp Val Val Gly Pro Leu Val Gly
            260                 265                 270
Glu Glu Lys Leu Ile Val Ala Thr Ile Asp His Cys Arg Val Arg Glu

        275                 280                 285
Glu Arg Gln Asn Phe Asp Pro Ser Gly His Tyr Ser Arg Pro Asp Val
    290                 295                 300
Leu Gln Leu Lys Ile Asn Arg Glu Arg Gln Ser Thr Ile Ser Phe Ser
305                 310                 315                 320
Glu

<210> 13
<211> 1014
<212> DNA
<213> Unknown

<220>
<223> Obtained from an environmental sample

<400> 13
   atgggcattc aacatccgaa atataaggtt gcggtggtgc aggcggcgcc ggcctggctc 60
   gatctcgatg cgtcgatcgc caaatcgatc gcgttgatcg aggaggcggc tgccaatggc 120
   gccaagctga tcgccttccc ggaggcgttc atccctggct atccctggta tatctggctg 180
   gactcgccgg cctgggcgat cggccgcggt tttgtgcagc gctatttcga caactcgctg 240
   gcctatgaca gcccgcaggc cgagaagctg cggctggcgg tgaagaaggc cggcctcacc 300
   gccgtgatcg gcctctccga gcgcgagggc ggcagccttt atctcgcgca atggctgatc 360
   gggcccgatg gcgagaccat cgcaaagcgc cgcaagctgc ggccgaccca tgccgagcgc 420
   accgtctatg gcgaaggcga tggcagcgat ctcgcggtgc atgaccgccc cggcatcggc 480
   cggctcggcg cgctgtgctg ctgggagcat ctgcagccgc tgtcgaaata cgcgatgtat 540
   gcccagaacg agcaggttca tgtcgcggcc tggccgagct tctcgctcta cgacccgttc 600
   gcgccggcgc tcggctggga ggtcaacaat gcggcctcac gcgtctatgc ggtggaaggc 660
   tcgtgcttcg tgctggcgcc ctgcgcgacg gtgtcgaagg cgatgatcga cgagctctgc 720
   gaccgcgacg acaagcacgg gctgctgcat gtcggcgggg gacacgccgc gatctatggg 780
   ccggacggct cttcgattgc ggagaaattg ccgccggagc aggagggcct gctctatgcc 840
   gacatcgatc tcggcgccat cgggattgcc aagaacgccg ccgatccggc cggacattac 900
   tcgcggcccg acgtgacgcg gctgttgctc aacaagaagc cgtcgaagcg tgtcgagcat 960
   tttcgctgcc cggtcgacaa tgtcgagccg gagatcgacg ccgccgccag ctga 1014

<210> 14
<211> 337
<212> PRT
<213> Unknown

<220>
<223> Obtained from an environmental sample

<400> 14
Met Gly Ile Gln His Pro Lys Tyr Lys Val Ala Val Val Gln Ala Ala
1               5                   10                  15
Pro Ala Trp Leu Asp Leu Asp Ala Ser Ile Ala Lys Ser Ile Ala Leu
            20                  25                  30
Ile Glu Glu Ala Ala Ala Asn Gly Ala Lys Leu Ile Ala Phe Pro Glu
        35                  40                  45
Ala Phe Ile Pro Gly Tyr Pro Trp Tyr Ile Trp Leu Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Lys Leu Arg Leu Ala Val Lys Lys
                85                  90                  95
Ala Gly Leu Thr Ala Val Ile Gly Leu Ser Glu Arg Glu Gly Gly Ser

                100                 105                 110
Leu Tyr Leu Ala Gln Trp Leu Ile Gly Pro Asp Gly Glu Thr Ile Ala
            115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asp Arg Pro Gly Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Pro Ala Leu Gly Trp Glu Val
            195                 200                 205
Asn Asn Ala Ala Ser Arg Val Tyr Ala Val Glu Gly Ser Cys Phe Val
    210                 215                 220
Leu Ala Pro Cys Ala Thr Val Ser Lys Ala Met Ile Asp Glu Leu Cys
225                 230                 235                 240
Asp Arg Asp Asp Lys His Gly Leu Leu His Val Gly Gly Gly His Ala
                245                 250                 255
Ala Ile Tyr Gly Pro Asp Gly Ser Ser Ile Ala Glu Lys Leu Pro Pro
            260                 265                 270
Glu Gln Glu Gly Leu Leu Tyr Ala Asp Ile Asp Leu Gly Ala Ile Gly
            275                 280                 285
Ile Ala Lys Asn Ala Ala Asp Pro Ala Gly His Tyr Ser Arg Pro Asp
    290                 295                 300
Val Thr Arg Leu Leu Leu Asn Lys Lys Pro Ser Lys Arg Val Glu His
305                 310                 315                 320
Phe Ser Leu Pro Val Asp Asn Val Glu Pro Glu Ile Asp Ala Ala Ala
                325                 330                 335
Ser

<210> 15
<211> 1047
<212> DNA
<213> Unknown

<220>
<223> Obtained from an environmental sample

<400> 15
atgccaacat caaaacaatt tagagtcgct gcagttcaag ccgccccggt atttcttgac      60
ctggagggcg caataagcaa aggcatctcc ctcattgagg aggccgcttc caatggagcc     120
aagctcattg ccttcccgga aacgtggatt cccggctacc cctggtggat ctggctggac     180
tcacccgctt ggggcatgcg ctttgtccag cgctattttg acaactcgct catgctgggt     240
agtgagcaag ccaagcgcat gaaccaggct gccgccaata acaagattta cgtggtgatg     300
ggttatagcg aacgcagtgg cggcagcctc tacatgggcc aatccattat caacgacaag     360
ggtgaaacga tttttacccg ccgcaaactc aagccaactc atgtcgagcg taccgtgttt     420
ggggagggag acggcagcca tctttgcgta atggataccg agattggccg cgtcggcgcg     480
atgtgctgtt gggaacattt gcagccgctc agcaaatatg caatgtattc tcaggatgaa     540
caaattcaca ttgcctcctg gccgagcttt tcgttatatc ggggggcagc ctatgcactc      600
ggccccgaac tgaacaacgc cgccagccaa atgtatgcag ccgaaggcca gtgctttgtc     660
cttgcccctt gcgccaccgt ctcaaaggag atgatcgaaa tgctgataga tgatcccagg     720
aaagagccgc ttctgctgga aggtggcggg ttcaccatga tttacggccc cgatgggcga     780
cctctggcta aaccgttgcc tgaaaacgag gaagggctgc tatatgccga tattgacctg     840
ggcatgattt caatggccaa ggctgccgcc gacccggcag gtcactacgc acgcccggat     900
gtcactcgcc tactattcaa ttccgcgccc gccaatcgcg tcgagtatat caacccagcg     960

tcaggcccaa ccgaatcctt aaaagatatg ggaaagatgc aaatggaggc cgaacagcaa 1020
aaggcggccc tgcgagagat gatctaa 1047

<210> 16
<211> 348
<212> PRT
<213> Unknown

<220>
<223> Obtained from an environmental sample

<400> 16
Met Pro Thr Ser Lys Gln Phe Arg Val Ala Ala Val Gln Ala Ala Pro
1               5                   10                  15
Val Phe Leu Asp Leu Glu Gly Ala Ile Ser Lys Gly Ile Ser Leu Ile
            20                  25                  30
Glu Glu Ala Ala Ser Asn Gly Ala Lys Leu Ile Ala Phe Pro Glu Thr
        35                  40                  45
Trp Ile Pro Gly Tyr Pro Trp Trp Ile Trp Leu Asp Ser Pro Ala Trp
    50                  55                  60
Gly Met Arg Phe Val Gln Arg Tyr Phe Asp Asn Ser Leu Met Leu Gly
65                  70                  75                  80
Ser Glu Gln Ala Lys Arg Met Asn Gln Ala Ala Ala Asn Asn Lys Ile
                85                  90                  95
Tyr Val Val Met Gly Tyr Ser Glu Arg Ser Gly Gly Ser Leu Tyr Met
            100                 105                 110
Gly Gln Ser Ile Ile Asn Asp Lys Gly Glu Thr Ile Phe Thr Arg Arg
        115                 120                 125
Lys Leu Lys Pro Thr His Val Glu Arg Thr Val Phe Gly Glu Gly Asp
    130                 135                 140
Gly Ser His Leu Cys Val Met Asp Thr Glu Ile Gly Arg Val Gly Ala
145                 150                 155                 160
Met Cys Cys Trp Glu His Leu Gln Pro Leu Ser Lys Tyr Ala Met Tyr
                165                 170                 175
Ser Gln Asp Glu Gln Ile His Ile Ala Ser Trp Pro Ser Phe Ser Leu
            180                 185                 190
Tyr Arg Gly Ala Ala Tyr Ala Leu Gly Pro Glu Leu Asn Asn Ala Ala
        195                 200                 205
Ser Gln Met Tyr Ala Ala Glu Gly Gln Cys Phe Val Leu Ala Pro Cys
    210                 215                 220
Ala Thr Val Ser Lys Glu Met Ile Glu Met Leu Ile Asp Asp Pro Arg
225                 230                 235                 240
Lys Glu Pro Leu Leu Leu Glu Gly Gly Gly Phe Thr Met Ile Tyr Gly
                245                 250                 255
Pro Asp Gly Arg Pro Leu Ala Lys Pro Leu Pro Glu Asn Glu Glu Gly
            260                 265                 270
Leu Leu Tyr Ala Asp Ile Asp Leu Gly Met Ile Ser Met Ala Lys Ala
        275                 280                 285
Ala Ala Asp Pro Ala Gly His Tyr Ala Arg Pro Asp Val Thr Arg Leu
    290                 295                 300
Leu Phe Asn Ser Ala Pro Ala Asn Arg Val Glu Tyr Ile Asn Pro Ala
305                 310                 315                 320
Ser Gly Pro Thr Glu Ser Leu Lys Asp Met Gly Lys Met Gln Met Glu
                325                 330                 335
Ala Glu Gln Gln Lys Ala Ala Leu Arg Glu Met Ile
            340                 345

<210> 17
<211> 993
<212> DNA
<213> Unknown

<220>
<223> Obtained from an environmental sample

<400> 17
atgagagttg ttaaagccgc agctgtccaa ctgagtcccg tcctctatag ccgcgaggga 60
acggtcgaga aggtcgtgcg gaagatccat gaacttgccg aagagggagt cgagttcgtc 120
acctttcctg agaccgtggt gccttattac ccgtactttt cgttcgttca gacgcccttg 180
cagcaaatct tcggaacaga gtatctgagg ctgctcgacc aggcagtcac cgtgccatcc 240
gccgccaccg acgcgatcgg cgaggctgcc aggttcgctg gagttgttgt ctcgatcggc 300
gtcaacgagc gagacggggg aactctgtac aacactcagc ttctcttcga tgccgacgga 360
agcttaattc agcggcgccg caagatcacg cccacccatt acgagcgcat gatctggggc 420
cagggtgacg gctcaggtct gcgggccgtt gatagcaagg ccggccgcat tggtcagctg 480
gcatgctggg agcacaacaa tccactggcg cgctacgcgc tgatagccga cggcgagcag 540
atccattcgg ccatgtatcc gggctccatg ttcggcgact cgtttgccaa aaagaccgaa 600
atcaatatcc ggcagcatgc gctggagtct gcgtgcttcg tcgtgaacgc aacggcctgg 660
ctggacggcg atcaacaggc gcaaatcatg aaggacaccg gctgcagcat cggcccgatc 720
tccggcggtt gcttcaccac tatcgtggcg ccggacggtt ccctgatcgg cgagcccctc 780
cgctcgggtg agggcgtggt catcgccgac ctcgacttca cgttaatcga caggcgtaag 840
caggtgatgg actcgcgagg ccactacagc cggccggagt tgctcagcct cttaatagac 900
cgcaccccta ccgcgcactt tcacgaacgc gcttcgcacc ccacgacagg agctgagcaa 960
ggctccgagg atgtgttcga ggctaacatt taa 993

<210> 18
<211> 330
<212> PRT
<213> Unknown

<220>
<223> Obtained from an environmental sample

<400> 18
Met Arg Val Val Lys Ala Ala Ala Val Gln Leu Ser Pro Val Leu Tyr
1               5                   10                  15
Ser Arg Glu Gly Thr Val Glu Lys Val Val Arg Lys Ile His Glu Leu
            20                  25                  30
Ala Glu Glu Gly Val Glu Phe Val Thr Phe Pro Glu Thr Val Val Pro
        35                  40                  45
Tyr Tyr Pro Tyr Phe Ser Phe Val Gln Thr Pro Leu Gln Gln Ile Phe
    50                  55                  60
Gly Thr Glu Tyr Leu Arg Leu Leu Asp Gln Ala Val Thr Val Pro Ser
65                  70                  75                  80
Ala Ala Thr Asp Ala Ile Gly Glu Ala Ala Arg Phe Ala Gly Val Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Ser Leu Ile Gln Arg Arg Arg Lys
        115                 120                 125
Ile Thr Pro Thr His Tyr Glu Arg Met Ile Trp Gly Gln Gly Asp Gly
    130                 135                 140
Ser Gly Leu Arg Ala Val Asp Ser Lys Ala Gly Arg Ile Gly Gln Leu
145                 150                 155                 160
Ala Cys Trp Glu His Asn Asn Pro Leu Ala Arg Tyr Ala Leu Ile Ala

165                 170                 175
Asp Gly Glu Gln Ile His Ser Ala Met Tyr Pro Gly Ser Met Phe Gly
            180                 185                 190
Asp Ser Phe Ala Lys Lys Thr Glu Ile Asn Ile Arg Gln His Ala Leu
        195                 200                 205
Glu Ser Ala Cys Phe Val Val Asn Ala Thr Ala Trp Leu Asp Gly Asp
    210                 215                 220
Gln Gln Ala Gln Ile Met Lys Asp Thr Gly Cys Ser Ile Gly Pro Ile
225                 230                 235                 240
Ser Gly Gly Cys Phe Thr Thr Ile Val Ala Pro Asp Gly Ser Leu Ile
                245                 250                 255
Gly Glu Pro Leu Arg Ser Gly Glu Gly Val Val Ile Ala Asp Leu Asp
            260                 265                 270
Phe Thr Leu Ile Asp Arg Arg Lys Gln Val Met Asp Ser Arg Gly His
        275                 280                 285
Tyr Ser Arg Pro Glu Leu Leu Ser Leu Leu Ile Asp Arg Thr Pro Thr
    290                 295                 300
Ala His Phe His Glu Arg Ala Ser His Pro Thr Thr Gly Ala Glu Gln
305                 310                 315                 320
Gly Ser Glu Asp Val Phe Glu Ala Asn Ile
                325                 330

<210> 19
<211> 1050
<212> DNA
<213> Unknown

<220>
<223> Obtained from an environmental sample

<400> 19
atggaaagca acttccttgc cgcagcagtg caagcagaac cggtttactt caatgctttt      60
cagacggccg aaaaggccgc gtcattgatt gacgatgccg gtcggcaggg ggctcgctta     120
gtgacatttc ccgaaacgtg gctgcccggt tacccgtact ggatctggct tggtgccccc     180
gcctggggaa tgcatcattt catcctaaag taccatcaaa actcgccggt tgcaggagga     240
ccagaggaac agatcctttg tcaggcggcc cgccgcaacg ggatttttgt cgtcatggga     300
ctcagcgaga aaatcggggc aagcctctac atggcgcagt ggttcatcag tccagacggc     360
aaagtggtcg ctcgccgacg caaattgaag cctactcacg tcgaacgttc ggtcttcggg     420
gaaggggatg gttccgacat tgtcgttctt gatacacccc ttggaaaggt cgggggcctt     480
tgctgctggg agcacatgca gccactttcg aagtacgcca tgtactcgca aggcgagcag     540
atccatgctg cttcttggcc gagtgttagc gtctatcgcg ataaaattta cgttctgggg     600
ccggagctga acggtgccgc caatcagatg tatgcggcag aaggtcagtg tttcgtcctg     660
gcatcctggg caacggtttc acaagcggct atcgatcttt tttgcgacac gcccgacaag     720
gccgcgctca tgaaaattgg tggtggtttt tcccagatct atgggccaga cgggtgcccc     780
ctggcgaagc cgttgccgga ggacgtcgaa ggattggtga ccgctgagat tgacttcaat     840
gccatcacgc gcgtgaaagc agcggcggac cccgtagggc actatagccg gcccgatgta     900
ttccgcctgt tgttcaatcg tacgcgccaa gaacgcgtgg tttctgtcaa cacgtttgtg     960
ccaggtgtca cccagcgaac cgccaagaat gggtcggcgg acgaattggt cggtcacccg    1020
gagaacgctg tcgcccgggc tgcagagtaa                                     1050

<210> 20
<211> 349
<212> PRT
<213> Unknown

<220>
<223> Obtained from an environmental sample

<400> 20
Met Glu Ser Asn Phe Leu Ala Ala Ala Val Gln Ala Glu Pro Val Tyr
1               5                   10                  15
Phe Asn Ala Phe Gln Thr Ala Glu Lys Ala Ala Ser Leu Ile Asp Asp
            20                  25                  30
Ala Gly Arg Gln Gly Ala Arg Leu Val Thr Phe Pro Glu Thr Trp Leu
        35                  40                  45
Pro Gly Tyr Pro Tyr Trp Ile Trp Leu Gly Ala Pro Ala Trp Gly Met
    50                  55                  60
His His Phe Ile Leu Lys Tyr His Gln Asn Ser Pro Val Ala Gly Gly
65                  70                  75                  80
Pro Glu Glu Gln Ile Leu Cys Gln Ala Ala Arg Arg Asn Gly Ile Phe
                85                  90                  95
Val Val Met Gly Leu Ser Glu Lys Ile Gly Ala Ser Leu Tyr Met Ala
            100                 105                 110
Gln Trp Phe Ile Ser Pro Asp Gly Lys Val Val Ala Arg Arg Arg Lys
        115                 120                 125
Leu Lys Pro Thr His Val Glu Arg Ser Val Phe Gly Glu Gly Asp Gly
    130                 135                 140
Ser Asp Ile Val Val Leu Asp Thr Pro Leu Gly Lys Val Gly Gly Leu
145                 150                 155                 160
Cys Cys Trp Glu His Met Gln Pro Leu Ser Lys Tyr Ala Met Tyr Ser
                165                 170                 175
Gln Gly Glu Gln Ile His Ala Ala Ser Trp Pro Ser Val Ser Val Tyr
            180                 185                 190
Arg Asp Lys Ile Tyr Val Leu Gly Pro Glu Leu Asn Gly Ala Ala Asn
        195                 200                 205
Gln Met Tyr Ala Ala Glu Gly Gln Cys Phe Val Leu Ala Ser Trp Ala
    210                 215                 220
Thr Val Ser Gln Ala Ala Ile Asp Leu Phe Cys Asp Thr Pro Asp Lys
225                 230                 235                 240
Ala Ala Leu Met Lys Ile Gly Gly Gly Phe Ser Gln Ile Tyr Gly Pro
                245                 250                 255
Asp Gly Cys Pro Leu Ala Lys Pro Leu Pro Glu Asp Val Glu Gly Leu
            260                 265                 270
Val Thr Ala Glu Ile Asp Phe Asn Ala Ile Thr Arg Val Lys Ala Ala
        275                 280                 285
Ala Asp Pro Val Gly His Tyr Ser Arg Pro Asp Val Phe Arg Leu Leu
    290                 295                 300
Phe Asn Arg Thr Arg Gln Glu Arg Val Val Ser Val Asn Thr Phe Val
305                 310                 315                 320
Pro Gly Val Thr Gln Arg Thr Ala Lys Asn Gly Ser Ala Asp Glu Leu
                325                 330                 335
Val Gly His Pro Glu Asn Ala Val Ala Arg Ala Ala Glu
            340                 345

<210> 21
<211> 1065
<212> DNA
<213> Unknown

<220>
<223> Obtained from an environmental sample

<400> 21
atggcactag aacatccgaa gtacgtggcg gccgtggttc aggccgcgcc cgaattcctg      60

aatctagaca gagggatcga aaagacgatc gcattgatcg acgaagcggg acagaaaggg 120
gcggccctga ttgcatttcc ggaaacctgg ctgccgggct atccgtttca tgtctggctc 180
ggtcctcccg catgggcgct tggctcagga ttcgtccagc gctatttcga caactcgatg 240
acgtacgata gtcctcaggc cgctgcactg agggacgctg ccgcgcgcaa cgggatcacg 300
gtggtattgg gcttgtcgga gcgatgcggc ggcagcctct atatcgcgca atggatcatc 360
ggcccggatg gcgcgacggt cgccacgcgc cgcaaattgc ggccgactca tatcgagcgc 420
accgttttcg gcgatggcga cggcagcgat ctggcagtac acgatctcaa catcggccgc 480
cttggcgcac tgtgctgctg ggagcacatt cagccgctga ccaagtacgc gatgtatgcg 540
cagcacgaac aggtgcacgt cgcggcctgg ccgagcttct ccatgtatga attcgcgccc 600
gcgctcggtc acgaggtgaa caacgcagtc agccgcgtct atgccgttga gggatcgtgc 660
ttcgtgctcg cgccgtgcgc ggtcatcagc gagcaaatgg tcgacatgtt gtgcgacacg 720
gcagacaagc gcgcgatgat acgtgccggc ggcgggcacg cagtggcgtt cgggccggac 780
ggcgaagctc tggtcgagaa actgccggaa aatgaggaag gcttgctgct ggtcgatatc 840
gatctcggtc gcatctcgct tgcgaaggct gcggccgacc ccgtcggtca ctacgcgcgc 900
cccgatgtct tgcggctctg gttcgacaag caaccgcggc ggtgcgtcga acatgccggc 960
gagaacgacg cgtcgcgcag gtcgcacggg tcgtccgggt cacaatcgcc ggcgcaggat 1020
gggccggcga acgacatggt agaccgtcag gaaaacgtcg attga 1065

<210> 22
<211> 354
<212> PRT
<213> Unknown

<220>
<223> Obtained from an environmental sample

<400> 22
Met Ala Leu Glu His Pro Lys Tyr Val Ala Ala Val Val Gln Ala Ala
1               5                   10                  15
Pro Glu Phe Leu Asn Leu Asp Arg Gly Ile Glu Lys Thr Ile Ala Leu
            20                  25                  30
Ile Asp Glu Ala Gly Gln Lys Gly Ala Ala Leu Ile Ala Phe Pro Glu
        35                  40                  45
Thr Trp Leu Pro Gly Tyr Pro Phe His Val Trp Leu Gly Pro Pro Ala
    50                  55                  60
Trp Ala Leu Gly Ser Gly Phe Val Gln Arg Tyr Phe Asp Asn Ser Met
65                  70                  75                  80
Thr Tyr Asp Ser Pro Gln Ala Ala Ala Leu Arg Asp Ala Ala Ala Arg
                85                  90                  95
Asn Gly Ile Thr Val Val Leu Gly Leu Ser Glu Arg Cys Gly Gly Ser
            100                 105                 110
Leu Tyr Ile Ala Gln Trp Ile Ile Gly Pro Asp Gly Ala Thr Val Ala
        115                 120                 125
Thr Arg Arg Lys Leu Arg Pro Thr His Ile Glu Arg Thr Val Phe Gly
    130                 135                 140
Asp Gly Asp Gly Ser Asp Leu Ala Val His Asp Leu Asn Ile Gly Arg
145                 150                 155                 160
Leu Gly Ala Leu Cys Cys Trp Glu His Ile Gln Pro Leu Thr Lys Tyr
                165                 170                 175
Ala Met Tyr Ala Gln His Glu Gln Val His Val Ala Ala Trp Pro Ser
            180                 185                 190
Phe Ser Met Tyr Glu Phe Ala Pro Ala Leu Gly His Glu Val Asn Asn
        195                 200                 205
Ala Val Ser Arg Val Tyr Ala Val Glu Gly Ser Cys Phe Val Leu Ala
    210                 215                 220
Pro Cys Ala Val Ile Ser Glu Gln Met Val Asp Met Leu Cys Asp Thr
225                 230                 235                 240

Ala Asp Lys Arg Ala Met Ile Arg Ala Gly Gly Gly His Ala Val Ala
                245                 250                 255
Phe Gly Pro Asp Gly Glu Ala Leu Val Glu Lys Leu Pro Glu Asn Glu
            260                 265                 270
Glu Gly Leu Leu Leu Val Asp Ile Asp Leu Gly Arg Ile Ser Leu Ala
        275                 280                 285
Lys Ala Ala Ala Asp Pro Val Gly His Tyr Ala Arg Pro Asp Val Leu
    290                 295                 300
Arg Leu Trp Phe Asp Lys Gln Pro Arg Arg Cys Val Glu His Ala Gly
305                 310                 315                 320
Glu Asn Asp Ala Ser Arg Arg Ser His Gly Ser Ser Gly Ser Gln Ser
                325                 330                 335
Pro Ala Gln Asp Gly Pro Ala Asn Asp Met Val Asp Arg Gln Glu Asn
            340                 345                 350
Val Asp

<210> 23
<211> 1005
<212> DNA
<213> Unknown

<220>
<223> Obtained from an environmental sample

<400> 23
atgaccgtta tcaaagcagc cgccattcaa atcagccccg tgctctacag ccgggcgggg      60
acagtcgaga aagttgttag gaaggttaga gagctcgggg ccaaaggtgt ccgattcgct     120
acctttcccg aaaccatcat accgtactac ccgtacttct cgttcgttca gtcggcgttc     180
gacatgaagc ttgggagtga acatcagcgg ctgctcgacg aatcagtcac aattccttcg     240
tccgagacgg acgcgatcgc ccaggccgcc aaggaagcgg gcatggtggt gtccgtcggg     300
gtcaatgagc gcgatgggcg atccatctac aacactcaac ttctgttcga cgctgatggc     360
acgctcattc agcgtaggcg aaagatcacc ccgacctatc acgagcgcat gatttggggt     420
caaggcgatg gatccggcct acgcgcggtc gatagcgccg tgggccggat cggccagctt     480
gcctgctggg agcactacct tcccctggcg cggtacgccc tcatcgcgga cggagagcaa     540
atccactcgg caatgtatcc aggctcgttc gctggtccgc tatttgccga gcagatagag     600
gttagtatcc gccagcacgc gcttgagtca gcctgcttcg tcgtcaacgc gaccggatgg     660
cttagcgccg agcagcaagc tcaaatagtg aaggataccg gatgcgtcgt tggaccaatc     720
tccggtggct gctttacggc gattgttgat ccggagggtc ggatcatggg ggcgccactc     780
aaggcaggtg agggggaggt catcgcagat ctcgattttg cgcagattga tttccgcaag     840
cgtgtgatgg atacgcgagg gcactacagc cgccccgaac ttctaagcct cacgatcgac     900
cgcagtcagc accatcacat gactgagcga ggcgccgatc accgtgtaga ccacgcaaag     960
ccaacggtca ccgcagagca gtcggccgtc gagccggcgg aatga                    1005

<210> 24
<211> 334
<212> PRT
<213> Unknown

<220>
<223> Obtained from an environmental sample

<400> 24
Met Thr Val Ile Lys Ala Ala Ala Ile Gln Ile Ser Pro Val Leu Tyr
 1               5                  10                  15
Ser Arg Ala Gly Thr Val Glu Lys Val Val Arg Lys Val Arg Glu Leu
            20                  25                  30

Gly Ala Lys Gly Val Arg Phe Ala Thr Phe Pro Glu Thr Ile Ile Pro
        35                  40                  45
Tyr Tyr Pro Tyr Phe Ser Phe Val Gln Ser Ala Phe Asp Met Lys Leu
    50                  55                  60
Gly Ser Glu His Gln Arg Leu Leu Asp Glu Ser Val Thr Ile Pro Ser
65                  70                  75                  80
Ser Glu Thr Asp Ala Ile Ala Gln Ala Ala Lys Glu Ala Gly Met Val
                85                  90                  95
Val Ser Val Gly Val Asn Glu Arg Asp Gly Arg Ser Ile Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr Tyr His Glu Arg Met Ile Trp Gly Gln Gly Asp Gly
    130                 135                 140
Ser Gly Leu Arg Ala Val Asp Ser Ala Val Gly Arg Ile Gly Gln Leu
145                 150                 155                 160
Ala Cys Trp Glu His Tyr Leu Pro Leu Ala Arg Tyr Ala Leu Ile Ala
                165                 170                 175
Asp Gly Glu Gln Ile His Ser Ala Met Tyr Pro Gly Ser Phe Ala Gly
            180                 185                 190
Pro Leu Phe Ala Glu Gln Ile Glu Val Ser Ile Arg Gln His Ala Leu
        195                 200                 205
Glu Ser Ala Cys Phe Val Val Asn Ala Thr Gly Trp Leu Ser Ala Glu
    210                 215                 220
Gln Gln Ala Gln Ile Val Lys Asp Thr Gly Cys Val Val Gly Pro Ile
225                 230                 235                 240
Ser Gly Gly Cys Phe Thr Ala Ile Val Asp Pro Glu Gly Arg Ile Met
                245                 250                 255
Gly Ala Pro Leu Lys Ala Gly Glu Gly Glu Val Ile Ala Asp Leu Asp
            260                 265                 270
Phe Ala Gln Ile Asp Phe Arg Lys Arg Val Met Asp Thr Arg Gly His
        275                 280                 285
Tyr Ser Arg Pro Glu Leu Leu Ser Leu Thr Ile Asp Arg Ser Gln His
    290                 295                 300
His His Met Thr Glu Arg Gly Ala Asp His Arg Val Asp His Ala Lys
305                 310                 315                 320
Pro Thr Val Thr Ala Glu Gln Ser Ala Val Glu Pro Ala Glu
                325                 330

<210> 25
<211> 939
<212> DNA
<213> Unknown

<220>
<223> Obtained from an environmental sample

<400> 25
gtgtcatcaa ctatcaaagt cgccattatt caggccgctc ctgcttacta cgacctgcag 60
gcgtcgctgg caaaggccgc cagtctgatc cgcgaggcgg cacgcggcgg cgcgcaattc 120
gtcgcgttcg gggagacatg gctgccgggc tatccgatgt ggctggattg gtgtcctggc 180
gcgatcatct gggataaccc cgccaccaaa accgtcttcg cgcgcctcca tgaaaacagc 240
gtcgccgttc ccggcaggga aacggcattt ctcgccgacc ttgcgatgtc gttaagcatc 300
gtattatgca tcggcgtcaa tgagaaggtc atgaatggcc cgggacacgg cacgctctac 360
aacacgctcc tgacgtttga tgcaacgggt gaaatcatca atcatcatcg caagttgatg 420
ccaacctatg gcgagagatt ggtatggggg ccgggcgacg cagttggcgt gcaagcggtt 480
gatagtacgg tcgggcgcat cggcgggctg atctgttggg agcactggat gccgctgcca 540

cgccaactca tgcacaacag cggcgagcag attcacgtct gcgcatggcc gggcgtgcac 600
gaaatgcacc agatcgcgag ccgtcattat gcattcgagg gccgctgctt tgtgctggcc 660
gccggattga tcatgcccgc gttcgacctg cccagcgaac tcgaatttcc gcccgaactg 720
gccgacaagc gcgactatct cctaatgaac ggcggcagcg ccatcatcaa gcccaatggc 780
aaatatctcg ccgggccggt ttatgacgaa gagactattc tctgcgccga ccttgacctg 840
actgagaaca tcaaggagca gatgacgctg gacgtgacag ggcattatgc gcgagcggaa 900
ctgtttgact tgaatgtggt gcggcggcgg aatgcgtag 939

<210> 26
<211> 312
<212> PRT
<213> Unknown

<220>
<223> Obtained from an environmental sample

<400> 26
Val Ser Ser Thr Ile Lys Val Ala Ile Ile Gln Ala Ala Pro Ala Tyr
1               5                   10                  15
Tyr Asp Leu Gln Ala Ser Leu Ala Lys Ala Ala Ser Leu Ile Arg Glu
            20                  25                  30
Ala Ala Arg Gly Gly Ala Gln Phe Val Ala Phe Gly Glu Thr Trp Leu
        35                  40                  45
Pro Gly Tyr Pro Met Trp Leu Asp Trp Cys Pro Gly Ala Ile Ile Trp
    50                  55                  60
Asp Asn Pro Ala Thr Lys Thr Val Phe Ala Arg Leu His Glu Asn Ser
65                  70                  75                  80
Val Ala Val Pro Gly Arg Glu Thr Ala Phe Leu Ala Asp Leu Ala Met
                85                  90                  95
Ser Leu Ser Ile Val Leu Cys Ile Gly Val Asn Glu Lys Val Met Asn
            100                 105                 110
Gly Pro Gly His Gly Thr Leu Tyr Asn Thr Leu Leu Thr Phe Asp Ala
        115                 120                 125
Thr Gly Glu Ile Ile Asn His His Arg Lys Leu Met Pro Thr Tyr Gly
    130                 135                 140
Glu Arg Leu Val Trp Gly Pro Gly Asp Ala Val Gly Val Gln Ala Val
145                 150                 155                 160
Asp Ser Thr Val Gly Arg Ile Gly Gly Leu Ile Cys Trp Glu His Trp
                165                 170                 175
Met Pro Leu Pro Arg Gln Leu Met His Asn Ser Gly Glu Gln Ile His
            180                 185                 190
Val Cys Ala Trp Pro Gly Val His Glu Met His Gln Ile Ala Ser Arg
        195                 200                 205
His Tyr Ala Phe Glu Gly Arg Cys Phe Val Leu Ala Ala Gly Leu Ile
    210                 215                 220
Met Pro Ala Phe Asp Leu Pro Ser Glu Leu Glu Phe Pro Pro Glu Leu
225                 230                 235                 240
Ala Asp Lys Arg Asp Tyr Leu Leu Met Asn Gly Gly Ser Ala Ile Ile
                245                 250                 255
Lys Pro Asn Gly Lys Tyr Leu Ala Gly Pro Val Tyr Asp Glu Glu Thr
            260                 265                 270
Ile Leu Cys Ala Asp Leu Asp Leu Thr Glu Asn Ile Lys Glu Gln Met
        275                 280                 285
Thr Leu Asp Val Thr Gly His Tyr Ala Arg Ala Glu Leu Phe Asp Leu
    290                 295                 300
Asn Val Val Arg Arg Arg Asn Ala
305                 310

<210> 27
<211> 1056
<212> DNA
<213> Unknown

<220>
<223> Obtained from an environmental sample

<400> 27
atgccaaccc ccagcgatca tttcaaaatc gccgctgttc aggcctcgcc cgtgtttctg 60
gaccgggagg ccactgtgga aaaggcctgc cggttgatcg ccgaagccgc aaagcagggc 120
gcccgcctca tcgtctttcc ggaatctttc atcccgacct acccggattg ggtgtgggcc 180
gttcccccgg gaagggaaag aatcctgaac cagctgtatt ctgaattcct ggccaatgcc 240
gtcgatgttc ccggcgcggc gaccgaacaa cttgcccagg ctgcacgaat ggccggcgcc 300
tatgtgatta tgggcgtcac cgaaagagac acctcggcca gcggggccag cctctacaac 360
accctgctct acttcagccc cgaaggcatc ctaatgggca aacaccggaa gctggttccc 420
acggggggcg aacggctggt ctgggcctac ggagacggca gcacgctgga ggtctacgac 480
actccgctgg gaaagatcgg cgggctgatc tgctgggaga actacatgcc cctggcccgg 540
tacacgatgt acgcctgggg cacccagatt tacatcgccg ccacctggga ccgcggggaa 600
ccgtggctct ccaccctgcg gcatatcgcc aaggaaggaa gggtctacgt catcgggtgc 660
tgcatcgccc tgcgccaggg ggatatcccg gaccggttcg agtacaaggg aaaattttat 720
tccgggtccc gggagtggat caatgagggc gacagcgcca tcgtgaaccc ggacggggaa 780
ttcatcgccg ggccggtgcg gatgaaggag gagatcctgt atgccgagat agacccccgg 840
cagatgcggg gccccaagtg gatgctcgat gtggccggtc attacgcccg gccggatatc 900
ttcgagctca tcgtccaccg gaatccccac ccgatgatca aaatcgccga agacaggggc 960
acggggatcg cctcaagttt gattcgcccc cgccctaacc ttcccccatc aagggggagg 1020
aaatcggcaa gaagcaaacg caagcccaaa aaatga 1056

<210> 28
<211> 351
<212> PRT
<213> Unknown

<220>
<223> Obtained from an environmental sample

<400> 28
Met Pro Thr Pro Ser Asp His Phe Lys Ile Ala Ala Val Gln Ala Ser
1               5                   10                  15
Pro Val Phe Leu Asp Arg Glu Ala Thr Val Glu Lys Ala Cys Arg Leu
            20                  25                  30
Ile Ala Glu Ala Ala Lys Gln Gly Ala Arg Leu Ile Val Phe Pro Glu
        35                  40                  45
Ser Phe Ile Pro Thr Tyr Pro Asp Trp Val Trp Ala Val Pro Pro Gly
    50                  55                  60
Arg Glu Arg Ile Leu Asn Gln Leu Tyr Ser Glu Phe Leu Ala Asn Ala
65                  70                  75                  80
Val Asp Val Pro Gly Ala Ala Thr Glu Gln Leu Ala Gln Ala Ala Arg
                85                  90                  95
Met Ala Gly Ala Tyr Val Ile Met Gly Val Thr Glu Arg Asp Thr Ser
            100                 105                 110
Ala Ser Gly Ala Ser Leu Tyr Asn Thr Leu Leu Tyr Phe Ser Pro Glu
        115                 120                 125
Gly Ile Leu Met Gly Lys His Arg Lys Leu Val Pro Thr Gly Gly Glu
    130                 135                 140
Arg Leu Val Trp Ala Tyr Gly Asp Gly Ser Thr Leu Glu Val Tyr Asp

145                 150                 155                 160
Thr Pro Leu Gly Lys Ile Gly Gly Leu Ile Cys Trp Glu Asn Tyr Met
                165                 170                 175
Pro Leu Ala Arg Tyr Thr Met Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Lys Glu Gly Arg Val Tyr Val Ile Gly Cys Cys Ile Ala Leu
    210                 215                 220
Arg Gln Gly Asp Ile Pro Asp Arg Phe Glu Tyr Lys Gly Lys Phe Tyr
225                 230                 235                 240
Ser Gly Ser Arg Glu Trp Ile Asn Glu Gly Asp Ser Ala Ile Val Asn
                245                 250                 255
Pro Asp Gly Glu Phe Ile Ala Gly Pro Val Arg Met Lys Glu Glu Ile
            260                 265                 270
Leu Tyr Ala Glu Ile Asp Pro Arg Gln Met Arg Gly Pro Lys Trp Met
        275                 280                 285
Leu Asp Val Ala Gly His Tyr Ala Arg Pro Asp Ile Phe Glu Leu Ile
    290                 295                 300
Val His Arg Asn Pro His Pro Met Ile Lys Ile Ala Glu Asp Arg Gly
305                 310                 315                 320
Thr Gly Ile Ala Ser Ser Leu Ile Arg Pro Arg Pro Asn Leu Pro Pro
                325                 330                 335
Ser Arg Gly Arg Lys Ser Ala Arg Ser Lys Arg Lys Pro Lys Lys
            340                 345                 350

<210> 29
<211> 1017
<212> DNA
<213> Unknown

<220>
<223> Obtained from an environmental sample

<400> 29
atgggcatcg aacatacgaa atacaaggtg gcggtggtgc aggcggcgcc ggcctggctc      60
gacctcgagg cctcgatcgg caagtccatc ggcctaatca aggaggccgc ggacaagggc     120
gccaagctga tcgcctttcc ggaggccttc atccccggtt acccctggta tatctggatg     180
gactcgccgg cctgggcgat cggccgcggc ttcgtccagc gctatttcga caattcgctc     240
tcctacgaca gtccccaggc cgagcggctg cgtgatgccg tgcgccaggc caagctcacc     300
gccgtgatcg gcctgtccga acgcgacggc ggcagccttt acctggcgca atggttgatc     360
gggcccgacg gcgaaaccat tgccaagcgc cgcaagctgc ggccgaccca tgccgagcgc     420
accgtctatg gcgaaggcga cggcagcgat ctggccgtac atgcccggcc cgacatcggt     480
cgcttgggcg cgctgtgctg ctgggagcat cttcagccgt tgtcgaagta cgcaatgtac     540
gcccagaacg agcaggtcca cgtcgctgcc tggccgagct tctcgctcta cgatcccttc     600
gccccggcgc tcggcgccga ggtcaacaac gctgcctcgc gcgtctatgc ggtggagggc     660
tcctgcttcg tgctcgcgcc ttgcgcgacg gtgtcgcagg ccatgatcga cgaactctgc     720
gatcggcccg ataagcatgc gctgctgcat gccggcggag gctttgccgc gatctacggc     780
cccgacggca gccagatcgg cgagaagctg gcgccggatc aggagggtct gctgatcgcc     840
gagattgatc tgggcgccat cggtgttgcc aagaacgcgg cagatcccgc cggtcattat     900
tcacggccgg atgtgacgcg gttgctgctc aacaagaagc ggtaccagcg cgtcgagcaa     960
tttgccttgc ccgccgacat ggtcgagccc gcggacatag gcgcggcggc gagctga       1017

<210> 30
<211> 338
<212> PRT
<213> Unknown

<220>
<223> Obtained from an environmental sample

<400> 30
Met Gly Ile Glu His Thr Lys Tyr Lys Val Ala Val Val Gln Ala Ala
1               5                   10                  15
Pro Ala Trp Leu Asp Leu Glu Ala Ser Ile Gly Lys Ser Ile Gly Leu
            20                  25                  30
Ile Lys Glu Ala Ala Asp Lys Gly Ala Lys Leu Ile Ala Phe Pro Glu
        35                  40                  45
Ala Phe Ile Pro Gly Tyr Pro Trp Tyr Ile Trp Met Asp Ser Pro Ala
    50                  55                  60
Trp Ala Ile Gly Arg Gly Phe Val Gln Arg Tyr Phe Asp Asn Ser Leu
65                  70                  75                  80
Ser Tyr Asp Ser Pro Gln Ala Glu Arg Leu Arg Asp Ala Val Arg Gln
                85                  90                  95
Ala Lys Leu Thr Ala Val Ile Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Ala Arg Pro Asp Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Pro Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Ala Gly Gly Gly Phe Ala
                245                 250                 255
Ala Ile Tyr Gly Pro Asp Gly Ser Gln Ile Gly Glu Lys Leu Ala Pro
            260                 265                 270
Asp Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Lys Arg Tyr Gln Arg Val Glu Gln
305                 310                 315                 320
Phe Ala Leu Pro Ala Asp Met Val Glu Pro Ala Asp Ile Gly Ala Ala
                325                 330                 335
Ala Ser


<210> 31
<211> 933
<212> DNA
<213> Unknown

<220>
<223> Obtained from an environmental sample

<400> 31
   atgaccagaa tagccattat tcagcgaccg cccgtgctgc tcgatcgaag cgccaccatt 60
   gcccgggccg tgcaatcggt cgccgaagcg gcagcgcaag gcgcgaccct gattgtcttg 120
   cccgaatcgt acatccctgg ctatccctca tggatctggc ggctcgcgcc tggcaaagac 180
   ggcgcgatcg tgggccagtt gcatgcgcgc ttgctggcca atgcggtcga cctgagcagc 240
   actgacctcg atgcgcttct tgaagcggcc cgtcagcacg gcgtgaccat tgtttgcggc 300
   atgaacgagt gcgaacggcg tcgcggcggc ggcaccttgt acaacacggt ggtcgtgatc 360
   ggaccggacg gcgtcatgct caaccggcat cgcaaattga tgccgaccaa tcccgagcgc     420
   atggtgcatg gctttggcga tgcatccgga ctgaaagcag ttgatacgcc tgccggccgg     480
   ctgggcacgc tgatctgctg ggagagctac atgccgctgg cacgctatgc cctgtacgag 540
   caaggcatcg agatctacat cgcaccaact tatgacagtg gtgacggctg gatcagcacc 600
   atgcgccaca ttgcactcga agggcgctgc tgggtgattg gcagcggcac ggtcctgaaa 660
   ggcagtgata ttccggacga tttcccggaa cgggcacgcc tgttccctga tccggatgag 720
   tggatcaacg atggtgattc ggtagttatc gatccgcagg gaaagatcgt tgccggtccg 780
   atgcgtaggg aagcaggcat tctatacgcc gatatcgacg tcgcgcgcgt agcaccatca 840
   cgccgcacgc tggatgtcgc ggggcattac gcgcgtccgg acgtcttcga gcttcgggta 900
   caccaggcac cgggggcacg agtaagtaat tga 933

<210> 32
<211> 310
<212> PRT
<213> Unknown

<220>
<223> Obtained from an environmental sample

<400> 32
Met Thr Arg Ile Ala Ile Ile Gln Arg Pro Pro Val Leu Leu Asp Arg
1               5                   10                  15
Ser Ala Thr Ile Ala Arg Ala Val Gln Ser Val Ala Glu Ala Ala Ala
            20                  25                  30
Gln Gly Ala Thr Leu Ile Val Leu Pro Glu Ser Tyr Ile Pro Gly Tyr
        35                  40                  45
Pro Ser Trp Ile Trp Arg Leu Ala Pro Gly Lys Asp Gly Ala Ile Val
    50                  55                  60
Gly Gln Leu His Ala Arg Leu Leu Ala Asn Ala Val Asp Leu Ser Ser
65                  70                  75                  80
Thr Asp Leu Asp Ala Leu Leu Glu Ala Ala Arg Gln His Gly Val Thr
                85                  90                  95
Ile Val Cys Gly Met Asn Glu Cys Glu Arg Arg Arg Gly Gly Gly Thr
            100                 105                 110
Leu Tyr Asn Thr Val Val Val Ile Gly Pro Asp Gly Val Met Leu Asn
        115                 120                 125
Arg His Arg Lys Leu Met Pro Thr Asn Pro Glu Arg Met Val His Gly
    130                 135                 140
Phe Gly Asp Ala Ser Gly Leu Lys Ala Val Asp Thr Pro Ala Gly Arg
145                 150                 155                 160
Leu Gly Thr Leu Ile Cys Trp Glu Ser Tyr Met Pro Leu Ala Arg Tyr
                165                 170                 175
Ala Leu Tyr Glu Gln Gly Ile Glu Ile Tyr Ile Ala Pro Thr Tyr Asp
            180                 185                 190
Ser Gly Asp Gly Trp Ile Ser Thr Met Arg His Ile Ala Leu Glu Gly
        195                 200                 205
Arg Cys Trp Val Ile Gly Ser Gly Thr Val Leu Lys Gly Ser Asp Ile
    210                 215                 220
Pro Asp Asp Phe Pro Glu Arg Ala Arg Leu Phe Pro Asp Pro Asp Glu

225                 230                 235                 240
Trp Ile Asn Asp Gly Asp Ser Val Val Ile Asp Pro Gln Gly Lys Ile
                245                 250                 255
Val Ala Gly Pro Met Arg Arg Glu Ala Gly Ile Leu Tyr Ala Asp Ile
            260                 265                 270
Asp Val Ala Arg Val Ala Pro Ser Arg Arg Thr Leu Asp Val Ala Gly
        275                 280                 285
His Tyr Ala Arg Pro Asp Val Phe Glu Leu Arg Val His Gln Ala Pro
    290                 295                 300
Gly Ala Arg Val Ser Asn
305                 310

<210> 33
<211> 1026
<212> DNA
<213> Unknown

<220>
<223> Obtained from an environmental sample

<400> 33
atgttaagtc ccgtgacgca gtatcgcgcc gccgcggtgc aggcggcgcc atcttttctc      60
gatctcgacc gcaccgtcga gaagacgatc gcgatcatcg agcaggcggc cgagcaggat     120
gtgcgcctga tcgcgtttcc ggaaacctgg attcccggct atccgctctg gatctggctc     180
ggctcgccgg cctggggcat gcgcttcgtg cagcgctatt tcgagaactc gctggtgcgc     240
ggcagcaaac agtggaacgc gatcgccgat gcggcgcggc gccaccgcat gaccgtcgtc     300
gtcggcttca gcgagcgcgc gggaggcagc ctctacatgg gccaggcgat cttcggcccc     360
gaaggcgagc tcatcgcggc gcgccggaag ctcaagccga cacacgccga gcgaacggtg     420
ttcggcgagg gcgacggcag ccacttggcc gtttacgaga cgggcgttgg tcgcatcggc     480
gccctctgct gctgggagca catccagccg ctctcgaaat acgcgatgta tgcggccaac     540
gaacaggtgc atgtggcctc gtggccgtgc ttcagccttt atcgcggcat ggcctatgcg     600
ctcgggccgg aggtgaacac cgccgcgagc caggtctacg cggtcgaggg cggctgctac     660
gtgctggcct cctgtctcgt cgtgacaccc gagatcctga aggtgctgat cgacacgccc     720
gacaaggagc cgttgctgct cgccggcggg gggttctcga tgatcttcgg ccccgacggc     780
cgcgcgctcg cccagccgct gccggagacc gaagaggggc tcgtcacggc cgagatcgat     840
ctcggcgcga tcgcgctcgc caaggccgcg gccgatcccg ccggccatta cgcgcggccc     900
gacgtgacgc ggttgttgct gaacccgcgc cccgcggcgc gcgtcgaagc gctgggtccg     960
cgcttcgagg tcgtgcagag cgagcaggcc gagccgccca cgcaaccggc cgaagcggcg    1020
gattga                                                               1026

<210> 34
<211> 341
<212> PRT
<213> Unknown

<220>
<223> Obtained from an environmental sample

<400> 34
Met Leu Ser Pro Val Thr Gln Tyr Arg Ala Ala Ala Val Gln Ala Ala
 1               5                  10                  15
Pro Ser Phe Leu Asp Leu Asp Arg Thr Val Glu Lys Thr Ile Ala Ile
            20                  25                  30
Ile Glu Gln Ala Ala Glu Gln Asp Val Arg Leu Ile Ala Phe Pro Glu
        35                  40                  45
Thr Trp Ile Pro Gly Tyr Pro Leu Trp Ile Trp Leu Gly Ser Pro Ala
    50                  55                  60

Trp Gly Met Arg Phe Val Gln Arg Tyr Phe Glu Asn Ser Leu Val Arg
65                  70                  75                  80
Gly Ser Lys Gln Trp Asn Ala Ile Ala Asp Ala Ala Arg Arg His Arg
                85                  90                  95
Met Thr Val Val Val Gly Phe Ser Glu Arg Ala Gly Gly Ser Leu Tyr
            100                 105                 110
Met Gly Gln Ala Ile Phe Gly Pro Glu Gly Glu Leu Ile Ala Ala Arg
        115                 120                 125
Arg Lys Leu Lys Pro Thr His Ala Glu Arg Thr Val Phe Gly Glu Gly
    130                 135                 140
Asp Gly Ser His Leu Ala Val Tyr Glu Thr Gly Val Gly Arg Ile Gly
145                 150                 155                 160
Ala Leu Cys Cys Trp Glu His Ile Gln Pro Leu Ser Lys Tyr Ala Met
                165                 170                 175
Tyr Ala Ala Asn Glu Gln Val His Val Ala Ser Trp Pro Cys Phe Ser
            180                 185                 190
Leu Tyr Arg Gly Met Ala Tyr Ala Leu Gly Pro Glu Val Asn Thr Ala
        195                 200                 205
Ala Ser Gln Val Tyr Ala Val Glu Gly Gly Cys Tyr Val Leu Ala Ser
    210                 215                 220
Cys Leu Val Val Thr Pro Glu Ile Leu Lys Val Leu Ile Asp Thr Pro
225                 230                 235                 240
Asp Lys Glu Pro Leu Leu Leu Ala Gly Gly Gly Phe Ser Met Ile Phe
                245                 250                 255
Gly Pro Asp Gly Arg Ala Leu Ala Gln Pro Leu Pro Glu Thr Glu Glu
            260                 265                 270
Gly Leu Val Thr Ala Glu Ile Asp Leu Gly Ala Ile Ala Leu Ala Lys
        275                 280                 285
Ala Ala Ala Asp Pro Ala Gly His Tyr Ala Arg Pro Asp Val Thr Arg
    290                 295                 300
Leu Leu Leu Asn Pro Arg Pro Ala Ala Arg Val Glu Ala Leu Gly Pro
305                 310                 315                 320
Arg Phe Glu Val Val Gln Ser Glu Gln Ala Glu Pro Pro Thr Gln Pro
                325                 330                 335
Ala Glu Ala Ala Asp
            340

<210> 35
<211> 942
<212> DNA
<213> Unknown

<220>
<223> Obtained from an environmental sample

<400> 35
atgacagctc taaaaattgc tgctgttcaa atgtgcgccg aattgggcgc tacagatcga      60
aacctgagtg cagctggatc attcgtgcgc gacgcatttc gcgaaggtgc ccagtgggta     120
atcctcccag agttttttac ctcggcaatg gcattcgcac cttcgatggc gcaagcttgg     180
ttgccactgg aaggaaaggc gctagcgatg atgcgcagcc ttgcgcgtca attcgatggg     240
gttgttggag gctcatatgt tgccagagag gggaacgact gcgtaaatgc ctttcttctc     300
gtctttccgg atggaagcta ctaccggcat gacaaagata ttccaacaat gtgggagaac     360
tgttactaca tcggcggcgt cgacgatggg gtgctggaaa caccaattgg tgcggtggga     420
gttgcactgt gttgggagtt catccgaaca caaaccgccc gaagactgaa ggatcgcgtt     480
caattagtgg ttggcggtac ttgctggtgg gattttccga tgcctgtacc tgaacgatat     540
ctgaggctga ccaggcatat ctccaggaac tttgagcgcg atgctccggc gcggttggcc     600
agtatgttgg gtgtgcctgt tgtacacgct tcccatgctg gggattttac tgctgtcacc     660

ccaggcaatg aaacgaagaa ttaccgatcc aactatctgg gagagaccca gatcgtcgat 720
gccaatggaa atgtgttgaa gcgaatgaca gtggctgatg gtgagggtta cgtcattgct 780
gacgttcaat tgggggccat atcaaccggt cgaacttcga tccccgacac cttctggacc 840
tgcaagctaa cgccaggggc acaacaggct tgggatgaac aaaatgcttt tgggtgtggc 900
tactatgaga acgtcacacg caaacaccta atcggtcgat ga 942

<210> 36
<211> 313
<212> PRT
<213> Unknown

<220>
<223> Obtained from an environmental sample

<400> 36
Met Thr Ala Leu Lys Ile Ala Ala Val Gln Met Cys Ala Glu Leu Gly
1               5                   10                  15
Ala Thr Asp Arg Asn Leu Ser Ala Ala Gly Ser Phe Val Arg Asp Ala
            20                  25                  30
Phe Arg Glu Gly Ala Gln Trp Val Ile Leu Pro Glu Phe Phe Thr Ser
        35                  40                  45
Ala Met Ala Phe Ala Pro Ser Met Ala Gln Ala Trp Leu Pro Leu Glu
    50                  55                  60
Gly Lys Ala Leu Ala Met Met Arg Ser Leu Ala Arg Gln Phe Asp Gly
65                  70                  75                  80
Val Val Gly Gly Ser Tyr Val Ala Arg Glu Gly Asn Asp Cys Val Asn
                85                  90                  95
Ala Phe Leu Leu Val Phe Pro Asp Gly Ser Tyr Tyr Arg His Asp Lys
            100                 105                 110
Asp Ile Pro Thr Met Trp Glu Asn Cys Tyr Tyr Ile Gly Gly Val Asp
        115                 120                 125
Asp Gly Val Leu Glu Thr Pro Ile Gly Ala Val Gly Val Ala Leu Cys
    130                 135                 140
Trp Glu Phe Ile Arg Thr Gln Thr Ala Arg Arg Leu Lys Asp Arg Val
145                 150                 155                 160
Gln Leu Val Val Gly Gly Thr Cys Trp Trp Asp Phe Pro Met Pro Val
                165                 170                 175
Pro Glu Arg Tyr Leu Arg Leu Thr Arg His Ile Ser Arg Asn Phe Glu
            180                 185                 190
Arg Asp Ala Pro Ala Arg Leu Ala Ser Met Leu Gly Val Pro Val Val
        195                 200                 205
His Ala Ser His Ala Gly Asp Phe Thr Ala Val Thr Pro Gly Asn Glu
    210                 215                 220
Thr Lys Asn Tyr Arg Ser Asn Tyr Leu Gly Glu Thr Gln Ile Val Asp
225                 230                 235                 240
Ala Asn Gly Asn Val Leu Lys Arg Met Thr Val Ala Asp Gly Glu Gly
                245                 250                 255
Tyr Val Ile Ala Asp Val Gln Leu Gly Ala Ile Ser Thr Gly Arg Thr
            260                 265                 270
Ser Ile Pro Asp Thr Phe Trp Thr Cys Lys Leu Thr Pro Gly Ala Gln
        275                 280                 285
Gln Ala Trp Asp Glu Gln Asn Ala Phe Gly Cys Gly Tyr Tyr Glu Asn
    290                 295                 300
Val Thr Arg Lys His Leu Ile Gly Arg
305                 310

<210> 37

<211> 993
<212> DNA
<213> Unknown

<220>
<223> Obtained from an environmental sample

<400> 37
atggattcag atatgacgga tacttttaag gcagccatta ttcagcacgc gcctgttttt 60
ttaaatctcg aagagagtct ggacaaagcc ggcagcctta tagaaaaggc tgccgatcaa 120
ggcgcgaaag tgatcgcctt tcctgaaaca tggctgcccg gttatcccgt atggctcgac 180
tactctccaa aagcgggtct gtgggactat cagcctgcaa aatctctcta tcgtctgcta 240
gtcgataatt cagtcacctt acccggcaaa cacctcgatc aactcctctc catagcgcaa 300
aagaccggcg catatgttgt aatgggggca cacgaacgag tgggtggaac actctataac 360
acgacgatct atgttgggat tgatgggaag gagtacaaac ttcatagaaa gctggtgccg 420
acctataccg aaagattgat ctgggggcgg ggagacggca gcacattgag tgtgttgatg 480
acggattatg gcgttcttgg aggattgatc tgctgggagc actggatgcc tctggcaaga 540
gccgcaatgc atgccagata tgaaaccctt catgtggcgc aatggccggc tgtaaaagat 600
atccatcaga tagcaagcag acattatgct tttgaaggcc ggtgtttcgt gctcgcggca 660
ggctctgttc tgactcgaag agatataata gaaggattca actcactggc tcgcgccgat 720
agtgatgcat tggaacttct gaaagctatt tcgggagaag atagtgatct tattttgaat 780
gggggaagcg cgataattgc gccgaatgga gagtatcttg cgggcccggt ctttaatgaa 840
ccctccatta tttatgctga aattgatcct gcactgataa gtgagggcca tcttacactg 900
gatacaagcg gacactactc gcgccctgac atttttcgtc tggagataaa cgatcaacct 960
caacatgatg taactttcag atcggggcat tag 993

<210> 38
<211> 330
<212> PRT
<213> Unknown

<220>
<223> Obtained from an environmental sample

<400> 38
Met Asp Ser Asp Met Thr Asp Thr Phe Lys Ala Ala Ile Ile Gln His
1               5                   10                  15
Ala Pro Val Phe Leu Asn Leu Glu Glu Ser Leu Asp Lys Ala Gly Ser
            20                  25                  30
Leu Ile Glu Lys Ala Ala Asp Gln Gly Ala Lys Val Ile Ala Phe Pro
        35                  40                  45
Glu Thr Trp Leu Pro Gly Tyr Pro Val Trp Leu Asp Tyr Ser Pro Lys
    50                  55                  60
Ala Gly Leu Trp Asp Tyr Gln Pro Ala Lys Ser Leu Tyr Arg Leu Leu
65                  70                  75                  80
Val Asp Asn Ser Val Thr Leu Pro Gly Lys His Leu Asp Gln Leu Leu
                85                  90                  95
Ser Ile Ala Gln Lys Thr Gly Ala Tyr Val Val Met Gly Ala His Glu
            100                 105                 110
Arg Val Gly Gly Thr Leu Tyr Asn Thr Thr Ile Tyr Val Gly Ile Asp
        115                 120                 125
Gly Lys Glu Tyr Lys Leu His Arg Lys Leu Val Pro Thr Tyr Thr Glu
    130                 135                 140
Arg Leu Ile Trp Gly Arg Gly Asp Gly Ser Thr Leu Ser Val Leu Met
145                 150                 155                 160
Thr Asp Tyr Gly Val Leu Gly Gly Leu Ile Cys Trp Glu His Trp Met
                165                 170                 175

Pro Leu Ala Arg Ala Ala Met His Ala Arg Tyr Glu Thr Leu His Val
            180                 185                 190
Ala Gln Trp Pro Ala Val Lys Asp Ile His Gln Ile Ala Ser Arg His
        195                 200                 205
Tyr Ala Phe Glu Gly Arg Cys Phe Val Leu Ala Ala Gly Ser Val Leu
    210                 215                 220
Thr Arg Arg Asp Ile Ile Glu Gly Phe Asn Ser Leu Ala Arg Ala Asp
225                 230                 235                 240
Ser Asp Ala Leu Glu Leu Leu Lys Ala Ile Ser Gly Glu Asp Ser Asp
                245                 250                 255
Leu Ile Leu Asn Gly Gly Ser Ala Ile Ile Ala Pro Asn Gly Glu Tyr
            260                 265                 270
Leu Ala Gly Pro Val Phe Asn Glu Pro Ser Ile Ile Tyr Ala Glu Ile
        275                 280                 285
Asp Pro Ala Leu Ile Ser Glu Gly His Leu Thr Leu Asp Thr Ser Gly
    290                 295                 300
His Tyr Ser Arg Pro Asp Ile Phe Arg Leu Glu Ile Asn Asp Gln Pro
305                 310                 315                 320
Gln His Asp Val Thr Phe Arg Ser Gly His
                325                 330

<210> 39
<211> 1008
<212> DNA
<213> Unknown

<220>
<223> Obtained from an environmental sample

<400> 39
atgaaaaata tcaaaaactc agaaaaaagc agcacagtaa gagtcgctgc ggtacaaatc      60
agtccggtgt tgtacaaccg cgaagctacc gttcaaaaag tagtcaacaa aatccttgaa     120
ctaggaaaac aaggggtaca attcgccact tttccggaaa cgatagtgcc ttattatcct     180
tatttctctt ttattcaggc gccttatgcc atgggcaaag aacacctgcg cttgcttgaa     240
caatcagtta ctgttccgtc agccgcgacc gatgccataa gtgaggcggc aaaggaagcc     300
aatatggtag tgtctattgg tgtcaatgaa cgagacggtg gtaccattta caatacgcaa     360
ctcctttttg atgctgacgg aacattaatt cagcgcagac gtaaacttac accaacgtat     420
catgaaagaa tgatttgggg acaaggtgac gcttcaggtc ttcgtgccac agacagcgct     480
gttgggcgta tcgggcagtt ggcttgttgg gaacattaca atccattgtt ccgttatgct     540
ttgattgctg atggagaaca aatccattct gccatgtatc ccggatcatt tttaggtgcg     600
ttgcacggtg aacaaaccga aatcaatgta cgccaacacg ctttagaatc ggccagcttc     660
gtcgtagtgg ctaccggttg gttggatgcc gatcaacaag cacaaattgc gaaagacacc     720
ggtggaccaa tcggaccaat ttcgggaggt tgttttacag ccgttatagg ccctgacgga     780
caactaatcg gggaagccct tacatcaggt gaaggggaag tgattgccga tattgatttg     840
gcacaaattg atgcccgcaa aagattaatg gatgccagtg gtcactacaa ccgtcctgaa     900
ttgttgagct tgcatatcga tcacactccg actgctccta tgcatgaaag agtagtttac     960
actgagccgg gattagcaaa aagacaaaat gaaaattcat caaattaa                 1008

<210> 40
<211> 335
<212> PRT
<213> Unknown

<220>
<223> Obtained from an environmental sample

<400> 40

Met Lys Asn Ile Lys Asn Ser Glu Lys Ser Ser Thr Val Arg Val Ala
1               5                   10                  15
Ala Val Gln Ile Ser Pro Val Leu Tyr Asn Arg Glu Ala Thr Val Gln
            20                  25                  30
Lys Val Val Asn Lys Ile Leu Glu Leu Gly Lys Gln Gly Val Gln Phe
        35                  40                  45
Ala Thr Phe Pro Glu Thr Ile Val Pro Tyr Tyr Pro Tyr Phe Ser Phe
    50                  55                  60
Ile Gln Ala Pro Tyr Ala Met Gly Lys Glu His Leu Arg Leu Leu Glu
65                  70                  75                  80
Gln Ser Val Thr Val Pro Ser Ala Ala Thr Asp Ala Ile Ser Glu Ala
                85                  90                  95
Ala Lys Glu Ala Asn Met Val Val Ser Ile Gly Val Asn Glu Arg Asp
            100                 105                 110
Gly Gly Thr Ile Tyr Asn Thr Gln Leu Leu Phe Asp Ala Asp Gly Thr
        115                 120                 125
Leu Ile Gln Arg Arg Arg Lys Leu Thr Pro Thr Tyr His Glu Arg Met
    130                 135                 140
Ile Trp Gly Gln Gly Asp Ala Ser Gly Leu Arg Ala Thr Asp Ser Ala
145                 150                 155                 160
Val Gly Arg Ile Gly Gln Leu Ala Cys Trp Glu His Tyr Asn Pro Leu
                165                 170                 175
Phe Arg Tyr Ala Leu Ile Ala Asp Gly Glu Gln Ile His Ser Ala Met
            180                 185                 190
Tyr Pro Gly Ser Phe Leu Gly Ala Leu His Gly Glu Gln Thr Glu Ile
        195                 200                 205
Asn Val Arg Gln His Ala Leu Glu Ser Ala Ser Phe Val Val Val Ala
    210                 215                 220
Thr Gly Trp Leu Asp Ala Asp Gln Gln Ala Gln Ile Ala Lys Asp Thr
225                 230                 235                 240
Gly Gly Pro Ile Gly Pro Ile Ser Gly Gly Cys Phe Thr Ala Val Ile
                245                 250                 255
Gly Pro Asp Gly Gln Leu Ile Gly Glu Ala Leu Thr Ser Gly Glu Gly
            260                 265                 270
Glu Val Ile Ala Asp Ile Asp Leu Ala Gln Ile Asp Ala Arg Lys Arg
        275                 280                 285
Leu Met Asp Ala Ser Gly His Tyr Asn Arg Pro Glu Leu Leu Ser Leu
    290                 295                 300
His Ile Asp His Thr Pro Thr Ala Pro Met His Glu Arg Val Val Tyr
305                 310                 315                 320
Thr Glu Pro Gly Leu Ala Lys Arg Gln Asn Glu Asn Ser Ser Asn
                325                 330                 335

<210> 41
<211> 966
<212> DNA
<213> Unknown

<220>
<223> Obtained from an environmental sample

<400> 41
atgcccaatg agaataacat cgctacattc aaagttgccg cagtccaggc cacaccggtg      60
tttcttgatc gtgaagcaac catcgacaaa gcttgcgggt tgattgccac tgccggcaat     120
gaaggagcgc gcctgattgt gtttccagaa gcgttcatcc caacctatcc tgaatgggtt     180
tggggtattc cttccggtga gcaaggttta ctcaatgaac tctatgcaga gctgctcacc     240
aatgcggtca ctattcccag tgacgcgact gacaggctgt gcgaggccgc gcagcttgcg     300

aatgcctacg tagtgatggg aatgagcgaa cggaatgtcg aggcgagtgg cgcaagccta 360
tataatacgc tgttgtacat caatgcgcag ggggagattt tagggaaaca tcgaaagctg 420
gtgccaacgg gcggcgaacg cctggtatgg gcgcagggtg atggcagcac gctgcaggtc 480
tacgatacgc cattgggaaa actcggtggt ctcatttgct gggaaaatta tatgccgctg 540
gcacgctatg ctatgtatgc ctgggggaca caaatctatg tcgcggcaac gtgggatcga 600
ggccaaccct ggctttctac attacggcat atcgccaaag aaggcagggt atacgtgatt 660
ggttgctgta tcgcgatgcg aaaagacgat attccggata gttactccat gaagcagaaa 720
taccatgctg aaatggatga atggattaat gttggcgaca gtgtgattgt caatcccgaa 780
ggacacttta tcgcagggcc tgtgcgcaag caagaagaaa ttctctacgc ggagatcgat 840
ccacgtatgg tgcaaggccc gaagtggatg ctcgatgtgg cggggcatta tgcgagacca 900
gatgtgttcc agttgacggt gcatacgaat gtgagagaga tgatgcgggt ggaagatgat 960
tcataa 966

<210> 42
<211> 321
<212> PRT
<213> Unknown

<220>
<223> Obtained from an environmental sample

<400> 42
Met Pro Asn Glu Asn Asn Ile Ala Thr Phe Lys Val Ala Ala Val Gln
1               5                   10                  15
Ala Thr Pro Val Phe Leu Asp Arg Glu Ala Thr Ile Asp Lys Ala Cys
            20                  25                  30
Gly Leu Ile Ala Thr Ala Gly Asn Glu Gly Ala Arg Leu Ile Val Phe
        35                  40                  45
Pro Glu Ala Phe Ile Pro Thr Tyr Pro Glu Trp Val Trp Gly Ile Pro
    50                  55                  60
Ser Gly Glu Gln Gly Leu Leu Asn Glu Leu Tyr Ala Glu Leu Leu Thr
65                  70                  75                  80
Asn Ala Val Thr Ile Pro Ser Asp Ala Thr Asp Arg Leu Cys Glu Ala
                85                  90                  95
Ala Gln Leu Ala Asn Ala Tyr Val Val Met Gly Met Ser Glu Arg Asn
            100                 105                 110
Val Glu Ala Ser Gly Ala Ser Leu Tyr Asn Thr Leu Leu Tyr Ile Asn
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Ala Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Asp Ser Tyr Ser Met Lys Gln Lys
225                 230                 235                 240
Tyr His Ala Glu Met Asp Glu Trp Ile Asn Val Gly Asp Ser Val Ile
                245                 250                 255
Val Asn Pro Glu Gly His Phe Ile Ala Gly Pro Val Arg Lys Gln Glu
            260                 265                 270
Glu Ile Leu Tyr Ala Glu Ile Asp Pro Arg Met Val Gln Gly Pro Lys

275                 280                 285
Trp Met Leu Asp Val Ala Gly His Tyr Ala Arg Pro Asp Val Phe Gln
    290                 295                 300
Leu Thr Val His Thr Asn Val Arg Glu Met Met Arg Val Glu Asp Asp
305                 310                 315                 320
Ser

<210> 43
<211> 993
<212> DNA
<213> Unknown

<220>
<223> Obtained from an environmental sample

<400> 43
atgagagtcg ttaaagccgc ggcggtccaa ctgaaacctg tcctctatag ccgagaggga      60
actgtcgaaa acgtcgtccg taaaatccac gagcttggac agcaaggggt acagttcgcg     120
acgtttccag agactgtggt gccttactac ccgtactttt cgatcgtgca gtccggctat     180
caaattcttg gcggtggtga gttcctgaag ctgcttgatc agtcagtaac cgtgccatct     240
ctcgctacgg aagcgatcgg cgaggcttgc aggcaggcgg gcgtcgttgt ctccatcggc     300
gtcaacgagc gtgatggagg aactctatac aacacgcaac ttctctttga tgccgacgga     360
acattgattc aaagacgacg caagatcaca cccacccatt acgagcgcat ggtctggggc     420
cagggcgatg gctcaggttt acgggccatt gacagcaagg tcgcgcgcat tggtcaactg     480
gcgtgttttg agcactacaa ccctctcgca cgttacgcga tgatggccga tggcgagcag     540
atccattctg cgatgttccc cggctccatg ttcggcgata atttttcaga gaaggtggaa     600
atcaacataa ggcagcatgc aatggagtct gggtgctttg tcgtttgcgc tactgcctgg     660
ttggatgctg accagcaggc tcaaatcatg aaagacacgg gatgtgagat cggaccgatc     720
tcaggaggtt gcttcacagc gatcgcggca ccagatggaa gccttatagg tgaacccatc     780
cgctcaggtg aaggcgtttg tattgccgac ctcgatttca aacttatcga caagcggaag     840
cacgtagtag acacacgcgg ccattatagc cggccagaat tgctcagcct cctgattgat     900
cggacgccga cggcccacat acacgaaagg accgagcaac cgagggcggc catcgagaaa     960
gagtcgcagg atgttttcac cgctgttgct taa                                  993

<210> 44
<211> 330
<212> PRT
<213> Unknown

<220>
<223> Obtained from an environmental sample

<400> 44
Met Arg Val Val Lys Ala Ala Ala Val Gln Leu Lys Pro Val Leu Tyr
1               5                   10                  15
Ser Arg Glu Gly Thr Val Glu Asn Val Val Arg Lys Ile His Glu Leu
            20                  25                  30
Gly Gln Gln Gly Val Gln Phe Ala Thr Phe Pro Glu Thr Val Val Pro
        35                  40                  45
Tyr Tyr Pro Tyr Phe Ser Ile Val Gln Ser Gly Tyr Gln Ile Leu Gly
    50                  55                  60
Gly Gly Glu Phe Leu Lys Leu Leu Asp Gln Ser Val Thr Val Pro Ser
65                  70                  75                  80
Leu Ala Thr Glu Ala Ile Gly Glu Ala Cys Arg Gln Ala Gly Val Val
                85                  90                  95
Val Ser Ile Gly Val Asn Glu Arg Asp Gly Gly Thr Leu Tyr Asn Thr

100                 105                 110
Gln Leu Leu Phe Asp Ala Asp Gly Thr Leu Ile Gln Arg Arg Arg Lys
        115                 120                 125
Ile Thr Pro Thr His Tyr Glu Arg Met Val Trp Gly Gln Gly Asp Gly
    130                 135                 140
Ser Gly Leu Arg Ala Ile Asp Ser Lys Val Ala Arg Ile Gly Gln Leu
145                 150                 155                 160
Ala Cys Phe Glu His Tyr Asn Pro Leu Ala Arg Tyr Ala Met Met Ala
                165                 170                 175
Asp Gly Glu Gln Ile His Ser Ala Met Phe Pro Gly Ser Met Phe Gly
            180                 185                 190
Asp Asn Phe Ser Glu Lys Val Glu Ile Asn Ile Arg Gln His Ala Met
        195                 200                 205
Glu Ser Gly Cys Phe Val Val Cys Ala Thr Ala Trp Leu Asp Ala Asp
    210                 215                 220
Gln Gln Ala Gln Ile Met Lys Asp Thr Gly Cys Glu Ile Gly Pro Ile
225                 230                 235                 240
Ser Gly Gly Cys Phe Thr Ala Ile Ala Ala Pro Asp Gly Ser Leu Ile
                245                 250                 255
Gly Glu Pro Ile Arg Ser Gly Glu Gly Val Cys Ile Ala Asp Leu Asp
            260                 265                 270
Phe Lys Leu Ile Asp Lys Arg Lys His Val Val Asp Thr Arg Gly His
        275                 280                 285
Tyr Ser Arg Pro Glu Leu Leu Ser Leu Leu Ile Asp Arg Thr Pro Thr
    290                 295                 300
Ala His Ile His Glu Arg Thr Glu Gln Pro Arg Ala Ala Ile Glu Lys
305                 310                 315                 320
Glu Ser Gln Asp Val Phe Thr Ala Val Ala
                325                 330

<210> 45
<211> 996
<212> DNA
<213> Unknown

<220>
<223> Obtained from an environmental sample

<400> 45
gtgaaaccgc cgacgtcatt ccgcgtggcc gccgttcagg cctgtcccgt ctacctcgat      60
cgcgacctga cgatcggcaa ggcagaaggg ttgatcgccg aggcggctgg aaacggcgcg     120
aacctcatcg tgttccccga agcgttcgtg cctggctatc cggtgtgggt gtggttcatc     180
ccgcccggtc gcacggcgga tcttcgcgaa gcgtacagcg tcctccacgc caactcgatc     240
gcggtcccca gccagtcgac cgagcgtctg tgcgcggcgg cgcggcgcgc tggcgtcgcc     300
gtggcgattg gtgtcaacga aagaaacagc gaggccagcg gcggcagcct cttcaacacg     360
ctgctgtaca tcggaccgga cggcacgctg ctcggtaaac accgaaagct ggtgccaaca     420
ggcggagagc gtcttgtctg ggccagcggc gacggcagcg accttgccgt gttcacactg     480
cctttcgcgc gagtcggcgg actgatctgc tgggagaact acatgccgct cgcccgctac     540
gcgctggcgg cctggggtgc gcaaatccac gtggcgccga cctgggaccg cggcgagccg     600
tggctctcaa cactgcgtca tgtcgcgaag gaaggtagag ccgtgacgat cggctgctgt     660
caggccgtcc gcaaggaaga cattccggac gggctggcat tcaagtcccg atacctggcc     720
gacgtgggcg cctgggtcaa cccaggcggg agcgtcatcg tcgatcccga cggaaaaatt     780
cttgccggac ctgcgaacga aaccgaaggc atcttgtacg ctgacatcag ggccgatcag     840
ctcgtcgggc cgagatggca actcgacatt gccggacact acgcgcggcc ggacgtcttc     900
gagctgatcg tgcatcggcg ttcgacgccg atgattcgcg aggtctcggc gcctcgtcgt     960
cgcgcaagaa cgggaaagcg accgcgacgc cgctga                               996

<210> 46
<211> 331
<212> PRT
<213> Unknown

<220>
<223> Obtained from an environmental sample

<400> 46
Val Lys Pro Pro Thr Ser Phe Arg Val Ala Ala Val Gln Ala Cys Pro
1               5                   10                  15
Val Tyr Leu Asp Arg Asp Leu Thr Ile Gly Lys Ala Glu Gly Leu Ile
            20                  25                  30
Ala Glu Ala Ala Gly Asn Gly Ala Asn Leu Ile Val Phe Pro Glu Ala
        35                  40                  45
Phe Val Pro Gly Tyr Pro Val Trp Val Trp Phe Ile Pro Pro Gly Arg
    50                  55                  60
Thr Ala Asp Leu Arg Glu Ala Tyr Ser Val Leu His Ala Asn Ser Ile
65                  70                  75                  80
Ala Val Pro Ser Gln Ser Thr Glu Arg Leu Cys Ala Ala Ala Arg Arg
                85                  90                  95
Ala Gly Val Ala Val Ala Ile Gly Val Asn Glu Arg Asn Ser Glu Ala
            100                 105                 110
Ser Gly Gly Ser Leu Phe Asn Thr Leu Leu Tyr Ile Gly Pro Asp Gly
        115                 120                 125
Thr Leu Leu Gly Lys His Arg Lys Leu Val Pro Thr Gly Gly Glu Arg
    130                 135                 140
Leu Val Trp Ala Ser Gly Asp Gly Ser Asp Leu Ala Val Phe Thr Leu
145                 150                 155                 160
Pro Phe Ala Arg Val Gly Gly Leu Ile Cys Trp Glu Asn Tyr Met Pro
                165                 170                 175
Leu Ala Arg Tyr Ala Leu Ala Ala Trp Gly Ala Gln Ile His Val Ala
            180                 185                 190
Pro Thr Trp Asp Arg Gly Glu Pro Trp Leu Ser Thr Leu Arg His Val
        195                 200                 205
Ala Lys Glu Gly Arg Ala Val Thr Ile Gly Cys Cys Gln Ala Val Arg
    210                 215                 220
Lys Glu Asp Ile Pro Asp Gly Leu Ala Phe Lys Ser Arg Tyr Leu Ala
225                 230                 235                 240
Asp Val Gly Ala Trp Val Asn Pro Gly Gly Ser Val Ile Val Asp Pro
                245                 250                 255
Asp Gly Lys Ile Leu Ala Gly Pro Ala Asn Glu Thr Glu Gly Ile Leu
            260                 265                 270
Tyr Ala Asp Ile Arg Ala Asp Gln Leu Val Gly Pro Arg Trp Gln Leu
        275                 280                 285
Asp Ile Ala Gly His Tyr Ala Arg Pro Asp Val Phe Glu Leu Ile Val
    290                 295                 300
His Arg Arg Ser Thr Pro Met Ile Arg Glu Val Ser Ala Pro Arg Arg
305                 310                 315                 320
Arg Ala Arg Thr Gly Lys Arg Pro Arg Arg Arg
                325                 330

<210> 47
<211> 1014
<212> DNA
<213> Unknown

<220>
<223> Obtained from an environmental sample

<400> 47
gtgaaagaag caatcaaagt agcctgtgtg caagcagctc cagtctttct cgacctggac 60
gccacagtgg acaagaccgt cgccctgatt gaggaggcag cccgtaacgg cgcacgccta 120
atcgcctttc cagagacctg gattccaggc tacccatggt tcctttggct ggactcacca 180
gcctggggga tgcaattcgt gcgccgatac cacgagaact cactggtcct cgacagccct 240
caggccaagc gcatcagtga ggccgcccag cgcgccggta tatacgtcgc gctagggtac 300
agcgaacgcg tgagcggaac cctctacatg gggcagtggc tcattgacga taagggcgaa 360
acagctgggc tgcgccgaaa gctgaaacca acccatgtag agcgaaccct cttcggtgaa 420
ggcgacggat catccctttc cactttcgac acaccgttgg gggtgctggg cggactctgc 480
tgttgggaac acttacaacc tctttcgaaa tatgcgctct acgcacagaa cgaggaaata 540
cacttcgccg cctggcctag cttcagcatc taccgtcaag cgacagaagt ccttggacca 600
gaagtaaatg tcgcagcttc tcggatctac gccgtggaag ggcagtgttt tgttctcgct 660
tcctgcgcgc tcgtctcgcc agagatgatc gaaatgctct gcactgacga aagcaagcac 720
agccttcttc aggccggcgg cgggtactcc cgcattatcg gtcccgatgg cagcgaccta 780
gcgcgcccct tgggcgaaaa cgaggaaggt attctctatg ccactctgga ccctgccgct 840
cgaatctatg caaagaccgc agctgatcca gccgggcact actccagacc agacgtcact 900
cggctgctga tcaatcgcag tgccaatcag ccagtcgtag aggttggaag ggaaatacct 960
gcatcggccc aaggctttga agttgaggcg gcccccgggt acgaaggcga ttga 1014

<210> 48
<211> 337
<212> PRT
<213> Unknown

<220>
<223> Obtained from an environmental sample

<400> 48
Val Lys Glu Ala Ile Lys Val Ala Cys Val Gln Ala Ala Pro Val Phe
1               5                   10                  15
Leu Asp Leu Asp Ala Thr Val Asp Lys Thr Val Ala Leu Ile Glu Glu
            20                  25                  30
Ala Ala Arg Asn Gly Ala Arg Leu Ile Ala Phe Pro Glu Thr Trp Ile
        35                  40                  45
Pro Gly Tyr Pro Trp Phe Leu Trp Leu Asp Ser Pro Ala Trp Gly Met
    50                  55                  60
Gln Phe Val Arg Arg Tyr His Glu Asn Ser Leu Val Leu Asp Ser Pro
65                  70                  75                  80
Gln Ala Lys Arg Ile Ser Glu Ala Ala Gln Arg Ala Gly Ile Tyr Val
                85                  90                  95
Ala Leu Gly Tyr Ser Glu Arg Val Ser Gly Thr Leu Tyr Met Gly Gln
            100                 105                 110
Trp Leu Ile Asp Asp Lys Gly Glu Thr Ala Gly Leu Arg Arg Lys Leu
        115                 120                 125
Lys Pro Thr His Val Glu Arg Thr Leu Phe Gly Glu Gly Asp Gly Ser
    130                 135                 140
Ser Leu Ser Thr Phe Asp Thr Pro Leu Gly Val Leu Gly Gly Leu Cys
145                 150                 155                 160
Cys Trp Glu His Leu Gln Pro Leu Ser Lys Tyr Ala Leu Tyr Ala Gln
                165                 170                 175
Asn Glu Glu Ile His Phe Ala Ala Trp Pro Ser Phe Ser Ile Tyr Arg
            180                 185                 190
Gln Ala Thr Glu Val Leu Gly Pro Glu Val Asn Val Ala Ala Ser Arg
        195                 200                 205

Ile Tyr Ala Val Glu Gly Gln Cys Phe Val Leu Ala Ser Cys Ala Leu
    210                 215                 220
Val Ser Pro Glu Met Ile Glu Met Leu Cys Thr Asp Glu Ser Lys His
225                 230                 235                 240
Ser Leu Leu Gln Ala Gly Gly Gly Tyr Ser Arg Ile Ile Gly Pro Asp
                245                 250                 255
Gly Ser Asp Leu Ala Arg Pro Leu Gly Glu Asn Glu Glu Gly Ile Leu
            260                 265                 270
Tyr Ala Thr Leu Asp Pro Ala Ala Arg Ile Tyr Ala Lys Thr Ala Ala
        275                 280                 285
Asp Pro Ala Gly His Tyr Ser Arg Pro Asp Val Thr Arg Leu Leu Ile
    290                 295                 300
Asn Arg Ser Ala Asn Gln Pro Val Val Glu Val Gly Arg Glu Ile Pro
305                 310                 315                 320
Ala Ser Ala Gln Gly Phe Glu Val Glu Ala Ala Pro Gly Tyr Glu Gly
                325                 330                 335
Asp

<210> 49
<211> 1038
<212> DNA
<213> Unknown

<220>
<223> Obtained from an environmental sample

<400> 49
atgaacaaag tcgtggctgc tgccgttcag tgcagcccgg tgctttactc ttgcgccgga      60
actgtaaata aaatttgcga gtggattgca gatttgggca aacaaggggt tgagctggcg     120
gtgttcgcgg aaaccctggt gccttactac ccgtattttt cttttatcca ggctccttgt     180
gcgatgggcg cgcaacattt gttgttgatg caagaatcag tagaggttcc ttccatctac     240
acgcaacaaa ttgccgctgc agcaaaagca gcgaagatgg tggtgtcagt tggtattaac     300
gaacgcgacg gcggttctat ttataacgcg caattattat ttgatgcggg cggtcagctt     360
gttcagcacc gccgaaaaat tacgccgaca tttcatgagc gcatggtgtg ggggcagggc      420
gatggctccg gtttgtgcgc agtggatacg gcagttggtc gtgttggttc gctcgcttgc     480
tgggaacatt acaacccact cgcgcgttac gcattgatgg cagatcgcga acaaattcac     540
gtgagtatgt ttcccggttc tttggtcggc gaaatttttg ccgagcaaat tgaagcaact      600
attcgtcacc acgcattgga gtccggttgc tttgtggtaa atgcgacggg ctggttaacg     660
ccggaacagc aagctcaaat cgtaaaagat actggtggtc ctatcgctgc cattagcggt     720
ggttgtttca ccgccattgt ttcaccggaa ggaaaattgc tcggcacgcc attgcgcagt     780
gattccgggg agggtgcctg tatcgccgaa ctggatttta atctcatcaa taagcgtaag     840
cgcatgatgg attctgtcgg ccattacagt cgtcctgaat tgctcagttt gctgattgat     900
aaaacaccga caagtcatac acatccgctt aaaaaacctt tggctcccag tgaaaaaaat     960
acgccagagg atatcgccac tggtttaaca ctggtcactc ccgtttcaaa tgcaaacctt    1020
ttcagcgcaa gcaactag                                                  1038

<210> 50
<211> 345
<212> PRT
<213> Unknown

<220>
<223> Obtained from an environmental sample

<400> 50
Met Asn Lys Val Val Ala Ala Ala Val Gln Cys Ser Pro Val Leu Tyr

1                   5                   10                  15
Ser Cys Ala Gly Thr Val Asn Lys Ile Cys Glu Trp Ile Ala Asp Leu
            20                  25                  30
Gly Lys Gln Gly Val Glu Leu Ala Val Phe Ala Glu Thr Leu Val Pro
        35                  40                  45
Tyr Tyr Pro Tyr Phe Ser Phe Ile Gln Ala Pro Cys Ala Met Gly Ala
    50                  55                  60
Gln His Leu Leu Leu Met Gln Glu Ser Val Glu Val Pro Ser Ile Tyr
65                  70                  75                  80
Thr Gln Gln Ile Ala Ala Ala Ala Lys Ala Ala Lys Met Val Val Ser
                85                  90                  95
Val Gly Ile Asn Glu Arg Asp Gly Gly Ser Ile Tyr Asn Ala Gln Leu
            100                 105                 110
Leu Phe Asp Ala Gly Gly Gln Leu Val Gln His Arg Arg Lys Ile Thr
        115                 120                 125
Pro Thr Phe His Glu Arg Met Val Trp Gly Gln Gly Asp Gly Ser Gly
    130                 135                 140
Leu Cys Ala Val Asp Thr Ala Val Gly Arg Val Gly Ser Leu Ala Cys
145                 150                 155                 160
Trp Glu His Tyr Asn Pro Leu Ala Arg Tyr Ala Leu Met Ala Asp Arg
                165                 170                 175
Glu Gln Ile His Val Ser Met Phe Pro Gly Ser Leu Val Gly Glu Ile
            180                 185                 190
Phe Ala Glu Gln Ile Glu Ala Thr Ile Arg His His Ala Leu Glu Ser
        195                 200                 205
Gly Cys Phe Val Val Asn Ala Thr Gly Trp Leu Thr Pro Glu Gln Gln
    210                 215                 220
Ala Gln Ile Val Lys Asp Thr Gly Gly Pro Ile Ala Ala Ile Ser Gly
225                 230                 235                 240
Gly Cys Phe Thr Ala Ile Val Ser Pro Glu Gly Lys Leu Leu Gly Thr
                245                 250                 255
Pro Leu Arg Ser Asp Ser Gly Glu Gly Ala Cys Ile Ala Glu Leu Asp
            260                 265                 270
Phe Asn Leu Ile Asn Lys Arg Lys Arg Met Met Asp Ser Val Gly His
        275                 280                 285
Tyr Ser Arg Pro Glu Leu Leu Ser Leu Leu Ile Asp Lys Thr Pro Thr
    290                 295                 300
Ser His Thr His Pro Leu Lys Lys Pro Leu Ala Pro Ser Glu Lys Asn
305                 310                 315                 320
Thr Pro Glu Asp Ile Ala Thr Gly Leu Thr Leu Val Thr Pro Val Ser
                325                 330                 335
Asn Ala Asn Leu Phe Ser Ala Ser Asn
            340                 345

<210> 51
<211> 897
<212> DNA
<213> Unknown

<220>
<223> Obtained from an environmental sample

<400> 51
gtgaacgtcc gcgtcgcggt ggtgcaggcc acgccggccg tgctcgacgg gccggcgtcg      60
gtgcggaagg cctgccgcct gatcggcgag gccgcggccg gcggcgcccg cctgatcgcc     120
ctgcccgagg gcttcgtgcc catcatgccg cgctcctgct gggggcacca cttcgcgctg     180
atcgcctcgc cgaagtcggc ggccctgcac cggcgcatct gggagaacgc cgtcgacgtc     240

ggcggcccgc tggcccgcga gctcggcgac gccgcgcgcc gcgcggacgc ctgggtggcc 300
atcggggtga acgagcgcga cgcccgccgg ccgggcacgc tctggaacac gctgctctgg 360
ttcgcgcccg acgggagcct ggcccggcgc caccgcaagc tcgtgcccac catgcacgag 420
cgcacgttct gggggcaggg cgcgggcgac gacctcgagg cgctggccgc ggacttcggc 480
cgcctgggcg gcctgatctg ctgggagaac ttcatgcccg ccgcgcgccg gcgcctgcac 540
cgggacgggg tcgacttcta cctggccccc acggcggacg accgggacat ctgggtcgcc 600
gcgatgcgca cgttcgcctt cgaggccggc gccttcgtcc tctcgccggt gcagtacctg 660
cggaccgccg acttcccgga ggacttcccg ctgcgcgagg agctcgccga ctgccccgag 720
gtccagttca ccgggggcag cgtgatctgc gacccgtggg gcaacctcct ggcggggccg 780
gtccacgggg gcgaggagat cctctacgcc gactgcgatc tcgacctcgt cctcgaggcc 840
cgacgggtgc tcgacacggc cggccactac gaccgcccgg acctcgcctc ggcctga 897

<210> 52
<211> 298
<212> PRT
<213> Unknown

<220>
<223> Obtained from an environmental sample

<400> 52
Val Asn Val Arg Val Ala Val Val Gln Ala Thr Pro Ala Val Leu Asp
1               5                   10                  15
Gly Pro Ala Ser Val Arg Lys Ala Cys Arg Leu Ile Gly Glu Ala Ala
            20                  25                  30
Ala Gly Gly Ala Arg Leu Ile Ala Leu Pro Glu Gly Phe Val Pro Ile
        35                  40                  45
Met Pro Arg Ser Cys Trp Gly His His Phe Ala Leu Ile Ala Ser Pro
    50                  55                  60
Lys Ser Ala Ala Leu His Arg Arg Ile Trp Glu Asn Ala Val Asp Val
65                  70                  75                  80
Gly Gly Pro Leu Ala Arg Glu Leu Gly Asp Ala Ala Arg Arg Ala Asp
                85                  90                  95
Ala Trp Val Ala Ile Gly Val Asn Glu Arg Asp Ala Arg Arg Pro Gly
            100                 105                 110
Thr Leu Trp Asn Thr Leu Leu Trp Phe Ala Pro Asp Gly Ser Leu Ala
        115                 120                 125
Arg Arg His Arg Lys Leu Val Pro Thr Met His Glu Arg Thr Phe Trp
    130                 135                 140
Gly Gln Gly Ala Gly Asp Asp Leu Glu Ala Leu Ala Ala Asp Phe Gly
145                 150                 155                 160
Arg Leu Gly Gly Leu Ile Cys Trp Glu Asn Phe Met Pro Ala Ala Arg
                165                 170                 175
Arg Arg Leu His Arg Asp Gly Val Asp Phe Tyr Leu Ala Pro Thr Ala
            180                 185                 190
Asp Asp Arg Asp Ile Trp Val Ala Ala Met Arg Thr Phe Ala Phe Glu
        195                 200                 205
Ala Gly Ala Phe Val Leu Ser Pro Val Gln Tyr Leu Arg Thr Ala Asp
    210                 215                 220
Phe Pro Glu Asp Phe Pro Leu Arg Glu Glu Leu Ala Asp Cys Pro Glu
225                 230                 235                 240
Val Gln Phe Thr Gly Gly Ser Val Ile Cys Asp Pro Trp Gly Asn Leu
                245                 250                 255
Leu Ala Gly Pro Val His Gly Gly Glu Glu Ile Leu Tyr Ala Asp Cys
            260                 265                 270
Asp Leu Asp Leu Val Leu Glu Ala Arg Arg Val Leu Asp Thr Ala Gly
        275                 280                 285

His Tyr Asp Arg Pro Asp Leu Ala Ser Ala
    290                 295

<210> 53
<211> 954
<212> DNA
<213> Unknown

<220>
<223> Obtained from an environmental sample

<400> 53
atggtcaatt caaagagtca gattaaaatc gcggcggtgc aggctgcccc ggtctttctg      60
gaacgggagg cgacgattga caaagcttgc cggttgattg cggaggcagg cgagcagggc     120
gcgaatctgg tggtcttccc tgagtcattc gtcccggctt atcccgattg ggtctgggcc     180
gttccggcag gtgaaacaac gctcctgaac acgctctatg ccgaactgct ggccaatgcc     240
gttgaaattc cgggtccggc gacagagcgg ctgagccagg cagccaacct ggccggggtt     300
tatgtcgcga ttggcttgac cgagcggaac atcgaggcca gtggggcgag cctgtacaat     360
actttgctct ttctcgactc agccggcggc atgttaggca agcatcgcaa actgatcccc     420
accggcggcg agcgcctggt ctgggctcag ggtgatggca gcactctggc ggtgtacgag     480
actaggtttg gaaaaatggg agggttgatt tgctgggaga attacatgcc cctggcccgt     540
tatgccttgt atgcctgggg gacgcagatt tacatcgcgg ccacctggga tcgaggcgag     600
ccgtggctgt caacgctgcg gcatatcgcc gcggaaggcc gggttgttgt cgtcggctgt     660
ggcatggccc tgcgcaaagc cgacctgccc gaccgctttg aactcaagca gcgattttac     720
cagaacgccg atgagtggat caatgtcggc gacagcgcga ttgttaaccc tgatggtgaa     780
ttcatcgccg ggccgctgcg cgagcaggaa ggcatcctct atgctgagat tgatctggcc     840
cagatgcgcg gccccaaatg gatgctcgac gtggccggcc attacgctcg cccggatgtg     900
tttgaactca tcgttcatcg ggaggcgcgg cccatgattg cgctaatttc atga           954

<210> 54
<211> 317
<212> PRT
<213> Unknown

<220>
<223> Obtained from an environmental sample

<400> 54
Met Val Asn Ser Lys Ser Gln Ile Lys Ile Ala Ala Val Gln Ala Ala
1               5                   10                  15
Pro Val Phe Leu Glu Arg Glu Ala Thr Ile Asp Lys Ala Cys Arg Leu
            20                  25                  30
Ile Ala Glu Ala Gly Glu Gln Gly Ala Asn Leu Val Val Phe Pro Glu
        35                  40                  45
Ser Phe Val Pro Ala Tyr Pro Asp Trp Val Trp Ala Val Pro Ala Gly
    50                  55                  60
Glu Thr Thr Leu Leu Asn Thr Leu Tyr Ala Glu Leu Leu Ala Asn Ala
65                  70                  75                  80
Val Glu Ile Pro Gly Pro Ala Thr Glu Arg Leu Ser Gln Ala Ala Asn
                85                  90                  95
Leu Ala Gly Val Tyr Val Ala Ile Gly Leu Thr Glu Arg Asn Ile Glu
            100                 105                 110
Ala Ser Gly Ala Ser Leu Tyr Asn Thr Leu Leu Phe Leu Asp Ser Ala
        115                 120                 125
Gly Gly Met Leu Gly Lys His Arg Lys Leu Ile Pro Thr Gly Gly Glu
    130                 135                 140
Arg Leu Val Trp Ala Gln Gly Asp Gly Ser Thr Leu Ala Val Tyr Glu

145                 150                 155                 160
Thr Arg Phe Gly Lys Met Gly Gly Leu Ile Cys Trp Glu Asn Tyr Met
                165                 170                 175
Pro Leu Ala Arg Tyr Ala Leu Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Ala Glu Gly Arg Val Val Val Val Gly Cys Gly Met Ala Leu
    210                 215                 220
Arg Lys Ala Asp Leu Pro Asp Arg Phe Glu Leu Lys Gln Arg Phe Tyr
225                 230                 235                 240
Gln Asn Ala Asp Glu Trp Ile Asn Val Gly Asp Ser Ala Ile Val Asn
                245                 250                 255
Pro Asp Gly Glu Phe Ile Ala Gly Pro Leu Arg Glu Gln Glu Gly Ile
            260                 265                 270
Leu Tyr Ala Glu Ile Asp Leu Ala Gln Met Arg Gly Pro Lys Trp Met
        275                 280                 285
Leu Asp Val Ala Gly His Tyr Ala Arg Pro Asp Val Phe Glu Leu Ile
    290                 295                 300
Val His Arg Glu Ala Arg Pro Met Ile Ala Leu Ile Ser
305                 310                 315

<210> 55
<211> 1017
<212> DNA
<213> Unknown

<220>
<223> Obtained from an environmental sample

<400> 55
atgggtatcg aacatccgaa gtacaaggtc gccgtggtgc aggcagctcc cgcctggctc      60
gatctcgacg cgtcgatcga caagtcgatc gcgctgatcg aggaggcggc ccaaaaaggc     120
gccaagctga tcgcattccc cgaggccttc attcccggct atccctggca catctggatg     180
gactcgccgg cctgggcgat cggccgcggc tttgtgcagc gctattttga caattcgctc     240
gcctatgaca gcccgcaggc cgagaagctg cgcgcggcgg tgcgcaaggc aaagctcacc     300
gccgtgctcg ggctgtccga gcgcgacggc ggcagtctct atctggcgca atggttgatc     360
gggcccgatg gcgagaccat cgcaaaacgc cgcaagctgc ggccgacaca tgccgagcgc     420
acggtgtacg gcgagggcga cggcagcgat ctcgcagtcc acaaccgtcc cgatatcggc     480
cgcctcggcg cgctctgctg ctgggagcat ttgcagccac tgtcgaaata cgcgatgtac     540
gcgcagaacg agcaggtgca tgtcgcggcc tggccgagct ttcgctctac cgatcccttt     600
gcggtggcgc tcggcgccga ggtgaacaac gcggcctcgc gcgtctatgc agtcgaaggc     660
tcctgcttcg tgctggcgcc atgcgccacc gtctcgcagg ccatgatcga cgagctctgc     720
gaccgaccgg acaagcatac gctgctgcat gtcggcggcg gttttgccgc gatctatggt     780
cctgacggca gccagatcgg cgacaagctc gcgcccgacc aggaagggct gttgatcgcg     840
gagatcgacc ttggggccat tggcgtcgcc aagaacgcgg ccgatcccgc cgggcattat     900
tcgcggcccg acgtgacgcg gctcctgctc aacaagaaac cgtacaagcg cgtcgagcag     960
ttctcgccac cggccgaggc ggtcgagccc acagatatcg cagcggcggc aagctga       1017

<210> 56
<211> 338
<212> PRT
<213> Unknown

<220>
<223> Obtained from an environmental sample

<400> 56
Met Gly Ile Glu His Pro Lys Tyr Lys Val Ala Val Val Gln Ala Ala
1               5                   10                  15
Pro Ala Trp Leu Asp Leu Asp Ala Ser Ile Asp Lys Ser Ile Ala Leu
            20                  25                  30
Ile Glu Glu Ala Ala Gln Lys Gly Ala Lys Leu Ile Ala Phe Pro Glu
        35                  40                  45
Ala Phe Ile Pro Gly Tyr Pro Trp His Ile Trp Met Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Lys Leu Arg Ala Ala Val Arg Lys
                85                  90                  95
Ala Lys Leu Thr Ala Val Leu Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asn Arg Pro Asp Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Val Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Thr Leu Leu His Val Gly Gly Gly Phe Ala
                245                 250                 255
Ala Ile Tyr Gly Pro Asp Gly Ser Gln Ile Gly Asp Lys Leu Ala Pro
            260                 265                 270
Asp Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Lys Pro Tyr Lys Arg Val Glu Gln
305                 310                 315                 320
Phe Ser Pro Pro Ala Glu Ala Val Glu Pro Thr Asp Ile Ala Ala Ala
                325                 330                 335
Ala Ser

<210> 57
<211> 1014
<212> DNA
<213> Unknown

<220>
<223> Obtained from an environmental sample

<400> 57
gtgaaagaag caatcaaagt agcctgtgtg caagcagctc cagtctttct cgacctggac      60
gccacagtgg acaagaccgt cgccctgatt gaggaggcag cccgtaacgg cgcacgccta     120

atcgcctttc cagagacctg gattccaggc tacccatggt tcctttggct ggactcacca 180
gcctggggga tgcaattcgt gcgccgatac cacgagaact cactggtcct cgacagccct 240
caggccaagc gcatcagtga ggccgcccag cgcgccggta tatacgtcgc gctagggtac 300
agcgaacgcg tgagcggaac cctctacatg gggcagtggc tcattgacga taagggcgaa 360
acagctgggc tgcgccgaaa gctgaaacca acccatgtag agcgaaccct cttcggtgaa 420
ggcgacggat catccctttc cactttcgac acaccgttgg gggtgctggg cggactctgc 480
tgttgggaac acttacaacc tctttcgaaa tatgcgctct acgcacagaa cgaggaaata 540
cacttcgccg cctggcctag cttcagcatc taccgtcaag cgacagaagt ccttggacca 600
gaagtaaatg tcgcagcttc tcggatctac gccgtggaag ggcagtgttt tgttctcgct 660
tcctgcgcgc tcgtctcgcc agagatgatc gaaatgctct gcactgacga aagcaagcac 720
agccttcttc aggccggcgg cgggtactcc cgcattatcg gtcccgatgg cagcgaccta 780
gcgcgcccct tgggcgaaaa cgaggaaggt attctctatg ccactctgga ccctgccgct 840
cgaatctatg caaagaccgc agctgatcca gccgggcact actccagacc agacgtcact 900
cggctgctga tcaatcgcag tgccaatcag ccagtcgtag aggttggacg ggaaatacct 960
gcatcggccc aaggctttga agttgaggcg gcccccgggt acggaggcga ttga 1014

<210> 58
<211> 337
<212> PRT
<213> Unknown

<220>
<223> Obtained from an environmental sample

<400> 58
Val Lys Glu Ala Ile Lys Val Ala Cys Val Gln Ala Ala Pro Val Phe
1               5                   10                  15
Leu Asp Leu Asp Ala Thr Val Asp Lys Thr Val Ala Leu Ile Glu Glu
            20                  25                  30
Ala Ala Arg Asn Gly Ala Arg Leu Ile Ala Phe Pro Glu Thr Trp Ile
        35                  40                  45
Pro Gly Tyr Pro Trp Phe Leu Trp Leu Asp Ser Pro Ala Trp Gly Met
    50                  55                  60
Gln Phe Val Arg Arg Tyr His Glu Asn Ser Leu Val Leu Asp Ser Pro
65                  70                  75                  80
Gln Ala Lys Arg Ile Ser Glu Ala Ala Gln Arg Ala Gly Ile Tyr Val
                85                  90                  95
Ala Leu Gly Tyr Ser Glu Arg Val Ser Gly Thr Leu Tyr Met Gly Gln
            100                 105                 110
Trp Leu Ile Asp Asp Lys Gly Glu Thr Ala Gly Leu Arg Arg Lys Leu
        115                 120                 125
Lys Pro Thr His Val Glu Arg Thr Leu Phe Gly Glu Gly Asp Gly Ser
    130                 135                 140
Ser Leu Ser Thr Phe Asp Thr Pro Leu Gly Val Leu Gly Gly Leu Cys
145                 150                 155                 160
Cys Trp Glu His Leu Gln Pro Leu Ser Lys Tyr Ala Leu Tyr Ala Gln
                165                 170                 175
Asn Glu Glu Ile His Phe Ala Ala Trp Pro Ser Phe Ser Ile Tyr Arg
            180                 185                 190
Gln Ala Thr Glu Val Leu Gly Pro Glu Val Asn Val Ala Ala Ser Arg
        195                 200                 205
Ile Tyr Ala Val Glu Gly Gln Cys Phe Val Leu Ala Ser Cys Ala Leu
    210                 215                 220
Val Ser Pro Glu Met Ile Glu Met Leu Cys Thr Asp Glu Ser Lys His
225                 230                 235                 240
Ser Leu Leu Gln Ala Gly Gly Gly Tyr Ser Arg Ile Ile Gly Pro Asp
                245                 250                 255

Gly Ser Asp Leu Ala Arg Pro Leu Gly Glu Asn Glu Glu Gly Ile Leu
            260                 265                 270
Tyr Ala Thr Leu Asp Pro Ala Ala Arg Ile Tyr Ala Lys Thr Ala Ala
        275                 280                 285
Asp Pro Ala Gly His Tyr Ser Arg Pro Asp Val Thr Arg Leu Leu Ile
    290                 295                 300
Asn Arg Ser Ala Asn Gln Pro Val Val Glu Val Gly Arg Glu Ile Pro
305                 310                 315                 320
Ala Ser Ala Gln Gly Phe Glu Val Glu Ala Ala Pro Gly Tyr Gly Gly
                325                 330                 335
Asp

<210> 59
<211> 987
<212> DNA
<213> Unknown

<220>
<223> Obtained from an environmental sample

<400> 59
atgcgagata ggaatttcaa actggcggcc attcaggcgg agccggtttt ctttaatcgc 60
cgggcctcga cggaaaaggc ctgcagattg atcaaagaag cgggcgcgat gggcgccgat 120
atcgcgggat tcagcgagac ctggcttccc gggtatccct tttttatctg gggcgcaagc 180
gccgatccat ccctgctctg gaaggcttct gcggaatacc tggccaatgc cgttcaaata 240
cccggtcccg agacggatca attatgcgag gcggcgaaaa aggccggcat cgatgtggcg 300
atcggagtgg ttgaactcga cgagtttacg aagggaacgg cttactgcac gctgctcttc 360
atcggcaaag aagggaagat cctgggaaag caccgcaaac tcaagccgac gcaccgggag 420
cgcacggtat ggggagaggg cgatgcgacg ggactcagtg tccatgagcg tccttacggg 480
cggatcagcg gcctgaactg ctgggagcat aatatggtcc tgcccggcta tgtcctgatg 540
tctcagggca cgcacattca tatcgcggcc tggccgggtt cggaagggaa agcacctccc 600
gcgccgtctc cgatgtggga gcgccagctt ctgctctccc gcgctttcgc ttcgcaatcc 660
gccgcatacg tgattctggt cggaggactc ctgaacccgc agaatattcc ggcgccctac 720
gatgaacttg ccgtcaagta ccggggagac agtttcatca tcgatccgcg cggggagatc 780
atcgccgggc cggccaaggg ggaaaccatt ctcatcgccg aaggctcgat ggaacaggtc 840
ctcgcggcaa agtccgcctt cgatgtcgcg ggacattatt cccgccccga cgtctttcaa 900
ctctgcgtca accgcaaacc gtaccggcgt gtaagggaaa cttcggagca ggaccaaccc 960
gcttctgaaa gagaatcgga atcgtaa 987

<210> 60
<211> 328
<212> PRT
<213> Unknown

<220>
<223> Obtained from an environmental sample

<400> 60
Met Arg Asp Arg Asn Phe Lys Leu Ala Ala Ile Gln Ala Glu Pro Val
1               5                   10                  15
Phe Phe Asn Arg Arg Ala Ser Thr Glu Lys Ala Cys Arg Leu Ile Lys
            20                  25                  30
Glu Ala Gly Ala Met Gly Ala Asp Ile Ala Gly Phe Ser Glu Thr Trp
        35                  40                  45
Leu Pro Gly Tyr Pro Phe Phe Ile Trp Gly Ala Ser Ala Asp Pro Ser
    50                  55                  60

Leu Leu Trp Lys Ala Ser Ala Glu Tyr Leu Ala Asn Ala Val Gln Ile
65                  70                  75                  80
Pro Gly Pro Glu Thr Asp Gln Leu Cys Glu Ala Ala Lys Lys Ala Gly
                85                  90                  95
Ile Asp Val Ala Ile Gly Val Val Glu Leu Asp Glu Phe Thr Lys Gly
            100                 105                 110
Thr Ala Tyr Cys Thr Leu Leu Phe Ile Gly Lys Glu Gly Lys Ile Leu
        115                 120                 125
Gly Lys His Arg Lys Leu Lys Pro Thr His Arg Glu Arg Thr Val Trp
    130                 135                 140
Gly Glu Gly Asp Ala Thr Gly Leu Ser Val His Glu Arg Pro Tyr Gly
145                 150                 155                 160
Arg Ile Ser Gly Leu Asn Cys Trp Glu His Asn Met Val Leu Pro Gly
                165                 170                 175
Tyr Val Leu Met Ser Gln Gly Thr His Ile His Ile Ala Ala Trp Pro
            180                 185                 190
Gly Ser Glu Gly Lys Ala Pro Pro Ala Pro Ser Pro Met Trp Glu Arg
        195                 200                 205
Gln Leu Leu Leu Ser Arg Ala Phe Ala Ser Gln Ser Ala Ala Tyr Val
    210                 215                 220
Ile Leu Val Gly Gly Leu Leu Asn Pro Gln Asn Ile Pro Ala Pro Tyr
225                 230                 235                 240
Asp Glu Leu Ala Val Lys Tyr Arg Gly Asp Ser Phe Ile Ile Asp Pro
                245                 250                 255
Arg Gly Glu Ile Ile Ala Gly Pro Ala Lys Gly Glu Thr Ile Leu Ile
            260                 265                 270
Ala Glu Gly Ser Met Glu Gln Val Leu Ala Ala Lys Ser Ala Phe Asp
        275                 280                 285
Val Ala Gly His Tyr Ser Arg Pro Asp Val Phe Gln Leu Cys Val Asn
    290                 295                 300
Arg Lys Pro Tyr Arg Arg Val Arg Glu Thr Ser Glu Gln Asp Gln Pro
305                 310                 315                 320
Ala Ser Glu Arg Glu Ser Glu Ser
                325

<210> 61
<211> 966
<212> DNA
<213> Unknown

<220>
<223> Obtained from an environmental sample

<400> 61
atgactcgat cttacccgaa tgacacactc acggttgggc ttgcgcaaat tgctccagtc      60
tggttggatc gtacagggac aatttcaaag atattagctc aagtccatgc ggcaaatgaa     120
gcgggctgtc atcttgtcgc gtttggcgaa ggtctcctcc ccggatatcc gttttggatt     180
gagcgaacaa atggtgcagt cttcaactcg cccacgcaga aagaaattca cgcgcattat     240
ctggatcagg ctgtccagat cgaagcaggt catcttgagg cgctttgcga agcagccaag     300
gaatatgaga tcgcaattgt cctgggatgc attgaacgtc cgcaagatcg tggagggcac     360
agtctgtatg caagccttgt atatattgat tcagacggca tcatccaatc tgtgcatcga     420
aagttaatgc caacatatga agaacggctc acctggtcgc caggtgacgg acatggatta     480
cgggtgcaca aattaggtgc ctttacggtt ggcggcctca actgttggga aaactggatg     540
cctttggcac gcgcggccat gtatggtcaa ggcgaggatt tgcatattgc catttggccc     600
ggcggctccc acaatacgca agacattaca cgctttattg cactagaatc gcgttcctat     660
gttttatctg tgtcaggttt aatgcgctca ggcgatttcc caaaagagac cccacatctt     720
gcatccatcc tggctaaagg tgaggatatt cttgccaacg gtggttcatg tatcgccggt     780

cctgacggca aatggatcgt tgagccgctt gtaggagaag agaagttaat tgttgcaacg 840
attgatcatt gtcgtgtgcg cgaagagcgt caaaattttg atccttcagg acattacagc 900
aggccagatg tattgcaact gaaaataaac aggcaacgcc agagtacaat ctcgtttgga 960
gagtaa 966

<210> 62
<211> 321
<212> PRT
<213> Unknown

<220>
<223> Obtained from an environmental sample

<400> 62
Met Thr Arg Ser Tyr Pro Asn Asp Thr Leu Thr Val Gly Leu Ala Gln
1               5                   10                  15
Ile Ala Pro Val Trp Leu Asp Arg Thr Gly Thr Ile Ser Lys Ile Leu
            20                  25                  30
Ala Gln Val His Ala Ala Asn Glu Ala Gly Cys His Leu Val Ala Phe
        35                  40                  45
Gly Glu Gly Leu Leu Pro Gly Tyr Pro Phe Trp Ile Glu Arg Thr Asn
    50                  55                  60
Gly Ala Val Phe Asn Ser Pro Thr Gln Lys Glu Ile His Ala His Tyr
65                  70                  75                  80
Leu Asp Gln Ala Val Gln Ile Glu Ala Gly His Leu Glu Ala Leu Cys
                85                  90                  95
Glu Ala Ala Lys Glu Tyr Glu Ile Ala Ile Val Leu Gly Cys Ile Glu
            100                 105                 110
Arg Pro Gln Asp Arg Gly Gly His Ser Leu Tyr Ala Ser Leu Val Tyr
        115                 120                 125
Ile Asp Ser Asp Gly Ile Ile Gln Ser Val His Arg Lys Leu Met Pro
    130                 135                 140
Thr Tyr Glu Glu Arg Leu Thr Trp Ser Pro Gly Asp Gly His Gly Leu
145                 150                 155                 160
Arg Val His Lys Leu Gly Ala Phe Thr Val Gly Gly Leu Asn Cys Trp
                165                 170                 175
Glu Asn Trp Met Pro Leu Ala Arg Ala Ala Met Tyr Gly Gln Gly Glu
            180                 185                 190
Asp Leu His Ile Ala Ile Trp Pro Gly Gly Ser His Asn Thr Gln Asp
        195                 200                 205
Ile Thr Arg Phe Ile Ala Leu Glu Ser Arg Ser Tyr Val Leu Ser Val
    210                 215                 220
Ser Gly Leu Met Arg Ser Gly Asp Phe Pro Lys Glu Thr Pro His Leu
225                 230                 235                 240
Ala Ser Ile Leu Ala Lys Gly Glu Asp Ile Leu Ala Asn Gly Gly Ser
                245                 250                 255
Cys Ile Ala Gly Pro Asp Gly Lys Trp Ile Val Glu Pro Leu Val Gly
            260                 265                 270
Glu Glu Lys Leu Ile Val Ala Thr Ile Asp His Cys Arg Val Arg Glu
        275                 280                 285
Glu Arg Gln Asn Phe Asp Pro Ser Gly His Tyr Ser Arg Pro Asp Val
    290                 295                 300
Leu Gln Leu Lys Ile Asn Arg Gln Arg Gln Ser Thr Ile Ser Phe Gly
305                 310                 315                 320
Glu

<210> 63
<211> 978
<212> DNA
<213> Unknown

<220>
<223> Obtained from an environmental sample

<400> 63
atgcaagata gagtaccgat tgtacgagct gcggctatcc aggctgaacc catagtcctt 60
gattgtgacg cgaccgtgga aaaagcctgc cgattgatcg gtgaagcagc agaaaatggt 120
gcaaacctga tcgtgtttcc cgaagccttc attcccgttt atcccaatgc ggcgatctgg 180
ggtcgaggtc tggccacttt tggcggacag cgccagaaat acgtatggac gcgactatgg 240
aacaattcgg tggaaatccc tggtccggcc accgacaggc tggcaaaggc agcacacgag 300
gctcgagcca ccgttgtcat gggattgaat gagcgcgcgg tcgataacaa cacgctttac 360
aacaccctgc tatttattgg gccagacggt cgcttgctgg gcaagcaccg taagctcatg 420
cccaccaatc acgaacggat gatctggggt atgggagatg ggagcaccct gcgggttttt 480
gatacaccct gtggaaaagt aggcggtctc atctgctggg aaaactacat gcctctggcg 540
cgttatgcac tctatggaca gggcgaacaa atccatgtcg cgccgactgc gcacgatggt 600
gagatcactc tggtcaatgc acgcaatacc gcctatgagg gacgcttatt cgtcatctcc 660
gtgtgcatga tccttcgcaa gtccagcttt ccccatgatt ttgagctggg cgaggaattg 720
gcggaggcag atgacttcat aaaatcaggc ggcagcgcga tcgttggccc agatggcgag 780
gtgctggcgg gtccattgtg gaatgaagag aatatactgt atgccgatct tgacttgaat 840
cgaattgtgg atgagagacg agtatttgat gtgacgggcc attattcacg tccagatgtt 900
ctacgactgc actttaatgc ttcccctcag aaaactattg aaagatatga gcaacctctc 960
gatccgtctg agggttaa 978

<210> 64
<211> 325
<212> PRT
<213> Unknown

<220>
<223> Obtained from an environmental sample

<400> 64
Met Gln Asp Arg Val Pro Ile Val Arg Ala Ala Ala Ile Gln Ala Glu
1               5                   10                  15
Pro Ile Val Leu Asp Cys Asp Ala Thr Val Glu Lys Ala Cys Arg Leu
            20                  25                  30
Ile Gly Glu Ala Ala Glu Asn Gly Ala Asn Leu Ile Val Phe Pro Glu
        35                  40                  45
Ala Phe Ile Pro Val Tyr Pro Asn Ala Ala Ile Trp Gly Arg Gly Leu
    50                  55                  60
Ala Thr Phe Gly Gly Gln Arg Gln Lys Tyr Val Trp Thr Arg Leu Trp
65                  70                  75                  80
Asn Asn Ser Val Glu Ile Pro Gly Pro Ala Thr Asp Arg Leu Ala Lys
                85                  90                  95
Ala Ala His Glu Ala Arg Ala Thr Val Val Met Gly Leu Asn Glu Arg
            100                 105                 110
Ala Val Asp Asn Asn Thr Leu Tyr Asn Thr Leu Leu Phe Ile Gly Pro
        115                 120                 125
Asp Gly Arg Leu Leu Gly Lys His Arg Lys Leu Met Pro Thr Asn His
    130                 135                 140
Glu Arg Met Ile Trp Gly Met Gly Asp Gly Ser Thr Leu Arg Val Phe
145                 150                 155                 160
Asp Thr Pro Cys Gly Lys Val Gly Gly Leu Ile Cys Trp Glu Asn Tyr

                    165                     170                     175
Met Pro Leu Ala Arg Tyr Ala Leu Tyr Gly Gln Gly Glu Gln Ile His
                180                     185                     190
Val Ala Pro Thr Ala His Asp Gly Glu Ile Thr Leu Val Asn Ala Arg
        195                     200                     205
Asn Thr Ala Tyr Glu Gly Arg Leu Phe Val Ile Ser Val Cys Met Ile
    210                     215                     220
Leu Arg Lys Ser Ser Phe Pro His Asp Phe Glu Leu Gly Glu Glu Leu
225                     230                     235                     240
Ala Glu Ala Asp Asp Phe Ile Lys Ser Gly Gly Ser Ala Ile Val Gly
                245                     250                     255
Pro Asp Gly Glu Val Leu Ala Gly Pro Leu Trp Asn Glu Glu Asn Ile
            260                     265                     270
Leu Tyr Ala Asp Leu Asp Leu Asn Arg Ile Val Asp Glu Arg Arg Val
        275                     280                     285
Phe Asp Val Thr Gly His Tyr Ser Arg Pro Asp Val Leu Arg Leu His
    290                     295                     300
Phe Asn Ala Ser Pro Gln Lys Thr Ile Glu Arg Tyr Glu Gln Pro Leu
305                     310                     315                     320
Asp Pro Ser Glu Gly
                325

<210> 65
<211> 1002
<212> DNA
<213> Unknown

<220>
<223> Obtained from an environmental sample

<400> 65
atgccgaccc ccacttcaaa attcaaaatc ggcgccgtgc aggcatcgcc ggtttttctg      60
gaccgggaag ccactgcgca aaaagcctgc aaattgattg ccgaagcggg agggcagggc     120
gcgcggctga tcgttttccc ggagtctttc attcccacct atcctgattg ggtctgggcg     180
gtcccgccgg gaagaggaaa agtgttaagc gaactttacg ccgagctgct ggccaatgcc     240
gtggaagtcc ccgggccggt caccgatcag ctgggtgaag cagcccaaaa aacgggcgcc     300
tatgtcgtca tgggcgtcac ggaaaaggac accgacgcaa gcggcgcgag cctttacaac     360
acgctcctct atttcaaccc cgcgggggac ctcctgggaa aacaccggaa gcttgttcct     420
accggcgggg agcggctggt ctgggcgcag ggcgacggca gcaccctgga agtgtacgac     480
actcccctgg gaaaaatcgg aggcctcatc tgctgggaaa actacatgcc cctcgcccgg     540
tacacgatgt atgcctgggg gacccagatt tatatcgcgg ccacatggga ccagggggag     600
acgtggcttg ccaccctgcg gcatatcgct aaggaaggac gggtgtacgt catcggctgc     660
tgcatcgcgc tgcggcggga cgacatcccc gaccggctgg aatacaagaa gaagttctac     720
tcggggtcgc gggaatggat caatatgggg gacagcgcca tcgtgaaccc ggaaggcgaa     780
ttcattgccg gccccgtgcg gatgaaggag gagatcctgt atgccgaggt ggaccccctc     840
ctgatggcgg gatcgaaatg gatgctcgac gtcgcggggc attacgcgcg ccccgacgtc     900
tttgaactca tcgtccaccg ccagccccac ccgatgatcc gggtaatcga gaaagaggga     960
ggggccggaa gaaccgggga cgagaagaag gaaaatgagt ga                       1002

<210> 66
<211> 333
<212> PRT
<213> Unknown

<220>
<223> Obtained from an environmental sample

<400> 66
Met Pro Thr Pro Thr Ser Lys Phe Lys Ile Gly Ala Val Gln Ala Ser
1               5                   10                  15
Pro Val Phe Leu Asp Arg Glu Ala Thr Ala Gln Lys Ala Cys Lys Leu
            20                  25                  30
Ile Ala Glu Ala Gly Gly Gln Gly Ala Arg Leu Ile Val Phe Pro Glu
        35                  40                  45
Ser Phe Ile Pro Thr Tyr Pro Asp Trp Val Trp Ala Val Pro Pro Gly
    50                  55                  60
Arg Gly Lys Val Leu Ser Glu Leu Tyr Ala Glu Leu Leu Ala Asn Ala
65                  70                  75                  80
Val Glu Val Pro Gly Pro Val Thr Asp Gln Leu Gly Glu Ala Ala Gln
                85                  90                  95
Lys Thr Gly Ala Tyr Val Val Met Gly Val Thr Glu Lys Asp Thr Asp
            100                 105                 110
Ala Ser Gly Ala Ser Leu Tyr Asn Thr Leu Leu Tyr Phe Asn Pro Ala
        115                 120                 125
Gly Asp Leu Leu Gly Lys His Arg Lys Leu Val Pro Thr Gly Gly Glu
    130                 135                 140
Arg Leu Val Trp Ala Gln Gly Asp Gly Ser Thr Leu Glu Val Tyr Asp
145                 150                 155                 160
Thr Pro Leu Gly Lys Ile Gly Gly Leu Ile Cys Trp Glu Asn Tyr Met
                165                 170                 175
Pro Leu Ala Arg Tyr Thr Met Tyr Ala Trp Gly Thr Gln Ile Tyr Ile
            180                 185                 190
Ala Ala Thr Trp Asp Gln Gly Glu Thr Trp Leu Ala Thr Leu Arg His
        195                 200                 205
Ile Ala Lys Glu Gly Arg Val Tyr Val Ile Gly Cys Cys Ile Ala Leu
    210                 215                 220
Arg Arg Asp Asp Ile Pro Asp Arg Leu Glu Tyr Lys Lys Lys Phe Tyr
225                 230                 235                 240
Ser Gly Ser Arg Glu Trp Ile Asn Met Gly Asp Ser Ala Ile Val Asn
                245                 250                 255
Pro Glu Gly Glu Phe Ile Ala Gly Pro Val Arg Met Lys Glu Glu Ile
            260                 265                 270
Leu Tyr Ala Glu Val Asp Pro Leu Leu Met Ala Gly Ser Lys Trp Met
        275                 280                 285
Leu Asp Val Ala Gly His Tyr Ala Arg Pro Asp Val Phe Glu Leu Ile
    290                 295                 300
Val His Arg Gln Pro His Pro Met Ile Arg Val Ile Glu Lys Glu Gly
305                 310                 315                 320
Gly Ala Gly Arg Thr Gly Asp Glu Lys Lys Glu Asn Glu
                325                 330

<210> 67
<211> 936
<212> DNA
<213> Unknown

<220>
<223> Obtained from an environmental sample

<400> 67
atgccccgtg tggcggtggt ccagcgcccg ccggtgtttc tcgaccgcgc cgcgaccctc      60
gagaacgccg tggcttcgct cgccgaggcc gcgtcgaacg gggctcgcct cgcggtcttt      120
ccggaagccc tggttcccgg ctatccggcg tggatgtggc ggctgcggcc cgggcccgac      180
atggcgctca ccgagcggat tcacgcgcgc ttgcgggcga actcggtgag cctcgccgcc      240

gacgagctcg cgccgctgcg cgaggcggcc cggcgccacg agctcaccgt agtgtgcggc 300
ctgcacgagc gcgacgaggc gctcggcggc ggcacgctct ataacaccgt cgtcacgatc 360
ggcgccgacg gcgcggtgct caaccgccac cggaagctga tgcccaccaa ccccgagcgc 420
atggtctggg gctgcggcga tgccagcggg ctcaggacgg tccccaccca gtgcgggcgc 480
gtcggcgccc tgatctgctg ggaaagctac atgccgcttg cacgctacgc gctgtacgcc 540
cagggaatcg acctctacgt cacgccgacc tacgacagcg gcgagcgggc ggttgcgacc 600
atgcagcaca ttgcccgcga aggcggctgc tgggtggtga gctgcggctc ggcgtttcag 660
gcgcgcgacg tcccggacgc gtttccgggg aagagcgagc ttttccgcga caacgacgag 720
tggatcaacc cgggcgactc ggtcgtggtc gcgccgggcg gcaaggtcgt cgccgggccg 780
ctgcacaaag aacgcgcgat cctgtacgcc gagatcgacc tcgagcgggt cggcgtggcg 840
cgccgcagcc tggacgtggt cggccattat gcgcggcccg acctcttcga cctgcacgtg 900
aacgcccgcc cgcaaagcgt ggttgaattg cgctga 936

<210> 68
<211> 311
<212> PRT
<213> Unknown

<220>
<223> Obtained from an environmental sample

<400> 68
Met Pro Arg Val Ala Val Val Gln Arg Pro Pro Val Phe Leu Asp Arg
1               5                   10                  15
Ala Ala Thr Leu Glu Asn Ala Val Ala Ser Leu Ala Glu Ala Ala Ser
            20                  25                  30
Asn Gly Ala Arg Leu Ala Val Phe Pro Glu Ala Leu Val Pro Gly Tyr
        35                  40                  45
Pro Ala Trp Met Trp Arg Leu Arg Pro Gly Pro Asp Met Ala Leu Thr
    50                  55                  60
Glu Arg Ile His Ala Arg Leu Arg Ala Asn Ser Val Ser Leu Ala Ala
65                  70                  75                  80
Asp Glu Leu Ala Pro Leu Arg Glu Ala Ala Arg Arg His Glu Leu Thr
                85                  90                  95
Val Val Cys Gly Leu His Glu Arg Asp Glu Ala Leu Gly Gly Gly Thr
            100                 105                 110
Leu Tyr Asn Thr Val Val Thr Ile Gly Ala Asp Gly Ala Val Leu Asn
        115                 120                 125
Arg His Arg Lys Leu Met Pro Thr Asn Pro Glu Arg Met Val Trp Gly
    130                 135                 140
Cys Gly Asp Ala Ser Gly Leu Arg Thr Val Pro Thr Gln Cys Gly Arg
145                 150                 155                 160
Val Gly Ala Leu Ile Cys Trp Glu Ser Tyr Met Pro Leu Ala Arg Tyr
                165                 170                 175
Ala Leu Tyr Ala Gln Gly Ile Asp Leu Tyr Val Thr Pro Thr Tyr Asp
            180                 185                 190
Ser Gly Glu Arg Ala Val Ala Thr Met Gln His Ile Ala Arg Glu Gly
        195                 200                 205
Gly Cys Trp Val Val Ser Cys Gly Ser Ala Phe Gln Ala Arg Asp Val
    210                 215                 220
Pro Asp Ala Phe Pro Gly Lys Ser Glu Leu Phe Arg Asp Asn Asp Glu
225                 230                 235                 240
Trp Ile Asn Pro Gly Asp Ser Val Val Val Ala Pro Gly Gly Lys Val
                245                 250                 255
Val Ala Gly Pro Leu His Lys Glu Arg Ala Ile Leu Tyr Ala Glu Ile
            260                 265                 270
Asp Leu Glu Arg Val Gly Val Ala Arg Arg Ser Leu Asp Val Val Gly

275                 280                 285
His Tyr Ala Arg Pro Asp Leu Phe Asp Leu His Val Asn Ala Arg Pro
    290                 295                 300
Gln Ser Val Val Glu Leu Arg
305                 310

<210> 69
<211> 939
<212> DNA
<213> Unknown

<220>
<223> Obtained from an environmental sample

<400> 69
gtgaccgagt ttcggacggt gcgggtcgca gcggtgcagg cgacgccggt gaccctcgac      60
gccgatgcct cggtcgagaa ggcgatcggg ctgatcggcg aggcggtggc cggtggagcg     120
cagctcgtcg tgctgcccga ggccttcgtg tcgctctacc cgtcgaacgc gtgggcgcga     180
gcggccgccg gattcggcgg cttcgacgag ctctgggagc ggatgtgggc cagctcgctc     240
gacgtcccgg gcccgctggt cgaccggctg gtcgatgcgt gccgcaggca tgacgtggta     300
tgcgtgatcg gcgtgaacga gcgcgaaagc gaaaggccgg gtcgctttta caacacgatg     360
ctgaccctcg gcccgtcggg cctcctgcac cggcaccgca agctcatgcc gacgcaccac     420
gagcggctgt tccatgggat cggcgacggt caagacctcg gcgttgtgga gaccgacgcg     480
ggacggatcg ggggactgat ctgctgggag aaccgaatgc cgctcgcgcg ctacgcggtc     540
taccagggtg gaccgcagat ctgggtcgcg ccgacggccg atgactccga cggctggctc     600
gcgagcatgc gccacatcgc gatcgagtcg ggcgcgttcg tcgtgtcggt gccgcagttc     660
atcccggcgt ccgcgttccc cgacgatttc cccgtcgagc taccgccggg caaggaggtg     720
ttcggccgcg gcggtgcggc gatcgtcgag ccgacctggg gcgaggtaat cgccgggccg     780
ctctacgatc gggaggggat cgtgttcgcc gactgtgacc tgcgacgcgg cttgcatgcc     840
aagcgctggt tcgactccgt cggccattac agccgcgcgg aggtgctcga tggcggcgtc     900
gagcgcgtcc cggcgccggt ggacggcgaa tcgccgtga                            939

<210> 70
<211> 312
<212> PRT
<213> Unknown

<220>
<223> Obtained from an environmental sample

<400> 70
Val Thr Glu Phe Arg Thr Val Arg Val Ala Ala Val Gln Ala Thr Pro
1               5                   10                  15
Val Thr Leu Asp Ala Asp Ala Ser Val Glu Lys Ala Ile Gly Leu Ile
            20                  25                  30
Gly Glu Ala Val Ala Gly Gly Ala Gln Leu Val Val Leu Pro Glu Ala
        35                  40                  45
Phe Val Ser Leu Tyr Pro Ser Asn Ala Trp Ala Arg Ala Ala Ala Gly
    50                  55                  60
Phe Gly Gly Phe Asp Glu Leu Trp Glu Arg Met Trp Ala Ser Ser Leu
65                  70                  75                  80
Asp Val Pro Gly Pro Leu Val Asp Arg Leu Val Asp Ala Cys Arg Arg
                85                  90                  95
His Asp Val Val Cys Val Ile Gly Val Asn Glu Arg Glu Ser Glu Arg
            100                 105                 110
Pro Gly Ser Leu Tyr Asn Thr Met Leu Thr Leu Gly Pro Ser Gly Leu
        115                 120                 125

Leu His Arg His Arg Lys Leu Met Pro Thr His His Glu Arg Leu Phe
    130                 135                 140
His Gly Ile Gly Asp Gly Gln Asp Leu Gly Val Val Glu Thr Asp Ala
145                 150                 155                 160
Gly Arg Ile Gly Gly Leu Ile Cys Trp Glu Asn Arg Met Pro Leu Ala
                165                 170                 175
Arg Tyr Ala Val Tyr Gln Gly Gly Pro Gln Ile Trp Val Ala Pro Thr
            180                 185                 190
Ala Asp Asp Ser Asp Gly Trp Leu Ala Ser Met Arg His Ile Ala Ile
        195                 200                 205
Glu Ser Gly Ala Phe Val Val Ser Val Pro Gln Phe Ile Pro Ala Ser
    210                 215                 220
Ala Phe Pro Asp Asp Phe Pro Val Glu Leu Pro Pro Gly Lys Glu Val
225                 230                 235                 240
Phe Gly Arg Gly Gly Ala Ala Ile Val Glu Pro Thr Trp Gly Glu Val
                245                 250                 255
Ile Ala Gly Pro Leu Tyr Asp Arg Glu Gly Ile Val Phe Ala Asp Cys
            260                 265                 270
Asp Leu Arg Arg Gly Leu His Ala Lys Arg Trp Phe Asp Ser Val Gly
        275                 280                 285
His Tyr Ser Arg Ala Glu Val Leu Asp Gly Gly Val Glu Arg Val Pro
    290                 295                 300
Ala Pro Val Asp Gly Glu Ser Pro
305                 310

<210> 71
<211> 966
<212> DNA
<213> Unknown

<220>
<223> Obtained from an environmental sample

<400> 71
atgccaaacg agaacaccaa cgccacattc aaagttgccg ctgtgcaggc ttcgcctgtg      60
tttcttgatc gtgccgcaac aatcgacaag gcttgcgatt tgatcgccgc tgctggcggt     120
gaaggggcac gcttgattgt ctttccagaa gcattcatcc cgtcttatcc tgattgggta     180
tgggcaattc cttcgggtga agagggcgta ctcaatgagt tgtacgcaga tctgctatcc     240
aactcggtca cgattcccag tgactcgacg gacaaactgt gcagagcagc caggcttgct     300
aatgcctacg tggtgatggg tatgagcgaa cgcaatgctg aggcaagcgg cgcgagcatg     360
tataacacgc tattgtatat tgatgcacag ggggagattc tgggcaagca tcggaagttg     420
gtgccaacgg gcggcgagcg gctagtctgg gcgcagggcg atggcagtac actgcaggtc     480
tatgatactc ccttagggaa actcggtggc ttaatttgct gggagaatta tatgccactg     540
gcccgctata ccatgtatgc ctggggcaca caaatctatg tcgcggcaac gtgggatcgg     600
ggtcagccct ggctctctac tttacgccac attgccaaag aaggcagggt gtatgtgatt     660
ggttgttgta tcgcgatgcg taaagacgat atcccagacc attatacaat gaaacagaag     720
ttttactcag atgcagatga gtggattaat attggcgata gtgcgattgt taatcccgaa     780
gggcaattta tcgctggacc ggtgcgcaag caggaagaga ttctctatgc ggagattgat     840
ccgcgcatgg tccaagggcc gaagtggatg ctcgacgtgg cgggacatta tgccaggccg     900
gatgtgttcg aactgattgt ccacacggat attcgaagga tgatcaaatc ggaaaagaat     960
tcataa                                                                966

<210> 72
<211> 321
<212> PRT
<213> Unknown

<220>
<223> Obtained from an environmental sample

<400> 72
Met Pro Asn Glu Asn Thr Asn Ala Thr Phe Lys Val Ala Ala Val Gln
1               5                   10                  15
Ala Ser Pro Val Phe Leu Asp Arg Ala Ala Thr Ile Asp Lys Ala Cys
            20                  25                  30
Asp Leu Ile Ala Ala Ala Gly Gly Glu Gly Ala Arg Leu Ile Val Phe
        35                  40                  45
Pro Glu Ala Phe Ile Pro Ser Tyr Pro Asp Trp Val Trp Ala Ile Pro
    50                  55                  60
Ser Gly Glu Glu Gly Val Leu Asn Glu Leu Tyr Ala Asp Leu Leu Ser
65                  70                  75                  80
Asn Ser Val Thr Ile Pro Ser Asp Ser Thr Asp Lys Leu Cys Arg Ala
                85                  90                  95
Ala Arg Leu Ala Asn Ala Tyr Val Val Met Gly Met Ser Glu Arg Asn
            100                 105                 110
Ala Glu Ala Ser Gly Ala Ser Met Tyr Asn Thr Leu Leu Tyr Ile Asp
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Thr Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Asp His Tyr Thr Met Lys Gln Lys
225                 230                 235                 240
Phe Tyr Ser Asp Ala Asp Glu Trp Ile Asn Ile Gly Asp Ser Ala Ile
                245                 250                 255
Val Asn Pro Glu Gly Gln Phe Ile Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Glu
    290                 295                 300
Leu Ile Val His Thr Asp Ile Arg Arg Met Ile Lys Ser Glu Lys Asn
305                 310                 315                 320
Ser

<210> 73
<211> 1035
<212> DNA
<213> Unknown

<220>
<223> Obtained from an environmental sample

<400> 73
   atgacagcaa tagactcaac gtttaaagtc gccgccgttc aggctgcgcc ggtcttcctc      60
   aatcgcgacg caaccgtgga gaaggcgtgc cggctgatca agtccgcggc agagggaggc 120
   gcgcgtctga tcgttttccc ggaagcgttc ataccggcct acccggactg ggtgtggacg 180
   gtccctgccg gtgagcaagg cctgctcaac gacctctacg gccaactcgt cgaccagtcc 240
   gtgacgattc ccagcgacat caccaccgag ttatgtaacg cggcacgggc agcaaacgcc 300
   tatgtcgtga ttggtgtcaa cgagcgcaac gcggaggcaa gcaatggaag cctctacaac 360
   tcgctcctct acatcgacgc aaacggcaaa attctcggta agcaccgcaa gctcgttccc 420
   acaggcggag aacggctcgt gtggcgcagg ggcgatggca gcacgctcga agcctacgac 480
   acggagctgg gcaaactcgg cggtctcatt tgctgggaga actatatgcc gctggcacgc 540
   tacgcgatgt acgcatgggg agtgcagctc tatgtcgccg cgacctggga ccgtggcggc 600
   ccctggactg ccacgctgcg tcatgtcgcc aaggaaggtc agatgtacgt catcgggtgc 660
   tgccaggccc tgcacaagga tgacctgccg gagctagacg ggctgaagga gaagtactac 720
   gccaacgcac gagagtggat caatgttggc gacagcgcta ttgtcggccc ggacggacaa 780
   ttccttgtcg agcccgtccg aatgcgggaa gacatcctct acgccgaggt ggacactcgc 840
   aacttccgcg gcccgaagtg gatgttcgac gcggctggac actacgcgcg tcccgacatt 900
   ttccaactca cagtgaaccg cgagcagcgg ccgatggtcc gcgtcgtcgg tgacagcagt 960
   gaccagaagg agcggccgct cccggacgac ggacggctct ggtacgccta cagcaccaat 1020
   cagcaccacg actga 1035

<210> 74
<211> 344
<212> PRT
<213> Unknown

<220>
<223> Obtained from an environmental sample

<400> 74
Met Thr Ala Ile Asp Ser Thr Phe Lys Val Ala Ala Val Gln Ala Ala
1               5                   10                  15
Pro Val Phe Leu Asn Arg Asp Ala Thr Val Glu Lys Ala Cys Arg Leu
            20                  25                  30
Ile Lys Ser Ala Ala Glu Gly Gly Ala Arg Leu Ile Val Phe Pro Glu
        35                  40                  45
Ala Phe Ile Pro Ala Tyr Pro Asp Trp Val Trp Thr Val Pro Ala Gly
    50                  55                  60
Glu Gln Gly Leu Leu Asn Asp Leu Tyr Gly Gln Leu Val Asp Gln Ser
65                  70                  75                  80
Val Thr Ile Pro Ser Asp Ile Thr Thr Glu Leu Cys Asn Ala Ala Arg
                85                  90                  95
Ala Ala Asn Ala Tyr Val Val Ile Gly Val Asn Glu Arg Asn Ala Glu
            100                 105                 110
Ala Ser Asn Gly Ser Leu Tyr Asn Ser Leu Leu Tyr Ile Asp Ala Asn
        115                 120                 125
Gly Lys Ile Leu Gly Lys His Arg Lys Leu Val Pro Thr Gly Gly Glu
    130                 135                 140
Arg Leu Val Trp Ala Gln Gly Asp Gly Ser Thr Leu Glu Ala Tyr Asp
145                 150                 155                 160
Thr Glu Leu Gly Lys Leu Gly Gly Leu Ile Cys Trp Glu Asn Tyr Met
                165                 170                 175
Pro Leu Ala Arg Tyr Ala Met Tyr Ala Trp Gly Val Gln Leu Tyr Val
            180                 185                 190
Ala Ala Thr Trp Asp Arg Gly Gly Pro Trp Thr Ala Thr Leu Arg His
        195                 200                 205
Val Ala Lys Glu Gly Gln Met Tyr Val Ile Gly Cys Cys Gln Ala Leu
    210                 215                 220
His Lys Asp Asp Leu Pro Glu Leu Asp Gly Leu Lys Glu Lys Tyr Tyr
225                 230                 235                 240

Ala Asn Ala Arg Glu Trp Ile Asn Val Gly Asp Ser Ala Ile Val Gly
                245                 250                 255
Pro Asp Gly Gln Phe Leu Val Glu Pro Val Arg Met Arg Glu Asp Ile
            260                 265                 270
Leu Tyr Ala Glu Val Asp Thr Arg Asn Phe Arg Gly Pro Lys Trp Met
        275                 280                 285
Phe Asp Ala Ala Gly His Tyr Ala Arg Pro Asp Ile Phe Gln Leu Thr
    290                 295                 300
Val Asn Arg Glu Gln Arg Pro Met Val Arg Val Val Gly Asp Ser Ser
305                 310                 315                 320
Asp Gln Lys Glu Arg Pro Leu Pro Asp Asp Gly Arg Leu Trp Tyr Ala
                325                 330                 335
Tyr Ser Thr Asn Gln His His Asp
            340

<210> 75
<211> 1125
<212> DNA
<213> Unknown

<220>
<223> Obtained from an environmental sample

<400> 75
atgagcacca ttgttaaagc cgctgcggtt caaatcagcc cagtcctcta cagccgcgag 60
gggacagtcg caaaagttgt gcggaagatc cacgaacttg gccaaaaggg ggtgcggttc 120
gccacgttcc cggagaccgt ggttccctac tatccatatt tttccgccgt ccagaccccc 180
attcaactat tgtccggaac cgagtacctg aagttgctcg accaaggcgt gaccgtgccg 240
tccacgacta ccgacgcaat cggggaggct gcccggaacg ccggcatggt tgtatctatc 300
ggcgtgaatg agcgtgacgg cgggaccctg tacaacgcgc agttgctctt cgatgcggat 360
gggaccttga ttcagcgtcg ccgcaagatc actcctacgc attacgagcg catgatctgg 420
ggccagggag atggttcggg tttgcgggcc gtcaagagcc aggttggtcg tattggccaa 480
cttgcatgct ttgagcacaa caacccactg gcgcgttacg cgatgatggc cgatggcgag 540
caaatccatt cggccatgta tccaggttcc gcgttcggcg aggggttcgc ggaaaagatg 600
gaaatcaata tccgccagca tgcgttggag tccgggtgct tcgttgtgaa tgcaacggcc 660
tggcttgacg ccagccagca ggcacaaatc atgaatgaca cgggttgcca aatcggtccg 720
atctcgggcg gttgctttac cacgatcgta acacccgacg gcacgtttct gggcgaacct 780
ctccggtcgg gtgagggcga ggtcatcgcc gatctcgatt tcaagctgat cgacaaacgc 840
aagatgttga tggactcgcg cggccactac agtcgcccgg aattgctcag tctgctgatc 900
gaccgcaccc ccaccgcgca cattcatgag cgaggtgcgc cgcagacgtc aggcgctgtg 960
caagaggcga cgaaagtggg ttcacacgcg ccgctcctgc gtgacggaca atgggatcag 1020
ctcaatgcgg gagcgggccg acatacaggg aatggagaag cacagataga aatcatggcc 1080
gcggcccact cgggcacccg tggaattgaa gcgaagggag cctaa 1125

<210> 76
<211> 374
<212> PRT
<213> Unknown

<220>
<223> Obtained from an environmental sample

<400> 76
Met Ser Thr Ile Val Lys Ala Ala Ala Val Gln Ile Ser Pro Val Leu
1               5                   10                  15
Tyr Ser Arg Glu Gly Thr Val Ala Lys Val Val Arg Lys Ile His Glu
            20                  25                  30

Leu Gly Gln Lys Gly Val Arg Phe Ala Thr Phe Pro Glu Thr Val Val
        35                  40                  45
Pro Tyr Tyr Pro Tyr Phe Ser Ala Val Gln Thr Pro Ile Gln Leu Leu
    50                  55                  60
Ser Gly Thr Glu Tyr Leu Lys Leu Leu Asp Gln Gly Val Thr Val Pro
65                  70                  75                  80
Ser Thr Thr Thr Asp Ala Ile Gly Glu Ala Ala Arg Asn Ala Gly Met
                85                  90                  95
Val Val Ser Ile Gly Val Asn Glu Arg Asp Gly Gly Thr Leu Tyr Asn
            100                 105                 110
Ala Gln Leu Leu Phe Asp Ala Asp Gly Thr Leu Ile Gln Arg Arg Arg
        115                 120                 125
Lys Ile Thr Pro Thr His Tyr Glu Arg Met Ile Trp Gly Gln Gly Asp
    130                 135                 140
Gly Ser Gly Leu Arg Ala Val Lys Ser Gln Val Gly Arg Ile Gly Gln
145                 150                 155                 160
Leu Ala Cys Phe Glu His Asn Asn Pro Leu Ala Arg Tyr Ala Met Met
                165                 170                 175
Ala Asp Gly Glu Gln Ile His Ser Ala Met Tyr Pro Gly Ser Ala Phe
            180                 185                 190
Gly Glu Gly Phe Ala Glu Lys Met Glu Ile Asn Ile Arg Gln His Ala
        195                 200                 205
Leu Glu Ser Gly Cys Phe Val Val Asn Ala Thr Ala Trp Leu Asp Ala
    210                 215                 220
Ser Gln Gln Ala Gln Ile Met Asn Asp Thr Gly Cys Gln Ile Gly Pro
225                 230                 235                 240
Ile Ser Gly Gly Cys Phe Thr Thr Ile Val Thr Pro Asp Gly Thr Phe
                245                 250                 255
Leu Gly Glu Pro Leu Arg Ser Gly Glu Gly Glu Val Ile Ala Asp Leu
            260                 265                 270
Asp Phe Lys Leu Ile Asp Lys Arg Lys Met Leu Met Asp Ser Arg Gly
        275                 280                 285
His Tyr Ser Arg Pro Glu Leu Leu Ser Leu Leu Ile Asp Arg Thr Pro
    290                 295                 300
Thr Ala His Ile His Glu Arg Gly Ala Pro Gln Thr Ser Gly Ala Val
305                 310                 315                 320
Gln Glu Ala Thr Lys Val Gly Ser His Ala Pro Leu Leu Arg Asp Gly
                325                 330                 335
Gln Trp Asp Gln Leu Asn Ala Gly Ala Gly Arg His Thr Gly Asn Gly
            340                 345                 350
Glu Ala Gln Ile Glu Ile Met Ala Ala Ala His Ser Gly Thr Arg Gly
        355                 360                 365
Ile Glu Ala Lys Gly Ala
    370

<210> 77
<211> 1056
<212> DNA
<213> Unknown

<220>
<223> Obtained from an environmental sample

<400> 77
atgccaaccc ccagcgatca tttcaaaatc gccgctgttc aggcctcgcc cgtgtttctg      60
gaccgggagg ccactgtgga aaaggcctgc cggttgatcg ccgaagccgc aaagcagggc     120
gtccgcctca tcgtctttcc ggaatcgttc atcccgacct acccggactg ggtatgggcc     180

gttcccccgg gaagggaaag aatcctgaac cagctgtatt ctgaattcct ggccaatgcc 240
gtcgatgttc ccggcgcggc gaccgaacaa cttgcccagg ctgcacgaat ggccggcgcc 300
tatgtgatta tgggcgtcac cgaaagagac acctcggcca gcggggccag cctctacaac 360
accctgctct acttcagccc cgaaggcatc ctaatgggca aacaccggaa gctggttccc 420
acggggggcg aacggctggt ctgggcctac ggagacggca gcacgctgga ggtctacgac 480
actccgctgg gaaagatcgg cgggctgatc tgctgggaga actacatgcc cctggcccgg 540
tacacgatgt acgcctgggg cacccagatt tacatcgccg ccacctggga ccgcggggaa 600
ccgtggctct ccaccctgcg gcatatcgca aaggaaggaa gggtctacgt catcgggtgc 660
tgcatcgccc tgcgccaggg ggatatcccg gaccggttcg agtacaaggg aaaattttat 720
tccgggtccc gggagtggat caatgagggc gacagcgcca tcgtgaaccc ggacggggaa 780
ttcatcgccg ggccggtgcg gacgaaggag gagatcctgt atgccgagat agacccccgg 840
cagatgcggg gccccaagtg gatgctcgat gtggccggtc attacgcccg gccggatatc 900
ttcgagctca tcgtccaccg gaatccccac ccgatgatca aaatcgccga agacaggggc 960
acggggatcg cctcaagttt gattcgcccc cgccctaacc ttcccccatc aaggggaggg 1020
aaatcggcaa gaagcaaacg caagcccaaa aaatga 1056

<210> 78
<211> 351
<212> PRT
<213> Unknown

<220>
<223> Obtained from an environmental sample

<400> 78
Met Pro Thr Pro Ser Asp His Phe Lys Ile Ala Ala Val Gln Ala Ser
1               5                   10                  15
Pro Val Phe Leu Asp Arg Glu Ala Thr Val Glu Lys Ala Cys Arg Leu
            20                  25                  30
Ile Ala Glu Ala Ala Lys Gln Gly Val Arg Leu Ile Val Phe Pro Glu
        35                  40                  45
Ser Phe Ile Pro Thr Tyr Pro Asp Trp Val Trp Ala Val Pro Pro Gly
    50                  55                  60
Arg Glu Arg Ile Leu Asn Gln Leu Tyr Ser Glu Phe Leu Ala Asn Ala
65                  70                  75                  80
Val Asp Val Pro Gly Ala Ala Thr Glu Gln Leu Ala Gln Ala Ala Arg
                85                  90                  95
Met Ala Gly Ala Tyr Val Ile Met Gly Val Thr Glu Arg Asp Thr Ser
            100                 105                 110
Ala Ser Gly Ala Ser Leu Tyr Asn Thr Leu Leu Tyr Phe Ser Pro Glu
        115                 120                 125
Gly Ile Leu Met Gly Lys His Arg Lys Leu Val Pro Thr Gly Gly Glu
    130                 135                 140
Arg Leu Val Trp Ala Tyr Gly Asp Gly Ser Thr Leu Glu Val Tyr Asp
145                 150                 155                 160
Thr Pro Leu Gly Lys Ile Gly Gly Leu Ile Cys Trp Glu Asn Tyr Met
                165                 170                 175
Pro Leu Ala Arg Tyr Thr Met Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Lys Glu Gly Arg Val Tyr Val Ile Gly Cys Cys Ile Ala Leu
    210                 215                 220
Arg Gln Gly Asp Ile Pro Asp Arg Phe Glu Tyr Lys Gly Lys Phe Tyr
225                 230                 235                 240
Ser Gly Ser Arg Glu Trp Ile Asn Glu Gly Asp Ser Ala Ile Val Asn
                245                 250                 255

Pro Asp Gly Glu Phe Ile Ala Gly Pro Val Arg Thr Lys Glu Glu Ile
            260                 265                 270
Leu Tyr Ala Glu Ile Asp Pro Arg Gln Met Arg Gly Pro Lys Trp Met
        275                 280                 285
Leu Asp Val Ala Gly His Tyr Ala Arg Pro Asp Ile Phe Glu Leu Ile
    290                 295                 300
Val His Arg Asn Pro His Pro Met Ile Lys Ile Ala Glu Asp Arg Gly
305                 310                 315                 320
Thr Gly Ile Ala Ser Ser Leu Ile Arg Pro Arg Pro Asn Leu Pro Pro
                325                 330                 335
Ser Arg Gly Arg Lys Ser Ala Arg Ser Lys Arg Lys Pro Lys Lys
340 345 . 350

<210> 79
<211> 990
<212> DNA
<213> Unknown

<220>
<223> Obtained from an environmental sample

<400> 79
atgacgaaga aaagcggccg cgattcgttt cgggtcgctg cggtccaggc ctcgtccgtc 60
tacctggatc gggaacggag catcgagaaa gcgtgccggc tgatcgacga cgcgggacga 120
aacgacgccg acctcgtcgt gttccccgaa gccttcgtgc ccggataccc actgtgggtg 180
tggctcgttc cgccggggcg caccgcagac ttgcgctccg cttatgcgac gctccacgcc 240
aacgcgatca gcattccgga cgactccacc gatcggctgt gcgccgccgc aaaagacgcc 300
ggcgtcgccg tcgcgatcgg cgtcaacgaa cgcaacaccg aagcgagcgg catgagcctg 360
ttcaacacgc tgctctatat cggagcggac ggccggattc tcggaaaaca ccggaagctg 420
gtaccgaccg gcggcgaacg gctcgtctgg gcatctggcg acggcagcga cctcgaggtc 480
tactcgctgc cgttcggtcg cgtaagcgga ctgatctgct gggagcacta catgccgctc 540
gcccggtatg cgctcgccgc gtggggcgaa caggtgcacg tcgctccaac ctgggatcgt 600
ggcgagccgt ggctgtccac gctaaggcac atcgcgaagg aaggccgcgt tctcgtcgtc 660
ggctgctgtc aagccgtgcg caaggacgac atccctgaca cgctcgcgtt caagtccaaa 720
tacctcgcag acgtggacgg ctggatcaac ccaggtggca gcgtcatcat caatcctgac 780
ggcaaggtcg tcgcgggacc ggcgatggaa accgaaactg tactgtacgc ggaccttcgc 840
accgagcagc tcgtcggacc gcgctggcag ctcgacgtcg gcggacatta cgctcgtccg 900
gacgtcttcg agctcgtcgt ccatcggcat ccgaagccgt tgattcggac agcgaccggt 960
gtcaggcgcc gcaagcgtgc acgtcgctaa 990

<210> 80
<211> 329
<212> PRT
<213> Unknown

<220>
<223> Obtained from an environmental sample

<400> 80
Met Thr Lys Lys Ser Gly Arg Asp Ser Phe Arg Val Ala Ala Val Gln
1               5                   10                  15
Ala Ser Ser Val Tyr Leu Asp Arg Glu Arg Ser Ile Glu Lys Ala Cys
            20                  25                  30
Arg Leu Ile Asp Asp Ala Gly Arg Asn Asp Ala Asp Leu Val Val Phe
        35                  40                  45
Pro Glu Ala Phe Val Pro Gly Tyr Pro Leu Trp Val Trp Leu Val Pro
    50                  55                  60

Pro Gly Arg Thr Ala Asp Leu Arg Ser Ala Tyr Ala Thr Leu His Ala
65                  70                  75                  80
Asn Ala Ile Ser Ile Pro Asp Asp Ser Thr Asp Arg Leu Cys Ala Ala
                85                  90                  95
Ala Lys Asp Ala Gly Val Ala Val Ala Ile Gly Val Asn Glu Arg Asn
            100                 105                 110
Thr Glu Ala Ser Gly Met Ser Leu Phe Asn Thr Leu Leu Tyr Ile Gly
        115                 120                 125
Ala Asp Gly Arg Ile Leu Gly Lys His Arg Lys Leu Val Pro Thr Gly
    130                 135                 140
Gly Glu Arg Leu Val Trp Ala Ser Gly Asp Gly Ser Asp Leu Glu Val
145                 150                 155                 160
Tyr Ser Leu Pro Phe Gly Arg Val Ser Gly Leu Ile Cys Trp Glu His
                165                 170                 175
Tyr Met Pro Leu Ala Arg Tyr Ala Leu Ala Ala Trp Gly Glu Gln Val
            180                 185                 190
His Val Ala Pro Thr Trp Asp Arg Gly Glu Pro Trp Leu Ser Thr Leu
        195                 200                 205
Arg His Ile Ala Lys Glu Gly Arg Val Leu Val Val Gly Cys Cys Gln
    210                 215                 220
Ala Val Arg Lys Asp Asp Ile Pro Asp Thr Leu Ala Phe Lys Ser Lys
225                 230                 235                 240
Tyr Leu Ala Asp Val Asp Gly Trp Ile Asn Pro Gly Gly Ser Val Ile
                245                 250                 255
Ile Asn Pro Asp Gly Lys Val Val Ala Gly Pro Ala Met Glu Thr Glu
            260                 265                 270
Thr Val Leu Tyr Ala Asp Leu Arg Thr Glu Gln Leu Val Gly Pro Arg
        275                 280                 285
Trp Gln Leu Asp Val Gly Gly His Tyr Ala Arg Pro Asp Val Phe Glu
    290                 295                 300
Leu Val Val His Arg His Pro Lys Pro Leu Ile Arg Thr Ala Thr Gly
305                 310                 315                 320
Val Arg Arg Arg Lys Arg Ala Arg Arg
                325

<210> 81
<211> 993
<212> DNA
<213> Unknown

<220>
<223> Obtained from an environmental sample

<400> 81
atgaaagtcg tcaaagccgc cgctgtccag ttcagcccgg tgctctatag ccgcgaagcg      60
accgtcgcca aggtcgtccg gaaaatccac gagctcggtc agaaaggcgt gcagttcgcc     120
acctttcctg aaacggtcgt gccttattac ccttacttcg cggccgtcca gacgggcatc     180
gagctcttgt cgggcaccga acatctgcgc ctgctcgaac aggccgtgac tgtgccctcc     240
gctgcgaccg atgcaatcgg cgaagccgcg cgacaggccg gcatggtcgt gtccatcggc     300
gtcaatgagc gtgacggcgg cacgctttac aacacgcaac tgctcttcga tgccgacggt     360
acgctgatcc agcgccgccg caagatcacg ccgacccatt tcgaacgcat gatctggggg     420
cagggagatg gctcgggctt gcgtgcagtc gacagcgcag tcggccgcat cggccagctc     480
gcatgcttcg agcacaacaa cccgcttgca cgttacgcaa tgatcgccga cggcgagcag     540
atccattcag cgatgtaccc tggctcggcc tttggcgagg gcttcgccca gcgtatggag     600
atcaacatcc gccagcatgc gctcgagtcc gccgctttcg tcgtcaacgc aacggcgtgg     660
cttgacgccg accagcaggc gcaaatcatg aaggacaccg gttgtggaat cggtccgatc     720
tcgggcggct gcttcaccac gatcgtttct cctgacggta tgctgatggc cgatccgctt     780

cgctcgggcg aaggcgaagt gattgtcgat ctcgacttca cgcagatcga ccgccgcaag 840
atgctgatgg actcggccgg ccactacaac cgccctgaac tgctgagtct gatgatcgac 900
cgtacgccgg ctgcgcatgt tcacgaacgc gcttcgcgcc cgatgaccgt cgacgaccag 960
agttccggcg atctgcgcac ccaggttgca tga 993

<210> 82
<211> 330
<212> PRT
<213> Unknown

<220>
<223> Obtained from an environmental sample

<400> 82
Met Lys Val Val Lys Ala Ala Ala Val Gln Phe Ser Pro Val Leu Tyr
1               5                   10                  15
Ser Arg Glu Ala Thr Val Ala Lys Val Val Arg Lys Ile His Glu Leu
            20                  25                  30
Gly Gln Lys Gly Val Gln Phe Ala Thr Phe Pro Glu Thr Val Val Pro
        35                  40                  45
Tyr Tyr Pro Tyr Phe Ala Ala Val Gln Thr Gly Ile Glu Leu Leu Ser
    50                  55                  60
Gly Thr Glu His Leu Arg Leu Leu Glu Gln Ala Val Thr Val Pro Ser
65                  70                  75                  80
Ala Ala Thr Asp Ala Ile Gly Glu Ala Ala Arg Gln Ala Gly Met Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Ala Val Gly Arg Ile Gly Gln Leu
145                 150                 155                 160
Ala Cys Phe Glu His Asn Asn Pro Leu Ala Arg Tyr Ala Met Ile Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Ala Ala Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Ser Gly Gly Cys Phe Thr Thr Ile Val Ser Pro Asp Gly Met Leu Met
                245                 250                 255
Ala Asp Pro Leu Arg Ser Gly Glu Gly Glu Val Ile Val Asp Leu Asp
            260                 265                 270
Phe Thr Gln Ile Asp Arg Arg Lys Met Leu Met Asp Ser Ala Gly His
        275                 280                 285
Tyr Asn Arg Pro Glu Leu Leu Ser Leu Met Ile Asp Arg Thr Pro Ala
    290                 295                 300
Ala His Val His Glu Arg Ala Ser Arg Pro Met Thr Val Asp Asp Gln
305                 310                 315                 320
Ser Ser Gly Asp Leu Arg Thr Gln Val Ala
                325                 330

<210> 83
<211> 1071
<212> DNA
<213> Unknown

<220>
<223> Obtained from an environmental sample

<400> 83
atgatgagtt cagcccgtgt aataaaactc gccgcagcac agctttcacc tgtgctgccg 60
ggggagtcca caaatagccg cgacggcacc attgccaaag tcgtcgcggc gattgcggag 120
gctgcgcgcg ccggcgcgca gctgatcgtg tttcccgaaa cggtggtgcc gtattacccg 180
tatttctcgt tcattacgcc ggcggtgacg atgggggcgg agcatttgcg cttgtacgat 240
cagtctgtcg tggtgccgag cgccgccact gatactgttg ccgccgctgc aaaaaaacac 300
agcatggtgg tcgtgctcgg tattaacgaa cgcgatcacg gcacgctcta caacgcgcaa 360
ttaattttcg atgcgagcgg cgaattatta ttaaaacgcc gaaaaattac cccgacctat 420
cacgagcgca tggtgtgggg tcagggcgac ggcagcggtt tgaaaaccgt cgacaccgcg 480
atcggccgtg tcggtgcgct cgcctgctgg gaacattaca acccattggc gcgttacagc 540
ctgatggccc agcacgaaga aattcattgc agtcaatttc cggggtcatt ggtcgggcca 600
attttcgccg agcaaatgga agtgacaatg cgccaccacg cgctcgaatc cggttgcttc 660
gtcgttaatg caacggcgtg gttatcggaa gcgcaaattc aatcgatcag cagcgatccc 720
gcgatgcaaa aagcactgcg cggcggttgc tacaccgcaa ttatttcgcc cgaaggcaaa 780
catctgtgcg agccgctacg cgaaggtgaa ggtttgattt ttgccgaagc cgatatggcg 840
ctcattacca aacgcaaacg catgatggat tcggttggtc attacgcgcg acccgaattg 900
ctgtcgctgt taatcgacca tcgcgccacc acaccattgc atagcgtcac cgcgagtgat 960
gccgccgccg taaaaaatac tcggagttcc gctcatgaat cagccgatag tgaaaccatc 1020
cgcgagtcag ttaataacgg aactccaatc gcacggcttg cgcctagttg a 1071

<210> 84
<211> 356
<212> PRT
<213> Unknown

<220>
<223> Obtained from an environmental sample

<400> 84
Met Met Ser Ser Ala Arg Val Ile Lys Leu Ala Ala Ala Gln Leu Ser
1               5                   10                  15
Pro Val Leu Pro Gly Glu Ser Thr Asn Ser Arg Asp Gly Thr Ile Ala
            20                  25                  30
Lys Val Val Ala Ala Ile Ala Glu Ala Ala Arg Ala Gly Ala Gln Leu
        35                  40                  45
Ile Val Phe Pro Glu Thr Val Val Pro Tyr Tyr Pro Tyr Phe Ser Phe
    50                  55                  60
Ile Thr Pro Ala Val Thr Met Gly Ala Glu His Leu Arg Leu Tyr Asp
65                  70                  75                  80
Gln Ser Val Val Val Pro Ser Ala Ala Thr Asp Thr Val Ala Ala Ala
                85                  90                  95
Ala Lys Lys His Ser Met Val Val Val Leu Gly Ile Asn Glu Arg Asp
            100                 105                 110
His Gly Thr Leu Tyr Asn Ala Gln Leu Ile Phe Asp Ala Ser Gly Glu
        115                 120                 125
Leu Leu Leu Lys Arg Arg Lys Ile Thr Pro Thr Tyr His Glu Arg Met
    130                 135                 140
Val Trp Gly Gln Gly Asp Gly Ser Gly Leu Lys Thr Val Asp Thr Ala
145                 150                 155                 160

Ile Gly Arg Val Gly Ala Leu Ala Cys Trp Glu His Tyr Asn Pro Leu
                165                 170                 175
Ala Arg Tyr Ser Leu Met Ala Gln His Glu Glu Ile His Cys Ser Gln
            180                 185                 190
Phe Pro Gly Ser Leu Val Gly Pro Ile Phe Ala Glu Gln Met Glu Val
        195                 200                 205
Thr Met Arg His His Ala Leu Glu Ser Gly Cys Phe Val Val Asn Ala
    210                 215                 220
Thr Ala Trp Leu Ser Glu Ala Gln Ile Gln Ser Ile Ser Ser Asp Pro
225                 230                 235                 240
Ala Met Gln Lys Ala Leu Arg Gly Gly Cys Tyr Thr Ala Ile Ile Ser
                245                 250                 255
Pro Glu Gly Lys His Leu Cys Glu Pro Leu Arg Glu Gly Glu Gly Leu
            260                 265                 270
Ile Phe Ala Glu Ala Asp Met Ala Leu Ile Thr Lys Arg Lys Arg Met
        275                 280                 285
Met Asp Ser Val Gly His Tyr Ala Arg Pro Glu Leu Leu Ser Leu Leu
    290                 295                 300
Ile Asp His Arg Ala Thr Thr Pro Leu His Ser Val Thr Ala Ser Asp
305                 310                 315                 320
Ala Ala Ala Val Lys Asn Thr Arg Ser Ser Ala His Glu Ser Ala Asp
                325                 330                 335
Ser Glu Thr Ile Arg Glu Ser Val Asn Asn Gly Thr Pro Ile Ala Arg
            340                 345                 350
Leu Ala Pro Ser
        355

<210> 85
<211> 1014
<212> DNA
<213> Unknown

<220>
<223> Obtained from an environmental sample

<400> 85
atgggtctgg ttcatcagaa atacaaggtt gcggtggttc aggcggcgcc ggtctttctc      60
gacctcgatg cgacggtgga caagacgatc gccctgatcg agcaggccgc agcacagggc     120
gcgaagctga tcgcgtttcc cgagaccttc attcccggat atccgtggca gatctggctt     180
ggggcgcccg cctgggcgat cggccgtggc ttcgtgcagc gctatttcga taactcgttg     240
tcatttgaca gcccgcaggc cgaaaaaatt cgcaaggccg tcaagcgcgc caagctgacc     300
gcggtgatcg gcgtctccga acgcgacggc ggcagcctct atatcggcca atggctgatc     360
ggtcccgacg gcgagaccat tgcgaagcgc cgcaagctgc ggccgaccca tgccgaacgc     420
accgtgttcg gcgagggcga cggcagcgac ctcgccgtcc atgatcgcgc cgacgtggga     480
cggctcggtg caatgtgctg ctgggagcat ctgcagccgc tgtcgaaata cgcgatgtac     540
gcccagaacg agcaggttca cgtcggcgcc tggccgagct tctcattgta cgacccattc     600
gcccatgcgc ttggctggga agtaaacaac gcggcgagca aggtttatgc tgtcgagggc     660
tcatgtttct tcctcggccc gtgcgcggtg gtctcgcagg ccatgatcga cgagctctgc     720
gattcccccg aaaagcacgc cttcctgcac gctggcggcg gccacgcggt aatctatggg     780
ccggacggga gttcgcttgc cgacaaactt ccacccgatc aggagggcat tctgtatgcc     840
gatatcgatc tcggcatgat cggcgtggca aagaacgccg ccgaccccgc aggacactat     900
tccaggccgg acgtcacgcg gctgctgctc aacacttccc gcgccaatcg cgtcgagcat     960
tttcattgcc cgatcgatgc cgaggtcatg agcgaaatca gacttcaggc ctga          1014

<210> 86
<211> 337
<212> PRT

<213> Unknown

<220>
<223> Obtained from an environmental sample

<400> 86
Met Gly Leu Val His Gln Lys Tyr Lys Val Ala Val Val Gln Ala Ala
1               5                   10                  15
Pro Val Phe Leu Asp Leu Asp Ala Thr Val Asp Lys Thr Ile Ala Leu
            20                  25                  30
Ile Glu Gln Ala Ala Ala Gln Gly Ala Lys Leu Ile Ala Phe Pro Glu
        35                  40                  45
Thr Phe Ile Pro Gly Tyr Pro Trp Gln Ile Trp Leu Gly Ala Pro Ala
    50                  55                  60
Trp Ala Ile Gly Arg Gly Phe Val Gln Arg Tyr Phe Asp Asn Ser Leu
65                  70                  75                  80
Ser Phe Asp Ser Pro Gln Ala Glu Lys Ile Arg Lys Ala Val Lys Arg
                85                  90                  95
Ala Lys Leu Thr Ala Val Ile Gly Val Ser Glu Arg Asp Gly Gly Ser
            100                 105                 110
Leu Tyr Ile Gly Gln Trp Leu Ile Gly Pro Asp Gly Glu Thr Ile Ala
        115                 120                 125
Lys Arg Arg Lys Leu Arg Pro Thr His Ala Glu Arg Thr Val Phe Gly
    130                 135                 140
Glu Gly Asp Gly Ser Asp Leu Ala Val His Asp Arg Ala Asp Val Gly
145                 150                 155                 160
Arg Leu Gly Ala Met Cys Cys Trp Glu His Leu Gln Pro Leu Ser Lys
                165                 170                 175
Tyr Ala Met Tyr Ala Gln Asn Glu Gln Val His Val Gly Ala Trp Pro
            180                 185                 190
Ser Phe Ser Leu Tyr Asp Pro Phe Ala His Ala Leu Gly Trp Glu Val
        195                 200                 205
Asn Asn Ala Ala Ser Lys Val Tyr Ala Val Glu Gly Ser Cys Phe Phe
    210                 215                 220
Leu Gly Pro Cys Ala Val Val Ser Gln Ala Met Ile Asp Glu Leu Cys
225                 230                 235                 240
Asp Ser Pro Glu Lys His Ala Phe Leu His Ala Gly Gly Gly His Ala
                245                 250                 255
Val Ile Tyr Gly Pro Asp Gly Ser Ser Leu Ala Asp Lys Leu Pro Pro
            260                 265                 270
Asp Gln Glu Gly Ile Leu Tyr Ala Asp Ile Asp Leu Gly Met Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Thr Ser Arg Ala Asn Arg Val Glu His
305                 310                 315                 320
Phe Ser Leu Pro Ile Asp Ala Glu Val Met Ser Glu Ile Arg Leu Gln
                325                 330                 335
Ala

<210> 87
<211> 1062
<212> DNA
<213> Unknown

<220>

<223> Obtained from an environmental sample

<400> 87
atggcggaat cgaagctgaa ggtcgccgca attcaagttg cgcccgtgtt catggatcgc      60
gatgccacga tcgcccgcgc ctgcgagcgg atcgccgaag ccgcccgcgc cggcgcggag     120
ttggtggtct ttcccgaggc attcgtgccc gggtatcccg actggatctg ggtggcgcgg     180
ccaagccaac gcaaactgct caatgatctt tacgcgcacc tcgtctcgca gtcggtcgac     240
gtgccgtcgg cctccgtgga tcgtttgcgc gacgcggctc gcgacggcgg ggtcacggtg     300
gtgatcggcg tcaacgagcg caacaccgaa gcgagcggcg cgagcctcta caacaccgcg     360
ctcgtgatcg gtccactggg gcagctgatc ggccgccacc gcaagcttgt gccgaccggg     420
ccggagcgca tggtgtgggc gcagggcgac ggcagcacgc tcgacgtcta cgacacaccc     480
gtcggcaagc tttcgacgtt gatctgctgg gagaactaca tgccgctcgc gcgctacgcc     540
atggcggcgt ggggcgcgcg catccacgtc gccggcacgt gggaccgcgg cgagccgtgg     600
atctcgacca tgcgtcatgt ggcgacggag ggccgcgtat tcgtgattag ctgttgcatg     660
gcgctgcgca aacgagacat tcccgccgag ctcgagttcg cgatgctcta tcccgacggg     720
cgcgaatgga tcaacgccgg tgattcgctg gtcgtgaatc ccgctggcca gatcatcgct     780
gggccgttgc acgagcagga aggaatcctc tacgccgagc tcgagcgcaa tcagatgacc     840
ggtccgcgtt ggatgttcga cgccgccggc cattacgcgc gaccggacgt cttccaactc     900
acggtaaacc gctccccgcg cccgatgctg cgggaggcgg gggcaaagac gagtgaggca     960
aacacgagag atgccgtacc catggacagc acgccctcga gatcgcggcc ccgcgcggtg    1020
gcgcgaaagg ccgcacgcac cggtcgctcc aagcggcggt ga                       1062

<210> 88
<211> 353
<212> PRT
<213> Unknown

<220>
<223> Obtained from an environmental sample

<400> 88
Met Ala Glu Ser Lys Leu Lys Val Ala Ala Ile Gln Val Ala Pro Val
1               5                   10                  15
Phe Met Asp Arg Asp Ala Thr Ile Ala Arg Ala Cys Glu Arg Ile Ala
            20                  25                  30
Glu Ala Ala Arg Ala Gly Ala Glu Leu Val Val Phe Pro Glu Ala Phe
        35                  40                  45
Val Pro Gly Tyr Pro Asp Trp Ile Trp Val Ala Arg Pro Ser Gln Arg
    50                  55                  60
Lys Leu Leu Asn Asp Leu Tyr Ala His Leu Val Ser Gln Ser Val Asp
65                  70                  75                  80
Val Pro Ser Ala Ser Val Asp Arg Leu Arg Asp Ala Ala Arg Asp Gly
                85                  90                  95
Gly Val Thr Val Val Ile Gly Val Asn Glu Arg Asn Thr Glu Ala Ser
            100                 105                 110
Gly Ala Ser Leu Tyr Asn Thr Ala Leu Val Ile Gly Pro Leu Gly Gln
        115                 120                 125
Leu Ile Gly Arg His Arg Lys Leu Val Pro Thr Gly Pro Glu Arg Met
    130                 135                 140
Val Trp Ala Gln Gly Asp Gly Ser Thr Leu Asp Val Tyr Asp Thr Pro
145                 150                 155                 160
Val Gly Lys Leu Ser Thr Leu Ile Cys Trp Glu Asn Tyr Met Pro Leu
                165                 170                 175
Ala Arg Tyr Ala Met Ala Ala Trp Gly Ala Arg Ile His Val Ala Gly
            180                 185                 190
Thr Trp Asp Arg Gly Glu Pro Trp Ile Ser Thr Met Arg His Val Ala
        195                 200                 205

Thr Glu Gly Arg Val Phe Val Ile Ser Cys Cys Met Ala Leu Arg Lys
    210                 215                 220
Arg Asp Ile Pro Ala Glu Leu Glu Phe Ala Met Leu Tyr Pro Asp Gly
225                 230                 235                 240
Arg Glu Trp Ile Asn Ala Gly Asp Ser Leu Val Val Asn Pro Ala Gly
                245                 250                 255
Gln Ile Ile Ala Gly Pro Leu His Glu Gln Glu Gly Ile Leu Tyr Ala
            260                 265                 270
Glu Leu Glu Arg Asn Gln Met Thr Gly Pro Arg Trp Met Phe Asp Ala
        275                 280                 285
Ala Gly His Tyr Ala Arg Pro Asp Val Phe Gln Leu Thr Val Asn Arg
    290                 295                 300
Ser Pro Arg Pro Met Leu Arg Glu Ala Gly Ala Lys Thr Ser Glu Ala
305                 310                 315                 320
Asn Thr Arg Asp Ala Val Pro Met Asp Ser Thr Pro Ser Arg Ser Arg
                325                 330                 335
Pro Arg Ala Val Ala Arg Lys Ala Ala Arg Thr Gly Arg Ser Lys Arg
            340                 345                 350
Arg

<210> 89
<211> 918
<212> DNA
<213> Unknown

<220>
<223> Obtained from an environmental sample

<400> 89
atgaatacca aagaagtaaa ggtcgcagcc gctcaatttg ccccacattt tctgaatttg      60
agcaaaacgg tggaaaaaac ctgcaacttg atttccgaag caggcaaaaa tggagcaaag     120
ctcattgtat ttccggaagc cttcctctct ggttatcccg attgggtctg gttaattccc     180
aatggaaatt caacaatgct ggatgattta tatcaggaat tggttgagaa cgctgtaaca     240
atccctgatt caacaacaca gaaactctgt caggcagcaa aagatgccgg ggtatatgtc     300
gcagtcggta tccatgaaag aaatgcagaa gcaagtggct tcacactttt caataccctt     360
ctatacatta atgatcaagg cagcatcatt ggaaaacacc gaaaactgat cccaacaggg     420
ggcgaacgcc tggtctgggg gcagggtaat ggggatacgc ttgctgcatt cgatacacac     480
tttggcaaat tgggaggatt gctttgctgg gaaaactaca tgcccctggc tcggcaagct     540
atgtacgcag ttgggactga agtttatgtt gccccaacct gggactccag tgagaattgg     600
ttgctgagta tgcgccatat agccagagag ggcggcatgt ttgtgatcaa tgtttgccag     660
gctgtccgaa aagacgatat tcctgaccgc tatgcattca agcaactcta ttctggtaat     720
tcagaatgga tcaatagcgg caacagttgc atcatcaatc cgcgcggtga aatcattgcc     780
ggaccatcct caaacaggca agaaatactc tacgcagatt tagatctgag tttgattaca     840
aaatctaaac gcatgttcga tgttaccggg cattatgccc ggccggatgt gtttagatat     900
gaaatcaaaa aaagctag                                                   918

<210> 90
<211> 305
<212> PRT
<213> Unknown

<220>
<223> Obtained from an environmental sample

<400> 90
Met Asn Thr Lys Glu Val Lys Val Ala Ala Ala Gln Phe Ala Pro His

1                   5                   10                  15
Phe Leu Asn Leu Ser Lys Thr Val Glu Lys Thr Cys Asn Leu Ile Ser
                20                  25                  30
Glu Ala Gly Lys Asn Gly Ala Lys Leu Ile Val Phe Pro Glu Ala Phe
            35                  40                  45
Leu Ser Gly Tyr Pro Asp Trp Val Trp Leu Ile Pro Asn Gly Asn Ser
    50                  55                  60
Thr Met Leu Asp Asp Leu Tyr Gln Glu Leu Val Glu Asn Ala Val Thr
65                  70                  75                  80
Ile Pro Asp Ser Thr Thr Gln Lys Leu Cys Gln Ala Ala Lys Asp Ala
                85                  90                  95
Gly Val Tyr Val Ala Val Gly Ile His Glu Arg Asn Ala Glu Ala Ser
            100                 105                 110
Gly Phe Thr Leu Phe Asn Thr Leu Leu Tyr Ile Asn Asp Gln Gly Ser
        115                 120                 125
Ile Ile Gly Lys His Arg Lys Leu Ile Pro Thr Gly Gly Glu Arg Leu
    130                 135                 140
Val Trp Gly Gln Gly Asn Gly Asp Thr Leu Ala Ala Phe Asp Thr His
145                 150                 155                 160
Phe Gly Lys Leu Gly Gly Leu Leu Cys Trp Glu Asn Tyr Met Pro Leu
                165                 170                 175
Ala Arg Gln Ala Met Tyr Ala Val Gly Thr Glu Val Tyr Val Ala Pro
            180                 185                 190
Thr Trp Asp Ser Ser Glu Asn Trp Leu Leu Ser Met Arg His Ile Ala
        195                 200                 205
Arg Glu Gly Gly Met Phe Val Ile Asn Val Cys Gln Ala Val Arg Lys
    210                 215                 220
Asp Asp Ile Pro Asp Arg Tyr Ala Phe Lys Gln Leu Tyr Ser Gly Asn
225                 230                 235                 240
Ser Glu Trp Ile Asn Ser Gly Asn Ser Cys Ile Ile Asn Pro Arg Gly
                245                 250                 255
Glu Ile Ile Ala Gly Pro Ser Ser Asn Arg Gln Glu Ile Leu Tyr Ala
            260                 265                 270
Asp Leu Asp Leu Ser Leu Ile Thr Lys Ser Lys Arg Met Phe Asp Val
        275                 280                 285
Thr Gly His Tyr Ala Arg Pro Asp Val Phe Arg Tyr Glu Ile Lys Lys
    290                 295                 300
Ser
305

<210> 91
<211> 939
<212> DNA
<213> Unknown

<220>
<223> Obtained from an environmental sample

<400> 91
atgaccaaaa tcgctgtcat tcaagaacct ccggtctatc tgaatctgag taaatcgatg      60
gacagagcgg tcgacttgat tgccaatgct gcaagcaagg ggtgtgagtt gattgtgttt     120
cccgaagcct ggcttgcagg ttaccccacc ttcgtctggc gtcttgcgcc gggcagcgga     180
atgggaaaaa ctgatgagct ttacgcgcgt ttgctcgcca actcggtcga ccgtagcaaa     240
gaggggctta gaccattgca ggaggccgca aaggagcatg gcgttgtcat tgtgctgggt     300
tatcaagagg tggatggcgc gggaagcagc agcacgatct tcaacagctg tgcgattatt     360
gatgcggacg ggcgactggc caacaatcat cgcaagttga tgcccaccaa tccggagagg     420
atggtttggg gttttggcga cggttcaggc ctgaacgtcg ttgacaccgc ggtgggcagg     480

atcggcacgc tgatttgctg ggaaaactac atgccgttag cgcgctacgc gctgtatgtc 540
caaaacatcg aaatctatgt tgccccgact tgggacagtg gtgccatgtg gcaggcgacc 600
ctgcagcata tcgcgcgcga aggtggctgc tgggtcatcg gatgtgcaac gtcgctggaa 660
gcctctgaca tcccggacga cgttccccat cgggatgagc tattcccgaa caaagacgaa 720
tgggtaaacc ctggcgatgc ggtggtttat aagccatttg gcggcattgt ggccggcccc 780
atgcatcagg aaaaggggct tctcatcgca gagttggacg tcgccgctgt tcagtcgtca 840
cgtcggaagt tcgatgcgag cgggcactac gctcgccccg atgtcttcaa actgcatgtg 900
aatcgcaccg cgatgcggcc agttgatttc acgaattag 939

<210> 92
<211> 312
<212> PRT
<213> Unknown

<220>
<223> Obtained from an environmental sample

<400> 92
Met Thr Lys Ile Ala Val Ile Gln Glu Pro Pro Val Tyr Leu Asn Leu
1               5                   10                  15
Ser Lys Ser Met Asp Arg Ala Val Asp Leu Ile Ala Asn Ala Ala Ser
            20                  25                  30
Lys Gly Cys Glu Leu Ile Val Phe Pro Glu Ala Trp Leu Ala Gly Tyr
        35                  40                  45
Pro Thr Phe Val Trp Arg Leu Ala Pro Gly Ser Gly Met Gly Lys Thr
    50                  55                  60
Asp Glu Leu Tyr Ala Arg Leu Leu Ala Asn Ser Val Asp Arg Ser Lys
65                  70                  75                  80
Glu Gly Leu Arg Pro Leu Gln Glu Ala Ala Lys Glu His Gly Val Val
                85                  90                  95
Ile Val Leu Gly Tyr Gln Glu Val Asp Gly Ala Gly Ser Ser Ser Thr
            100                 105                 110
Ile Phe Asn Ser Cys Ala Ile Ile Asp Ala Asp Gly Arg Leu Ala Asn
        115                 120                 125
Asn His Arg Lys Leu Met Pro Thr Asn Pro Glu Arg Met Val Trp Gly
    130                 135                 140
Phe Gly Asp Gly Ser Gly Leu Asn Val Val Asp Thr Ala Val Gly Arg
145                 150                 155                 160
Ile Gly Thr Leu Ile Cys Trp Glu Asn Tyr Met Pro Leu Ala Arg Tyr
                165                 170                 175
Ala Leu Tyr Val Gln Asn Ile Glu Ile Tyr Val Ala Pro Thr Trp Asp
            180                 185                 190
Ser Gly Ala Met Trp Gln Ala Thr Leu Gln His Ile Ala Arg Glu Gly
        195                 200                 205
Gly Cys Trp Val Ile Gly Cys Ala Thr Ser Leu Glu Ala Ser Asp Ile
    210                 215                 220
Pro Asp Asp Val Pro His Arg Asp Glu Leu Phe Pro Asn Lys Asp Glu
225                 230                 235                 240
Trp Val Asn Pro Gly Asp Ala Val Val Tyr Lys Pro Phe Gly Gly Ile
                245                 250                 255
Val Ala Gly Pro Met His Gln Glu Lys Gly Leu Leu Ile Ala Glu Leu
            260                 265                 270
Asp Val Ala Ala Val Gln Ser Ser Arg Arg Lys Phe Asp Ala Ser Gly
        275                 280                 285
His Tyr Ala Arg Pro Asp Val Phe Lys Leu His Val Asn Arg Thr Ala
    290                 295                 300
Met Arg Pro Val Asp Phe Thr Asn

305                310

<210> 93
<211> 978
<212> DNA
<213> Unknown

<220>
<223> Obtained from an environmental sample

<400> 93
atgcccatca tcaaagccgc tgccgtgcaa atcagcccgg tgctttacag tcgcgaaggc      60
accgtggaca aggtctgtca acagatcatc gacctcggtc ggcaaggcgt gcagttcgcc     120
gtctttccgg aaacggtggt gccttactac ccgtactttt cgtttgtgca gccggccttt     180
gccatgggcg cacagcacct caagttgctg gatcaatcgg tgacagtgcc gtcggccgcc     240
accttggcca tcggtgaagc ttgcaagcaa gcagggatag tggtgtccat cggcgtcaac     300
gaacgcgatg gcggtacgat ctacaacgcg caattactct tcgatgccga cggcagcctg     360
attcagcatc gccgcaaaat caccccgacc tatcacgaac gcatggtctg ggggcaaggc     420
gatggttccg gcctgcgcgc catcgacagt gcagtggggc gcattggctc cctggcctgt     480
tgggagcatt acaacccgct ggctcgttat gccttgatgg ccgatggcga gcagatccac     540
gccgcgatgt ttcccggctc gctggtgggc gacatttttg ccgagcagat cgaagtcacc     600
atccgccatc acgccttgga gtccggctgt ttcgtggtca acgccaccgc ctggctggac     660
gccgatcagc agggccaaat catgcaagac accggttgca gcctcggccc gatctcgggt     720
ggctgcttca ccgccatcgt ttcccctgaa ggcaagttgc tcggtgagcc gctgcgttcc     780
ggcgaagggg tggtgatcgc cgatctcgat ctggcactga tcgataagcg taaacggatg     840
atggattcgg tcgggcatta cagtcgcccg gaactgctca gcctgttgat cgaccgcacg     900
cccacagcgc atgtgcatga acgcagcgcg cacctggtgg ctgtcgctac cgaggagttc     960
gatcatgcaa accaatga                                                   978

<210> 94
<211> 325
<212> PRT
<213> Unknown

<220>
<223> Obtained from an environmental sample

<400> 94
Met Pro Ile Ile Lys Ala Ala Ala Val Gln Ile Ser Pro Val Leu Tyr
1               5                   10                  15
Ser Arg Glu Gly Thr Val Asp Lys Val Cys Gln Gln Ile Ile Asp Leu
            20                  25                  30
Gly Arg Gln Gly Val Gln Phe Ala Val Phe Pro Glu Thr Val Val Pro
        35                  40                  45
Tyr Tyr Pro Tyr Phe Ser Phe Val Gln Pro Ala Phe Ala Met Gly Ala
    50                  55                  60
Gln His Leu Lys Leu Leu Asp Gln Ser Val Thr Val Pro Ser Ala Ala
65                  70                  75                  80
Thr Leu Ala Ile Gly Glu Ala Cys Lys Gln Ala Gly Ile Val Val Ser
                85                  90                  95
Ile Gly Val Asn Glu Arg Asp Gly Gly Thr Ile Tyr Asn Ala Gln Leu
            100                 105                 110
Leu Phe Asp Ala Asp Gly Ser Leu Ile Gln His Arg Arg Lys Ile Thr
        115                 120                 125
Pro Thr Tyr His Glu Arg Met Val Trp Gly Gln Gly Asp Gly Ser Gly
    130                 135                 140
Leu Arg Ala Ile Asp Ser Ala Val Gly Arg Ile Gly Ser Leu Ala Cys

145                 150                 155                 160
Trp Glu His Tyr Asn Pro Leu Ala Arg Tyr Ala Leu Met Ala Asp Gly
                165                 170                 175
Glu Gln Ile His Ala Ala Met Phe Pro Gly Ser Leu Val Gly Asp Ile
            180                 185                 190
Phe Ala Glu Gln Ile Glu Val Thr Ile Arg His His Ala Leu Glu Ser
        195                 200                 205
Gly Cys Phe Val Val Asn Ala Thr Ala Trp Leu Asp Ala Asp Gln Gln
    210                 215                 220
Gly Gln Ile Met Gln Asp Thr Gly Cys Ser Leu Gly Pro Ile Ser Gly
225                 230                 235                 240
Gly Cys Phe Thr Ala Ile Val Ser Pro Glu Gly Lys Leu Leu Gly Glu
                245                 250                 255
Pro Leu Arg Ser Gly Glu Gly Val Val Ile Ala Asp Leu Asp Leu Ala
            260                 265                 270
Leu Ile Asp Lys Arg Lys Arg Met Met Asp Ser Val Gly His Tyr Ser
        275                 280                 285
Arg Pro Glu Leu Leu Ser Leu Leu Ile Asp Arg Thr Pro Thr Ala His
    290                 295                 300
Val His Glu Arg Ser Ala His Leu Val Ala Val Ala Thr Glu Glu Phe
305                 310                 315                 320
Asp His Ala Asn Gln
                325

<210> 95
<211> 966
<212> DNA
<213> Unknown

<220>
<223> Obtained from an environmental sample

<400> 95
atgtccaacg agaataacat tgctacattc aaagttgccg cagtccaggc cacacctgtg      60
tttcttgatc gtgaagcaac catcgacaaa gcttgcgcgt tgattgccac tgctggcagt     120
gaaggagcgc gcctgattgt gtttccagaa gcattcatcc caacttatcc tgaatgggta     180
tggggtattc cctccggtga gcaaggttta ctcaacgaac tctatgcaga gttgctcacc     240
aatgcggtca ccattcccag cgatgcgact gacaggctgt gcgaggctgc gcagcttgcg     300
aatgcctacg tagtgatggg catgagcgaa cggaacgtcg aggcgagtgg cgcaagcctg     360
tataatacgc tgttgtacat aaatgcgcag ggggagattt tagggaaaca tcgaaagctg     420
gtgccaacgg gcggcgaacg cctggtatgg gcgcagggtg atggcagtac gctgcaggtc     480
tacgatactc cattgggaaa actcggtggc ttaatttgct gggaaaatta tatgccgctg     540
gcacggtatg ctatgtatgc ctggggaaca caaatctatg tcgcggcaac gtgggatcgc     600
ggtcaaccct ggctttctac attaaggcat atcgccaaag aaggcagggt atacgtgatt     660
ggttgctgta tcgcgatgcg taaagacgat attccagatc gttacaccat gaagcaaaaa     720
tattatgctg aaatggatga atggatgaat gttggtgaca gtgtgattgt caatcccgag     780
ggcactttta ttgccgggcc tgtgcgcaag caggaagaaa ttctctacgc ggagattgat     840
cctcgcatgg tgcaaggccc gaagtggatg ctcgatgtgg cagggcatta tgcgagaccg     900
gatgtgttcc agttgacggt gcatacggat gtgaggcgga tgatgcgggt ggaagatgat     960
tcataa                                                                966

<210> 96
<211> 321
<212> PRT
<213> Unknown

<220>

<223> Obtained from an environmental sample

<400> 96
Met Ser Asn Glu Asn Asn Ile Ala Thr Phe Lys Val Ala Ala Val Gln
1               5                   10                  15
Ala Thr Pro Val Phe Leu Asp Arg Glu Ala Thr Ile Asp Lys Ala Cys
            20                  25                  30
Ala Leu Ile Ala Thr Ala Gly Ser Glu Gly Ala Arg Leu Ile Val Phe
        35                  40                  45
Pro Glu Ala Phe Ile Pro Thr Tyr Pro Glu Trp Val Trp Gly Ile Pro
    50                  55                  60
Ser Gly Glu Gln Gly Leu Leu Asn Glu Leu Tyr Ala Glu Leu Leu Thr
65                  70                  75                  80
Asn Ala Val Thr Ile Pro Ser Asp Ala Thr Asp Arg Leu Cys Glu Ala
                85                  90                  95
Ala Gln Leu Ala Asn Ala Tyr Val Val Met Gly Met Ser Glu Arg Asn
            100                 105                 110
Val Glu Ala Ser Gly Ala Ser Leu Tyr Asn Thr Leu Leu Tyr Ile Asn
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Ala Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Asp Arg Tyr Thr Met Lys Gln Lys
225                 230                 235                 240
Tyr Tyr Ala Glu Met Asp Glu Trp Met Asn Val Gly Asp Ser Val Ile
                245                 250                 255
Val Asn Pro Glu Gly His Phe Ile Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asp Val Arg Arg Met Met Arg Val Glu Asp Asp
305                 310                 315                 320
Ser


<210> 97
<211> 1017
<212> DNA
<213> Unknown

<220>
<223> Obtained from an environmental sample

<400> 97
atgggcatcg aacatccgaa atacaaggtc gccgtggtgc aagctgcgcc cgcctggctc      60
gacctcgacg cgtcgatcga caagacgatc gggctgatcg aggaggcggc gaagaaaggc     120

gccaagctga tcgctttccc cgaagccttc attcccggct acccttggca catctggctc 180
gactcacccg cctgggcgat cggccgcggt ttcgtgcagc gctatttcga caattcgctc 240
gcctacgaca gcccacaggc ggaaaggctg cgacaggccg tgcggaaggc caagctcacc 300
gccgtgatcg gcctgtccga gcgcgacggc ggcagcctct atctcgcgca gtggctgatc 360
gggcccgacg gtgagaccat cgcaaagcgc cgcaagctgc ggccgaccca tgccgagcgc 420
accgtctatg gcgaaggcga cggcagcgat ctcgccgtcc atgagcgggc cgacatcggc 480
cggctcggcg cgctgtgctg ctgggagcat ctgcagccgc tgtcgaaatt cgccatgtac 540
gcccagaacg agcaggtaca tgtcgcggcc tggccgagct tctcgctcta cgatcccttc 600
gcgcctgcgc tgggcgcgga ggtgaacaac gccgcctccc gcatctatgc ggtggaaggc 660
tcctgcttcg tgctcgcacc gtgcgcgacg gtctcgcagg ccatgatcga cgagctctgc 720
gatcggccgg acaagcacgc gctgctgcat gccggcggcg gcttcgccgc gatctacggg 780
cccgacggca gccagatcgg cgacaagctg ccgcccgagc aggagggcct gctgatcgcc 840
gagatcgatc tgggcgcgat cggcgtcgcc aagaacgcgg ccgatcccgc cgggcattat 900
tcgcggcccg acgtcacgcg gctcctgctc aacaggaagc cgaacaagcg cgtggagcag 960
ttcgcgctgc ccgtcgacac ggtcgagccc gtcgacgtcg cggcggcagc aagctga 1017

<210> 98
<211> 338
<212> PRT
<213> Unknown

<220>
<223> Obtained from an environmental sample

<400> 98
Met Gly Ile Glu His Pro Lys Tyr Lys Val Ala Val Val Gln Ala Ala
1               5                   10                  15
Pro Ala Trp Leu Asp Leu Asp Ala Ser Ile Asp Lys Thr Ile Gly Leu
            20                  25                  30
Ile Glu Glu Ala Ala Lys Lys Gly Ala Lys Leu Ile Ala Phe Pro Glu
        35                  40                  45
Ala Phe Ile Pro Gly Tyr Pro Trp His Ile Trp Leu Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Arg Leu Arg Gln Ala Val Arg Lys
                85                  90                  95
Ala Lys Leu Thr Ala Val Ile Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Glu Arg Ala Asp Ile Gly
145                 150                 155                 160
Arg Leu Gly Ala Leu Cys Cys Trp Glu His Leu Gln Pro Leu Ser Lys
                165                 170                 175
Phe Ala Met Tyr Ala Gln Asn Glu Gln Val His Val Ala Ala Trp Pro
            180                 185                 190
Ser Phe Ser Leu Tyr Asp Pro Phe Ala Pro Ala Leu Gly Ala Glu Val
        195                 200                 205
Asn Asn Ala Ala Ser Arg Ile Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Ala Gly Gly Gly Phe Ala
                245                 250                 255

Ala Ile Tyr Gly Pro Asp Gly Ser Gln Ile Gly Asp Lys Leu Pro Pro
            260                 265                 270
Glu Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Arg Lys Pro Asn Lys Arg Val Glu Gln
305                 310                 315                 320
Phe Ala Leu Pro Val Asp Thr Val Glu Pro Val Asp Val Ala Ala Ala
                325                 330                 335
Ala Ser

<210> 99
<211> 1014
<212> DNA
<213> Unknown

<220>
<223> Obtained from an environmental sample

<400> 99
atgcctgaca agagaatcgt ccgcgccgcc gcggtccaga tagcaccgga cctcgaacgg 60
cccggtggca cgctcgagaa ggtcctcgag acgatcgacg acgccgcacg ccagggcgtg 120
cagctcatcg tcttccccga gaccttcctg ccctactacc cgtacttttc gttcgtgcgg 180
gcgccggtgg catcgggtgc agagcacatg cggctctatg acgaagcggt ggtcgtgccc 240
gggccggtga cgcatgcggt ggccgagcgg gcacggcggc acggcatggt cgtcgtgctc 300
ggcgtgaacg agcgcgatca cggcagctta tacaacgcac aactgatctt cgataccgac 360
ggcgagctgc tgctcaagcg ccgcaagatc acgccgacgt ttcacgaacg gatgatctgg 420
ggcatgggcg acgcagccgg cctgaaggta gcggaaacgc gtatcggccg ggtgggtgca 480
ctcgcttgct gggaacacta caacccgctt gcacgttatg cactgatgac ccagcacgaa 540
gagattcatt gcagccagtt tcccggctcg ctggtcggac ccatcttcgg tgaacagatc 600
gaagtgacca tccggcatca cgcactggaa tccggctgct tcgtgatcaa ttccaccggc 660
tggctgaccg agccgcagat cgagtcgatc acgaaagatc cgggcctgca gaaggcgctt 720
cgcggcggct gcaacacggc gatcatctcg cccgaaggcc agcatctcgc cccgccgctg 780
cgtgagggcg agggcatggt catcgctgac ctggacatgt cgctgatcac caaacgcaaa 840
cgcatgatgg attctgtcgg ccactacgcg cggcccgaac tgctgagcct cgccatcaac 900
gaccggccgg cggtcacgtc ggcacccatg aacagcttct catcttcaac cgggggattg 960
caccttgaac gcgaacgaga ccttgtcggc cgtgagccgg caattgatga ctga 1014

<210> 100
<211> 337
<212> PRT
<213> Unknown

<220>
<223> Obtained from an environmental sample

<400> 100
Met Pro Asp Lys Arg Ile Val Arg Ala Ala Ala Val Gln Ile Ala Pro
1               5                   10                  15
Asp Leu Glu Arg Pro Gly Gly Thr Leu Glu Lys Val Leu Glu Thr Ile
            20                  25                  30
Asp Asp Ala Ala Arg Gln Gly Val Gln Leu Ile Val Phe Pro Glu Thr
        35                  40                  45
Phe Leu Pro Tyr Tyr Pro Tyr Phe Ser Phe Val Arg Ala Pro Val Ala
    50                  55                  60

Ser Gly Ala Glu His Met Arg Leu Tyr Asp Glu Ala Val Val Val Pro
65                  70                  75                  80
Gly Pro Val Thr His Ala Val Ala Glu Arg Ala Arg Arg His Gly Met
                85                  90                  95
Val Val Val Leu Gly Val Asn Glu Arg Asp His Gly Ser Leu Tyr Asn
            100                 105                 110
Ala Gln Leu Ile Phe Asp Thr Asp Gly Glu Leu Leu Leu Lys Arg Arg
        115                 120                 125
Lys Ile Thr Pro Thr Phe His Glu Arg Met Ile Trp Gly Met Gly Asp
    130                 135                 140
Ala Ala Gly Leu Lys Val Ala Glu Thr Arg Ile Gly Arg Val Gly Ala
145                 150                 155                 160
Leu Ala Cys Trp Glu His Tyr Asn Pro Leu Ala Arg Tyr Ala Leu Met
                165                 170                 175
Thr Gln His Glu Glu Ile His Cys Ser Gln Phe Pro Gly Ser Leu Val
            180                 185                 190
Gly Pro Ile Phe Gly Glu Gln Ile Glu Val Thr Ile Arg His His Ala
        195                 200                 205
Leu Glu Ser Gly Cys Phe Val Ile Asn Ser Thr Gly Trp Leu Thr Glu
    210                 215                 220
Pro Gln Ile Glu Ser Ile Thr Lys Asp Pro Gly Leu Gln Lys Ala Leu
225                 230                 235                 240
Arg Gly Gly Cys Asn Thr Ala Ile Ile Ser Pro Glu Gly Gln His Leu
                245                 250                 255
Ala Pro Pro Leu Arg Glu Gly Glu Gly Met Val Ile Ala Asp Leu Asp
            260                 265                 270
Met Ser Leu Ile Thr Lys Arg Lys Arg Met Met Asp Ser Val Gly His
        275                 280                 285
Tyr Ala Arg Pro Glu Leu Leu Ser Leu Ala Ile Asn Asp Arg Pro Ala
    290                 295                 300
Val Thr Ser Ala Pro Met Asn Ser Phe Ser Ser Ser Thr Gly Gly Leu
305                 310                 315                 320
His Leu Glu Arg Glu Arg Asp Leu Val Gly Arg Glu Pro Ala Ile Asp
                325                 330                 335
Asp

<210> 101
<211> 1065
<212> DNA
<213> Unknown

<220>
<223> Obtained from an environmental sample

<400> 101
atggcgacag tccatccgaa atttaaagta gccgccgtcc aggcggcccc ggcctttctc 60
gacctcgacg cgtcggtgga aaaagcggtg cgcctgattg atgaagccgg cgccgctggt 120
gcccggctca tcgcgtttcc agagactttt atccccggtt atccgtggtg gatctggctc 180
ggtgctccgg cctgggcgat catgcgcggc ttcgtctccc gctatttcga caactcgctg 240
cagtacggca ccccggaagc cgaccggctg cgggcagccg ccaaacgcaa caaaatgttc 300
gtcgcgctcg gactgtcaga gcgcgacggc ggcagtctct acatcgccca atggattatc 360
ggacccgacg gcgagacggt cgcaacgcgc cgcaagctca agcctactca cgccgagcgg 420
acggtgttcg gcgaaggcga tggctcgcac cttgcggtcc acgaacttga tatcgggcgg 480
gtcggtgcgc tgtgctgttg ggagcacctg cagccactgt cgaagtacgc gatgtatgcg 540
cagaacgagc aagttcatat cgcggcgtgg ccgagctttt cgctttacga tccgttcgcg 600
catgcgcttg gcgccgaggt caacaacgcg gcgagcaaga tctacgcggt cgaaggctca 660

tgctttgtga ttgcgccatg cgcgaccgtt tcccaggcga tgatcgacga attgtgtgac     720
tcgcccgaga agcatcagtt cctgcacgtc ggcggcggtt tcgccgtgat ctatggtccc     780
gacggcgcgc cactcgccaa gccactggcg cccgatcagg agggtctcct ttacgcggat     840
atcgacctcg gcatgatttc ggtcgcgaaa gcggcggccg atccggctgg acattacgcg     900
cgcccggacg tgacccgtct gttgttcaac aatcgtcctg ggaaccgggt ggagacactc     960
gcgctgccgg tcgaccagga ggcagaggcg ggagcaggcg gcaaacctgc gcccaagtca    1020
ccgagtgtcg ctgcgttcac actgacgcag gcggcagccg agtag                    1065

<210> 102
<211> 354
<212> PRT
<213> Unknown

<220>
<223> Obtained from an environmental sample

<400> 102
Met Ala Thr Val His Pro Lys Phe Lys Val Ala Ala Val Gln Ala Ala
1               5                   10                  15
Pro Ala Phe Leu Asp Leu Asp Ala Ser Val Glu Lys Ala Val Arg Leu
            20                  25                  30
Ile Asp Glu Ala Gly Ala Ala Gly Ala Arg Leu Ile Ala Phe Pro Glu
        35                  40                  45
Thr Phe Ile Pro Gly Tyr Pro Trp Trp Ile Trp Leu Gly Ala Pro Ala
    50                  55                  60
Trp Ala Ile Met Arg Gly Phe Val Ser Arg Tyr Phe Asp Asn Ser Leu
65                  70                  75                  80
Gln Tyr Gly Thr Pro Glu Ala Asp Arg Leu Arg Ala Ala Ala Lys Arg
                85                  90                  95
Asn Lys Met Phe Val Ala Leu Gly Leu Ser Glu Arg Asp Gly Gly Ser
            100                 105                 110
Leu Tyr Ile Ala Gln Trp Ile Ile Gly Pro Asp Gly Glu Thr Val Ala
        115                 120                 125
Thr Arg Arg Lys Leu Lys Pro Thr His Ala Glu Arg Thr Val Phe Gly
    130                 135                 140
Glu Gly Asp Gly Ser His Leu Ala Val His Glu Leu Asp Ile Gly Arg
145                 150                 155                 160
Val Gly Ala Leu Cys Cys Trp Glu His Leu Gln Pro Leu Ser Lys Tyr
                165                 170                 175
Ala Met Tyr Ala Gln Asn Glu Gln Val His Ile Ala Ala Trp Pro Ser
            180                 185                 190
Phe Ser Leu Tyr Asp Pro Phe Ala His Ala Leu Gly Ala Glu Val Asn
        195                 200                 205
Asn Ala Ala Ser Lys Ile Tyr Ala Val Glu Gly Ser Cys Phe Val Ile
    210                 215                 220
Ala Pro Cys Ala Thr Val Ser Gln Ala Met Ile Asp Glu Leu Cys Asp
225                 230                 235                 240
Ser Pro Glu Lys His Gln Phe Leu His Val Gly Gly Gly Phe Ala Val
                245                 250                 255
Ile Tyr Gly Pro Asp Gly Ala Pro Leu Ala Lys Pro Leu Ala Pro Asp
            260                 265                 270
Gln Glu Gly Leu Leu Tyr Ala Asp Ile Asp Leu Gly Met Ile Ser Val
        275                 280                 285
Ala Lys Ala Ala Ala Asp Pro Ala Gly His Tyr Ala Arg Pro Asp Val
    290                 295                 300
Thr Arg Leu Leu Phe Asn Asn Arg Pro Gly Asn Arg Val Glu Thr Leu
305                 310                 315                 320

Ala Leu Pro Val Asp Gln Glu Ala Glu Ala Gly Ala Gly Gly Lys Pro
                325                 330                 335
Ala Pro Lys Ser Pro Ser Val Ala Ala Phe Thr Leu Thr Gln Ala Ala
            340                 345                 350
Ala Glu

<210> 103
<211> 945
<212> DNA
<213> Unknown

<220>
<223> Obtained from an environmental sample

<400> 103
atgggcgagt tcggcgaggt gacgctgggg gtggcgcagg cggcgccggt gtacttcgac      60
cgggaggcgt cgacggagaa ggctcgcggc ctgatccggg aggcggggga gaagggcgtc     120
gacctgttgg cgttcgggga gacgtggctg acggggtacc cgtactggaa ggatgcgccg     180
tggtctcggg agtacaacga cctgcgcgcg cggtacgtgg cgaatggcgt gatgataccg     240
gggccggaga cggacgcgct atgccaggca gcggcggaag cgggggtgga cgtggcaatc     300
ggcgtggtgg agctggagcc ggggagcctt tcgagcgtgt attgcacgtt gctgttcatc     360
tcgcgcgagg gcgagatcct ggggcggcac cggaagctga agccgacgga ttcggaacgg     420
cggtactggt cagagggtga tgcgacgggg ctgcgggtgt acgagcggcc atatggccgg     480
ttgagcggat tgaactgctg ggaacacctt atgatgttgc cggggtacgc gctggcggca     540
caggggacgc agtttcatgt ggcagcgtgg ccgaacatgg cgagctcggc gagcgagctg     600
ctgtcgcggg cgtatgcgta ccaggccgga tgctacgtgt tgtgcgcggg cgggctcggg      660
cctgcgccgg gagagctacc ggacggcatc gcggcggagt cgctggacca cctgacgggc     720
gagagctgca tcatcgaccc gtggggaaaa gtgatcgcgg ggccggtgtc gtgcgaggag     780
acgctgatta cggcgcgggt atcgaccgcg tcaatctacc ggcgcaagtc gctgacggac     840
gtgggtggcc actactcgcg accggacgtg ttccggttcg aggtggatag gtcggagcgc     900
ccgcgagtgg tgtttcggga tggggatgtg gacgaccggg ggtaa                     945

<210> 104
<211> 314
<212> PRT
<213> Unknown

<220>
<223> Obtained from an environmental sample

<400> 104
Met Gly Glu Phe Gly Glu Val Thr Leu Gly Val Ala Gln Ala Ala Pro
1               5                   10                  15
Val Tyr Phe Asp Arg Glu Ala Ser Thr Glu Lys Ala Arg Gly Leu Ile
            20                  25                  30
Arg Glu Ala Gly Glu Lys Gly Val Asp Leu Leu Ala Phe Gly Glu Thr
        35                  40                  45
Trp Leu Thr Gly Tyr Pro Tyr Trp Lys Asp Ala Pro Trp Ser Arg Glu
    50                  55                  60
Tyr Asn Asp Leu Arg Ala Arg Tyr Val Ala Asn Gly Val Met Ile Pro
65                  70                  75                  80
Gly Pro Glu Thr Asp Ala Leu Cys Gln Ala Ala Ala Glu Ala Gly Val
                85                  90                  95
Asp Val Ala Ile Gly Val Val Glu Leu Glu Pro Gly Ser Leu Ser Ser
            100                 105                 110
Val Tyr Cys Thr Leu Leu Phe Ile Ser Arg Glu Gly Glu Ile Leu Gly

115                 120                 125
Arg His Arg Lys Leu Lys Pro Thr Asp Ser Glu Arg Arg Tyr Trp Ser
    130                 135                 140
Glu Gly Asp Ala Thr Gly Leu Arg Val Tyr Glu Arg Pro Tyr Gly Arg
145                 150                 155                 160
Leu Ser Gly Leu Asn Cys Trp Glu His Leu Met Met Leu Pro Gly Tyr
                165                 170                 175
Ala Leu Ala Ala Gln Gly Thr Gln Phe His Val Ala Ala Trp Pro Asn
            180                 185                 190
Met Ala Ser Ser Ala Ser Glu Leu Leu Ser Arg Ala Tyr Ala Tyr Gln
        195                 200                 205
Ala Gly Cys Tyr Val Leu Cys Ala Gly Gly Leu Gly Pro Ala Pro Gly
    210                 215                 220
Glu Leu Pro Asp Gly Ile Ala Ala Glu Ser Leu Asp His Leu Thr Gly
225                 230                 235                 240
Glu Ser Cys Ile Ile Asp Pro Trp Gly Lys Val Ile Ala Gly Pro Val
                245                 250                 255
Ser Cys Glu Glu Thr Leu Ile Thr Ala Arg Val Ser Thr Ala Ser Ile
            260                 265                 270
Tyr Arg Arg Lys Ser Leu Thr Asp Val Gly Gly His Tyr Ser Arg Pro
        275                 280                 285
Asp Val Phe Arg Phe Glu Val Asp Arg Ser Glu Arg Pro Arg Val Val
    290                 295                 300
Phe Arg Asp Gly Asp Val Asp Asp Arg Gly
305                 310

<210> 105
<211> 975
<212> DNA
<213> Unknown

<220>
<223> Obtained from an environmental sample

<400> 105
atgaccattg tcaaagccgc tgccgtccag attgcccccg ttctctacag ccgtgaaggc      60
actgtagaaa aggtcgttaa caagattcgc gaactcggcg agaagggcgt gcagttcgcc     120
gttttccctg aaaccgtcgt accgtactac ccgtactttt cctttgtgca gagccctttc     180
aaaatgggtt ccgagcacta caaattgctc gaccaggccg ttgtcgtgcc gtcggcgacc     240
accgatgcca tcggcaaagc ggccaaggaa gccaacatgg tggtgtccat cggcgtcaac     300
gaacgcgatg gcagcaccct ctacaacacg cagttgctgt ttgatgccga cggcactttg     360
attcaggccc gtcgcaagat ttcaccgacc taccacgaac gcatgatctg gggcatgggc      420
gacggttccg gcctgcgcgc caccgacagc gcggtcgggc gcatcggaca attggcctgc     480
tgggaacatt acaatccgct ggcgcgttac gccttgatcg aagacggcga acagatccac     540
gcctcgatgt acccgggctc gttcgcaggt cctttattca ctcgccagat ggaagtcagc     600
atccgcatgc atgccctgga atcggcgtgc ttcgtggtca actcgaccgc gtggttgtac     660
ccggaacagc aagcccagat catggccgac accggttgcg agatcgggcc gatctccggc     720
ggctgctaca ccgcgatcat cgacccacag ggtgaagtcg tcggcgcact gaccgaaggc     780
gagggcgaag tgattgccga catcgatctg ttccagatcg aaatccgtaa acgtcagatg     840
gacggccgtg gtcactacag ccgtccggaa atcctgagcc tgaacatcga ccgtacgccg     900
catcgccatg ttcacgaacg caacgaccag cagaaaccgg gtgtgatcga cactgctgaa     960
gaaaccgggc gttga                                                      975

<210> 106
<211> 324
<212> PRT
<213> Unknown

<220>
<223> Obtained from an environmental sample

<400> 106
Met Thr Ile Val Lys Ala Ala Ala Val Gln Ile Ala Pro Val Leu Tyr
1               5                   10                  15
Ser Arg Glu Gly Thr Val Glu Lys Val Val Asn Lys Ile Arg Glu Leu
            20                  25                  30
Gly Glu Lys Gly Val Gln Phe Ala Val Phe Pro Glu Thr Val Val Pro
        35                  40                  45
Tyr Tyr Pro Tyr Phe Ser Phe Val Gln Ser Pro Phe Lys Met Gly Ser
    50                  55                  60
Glu His Tyr Lys Leu Leu Asp Gln Ala Val Val Val Pro Ser Ala Thr
65                  70                  75                  80
Thr Asp Ala Ile Gly Lys Ala Ala Lys Glu Ala Asn Met Val Val Ser
                85                  90                  95
Ile Gly Val Asn Glu Arg Asp Gly Ser Thr Leu Tyr Asn Thr Gln Leu
            100                 105                 110
Leu Phe Asp Ala Asp Gly Thr Leu Ile Gln Ala Arg Arg Lys Ile Ser
        115                 120                 125
Pro Thr Tyr His Glu Arg Met Ile Trp Gly Met Gly Asp Gly Ser Gly
    130                 135                 140
Leu Arg Ala Thr Asp Ser Ala Val Gly Arg Ile Gly Gln Leu Ala Cys
145                 150                 155                 160
Trp Glu His Tyr Asn Pro Leu Ala Arg Tyr Ala Leu Ile Glu Asp Gly
                165                 170                 175
Glu Gln Ile His Ala Ser Met Tyr Pro Gly Ser Phe Ala Gly Pro Leu
            180                 185                 190
Phe Thr Arg Gln Met Glu Val Ser Ile Arg Met His Ala Leu Glu Ser
        195                 200                 205
Ala Cys Phe Val Val Asn Ser Thr Ala Trp Leu Tyr Pro Glu Gln Gln
    210                 215                 220
Ala Gln Ile Met Ala Asp Thr Gly Cys Glu Ile Gly Pro Ile Ser Gly
225                 230                 235                 240
Gly Cys Tyr Thr Ala Ile Ile Asp Pro Gln Gly Glu Val Val Gly Ala
                245                 250                 255
Leu Thr Glu Gly Glu Gly Glu Val Ile Ala Asp Ile Asp Leu Phe Gln
            260                 265                 270
Ile Glu Ile Arg Lys Arg Gln Met Asp Gly Arg Gly His Tyr Ser Arg
        275                 280                 285
Pro Glu Ile Leu Ser Leu Asn Ile Asp Arg Thr Pro His Arg His Val
    290                 295                 300
His Glu Arg Asn Asp Gln Gln Lys Pro Gly Val Ile Asp Thr Ala Glu
305                 310                 315                 320
Glu Thr Gly Arg


<210> 107
<211> 981
<212> DNA
<213> Unknown

<220>
<223> Obtained from an environmental sample

<400> 107

atggccatca ttcgcgcagc agccgtacag atcagcccgg ttctttacag ccgcgaaggc 60
accgtggaca aggtctgcca gcagatcatc acccttggca aacagggtgt gcagttcgcc 120
gtgttcccgg aaacggtggt gccgtactac ccctattttt cctttgtgca gccggcgttc 180
gccatgggtg cgcaacacct caaattgcta gatcaatctg taaccgtgcc atcggccgcc 240
accctggcga ttggcgaagc gtgcaagcaa gcaggaatgg tcgtttccat cggagtcaat 300
gaacgcgatg gcggtacgat ttacaacgcg caattactct tcgatgctga cggcacgctg 360
attcagcatc ggcgcaaaat caccccgacc taccacgagc gcatggtctg ggggcagggc 420
gatggttccg gtctgcgcgc catcgacagc gcggtcgggc gcatcggctc cctggcatgc 480
tgggaacatt acaacccgct ggcccgttac gccttgatgg cagacggcga acagatccac 540
gccgcgatgt ttcccggttc cctggtgggt gacatcttcg ccgagcagat cgaggtcacc 600
atccgccatc acgcattgga gtcaggatgc ttcgtggtca atgcaacagc ctggctggat 660
gcggatcagc agggccaaat aatgcaggac acaggttgcg gccttggtcc catctcgggc 720
ggctgcttca ccgcgatcgt atcgccggaa gggaagctac ttggagagcc gcttcgctcc 780
ggggaaggcg tagtgattgc cgacctcgat acggccttga tcgacaagcg caaacggatg 840
atggattcag taggtcatta cagtcgtccc gagctgctca gcctattgat cgatcgatcg 900
ccgactgcgc atgttcatga acgcgccggc tttgtttcga gcaacgccgg tttgcaggag 960
gtcgcccatg cagaccaatg a 981

<210> 108
<211> 326
<212> PRT
<213> Unknown

<220>
<223> Obtained from an environmental sample

<400> 108
Met Ala Ile Ile Arg Ala Ala Ala Val Gln Ile Ser Pro Val Leu Tyr
1               5                   10                  15
Ser Arg Glu Gly Thr Val Asp Lys Val Cys Gln Gln Ile Ile Thr Leu
            20                  25                  30
Gly Lys Gln Gly Val Gln Phe Ala Val Phe Pro Glu Thr Val Val Pro
        35                  40                  45
Tyr Tyr Pro Tyr Phe Ser Phe Val Gln Pro Ala Phe Ala Met Gly Ala
    50                  55                  60
Gln His Leu Lys Leu Leu Asp Gln Ser Val Thr Val Pro Ser Ala Ala
65                  70                  75                  80
Thr Leu Ala Ile Gly Glu Ala Cys Lys Gln Ala Gly Met Val Val Ser
                85                  90                  95
Ile Gly Val Asn Glu Arg Asp Gly Gly Thr Ile Tyr Asn Ala Gln Leu
            100                 105                 110
Leu Phe Asp Ala Asp Gly Thr Leu Ile Gln His Arg Arg Lys Ile Thr
        115                 120                 125
Pro Thr Tyr His Glu Arg Met Val Trp Gly Gln Gly Asp Gly Ser Gly
    130                 135                 140
Leu Arg Ala Ile Asp Ser Ala Val Gly Arg Ile Gly Ser Leu Ala Cys
145                 150                 155                 160
Trp Glu His Tyr Asn Pro Leu Ala Arg Tyr Ala Leu Met Ala Asp Gly
                165                 170                 175
Glu Gln Ile His Ala Ala Met Phe Pro Gly Ser Leu Val Gly Asp Ile
            180                 185                 190
Phe Ala Glu Gln Ile Glu Val Thr Ile Arg His His Ala Leu Glu Ser
        195                 200                 205
Gly Cys Phe Val Val Asn Ala Thr Ala Trp Leu Asp Ala Asp Gln Gln
    210                 215                 220
Gly Gln Ile Met Gln Asp Thr Gly Cys Gly Leu Gly Pro Ile Ser Gly
225                 230                 235                 240

Gly Cys Phe Thr Ala Ile Val Ser Pro Glu Gly Lys Leu Leu Gly Glu
                245                 250                 255
Pro Leu Arg Ser Gly Glu Gly Val Val Ile Ala Asp Leu Asp Thr Ala
            260                 265                 270
Leu Ile Asp Lys Arg Lys Arg Met Met Asp Ser Val Gly His Tyr Ser
        275                 280                 285
Arg Pro Glu Leu Leu Ser Leu Leu Ile Asp Arg Ser Pro Thr Ala His
    290                 295                 300
Val His Glu Arg Ala Gly Phe Val Ser Ser Asn Ala Gly Leu Gln Glu
305                 310                 315                 320
Val Ala His Ala Asp Gln
                325

<210> 109
<211> 1092
<212> DNA
<213> Unknown

<220>
<223> Obtained from an environmental sample

<400> 109
atggccatca ttcgcgcagc agccgtacag atcagcccgg ttctttacag ccgcgaaggc 60
accgtggaca gggtctgcca gcagatcatc acccttggca aacaaggtgt gcagttcgcc 120
gtgttcccgg aaacggtggt gccgtactac ccctattttt cctttgtgca gccggcattt 180
gcgatgggtg cacaacacct caaattgctc gatcaatctg taaccgtgcc atcggccgcc 240
accctggcga ttggcgaagc gtgcaagcaa gcaggaatgg tcgtttccat cggcgtcaat 300
gaacgcgatg gcggtacgat ttacaacgcg caattactct tcgatgctga cggcactctg 360
attcagcatc ggcgcaaaat caccccgacc taccacgagc gcatggtctg ggggcagggc 420
gatggttccg gtctgcgcgc catcgacagc gcggtcgggc gcatcggctc cctggcatgc 480
tgggaacatt acaacccgct ggcccgttac gccttgatgg cagacggcga acagatccac 540
gccgcgatgt ttcccggttc cctggtgggt gacatcttcg ccgagcagat cgaggtcacc 600
atccgccatc acgcattgga atcaggatgc ttcgtggtca atgcaacagc ttggctggat 660
gcggatcagc agggccaaat aatgcaggac acaggttgcg gccttggtcc catctcgggc 720
ggctgcttca ccgcgatcgt atcgccggaa gggaagctac ttggagagcc gcttcgctca 780
ggggaaggcg tagtgattgc cgacctcgat atggccttga tcgacaagcg caaacggatg 840
atggattcag taggtcatta cagtcgtccc gagctgctca gcctattgat cgatcgatcg 900
ccgactgcgc attttcatga acgcgccggg ctttgttccg agcgacgccg gtttgcagga 960
ggtcgcgcat gcagaccaat gaattgctcg ctgacctgca aatccaaggc ctgcgttggc 1020
cggccgcgca aatggcttgt cgcgccaagg cggcgccggt ccttcagacc acaaggcgct 1080
gagcctaggt aa 1092

<210> 110
<211> 363
<212> PRT
<213> Unknown

<220>
<223> Obtained from an environmental sample

<400> 110
Met Ala Ile Ile Arg Ala Ala Ala Val Gln Ile Ser Pro Val Leu Tyr
1               5                   10                  15
Ser Arg Glu Gly Thr Val Asp Arg Val Cys Gln Gln Ile Ile Thr Leu
            20                  25                  30
Gly Lys Gln Gly Val Gln Phe Ala Val Phe Pro Glu Thr Val Val Pro
        35                  40                  45

Tyr Tyr Pro Tyr Phe Ser Phe Val Gln Pro Ala Phe Ala Met Gly Ala
50                  55                  60
Gln His Leu Lys Leu Leu Asp Gln Ser Val Thr Val Pro Ser Ala Ala
65                  70                  75                  80
Thr Leu Ala Ile Gly Glu Ala Cys Lys Gln Ala Gly Met Val Val Ser
                85                  90                  95
Ile Gly Val Asn Glu Arg Asp Gly Gly Thr Ile Tyr Asn Ala Gln Leu
            100                 105                 110
Leu Phe Asp Ala Asp Gly Thr Leu Ile Gln His Arg Arg Lys Ile Thr
        115                 120                 125
Pro Thr Tyr His Glu Arg Met Val Trp Gly Gln Gly Asp Gly Ser Gly
    130                 135                 140
Leu Arg Ala Ile Asp Ser Ala Val Gly Arg Ile Gly Ser Leu Ala Cys
145                 150                 155                 160
Trp Glu His Tyr Asn Pro Leu Ala Arg Tyr Ala Leu Met Ala Asp Gly
                165                 170                 175
Glu Gln Ile His Ala Ala Met Phe Pro Gly Ser Leu Val Gly Asp Ile
            180                 185                 190
Phe Ala Glu Gln Ile Glu Val Thr Ile Arg His His Ala Leu Glu Ser
        195                 200                 205
Gly Cys Phe Val Val Asn Ala Thr Ala Trp Leu Asp Ala Asp Gln Gln
    210                 215                 220
Gly Gln Ile Met Gln Asp Thr Gly Cys Gly Leu Gly Pro Ile Ser Gly
225                 230                 235                 240
Gly Cys Phe Thr Ala Ile Val Ser Pro Glu Gly Lys Leu Leu Gly Glu
                245                 250                 255
Pro Leu Arg Ser Gly Glu Gly Val Val Ile Ala Asp Leu Asp Met Ala
            260                 265                 270
Leu Ile Asp Lys Arg Lys Arg Met Met Asp Ser Val Gly His Tyr Ser
        275                 280                 285
Arg Pro Glu Leu Leu Ser Leu Leu Ile Asp Arg Ser Pro Thr Ala His
    290                 295                 300
Phe His Glu Arg Ala Gly Leu Cys Ser Glu Arg Arg Arg Phe Ala Gly
305                 310                 315                 320
Gly Arg Ala Cys Arg Pro Met Asn Cys Ser Leu Thr Cys Lys Ser Lys
                325                 330                 335
Ala Cys Val Gly Arg Pro Arg Lys Trp Leu Val Ala Pro Arg Arg Arg
            340                 345                 350
Arg Ser Phe Arg Pro Gln Gly Ala Glu Pro Arg
        355                 360

<210> 111
<211> 990
<212> DNA
<213> Unknown

<220>
<223> Obtained from an environmental sample

<400> 111
atgcccaaaa cagtacgtgc cgcagcagtc cagatcgcgc ccgacctgac gtcacgcgcc      60
ggcaccgtcg agcgggtcct caatgcaatc gccgaagctg ctgacaaagg cgccgagctg     120
atcgtatttc ccgagacctt cgtgccctgg tatccctatt tcagtttcgt tctgccacct     180
gtccagcaag gccctgagca tcttcgtctt tatgaggaag cagtcacggt accatcagca     240
gaaacacggg ccgtcgcgga cgccgcgcgc aaacgcaatg cggttatcgt ccttggcgtc     300
aatgagcgcg accacggctc gctctataac actcagctga tcttcgacgc ggatggcagc     360
ctgaaactca agcgtcgcaa gatcacgccg acctatcacg aacggatgat ctggggccaa     420

ggcgatggcg ccggcctgaa ggttgtcgac actgccgtcg gtcgcgtggg tgccctggca 480
tgctgggagc attacaatcc tctggcccgc tatactttga tggcccagca tgaggaaatt 540
cacgcctctc atttcccggg ctcactggtc ggcccgatat tcggcgagca aatcgaagtc 600
accatgcgcc accacgcgtt ggaatcgggc tgtttcgtgg tcaatgccac cggctggctg 660
agcgaggagc agatcgcatc tattcatccg gaccccgcct tgcaaaaggg cctgcgcgat 720
ggctgcatga cctgcatcat cacgccggaa ggacgccatg tcgtaccgcc gctgacctcg 780
ggcgaaggca tcctgatcgg cgatctggac atgcggctca ttaccaagcg caagcggatg 840
atggattcgg tcggacacta tgctcggcct gaactgctgc accttgtcca tgacacgacg 900
cccgcacgcg cacgcgagca ggtcggcctt tcaggcgatt ttcccgatgc ggagcaagac 960
aagctatttg aggaggttca taatgcgtga 990

<210> 112
<211> 329
<212> PRT
<213> Unknown

<220>
<223> Obtained from an environmental sample

<400> 112
Met Pro Lys Thr Val Arg Ala Ala Ala Val Gln Ile Ala Pro Asp Leu
1               5                   10                  15
Thr Ser Arg Ala Gly Thr Val Glu Arg Val Leu Asn Ala Ile Ala Glu
            20                  25                  30
Ala Ala Asp Lys Gly Ala Glu Leu Ile Val Phe Pro Glu Thr Phe Val
        35                  40                  45
Pro Trp Tyr Pro Tyr Phe Ser Phe Val Leu Pro Pro Val Gln Gln Gly
    50                  55                  60
Pro Glu His Leu Arg Leu Tyr Glu Glu Ala Val Thr Val Pro Ser Ala
65                  70                  75                  80
Glu Thr Arg Ala Val Ala Asp Ala Ala Arg Lys Arg Asn Ala Val Ile
                85                  90                  95
Val Leu Gly Val Asn Glu Arg Asp His Gly Ser Leu Tyr Asn Thr Gln
            100                 105                 110
Leu Ile Phe Asp Ala Asp Gly Ser Leu Lys Leu Lys Arg Arg Lys Ile
        115                 120                 125
Thr Pro Thr Tyr His Glu Arg Met Ile Trp Gly Gln Gly Asp Gly Ala
    130                 135                 140
Gly Leu Lys Val Val Asp Thr Ala Val Gly Arg Val Gly Ala Leu Ala
145                 150                 155                 160
Cys Trp Glu His Tyr Asn Pro Leu Ala Arg Tyr Thr Leu Met Ala Gln
                165                 170                 175
His Glu Glu Ile His Ala Ser His Phe Pro Gly Ser Leu Val Gly Pro
            180                 185                 190
Ile Phe Gly Glu Gln Ile Glu Val Thr Met Arg His His Ala Leu Glu
        195                 200                 205
Ser Gly Cys Phe Val Val Asn Ala Thr Gly Trp Leu Ser Glu Glu Gln
    210                 215                 220
Ile Ala Ser Ile His Pro Asp Pro Ala Leu Gln Lys Gly Leu Arg Asp
225                 230                 235                 240
Gly Cys Met Thr Cys Ile Ile Thr Pro Glu Gly Arg His Val Val Pro
                245                 250                 255
Pro Leu Thr Ser Gly Glu Gly Ile Leu Ile Gly Asp Leu Asp Met Arg
            260                 265                 270
Leu Ile Thr Lys Arg Lys Arg Met Met Asp Ser Val Gly His Tyr Ala
        275                 280                 285
Arg Pro Glu Leu Leu His Leu Val His Asp Thr Thr Pro Ala Arg Ala

290                 295                 300
Arg Glu Gln Val Gly Leu Ser Gly Asp Phe Pro Asp Ala Glu Gln Asp
305                 310                 315                 320
Lys Leu Phe Glu Glu Val His Asn Ala
                325

<210> 113
<211> 993
<212> DNA
<213> Unknown

<220>
<223> Obtained from an environmental sample

<400> 113
atgacgaagg aacgcgccgc gcgcagcctg cgcgcagctg ccatacagct tgaagccgaa      60
gtcggcgaca tcgccgccaa tctcgcacgc atcgaggcga tggtcgagga ggctgcgggc     120
aagggcgccg aactgatcgc cattccggag ttctgcacct cccgcatgcc cttcgatgca     180
cgcgtgcacg acgccgtgct gccgccggac aacttcgtgg tcgatgcctt tcgccgcatg     240
gcagcgacgc acaactgccg gctcggcggc tccatgctca ttgccgacgg tggcgagatc     300
tacaaccgct accacttcgt cgaacccgac ggcagcgtgc atctgcacga caaggatctg     360
ccgacgatgt gggagaacgc cttctacacc ggcggctccg acgacggcgt cttcgacacc     420
ggcatcggcg gcgtcggcgc cgcggtgtgc tgggaactgg tacgcaccgg caccgtgcga     480
cgcatgctcg gtcgcgtcga cgtcgccatg accggcacgc attggtggac gatgccgcac     540
aactggggca gcgccgtcgc gcgcacgctg gccgcgatga cgcagtacaa ccgctacatg     600
tccgagaatg cacccaccga attcgcccgc cgcctgggtg tgccggtgct gcaggcctcg     660
cactgcggaa gcttccgcac cggtttcttg ctgctgccag gcagcgggcg tgcactgccc     720
tatgacaccg agtacgtcgg cgccacacag atcgtcgatg ccgatggcca catcctcgcc     780
caccgtcgca cgcaggaagg ccccggtgtc gtcgtcgccg acatcacgct cggtgcccgc     840
acgcccgagc tgccactgga agaccgcttc tggattcccg agctgccgct cttcctcaag     900
gcctactggc accaccagaa cctgtgcggc aagtcctact accgtcgcgt cggccgcgat     960
gccggcctgg cggcggcgga gcgttcggca tga                                  993

<210> 114
<211> 330
<212> PRT
<213> Unknown

<220>
<223> Obtained from an environmental sample

<400> 114
Met Thr Lys Glu Arg Ala Ala Arg Ser Leu Arg Ala Ala Ala Ile Gln
1               5                   10                  15
Leu Glu Ala Glu Val Gly Asp Ile Ala Ala Asn Leu Ala Arg Ile Glu
            20                  25                  30
Ala Met Val Glu Glu Ala Ala Gly Lys Gly Ala Glu Leu Ile Ala Ile
        35                  40                  45
Pro Glu Phe Cys Thr Ser Arg Met Pro Phe Asp Ala Arg Val His Asp
    50                  55                  60
Ala Val Leu Pro Pro Asp Asn Phe Val Val Asp Ala Phe Arg Arg Met
65                  70                  75                  80
Ala Ala Thr His Asn Cys Arg Leu Gly Gly Ser Met Leu Ile Ala Asp
                85                  90                  95
Gly Gly Glu Ile Tyr Asn Arg Tyr His Phe Val Glu Pro Asp Gly Ser
            100                 105                 110
Val His Leu His Asp Lys Asp Leu Pro Thr Met Trp Glu Asn Ala Phe

115                 120                 125
Tyr Thr Gly Gly Ser Asp Asp Gly Val Phe Asp Thr Gly Ile Gly Gly
    130                 135                 140
Val Gly Ala Ala Val Cys Trp Glu Leu Val Arg Thr Gly Thr Val Arg
145                 150                 155                 160
Arg Met Leu Gly Arg Val Asp Val Ala Met Thr Gly Thr His Trp Trp
                165                 170                 175
Thr Met Pro His Asn Trp Gly Ser Ala Val Ala Arg Thr Leu Ala Ala
            180                 185                 190
Met Thr Gln Tyr Asn Arg Tyr Met Ser Glu Asn Ala Pro Thr Glu Phe
        195                 200                 205
Ala Arg Arg Leu Gly Val Pro Val Leu Gln Ala Ser His Cys Gly Ser
    210                 215                 220
Phe Arg Thr Gly Phe Leu Leu Leu Pro Gly Ser Gly Arg Ala Leu Pro
225                 230                 235                 240
Tyr Asp Thr Glu Tyr Val Gly Ala Thr Gln Ile Val Asp Ala Asp Gly
                245                 250                 255
His Ile Leu Ala His Arg Arg Thr Gln Glu Gly Pro Gly Val Val Val
            260                 265                 270
Ala Asp Ile Thr Leu Gly Ala Arg Thr Pro Glu Leu Pro Leu Glu Asp
        275                 280                 285
Arg Phe Trp Ile Pro Glu Leu Pro Leu Phe Leu Lys Ala Tyr Trp His
    290                 295                 300
His Gln Asn Leu Cys Gly Lys Ser Tyr Tyr Arg Arg Val Gly Arg Asp
305                 310                 315                 320
Ala Gly Leu Ala Ala Ala Glu Arg Ser Ala
                325                 330

<210> 115
<211> 993
<212> DNA
<213> Unknown

<220>
<223> Obtained from an environmental sample

<400> 115
atgaaccaaa tcattaaagc ggcggcagtt caatgtagcc ctgtgttgta tagccaagcg      60
ggtacagtca agaaaatctg tgacacgatt ttggagttgg ggcagcaagg tgtgcaattt     120
gccgtatttc ctgaaactgt tgtgccttat tacccttatt tttcttttgt gcaaccaccg     180
tttgccatgg gtaaagaaca tttaaagcta ttgcatgaat cggttgtcgt gccatcggca     240
gcaacaactt taattggaca ggcatgcaaa gaagcgaaca tggtggtttc tattggtatt     300
aatgagcgtg caggcggcac gatttataac gctcaattgt tgtttgatgc ggatggttcg     360
attattcagc atcgccgtaa aattacccca acgtatcatg aacgtatggt gtgggggcaa     420
ggcgatggca gtggtttacg tgcgatagat tctgctgtag gacgtattgg gtcgctggca     480
tgttgggagc attacaaccc tttggctcgg tttgctttga tggcggatgg tgagcaaatt     540
catgcggcga tgtttccggg atcactcgtg gggcagattt ttgcagatca gatcagtgcc     600
accattcagc accatgcttt agagtcgggc tgttttgtgg tgaatgccac agcatggctt     660
gacccagagc aacaacaaca aattatgcaa gatacaggct gtgaactcgg tccaatttcg     720
gggggatgtt ttacggccat cgtttctcca gaaggcaaat ttttgtctga accgatcaca     780
caaggcgaag gttatgtgat tgccgattta gacttttcct taatcgaaaa acgtaaacgg     840
atgatggatt ctgttgggca ttatagtcgt ccagaattac tcagtttgtt gattgatcgt     900
cgtcctacct cagttttgca tgagttaaaa ctagagaatc catcgaataa cagcatcgaa     960
aaagtgtctg aatttgccga ggtacacgca tag                                  993

<210> 116
<211> 330

<212> PRT
<213> Unknown

<220>
<223> Obtained from an environmental sample

<400> 116
Met Asn Gln Ile Ile Lys Ala Ala Ala Val Gln Cys Ser Pro Val Leu
1               5                   10                  15
Tyr Ser Gln Ala Gly Thr Val Lys Lys Ile Cys Asp Thr Ile Leu Glu
            20                  25                  30
Leu Gly Gln Gln Gly Val Gln Phe Ala Val Phe Pro Glu Thr Val Val
        35                  40                  45
Pro Tyr Tyr Pro Tyr Phe Ser Phe Val Gln Pro Pro Phe Ala Met Gly
    50                  55                  60
Lys Glu His Leu Lys Leu Leu His Glu Ser Val Val Val Pro Ser Ala
65                  70                  75                  80
Ala Thr Thr Leu Ile Gly Gln Ala Cys Lys Glu Ala Asn Met Val Val
                85                  90                  95
Ser Ile Gly Ile Asn Glu Arg Ala Gly Gly Thr Ile Tyr Asn Ala Gln
            100                 105                 110
Leu Leu Phe Asp Ala Asp Gly Ser Ile Ile Gln His Arg Arg Lys Ile
        115                 120                 125
Thr Pro Thr Tyr His Glu Arg Met Val Trp Gly Gln Gly Asp Gly Ser
    130                 135                 140
Gly Leu Arg Ala Ile Asp Ser Ala Val Gly Arg Ile Gly Ser Leu Ala
145                 150                 155                 160
Cys Trp Glu His Tyr Asn Pro Leu Ala Arg Phe Ala Leu Met Ala Asp
                165                 170                 175
Gly Glu Gln Ile His Ala Ala Met Phe Pro Gly Ser Leu Val Gly Gln
            180                 185                 190
Ile Phe Ala Asp Gln Ile Ser Ala Thr Ile Gln His His Ala Leu Glu
        195                 200                 205
Ser Gly Cys Phe Val Val Asn Ala Thr Ala Trp Leu Asp Pro Glu Gln
    210                 215                 220
Gln Gln Gln Ile Met Gln Asp Thr Gly Cys Glu Leu Gly Pro Ile Ser
225                 230                 235                 240
Gly Gly Cys Phe Thr Ala Ile Val Ser Pro Glu Gly Lys Phe Leu Ser
                245                 250                 255
Glu Pro Ile Thr Gln Gly Glu Gly Tyr Val Ile Ala Asp Leu Asp Phe
            260                 265                 270
Ser Leu Ile Glu Lys Arg Lys Arg Met Met Asp Ser Val Gly His Tyr
        275                 280                 285
Ser Arg Pro Glu Leu Leu Ser Leu Leu Ile Asp Arg Arg Pro Thr Ser
    290                 295                 300
Val Leu His Glu Leu Lys Leu Glu Asn Pro Ser Asn Asn Ser Ile Glu
305                 310                 315                 320
Lys Val Ser Glu Phe Ala Glu Val His Ala
                325                 330

<210> 117
<211> 957
<212> DNA
<213> Unknown

<220>
<223> Obtained from an environmental sample

<400> 117
atgactcaat ccaggataat tcgtgctgcg gcagcgcaga tcgctccgga tttgcaggtt 60
ccaggtaaca cgatcgacaa agtttgccgc accatcagcg aggcggccgc aaaaggcgta 120
cagattattg ttttccctga aaccttggtg ccttattacc cttacttctc ttacatttca 180
ccgcccattc aacagggcaa agaacatttg cggctgtatg accatgcagt ggttgtgccc 240
ggctcggaaa ccgaggcaat ttcagctctt gccgcccaac acaatatggt ggtggttttg 300
ggtgtgaacg agcgcgatca cggcacactt tacaacgcac aaattatttt caacagcgac 360
ggaaagattc tgttgaagcg ccgaaaaatt acaccaactt atcacgagcg gatggtgtgg 420
gggcagggtg acgcttcagg cttgaaggtg gttgattccg cagtgggccg tgtgggtgca 480
ttggcctgtt gggaacacta caaccccttg gctcgctatt gtttgatggc ccagcacgaa 540
gaaattcact gtgcgcagtt tcccggttca ttggtggggc aagtttttgc cgaccaaatg 600
gaagtgacca ttcgtcacca cgcacttgag tcgggctgtt ttgtcatcaa cagcaccgct 660
tggctttctg aagaacaggt tcaaagtatt tcatccgaca gcgcattgca gaaagggctt 720
agaggcggtt gtttcacggc cattgtcagc cctgagggaa agctgttggc tgagccgctc 780
accgagggtg agggcatggt gatcgccgac ctcgacatgg cgttggttac gaaacgcaaa 840
cgcatgatgg attcagtggg ccattatgcg cgccccgagt tgttgagttt gctggttcgg 900
gatgaggctt caagccccat gaaaaaaatt cagggagttc aacatgctga gtactga 957

<210> 118
<211> 318
<212> PRT
<213> Unknown

<220>
<223> Obtained from an environmental sample

<400> 118
Met Thr Gln Ser Arg Ile Ile Arg Ala Ala Ala Ala Gln Ile Ala Pro
1               5                   10                  15
Asp Leu Gln Val Pro Gly Asn Thr Ile Asp Lys Val Cys Arg Thr Ile
            20                  25                  30
Ser Glu Ala Ala Ala Lys Gly Val Gln Ile Ile Val Phe Pro Glu Thr
        35                  40                  45
Leu Val Pro Tyr Tyr Pro Tyr Phe Ser Tyr Ile Ser Pro Pro Ile Gln
    50                  55                  60
Gln Gly Lys Glu His Leu Arg Leu Tyr Asp His Ala Val Val Val Pro
65                  70                  75                  80
Gly Ser Glu Thr Glu Ala Ile Ser Ala Leu Ala Ala Gln His Asn Met
                85                  90                  95
Val Val Val Leu Gly Val Asn Glu Arg Asp His Gly Thr Leu Tyr Asn
            100                 105                 110
Ala Gln Ile Ile Phe Asn Ser Asp Gly Lys Ile Leu Leu Lys Arg Arg
        115                 120                 125
Lys Ile Thr Pro Thr Tyr His Glu Arg Met Val Trp Gly Gln Gly Asp
    130                 135                 140
Ala Ser Gly Leu Lys Val Val Asp Ser Ala Val Gly Arg Val Gly Ala
145                 150                 155                 160
Leu Ala Cys Trp Glu His Tyr Asn Pro Leu Ala Arg Tyr Cys Leu Met
                165                 170                 175
Ala Gln His Glu Glu Ile His Cys Ala Gln Phe Pro Gly Ser Leu Val
            180                 185                 190
Gly Gln Val Phe Ala Asp Gln Met Glu Val Thr Ile Arg His His Ala
        195                 200                 205
Leu Glu Ser Gly Cys Phe Val Ile Asn Ser Thr Ala Trp Leu Ser Glu
    210                 215                 220
Glu Gln Val Gln Ser Ile Ser Ser Asp Ser Ala Leu Gln Lys Gly Leu

225                 230                 235                 240
Arg Gly Gly Cys Phe Thr Ala Ile Val Ser Pro Glu Gly Lys Leu Leu
                245                 250                 255
Ala Glu Pro Leu Thr Glu Gly Glu Gly Met Val Ile Ala Asp Leu Asp
            260                 265                 270
Met Ala Leu Val Thr Lys Arg Lys Arg Met Met Asp Ser Val Gly His
        275                 280                 285
Tyr Ala Arg Pro Glu Leu Leu Ser Leu Leu Val Arg Asp Glu Ala Ser
    290                 295                 300
Ser Pro Met Lys Lys Ile Gln Gly Val Gln His Ala Glu Tyr
305                 310                 315

<210> 119
<211> 984
<212> DNA
<213> Unknown

<220>
<223> Obtained from an environmental sample

<400> 119
atggatacac tcaaagttgg attggttcag atggccccca tctggttgaa ccgggataaa      60
accctgatca aagttgagga atacatgcag aaagcaggca aacagggctg caacctggta     120
gcttttggtg aagcgctggt tcccggctac cccttctggg tggaacgcac agagggcgcc     180
agattcaatt ccaaagtcca gaaagaactc tttgcacatt accttgatca ggcggtgcag     240
atcgaagccg gccaccttga tcctctccag gcattagccc aacaatacaa gatggctgtg     300
tacgtgggga cgattgaacg cccgcctgag cggagcggcc acagcctgta ctgctcccta     360
atatttatag acccagaagg cgagatcggc tcggttcacc gcaagttgat gcccacccat     420
gaggaacgcc tggtctggtc aactggcgat gggcacggcc tgcgaacaca ttctctgggc     480
gcctttaccg ttggcggact caactgctgg gaaaactgga tgccgctctc ccgcacagct     540
ctttatgcca tgggagagga tcttcatgtt gctgcctggc ccgggagtca gcgcaatact     600
tatgatataa ccaaattcat tgccaaggaa tctcgctctt atgtgatctc cgtatccggg     660
atgatgaaaa aagaaaatat cctctctgaa attccccaca gccaattgat gctggaaaat     720
agcgaggata ttatggctga tggcggatcc tgtctggctg gaccagatgg agaatggatc     780
atcgagccca tcgtcggaga ggaaaccctg gtaactgctg aactatcaca tcagcgggtc     840
agagaagaaa gacagaattt cgacccaaca ggtcactaca gtcggcctga tgtgacccgc     900
ctggtagtcg accgcaggcg ccagcagatc ctggagatca ccccggacga aaaaggaaga     960
tcggatgaaa atcaatccct ttaa                                            984

<210> 120
<211> 327
<212> PRT
<213> Unknown

<220>
<223> Obtained from an environmental sample

<400> 120
Met Asp Thr Leu Lys Val Gly Leu Val Gln Met Ala Pro Ile Trp Leu
 1               5                  10                  15
Asn Arg Asp Lys Thr Leu Ile Lys Val Glu Glu Tyr Met Gln Lys Ala
            20                  25                  30
Gly Lys Gln Gly Cys Asn Leu Val Ala Phe Gly Glu Ala Leu Val Pro
        35                  40                  45
Gly Tyr Pro Phe Trp Val Glu Arg Thr Glu Gly Ala Arg Phe Asn Ser
    50                  55                  60
Lys Val Gln Lys Glu Leu Phe Ala His Tyr Leu Asp Gln Ala Val Gln

65                  70                  75                  80
Ile Glu Ala Gly His Leu Asp Pro Leu Gln Ala Leu Ala Gln Gln Tyr
                85                  90                  95
Lys Met Ala Val Tyr Val Gly Thr Ile Glu Arg Pro Pro Glu Arg Ser
            100                 105                 110
Gly His Ser Leu Tyr Cys Ser Leu Ile Phe Ile Asp Pro Glu Gly Glu
        115                 120                 125
Ile Gly Ser Val His Arg Lys Leu Met Pro Thr His Glu Glu Arg Leu
    130                 135                 140
Val Trp Ser Thr Gly Asp Gly His Gly Leu Arg Thr His Ser Leu Gly
145                 150                 155                 160
Ala Phe Thr Val Gly Gly Leu Asn Cys Trp Glu Asn Trp Met Pro Leu
                165                 170                 175
Ser Arg Thr Ala Leu Tyr Ala Met Gly Glu Asp Leu His Val Ala Ala
            180                 185                 190
Trp Pro Gly Ser Gln Arg Asn Thr Tyr Asp Ile Thr Lys Phe Ile Ala
        195                 200                 205
Lys Glu Ser Arg Ser Tyr Val Ile Ser Val Ser Gly Met Met Lys Lys
    210                 215                 220
Glu Asn Ile Leu Ser Glu Ile Pro His Ser Gln Leu Met Leu Glu Asn
225                 230                 235                 240
Ser Glu Asp Ile Met Ala Asp Gly Gly Ser Cys Leu Ala Gly Pro Asp
                245                 250                 255
Gly Glu Trp Ile Ile Glu Pro Ile Val Gly Glu Glu Thr Leu Val Thr
            260                 265                 270
Ala Glu Leu Ser His Gln Arg Val Arg Glu Glu Arg Gln Asn Phe Asp
        275                 280                 285
Pro Thr Gly His Tyr Ser Arg Pro Asp Val Thr Arg Leu Val Val Asp
    290                 295                 300
Arg Arg Arg Gln Gln Ile Leu Glu Ile Thr Pro Asp Glu Lys Gly Arg
305                 310                 315                 320
Ser Asp Glu Asn Gln Ser Leu
                325

<210> 121
<211> 1158
<212> DNA
<213> Unknown

<220>
<223> Obtained from an environmental sample

<400> 121
atgagcaaaa aagttctagg cggcagagaa aaagtaaaag ttgcagtagt tcaggctgcg      60
cccgttttca tggacaagga gaagacgatt gaaaaggctt gcaagctaat aaaagaagcg     120
gggagaaatc gagccgagct catagcgttc tcagagtcat tcatccccgt ctatcctgca     180
tactataccg tcggctatga aaccccttct caagaatgga gagattacgt gattgcgcta     240
caggataact ccgtgctgat tccgagcgag gataccgagg tactcggaca ggctgcaaag     300
gaggcagggg cttatgcagt aataggatgc agcgagatgg acgaccgtcc gggaagccga     360
acagtttaca acacgctcct cttcatcggc aaagacggca aggtcatggg aaggcataga     420
aaactcaaac ccacgttcac ggagagaata tactggggag agggagatgc tggagacata     480
aaggtttttg ataccgagat cggcaggatc ggaggcctcg tatgctggga gaaccatatg     540
actctagtca gggccgcgat gatacacagg ggagaggagt ttcatatcgc ggtctggccg     600
ggaaactgga agggtgcgga aaacaagctt ctccaagcag ataatagccc aggaggcgcc     660
ctctgcaacc ttcaatctct cattaaagta cacgcctttg aggccggggc gtttgtgctg     720
agcgcttgcg gctttttgac gccagaggat ttcccggaaa ggtggcatta tataagggat     780
ggtaaccata ttaactgcga ctgggcactg ggcggaagct caatcgtcaa tcccgccggc     840

cgttatctcg tcgagcctaa ctttgagaag gatgcaatcc tctatgcgga ttgttatgca 900
aaccagataa aagcagtaaa agcggttttt gattcccttg gccactattc ccgctgggat 960
attgcccaac tggcgataag gcaggaagcc tggaatccag aggtttcttt gatcgattcc 1020
tcttcgactg aagttgagct tccggcagac gagcttcgaa ggatttcgga gaagtttgaa 1080
gtaactgcgg ataagttgga atctttgctt gaggaaattg gaaagattaa aaagcccagg 1140
aaacaagccg gttcctaa 1158

<210> 122
<211> 385
<212> PRT
<213> Unknown

<220>
<223> Obtained from an environmental sample

<400> 122
Met Ser Lys Lys Val Leu Gly Gly Arg Glu Lys Val Lys Val Ala Val
1               5                   10                  15
Val Gln Ala Ala Pro Val Phe Met Asp Lys Glu Lys Thr Ile Glu Lys
            20                  25                  30
Ala Cys Lys Leu Ile Lys Glu Ala Gly Arg Asn Arg Ala Glu Leu Ile
        35                  40                  45
Ala Phe Ser Glu Ser Phe Ile Pro Val Tyr Pro Ala Tyr Tyr Thr Val
    50                  55                  60
Gly Tyr Glu Thr Pro Ser Gln Glu Trp Arg Asp Tyr Val Ile Ala Leu
65                  70                  75                  80
Gln Asp Asn Ser Val Leu Ile Pro Ser Glu Asp Thr Glu Val Leu Gly
                85                  90                  95
Gln Ala Ala Lys Glu Ala Gly Ala Tyr Ala Val Ile Gly Cys Ser Glu
            100                 105                 110
Met Asp Asp Arg Pro Gly Ser Arg Thr Val Tyr Asn Thr Leu Leu Phe
        115                 120                 125
Ile Gly Lys Asp Gly Lys Val Met Gly Arg His Arg Lys Leu Lys Pro
    130                 135                 140
Thr Phe Thr Glu Arg Ile Tyr Trp Gly Glu Gly Asp Ala Gly Asp Ile
145                 150                 155                 160
Lys Val Phe Asp Thr Glu Ile Gly Arg Ile Gly Gly Leu Val Cys Trp
                165                 170                 175
Glu Asn His Met Thr Leu Val Arg Ala Ala Met Ile His Arg Gly Glu
            180                 185                 190
Glu Phe His Ile Ala Val Trp Pro Gly Asn Trp Lys Gly Ala Glu Asn
        195                 200                 205
Lys Leu Leu Gln Ala Asp Asn Ser Pro Gly Gly Ala Leu Cys Asn Leu
    210                 215                 220
Gln Ser Leu Ile Lys Val His Ala Phe Glu Ala Gly Ala Phe Val Leu
225                 230                 235                 240
Ser Ala Cys Gly Phe Leu Thr Pro Glu Asp Phe Pro Glu Arg Trp His
                245                 250                 255
Tyr Ile Arg Asp Gly Asn His Ile Asn Cys Asp Trp Ala Leu Gly Gly
            260                 265                 270
Ser Ser Ile Val Asn Pro Ala Gly Arg Tyr Leu Val Glu Pro Asn Phe
        275                 280                 285
Glu Lys Asp Ala Ile Leu Tyr Ala Asp Cys Tyr Ala Asn Gln Ile Lys
    290                 295                 300
Ala Val Lys Ala Val Phe Asp Ser Leu Gly His Tyr Ser Arg Trp Asp
305                 310                 315                 320
Ile Ala Gln Leu Ala Ile Arg Gln Glu Ala Trp Asn Pro Glu Val Ser

                325                 330                 335
Leu Ile Asp Ser Ser Ser Thr Glu Val Glu Leu Pro Ala Asp Glu Leu
            340                 345                 350
Arg Arg Ile Ser Glu Lys Phe Glu Val Thr Ala Asp Lys Leu Glu Ser
        355                 360                 365
Leu Leu Glu Glu Ile Gly Lys Ile Lys Lys Pro Arg Lys Gln Ala Gly
    370                 375                 380
Ser
385

<210> 123
<211> 990
<212> DNA
<213> Unknown

<220>
<223> Obtained from an environmental sample

<400> 123
atgtcaactt tcaagatcgc taccgtgcag agtgcaccag tatttatgga ccgcgaagct 60
accattgaca agacttgcga gctgatcgcc gaagcagcac aagatgacga cgttcgccta 120
gtggtcttcc ccgaggcctt tatccccacc tatccggact gggtatggcg tatccctccc 180
ggacagcacc agatgcttgc cgacctgtac ggggagttgc tcgagcagtc ggtgacgata 240
cccagtctgg ctaccgagcg gctctgtcag gctgcaaaga aagcgggcgt ttatgtagct 300
gtgggcctta acgaacgcaa tacagaggcc agcaacgcta ccctgtacaa caccctgctc 360
tacattgacg ccgagggcaa cttgctaggt aagcaccgaa agctggtacc gaccgctccc 420
gaacgcatgg tctgggcaca gggagatggc agtacccttg aggtctacga gacctccttc 480
ggaaaactca gcggactaat ctgttgggag aactacatgc ctctcgctcg ttatgccctg 540
tatgcctggg gagtacagct ctatttggct cctacttggg atcgaggcga gccctggctt 600
tccactctgc ggcacattgc caaggaagga cgagtatacg tggtcggctg ctctatcgcc 660
ttacgtaagg aagacatccc cgaccgattc gaattcaagg cgaagtacta cgcagaggca 720
ggagagtgga taaacaaagg tgacagcgtc atcgtcggtc ccgatggcga gctcatcgcc 780
gggcctctac ataaggaaca ggggatactc tatgctgagc tggacacaag gcagatgcac 840
gcccccaagt ggaacctgga tgtagccgga cactacgcgc gcccggacgt gtttcggctg 900
accgtgagca aggatggcca tccgatgctc ggcgttgccc aagggcccaa gcatgagccg 960
caagataaga ccgaagtatt agagggctag 990

<210> 124
<211> 329
<212> PRT
<213> Unknown

<220>
<223> Obtained from an environmental sample

<400> 124
Met Ser Thr Phe Lys Ile Ala Thr Val Gln Ser Ala Pro Val Phe Met
1               5                   10                  15
Asp Arg Glu Ala Thr Ile Asp Lys Thr Cys Glu Leu Ile Ala Glu Ala
            20                  25                  30
Ala Gln Asp Asp Asp Val Arg Leu Val Val Phe Pro Glu Ala Phe Ile
        35                  40                  45
Pro Thr Tyr Pro Asp Trp Val Trp Arg Ile Pro Pro Gly Gln His Gln
    50                  55                  60
Met Leu Ala Asp Leu Tyr Gly Glu Leu Leu Glu Gln Ser Val Thr Ile
65                  70                  75                  80
Pro Ser Leu Ala Thr Glu Arg Leu Cys Gln Ala Ala Lys Lys Ala Gly

                    85                  90                  95
Val Tyr Val Ala Val Gly Leu Asn Glu Arg Asn Thr Glu Ala Ser Asn
            100                 105                 110
Ala Thr Leu Tyr Asn Thr Leu Leu Tyr Ile Asp Ala Glu Gly Asn Leu
        115                 120                 125
Leu Gly Lys His Arg Lys Leu Val Pro Thr Ala Pro Glu Arg Met Val
    130                 135                 140
Trp Ala Gln Gly Asp Gly Ser Thr Leu Glu Val Tyr Glu Thr Ser Phe
145                 150                 155                 160
Gly Lys Leu Ser Gly Leu Ile Cys Trp Glu Asn Tyr Met Pro Leu Ala
                165                 170                 175
Arg Tyr Ala Leu Tyr Ala Trp Gly Val Gln Leu Tyr Leu Ala Pro Thr
            180                 185                 190
Trp Asp Arg Gly Glu Pro Trp Leu Ser Thr Leu Arg His Ile Ala Lys
        195                 200                 205
Glu Gly Arg Val Tyr Val Val Gly Cys Ser Ile Ala Leu Arg Lys Glu
    210                 215                 220
Asp Ile Pro Asp Arg Phe Glu Phe Lys Ala Lys Tyr Tyr Ala Glu Ala
225                 230                 235                 240
Gly Glu Trp Ile Asn Lys Gly Asp Ser Val Ile Val Gly Pro Asp Gly
                245                 250                 255
Glu Leu Ile Ala Gly Pro Leu His Lys Glu Gln Gly Ile Leu Tyr Ala
            260                 265                 270
Glu Leu Asp Thr Arg Gln Met His Ala Pro Lys Trp Asn Leu Asp Val
        275                 280                 285
Ala Gly His Tyr Ala Arg Pro Asp Val Phe Arg Leu Thr Val Ser Lys
    290                 295                 300
Asp Gly His Pro Met Leu Gly Val Ala Gln Gly Pro Lys His Glu Pro
305                 310                 315                 320
Gln Asp Lys Thr Glu Val Leu Glu Gly
                325

<210> 125
<211> 1050
<212> DNA
<213> Unknown

<220>
<223> Obtained from an environmental sample

<400> 125
atgacaactg taaaaaagac ggtacgcgca gcagcgatcc agatcgcacc tgacctcgac      60
agtgcaggcg gtacgctgga caaggtttgc acggccattc aaaaggcggc ggcacaaggc     120
gcggagctgg tggtttttcc cgaaaccttc ttgccctact atccttactt ttcattcgtg     180
cggccgccct tcgcatccgg cccggaacac ttgctgctat atgaacgcgc agtggcggtg     240
ccaggcccgg tgaccgatgc cgtctctgcc gtcgcgcgca gccacggcgt ggtggtggta     300
ctcggcgtca atgaacgcga ccatggcacg ctgtacaaca cccaactggt gttcgacgcg     360
aatggcgaac tggtgttgaa acgcagaaaa atcacgccga cttatcacga gcggatgatc     420
tggggtcaag gcgacggcag cggactcaaa gtagtgcaaa cggcggtcgg ccggctaggc     480
gcgctagcct gttgggaaca ctacaaccca ctggcccgtt atgcattgat ggcgcaacac     540
gaagaaatcc attgcgccca gtttcccggg tccatggtcg ggcaaatatt cgccgaccag     600
atggaagtga cgatacgcca tcacgctctc gagtcggctt gcttcgtggt gaatgccaca     660
ggctggctga ccgatgcgca aatcacatcg atcacgccgg accccgcgct acaaaaggca     720
ttacgtggcg gttgctgcac cgccatcgtc tcgccggaag gtgtgctcct ggcagagccg     780
ctacgcagcg gcgaaggcat ggtgatcgcc gatctcgata tggcactcat caccaaacgc     840
aaacggatga tggattcggt cggccactat gcgcggcccg aattgttaag cctgcttgtc     900
gacgaccggc gcaaggtacc ggtatccgcg ctatttgccg acagcaaccc tgccaacggg     960

cacacagttt tcaccccatc cgacatacca acccttggga gcgcacatca tgcaaacagt 1020
taccaaaccg aaccagcaac tgatcactga 1050

<210> 126
<211> 349
<212> PRT
<213> Unknown

<220>
<223> Obtained from an environmental sample

<400> 126
Met Thr Thr Val Lys Lys Thr Val Arg Ala Ala Ala Ile Gln Ile Ala
1               5                   10                  15
Pro Asp Leu Asp Ser Ala Gly Gly Thr Leu Asp Lys Val Cys Thr Ala
            20                  25                  30
Ile Gln Lys Ala Ala Ala Gln Gly Ala Glu Leu Val Val Phe Pro Glu
        35                  40                  45
Thr Phe Leu Pro Tyr Tyr Pro Tyr Phe Ser Phe Val Arg Pro Pro Phe
    50                  55                  60
Ala Ser Gly Pro Glu His Leu Leu Leu Tyr Glu Arg Ala Val Ala Val
65                  70                  75                  80
Pro Gly Pro Val Thr Asp Ala Val Ser Ala Val Ala Arg Ser His Gly
                85                  90                  95
Val Val Val Val Leu Gly Val Asn Glu Arg Asp His Gly Thr Leu Tyr
            100                 105                 110
Asn Thr Gln Leu Val Phe Asp Ala Asn Gly Glu Leu Val Leu Lys Arg
        115                 120                 125
Arg Lys Ile Thr Pro Thr Tyr His Glu Arg Met Ile Trp Gly Gln Gly
    130                 135                 140
Asp Gly Ser Gly Leu Lys Val Val Gln Thr Ala Val Gly Arg Leu Gly
145                 150                 155                 160
Ala Leu Ala Cys Trp Glu His Tyr Asn Pro Leu Ala Arg Tyr Ala Leu
                165                 170                 175
Met Ala Gln His Glu Glu Ile His Cys Ala Gln Phe Pro Gly Ser Met
            180                 185                 190
Val Gly Gln Ile Phe Ala Asp Gln Met Glu Val Thr Ile Arg His His
        195                 200                 205
Ala Leu Glu Ser Ala Cys Phe Val Val Asn Ala Thr Gly Trp Leu Thr
    210                 215                 220
Asp Ala Gln Ile Thr Ser Ile Thr Pro Asp Pro Ala Leu Gln Lys Ala
225                 230                 235                 240
Leu Arg Gly Gly Cys Cys Thr Ala Ile Val Ser Pro Glu Gly Val Leu
                245                 250                 255
Leu Ala Glu Pro Leu Arg Ser Gly Glu Gly Met Val Ile Ala Asp Leu
            260                 265                 270
Asp Met Ala Leu Ile Thr Lys Arg Lys Arg Met Met Asp Ser Val Gly
        275                 280                 285
His Tyr Ala Arg Pro Glu Leu Leu Ser Leu Leu Val Asp Asp Arg Arg
    290                 295                 300
Lys Val Pro Val Ser Ala Leu Phe Ala Asp Ser Asn Pro Ala Asn Gly
305                 310                 315                 320
His Thr Val Phe Thr Pro Ser Asp Ile Pro Thr Leu Gly Ser Ala His
                325                 330                 335
His Ala Asn Ser Tyr Gln Thr Glu Pro Ala Thr Asp His
            340                 345

<210> 127
<211> 1005
<212> DNA
<213> Unknown

<220>
<223> Obtained from an environmental sample

<400> 127
atgatagcac ggaagacaat aagggcggcg gcggtgcaga tagcgcctgt gatggaagat 60
cggaaggcga cgaccgacaa ggtgtgcgcc tacattcagg aagcaggcga gaatggagcc 120
gaaattgtgg tgtttcctga aaccttcatt cccaattatc cctatttctc ttttgtaaaa 180
cctcccgtgt tggcaggtaa ggatcacctt accttgtatg accaagcggt ggaaatccct 240
agccctacta ccgaccaagt ggggtctatg gccaaaaaat ggggaatcgt agtggtgttg 300
ggcgtgaacg aaagaagcca cggcactttg tacaatgccc aaattgtctt tgacgctact 360
ggtgatattg tattggtgag acgcaaaatc acccctacct atcatgaacg gatgatctgg 420
ggacagggag atggcagtgg attaaaagca gtagacacag ctgtgggaag agtgggcgct 480
ttggcgtgtt gggaacacta taatccactt gcgcgctacg cccttatggt agaccatgag 540
gaaattcatt gcagccaatt ccctggctct atggtcggcc ccattttcgg tgaccagata 600
gaagtgacga ttcgccacca tgcgttggaa tcgggttgtt ttgtcatcaa ttccacaggt 660
tggctgtttg aagagcaaat ccaagccatc accgatgatc cgaaactgca caaagcattg 720
aaagacggct gtatgaccgc cattatttct cccgaaggcg tgcatttgac caaaccctta 780
acagaaggcg aaggcatcat ctacgcctat ctggacatga aactcataga caagcggaaa 840
cggatgatgg actcggtagg acactatgca cgtccagagt tgctctcttt gcatatcaac 900
aatgcagagc aaaaaccagc cgtttacacc tctcctctta ccaaaacgga aaccaaagaa 960
gacgtaaaaa gctatgatcg caacaaagaa cagcttatcg tctga 1005

<210> 128
<211> 334
<212> PRT
<213> Unknown

<220>
<223> Obtained from an environmental sample

<400> 128
Met Ile Ala Arg Lys Thr Ile Arg Ala Ala Ala Val Gln Ile Ala Pro
1               5                   10                  15
Val Met Glu Asp Arg Lys Ala Thr Thr Asp Lys Val Cys Ala Tyr Ile
            20                  25                  30
Gln Glu Ala Gly Glu Asn Gly Ala Glu Ile Val Val Phe Pro Glu Thr
        35                  40                  45
Phe Ile Pro Asn Tyr Pro Tyr Phe Ser Phe Val Lys Pro Pro Val Leu
    50                  55                  60
Ala Gly Lys Asp His Leu Thr Leu Tyr Asp Gln Ala Val Glu Ile Pro
65                  70                  75                  80
Ser Pro Thr Thr Asp Gln Val Gly Ser Met Ala Lys Lys Trp Gly Ile
                85                  90                  95
Val Val Val Leu Gly Val Asn Glu Arg Ser His Gly Thr Leu Tyr Asn
            100                 105                 110
Ala Gln Ile Val Phe Asp Ala Thr Gly Asp Ile Val Leu Val Arg Arg
        115                 120                 125
Lys Ile Thr Pro Thr Tyr His Glu Arg Met Ile Trp Gly Gln Gly Asp
    130                 135                 140
Gly Ser Gly Leu Lys Ala Val Asp Thr Ala Val Gly Arg Val Gly Ala
145                 150                 155                 160
Leu Ala Cys Trp Glu His Tyr Asn Pro Leu Ala Arg Tyr Ala Leu Met

                    165                     170                     175
Val Asp His Glu Glu Ile His Cys Ser Gln Phe Pro Gly Ser Met Val
            180                     185                     190
Gly Pro Ile Phe Gly Asp Gln Ile Glu Val Thr Ile Arg His His Ala
        195                     200                     205
Leu Glu Ser Gly Cys Phe Val Ile Asn Ser Thr Gly Trp Leu Phe Glu
    210                     215                     220
Glu Gln Ile Gln Ala Ile Thr Asp Asp Pro Lys Leu His Lys Ala Leu
225                     230                     235                     240
Lys Asp Gly Cys Met Thr Ala Ile Ile Ser Pro Glu Gly Val His Leu
                    245                     250                     255
Thr Lys Pro Leu Thr Glu Gly Glu Gly Ile Ile Tyr Ala Tyr Leu Asp
            260                     265                     270
Met Lys Leu Ile Asp Lys Arg Lys Arg Met Met Asp Ser Val Gly His
        275                     280                     285
Tyr Ala Arg Pro Glu Leu Leu Ser Leu His Ile Asn Asn Ala Glu Gln
    290                     295                     300
Lys Pro Ala Val Tyr Thr Ser Pro Leu Thr Lys Thr Glu Thr Lys Glu
305                     310                     315                     320
Asp Val Lys Ser Tyr Asp Arg Asn Lys Glu Gln Leu Ile Val
                    325                     330

<210> 129
<211> 1011
<212> DNA
<213> Unknown

<220>
<223> Obtained from an environmental sample

<400> 129
atgtcagaaa agcgaattat taaagcggct gcagttcaaa tcacaccaga ttttgaatcg      60
catgatggaa ccgtaaagaa ggtttgtaat gtaattgatg aagcgggtgc taaaggtgta     120
cagatcattg tattccctga aacctttatt ccatattacc catatttttc tttcatcact     180
ccaccagtga ctgctggcgc ggagcatttg cggctctatg aaaaaagtgt cgtgatacct     240
ggtcccgtta ctcaagccat ttccgaacgt gcacgcatga ataatatggt tgttgtactt     300
ggtgtaaatg agcgtgataa cggcagtcta tataacaccc agattatttt tgatgctacc     360
ggtgagatgc ttctgaagag aagaaaaatc acacctacct atcatgagcg catgatttgg     420
gggcaaggag atgcttcagg cctgaaggtc gtcgatacgg ctattggcgg agtcggagca     480
ttggcatgct gggagcacta taaccctttg gctagataca gcctcatgac acagcatgaa     540
gaaattcact gtgctcaatt tccaggctcc atggttggtc agatcttcgc agatcaaatg     600
gatgtcacga ttcgtcatca tgccttggag tcaggttgct tcgtcatcaa ctccactggc     660
tggttaactg atgatcagat caaatctatc accgacgatc ccaaaatgca gaaagcttta     720
agaggtggtt gcaacacggc cattatttct ccagaaggga atcatttaac cgagcctttg     780
cgagaaggtg aaggcatggt gattgctgat cttgatatgg cactcatcac caaacgaaaa     840
agaatgatgg actcagttgg ccactacgcc agaccagaac tgttgagctt agcgatcaat     900
gatgctccgg ctactccttc attccagatg aacgaacatc gtcttaaatc agtgcaatta     960
cctatcgcag aggagcttaa aaatgacaac aagcttagca gtggacagta a             1011

<210> 130
<211> 336
<212> PRT
<213> Unknown

<220>
<223> Obtained from an environmental sample

<400> 130
Met Ser Glu Lys Arg Ile Ile Lys Ala Ala Ala Val Gln Ile Thr Pro
1               5                   10                  15
Asp Phe Glu Ser His Asp Gly Thr Val Lys Lys Val Cys Asn Val Ile
            20                  25                  30
Asp Glu Ala Gly Ala Lys Gly Val Gln Ile Ile Val Phe Pro Glu Thr
        35                  40                  45
Phe Ile Pro Tyr Tyr Pro Tyr Phe Ser Phe Ile Thr Pro Pro Val Thr
    50                  55                  60
Ala Gly Ala Glu His Leu Arg Leu Tyr Glu Lys Ser Val Val Ile Pro
65                  70                  75                  80
Gly Pro Val Thr Gln Ala Ile Ser Glu Arg Ala Arg Met Asn Asn Met
                85                  90                  95
Val Val Val Leu Gly Val Asn Glu Arg Asp Asn Gly Ser Leu Tyr Asn
            100                 105                 110
Thr Gln Ile Ile Phe Asp Ala Thr Gly Glu Met Leu Leu Lys Arg Arg
        115                 120                 125
Lys Ile Thr Pro Thr Tyr His Glu Arg Met Ile Trp Gly Gln Gly Asp
    130                 135                 140
Ala Ser Gly Leu Lys Val Val Asp Thr Ala Ile Gly Arg Val Gly Ala
145                 150                 155                 160
Leu Ala Cys Trp Glu His Tyr Asn Pro Leu Ala Arg Tyr Ser Leu Met
                165                 170                 175
Thr Gln His Glu Glu Ile His Cys Ala Gln Phe Pro Gly Ser Met Val
            180                 185                 190
Gly Gln Ile Phe Ala Asp Gln Met Asp Val Thr Ile Arg His His Ala
        195                 200                 205
Leu Glu Ser Gly Cys Phe Val Ile Asn Ser Thr Gly Trp Leu Thr Asp
    210                 215                 220
Asp Gln Ile Lys Ser Ile Thr Asp Asp Pro Lys Met Gln Lys Ala Leu
225                 230                 235                 240
Arg Gly Gly Cys Asn Thr Ala Ile Ile Ser Pro Glu Gly Asn His Leu
                245                 250                 255
Thr Glu Pro Leu Arg Glu Gly Glu Gly Met Val Ile Ala Asp Leu Asp
            260                 265                 270
Met Ala Leu Ile Thr Lys Arg Lys Arg Met Met Asp Ser Val Gly His
        275                 280                 285
Tyr Ala Arg Pro Glu Leu Leu Ser Leu Ala Ile Asn Asp Ala Pro Ala
    290                 295                 300
Thr Pro Ser Phe Gln Met Asn Glu His Arg Leu Lys Ser Val Gln Leu
305                 310                 315                 320
Pro Ile Ala Glu Glu Leu Lys Asn Asp Asn Lys Leu Ser Ser Gly Gln
                325                 330                 335

<210> 131
<211> 1011
<212> DNA
<213> Unknown

<220>
<223> Obtained from an environmental sample

<400> 131
atgtcagaaa agcgaattat taaagcggct gcagttcaaa tcacaccaga ttttgaatcg      60
catgatggaa ccgtaaagaa ggtttgtaat gtaattgatg aagcgggtgc taaaggtgta     120
cagatcattg tattccctga aacctttatt ccatattacc catatttttc tttcatcact     180
ccaccagtga ctgctggcgc ggagcatttg cggctctatg aaaaaagtgt cgtgatacct     240

ggtcccgtta ctcaagacat ttccgaacgt gcacgcatga ataatatggt tgttgtactt 300
ggtgtaaatg agcgtgataa cggcagtcta tataacaccc agattatttt tgatgctacc 360
ggtgagatgc ttctgaagag aagaaaaatc acacctacct atcatgagcg catgatttgg 420
gggcaaggag atgcttcagg cctgaaggtc gtcgatacgg ctattgggcg agtcggagca 480
ttggcatgct gggagcacta taaccctttg gctagataca gcctcatgac acagcatgaa 540
gaaattcact gtgctcaatt tccaggctcc atggttggtc agatcttcgc agatcaaatg 600
gatgtcacga ttcgtcatca tgccttggag tcaggttgct tcgtcatcaa ctccactggc 660
tggttaactg atgatcagat caaatctatc accgacgatc ccaaaatgca gaaagcttta 720
agaggtggtt gcaacacggc cattatttct ccagaaggga atcatttaac cgagcctttg 780
cgagaaggtg aaggcatggt gattgctgat cttgatatgg cactcatcac caaacgaaaa 840
agaatgatgg actcagttgg ccactacgcc agaccagaac tgttgagctt agcgatcaat 900
gatgctccgg ctactccttc attccagatg aacgaacatc gtcttaaatc agtgcaatta 960
cctatcgcag aggagcttaa aaatgacaac aagcttagca gtggacagta a 1011

<210> 132
<211> 336
<212> PRT
<213> Unknown

<220>
<223> Obtained from an environmental sample

<400> 132
Met Ser Glu Lys Arg Ile Ile Lys Ala Ala Ala Val Gln Ile Thr Pro
1               5                   10                  15
Asp Phe Glu Ser His Asp Gly Thr Val Lys Lys Val Cys Asn Val Ile
            20                  25                  30
Asp Glu Ala Gly Ala Lys Gly Val Gln Ile Ile Val Phe Pro Glu Thr
        35                  40                  45
Phe Ile Pro Tyr Tyr Pro Tyr Phe Ser Phe Ile Thr Pro Pro Val Thr
    50                  55                  60
Ala Gly Ala Glu His Leu Arg Leu Tyr Glu Lys Ser Val Val Ile Pro
65                  70                  75                  80
Gly Pro Val Thr Gln Asp Ile Ser Glu Arg Ala Arg Met Asn Asn Met
                85                  90                  95
Val Val Val Leu Gly Val Asn Glu Arg Asp Asn Gly Ser Leu Tyr Asn
            100                 105                 110
Thr Gln Ile Ile Phe Asp Ala Thr Gly Glu Met Leu Leu Lys Arg Arg
        115                 120                 125
Lys Ile Thr Pro Thr Tyr His Glu Arg Met Ile Trp Gly Gln Gly Asp
    130                 135                 140
Ala Ser Gly Leu Lys Val Val Asp Thr Ala Ile Gly Arg Val Gly Ala
145                 150                 155                 160
Leu Ala Cys Trp Glu His Tyr Asn Pro Leu Ala Arg Tyr Ser Leu Met
                165                 170                 175
Thr Gln His Glu Glu Ile His Cys Ala Gln Phe Pro Gly Ser Met Val
            180                 185                 190
Gly Gln Ile Phe Ala Asp Gln Met Asp Val Thr Ile Arg His His Ala
        195                 200                 205
Leu Glu Ser Gly Cys Phe Val Ile Asn Ser Thr Gly Trp Leu Thr Asp
    210                 215                 220
Asp Gln Ile Lys Ser Ile Thr Asp Asp Pro Lys Met Gln Lys Ala Leu
225                 230                 235                 240
Arg Gly Gly Cys Asn Thr Ala Ile Ile Ser Pro Glu Gly Asn His Leu
                245                 250                 255
Thr Glu Pro Leu Arg Glu Gly Glu Gly Met Val Ile Ala Asp Leu Asp
            260                 265                 270

Met Ala Leu Ile Thr Lys Arg Lys Arg Met Met Asp Ser Val Gly His
        275                 280                 285
Tyr Ala Arg Pro Glu Leu Leu Ser Leu Ala Ile Asn Asp Ala Pro Ala
    290                 295                 300
Thr Pro Ser Phe Gln Met Asn Glu His Arg Leu Lys Ser Val Gln Leu
305                 310                 315                 320
Pro Ile Ala Glu Glu Leu Lys Asn Asp Asn Lys Leu Ser Ser Gly Gln
                325                 330                 335

<210> 133
<211> 1026
<212> DNA
<213> Unknown

<220>
<223> Obtained from an environmental sample

<400> 133
atgtcgacca agcggatcgt acgcgccgct gccgttcagc tggcaccgga tctggagcgg      60
ccggagggca cactggagaa ggtttgcgcg gccatcgaca aggcggcggg ggacggtgtg     120
cagctcatcg tcttccccga gaccttcgta ccgtactacc cgtacttctc tttcgtgcgt     180
gcgccggtcg cgatgggtgc cgagcacatg cggttatacg agcgcgcggt agcggtgccc     240
ggtccagtaa cggccaccgt ggcggagcgg gcaaaagcgc acgcgatggt cgtcgtgctg     300
ggtgtaaacg agcgcgatca cggctcactg tataacgcgc aactgatctt cgacgagacc     360
ggccgtctcg tcctcaaacg ccgcaagatc actccgacct atcacgagcg catggtgtgg     420
gggcagggcg acggcagcgg ccttaaggtt gtagacaccg gtatcggcag gatcggagcc     480
ctcgcctgct gggagcacta caacccgctc gcgcgctatg cgctcatggc gcagcacgaa     540
gagattcatt gcgcgcagtt tccgggctcg atggtggggc cgatcttcgc ggatcagatc     600
gaggtcacga tccgccatca cgcgctggag tcgggctgct tcgtcgtcaa tgcgaccggc     660
tggctgacac ccgaacagat cgcgtcgatc acaccggacg cgggtctgca aaaggcaatc      720
agcgggggct gcaacaccgc gatcatctcg ccggagggcg tgcacctggc cccgccgttg     780
cgagaaggtg agggcatggt cgtggccgac ctcgacatgg cgctcatcac caaacgcaaa     840
cgcatgatgg attcggtggg tcactacgct cgcccggagt tgctcagcct gcgcatcgat     900
agccgcgccg cttcgccgat gtcgtcacaa atggaaatac ccgggagctt gcatgaaatc     960
accagccacg atgtccagcc agcaactgat gaccgagctc cagtcctccg gcttgaggtt    1020
ggctga                                                               1026

<210> 134
<211> 341
<212> PRT
<213> Unknown

<220>
<223> Obtained from an environmental sample

<400> 134
Met Ser Thr Lys Arg Ile Val Arg Ala Ala Ala Val Gln Leu Ala Pro
1               5                   10                  15
Asp Leu Glu Arg Pro Glu Gly Thr Leu Glu Lys Val Cys Ala Ala Ile
            20                  25                  30
Asp Lys Ala Ala Gly Asp Gly Val Gln Leu Ile Val Phe Pro Glu Thr
        35                  40                  45
Phe Val Pro Tyr Tyr Pro Tyr Phe Ser Phe Val Arg Ala Pro Val Ala
    50                  55                  60
Met Gly Ala Glu His Met Arg Leu Tyr Glu Arg Ala Val Ala Val Pro
65                  70                  75                  80
Gly Pro Val Thr Ala Thr Val Ala Glu Arg Ala Lys Ala His Ala Met

85                  90                  95
Val Val Val Leu Gly Val Asn Glu Arg Asp His Gly Ser Leu Tyr Asn
            100                 105                 110
Ala Gln Leu Ile Phe Asp Glu Thr Gly Arg Leu Val Leu Lys Arg Arg
        115                 120                 125
Lys Ile Thr Pro Thr Tyr His Glu Arg Met Val Trp Gly Gln Gly Asp
    130                 135                 140
Gly Ser Gly Leu Lys Val Val Asp Thr Gly Ile Gly Arg Ile Gly Ala
145                 150                 155                 160
Leu Ala Cys Trp Glu His Tyr Asn Pro Leu Ala Arg Tyr Ala Leu Met
                165                 170                 175
Ala Gln His Glu Glu Ile His Cys Ala Gln Phe Pro Gly Ser Met Val
            180                 185                 190
Gly Pro Ile Phe Ala Asp Gln Ile Glu Val Thr Ile Arg His His Ala
        195                 200                 205
Leu Glu Ser Gly Cys Phe Val Val Asn Ala Thr Gly Trp Leu Thr Pro
    210                 215                 220
Glu Gln Ile Ala Ser Ile Thr Pro Asp Ala Gly Leu Gln Lys Ala Ile
225                 230                 235                 240
Ser Gly Gly Cys Asn Thr Ala Ile Ile Ser Pro Glu Gly Val His Leu
                245                 250                 255
Ala Pro Pro Leu Arg Glu Gly Glu Gly Met Val Val Ala Asp Leu Asp
            260                 265                 270
Met Ala Leu Ile Thr Lys Arg Lys Arg Met Met Asp Ser Val Gly His
        275                 280                 285
Tyr Ala Arg Pro Glu Leu Leu Ser Leu Arg Ile Asp Ser Arg Ala Ala
    290                 295                 300
Ser Pro Met Ser Ser Gln Met Glu Ile Pro Gly Ser Leu His Glu Ile
305                 310                 315                 320
Thr Ser His Asp Val Gln Pro Ala Thr Asp Asp Arg Ala Pro Val Leu
                325                 330                 335
Arg Leu Glu Val Gly
            340

<210> 135
<211> 1011
<212> DNA
<213> Unknown

<220>
<223> Obtained from an environmental sample

<400> 135
atgtcagaca agcgaatcat taaagcggct gcagttcaaa tcactcctga ctttgactca      60
gcagatggaa ccgttaagaa agtgtgcaag gtaatcgatg aagcaggtgc aaagggagtt     120
caaattattg tattcccgga aaccttcatc ccttactacc catacttttc attcattaca     180
cctccagtca ctgctggcgc tgagcattta aagctttatg agaaaagtgt cgtgatacct     240
ggcccggtta cccaagcgat tgccgagcga gccagggtta atcagatggt tgtcgtgctt     300
ggtgtcaacg agcgagataa cggtagcctc tacaacacac aattgatctt tgataccaac     360
ggcgaactgc tacttaaaag aagaaaaatc acccctacct accatgaacg tatgatctgg     420
gggcaaggtg atgcatcagg tctcaaagta gttgaaacag agatcgcccg agtaggtgcc     480
ttggcttgtt gggaacacta caacccactg gccagatatg cactcatgac acagcatgaa     540
gaaattcact gtgcgcaatt cccaggctct atggttggcc agatatttgc cgatcagatg     600
gatgtcacta tccgacatca cgccttagag tcaggctgct tcgtcatcaa cgccactggc     660
tggctcaccg acgcgcaaat ccaatcgatt actgatgacc caaaaatgca aaaagcatta     720
cgtggcggct gcaacacagc catcatctcc cccgaagggg tgcacttaac agagccacta     780
cgtgaaggag aaggcatggt gattgccaat cttgatatgg cactcatcac aaaacgaaaa     840

agaatgatgg attcggtagg ccattattca agaccagaat tattaagcct ggcaattaac 900
gacaaaccag caactacaac attttcaatg actgaggggc gtactcaaac agagccattt 960
cgaatcgcag aggagttgaa aaatgacgac aagcttagca ctggaaacta a 1011

<210> 136
<211> 336
<212> PRT
<213> Unknown

<220>
<223> Obtained from an environmental sample

<400> 136
Met Ser Asp Lys Arg Ile Ile Lys Ala Ala Ala Val Gln Ile Thr Pro
1               5                   10                  15
Asp Phe Asp Ser Ala Asp Gly Thr Val Lys Lys Val Cys Lys Val Ile
            20                  25                  30
Asp Glu Ala Gly Ala Lys Gly Val Gln Ile Ile Val Phe Pro Glu Thr
        35                  40                  45
Phe Ile Pro Tyr Tyr Pro Tyr Phe Ser Phe Ile Thr Pro Pro Val Thr
    50                  55                  60
Ala Gly Ala Glu His Leu Lys Leu Tyr Glu Lys Ser Val Val Ile Pro
65                  70                  75                  80
Gly Pro Val Thr Gln Ala Ile Ala Glu Arg Ala Arg Val Asn Gln Met
                85                  90                  95
Val Val Val Leu Gly Val Asn Glu Arg Asp Asn Gly Ser Leu Tyr Asn
            100                 105                 110
Thr Gln Leu Ile Phe Asp Thr Asn Gly Glu Leu Leu Leu Lys Arg Arg
        115                 120                 125
Lys Ile Thr Pro Thr Tyr His Glu Arg Met Ile Trp Gly Gln Gly Asp
    130                 135                 140
Ala Ser Gly Leu Lys Val Val Glu Thr Glu Ile Ala Arg Val Gly Ala
145                 150                 155                 160
Leu Ala Cys Trp Glu His Tyr Asn Pro Leu Ala Arg Tyr Ala Leu Met
                165                 170                 175
Thr Gln His Glu Glu Ile His Cys Ala Gln Phe Pro Gly Ser Met Val
            180                 185                 190
Gly Gln Ile Phe Ala Asp Gln Met Asp Val Thr Ile Arg His His Ala
        195                 200                 205
Leu Glu Ser Gly Cys Phe Val Ile Asn Ala Thr Gly Trp Leu Thr Asp
    210                 215                 220
Ala Gln Ile Gln Ser Ile Thr Asp Asp Pro Lys Met Gln Lys Ala Leu
225                 230                 235                 240
Arg Gly Gly Cys Asn Thr Ala Ile Ile Ser Pro Glu Gly Val His Leu
                245                 250                 255
Thr Glu Pro Leu Arg Glu Gly Glu Gly Met Val Ile Ala Asn Leu Asp
            260                 265                 270
Met Ala Leu Ile Thr Lys Arg Lys Arg Met Met Asp Ser Val Gly His
        275                 280                 285
Tyr Ser Arg Pro Glu Leu Leu Ser Leu Ala Ile Asn Asp Lys Pro Ala
    290                 295                 300
Thr Thr Thr Phe Ser Met Thr Glu Gly Arg Thr Gln Thr Glu Pro Phe
305                 310                 315                 320
Arg Ile Ala Glu Glu Leu Lys Asn Asp Asp Lys Leu Ser Thr Gly Asn
                325                 330                 335

<210> 137

<211> 978
<212> DNA
<213> Unknown

<220>
<223> Obtained from an environmental sample

<400> 137
atggctattg tcaaggccgc ggcggtgcag atcagtccgg tgctctacag tcgcgccggc 60
acagtggaca aggtcgtcgc gaagatccgc gagctgggcc gacgaggggt cgagttcgcc 120
gtcttccccg agaccgtcat tccctactat ccctacttct ctttcgtgca gcccccctac 180
acccaggcca ccgaacacct gcgcctgctc gaggaatcgg tgaccgtgcc ctccgccgaa 240
accgacgcga tcgccaaggc cgctcgcgag gcgggcatgg tcgtctccat cggcgtcaac 300
gagcgcgacg gcggaaccat ctacaacacc caactcctct tcgacgccga cggcactctc 360
atccagcgcc gccgcaagat cacccccacc tatcacgaac gcatggtctg gggcagggga 420
gacggctcag gtctgcgcgc cgtcgacagt gcggtcggcc gcatcggcca gctcgcctgc 480
tgggagcact accagccact ggcccggtac gccctcatcg ctgacggcga gcagatccac 540
gccgcgatgt accccggcgc cttcggcggc gatctgttcg ccgagcagat cgaggtcaac 600
atccgccagc acgccctgga atccgccagc ttcgtcgtca acgccaccgc ctggctcgac 660
gccgatcagc aggcccagat cgccaaggac accggaggcc cggtcccggc cttctccggt 720
ggcttcttca ccgccatcgt cgaccccgaa ggccgtatca tcggcgaccc cctcaccagc 780
ggcgaaggcg aagtgatcgc cgacctcgat ctcgctctca tcaaccgccg caagcgcctc 840
atggacgcca gtggacacta ccagccgccc gaaattctta gcttcacatt gaccggtgca 900
ccggcgcctt atgtcaagag cgcggcgtgc cggggaaccc cgggtacgac cgtggccgag 960
gagggacggt ccgcttag 978

<210> 138
<211> 325
<212> PRT
<213> Unknown

<220>
<223> Obtained from an environmental sample

<400> 138
Met Ala Ile Val Lys Ala Ala Ala Val Gln Ile Ser Pro Val Leu Tyr
1               5                   10                  15
Ser Arg Ala Gly Thr Val Asp Lys Val Val Ala Lys Ile Arg Glu Leu
            20                  25                  30
Gly Arg Arg Gly Val Glu Phe Ala Val Phe Pro Glu Thr Val Ile Pro
        35                  40                  45
Tyr Tyr Pro Tyr Phe Ser Phe Val Gln Pro Pro Tyr Thr Gln Ala Thr
    50                  55                  60
Glu His Leu Arg Leu Leu Glu Glu Ser Val Thr Val Pro Ser Ala Glu
65                  70                  75                  80
Thr Asp Ala Ile Ala Lys Ala Ala Arg Glu Ala Gly Met Val Val Ser
                85                  90                  95
Ile Gly Val Asn Glu Arg Asp Gly Gly Thr Ile Tyr Asn Thr Gln Leu
            100                 105                 110
Leu Phe Asp Ala Asp Gly Thr Leu Ile Gln Arg Arg Arg Lys Ile Thr
        115                 120                 125
Pro Thr Tyr His Glu Arg Met Val Trp Gly Gln Gly Asp Gly Ser Gly
    130                 135                 140
Leu Arg Ala Val Asp Ser Ala Val Gly Arg Ile Gly Gln Leu Ala Cys
145                 150                 155                 160
Trp Glu His Tyr Gln Pro Leu Ala Arg Tyr Ala Leu Ile Ala Asp Gly
                165                 170                 175

Glu Gln Ile His Ala Ala Met Tyr Pro Gly Ala Phe Gly Gly Asp Leu
            180                 185                 190
Phe Ala Glu Gln Ile Glu Val Asn Ile Arg Gln His Ala Leu Glu Ser
        195                 200                 205
Ala Ser Phe Val Val Asn Ala Thr Ala Trp Leu Asp Ala Asp Gln Gln
    210                 215                 220
Ala Gln Ile Ala Lys Asp Thr Gly Gly Pro Val Pro Ala Phe Ser Gly
225                 230                 235                 240
Gly Phe Phe Thr Ala Ile Val Asp Pro Glu Gly Arg Ile Ile Gly Asp
                245                 250                 255
Pro Leu Thr Ser Gly Glu Gly Glu Val Ile Ala Asp Leu Asp Leu Ala
            260                 265                 270
Leu Ile Asn Arg Arg Lys Arg Leu Met Asp Ala Ser Gly His Tyr Gln
        275                 280                 285
Pro Pro Glu Ile Leu Ser Phe Thr Leu Thr Gly Ala Pro Ala Pro Tyr
    290                 295                 300
Val Lys Ser Ala Ala Cys Arg Gly Thr Pro Gly Thr Thr Val Ala Glu
305                 310                 315                 320
Glu Gly Arg Ser Ala
                325

<210> 139
<211> 999
<212> DNA
<213> Unknown

<220>
<223> Obtained from an environmental sample

<400> 139
atgaaaacaa cggttaccgt tgcctgcgtt caggccgccc ccgtatttat ggatttagaa      60
ggcaccgtag ataaaacaat caccctcatc tctgaagccg cacagaaagg cgcggagctc     120
atcgcttttc cggagacctg gatacccggt tacccgtggt tcttatggct gaactcgccc     180
gccacaaata tgcccctggt ttatcagtat catcagaact ctctggtgct ggacagtacc     240
caggcgaagc gaattgcgga tgcggcacgg cagaataaca tcactgtcgc tctgggcttc     300
agcgaacgcg atcatggaag cctctatatc gcacagtggc tgattggcag cgacggggag     360
accattggca tccggcgcaa gctcaaggcc acgcacgtgg agcgtacgct gttcggcgaa     420
agcgacggct cctccctgac cacctgggag acacctctgg gtaacgtcgg ggccctctgc     480
tgctgggagc acctgcagcc gctgtcccgc tatgcaatgt attcccagca tgaggagatc     540
cacatcgctg cctggcccag tttcagtctc tacaccagtg caacggccgc actgggtcct     600
gacgtcaata cggcggcttc acgcctctat gccgcggagg ggcagtgctt cgtgatagcc     660
ccgtgtgccg tggtttctga tgaaatgatt gatttactct gtcctgatga tgaccggaga     720
gcgttactca gtgccggagg gggacatgcc cgtatttacg gcccggacgg aagagaactc     780
gtcacccctc tcggggaaaa tgaggaagga ctgcttatcg ctgagctcga ctctgctgcg     840
attacctttg ccaaactggc ggcagacccg gttggccact attcccgtcc tgacgtgacc     900
cgcctccttt ttaatccttc agccaacaag actgtgatta aacgacattc gcctcctgag     960
ttaattgccg agcagactgc agaagaagag gaggagtag                            999

<210> 140
<211> 332
<212> PRT
<213> Unknown

<220>
<223> Obtained from an environmental sample

<400> 140

Met Lys Thr Thr Val Thr Val Ala Cys Val Gln Ala Ala Pro Val Phe
1               5                   10                  15
Met Asp Leu Glu Gly Thr Val Asp Lys Thr Ile Thr Leu Ile Ser Glu
            20                  25                  30
Ala Ala Gln Lys Gly Ala Glu Leu Ile Ala Phe Pro Glu Thr Trp Ile
        35                  40                  45
Pro Gly Tyr Pro Trp Phe Leu Trp Leu Asn Ser Pro Ala Thr Asn Met
    50                  55                  60
Pro Leu Val Tyr Gln Tyr His Gln Asn Ser Leu Val Leu Asp Ser Thr
65                  70                  75                  80
Gln Ala Lys Arg Ile Ala Asp Ala Ala Arg Gln Asn Asn Ile Thr Val
                85                  90                  95
Ala Leu Gly Phe Ser Glu Arg Asp His Gly Ser Leu Tyr Ile Ala Gln
            100                 105                 110
Trp Leu Ile Gly Ser Asp Gly Glu Thr Ile Gly Ile Arg Arg Lys Leu
        115                 120                 125
Lys Ala Thr His Val Glu Arg Thr Leu Phe Gly Glu Ser Asp Gly Ser
    130                 135                 140
Ser Leu Thr Thr Trp Glu Thr Pro Leu Gly Asn Val Gly Ala Leu Cys
145                 150                 155                 160
Cys Trp Glu His Leu Gln Pro Leu Ser Arg Tyr Ala Met Tyr Ser Gln
                165                 170                 175
His Glu Glu Ile His Ile Ala Ala Trp Pro Ser Phe Ser Leu Tyr Thr
            180                 185                 190
Ser Ala Thr Ala Ala Leu Gly Pro Asp Val Asn Thr Ala Ala Ser Arg
        195                 200                 205
Leu Tyr Ala Ala Glu Gly Gln Cys Phe Val Ile Ala Pro Cys Ala Val
    210                 215                 220
Val Ser Asp Glu Met Ile Asp Leu Leu Cys Pro Asp Asp Asp Arg Arg
225                 230                 235                 240
Ala Leu Leu Ser Ala Gly Gly Gly His Ala Arg Ile Tyr Gly Pro Asp
                245                 250                 255
Gly Arg Glu Leu Val Thr Pro Leu Gly Glu Asn Glu Glu Gly Leu Leu
            260                 265                 270
Ile Ala Glu Leu Asp Ser Ala Ala Ile Thr Phe Ala Lys Leu Ala Ala
        275                 280                 285
Asp Pro Val Gly His Tyr Ser Arg Pro Asp Val Thr Arg Leu Leu Phe
    290                 295                 300
Asn Pro Ser Ala Asn Lys Thr Val Ile Lys Arg His Ser Pro Pro Glu
305                 310                 315                 320
Leu Ile Ala Glu Gln Thr Ala Glu Glu Glu Glu Glu
                325                 330

<210> 141
<211> 1026
<212> DNA
<213> Unknown

<220>
<223> Obtained from an environmental sample

<400> 141
atggtgttca aggcagcgac tgttcatgca gctccggtat tcatggacaa ggaagcgtcg      60
atagataagg ctatcgacct catcaagaag gccggtcagg aagggattaa gcttctggtt     120
tttccggaaa cgtttattcc gggctatccg tattttatcg aatgctatcc gccgcttgcg     180
caggtggaag cgctcgccca gtacactgac gcttccgtgg agatcgacgg cccggaagtc     240
acccggcttc agcaggtagc caaggcggca ggcgttgcag tcgtcatggg catcagcgaa     300

cgaatggctg agacccgaac ctgcttcaac tcgcaggtgt tcattgacgt cgacggcacg     360
ctgctcggcg tgcatcgcaa gctgcagccg acttatgccg agcgcaaggt atgggcacag     420
ggcggtggtt atacgctgag gacctacaag agctcgcttg gcgtgctcgg cggtcttgcc     480
tgctgggagc acacgatgaa cctcgcgcgg caggccctga tcatgcagag cgagcagatc     540
catgcggctg catggcccgg actatcgacg atgcgaggtt tcgagcccgt ggccgatatc     600
cagatcgacg ccatgatgaa gactcacgcg cttaccgcac agtgctgggt gctttcggcc     660
ggcaatcccg tcgaccggac ctgcctcgac tggatggaaa agaacatcgg accgcaggat     720
tacgtcaccg agggcggcgg atggagcgcc gttatccatc cgttcaacag ctatctcggc     780
ggccctcaca cgggccttga ggaaaagctg gtcgtcggcg agatcaatct ggacgatctc     840
aagttcgtca aagtctggct cgacagcaaa gggcactatg ctcggccgga aatcctgaaa     900
cttggcgtca accaaaagca gatttggcct gatgaacatt tgctggcgcg gcaggatgtg     960
accgagttgc tggaggcgga tatcatcgaa taccccttgc aactgttgca agaccgcgcg    1020
caatag                                                              1026

<210> 142
<211> 341
<212> PRT
<213> Unknown

<220>
<223> Obtained from an environmental sample

<400> 142
Met Val Phe Lys Ala Ala Thr Val His Ala Ala Pro Val Phe Met Asp
1               5                   10                  15
Lys Glu Ala Ser Ile Asp Lys Ala Ile Asp Leu Ile Lys Lys Ala Gly
            20                  25                  30
Gln Glu Gly Ile Lys Leu Leu Val Phe Pro Glu Thr Phe Ile Pro Gly
        35                  40                  45
Tyr Pro Tyr Phe Ile Glu Cys Tyr Pro Pro Leu Ala Gln Val Glu Ala
    50                  55                  60
Leu Ala Gln Tyr Thr Asp Ala Ser Val Glu Ile Asp Gly Pro Glu Val
65                  70                  75                  80
Thr Arg Leu Gln Gln Val Ala Lys Ala Ala Gly Val Ala Val Val Met
                85                  90                  95
Gly Ile Ser Glu Arg Met Ala Glu Thr Arg Thr Cys Phe Asn Ser Gln
            100                 105                 110
Val Phe Ile Asp Val Asp Gly Thr Leu Leu Gly Val His Arg Lys Leu
        115                 120                 125
Gln Pro Thr Tyr Ala Glu Arg Lys Val Trp Ala Gln Gly Gly Gly Tyr
    130                 135                 140
Thr Leu Arg Thr Tyr Lys Ser Ser Leu Gly Val Leu Gly Gly Leu Ala
145                 150                 155                 160
Cys Trp Glu His Thr Met Asn Leu Ala Arg Gln Ala Leu Ile Met Gln
                165                 170                 175
Ser Glu Gln Ile His Ala Ala Ala Trp Pro Gly Leu Ser Thr Met Arg
            180                 185                 190
Gly Phe Glu Pro Val Ala Asp Ile Gln Ile Asp Ala Met Met Lys Thr
        195                 200                 205
His Ala Leu Thr Ala Gln Cys Trp Val Leu Ser Ala Gly Asn Pro Val
    210                 215                 220
Asp Arg Thr Cys Leu Asp Trp Met Glu Lys Asn Ile Gly Pro Gln Asp
225                 230                 235                 240
Tyr Val Thr Glu Gly Gly Gly Trp Ser Ala Val Ile His Pro Phe Asn
                245                 250                 255
Ser Tyr Leu Gly Gly Pro His Thr Gly Leu Glu Glu Lys Leu Val Val
            260                 265                 270

Gly Glu Ile Asn Leu Asp Asp Leu Lys Phe Val Lys Val Trp Leu Asp
        275                 280                 285
Ser Lys Gly His Tyr Ala Arg Pro Glu Ile Leu Lys Leu Gly Val Asn
    290                 295                 300
Gln Lys Gln Ile Trp Pro Asp Glu His Leu Leu Ala Arg Gln Asp Val
305                 310                 315                 320
Thr Glu Leu Leu Glu Ala Asp Ile Ile Glu Tyr Pro Leu Gln Leu Leu
                325                 330                 335
Gln Asp Arg Ala Gln
            340

<210> 143
<211> 1122
<212> DNA
<213> Unknown

<220>
<223> Obtained from an environmental sample

<400> 143
atgacgatca ttgcaggcgc ggttcatgcg gcgccggtat tcatggatgt cgatgccact      60
atcgacaagg catgcgaaat cattcgcaag gcaggcaaag acggaatcga gcttctcgtc     120
ttccctgagg ttttcgtacc cggctacccc tacttcatcg agtgctatcc gaccttgaac     180
caaaccgctg cgctggccgc ctatacggat gcctcgatcg aggttccagg cccggaagtc     240
cggcgcttgc aggtggccgc acatcaggcc ggcgtgatgg ttgtgatggg cgtgagcgag     300
cgtctgcgcg gatctcgcac ctgcttcaac agccaggtgt tcatcgaccg tgacggcacc     360
ttgctgggcg tgcaccgcaa actccagccg acctatgtcg agcgcatcgt ctggggccag     420
ggcggcggac acaccctcaa ggtattcgac agcacactgg gcaaggtggg cggactggcc     480
tgctgggagc acacgatgaa cctcgcgcgc catgcgttga tcgcccaggg tatccagatc     540
catgccgccg cctggcctgg gctttcgaca atggccgggt tcgaagcggt ggctgacgtc     600
cagatcgacg cgatgatgaa aactcatgcg ttgagcgcgc aatgctttgt cgtatcggcc     660
gcaaaccctg tggatcagac ctgcctggag tggatggaga aacacctcgg cccgcagcaa     720
ctcgttaccg ccggcggagg ctggtcggca atcgtccatc ctttctgtgg ttatatcgcc     780
gcccctcaca ccggtgccga ggagaaggtt ctggtaggcg aaatcaatct ggacgacctc     840
aagcaggtca aggtatgggt tgattccgca ggtcattatg cgcgcccgga agtcgtgcaa     900
ttgcgcgacg ccctggagag ccgtggcaat tatcgcgttg cgctgacccg cgacgccgac     960
accttcgtgc cgctggaaga ccgcgtgcgc tttgcgcgcc agcagaacgc cgacctcttc    1020
atctcgatcc acgccgacgc caacgccaac cacgatgcgc gcggggctgg cttcacttcg    1080
aaggttgaaa acctttccac gggcatttta ccaggcgatt ga                       1122

<210> 144
<211> 373
<212> PRT
<213> Unknown

<220>
<223> Obtained from an environmental sample

<400> 144
Met Thr Ile Ile Ala Gly Ala Val His Ala Ala Pro Val Phe Met Asp
 1               5                  10                  15
Val Asp Ala Thr Ile Asp Lys Ala Cys Glu Ile Ile Arg Lys Ala Gly
            20                  25                  30
Lys Asp Gly Ile Glu Leu Leu Val Phe Pro Glu Val Phe Val Pro Gly
        35                  40                  45
Tyr Pro Tyr Phe Ile Glu Cys Tyr Pro Thr Leu Asn Gln Thr Ala Ala
    50                  55                  60

Leu Ala Ala Tyr Thr Asp Ala Ser Ile Glu Val Pro Gly Pro Glu Val
65                  70                  75                  80
Arg Arg Leu Gln Val Ala Ala His Gln Ala Gly Val Met Val Val Met
                85                  90                  95
Gly Val Ser Glu Arg Leu Arg Gly Ser Arg Thr Cys Phe Asn Ser Gln
            100                 105                 110
Val Phe Ile Asp Arg Asp Gly Thr Leu Leu Gly Val His Arg Lys Leu
        115                 120                 125
Gln Pro Thr Tyr Val Glu Arg Ile Val Trp Gly Gln Gly Gly Gly His
    130                 135                 140
Thr Leu Lys Val Phe Asp Ser Thr Leu Gly Lys Val Gly Gly Leu Ala
145                 150                 155                 160
Cys Trp Glu His Thr Met Asn Leu Ala Arg His Ala Leu Ile Ala Gln
                165                 170                 175
Gly Ile Gln Ile His Ala Ala Ala Trp Pro Gly Leu Ser Thr Met Ala
            180                 185                 190
Gly Phe Glu Ala Val Ala Asp Val Gln Ile Asp Ala Met Met Lys Thr
        195                 200                 205
His Ala Leu Ser Ala Gln Cys Phe Val Val Ser Ala Ala Asn Pro Val
    210                 215                 220
Asp Gln Thr Cys Leu Glu Trp Met Glu Lys His Leu Gly Pro Gln Gln
225                 230                 235                 240
Leu Val Thr Ala Gly Gly Gly Trp Ser Ala Ile Val His Pro Phe Cys
                245                 250                 255
Gly Tyr Ile Ala Ala Pro His Thr Gly Ala Glu Glu Lys Val Leu Val
            260                 265                 270
Gly Glu Ile Asn Leu Asp Asp Leu Lys Gln Val Lys Val Trp Val Asp
        275                 280                 285
Ser Ala Gly His Tyr Ala Arg Pro Glu Val Val Gln Leu Arg Asp Ala
    290                 295                 300
Leu Glu Ser Arg Gly Asn Tyr Arg Val Ala Leu Thr Arg Asp Ala Asp
305                 310                 315                 320
Thr Phe Val Pro Leu Glu Asp Arg Val Arg Phe Ala Arg Gln Gln Asn
                325                 330                 335
Ala Asp Leu Phe Ile Ser Ile His Ala Asp Ala Asn Ala Asn His Asp
            340                 345                 350
Ala Arg Gly Ala Gly Phe Thr Ser Lys Val Glu Asn Leu Ser Thr Gly
        355                 360                 365
Ile Leu Pro Gly Asp
    370

<210> 145
<211> 1014
<212> DNA
<213> Unknown

<220>
<223> Obtained from an environmental sample

<400> 145
atgggcatca cccacccgaa ctacaaggtc gcagtggtcc aggctgcgcc ggtctggttg      60
aacctcgagg caacggtcga gaagacaatc aggtatattg aagaggcggc caaggctgga     120
gcgaagctga tagcgtttcc ggaaacctgg attccgggct atccatggca catttggatc     180
ggaacgcccg catgggcaat cggtaagggc ttcgtccagc gctatttcga caactcgctc     240
agctatgaca gcccgctcgc gcggcagatc gctgacgccg cagcaaagag caagatcacg     300
gttgttctcg gcctctccga gcgcgacggt ggaagcctat acatcgcgca atggctgatc     360
ggaccagatg gcgagaccat cgcgaagcgg cgcaagctgc gtccgaccca cgtcgagcgc     420

acggtgttcg gtgacggtga cggcagccac atcgccgtgc atgaccgatc cgatctgggc 480
cggctcgggg cgttgtgctg ctgggagcac gtgcagccgt tgacgaaatt cgcgatgtac 540
gcgcagaacg agcaggttca cgtggcagca tggccgagct tctcgatgta cgaacccttt 600
gcgcatgcgc tgggttggga gacgaacaac gcggtcagca aggtctacgc ggtcgaggga 660
tcgtgcttcg tgctcgctcc ctgtgccgtt atttcgcaag cgatggtgga cgagatgtgc 720
gacactcccg acaagcgcga gcttgttcac gccggcggcg gccacgcggt gatttacggc 780
cctgacggaa gcccgctcgc agaaaagctc ggggaaaacg aagaggggct tctctacgcg 840
acggtcaatc ttgctgcgat cggggttgcc aagaatgccg cggatccggc cgggcactat 900
tcgcgtccgg acgttctaag gctgctattc aacaagagcc cggcccgaag agtggagcat 960
tttgcgctgc cgcacgagca gctcgagatc ggggcaggcc cgtctggcga ctga 1014

<210> 146
<211> 337
<212> PRT
<213> Unknown

<220>
<223> Obtained from an environmental sample

<400> 146
Met Gly Ile Thr His Pro Asn Tyr Lys Val Ala Val Val Gln Ala Ala
1               5                   10                  15
Pro Val Trp Leu Asn Leu Glu Ala Thr Val Glu Lys Thr Ile Arg Tyr
            20                  25                  30
Ile Glu Glu Ala Ala Lys Ala Gly Ala Lys Leu Ile Ala Phe Pro Glu
        35                  40                  45
Thr Trp Ile Pro Gly Tyr Pro Trp His Ile Trp Ile Gly Thr Pro Ala
    50                  55                  60
Trp Ala Ile Gly Lys Gly Phe Val Gln Arg Tyr Phe Asp Asn Ser Leu
65                  70                  75                  80
Ser Tyr Asp Ser Pro Leu Ala Arg Gln Ile Ala Asp Ala Ala Ala Lys
                85                  90                  95
Ser Lys Ile Thr Val Val Leu Gly Leu Ser Glu Arg Asp Gly Gly Ser
            100                 105                 110
Leu Tyr Ile Ala Gln Trp Leu Ile Gly Pro Asp Gly Glu Thr Ile Ala
        115                 120                 125
Lys Arg Arg Lys Leu Arg Pro Thr His Val Glu Arg Thr Val Phe Gly
    130                 135                 140
Asp Gly Asp Gly Ser His Ile Ala Val His Asp Arg Ser Asp Leu Gly
145                 150                 155                 160
Arg Leu Gly Ala Leu Cys Cys Trp Glu His Val Gln Pro Leu Thr Lys
                165                 170                 175
Phe Ala Met Tyr Ala Gln Asn Glu Gln Val His Val Ala Ala Trp Pro
            180                 185                 190
Ser Phe Ser Met Tyr Glu Pro Phe Ala His Ala Leu Gly Trp Glu Thr
        195                 200                 205
Asn Asn Ala Val Ser Lys Val Tyr Ala Val Glu Gly Ser Cys Phe Val
    210                 215                 220
Leu Ala Pro Cys Ala Val Ile Ser Gln Ala Met Val Asp Glu Met Cys
225                 230                 235                 240
Asp Thr Pro Asp Lys Arg Glu Leu Val His Ala Gly Gly Gly His Ala
                245                 250                 255
Val Ile Tyr Gly Pro Asp Gly Ser Pro Leu Ala Glu Lys Leu Gly Glu
            260                 265                 270
Asn Glu Glu Gly Leu Leu Tyr Ala Thr Val Asn Leu Ala Ala Ile Gly
        275                 280                 285
Val Ala Lys Asn Ala Ala Asp Pro Ala Gly His Tyr Ser Arg Pro Asp

290                 295                 300
Val Leu Arg Leu Leu Phe Asn Lys Ser Pro Ala Arg Arg Val Glu His
305                 310                 315                 320
Phe Ala Leu Pro His Glu Gln Leu Glu Ile Gly Ala Gly Pro Ser Gly
                325                 330                 335
Asp

<210> 147
<211> 1098
<212> DNA
<213> Unknown

<220>
<223> Obtained from an environmental sample

<400> 147
atgacccagc acgagaccac tgcccggagg ctggcagctg tgcatgccgc gcctgtgttc      60
atggacaccg acgcgaccat cgacaaggtg atcggcttcg tcgaacaggc cggccgcgaa     120
ggcatcgaac tcctggtgtt ccccgagacc ttcgtgcctg gttaccccta ctggatcgag     180
tgctatccgc cgctgcagca ggtggccgcc aacgcgcagt acacggacgc ctccgtcgag     240
gtgcctggtc cggagatcaa gcgggtgcag gcggcctgtg cccgcgctgg cgtcgaagtc     300
gtcctcggcg tcagcgagcg actcaggggt accaggacat gcttcaactc ccaggtgttc     360
atcgacgccg acgggagcct gctcggcgtg caccgcaagc tgcagccgac gtacgtggag     420
cgcatcgtgt gggcccaggg cggaggcgcg accctgtcgg tgttcggctc ccgctccggc     480
cggatcggcg gtctggcctg ctgggagcac acgatgaacc tggctcgtca ggcactgctt     540
gagcaggagc agcagatcca cgcggcggcg tggcctgccc tgtcgacgat ggcggggttc     600
gagaccgtcg cggacgccca gatcgaggcc atgatgaaga cccatgcgct cacggcacag     660
gtgttcgtca tctgcgcgtc caacccggtc gacggcactt gcctggaatg gatgcgggac     720
aacctcggtg aacagaagtt cgtgaccgcc ggagggggct ggtccgcggt catccacccc     780
ttcaactcct tcctcggcgg gccgcatacc ggtttggagg agaagctcgt cagcgcgacg     840
atcgacttct ccgacatccg cttggtcaag gcctgggttg attcgaaggg gcactacgcg     900
cggcccgagg tcctgcgact cgcggtcgac cgcaagccac tgtggcacga cgagtgcgag     960
gtgccgggac aggcgcaggt acgcacccgc gctgcttctc tggcagtgca ggagcacccg    1020
gtggtgctgc ctcagggggc ggcgcggccc gctccgcaag actgggacac ctctgcggcg    1080
caggagctga cttcctga                                                  1098

<210> 148
<211> 365
<212> PRT
<213> Unknown

<220>
<223> Obtained from an environmental sample

<400> 148
Met Thr Gln His Glu Thr Thr Ala Arg Arg Leu Ala Ala Val His Ala
 1               5                  10                  15
Ala Pro Val Phe Met Asp Thr Asp Ala Thr Ile Asp Lys Val Ile Gly
            20                  25                  30
Phe Val Glu Gln Ala Gly Arg Glu Gly Ile Glu Leu Leu Val Phe Pro
        35                  40                  45
Glu Thr Phe Val Pro Gly Tyr Pro Tyr Trp Ile Glu Cys Tyr Pro Pro
    50                  55                  60
Leu Gln Gln Val Ala Ala Asn Ala Gln Tyr Thr Asp Ala Ser Val Glu
65                  70                  75                  80
Val Pro Gly Pro Glu Ile Lys Arg Val Gln Ala Ala Cys Ala Arg Ala

                    85                  90                  95
Gly Val Glu Val Val Leu Gly Val Ser Glu Arg Leu Arg Gly Thr Arg
            100                 105                 110
Thr Cys Phe Asn Ser Gln Val Phe Ile Asp Ala Asp Gly Ser Leu Leu
        115                 120                 125
Gly Val His Arg Lys Leu Gln Pro Thr Tyr Val Glu Arg Ile Val Trp
    130                 135                 140
Ala Gln Gly Gly Gly Ala Thr Leu Ser Val Phe Gly Ser Arg Ser Gly
145                 150                 155                 160
Arg Ile Gly Gly Leu Ala Cys Trp Glu His Thr Met Asn Leu Ala Arg
                165                 170                 175
Gln Ala Leu Leu Glu Gln Glu Gln Gln Ile His Ala Ala Ala Trp Pro
            180                 185                 190
Ala Leu Ser Thr Met Ala Gly Phe Glu Thr Val Ala Asp Ala Gln Ile
        195                 200                 205
Glu Ala Met Met Lys Thr His Ala Leu Thr Ala Gln Val Phe Val Ile
    210                 215                 220
Cys Ala Ser Asn Pro Val Asp Gly Thr Cys Leu Glu Trp Met Arg Asp
225                 230                 235                 240
Asn Leu Gly Glu Gln Lys Phe Val Thr Ala Gly Gly Gly Trp Ser Ala
                245                 250                 255
Val Ile His Pro Phe Asn Ser Phe Leu Gly Gly Pro His Thr Gly Leu
            260                 265                 270
Glu Glu Lys Leu Val Ser Ala Thr Ile Asp Phe Ser Asp Ile Arg Leu
        275                 280                 285
Val Lys Ala Trp Val Asp Ser Lys Gly His Tyr Ala Arg Pro Glu Val
    290                 295                 300
Leu Arg Leu Ala Val Asp Arg Lys Pro Leu Trp His Asp Glu Cys Glu
305                 310                 315                 320
Val Pro Gly Gln Ala Gln Val Arg Thr Arg Ala Ala Ser Leu Ala Val
                325                 330                 335
Gln Glu His Pro Val Val Leu Pro Gln Gly Ala Ala Arg Pro Ala Pro
            340                 345                 350
Gln Asp Trp Asp Thr Ser Ala Ala Gln Glu Leu Thr Ser
        355                 360                 365

<210> 149
<211> 942
<212> DNA
<213> Unknown

<220>
<223> Obtained from an environmental sample

<400> 149
atgacgaagc ttgagaaggt ggtcgcggcg gcggtccagg cgacgccgga gttcctcgac      60
cgcgaggcga ccgtcgagaa ggccgtgcgg ctgatcaagg aagcggccgg ggagggcgcc     120
ggcctgatcg tgttccccga gacgttcatc ccgacgtacc cggactgggt ctggcgcgcg     180
ccggcctggg acggcccatc cgcggacctg tacgcaatgc tgctggagaa cgcggtggag     240
atccccgggc cggtgacgga gaccctgggg aaggcggcga agcaggccaa ggccttcgtg     300
tcgatgggcg tcaacgagcg cgagccgggc ggcgggacga tctacaacac gcaggtcacg     360
ttcggacccg acgggagcgt gctcggcaag caccgcaagc tgatgccgac cggcggcgag     420
cgcctggtgt gggggatggg cgacgggtcg atgctccagg tctatgacac gccgttcggc     480
cgcctgggcg ggctgatctg ctgggagaac tacatgccgc tcgcgcgcta ctcgatgtac     540
gccaagggcg tggacgtcta cgttgcgccg acgtgggaca acagcgacat gtgggtggcg     600
acgctccgcc acatcgccaa ggaggggcgg ctgtacgtga tcggcgtggc gccgctgctg     660
cgcgggtcgg acgtccccga cgacgtgccg gggaaggccg agctgtgggg cggcgatgac     720

gactggatgt cgcgcggctt ctccaccatc gtcgcgccgg gcggcgaggt gctggccggt 780
ccgctgacgg aggaggaagg catcctctac gcggagatcg acccggcgag agcccgttcg 840
tcacggcacc agttcgatcc ggtggggcac tactcgcgcc ccgacgtgtt tcggctcgtc 900
gtggacgagt cgcccaagcc ccagacgtcc ggcccgggct ag 942

<210> 150
<211> 313
<212> PRT
<213> Unknown

<220>
<223> Obtained from an environmental sample

<400> 150
Met Thr Lys Leu Glu Lys Val Val Ala Ala Ala Val Gln Ala Thr Pro
1               5                   10                  15
Glu Phe Leu Asp Arg Glu Ala Thr Val Glu Lys Ala Val Arg Leu Ile
            20                  25                  30
Lys Glu Ala Ala Gly Glu Gly Ala Gly Leu Ile Val Phe Pro Glu Thr
        35                  40                  45
Phe Ile Pro Thr Tyr Pro Asp Trp Val Trp Arg Ala Pro Ala Trp Asp
    50                  55                  60
Gly Pro Ser Ala Asp Leu Tyr Ala Met Leu Leu Glu Asn Ala Val Glu
65                  70                  75                  80
Ile Pro Gly Pro Val Thr Glu Thr Leu Gly Lys Ala Ala Lys Gln Ala
                85                  90                  95
Lys Ala Phe Val Ser Met Gly Val Asn Glu Arg Glu Pro Gly Gly Gly
            100                 105                 110
Thr Ile Tyr Asn Thr Gln Val Thr Phe Gly Pro Asp Gly Ser Val Leu
        115                 120                 125
Gly Lys His Arg Lys Leu Met Pro Thr Gly Gly Glu Arg Leu Val Trp
    130                 135                 140
Gly Met Gly Asp Gly Ser Met Leu Gln Val Tyr Asp Thr Pro Phe Gly
145                 150                 155                 160
Arg Leu Gly Gly Leu Ile Cys Trp Glu Asn Tyr Met Pro Leu Ala Arg
                165                 170                 175
Tyr Ser Met Tyr Ala Lys Gly Val Asp Val Tyr Val Ala Pro Thr Trp
            180                 185                 190
Asp Asn Ser Asp Met Trp Val Ala Thr Leu Arg His Ile Ala Lys Glu
        195                 200                 205
Gly Arg Leu Tyr Val Ile Gly Val Ala Pro Leu Leu Arg Gly Ser Asp
    210                 215                 220
Val Pro Asp Asp Val Pro Gly Lys Ala Glu Leu Trp Gly Gly Asp Asp
225                 230                 235                 240
Asp Trp Met Ser Arg Gly Phe Ser Thr Ile Val Ala Pro Gly Gly Glu
                245                 250                 255
Val Leu Ala Gly Pro Leu Thr Glu Glu Glu Gly Ile Leu Tyr Ala Glu
            260                 265                 270
Ile Asp Pro Ala Arg Ala Arg Ser Ser Arg His Gln Phe Asp Pro Val
        275                 280                 285
Gly His Tyr Ser Arg Pro Asp Val Phe Arg Leu Val Val Asp Glu Ser
    290                 295                 300
Pro Lys Pro Gln Thr Ser Gly Pro Gly
305                 310

<210> 151
<211> 993

<212> DNA
<213> Unknown

<220>
<223> Obtained from an environmental sample

<400> 151
atgagagtcg ttaaagccgc cgcggtccaa ctgaaaccag tcctttatag ccgtgaggga 60
acagtcgata acgtcgtcaa gaagatccac gagctgggcc aacaaggagt gcagttcgca 120
acgttcccgg aaaccgtggt gccttactat ccgtactttt cgatcgtgca gtccggctat 180
caaatccttg ccggcggtga gttcctaaag ctgcttgatc agtcagtgac cgtgccatct 240
cttgccaccg aagcgatcgg cgaggcctgc aggcaagcgg gcgtcgttgt ctccatcggc 300
gtcaacgagc gtgacggggg aactctgtac aatacgcaac ttctctttga tgccgacggc 360
acgttgattc aaagacgacg caagatcacg cccacccatt acgagcgcat ggtctggggc 420
cagggcgatg gctcaggttt acgggcggtt gacagcaagg tcgcgcgcat tggtcaactg 480
gcttgttttg agcactacaa cccgcttgcg cgttacgcca tgatggccga tggcgagcaa 540
atccactctg cgatgttccc gggctccatg ttcggcgatg cgttttcaga gaaggtggaa 600
atcaacgtaa ggcagcatgc aatggagtct ggatgctttg tcgtctgcgc tacggcctgg 660
ctggatgccg accaacaggc acaaatcatg aaggacacag gctgcgagat cggtccgatc 720
tcgggcggtt gcttcaccgc tatcgtgaca cccgacggga cgctgatagg cgaacccatc 780
cactcgggcg aaggcgtttg tattgccgac ctcgatttca agctcatcga caagcggaag 840
cacgtggtgg acacgcgcgg ccactacagc cggccagaat tgctcagcct cctaattgat 900
cggactccca cggcacacat acacgaacgg aacgagcaac cgaagtcggc cgttgagcaa 960
gactcgcaga atgtattcac cgctattgct taa 993

<210> 152
<211> 330
<212> PRT
<213> Unknown

<220>
<223> Obtained from an environmental sample

<400> 152
Met Arg Val Val Lys Ala Ala Ala Val Gln Leu Lys Pro Val Leu Tyr
1               5                   10                  15
Ser Arg Glu Gly Thr Val Asp Asn Val Val Lys Lys Ile His Glu Leu
            20                  25                  30
Gly Gln Gln Gly Val Gln Phe Ala Thr Phe Pro Glu Thr Val Val Pro
        35                  40                  45
Tyr Tyr Pro Tyr Phe Ser Ile Val Gln Ser Gly Tyr Gln Ile Leu Ala
    50                  55                  60
Gly Gly Glu Phe Leu Lys Leu Leu Asp Gln Ser Val Thr Val Pro Ser
65                  70                  75                  80
Leu Ala Thr Glu Ala Ile Gly Glu Ala Cys Arg Gln Ala Gly Val Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Tyr Glu Arg Met Val Trp Gly Gln Gly Asp Gly
    130                 135                 140
Ser Gly Leu Arg Ala Val Asp Ser Lys Val Ala Arg Ile Gly Gln Leu
145                 150                 155                 160
Ala Cys Phe Glu His Tyr Asn Pro Leu Ala Arg Tyr Ala Met Met Ala
                165                 170                 175
Asp Gly Glu Gln Ile His Ser Ala Met Phe Pro Gly Ser Met Phe Gly

                180                 185                 190
Asp Ala Phe Ser Glu Lys Val Glu Ile Asn Val Arg Gln His Ala Met
        195                 200                 205
Glu Ser Gly Cys Phe Val Val Cys Ala Thr Ala Trp Leu Asp Ala Asp
    210                 215                 220
Gln Gln Ala Gln Ile Met Lys Asp Thr Gly Cys Glu Ile Gly Pro Ile
225                 230                 235                 240
Ser Gly Gly Cys Phe Thr Ala Ile Val Thr Pro Asp Gly Thr Leu Ile
                245                 250                 255
Gly Glu Pro Ile His Ser Gly Glu Gly Val Cys Ile Ala Asp Leu Asp
            260                 265                 270
Phe Lys Leu Ile Asp Lys Arg Lys His Val Val Asp Thr Arg Gly His
        275                 280                 285
Tyr Ser Arg Pro Glu Leu Leu Ser Leu Leu Ile Asp Arg Thr Pro Thr
    290                 295                 300
Ala His Ile His Glu Arg Asn Glu Gln Pro Lys Ser Ala Val Glu Gln
305                 310                 315                 320
Asp Ser Gln Asn Val Phe Thr Ala Ile Ala
                325                 330

<210> 153
<211> 1074
<212> DNA
<213> Unknown

<220>
<223> Obtained from an environmental sample

<400> 153
atgccaaacg caagaaagat tgttggagcc gtggcccaag ttgcacagga attcttcgac      60
actgaagcga atctcggtaa agcgatagcg gcgattcaca atgctgcgaa gcaaggcgca     120
gatatcgtcg tcttcgccga atgctatttg ggccaatatc catattgggc gcaattttac     180
gacaactctg ccaagaacta ttccaaggtt tggacggccc tgtacgacgg tgcgatcact     240
gtgggtggcg atgaatgccg ggctattgct gctgcggcta gacagtccaa gattcatgtc     300
gtcatgggtt gcaatgagct atccgaccga gccggcggcg caacgttata caacagcctc     360
ttgtttttcg accgaaaggg cgagttgatc ggtcgacacc ggaaattgat gccgtcgatg     420
cacgagcggt tgatccatgg cacaggcgac ggaagagact tgaatgttta cgataccgat     480
atcggtatgt tgggtgggtt gatttgctgg gagcaccata tgtcgctctc gaagtatgcc     540
atggcgacta tgggtgaaga agttcatgtt gcaagctggc ctgggatgtg gcgcggagga     600
gacgcggcaa tcggtgagag gatggtcgaa gcggatcttg gggcgccgtt tgtttgtgac      660
gccgaatttg cgatccgaga atatgcggca gagacaggaa atttcgttct aagcgcgtct     720
ggatattttc cgaaggacaa tatatccgat gagtggcgcg aagcgattcc aaaccttcaa     780
gcgcagtggg ctgtgggcgg gagttctatc gtggcaccgg ggggctccta tctggtccca     840
ccactcatta atgaggagaa gatcctctgc gccgaactcg atttcaatct caggcgtctt     900
tggaaagcct ggatcgatcc gattggtcac tattcgcgtc ccgatgttta tagcctgcaa     960
ctgcataacg ttgctgggcg tgagtattcc tatcaggccg tagatttgaa gcgcacgcca    1020
aagccccaat cgctgtgggt agatgcgtcc gaggaagacg gtgcgctgaa ttga          1074

<210> 154
<211> 357
<212> PRT
<213> Unknown

<220>
<223> Obtained from an environmental sample

<400> 154

Met Pro Asn Ala Arg Lys Ile Val Gly Ala Val Ala Gln Val Ala Gln
1               5                   10                  15
Glu Phe Phe Asp Thr Glu Ala Asn Leu Gly Lys Ala Ile Ala Ala Ile
            20                  25                  30
His Asn Ala Ala Lys Gln Gly Ala Asp Ile Val Val Phe Ala Glu Cys
        35                  40                  45
Tyr Leu Gly Gln Tyr Pro Tyr Trp Ala Gln Phe Tyr Asp Asn Ser Ala
    50                  55                  60
Lys Asn Tyr Ser Lys Val Trp Thr Ala Leu Tyr Asp Gly Ala Ile Thr
65                  70                  75                  80
Val Gly Gly Asp Glu Cys Arg Ala Ile Ala Ala Ala Ala Arg Gln Ser
                85                  90                  95
Lys Ile His Val Val Met Gly Cys Asn Glu Leu Ser Asp Arg Ala Gly
            100                 105                 110
Gly Ala Thr Leu Tyr Asn Ser Leu Leu Phe Phe Asp Arg Lys Gly Glu
        115                 120                 125
Leu Ile Gly Arg His Arg Lys Leu Met Pro Ser Met His Glu Arg Leu
    130                 135                 140
Ile His Gly Thr Gly Asp Gly Arg Asp Leu Asn Val Tyr Asp Thr Asp
145                 150                 155                 160
Ile Gly Met Leu Gly Gly Leu Ile Cys Trp Glu His His Met Ser Leu
                165                 170                 175
Ser Lys Tyr Ala Met Ala Thr Met Gly Glu Glu Val His Val Ala Ser
            180                 185                 190
Trp Pro Gly Met Trp Arg Gly Gly Asp Ala Ala Ile Gly Glu Arg Met
        195                 200                 205
Val Glu Ala Asp Leu Gly Ala Pro Phe Val Cys Asp Ala Glu Phe Ala
    210                 215                 220
Ile Arg Glu Tyr Ala Ala Glu Thr Gly Asn Phe Val Leu Ser Ala Ser
225                 230                 235                 240
Gly Tyr Phe Pro Lys Asp Asn Ile Ser Asp Glu Trp Arg Glu Ala Ile
                245                 250                 255
Pro Asn Leu Gln Ala Gln Trp Ala Val Gly Gly Ser Ser Ile Val Ala
            260                 265                 270
Pro Gly Gly Ser Tyr Leu Val Pro Pro Leu Ile Asn Glu Glu Lys Ile
        275                 280                 285
Leu Cys Ala Glu Leu Asp Phe Asn Leu Arg Arg Leu Trp Lys Ala Trp
    290                 295                 300
Ile Asp Pro Ile Gly His Tyr Ser Arg Pro Asp Val Tyr Ser Leu Gln
305                 310                 315                 320
Leu His Asn Val Ala Gly Arg Glu Tyr Ser Tyr Gln Ala Val Asp Leu
                325                 330                 335
Lys Arg Thr Pro Lys Pro Gln Ser Leu Trp Val Asp Ala Ser Glu Glu
            340                 345                 350
Asp Gly Ala Leu Asn
        355

<210> 155
<211> 1041
<212> DNA
<213> Unknown

<220>
<223> Obtained from an environmental sample

<400> 155
atgggcatcg aacatccgaa atacaaggtg gccgtggtgc aggccgcgcc ggcctggctc      60

gatctcgacg gctcgatcaa gaaggcgatt gcgctgatcg aggaagcggc cgccaagggc 120
gctaagctga tcgctttccc cgaaaccttc attcccggct atccctggca catctggctg 180
gactcgccgg cctgggcgat cggccgcggc tttgtgcagc gctacttcga taactcgctg 240
gcctacgaca gcccgcaagc cgaaaagctg cgcgccgcgg tcaagaaggc caagctcact 300
gccgtgattg gcctgtcgga gcgcgacggc ggcagcctct atatagcgca atggctgatt 360
ggccctgatg gcgagaccat cgcaaaacgc agaaagctgc ggccaacgca cgcggaacgc 420
accgtttttg gcgagggtga cggcagcgac cttgccgtgc acgaccggcc cggaatcggg 480
cggctgggag cgctgtgctg ctgggagcac ctgcaaccgc tttcgaaata cgcgatgtat 540
gcgcagaacg aacaggtcca tgtcgcgtca tggccgagct tctcgctcta cgaccccttc 600
gcgccggcgc tcggcgccga ggtcaacaat gcggcttccc gcgtctacgc ggtcgagggc 660
tcgtgcttcg tgctggcgcc gtgcgccacg gtttcgcaag ccatgatcga cgagctgtgt 720
gaccggccgg acaagcatgc gctgttgcac gccggtggcg gacacgccgc gatttacggc 780
ccggacggca gctcgatcgc ggagaagctg ccgcaggacg cggagggcct gttgatcgcc 840
gagatcgatc tcggggcgat cggggttgcc aagaatgcag ccgacccggc cggtcattat 900
tcgcggccgg acgtgacgcg actcctgctg aacaagaacc ggatgcgaag ggtcgaggag 960
tttgcgctgc cggtcgatcc ggtcgcaacg accgaggagg agcaagtcgc gacgccgtcg 1020
aggcccagcc aggccgcgta a 1041

<210> 156
<211> 346
<212> PRT
<213> Unknown

<220>
<223> Obtained from an environmental sample

<400> 156
Met Gly Ile Glu His Pro Lys Tyr Lys Val Ala Val Val Gln Ala Ala
1               5                   10                  15
Pro Ala Trp Leu Asp Leu Asp Gly Ser Ile Lys Lys Ala Ile Ala Leu
            20                  25                  30
Ile Glu Glu Ala Ala Ala Lys Gly Ala Lys Leu Ile Ala Phe Pro Glu
        35                  40                  45
Thr Phe Ile Pro Gly Tyr Pro Trp His Ile Trp Leu Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Lys Leu Arg Ala Ala Val Lys Lys
                85                  90                  95
Ala Lys Leu Thr Ala Val Ile Gly Leu Ser Glu Arg Asp Gly Gly Ser
            100                 105                 110
Leu Tyr Ile Ala Gln Trp Leu Ile Gly Pro Asp Gly Glu Thr Ile Ala
        115                 120                 125
Lys Arg Arg Lys Leu Arg Pro Thr His Ala Glu Arg Thr Val Phe Gly
    130                 135                 140
Glu Gly Asp Gly Ser Asp Leu Ala Val His Asp Arg Pro Gly Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ser Trp Pro
            180                 185                 190
Ser Phe Ser Leu Tyr Asp Pro Phe Ala Pro Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240

Asp Arg Pro Asp Lys His Ala Leu Leu His Ala Gly Gly Gly His Ala
                245                 250                 255
Ala Ile Tyr Gly Pro Asp Gly Ser Ser Ile Ala Glu Lys Leu Pro Gln
            260                 265                 270
Asp Ala Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Asn Arg Met Arg Arg Val Glu Glu
305                 310                 315                 320
Phe Ala Leu Pro Val Asp Pro Val Ala Thr Thr Glu Glu Glu Gln Val
                325                 330                 335
Ala Thr Pro Ser Arg Pro Ser Gln Ala Ala
            340                 345

<210> 157
<211> 1011
<212> DNA
<213> Unknown

<220>
<223> Obtained from an environmental sample

<400> 157
atgagagtcg ttaaagctgc tgcggtccaa ctgagtcccg tgctgtatag ccgtgaggga      60
acagtagaaa aggtcgttcg gaagatccac gagcttggcg atcaaggagt cgagttcgcc     120
acgttcccgg agaccgtagt gccctactat ccgtacttct cggccgtcca gacgccgatt     180
cagaacatgc acggcccgga gcacctgaag ttgctcgagc aatcggtgac cgtcccgtcg     240
cccgccaccg acgcgatcgg cgacgcctgc cgccacgccg gcgtcgtcgt ctcgatcggc     300
gtcaacgaac gcgatggcgg cacgatctac aacacgcagc tcctgttcga cgccgacggc     360
accttgatcc agcgccggcg aaagatcacg ccgaccttct acgaacgaat ggtctgggga     420
cagggtgacg gttcggggct gcgcgccgtc gacagccgcg taggacgcat cggccagctc     480
gcctgtttcg agcactacaa cccgctggcg cgctacgcca tgatggccga cggcgagcag     540
attcactccg cgatgtaccc cggctccatc tttggagacg cattcgcgca gaaaatcgag     600
atcaacatcc gccagcacgc gctcgagtcc ggtgcgttcg tcgtcaacgc caccgcctgg     660
ctcgatgccg accagcaggc gcggatcatg aaggataccg gctgcaccat cgaaccgatc     720
tcgggcggtt gcttcaccgc catcgtcacc ccggacggga ccctgctggg cgaagcgata     780
cgttcggggg agggagtggt ggtcgccgat ctcgacttca cgctgatcga caggcgcaag     840
caagtgatgg actctcgtgg tcactacagt cggccggagt tgctcagcct tctgatcgac     900
cgcacaccca ccgcacacct acacgaacgc gaagcgcacc ccagagcaag tgaggactgg     960
caaggttccg agagtctgcg cgccatgcag gcctcggcac cgaaggtctg a             1011

<210> 158
<211> 336
<212> PRT
<213> Unknown

<220>
<223> Obtained from an environmental sample

<400> 158
Met Arg Val Val Lys Ala Ala Ala Val Gln Leu Ser Pro Val Leu Tyr
 1               5                  10                  15
Ser Arg Glu Gly Thr Val Glu Lys Val Val Arg Lys Ile His Glu Leu
            20                  25                  30
Gly Asp Gln Gly Val Glu Phe Ala Thr Phe Pro Glu Thr Val Val Pro
        35                  40                  45

Tyr Tyr Pro Tyr Phe Ser Ala Val Gln Thr Pro Ile Gln Asn Met His
    50                  55                  60
Gly Pro Glu His Leu Lys Leu Leu Glu Gln Ser Val Thr Val Pro Ser
65                  70                  75                  80
Pro Ala Thr Asp Ala Ile Gly Asp Ala Cys Arg His Ala Gly Val Val
                85                  90                  95
Val Ser Ile Gly Val Asn Glu Arg Asp Gly Gly Thr Ile Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr Phe Tyr Glu Arg Met Val Trp Gly Gln Gly Asp Gly
    130                 135                 140
Ser Gly Leu Arg Ala Val Asp Ser Arg Val Gly Arg Ile Gly Gln Leu
145                 150                 155                 160
Ala Cys Phe Glu His Tyr Asn Pro Leu Ala Arg Tyr Ala Met Met Ala
                165                 170                 175
Asp Gly Glu Gln Ile His Ser Ala Met Tyr Pro Gly Ser Ile Phe Gly
            180                 185                 190
Asp Ala Phe Ala Gln Lys Ile Glu Ile Asn Ile Arg Gln His Ala Leu
        195                 200                 205
Glu Ser Gly Ala Phe Val Val Asn Ala Thr Ala Trp Leu Asp Ala Asp
    210                 215                 220
Gln Gln Ala Arg Ile Met Lys Asp Thr Gly Cys Thr Ile Glu Pro Ile
225                 230                 235                 240
Ser Gly Gly Cys Phe Thr Ala Ile Val Thr Pro Asp Gly Thr Leu Leu
                245                 250                 255
Gly Glu Ala Ile Arg Ser Gly Glu Gly Val Val Val Ala Asp Leu Asp
            260                 265                 270
Phe Thr Leu Ile Asp Arg Arg Lys Gln Val Met Asp Ser Arg Gly His
        275                 280                 285
Tyr Ser Arg Pro Glu Leu Leu Ser Leu Leu Ile Asp Arg Thr Pro Thr
    290                 295                 300
Ala His Leu His Glu Arg Glu Ala His Pro Arg Ala Ser Glu Asp Trp
305                 310                 315                 320
Gln Gly Ser Glu Ser Leu Arg Ala Met Gln Ala Ser Ala Pro Lys Val
                325                 330                 335

<210> 159
<211> 930
<212> DNA
<213> Unknown

<220>
<223> Obtained from an environmental sample

<400> 159
atgtcatcaa ccgtgacggt tgccattatt caggcagcac ccgtgtatta tgacctgcct      60
gccacgctgg acaaagccgc caaactggtg gcggatgcgg cggcacaggg cgcaacgctg     120
attgtcttcg gcgagacatg gtttccgggg tatccggcat ggctggatta ctgccccaat     180
gtcgcgctgt ggaatcatcc cccgaccaag caggtatttg agcgcctgca tcgcaacagc     240
atcgctgtgc caagcaagga actcgatttt ctgggggcgc tggcacgcaa gcatcaggtg     300
gtgctggtgt tgagcattaa tgaacgtgtg gagcagggcg cggggcatgg cacgctgtat     360
aacacgctgc tcacgattga cgccgatggc acgctggcaa atcatcatcg caaactgatg     420
ccgacctata ccgagcgcat ggtgtggggc atgggcgacg gggtggggtt gcaagcggtg      480
gatactgccg tcgggcgcgt aggcggctta atctgctggg aacactggat gccgttggca      540
cgccagacca tgcacatcag cggcgaacag attcatattt ccgtcttccc aaccgtccat     600
gagatgcacc agattgccag ccgccagtat gcctttgaag ggcggacgtt tgtgctgacc     660

gttggcggca ttcttgcggc acaggacttg cccgccgaac tggaacgccc cgccgatttg 720
ccgcccacgc agcttgtcca gcgcggcggc agcgccatta tcgcgccgga tggtcgttat 780
ctggcgggtc cagtctataa tgaggaaacc atcctgaccg caacgctgga tttgggcgag 840
atcatccgcg agagcatgac gctggatgtc accggacatt atgcccgccc ggatgttttt 900
gacctgaccg tgaagcgcag ccgaccatga 930

<210> 160
<211> 309
<212> PRT
<213> Unknown

<220>
<223> Obtained from an environmental sample

<400> 160
Met Ser Ser Thr Val Thr Val Ala Ile Ile Gln Ala Ala Pro Val Tyr
1               5                   10                  15
Tyr Asp Leu Pro Ala Thr Leu Asp Lys Ala Ala Lys Leu Val Ala Asp
            20                  25                  30
Ala Ala Ala Gln Gly Ala Thr Leu Ile Val Phe Gly Glu Thr Trp Phe
        35                  40                  45
Pro Gly Tyr Pro Ala Trp Leu Asp Tyr Cys Pro Asn Val Ala Leu Trp
    50                  55                  60
Asn His Pro Pro Thr Lys Gln Val Phe Glu Arg Leu His Arg Asn Ser
65                  70                  75                  80
Ile Ala Val Pro Ser Lys Glu Leu Asp Phe Leu Gly Ala Leu Ala Arg
                85                  90                  95
Lys His Gln Val Val Leu Val Leu Ser Ile Asn Glu Arg Val Glu Gln
            100                 105                 110
Gly Ala Gly His Gly Thr Leu Tyr Asn Thr Leu Leu Thr Ile Asp Ala
        115                 120                 125
Asp Gly Thr Leu Ala Asn His His Arg Lys Leu Met Pro Thr Tyr Thr
    130                 135                 140
Glu Arg Met Val Trp Gly Met Gly Asp Gly Val Gly Leu Gln Ala Val
145                 150                 155                 160
Asp Thr Ala Val Gly Arg Val Gly Gly Leu Ile Cys Trp Glu His Trp
                165                 170                 175
Met Pro Leu Ala Arg Gln Thr Met His Ile Ser Gly Glu Gln Ile His
            180                 185                 190
Ile Ser Val Phe Pro Thr Val His Glu Met His Gln Ile Ala Ser Arg
        195                 200                 205
Gln Tyr Ala Phe Glu Gly Arg Thr Phe Val Leu Thr Val Gly Gly Ile
    210                 215                 220
Leu Ala Ala Gln Asp Leu Pro Ala Glu Leu Glu Arg Pro Ala Asp Leu
225                 230                 235                 240
Pro Pro Thr Gln Leu Val Gln Arg Gly Gly Ser Ala Ile Ile Ala Pro
                245                 250                 255
Asp Gly Arg Tyr Leu Ala Gly Pro Val Tyr Asn Glu Glu Thr Ile Leu
            260                 265                 270
Thr Ala Thr Leu Asp Leu Gly Glu Ile Ile Arg Glu Ser Met Thr Leu
        275                 280                 285
Asp Val Thr Gly His Tyr Ala Arg Pro Asp Val Phe Asp Leu Thr Val
    290                 295                 300
Lys Arg Ser Arg Pro
305

<210> 161

<211> 1008
<212> DNA
<213> Unknown

<220>
<223> Obtained from an environmental sample

<400> 161
atgaccacca tccgcgccgc cgccgtgcag tttagcccgg tgctgtactc gcgccaggcc 60
accgtcgaca agctgtgccg caccctgctg gaactgggcc gcgaaggggt gcagttcgcg 120
gtattcccgg aaaccgtggt gccgtactac ccatattttt ccttcgtgca gccaccgttc 180
gccatgggca aacaacacct gttgctgctc gagcaatccg tcactgtgcc ctctgacgtc 240
acccggcaga tcggtgaggc ctgccgggaa gcggggatcg tcgccagcat cggcgtcaac 300
gaacgcgacg gcggcactat ttataacgcg cagttgctgt tcgatgccga cggcagcctg 360
attcagcagc ggcgcaagat caccccgacc tatcacgaac gcatggtctg ggggcagggc 420
gatggttccg gcctgcgcgc cgtggacagt gcggtggggc gtatcggttc cctggcctgc 480
tgggaacatt acaaccccct ggcgcgctac gcgctgatgg ccgatggcga acagattcat 540
gtggcgatgt ttcccggctc cctggtcggc gacatctttg ccgagcagat cgaagtcacc 600
atccgccacc acgccctgga aagcggctgc ttcgtggtca acgccacggc ttggctggat 660
gccgaccagc agggccggat catgcaggac accggctgcg agttggggcc gatttccggc 720
ggctgtttta ccgcgatcat ttccccggag ggcaaggttc tcggcgagcc gctgcgcagc 780
ggcgaagggg tggtcattgc tgacctcgac ctggccctga tcgacaagcg caaacgcatg 840
atggattcgg tcggtcacta cagccgcccg gaactgctca gcctgcttat cgaccgcagc 900
ccgaccgccc acgtgcatga acttgccgcc gcgcttaatc ctgccaggga gtctgatcca 960
ctagtgtcga cctgcaggcg cgcgagctcc agcttttgtt ccctttag 1008

<210> 162
<211> 335
<212> PRT
<213> Unknown

<220>
<223> Obtained from an environmental sample

<400> 162
Met Thr Thr Ile Arg Ala Ala Ala Val Gln Phe Ser Pro Val Leu Tyr
1               5                   10                  15
Ser Arg Gln Ala Thr Val Asp Lys Leu Cys Arg Thr Leu Leu Glu Leu
            20                  25                  30
Gly Arg Glu Gly Val Gln Phe Ala Val Phe Pro Glu Thr Val Val Pro
        35                  40                  45
Tyr Tyr Pro Tyr Phe Ser Phe Val Gln Pro Pro Phe Ala Met Gly Lys
    50                  55                  60
Gln His Leu Leu Leu Leu Glu Gln Ser Val Thr Val Pro Ser Asp Val
65                  70                  75                  80
Thr Arg Gln Ile Gly Glu Ala Cys Arg Glu Ala Gly Ile Val Ala Ser
                85                  90                  95
Ile Gly Val Asn Glu Arg Asp Gly Gly Thr Ile Tyr Asn Ala Gln Leu
            100                 105                 110
Leu Phe Asp Ala Asp Gly Ser Leu Ile Gln Gln Arg Arg Lys Ile Thr
        115                 120                 125
Pro Thr Tyr His Glu Arg Met Val Trp Gly Gln Gly Asp Gly Ser Gly
    130                 135                 140
Leu Arg Ala Val Asp Ser Ala Val Gly Arg Ile Gly Ser Leu Ala Cys
145                 150                 155                 160
Trp Glu His Tyr Asn Pro Leu Ala Arg Tyr Ala Leu Met Ala Asp Gly
                165                 170                 175

Glu Gln Ile His Val Ala Met Phe Pro Gly Ser Leu Val Gly Asp Ile
            180                 185                 190
Phe Ala Glu Gln Ile Glu Val Thr Ile Arg His His Ala Leu Glu Ser
        195                 200                 205
Gly Cys Phe Val Val Asn Ala Thr Ala Trp Leu Asp Ala Asp Gln Gln
    210                 215                 220
Gly Arg Ile Met Gln Asp Thr Gly Cys Glu Leu Gly Pro Ile Ser Gly
225                 230                 235                 240
Gly Cys Phe Thr Ala Ile Ile Ser Pro Glu Gly Lys Val Leu Gly Glu
                245                 250                 255
Pro Leu Arg Ser Gly Glu Gly Val Val Ile Ala Asp Leu Asp Leu Ala
            260                 265                 270
Leu Ile Asp Lys Arg Lys Arg Met Met Asp Ser Val Gly His Tyr Ser
        275                 280                 285
Arg Pro Glu Leu Leu Ser Leu Leu Ile Asp Arg Ser Pro Thr Ala His
    290                 295                 300
Val His Glu Leu Ala Ala Ala Leu Asn Pro Ala Arg Glu Ser Asp Pro
305                 310                 315                 320
Leu Val Ser Thr Cys Arg Arg Ala Ser Ser Ser Phe Cys Ser Leu
                325                 330                 335

<210> 163
<211> 978
<212> DNA
<213> Unknown

<220>
<223> Obtained from an environmental sample

<400> 163
gtgaccatca tcaaagccgc cgcagtgcag atcagccccg tgctttacag ccgggaagcc      60
accgtcgaaa aggtcgttcg cgagacccgc gaactcggcc agaagggcgt gcagttcgca     120
acgtttccgg aaaccgtggt gccgtactac ccatacttct ccgccgtcca gacgggcatc     180
gaactgctgt ccggcaaaga gcacctgcga ctgctggagc aggccgtgac tgttccttcc     240
cccgccactg atgcgattgc ccaggcggca cgcgaggccg gcatggtggt gtcgatcggc     300
gtcaacgagc gtgacggcgg caccatctac aacacgcagc tgctctttga tgccgacggc     360
acgctggtgc agcgccgccg caagatcacg ccgacgcatt tcgagcgcat ggtgtggggc     420
cagggcgacg gttcgggcct gcgcgcagtg gataccaagg tcggccgcat tggccagctg     480
gcctgcttcg agcacaacaa cccgctcgcg cgctacgcaa tgatggccga tggcgagcag     540
atccattcct ccatgtaccc gggctccgcc ttcggcgacg gattcgcgca gcgcatggag     600
atcaacattc gccaacacgc cctggagtcg ggttgcttcg tggtgaatgc caccgcgtgg     660
ctcgacgccg accagcaggc gcagatcatg aaggacacgg gctgcgccat cgggccgatc     720
tctggcggct gcttcacgac catcgtcacg ccggacggca tgctgatcgg cgaacccctc     780
cgcgagggcg agggcgagat catcgccgac ctcgatttca ccctgatcga ccgccgcaag     840
ctgctgatgg actcggtcgg ccactacaac cgtccggagc tgctgagcct gctgatcgac     900
cgcacacccg cggcgaactt ccatgagcgc agtacgcatc cggccgtcga tgccgccagc     960
ggcctcgaaa tcctctaa                                                   978

<210> 164
<211> 325
<212> PRT
<213> Unknown

<220>
<223> Obtained from an environmental sample

<400> 164

Val Thr Ile Ile Lys Ala Ala Ala Val Gln Ile Ser Pro Val Leu Tyr
1               5                   10                  15
Ser Arg Glu Ala Thr Val Glu Lys Val Val Arg Glu Thr Arg Glu Leu
            20                  25                  30
Gly Gln Lys Gly Val Gln Phe Ala Thr Phe Pro Glu Thr Val Val Pro
        35                  40                  45
Tyr Tyr Pro Tyr Phe Ser Ala Val Gln Thr Gly Ile Glu Leu Leu Ser
    50                  55                  60
Gly Lys Glu His Leu Arg Leu Leu Glu Gln Ala Val Thr Val Pro Ser
65                  70                  75                  80
Pro Ala Thr Asp Ala Ile Ala Gln Ala Ala Arg Glu Ala Gly Met Val
                85                  90                  95
Val Ser Ile Gly Val Asn Glu Arg Asp Gly Gly Thr Ile Tyr Asn Thr
            100                 105                 110
Gln Leu Leu Phe Asp Ala Asp Gly Thr Leu Val Gln Arg Arg Arg Lys
        115                 120                 125
Ile Thr Pro Thr His Phe Glu Arg Met Val Trp Gly Gln Gly Asp Gly
    130                 135                 140
Ser Gly Leu Arg Ala Val Asp Thr Lys Val Gly Arg Ile Gly Gln Leu
145                 150                 155                 160
Ala Cys Phe Glu His Asn Asn Pro Leu Ala Arg Tyr Ala Met Met Ala
                165                 170                 175
Asp Gly Glu Gln Ile His Ser Ser Met Tyr Pro Gly Ser Ala Phe Gly
            180                 185                 190
Asp Gly Phe Ala Gln Arg Met Glu Ile Asn Ile Arg Gln His Ala Leu
        195                 200                 205
Glu Ser Gly Cys Phe Val Val Asn Ala Thr Ala Trp Leu Asp Ala Asp
    210                 215                 220
Gln Gln Ala Gln Ile Met Lys Asp Thr Gly Cys Ala Ile Gly Pro Ile
225                 230                 235                 240
Ser Gly Gly Cys Phe Thr Thr Ile Val Thr Pro Asp Gly Met Leu Ile
                245                 250                 255
Gly Glu Pro Leu Arg Glu Gly Glu Gly Glu Ile Ile Ala Asp Leu Asp
            260                 265                 270
Phe Thr Leu Ile Asp Arg Arg Lys Leu Leu Met Asp Ser Val Gly His
        275                 280                 285
Tyr Asn Arg Pro Glu Leu Leu Ser Leu Leu Ile Asp Arg Thr Pro Ala
    290                 295                 300
Ala Asn Phe His Glu Arg Ser Thr His Pro Ala Val Asp Ala Ala Ser
305                 310                 315                 320
Gly Leu Glu Ile Leu
                325

<210> 165
<211> 1008
<212> DNA
<213> Unknown

<220>
<223> Obtained from an environmental sample

<400> 165
atggccaatt tcaaattcaa ggcggcggcg gtgcaggccg cgcccgcttt cctcgatctc      60
gaggctagca tcgccaagtc gatcgccctg atcgaacaag ccgccgccaa cggcgccaag     120
ctgatcgcct ttcccgaagt cttcattccc ggctacccct ggcacatctg gctcgacagt     180
cccgcctggg cgatcggcgg cggcttcgtc tcgcgctatt tcgagaactc gctggactac     240
aacagccccg aggccgagcg cctcaggctc gccgtcaaga aggcgggcct gacggcggtg     300

atcggcctct ccgagcgcga cggcggcagc ctctacatcg cgcaatggat catcggccct 360
gacggcgaga ccgttgcgaa acggcgtaag ctccggccga cccattgcga gcgcacggtc 420
tatggagaag gcgacggcag cgacctcgcg gttcacgacg tatctggcat cggccgtctc 480
ggcgcgctct gctgctggga gcatatccag ccgctgtcga aattcgcgat gtattcgcaa 540
aatgagcaag tgcacgtcgc gtcctggccg agcttctcgc tctacgaccc gttcgcgccg 600
gcgctgggcg ccgaggtcaa caacgcagcc tcgcggatct atgcggtcga aggctcatgc 660
ttcgtcattg cgccctgcgc gaccgtttcg cctgcaatga tcgaggaact gtgcgacgcg 720
ccaaacaaac atgcgcttct gcacgcgggc ggcggcttcg cgcgcatcta tgggccggac 780
ggcgcttcga tcgccgagac gctgccgcca gatcaggaag gcttgatcta cgccgacatc 840
gacctcaccg cgatcggcgt cgccaaggcc gccgccgatc ccgccggcca ttattcgcgc 900
cccgacgtca cgcgcctgct cttcaacaag aagcccgctc ggcgagtcga aacttttgct 960
ttgcccgtcg atgcgccggc gccggagacg cagaccgccg cgagctga 1008

<210> 166
<211> 335
<212> PRT
<213> Unknown

<220>
<223> Obtained from an environmental sample

<400> 166
Met Ala Asn Phe Lys Phe Lys Ala Ala Ala Val Gln Ala Ala Pro Ala
1               5                   10                  15
Phe Leu Asp Leu Glu Ala Ser Ile Ala Lys Ser Ile Ala Leu Ile Glu
            20                  25                  30
Gln Ala Ala Ala Asn Gly Ala Lys Leu Ile Ala Phe Pro Glu Val Phe
        35                  40                  45
Ile Pro Gly Tyr Pro Trp His Ile Trp Leu Asp Ser Pro Ala Trp Ala
    50                  55                  60
Ile Gly Arg Gly Phe Val Ser Arg Tyr Phe Glu Asn Ser Leu Asp Tyr
65                  70                  75                  80
Asn Ser Pro Glu Ala Glu Arg Leu Arg Leu Ala Val Lys Lys Ala Gly
                85                  90                  95
Leu Thr Ala Val Ile Gly Leu Ser Glu Arg Asp Gly Gly Ser Leu Tyr
            100                 105                 110
Ile Ala Gln Trp Ile Ile Gly Pro Asp Gly Glu Thr Val Ala Lys Arg
        115                 120                 125
Arg Lys Leu Arg Pro Thr His Cys Glu Arg Thr Val Tyr Gly Glu Gly
    130                 135                 140
Asp Gly Ser Asp Leu Ala Val His Asp Val Ser Gly Ile Gly Arg Leu
145                 150                 155                 160
Gly Ala Leu Cys Cys Trp Glu His Ile Gln Pro Leu Ser Lys Phe Ala
                165                 170                 175
Met Tyr Ser Gln Asn Glu Gln Val His Val Ala Ser Trp Pro Ser Phe
            180                 185                 190
Ser Leu Tyr Asp Pro Phe Ala Pro Ala Leu Gly Ala Glu Val Asn Asn
        195                 200                 205
Ala Ala Ser Arg Ile Tyr Ala Val Glu Gly Ser Cys Phe Val Ile Ala
    210                 215                 220
Pro Cys Ala Thr Val Ser Pro Ala Met Ile Glu Glu Leu Cys Asp Ala
225                 230                 235                 240
Pro Asn Lys His Ala Leu Leu His Ala Gly Gly Gly Phe Ala Arg Ile
                245                 250                 255
Tyr Gly Pro Asp Gly Ala Ser Ile Ala Glu Thr Leu Pro Pro Asp Gln
            260                 265                 270
Glu Gly Leu Ile Tyr Ala Asp Ile Asp Leu Thr Ala Ile Gly Val Ala

        275                 280                 285
Lys Ala Ala Ala Asp Pro Ala Gly His Tyr Ser Arg Pro Asp Val Thr
    290                 295                 300
Arg Leu Leu Phe Asn Lys Lys Pro Ala Arg Arg Val Glu Thr Phe Ala
305                 310                 315                 320
Leu Pro Val Asp Ala Pro Ala Pro Glu Thr Gln Thr Ala Ala Ser
                325                 330                 335

<210> 167
<211> 1017
<212> DNA
<213> Unknown

<220>
<223> Obtained from an environmental sample

<400> 167
atgggtattg aacatccgaa gtacagggtt gccgtggtgc aggccgcacc ggcctggctc 60
gatcttgacg cgtcgatcga caagtcgatc gcgctgatcg aggaggctgc ccagaaaggc 120
gccaagctga tcgcattccc cgaggccttc atccccggct acccctggca tatctggatg 180
gactcgcccg cctgggcgat tggccgcggt tttgtgcagc gctacttcga caattcgctg 240
gcctatgaca gcccgcaggc cgagaagctg cgcgcggccg tgcgcaaggc aaaactcacg 300
gccgtgatcg gcttgtcgga gcgtgacggc ggcagccttt atctcgcaca atggctgatc 360
ggccccgacg gcgagaccat cgcaaaacgg cgcaagctgc ggccgacaca tgccgagcgc 420
actgtgtacg gcgagggcga cggcagcgac cttgcggtcc acaatcgtcc ggacatcggc 480
aggctcggtg cgctctgctg ctgggagcat cttcagccac tgtcgaaata cgcgatgtac 540
gcgcagaacg agcaggtgca cgtcgcggcc tggccgagct tttcgctcta cgatcccttc 600
gccgtggcgc tcggcgccga ggtgaacaac gcggcctccc gcgtctatgc ggtcgaaggc 660
tcctgcttcg tgctggcgcc gtgcgcgaca gtctcgcaag ccatgatcga cgagctctgc 720
gatcggccgg acaagcacgc gctgctgcat gtcggcggcg gctttgccgc gatctacggg 780
cccgacggca gccagatcgg cgacaagctc gcccccgacc aggagggcct gttgatcgcc 840
gagatcgatc tcggcgccat aggtgtcgcc aagaacgccg cggatcccgc cgggcactat 900
tcgcggcccg acgtgacgcg gctgttgctc aacaagaaac cgtacaagcg cgtcgaacag 960
ttctcgccgc cgtcggaggc ggttgaaccc acggatatcg cggcggcggc aagctga 1017

<210> 168
<211> 338
<212> PRT
<213> Unknown

<220>
<223> Obtained from an environmental sample

<400> 168
Met Gly Ile Glu His Pro Lys Tyr Arg Val Ala Val Val Gln Ala Ala
1               5                   10                  15
Pro Ala Trp Leu Asp Leu Asp Ala Ser Ile Asp Lys Ser Ile Ala Leu
            20                  25                  30
Ile Glu Glu Ala Ala Gln Lys Gly Ala Lys Leu Ile Ala Phe Pro Glu
        35                  40                  45
Ala Phe Ile Pro Gly Tyr Pro Trp His Ile Trp Met Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Lys Leu Arg Ala Ala Val Arg Lys
                85                  90                  95
Ala Lys Leu Thr Ala Val Ile Gly Leu Ser Glu Arg Asp Gly Gly Ser

                100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asn Arg Pro Asp Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
                180                 185                 190
Ser Phe Ser Leu Tyr Asp Pro Phe Ala Val Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Val Gly Gly Gly Phe Ala
                245                 250                 255
Ala Ile Tyr Gly Pro Asp Gly Ser Gln Ile Gly Asp Lys Leu Ala Pro
                260                 265                 270
Asp Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Lys Pro Tyr Lys Arg Val Glu Gln
305                 310                 315                 320
Phe Ser Pro Pro Ser Glu Ala Val Glu Pro Thr Asp Ile Ala Ala Ala
                325                 330                 335
Ala Ser

<210> 169
<211> 1077
<212> DNA
<213> Unknown

<220>
<223> Obtained from an environmental sample

<400> 169
atggccctca cccatccgaa attgaaagtc gccgccgtgc aggcagctcc cgcgttcctc      60
gatgtcgatg ccgcagtgga caaagcggtg cggctaatcg acgaagcggc agcaaacggc     120
tccagtctgg tggcattccc cgagacctgg atccccggct atccgttttg gatctggctt     180
ggctcgccgg cctgggcaat catgcgcggg tttgtgtctc gctatttcga taattcgctc     240
agctatgaca gccggcaggc agagcgcctg cgcgacgccg cgaagcgcca caaactgacc     300
gtcgtcatgg gcctgtccga gcgcgccggc ggtagccttt acatcgcgca gtggatcatt     360
ggtcccaatg gcgagaccgt cgcacagcgg cgcaagctca agcccaccca tgcggagcgc     420
accgtcttcg gcgagggtga cggcagccac ctggcggtac acaatcttcc aatcggacgg     480
ctcggtgcgc tgtgctgctg ggagcacctc cagccgctct ccaaatacgc gatgtacgcc     540
cagaacgaag agatccacgt ggcggcatgg ccgtccttct cgctctacga cccgtttgcg     600
cacgcgctcg gcgccgaagt caacaacgca gcgagccaga tctacgcggt tgaaggttcc     660
tgctttgtcg tcgcgccatg tgcggtgatc tcgcaggaaa tgatcgatct tatgtgcgat     720
acccccgaca agcatcagct tattcacgtc ggtggcggct tcaccgtgat ctatggcccg     780
gacggtgcgc gcatcggcga caagctcgcg ccagatcagg aaggcattgt ctatgccgac     840
atcgatctcg gcatgatccc gatcgcgaaa gctgccgccg atcctgccgg ccactatgcg     900
cgacccgacg ttacccgcct tctgttcaac aatcgtcccg ccaatcgggt ggaaaccctc     960

gtgctccccg ttgatcaggt ccgtgacatc gatgcacgtg tggaggccgc ggcacctcag     1020
gcgcgaccag caaccgggaa cgaggatccc gccgcaaagc ctatggccgc cgaatga        1077

<210> 170
<211> 358
<212> PRT
<213> Unknown

<220>
<223> Obtained from an environmental sample

<400> 170
Met Ala Leu Thr His Pro Lys Leu Lys Val Ala Ala Val Gln Ala Ala
1               5                   10                  15
Pro Ala Phe Leu Asp Val Asp Ala Ala Val Asp Lys Ala Val Arg Leu
            20                  25                  30
Ile Asp Glu Ala Ala Ala Asn Gly Ser Ser Leu Val Ala Phe Pro Glu
        35                  40                  45
Thr Trp Ile Pro Gly Tyr Pro Phe Trp Ile Trp Leu Gly Ser Pro Ala
    50                  55                  60
Trp Ala Ile Met Arg Gly Phe Val Ser Arg Tyr Phe Asp Asn Ser Leu
65                  70                  75                  80
Ser Tyr Asp Ser Arg Gln Ala Glu Arg Leu Arg Asp Ala Ala Lys Arg
                85                  90                  95
His Lys Leu Thr Val Val Met Gly Leu Ser Glu Arg Ala Gly Gly Ser
            100                 105                 110
Leu Tyr Ile Ala Gln Trp Ile Ile Gly Pro Asn Gly Glu Thr Val Ala
        115                 120                 125
Gln Arg Arg Lys Leu Lys Pro Thr His Ala Glu Arg Thr Val Phe Gly
    130                 135                 140
Glu Gly Asp Gly Ser His Leu Ala Val His Asn Leu Pro Ile Gly Arg
145                 150                 155                 160
Leu Gly Ala Leu Cys Cys Trp Glu His Leu Gln Pro Leu Ser Lys Tyr
                165                 170                 175
Ala Met Tyr Ala Gln Asn Glu Glu Ile His Val Ala Ala Trp Pro Ser
            180                 185                 190
Phe Ser Leu Tyr Asp Pro Phe Ala His Ala Leu Gly Ala Glu Val Asn
        195                 200                 205
Asn Ala Ala Ser Gln Ile Tyr Ala Val Glu Gly Ser Cys Phe Val Val
    210                 215                 220
Ala Pro Cys Ala Val Ile Ser Gln Glu Met Ile Asp Leu Met Cys Asp
225                 230                 235                 240
Thr Pro Asp Lys His Gln Leu Ile His Val Gly Gly Gly Phe Thr Val
                245                 250                 255
Ile Tyr Gly Pro Asp Gly Ala Arg Ile Gly Asp Lys Leu Ala Pro Asp
            260                 265                 270
Gln Glu Gly Ile Val Tyr Ala Asp Ile Asp Leu Gly Met Ile Pro Ile
        275                 280                 285
Ala Lys Ala Ala Ala Asp Pro Ala Gly His Tyr Ala Arg Pro Asp Val
    290                 295                 300
Thr Arg Leu Leu Phe Asn Asn Arg Pro Ala Asn Arg Val Glu Thr Leu
305                 310                 315                 320
Val Leu Pro Val Asp Gln Val Arg Asp Ile Asp Ala Arg Val Glu Ala
                325                 330                 335
Ala Ala Pro Gln Ala Arg Pro Ala Thr Gly Asn Glu Asp Pro Ala Ala
            340                 345                 350
Lys Pro Met Ala Ala Glu

        355

<210> 171
<211> 1011
<212> DNA
<213> Unknown

<220>
<223> Obtained from an environmental sample

<400> 171
atgactaagg gaacagtgaa agtcgcggcg gcgcaggtca cccccgtgtt catggatcgt 60
aaagccacga tcgtcaaggc ctgcgacacg atcgccgagg cgggcaagaa cggcgcgcgg 120
ctggtggtgt tcccggagac gtttgttcct ggctacccgg actgggtctg gacggcgacg 180
gctgggacgc atcgcgatat ccaccaggcg atgtacgcgg aactgctgga ccaggctgtc 240
tcgattccga gcccggcgac ggacgccctc tgccgtgctg caaagaaggc gggcgtctac 300
gtcgtcatcg gcgtcaatga gctgagtggg ccgggcggaa gcctgtacaa cacgctgatc 360
tacatcgatg acgaaggcga gatcatgggc cgccaccgca agctggtccc cacgatgggc 420
gagcgcctgg tctgggcacc cggcgacggc agcacgctgg aggcgtacga gacatcgatc 480
ggcaggctgg gcggactgat ctgctgggag aactacatgc cgctggcccg ctacgccatg 540
tacgcctggg gcgtgcagat ctacgtcgcg ccgacgtggg acagctcgga cgggtgggtt 600
ggcagcatgc agcacatcgc ccgcgaaggg cggacggcgg tgatcggctg ctgcatggcg 660
atccgtcgca gcgacatccc ggacaagtac gagttcaaga agctgtaccc gccgagcaag 720
agcaaagacg aagaatgggt gaacgatggc aacagcgtca tcgtcgcacc cggtggacga 780
atactcgccg ggccggtcgc caaagaggag acgatcctct acgccgatct ggacccggca 840
gccgagcgcg gttcaaagtt ctcgttagat gtggcagggc actacgcgcg gccggacgtc 900
ttccagctga cggtgaatcg cggtccggca gaactggtga atgtggccgg tgatatcgca 960
ccggcaacca acggcaaagt caaaacaccg gcgaaattac gccgcaagta a 1011

<210> 172
<211> 336
<212> PRT
<213> Unknown

<220>
<223> Obtained from an environmental sample

<400> 172
Met Thr Lys Gly Thr Val Lys Val Ala Ala Ala Gln Val Thr Pro Val
1               5                   10                  15
Phe Met Asp Arg Lys Ala Thr Ile Val Lys Ala Cys Asp Thr Ile Ala
            20                  25                  30
Glu Ala Gly Lys Asn Gly Ala Arg Leu Val Val Phe Pro Glu Thr Phe
        35                  40                  45
Val Pro Gly Tyr Pro Asp Trp Val Trp Thr Ala Thr Ala Gly Thr His
    50                  55                  60
Arg Asp Ile His Gln Ala Met Tyr Ala Glu Leu Leu Asp Gln Ala Val
65                  70                  75                  80
Ser Ile Pro Ser Pro Ala Thr Asp Ala Leu Cys Arg Ala Ala Lys Lys
                85                  90                  95
Ala Gly Val Tyr Val Val Ile Gly Val Asn Glu Leu Ser Gly Pro Gly
            100                 105                 110
Gly Ser Leu Tyr Asn Thr Leu Ile Tyr Ile Asp Asp Glu Gly Glu Ile
        115                 120                 125
Met Gly Arg His Arg Lys Leu Val Pro Thr Met Gly Glu Arg Leu Val
    130                 135                 140
Trp Ala Pro Gly Asp Gly Ser Thr Leu Glu Ala Tyr Glu Thr Ser Ile

145                 150                 155                 160
Gly Arg Leu Gly Gly Leu Ile Cys Trp Glu Asn Tyr Met Pro Leu Ala
                165                 170                 175
Arg Tyr Ala Met Tyr Ala Trp Gly Val Gln Ile Tyr Val Ala Pro Thr
            180                 185                 190
Trp Asp Ser Ser Asp Gly Trp Val Gly Ser Met Gln His Ile Ala Arg
        195                 200                 205
Glu Gly Arg Thr Ala Val Ile Gly Cys Cys Met Ala Ile Arg Arg Ser
    210                 215                 220
Asp Ile Pro Asp Lys Tyr Glu Phe Lys Lys Leu Tyr Pro Pro Ser Lys
225                 230                 235                 240
Ser Lys Asp Glu Glu Trp Val Asn Asp Gly Asn Ser Val Ile Val Ala
                245                 250                 255
Pro Gly Gly Arg Ile Leu Ala Gly Pro Val Ala Lys Glu Glu Thr Ile
            260                 265                 270
Leu Tyr Ala Asp Leu Asp Pro Ala Ala Glu Arg Gly Ser Lys Phe Ser
        275                 280                 285
Leu Asp Val Ala Gly His Tyr Ala Arg Pro Asp Val Phe Gln Leu Thr
    290                 295                 300
Val Asn Arg Gly Pro Ala Glu Leu Val Asn Val Ala Gly Asp Ile Ala
305                 310                 315                 320
Pro Ala Thr Asn Gly Lys Val Lys Thr Pro Ala Lys Leu Arg Arg Lys
                325                 330                 335

<210> 173
<211> 993
<212> DNA
<213> Unknown

<220>
<223> Obtained from an environmental sample

<400> 173
atgaaagttg ttaaagccgc cgcagtccaa ctgagtcccg tcctctatag ccgcgaggga      60
acggtcgaga gggtcgttcg gaagatccac gagcttggcc ggcagggggt acagttcgcc     120
accttcccgg agaccgtagt gccttactac ccgtactttt ccttcgtcca gacgccctta     180
cagattatag ccggacctga gcatctaaag ctgctcgacc aggcagtgac cgtgccgtcc     240
cctgccaccg acgctatcag cgaggctgcc aggcaggcgg gagttgtggt gtccataggc     300
gtcaacgagc gtgacggcgg aaccctgtac aacacgcagc tgctcttcga tgccgatggc     360
gccttgatcc agcgccgccg caagattacg cccactcatt tcgagcgcat gatctggggc     420
cagggcgacg ggtcgggcct gcgcgctgtc gacagcaagg tcggtcgcat tggccagctc     480
gcatgctggg agcacaacaa ccccctggcg cgctacgcga tgatagccga cggcgagcag     540
atccattcgg caatgtatcc gggctccatg ttcggcgacc cgtttgccca gaagacggaa     600
atcaatatcc ggcagcatgc attggagtct gcgtgcttcg tcgtgtgcgc cacggcctgg     660
ctggacgccg atcagcaggc gcaaatctgc aaggacactg gctgcgacat cggcccgatc     720
tccggcggtt gcttcaccgc gatcgtggcg cctgatggaa ccttgctggg cgagcccatc     780
cgctcgggcg aaggcatggt catcgtcgac ctcgacttca cgctcatcga caagcgcaag     840
caggtgatgg actcgcgcgg ccactacaac cggccggaat tgctcagtct cctgatcgac     900
cgcacaccca ctgcgcatgt tcacgaccgc gctgtgcgcc ccgagtcagc cgcggagcaa     960
cgttcggagg aacttctcgc tacggctgtc taa                                  993

<210> 174
<211> 330
<212> PRT
<213> Unknown

<220>

<223> Obtained from an environmental sample

<400> 174
Met Lys Val Val Lys Ala Ala Ala Val Gln Leu Ser Pro Val Leu Tyr
1               5                   10                  15
Ser Arg Glu Gly Thr Val Glu Arg Val Val Arg Lys Ile His Glu Leu
            20                  25                  30
Gly Arg Gln Gly Val Gln Phe Ala Thr Phe Pro Glu Thr Val Val Pro
        35                  40                  45
Tyr Tyr Pro Tyr Phe Ser Phe Val Gln Thr Pro Leu Gln Ile Ile Ala
    50                  55                  60
Gly Pro Glu His Leu Lys Leu Leu Asp Gln Ala Val Thr Val Pro Ser
65                  70                  75                  80
Pro Ala Thr Asp Ala Ile Ser Glu Ala Ala Arg Gln Ala Gly Val Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Ala Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Lys Val Gly Arg Ile Gly Gln Leu
145                 150                 155                 160
Ala Cys Trp Glu His Asn Asn Pro Leu Ala Arg Tyr Ala Met Ile Ala
                165                 170                 175
Asp Gly Glu Gln Ile His Ser Ala Met Tyr Pro Gly Ser Met Phe Gly
            180                 185                 190
Asp Pro Phe Ala Gln Lys Thr Glu Ile Asn Ile Arg Gln His Ala Leu
        195                 200                 205
Glu Ser Ala Cys Phe Val Val Cys Ala Thr Ala Trp Leu Asp Ala Asp
    210                 215                 220
Gln Gln Ala Gln Ile Cys Lys Asp Thr Gly Cys Asp Ile Gly Pro Ile
225                 230                 235                 240
Ser Gly Gly Cys Phe Thr Ala Ile Val Ala Pro Asp Gly Thr Leu Leu
                245                 250                 255
Gly Glu Pro Ile Arg Ser Gly Glu Gly Met Val Ile Val Asp Leu Asp
            260                 265                 270
Phe Thr Leu Ile Asp Lys Arg Lys Gln Val Met Asp Ser Arg Gly His
        275                 280                 285
Tyr Asn Arg Pro Glu Leu Leu Ser Leu Leu Ile Asp Arg Thr Pro Thr
    290                 295                 300
Ala His Val His Asp Arg Ala Val Arg Pro Glu Ser Ala Ala Glu Gln
305                 310                 315                 320
Arg Ser Glu Glu Leu Leu Ala Thr Ala Val
                325                 330

<210> 175
<211> 945
<212> DNA
<213> Unknown

<220>
<223> Obtained from an environmental sample

<400> 175
atgaccacct acgggcagtt tagacttgcc gcgatcaatg ccgcgcctgt ctacttcgac      60
agggaagcat ccaccgaaaa agcttgccgg ctcattctcg aagcgggggc atcaggggcg     120

acgctggcgg cgtttggcga gacgtggctt cccggctatc cattccacat ctggagggaa 180
gttccgactg ctctccgagt ggaatacatc gctaatgccg tcgagattcc cagcccaacg 240
accgaccgat tgtgcgcggc ggctcgtcag gcgaacatcg atgttgtgat cggcgttgtc 300
gaactggatg cgcagacaca cgggacggtc tactgtacgc tcttgttcat tggcagcgat 360
ggctcaattc tgggacgtca tcgaaagatt aaaccgactt tcgtggagcg aaccgcatgg 420
ggggaaggtg acggcagcag cctgatcgtc tacgagcgcc cgtatggcaa gatcagtggt 480
ctgtgttgct gggaacacaa tatggttctg ccgggctacg cgctgatggc gcaggggacg 540
cagattcata tcgccgcatg gcccggctgg gaaagcactc gccatctgct cttatcaaga 600
gcattcgctt ctcaggcagc ggcgtatgtg attgatgtag gcgctatcgt caatcgtgac 660
gaccttcggg aagattacca ggctttgatt gctggaagct actggggcgg aagttgcatc 720
atcaacccag aaggcgaggt catcgctggt ccagcgaaat cggagaccat tctggttgca 780
gattgctcaa ccgagcagat ctttagctca aaagtgctct gtgatgtggg cgggcattat 840
tctcgcccgg atatttttca gctccatgtc aatcgaaagc catatcaacg tatcgtcgag 900
acgaacaacc cacaccccgc tccgattgag ttcgattacc gttga 945

<210> 176
<211> 314
<212> PRT
<213> Unknown

<220>
<223> Obtained from an environmental sample

<400> 176
Met Thr Thr Tyr Gly Gln Phe Arg Leu Ala Ala Ile Asn Ala Ala Pro
1               5                   10                  15
Val Tyr Phe Asp Arg Glu Ala Ser Thr Glu Lys Ala Cys Arg Leu Ile
            20                  25                  30
Leu Glu Ala Gly Ala Ser Gly Ala Thr Leu Ala Ala Phe Gly Glu Thr
        35                  40                  45
Trp Leu Pro Gly Tyr Pro Phe His Ile Trp Arg Glu Val Pro Thr Ala
    50                  55                  60
Leu Arg Val Glu Tyr Ile Ala Asn Ala Val Glu Ile Pro Ser Pro Thr
65                  70                  75                  80
Thr Asp Arg Leu Cys Ala Ala Ala Arg Gln Ala Asn Ile Asp Val Val
                85                  90                  95
Ile Gly Val Val Glu Leu Asp Ala Gln Thr His Gly Thr Val Tyr Cys
            100                 105                 110
Thr Leu Leu Phe Ile Gly Ser Asp Gly Ser Ile Leu Gly Arg His Arg
        115                 120                 125
Lys Ile Lys Pro Thr Phe Val Glu Arg Thr Ala Trp Gly Glu Gly Asp
    130                 135                 140
Gly Ser Ser Leu Ile Val Tyr Glu Arg Pro Tyr Gly Lys Ile Ser Gly
145                 150                 155                 160
Leu Cys Cys Trp Glu His Asn Met Val Leu Pro Gly Tyr Ala Leu Met
                165                 170                 175
Ala Gln Gly Thr Gln Ile His Ile Ala Ala Trp Pro Gly Trp Glu Ser
            180                 185                 190
Thr Arg His Leu Leu Leu Ser Arg Ala Phe Ala Ser Gln Ala Ala Ala
        195                 200                 205
Tyr Val Ile Asp Val Gly Ala Ile Val Asn Arg Asp Asp Leu Arg Glu
    210                 215                 220
Asp Tyr Gln Ala Leu Ile Ala Gly Ser Tyr Trp Gly Gly Ser Cys Ile
225                 230                 235                 240
Ile Asn Pro Glu Gly Glu Val Ile Ala Gly Pro Ala Lys Ser Glu Thr
                245                 250                 255
Ile Leu Val Ala Asp Cys Ser Thr Glu Gln Ile Phe Ser Ser Lys Val

            260                 265                 270
Leu Cys Asp Val Gly Gly His Tyr Ser Arg Pro Asp Ile Phe Gln Leu
        275                 280                 285
His Val Asn Arg Lys Pro Tyr Gln Arg Ile Val Glu Thr Asn Asn Pro
    290                 295                 300
His Pro Ala Pro Ile Glu Phe Asp Tyr Arg
305                 310

<210> 177
<211> 948
<212> DNA
<213> Unknown

<220>
<223> Obtained from an environmental sample

<400> 177
atggactcac ttaccgttgg tctcgcgcaa atcgcgcccg tttggttgaa tcgggcgggg 60
acgttgtcaa agatgttgga acaggttcga gcggcgaaag aggcgggctg tcagcttgtt 120
gtgtttggtg aggcgttgct ccccggttat ccattttgga tcgaactgac gaacggcgca 180
gtcttcaatt cgccgatgca aaaggaaatc cacgcgcact acatggatca agctgtgcag 240
atcgaagcag ggcatcttga tccattgtgc ggcgcggcaa aagcgcacgg catcaccgtg 300
gtcgcgggca tcatcgagcg tccgttggat cgcggcggac atagtttata tgcgagtctg 360
gtgtatatcg atttgaacgg tgtcatccaa tcggtgcatc gcaaactgat gcccacctat 420
gaagaacgac tcacctggtc gcctggcgat ggtcatgggt tacgcgtgca tacactgggc 480
gcttttacgg ttggcaaact caattgttgg gaaaactgga tgccgctgcc gcgcgcggct 540
ctgtatgcgc aaggcgaaga tctgcacgtt gctgtctggc ccgggtccgt gcgcaacaca 600
caggatatta cgcgctttat cgcaatggag tcgcgatcgt ttgtcgtttc ggtttcgagt 660
ttgatgcgca agagtgactt cccacaagat acgcctcatc tctccgccat tcttgaatct 720
gcacccgatc cactcgccaa cggaggttcg tgtctggctg gacctgacgg taaatggatc 780
gttgaaccgg ttgcggatga agagaagttg atcgtcgcca ccattgacca tgcccgtgta 840
cgtgaagaac gccagaactt tgatccatcc ggtcattaca gccgaccaga tgtgacacaa 900
ttgagagtca accgccagcg acaaagcgtt atcgcttttg atgagtag 948

<210> 178
<211> 315
<212> PRT
<213> Unknown

<220>
<223> Obtained from an environmental sample

<400> 178
Met Asp Ser Leu Thr Val Gly Leu Ala Gln Ile Ala Pro Val Trp Leu
1               5                   10                  15
Asn Arg Ala Gly Thr Leu Ser Lys Met Leu Glu Gln Val Arg Ala Ala
            20                  25                  30
Lys Glu Ala Gly Cys Gln Leu Val Val Phe Gly Glu Ala Leu Leu Pro
        35                  40                  45
Gly Tyr Pro Phe Trp Ile Glu Leu Thr Asn Gly Ala Val Phe Asn Ser
    50                  55                  60
Pro Met Gln Lys Glu Ile His Ala His Tyr Met Asp Gln Ala Val Gln
65                  70                  75                  80
Ile Glu Ala Gly His Leu Asp Pro Leu Cys Gly Ala Ala Lys Ala His
                85                  90                  95
Gly Ile Thr Val Val Ala Gly Ile Ile Glu Arg Pro Leu Asp Arg Gly
            100                 105                 110

Gly His Ser Leu Tyr Ala Ser Leu Val Tyr Ile Asp Leu Asn Gly Val
        115                 120                 125
Ile Gln Ser Val His Arg Lys Leu Met Pro Thr Tyr Glu Glu Arg Leu
    130                 135                 140
Thr Trp Ser Pro Gly Asp Gly His Gly Leu Arg Val His Thr Leu Gly
145                 150                 155                 160
Ala Phe Thr Val Gly Lys Leu Asn Cys Trp Glu Asn Trp Met Pro Leu
                165                 170                 175
Pro Arg Ala Ala Leu Tyr Ala Gln Gly Glu Asp Leu His Val Ala Val
            180                 185                 190
Trp Pro Gly Ser Val Arg Asn Thr Gln Asp Ile Thr Arg Phe Ile Ala
        195                 200                 205
Met Glu Ser Arg Ser Phe Val Val Ser Val Ser Ser Leu Met Arg Lys
    210                 215                 220
Ser Asp Phe Pro Gln Asp Thr Pro His Leu Ser Ala Ile Leu Glu Ser
225                 230                 235                 240
Ala Pro Asp Pro Leu Ala Asn Gly Gly Ser Cys Leu Ala Gly Pro Asp
                245                 250                 255
Gly Lys Trp Ile Val Glu Pro Val Ala Asp Glu Glu Lys Leu Ile Val
            260                 265                 270
Ala Thr Ile Asp His Ala Arg Val Arg Glu Glu Arg Gln Asn Phe Asp
        275                 280                 285
Pro Ser Gly His Tyr Ser Arg Pro Asp Val Thr Gln Leu Arg Val Asn
    290                 295                 300
Arg Gln Arg Gln Ser Val Ile Ala Phe Asp Glu
305                 310                 315

<210> 179
<211> 915
<212> DNA
<213> Unknown

<220>
<223> Obtained from an environmental sample

<400> 179
atgaccacca aagcagccat cattcaggcg cgccccatat actacgatct ggcggcgtgt 60
gtcgataagg cgcttgccct catcaccgag gcggcggcac gcggcgcgaa catcgtcacg 120
ctcggcgaga cgtggctgcc gggctatccc gcgtggctgg atgtgtgcgt cgagatgggg 180
ctgtgggatc acgcgccgac caaagccgtc tttcagcggc tccatgccaa cagcgtcacc 240
atccccggcg cggagatcag ccagttctgc gacatcgccc gccgccttag catcgtgctg 300
gtgctcagcg tcaacgagcg cgtccgcaac accttgttca acaccctgct cacgattgac 360
gagcgcggcg acatccgcaa ccaccaccgc aagctgatgc cgacctacac tgagcgcatc 420
gtctgggggc agggcgacgg cgcgggctta caggcggtcg agacggcaac cgggcgcgtc 480
ggcgggctga tctgctggga acactggatg ccgctggcac ggcaggcgct gcacaacgcc 540
ggggagcaaa ttcacgtttc ggtcttcccg accgtcaacg acccgcgcca ccaagtcgcc 600
agccgccagt acgctttcga ggggcgctgc ttcgtgctga ccgccggcag catccagcgc 660
gccgacgacc taccgccgga actgaccgtc aaggcgggca tcgcgccgga tgatctggtg 720
cagggcggcg gcagcgccat catcgcgccg gacatgcgct acctcgccgg accctgcttc 780
gacgaggaaa ccatcctcta cgccgacctc gacctgagcg agacgatccg cgagagcatg 840
acgctggacg tgagcgggca ttactcgcgc cccgacgtgt tcaccttcga ggttaatcgg 900
cagcggaaaa tttag 915

<210> 180
<211> 304
<212> PRT
<213> Unknown

<220>
<223> Obtained from an environmental sample

<400> 180
Met Thr Thr Lys Ala Ala Ile Ile Gln Ala Arg Pro Ile Tyr Tyr Asp
1               5                   10                  15
Leu Ala Ala Cys Val Asp Lys Ala Leu Ala Leu Ile Thr Glu Ala Ala
            20                  25                  30
Ala Arg Gly Ala Asn Ile Val Thr Leu Gly Glu Thr Trp Leu Pro Gly
        35                  40                  45
Tyr Pro Ala Trp Leu Asp Val Cys Val Glu Met Gly Leu Trp Asp His
    50                  55                  60
Ala Pro Thr Lys Ala Val Phe Gln Arg Leu His Ala Asn Ser Val Thr
65                  70                  75                  80
Ile Pro Gly Ala Glu Ile Ser Gln Phe Cys Asp Ile Ala Arg Arg Leu
                85                  90                  95
Ser Ile Val Leu Val Leu Ser Val Asn Glu Arg Val Arg Asn Thr Leu
            100                 105                 110
Phe Asn Thr Leu Leu Thr Ile Asp Glu Arg Gly Asp Ile Arg Asn His
        115                 120                 125
His Arg Lys Leu Met Pro Thr Tyr Thr Glu Arg Ile Val Trp Gly Gln
    130                 135                 140
Gly Asp Gly Ala Gly Leu Gln Ala Val Glu Thr Ala Thr Gly Arg Val
145                 150                 155                 160
Gly Gly Leu Ile Cys Trp Glu His Trp Met Pro Leu Ala Arg Gln Ala
                165                 170                 175
Leu His Asn Ala Gly Glu Gln Ile His Val Ser Val Phe Pro Thr Val
            180                 185                 190
Asn Asp Pro Arg His Gln Val Ala Ser Arg Gln Tyr Ala Phe Glu Gly
        195                 200                 205
Arg Cys Phe Val Leu Thr Ala Gly Ser Ile Gln Arg Ala Asp Asp Leu
    210                 215                 220
Pro Pro Glu Leu Thr Val Lys Ala Gly Ile Ala Pro Asp Asp Leu Val
225                 230                 235                 240
Gln Gly Gly Gly Ser Ala Ile Ile Ala Pro Asp Met Arg Tyr Leu Ala
                245                 250                 255
Gly Pro Cys Phe Asp Glu Glu Thr Ile Leu Tyr Ala Asp Leu Asp Leu
            260                 265                 270
Ser Glu Thr Ile Arg Glu Ser Met Thr Leu Asp Val Ser Gly His Tyr
        275                 280                 285
Ser Arg Pro Asp Val Phe Thr Phe Glu Val Asn Arg Gln Arg Lys Ile
    290                 295                 300

<210> 181
<211> 990
<212> DNA
<213> Unknown

<220>
<223> Obtained from an environmental sample

<400> 181
atgcccaaga ccgtacgcgc agccgcagtc cagatcgcgc ccgacctgac gtcacgcgct      60
ggcaccgtcg agcgggtcct gaatgcgatt gccgaagcct ctgacaaagg cgcggagctg     120
attgtatttc cagaaacctt tgtgccttgg tatccgtatt tcagcttcgt tctgccgcct     180
gtccagcagg gacccgagca cctgcggctt tatgaagaag cggtgacggt accatcagca     240

gaaacacggg ccgtcgccga cgccgcgcgc aaacgcaatg cggtcatcgt ccttggcgtc 300
aatgagcgcg accacggctc gctctacaac acccagctga tcttcgacgc ggatggcagc 360
ctgaaactca agcgccgcaa gatcacgccc acctatcacg agcggatgat ctggggacag 420
ggcgatggcg ccggtctaaa agtggtcgaa actgccatcg gccgcatggg cgcattggcg 480
tgctgggagc actacaaccc cctcgcccga tacgcgctga tggctcagca tgaggaaatt 540
cacgcctctc attttccggg ctcactggtc ggcccgatat tcggcgagca gatcgaagtc 600
acgatgcgcc accacgcgtt ggaatcgggc tgtttcgtgg tcaatgccac cggctggtta 660
agcgaggagc agatcgcgtc cattcaccca gatcccagcc tgcagaaggg tcttcgagat 720
ggctgcatga cctgcatcat aaccccggaa ggccgccacg tcgttcctcc tctgacatcg 780
ggtgaaggaa tcctgattgg cgacctggac atgcggctca tcaccaagcg caagcgaatg 840
atggattccg tcggacacta tgcacgtcct gagctgctgc accttgtcca tgacacgacg 900
cccgcacgcg cacgcgagca ggtgggcctt tcaggcgatt tttccgatgc agggcaagac 960
aagctatttg aggaggttca agatgcgtga 990

<210> 182
<211> 329
<212> PRT
<213> Unknown

<220>
<223> Obtained from an environmental sample

<400> 182
Met Pro Lys Thr Val Arg Ala Ala Ala Val Gln Ile Ala Pro Asp Leu
1               5                   10                  15
Thr Ser Arg Ala Gly Thr Val Glu Arg Val Leu Asn Ala Ile Ala Glu
            20                  25                  30
Ala Ser Asp Lys Gly Ala Glu Leu Ile Val Phe Pro Glu Thr Phe Val
        35                  40                  45
Pro Trp Tyr Pro Tyr Phe Ser Phe Val Leu Pro Pro Val Gln Gln Gly
    50                  55                  60
Pro Glu His Leu Arg Leu Tyr Glu Glu Ala Val Thr Val Pro Ser Ala
65                  70                  75                  80
Glu Thr Arg Ala Val Ala Asp Ala Ala Arg Lys Arg Asn Ala Val Ile
                85                  90                  95
Val Leu Gly Val Asn Glu Arg Asp His Gly Ser Leu Tyr Asn Thr Gln
            100                 105                 110
Leu Ile Phe Asp Ala Asp Gly Ser Leu Lys Leu Lys Arg Arg Lys Ile
        115                 120                 125
Thr Pro Thr Tyr His Glu Arg Met Ile Trp Gly Gln Gly Asp Gly Ala
    130                 135                 140
Gly Leu Lys Val Val Glu Thr Ala Ile Gly Arg Met Gly Ala Leu Ala
145                 150                 155                 160
Cys Trp Glu His Tyr Asn Pro Leu Ala Arg Tyr Ala Leu Met Ala Gln
                165                 170                 175
His Glu Glu Ile His Ala Ser His Phe Pro Gly Ser Leu Val Gly Pro
            180                 185                 190
Ile Phe Gly Glu Gln Ile Glu Val Thr Met Arg His His Ala Leu Glu
        195                 200                 205
Ser Gly Cys Phe Val Val Asn Ala Thr Gly Trp Leu Ser Glu Glu Gln
    210                 215                 220
Ile Ala Ser Ile His Pro Asp Pro Ser Leu Gln Lys Gly Leu Arg Asp
225                 230                 235                 240
Gly Cys Met Thr Cys Ile Ile Thr Pro Glu Gly Arg His Val Val Pro
                245                 250                 255
Pro Leu Thr Ser Gly Glu Gly Ile Leu Ile Gly Asp Leu Asp Met Arg
            260                 265                 270

Leu Ile Thr Lys Arg Lys Arg Met Met Asp Ser Val Gly His Tyr Ala
        275                 280                 285
Arg Pro Glu Leu Leu His Leu Val His Asp Thr Thr Pro Ala Arg Ala
    290                 295                 300
Arg Glu Gln Val Gly Leu Ser Gly Asp Phe Ser Asp Ala Gly Gln Asp
305                 310                 315                 320
Lys Leu Phe Glu Glu Val Gln Asp Ala
                325

<210> 183
<211> 1002
<212> DNA
<213> Unknown

<220>
<223> Obtained from an environmental sample

<400> 183
atggctgagt cacgcattat tcgtgccgcc gccgcccaga ttgcgccaga tctccatgag      60
gccagcaaaa cgctggcccg ggtgctggac gcgatcgatc aggcagccgc acagggggca     120
gagatcatcg tctttcccga gacctttgtg ccttattacc cctacttctc gtttatcacg     180
cccgcgatga ccgccggagc ggcccatctg aaattgtatg accaggcggt ggtggtgccc     240
ggcccgatca cccatgcggt gggcgaacgc gcccgcctgc gcaacatcgt cgtggtgctg     300
ggggtgaatg aacgtgacca cggcacgctc tacaacaccc aactggtatt tgatgccagc     360
ggggaactgg tgctgaaacg ccgcaaaatc accccgacct atcacgaacg gatgatctgg     420
ggacagggag acggtgccgg attaaaggtg gtggactcgg cggttgggcg catcggggct     480
ttagcctgct gggagcacta caacccactg gcgcgctaca gcctgatgac tcagcacgag     540
gagatccatt gcagccagtt ccctggttca ctggtggggc cgatttttgc cgagcagatg     600
gacgtcacca ttcgccatca tgcactggag tccggttgct ttgtcatcaa tgccaccggc     660
tggctgaccg aggagcagat caacgagctg accagcgacc cggcgttaca aaaggggctg      720
cgtggtggct gcaacaccgc catcatctcg ccggaaggcc gccatctggt gccgccactg     780
accgaaggtg aggggatttt gattgccgat ctggacatgg ccctgatcac caaacgcaaa     840
cgcatgatgg attctgtcgg ccactatgcc cgaccggaat tactcagcct gcgcctcgat     900
gcgacgcctg cccgttatgt ggtggcgcgt gataatgagt ccgaaaccgg aggaggcaac     960
gatgcagaac gtaccgtcta cgcgccagca gctgatcact ga                       1002

<210> 184
<211> 333
<212> PRT
<213> Unknown

<220>
<223> Obtained from an environmental sample

<400> 184
Met Ala Glu Ser Arg Ile Ile Arg Ala Ala Ala Ala Gln Ile Ala Pro
 1               5                  10                  15
Asp Leu His Glu Ala Ser Lys Thr Leu Ala Arg Val Leu Asp Ala Ile
            20                  25                  30
Asp Gln Ala Ala Ala Gln Gly Ala Glu Ile Ile Val Phe Pro Glu Thr
        35                  40                  45
Phe Val Pro Tyr Tyr Pro Tyr Phe Ser Phe Ile Thr Pro Ala Met Thr
    50                  55                  60
Ala Gly Ala Ala His Leu Lys Leu Tyr Asp Gln Ala Val Val Val Pro
65                  70                  75                  80
Gly Pro Ile Thr His Ala Val Gly Glu Arg Ala Arg Leu Arg Asn Ile
                85                  90                  95

Val Val Val Leu Gly Val Asn Glu Arg Asp His Gly Thr Leu Tyr Asn
                100                 105                 110
Thr Gln Leu Val Phe Asp Ala Ser Gly Glu Leu Val Leu Lys Arg Arg
        115                 120                 125
Lys Ile Thr Pro Thr Tyr His Glu Arg Met Ile Trp Gly Gln Gly Asp
    130                 135                 140
Gly Ala Gly Leu Lys Val Val Asp Ser Ala Val Gly Arg Ile Gly Ala
145                 150                 155                 160
Leu Ala Cys Trp Glu His Tyr Asn Pro Leu Ala Arg Tyr Ser Leu Met
                165                 170                 175
Thr Gln His Glu Glu Ile His Cys Ser Gln Phe Pro Gly Ser Leu Val
                180                 185                 190
Gly Pro Ile Phe Ala Glu Gln Met Asp Val Thr Ile Arg His His Ala
        195                 200                 205
Leu Glu Ser Gly Cys Phe Val Ile Asn Ala Thr Gly Trp Leu Thr Glu
    210                 215                 220
Glu Gln Ile Asn Glu Leu Thr Ser Asp Pro Ala Leu Gln Lys Gly Leu
225                 230                 235                 240
Arg Gly Gly Cys Asn Thr Ala Ile Ile Ser Pro Glu Gly Arg His Leu
                245                 250                 255
Val Pro Pro Leu Thr Glu Gly Glu Gly Ile Leu Ile Ala Asp Leu Asp
                260                 265                 270
Met Ala Leu Ile Thr Lys Arg Lys Arg Met Met Asp Ser Val Gly His
        275                 280                 285
Tyr Ala Arg Pro Glu Leu Leu Ser Leu Arg Leu Asp Ala Thr Pro Ala
    290                 295                 300
Arg Tyr Val Val Ala Arg Asp Asn Glu Ser Glu Thr Gly Gly Gly Asn
305                 310                 315                 320
Asp Ala Glu Arg Thr Val Tyr Ala Pro Ala Ala Asp His
                325                 330

<210> 185
<211> 1017
<212> DNA
<213> Unknown

<220>
<223> Obtained from an environmental sample

<400> 185
atgggcattg aacatccgaa atacaaggtc gcggtggtgc aggcggcccc cgcctggctc      60
gatctcgacg gctcggtcga taagtcgatc gcgctgatca aggaggcggc cgagaagggg     120
gcgaagctga tcgcctttcc cgaggccttc atccccggtt acccctggca tatctggatg     180
gactcgccgg cctgggcgat cggccgcggc ttcgtgcagc gttatttcga caattcgctg     240
tcctatgaca gtccgcaggc cgagcggctg cgcgatgcgg tgaagaaggc gaagctcacc     300
gccgtgttcg gactgtccga gcgcgacggc ggcagcctct acctcgcgca atggctgatc     360
gggcccgatg gcgagaccat cgccaagcgc cgcaagctgc ggccgaccca cgccgaacgt     420
accgtctatg gcgaaggcga cggcagcgat cttgccgtgc atgcgcgcgc cgacatcggc     480
cggatcggcg cgctctgctg ctgggagcat ctgcagccac tgtcgaaata cgcgatgtac     540
gcccagaacg aacaggtcca tgtcgcagcc tggcccagct tctcgctgta cgaccccttc     600
gcgccggcgt taggggccga ggtcaacaac gcggcctccc gcgtctatgc ggtggaaggc     660
tcctgcttcg tgctcgcgcc gtgcgcgacg gtgtcgcagg cgatgatcga cgagctctgc     720
gaccggcccg acaagaacgc gctgctgcac gtcggcggcg gctttgccgc gatctatggc     780
cccgacggca gccagatcgg cgacaagctg gcgccggacc aggaggggct gctgatcgcc     840
gagatcgacc ttggcgccat cggtgtcgcc aagaacgccg ccgatcccgc cgggcactat     900
tcgcgtcccg acgtgacgcg gttgctgctc aacaagaagc gataccagcg cgtcgagcag     960
ttcgcgctgc cggtcgacac cgtcgagccg gcggatatcg gcgcagcggc gagctga       1017

<210> 186
<211> 338
<212> PRT
<213> Unknown

<220>
<223> Obtained from an environmental sample

<400> 186
Met Gly Ile Glu His Pro Lys Tyr Lys Val Ala Val Val Gln Ala Ala
1               5                   10                  15
Pro Ala Trp Leu Asp Leu Asp Gly Ser Val Asp Lys Ser Ile Ala Leu
            20                  25                  30
Ile Lys Glu Ala Ala Glu Lys Gly Ala Lys Leu Ile Ala Phe Pro Glu
        35                  40                  45
Ala Phe Ile Pro Gly Tyr Pro Trp His Ile Trp Met Asp Ser Pro Ala
    50                  55                  60
Trp Ala Ile Gly Arg Gly Phe Val Gln Arg Tyr Phe Asp Asn Ser Leu
65                  70                  75                  80
Ser Tyr Asp Ser Pro Gln Ala Glu Arg Leu Arg Asp Ala Val Lys Lys
                85                  90                  95
Ala Lys Leu Thr Ala Val Phe Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Ala Arg Ala Asp Ile Gly
145                 150                 155                 160
Arg Ile Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Pro Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys Asn Ala Leu Leu His Val Gly Gly Gly Phe Ala
                245                 250                 255
Ala Ile Tyr Gly Pro Asp Gly Ser Gln Ile Gly Asp Lys Leu Ala Pro
            260                 265                 270
Asp Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Lys Arg Tyr Gln Arg Val Glu Gln
305                 310                 315                 320
Phe Ala Leu Pro Val Asp Thr Val Glu Pro Ala Asp Ile Gly Ala Ala
                325                 330                 335
Ala Ser


<210> 187
<211> 1059

<212> DNA
<213> Unknown

<220>
<223> Obtained from an environmental sample

<400> 187
atgggcatca atcatcccaa gtacaaagtg gccgtcgtgc aagcggcacc tgtctggctc 60
gacctggatg gaacagtcga caagtgcatt cggctgatag gcgaggccgc tgagaaggga 120
tgcaagctca ttgcatttcc cgagacgttc atcccggggt acccctggca catctggatg 180
ggagctccgg cctggacgat cgggcgcgga ttcgtgcagc gatacttcga caattcgctt 240
gcgtacgaca gtccgcaggc aaacaagctt cgcgccgcgg tgaagcgcgc cggagtgacg 300
gcagttctcg gcttgtcgga gcgccgcgga ggctccctgt acatcgccca gtggctcatc 360
ggacctgatg gcgagaccat cgctcaacgg cgaaagctgc gccccaccca tgcggagcgc 420
accgtcttcg gcgagggcga tggcagcgat ttggcggtgc acagccgccc cgacatcggc 480
cgactgggtg ccctttgctg ctgggaacat ctccagcctt tgaccaagta cgcgatgtac 540
gcgcaagacg agcaagtgca cgtcgctgca tggccgagct tctcgatgta cgagcctttc 600
gcgcacgccc tggggtggga gacgaacaac gcggtgagca aggtgtacgc ggtcgaaggt 660
tcgtgctacg tcctggcccc ctgcgccatc atctctcagg cgatggtgga cgaactcgtc 720
gacagcgagg acaagaagcc gctggttcat gccggcgggg ggcatgcggt gatctatggt 780
cccgatggca ccctgcttac tcccaagctt gcagaagacg aggagggcct actgatcgcg 840
gagatcgatc tgggggcaat cggggtcgcc aagaacgcgg cagaccccgc cggccactac 900
tcgcggcccg atgtcacccg cctgctcttc aacaaccggc cggccaagcg cgtggagacg 960
atgctgctcc cggtcgacgc ggcagaagtc gtggagccgg cggacggagc gctcaatgcg 1020
tccgagggac gccagcgaca gttcaagctg cccgcctag 1059

<210> 188
<211> 352
<212> PRT
<213> Unknown

<220>
<223> Obtained from an environmental sample

<400> 188
Met Gly Ile Asn His Pro Lys Tyr Lys Val Ala Val Val Gln Ala Ala
1               5                   10                  15
Pro Val Trp Leu Asp Leu Asp Gly Thr Val Asp Lys Cys Ile Arg Leu
            20                  25                  30
Ile Gly Glu Ala Ala Glu Lys Gly Cys Lys Leu Ile Ala Phe Pro Glu
        35                  40                  45
Thr Phe Ile Pro Gly Tyr Pro Trp His Ile Trp Met Gly Ala Pro Ala
    50                  55                  60
Trp Thr Ile Gly Arg Gly Phe Val Gln Arg Tyr Phe Asp Asn Ser Leu
65                  70                  75                  80
Ala Tyr Asp Ser Pro Gln Ala Asn Lys Leu Arg Ala Ala Val Lys Arg
                85                  90                  95
Ala Gly Val Thr Ala Val Leu Gly Leu Ser Glu Arg Arg Gly Gly Ser
            100                 105                 110
Leu Tyr Ile Ala Gln Trp Leu Ile Gly Pro Asp Gly Glu Thr Ile Ala
        115                 120                 125
Gln Arg Arg Lys Leu Arg Pro Thr His Ala Glu Arg Thr Val Phe Gly
    130                 135                 140
Glu Gly Asp Gly Ser Asp Leu Ala Val His Ser Arg Pro Asp Ile Gly
145                 150                 155                 160
Arg Leu Gly Ala Leu Cys Cys Trp Glu His Leu Gln Pro Leu Thr Lys
                165                 170                 175

Tyr Ala Met Tyr Ala Gln Asp Glu Gln Val His Val Ala Ala Trp Pro
            180                 185                 190
Ser Phe Ser Met Tyr Glu Pro Phe Ala His Ala Leu Gly Trp Glu Thr
        195                 200                 205
Asn Asn Ala Val Ser Lys Val Tyr Ala Val Glu Gly Ser Cys Tyr Val
    210                 215                 220
Leu Ala Pro Cys Ala Ile Ile Ser Gln Ala Met Val Asp Glu Leu Val
225                 230                 235                 240
Asp Ser Glu Asp Lys Lys Pro Leu Val His Ala Gly Gly Gly His Ala
                245                 250                 255
Val Ile Tyr Gly Pro Asp Gly Thr Leu Leu Thr Pro Lys Leu Ala Glu
            260                 265                 270
Asp Glu Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Phe Asn Asn Arg Pro Ala Lys Arg Val Glu Thr
305                 310                 315                 320
Met Leu Leu Pro Val Asp Ala Ala Glu Val Val Glu Pro Ala Asp Gly
                325                 330                 335
Ala Leu Asn Ala Ser Glu Gly Arg Gln Arg Gln Phe Lys Leu Pro Ala
            340                 345                 350

<210> 189
<211> 1005
<212> DNA
<213> Unknown

<220>
<223> Obtained from an environmental sample

<400> 189
atgcaagaca cgaaattcaa agttgcagtc gtccaggccg cgccggtatt catggatgcg      60
ccagcctccg tggccaaggc gatcggtttc atccaggagg cgggcgcagc cggggcgaag     120
ctgctggcgt tcccggaggt ctggattccg ggctaccctt ggtggctttg gctcgggacg     180
ccggcgtggg gaatgcagtt tgtgccgcgc tatcacgcca attcgctgcg tgctgatgga     240
cccgaaatcc tcgctctttg tgcggccgcc gccgaagcga agatcaacgt cgtgatgggc     300
ttctccgaaa tcgacggagg aacgctctac ctaagtcagg ttttcatcag cgatgcgggc     360
aagatcatct tcaagcgccg aaagctcaag ccgacccacg tcgaacgtac gctatttggt     420
gaaggagatg ggtctgattt ccgagtcgtc gacagcagcg tcgggcgcct cggagccctg     480
tgctgtgccg aacacattca gccgttgtcg aaatacgcca tgtacgcgat gaacgagcaa     540
attcatgtgg cgtcgtggcc atctttcacg ctctatcgcg gcaaagccta cgctttgggt     600
catgaggtga atcttgccgc cagccaaatc tacgcgctcg aaggaggttg cttcgtcttg     660
catgccacgg caattaccgg tcaggatatg ttcgacatgc tttgcgacac tccggaaagg     720
gcggatttgc tgaatgcgga gggagcaaag ccgggtggag gctattcgat gatttttggt     780
cccgatggtc agccgatgtg cgagcatctg ccgcaggaca aggaaggcat cctctatgcc     840
ggcgtagacc tgtcgatgat tgcgatcgcc aaagcggcct acgatcctac ggggcactac     900
gcccgcggtg atgtcgtccg tctcatggtc aaccgcagcc cccgtcgcac gagcgtcagc     960
ttcagcgaag acgagaacgc ggcggtcact ttcaccgaga cctga                    1005

<210> 190
<211> 334
<212> PRT
<213> Unknown

<220>
<223> Obtained from an environmental sample

<400> 190
Met Gln Asp Thr Lys Phe Lys Val Ala Val Val Gln Ala Ala Pro Val
1               5                   10                  15
Phe Met Asp Ala Pro Ala Ser Val Ala Lys Ala Ile Gly Phe Ile Gln
            20                  25                  30
Glu Ala Gly Ala Ala Gly Ala Lys Leu Leu Ala Phe Pro Glu Val Trp
        35                  40                  45
Ile Pro Gly Tyr Pro Trp Trp Leu Trp Leu Gly Thr Pro Ala Trp Gly
    50                  55                  60
Met Gln Phe Val Pro Arg Tyr His Ala Asn Ser Leu Arg Ala Asp Gly
65                  70                  75                  80
Pro Glu Ile Leu Ala Leu Cys Ala Ala Ala Ala Glu Ala Lys Ile Asn
                85                  90                  95
Val Val Met Gly Phe Ser Glu Ile Asp Gly Gly Thr Leu Tyr Leu Ser
            100                 105                 110
Gln Val Phe Ile Ser Asp Ala Gly Lys Ile Ile Phe Lys Arg Arg Lys
        115                 120                 125
Leu Lys Pro Thr His Val Glu Arg Thr Leu Phe Gly Glu Gly Asp Gly
    130                 135                 140
Ser Asp Phe Arg Val Val Asp Ser Ser Val Gly Arg Leu Gly Ala Leu
145                 150                 155                 160
Cys Cys Ala Glu His Ile Gln Pro Leu Ser Lys Tyr Ala Met Tyr Ala
                165                 170                 175
Met Asn Glu Gln Ile His Val Ala Ser Trp Pro Ser Phe Thr Leu Tyr
            180                 185                 190
Arg Gly Lys Ala Tyr Ala Leu Gly His Glu Val Asn Leu Ala Ala Ser
        195                 200                 205
Gln Ile Tyr Ala Leu Glu Gly Gly Cys Phe Val Leu His Ala Thr Ala
    210                 215                 220
Ile Thr Gly Gln Asp Met Phe Asp Met Leu Cys Asp Thr Pro Glu Arg
225                 230                 235                 240
Ala Asp Leu Leu Asn Ala Glu Gly Ala Lys Pro Gly Gly Gly Tyr Ser
                245                 250                 255
Met Ile Phe Gly Pro Asp Gly Gln Pro Met Cys Glu His Leu Pro Gln
            260                 265                 270
Asp Lys Glu Gly Ile Leu Tyr Ala Gly Val Asp Leu Ser Met Ile Ala
        275                 280                 285
Ile Ala Lys Ala Ala Tyr Asp Pro Thr Gly His Tyr Ala Arg Gly Asp
    290                 295                 300
Val Val Arg Leu Met Val Asn Arg Ser Pro Arg Arg Thr Ser Val Ser
305                 310                 315                 320
Phe Ser Glu Asp Glu Asn Ala Ala Val Thr Phe Thr Glu Thr
                325                 330

<210> 191
<211> 945
<212> DNA
<213> Unknown

<220>
<223> Obtained from an environmental sample

<400> 191
atgaaaaagt tagctgtggt tcaacgtgcg tcaggatttt tagataagca gcagagcatc      60
gcgttggcgg tggaaagtat tcagtctgct gcgaataatg gcgcagagct tgttgttttt     120
acggaagcct ttattcctgg ttatcctgtc tggttatggc gtctgcgccc tggcaaagac     180

tgggggacaa cagacagtct ttatcaacgc ttaataagca acgcggttga tttaagctca 240
tcggatttgg atccgattta tgaagcggca aaacgtcatc acgtcacggt tgtatgcggc 300
attaatgaac gcgactccag cgtcagccga acaacgctat acaacactta catcacggtt 360
tgtcatgagg gcaatctcat caatgttcat cgaaaactga tgccgaccaa cccagagaga 420
atggtgtggg gctttggtga tgcgactgga ttaagggtag tagacactcc tgtcggaagg 480
attggctcac tcgtttgctg ggagaactac atgccgttgg cacgctatgc actttatgct 540
cagggcgtcg aaatttacat tgcgcctact tatgacagtg gctcggactg gactgaaagc 600
ttgcgccata tcgccagaga gggcagatgc tacgttgtcg gcagcggtaa cttgttgaga 660
gccagcgacc tgcctgatga ttttccagaa aaagaaaccc tctatcctga taaagacgag 720
tggattaacg gcggagactc taccgttatc gctcccggcg gtgaaacatt agttgctccg 780
ctgcatgcag aggaaggcat actgtattgc gatattgata ctgataaagt ggcggcggct 840
cggcgttctt tcgacgttgc aggccattac tctcgcccag acatatttac actcaacgta 900
aatcgagcgc cgcaaacatc tctgcgtatc agggaagccg agtaa 945

<210> 192
<211> 314
<212> PRT
<213> Unknown

<220>
<223> Obtained from an environmental sample

<400> 192
Met Lys Lys Leu Ala Val Val Gln Arg Ala Ser Gly Phe Leu Asp Lys
1               5                   10                  15
Gln Gln Ser Ile Ala Leu Ala Val Glu Ser Ile Gln Ser Ala Ala Asn
            20                  25                  30
Asn Gly Ala Glu Leu Val Val Phe Thr Glu Ala Phe Ile Pro Gly Tyr
        35                  40                  45
Pro Val Trp Leu Trp Arg Leu Arg Pro Gly Lys Asp Trp Gly Thr Thr
    50                  55                  60
Asp Ser Leu Tyr Gln Arg Leu Ile Ser Asn Ala Val Asp Leu Ser Ser
65                  70                  75                  80
Ser Asp Leu Asp Pro Ile Tyr Glu Ala Ala Lys Arg His His Val Thr
                85                  90                  95
Val Val Cys Gly Ile Asn Glu Arg Asp Ser Ser Val Ser Arg Thr Thr
            100                 105                 110
Leu Tyr Asn Thr Tyr Ile Thr Val Cys His Glu Gly Asn Leu Ile Asn
        115                 120                 125
Val His Arg Lys Leu Met Pro Thr Asn Pro Glu Arg Met Val Trp Gly
    130                 135                 140
Phe Gly Asp Ala Thr Gly Leu Arg Val Val Asp Thr Pro Val Gly Arg
145                 150                 155                 160
Ile Gly Ser Leu Val Cys Trp Glu Asn Tyr Met Pro Leu Ala Arg Tyr
                165                 170                 175
Ala Leu Tyr Ala Gln Gly Val Glu Ile Tyr Ile Ala Pro Thr Tyr Asp
            180                 185                 190
Ser Gly Ser Asp Trp Thr Glu Ser Leu Arg His Ile Ala Arg Glu Gly
        195                 200                 205
Arg Cys Tyr Val Val Gly Ser Gly Asn Leu Leu Arg Ala Ser Asp Leu
    210                 215                 220
Pro Asp Asp Phe Pro Glu Lys Glu Thr Leu Tyr Pro Asp Lys Asp Glu
225                 230                 235                 240
Trp Ile Asn Gly Gly Asp Ser Thr Val Ile Ala Pro Gly Gly Glu Thr
                245                 250                 255
Leu Val Ala Pro Leu His Ala Glu Glu Gly Ile Leu Tyr Cys Asp Ile
            260                 265                 270

Asp Thr Asp Lys Val Ala Ala Ala Arg Arg Ser Phe Asp Val Ala Gly
        275                 280                 285
His Tyr Ser Arg Pro Asp Ile Phe Thr Leu Asn Val Asn Arg Ala Pro
    290                 295                 300
Gln Thr Ser Leu Arg Ile Arg Glu Ala Glu
305                 310

<210> 193
<211> 966
<212> DNA
<213> Unknown

<220>
<223> Obtained from an environmental sample

<400> 193
atgtcaaacg agaaccacaa ccaaacattc aaagttgccg cggtgcaggc cacacctgta      60
ttcctcgatc gtgaagcgac catcgacaaa gcttgtgagt tgattgctgc agccggcaat     120
gaaggagcgc ggctggttgt cttcccggag gcattcatcc catcctaccc agattgggta     180
tgggcaatcc caccgggcga agaaggcgtg ctcaatgagt tgtacgcgga actgctctcc     240
aattcggtca cgattcccag tgatgtgacg gatagactgt gccgagccgc gagacttgcc     300
aatgcctacg tagtgatggg gatgagcgaa cgcaatgccg aggccagtgg cgcaagcctg     360
tataacacgc tgttgtacat cgatgcgcag ggcgagattc tgggcaaaca tcgaaagctg     420
gtgcccacag gcggcgaacg gctggtgtgg gcgcagggcg atggcagcac gctgcaggtc     480
tacgatactc cactgggtaa actcggcggt ttgatttgct gggagaatta tatgccgctg     540
gcccgctaca ccatgtacgc atggggcaca caaatctatg ttgcggcgac atgggatcgc     600
gggcaaccct ggctctccac tttacggcat atcgccaaag aaggcagggt gtacgtgatc     660
ggctgctgta tcgtgatgcg caaagacgat atcccagatc gttacccgat gaagcagaag     720
ttttacgcgg aggccgatga gtggatcaac ataggggaca gcgcaatcgt caatcctgaa     780
gggcagttta gcgccgggcc ggtacgcaaa caggaagaga ttctctacgc ggaaattgat     840
ccgcgcatgg tgcaaggccc gaagtggatg ctcgacgtag cagggcacta cgcgaggccg     900
gacgtattcc agttgacggt gcatacggat gcgaggcaga tgatcaggtt ggaacacgat     960
gtttaa                                                                966

<210> 194
<211> 321
<212> PRT
<213> Unknown

<220>
<223> Obtained from an environmental sample

<400> 194
Met Ser Asn Glu Asn His Asn Gln Thr Phe Lys Val Ala Ala Val Gln
 1               5                  10                  15
Ala Thr Pro Val Phe Leu Asp Arg Glu Ala Thr Ile Asp Lys Ala Cys
            20                  25                  30
Glu Leu Ile Ala Ala Ala Gly Asn Glu Gly Ala Arg Leu Val Val Phe
        35                  40                  45
Pro Glu Ala Phe Ile Pro Ser Tyr Pro Asp Trp Val Trp Ala Ile Pro
    50                  55                  60
Pro Gly Glu Glu Gly Val Leu Asn Glu Leu Tyr Ala Glu Leu Leu Ser
65                  70                  75                  80
Asn Ser Val Thr Ile Pro Ser Asp Val Thr Asp Arg Leu Cys Arg Ala
                85                  90                  95
Ala Arg Leu Ala Asn Ala Tyr Val Val Met Gly Met Ser Glu Arg Asn
            100                 105                 110

Ala Glu Ala Ser Gly Ala Ser Leu Tyr Asn Thr Leu Leu Tyr Ile Asp
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Thr Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Val Met Arg Lys Asp Asp Ile Pro Asp Arg Tyr Pro Met Lys Gln Lys
225                 230                 235                 240
Phe Tyr Ala Glu Ala Asp Glu Trp Ile Asn Ile Gly Asp Ser Ala Ile
                245                 250                 255
Val Asn Pro Glu Gly Gln Phe Ser Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asp Ala Arg Gln Met Ile Arg Leu Glu His Asp
305                 310                 315                 320
Val

<210> 195
<211> 993
<212> DNA
<213> Unknown

<220>
<223> Obtained from an environmental sample

<400> 195
atgaaagtcg tcaaagccgc cgcggtacag ttcagcccgg tgctctatag ccgtgaggca      60
accgtcgcca aggtcgtgca gaagatccac gaactcggcc agaaaggcgt gcagttcgcc     120
accttccccg aaacggtcgt gccttattac ccttactttg cggccgtcca gacgggcatc     180
gagcttctct cgggcaccga acatctgcgc ctgctcgaac aggctgtgac tgtcccgtcc     240
gctgctaccg acgcgatcgg cgaagccgcg cgaaaggccg gcatggtcgt gtccattggc     300
gtcaatgagc gcgatggcgg cacgctgtac aacgcacaac tgctcttcga tgccgacggt     360
acgctgatcc agcgccgccg caagatcacg ccgacgcatt tcgaacgcat gatctggggc     420
cagggagatg gctcgggctt gcgtgcagtc gacagcgccg tcggccgcgt cggccagctc     480
gcatgtttcg agcacaacaa cccgctcgcc cgctacgcaa tgatcgccga cggcgagcag     540
atccattcgg cgatgtaccc tggctcggcc tttggcgagg gcttcgccca gcgtatggaa     600
atcaacatcc gccagcatgc gctcgagtcc gccgctttcg tcgtcaacgc aacagcgtgg     660
ctggacgccg accagcaggc gcaaatcatg aaggacaccg gttgtggaat cggtccgatc     720
acgggcggct gcttcaccac gatcgtctct cctgacggca tgctgatggc cgagccgctt     780
cgctcgggtg aaggcgaagt gatcgtcgat ctcgacttca cgcagatcga ccgccgcaag     840
atgctgatgg actcggccgg ccactacaac cgccctgaac tgctgagtct gatgatcgac     900
cgtacgccga ccgcgcatgt tcacgaacgc gcttcgcacc cgatgatcgt caacgaccag     960
ggttccgacg atctgcgcac ccaggctgca tga                                  993

<210> 196

<211> 330
<212> PRT
<213> Unknown

<220>
<223> Obtained from an environmental sample

<400> 196
Met Lys Val Val Lys Ala Ala Ala Val Gln Phe Ser Pro Val Leu Tyr
1               5                   10                  15
Ser Arg Glu Ala Thr Val Ala Lys Val Val Gln Lys Ile His Glu Leu
            20                  25                  30
Gly Gln Lys Gly Val Gln Phe Ala Thr Phe Pro Glu Thr Val Val Pro
        35                  40                  45
Tyr Tyr Pro Tyr Phe Ala Ala Val Gln Thr Gly Ile Glu Leu Leu Ser
    50                  55                  60
Gly Thr Glu His Leu Arg Leu Leu Glu Gln Ala Val Thr Val Pro Ser
65                  70                  75                  80
Ala Ala Thr Asp Ala Ile Gly Glu Ala Ala Arg Lys Ala Gly Met Val
                85                  90                  95
Val Ser Ile Gly Val Asn Glu Arg Asp Gly Gly Thr Leu Tyr Asn Ala
            100                 105                 110
Gln Leu Leu Phe Asp Ala Asp Gly Thr Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Ala Val Gly Arg Val Gly Gln Leu
145                 150                 155                 160
Ala Cys Phe Glu His Asn Asn Pro Leu Ala Arg Tyr Ala Met Ile Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Ala Ala Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Thr Gly Gly Cys Phe Thr Thr Ile Val Ser Pro Asp Gly Met Leu Met
                245                 250                 255
Ala Glu Pro Leu Arg Ser Gly Glu Gly Glu Val Ile Val Asp Leu Asp
            260                 265                 270
Phe Thr Gln Ile Asp Arg Arg Lys Met Leu Met Asp Ser Ala Gly His
        275                 280                 285
Tyr Asn Arg Pro Glu Leu Leu Ser Leu Met Ile Asp Arg Thr Pro Thr
    290                 295                 300
Ala His Val His Glu Arg Ala Ser His Pro Met Ile Val Asn Asp Gln
305                 310                 315                 320
Gly Ser Asp Asp Leu Arg Thr Gln Ala Ala
                325                 330

<210> 197
<211> 1017
<212> DNA
<213> Unknown

<220>

<223> Obtained from an environmental sample

<400> 197
atgggcatcg aacatccaaa ataccgcgtt gccgccgtgc aggctgcgcc ggcctggctc 60
gacctcgatc gctcgatcga caaggctatt gcactgatcg aggaggccgc cgcgaacggc 120
gccagattga tcgcattccc ggaggtcttc atccccggct acccctggca tatctggctc 180
gactcgccgg cctgggcgat cggccgcggc ttcgtgcagc gttatttcga caattcgctc 240
gcttatgata gtccgcaggc cgagcggctc cgcgcagcgg tccgcaaggc gcgcctgacc 300
gccgtgatcg gcctttcgga gcggagcggc ggcagcctct acatcgcgca atggctcgtt 360
ggccccgacg gcgagaccat cgcgaagcgc cgcaagctcc gtccgacgca tgccgagcgc 420
acggtctatg gcgagggcga cggcagcgat ctggcggtcc atgaccggcc cgatatcgga 480
cggctcggcg cgctgtgctg ctgggaacat ctgcaaccgt tgtcgaaata tgcgatgtat 540
gcccagaacg agcaggtcca tgtggcgtca tggccgagtt tttcgctcta cgatcccttt 600
gccccggcgc tcggcgcgga ggtgaacaat gcggcctccc gggtctatgc ggtcgaaggc 660
tcctgcttcg tgctggcgcc gtgcgcgacc gtctcgcagg ccatgatcga tgagctgtgc 720
gaccggcccg acaagcacgc gctgctccat gccggcggtg gctttgccgc gatctacggg 780
cccgacggca gttcgctggc cgaaaagctc gcgccggacc aggagggcct gctttacgcc 840
gacatcgatc tcggcgcgat cggcgtcgcg aagaacgccg ccgacccggc agggcattat 900
tcgcggcccg atgtcacgcg gctgctgctg aacaacaagc cctacaagcg cgtggagcat 960
tttgctttgc ccggcgatac cgtggcgcct gccgatgtgg atgcggcggc gagctga 1017

<210> 198
<211> 338
<212> PRT
<213> Unknown

<220>
<223> Obtained from an environmental sample

<400> 198
Met Gly Ile Glu His Pro Lys Tyr Arg Val Ala Ala Val Gln Ala Ala
1               5                   10                  15
Pro Ala Trp Leu Asp Leu Asp Arg Ser Ile Asp Lys Ala Ile Ala Leu
            20                  25                  30
Ile Glu Glu Ala Ala Ala Asn Gly Ala Arg Leu Ile Ala Phe Pro Glu
        35                  40                  45
Val Phe Ile Pro Gly Tyr Pro Trp His Ile Trp Leu Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Arg Leu Arg Ala Ala Val Arg Lys
                85                  90                  95
Ala Arg Leu Thr Ala Val Ile Gly Leu Ser Glu Arg Ser Gly Gly Ser
            100                 105                 110
Leu Tyr Ile Ala Gln Trp Leu Val Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asp Arg Pro Asp Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ser Trp Pro
            180                 185                 190
Ser Phe Ser Leu Tyr Asp Pro Phe Ala Pro Ala Leu Gly Ala Glu Val
        195                 200                 205
Asn Asn Ala Ala Ser Arg Val Tyr Ala Val Glu Gly Ser Cys Phe Val

210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Ala Gly Gly Gly Phe Ala
                245                 250                 255
Ala Ile Tyr Gly Pro Asp Gly Ser Ser Leu Ala Glu Lys Leu Ala Pro
            260                 265                 270
Asp Gln Glu Gly Leu Leu Tyr Ala Asp Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Asn Lys Pro Tyr Lys Arg Val Glu His
305                 310                 315                 320
Phe Ala Leu Pro Gly Asp Thr Val Ala Pro Ala Asp Val Asp Ala Ala
                325                 330                 335
Ala Ser

<210> 199
<211> 993
<212> DNA
<213> Unknown

<220>
<223> Obtained from an environmental sample

<400> 199
atgtcaaacg cactgaagcc gttcaaaatc gccgccgttc aggcgacgcc ggtttttctg      60
aaccgggaag ccacggcgga gaaggccgcg gctttgatcc gcgaagcggg aagcgccgga     120
gccaagctca tcgttttccc ggaatcgttt attccggcct atccggactg ggtctgggtg     180
gtcccctcgg ggagggatcg ccttctcagc ggcctctacg gggagatgct cgaaaacgcc     240
gtggaaatcc ccggcccggc cacggggcat atcggccggg cggcgaagga atcgggcgct     300
tatgtcgtca tgggcgtgac cgagcgggac acggaggcga gcggagccag tttgttcaac     360
accttgattt atttcggtcc gaccggggaa attttgggca aacaccggaa gctggttccc     420
accgggggcg aacggatcgt ctgggcccag ggggacggaa gcaccctgga ggtctacgat     480
acgcccctgg gaaaactggg cgggctgatc tgctgggaaa actacatgcc cctggcccgg     540
tacgccatgt acgcctgggg aacccagctt tacgtggccg ccacctggga ccgaggcgaa     600
ccctggcttt cgacgcttcg gcatatcgcc aaggaagggc gggtgtatgt catcgggtgc     660
tgcatcgcca tgcggaaagg ggatatcccg gatcggttcg aacacaaggg gctctacgcc     720
cccgaccggg actggatcaa ccccggcgac agcgcgatcg tcaaccccca gggggagatg     780
atcgccgggc ccgcttccaa taaggaagag atcctttatg cggaagtcga cccgcagatg     840
atgcgcgggc ccaaatggat gctcgatgtg gccggccatt acgcgcggcc cgatgtcttc     900
gagctcaccg tccgccggga accgcggccg atgatccgcg tggcgggagg cgcgggcggg     960
accgaaccca aagagaagaa gaccgccggc tga                                  993

<210> 200
<211> 330
<212> PRT
<213> Unknown

<220>
<223> Obtained from an environmental sample

<400> 200
Met Ser Asn Ala Leu Lys Pro Phe Lys Ile Ala Ala Val Gln Ala Thr
1               5                   10                  15
Pro Val Phe Leu Asn Arg Glu Ala Thr Ala Glu Lys Ala Ala Ala Leu

20                  25                  30
Ile Arg Glu Ala Gly Ser Ala Gly Ala Lys Leu Ile Val Phe Pro Glu
        35                  40                  45
Ser Phe Ile Pro Ala Tyr Pro Asp Trp Val Trp Val Val Pro Ser Gly
    50                  55                  60
Arg Asp Arg Leu Leu Ser Gly Leu Tyr Gly Glu Met Leu Glu Asn Ala
65                  70                  75                  80
Val Glu Ile Pro Gly Pro Ala Thr Gly His Ile Gly Arg Ala Ala Lys
                85                  90                  95
Glu Ser Gly Ala Tyr Val Val Met Gly Val Thr Glu Arg Asp Thr Glu
            100                 105                 110
Ala Ser Gly Ala Ser Leu Phe Asn Thr Leu Ile Tyr Phe Gly Pro Thr
        115                 120                 125
Gly Glu Ile Leu Gly Lys His Arg Lys Leu Val Pro Thr Gly Gly Glu
    130                 135                 140
Arg Ile Val Trp Ala Gln Gly Asp Gly Ser Thr Leu Glu Val Tyr Asp
145                 150                 155                 160
Thr Pro Leu Gly Lys Leu Gly Gly Leu Ile Cys Trp Glu Asn Tyr Met
                165                 170                 175
Pro Leu Ala Arg Tyr Ala Met Tyr Ala Trp Gly Thr Gln Leu Tyr Val
            180                 185                 190
Ala Ala Thr Trp Asp Arg Gly Glu Pro Trp Leu Ser Thr Leu Arg His
        195                 200                 205
Ile Ala Lys Glu Gly Arg Val Tyr Val Ile Gly Cys Cys Ile Ala Met
    210                 215                 220
Arg Lys Gly Asp Ile Pro Asp Arg Phe Glu His Lys Gly Leu Tyr Ala
225                 230                 235                 240
Pro Asp Arg Asp Trp Ile Asn Pro Gly Asp Ser Ala Ile Val Asn Pro
                245                 250                 255
Gln Gly Glu Met Ile Ala Gly Pro Ala Ser Asn Lys Glu Glu Ile Leu
            260                 265                 270
Tyr Ala Glu Val Asp Pro Gln Met Met Arg Gly Pro Lys Trp Met Leu
        275                 280                 285
Asp Val Ala Gly His Tyr Ala Arg Pro Asp Val Phe Glu Leu Thr Val
    290                 295                 300
Arg Arg Glu Pro Arg Pro Met Ile Arg Val Ala Gly Gly Ala Gly Gly
305                 310                 315                 320
Thr Glu Pro Lys Glu Lys Lys Thr Ala Gly
                325                 330

<210> 201
<211> 930
<212> DNA
<213> Unknown

<220>
<223> Obtained from an environmental sample

<400> 201
atgaccacga agagcatccg catcgcggcc gtacaggcgg ctcccgcgtt tctcgacctg      60
gctggtacgc tggaccggct cgaggcctgg gcccgcaagg ccgccgccac cggtgcccgc     120
gtcatcgcgt tccccgagac ctggctgccg ggctacccgg cgtggatcga ctcgtcgccg     180
gaggccgcga tctggggcca tcccggctcg cgcgacctgc accagcgcct gatggagaat     240
gccgtcgagg tcccgggccc cgcgaccgcg cgcatcgcga agctcgccgg cgagctcggc     300
gtgacgatcg tggtcggcgc gcacgagcgg gcggggaaca ccctctacaa cacggcgctg     360
acgttcgggc ccgagggcag gctgctcaat caccaccgga agctggtgcc gacctacagc     420
gaacggctgc tgtggggcta cggcgacggc gctggactgg tggcgccggc ggtggacggt     480

gtgaaggtcg gggcgctggt gtgctgggag cactggatgc cgctcacccg ccaggcgatg 540
cacgacgtcg gcgagcacgt gcacgtcgcc ctgtggcccg gcgtccacga gatgcaccag 600
gtggcctcgc ggcactatgc gttcgagggc cgctgtttcg tgatcgcggt cgggagcatc 660
ctgcgcgtgg accagatgcc gaagcagctg ccgccgctgg agaagtacgc gaagagcgcc 720
aaggggctga tgatcgcggg cggcagcgcc atcatcgcgc cgaacggccg ctacgtcgcg 780
gcgccggtgt acgacgagga gacgatcgtc accgccgact gcgacctcgg cgagatcccg 840
cgcgaggcgc agacgctcga tgtctcgggc cactacagcc ggccggacgt gttcagcttc 900
ggggtggtca gacaccggcc gcgtgcgtaa 930

<210> 202
<211> 309
<212> PRT
<213> Unknown

<220>
<223> Obtained from an environmental sample

<400> 202
Met Thr Thr Lys Ser Ile Arg Ile Ala Ala Val Gln Ala Ala Pro Ala
1               5                   10                  15
Phe Leu Asp Leu Ala Gly Thr Leu Asp Arg Leu Glu Ala Trp Ala Arg
            20                  25                  30
Lys Ala Ala Ala Thr Gly Ala Arg Val Ile Ala Phe Pro Glu Thr Trp
        35                  40                  45
Leu Pro Gly Tyr Pro Ala Trp Ile Asp Ser Ser Pro Glu Ala Ala Ile
    50                  55                  60
Trp Gly His Pro Gly Ser Arg Asp Leu His Gln Arg Leu Met Glu Asn
65                  70                  75                  80
Ala Val Glu Val Pro Gly Pro Ala Thr Ala Arg Ile Ala Lys Leu Ala
                85                  90                  95
Gly Glu Leu Gly Val Thr Ile Val Val Gly Ala His Glu Arg Ala Gly
            100                 105                 110
Asn Thr Leu Tyr Asn Thr Ala Leu Thr Phe Gly Pro Glu Gly Arg Leu
        115                 120                 125
Leu Asn His His Arg Lys Leu Val Pro Thr Tyr Ser Glu Arg Leu Leu
    130                 135                 140
Trp Gly Tyr Gly Asp Gly Ala Gly Leu Val Ala Pro Ala Val Asp Gly
145                 150                 155                 160
Val Lys Val Gly Ala Leu Val Cys Trp Glu His Trp Met Pro Leu Thr
                165                 170                 175
Arg Gln Ala Met His Asp Val Gly Glu His Val His Val Ala Leu Trp
            180                 185                 190
Pro Gly Val His Glu Met His Gln Val Ala Ser Arg His Tyr Ala Phe
        195                 200                 205
Glu Gly Arg Cys Phe Val Ile Ala Val Gly Ser Ile Leu Arg Val Asp
    210                 215                 220
Gln Met Pro Lys Gln Leu Pro Pro Leu Glu Lys Tyr Ala Lys Ser Ala
225                 230                 235                 240
Lys Gly Leu Met Ile Ala Gly Gly Ser Ala Ile Ile Ala Pro Asn Gly
                245                 250                 255
Arg Tyr Val Ala Ala Pro Val Tyr Asp Glu Glu Thr Ile Val Thr Ala
            260                 265                 270
Asp Cys Asp Leu Gly Glu Ile Pro Arg Glu Ala Gln Thr Leu Asp Val
        275                 280                 285
Ser Gly His Tyr Ser Arg Pro Asp Val Phe Ser Phe Gly Val Val Arg
    290                 295                 300
His Arg Pro Arg Ala

305

<210> 203
<211> 966
<212> DNA
<213> Unknown

<220>
<223> Obtained from an environmental sample

<400> 203
atgtcaagct tacccacatc cgcgttcacc gtcgccgccg cgcaagcgtc gccagtgttc      60
ctcgaccgcg acgcgacgct gcagaaggct tgcgggctga tcgccgacgc cgggcgcgcg     120
ggcgcgcgcc tgatcgtctt ccccgaagcc ttcatcccgg cctaccccga ttgggtgtgg     180
gcggtcccag ctggcgaaga ggggatgctg agcgagctct acgccgagct ggtcgcgaat     240
tcgctggcta ttccgagcga cgcgaccgat cggctatgtc gcgcggcgca ggccgcgcat     300
atcaatgtgg tcgtggggtt gagcgagcgc aatgtcgagg ccagcggcgc cagcctctac     360
aacacgctgc tgtatatcga cgcggcggga acgatcctgg gtaaacaccg caagcttgtg     420
ccgaccggcg gggagcgcct ggtctgggcg cagggcgacg gcagcacgct cgatgtgtac     480
gacaccgcgc tcggcaagct cggcggcctg atctgttggg aaaactacat gccgctggca     540
cgctacgcgc tgtacgcctg gggtgtgcaa atctatgtcg cggccacctg ggatcgcggc     600
gagccctggc tttctactct gcgacatatc gccaaggaag gccgtgtcta cgtgatcggc     660
tgtggcatgg cgctgcgcag agatgatatt cccgatcgct tcgctttcaa gcagcgcttc     720
tatgcccagg ccggcgaatg gatcaacgtc ggcgacagcg cgatcgtcaa cccgagcggc     780
gagtttattg ccggacctgt gcgcgaacgc gaggagattc tgtacgcgga ggtcgacccg     840
gagcagatga gcgggccaaa gtggatgctc gacgtggccg ggcactacgg gcggccggat     900
gtcttccggc tcagcgtcaa ccgggcgccg caccagatga tccagacgga gaaccgggag     960
acctga                                                                966

<210> 204
<211> 321
<212> PRT
<213> Unknown

<220>
<223> Obtained from an environmental sample

<400> 204
Met Ser Ser Leu Pro Thr Ser Ala Phe Thr Val Ala Ala Ala Gln Ala
1               5                   10                  15
Ser Pro Val Phe Leu Asp Arg Asp Ala Thr Leu Gln Lys Ala Cys Gly
            20                  25                  30
Leu Ile Ala Asp Ala Gly Arg Ala Gly Ala Arg Leu Ile Val Phe Pro
        35                  40                  45
Glu Ala Phe Ile Pro Ala Tyr Pro Asp Trp Val Trp Ala Val Pro Ala
    50                  55                  60
Gly Glu Glu Gly Met Leu Ser Glu Leu Tyr Ala Glu Leu Val Ala Asn
65                  70                  75                  80
Ser Leu Ala Ile Pro Ser Asp Ala Thr Asp Arg Leu Cys Arg Ala Ala
                85                  90                  95
Gln Ala Ala His Ile Asn Val Val Val Gly Leu Ser Glu Arg Asn Val
            100                 105                 110
Glu Ala Ser Gly Ala Ser Leu Tyr Asn Thr Leu Leu Tyr Ile Asp Ala
        115                 120                 125
Ala Gly Thr Ile Leu Gly Lys His Arg Lys Leu Val Pro Thr Gly Gly
    130                 135                 140
Glu Arg Leu Val Trp Ala Gln Gly Asp Gly Ser Thr Leu Asp Val Tyr

145                 150                 155                 160
Asp Thr Ala Leu Gly Lys Leu Gly Gly Leu Ile Cys Trp Glu Asn Tyr
                165                 170                 175
Met Pro Leu Ala Arg Tyr Ala Leu Tyr Ala Trp Gly Val Gln Ile Tyr
            180                 185                 190
Val Ala Ala Thr Trp Asp Arg Gly Glu Pro Trp Leu Ser Thr Leu Arg
        195                 200                 205
His Ile Ala Lys Glu Gly Arg Val Tyr Val Ile Gly Cys Gly Met Ala
    210                 215                 220
Leu Arg Arg Asp Asp Ile Pro Asp Arg Phe Ala Phe Lys Gln Arg Phe
225                 230                 235                 240
Tyr Ala Gln Ala Gly Glu Trp Ile Asn Val Gly Asp Ser Ala Ile Val
                245                 250                 255
Asn Pro Ser Gly Glu Phe Ile Ala Gly Pro Val Arg Glu Arg Glu Glu
            260                 265                 270
Ile Leu Tyr Ala Glu Val Asp Pro Glu Gln Met Ser Gly Pro Lys Trp
        275                 280                 285
Met Leu Asp Val Ala Gly His Tyr Gly Arg Pro Asp Val Phe Arg Leu
    290                 295                 300
Ser Val Asn Arg Ala Pro His Gln Met Ile Gln Thr Glu Asn Arg Glu
305                 310                 315                 320
Thr

<210> 205
<211> 969
<212> DNA
<213> Unknown

<220>
<223> Obtained from an environmental sample

<400> 205
atgaccaccg taaaagccgc cgcagtacag atcagccccg tgctctatag ccgggaggcc      60
accgtagaca aggtcgttcg caagatccgc gagctcggcc aaaagggagt gcagttcgcc     120
accttcccgg aaaccgtagt gccgtactac ccctacttcg ctgcagtcca gacaggcatc     180
gaactgttgt ccggcaagga acacatgcgc ctgctggagc aggccgttac cgtcccctcg     240
cccgccacgg atgcgattgc tcaggcggcg cgcgaagcca atatggtggt gtccatcggc     300
gtcaacgagc gcgacggcgg caccatctac aacacgcagc tgctcttcga tgccgacggc     360
acgctcgtgc agcgccgccg caagataacg ccaacgcact tcgagcgcat ggtctggggc     420
cagggcgatg gttcgggatt gcgcgccgcg gacaccaagg ttggccgcat cggccagttg     480
gcctgcttcg agcacaacaa cccgctcgcc cgttacgcca tgatggccga tggcgagcag     540
atccactccg ccatgtaccc gggctcggcc ttcggcgagg gcttcgcgca gcgcatggag     600
atcaacatcc gccagcatgc cctggagtct ggctgcttcg tggtgaatgc gaccgcctgg     660
ctcgatgccg accaacaggc gcagatcatg aaggacaccg gttgctcgat cggcccgatc     720
tccggcggct gcttcacgac catcgtcacg cctgagggca tgctgattgg cgagccgctc     780
cgcgagggcg aaggcgaaat catcgccgac ctcgatttct cgatgatcga tcgccgcaag     840
ctgctgatgg actcggtcgg tcactacaac cgtccggagc ttctgagcct cctgatcgat     900
cgcacgcctg ccgcgaactt ccatgaacgt accgcgagcc aggcgaacgc cggcgtcgaa     960
atcctctga                                                             969

<210> 206
<211> 322
<212> PRT
<213> Unknown

<220>

<223> Obtained from an environmental sample

<400> 206
Met Thr Thr Val Lys Ala Ala Ala Val Gln Ile Ser Pro Val Leu Tyr
1               5                   10                  15
Ser Arg Glu Ala Thr Val Asp Lys Val Val Arg Lys Ile Arg Glu Leu
            20                  25                  30
Gly Gln Lys Gly Val Gln Phe Ala Thr Phe Pro Glu Thr Val Val Pro
        35                  40                  45
Tyr Tyr Pro Tyr Phe Ala Ala Val Gln Thr Gly Ile Glu Leu Leu Ser
    50                  55                  60
Gly Lys Glu His Met Arg Leu Leu Glu Gln Ala Val Thr Val Pro Ser
65                  70                  75                  80
Pro Ala Thr Asp Ala Ile Ala Gln Ala Ala Arg Glu Ala Asn Met Val
                85                  90                  95
Val Ser Ile Gly Val Asn Glu Arg Asp Gly Gly Thr Ile Tyr Asn Thr
            100                 105                 110
Gln Leu Leu Phe Asp Ala Asp Gly Thr Leu Val Gln Arg Arg Arg Lys
        115                 120                 125
Ile Thr Pro Thr His Phe Glu Arg Met Val Trp Gly Gln Gly Asp Gly
    130                 135                 140
Ser Gly Leu Arg Ala Ala Asp Thr Lys Val Gly Arg Ile Gly Gln Leu
145                 150                 155                 160
Ala Cys Phe Glu His Asn Asn Pro Leu Ala Arg Tyr Ala Met Met Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Gly Cys Phe Val Val Asn Ala Thr Ala Trp Leu Asp Ala Asp
    210                 215                 220
Gln Gln Ala Gln Ile Met Lys Asp Thr Gly Cys Ser Ile Gly Pro Ile
225                 230                 235                 240
Ser Gly Gly Cys Phe Thr Thr Ile Val Thr Pro Glu Gly Met Leu Ile
                245                 250                 255
Gly Glu Pro Leu Arg Glu Gly Glu Gly Glu Ile Ile Ala Asp Leu Asp
            260                 265                 270
Phe Ser Met Ile Asp Arg Arg Lys Leu Leu Met Asp Ser Val Gly His
        275                 280                 285
Tyr Asn Arg Pro Glu Leu Leu Ser Leu Leu Ile Asp Arg Thr Pro Ala
    290                 295                 300
Ala Asn Phe His Glu Arg Thr Ala Ser Gln Ala Asn Ala Gly Val Glu
305                 310                 315                 320
Ile Leu


<210> 207
<211> 966
<212> DNA
<213> Unknown

<220>
<223> Obtained from an environmental sample

<400> 207
atgtcaaacg agaacaacaa cgctacattc aaagttgccg cagtacaggc tacacctgtt      60
tttctcgatc gtgaagcgac tctcgacaag gcttgcgatt tgatcgccgc cgccggaggt     120

gaaggggcac gattggttgt ctttccagaa gccttcatac cggcctatcc ggattgggta 180
tgggcaatcc caccgggtga agagggcgta cttaatgagt tgtacgcaga gctgctctcc 240
aactcggtca cgattcccag tgacgcgacg gacagactgt gccgggccgc gaggcttgct 300
aatgcttacg tggtgatggg gataagcgaa cgcaatgtcg aggcgagtgg agcaagcctg 360
tataacacgc tgttgtacat cgatgcgcag ggtgagattc taggcaaaca tcgaaagcta 420
gtgccaacgg gcggcgagcg gctggtgtgg gcgcagggcg atggcagcac actgcaggtc 480
tacgatactc cactgggaaa actcggcggt ttaatttgct gggagaatta tatgccgctg 540
gcccgctata ccatgtatgc ctggggcaca caaatctatg tcgccgctac gtgggatcgc 600
gggcaaccct ggctctccac tttgcggcat atcgccaaag aaggcagggt gtacgtgatt 660
ggttgttgta tcgcgatgcg caaagacgat atccctgatc gttacgcaat gaagcagaag 720
ttttacgcgg aggcagatga gtggatcaat ataggtgaca gcgcgattgt caatcctgaa 780
gggcaattta tcgcagggcc agtacgcaag caggaagaga ttctctacgc agagattgat 840
ccgcgcatgg tacaagggcc gaagtggatg ctcgacgtgg cggggcacta tgccaggccg 900
gatgtgttcc agttgacggt gcatacggat gtgcgacaga tgattcggat ggaacacgat 960
tcttaa 966

<210> 208
<211> 321
<212> PRT
<213> Unknown

<220>
<223> Obtained from an environmental sample

<400> 208
Met Ser Asn Glu Asn Asn Asn Ala Thr Phe Lys Val Ala Ala Val Gln
1               5                   10                  15
Ala Thr Pro Val Phe Leu Asp Arg Glu Ala Thr Leu Asp Lys Ala Cys
            20                  25                  30
Asp Leu Ile Ala Ala Ala Gly Gly Glu Gly Ala Arg Leu Val Val Phe
        35                  40                  45
Pro Glu Ala Phe Ile Pro Ala Tyr Pro Asp Trp Val Trp Ala Ile Pro
    50                  55                  60
Pro Gly Glu Glu Gly Val Leu Asn Glu Leu Tyr Ala Glu Leu Leu Ser
65                  70                  75                  80
Asn Ser Val Thr Ile Pro Ser Asp Ala Thr Asp Arg Leu Cys Arg Ala
                85                  90                  95
Ala Arg Leu Ala Asn Ala Tyr Val Val Met Gly Ile Ser Glu Arg Asn
            100                 105                 110
Val Glu Ala Ser Gly Ala Ser Leu Tyr Asn Thr Leu Leu Tyr Ile Asp
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Thr Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Asp Arg Tyr Ala Met Lys Gln Lys
225                 230                 235                 240
Phe Tyr Ala Glu Ala Asp Glu Trp Ile Asn Ile Gly Asp Ser Ala Ile
                245                 250                 255

Val Asn Pro Glu Gly Gln Phe Ile Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asp Val Arg Gln Met Ile Arg Met Glu His Asp
305                 310                 315                 320
Ser

<210> 209
<211> 993
<212> DNA
<213> Unknown

<220>
<223> Obtained from an environmental sample

<400> 209
atgaaagtcg tcaaagccgc cgctgtccag atcagtcccg ttctctacag ccgtgaggca      60
accgtcgaaa aggtcgtgaa gaaaattcac gagcttggcc aactgggcgt gcagttcgcc     120
acctttcccg agaccgtagt gccttactac ccgtactttt ccgccgtcca gacaggcatt     180
gagcttctgt ccggaactga gcatctgcgg ctgctcgatc aggccgtgac ggtaccgtct     240
cccgctaccg atgcgatcgg agaggcggcc cgcaaggcgg gcatggtggt gtccatcggc     300
gtgaatgaac gcgacggcgg caccttgtac aacacacagt tgctcttcga tgccgatggc     360
accttgatcc agcgccgccg caagatcacg cccacccact tcgaacggat gatctggggc     420
cagggggacg gctcgggcct gcgcgccgtc gacagcaagg ttggtcgcat tggtcagctt     480
gcctgcttcg agcacaacaa cccgctggcc cgctacgcgc tgattgccga cggcgagcag     540
atccattccg ccatgtatcc gggttctgct ttcggcgaag gctttgccca aaggatggaa     600
atcaatatcc gccagcatgc gctggagtct ggtgcctttg tcgtcaacgc aacggcctgg     660
ctggatgctg accagcaggc gcaaatcatc aaggacaccg gctgtgggat tggcccgatc     720
tcgggcggct gcttcaccac gatcgtggca cccgacggca tgctgatggc cgaacctctg     780
cgttcgggcg agggtgaggt catcgtggat ctcgacttca cgctgatcga ccgacgcaag     840
atgttgatgg actcggcggg ccactataac cgtccagaac tgctcagtct catgattgac     900
cgtaccgcga cggcgcatgt tcacgaacgc gctgcgcatc cggtgtcggg cgcggagcag     960
ggtccggagg atctgcgcac tccggccgcg tga                                  993

<210> 210
<211> 330
<212> PRT
<213> Unknown

<220>
<223> Obtained from an environmental sample

<400> 210
Met Lys Val Val Lys Ala Ala Ala Val Gln Ile Ser Pro Val Leu Tyr
 1               5                  10                  15
Ser Arg Glu Ala Thr Val Glu Lys Val Val Lys Lys Ile His Glu Leu
            20                  25                  30
Gly Gln Leu Gly Val Gln Phe Ala Thr Phe Pro Glu Thr Val Val Pro
        35                  40                  45
Tyr Tyr Pro Tyr Phe Ser Ala Val Gln Thr Gly Ile Glu Leu Leu Ser
    50                  55                  60
Gly Thr Glu His Leu Arg Leu Leu Asp Gln Ala Val Thr Val Pro Ser
65                  70                  75                  80

Pro Ala Thr Asp Ala Ile Gly Glu Ala Ala Arg Lys Ala Gly Met Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Lys Val Gly Arg Ile Gly Gln Leu
145                 150                 155                 160
Ala Cys Phe Glu His Asn Asn Pro Leu Ala Arg Tyr Ala Leu Ile Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Gly Ala Phe Val Val Asn Ala Thr Ala Trp Leu Asp Ala Asp
    210                 215                 220
Gln Gln Ala Gln Ile Ile Lys Asp Thr Gly Cys Gly Ile Gly Pro Ile
225                 230                 235                 240
Ser Gly Gly Cys Phe Thr Thr Ile Val Ala Pro Asp Gly Met Leu Met
                245                 250                 255
Ala Glu Pro Leu Arg Ser Gly Glu Gly Glu Val Ile Val Asp Leu Asp
            260                 265                 270
Phe Thr Leu Ile Asp Arg Arg Lys Met Leu Met Asp Ser Ala Gly His
        275                 280                 285
Tyr Asn Arg Pro Glu Leu Leu Ser Leu Met Ile Asp Arg Thr Ala Thr
    290                 295                 300
Ala His Val His Glu Arg Ala Ala His Pro Val Ser Gly Ala Glu Gln
305                 310                 315                 320
Gly Pro Glu Asp Leu Arg Thr Pro Ala Ala
                325                 330

<210> 211
<211> 1062
<212> DNA
<213> Unknown

<220>
<223> Obtained from an environmental sample

<400> 211
atgggcgtcg cacatccgaa atacaaagtg gccgccgtgc aggcagcgcc cgcttttctc      60
gacctggacg cctcggtcga aaaggccgtc cgtttcatcg acgaagccgg cgccgccggc     120
gcccgcctca tcgcctttcc ggagacctgg atacccggtt acccctggtg gatctggcta     180
ggcgcgccgg cctgggctat catgcgcggc ttcgtctcgc gctatttcga caactcgctc     240
agctacgaca gcccgcaggc cgagaagctc cgcgccgccg ccaagcgcaa caagatggtg     300
gtggtgctcg gcctctccga gcgcgacggc ggcagccttt acatcgcgca atggatcatc     360
ggcccggacg gcgaaaccat cgccaagcgc cgcaagctca agccgaccca cgcggagcgg     420
accgtgttcg gcgaaggcga cggctcgcat cttgcggtgc acgagcttga tgttggccgg     480
ctcggcgcgc tgtgctgctg ggaacacctg cagccgctgt ccaaatacgc catgtatgcg     540
cagaacgaac aggtgcatgt cgcggcctgg ccgagctttt cgctttacga tccgttcgcg     600
cacgcgctcg gcgcggaagt gaacaatgcg gcgagcaaaa tctatgcggt cgagggctcg     660
tgtttcgtca tcgcgccgtg cgcgaccgtt tcgcaggcga tgatcgacga actctgcgat     720
acgccggaga agcatcagtt cctgcatgcc ggcggcggct ttgccgtgat ttacggcccc     780
gacggcgccc cgctcgcggc gccgctgccg cccgacaagg aaggcttgct ctacgccgac     840
atcgatctcg ggatgatttc ggttgccaaa gcggcagccg atccggccgg gcattatgca     900

cgccccgacg tcacccggct tctgttcaac aatcggcctg ggtatcgggt cgagaccatg 960
gcgttgccga tcgatgcgga gaccaaggcg gaagcaccgg ctaagccgga acccaaggca 1020
ccgaacgtgg cgccgttcgc gccggtgcaa gcggccgagt ga 1062

<210> 212
<211> 353
<212> PRT
<213> Unknown

<220>
<223> Obtained from an environmental sample

<400> 212
Met Gly Val Ala His Pro Lys Tyr Lys Val Ala Ala Val Gln Ala Ala
1               5                   10                  15
Pro Ala Phe Leu Asp Leu Asp Ala Ser Val Glu Lys Ala Val Arg Phe
            20                  25                  30
Ile Asp Glu Ala Gly Ala Ala Gly Ala Arg Leu Ile Ala Phe Pro Glu
        35                  40                  45
Thr Trp Ile Pro Gly Tyr Pro Trp Trp Ile Trp Leu Gly Ala Pro Ala
    50                  55                  60
Trp Ala Ile Met Arg Gly Phe Val Ser Arg Tyr Phe Asp Asn Ser Leu
65                  70                  75                  80
Ser Tyr Asp Ser Pro Gln Ala Glu Lys Leu Arg Ala Ala Ala Lys Arg
                85                  90                  95
Asn Lys Met Val Val Val Leu Gly Leu Ser Glu Arg Asp Gly Gly Ser
            100                 105                 110
Leu Tyr Ile Ala Gln Trp Ile Ile Gly Pro Asp Gly Glu Thr Ile Ala
        115                 120                 125
Lys Arg Arg Lys Leu Lys Pro Thr His Ala Glu Arg Thr Val Phe Gly
    130                 135                 140
Glu Gly Asp Gly Ser His Leu Ala Val His Glu Leu Asp Val Gly Arg
145                 150                 155                 160
Leu Gly Ala Leu Cys Cys Trp Glu His Leu Gln Pro Leu Ser Lys Tyr
                165                 170                 175
Ala Met Tyr Ala Gln Asn Glu Gln Val His Val Ala Ala Trp Pro Ser
            180                 185                 190
Phe Ser Leu Tyr Asp Pro Phe Ala His Ala Leu Gly Ala Glu Val Asn
        195                 200                 205
Asn Ala Ala Ser Lys Ile Tyr Ala Val Glu Gly Ser Cys Phe Val Ile
    210                 215                 220
Ala Pro Cys Ala Thr Val Ser Gln Ala Met Ile Asp Glu Leu Cys Asp
225                 230                 235                 240
Thr Pro Glu Lys His Gln Phe Leu His Ala Gly Gly Gly Phe Ala Val
                245                 250                 255
Ile Tyr Gly Pro Asp Gly Ala Pro Leu Ala Ala Pro Leu Pro Pro Asp
            260                 265                 270
Lys Glu Gly Leu Leu Tyr Ala Asp Ile Asp Leu Gly Met Ile Ser Val
        275                 280                 285
Ala Lys Ala Ala Ala Asp Pro Ala Gly His Tyr Ala Arg Pro Asp Val
    290                 295                 300
Thr Arg Leu Leu Phe Asn Asn Arg Pro Gly Tyr Arg Val Glu Thr Met
305                 310                 315                 320
Ala Leu Pro Ile Asp Ala Glu Thr Lys Ala Glu Ala Pro Ala Lys Pro
                325                 330                 335
Glu Pro Lys Ala Pro Asn Val Ala Pro Phe Ala Pro Val Gln Ala Ala
            340                 345                 350

Glu

<210> 213
<211> 993
<212> DNA
<213> Unknown

<220>
<223> Obtained from an environmental sample

<400> 213
atgagagttg ttaaagccgc tgctgtccaa ctgagtcccg tcctctatag tcgcgaggga 60
acggtcgaaa aggtcgtgcg gaagatccat gaacttgccg aagagggagt cgagttcgcc 120
acctttcctg agaccgtggt gccttactac ccgtactttt ccttcgttca gacgcccttg 180
gagcaaatct tcggaacaga gtatctgagg ctgctcgacc aggcagtcac cgtgccatcc 240
cctgccaccg acgcgatcgg cgaggcagcc aggttcgctg gagttgttgt ctcgatcggc 300
gtcaacgagc gagacggggg aactctatac aacactcagc ttctcttcga tgccgacggc 360
aggataattc agcggcgccg caagatcacg cccacccatt acgagcgcat gatctggggc 420
cagggcgacg gctcaggtct gcgggccgtt gatagcaagg ccggccgtat tggtcagctg 480
gcatgctggg agcacaacaa tccactggcg cgctacgcgc tgatggccga cggcgagcag 540
atccattccg ccatgtatcc gggctccatg ttcggcgact cgtttgccca gaagaccgaa 600
atcaatatcc ggcagcatgc cctagagtct gggtgcttcg tcgtgaacgc aacggcctgg 660
ctggacggcg atcagcaggc gcatatcatg aaggacaccg gctgcagcat cggcccgatc 720
tccggcggtt gcttcactgc gatcgtcgca cccgatggta gcctgctggg cgaacccatc 780
cgttccggtg agggcgtggt catcgccgac ctcgacttca cgttgatcga caggcgtaag 840
caggtgatgg actcgcgagg ccattacagc cggccggagt tgctcagcct cttaatagac 900
cgcaccccta ccgcgcactt tcacgaacgc gcttcgcccc ccacgacaga agctgagcaa 960
ggctccgagg atgtgttcga ggctcgcatt taa 993

<210> 214
<211> 330
<212> PRT
<213> Unknown

<220>
<223> Obtained from an environmental sample

<400> 214
Met Arg Val Val Lys Ala Ala Ala Val Gln Leu Ser Pro Val Leu Tyr
1               5                   10                  15
Ser Arg Glu Gly Thr Val Glu Lys Val Val Arg Lys Ile His Glu Leu
            20                  25                  30
Ala Glu Glu Gly Val Glu Phe Ala Thr Phe Pro Glu Thr Val Val Pro
        35                  40                  45
Tyr Tyr Pro Tyr Phe Ser Phe Val Gln Thr Pro Leu Glu Gln Ile Phe
    50                  55                  60
Gly Thr Glu Tyr Leu Arg Leu Leu Asp Gln Ala Val Thr Val Pro Ser
65                  70                  75                  80
Pro Ala Thr Asp Ala Ile Gly Glu Ala Ala Arg Phe Ala Gly Val Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Arg Ile Ile Gln Arg Arg Arg Lys
        115                 120                 125
Ile Thr Pro Thr His Tyr Glu Arg Met Ile Trp Gly Gln Gly Asp Gly
    130                 135                 140

Ser Gly Leu Arg Ala Val Asp Ser Lys Ala Gly Arg Ile Gly Gln Leu
145                 150                 155                 160
Ala Cys Trp Glu His Asn Asn Pro Leu Ala Arg Tyr Ala Leu Met Ala
                165                 170                 175
Asp Gly Glu Gln Ile His Ser Ala Met Tyr Pro Gly Ser Met Phe Gly
            180                 185                 190
Asp Ser Phe Ala Gln Lys Thr Glu Ile Asn Ile Arg Gln His Ala Leu
        195                 200                 205
Glu Ser Gly Cys Phe Val Val Asn Ala Thr Ala Trp Leu Asp Gly Asp
    210                 215                 220
Gln Gln Ala His Ile Met Lys Asp Thr Gly Cys Ser Ile Gly Pro Ile
225                 230                 235                 240
Ser Gly Gly Cys Phe Thr Ala Ile Val Ala Pro Asp Gly Ser Leu Leu
                245                 250                 255
Gly Glu Pro Ile Arg Ser Gly Glu Gly Val Val Ile Ala Asp Leu Asp
            260                 265                 270
Phe Thr Leu Ile Asp Arg Arg Lys Gln Val Met Asp Ser Arg Gly His
        275                 280                 285
Tyr Ser Arg Pro Glu Leu Leu Ser Leu Leu Ile Asp Arg Thr Pro Thr
    290                 295                 300
Ala His Phe His Glu Arg Ala Ser Pro Pro Thr Thr Glu Ala Glu Gln
305                 310                 315                 320
Gly Ser Glu Asp Val Phe Glu Ala Arg Ile
                325                 330

<210> 215
<211> 1008
<212> DNA
<213> Unknown

<220>
<223> Obtained from an environmental sample

<400> 215
atgagtatta cccaccccaa atttaaagct gctgttgttc aagctgcccc agtatttcta      60
gacctagatg ggtctgttaa taaggcgatt aatctcattg atgaagctgc cgctgccgga     120
gccaagctca ttgccttccc tgaaaccttc attccaggct atccatggtg gatttggctg     180
ggatcgccgg cgtgggctct gggccggggg ttcgttcagc gttacttcga caattccctg     240
cagtacgaca gcccgcaggc ggatcgctta cgcgaggcgg cacgacgcaa cagcattacg     300
gtcgtgctgg gcttgtccga gcgtgatggc ggttctctct atatcgcaca gtggctgatc     360
ggcccggatg gcgaaaccat cgcgcagcgg cgcaagcttc gtcctactca tggggagcgc     420
acggtattcg gtgaagggga tggcagcgat ctggtggttc atcaaaccga actgggccgt     480
cttggcgcgc ttaactgctg ggagaacatc ctgtctctga acaaatatgt gatgtactcc     540
cagcatgaac aggtccatgt agcatcctgg cccagtttct cgacgtatga accgttcgcg     600
catgcgctcg gctatgaggt aaacaacgca attagccagg tctatgcggt ggaaggcggg     660
tgcttcgtgt tggccccgtg ctctaccatc tctgaagaaa tgattgccga actgtgcgat     720
acacccgata aattcgagct gacgcatgct ggtggcggcc acgcaatcat ctatggtccg     780
gacggtcgtg ctctgtgcga aaagctgccc gagaaccagg agggcctgct gtacgcggaa     840
atcgatctgg gggtgatttc tatggcaaaa agtgccatgg atcctgtcgg ccattactct     900
cgccccgatg tctaccgtgt gctgttcaat aagatcccgg caaagcgtat cgagcacttc     960
aatttgccgt tggatgagca agcaggggaa gagccaccag ctgattaa                 1008

<210> 216
<211> 335
<212> PRT
<213> Unknown

<220>
<223> Obtained from an environmental sample

<400> 216
Met Ser Ile Thr His Pro Lys Phe Lys Ala Ala Val Val Gln Ala Ala
1               5                   10                  15
Pro Val Phe Leu Asp Leu Asp Gly Ser Val Asn Lys Ala Ile Asn Leu
            20                  25                  30
Ile Asp Glu Ala Ala Ala Ala Gly Ala Lys Leu Ile Ala Phe Pro Glu
        35                  40                  45
Thr Phe Ile Pro Gly Tyr Pro Trp Trp Ile Trp Leu Gly Ser Pro Ala
    50                  55                  60
Trp Ala Leu Gly Arg Gly Phe Val Gln Arg Tyr Phe Asp Asn Ser Leu
65                  70                  75                  80
Gln Tyr Asp Ser Pro Gln Ala Asp Arg Leu Arg Glu Ala Ala Arg Arg
                85                  90                  95
Asn Ser Ile Thr Val Val Leu Gly Leu Ser Glu Arg Asp Gly Gly Ser
            100                 105                 110
Leu Tyr Ile Ala Gln Trp Leu Ile Gly Pro Asp Gly Glu Thr Ile Ala
        115                 120                 125
Gln Arg Arg Lys Leu Arg Pro Thr His Gly Glu Arg Thr Val Phe Gly
    130                 135                 140
Glu Gly Asp Gly Ser Asp Leu Val Val His Gln Thr Glu Leu Gly Arg
145                 150                 155                 160
Leu Gly Ala Leu Asn Cys Trp Glu Asn Ile Leu Ser Leu Asn Lys Tyr
                165                 170                 175
Val Met Tyr Ser Gln His Glu Gln Val His Val Ala Ser Trp Pro Ser
            180                 185                 190
Phe Ser Thr Tyr Glu Pro Phe Ala His Ala Leu Gly Tyr Glu Val Asn
        195                 200                 205
Asn Ala Ile Ser Gln Val Tyr Ala Val Glu Gly Gly Cys Phe Val Leu
    210                 215                 220
Ala Pro Cys Ser Thr Ile Ser Glu Glu Met Ile Ala Glu Leu Cys Asp
225                 230                 235                 240
Thr Pro Asp Lys Phe Glu Leu Thr His Ala Gly Gly Gly His Ala Ile
                245                 250                 255
Ile Tyr Gly Pro Asp Gly Arg Ala Leu Cys Glu Lys Leu Pro Glu Asn
            260                 265                 270
Gln Glu Gly Leu Leu Tyr Ala Glu Ile Asp Leu Gly Val Ile Ser Met
        275                 280                 285
Ala Lys Ser Ala Met Asp Pro Val Gly His Tyr Ser Arg Pro Asp Val
    290                 295                 300
Tyr Arg Val Leu Phe Asn Lys Ile Pro Ala Lys Arg Ile Glu His Phe
305                 310                 315                 320
Asn Leu Pro Leu Asp Glu Gln Ala Gly Glu Glu Pro Pro Ala Asp
                325                 330                 335

<210> 217
<211> 1011
<212> DNA
<213> Unknown

<220>
<223> Obtained from an environmental sample

<400> 217
gtgggtatca gccatccgaa attcaaggct gcggtcgtac aggccgggcc tgccttcctc      60

gacctcgacg gcggcgtcga acgagccgtg tcgctcatcg gccaagcagc ggccgaaggg 120
gcacagctga ttgcctttcc tgaaacgtgg attcccggtt acccgtggca cacctggctt 180
ggcagcccgg cgtgggcaat ggaaaaaggc tttgtccaac gatatttcga caacgcgttg 240
cggcatggtt ctccgcaagc cgagcgaatc tccggggctg cggcggagca caagattatg 300
gtgtcgcttg ggtttgcgga acgcgatgga ggcacgcttt atatcgcgca gtggctcatc 360
ggacccgacg gccaaactat ctcacgacgg cggaagctta agccgactca cgtcgagcgc 420
actgtatttg gcgagggaga cggaagcgat ctctccgtgc atgatacggc gcttggacgt 480
atcggctcac tttgttgctg ggagcatttg caaccgttgt cgaaatacgc aatgtacgcc 540
cagaatgaac agattcacat tggcgcatgg cccagctttt cgctatacca gccatttgcg 600
aatgcgctga gtcccgaagt caatatcgca gtaagccgcg tgtacgccgt ggaaggccag 660
tgtttcttcc tcgcgccgtg cgcgacggtt tcggacgcca tgatcgaaac actgtgcgat 720
acgcccgaaa agcagggact gattcgggcg ggtggcgggc acgccgcgat cttcggccca 780
gatggaagtc tgctgacgcc tacggtagcg gatacttacg agggcctgct gtatgcagaa 840
ctcgacctcg gcgtcatttc gatcgccaag agtgcagcgg accccgccgg ccactattcg 900
cggccagatg tcacacgcct tctattgaat cagacgcctt cgaagcgcgt tcagaatatg 960
gtgttaccac tggagacggt cacggagccc gaaggcccgg ttcagcccta g 1011

<210> 218
<211> 336
<212> PRT
<213> Unknown

<220>
<223> Obtained from an environmental sample

<400> 218
Val Gly Ile Ser His Pro Lys Phe Lys Ala Ala Val Val Gln Ala Gly
1               5                   10                  15
Pro Ala Phe Leu Asp Leu Asp Gly Gly Val Glu Arg Ala Val Ser Leu
            20                  25                  30
Ile Gly Gln Ala Ala Ala Glu Gly Ala Gln Leu Ile Ala Phe Pro Glu
        35                  40                  45
Thr Trp Ile Pro Gly Tyr Pro Trp His Thr Trp Leu Gly Ser Pro Ala
    50                  55                  60
Trp Ala Met Glu Lys Gly Phe Val Gln Arg Tyr Phe Asp Asn Ala Leu
65                  70                  75                  80
Arg His Gly Ser Pro Gln Ala Glu Arg Ile Ser Gly Ala Ala Ala Glu
                85                  90                  95
His Lys Ile Met Val Ser Leu Gly Phe Ala Glu Arg Asp Gly Gly Thr
            100                 105                 110
Leu Tyr Ile Ala Gln Trp Leu Ile Gly Pro Asp Gly Gln Thr Ile Ser
        115                 120                 125
Arg Arg Arg Lys Leu Lys Pro Thr His Val Glu Arg Thr Val Phe Gly
    130                 135                 140
Glu Gly Asp Gly Ser Asp Leu Ser Val His Asp Thr Ala Leu Gly Arg
145                 150                 155                 160
Ile Gly Ser Leu Cys Cys Trp Glu His Leu Gln Pro Leu Ser Lys Tyr
                165                 170                 175
Ala Met Tyr Ala Gln Asn Glu Gln Ile His Ile Gly Ala Trp Pro Ser
            180                 185                 190
Phe Ser Leu Tyr Gln Pro Phe Ala Asn Ala Leu Ser Pro Glu Val Asn
        195                 200                 205
Ile Ala Val Ser Arg Val Tyr Ala Val Glu Gly Gln Cys Phe Phe Leu
    210                 215                 220
Ala Pro Cys Ala Thr Val Ser Asp Ala Met Ile Glu Thr Leu Cys Asp
225                 230                 235                 240
Thr Pro Glu Lys Gln Gly Leu Ile Arg Ala Gly Gly Gly His Ala Ala

245                 250                 255
Ile Phe Gly Pro Asp Gly Ser Leu Leu Thr Pro Thr Val Ala Asp Thr
            260                 265                 270
Tyr Glu Gly Leu Leu Tyr Ala Glu Leu Asp Leu Gly Val Ile Ser Ile
        275                 280                 285
Ala Lys Ser Ala Ala Asp Pro Ala Gly His Tyr Ser Arg Pro Asp Val
    290                 295                 300
Thr Arg Leu Leu Leu Asn Gln Thr Pro Ser Lys Arg Val Gln Asn Met
305                 310                 315                 320
Val Leu Pro Leu Glu Thr Val Thr Glu Pro Glu Gly Pro Val Gln Pro
                325                 330                 335

<210> 219
<211> 996
<212> DNA
<213> Unknown

<220>
<223> Obtained from an environmental sample

<400> 219
atgaaaatcg tcaaagctgc cgccgttcaa attagtcccg tgctttatag ccgcgatgga      60
acggtggaaa aagttgtgcg gaagatccat gagctcggcc aacagggagt gcagttcgca     120
accttccccg agaccgtgat tccgtactat ccctattttt cctttctgca acccgcctac     180
cagatcgcgg ccgggcagga gcacctcaag ttgctcgacc aggcggtgac ggtgccgtcc     240
gctgccacgc acgcgatcgg ccaggcatgc aaacaggcag gagtggttgt ttcgatcggc     300
atcaatgagc gggacaatgg aacgatctac aacacccagc ttctcttcga ctccgacgga     360
accctgctgc agcggcgccg caagatctca cccacttacc acgaacggat gatctggggc     420
cagggcgacg gttccggcct gcgcgccgtg gatagcaagg ccggccggat tgggcagctc      480
gcctgttggg agcactacaa ccctttggcg cgctacgcga tgatggccga cggcgagcag     540
atccattccg ccatgtaccc tggctccatc ttcggcgatc tgtttgccga acagacccag     600
atcaacgtcc ggcagcacgc gttggagtcc ggctgtttcg tagtctgctc caccgcctgg     660
ttggatcccg atcaacaagc gcaaatcgtc aaggacacag gcggcgccat tggtgcgatc     720
tcgggcggct gtttcacggc gattgtggcc cccgatagca ctctgctcgg agagccgatc     780
cgctccggcg aaggcgtggt cattgcggat ctcgacttca cccggatcga caagcgtaaa     840
cagttgatgg attcccgagg tcattacagc cggccggaat tgctgagtct gctgattgat     900
cgcacttcca ccgcacatgt gcaaaaccgc gtgccggccg atccattcgg cgccggctgt     960
gttgcagaac caggagtaga aacatgccca cgatag                               996

<210> 220
<211> 331
<212> PRT
<213> Unknown

<220>
<223> Obtained from an environmental sample

<400> 220
Met Lys Ile Val Lys Ala Ala Ala Val Gln Ile Ser Pro Val Leu Tyr
1               5                   10                  15
Ser Arg Asp Gly Thr Val Glu Lys Val Val Arg Lys Ile His Glu Leu
            20                  25                  30
Gly Gln Gln Gly Val Gln Phe Ala Thr Phe Pro Glu Thr Val Ile Pro
        35                  40                  45
Tyr Tyr Pro Tyr Phe Ser Phe Leu Gln Pro Ala Tyr Gln Ile Ala Ala
    50                  55                  60
Gly Gln Glu His Leu Lys Leu Leu Asp Gln Ala Val Thr Val Pro Ser

65                  70                  75                  80
Ala Ala Thr His Ala Ile Gly Gln Ala Cys Lys Gln Ala Gly Val Val
                85                  90                  95
Val Ser Ile Gly Ile Asn Glu Arg Asp Asn Gly Thr Ile Tyr Asn Thr
            100                 105                 110
Gln Leu Leu Phe Asp Ser Asp Gly Thr Leu Leu Gln Arg Arg Arg Lys
        115                 120                 125
Ile Ser Pro Thr Tyr His Glu Arg Met Ile Trp Gly Gln Gly Asp Gly
    130                 135                 140
Ser Gly Leu Arg Ala Val Asp Ser Lys Ala Gly Arg Ile Gly Gln Leu
145                 150                 155                 160
Ala Cys Trp Glu His Tyr Asn Pro Leu Ala Arg Tyr Ala Met Met Ala
                165                 170                 175
Asp Gly Glu Gln Ile His Ser Ala Met Tyr Pro Gly Ser Ile Phe Gly
            180                 185                 190
Asp Leu Phe Ala Glu Gln Thr Gln Ile Asn Val Arg Gln His Ala Leu
        195                 200                 205
Glu Ser Gly Cys Phe Val Val Cys Ser Thr Ala Trp Leu Asp Pro Asp
    210                 215                 220
Gln Gln Ala Gln Ile Val Lys Asp Thr Gly Gly Ala Ile Gly Ala Ile
225                 230                 235                 240
Ser Gly Gly Cys Phe Thr Ala Ile Val Ala Pro Asp Ser Thr Leu Leu
                245                 250                 255
Gly Glu Pro Ile Arg Ser Gly Glu Gly Val Val Ile Ala Asp Leu Asp
            260                 265                 270
Phe Thr Arg Ile Asp Lys Arg Lys Gln Leu Met Asp Ser Arg Gly His
        275                 280                 285
Tyr Ser Arg Pro Glu Leu Leu Ser Leu Leu Ile Asp Arg Thr Ser Thr
    290                 295                 300
Ala His Val Gln Asn Arg Val Pro Ala Asp Pro Phe Gly Ala Gly Cys
305                 310                 315                 320
Val Ala Glu Pro Gly Val Glu Thr Cys Pro Arg
                325                 330

<210> 221
<211> 1146
<212> DNA
<213> Unknown

<220>
<223> Obtained from an environmental sample

<400> 221
atgttgacct acaaaggcgt gttcaaagca gcgacagttc aggcggagcc ggtgtggatg      60
gacgctgacg ccaccattac caaagccatc cggatcatcg aagaggcggc tgataacggg     120
gcgaagtttg tcgcattccc ggaggtgttc atccctggat acccttggtg gatctggctg     180
ggcactgcga tgtggggagc caaattcgtc gtgcccttcc acgaaaattg ccttgagctt     240
ggcgacaagc ggatgcagcg cattcaagct gcggcaaaac aaaatggcat tgcactcgtc     300
atgggttacg gtgaacgtga cggaggtagc cgctacatga gccaggtatt catcgacgat     360
agcggaaaaa ttgtggcgaa ccgccgcaag ctcaaaccga cgcacgaaga gcgaacgatt     420
ttcggcgagg gaaacggatc cgatttcatc acccatgact tcccatttgc gagagtcgga     480
ggcttcaact gctgggaaca ccttcagccc ctgagcaaat acatgatgta cagcctgcag     540
gaacaggtgc atgtcgcgtc gtggccggcc atgtgcacct atcagcctga cgtgccccag     600
ttaggtgcag gcgctaacga ggcggtcacc cgctcttacg cgatcgaagg cgcatgttat     660
gtgctgggag cgacactcgt tatcggcaag gcagcccatg atgcattctg cgacactgaa     720
gagcatcaca agctgcttgg catgggcgga gggtgggccc gaattttcgg cccggacggt     780
gagtatctcg cagaatcgct cgctcatgac gcggagggca tcctgtacgc tgacattgat     840

ctgagcaaga tcctgctcgc caaagcaaac acggacacag ttggccacta cgcgcgacct 900
gacgtcttgt cattgctcgt caacacacac aatcccgggc ctgtacgcta tcttgacgaa 960
gagggccggc aggtttcaac gtcaattcgc aggcacgaaa aactggaagg ccagagcctc 1020
gacctcgagg ttacgccagc gacaccagcg accctggaca tcgcgagcct ggtacaacag 1080
gctaagccat cgaccgttaa gagcgagagt aacgcgagca caaaacaacc ggatctcgcg 1140
gtctga 1146

<210> 222
<211> 381
<212> PRT
<213> Unknown

<220>
<223> Obtained from an environmental sample

<400> 222
Met Leu Thr Tyr Lys Gly Val Phe Lys Ala Ala Thr Val Gln Ala Glu
1               5                   10                  15
Pro Val Trp Met Asp Ala Asp Ala Thr Ile Thr Lys Ala Ile Arg Ile
            20                  25                  30
Ile Glu Glu Ala Ala Asp Asn Gly Ala Lys Phe Val Ala Phe Pro Glu
        35                  40                  45
Val Phe Ile Pro Gly Tyr Pro Trp Trp Ile Trp Leu Gly Thr Ala Met
    50                  55                  60
Trp Gly Ala Lys Phe Val Val Pro Phe His Glu Asn Cys Leu Glu Leu
65                  70                  75                  80
Gly Asp Lys Arg Met Gln Arg Ile Gln Ala Ala Ala Lys Gln Asn Gly
                85                  90                  95
Ile Ala Leu Val Met Gly Tyr Gly Glu Arg Asp Gly Gly Ser Arg Tyr
            100                 105                 110
Met Ser Gln Val Phe Ile Asp Asp Ser Gly Lys Ile Val Ala Asn Arg
        115                 120                 125
Arg Lys Leu Lys Pro Thr His Glu Glu Arg Thr Ile Phe Gly Glu Gly
    130                 135                 140
Asn Gly Ser Asp Phe Ile Thr His Asp Phe Pro Phe Ala Arg Val Gly
145                 150                 155                 160
Gly Phe Asn Cys Trp Glu His Leu Gln Pro Leu Ser Lys Tyr Met Met
                165                 170                 175
Tyr Ser Leu Gln Glu Gln Val His Val Ala Ser Trp Pro Ala Met Cys
            180                 185                 190
Thr Tyr Gln Pro Asp Val Pro Gln Leu Gly Ala Gly Ala Asn Glu Ala
        195                 200                 205
Val Thr Arg Ser Tyr Ala Ile Glu Gly Ala Cys Tyr Val Leu Gly Ala
    210                 215                 220
Thr Leu Val Ile Gly Lys Ala Ala His Asp Ala Phe Cys Asp Thr Glu
225                 230                 235                 240
Glu His His Lys Leu Leu Gly Met Gly Gly Gly Trp Ala Arg Ile Phe
                245                 250                 255
Gly Pro Asp Gly Glu Tyr Leu Ala Glu Ser Leu Ala His Asp Ala Glu
            260                 265                 270
Gly Ile Leu Tyr Ala Asp Ile Asp Leu Ser Lys Ile Leu Leu Ala Lys
        275                 280                 285
Ala Asn Thr Asp Thr Val Gly His Tyr Ala Arg Pro Asp Val Leu Ser
    290                 295                 300
Leu Leu Val Asn Thr His Asn Pro Gly Pro Val Arg Tyr Leu Asp Glu
305                 310                 315                 320
Glu Gly Arg Gln Val Ser Thr Ser Ile Arg Arg His Glu Lys Leu Glu

                325                 330                 335
Gly Gln Ser Leu Asp Leu Glu Val Thr Pro Ala Thr Pro Ala Thr Leu
            340                 345                 350
Asp Ile Ala Ser Leu Val Gln Gln Ala Lys Pro Ser Thr Val Lys Ser
        355                 360                 365
Glu Ser Asn Ala Ser Thr Lys Gln Pro Asp Leu Ala Val
    370                 375                 380

<210> 223
<211> 996
<212> DNA
<213> Unknown

<220>
<223> Obtained from an environmental sample

<400> 223
atgagagttg ttaaagccag cgcagttcaa ctgaagcctg tactttatag ccgcgagggg 60
acggtcgaaa aggtcgtagc gaagattcat gagctagggc agcagggtgt gcagttcgcc 120
gcgttccctg agaccgtggt gccttattac ccgtactttt cgatcgtgca gtccggctat 180
caaatccttc gcggcggtga gttcgtcaag ctcctcgatc agtccgtgac ggtgccatct 240
tatagcactg aagccatcgg cgaagcctgc aggcaggagg agatggttgt ctccataggc 300
gtcaacgagc gtgatggcgg aacgatctac aacgcgcagt tactctttga ttccgacggc 360
acgttgatcc aaagacgacg caagatcacc cccacccatt acgaacgcat gatctggggc 420
cagggcgatg gctcaggtct gcgcgctgtt gacagcaatg tcgcacgcat tggtcaactg 480
gcatgctttg agcactacaa ccctcttgcg cggtacgcga tgatggccga tggcgagcag 540
atccattccg ccatgttccc cggttccatg ttcggcgatg gttttgcgga gaggacggaa 600
attgccgtaa ggcaacatgc gatggagtcc gggtgctttg tcgtttgcgc tacggcctgg 660
ctcgatcccg gccagcaggc tcagatcgcc aacgacaccg gtatcaccga catcggcccg 720
atctccgggg gttgcttcac tgcgatcatc gcacccgatg ggagcctgct gggccaacct 780
atccgctcgg gtgaaggcga agtcatcgtc gacctcgatt tcacgttaat tgacaagcgg 840
aaacatattg tcgactcgag aggacattac agccggccag aattgctgag cctgctgatc 900
gatcgcactc ccaccgcgca ccttcacgac cgcgctgtgc agcacaatgc cggatcggaa 960
ggagcgtcgg aacatcttcg cgaagacgcc gcctga 996

<210> 224
<211> 331
<212> PRT
<213> Unknown

<220>
<223> Obtained from an environmental sample

<400> 224
Met Arg Val Val Lys Ala Ser Ala Val Gln Leu Lys Pro Val Leu Tyr
1               5                   10                  15
Ser Arg Glu Gly Thr Val Glu Lys Val Val Ala Lys Ile His Glu Leu
            20                  25                  30
Gly Gln Gln Gly Val Gln Phe Ala Ala Phe Pro Glu Thr Val Val Pro
        35                  40                  45
Tyr Tyr Pro Tyr Phe Ser Ile Val Gln Ser Gly Tyr Gln Ile Leu Arg
    50                  55                  60
Gly Gly Glu Phe Val Lys Leu Leu Asp Gln Ser Val Thr Val Pro Ser
65                  70                  75                  80
Tyr Ser Thr Glu Ala Ile Gly Glu Ala Cys Arg Gln Glu Glu Met Val
                85                  90                  95
Val Ser Ile Gly Val Asn Glu Arg Asp Gly Gly Thr Ile Tyr Asn Ala

                    100                 105                 110
Gln Leu Leu Phe Asp Ser Asp Gly Thr Leu Ile Gln Arg Arg Arg Lys
        115                 120                 125
Ile Thr Pro Thr His Tyr Glu Arg Met Ile Trp Gly Gln Gly Asp Gly
    130                 135                 140
Ser Gly Leu Arg Ala Val Asp Ser Asn Val Ala Arg Ile Gly Gln Leu
145                 150                 155                 160
Ala Cys Phe Glu His Tyr Asn Pro Leu Ala Arg Tyr Ala Met Met Ala
                165                 170                 175
Asp Gly Glu Gln Ile His Ser Ala Met Phe Pro Gly Ser Met Phe Gly
            180                 185                 190
Asp Gly Phe Ala Glu Arg Thr Glu Ile Ala Val Arg Gln His Ala Met
        195                 200                 205
Glu Ser Gly Cys Phe Val Val Cys Ala Thr Ala Trp Leu Asp Pro Gly
    210                 215                 220
Gln Gln Ala Gln Ile Ala Asn Asp Thr Gly Ile Thr Asp Ile Gly Pro
225                 230                 235                 240
Ile Ser Gly Gly Cys Phe Thr Ala Ile Ile Ala Pro Asp Gly Ser Leu
                245                 250                 255
Leu Gly Gln Pro Ile Arg Ser Gly Glu Gly Glu Val Ile Val Asp Leu
            260                 265                 270
Asp Phe Thr Leu Ile Asp Lys Arg Lys His Ile Val Asp Ser Arg Gly
        275                 280                 285
His Tyr Ser Arg Pro Glu Leu Leu Ser Leu Leu Ile Asp Arg Thr Pro
    290                 295                 300
Thr Ala His Leu His Asp Arg Ala Val Gln His Asn Ala Gly Ser Glu
305                 310                 315                 320
Gly Ala Ser Glu His Leu Arg Glu Asp Ala Ala
                325                 330

<210> 225
<211> 951
<212> DNA
<213> Unknown

<220>
<223> Obtained from an environmental sample

<400> 225
atgaccaccg tcaaagccgc cgctgtccag atgagtcccg tgctttacag tcgcgacgat      60
accatcgaga aaatttgtcg acagatcatc gagcttggcc gacagggagt gcagttcgcc     120
acgtttccag agacagttat tccgtattat ccatatttcg ccttcgtgca gcggccttac     180
gaaatgtcgg cccaatatca tcagttgctt gatcaagcgg tgaccgtgcc ttccggttca     240
acgcacgcca ttggggccgc ctgtaaacaa gccggaattg ttgtctcaat cggcgtcaac     300
gagcgggagg gcggcacgct ctatggcaca cagttgctgt ttgatgcaga cggcctcttg     360
atccagcgtc gccgcaagat cactccgacc taccacgagc ggatgatttg gggacagggt      420
gacggttccg ggctgcgagc cgtagatagc gcggtcggtc gaatcggaca gttggcgtgt     480
tgggaacacc acaatccgct ggctcgttat gctttagcgg ccgatggcga acaaattcac     540
gcggcgatgt accctggctc gatcttgggt gaactatttg ccgagcagat tcaggtcaac     600
atccggcagc acgccatgga atctggttgc ttcgtcgtga acgccacggc ctggctaagc     660
gaggaacagc aagcccgaat catgaaggac accggatcat tcgatagccc aatcaccggt     720
ggttgcttta ccgccattgt cgcgcccaac gggcagataa tcggtgaacc gctgcgcatc     780
ggcgaaggcg tcgtgattgc cgatttggac ttcgctttga ttgatgagag gaagcggctg     840
atggactcac gcggcctcta tagccgccct gagttgctaa gcttgttaat cgacagaatg     900
cctacatccc atgtgcatga acgggttgag cgtagcatgg cgatggcatg a              951

<210> 226

<211> 316
<212> PRT
<213> Unknown

<220>
<223> Obtained from an environmental sample

<400> 226
Met Thr Thr Val Lys Ala Ala Ala Val Gln Met Ser Pro Val Leu Tyr
1               5                   10                  15
Ser Arg Asp Asp Thr Ile Glu Lys Ile Cys Arg Gln Ile Ile Glu Leu
            20                  25                  30
Gly Arg Gln Gly Val Gln Phe Ala Thr Phe Pro Glu Thr Val Ile Pro
        35                  40                  45
Tyr Tyr Pro Tyr Phe Ala Phe Val Gln Arg Pro Tyr Glu Met Ser Ala
    50                  55                  60
Gln Tyr His Gln Leu Leu Asp Gln Ala Val Thr Val Pro Ser Gly Ser
65                  70                  75                  80
Thr His Ala Ile Gly Ala Ala Cys Lys Gln Ala Gly Ile Val Val Ser
                85                  90                  95
Ile Gly Val Asn Glu Arg Glu Gly Gly Thr Leu Tyr Gly Thr Gln Leu
            100                 105                 110
Leu Phe Asp Ala Asp Gly Leu Leu Ile Gln Arg Arg Arg Lys Ile Thr
        115                 120                 125
Pro Thr Tyr His Glu Arg Met Ile Trp Gly Gln Gly Asp Gly Ser Gly
    130                 135                 140
Leu Arg Ala Val Asp Ser Ala Val Gly Arg Ile Gly Gln Leu Ala Cys
145                 150                 155                 160
Trp Glu His His Asn Pro Leu Ala Arg Tyr Ala Leu Ala Ala Asp Gly
                165                 170                 175
Glu Gln Ile His Ala Ala Met Tyr Pro Gly Ser Ile Leu Gly Glu Leu
            180                 185                 190
Phe Ala Glu Gln Ile Gln Val Asn Ile Arg Gln His Ala Met Glu Ser
        195                 200                 205
Gly Cys Phe Val Val Asn Ala Thr Ala Trp Leu Ser Glu Glu Gln Gln
    210                 215                 220
Ala Arg Ile Met Lys Asp Thr Gly Ser Phe Asp Ser Pro Ile Thr Gly
225                 230                 235                 240
Gly Cys Phe Thr Ala Ile Val Ala Pro Asn Gly Gln Ile Ile Gly Glu
                245                 250                 255
Pro Leu Arg Ile Gly Glu Gly Val Val Ile Ala Asp Leu Asp Phe Ala
            260                 265                 270
Leu Ile Asp Glu Arg Lys Arg Leu Met Asp Ser Arg Gly Leu Tyr Ser
        275                 280                 285
Arg Pro Glu Leu Leu Ser Leu Leu Ile Asp Arg Met Pro Thr Ser His
    290                 295                 300
Val His Glu Arg Val Glu Arg Ser Met Ala Met Ala
305                 310                 315

<210> 227
<211> 1035
<212> DNA
<213> Unknown

<220>
<223> Obtained from an environmental sample

<400> 227
atgtccgatc gacgcatcgt tcgcgcggca gccattcaga tcgccccgga tctcgagcgc      60
tgcagtgtaa cgctcgagaa ggtttgctcg gcgatcgacg aagccgcggg caaaggcgct     120
caactgagcg tctttcccga gaccttcgtc ccgtactacc cctatttctc gtttgtccgc     180
cctccggtcg catcgggggc cgatcacatg cggctatacg aggaagcggt ggtggtgccg     240
ggtcccgtga cgcaggctgt ttccgaacgt gcgcgcatgc atgggatggt ggtggtgctc     300
ggcgtcaacg agcgcgacca cgggagcctc tacaacaccc aactgatttt cgattgcgat     360
ggtcggctcg ctctgaaacg ccgcaagatc acgccgacgt ttcacgagcg catgatctgg     420
ggccagggcg acgccagtgg actgaaagtc gcgcgcacgg gtatcgggcg ggtcggcgcg     480
ctcgcgtgct gggagcatta caacccgctc gcgcgctacg cgctgatgac ccagcacgaa     540
gagatccatt gcagtcaatt tccaggctcg ctcgtcggtc cgatcttctc cgagcagatg     600
gacgtgacga tccgccatca cgccctcgaa tcggggtgct tcgtcgtgaa cgcaaccggt     660
tggctcacgg acgcgcagat cgcctcgatc accgatgacc cgaagcttca acgggcgctg     720
cgcggtggct gcaacacggc gatcgtttca ccggaaggcc agcatctggc ggagccgttg     780
cgcgaaggcg aggggatggt ggtcgcggac ctcgacatgt cgctcatcac caagcgcaag     840
cgaatgatgg attcggtcgg ccactatgcg cggccggaac tgttgagcct cgcgatcaac     900
gatcgtcccg cagccccttt cggccggatg tgcgctgccg aagcaatgcg gggagccgac      960
gacgtcgtca cagtaggagc atttcatgag cgccagcgag aacgtgtcgg cgaagagccg    1020
gcaattgatg actga                                                     1035

<210> 228
<211> 344
<212> PRT
<213> Unknown

<220>
<223> Obtained from an environmental sample

<400> 228
Met Ser Asp Arg Arg Ile Val Arg Ala Ala Ala Ile Gln Ile Ala Pro
1               5                   10                  15
Asp Leu Glu Arg Cys Ser Val Thr Leu Glu Lys Val Cys Ser Ala Ile
            20                  25                  30
Asp Glu Ala Ala Gly Lys Gly Ala Gln Leu Ser Val Phe Pro Glu Thr
        35                  40                  45
Phe Val Pro Tyr Tyr Pro Tyr Phe Ser Phe Val Arg Pro Pro Val Ala
    50                  55                  60
Ser Gly Ala Asp His Met Arg Leu Tyr Glu Glu Ala Val Val Val Pro
65                  70                  75                  80
Gly Pro Val Thr Gln Ala Val Ser Glu Arg Ala Arg Met His Gly Met
                85                  90                  95
Val Val Val Leu Gly Val Asn Glu Arg Asp His Gly Ser Leu Tyr Asn
            100                 105                 110
Thr Gln Leu Ile Phe Asp Cys Asp Gly Arg Leu Ala Leu Lys Arg Arg
        115                 120                 125
Lys Ile Thr Pro Thr Phe His Glu Arg Met Ile Trp Gly Gln Gly Asp
    130                 135                 140
Ala Ser Gly Leu Lys Val Ala Arg Thr Gly Ile Gly Arg Val Gly Ala
145                 150                 155                 160
Leu Ala Cys Trp Glu His Tyr Asn Pro Leu Ala Arg Tyr Ala Leu Met
                165                 170                 175
Thr Gln His Glu Glu Ile His Cys Ser Gln Phe Pro Gly Ser Leu Val
            180                 185                 190
Gly Pro Ile Phe Ser Glu Gln Met Asp Val Thr Ile Arg His His Ala
        195                 200                 205
Leu Glu Ser Gly Cys Phe Val Val Asn Ala Thr Gly Trp Leu Thr Asp
    210                 215                 220

Ala Gln Ile Ala Ser Ile Thr Asp Asp Pro Lys Leu Gln Arg Ala Leu
225                 230                 235                 240
Arg Gly Gly Cys Asn Thr Ala Ile Val Ser Pro Glu Gly Gln His Leu
                245                 250                 255
Ala Glu Pro Leu Arg Glu Gly Glu Gly Met Val Val Ala Asp Leu Asp
            260                 265                 270
Met Ser Leu Ile Thr Lys Arg Lys Arg Met Met Asp Ser Val Gly His
        275                 280                 285
Tyr Ala Arg Pro Glu Leu Leu Ser Leu Ala Ile Asn Asp Arg Pro Ala
    290                 295                 300
Ala Pro Phe Gly Arg Met Cys Ala Ala Glu Ala Met Arg Gly Ala Asp
305                 310                 315                 320
Asp Val Val Thr Val Gly Ala Phe His Glu Arg Gln Arg Glu Arg Val
                325                 330                 335
Gly Glu Glu Pro Ala Ile Asp Asp
            340

<210> 229
<211> 975
<212> DNA
<213> Unknown

<220>
<223> Obtained from an environmental sample

<400> 229
atgcctgaaa acaaagtcaa agttgccctc gttcagcatc cgcctgtttt tctgaatttg      60
ccgaaaacgt tggaaaaagt agaaggtttg gcgcgagagt gcgccgccaa tgaagcgaaa     120
atcgttgtct ttcctgaaac ctggctgacc ggctatccgg tctggcttga tgaagcgccg     180
agagccgcgc tgtgggatta tccgcccgcc aagcgtttgt atcaatacct cacggaaaat     240
tcattgcaga ttccgagcgc tgagtttgaa tctttgcgcg aaatcgctaa gaaaaattcc     300
ctttatttag tcgttggagt tcacgaacga agcggcggaa cgctctacaa tacgataatt     360
tatctcacgc ccgacggcag ttataaaact caccgcaaat tggttccaac ttacacggaa     420
agacttgtct ggggcgcagg cgacggaagc ggtctaaatg ttgtggaaac gccttacggg     480
attctcggag gtttgatttg ctgggaacat tggatgcctc tggctagggc ggcaatgcat     540
tcaaaaaatg aagcgattca cgtttgccaa tttcccacgg ttcacgagcg acatcaaatc     600
gccagccgtc attacgcctt cgaagggcag tgttttgtct tgacttccgg ttgcgcgatg     660
acgaaaacgg atgttttgga aggttttgaa tcgctcgaaa caaacgacca cgaagttttc     720
gggcttttgg attcgataga aaaggaagaa ctgatgcgtg gcggaagcgc gattattgcg     780
cccgatttga gctattcggt cgagccggtt tttgacgaaa aaacgattgt ttacggcgaa     840
ttaaatctcg atttaaccaa gcagggacat ctgtttttgg ataccgacgg acattattcg     900
cgtcccgatg ttttcgagtt gcgcgtcaac gataaagcga accgaaacgt ccgttttgca     960
tccgaaacag tatag                                                      975

<210> 230
<211> 324
<212> PRT
<213> Unknown

<220>
<223> Obtained from an environmental sample

<400> 230
Met Pro Glu Asn Lys Val Lys Val Ala Leu Val Gln His Pro Pro Val
1               5                   10                  15
Phe Leu Asn Leu Pro Lys Thr Leu Glu Lys Val Glu Gly Leu Ala Arg
            20                  25                  30

Glu Cys Ala Ala Asn Glu Ala Lys Ile Val Val Phe Pro Glu Thr Trp
        35                  40                  45
Leu Thr Gly Tyr Pro Val Trp Leu Asp Glu Ala Pro Arg Ala Ala Leu
    50                  55                  60
Trp Asp Tyr Pro Pro Ala Lys Arg Leu Tyr Gln Tyr Leu Thr Glu Asn
65                  70                  75                  80
Ser Leu Gln Ile Pro Ser Ala Glu Phe Glu Ser Leu Arg Glu Ile Ala
                85                  90                  95
Lys Lys Asn Ser Leu Tyr Leu Val Val Gly Val His Glu Arg Ser Gly
            100                 105                 110
Gly Thr Leu Tyr Asn Thr Ile Ile Tyr Leu Thr Pro Asp Gly Ser Tyr
        115                 120                 125
Lys Thr His Arg Lys Leu Val Pro Thr Tyr Thr Glu Arg Leu Val Trp
    130                 135                 140
Gly Ala Gly Asp Gly Ser Gly Leu Asn Val Val Glu Thr Pro Tyr Gly
145                 150                 155                 160
Ile Leu Gly Gly Leu Ile Cys Trp Glu His Trp Met Pro Leu Ala Arg
                165                 170                 175
Ala Ala Met His Ser Lys Asn Glu Ala Ile His Val Cys Gln Phe Pro
            180                 185                 190
Thr Val His Glu Arg His Gln Ile Ala Ser Arg His Tyr Ala Phe Glu
        195                 200                 205
Gly Gln Cys Phe Val Leu Thr Ser Gly Cys Ala Met Thr Lys Thr Asp
    210                 215                 220
Val Leu Glu Gly Phe Glu Ser Leu Glu Thr Asn Asp His Glu Val Phe
225                 230                 235                 240
Gly Leu Leu Asp Ser Ile Glu Lys Glu Glu Leu Met Arg Gly Gly Ser
                245                 250                 255
Ala Ile Ile Ala Pro Asp Leu Ser Tyr Ser Val Glu Pro Val Phe Asp
            260                 265                 270
Glu Lys Thr Ile Val Tyr Gly Glu Leu Asn Leu Asp Leu Thr Lys Gln
        275                 280                 285
Gly His Leu Phe Leu Asp Thr Asp Gly His Tyr Ser Arg Pro Asp Val
    290                 295                 300
Phe Glu Leu Arg Val Asn Asp Lys Ala Asn Arg Asn Val Arg Phe Ala
305                 310                 315                 320
Ser Glu Thr Val

<210> 231
<211> 1062
<212> DNA
<213> Unknown

<220>
<223> Obtained from an environmental sample

<400> 231
atgagtcaga tccatccaaa acttaaggtc gcggccgtgc aggctgcccc cgcctttctc      60
gatctcgatg catcgatcga aaagacaata cgctatgtcg acgaagcggc tgcggccggg     120
gcgaagttga ttgcgtttcc ggaaacctgg attcccggct acccatggtg gatctggctc     180
ggcgctccgg cctgggcgat catgcgtggc ttcgtctcgc gctatttcga caactcgctg     240
caatatggca gtccggaagc tgaacggctg cgggacgccg ccaggcgcaa caagatatac     300
atcgccctcg gcctgtccga gcgcgacggg ggcagtcttt atatcgcgca atggatcatc     360
gggcctggcg gcgaaacggt tgcacaacgc cgcaagctca agcccacgca cgccgagcgc     420
actgtattcg gcgaaggcga tggttcacat ctggccgtgc atgatctcga tattggaaga     480
ttgggcgcgc tttgttgctg ggaacatctg caaccgttgt cgaaatatgc aatgtacgcc     540

cagaacgagc aaattcacgt cgccgcctgg ccgagcttct cgctatacga tccctttgca 600
cacgcactcg gcgccgaggt caataacgct gcgagcaaga tctatgcggt cgagggatcg 660
tgcttcgtca ttgcgccgtg cgcaacggtt tcgcaggtga tgatcgatga gctctgcgat 720
acccccgaaa agcatcaatt ccttcacgtc ggcggcggct tcgccgtcat ttacggtccc 780
gacggctcgc cactggccaa acctcttccg ccagaccagg agggacttct ctatgccgac 840
atcgatctcg gcatgatctc ggtcgccaag gccgcagccg atcccgccgg acattatgca 900
cgtcccgatg taactcgcct gctgttcaac aatcgcccgg caaaccgcgt cgagaagctg 960
gcgttgccgg tcgatcagga ggccgaagtg gatagtccgc tgaaggctcc cgacgcatct 1020
cccaaagtga cggcgctcaa gccgtcgcag gctgcggagt ag 1062

<210> 232
<211> 353
<212> PRT
<213> Unknown

<220>
<223> Obtained from an environmental sample

<400> 232
Met Ser Gln Ile His Pro Lys Leu Lys Val Ala Ala Val Gln Ala Ala
1               5                   10                  15
Pro Ala Phe Leu Asp Leu Asp Ala Ser Ile Glu Lys Thr Ile Arg Tyr
            20                  25                  30
Val Asp Glu Ala Ala Ala Ala Gly Ala Lys Leu Ile Ala Phe Pro Glu
        35                  40                  45
Thr Trp Ile Pro Gly Tyr Pro Trp Trp Ile Trp Leu Gly Ala Pro Ala
    50                  55                  60
Trp Ala Ile Met Arg Gly Phe Val Ser Arg Tyr Phe Asp Asn Ser Leu
65                  70                  75                  80
Gln Tyr Gly Ser Pro Glu Ala Glu Arg Leu Arg Asp Ala Ala Arg Arg
                85                  90                  95
Asn Lys Ile Tyr Ile Ala Leu Gly Leu Ser Glu Arg Asp Gly Gly Ser
            100                 105                 110
Leu Tyr Ile Ala Gln Trp Ile Ile Gly Pro Gly Gly Glu Thr Val Ala
        115                 120                 125
Gln Arg Arg Lys Leu Lys Pro Thr His Ala Glu Arg Thr Val Phe Gly
    130                 135                 140
Glu Gly Asp Gly Ser His Leu Ala Val His Asp Leu Asp Ile Gly Arg
145                 150                 155                 160
Leu Gly Ala Leu Cys Cys Trp Glu His Leu Gln Pro Leu Ser Lys Tyr
                165                 170                 175
Ala Met Tyr Ala Gln Asn Glu Gln Ile His Val Ala Ala Trp Pro Ser
            180                 185                 190
Phe Ser Leu Tyr Asp Pro Phe Ala His Ala Leu Gly Ala Glu Val Asn
        195                 200                 205
Asn Ala Ala Ser Lys Ile Tyr Ala Val Glu Gly Ser Cys Phe Val Ile
    210                 215                 220
Ala Pro Cys Ala Thr Val Ser Gln Val Met Ile Asp Glu Leu Cys Asp
225                 230                 235                 240
Thr Pro Glu Lys His Gln Phe Leu His Val Gly Gly Gly Phe Ala Val
                245                 250                 255
Ile Tyr Gly Pro Asp Gly Ser Pro Leu Ala Lys Pro Leu Pro Pro Asp
            260                 265                 270
Gln Glu Gly Leu Leu Tyr Ala Asp Ile Asp Leu Gly Met Ile Ser Val
        275                 280                 285
Ala Lys Ala Ala Ala Asp Pro Ala Gly His Tyr Ala Arg Pro Asp Val
    290                 295                 300

Thr Arg Leu Leu Phe Asn Asn Arg Pro Ala Asn Arg Val Glu Lys Leu
305                 310                 315                 320
Ala Leu Pro Val Asp Gln Glu Ala Glu Val Asp Ser Pro Leu Lys Ala
                325                 330                 335
Pro Asp Ala Ser Pro Lys Val Thr Ala Leu Lys Pro Ser Gln Ala Ala
            340                 345                 350
Glu

<210> 233
<211> 1002
<212> DNA
<213> Unknown

<220>
<223> Obtained from an environmental sample

<400> 233
gtgagcacca tcgtcaaagc cgcggccgtg cagatcagcc ccgtgctgta cagccgcgag      60
ggcaccgtcg agcgggtcgt gaagaagatc cgggagctgg gcgaaaaggg cgtccagttc     120
gccaccttcc ccgagaccgt catcccttac tacccgtact tttccttcgt tcagacgccc     180
ttgcagatcc tcgccggccc cgagcatctg aagctgctcg accagtcggt gaccgtgccg     240
tcccccgcca cggacgcgat cggccaggcc gcccggcagg caggaatggt ggtgtccatc     300
ggcgtcaacg agcgtgacgg cggcaccctg tacaacacgc agctgctctt cgacgcggac     360
ggcgcgctga tccagcgtcg ccgcaagatc aagcccaccc actacgagcg catgatctgg     420
ggcgagggcg acggctccgg cctgcgcgcc gtcgacagcc aggtcggtcg tatcggccag     480
ctggcctgct gggagcacaa caaccccctg gcgcgctacg ccatgatggc cgacggcgag     540
cagatccatt cggccatgta tccgggctcg atgttcggcg acccgttcgc ccagaagacg     600
gaaatcaaca tccggcagca tgcgctggaa tccggatgct tcgtcgtctg ctcgacggcc     660
tggttggacg ccgatcagca ggcgcaaatc atgcaggaca cgggctgcgc catcggcccg     720
atctcgggcg gctgcctcac ggcgatcgtg gcgcccgacg gcacgttcct gggcgaaccg     780
ctcacgtcgg gcgagggcga ggtcatcgcc gacctcgatt tcaagctgat cgacaagcgc     840
aagcagacga tggactcgcg cggccactac aaccgccccg aactgctcag cctgctgatc     900
gatcgaacgc cgacgtcgaa cgtccatgag cgcgccgcgc acccgaaggt cgaggcgtca     960
caaacggctg gcgacacgga gcggacccgc gaggtcctgt aa                       1002

<210> 234
<211> 333
<212> PRT
<213> Unknown

<220>
<223> Obtained from an environmental sample

<400> 234
Val Ser Thr Ile Val Lys Ala Ala Ala Val Gln Ile Ser Pro Val Leu
 1               5                  10                  15
Tyr Ser Arg Glu Gly Thr Val Glu Arg Val Val Lys Lys Ile Arg Glu
            20                  25                  30
Leu Gly Glu Lys Gly Val Gln Phe Ala Thr Phe Pro Glu Thr Val Ile
        35                  40                  45
Pro Tyr Tyr Pro Tyr Phe Ser Phe Val Gln Thr Pro Leu Gln Ile Leu
    50                  55                  60
Ala Gly Pro Glu His Leu Lys Leu Leu Asp Gln Ser Val Thr Val Pro
65                  70                  75                  80
Ser Pro Ala Thr Asp Ala Ile Gly Gln Ala Ala Arg Gln Ala Gly Met
                85                  90                  95

Val Val Ser Ile Gly Val Asn Glu Arg Asp Gly Gly Thr Leu Tyr Asn
            100                 105                 110
Thr Gln Leu Leu Phe Asp Ala Asp Gly Ala Leu Ile Gln Arg Arg Arg
        115                 120                 125
Lys Ile Lys Pro Thr His Tyr Glu Arg Met Ile Trp Gly Glu Gly Asp
    130                 135                 140
Gly Ser Gly Leu Arg Ala Val Asp Ser Gln Val Gly Arg Ile Gly Gln
145                 150                 155                 160
Leu Ala Cys Trp Glu His Asn Asn Pro Leu Ala Arg Tyr Ala Met Met
                165                 170                 175
Ala Asp Gly Glu Gln Ile His Ser Ala Met Tyr Pro Gly Ser Met Phe
            180                 185                 190
Gly Asp Pro Phe Ala Gln Lys Thr Glu Ile Asn Ile Arg Gln His Ala
        195                 200                 205
Leu Glu Ser Gly Cys Phe Val Val Cys Ser Thr Ala Trp Leu Asp Ala
    210                 215                 220
Asp Gln Gln Ala Gln Ile Met Gln Asp Thr Gly Cys Ala Ile Gly Pro
225                 230                 235                 240
Ile Ser Gly Gly Cys Leu Thr Ala Ile Val Ala Pro Asp Gly Thr Phe
                245                 250                 255
Leu Gly Glu Pro Leu Thr Ser Gly Glu Gly Glu Val Ile Ala Asp Leu
            260                 265                 270
Asp Phe Lys Leu Ile Asp Lys Arg Lys Gln Thr Met Asp Ser Arg Gly
        275                 280                 285
His Tyr Asn Arg Pro Glu Leu Leu Ser Leu Leu Ile Asp Arg Thr Pro
    290                 295                 300
Thr Ser Asn Val His Glu Arg Ala Ala His Pro Lys Val Glu Ala Ser
305                 310                 315                 320
Gln Thr Ala Gly Asp Thr Glu Arg Thr Arg Glu Val Leu
                325                 330

<210> 235
<211> 993
<212> DNA
<213> Unknown

<220>
<223> Obtained from an environmental sample

<400> 235
atgaaaattg ttaaagcggc agcagttcag ctgagccccg tcctctatag ccgcgagggg      60
acggtcgaaa gagtagtgcg gaagattcat caacttggtc aacagggagt gcagtttgcc     120
accttcccgg aaacagtggt gccttactac ccgtattttt cgatcgtgca gtccgggtat     180
caaatccttc gcggcggtga gttcgtgaag ctgctcgatc agtcagtgac ggtgccatct     240
cttgccaccg aagcgatcgc cgaggcctgc aggcaggcgg gcgtcgttgt ctccatcggc     300
gtcaatgagc gtgacggcgg aactatatac aatgcgcagc ttctgtttga ttcggacggc     360
acattgattc agaggcgacg caagatcacg cccacccact acgagcgcat gatctggggc     420
cagggcgatg gctcgggtct gcgggctgtg gacagcaagg tggcacgtat tggtcaactg     480
gcgtgctttg agcattacaa ccccctcgca cgatacgcga tgatcgccga tggcgagcag     540
atccactctg caatgtttcc cggttccatg ttcggcgatg gtttcgcgga gaggaccgag     600
atcgcggtca ggcagcatgc gcaggagtcc ggatgctttg tagtttgtgc tacggcgtgg     660
ctggatgccg accagcaggc tcaaattgcc gcggacacag gcatcaccga cctgggaccg     720
atctccggcg gttgcttcac tgcgatcatt gcacctgatg ggagcctgct gggtcaacca     780
atccgctcgg gcgaaggtga cgtcattgtc gatctcgatt tcactctgat cgacaggcgg     840
aagcatgttg tggactcgag aggtcactac agccggccgg aattgctaag cctgctgatc     900
gaccgtactc ccacagcgca cgttcacgaa cgggccgcgc actctcactt ggccgccgag     960
caatgcttgg aggatcttaa cgcgcttgct taa                                  993

<210> 236
<211> 330
<212> PRT
<213> Unknown

<220>
<223> Obtained from an environmental sample

<400> 236
Met Lys Ile Val Lys Ala Ala Ala Val Gln Leu Ser Pro Val Leu Tyr
1               5                   10                  15
Ser Arg Glu Gly Thr Val Glu Arg Val Val Arg Lys Ile His Gln Leu
            20                  25                  30
Gly Gln Gln Gly Val Gln Phe Ala Thr Phe Pro Glu Thr Val Val Pro
        35                  40                  45
Tyr Tyr Pro Tyr Phe Ser Ile Val Gln Ser Gly Tyr Gln Ile Leu Arg
    50                  55                  60
Gly Gly Glu Phe Val Lys Leu Leu Asp Gln Ser Val Thr Val Pro Ser
65                  70                  75                  80
Leu Ala Thr Glu Ala Ile Ala Glu Ala Cys Arg Gln Ala Gly Val Val
                85                  90                  95
Val Ser Ile Gly Val Asn Glu Arg Asp Gly Gly Thr Ile Tyr Asn Ala
            100                 105                 110
Gln Leu Leu Phe Asp Ser Asp Gly Thr Leu Ile Gln Arg Arg Arg Lys
        115                 120                 125
Ile Thr Pro Thr His Tyr Glu Arg Met Ile Trp Gly Gln Gly Asp Gly
    130                 135                 140
Ser Gly Leu Arg Ala Val Asp Ser Lys Val Ala Arg Ile Gly Gln Leu
145                 150                 155                 160
Ala Cys Phe Glu His Tyr Asn Pro Leu Ala Arg Tyr Ala Met Ile Ala
                165                 170                 175
Asp Gly Glu Gln Ile His Ser Ala Met Phe Pro Gly Ser Met Phe Gly
            180                 185                 190
Asp Gly Phe Ala Glu Arg Thr Glu Ile Ala Val Arg Gln His Ala Gln
        195                 200                 205
Glu Ser Gly Cys Phe Val Val Cys Ala Thr Ala Trp Leu Asp Ala Asp
    210                 215                 220
Gln Gln Ala Gln Ile Ala Ala Asp Thr Gly Ile Thr Asp Leu Gly Pro
225                 230                 235                 240
Ile Ser Gly Gly Cys Phe Thr Ala Ile Ile Ala Pro Asp Gly Ser Leu
                245                 250                 255
Leu Gly Gln Pro Ile Arg Ser Gly Glu Gly Asp Val Ile Val Asp Leu
            260                 265                 270
Asp Phe Thr Leu Ile Asp Arg Arg Lys His Val Val Asp Ser Arg Gly
        275                 280                 285
His Tyr Ser Arg Pro Glu Leu Leu Ser Leu Leu Ile Asp Arg Thr Pro
    290                 295                 300
Thr Ala His Val His Glu Arg Ala Ala His Ser His Leu Ala Ala Glu
305                 310                 315                 320
Gln Cys Leu Glu Asp Leu Asn Ala Leu Ala
                325                 330

<210> 237
<211> 993
<212> DNA
<213> Unknown

<220>
<223> Obtained from an environmental sample

<400> 237
atgaaagtcg tcaaagccgc cgcagtgcag atcagtcctg ttctctacag ccgagaggca 60
accgtcgcca aggtcgtgca gaagatccac gaactcggcc agaaaggcgt gcagttcgcc 120
acctttccag agaccgtagt gccttactac ccgtactttt ccgccgtcca gacaggcatc 180
gagcttctgt ccggcacgga gcatctccgg ctgctcgatc aggccgtgac ggtgccgtct 240
gccgctaccg acgcgatcgg agaggcagcc cggaaggcag gcatggtggt gtcgatcggc 300
gtcaatgaac gcgatggcgg caccttgtac aacacacagt tgctcttcga tgccgatggc 360
accttgatcc agcgccgccg caagatcacg ccgacccact tcgaacgcat gatctggggc 420
cagggggacg gttcgggcct gcgcgctgtc gacagcaagg tcggtcgcat tggccagttg 480
gcctgcttcg agcacaacaa cccgctggcc cgctacgcgt tgattgccga cggcgagcag 540
atccattccg ccatgtatcc gggttctgct tttggcgaag gatttgccca aaggatggaa 600
atcaatatcc gccagcatgc gctggagtcg ggtgcgttcg tcgtcaacgc aacggcctgg 660
ctggatgctg accagcaggc gcagatcatg aaggacaccg gctgcgggat tggcccgatc 720
tcgggcggtt gcttcaccac gatcgtgtca cccgacggca tgctgatggc cgaacccctg 780
cgctcgggcg agggtgaggt catcgtcgat ctcgacttca cgctgatcga ccgtcgcaag 840
atgttgatgg actcggcggg ccactataac cgcccggaac tgctcagtct catgatcgac 900
cgcaccccga ccgcgcacgt tcatgaacgc gctgcgcgtc cggtgtcggg cgttgagcag 960
aacccggagg aacttcgcat cccggccgcg tga 993

<210> 238
<211> 330
<212> PRT
<213> Unknown

<220>
<223> Obtained from an environmental sample

<400> 238
Met Lys Val Val Lys Ala Ala Ala Val Gln Ile Ser Pro Val Leu Tyr
1               5                   10                  15
Ser Arg Glu Ala Thr Val Ala Lys Val Val Gln Lys Ile His Glu Leu
            20                  25                  30
Gly Gln Lys Gly Val Gln Phe Ala Thr Phe Pro Glu Thr Val Val Pro
        35                  40                  45
Tyr Tyr Pro Tyr Phe Ser Ala Val Gln Thr Gly Ile Glu Leu Leu Ser
    50                  55                  60
Gly Thr Glu His Leu Arg Leu Leu Asp Gln Ala Val Thr Val Pro Ser
65                  70                  75                  80
Ala Ala Thr Asp Ala Ile Gly Glu Ala Ala Arg Lys Ala Gly Met Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Lys Val Gly Arg Ile Gly Gln Leu
145                 150                 155                 160
Ala Cys Phe Glu His Asn Asn Pro Leu Ala Arg Tyr Ala Leu Ile Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu

195                 200                 205
Glu Ser Gly Ala Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Ser Gly Gly Cys Phe Thr Thr Ile Val Ser Pro Asp Gly Met Leu Met
                245                 250                 255
Ala Glu Pro Leu Arg Ser Gly Glu Gly Glu Val Ile Val Asp Leu Asp
            260                 265                 270
Phe Thr Leu Ile Asp Arg Arg Lys Met Leu Met Asp Ser Ala Gly His
        275                 280                 285
Tyr Asn Arg Pro Glu Leu Leu Ser Leu Met Ile Asp Arg Thr Pro Thr
    290                 295                 300
Ala His Val His Glu Arg Ala Ala Arg Pro Val Ser Gly Val Glu Gln
305                 310                 315                 320
Asn Pro Glu Glu Leu Arg Ile Pro Ala Ala
                325                 330

<210> 239
<211> 969
<212> DNA
<213> Unknown

<220>
<223> Obtained from an environmental sample

<400> 239
atgtccaatg agaataagta tgacacattt aaagttgctg cagtccaggc cacacctgtg      60
tttcttgatc gtgaagcaac catcgacaaa gcttgcgagt tgattgctac tgcaggtcgt     120
gaaggtgctc gcctgattgt ctttccagaa gcgttcatcc catcctatcc cgagtgggta     180
tggggtattc cctctggtga gcaaggttta ctcaacgaac tttattcaga gttgctcacc     240
aatgcggtta ccatacccag cgacgcgact gacagattgt gcgaggcggc aaagcttgcg     300
aatgcctatg tagtgatggg aatgagcgaa cggaatgtcg aagcgagtgg tgcaagcctg     360
tataacacga tgttgtatat agatgcacag ggggagattt tagggaaaca tcggaagctg     420
gtgccaacgg gtggtgaacg cctggtatgg gcgcaaggtg atggcagcac gctgcaggtc     480
tacgatactc cattgggaaa acttggtggg ttaatttgct gggaaaatta tatgccgctg     540
gcacgctata cgatgtatgc ctggggaaca caaatctatg ttgcagcaac gtgggattgc     600
ggccaaccct ggctctcaac gatacggcat attgctaaag aaggcagggt atacgtggtt     660
ggttgctgta tcgcgatgcg taaagatgat attccagatc gttactctat gaagcagaaa     720
tattatgctg aaatggatga atggataaat gttggggata gcgcgattgt caatcccgaa     780
ggacatttta ttgcagggcc tgtgcgcaag caagaagaaa ttctctatgc ggagattgat     840
ccacgtatga tgcaaggccc gaagtggatg cttgacgtgg cgggacatta tgcaagacca     900
gatgtgttcc agttgacggt gcatacggat gtgaggcaga tgatacgggt ggaagatgat     960
tctcaatga                                                             969

<210> 240
<211> 322
<212> PRT
<213> Unknown

<220>
<223> Obtained from an environmental sample

<400> 240
Met Ser Asn Glu Asn Lys Tyr Asp Thr Phe Lys Val Ala Ala Val Gln
1               5                   10                  15
Ala Thr Pro Val Phe Leu Asp Arg Glu Ala Thr Ile Asp Lys Ala Cys

                    20                  25                  30
Glu Leu Ile Ala Thr Ala Gly Arg Glu Gly Ala Arg Leu Ile Val Phe
        35                  40                  45
Pro Glu Ala Phe Ile Pro Ser Tyr Pro Glu Trp Val Trp Gly Ile Pro
    50                  55                  60
Ser Gly Glu Gln Gly Leu Leu Asn Glu Leu Tyr Ser Glu Leu Leu Thr
65                  70                  75                  80
Asn Ala Val Thr Ile Pro Ser Asp Ala Thr Asp Arg Leu Cys Glu Ala
                85                  90                  95
Ala Lys Leu Ala Asn Ala Tyr Val Val Met Gly Met Ser Glu Arg Asn
            100                 105                 110
Val Glu Ala Ser Gly Ala Ser Leu Tyr Asn Thr Met Leu Tyr Ile Asp
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Thr Met Tyr Ala Trp Gly Thr Gln Ile
            180                 185                 190
Tyr Val Ala Ala Thr Trp Asp Cys Gly Gln Pro Trp Leu Ser Thr Ile
        195                 200                 205
Arg His Ile Ala Lys Glu Gly Arg Val Tyr Val Val Gly Cys Cys Ile
    210                 215                 220
Ala Met Arg Lys Asp Asp Ile Pro Asp Arg Tyr Ser Met Lys Gln Lys
225                 230                 235                 240
Tyr Tyr Ala Glu Met Asp Glu Trp Ile Asn Val Gly Asp Ser Ala Ile
                245                 250                 255
Val Asn Pro Glu Gly His Phe Ile Ala Gly Pro Val Arg Lys Gln Glu
            260                 265                 270
Glu Ile Leu Tyr Ala Glu Ile Asp Pro Arg Met Met Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asp Val Arg Gln Met Ile Arg Val Glu Asp Asp
305                 310                 315                 320
Ser Gln

<210> 241
<211> 972
<212> DNA
<213> Unknown

<220>
<223> Obtained from an environmental sample

<400> 241
atgatatcca acgagcataa caatactcca ttcaaggttg ctgctgtgca ggctacacct      60
gtgtttcttg atcgcgaagc aacgatcgat aaagcgtgtg aactgatcgc tactgccggt     120
catgaaggcg ctcgtttgat tgtttttcca gaagcgttca tcccatccta tcccgagtgg     180
gtatggggaa ttccctctgg cgagcaaggt ttgctcaacg atctgtatgc agagttactc     240
accaattcag ttacgatacc cagcaacgca actgacaggc tttgtagagc cgcgaagctt     300
gctaatgcct acgtggtgat ggggatgagc gaacggaata tcgaagcgag cggcgcaagc     360
ctgtacaata cgatgttata tatagatgca cagggtgaga ttttgggcaa acatcgaaag     420
ctggtgccaa cgggcggaga acgcctggta tgggcacaag gagatggaag cacgctgcag     480

gtttacgata cacctctagg aaagcttggt ggtttaattt gctgggaaaa ttatatgccg 540
ctggcacgct acgctatgta tgcctgggga actcaaatct acgtcgcggc aacgtgggat 600
cgcggccaac cctggctctc aacgatacgg catatcgcta aagagggcag ggtatacgta 660
atcggttgct gtatcgcgat gcgtaaagac gatattccag ataggtactc catgaagcag 720
aagtattatg cggagatgga tgaatggatc aacgtaggtg acagcgcgat tgtcaatcct 780
gagggggact tcattgcggg gcctgtgagc aagcaggagg aaattctcta tgcggagatt 840
gatccgcgga tggtgcaagg tccgaagtgg atgctggatg tggcggggca ttacgcgagg 900
cctgatgtgt tcgagttgac ggtgcatacg gatgtgaggg agatgatgcg ggtggagcat 960
gattatcaat ga 972

<210> 242
<211> 323
<212> PRT
<213> Unknown

<220>
<223> Obtained from an environmental sample

<400> 242
Met Ile Ser Asn Glu His Asn Asn Thr Pro Phe Lys Val Ala Ala Val
1               5                   10                  15
Gln Ala Thr Pro Val Phe Leu Asp Arg Glu Ala Thr Ile Asp Lys Ala
            20                  25                  30
Cys Glu Leu Ile Ala Thr Ala Gly His Glu Gly Ala Arg Leu Ile Val
        35                  40                  45
Phe Pro Glu Ala Phe Ile Pro Ser Tyr Pro Glu Trp Val Trp Gly Ile
    50                  55                  60
Pro Ser Gly Glu Gln Gly Leu Leu Asn Asp Leu Tyr Ala Glu Leu Leu
65                  70                  75                  80
Thr Asn Ser Val Thr Ile Pro Ser Asn Ala Thr Asp Arg Leu Cys Arg
                85                  90                  95
Ala Ala Lys Leu Ala Asn Ala Tyr Val Val Met Gly Met Ser Glu Arg
            100                 105                 110
Asn Ile Glu Ala Ser Gly Ala Ser Leu Tyr Asn Thr Met Leu Tyr Ile
        115                 120                 125
Asp Ala Gln Gly Glu Ile Leu Gly Lys His Arg Lys Leu Val Pro Thr
    130                 135                 140
Gly Gly Glu Arg Leu Val Trp Ala Gln Gly Asp Gly Ser Thr Leu Gln
145                 150                 155                 160
Val Tyr Asp Thr Pro Leu Gly Lys Leu Gly Gly Leu Ile Cys Trp Glu
                165                 170                 175
Asn Tyr Met Pro Leu Ala Arg Tyr Ala Met Tyr Ala Trp Gly Thr Gln
            180                 185                 190
Ile Tyr Val Ala Ala Thr Trp Asp Arg Gly Gln Pro Trp Leu Ser Thr
        195                 200                 205
Ile Arg His Ile Ala Lys Glu Gly Arg Val Tyr Val Ile Gly Cys Cys
    210                 215                 220
Ile Ala Met Arg Lys Asp Asp Ile Pro Asp Arg Tyr Ser Met Lys Gln
225                 230                 235                 240
Lys Tyr Tyr Ala Glu Met Asp Glu Trp Ile Asn Val Gly Asp Ser Ala
                245                 250                 255
Ile Val Asn Pro Glu Gly Asp Phe Ile Ala Gly Pro Val Ser Lys Gln
            260                 265                 270
Glu Glu Ile Leu Tyr Ala Glu Ile Asp Pro Arg Met Val Gln Gly Pro
        275                 280                 285
Lys Trp Met Leu Asp Val Ala Gly His Tyr Ala Arg Pro Asp Val Phe
    290                 295                 300

Glu Leu Thr Val His Thr Asp Val Arg Glu Met Met Arg Val Glu His
305                 310                 315                 320
Asp Tyr Gln

<210> 243
<211> 999
<212> DNA
<213> Unknown

<220>
<223> Obtained from an environmental sample

<400> 243
atgaagcaaa ctcgagtagc gatcattcag gcggaacctg tatatctcaa tttgcaagcg      60
agtgttgcga gggctatcga tcttgccgga cgcgccgcga agcaaggagc gcgtctgata     120
gtgtttggag agacctggtt gccgggttat cccgcgtggc tggattactg tcccggcatg     180
gcgttctggg atcaccggcc gacaaaagaa gtgtttgccc ggacccgcga gaacagtgtt     240
gtaattccgg gaaaggaaat cgaacagctc tgtaaaactg cggcggagct gggagttgta     300
atttcgatcg gtgtaaacga aaaaattctg gaaggccccg gaaacggcac gctctacaat     360
tctcttttgc tgattgatga atcaggaaaa ctggccggcc atcaccgcaa actggttccg     420
acttatacgg aacggatggt gtggggaatg ggtgatggag gtggaatgga agccatatcg     480
actgcagccg gcagggttgg cggattgatt tgctgggagc actggatgcc attgagccgg     540
caggtgctgc acatgtcggg tgaggaaatt catgtggcag tgtggcccac ggttcatgag     600
gtgcaccagc ttgcatcacg ccattatgca tttgaagggc gttgttttgt gctcgcagcc     660
ggattgttga tgaaggtccg ggatattcct ccggagctgg aattgccttc tcagatgtcg     720
cgtgaatccg aagactggct tctgcgcggc gggagcgccg tcattggtcc ggatggaaag     780
tacattgtgg agccgttgtt tgatcgagag gcgattctca cagccgatct tgaattagcc     840
gcatgcgatc gtgaaaaaat gacgctggac gtaacgggac attattcccg ccccgatctt     900
tttcacctgg aattcaggaa acagcaatcc ggccatattg cgggagcagg aacgatcagc     960
cggcaaaaat cagcgccgga ccgcgcggac gatcactaa                            999

<210> 244
<211> 332
<212> PRT
<213> Unknown

<220>
<223> Obtained from an environmental sample

<400> 244
Met Lys Gln Thr Arg Val Ala Ile Ile Gln Ala Glu Pro Val Tyr Leu
1               5                   10                  15
Asn Leu Gln Ala Ser Val Ala Arg Ala Ile Asp Leu Ala Gly Arg Ala
            20                  25                  30
Ala Lys Gln Gly Ala Arg Leu Ile Val Phe Gly Glu Thr Trp Leu Pro
        35                  40                  45
Gly Tyr Pro Ala Trp Leu Asp Tyr Cys Pro Gly Met Ala Phe Trp Asp
    50                  55                  60
His Arg Pro Thr Lys Glu Val Phe Ala Arg Thr Arg Glu Asn Ser Val
65                  70                  75                  80
Val Ile Pro Gly Lys Glu Ile Glu Gln Leu Cys Lys Thr Ala Ala Glu
                85                  90                  95
Leu Gly Val Val Ile Ser Ile Gly Val Asn Glu Lys Ile Leu Glu Gly
            100                 105                 110
Pro Gly Asn Gly Thr Leu Tyr Asn Ser Leu Leu Leu Ile Asp Glu Ser
        115                 120                 125

Gly Lys Leu Ala Gly His His Arg Lys Leu Val Pro Thr Tyr Thr Glu
    130                 135                 140
Arg Met Val Trp Gly Met Gly Asp Gly Gly Gly Met Glu Ala Ile Ser
145                 150                 155                 160
Thr Ala Ala Gly Arg Val Gly Gly Leu Ile Cys Trp Glu His Trp Met
                165                 170                 175
Pro Leu Ser Arg Gln Val Leu His Met Ser Gly Glu Glu Ile His Val
            180                 185                 190
Ala Val Trp Pro Thr Val His Glu Val His Gln Leu Ala Ser Arg His
        195                 200                 205
Tyr Ala Phe Glu Gly Arg Cys Phe Val Leu Ala Ala Gly Leu Leu Met
    210                 215                 220
Lys Val Arg Asp Ile Pro Pro Glu Leu Glu Leu Pro Ser Gln Met Ser
225                 230                 235                 240
Arg Glu Ser Glu Asp Trp Leu Leu Arg Gly Gly Ser Ala Val Ile Gly
                245                 250                 255
Pro Asp Gly Lys Tyr Ile Val Glu Pro Leu Phe Asp Arg Glu Ala Ile
            260                 265                 270
Leu Thr Ala Asp Leu Glu Leu Ala Ala Cys Asp Arg Glu Lys Met Thr
        275                 280                 285
Leu Asp Val Thr Gly His Tyr Ser Arg Pro Asp Leu Phe His Leu Glu
    290                 295                 300
Phe Arg Lys Gln Gln Ser Gly His Ile Ala Gly Ala Gly Thr Ile Ser
305                 310                 315                 320
Arg Gln Lys Ser Ala Pro Asp Arg Ala Asp Asp His
                325                 330

<210> 245
<211> 999
<212> DNA
<213> Unknown

<220>
<223> Obtained from an environmental sample

<400> 245
atgggtgaga atgccaactt caccgtcgca gctgtccagg caacgccggt cttcttagac 60
cgggatgcga cggtcgagaa ggcttgcgag ctcatcgctg aagccgggcg aaacggagcg 120
cgcctggccg tttttcccga ggcgtttgtg ccggcttacc cggactgggt ctgggctgtt 180
ccgccgggcg attcaaggct gctgcacgag ctctacggtg agctgatcca gaactctgtc 240
acgattccca gcgagtcgac ggagaaactc tgccgggccg cccgcggggc caaagtctgc 300
gtggcgatcg gcatcaacga gaggaatgcg gaggcaagcg ggggtagcct ctacaacagc 360
ctcctgtaca tcagcccgga cggccaggtc ctcgggaagc accgcaagct cgttcccacc 420
ggagcggagc ggcttgtctg ggcgcagggc gacggcagca ctatcgacgt gtttgagttg 480
cctttctgtc gtttgggtgg cctcatctgt tgggagaact acatgccgct ggcccgttat 540
gcgatgtacg cctggggcac gcaggtctac gtcgcggcaa cgtgggacca cggcgaacct 600
tggctctcaa ccttgaggca tatcgccagg gaggggcgtg catatgtcat tggcgtttgc 660
atgccgatgc gcatgagcga catcccggac cgatacgagt tcaagcgcaa gtactatggc 720
gggcgcgact ggatcaatac tggtgacagc gccatcgtgg gtccggacgg aaacttcatc 780
gccggccccc tgagcgagcg cgaagagatc ctgtacgccg atatagacct gaatcggctt 840
gcgaactcga agtggatgct ggacgtcgcc gggcactatg cacggccgga cgtcttccag 900
ttgaccgtta accgcgagcc gaacccgatg atctctgagg atgggcacaa gacggttccc 960
acgctaccga aacgtgcggg gaagagtagg acgagatga 999

<210> 246
<211> 332
<212> PRT

<213> Unknown

<220>
<223> Obtained from an environmental sample

<400> 246
Met Gly Glu Asn Ala Asn Phe Thr Val Ala Ala Val Gln Ala Thr Pro
1               5                   10                  15
Val Phe Leu Asp Arg Asp Ala Thr Val Glu Lys Ala Cys Glu Leu Ile
            20                  25                  30
Ala Glu Ala Gly Arg Asn Gly Ala Arg Leu Ala Val Phe Pro Glu Ala
        35                  40                  45
Phe Val Pro Ala Tyr Pro Asp Trp Val Trp Ala Val Pro Pro Gly Asp
    50                  55                  60
Ser Arg Leu Leu His Glu Leu Tyr Gly Glu Leu Ile Gln Asn Ser Val
65                  70                  75                  80
Thr Ile Pro Ser Glu Ser Thr Glu Lys Leu Cys Arg Ala Ala Arg Gly
                85                  90                  95
Ala Lys Val Cys Val Ala Ile Gly Ile Asn Glu Arg Asn Ala Glu Ala
            100                 105                 110
Ser Gly Gly Ser Leu Tyr Asn Ser Leu Leu Tyr Ile Ser Pro Asp Gly
        115                 120                 125
Gln Val Leu Gly Lys His Arg Lys Leu Val Pro Thr Gly Ala Glu Arg
    130                 135                 140
Leu Val Trp Ala Gln Gly Asp Gly Ser Thr Ile Asp Val Phe Glu Leu
145                 150                 155                 160
Pro Phe Cys Arg Leu Gly Gly Leu Ile Cys Trp Glu Asn Tyr Met Pro
                165                 170                 175
Leu Ala Arg Tyr Ala Met Tyr Ala Trp Gly Thr Gln Val Tyr Val Ala
            180                 185                 190
Ala Thr Trp Asp His Gly Glu Pro Trp Leu Ser Thr Leu Arg His Ile
        195                 200                 205
Ala Arg Glu Gly Arg Ala Tyr Val Ile Gly Val Cys Met Pro Met Arg
    210                 215                 220
Met Ser Asp Ile Pro Asp Arg Tyr Glu Phe Lys Arg Lys Tyr Tyr Gly
225                 230                 235                 240
Gly Arg Asp Trp Ile Asn Thr Gly Asp Ser Ala Ile Val Gly Pro Asp
                245                 250                 255
Gly Asn Phe Ile Ala Gly Pro Leu Ser Glu Arg Glu Glu Ile Leu Tyr
            260                 265                 270
Ala Asp Ile Asp Leu Asn Arg Leu Ala Asn Ser Lys Trp Met Leu Asp
        275                 280                 285
Val Ala Gly His Tyr Ala Arg Pro Asp Val Phe Gln Leu Thr Val Asn
    290                 295                 300
Arg Glu Pro Asn Pro Met Ile Ser Glu Asp Gly His Lys Thr Val Pro
305                 310                 315                 320
Thr Leu Pro Lys Arg Ala Gly Lys Ser Arg Thr Arg
                325                 330

<210> 247
<211> 990
<212> DNA
<213> Unknown

<220>
<223> Obtained from an environmental sample

<400> 247
atgccgaccc ccaaggaaaa gtttagaatc gccgccgttc aggcttgccc cgtttttctg 60
gaccgggggg agacggtaaa gaaggcctgc cggctggcgg ccgaggccgg gggccagggc 120
gcccggctga tcgtgtttcc ggagtccttc atccccgctt acccggactg ggtgtgggcc 180
gttccgccgg ggagggagaa gctcctgaat gaaatgtacg ccgaattcct ggccggcgcg 240
gtggaagtcc cggggccggt gacggaggaa ttgggccggg cggcggaaag ggccggcgct 300
tacctggtca tgggggtcac cgagcgggac accgaggcca gcggggcaag cctgtacaac 360
accctcctct atttcggtcc ccagggaagc ctgctgggaa aacaccgcaa actggtgccc 420
acgggagggg aacggaccgt ctgggcccgg ggggacggca gcacgctgca ggtgtacgat 480
acccccctgg gaaagatcgg cggcctgatc tgctgggaga actacatgcc cctggcccgc 540
tacgccatgt atgcgtgggg cactcagatt tacctggccc ccacctggga ccggggggaa 600
ccctggcttt caaccctgcg gcacatcgcc aaggaagggc gggtttacgt ggtggggtgc 660
tgcatggcta tgcaaaaagg ggacatcccg gatcgcttcg aatacaagca aaaatactat 720
cccgcagccc gggagtggat caacacgggc gacagcgcca tcctgaaccc ggagggggaa 780
ttcatcgccg ggccggcggg aaagaaggaa gagatcctgt acgctgaaat agacccccgg 840
cagatggggg ggcccaagtg gatgctggac gtggccggcc attacgcccg gccggatgtc 900
ttcgaactga tcgttcaccg ggaggcccgg ccgatgatcc gggtgacgga agcgccgtct 960
ccgggagaga aagaaacggg tgaaggatag 990

<210> 248
<211> 329
<212> PRT
<213> Unknown

<220>
<223> Obtained from an environmental sample

<400> 248
Met Pro Thr Pro Lys Glu Lys Phe Arg Ile Ala Ala Val Gln Ala Cys
1               5                   10                  15
Pro Val Phe Leu Asp Arg Gly Glu Thr Val Lys Lys Ala Cys Arg Leu
            20                  25                  30
Ala Ala Glu Ala Gly Gly Gln Gly Ala Arg Leu Ile Val Phe Pro Glu
        35                  40                  45
Ser Phe Ile Pro Ala Tyr Pro Asp Trp Val Trp Ala Val Pro Pro Gly
    50                  55                  60
Arg Glu Lys Leu Leu Asn Glu Met Tyr Ala Glu Phe Leu Ala Gly Ala
65                  70                  75                  80
Val Glu Val Pro Gly Pro Val Thr Glu Glu Leu Gly Arg Ala Ala Glu
                85                  90                  95
Arg Ala Gly Ala Tyr Leu Val Met Gly Val Thr Glu Arg Asp Thr Glu
            100                 105                 110
Ala Ser Gly Ala Ser Leu Tyr Asn Thr Leu Leu Tyr Phe Gly Pro Gln
        115                 120                 125
Gly Ser Leu Leu Gly Lys His Arg Lys Leu Val Pro Thr Gly Gly Glu
    130                 135                 140
Arg Thr Val Trp Ala Arg Gly Asp Gly Ser Thr Leu Gln Val Tyr Asp
145                 150                 155                 160
Thr Pro Leu Gly Lys Ile Gly Gly Leu Ile Cys Trp Glu Asn Tyr Met
                165                 170                 175
Pro Leu Ala Arg Tyr Ala Met Tyr Ala Trp Gly Thr Gln Ile Tyr Leu
            180                 185                 190
Ala Pro Thr Trp Asp Arg Gly Glu Pro Trp Leu Ser Thr Leu Arg His
        195                 200                 205
Ile Ala Lys Glu Gly Arg Val Tyr Val Val Gly Cys Cys Met Ala Met
    210                 215                 220
Gln Lys Gly Asp Ile Pro Asp Arg Phe Glu Tyr Lys Gln Lys Tyr Tyr

225                 230                 235                 240
Pro Ala Ala Arg Glu Trp Ile Asn Thr Gly Asp Ser Ala Ile Leu Asn
                245                 250                 255
Pro Glu Gly Glu Phe Ile Ala Gly Pro Ala Gly Lys Lys Glu Glu Ile
            260                 265                 270
Leu Tyr Ala Glu Ile Asp Pro Arg Gln Met Gly Gly Pro Lys Trp Met
        275                 280                 285
Leu Asp Val Ala Gly His Tyr Ala Arg Pro Asp Val Phe Glu Leu Ile
    290                 295                 300
Val His Arg Glu Ala Arg Pro Met Ile Arg Val Thr Glu Ala Pro Ser
305                 310                 315                 320
Pro Gly Glu Lys Glu Thr Gly Glu Gly
                325

<210> 249
<211> 1017
<212> DNA
<213> Unknown

<220>
<223> Obtained from an environmental sample

<400> 249
atgggcattc aacatccgaa atatcgcgtc gcggtggtgc aggcggcacc ggcctggctc      60
gacctcgagg cgtcggtcag caagagcatc gcgctgatag aggaggccgc cgccaagggc     120
gccaagctga tcgcgttccc cgaggccttc atccccggct atccctggta catctggctg     180
gactcgccgg cctgggcgat cggccgcggc ttcgtgcagc gctatttcga caattcgctc     240
agctatgaca gcccgcaggc ggagcgcctg aggctcgcag tgaagaaggc cggcatgacc     300
gcagtgctcg gcctgtccga gcgcgacggc ggcagcctct atctcgcgca atggttgatc     360
ggacccgacg gcgagaccat cgcaaagcgg cgcaagctgc ggccgaccca tgccgagcgc     420
accgtctacg gcgagggcga cggcagcgac cttgcggtgc atgaccgccc cggcatcggc     480
cggctcggtg cgctgtgctg ctgggagcat ctgcagccgc tgtcgaaata cgcgatgtac     540
gcccagaacg agcaggtgca tgtcgcggcc tggccgagct tctcgctgta cgatccgttc     600
gcgccggcgc tcggctggga ggtcaacaat gcggcctcgc gcgtctatgc cgtcgagggc     660
tcctgcttcg tgctggcgcc ctgcgccacc gtctcgcagg cgatgatcga cgagctctgc     720
gaccgcgacg acaagcatgc gctgctgcat gttggcggcg gccatgccgc gatcttcggc     780
cccgacggca gcgcgatcgc ggacaagctt ccgtccgacc aggagggcct cctgttcgcc     840
gacatcgatc tcggcgcgat cgggatcgcg aagaatgccg ctgatccggc cgggcactat     900
tcgcgcccgg acgtgacgcg gctgctgctc aacaagaagc cctcgaagcg cgtcgagcac     960
ttcgcgctgc cgctcgacac gctcgcgggc gaggagatcg acgcggccgc aagctaa       1017

<210> 250
<211> 338
<212> PRT
<213> Unknown

<220>
<223> Obtained from an environmental sample

<400> 250
Met Gly Ile Gln His Pro Lys Tyr Arg Val Ala Val Val Gln Ala Ala
1               5                   10                  15
Pro Ala Trp Leu Asp Leu Glu Ala Ser Val Ser Lys Ser Ile Ala Leu
            20                  25                  30
Ile Glu Glu Ala Ala Ala Lys Gly Ala Lys Leu Ile Ala Phe Pro Glu
        35                  40                  45
Ala Phe Ile Pro Gly Tyr Pro Trp Tyr Ile Trp Leu Asp Ser Pro Ala

50                  55                  60
Trp Ala Ile Gly Arg Gly Phe Val Gln Arg Tyr Phe Asp Asn Ser Leu
65                  70                  75                  80
Ser Tyr Asp Ser Pro Gln Ala Glu Arg Leu Arg Leu Ala Val Lys Lys
                85                  90                  95
Ala Gly Met Thr Ala Val Leu Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asp Arg Pro Gly Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Pro Ala Leu Gly Trp Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Asp Asp Lys His Ala Leu Leu His Val Gly Gly Gly His Ala
                245                 250                 255
Ala Ile Phe Gly Pro Asp Gly Ser Ala Ile Ala Asp Lys Leu Pro Ser
            260                 265                 270
Asp Gln Glu Gly Leu Leu Phe Ala Asp Ile Asp Leu Gly Ala Ile Gly
        275                 280                 285
Ile Ala Lys Asn Ala Ala Asp Pro Ala Gly His Tyr Ser Arg Pro Asp
    290                 295                 300
Val Thr Arg Leu Leu Leu Asn Lys Lys Pro Ser Lys Arg Val Glu His
305                 310                 315                 320
Phe Ala Leu Pro Leu Asp Thr Leu Ala Gly Glu Glu Ile Asp Ala Ala
                325                 330                 335
Ala Ser

<210> 251
<211> 978
<212> DNA
<213> Unknown

<220>
<223> Obtained from an environmental sample

<400> 251
gtgaccatcg tgagagctgc cgccgtgcag atcagtcccg tgctctacag ccgggaagcc      60
accgtagaaa aagtcgttcg caagatccgc gaactgggaa gaaacggcgt gcagttcgcc     120
accttcccgg aaaccctggt gccctactac ccgtacttcg cggccgtgca gacgggcatc     180
gaactgctgt ccggcaagga gcacctgcga ctgctggaac aatccgtaac ggttccctcg     240
cccgccaccg atgccattgc ccaggcggca cgcgaagccg gcatggtggt gtccatcggt     300
gtcaacgagc gcgacggagg caccatctac aacacgcagc tgctgtttga cgccgacggc     360
acgctggtac agcgccgccg caagatcacg ccgacgcatt tcgagcgcat ggtctggggc     420
cagggcgacg gctcgggcct gcgagccgtg gacaccaagg ccggccgcat cggtcagctc     480
gcctgcttcg agcacaacaa cccgctggcg cgctacgcca tgatcgccga cggtgagcag     540
atccattcgg ccatgtaccc gggctctgcc ttcggcgagg gcttcgcgca gcgcatggaa     600

atcaacatac gccagcacgc cctggagtct ggctgcttcg tggtgaatgc gaccgcgtgg 660
ctggatgccg accagcaggc gcagatcatg aaggatacgg gctgcggcat cggcccgatc 720
tccggcggct gcttcacgac catcgtcacg ccggacggca tgctgatcgg tgaacccctc 780
cgcgaaggcg aaggcgaagt catcgccgac ctcgatttca ccctgatcga ccggcgcaag 840
ctgctggtgg actcggtggg ccactacaac cgtccggagc tgctgagcct gctgatcgat 900
cgcacccctg cggcgaactt ccatgagcgc aatgcgcttc cgtccgtcaa caccgccagc 960
agcctcgaaa tcgtctga 978

<210> 252
<211> 325
<212> PRT
<213> Unknown

<220>
<223> Obtained from an environmental sample

<400> 252
Val Thr Ile Val Arg Ala Ala Ala Val Gln Ile Ser Pro Val Leu Tyr
1               5                   10                  15
Ser Arg Glu Ala Thr Val Glu Lys Val Val Arg Lys Ile Arg Glu Leu
            20                  25                  30
Gly Arg Asn Gly Val Gln Phe Ala Thr Phe Pro Glu Thr Leu Val Pro
        35                  40                  45
Tyr Tyr Pro Tyr Phe Ala Ala Val Gln Thr Gly Ile Glu Leu Leu Ser
    50                  55                  60
Gly Lys Glu His Leu Arg Leu Leu Glu Gln Ser Val Thr Val Pro Ser
65                  70                  75                  80
Pro Ala Thr Asp Ala Ile Ala Gln Ala Ala Arg Glu Ala Gly Met Val
                85                  90                  95
Val Ser Ile Gly Val Asn Glu Arg Asp Gly Gly Thr Ile Tyr Asn Thr
            100                 105                 110
Gln Leu Leu Phe Asp Ala Asp Gly Thr Leu Val Gln Arg Arg Arg Lys
        115                 120                 125
Ile Thr Pro Thr His Phe Glu Arg Met Val Trp Gly Gln Gly Asp Gly
    130                 135                 140
Ser Gly Leu Arg Ala Val Asp Thr Lys Ala Gly Arg Ile Gly Gln Leu
145                 150                 155                 160
Ala Cys Phe Glu His Asn Asn Pro Leu Ala Arg Tyr Ala Met Ile Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Gly Cys Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Ser Gly Gly Cys Phe Thr Thr Ile Val Thr Pro Asp Gly Met Leu Ile
                245                 250                 255
Gly Glu Pro Leu Arg Glu Gly Glu Gly Glu Val Ile Ala Asp Leu Asp
            260                 265                 270
Phe Thr Leu Ile Asp Arg Arg Lys Leu Leu Val Asp Ser Val Gly His
        275                 280                 285
Tyr Asn Arg Pro Glu Leu Leu Ser Leu Leu Ile Asp Arg Thr Pro Ala
    290                 295                 300
Ala Asn Phe His Glu Arg Asn Ala Leu Pro Ser Val Asn Thr Ala Ser
305                 310                 315                 320

Ser Leu Glu Ile Val
                325

<210> 253
<211> 924
<212> DNA
<213> Unknown

<220>
<223> Obtained from an environmental sample

<400> 253
atgtcaaacg agaaccacaa ccaaacattc aaagttgccg cggtgcaggc cacacctgta      60
ttcctcgatc gtgaagcgac catcgacaaa gcttgcgagt tgattgctgc agccggcaat     120
gaaggggcga ggctggttgt cttcccggag gcattcatcc cgtcctatcc agattgggta     180
tgggcaatcc caccgggcga agaaggcgtg ctcaatgagt tgtacgcgga actgctctcc     240
aattcggtca cgattcccag tgacgtgacg gatagactgt gccgggccgc gaggcttgcc     300
aatgcctacg tagtgatggg gatgagcgaa tgcaatgccg aggccagtgg cgcaagcctg     360
tataacacgc tattgtacat cgatgcgaag ggtgaaatcc tgggtaaaca tcgaaagttg     420
gtgccaactg gcggcgagcg actggtgtgg gcacagggcg atggcagcac gctgcaggtc     480
tacgatactc cactgggtaa actcggcggt ttaatttgct gggagaatta tatgccgctg     540
gcccgctaca ccatgtacgc ctggggcaca caaatctata tcgcagcgac atgggatcgc     600
gggcaaccct ggctctccac cttgcggcat atcgccaaag aaggcagggt gtacgtgatc     660
ggctgttgta tcgcgatgcg caaagatgat atcccagagc gttacccaat gaagcagaag     720
ttttacgcgg aggccgatga gtggatcaat ataggcgaca gcgcgatcgt caatcctgaa     780
gggcagttta tcgcggggcc ggtacgcaaa caggaagaga ttctctacgc ggagattaat     840
ccgcgcatgg tgcaaggccc gaagtggatg ctcgacgtgg cagggcacta cgccaggccg     900
gacgtattcc agttgacagt gtaa                                            924

<210> 254
<211> 307
<212> PRT
<213> Unknown

<220>
<223> Obtained from an environmental sample

<400> 254
Met Ser Asn Glu Asn His Asn Gln Thr Phe Lys Val Ala Ala Val Gln
1               5                   10                  15
Ala Thr Pro Val Phe Leu Asp Arg Glu Ala Thr Ile Asp Lys Ala Cys
            20                  25                  30
Glu Leu Ile Ala Ala Ala Gly Asn Glu Gly Ala Arg Leu Val Val Phe
        35                  40                  45
Pro Glu Ala Phe Ile Pro Ser Tyr Pro Asp Trp Val Trp Ala Ile Pro
    50                  55                  60
Pro Gly Glu Glu Gly Val Leu Asn Glu Leu Tyr Ala Glu Leu Leu Ser
65                  70                  75                  80
Asn Ser Val Thr Ile Pro Ser Asp Val Thr Asp Arg Leu Cys Arg Ala
                85                  90                  95
Ala Arg Leu Ala Asn Ala Tyr Val Val Met Gly Met Ser Glu Cys Asn
            100                 105                 110
Ala Glu Ala Ser Gly Ala Ser Leu Tyr Asn Thr Leu Leu Tyr Ile Asp
        115                 120                 125
Ala Lys Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val

145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Thr Met Tyr Ala Trp Gly Thr Gln Ile
            180                 185                 190
Tyr Ile Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Glu Arg Tyr Pro Met Lys Gln Lys
225                 230                 235                 240
Phe Tyr Ala Glu Ala Asp Glu Trp Ile Asn Ile Gly Asp Ser Ala Ile
                245                 250                 255
Val Asn Pro Glu Gly Gln Phe Ile Ala Gly Pro Val Arg Lys Gln Glu
            260                 265                 270
Glu Ile Leu Tyr Ala Glu Ile Asn Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val
305

<210> 255
<211> 1005
<212> DNA
<213> Unknown

<220>
<223> Obtained from an environmental sample

<400> 255
atgacaatgt ctaagaccaa atttagggtt gcagctgtgc aggcggcgcc ggttttcctt      60
gatcgggaag cgacgttgga taaagcttgt ggattgattg aggaggcggg ccgcaacggc     120
gccagcctcg tcgtcttccc tgagtcattc attccggcct accccgattg ggtttgggct     180
gtgccggcgg gcgaagaagc tttactcaat gaactgtacg cacaactgtt ggccaacgcc     240
gttgaaattc ccggcccggc cactcaacgt ttgagccagg cggctaaaaa ggctaaggtt     300
cacctggcta tgggcctgac cgaacgcaac agcgaggcca gcggcggcag cctttacaac     360
accttgctct atcttgaccc gcagggccac attctgggca agcatcgtaa gctggtgccc     420
accggcggtg agcggctggt ttgggcccag ggcgacggca gcactttgca agtttacgat     480
acgcctctgg gtaaactcag cggcctgatt tgctgggaaa attatatgcc gctggcgcgc     540
tacgcgctgt atgcctgggg tacgcaaatt tatattgcgg ccacctggga tcggggtgag     600
ccgtggcttt cgacgttgcg gcatattgcc aaagagggcc gggtgttggt catcggttgc     660
ggtatggcct tgcgcaaggc tgatattcct gatcattttg aattcaagca gcgcttttat     720
caaaacgccg ccgagtggat caacgggggc gacagcgcca ttgtcaaccc tgatggtgaa     780
tttattgctg gccccttaag cgagcaggaa ggcattttgt acgccgagat tgatccggcc     840
cagatgggcg ggccaaagtg gatgctcgac gtggccgggc attacgctcg cccggatgtg     900
tttgaactga cggtccatac cgccgcccga cccatgatca cctcgaaaaa ggatggccta     960
acacccgccg aggccgttac gcaagtaacg aaagcattat tgtaa                    1005

<210> 256
<211> 334
<212> PRT
<213> Unknown

<220>
<223> Obtained from an environmental sample

<400> 256
Met Thr Met Ser Lys Thr Lys Phe Arg Val Ala Ala Val Gln Ala Ala
1               5                   10                  15
Pro Val Phe Leu Asp Arg Glu Ala Thr Leu Asp Lys Ala Cys Gly Leu
            20                  25                  30
Ile Glu Glu Ala Gly Arg Asn Gly Ala Ser Leu Val Val Phe Pro Glu
        35                  40                  45
Ser Phe Ile Pro Ala Tyr Pro Asp Trp Val Trp Ala Val Pro Ala Gly
    50                  55                  60
Glu Glu Ala Leu Leu Asn Glu Leu Tyr Ala Gln Leu Leu Ala Asn Ala
65                  70                  75                  80
Val Glu Ile Pro Gly Pro Ala Thr Gln Arg Leu Ser Gln Ala Ala Lys
                85                  90                  95
Lys Ala Lys Val His Leu Ala Met Gly Leu Thr Glu Arg Asn Ser Glu
            100                 105                 110
Ala Ser Gly Gly Ser Leu Tyr Asn Thr Leu Leu Tyr Leu Asp Pro Gln
        115                 120                 125
Gly His Ile Leu Gly Lys His Arg Lys Leu Val Pro Thr Gly Gly Glu
    130                 135                 140
Arg Leu Val Trp Ala Gln Gly Asp Gly Ser Thr Leu Gln Val Tyr Asp
145                 150                 155                 160
Thr Pro Leu Gly Lys Leu Ser Gly Leu Ile Cys Trp Glu Asn Tyr Met
                165                 170                 175
Pro Leu Ala Arg Tyr Ala Leu Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Lys Glu Gly Arg Val Leu Val Ile Gly Cys Gly Met Ala Leu
    210                 215                 220
Arg Lys Ala Asp Ile Pro Asp His Phe Glu Phe Lys Gln Arg Phe Tyr
225                 230                 235                 240
Gln Asn Ala Ala Glu Trp Ile Asn Gly Gly Asp Ser Ala Ile Val Asn
                245                 250                 255
Pro Asp Gly Glu Phe Ile Ala Gly Pro Leu Ser Glu Gln Glu Gly Ile
            260                 265                 270
Leu Tyr Ala Glu Ile Asp Pro Ala Gln Met Gly Gly Pro Lys Trp Met
        275                 280                 285
Leu Asp Val Ala Gly His Tyr Ala Arg Pro Asp Val Phe Glu Leu Thr
    290                 295                 300
Val His Thr Ala Ala Arg Pro Met Ile Thr Ser Lys Lys Asp Gly Leu
305                 310                 315                 320
Thr Pro Ala Glu Ala Val Thr Gln Val Thr Lys Ala Leu Leu
                325                 330

<210> 257
<211> 942
<212> DNA
<213> Unknown

<220>
<223> Obtained from an environmental sample

<400> 257
atgaacaaga tcgcgatcat tcagcgccca cccgtgctac tcgaccgcat cgccacgctg      60
gcggttgcgg tggagtcgat cgacgaagct gccgcagccg gtgcctcact gatcgttctt     120
ccagaaacct tcatccccgg ctacccgtcc tggatctggc gtctcgcgcc ggggagggat     180
ggtgcgctca ttgcccagtt gcatgcccga ctgctcgcca acgcggtcga tcttgcggct     240

ggagatctgg atgccctgtg tgaagttgcg cacggccacc gggtgaccgt ggtgtgcggc 300
ctcaacgaat gcgagcgcag tcgcggcggg ggcactctct acaacacggt cgtcgtgatc 360
gaccccgacg gcaagctgtg caatcgccac cgcaagctga tgccgaccaa cccggaacgc 420
atggtgcacg gtctgggtga tgcatcgggc ctgcgcgccg tcgacacccc ggtgggtcga 480
gtgggcgcac tcatctgctg ggaaaactat atgccgctgg cacgctacgc actttacgcc 540
gagggggtgg aagtctacgt ggcgcccacc tatgacagcg gcgatggctg gatcagtacg 600
atgcgtcata ttgcgcttga gggacgctgc tgggtgctgg gtagcggaac cgtactgcgt 660
ggcagcgacg tcccagaaga ctttccgtca cacctggacc tgtttcccga cgcggaggaa 720
tggatcaatc cgggcgactc ggtggtcgtc gatcctcagg gcaagatcgt cgcaggcccg 780
atgcgacgtg agacaggcat tctctacgca gaaatcgacg ccgaacgggt cgcgccttcg 840
cgccgcacgc tcgatgtcgc cggacactac gcccgcccgg atattttcga gctccatgtc 900
cgacgtacgc cggcgatgcc ggtccacgcc gttgatgcat ga 942

<210> 258
<211> 313
<212> PRT
<213> Unknown

<220>
<223> Obtained from an environmental sample

<400> 258
Met Asn Lys Ile Ala Ile Ile Gln Arg Pro Pro Val Leu Leu Asp Arg
1               5                   10                  15
Ile Ala Thr Leu Ala Val Ala Val Glu Ser Ile Asp Glu Ala Ala Ala
            20                  25                  30
Ala Gly Ala Ser Leu Ile Val Leu Pro Glu Thr Phe Ile Pro Gly Tyr
        35                  40                  45
Pro Ser Trp Ile Trp Arg Leu Ala Pro Gly Arg Asp Gly Ala Leu Ile
    50                  55                  60
Ala Gln Leu His Ala Arg Leu Leu Ala Asn Ala Val Asp Leu Ala Ala
65                  70                  75                  80
Gly Asp Leu Asp Ala Leu Cys Glu Val Ala His Gly His Arg Val Thr
                85                  90                  95
Val Val Cys Gly Leu Asn Glu Cys Glu Arg Ser Arg Gly Gly Gly Thr
            100                 105                 110
Leu Tyr Asn Thr Val Val Val Ile Asp Pro Asp Gly Lys Leu Cys Asn
        115                 120                 125
Arg His Arg Lys Leu Met Pro Thr Asn Pro Glu Arg Met Val His Gly
    130                 135                 140
Leu Gly Asp Ala Ser Gly Leu Arg Ala Val Asp Thr Pro Val Gly Arg
145                 150                 155                 160
Val Gly Ala Leu Ile Cys Trp Glu Asn Tyr Met Pro Leu Ala Arg Tyr
                165                 170                 175
Ala Leu Tyr Ala Glu Gly Val Glu Val Tyr Val Ala Pro Thr Tyr Asp
            180                 185                 190
Ser Gly Asp Gly Trp Ile Ser Thr Met Arg His Ile Ala Leu Glu Gly
        195                 200                 205
Arg Cys Trp Val Leu Gly Ser Gly Thr Val Leu Arg Gly Ser Asp Val
    210                 215                 220
Pro Glu Asp Phe Pro Ser His Leu Asp Leu Phe Pro Asp Ala Glu Glu
225                 230                 235                 240
Trp Ile Asn Pro Gly Asp Ser Val Val Val Asp Pro Gln Gly Lys Ile
                245                 250                 255
Val Ala Gly Pro Met Arg Arg Glu Thr Gly Ile Leu Tyr Ala Glu Ile
            260                 265                 270
Asp Ala Glu Arg Val Ala Pro Ser Arg Arg Thr Leu Asp Val Ala Gly

275                 280                 285
His Tyr Ala Arg Pro Asp Ile Phe Glu Leu His Val Arg Arg Thr Pro
    290                 295                 300
Ala Met Pro Val His Ala Val Asp Ala
305                 310

<210> 259
<211> 981
<212> DNA
<213> Unknown

<220>
<223> Obtained from an environmental sample

<400> 259
atggccatca tcaaagccgc cgccgtccag atcagtccgg tgctgtatag ccgcgaggga      60
accgtggaca aggtctgcca gcagatcatc gccctcggtc agcaaggcgt gcaatttgcg     120
gtctttccgg aaacggtggt gccgtactac ccctatttct ccttcgtcca gccgccgttc     180
gccatgggca aggaacacct gaaactgttg gaacaatcgg tgattgtgcc gtcggctgcc     240
accttggcga tcggcgaagc gtgcaaacaa gcgggaatgg tggtgtctat cggcgtcaat     300
gagcgcgatg gcggcacgat ctacaacgcc cagttgctgt ttgacgctga tggcagcttg     360
attcagcacc gtcgcaaaat aaccccgacg taccacgagc ggatgatctg gggtcaaggc     420
gacggctccg ggttgcgcgc catcgacagc gcggtcgggc gtattggctc gctggcctgc     480
tgggaacatt acaacccctt ggcccgctac gccctgatgg ccgacggcga gcagattcac     540
gcggctatgt ttcccggctc tctggtgggt gacatttttg ccgatcagat agaggtcact     600
attcgtcatc acgccttgga gtccggctgc ttcgtggtca actccaccgc gtggcttgat     660
gctgatcagc aaggccaaat catgcaggac accggttgca gcattggccc aatctcgggt     720
ggctgcttca cggccatcgt ttccccggaa ggcaaattac tcggcgaacc gctgcgttca     780
ggtgagggcg cagtcatcgc cgacctggac atggcattga tcgacaagcg caaacggatg     840
atggattccg tcggccatta cagccgccca gaactgctca gtttattgat cgaccgcacg     900
cccaccgctc atgtgcatga gcgcggcgcc catcaccttg ccgtagcctc tatcggggag     960
cttgaccatg caaaccaatg a                                               981

<210> 260
<211> 326
<212> PRT
<213> Unknown

<220>
<223> Obtained from an environmental sample

<400> 260
Met Ala Ile Ile Lys Ala Ala Ala Val Gln Ile Ser Pro Val Leu Tyr
1               5                   10                  15
Ser Arg Glu Gly Thr Val Asp Lys Val Cys Gln Gln Ile Ile Ala Leu
            20                  25                  30
Gly Gln Gln Gly Val Gln Phe Ala Val Phe Pro Glu Thr Val Val Pro
        35                  40                  45
Tyr Tyr Pro Tyr Phe Ser Phe Val Gln Pro Pro Phe Ala Met Gly Lys
    50                  55                  60
Glu His Leu Lys Leu Leu Glu Gln Ser Val Ile Val Pro Ser Ala Ala
65                  70                  75                  80
Thr Leu Ala Ile Gly Glu Ala Cys Lys Gln Ala Gly Met Val Val Ser
                85                  90                  95
Ile Gly Val Asn Glu Arg Asp Gly Gly Thr Ile Tyr Asn Ala Gln Leu
            100                 105                 110
Leu Phe Asp Ala Asp Gly Ser Leu Ile Gln His Arg Arg Lys Ile Thr

115                 120                 125
Pro Thr Tyr His Glu Arg Met Ile Trp Gly Gln Gly Asp Gly Ser Gly
    130                 135                 140
Leu Arg Ala Ile Asp Ser Ala Val Gly Arg Ile Gly Ser Leu Ala Cys
145                 150                 155                 160
Trp Glu His Tyr Asn Pro Leu Ala Arg Tyr Ala Leu Met Ala Asp Gly
                165                 170                 175
Glu Gln Ile His Ala Ala Met Phe Pro Gly Ser Leu Val Gly Asp Ile
            180                 185                 190
Phe Ala Asp Gln Ile Glu Val Thr Ile Arg His His Ala Leu Glu Ser
        195                 200                 205
Gly Cys Phe Val Val Asn Ser Thr Ala Trp Leu Asp Ala Asp Gln Gln
    210                 215                 220
Gly Gln Ile Met Gln Asp Thr Gly Cys Ser Ile Gly Pro Ile Ser Gly
225                 230                 235                 240
Gly Cys Phe Thr Ala Ile Val Ser Pro Glu Gly Lys Leu Leu Gly Glu
                245                 250                 255
Pro Leu Arg Ser Gly Glu Gly Ala Val Ile Ala Asp Leu Asp Met Ala
            260                 265                 270
Leu Ile Asp Lys Arg Lys Arg Met Met Asp Ser Val Gly His Tyr Ser
        275                 280                 285
Arg Pro Glu Leu Leu Ser Leu Leu Ile Asp Arg Thr Pro Thr Ala His
    290                 295                 300
Val His Glu Arg Gly Ala His His Leu Ala Val Ala Ser Ile Gly Glu
305                 310                 315                 320
Leu Asp His Ala Asn Gln
                325

<210> 261
<211> 1014
<212> DNA
<213> Unknown

<220>
<223> Obtained from an environmental sample

<400> 261
atgggtctgg ttcatcagaa atacaaggta gcggtggttc aggcggcacc ggtttttctc      60
gatctcgatg caaccgtgga caagaccatt gcgctgatcg aggaagcttc cgcgcaaggc     120
gcaaaactgg tcgcgtttcc cgagaccttc attcccggat atccgtggca gatctggctc     180
ggcgcgccgg cctgggcgat cggccgcggc tttgtgcagc gctacttcga caattcgctg     240
ggcttcgaca gcccgcaggc ggaaaaaatc cgccaggccg tgaagcgcgc caagctgacc     300
gcggtgcttg ggctatccga acgcgacggc ggcagcctct atatcgcgca gtggctgatc     360
ggccctgacg gcgagaccat cgccaagcgc cggaaactgc gtccgaccca tgccgaacgc     420
accgtgttcg gcgaaggcga cggcagcgat cttgcggtcc acgatcgcgc cgacgtcggg     480
cgcttgggcg cactgtgctg ctgggagcac ctgcagccgc tatcgaaata cgcgatgtac     540
gcccagaacg aacaggtgca tgtcggcgcc tggccgagct tttcgctgta cgatccgttc     600
gcacatgcgc tcggccatga ggtcaacaac gccgccagca aggtctatgc ggtcgaaggc     660
tcctgcttct tcctgggtcc ctgcgctgtc gtctcgcagg cgatgatcga cgagctctgc     720
gactctcccg agaaacatgc cttcctgcat gtcggcggcg gccacgcggt gatctacggc     780
ccggacggca gttcgctggc cgagaaactc ccgcccgacc aggaaggcat tctgtacgcc     840
gatatcgatc tcggcatgat cggggtcgcg aagaacgccg ccgatccggc cgggcattat     900
tcgcggcccg acgtcacgcg gctgttgctc aacaccacgc gcgccaaccg cgtcgagcat     960
ttctcgcttc ccgtcgatgc cgaggtcatg agcgaaatca ggctgcaggc ctga          1014

<210> 262
<211> 337

<212> PRT
<213> Unknown

<220>
<223> Obtained from an environmental sample

<400> 262
Met Gly Leu Val His Gln Lys Tyr Lys Val Ala Val Val Gln Ala Ala
1               5                   10                  15
Pro Val Phe Leu Asp Leu Asp Ala Thr Val Asp Lys Thr Ile Ala Leu
            20                  25                  30
Ile Glu Glu Ala Ser Ala Gln Gly Ala Lys Leu Val Ala Phe Pro Glu
        35                  40                  45
Thr Phe Ile Pro Gly Tyr Pro Trp Gln Ile Trp Leu Gly Ala Pro Ala
    50                  55                  60
Trp Ala Ile Gly Arg Gly Phe Val Gln Arg Tyr Phe Asp Asn Ser Leu
65                  70                  75                  80
Gly Phe Asp Ser Pro Gln Ala Glu Lys Ile Arg Gln Ala Val Lys Arg
                85                  90                  95
Ala Lys Leu Thr Ala Val Leu Gly Leu Ser Glu Arg Asp Gly Gly Ser
            100                 105                 110
Leu Tyr Ile Ala Gln Trp Leu Ile Gly Pro Asp Gly Glu Thr Ile Ala
        115                 120                 125
Lys Arg Arg Lys Leu Arg Pro Thr His Ala Glu Arg Thr Val Phe Gly
    130                 135                 140
Glu Gly Asp Gly Ser Asp Leu Ala Val His Asp Arg Ala Asp Val Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Gly Ala Trp Pro
            180                 185                 190
Ser Phe Ser Leu Tyr Asp Pro Phe Ala His Ala Leu Gly His Glu Val
        195                 200                 205
Asn Asn Ala Ala Ser Lys Val Tyr Ala Val Glu Gly Ser Cys Phe Phe
    210                 215                 220
Leu Gly Pro Cys Ala Val Val Ser Gln Ala Met Ile Asp Glu Leu Cys
225                 230                 235                 240
Asp Ser Pro Glu Lys His Ala Phe Leu His Val Gly Gly Gly His Ala
                245                 250                 255
Val Ile Tyr Gly Pro Asp Gly Ser Ser Leu Ala Glu Lys Leu Pro Pro
            260                 265                 270
Asp Gln Glu Gly Ile Leu Tyr Ala Asp Ile Asp Leu Gly Met Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Thr Thr Arg Ala Asn Arg Val Glu His
305                 310                 315                 320
Phe Ser Leu Pro Val Asp Ala Glu Val Met Ser Glu Ile Arg Leu Gln
                325                 330                 335
Ala

<210> 263
<211> 1014
<212> DNA
<213> Unknown

<220>
<223> Obtained from an environmental sample

<400> 263
atgaaagtcg tcaaagcggc agcggttcag ttgagccctg tcctctacag ccgcgaggca 60
accgtcgcga aggtcgtgcg gaagatccac gagcttgggc agcagggcgt gcagttcgcc 120
accttcccgg aaaccgttgt gccgtactac ccgtatttct ccgcggtcca gacgccgatg 180
cagcttctgg ctggaaccga gcatctgaaa ttgctcgacc aggccgtgac ggtgccgtct 240
cccgcgaccg acgcgatcgg cgaggcagcc cggaaggcgg gcatggtggt gtccatcggc 300
gtcaacgagc gtgatggtgg aaccctgtac aacacccaat tgctcttcga cgccgatggc 360
accctgatcc agcgccgccg caagatcacg cccacccatt tcgagcgcat gatctggggc 420
cagggtgacg ggtcgggcct gcgtgccgtc gacagcaagg tcggccgcat tggccagctg 480
gcatgcttcg agcacaacaa tcctctggcg cgctacgcga tgatggccga cggcgagcag 540
atccattcgg ccatgtatcc gggttctgcc ttcggcgagg gctttgccca gaggatggaa 600
atcaatatcc gccagcacgc actggagtcc gggtgcttcg tcgtgaacgc gacggcctgg 660
ctggatgccg accagcaggc gcaaatcatg aaagacacgg gctgcgggat cggtccgatc 720
tcgggcggtt gcttcaccac gatcgtggca cccgacggca cgctgctggg ggaacctctg 780
cgctcgggcg agggcgaggt catcgccgat ctcgatttca cggagatcga ccggcgcaag 840
atgctgatgg actcggcagg ccactacaac cgtccggaac tgctcagtct gctgatcgac 900
cgcacgccga ccgcaaacgt gcacgaacgg atggcgcatc cccaagcgag cacgaagcag 960
ccgcgctccg gcgatctgcc cgctgcgctg gctggcgcgc aggagatcct gtga 1014

<210> 264
<211> 337
<212> PRT
<213> Unknown

<220>
<223> Obtained from an environmental sample

<400> 264
Met Lys Val Val Lys Ala Ala Ala Val Gln Leu Ser Pro Val Leu Tyr
1               5                   10                  15
Ser Arg Glu Ala Thr Val Ala Lys Val Val Arg Lys Ile His Glu Leu
            20                  25                  30
Gly Gln Gln Gly Val Gln Phe Ala Thr Phe Pro Glu Thr Val Val Pro
        35                  40                  45
Tyr Tyr Pro Tyr Phe Ser Ala Val Gln Thr Pro Met Gln Leu Leu Ala
    50                  55                  60
Gly Thr Glu His Leu Lys Leu Leu Asp Gln Ala Val Thr Val Pro Ser
65                  70                  75                  80
Pro Ala Thr Asp Ala Ile Gly Glu Ala Ala Arg Lys Ala Gly Met Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Lys Val Gly Arg Ile Gly Gln Leu
145                 150                 155                 160
Ala Cys Phe Glu His Asn Asn Pro Leu Ala Arg Tyr Ala Met Met Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205

Glu Ser Gly Cys Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Ser Gly Gly Cys Phe Thr Thr Ile Val Ala Pro Asp Gly Thr Leu Leu
                245                 250                 255
Gly Glu Pro Leu Arg Ser Gly Glu Gly Glu Val Ile Ala Asp Leu Asp
            260                 265                 270
Phe Thr Glu Ile Asp Arg Arg Lys Met Leu Met Asp Ser Ala Gly His
        275                 280                 285
Tyr Asn Arg Pro Glu Leu Leu Ser Leu Leu Ile Asp Arg Thr Pro Thr
    290                 295                 300
Ala Asn Val His Glu Arg Met Ala His Pro Gln Ala Ser Thr Lys Gln
305                 310                 315                 320
Pro Arg Ser Gly Asp Leu Pro Ala Ala Leu Ala Gly Ala Gln Glu Ile
                325                 330                 335
Leu

<210> 265
<211> 999
<212> DNA
<213> Unknown

<220>
<223> Obtained from an environmental sample

<400> 265
atgcttaatc taggcatagt ccagatgaac gcagagccgc tcaacgtgga aggcaacctg      60
ctcaaggcgg agcgctatgt cgcgaagtgc gccgcggacg gcgcccaact cgtggtgctg     120
ccggagatgt tcaacgtcgg cttccacctc ggcgagtccc tgatgatggt cgccgagccc     180
ctggacggca agaccgtgca gtggctgcaa cggcaggcgt ccacccataa catatatatc     240
accgggagct tatacgagcg ttacgacgag catttctaca acaccatggt catggtggga     300
tacgacggca gcgtgcagta ctaccgcaag cgcaatccta cctggtccga gtcggcggtg     360
tggcgccgca gcgaggtgcc aggccccggt atattcgata ccccgttcgg gcgcatcggg     420
ggcgtcatct gcttcgattc cttcgcgcgc gagacccacg agggcttcaa gcagagcggg     480
gtcgaggcgg tggtaatcat cgccctgtgg ggcgccaacc gtgcgcgggc attcttctgg     540
cgcccggacc tcctgctaag ccgggaaggg ctggtccgtt ggtcccggct ggcctcggag     600
gacgtccccc gaaatcacgc gaaagagctc ggggtcccgg tcgccttcgt caaccagagt     660
ggcaccatcc gcatgaccag ccccatccct ttccccgact ggccggtgca gagctccttc     720
tacgacttca tcggcaagtc ccacgtccgg gacgcatccg gagaggtgat cgcgagggtg      780
gacgaggggg agatcgactc ctgcctggtg gtcccggtag aggtcgagca ggcgcagagc     840
aggccggaga tcaggaagtc aaatatatcg cccggctacc tcggcaagga ttactatttc     900
gtggagccgc cgcttatctg caagctcttc caggtctggt tcctcagcgg cctggtgccc     960
accgaatacg aggcgcggcg tctgcgccac ctgttctga                            999

<210> 266
<211> 332
<212> PRT
<213> Unknown

<220>
<223> Obtained from an environmental sample

<400> 266
Met Leu Asn Leu Gly Ile Val Gln Met Asn Ala Glu Pro Leu Asn Val
1               5                   10                  15

Glu Gly Asn Leu Leu Lys Ala Glu Arg Tyr Val Ala Lys Cys Ala Ala
            20                  25                  30
Asp Gly Ala Gln Leu Val Val Leu Pro Glu Met Phe Asn Val Gly Phe
        35                  40                  45
His Leu Gly Glu Ser Leu Met Met Val Ala Glu Pro Leu Asp Gly Lys
    50                  55                  60
Thr Val Gln Trp Leu Gln Arg Gln Ala Ser Thr His Asn Ile Tyr Ile
65                  70                  75                  80
Thr Gly Ser Leu Tyr Glu Arg Tyr Asp Glu His Phe Tyr Asn Thr Met
                85                  90                  95
Val Met Val Gly Tyr Asp Gly Ser Val Gln Tyr Tyr Arg Lys Arg Asn
            100                 105                 110
Pro Thr Trp Ser Glu Ser Ala Val Trp Arg Arg Ser Glu Val Pro Gly
        115                 120                 125
Pro Gly Ile Phe Asp Thr Pro Phe Gly Arg Ile Gly Gly Val Ile Cys
    130                 135                 140
Phe Asp Ser Phe Ala Arg Glu Thr His Glu Gly Phe Lys Gln Ser Gly
145                 150                 155                 160
Val Glu Ala Val Val Ile Ile Ala Leu Trp Gly Ala Asn Arg Ala Arg
                165                 170                 175
Ala Phe Phe Trp Arg Pro Asp Leu Leu Leu Ser Arg Glu Gly Leu Val
            180                 185                 190
Arg Trp Ser Arg Leu Ala Ser Glu Asp Val Pro Arg Asn His Ala Lys
        195                 200                 205
Glu Leu Gly Val Pro Val Ala Phe Val Asn Gln Ser Gly Thr Ile Arg
    210                 215                 220
Met Thr Ser Pro Ile Pro Phe Pro Asp Trp Pro Val Gln Ser Ser Phe
225                 230                 235                 240
Tyr Asp Phe Ile Gly Lys Ser His Val Arg Asp Ala Ser Gly Glu Val
                245                 250                 255
Ile Ala Arg Val Asp Glu Gly Glu Ile Asp Ser Cys Leu Val Val Pro
            260                 265                 270
Val Glu Val Glu Gln Ala Gln Ser Arg Pro Glu Ile Arg Lys Ser Asn
        275                 280                 285
Ile Ser Pro Gly Tyr Leu Gly Lys Asp Tyr Tyr Phe Val Glu Pro Pro
    290                 295                 300
Leu Ile Cys Lys Leu Phe Gln Val Trp Phe Leu Ser Gly Leu Val Pro
305                 310                 315                 320
Thr Glu Tyr Glu Ala Arg Arg Leu Arg His Leu Phe
                325                 330

<210> 267
<211> 1038
<212> DNA
<213> Unknown

<220>
<223> Obtained from an environmental sample

<400> 267
atgggcatta cgcatccgaa gttcaaggcc gcggcggtac aggcggcgcc gggctttctc 60
gacagcgagg ccaccgtcga caagacgatc cgcctgatgc aggaagcggc ggaccacggc 120
gcctcgctga tcgtctttcc ggaagcctgg ctgcccggtt atccgtggtg gatctggctc 180
ggtccgcccg cctggggcat gcagttcgtg cagcgctact tcgacaattc gccgagcgtc 240
ggcgatgatc ttttccgccg gatcgagcgc gcggccgcca aggcgaagat cgaagtggtc 300
ctcggtctca gcgagcgcgc tgccggctcg ctgtacctcg cgcaggcgtt catctcctca 360
acgggcgaga cgcgcgcagt gcgccgcaag ttgcgaccaa cgcacgtcga gcgaaccgtt 420

ttcggcgagg gcgatggcag cgacttcaag gtgttcgaca ctccgctggg ccgcgtcggt 480
ggtctcttgt gctgggaaca cctgcaaccg ctgtcgcgct acgcgatgtt ctcgatgaac 540
gagcaggtgc acgccgccgc ctggccgacg ttcagcctct acacggattt tgcccatgcc 600
ctcggccacg aactgaatct cgcagccagc gctacttacg cggctgaagg gcagtgctac 660
gtgattgccg cctgtggcgt ggtcacgcag gagatgctgg atctgatgaa ggcgccgtgc 720
cccccggaat atctgcgggt cggcggcgga tacgccatga tctttgcgcc cgacggacgg 780
cgcattgcgg cggcgctgcc gccggaacaa gaagggctga tttacgccga catcgatctt 840
tcgatgatct ctctcgccaa ggcggctgcc gatcccaccg gtcactactc gcggccggat 900
gtcgtgcggc ttatgctgaa taccgaaccg atgcagcggg tcgaaaagct gcagccgccg 960
ctggactcag ccgcgcgccg tgagaatgaa ccggcacgcg agaccgcagc ggcgaccgag 1020
agccgccagc cccagtaa 1038

<210> 268
<211> 344
<212> PRT
<213> Unknown

<220>
<223> Obtained from an environmental sample

<400> 268
Met Gly Ile Thr His Pro Lys Phe Lys Ala Ala Ala Val Gln Ala Ala
1               5                   10                  15
Pro Gly Phe Leu Asp Ser Glu Ala Thr Val Asp Lys Thr Ile Arg Leu
            20                  25                  30
Met Gln Glu Ala Ala Asp His Gly Ala Ser Leu Ile Val Phe Pro Glu
        35                  40                  45
Ala Trp Leu Pro Gly Tyr Pro Trp Trp Ile Trp Leu Gly Pro Pro Ala
    50                  55                  60
Trp Gly Met Gln Phe Val Gln Arg Tyr Phe Asp Asn Ser Pro Ser Val
65                  70                  75                  80
Gly Asp Asp Leu Phe Arg Arg Ile Glu Arg Ala Ala Ala Lys Ala Lys
                85                  90                  95
Ile Glu Val Val Leu Gly Leu Ser Glu Arg Ala Ala Gly Ser Leu Tyr
            100                 105                 110
Leu Ala Gln Ala Phe Ile Ser Ser Thr Gly Glu Thr Arg Ala Val Arg
        115                 120                 125
Arg Lys Leu Arg Pro Thr His Val Glu Arg Thr Val Phe Gly Glu Gly
    130                 135                 140
Asp Gly Ser Asp Phe Lys Val Phe Asp Thr Pro Leu Gly Arg Val Gly
145                 150                 155                 160
Gly Leu Leu Cys Trp Glu His Leu Gln Pro Leu Ser Arg Tyr Ala Met
                165                 170                 175
Phe Ser Met Asn Glu Gln Val His Ala Ala Ala Trp Pro Thr Phe Ser
            180                 185                 190
Leu Tyr Thr Asp Phe Ala His Ala Leu Gly His Glu Leu Asn Leu Ala
        195                 200                 205
Ala Ser Ala Thr Tyr Ala Ala Glu Gly Gln Cys Tyr Val Ile Ala Ala
    210                 215                 220
Cys Gly Val Val Thr Gln Glu Met Leu Asp Leu Met Lys Ala Pro Cys
225                 230                 235                 240
Pro Pro Glu Tyr Leu Arg Val Gly Gly Gly Tyr Ala Met Ile Phe Ala
                245                 250                 255
Pro Asp Gly Arg Arg Ile Ala Ala Ala Leu Pro Pro Glu Gln Glu Gly
            260                 265                 270
Leu Ile Tyr Ala Asp Ile Asp Leu Ser Met Ile Ser Leu Ala Lys Ala
        275                 280                 285

Ala Ala Asp Pro Thr Gly His Tyr Ser Arg Pro Asp Val Val Arg Leu
    290                 295                 300
Met Leu Asn Thr Glu Pro Met Gln Arg Val Glu Lys Leu Gln Pro Pro
305                 310                 315                 320
Leu Asp Ser Ala Ala Arg Arg Glu Asn Glu Pro Ala Arg Glu Thr Ala
                325                 330                 335
Ala Ala Thr Glu Ser Arg Gln Pro
            340

<210> 269
<211> 1014
<212> DNA
<213> Unknown

<220>
<223> Obtained from an environmental sample

<400> 269
atgtctgaaa cagccttcaa gatcgcggtc gtacaggcgg ctccggtttt tctcgacgca      60
aaggcgacgg tggacaaggc gatcggtctg atggccgaag ccggcgccaa gggcgccaag     120
ctgctcgcat tcccggaagt attcatcccc ggctaccctt ggtggctgtg gctgggcaca     180
ccggcatggg gcatgcagtt tgttgccaag tatcacgcga actcgcttcg tgcagacggg     240
cctgaattgg cagccctcgc ggcggcggcg gcgaagtccg atatcaatgc cgtcatcggc     300
ttctcggaga tcgacggcgg ttccctctac atcagccagg cgctcatcag cgacaagggc     360
gagataatgt tcaaacggcg caagctgaag ccgacgcacg tcgaacgcac gttgttcggc     420
gaaggggacg ggtccgactt ccaggtcgtg gacacgagcg tcggcaggct cggtgccttg     480
tgttgcgccg aacacataca gccgctgtcg aagtacgcga tgtactccat gcacgaacag     540
gtgcacgtcg cctcctggcc gtcatttact ttgtaccgcg gcacggcata tgccttgggc     600
cacgaggtca atctggccgc gagccagatt tatgcgctcg agggaggctg tttcgtcctt     660
catgcgagcg ccatcaccgg ccaggacatg tttgacgtgc tgtgcgacac tccggagagg     720
acgcaactgc tgaactccga cggcggcaag gtcggcggcg gctactcgat gatcttcggt     780
cccgatggcc agccccttgt tgggcatctg cctcaagaca ccgagggaat actctacgca     840
gatattgacc tggcgaacat ttccgttgcc aaagcggcct acgacccgtc cggacactat     900
gcgcgcggag acgtggtgcg cttgatggtc aatcgcaacc cgcggcatac gagtgttgcg     960
ttcggcgggg gcgccggcga ggcagcaacc tggacggaag caaaagcgga gtga          1014

<210> 270
<211> 337
<212> PRT
<213> Unknown

<220>
<223> Obtained from an environmental sample

<400> 270
Met Ser Glu Thr Ala Phe Lys Ile Ala Val Val Gln Ala Ala Pro Val
1               5                   10                  15
Phe Leu Asp Ala Lys Ala Thr Val Asp Lys Ala Ile Gly Leu Met Ala
            20                  25                  30
Glu Ala Gly Ala Lys Gly Ala Lys Leu Leu Ala Phe Pro Glu Val Phe
        35                  40                  45
Ile Pro Gly Tyr Pro Trp Trp Leu Trp Leu Gly Thr Pro Ala Trp Gly
    50                  55                  60
Met Gln Phe Val Ala Lys Tyr His Ala Asn Ser Leu Arg Ala Asp Gly
65                  70                  75                  80
Pro Glu Leu Ala Ala Leu Ala Ala Ala Ala Ala Lys Ser Asp Ile Asn
                85                  90                  95

Ala Val Ile Gly Phe Ser Glu Ile Asp Gly Gly Ser Leu Tyr Ile Ser
            100                 105                 110
Gln Ala Leu Ile Ser Asp Lys Gly Glu Ile Met Phe Lys Arg Arg Lys
        115                 120                 125
Leu Lys Pro Thr His Val Glu Arg Thr Leu Phe Gly Glu Gly Asp Gly
    130                 135                 140
Ser Asp Phe Gln Val Val Asp Thr Ser Val Gly Arg Leu Gly Ala Leu
145                 150                 155                 160
Cys Cys Ala Glu His Ile Gln Pro Leu Ser Lys Tyr Ala Met Tyr Ser
                165                 170                 175
Met His Glu Gln Val His Val Ala Ser Trp Pro Ser Phe Thr Leu Tyr
            180                 185                 190
Arg Gly Thr Ala Tyr Ala Leu Gly His Glu Val Asn Leu Ala Ala Ser
        195                 200                 205
Gln Ile Tyr Ala Leu Glu Gly Gly Cys Phe Val Leu His Ala Ser Ala
    210                 215                 220
Ile Thr Gly Gln Asp Met Phe Asp Val Leu Cys Asp Thr Pro Glu Arg
225                 230                 235                 240
Thr Gln Leu Leu Asn Ser Asp Gly Gly Lys Val Gly Gly Gly Tyr Ser
                245                 250                 255
Met Ile Phe Gly Pro Asp Gly Gln Pro Leu Val Gly His Leu Pro Gln
            260                 265                 270
Asp Thr Glu Gly Ile Leu Tyr Ala Asp Ile Asp Leu Ala Asn Ile Ser
        275                 280                 285
Val Ala Lys Ala Ala Tyr Asp Pro Ser Gly His Tyr Ala Arg Gly Asp
    290                 295                 300
Val Val Arg Leu Met Val Asn Arg Asn Pro Arg His Thr Ser Val Ala
305                 310                 315                 320
Phe Gly Gly Gly Ala Gly Glu Ala Ala Thr Trp Thr Glu Ala Lys Ala
                325                 330                 335
Glu

<210> 271
<211> 966
<212> DNA
<213> Unknown

<220>
<223> Obtained from an environmental sample

<400> 271
atgtccagcg agaataacaa cgctacattc aaagttgccg cagttcaggc cacacctgtg      60
tatcttgatc gtgaagcaac catcgacaag gcttgcgagt tgatcgctac tgctggcagc     120
gaaggagctc gcctgattat ctttccagaa gcgttcatcc caacctatcc tgagtgggta     180
tggggtattc cttctggtga gcaaggttta ctcaacgagc tctattcaga gttgctcacc     240
aattcggtca cgattcccag cgacgcgact gacagactgt gcgaggccgc gaagcttgct     300
aatgcctacg tggtgatggg aatgagtgaa cggaatgtcg aagcgagtgg tgcaagcctg     360
tataatacgc tcttgtacat agatgcgcag ggggagattt tagggaaaca tcgaaagttg      420
gtaccaacgg gcggtgagcg cctggtatgg gcgcaaggtg atggcagcac gctgcaggtc     480
tacgatactc cattgggaaa actcggtggt ttaatttgct gggaaaatta tatgccactg     540
gcacgctacg ctatgtatgc ctggggtaca caaatctatg tcgcagcaac gtgggatcgc     600
ggccaaccct ggctctcaac gttacggcat attgccaaag aaggcagggt atacgtaatt     660
ggttgctgta ttgcgatgcg taaagatgat attccagatc gttactccat gaagcagaag     720
tattacgctg aaatggagga atggattaat attggtgaca gcgcgattgt caatcccgaa     780
ggacacttta ttgcagggcc tgtgcgcaag caagaagaaa ttctttacgc ggagatcgat     840
ccacgcatgg tgcaaggccc gaagtggatg ctcgatgtgg ctgggcacta tgcgagacca     900

gatgtgttcc agttgacggt gcatacggat gtgaggcaga tgattcgggt ggaacatgat     960
tcataa                                                                966

<210> 272
<211> 321
<212> PRT
<213> Unknown

<220>
<223> Obtained from an environmental sample

<400> 272
Met Ser Ser Glu Asn Asn Asn Ala Thr Phe Lys Val Ala Ala Val Gln
1               5                   10                  15
Ala Thr Pro Val Tyr Leu Asp Arg Glu Ala Thr Ile Asp Lys Ala Cys
            20                  25                  30
Glu Leu Ile Ala Thr Ala Gly Ser Glu Gly Ala Arg Leu Ile Ile Phe
        35                  40                  45
Pro Glu Ala Phe Ile Pro Thr Tyr Pro Glu Trp Val Trp Gly Ile Pro
    50                  55                  60
Ser Gly Glu Gln Gly Leu Leu Asn Glu Leu Tyr Ser Glu Leu Leu Thr
65                  70                  75                  80
Asn Ser Val Thr Ile Pro Ser Asp Ala Thr Asp Arg Leu Cys Glu Ala
                85                  90                  95
Ala Lys Leu Ala Asn Ala Tyr Val Val Met Gly Met Ser Glu Arg Asn
            100                 105                 110
Val Glu Ala Ser Gly Ala Ser Leu Tyr Asn Thr Leu Leu Tyr Ile Asp
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Ala Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Asp Arg Tyr Ser Met Lys Gln Lys
225                 230                 235                 240
Tyr Tyr Ala Glu Met Glu Glu Trp Ile Asn Ile Gly Asp Ser Ala Ile
                245                 250                 255
Val Asn Pro Glu Gly His Phe Ile Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asp Val Arg Gln Met Ile Arg Val Glu His Asp
305                 310                 315                 320
Ser

<210> 273
<211> 1023

<212> DNA
<213> Unknown

<220>
<223> Obtained from an environmental sample

<400> 273
atgaataact caccaccaac catccgcgct gctgccattc agctcagtcc agttctgttt 60
agtcgggatg ggactaccga gaaggtgttg caggcgatcg ctagtgctgc caaggaaggg 120
gcacaactgg ttgtttttcc agaaactttc atcccctact acccctattt ctcattcatt 180
caacccccctg tgttgatggg caaagagcac atgcggctct atgaggaagc cgtgacggtc 240
ccgggtccgg tgacagatgc ggtcagtcga gcagcccgtt cttacggcat ggtggtagtg 300
ctgggggtga atgagcgaga tggtggctca atttacaata cacagttgat tttcgatgct 360
gacggcacat tgttgctgaa gcgacgcaaa atcaccccta cctatcatga gcgcatggtc 420
tgggggcagg gagacggtgc tggattgaag gtattggata cagcagtcgg taaggtgggt 480
gcgctggcat gttgggaaca ttacaatccc ctggcacgat ttgcgctgat ggcacagcat 540
gagcagattc actgcgctca gttccccggt tctctggtgg gacaaatttt cactgatcag 600
attgaggtaa cgattcggca tcatgcgttg gaatcgggtt gttttgtggt gaatgctact 660
ggctggctct ctccagaaca ggtggcacaa atcaccacgg atgaaaagtt gcaacgggtg 720
ctgagtggcg ggtgtaatac cgccattatt ggacctgaag gcaatcatct ctgtcctccc 780
attaccgatg gtgagggcat agcgatcgcc gatctcgact tctcactaat caccaaacgc 840
aaacgcatga tggattgcgt cggtcactac tcccgccctg acttgttgaa gctgcaactc 900
aatgcaacgg catggtcggt gctggctggg gagcaggggg caggtgccag ggagcagggg 960
ctaggtgtgc cggatgccat gctgtctacg cctaagccag aatactcaac actggatcag 1020
tag 1023

<210> 274
<211> 340
<212> PRT
<213> Unknown

<220>
<223> Obtained from an environmental sample

<400> 274
Met Asn Asn Ser Pro Pro Thr Ile Arg Ala Ala Ala Ile Gln Leu Ser
1               5                   10                  15
Pro Val Leu Phe Ser Arg Asp Gly Thr Thr Glu Lys Val Leu Gln Ala
            20                  25                  30
Ile Ala Ser Ala Ala Lys Glu Gly Ala Gln Leu Val Val Phe Pro Glu
        35                  40                  45
Thr Phe Ile Pro Tyr Tyr Pro Tyr Phe Ser Phe Ile Gln Pro Pro Val
    50                  55                  60
Leu Met Gly Lys Glu His Met Arg Leu Tyr Glu Glu Ala Val Thr Val
65                  70                  75                  80
Pro Gly Pro Val Thr Asp Ala Val Ser Arg Ala Ala Arg Ser Tyr Gly
                85                  90                  95
Met Val Val Val Leu Gly Val Asn Glu Arg Asp Gly Gly Ser Ile Tyr
            100                 105                 110
Asn Thr Gln Leu Ile Phe Asp Ala Asp Gly Thr Leu Leu Leu Lys Arg
        115                 120                 125
Arg Lys Ile Thr Pro Thr Tyr His Glu Arg Met Val Trp Gly Gln Gly
    130                 135                 140
Asp Gly Ala Gly Leu Lys Val Leu Asp Thr Ala Val Gly Lys Val Gly
145                 150                 155                 160
Ala Leu Ala Cys Trp Glu His Tyr Asn Pro Leu Ala Arg Phe Ala Leu
                165                 170                 175

Met Ala Gln His Glu Gln Ile His Cys Ala Gln Phe Pro Gly Ser Leu
            180                 185                 190
Val Gly Gln Ile Phe Thr Asp Gln Ile Glu Val Thr Ile Arg His His
        195                 200                 205
Ala Leu Glu Ser Gly Cys Phe Val Val Asn Ala Thr Gly Trp Leu Ser
    210                 215                 220
Pro Glu Gln Val Ala Gln Ile Thr Thr Asp Glu Lys Leu Gln Arg Val
225                 230                 235                 240
Leu Ser Gly Gly Cys Asn Thr Ala Ile Ile Gly Pro Glu Gly Asn His
                245                 250                 255
Leu Cys Pro Pro Ile Thr Asp Gly Glu Gly Ile Ala Ile Ala Asp Leu
            260                 265                 270
Asp Phe Ser Leu Ile Thr Lys Arg Lys Arg Met Met Asp Cys Val Gly
        275                 280                 285
His Tyr Ser Arg Pro Asp Leu Leu Lys Leu Gln Leu Asn Ala Thr Ala
    290                 295                 300
Trp Ser Val Leu Ala Gly Glu Gln Gly Ala Gly Ala Arg Glu Gln Gly
305                 310                 315                 320
Leu Gly Val Pro Asp Ala Met Leu Ser Thr Pro Lys Pro Glu Tyr Ser
                325                 330                 335
Thr Leu Asp Gln
            340

<210> 275
<211> 849
<212> DNA
<213> Unknown

<220>
<223> Obtained from an environmental sample

<400> 275
atggagacgg ctcacaaagc aaaggtcgat ttccttgtgc tgggtgagac gtggctctca      60
ggctacccgg cttggctgga ccactgcccc gatgttggcc ggtgggatta tgaaccgatg     120
aaaaaagtgt atttgagatt tcgacaaagt gctatttctg ttcctggcaa agaatttgat     180
ttccttactg gcctctgtaa aaaatattca caaacgcttg ccatcggtgt taatgagaaa     240
gtagatcatg gggtaggtaa tggtaccatt tataattcat ttctactgat tgattctgat     300
ggaacactgt tgaatcatca tcgcaagtta gttcccactt ttactgagaa attattatac     360
ggccatggag atggccatgg gctgaagtcg atggatactt cggtgggaag aatcggaggg     420
agcatttgtt gggaacattg gatgccacta tgcagacaag cacttcatga tgcaggtgag     480
caaatccatg ttgccctttg gccgactgtt catgacatcc atcaagtggc aagtagaagc     540
tatgcatttg aagggcgctg ctttgtattg gctgccgggc agatttttgc tgctaaagat     600
tttccaaagg aacttgtctt accagactat ctaaagcaaa atccggatca gctcattttg      660
aatgggggga gctgcgtgat cggccctgat gggaaatatt tgattgagcc cgtgtttgat     720
cgggaagaac tgattgtgtg tgaacttgac cttgacgaag cttataaaga aagaatgacg     780
atggacgttt caggtcacta ccaaagacga gacgttttca gttttgacgt gaaccaacat     840
cgacattga                                                             849

<210> 276
<211> 310
<212> PRT
<213> Unknown

<220>
<223> Obtained from an environmental sample

<400> 276

Met Thr Lys Leu Lys Ile Ala Ile Gly Gln Phe Ser Ser Asn His Leu
1               5                   10                  15
Asp Leu Lys Cys Ser Leu Glu Lys Leu Glu Lys Ile Met Glu Thr Ala
            20                  25                  30
His Lys Ala Lys Val Asp Phe Leu Val Leu Gly Glu Thr Trp Leu Ser
        35                  40                  45
Gly Tyr Pro Ala Trp Leu Asp His Cys Pro Asp Val Gly Arg Trp Asp
    50                  55                  60
Tyr Glu Pro Met Lys Lys Val Tyr Leu Arg Phe Arg Gln Ser Ala Ile
65                  70                  75                  80
Ser Val Pro Gly Lys Glu Phe Asp Phe Leu Thr Gly Leu Cys Lys Lys
                85                  90                  95
Tyr Ser Gln Thr Leu Ala Ile Gly Val Asn Glu Lys Val Asp His Gly
            100                 105                 110
Val Gly Asn Gly Thr Ile Tyr Asn Ser Phe Leu Leu Ile Asp Ser Asp
        115                 120                 125
Gly Thr Leu Leu Asn His His Arg Lys Leu Val Pro Thr Phe Thr Glu
    130                 135                 140
Lys Leu Leu Tyr Gly His Gly Asp Gly His Gly Leu Lys Ser Met Asp
145                 150                 155                 160
Thr Ser Val Gly Arg Ile Gly Gly Ser Ile Cys Trp Glu His Trp Met
                165                 170                 175
Pro Leu Cys Arg Gln Ala Leu His Asp Ala Gly Glu Gln Ile His Val
            180                 185                 190
Ala Leu Trp Pro Thr Val His Asp Ile His Gln Val Ala Ser Arg Ser
        195                 200                 205
Tyr Ala Phe Glu Gly Arg Cys Phe Val Leu Ala Ala Gly Gln Ile Phe
    210                 215                 220
Ala Ala Lys Asp Phe Pro Lys Glu Leu Val Leu Pro Asp Tyr Leu Lys
225                 230                 235                 240
Gln Asn Pro Asp Gln Leu Ile Leu Asn Gly Gly Ser Cys Val Ile Gly
                245                 250                 255
Pro Asp Gly Lys Tyr Leu Ile Glu Pro Val Phe Asp Arg Glu Glu Leu
            260                 265                 270
Ile Val Cys Glu Leu Asp Leu Asp Glu Ala Tyr Lys Glu Arg Met Thr
        275                 280                 285
Met Asp Val Ser Gly His Tyr Gln Arg Arg Asp Val Phe Ser Phe Asp
    290                 295                 300
Val Asn Gln His Arg His
305                 310

<210> 277
<211> 1056
<212> DNA
<213> Unknown

<220>
<223> Obtained from an environmental sample

<400> 277
atgccaaccc ccagcgatca tttcaaaatc gccgctgttc aggcctcgcc cgtgtttctg      60
gaccgggagg ccactgtgga aaaggcctgc cggttgatcg ccgaagccgc aaagcagggc     120
gcccgcctca tcgtctttcc ggaatctttc atcccgacct acccggactg ggtatgggcc     180
gttcccccgg gaagggaaag aatcctgaac cagctgtatt ctgaattcct ggccaatgcc     240
gtcgatgttc ccggcgcggc gaccgaacaa cttgcccagg ctgcacgaat ggccggcgcc     300
tatgtgatta tgggcgtcac cgaaagagac acctcggcca gcggggccag cctctacaac     360
accctgctct acttcagccc cgaaggcatc ctaatgggca aacaccggaa gctggttccc     420

acggggggcg aacggctggt ctgggcctac ggagacggca gcacgctgga ggtctacgac 480
actccgctgg gaaagatcgg cgggctgatc tgctgggaga actacatgcc cctggcccgg 540
tacacgatgt acgcctgggg cacccagatt tacatcgccg ccacctggga ccgcggggaa 600
ccgtggctct ccaccctgcg gcatatcgcc aaggaaggaa gggtctacgt catcgggtgc 660
tgcatcgccc tgcgccaggg ggatatcccg gaccggttcg agtacaaggg aaaattttat 720
tccgggtccc gggagtggat caatgagggc gacagcgcca tcgtgaaccc ggacggggaa 780
ttcatcgccg ggccggtgcg gatgaaggag gagatcctgt atgccgagat agacccccgg 840
cagatgcggg gccccaagtg gatgctcgat gtggccggtc attacgcccg gccggatatc 900
ttcgagctca tcgtccaccg gaatccccac ccgatgatca aaatcgccga agacaggggc 960
gcggggatcg cctcaagttt gattcgcccc cgccctaacc ttcccccatc aagggggagg 1020
aaatcggcaa gaagcaaacg caagcccaaa aaatga 1056

<210> 278
<211> 351
<212> PRT
<213> Unknown

<220>
<223> Obtained from an environmental sample

<400> 278
Met Pro Thr Pro Ser Asp His Phe Lys Ile Ala Ala Val Gln Ala Ser
1               5                   10                  15
Pro Val Phe Leu Asp Arg Glu Ala Thr Val Glu Lys Ala Cys Arg Leu
            20                  25                  30
Ile Ala Glu Ala Ala Lys Gln Gly Ala Arg Leu Ile Val Phe Pro Glu
        35                  40                  45
Ser Phe Ile Pro Thr Tyr Pro Asp Trp Val Trp Ala Val Pro Pro Gly
    50                  55                  60
Arg Glu Arg Ile Leu Asn Gln Leu Tyr Ser Glu Phe Leu Ala Asn Ala
65                  70                  75                  80
Val Asp Val Pro Gly Ala Ala Thr Glu Gln Leu Ala Gln Ala Ala Arg
                85                  90                  95
Met Ala Gly Ala Tyr Val Ile Met Gly Val Thr Glu Arg Asp Thr Ser
            100                 105                 110
Ala Ser Gly Ala Ser Leu Tyr Asn Thr Leu Leu Tyr Phe Ser Pro Glu
        115                 120                 125
Gly Ile Leu Met Gly Lys His Arg Lys Leu Val Pro Thr Gly Gly Glu
    130                 135                 140
Arg Leu Val Trp Ala Tyr Gly Asp Gly Ser Thr Leu Glu Val Tyr Asp
145                 150                 155                 160
Thr Pro Leu Gly Lys Ile Gly Gly Leu Ile Cys Trp Glu Asn Tyr Met
                165                 170                 175
Pro Leu Ala Arg Tyr Thr Met Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Lys Glu Gly Arg Val Tyr Val Ile Gly Cys Cys Ile Ala Leu
    210                 215                 220
Arg Gln Gly Asp Ile Pro Asp Arg Phe Glu Tyr Lys Gly Lys Phe Tyr
225                 230                 235                 240
Ser Gly Ser Arg Glu Trp Ile Asn Glu Gly Asp Ser Ala Ile Val Asn
                245                 250                 255
Pro Asp Gly Glu Phe Ile Ala Gly Pro Val Arg Met Lys Glu Glu Ile
            260                 265                 270
Leu Tyr Ala Glu Ile Asp Pro Arg Gln Met Arg Gly Pro Lys Trp Met
        275                 280                 285

Leu Asp Val Ala Gly His Tyr Ala Arg Pro Asp Ile Phe Glu Leu Ile
    290                 295                 300
Val His Arg Asn Pro His Pro Met Ile Lys Ile Ala Glu Asp Arg Gly
305                 310                 315                 320
Ala Gly Ile Ala Ser Ser Leu Ile Arg Pro Arg Pro Asn Leu Pro Pro
                325                 330                 335
Ser Arg Gly Arg Lys Ser Ala Arg Ser Lys Arg Lys Pro Lys Lys
            340                 345                 350

<210> 279
<211> 1014
<212> DNA
<213> Unknown

<220>
<223> Obtained from an environmental sample

<400> 279
atgggcatta cccatcccaa atacaaagtc gctgcagtcc aggccgcgcc ggtctggctg      60
gacctggatg ccacggtgga caagtgcatc cgcctgatcc aggaggccgc tgacaagggc     120
tgcaagctga tcgcgtttcc ggagacgttc attcccggct atccctggca catctggatg     180
ggtgcgccgg cctgggccat cggccggggc tttgtgcagc ggtatttcga caactccctg     240
tcctatgaca gcccgcaggc cgaaaagctg cgccaggccg tcaaggccgc aggcatcacc     300
gcgtcgctgg gactgtcgga gcgctcgggc ggcagtctct acatcgcgca gtggctcatt     360
ggccccgacg gcgaaacgat ctcgcagcgg cgtaagctgc ggcccacgca ttccgaacgc     420
accgtcttcg gcgacggcga tgggagcgac ctcaaggtgc acgacacgcc gctgggccgt     480
gtgggtgagc tggcgtgctg ggagaacatc ctgtcgctga acaagtacgc catgttctcg     540
cagcacgagc aggtgcacat tgcagcctgg cctagcttct ccacctacga gccctttgca     600
catgccctgg gctgggaagt gaacaacgca gtcagcaagg tgtacgcagt ggaaggcggg     660
tgtttcgtgg tggcaccctg cgccatcatt tccaaggaga tggtcgatga actgtgcgac     720
accccggaca agcacaccct gacccatgtg ggaggcggcc acgcggtgat ctacggcccg     780
gatggcgccc ccctggcgga caagctgcct gaagacgccg agggcctgct gatcgcggag     840
atcgacctcg ggatgattgg cgtggccaag aacgccatgg acccggtagg gcactactcg     900
cgccccgacg tgcaccgcct gcttctcaac cgcaagaagg cagcgcaggt ggagcacttt     960
gcgctgccgg tggacgccat cgattccgag ccccaagcca ccacggcgca ctga          1014

<210> 280
<211> 337
<212> PRT
<213> Unknown

<220>
<223> Obtained from an environmental sample

<400> 280
Met Gly Ile Thr His Pro Lys Tyr Lys Val Ala Ala Val Gln Ala Ala
 1               5                  10                  15
Pro Val Trp Leu Asp Leu Asp Ala Thr Val Asp Lys Cys Ile Arg Leu
            20                  25                  30
Ile Gln Glu Ala Ala Asp Lys Gly Cys Lys Leu Ile Ala Phe Pro Glu
        35                  40                  45
Thr Phe Ile Pro Gly Tyr Pro Trp His Ile Trp Met Gly Ala Pro Ala
    50                  55                  60
Trp Ala Ile Gly Arg Gly Phe Val Gln Arg Tyr Phe Asp Asn Ser Leu
65                  70                  75                  80
Ser Tyr Asp Ser Pro Gln Ala Glu Lys Leu Arg Gln Ala Val Lys Ala
                85                  90                  95

Ala Gly Ile Thr Ala Ser Leu Gly Leu Ser Glu Arg Ser Gly Gly Ser
            100                 105                 110
Leu Tyr Ile Ala Gln Trp Leu Ile Gly Pro Asp Gly Glu Thr Ile Ser
        115                 120                 125
Gln Arg Arg Lys Leu Arg Pro Thr His Ser Glu Arg Thr Val Phe Gly
    130                 135                 140
Asp Gly Asp Gly Ser Asp Leu Lys Val His Asp Thr Pro Leu Gly Arg
145                 150                 155                 160
Val Gly Glu Leu Ala Cys Trp Glu Asn Ile Leu Ser Leu Asn Lys Tyr
                165                 170                 175
Ala Met Phe Ser Gln His Glu Gln Val His Ile Ala Ala Trp Pro Ser
            180                 185                 190
Phe Ser Thr Tyr Glu Pro Phe Ala His Ala Leu Gly Trp Glu Val Asn
        195                 200                 205
Asn Ala Val Ser Lys Val Tyr Ala Val Glu Gly Gly Cys Phe Val Val
    210                 215                 220
Ala Pro Cys Ala Ile Ile Ser Lys Glu Met Val Asp Glu Leu Cys Asp
225                 230                 235                 240
Thr Pro Asp Lys His Thr Leu Thr His Val Gly Gly Gly His Ala Val
                245                 250                 255
Ile Tyr Gly Pro Asp Gly Ala Pro Leu Ala Asp Lys Leu Pro Glu Asp
            260                 265                 270
Ala Glu Gly Leu Leu Ile Ala Glu Ile Asp Leu Gly Met Ile Gly Val
        275                 280                 285
Ala Lys Asn Ala Met Asp Pro Val Gly His Tyr Ser Arg Pro Asp Val
    290                 295                 300
His Arg Leu Leu Leu Asn Arg Lys Lys Ala Ala Gln Val Glu His Phe
305                 310                 315                 320
Ala Leu Pro Val Asp Ala Ile Asp Ser Glu Pro Gln Ala Thr Thr Ala
                325                 330                 335
His

<210> 281
<211> 936
<212> DNA
<213> Unknown

<220>
<223> Obtained from an environmental sample

<400> 281
atgtccaaga tcgccgtcgt ccaagagcct ccggtgctgc tcgatcgcgc cgccaccctc      60
gagcgcgccg tctcggccat cgagcgggcg gccgacgtgg gggcgcacct cgtcgtgttc     120
cccgagacgt acgtcccggg gtaccccgac tgggtctggc ggacgcgccc cgacgacttc     180
aagctcgcgg gcgcgctgca cgagcgcctc ctcgcgaacg cggtcgacct cgaaaaggac     240
cagctcgcgc cgctccgcga ggccgcgcgc cggcgaggcg tcaccatcgc gtgcggcgtg     300
aacgagcgcg aggggagcca cggccgcgcg accctctaca acaccgtcgt cgtcgtcgga     360
cccgacggcg cgatcctcaa ccgccaccgc aagctcgtcc ccacgaaccc cgagcgcatg     420
gtctggggcc cgggtgacgc gagcgggctg cgcgtcgtcg acacgccggc cggccgcgtg     480
ggggcgctca tctgctggga gaactacatg ccgctcgcgc gcttcgcgct ctacgcgcag     540
ggcgtcgagg tctacctcgc gccgacgtgg gatcacggcg acacttggct cgcctccatg     600
cggcacatcg cgcgcgaggc gcgcgcctgg gtcgtctcgg gggccatctg catgcaggcg     660
aaggacgtcc ccgccgactt cccgcagcgc gcggcgatct accccgacga ggaggagtgg     720
ctcaaccccg gcgatgccgt cgtcgtcgat cccaccggcg ccgtcgccgc cggcccgctg     780
caccgcgagc gcggcatcct ctacgccgag tgcgatcccg cgcgggcgtc gctcgcccgc     840
cgcacgctcg acgtctccgg gcactacgga cggcccgacg tctttcacct gcagatcgac     900

cgcacaccgc gcgtgccggc gtcgttccgg gactga                                936

<210> 282
<211> 311
<212> PRT
<213> Unknown

<220>
<223> Obtained from an environmental sample

<400> 282
Met Ser Lys Ile Ala Val Val Gln Glu Pro Pro Val Leu Leu Asp Arg
1               5                   10                  15
Ala Ala Thr Leu Glu Arg Ala Val Ser Ala Ile Glu Arg Ala Ala Asp
            20                  25                  30
Val Gly Ala His Leu Val Val Phe Pro Glu Thr Tyr Val Pro Gly Tyr
        35                  40                  45
Pro Asp Trp Val Trp Arg Thr Arg Pro Asp Asp Phe Lys Leu Ala Gly
    50                  55                  60
Ala Leu His Glu Arg Leu Leu Ala Asn Ala Val Asp Leu Glu Lys Asp
65                  70                  75                  80
Gln Leu Ala Pro Leu Arg Glu Ala Ala Arg Arg Arg Gly Val Thr Ile
                85                  90                  95
Ala Cys Gly Val Asn Glu Arg Glu Gly Ser His Gly Arg Ala Thr Leu
            100                 105                 110
Tyr Asn Thr Val Val Val Val Gly Pro Asp Gly Ala Ile Leu Asn Arg
        115                 120                 125
His Arg Lys Leu Val Pro Thr Asn Pro Glu Arg Met Val Trp Gly Pro
    130                 135                 140
Gly Asp Ala Ser Gly Leu Arg Val Val Asp Thr Pro Ala Gly Arg Val
145                 150                 155                 160
Gly Ala Leu Ile Cys Trp Glu Asn Tyr Met Pro Leu Ala Arg Phe Ala
                165                 170                 175
Leu Tyr Ala Gln Gly Val Glu Val Tyr Leu Ala Pro Thr Trp Asp His
            180                 185                 190
Gly Asp Thr Trp Leu Ala Ser Met Arg His Ile Ala Arg Glu Ala Arg
        195                 200                 205
Ala Trp Val Val Ser Gly Ala Ile Cys Met Gln Ala Lys Asp Val Pro
    210                 215                 220
Ala Asp Phe Pro Gln Arg Ala Ala Ile Tyr Pro Asp Glu Glu Glu Trp
225                 230                 235                 240
Leu Asn Pro Gly Asp Ala Val Val Val Asp Pro Thr Gly Ala Val Ala
                245                 250                 255
Ala Gly Pro Leu His Arg Glu Arg Gly Ile Leu Tyr Ala Glu Cys Asp
            260                 265                 270
Pro Ala Arg Ala Ser Leu Ala Arg Arg Thr Leu Asp Val Ser Gly His
        275                 280                 285
Tyr Gly Arg Pro Asp Val Phe His Leu Gln Ile Asp Arg Thr Pro Arg
    290                 295                 300
Val Pro Ala Ser Phe Arg Asp
305                 310

<210> 283
<211> 1017
<212> DNA
<213> Unknown

<220>
<223> Obtained from an environmental sample

<400> 283
atgggcatcg aacatccgaa gtacaaggtc gcggtggtgc aggcggcacc ggcctggctc      60
gatctcgacg cgtcgatcga caagaccatc gggctgatcg aggaggccgc ccaaaaaggc     120
gccaagctga ttgcattccc cgaggccttc atccccggtt acccctggca catctggatg     180
gactcgccgg cctgggcgat cggccgcggt ttcgtgcagc gctattttga caattcgctc     240
gcctatgaca gcccgcaggc cgagaaactg cgcgcggcgg ttcgcaaggc aaagctcacg     300
gccgtgatcg ggctgtccga gcgcgacggc ggcagtcttt acctcgcgca atggctgatc     360
gggcccgacg gtgagaccat cgccaagcgc cgcaagctgc ggccgacaca tgcggagcgc     420
acggtatacg gcgagggcga cggcagcgac ctcgcggttc acaaccgtcc ggacattggc     480
cgccttggcg cgctctgctg ctgggagcat ctccagccgc tgtcgaaata cgcgatgtac     540
gcgcagaacg agcaggtgca tgtcgcggcc tggccgagct tttcgctgta cgatcccttc     600
gcggtggcgc tcggcgccga agtgaacaac gcggcctcgc gcgtctatgc ggtcgaaggc     660
tcctgcttcg tgctggcgcc gtgcgcgacg gtctcgcagg ccatgatcga cgaactctgc     720
gaccggccgg acaagcacgc gctgttgcat gtcggcggcg gttttgccgc gatctacggt     780
cctgacggca gccagatcgg cgacaaactc gctcccgacc aggaagggtt gctgatcgcg     840
gagatcgatc tcggcgccat cggcgtcgcc aagaatgcgg cggatcccgc cgggcattat     900
tcgcggcctg acgtgacgcg actgttgctc aacaagaagc cgtacaagcg cgtcgagcag     960
ttttcgccgc cggccgaggc gctcgagccg acggatatcg cagcagcagc aagctaa       1017

<210> 284
<211> 338
<212> PRT
<213> Unknown

<220>
<223> Obtained from an environmental sample

<400> 284
Met Gly Ile Glu His Pro Lys Tyr Lys Val Ala Val Val Gln Ala Ala
1               5                   10                  15
Pro Ala Trp Leu Asp Leu Asp Ala Ser Ile Asp Lys Thr Ile Gly Leu
            20                  25                  30
Ile Glu Glu Ala Ala Gln Lys Gly Ala Lys Leu Ile Ala Phe Pro Glu
        35                  40                  45
Ala Phe Ile Pro Gly Tyr Pro Trp His Ile Trp Met Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Lys Leu Arg Ala Ala Val Arg Lys
                85                  90                  95
Ala Lys Leu Thr Ala Val Ile Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asn Arg Pro Asp Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Val Ala Leu Gly Ala Glu Val
        195                 200                 205

Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Val Gly Gly Gly Phe Ala
                245                 250                 255
Ala Ile Tyr Gly Pro Asp Gly Ser Gln Ile Gly Asp Lys Leu Ala Pro
            260                 265                 270
Asp Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Lys Pro Tyr Lys Arg Val Glu Gln
305                 310                 315                 320
Phe Ser Pro Pro Ala Glu Ala Leu Glu Pro Thr Asp Ile Ala Ala Ala
                325                 330                 335
Ala Ser

<210> 285
<211> 918
<212> DNA
<213> Unknown

<220>
<223> Obtained from an environmental sample

<400> 285
atgaatacta atctagtaaa ggtcgcggcg gctcaagttg ctccccattt tctcaatttg      60
agcaatacgg tggaaaaaac ctgcaactta atttctgaag caggcaaaaa tggagcaaag     120
ctaatcgtat ttccagaagc cttcatctct ggttatcccg attgggtctg gcttattccc     180
aatgcgaatt ctgcaatgct ggatgattta taccaggaat tggttgagaa cgcagtaacg     240
atccctgata caacaacaca taaactatgt caggctgcaa aagatgcagg ggtatatgtt     300
gcggtaggta tacatgagag aaattcagaa gcaagtggct tcacgctttt caatacgctt     360
ctatacatca acgatcaagg cgtaatcatc ggaaaacacc gaaaattaat ccctacaggg     420
ggcgaacggc tggtctgggg gcagggtaat ggggatacac tttctgcatt cgatacagac     480
ttcggcaaat taggaggatt gctttgttgg gaaaattata tgccactcgc gcgtcaagct     540
atgtattccg ttggaactga agtatatgta gccccaacct gggactccag cgagaattgg     600
ttgttaagca tgcgccatat tgcccgagag ggcggtatgt ttgtaattag tgtttgccag     660
gctctccgaa aagacgacat ccctgatcag tatgaattta agaaactcta tcctgataat     720
tcagaatgga tcaatagcgg taacagttgc atcatcaacc cgcgcggtga gattattgct     780
ggaccaatct caaaccaaca agaaatactt tatgcagatt tagacctgag tttaattgca     840
aaatctaaac gtatgttcga tgttactggg cattattccc ggccggatgt gtttagctat     900
gaaatcaaca aaagctag                                                   918

<210> 286
<211> 305
<212> PRT
<213> Unknown

<220>
<223> Obtained from an environmental sample

<400> 286
Met Asn Thr Asn Leu Val Lys Val Ala Ala Ala Gln Val Ala Pro His
 1               5                  10                  15
Phe Leu Asn Leu Ser Asn Thr Val Glu Lys Thr Cys Asn Leu Ile Ser

                20                  25                  30
Glu Ala Gly Lys Asn Gly Ala Lys Leu Ile Val Phe Pro Glu Ala Phe
        35                  40                  45
Ile Ser Gly Tyr Pro Asp Trp Val Trp Leu Ile Pro Asn Ala Asn Ser
    50                  55                  60
Ala Met Leu Asp Asp Leu Tyr Gln Glu Leu Val Glu Asn Ala Val Thr
65                  70                  75                  80
Ile Pro Asp Thr Thr Thr His Lys Leu Cys Gln Ala Ala Lys Asp Ala
                85                  90                  95
Gly Val Tyr Val Ala Val Gly Ile His Glu Arg Asn Ser Glu Ala Ser
            100                 105                 110
Gly Phe Thr Leu Phe Asn Thr Leu Leu Tyr Ile Asn Asp Gln Gly Val
        115                 120                 125
Ile Ile Gly Lys His Arg Lys Leu Ile Pro Thr Gly Gly Glu Arg Leu
    130                 135                 140
Val Trp Gly Gln Gly Asn Gly Asp Thr Leu Ser Ala Phe Asp Thr Asp
145                 150                 155                 160
Phe Gly Lys Leu Gly Gly Leu Leu Cys Trp Glu Asn Tyr Met Pro Leu
                165                 170                 175
Ala Arg Gln Ala Met Tyr Ser Val Gly Thr Glu Val Tyr Val Ala Pro
            180                 185                 190
Thr Trp Asp Ser Ser Glu Asn Trp Leu Leu Ser Met Arg His Ile Ala
        195                 200                 205
Arg Glu Gly Gly Met Phe Val Ile Ser Val Cys Gln Ala Leu Arg Lys
    210                 215                 220
Asp Asp Ile Pro Asp Gln Tyr Glu Phe Lys Lys Leu Tyr Pro Asp Asn
225                 230                 235                 240
Ser Glu Trp Ile Asn Ser Gly Asn Ser Cys Ile Ile Asn Pro Arg Gly
                245                 250                 255
Glu Ile Ile Ala Gly Pro Ile Ser Asn Gln Gln Glu Ile Leu Tyr Ala
            260                 265                 270
Asp Leu Asp Leu Ser Leu Ile Ala Lys Ser Lys Arg Met Phe Asp Val
        275                 280                 285
Thr Gly His Tyr Ser Arg Pro Asp Val Phe Ser Tyr Glu Ile Asn Lys
    290                 295                 300
Ser
305

<210> 287
<211> 936
<212> DNA
<213> Unknown

<220>
<223> Obtained from an environmental sample

<400> 287
gtgatcaagg tagcaatcgc ccaggtggca ccggtggttc tggacaaggc gcgcaccatt      60
gagaaagcgg taggaattat tcgcgctgcc gcgcaagagg gcattgagct cctggttttc     120
ccggagacgt ttatcccgac ctatccagcc tgggtatggc gcttgcgtcc gggtactgat     180
tacggcctga gcgaggaact gcacgcgctc ctgctggata attcggtaga tatggagagc     240
aaggacctgg agccattgca agctgttgct gcagagacca gcatgaccgt ggtaataggt     300
atgaacgagc gagacggccg attcagccgg ggtacaatct acaatgccct ggttgtgatc     360
ggtccaggtg gcacgatcct gaacaggcac cgcaagctta tgcccaccaa ccccgagcgt     420
atggtttggg gtatgggcga tgccagcggg ctgaaggtag tggaaatgtc ttacgggcgc      480
ctgggtgggc tgatttgctg ggagaatttc atgcctctcg cgcgctatgg cttgtatgcc     540
cagggtgtgg agatttacgt ggcgcccacc tatgaccagg gcgacggctg ggtcggcagc     600

atgcagcata tagcccggga gggtcgttgc tgggtactct cggccgggac ccttttgcgt 660
ggcagtgatt ttctgccgga ttttccgggc aagaccgagt tatatcccga tgaccaggag 720
tgggtgaatc cgggtggctc ggtgatcgtg gcaccggggg gagagattgt ggccggcccc 780
atgtatcgcg acgaaggtct gctggtctgc gagttggatg cgacgcttag tgtccgcggc 840
aagcgctcgc tggatgtggc cggccattac tcccggccgg atttgtttga actggaaata 900
gatggcgacc cgctggaacc catagagtgg gattga 936

<210> 288
<211> 311
<212> PRT
<213> Unknown

<220>
<223> Obtained from an environmental sample

<400> 288
Val Ile Lys Val Ala Ile Ala Gln Val Ala Pro Val Val Leu Asp Lys
1               5                   10                  15
Ala Arg Thr Ile Glu Lys Ala Val Gly Ile Ile Arg Ala Ala Ala Gln
            20                  25                  30
Glu Gly Ile Glu Leu Leu Val Phe Pro Glu Thr Phe Ile Pro Thr Tyr
        35                  40                  45
Pro Ala Trp Val Trp Arg Leu Arg Pro Gly Thr Asp Tyr Gly Leu Ser
    50                  55                  60
Glu Glu Leu His Ala Leu Leu Leu Asp Asn Ser Val Asp Met Glu Ser
65                  70                  75                  80
Lys Asp Leu Glu Pro Leu Gln Ala Val Ala Ala Glu Thr Ser Met Thr
                85                  90                  95
Val Val Ile Gly Met Asn Glu Arg Asp Gly Arg Phe Ser Arg Gly Thr
            100                 105                 110
Ile Tyr Asn Ala Leu Val Val Ile Gly Pro Gly Gly Thr Ile Leu Asn
        115                 120                 125
Arg His Arg Lys Leu Met Pro Thr Asn Pro Glu Arg Met Val Trp Gly
    130                 135                 140
Met Gly Asp Ala Ser Gly Leu Lys Val Val Glu Met Ser Tyr Gly Arg
145                 150                 155                 160
Leu Gly Gly Leu Ile Cys Trp Glu Asn Phe Met Pro Leu Ala Arg Tyr
                165                 170                 175
Gly Leu Tyr Ala Gln Gly Val Glu Ile Tyr Val Ala Pro Thr Tyr Asp
            180                 185                 190
Gln Gly Asp Gly Trp Val Gly Ser Met Gln His Ile Ala Arg Glu Gly
        195                 200                 205
Arg Cys Trp Val Leu Ser Ala Gly Thr Leu Leu Arg Gly Ser Asp Phe
    210                 215                 220
Leu Pro Asp Phe Pro Gly Lys Thr Glu Leu Tyr Pro Asp Asp Gln Glu
225                 230                 235                 240
Trp Val Asn Pro Gly Gly Ser Val Ile Val Ala Pro Gly Gly Glu Ile
                245                 250                 255
Val Ala Gly Pro Met Tyr Arg Asp Glu Gly Leu Leu Val Cys Glu Leu
            260                 265                 270
Asp Ala Thr Leu Ser Val Arg Gly Lys Arg Ser Leu Asp Val Ala Gly
        275                 280                 285
His Tyr Ser Arg Pro Asp Leu Phe Glu Leu Glu Ile Asp Gly Asp Pro
    290                 295                 300
Leu Glu Pro Ile Glu Trp Asp
305                 310

<210> 289
<211> 921
<212> DNA
<213> Unknown

<220>
<223> Obtained from an environmental sample

<400> 289
atggtgacgg tggccgccgt acaggcaacg ccggtgttcc tcgaccgcga ggcgacctcg 60
gacaaggtct gcgccttggt caaggaggcg gccggccacg gggcagaact gatcgtcttc 120
cccgagtcct tcgtccctgc ctatccggac tgggtgtggc gcacccctgc ctggagtgac 180
accgagttcg tgaagcgctt ctacgcgaac gcggtgaccg tccccggcgc gaccctcgag 240
cgcatcggcg cagcggcggc ggaggcggag gcgtacgtcg tgatcggcgt gaccgagatc 300
gacggcggaa ctctctacaa caccctcctc tacctgggcc cggacggaca gctgttgcaa 360
cggcatcgca agctcatgcc caccggtggg gagcggaccg tgtggggaat gggagacggc 420
tctgagctcg acgtcgtgag cacgccgttc ggcgtcgtcg gtgggttgtt gtgctgggag 480
aactacatgc cgctcgcccg ggcggcgatc tacgcccagc actgtgacat ctacctggct 540
ccgacatggg acaacagcga cacgtgggta gccacgttgc gtcacatcgc caaggagggg 600
cggcagttcg tcatcggcgt cgccccgctg ctgcgcggct ccgacgtacc ggaggacctc 660
cgcggcacgc tctacgggct gtcggacgac tggatgtcgc gcggctacac caccatcgtc 720
gcaccaagcg gcgaggtgat cgccggcccg gtcctggagc gtgaggagat cctcttcgcg 780
gacctcgacc tggccgacgt gcaggagcag agaaggatgt tcgaccctgt cggccactac 840
tcacgacccg acgtcttcac gctccacgtc gacgcacgac cgaagagccc ggtcgtcttc 900
gagagggatg caccgacctg a 921

<210> 290
<211> 306
<212> PRT
<213> Unknown

<220>
<223> Obtained from an environmental sample

<400> 290
Met Val Thr Val Ala Ala Val Gln Ala Thr Pro Val Phe Leu Asp Arg
1               5                   10                  15
Glu Ala Thr Ser Asp Lys Val Cys Ala Leu Val Lys Glu Ala Ala Gly
            20                  25                  30
His Gly Ala Glu Leu Ile Val Phe Pro Glu Ser Phe Val Pro Ala Tyr
        35                  40                  45
Pro Asp Trp Val Trp Arg Thr Pro Ala Trp Ser Asp Thr Glu Phe Val
    50                  55                  60
Lys Arg Phe Tyr Ala Asn Ala Val Thr Val Pro Gly Ala Thr Leu Glu
65                  70                  75                  80
Arg Ile Gly Ala Ala Ala Ala Glu Ala Glu Ala Tyr Val Val Ile Gly
                85                  90                  95
Val Thr Glu Ile Asp Gly Gly Thr Leu Tyr Asn Thr Leu Leu Tyr Leu
            100                 105                 110
Gly Pro Asp Gly Gln Leu Leu Gln Arg His Arg Lys Leu Met Pro Thr
        115                 120                 125
Gly Gly Glu Arg Thr Val Trp Gly Met Gly Asp Gly Ser Glu Leu Asp
    130                 135                 140
Val Val Ser Thr Pro Phe Gly Val Val Gly Gly Leu Leu Cys Trp Glu
145                 150                 155                 160
Asn Tyr Met Pro Leu Ala Arg Ala Ala Ile Tyr Ala Gln His Cys Asp
                165                 170                 175

Ile Tyr Leu Ala Pro Thr Trp Asp Asn Ser Asp Thr Trp Val Ala Thr
            180                 185                 190
Leu Arg His Ile Ala Lys Glu Gly Arg Gln Phe Val Ile Gly Val Ala
        195                 200                 205
Pro Leu Leu Arg Gly Ser Asp Val Pro Glu Asp Leu Arg Gly Thr Leu
    210                 215                 220
Tyr Gly Leu Ser Asp Asp Trp Met Ser Arg Gly Tyr Thr Thr Ile Val
225                 230                 235                 240
Ala Pro Ser Gly Glu Val Ile Ala Gly Pro Val Leu Glu Arg Glu Glu
                245                 250                 255
Ile Leu Phe Ala Asp Leu Asp Leu Ala Asp Val Gln Glu Gln Arg Arg
            260                 265                 270
Met Phe Asp Pro Val Gly His Tyr Ser Arg Pro Asp Val Phe Thr Leu
        275                 280                 285
His Val Asp Ala Arg Pro Lys Ser Pro Val Val Phe Glu Arg Asp Ala
    290                 295                 300
Pro Thr
305

<210> 291
<211> 1002
<212> DNA
<213> Unknown

<220>
<223> Obtained from an environmental sample

<400> 291
atgatgaaaa caactgttac cgttgcctgc gttcaggccg cccccgtatt tatggattta      60
gaaggcacca tagataaaac gatcaccctc atctctgaag ccgcacagaa aggcgcggag     120
ctcatcgctt ttccggagac ctggataccc ggttacccgt ggttcttatg gctgaactcg     180
cccgcgacaa atatgcccct ggtttatcaa tatcatcaga actctctggt gctggacagt     240
gcccaggcga agcgaattgc ggatgctgca cagcagaata acatcactgt cgttctggga     300
ttcagcgagc gcgatcatgg aagcctctat atctcacagt ggctgattgg cagcgacggg     360
gaaactattg gcatccggcg caagctcaag gccacacacg tggagcgtac gctgttcggc     420
gaaagcgacg gctcctccct gaccacctgg gagacacctc tgggtaacgt cggggccctc     480
tgctgctggg agcacctgca gccgctgtct cgctatgcga tgtattccca gcatgaagag     540
atccatatcg ctgcgtggcc cagcttcagt ctttacacca gcgcaactgc cgcgctcgga     600
cctgacgtca acacggcggc ttcacgcctc tatgccgcgg aggggcagtg cttcgtgtta     660
gccccatgtg ccgtggtttc tgatgagatg attgatttac tctgtcctga tgatgaccgg     720
agagcgttac tcagtgccgg agggggacat gcccgtattt acggacctga tggaagagaa     780
ctcgtcaccc ctctcgggga aaatgaggaa ggactgctta tcgctgagct cgactctgct     840
gcgatcacct ttgccaaact ggcggcagat cccgtaggcc actattcccg ccctgacgtg     900
acccgccttc tttttaatcc ttcagccaac aagactgtta ttaaacggca ttcgcctcct     960
gagttaattg ccgaacaggc tccggaagaa gaggaggagt ag                       1002

<210> 292
<211> 333
<212> PRT
<213> Unknown

<220>
<223> Obtained from an environmental sample

<400> 292
Met Met Lys Thr Thr Val Thr Val Ala Cys Val Gln Ala Ala Pro Val
1               5                   10                  15

Phe Met Asp Leu Glu Gly Thr Ile Asp Lys Thr Ile Thr Leu Ile Ser
            20                  25                  30
Glu Ala Ala Gln Lys Gly Ala Glu Leu Ile Ala Phe Pro Glu Thr Trp
        35                  40                  45
Ile Pro Gly Tyr Pro Trp Phe Leu Trp Leu Asn Ser Pro Ala Thr Asn
    50                  55                  60
Met Pro Leu Val Tyr Gln Tyr His Gln Asn Ser Leu Val Leu Asp Ser
65                  70                  75                  80
Ala Gln Ala Lys Arg Ile Ala Asp Ala Ala Gln Gln Asn Asn Ile Thr
                85                  90                  95
Val Val Leu Gly Phe Ser Glu Arg Asp His Gly Ser Leu Tyr Ile Ser
            100                 105                 110
Gln Trp Leu Ile Gly Ser Asp Gly Glu Thr Ile Gly Ile Arg Arg Lys
        115                 120                 125
Leu Lys Ala Thr His Val Glu Arg Thr Leu Phe Gly Glu Ser Asp Gly
    130                 135                 140
Ser Ser Leu Thr Thr Trp Glu Thr Pro Leu Gly Asn Val Gly Ala Leu
145                 150                 155                 160
Cys Cys Trp Glu His Leu Gln Pro Leu Ser Arg Tyr Ala Met Tyr Ser
                165                 170                 175
Gln His Glu Glu Ile His Ile Ala Ala Trp Pro Ser Phe Ser Leu Tyr
            180                 185                 190
Thr Ser Ala Thr Ala Ala Leu Gly Pro Asp Val Asn Thr Ala Ala Ser
        195                 200                 205
Arg Leu Tyr Ala Ala Glu Gly Gln Cys Phe Val Leu Ala Pro Cys Ala
    210                 215                 220
Val Val Ser Asp Glu Met Ile Asp Leu Leu Cys Pro Asp Asp Asp Arg
225                 230                 235                 240
Arg Ala Leu Leu Ser Ala Gly Gly Gly His Ala Arg Ile Tyr Gly Pro
                245                 250                 255
Asp Gly Arg Glu Leu Val Thr Pro Leu Gly Glu Asn Glu Glu Gly Leu
            260                 265                 270
Leu Ile Ala Glu Leu Asp Ser Ala Ala Ile Thr Phe Ala Lys Leu Ala
        275                 280                 285
Ala Asp Pro Val Gly His Tyr Ser Arg Pro Asp Val Thr Arg Leu Leu
    290                 295                 300
Phe Asn Pro Ser Ala Asn Lys Thr Val Ile Lys Arg His Ser Pro Pro
305                 310                 315                 320
Glu Leu Ile Ala Glu Gln Ala Pro Glu Glu Glu Glu Glu
                325                 330

<210> 293
<211> 1008
<212> DNA
<213> Unknown

<220>
<223> Obtained from an environmental sample

<400> 293
atgaaaaata tcaaaaactc agaaaaaagc agcacagtaa gagtcgctgc ggtacaaatc      60
agtccggtgt tgtacaaccg cgaagctacc gttcaaaaag tagtcaacaa aatccttgaa     120
ctaggaaaac aaggggtaca attcgccact tttccggaaa cgatagtgcc ttattatcct     180
tatttctctt ttattcaggc gccttatgcc atgggcaaag aacacctgcg cttgcttgaa     240
caatcagtta ctgttccgtc agccgcgacc gatgccataa gtgaggcggc aaaggaagcc     300
aatatggtag tgtctattgg tgtcaatgaa cgagacggtg gtaccattta caatacgcaa     360
ctcctttttg atgctgacgg aacattaatt cagcgcagac gtaaacttac accaacgtat     420

catgaaagaa tgatttgggg acaaggtgac gcttcaggtc ttcgtgccac agacagcgct 480
gttgggcgta tcgggcagtt ggcttgttgg gaacattaca atccattgtt ccgttatgct 540
ttgattgctg atggagaaca aatccattct gccatgtatc ccggatcatt tttaggtgcg 600
ttgcacggtg aacaaaccga aatcaatgta cgccaacacg ctttagaatc ggccagcttc 660
gtcgtagtgg ctaccggttg gttggatgcc gatcaacaag cacaaattgc gaaagacacc 720
ggtggaccaa tcggaccaat ttcgggaggt tgttttacag ccgttatagg ccctgacgga 780
caactaatcg gggaagccct tacatcaggt gaaggggaag tgattgccga tattgatttg 840
gcacaaattg atgcccgcaa aagattaatg gatgccagtg gtcactacaa ccgtcctgaa 900
ttgttgagct tgcatatcga tcacactccg actgctccta tgcatgaaag agtagtttac 960
actgagccgg gattagcaaa aagacaaaat gaaaattcat caaattaa 1008

<210> 294
<211> 335
<212> PRT
<213> Unknown

<220>
<223> Obtained from an environmental sample

<400> 294
Met Lys Asn Ile Lys Asn Ser Glu Lys Ser Ser Thr Val Arg Val Ala
1               5                   10                  15
Ala Val Gln Ile Ser Pro Val Leu Tyr Asn Arg Glu Ala Thr Val Gln
            20                  25                  30
Lys Val Val Asn Lys Ile Leu Glu Leu Gly Lys Gln Gly Val Gln Phe
        35                  40                  45
Ala Thr Phe Pro Glu Thr Ile Val Pro Tyr Tyr Pro Tyr Phe Ser Phe
    50                  55                  60
Ile Gln Ala Pro Tyr Ala Met Gly Lys Glu His Leu Arg Leu Leu Glu
65                  70                  75                  80
Gln Ser Val Thr Val Pro Ser Ala Ala Thr Asp Ala Ile Ser Glu Ala
                85                  90                  95
Ala Lys Glu Ala Asn Met Val Val Ser Ile Gly Val Asn Glu Arg Asp
            100                 105                 110
Gly Gly Thr Ile Tyr Asn Thr Gln Leu Leu Phe Asp Ala Asp Gly Thr
        115                 120                 125
Leu Ile Gln Arg Arg Arg Lys Leu Thr Pro Thr Tyr His Glu Arg Met
    130                 135                 140
Ile Trp Gly Gln Gly Asp Ala Ser Gly Leu Arg Ala Thr Asp Ser Ala
145                 150                 155                 160
Val Gly Arg Ile Gly Gln Leu Ala Cys Trp Glu His Tyr Asn Pro Leu
                165                 170                 175
Phe Arg Tyr Ala Leu Ile Ala Asp Gly Glu Gln Ile His Ser Ala Met
            180                 185                 190
Tyr Pro Gly Ser Phe Leu Gly Ala Leu His Gly Glu Gln Thr Glu Ile
        195                 200                 205
Asn Val Arg Gln His Ala Leu Glu Ser Ala Ser Phe Val Val Val Ala
    210                 215                 220
Thr Gly Trp Leu Asp Ala Asp Gln Gln Ala Gln Ile Ala Lys Asp Thr
225                 230                 235                 240
Gly Gly Pro Ile Gly Pro Ile Ser Gly Gly Cys Phe Thr Ala Val Ile
                245                 250                 255
Gly Pro Asp Gly Gln Leu Ile Gly Glu Ala Leu Thr Ser Gly Glu Gly
            260                 265                 270
Glu Val Ile Ala Asp Ile Asp Leu Ala Gln Ile Asp Ala Arg Lys Arg
        275                 280                 285
Leu Met Asp Ala Ser Gly His Tyr Asn Arg Pro Glu Leu Leu Ser Leu

290                 295                 300
His Ile Asp His Thr Pro Thr Ala Pro Met His Glu Arg Val Val Tyr
305                 310                 315                 320
Thr Glu Pro Gly Leu Ala Lys Arg Gln Asn Glu Asn Ser Ser Asn
                325                 330                 335

<210> 295
<211> 1134
<212> DNA
<213> Unknown

<220>
<223> Obtained from an environmental sample

<400> 295
atggccgcaa aagtacttgg aggacgtgac acagtaaaag tagcagttgt tcaaacacca      60
tcagtcttta tggacaagaa agcctgtctc gaacttgcct gcgataagat tatcgaagcc     120
ggcaaagagg gcgcggagct tgttgttttt cctgaaacct ggattccgac atatccttat     180
tggaccatgg gatgggatac catcgcccat ggcttccatg atgtgatggc ggacctgcag     240
gacaattccg tggtcgtcgg gagcgaagac accgacatat tgggcaaagc cgcccgggaa     300
gctggcgcct acgtcgtcat gggctgtaat gagctcgatg accgggtcgg cagcaggacc     360
ttgttcaact cgctggtcta tatggacaaa tatggcggcg tgctcggccg tcaccgtaaa     420
ttaatcccgt cctttatcga acgcatctgg tggggcaatg gggacagccg cgatctcaaa     480
gtttacgaca cggaaattgg gcgcatcggc ggtcaaatct gctgggaaaa tcacattgtg     540
aacatcaccg catggtacat tgcccaaggc gtggatattc atgtctcggt ttggccggga     600
atgtggaact gtggcgcaga agaaggagaa tccttcatat acgccggtca cgacatcaac     660
aaatgtgacc tcatccctgc tacacgcgga cgggcattta cgggtcagtg ttatgtcctc     720
tcagccaaca acctactgcg gatggaagac attcctgacg atttcccgtt ccgggatacc     780
atgaactatg gcggtccggg ccaggaggat tttgtcggat gggcttgcgg tggcagccat     840
attgttgcgc caacgtctga atttatggtg ccgccgacgt ttgatataga caccatcatc     900
tatgcagaac ttcaggcgaa atacatcaaa gtggtgaagt cggtcttcga ttccctcggc     960
cactacgcgc ggtgggacct cgtcaacttg accacgccgc caccgccgta tgaacctgaa    1020
accgacgcac cagctctcac ggccgatatc cgtgatcggg tcatcgagag tgtggctaaa    1080
gagttcaagc tcgaaccaga aaaagtggct gaagttgtgc gcaatgccgc ctag          1134

<210> 296
<211> 377
<212> PRT
<213> Unknown

<220>
<223> Obtained from an environmental sample

<400> 296
Met Ala Ala Lys Val Leu Gly Gly Arg Asp Thr Val Lys Val Ala Val
1               5                   10                  15
Val Gln Thr Pro Ser Val Phe Met Asp Lys Lys Ala Cys Leu Glu Leu
            20                  25                  30
Ala Cys Asp Lys Ile Ile Glu Ala Gly Lys Glu Gly Ala Glu Leu Val
        35                  40                  45
Val Phe Pro Glu Thr Trp Ile Pro Thr Tyr Pro Tyr Trp Thr Met Gly
    50                  55                  60
Trp Asp Thr Ile Ala His Gly Phe His Asp Val Met Ala Asp Leu Gln
65                  70                  75                  80
Asp Asn Ser Val Val Val Gly Ser Glu Asp Thr Asp Ile Leu Gly Lys
                85                  90                  95
Ala Ala Arg Glu Ala Gly Ala Tyr Val Val Met Gly Cys Asn Glu Leu

                100                 105                 110
Asp Asp Arg Val Gly Ser Arg Thr Leu Phe Asn Ser Leu Val Tyr Met
        115                 120                 125
Asp Lys Tyr Gly Gly Val Leu Gly Arg His Arg Lys Leu Ile Pro Ser
    130                 135                 140
Phe Ile Glu Arg Ile Trp Trp Gly Asn Gly Asp Ser Arg Asp Leu Lys
145                 150                 155                 160
Val Tyr Asp Thr Glu Ile Gly Arg Ile Gly Gly Gln Ile Cys Trp Glu
                165                 170                 175
Asn His Ile Val Asn Ile Thr Ala Trp Tyr Ile Ala Gln Gly Val Asp
            180                 185                 190
Ile His Val Ser Val Trp Pro Gly Met Trp Asn Cys Gly Ala Glu Glu
        195                 200                 205
Gly Glu Ser Phe Ile Tyr Ala Gly His Asp Ile Asn Lys Cys Asp Leu
    210                 215                 220
Ile Pro Ala Thr Arg Gly Arg Ala Phe Thr Gly Gln Cys Tyr Val Leu
225                 230                 235                 240
Ser Ala Asn Asn Leu Leu Arg Met Glu Asp Ile Pro Asp Asp Phe Pro
                245                 250                 255
Phe Arg Asp Thr Met Asn Tyr Gly Gly Pro Gly Gln Glu Asp Phe Val
            260                 265                 270
Gly Trp Ala Cys Gly Gly Ser His Ile Val Ala Pro Thr Ser Glu Phe
        275                 280                 285
Met Val Pro Pro Thr Phe Asp Ile Asp Thr Ile Ile Tyr Ala Glu Leu
    290                 295                 300
Gln Ala Lys Tyr Ile Lys Val Val Lys Ser Val Phe Asp Ser Leu Gly
305                 310                 315                 320
His Tyr Ala Arg Trp Asp Leu Val Asn Leu Thr Thr Pro Pro Pro Pro
                325                 330                 335
Tyr Glu Pro Glu Thr Asp Ala Pro Ala Leu Thr Ala Asp Ile Arg Asp
            340                 345                 350
Arg Val Ile Glu Ser Val Ala Lys Glu Phe Lys Leu Glu Pro Glu Lys
        355                 360                 365
Val Ala Glu Val Val Arg Asn Ala Ala
    370                 375

<210> 297
<211> 1059
<212> DNA
<213> Unknown

<220>
<223> Obtained from an environmental sample

<400> 297
atgacacgtt ttcgggacgt cacggtggcg gcggttcagg ccgcacccgt ctatttcgat      60
cgggaggcct ccacagataa ggcgtgccaa ttgattcacg aagcggcgaa gaaaggcgca     120
gccctcgcgg cgttcggcga aacgtggttg ccgggatatc cgttctttgc atgggggttc     180
gcgcacaacc ggagcctgtt ctggaatgcg gccgccgagt acatcgccaa tgctgtggag     240
attccgagtc caacgacgga ccgcctctgc gccgcagcga agatcgccgg gattgacgtg     300
gtaatcggcg tcgtagaact ggatggacga acgcgagcgt cggtttacag cacactgctg     360
ttcatcggga gagagggggc gatcctgggg cgccaccgca aattgaagcc aacccacatg     420
gagcgaacgg tgtggggtga aggggacgct cacgggctcc gcgttcacga gcgtccgtac     480
ggccgcctca gcgggctgaa ttgctgggaa cacaacatga tgctgcccgg ctatgtgctt     540
gccgcgcagg gcacgcagtt tcacgtcgcg acatggcctg ggaaagagag gctcacagtt     600
ccgccgaacg aggcggctta tacgcgccag cttctcctct ctcgcgccta tgcatcccag     660
gcgggcgcgt acgtgatcag cgtcgcgggg ctgctcggac ccgactcgat gccggagcgt     720

tatcgcgaac tgggacagtc ctatgagttg accggcgaca gcgtcatcat cgatccgcgc 780
ggcgaggtca tcgcggggcc cgcgaagggc gagaccatcc tgctcgcgca atgcagccag 840
gaagccctct tcgccgcaaa gtccgccatt gacgtcggcg gtcattactc gcgcccggat 900
atttttcagc tgcgtgtcaa cgatcagcta cagcatcagg tccggagact cgaggcgact 960
ctcacgcccc cagtcgccgt agttgtcggg cctgagggta gctcacatga gcaggagacg 1020
gccttcgggc catccagcct cctggccacg acaagctag 1059

<210> 298
<211> 352
<212> PRT
<213> Unknown

<220>
<223> Obtained from an environmental sample

<400> 298
Met Thr Arg Phe Arg Asp Val Thr Val Ala Ala Val Gln Ala Ala Pro
1               5                   10                  15
Val Tyr Phe Asp Arg Glu Ala Ser Thr Asp Lys Ala Cys Gln Leu Ile
            20                  25                  30
His Glu Ala Ala Lys Lys Gly Ala Ala Leu Ala Ala Phe Gly Glu Thr
        35                  40                  45
Trp Leu Pro Gly Tyr Pro Phe Phe Ala Trp Gly Phe Ala His Asn Arg
    50                  55                  60
Ser Leu Phe Trp Asn Ala Ala Ala Glu Tyr Ile Ala Asn Ala Val Glu
65                  70                  75                  80
Ile Pro Ser Pro Thr Thr Asp Arg Leu Cys Ala Ala Ala Lys Ile Ala
                85                  90                  95
Gly Ile Asp Val Val Ile Gly Val Val Glu Leu Asp Gly Arg Thr Arg
            100                 105                 110
Ala Ser Val Tyr Ser Thr Leu Leu Phe Ile Gly Arg Glu Gly Ala Ile
        115                 120                 125
Leu Gly Arg His Arg Lys Leu Lys Pro Thr His Met Glu Arg Thr Val
    130                 135                 140
Trp Gly Glu Gly Asp Ala His Gly Leu Arg Val His Glu Arg Pro Tyr
145                 150                 155                 160
Gly Arg Leu Ser Gly Leu Asn Cys Trp Glu His Asn Met Met Leu Pro
                165                 170                 175
Gly Tyr Val Leu Ala Ala Gln Gly Thr Gln Phe His Val Ala Thr Trp
            180                 185                 190
Pro Gly Lys Glu Arg Leu Thr Val Pro Pro Asn Glu Ala Ala Tyr Thr
        195                 200                 205
Arg Gln Leu Leu Leu Ser Arg Ala Tyr Ala Ser Gln Ala Gly Ala Tyr
    210                 215                 220
Val Ile Ser Val Ala Gly Leu Leu Gly Pro Asp Ser Met Pro Glu Arg
225                 230                 235                 240
Tyr Arg Glu Leu Gly Gln Ser Tyr Glu Leu Thr Gly Asp Ser Val Ile
                245                 250                 255
Ile Asp Pro Arg Gly Glu Val Ile Ala Gly Pro Ala Lys Gly Glu Thr
            260                 265                 270
Ile Leu Leu Ala Gln Cys Ser Gln Glu Ala Leu Phe Ala Ala Lys Ser
        275                 280                 285
Ala Ile Asp Val Gly Gly His Tyr Ser Arg Pro Asp Ile Phe Gln Leu
    290                 295                 300
Arg Val Asn Asp Gln Leu Gln His Gln Val Arg Arg Leu Glu Ala Thr
305                 310                 315                 320
Leu Thr Pro Pro Val Ala Val Val Val Gly Pro Glu Gly Ser Ser His

                325                 330                 335
Glu Gln Glu Thr Ala Phe Gly Pro Ser Ser Leu Leu Ala Thr Thr Ser
            340                 345                 350

<210> 299
<211> 987
<212> DNA
<213> Unknown

<220>
<223> Obtained from an environmental sample

<400> 299
atgactgttg ttaaggccgc cgcagtgcag atcagcccgg tgctctacag ccgtgcagga 60
accgtcgaga aggtcgtgaa gaagatcgac gagctgggcc agaagggtgt cgagtttgcc 120
gtcttccctg aaaccgttgt cccctactac ccctacttct ccttcgtgca gcccccctac 180
aaactggcca cggagcacct gcgcctgctt gaggagtcgg tgaccgtgcc ctctgccgag 240
acggacgcca tcggcgacgc cgcccgcaag gccaacatgg tcgtctcgat cggtgtcaac 300
gaacgtgatg gcggcaccat ttacaacacc caactcctgt tcgacgccga cggaaccctg 360
atccagcgcc gccgcaagat cacgccgacc taccacgagc ggatgatctg gggacaggga 420
gacggatcag gcttgcgcgc ggtcgacagc gtcgtcggcc gcatcggcca gctcgcctgc 480
tgggagcact accagccgct ggcccgttac gctctcatcg ccgacggcga gcagatccac 540
gccgcgatgt accccggcgc cttcgggggc gatctgttcg cagagcagat cgaagtcaat 600
gtccgtcagc acgctctgga atcggccagt ttcgtcgtca gcgccaccgc ctggctcgac 660
gccgaccagc aggcccagat tgcgaaggac accggcggcc ccgtacaggc gatctccggc 720
ggcttcttca cagccatcat cgaccccgac ggccgcatca tcggcgaacc gatcacctcc 780
ggcgaaggcg aagtcatcgc tgacctcgac tttgcgctca tcgaccgccg caagcgcctg 840
atggacgcca gcggccacta cagccgcccc gaactgctca gcctgcagat cgaccggacg 900
ccggcacccg ccgtccacga tcgcaatcgc caggggtcct caagcgctcc ggcaactgaa 960
aagggccgct cagccgaggc caagtga 987

<210> 300
<211> 328
<212> PRT
<213> Unknown

<220>
<223> Obtained from an environmental sample

<400> 300
Met Thr Val Val Lys Ala Ala Ala Val Gln Ile Ser Pro Val Leu Tyr
1               5                   10                  15
Ser Arg Ala Gly Thr Val Glu Lys Val Val Lys Lys Ile Asp Glu Leu
            20                  25                  30
Gly Gln Lys Gly Val Glu Phe Ala Val Phe Pro Glu Thr Val Val Pro
        35                  40                  45
Tyr Tyr Pro Tyr Phe Ser Phe Val Gln Pro Pro Tyr Lys Leu Ala Thr
    50                  55                  60
Glu His Leu Arg Leu Leu Glu Glu Ser Val Thr Val Pro Ser Ala Glu
65                  70                  75                  80
Thr Asp Ala Ile Gly Asp Ala Ala Arg Lys Ala Asn Met Val Val Ser
                85                  90                  95
Ile Gly Val Asn Glu Arg Asp Gly Gly Thr Ile Tyr Asn Thr Gln Leu
            100                 105                 110
Leu Phe Asp Ala Asp Gly Thr Leu Ile Gln Arg Arg Arg Lys Ile Thr
        115                 120                 125
Pro Thr Tyr His Glu Arg Met Ile Trp Gly Gln Gly Asp Gly Ser Gly

130                 135                 140
Leu Arg Ala Val Asp Ser Val Val Gly Arg Ile Gly Gln Leu Ala Cys
145                 150                 155                 160
Trp Glu His Tyr Gln Pro Leu Ala Arg Tyr Ala Leu Ile Ala Asp Gly
                165                 170                 175
Glu Gln Ile His Ala Ala Met Tyr Pro Gly Ala Phe Gly Gly Asp Leu
            180                 185                 190
Phe Ala Glu Gln Ile Glu Val Asn Val Arg Gln His Ala Leu Glu Ser
        195                 200                 205
Ala Ser Phe Val Val Ser Ala Thr Ala Trp Leu Asp Ala Asp Gln Gln
    210                 215                 220
Ala Gln Ile Ala Lys Asp Thr Gly Gly Pro Val Gln Ala Ile Ser Gly
225                 230                 235                 240
Gly Phe Phe Thr Ala Ile Ile Asp Pro Asp Gly Arg Ile Ile Gly Glu
                245                 250                 255
Pro Ile Thr Ser Gly Glu Gly Glu Val Ile Ala Asp Leu Asp Phe Ala
            260                 265                 270
Leu Ile Asp Arg Arg Lys Arg Leu Met Asp Ala Ser Gly His Tyr Ser
        275                 280                 285
Arg Pro Glu Leu Leu Ser Leu Gln Ile Asp Arg Thr Pro Ala Pro Ala
    290                 295                 300
Val His Asp Arg Asn Arg Gln Gly Ser Ser Ser Ala Pro Ala Thr Glu
305                 310                 315                 320
Lys Gly Arg Ser Ala Glu Ala Lys
                325

<210> 301
<211> 1032
<212> DNA
<213> Unknown

<220>
<223> Obtained from an environmental sample

<400> 301
atgaccacgc ccgcgccgtt caccgccgcc gtcgtccagg cgtcgcccgt gttcctcgac      60
cgcgatgcca cggtcgataa ggcgtgcgcc ctgatcaccg cggccggagc gcgcggcgcg     120
aagctcgtcg tgctgcccga gaccttcgtg cccgcatacc ccgcgtgggt ctggtacctg     180
ccgctcacgc gtcggcccga cgtcgcgaag ctctaccgcg cgctcgtcga gaacgcgatc     240
gacgtgccgg gtccggaaac cgagcgcctc gggcgcgcgg cgcgtgacgc gggcgcgtgg     300
gtggcgatcg gtgtcaacga gcgcaacgcg aaggcgagcc gcacgtcgct ctacaacacc     360
gtgctcctgt tcgacgacca gggcacgctg gtcgagtcgc ggcgcaagct gatgccgacc     420
ggcggcgagc gcctggtgtg gacgccgggc gagccggtgc cgttgcgggt tcacgacacg     480
ccgctcggcc gcgtcggcgc gctgatctgc tgggagaact acatgccgct cgcgcgcttc     540
gcgctctacg agcagggcgt ccagatctat ctcgcgccga cctgggacta cagcgaggcg     600
tggctcgcgt ccatgcggca cgtcgcgcgt gaaggtcgca cgtgggtgat cgggtgcagc     660
caggcggtgc ggcgtgacga gatcccggac cacctgccgt tcaaggacgc gatccccgag     720
agcctcgagt ggatcaacgc cgggaacagc atcgtcgtcg atccggacgg cgcggtggtc     780
gcgggaccgc tggcgcgggc gcacgacacg ctctacgtcg agatcgatcc gggccgcgcc     840
gccggctccc gatggatctt cgacgccgcc ggtcactatc accgtcccga cctgttccat     900
ttcgcgatgc gcgcgccggt ggcgccagag accgcgccgg ttgtgcggaa gcgcggccgg     960
cggctagcct cggccccggc gcggaagcca cggaaacggc cggcgcgtcc cacccgaagg    1020
aggactcgat ga                                                        1032

<210> 302
<211> 335
<212> PRT

<213> Unknown

<220>
<223> Obtained from an environmental sample

<400> 302
Met Thr Thr Pro Ala Pro Phe Thr Ala Ala Val Val Gln Ala Ser Pro
1               5                   10                  15
Val Phe Leu Asp Arg Asp Ala Thr Val Asp Lys Ala Cys Ala Leu Ile
            20                  25                  30
Thr Ala Ala Gly Ala Arg Gly Ala Lys Leu Val Val Leu Pro Glu Thr
        35                  40                  45
Phe Val Pro Ala Tyr Pro Ala Trp Val Trp Tyr Leu Pro Leu Thr Arg
    50                  55                  60
Arg Pro Asp Val Ala Lys Leu Tyr Arg Ala Leu Val Glu Asn Ala Ile
65                  70                  75                  80
Asp Val Pro Gly Pro Glu Thr Glu Arg Leu Gly Arg Ala Ala Arg Asp
                85                  90                  95
Ala Gly Ala Trp Val Ala Ile Gly Val Asn Glu Arg Asn Ala Lys Ala
            100                 105                 110
Ser Arg Thr Ser Leu Tyr Asn Thr Val Leu Leu Phe Asp Asp Gln Gly
        115                 120                 125
Thr Leu Val Glu Ser Arg Arg Lys Leu Met Pro Thr Gly Gly Glu Arg
    130                 135                 140
Leu Val Trp Thr Pro Gly Glu Pro Val Pro Leu Arg Val His Asp Thr
145                 150                 155                 160
Pro Leu Gly Arg Val Gly Ala Leu Ile Cys Trp Glu Asn Tyr Met Pro
                165                 170                 175
Leu Ala Arg Phe Ala Leu Tyr Glu Gln Gly Val Gln Ile Tyr Leu Ala
            180                 185                 190
Pro Thr Trp Asp Tyr Ser Glu Ala Trp Leu Ala Ser Met Arg His Val
        195                 200                 205
Ala Arg Glu Gly Arg Thr Trp Val Ile Gly Cys Ser Gln Ala Val Arg
    210                 215                 220
Arg Asp Glu Ile Pro Asp His Leu Pro Phe Lys Asp Ala Ile Pro Glu
225                 230                 235                 240
Ser Leu Glu Trp Ile Asn Ala Gly Asn Ser Ile Val Val Asp Pro Asp
                245                 250                 255
Gly Ala Val Val Ala Gly Pro Leu Ala Arg Ala His Asp Thr Leu Tyr
            260                 265                 270
Val Glu Ile Asp Pro Gly Arg Ala Ala Gly Ser Arg Trp Ile Phe Asp
        275                 280                 285
Ala Ala Gly His Tyr His Arg Pro Asp Leu Phe His Phe Ala Met Arg
    290                 295                 300
Ala Pro Val Ala Pro Glu Thr Ala Pro Val Val Arg Lys Arg Gly Arg
305                 310                 315                 320
Arg Leu Ala Ser Ala Pro Ala Arg Lys Pro Arg Lys Arg Pro Ala
                325                 330                 335

<210> 303
<211> 1011
<212> DNA
<213> Unknown

<220>
<223> Obtained from an environmental sample

<400> 303
atgggcatcg ttcaccccaa gtacaaagtc gctgtcgttc aggctgcccc tgtatggcta 60
gacctggagg ccactgttga taaatgcatt cagttgattg aagaagcagc cagcaagggc 120
tgcaagctca tcgccttccc tgagaccttc attcccggat acccttggta tatctggatg 180
ggaacgcctg cctggactat tcagcgcggc tttgtacagc gctattttga taattctctg 240
tcttatgaca gtccgcaagc ggagaagcta aggcaggcag tcaaaaaggc tgagatcacg 300
gccgtactag gtctttctga gcgcagcggc ggtagcttgt acattgcaca atggaccatt 360
ggccctgacg gagaaaccat acacaaacgc agaaaagtgc gtccaacgca tggtgagcgt 420
acggtatttg gcgacggtga cggtagtgat cttgcggtgc acgatacccc cctggggcgg 480
ctgggcgcgc ttgcgtgctg ggagaacata ctgtcactga acaagtatgc gatgtattca 540
cagaatgagc aggtgcacgt agccgcttgg cctagcttct cggtctacga gcctttcgcc 600
catgcattgg gttgggaggt caataacgca gtcagcaagg tttacgcggt agaaggcggc 660
tgttttgtat tggcgccttg cgcagtggtc tccgaggaaa tgatcgaagc actgtgcgat 720
acacccgata agcaccaact ggctcatgcg ggtggagggc atgctgtcat ttacggacca 780
gatggcagtc ctctggcaga taagttaccc gaaggagagg aggggctatt aattgcagaa 840
attgatctcg gtctcatcag cttggcgaaa aatgccatgg acccggtggg gcattactct 900
cgacctgacg tacatcgctt gctattgaat cgcaatccag caaagcgggt tgaggaattt 960
tctctgccca ttgatttggc agagacaact ccgccaatat taggcacgta g 1011

<210> 304
<211> 336
<212> PRT
<213> Unknown

<220>
<223> Obtained from an environmental sample

<400> 304
Met Gly Ile Val His Pro Lys Tyr Lys Val Ala Val Val Gln Ala Ala
1               5                   10                  15
Pro Val Trp Leu Asp Leu Glu Ala Thr Val Asp Lys Cys Ile Gln Leu
            20                  25                  30
Ile Glu Glu Ala Ala Ser Lys Gly Cys Lys Leu Ile Ala Phe Pro Glu
        35                  40                  45
Thr Phe Ile Pro Gly Tyr Pro Trp Tyr Ile Trp Met Gly Thr Pro Ala
    50                  55                  60
Trp Thr Ile Gln Arg Gly Phe Val Gln Arg Tyr Phe Asp Asn Ser Leu
65                  70                  75                  80
Ser Tyr Asp Ser Pro Gln Ala Glu Lys Leu Arg Gln Ala Val Lys Lys
                85                  90                  95
Ala Glu Ile Thr Ala Val Leu Gly Leu Ser Glu Arg Ser Gly Gly Ser
            100                 105                 110
Leu Tyr Ile Ala Gln Trp Thr Ile Gly Pro Asp Gly Glu Thr Ile His
        115                 120                 125
Lys Arg Arg Lys Val Arg Pro Thr His Gly Glu Arg Thr Val Phe Gly
    130                 135                 140
Asp Gly Asp Gly Ser Asp Leu Ala Val His Asp Thr Pro Leu Gly Arg
145                 150                 155                 160
Leu Gly Ala Leu Ala Cys Trp Glu Asn Ile Leu Ser Leu Asn Lys Tyr
                165                 170                 175
Ala Met Tyr Ser Gln Asn Glu Gln Val His Val Ala Ala Trp Pro Ser
            180                 185                 190
Phe Ser Val Tyr Glu Pro Phe Ala His Ala Leu Gly Trp Glu Val Asn
        195                 200                 205
Asn Ala Val Ser Lys Val Tyr Ala Val Glu Gly Gly Cys Phe Val Leu
    210                 215                 220
Ala Pro Cys Ala Val Val Ser Glu Glu Met Ile Glu Ala Leu Cys Asp

225                 230                 235                 240
Thr Pro Asp Lys His Gln Leu Ala His Ala Gly Gly Gly His Ala Val
                245                 250                 255
Ile Tyr Gly Pro Asp Gly Ser Pro Leu Ala Asp Lys Leu Pro Glu Gly
            260                 265                 270
Glu Glu Gly Leu Leu Ile Ala Glu Ile Asp Leu Gly Leu Ile Ser Leu
        275                 280                 285
Ala Lys Asn Ala Met Asp Pro Val Gly His Tyr Ser Arg Pro Asp Val
    290                 295                 300
His Arg Leu Leu Leu Asn Arg Asn Pro Ala Lys Arg Val Glu Glu Phe
305                 310                 315                 320
Ser Leu Pro Ile Asp Leu Ala Glu Thr Thr Pro Pro Ile Leu Gly Thr
                325                 330                 335

<210> 305
<211> 1068
<212> DNA
<213> Unknown

<220>
<223> Obtained from an environmental sample

<400> 305
atgacgatgc aacatcccaa gtttcgcgct gctgccgtgc aggcggcacc ggtcttcctt      60
gaccttgatg cgtcgataga taaagccatc gacctgatcg cgcaagccgc caaaggcggc     120
gcgcaattga ttgcctttcc ggaaacctgg ctgcccggct accccttttt catctggctc     180
gattcgccgg cctggggcat gcaattcatc cagcgctacc atgacaattc cctggtctac     240
ggcacaccgc aggccgagcg catcgcgcag gctgcgaaaa agcatcgcat catggtcgtc     300
atggggcaca gcgagcggga tcatggaagt ctgtacatcg ctcagtggat catcggtgcc     360
gatggggaaa cggttgcgac acgtcgtaag ctcaaaccga ctcatgccga gcgcaccttg     420
tttggggaag gcgatggcag tgacctgagc gtgttcgata caccgctggg aagggttggc     480
gcactatgct gctgggagca cctccagccc ctgtcgaaat acgcgctata cgcacagaat     540
gagcaggtcc acattgcttc ctggccaagc ttttccctgt atcgcggggg cgcctacgcg     600
ctcggcgccg aagtgaacaa tgcggccagc cagatttatg ctgtcgaagg ccagtgtttt     660
gtgatcgcgc cgtgcggggt cgtcacgaaa gaaatgctgg acgtgctgtg caccgacgaa     720
atgaaaaagc agttgttggt tgaaggcggc gggttcgcgc gaatttacgc gcccgatgga     780
cagatgatgc acgcgccgct ggcggaaaac gaagagggcc tggtgtatgc cgatctcgac     840
ctgggcatga tctcgctggc caaagtagtc gccgacccgg ccgggcatta tgcgcggccc     900
gacgtaaccc ggttactgct ggacaagact ccgggagacc gcgtcatgct ggcgagccgt     960
cgcggcaagg aggtcagccg cgctggtaac gacgagccgc aagtgctggt ctcgcgcaac    1020
gacacgctga cctcgccgaa gccagcgtct tcgcgcaaag caagctga                 1068

<210> 306
<211> 355
<212> PRT
<213> Unknown

<220>
<223> Obtained from an environmental sample

<400> 306
Met Thr Met Gln His Pro Lys Phe Arg Ala Ala Ala Val Gln Ala Ala
 1               5                  10                  15
Pro Val Phe Leu Asp Leu Asp Ala Ser Ile Asp Lys Ala Ile Asp Leu
            20                  25                  30
Ile Ala Gln Ala Ala Lys Gly Gly Ala Gln Leu Ile Ala Phe Pro Glu
        35                  40                  45

Thr Trp Leu Pro Gly Tyr Pro Phe Phe Ile Trp Leu Asp Ser Pro Ala
    50                  55                  60
Trp Gly Met Gln Phe Ile Gln Arg Tyr His Asp Asn Ser Leu Val Tyr
65                  70                  75                  80
Gly Thr Pro Gln Ala Glu Arg Ile Ala Gln Ala Ala Lys Lys His Arg
                85                  90                  95
Ile Met Val Val Met Gly His Ser Glu Arg Asp His Gly Ser Leu Tyr
            100                 105                 110
Ile Ala Gln Trp Ile Ile Gly Ala Asp Gly Glu Thr Val Ala Thr Arg
        115                 120                 125
Arg Lys Leu Lys Pro Thr His Ala Glu Arg Thr Leu Phe Gly Glu Gly
    130                 135                 140
Asp Gly Ser Asp Leu Ser Val Phe Asp Thr Pro Leu Gly Arg Val Gly
145                 150                 155                 160
Ala Leu Cys Cys Trp Glu His Leu Gln Pro Leu Ser Lys Tyr Ala Leu
                165                 170                 175
Tyr Ala Gln Asn Glu Gln Val His Ile Ala Ser Trp Pro Ser Phe Ser
            180                 185                 190
Leu Tyr Arg Gly Gly Ala Tyr Ala Leu Gly Ala Glu Val Asn Asn Ala
        195                 200                 205
Ala Ser Gln Ile Tyr Ala Val Glu Gly Gln Cys Phe Val Ile Ala Pro
    210                 215                 220
Cys Gly Val Val Thr Lys Glu Met Leu Asp Val Leu Cys Thr Asp Glu
225                 230                 235                 240
Met Lys Lys Gln Leu Leu Val Glu Gly Gly Gly Phe Ala Arg Ile Tyr
                245                 250                 255
Ala Pro Asp Gly Gln Met Met His Ala Pro Leu Ala Glu Asn Glu Glu
            260                 265                 270
Gly Leu Val Tyr Ala Asp Leu Asp Leu Gly Met Ile Ser Leu Ala Lys
        275                 280                 285
Val Val Ala Asp Pro Ala Gly His Tyr Ala Arg Pro Asp Val Thr Arg
    290                 295                 300
Leu Leu Leu Asp Lys Thr Pro Gly Asp Arg Val Met Leu Ala Ser Arg
305                 310                 315                 320
Arg Gly Lys Glu Val Ser Arg Ala Gly Asn Asp Glu Pro Gln Val Leu
                325                 330                 335
Val Ser Arg Asn Asp Thr Leu Thr Ser Pro Lys Pro Ala Ser Ser Arg
            340                 345                 350
Lys Ala Ser
        355

<210> 307
<211> 942
<212> DNA
<213> Unknown

<220>
<223> Obtained from an environmental sample

<400> 307
atggctgaac cggaatcctt tatcgtcgct gctgtacagg ctacaccgat ctttcttgac      60
cgccaggcaa cccttgagaa agcgtgcgac ctgattgccg aagctggcag caatggcgca     120
aaactcgttc tttttcccga agcctttatc cccacctatc ctgattggat atgggcggtg     180
acaggctcac aatctgcgct gctcgacgaa ctttatgtgg aactactgga aaactccgtg     240
accatccccg acgcgaccac tgagcaactt tgtgaagcag cacgtaacgc cggtctctac     300
gtcgtcatgg gagtgaatga gcgcaacgcc gaggcgagca acgccacact ctataacacc     360
ctgctctata ttgacgatca gggcaaaatt ctcggcaagc atcgcaaatt ggtcccgacc     420

gccctggagc gaatcgtctg gggctatggc gatggcagca cgcttgacgc ctttgaaacg 480
ccgctgggca agattggcgg gctgatctgt tgggaaaatt acatgccact ggcgcgccaa 540
acactttatg cctggggggt gcaaatttac ttggccgcaa cgtgggatcg cggcgaagtt 600
tggcaggcga ccatgcgcca tattgccagg gaaggcggcg tctatgtagt cgcctcctgt 660
attccatttc acatcaaaga cattcctgac cacatgcctg aaatccgcaa tctctatgca 720
ccggggacag actggatcaa cgtcggccaa agctgcatca tcaaccccag cggcgactat 780
attgcaggcc ctgtcgagtg tcgcgaggag attctttatg ccgaggtaaa tctgcgccag 840
agtgcggcgg caaaacgtat gttggatgtg gcgggccatt atggacgccc tgatgtcttt 900
cacctcaccg tcaaccgcac gcccaatccg catattcgat aa 942

<210> 308
<211> 313
<212> PRT
<213> Unknown

<220>
<223> Obtained from an environmental sample

<400> 308
Met Ala Glu Pro Glu Ser Phe Ile Val Ala Ala Val Gln Ala Thr Pro
1               5                   10                  15
Ile Phe Leu Asp Arg Gln Ala Thr Leu Glu Lys Ala Cys Asp Leu Ile
            20                  25                  30
Ala Glu Ala Gly Ser Asn Gly Ala Lys Leu Val Leu Phe Pro Glu Ala
        35                  40                  45
Phe Ile Pro Thr Tyr Pro Asp Trp Ile Trp Ala Val Thr Gly Ser Gln
    50                  55                  60
Ser Ala Leu Leu Asp Glu Leu Tyr Val Glu Leu Leu Glu Asn Ser Val
65                  70                  75                  80
Thr Ile Pro Asp Ala Thr Thr Glu Gln Leu Cys Glu Ala Ala Arg Asn
                85                  90                  95
Ala Gly Leu Tyr Val Val Met Gly Val Asn Glu Arg Asn Ala Glu Ala
            100                 105                 110
Ser Asn Ala Thr Leu Tyr Asn Thr Leu Leu Tyr Ile Asp Asp Gln Gly
        115                 120                 125
Lys Ile Leu Gly Lys His Arg Lys Leu Val Pro Thr Ala Leu Glu Arg
    130                 135                 140
Ile Val Trp Gly Tyr Gly Asp Gly Ser Thr Leu Asp Ala Phe Glu Thr
145                 150                 155                 160
Pro Leu Gly Lys Ile Gly Gly Leu Ile Cys Trp Glu Asn Tyr Met Pro
                165                 170                 175
Leu Ala Arg Gln Thr Leu Tyr Ala Trp Gly Val Gln Ile Tyr Leu Ala
            180                 185                 190
Ala Thr Trp Asp Arg Gly Glu Val Trp Gln Ala Thr Met Arg His Ile
        195                 200                 205
Ala Arg Glu Gly Gly Val Tyr Val Val Ala Ser Cys Ile Pro Phe His
    210                 215                 220
Ile Lys Asp Ile Pro Asp His Met Pro Glu Ile Arg Asn Leu Tyr Ala
225                 230                 235                 240
Pro Gly Thr Asp Trp Ile Asn Val Gly Gln Ser Cys Ile Ile Asn Pro
                245                 250                 255
Ser Gly Asp Tyr Ile Ala Gly Pro Val Glu Cys Arg Glu Glu Ile Leu
            260                 265                 270
Tyr Ala Glu Val Asn Leu Arg Gln Ser Ala Ala Ala Lys Arg Met Leu
        275                 280                 285
Asp Val Ala Gly His Tyr Gly Arg Pro Asp Val Phe His Leu Thr Val
    290                 295                 300

Asn Arg Thr Pro Asn Pro His Ile Arg
305                 310

<210> 309
<211> 951
<212> DNA
<213> Unknown

<220>
<223> Obtained from an environmental sample

<400> 309
ttgaccgact gtctcaaaat agccgccgcc caaatcactc cggtcttcct cgaccgcgtt      60
gcgaccacga agaaggtcgt cgaaaccatc gaaaaagcgg ccgccggcgg tgcgcggctg     120
gtcgcattcg gcgaagcgct gttgcccgcc tatccattgt ggctgacgcg caccgacgcc     180
gcgcggttca attccgacgt gcaaaaaaac ttgcacgcga tctatctcaa gcaatccgtc     240
tcgatagcag gcggtcacct atctccgatt tgcaaaatcg caagcgaacg caagattgcc     300
gtcatcctcg gcatcgccga gcgcgcgacc gaccggggcg accacaccat ttactgctcg     360
tgcgtgttca tcgatgccga cggccgaatc gcgtcggtcc atcgcaagct gatgccgaca     420
tacgaagaac gcctcagttg gggattcggc gacggtgcgg gactcgtcac gcatccggtc     480
gggccgttca cggtgggcgc gttgaactgc tgggaaaact ggatgcccct cgcgcgcacc     540
gcgctgtatg ccggcggaga agatttgcac gttgcgatct ggcccggcgg atcggtgctc     600
acggaagaca tcacgcgctt catcgcacgc gagtcgcgct cgttcgtcct gtccgtcagc     660
ggcatcattc gcgaaagcga catccccagc ggggtcccct atcgcgatga aatgtgtgcg     720
aaaggcgaaa ccatctacaa cggcggaagc tgcatcgccg gacccgacgg tcagtggatc     780
atcgcgcccg taaccgaccg tgaagagttg atcttcgcgg agatcgacca cgaacacgtc     840
cgccgcgagc ggcagaattt cgacccggcc gggcattacg cgcggcccga tgtgttgcaa     900
ataaccgttg atcgtcgacg acaaacagcg gcgaatttta ttgatgacta a              951

<210> 310
<211> 316
<212> PRT
<213> Unknown

<220>
<223> Obtained from an environmental sample

<400> 310
Leu Thr Asp Cys Leu Lys Ile Ala Ala Ala Gln Ile Thr Pro Val Phe
1               5                   10                  15
Leu Asp Arg Val Ala Thr Thr Lys Lys Val Val Glu Thr Ile Glu Lys
            20                  25                  30
Ala Ala Ala Gly Gly Ala Arg Leu Val Ala Phe Gly Glu Ala Leu Leu
        35                  40                  45
Pro Ala Tyr Pro Leu Trp Leu Thr Arg Thr Asp Ala Ala Arg Phe Asn
    50                  55                  60
Ser Asp Val Gln Lys Asn Leu His Ala Ile Tyr Leu Lys Gln Ser Val
65                  70                  75                  80
Ser Ile Ala Gly Gly His Leu Ser Pro Ile Cys Lys Ile Ala Ser Glu
                85                  90                  95
Arg Lys Ile Ala Val Ile Leu Gly Ile Ala Glu Arg Ala Thr Asp Arg
            100                 105                 110
Gly Asp His Thr Ile Tyr Cys Ser Cys Val Phe Ile Asp Ala Asp Gly
        115                 120                 125
Arg Ile Ala Ser Val His Arg Lys Leu Met Pro Thr Tyr Glu Glu Arg
    130                 135                 140
Leu Ser Trp Gly Phe Gly Asp Gly Ala Gly Leu Val Thr His Pro Val

145                 150                 155                 160
Gly Pro Phe Thr Val Gly Ala Leu Asn Cys Trp Glu Asn Trp Met Pro
                165                 170                 175
Leu Ala Arg Thr Ala Leu Tyr Ala Gly Gly Glu Asp Leu His Val Ala
            180                 185                 190
Ile Trp Pro Gly Gly Ser Val Leu Thr Glu Asp Ile Thr Arg Phe Ile
        195                 200                 205
Ala Arg Glu Ser Arg Ser Phe Val Leu Ser Val Ser Gly Ile Ile Arg
    210                 215                 220
Glu Ser Asp Ile Pro Ser Gly Val Pro Tyr Arg Asp Glu Met Cys Ala
225                 230                 235                 240
Lys Gly Glu Thr Ile Tyr Asn Gly Gly Ser Cys Ile Ala Gly Pro Asp
                245                 250                 255
Gly Gln Trp Ile Ile Ala Pro Val Thr Asp Arg Glu Glu Leu Ile Phe
            260                 265                 270
Ala Glu Ile Asp His Glu His Val Arg Arg Glu Arg Gln Asn Phe Asp
        275                 280                 285
Pro Ala Gly His Tyr Ala Arg Pro Asp Val Leu Gln Ile Thr Val Asp
    290                 295                 300
Arg Arg Arg Gln Thr Ala Ala Asn Phe Ile Asp Asp
305                 310                 315

<210> 311
<211> 1011
<212> DNA
<213> Unknown

<220>
<223> Obtained from an environmental sample

<400> 311
atgtcagaaa agcgaataat cagagcagct gcagttcaga tcacacctga atttgactca      60
gcagatggaa cagttaagaa ggtatgcaag gtaatcgatg aagcaggcgc aaagggtgta     120
caaattatag tattccccga aaccttcatt ccgtattacc catacttctc attcattacc     180
cccccagttt ctgctggcgc tgagcatttg aagctttatg aaaaaagtgt cgtgatacct     240
ggtcctgtca ctcaagcgat cgccgagaga gctagagtga atcaaatggt cgtcgtactc     300
ggtgtaaacg agagagataa cggtagcctc tataacactc aactgatctt cgataccaat     360
ggtgagttga tgttgaagag aagaaaaatc actcctacat atcatgagcg catgatctgg     420
ggacaaggtg atgcttcagg cttaaaagta gttgaaacga gcattgcccg ggtaggtgct     480
ctagcttgct gggaacatta caacccgctg gccagatatt ctctcatgac acagcatgaa     540
gaaattcact gcgcacaatt cccaggttct atggttggcc aaatatttgc cgaccaaatg     600
gatgtcacta tcagacatca cgcattggaa tctggctgtt tcgtcattaa tgccaccggc     660
tggctcacag acgagcaaat ccagtccatt acagatgacc caaaaatgca gaaagcatta     720
cgtggcggct gcaacacagc aatcatttct cccgaaggtg tgcacttaac agagccctta     780
cgtgaaggtg aaggcatttt gattgctgac ctggacatgt cactcatcac aaaacgaaaa     840
agaatgatgg attcagtagg tcattattca agacctgaac tattaagtct ggcgatcaat     900
gacaagccag caacaacaaa attttcaatg actgaggggt gtactcaaac tgagcaattt     960
cgaatcgcag aggagttgaa aaatgacgac aagcttagca ccggaaacta a             1011

<210> 312
<211> 336
<212> PRT
<213> Unknown

<220>
<223> Obtained from an environmental sample

<400> 312
Met Ser Glu Lys Arg Ile Ile Arg Ala Ala Ala Val Gln Ile Thr Pro
1               5                   10                  15
Glu Phe Asp Ser Ala Asp Gly Thr Val Lys Lys Val Cys Lys Val Ile
            20                  25                  30
Asp Glu Ala Gly Ala Lys Gly Val Gln Ile Ile Val Phe Pro Glu Thr
        35                  40                  45
Phe Ile Pro Tyr Tyr Pro Tyr Phe Ser Phe Ile Thr Pro Pro Val Ser
    50                  55                  60
Ala Gly Ala Glu His Leu Lys Leu Tyr Glu Lys Ser Val Val Ile Pro
65                  70                  75                  80
Gly Pro Val Thr Gln Ala Ile Ala Glu Arg Ala Arg Val Asn Gln Met
                85                  90                  95
Val Val Val Leu Gly Val Asn Glu Arg Asp Asn Gly Ser Leu Tyr Asn
            100                 105                 110
Thr Gln Leu Ile Phe Asp Thr Asn Gly Glu Leu Met Leu Lys Arg Arg
        115                 120                 125
Lys Ile Thr Pro Thr Tyr His Glu Arg Met Ile Trp Gly Gln Gly Asp
    130                 135                 140
Ala Ser Gly Leu Lys Val Val Glu Thr Ser Ile Ala Arg Val Gly Ala
145                 150                 155                 160
Leu Ala Cys Trp Glu His Tyr Asn Pro Leu Ala Arg Tyr Ser Leu Met
                165                 170                 175
Thr Gln His Glu Glu Ile His Cys Ala Gln Phe Pro Gly Ser Met Val
            180                 185                 190
Gly Gln Ile Phe Ala Asp Gln Met Asp Val Thr Ile Arg His His Ala
        195                 200                 205
Leu Glu Ser Gly Cys Phe Val Ile Asn Ala Thr Gly Trp Leu Thr Asp
    210                 215                 220
Glu Gln Ile Gln Ser Ile Thr Asp Asp Pro Lys Met Gln Lys Ala Leu
225                 230                 235                 240
Arg Gly Gly Cys Asn Thr Ala Ile Ile Ser Pro Glu Gly Val His Leu
                245                 250                 255
Thr Glu Pro Leu Arg Glu Gly Glu Gly Ile Leu Ile Ala Asp Leu Asp
            260                 265                 270
Met Ser Leu Ile Thr Lys Arg Lys Arg Met Met Asp Ser Val Gly His
        275                 280                 285
Tyr Ser Arg Pro Glu Leu Leu Ser Leu Ala Ile Asn Asp Lys Pro Ala
    290                 295                 300
Thr Thr Lys Phe Ser Met Thr Glu Gly Cys Thr Gln Thr Glu Gln Phe
305                 310                 315                 320
Arg Ile Ala Glu Glu Leu Lys Asn Asp Asp Lys Leu Ser Thr Gly Asn
                325                 330                 335

<210> 313
<211> 987
<212> DNA
<213> Unknown

<220>
<223> Obtained from an environmental sample

<400> 313
atggcggccg ttcaggctgc gccggttccg tttgatgctg aggcttcggt ggataaggcc      60
tgtcgcttaa ttcaagaagc tgcagccaaa ggcgcagata tagttgcttt cggtgaggca     120
tggctacccg gctaccccta ttttgcctgg ttaccccaag taacaccaga gtggtatagt     180
gcggctgccg attatcttgc cagctctgtt gatatccctg gcccgatcac cgataaactt     240

tgccaggctg cccgtcgtgc atcggttgaa ctcatcatgg gtgtggtaga acgcagcaag 300
tctcagggaa ccacctattg cacgcttctt tttattagca aggatggcga aataattggc 360
aagcaccgca agctgaagcc cacactcgcc gaacgaaccg tctggggtga aggcgatgcc 420
agcggactga gggttcacga tcggcctatt gccagaatca gtgggctctc ctgctgggag 480
aacaaaatga tgctgccagg ttacgcactg atggcgcagg gtacgcaggt gcatgtctcc 540
gcctggccag ggatcccaga ggattcaccc atggaggtgc ctgcacaccc ccgtcaaaag 600
ctgctttccc aagcctttgc actgcaaggc gggtgctatg ttatttctcc ctccattgtc 660
cttagggcag aagatgtgcc cgagaaacac gccgctctac tgatgggaga ccaagtgggc 720
ggtagctata tcattgaccc ctgcggaaaa gtgatcgccg aggccggtgc gggtgagact 780
atcctgattg ccaaaggcaa ccttgacctc gtaagggccg ccaaaatggc cagtgatgta 840
ggggggtctt attcacgccc ggatcttttg cagttgatga tcaataaccg accactcgaa 900
cagctgattg agttcagtgc tgaaggtgca ggtaggggga atctagtatc caactcaccc 960
gaggtgtcag aacaagaagg tgagtaa 987

<210> 314
<211> 328
<212> PRT
<213> Unknown

<220>
<223> Obtained from an environmental sample

<400> 314
Met Ala Ala Val Gln Ala Ala Pro Val Pro Phe Asp Ala Glu Ala Ser
1               5                   10                  15
Val Asp Lys Ala Cys Arg Leu Ile Gln Glu Ala Ala Ala Lys Gly Ala
            20                  25                  30
Asp Ile Val Ala Phe Gly Glu Ala Trp Leu Pro Gly Tyr Pro Tyr Phe
        35                  40                  45
Ala Trp Leu Pro Gln Val Thr Pro Glu Trp Tyr Ser Ala Ala Ala Asp
    50                  55                  60
Tyr Leu Ala Ser Ser Val Asp Ile Pro Gly Pro Ile Thr Asp Lys Leu
65                  70                  75                  80
Cys Gln Ala Ala Arg Arg Ala Ser Val Glu Leu Ile Met Gly Val Val
                85                  90                  95
Glu Arg Ser Lys Ser Gln Gly Thr Thr Tyr Cys Thr Leu Leu Phe Ile
            100                 105                 110
Ser Lys Asp Gly Glu Ile Ile Gly Lys His Arg Lys Leu Lys Pro Thr
        115                 120                 125
Leu Ala Glu Arg Thr Val Trp Gly Glu Gly Asp Ala Ser Gly Leu Arg
    130                 135                 140
Val His Asp Arg Pro Ile Ala Arg Ile Ser Gly Leu Ser Cys Trp Glu
145                 150                 155                 160
Asn Lys Met Met Leu Pro Gly Tyr Ala Leu Met Ala Gln Gly Thr Gln
                165                 170                 175
Val His Val Ser Ala Trp Pro Gly Ile Pro Glu Asp Ser Pro Met Glu
            180                 185                 190
Val Pro Ala His Pro Arg Gln Lys Leu Leu Ser Gln Ala Phe Ala Leu
        195                 200                 205
Gln Gly Gly Cys Tyr Val Ile Ser Pro Ser Ile Val Leu Arg Ala Glu
    210                 215                 220
Asp Val Pro Glu Lys His Ala Ala Leu Leu Met Gly Asp Gln Val Gly
225                 230                 235                 240
Gly Ser Tyr Ile Ile Asp Pro Cys Gly Lys Val Ile Ala Glu Ala Gly
                245                 250                 255
Ala Gly Glu Thr Ile Leu Ile Ala Lys Gly Asn Leu Asp Leu Val Arg
            260                 265                 270

Ala Ala Lys Met Ala Ser Asp Val Gly Gly Ser Tyr Ser Arg Pro Asp
        275                 280                 285
Leu Leu Gln Leu Met Ile Asn Asn Arg Pro Leu Glu Gln Leu Ile Glu
    290                 295                 300
Phe Ser Ala Glu Gly Ala Gly Arg Gly Asn Leu Val Ser Asn Ser Pro
305                 310                 315                 320
Glu Val Ser Glu Gln Glu Gly Glu
                325

<210> 315
<211> 960
<212> DNA
<213> Unknown

<220>
<223> Obtained from an environmental sample

<400> 315
atgacaatgc ttaagaccaa attccgggtt gctgcggtgc aggcagcgcc tgtattcctg      60
gatcgagagg ctacgctgga gaaagcctgc ggactgattg aggaggcggg tcgcaacggg     120
gccagcctgg tggtctttcc tgagtcgttc attccggcct atcccgattg ggtctgggct     180
gtgccggcgg gtgaagaagc tttacttaat gaactgtatg ctcaactgct ggccaacgcc     240
gttgaaattc ccagcccggc caccgaacgc ttgagccagg cagcgaaaaa ggctaaagtc     300
catgtggtta tgggcctgac cgaacgcaac agcgaggcca gcggcggcag cctctacaat     360
accttgctct atcttgaccc acagggccaa attctgggca aacatcgcaa gctggtgccc     420
accggcggcg agcggctggt ttgggcccag ggcgacggca gtaccctgca agtctacgag     480
acccccttgg gtaaactcag cggcttgatt tgctgggaaa attatatgcc gctggcccgc     540
tacgcgctct atgcctgggg tacgcaaatc tatatcgccg ccacctggga tcgaggcgag     600
ccgtggcttt cgacgctgcg gcatatcgcc aaagagggcc gggtgtttgt catcggctgt     660
ggcatggcct tgcgtaaggc tgatattccc gaccattttg aattcaagca gcgcttttat     720
caaaatgccg gcgagtggat caatggaggc gacagcgcca ttgtcaatcc tgagggtgaa     780
tttattgctg gacccctgag cgagcaggaa ggtattttgt acgccgagat tgatcctggc     840
cagatggccg gaccaaaatg gatgctcgat gtggccgggc actatgctcg cccggatgtc     900
tttgaactga ccgtccagac cgtagctcgg cccatgatta cctctactca gccgcgatag     960

<210> 316
<211> 319
<212> PRT
<213> Unknown

<220>
<223> Obtained from an environmental sample

<400> 316
Met Thr Met Leu Lys Thr Lys Phe Arg Val Ala Ala Val Gln Ala Ala
 1               5                  10                  15
Pro Val Phe Leu Asp Arg Glu Ala Thr Leu Glu Lys Ala Cys Gly Leu
            20                  25                  30
Ile Glu Glu Ala Gly Arg Asn Gly Ala Ser Leu Val Val Phe Pro Glu
        35                  40                  45
Ser Phe Ile Pro Ala Tyr Pro Asp Trp Val Trp Ala Val Pro Ala Gly
    50                  55                  60
Glu Glu Ala Leu Leu Asn Glu Leu Tyr Ala Gln Leu Leu Ala Asn Ala
65                  70                  75                  80
Val Glu Ile Pro Ser Pro Ala Thr Glu Arg Leu Ser Gln Ala Ala Lys
                85                  90                  95
Lys Ala Lys Val His Val Val Met Gly Leu Thr Glu Arg Asn Ser Glu

                100                 105                 110
Ala Ser Gly Gly Ser Leu Tyr Asn Thr Leu Leu Tyr Leu Asp Pro Gln
        115                 120                 125
Gly Gln Ile Leu Gly Lys His Arg Lys Leu Val Pro Thr Gly Gly Glu
    130                 135                 140
Arg Leu Val Trp Ala Gln Gly Asp Gly Ser Thr Leu Gln Val Tyr Glu
145                 150                 155                 160
Thr Pro Leu Gly Lys Leu Ser Gly Leu Ile Cys Trp Glu Asn Tyr Met
                165                 170                 175
Pro Leu Ala Arg Tyr Ala Leu Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Lys Glu Gly Arg Val Phe Val Ile Gly Cys Gly Met Ala Leu
    210                 215                 220
Arg Lys Ala Asp Ile Pro Asp His Phe Glu Phe Lys Gln Arg Phe Tyr
225                 230                 235                 240
Gln Asn Ala Gly Glu Trp Ile Asn Gly Gly Asp Ser Ala Ile Val Asn
                245                 250                 255
Pro Glu Gly Glu Phe Ile Ala Gly Pro Leu Ser Glu Gln Glu Gly Ile
            260                 265                 270
Leu Tyr Ala Glu Ile Asp Pro Gly Gln Met Ala Gly Pro Lys Trp Met
        275                 280                 285
Leu Asp Val Ala Gly His Tyr Ala Arg Pro Asp Val Phe Glu Leu Thr
    290                 295                 300
Val Gln Thr Val Ala Arg Pro Met Ile Thr Ser Thr Gln Pro Arg
305                 310                 315

<210> 317
<211> 993
<212> DNA
<213> Unknown

<220>
<223> Obtained from an environmental sample

<400> 317
atgaccattg taaaagccgc tgccgtccaa ataagccctg tcctttacag ccgcgaaggc      60
accgtggaca aggttgtcca gaagatcctc gaactcggca agcaaggcgt ccagttcgcc     120
actttcccgg agacggtggt cccctactac ccctacttct ccttcgtcca gtcgggctac     180
gccctcaagg tgggcaagga acatctgcgc ttgctcgaac agtcggtcac cgtgccatcg     240
gccaccacgc tcgccatcgg cgaagcctgc aagcaggcgg ggatggtggt gtccatcggc     300
gtcaacgaac gcgacggcag cacgatctac aacacgcagc tgctcttcga tgccgacggc     360
accttgattc agcgccgccg aaagatcagc ccgaccttcc atgaacgcat ggtctggggc     420
cagggcgacg gctccgggct gcgcgcggtc gacagcgcgg tcgggcgcat cggccagttg     480
gcgtgctggg agcactacaa cccgctggcc cgctacgcca tgatggccga cggcgagcag     540
atccactcgg cgatgtaccc cggttccttc gcaggcgacg ccttctccga acagatccag     600
gtcaacatcc gccagcacgc attggaagcc ggctgcttcg tcgtgaacgc caccgcgtgg     660
ctggacgccg atcagcaggc gcagatcatg caggacaccg gttgcgccat cggcccgatc     720
tccagtggtt gcttcaccgc catcgtttcg ccggacggcg tgttgctggg cgagcctctg     780
cggtccggtg agggcgaggt gattgccgat ctcgacttca cgctgatcga caagcgcaag     840
cagatgatgg attcacgcgg gcactatgcg cgcccggaat tgctcagcct gttgatcgac     900
cgcacggcga ccgcgcatgt gcatgagcgc agcgcgcatc cgaaggcgac tgccgagcag     960
gcggacggtc catccgccgt gaatgcgcag taa                                  993

<210> 318
<211> 330

<212> PRT
<213> Unknown

<220>
<223> Obtained from an environmental sample

<400> 318
Met Thr Ile Val Lys Ala Ala Ala Val Gln Ile Ser Pro Val Leu Tyr
1               5                   10                  15
Ser Arg Glu Gly Thr Val Asp Lys Val Val Gln Lys Ile Leu Glu Leu
            20                  25                  30
Gly Lys Gln Gly Val Gln Phe Ala Thr Phe Pro Glu Thr Val Val Pro
        35                  40                  45
Tyr Tyr Pro Tyr Phe Ser Phe Val Gln Ser Gly Tyr Ala Leu Lys Val
    50                  55                  60
Gly Lys Glu His Leu Arg Leu Leu Glu Gln Ser Val Thr Val Pro Ser
65                  70                  75                  80
Ala Thr Thr Leu Ala Ile Gly Glu Ala Cys Lys Gln Ala Gly Met Val
                85                  90                  95
Val Ser Ile Gly Val Asn Glu Arg Asp Gly Ser Thr Ile Tyr Asn Thr
            100                 105                 110
Gln Leu Leu Phe Asp Ala Asp Gly Thr Leu Ile Gln Arg Arg Arg Lys
        115                 120                 125
Ile Ser Pro Thr Phe His Glu Arg Met Val Trp Gly Gln Gly Asp Gly
    130                 135                 140
Ser Gly Leu Arg Ala Val Asp Ser Ala Val Gly Arg Ile Gly Gln Leu
145                 150                 155                 160
Ala Cys Trp Glu His Tyr Asn Pro Leu Ala Arg Tyr Ala Met Met Ala
                165                 170                 175
Asp Gly Glu Gln Ile His Ser Ala Met Tyr Pro Gly Ser Phe Ala Gly
            180                 185                 190
Asp Ala Phe Ser Glu Gln Ile Gln Val Asn Ile Arg Gln His Ala Leu
        195                 200                 205
Glu Ala Gly Cys Phe Val Val Asn Ala Thr Ala Trp Leu Asp Ala Asp
    210                 215                 220
Gln Gln Ala Gln Ile Met Gln Asp Thr Gly Cys Ala Ile Gly Pro Ile
225                 230                 235                 240
Ser Ser Gly Cys Phe Thr Ala Ile Val Ser Pro Asp Gly Val Leu Leu
                245                 250                 255
Gly Glu Pro Leu Arg Ser Gly Glu Gly Glu Val Ile Ala Asp Leu Asp
            260                 265                 270
Phe Thr Leu Ile Asp Lys Arg Lys Gln Met Met Asp Ser Arg Gly His
        275                 280                 285
Tyr Ala Arg Pro Glu Leu Leu Ser Leu Leu Ile Asp Arg Thr Ala Thr
    290                 295                 300
Ala His Val His Glu Arg Ser Ala His Pro Lys Ala Thr Ala Glu Gln
305                 310                 315                 320
Ala Asp Gly Pro Ser Ala Val Asn Ala Gln
                325                 330

<210> 319
<211> 1017
<212> DNA
<213> Unknown

<220>
<223> Obtained from an environmental sample

<400> 319
atgacgaccc cgcgtatcgt ccgcgtcgcc gcggtgcaga tggcgcccga cctggaatcc 60
gcacacggca cggtagacaa ggtctgccgc gccattctgg aggccggcga aaaaggcgcg 120
cgcatggtgg tctttcccga gaccttcgtg ccctactacc cctacttctc cttcatccag 180
ccggcggtga cgatgggcgc cgcccacctc gagctctacg agcgcgcggt gacggtgccc 240
ggcccggtca ccctggccgt gggcgaagcg gcgcggcggg cgggcgcggt cgtcgtgctc 300
ggcgtcaatg aacgcgacca cggctcgctc tacaacaccc agctgatctt cgacgagacc 360
ggcgcgctgg tgctcaagcg ccgcaagctc accccgacct atcacgagcg catggtgtgg 420
ggccagggcg atggcagcgg tctcaaggtg gtggacaccg gcatcggccg catcggcgcg 480
ctggcctgct gggagcacta caacccgctc gcccgctaca ccctgatggc gcagcatgaa 540
gagatccacg ccgcgcagtt ccccggttcc atggtcggcc agatctttgc cgaccagatg 600
gcggtgacca tccgccacca cgcgctggaa tccggctgct tcgtggtgaa cgccaccggc 660
tggctcaccg atgcgcagat caccgccatc acccccgacc cggccatgca gcgcgcgctg 720
cgcggcggct gccacaccgc catcgtgtcg ccggaaggca gctacgtctg cgaaccgctc 780
accgagggcg aaggcatgct ggtggccgac ctcgacatgc ggctggtcac caagcgcaag 840
cggatgatgg attcggtcgg ccactacgcc cggccggagc tgctttcgct caacgccgat 900
cttgccccca agccggcgct gcacacacag ccggctgcgt ccctccctct ctcccttcag 960
gcaggagccg accatgtcga cgacgaccgc accgcgtccg caacagctga tctctga 1017

<210> 320
<211> 338
<212> PRT
<213> Unknown

<220>
<223> Obtained from an environmental sample

<400> 320
Met Thr Thr Pro Arg Ile Val Arg Val Ala Ala Val Gln Met Ala Pro
1               5                   10                  15
Asp Leu Glu Ser Ala His Gly Thr Val Asp Lys Val Cys Arg Ala Ile
            20                  25                  30
Leu Glu Ala Gly Glu Lys Gly Ala Arg Met Val Val Phe Pro Glu Thr
        35                  40                  45
Phe Val Pro Tyr Tyr Pro Tyr Phe Ser Phe Ile Gln Pro Ala Val Thr
    50                  55                  60
Met Gly Ala Ala His Leu Glu Leu Tyr Glu Arg Ala Val Thr Val Pro
65                  70                  75                  80
Gly Pro Val Thr Leu Ala Val Gly Glu Ala Ala Arg Arg Ala Gly Ala
                85                  90                  95
Val Val Val Leu Gly Val Asn Glu Arg Asp His Gly Ser Leu Tyr Asn
            100                 105                 110
Thr Gln Leu Ile Phe Asp Glu Thr Gly Ala Leu Val Leu Lys Arg Arg
        115                 120                 125
Lys Leu Thr Pro Thr Tyr His Glu Arg Met Val Trp Gly Gln Gly Asp
    130                 135                 140
Gly Ser Gly Leu Lys Val Val Asp Thr Gly Ile Gly Arg Ile Gly Ala
145                 150                 155                 160
Leu Ala Cys Trp Glu His Tyr Asn Pro Leu Ala Arg Tyr Thr Leu Met
                165                 170                 175
Ala Gln His Glu Glu Ile His Ala Ala Gln Phe Pro Gly Ser Met Val
            180                 185                 190
Gly Gln Ile Phe Ala Asp Gln Met Ala Val Thr Ile Arg His His Ala
        195                 200                 205
Leu Glu Ser Gly Cys Phe Val Val Asn Ala Thr Gly Trp Leu Thr Asp
    210                 215                 220

Ala Gln Ile Thr Ala Ile Thr Pro Asp Pro Ala Met Gln Arg Ala Leu
225                 230                 235                 240
Arg Gly Gly Cys His Thr Ala Ile Val Ser Pro Glu Gly Ser Tyr Val
                245                 250                 255
Cys Glu Pro Leu Thr Glu Gly Glu Gly Met Leu Val Ala Asp Leu Asp
            260                 265                 270
Met Arg Leu Val Thr Lys Arg Lys Arg Met Met Asp Ser Val Gly His
        275                 280                 285
Tyr Ala Arg Pro Glu Leu Leu Ser Leu Asn Ala Asp Leu Ala Pro Lys
    290                 295                 300
Pro Ala Leu His Thr Gln Pro Ala Ala Ser Leu Pro Leu Ser Leu Gln
305                 310                 315                 320
Ala Gly Ala Asp His Val Asp Asp Asp Arg Thr Ala Ser Ala Thr Ala
                325                 330                 335
Asp Leu


<210> 321
<211> 993
<212> DNA
<213> Unknown

<220>
<223> Obtained from an environmental sample

<400> 321
atgagagttg tcaaagccgc tgctgtccaa ctgagtcctg tcctccatag ccgcgacgga      60
acggtcgaaa aggtcgtgcg gaagatccat gaactcgccg aagagggagt cgagttcgcc     120
acctttcctg agaccgtggt gccttactac ccgtactttt ccttcgttca gacgcccttg     180
cagcaaatct tcggaactga gtatctgagg ctgctcgacc aggcagtcac cgtgccatcc     240
gctgccaccg acgcgatcgg cgaggctgcc aggtgggctg gacttgttgt ctcgatcggc     300
gtcaacgagc gagacggggg aactctctac aacactcagc ttctcttcga tgccgacggc     360
agcttaattc agcggcgtcg caagatcaca cccacccatt acgagcgcat gatctggggc     420
cagggcgacg gctcaggtct gcgggccgtt gatagcaagg ccggccgcat tggtcagctg     480
gcatgctggg aacacaacaa ccccctggcg cgctacgcgc tgatggccga cggcgagcag     540
atccattcgg ccatgtatcc gggctccatg ttcggcgact cgttttccca aaagaccgaa     600
atcaatatcc ggcagcatgc gctggaatct gcgtgcttcg tcgtgaacgc aacggcctgg     660
ctggacgccg atcagcaggc gcaaatcatg aaggacaccg gctgcggcat cggcccgatc     720
tccggcggtt gcttcactgc gatcgttgca cccgatggta gcctgctggg cgaacccatc     780
cgttccggtg agggcgtcgt cgtcgccaac ctcgacttca cgctgatcga caggcgtaag     840
caggtgatgg actcgcgagg ccactacagc cggccggagt tgctcagcct cttaatagac     900
cgcactccta ccgcgcacgt tcacgaacgc gctacgcacc ccacgacagg agctgagcaa     960
ggctccgagg atgtgttcga ggctcgcatt taa                                  993

<210> 322
<211> 330
<212> PRT
<213> Unknown

<220>
<223> Obtained from an environmental sample

<400> 322
Met Arg Val Val Lys Ala Ala Ala Val Gln Leu Ser Pro Val Leu His
1               5                   10                  15
Ser Arg Asp Gly Thr Val Glu Lys Val Val Arg Lys Ile His Glu Leu
            20                  25                  30

Ala Glu Glu Gly Val Glu Phe Ala Thr Phe Pro Glu Thr Val Val Pro
        35                  40                  45
Tyr Tyr Pro Tyr Phe Ser Phe Val Gln Thr Pro Leu Gln Gln Ile Phe
    50                  55                  60
Gly Thr Glu Tyr Leu Arg Leu Leu Asp Gln Ala Val Thr Val Pro Ser
65                  70                  75                  80
Ala Ala Thr Asp Ala Ile Gly Glu Ala Ala Arg Trp Ala Gly Leu Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Ser Leu Ile Gln Arg Arg Arg Lys
        115                 120                 125
Ile Thr Pro Thr His Tyr Glu Arg Met Ile Trp Gly Gln Gly Asp Gly
    130                 135                 140
Ser Gly Leu Arg Ala Val Asp Ser Lys Ala Gly Arg Ile Gly Gln Leu
145                 150                 155                 160
Ala Cys Trp Glu His Asn Asn Pro Leu Ala Arg Tyr Ala Leu Met Ala
                165                 170                 175
Asp Gly Glu Gln Ile His Ser Ala Met Tyr Pro Gly Ser Met Phe Gly
            180                 185                 190
Asp Ser Phe Ser Gln Lys Thr Glu Ile Asn Ile Arg Gln His Ala Leu
        195                 200                 205
Glu Ser Ala Cys Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Ser Gly Gly Cys Phe Thr Ala Ile Val Ala Pro Asp Gly Ser Leu Leu
                245                 250                 255
Gly Glu Pro Ile Arg Ser Gly Glu Gly Val Val Val Ala Asn Leu Asp
            260                 265                 270
Phe Thr Leu Ile Asp Arg Arg Lys Gln Val Met Asp Ser Arg Gly His
        275                 280                 285
Tyr Ser Arg Pro Glu Leu Leu Ser Leu Leu Ile Asp Arg Thr Pro Thr
    290                 295                 300
Ala His Val His Glu Arg Ala Thr His Pro Thr Thr Gly Ala Glu Gln
305                 310                 315                 320
Gly Ser Glu Asp Val Phe Glu Ala Arg Ile
                325                 330

<210> 323
<211> 951
<212> DNA
<213> Unknown

<220>
<223> Obtained from an environmental sample

<400> 323
atgagcaacg tgaaagtcgc ggtcgtgcaa cgcgcgccgg tgttcggcaa ccgcgccgcc 60
accctcgatc gcgccgtcgc cgcgctggcc gaagcggcgc agcagggggc gaagctggtc 120
gtgatgcccg agcacttcat ccccggctac ccggcctgga tctggcgcct gcgtccgggc 180
accgacctgc gattgtgcga acagctgcac gcgatgctgc gcgccaacgc cgtgaggctg 240
gatgacggtg acctggcccc gttgaccgag gccgcgcagc ggcatgcgct caccgtggtc 300
tgcggcgtct gcgagatcga caccgaattc agtcgcggca ccctgtacaa caccgtggtc 360
gtgatcgggc ccgacggcac gctgctcaac cggcatcgca agctgatgcc caccaacccc 420
gagcgcatgg tctggggcat gggcgacgcc acggggctga aggtggtcga cacgccctgc 480
gggcgcatcg gcacgctgat ttgctgggag aactacatgc cattcgcacg cgccgcgctg 540

tacgcgcagg gggtcgaggt cctggttgca ccgacctacg acgaaggccc ggtatggctg 600
gcgtcgatgc agcacatcgc ccgcgaaggc ggctgctggg tggtgggcaa cggctgcgca 660
ttccagggcc gcgacatgcc ggacaccttg ccgggcaagg cccagctgtt tcccgaggcc 720
gacgcctggg tcaacgccgg ggactcggtc atcgtcgcgc caggcggccg gacagtggcg 780
ggtccgttgc acgaggcgtt cgggctgttc accgccgaga tcgacctctc ccgggtcgga 840
atggcccggc gcagcctgga tgtggccggg cactatggac ggcccgacat cttctgcctg 900
caggtcaacg cccgggcgca gccgccggtt gaggtgacgc accatggctg a 951

<210> 324
<211> 316
<212> PRT
<213> Unknown

<220>
<223> Obtained from an environmental sample

<400> 324
Met Ser Asn Val Lys Val Ala Val Val Gln Arg Ala Pro Val Phe Gly
1               5                   10                  15
Asn Arg Ala Ala Thr Leu Asp Arg Ala Val Ala Ala Leu Ala Glu Ala
            20                  25                  30
Ala Gln Gln Gly Ala Lys Leu Val Val Met Pro Glu His Phe Ile Pro
        35                  40                  45
Gly Tyr Pro Ala Trp Ile Trp Arg Leu Arg Pro Gly Thr Asp Leu Arg
    50                  55                  60
Leu Cys Glu Gln Leu His Ala Met Leu Arg Ala Asn Ala Val Arg Leu
65                  70                  75                  80
Asp Asp Gly Asp Leu Ala Pro Leu Thr Glu Ala Ala Gln Arg His Ala
                85                  90                  95
Leu Thr Val Val Cys Gly Val Cys Glu Ile Asp Thr Glu Phe Ser Arg
            100                 105                 110
Gly Thr Leu Tyr Asn Thr Val Val Val Ile Gly Pro Asp Gly Thr Leu
        115                 120                 125
Leu Asn Arg His Arg Lys Leu Met Pro Thr Asn Pro Glu Arg Met Val
    130                 135                 140
Trp Gly Met Gly Asp Ala Thr Gly Leu Lys Val Val Asp Thr Pro Cys
145                 150                 155                 160
Gly Arg Ile Gly Thr Leu Ile Cys Trp Glu Asn Tyr Met Pro Phe Ala
                165                 170                 175
Arg Ala Ala Leu Tyr Ala Gln Gly Val Glu Val Leu Val Ala Pro Thr
            180                 185                 190
Tyr Asp Glu Gly Pro Val Trp Leu Ala Ser Met Gln His Ile Ala Arg
        195                 200                 205
Glu Gly Gly Cys Trp Val Val Gly Asn Gly Cys Ala Phe Gln Gly Arg
    210                 215                 220
Asp Met Pro Asp Thr Leu Pro Gly Lys Ala Gln Leu Phe Pro Glu Ala
225                 230                 235                 240
Asp Ala Trp Val Asn Ala Gly Asp Ser Val Ile Val Ala Pro Gly Gly
                245                 250                 255
Arg Thr Val Ala Gly Pro Leu His Glu Ala Phe Gly Leu Phe Thr Ala
            260                 265                 270
Glu Ile Asp Leu Ser Arg Val Gly Met Ala Arg Arg Ser Leu Asp Val
        275                 280                 285
Ala Gly His Tyr Gly Arg Pro Asp Ile Phe Cys Leu Gln Val Asn Ala
    290                 295                 300
Arg Ala Gln Pro Pro Val Glu Val Thr His His Gly
305                 310                 315

<210> 325
<211> 1077
<212> DNA
<213> Unknown

<220>
<223> Obtained from an environmental sample

<400> 325
atgactgcaa aaaagattgt ccgcgccgcc gctgtccagc tcaatcccgt gctggacagc 60
gccgacggca cccttgtgaa agtgttgcag gcgattgccg acgccgcagc gcagggcgtg 120
caactgatcg tctttcccga gacggtggtg ccctactatc cttacttttc cttcgtcacg 180
cctgcggttt cgatgggcgc ggcgcatctg aaactgtacg aacaatcgcc cacggtgcca 240
ggtccactga ccgacgccgt cgccgcagcc gcgcgggcac atcagatggt ggttgtgctc 300
ggcgtcaacg agcgcgatca cggcacgctc tacaacacgc aactgatctt cgacgccgac 360
ggcacgctcc cactgaagcg tcgcaagatc acgccgacct atcacgagcg catggtctgg 420
ggcatgggcg atggctccgg cctgcgcacg gtgaagaccg aggtcggaac cgttggcgcg 480
ctggcctgct gggaacacta caacccgctg gcacgctacg cgctgatggc gcagcacgaa 540
gagatccatt gcagccagtt ccccggctcg ctggtcggcc cgatcttttc cgagcagatg 600
gaaatcacca tgcgtcatca cgcgctggaa tccggctgct tcgtcgtcaa cgccactgca 660
tggctgacgc ctgagcaggt gcgatcacag gcgccaacac cggcaatgga aaaagccttc 720
tccggtggtt gctacaccgc gatcatttcg ccggaaggaa aacatctggg cgaacctctt 780
cgcgacggcg aaggcatggt catcgccgat cttgattttg atctcatcac caagcgcaag 840
cgaatgatgg attcggttgg ccactacgca cggccggaat tgttgagcct gcagctcgac 900
aaccgatcaa ctgcaccgct gacaacgtcg ccggtggccg ccgcagcgcc gtcgcttgca 960
gagatggaag cacagcgcct gtcacgttat ctcgatgcca gctccggcag cgccgcacaa 1020
ggcatcgaag ccgcctacat caatgccctc agctcttttt caggaaaacc ctcatga 1077

<210> 326
<211> 358
<212> PRT
<213> Unknown

<220>
<223> Obtained from an environmental sample

<400> 326
   Met Thr Ala Lys Lys Ile Val Arg Ala Ala Ala Val Gln Leu Asn Pro
   1               5                   10                  15
   Val Leu Asp Ser Ala Asp Gly Thr Leu Val Lys Val Leu Gln Ala Ile
               20                  25                  30
   Ala Asp Ala Ala Ala Gln Gly Val Gln Leu Ile Val Phe Pro Glu Thr
           35                  40                  45
   Val Val Pro Tyr Tyr Pro Tyr Phe Ser Phe Val Thr Pro Ala Val Ser
       50                  55                  60
   Met Gly Ala Ala His Leu Lys Leu Tyr Glu Gln Ser Pro Thr Val Pro
   65                  70                  75                  80
   Gly Pro Leu Thr Asp Ala Val Ala Ala Ala Ala Arg Ala His Gln Met
                   85                  90                  95
   Val Val Val Leu Gly Val Asn Glu Arg Asp His Gly Thr Leu Tyr Asn
               100                 105                 110
   Thr Gln Leu Ile Phe Asp Ala Asp Gly Thr Leu Pro Leu Lys Arg Arg
           115                 120                 125
   Lys Ile Thr Pro Thr Tyr His Glu Arg Met Val Trp Gly Met Gly Asp
       130                 135                 140
   Gly Ser Gly Leu Arg Thr Val Lys Thr Glu Val Gly Thr Val Gly Ala
   145                 150                 155                 160
   Leu Ala Cys Trp Glu His Tyr Asn Pro Leu Ala Arg Tyr Ala Leu Met
                   165                 170                 175
   Ala Gln His Glu Glu Ile His Cys Ser Gln Phe Pro Gly Ser Leu Val
               180                 185                 190
   Gly Pro Ile Phe Ser Glu Gln Met Glu Ile Thr Met Arg His His Ala
           195                 200                 205
   Leu Glu Ser Gly Cys Phe Val Val Asn Ala Thr Ala Trp Leu Thr Pro
       210                 215                 220
   Glu Gln Val Arg Ser Gln Ala Pro Thr Pro Ala Met Glu Lys Ala Phe
   225                 230                 235                 240
   Ser Gly Gly Cys Tyr Thr Ala Ile Ile Ser Pro Glu Gly Lys His Leu
                   245                 250                 255
   Gly Glu Pro Leu Arg Asp Gly Glu Gly Met Val Ile Ala Asp Leu Asp
               260                 265                 270
   Phe Asp Leu Ile Thr Lys Arg Lys Arg Met Met Asp Ser Val Gly His
           275                 280                 285
   Tyr Ala Arg Pro Glu Leu Leu Ser Leu Gln Leu Asp Asn Arg Ser Thr
       290                 295                 300
   Ala Pro Leu Thr Thr Ser Pro Val Ala Ala Ala Ala Pro Ser Leu Ala
   305                 310                 315                 320
   Glu Met Glu Ala Gln Arg Leu Ser Arg Tyr Leu Asp Ala Ser Ser Gly
                   325                 330                 335
   Ser Ala Ala Gln Gly Ile Glu Ala Ala Tyr Ile Asn Ala Leu Ser Ser
               340                 345                 350
   Phe Ser Gly Lys Pro Ser
           355

<210> 327
<211> 975
<212> DNA
<213> Unknown

<220>
<223> Obtained from an environmental sample

<400> 327
atggttgatc aaatacttaa cgatcgttct gaattattaa cagttggttt ggcgcagatt      60
gctccaatct ggctgaaccg cgaaaaaaca cttgcgaaag tggttgagaa agtaaatcaa     120
gctgcaaagc aagattgtca tcttgttgct tttggtgaag ctttggtccc cggatatccc     180
ttttggattg aacttacaga tggcgcgcga ttcaactcca atgttcaaaa agaaattcac     240
gcgcactata tggatcaggc agtacagata gagaatggtc atttaaaagc gctttgtgaa     300
acatcggccg caaacaagat tgccgtgatc gttgggtgca ttgaacgcgc agccgatcgc     360
ggcgggcaca gcttatatgc ttcattggtt tttattaatc ctcaagggca aatcggatcg     420
gtgcatcgca aacttatgcc aacttatgaa gaacgtttaa cctggtcacc tggcgatgga     480
catggtttgc gaacgcatca actaggtgct ttcactgttg gcggcttgaa ttgttgggaa     540
aactggatgc cattgccacg cgccgcattg tacgcacaag gtgaagactt tcatgtcgca     600
atctggccgg gaagcattca caatacgcaa gatattacgc gctatattgc gaaggaatcc     660
agatcgtttg taatgtctac ttccgggttc atgcgtaaag aagactttcc atctgatacg     720
cctcacttag acaaaatact tgcaaactct ccgaatgtac ttgcaaatgg tggatcctgt     780
ctcgccggac cggacggtca gtggatcgtt gaaccatttg tgaatgagga aaaattagtt     840
gttgcgactg tgaaccacaa gcaggtccgt gaagaaagac aaaattttga tccggtcgga     900
cactattccc gtcctgatgt cacgcagttg attgtgaaca gacaaagaca atcaacaatc     960
aagctcaacg attaa                                                      975

<210> 328
<211> 324

<212> PRT
<213> Unknown

<220>
<223> Obtained from an environmental sample

<400> 328
Met Val Asp Gln Ile Leu Asn Asp Arg Ser Glu Leu Leu Thr Val Gly
1               5                   10                  15
Leu Ala Gln Ile Ala Pro Ile Trp Leu Asn Arg Glu Lys Thr Leu Ala
            20                  25                  30
Lys Val Val Glu Lys Val Asn Gln Ala Ala Lys Gln Asp Cys His Leu
        35                  40                  45
Val Ala Phe Gly Glu Ala Leu Val Pro Gly Tyr Pro Phe Trp Ile Glu
    50                  55                  60
Leu Thr Asp Gly Ala Arg Phe Asn Ser Asn Val Gln Lys Glu Ile His
65                  70                  75                  80
Ala His Tyr Met Asp Gln Ala Val Gln Ile Glu Asn Gly His Leu Lys
                85                  90                  95
Ala Leu Cys Glu Thr Ser Ala Ala Asn Lys Ile Ala Val Ile Val Gly
            100                 105                 110
Cys Ile Glu Arg Ala Ala Asp Arg Gly Gly His Ser Leu Tyr Ala Ser
        115                 120                 125
Leu Val Phe Ile Asn Pro Gln Gly Gln Ile Gly Ser Val His Arg Lys
    130                 135                 140
Leu Met Pro Thr Tyr Glu Glu Arg Leu Thr Trp Ser Pro Gly Asp Gly
145                 150                 155                 160
His Gly Leu Arg Thr His Gln Leu Gly Ala Phe Thr Val Gly Gly Leu
                165                 170                 175
Asn Cys Trp Glu Asn Trp Met Pro Leu Pro Arg Ala Ala Leu Tyr Ala
            180                 185                 190
Gln Gly Glu Asp Phe His Val Ala Ile Trp Pro Gly Ser Ile His Asn
        195                 200                 205
Thr Gln Asp Ile Thr Arg Tyr Ile Ala Lys Glu Ser Arg Ser Phe Val
    210                 215                 220
Met Ser Thr Ser Gly Phe Met Arg Lys Glu Asp Phe Pro Ser Asp Thr
225                 230                 235                 240
Pro His Leu Asp Lys Ile Leu Ala Asn Ser Pro Asn Val Leu Ala Asn
                245                 250                 255
Gly Gly Ser Cys Leu Ala Gly Pro Asp Gly Gln Trp Ile Val Glu Pro
            260                 265                 270
Phe Val Asn Glu Glu Lys Leu Val Val Ala Thr Val Asn His Lys Gln
        275                 280                 285
Val Arg Glu Glu Arg Gln Asn Phe Asp Pro Val Gly His Tyr Ser Arg
    290                 295                 300
Pro Asp Val Thr Gln Leu Ile Val Asn Arg Gln Arg Gln Ser Thr Ile
305                 310                 315                 320
Lys Leu Asn Asp


<210> 329
<211> 1023
<212> DNA
<213> Unknown

<220>
<223> Obtained from an environmental sample

<400> 329
gtgccggcca aagtacgcgc cgccgccgtg cagctcagcc ccgtgctctt cagccgtgaa      60
ggcacgacca gcaaggtctg cgacaagatt gccgaggcgg cggcgcaggg cgccgagctg     120
gtggtgtttc ccgaaaccgt agtgccgtat tacccgtatt tctcgttcat caaggctccg     180
gccgtgatcg gcgccgagca cttactcttg ctcgaacaag ccgtcacggt gcccgggccc     240
agcgtcgaag ccatcgcaga agccgctcgc aaggcgggcg cggtggtttc gatcggcgtc     300
aacgaacgcg atcacggcac gctgtacaac acccagctgt tgttcgacgc cgacggtcgg     360
ttggcgcaag cccgccgcaa gatcaccccc acgtatcacg agcggatgat ctgggggcag     420
ggcgatggct cgggcttggt ggcggtggat acgcgagtcg gcaggattgg ctccctggcg     480
tgctgggagc actacaaccc gctggctcgc tatgcgctga tggccgacca cgagcaaatt     540
cacgtggcca tgttccctgg ctcgctcgtg ggcgacatct tccgcgagca aatcgaggtc     600
acgattcggc accacgcgct cgagtcgggc tgcttcgtcg tcaacgcgac gggctacctg     660
agcgacgcgc aggtgacgca gatcgcgggc gacaccaagc tcgaccgcgc cctgcgcggc     720
ggttgcttca ccgccatcgt atcgcccgag ggcacgctgc tggcgccacc gctcaccgac     780
ggtgagggca tggtcattgc cgacctcgat ctgtcgctca tcgccaaacg caaacgcatg     840
atggacagcg tcggccatta cagccggccc gagctgctca gcgtgctgat cgaccgctcg     900
ccgcagccgc atttgcgcga gaaaactgcc tctttacccg aacctcggat gtcccatgaa     960
tcgctcgctc ccgatagtaa cggcctccgc gacgctgacg cgaaagcctc gaccctctcc    1020
tga                                                                  1023

<210> 330
<211> 340
<212> PRT
<213> Unknown

<220>
<223> Obtained from an environmental sample

<400> 330
Val Pro Ala Lys Val Arg Ala Ala Ala Val Gln Leu Ser Pro Val Leu
1               5                   10                  15
Phe Ser Arg Glu Gly Thr Thr Ser Lys Val Cys Asp Lys Ile Ala Glu
            20                  25                  30
Ala Ala Ala Gln Gly Ala Glu Leu Val Val Phe Pro Glu Thr Val Val
        35                  40                  45
Pro Tyr Tyr Pro Tyr Phe Ser Phe Ile Lys Ala Pro Ala Val Ile Gly
    50                  55                  60
Ala Glu His Leu Leu Leu Leu Glu Gln Ala Val Thr Val Pro Gly Pro
65                  70                  75                  80
Ser Val Glu Ala Ile Ala Glu Ala Ala Arg Lys Ala Gly Ala Val Val
                85                  90                  95
Ser Ile Gly Val Asn Glu Arg Asp His Gly Thr Leu Tyr Asn Thr Gln
            100                 105                 110
Leu Leu Phe Asp Ala Asp Gly Arg Leu Ala Gln Ala Arg Arg Lys Ile
        115                 120                 125
Thr Pro Thr Tyr His Glu Arg Met Ile Trp Gly Gln Gly Asp Gly Ser
    130                 135                 140
Gly Leu Val Ala Val Asp Thr Arg Val Gly Arg Ile Gly Ser Leu Ala
145                 150                 155                 160
Cys Trp Glu His Tyr Asn Pro Leu Ala Arg Tyr Ala Leu Met Ala Asp
                165                 170                 175
His Glu Gln Ile His Val Ala Met Phe Pro Gly Ser Leu Val Gly Asp
            180                 185                 190
Ile Phe Arg Glu Gln Ile Glu Val Thr Ile Arg His His Ala Leu Glu
        195                 200                 205
Ser Gly Cys Phe Val Val Asn Ala Thr Gly Tyr Leu Ser Asp Ala Gln

210                 215                 220
Val Thr Gln Ile Ala Gly Asp Thr Lys Leu Asp Arg Ala Leu Arg Gly
225                 230                 235                 240
Gly Cys Phe Thr Ala Ile Val Ser Pro Glu Gly Thr Leu Leu Ala Pro
                245                 250                 255
Pro Leu Thr Asp Gly Glu Gly Met Val Ile Ala Asp Leu Asp Leu Ser
            260                 265                 270
Leu Ile Ala Lys Arg Lys Arg Met Met Asp Ser Val Gly His Tyr Ser
        275                 280                 285
Arg Pro Glu Leu Leu Ser Val Leu Ile Asp Arg Ser Pro Gln Pro His
    290                 295                 300
Leu Arg Glu Lys Thr Ala Ser Leu Pro Glu Pro Arg Met Ser His Glu
305                 310                 315                 320
Ser Leu Ala Pro Asp Ser Asn Gly Leu Arg Asp Ala Asp Ala Lys Ala
                325                 330                 335
Ser Thr Leu Ser
            340

<210> 331
<211> 1041
<212> DNA
<213> Unknown

<220>
<223> Obtained from an environmental sample

<400> 331
atgtccggaa caggacctat cggaccggtc gtgaaggtcg ccgtcgtaca agccgcgcca      60
tgtctgctcg atcttgatgc gggcgtcgag aaggcgatcg ccttgatcga ccaagcgggc     120
aaggctggcg cgcggctcat caattttccc gaaatttggc tgccgggtta tccttggtgg     180
atctggctga atccacccgc catcaacatg cagtatgtcg cgccttacat gaacaactcc     240
attgtcgcag gcagcaagca tgaccacgca cttcgggccg ccgcgcgccg caacaatatt     300
cacgtcgtga tcggtgtctc cgagcgcgcc ggcggcagtc tgtacatggc tcagtggcac     360
tatgggcccg agggcgaggt gatctcgcgt cgtcgtaagc taaaacccac ccatgtcgaa     420
cgcagcgtct ttggcgaagg cgacggcagc gacatgatcg ttactcagac cgatttcggg     480
cgcgtcggcg cgctatgctg ttgggaacac ctgcaaccgc tgtcgaaata cgcgctcttt     540
tcccaggacg agcagattca ttgcgcggcg tggcccgctt tcagcctcta tgcaaaactc     600
tcgaaggcct tcagccccga agtcagcgtc aacgtgaacc aaatctacgc cgtagaaggg     660
caatgcttcg tcctgtcgtc gtgctcggtt atcgatcagg cgatctacga cacgttggtg     720
cagaacgaat tgcaccagaa gttcctcgag gtgggcgggg gctacagccg aatcttcggg     780
ccgaacggtg cggaattcgg tgagaatctc ccacccgata gggaaggcct ggtggttgcc     840
gatatcgatc tcggcctgat ctcgcactcc aagagtgccg ctgatccggc tggtcactac     900
gcgagggccg acgctctggc gctcatgcat aaccgtaatc cccgccgtcc ggttatcggt     960
ttcggcgagg cgacgcgcaa ggttgccgac gcactgccta aaggcgcgga acccgcggaa    1020
gcgctcgaag cggccgagtg a                                              1041

<210> 332
<211> 346
<212> PRT
<213> Unknown

<220>
<223> Obtained from an environmental sample

<400> 332
Met Ser Gly Thr Gly Pro Ile Gly Pro Val Val Lys Val Ala Val Val
1               5                   10                  15

Gln Ala Ala Pro Cys Leu Leu Asp Leu Asp Ala Gly Val Glu Lys Ala
            20                  25                  30
Ile Ala Leu Ile Asp Gln Ala Gly Lys Ala Gly Ala Arg Leu Ile Asn
        35                  40                  45
Phe Pro Glu Ile Trp Leu Pro Gly Tyr Pro Trp Trp Ile Trp Leu Asn
    50                  55                  60
Pro Pro Ala Ile Asn Met Gln Tyr Val Ala Pro Tyr Met Asn Asn Ser
65                  70                  75                  80
Ile Val Ala Gly Ser Lys His Asp His Ala Leu Arg Ala Ala Ala Arg
                85                  90                  95
Arg Asn Asn Ile His Val Val Ile Gly Val Ser Glu Arg Ala Gly Gly
            100                 105                 110
Ser Leu Tyr Met Ala Gln Trp His Tyr Gly Pro Glu Gly Glu Val Ile
        115                 120                 125
Ser Arg Arg Arg Lys Leu Lys Pro Thr His Val Glu Arg Ser Val Phe
    130                 135                 140
Gly Glu Gly Asp Gly Ser Asp Met Ile Val Thr Gln Thr Asp Phe Gly
145                 150                 155                 160
Arg Val Gly Ala Leu Cys Cys Trp Glu His Leu Gln Pro Leu Ser Lys
                165                 170                 175
Tyr Ala Leu Phe Ser Gln Asp Glu Gln Ile His Cys Ala Ala Trp Pro
            180                 185                 190
Ala Phe Ser Leu Tyr Ala Lys Leu Ser Lys Ala Phe Ser Pro Glu Val
        195                 200                 205
Ser Val Asn Val Asn Gln Ile Tyr Ala Val Glu Gly Gln Cys Phe Val
    210                 215                 220
Leu Ser Ser Cys Ser Val Ile Asp Gln Ala Ile Tyr Asp Thr Leu Val
225                 230                 235                 240
Gln Asn Glu Leu His Gln Lys Phe Leu Glu Val Gly Gly Gly Tyr Ser
                245                 250                 255
Arg Ile Phe Gly Pro Asn Gly Ala Glu Phe Gly Glu Asn Leu Pro Pro
            260                 265                 270
Asp Arg Glu Gly Leu Val Val Ala Asp Ile Asp Leu Gly Leu Ile Ser
        275                 280                 285
His Ser Lys Ser Ala Ala Asp Pro Ala Gly His Tyr Ala Arg Ala Asp
    290                 295                 300
Ala Leu Ala Leu Met His Asn Arg Asn Pro Arg Arg Pro Val Ile Gly
305                 310                 315                 320
Phe Gly Glu Ala Thr Arg Lys Val Ala Asp Ala Leu Pro Lys Gly Ala
                325                 330                 335
Glu Pro Ala Glu Ala Leu Glu Ala Ala Glu
            340                 345

<210> 333
<211> 1038
<212> DNA
<213> Unknown

<220>
<223> Obtained from an environmental sample

<400> 333
atggccattg aacatccccg ctatcgggtc gctgccgttc aggcggcgcc agagttcctt      60
aatctcgagg caaccgttga caagacgatt gcgcttatcg aggaggccgc ccgcggaggc     120
gcaagtctca ttgctttccc tgaaacttgg atacctggat atccatggtt tgcctggctt     180
ggtgcgccga tctggggcat gaaattcatc caggcgtacc atgacaactc gatggtgatc     240
gacggcgccc agtttgagcg tattgcgcaa gcagcatcgc gttgcaacat cactgttgtg     300

cttggcttta gcgagaagga cgcaggaagc ctgtatattg cccaggccat cctgagccct 360
gaagggaaga ccatcgccac gcgtcgcaag ctgaaaccca ctcatgtcga acgcgcgatc 420
ttcggcgaag gcgacggcag cgacctggca gttcacgaca ccaagctcgg cagggtgggc 480
gccctttgct gctgggagca tcttcagcca ctttccaaat acgcgatgta tgctcagaac 540
gagcaggtcc atatcgccgc ctggcccagt tttccctttt acgtcgacgc ggcttacgcg 600
cttgggccag aggtgaacaa cgccgcgagt cggttgtatg cggtcgaggg ccagtgcttc 660
gtggttgcgc cgtgtgcaac ggtttcgcaa aagatgatcg atatgctttg cgagacaccc 720
gagcaacaag cgctcttgaa gccggggggt ggtcacgcgc aaatctacgg tcccgacgga 780
cgatcgctgg ccgatccgct gcctccggac gcggaggggc tgttgtatgc agacattgac 840
cttgcagcca tcaccctcgc gaaagcagct gcagatcctg ctggccatta ctctcgccct 900
gacgtgacac aactgctgct tgaccgcaat ccaaagcccc gtgtcgtgca tgctaagccc 960
ggccaaagcg ccaacaacag ctcacccggc atgcgggccg tcgagcatac cgagctcgaa 1020
gaaggtgaac aggcctga 1038

<210> 334
<211> 345
<212> PRT
<213> Unknown

<220>
<223> Obtained from an environmental sample

<400> 334
Met Ala Ile Glu His Pro Arg Tyr Arg Val Ala Ala Val Gln Ala Ala
1               5                   10                  15
Pro Glu Phe Leu Asn Leu Glu Ala Thr Val Asp Lys Thr Ile Ala Leu
            20                  25                  30
Ile Glu Glu Ala Ala Arg Gly Gly Ala Ser Leu Ile Ala Phe Pro Glu
        35                  40                  45
Thr Trp Ile Pro Gly Tyr Pro Trp Phe Ala Trp Leu Gly Ala Pro Ile
    50                  55                  60
Trp Gly Met Lys Phe Ile Gln Ala Tyr His Asp Asn Ser Met Val Ile
65                  70                  75                  80
Asp Gly Ala Gln Phe Glu Arg Ile Ala Gln Ala Ala Ser Arg Cys Asn
                85                  90                  95
Ile Thr Val Val Leu Gly Phe Ser Glu Lys Asp Ala Gly Ser Leu Tyr
            100                 105                 110
Ile Ala Gln Ala Ile Leu Ser Pro Glu Gly Lys Thr Ile Ala Thr Arg
        115                 120                 125
Arg Lys Leu Lys Pro Thr His Val Glu Arg Ala Ile Phe Gly Glu Gly
    130                 135                 140
Asp Gly Ser Asp Leu Ala Val His Asp Thr Lys Leu Gly Arg Val Gly
145                 150                 155                 160
Ala Leu Cys Cys Trp Glu His Leu Gln Pro Leu Ser Lys Tyr Ala Met
                165                 170                 175
Tyr Ala Gln Asn Glu Gln Val His Ile Ala Ala Trp Pro Ser Phe Ser
            180                 185                 190
Leu Tyr Val Asp Ala Ala Tyr Ala Leu Gly Pro Glu Val Asn Asn Ala
        195                 200                 205
Ala Ser Arg Leu Tyr Ala Val Glu Gly Gln Cys Phe Val Val Ala Pro
    210                 215                 220
Cys Ala Thr Val Ser Gln Lys Met Ile Asp Met Leu Cys Glu Thr Pro
225                 230                 235                 240
Glu Gln Gln Ala Leu Leu Lys Pro Gly Gly Gly His Ala Gln Ile Tyr
                245                 250                 255
Gly Pro Asp Gly Arg Ser Leu Ala Asp Pro Leu Pro Pro Asp Ala Glu
            260                 265                 270

Gly Leu Leu Tyr Ala Asp Ile Asp Leu Ala Ala Ile Thr Leu Ala Lys
        275                 280                 285
Ala Ala Ala Asp Pro Ala Gly His Tyr Ser Arg Pro Asp Val Thr Gln
    290                 295                 300
Leu Leu Leu Asp Arg Asn Pro Lys Pro Arg Val Val His Ala Lys Pro
305                 310                 315                 320
Gly Gln Ser Ala Asn Asn Ser Ser Pro Gly Met Arg Ala Val Glu His
                325                 330                 335
Thr Glu Leu Glu Glu Gly Glu Gln Ala
            340                 345

<210> 335
<211> 1053
<212> DNA
<213> Unknown

<220>
<223> Obtained from an environmental sample

<400> 335
atgactacgc gcgtgattaa agtcgctgcc gcgcagctgt ctccggtgct ggcaactgca      60
tcggagcaca gccgcgagga cacgattgcg aaagtgatcg acgccatcgc tgcggcgtcg     120
caacagggcg cgcaactgat tgtgtttccg gaaacagtcg ttccgtatta cccgtatttt     180
tcatttatca cgcccgcggt aacgatgggt gccgaacatt tgaaattgta cgaacaggca     240
gtgacggtac ccagcgcagc gacagatgct gtcgctgcgg cggcaaaaaa ttatggcatg     300
gtagtggtgc tcggaattaa tgaacgcgat cacggctcgc tgtacaacgc gcaattaatt     360
ttcgacgccg atggtgagct gctattaaag cgtcgaaaaa ttacgccgac ttatcacgaa     420
cgcatggtgt ggggacaagg cgacggcagc ggtttaaaag ttgtcgatac tgctgccggc     480
cgtgtcggcg cgctcgcgtg ctgggaacat tacaacccgc tcgcgcgtta cagcctgatg     540
gcacaacacg aagaaattca ttgcagtcaa tttccgggat cgttggtcgg ccctattttc     600
gccgaacaaa tggaaatcac catgcgtcat cacgcactcg aatccggctg ttttgtggtg     660
aatgcaacgg cctggcttag cgatacacaa atccaatcaa ttacccccga taaagccatg     720
cagaaagcac tgcgcggcgg ttgctacacc gcaatcatct cgcccgaagg caaacatctg     780
tgcccaccgc tgtatgacgg agaaggaata attgtggcgg aattggactt cgcgttaatc     840
accaaacgta aacgcatgat ggattccgtc ggccattacg cgcgaccaga actactttct     900
ttgctcctcg atgatcgcgt aactgcgccg ctcaaaaact tacagacgac gatggcctct     960
gccaaatccg ctgaagatgg ttttccttta tttgcagacg ttttatatcc agacagttct    1020
ttcattgaga cgtcgaaatt cgcggagtca tga                                 1053

<210> 336
<211> 350
<212> PRT
<213> Unknown

<220>
<223> Obtained from an environmental sample

<400> 336
Met Thr Thr Arg Val Ile Lys Val Ala Ala Ala Gln Leu Ser Pro Val
 1               5                  10                  15
Leu Ala Thr Ala Ser Glu His Ser Arg Glu Asp Thr Ile Ala Lys Val
            20                  25                  30
Ile Asp Ala Ile Ala Ala Ala Ser Gln Gln Gly Ala Gln Leu Ile Val
        35                  40                  45
Phe Pro Glu Thr Val Val Pro Tyr Tyr Pro Tyr Phe Ser Phe Ile Thr
    50                  55                  60
Pro Ala Val Thr Met Gly Ala Glu His Leu Lys Leu Tyr Glu Gln Ala

65                  70                  75                  80
Val Thr Val Pro Ser Ala Ala Thr Asp Ala Val Ala Ala Ala Ala Lys
                85                  90                  95
Asn Tyr Gly Met Val Val Val Leu Gly Ile Asn Glu Arg Asp His Gly
            100                 105                 110
Ser Leu Tyr Asn Ala Gln Leu Ile Phe Asp Ala Asp Gly Glu Leu Leu
        115                 120                 125
Leu Lys Arg Arg Lys Ile Thr Pro Thr Tyr His Glu Arg Met Val Trp
    130                 135                 140
Gly Gln Gly Asp Gly Ser Gly Leu Lys Val Val Asp Thr Ala Ala Gly
145                 150                 155                 160
Arg Val Gly Ala Leu Ala Cys Trp Glu His Tyr Asn Pro Leu Ala Arg
                165                 170                 175
Tyr Ser Leu Met Ala Gln His Glu Glu Ile His Cys Ser Gln Phe Pro
            180                 185                 190
Gly Ser Leu Val Gly Pro Ile Phe Ala Glu Gln Met Glu Ile Thr Met
        195                 200                 205
Arg His His Ala Leu Glu Ser Gly Cys Phe Val Val Asn Ala Thr Ala
    210                 215                 220
Trp Leu Ser Asp Thr Gln Ile Gln Ser Ile Thr Pro Asp Lys Ala Met
225                 230                 235                 240
Gln Lys Ala Leu Arg Gly Gly Cys Tyr Thr Ala Ile Ile Ser Pro Glu
                245                 250                 255
Gly Lys His Leu Cys Pro Pro Leu Tyr Asp Gly Glu Gly Ile Ile Val
            260                 265                 270
Ala Glu Leu Asp Phe Ala Leu Ile Thr Lys Arg Lys Arg Met Met Asp
        275                 280                 285
Ser Val Gly His Tyr Ala Arg Pro Glu Leu Leu Ser Leu Leu Leu Asp
    290                 295                 300
Asp Arg Val Thr Ala Pro Leu Lys Asn Leu Gln Thr Thr Met Ala Ser
305                 310                 315                 320
Ala Lys Ser Ala Glu Asp Gly Phe Pro Leu Phe Ala Asp Val Leu Tyr
                325                 330                 335
Pro Asp Ser Ser Phe Ile Glu Thr Ser Lys Phe Ala Glu Ser
            340                 345                 350

<210> 337
<211> 957
<212> DNA
<213> Unknown

<220>
<223> Obtained from an environmental sample

<400> 337
atgaagggag tgtgtgccat gtcgacgaaa gttgccatcg tccaggcgcc gccggtcctg      60
ctgcatcgcg acaggacgat tgcgaaggtg cgttcgtcga tcgaggatgc cgccaatgcg     120
ggcgcctcgc tgatcgtatt tcccgaagct tatgtacccg gctatccgag ttggatctgg     180
cgtctcaggc ccggaggcga catgggactg tcgtctgaga ttcacgcaag attgcgggaa     240
aatgccgttg atctcgcgaa cggaggcctg gcgcatgtcc agggggctgc agcaaaattc     300
ggcgcgactg tcgttatcgg catcaatgaa ctcgacagcg agttcagcgg aacgacattg     360
ttcaacaccg tggtggtcat cggccccgac ggaacgcgcc tcaacaggca tcgaaaatta     420
atgccgacca acccggagcg catggtgtgg ggcacgggcg atgcctcggg tctgcgtgtc     480
atcgatacgc cggcgggacg gctgggaacc atgatctgct gggagagcta catgccgctg     540
gcgcgctatg ctctctatgc gcaaggcatc gagatatacg tcgctcccac gtgggacgca     600
ggcgagagct ggattgctac gatgcgccac atcgccaagg aggccggctg ctgggtgatc     660
ggcacggcaa ccgtcatcca gggcagcgat gttccggacg attttcccga acgcgacaag      720

ctcttcaagc cggaggagtg gatcaacgac ggcgatgcgg tcgtggtcaa gcccatgggc 780
gcgattgctg ccggaccgca caatcgacag aaaagcatac tctacgccga catcgaccgg 840
gaggccgcgc ggcgagcccg ccggtcgctc gatgtctgtg gccactattc ccgcccagac 900
gttttctctt tctcggtcaa ccgaaagcca ttccgccctg ccgactttgt gggttga 957

<210> 338
<211> 313
<212> PRT
<213> Unknown

<220>
<223> Obtained from an environmental sample

<400> 338
Met Lys Gly Val Cys Ala Met Ser Thr Lys Val Ala Ile Val Gln Ala
1               5                   10                  15
Pro Pro Val Leu Leu His Arg Asp Gly Arg Leu Arg Arg Cys Val Arg
            20                  25                  30
Arg Ser Arg Met Pro Pro Met Arg Ala Pro Arg Ser Tyr Phe Pro Ala
        35                  40                  45
Tyr Val Pro Gly Tyr Pro Ser Trp Ile Trp Arg Leu Arg Pro Gly Gly
    50                  55                  60
Asp Met Gly Leu Ser Ser Glu Ile Thr Gln Asp Cys Gly Lys Met Pro
65                  70                  75                  80
Leu Ile Ser Arg Thr Glu Ala Trp Arg Met Ser Arg Gly Leu Gln Gln
                85                  90                  95
Asn Ser Ala Arg Leu Ser Leu Ser Ala Ser Met Asn Ser Thr Ala Ser
            100                 105                 110
Ser Ala Glu Arg His Cys Ser Thr Val Val Val Ile Gly Pro Asp Gly
        115                 120                 125
Thr Arg Leu Asn Arg His Arg Lys Leu Met Pro Thr Asn Pro Glu His
    130                 135                 140
Gly Val Gly His Gly Arg Cys Leu Gly Ser Ala Cys His Arg Tyr Ala
145                 150                 155                 160
Gly Gly Thr Ala Gly Asn His Ile Cys Trp Glu Ser Tyr Met Pro Leu
                165                 170                 175
Ala Arg Tyr Ala Leu Tyr Ala Gln Gly Ile Glu Ile Tyr Val Ala Pro
            180                 185                 190
Thr Trp Asp Ala Gly Glu Ser Trp Ile Ala Thr Met Arg His Ile Ala
        195                 200                 205
Lys Glu Ala Gly Cys Trp Val Ile Gly Thr Ala Thr Val Ile Gln Gly
    210                 215                 220
Ser Asp Val Pro Asp Asp Phe Pro Glu Arg Asp Lys Leu Phe Lys Pro
225                 230                 235                 240
Arg Ser Gly Ser Thr Thr Ala Met Arg Ser Trp Ser Ser Pro Trp Ala
                245                 250                 255
Arg Leu Leu Pro Asp Arg Thr Ile Asp Arg Lys Ala Tyr Ser Thr Pro
            260                 265                 270
Thr Ser Thr Gly Arg Pro Arg Gly Glu Pro Ala Gly Arg Ser Met Cys
        275                 280                 285
Gly His Tyr Ser Arg Pro Asp Val Phe Ser Phe Ser Val Asn Arg Lys
    290                 295                 300
Pro Phe Arg Pro Ala Asp Phe Val Gly
305                 310

<210> 339
<211> 1020

<212> DNA
<213> Unknown

<220>
<223> Obtained from an environmental sample

<400> 339
atgtcaggat cattcaaagt tgcagcagtg caggcggctc cggccttcct caatctcgat 60
gcgggcatcg acaaggcggt ggcgctgatc gagcaagccg ccgcgcagga cgttcagctc 120
atcgcctttc ccgagacctg gttgcccgga tacccctggt ggatctggct cgatgcaccc 180
gccgttacca tgggctatgt cgttccctac aatctcaatt cactagaggc gggcagcccg 240
caggacaagc gtctggcaaa tgccgcgcgt gagaacaaca tccaggtggt gatgggtctg 300
tctgaacgcc atgacggcac gctctacatc gcgcagtggc actatggtga agatggcgag 360
gtgatctcgc gacggcgcaa gctcaagccg acccatgtcg aacgcacggt gttcggggaa 420
ggcgacggca gcgacatggt ggtcaaggac acaagtctgg gacgggtcgg cgctctgtgc 480
tgttgggaac acctgcagcc gctcaacaaa tacgcgatgt actcccagaa cgagcagatc 540
cacatcggtt cctggcccag cttcagcctc tacaagggcg gcgcctatgc gcttggggca 600
gacctcaaca cggcggcaag ccagatgtat gcggccgagg gccagtgctt tgttctggct 660
gcctgcgcca cggtcagtca ggacatgttc gacatgctct gcgacacgga aatgaagcag 720
cagttcctga ccaccggtgg cggattcgcc cgcattttcg gacctgacgg ctcgcccatg 780
ggcaatgtgc ttgaagaaca tgaagaaggg ctggtgatcg ccgaaatcga tctcaccatg 840
attgcgatcg ccaaggcggc ggctgacccg tgcgggcact attcccggcc cgatgtgttc 900
cgactgatgt tcaaccagaa gccaagcccg gtggtgatgc cattcgaaaa cgacgtggcc 960
cgcgagattg tcgaggccgc cgaggatcag gtcagcgcaa accggctcgc agcggaatga 1020

<210> 340
<211> 329
<212> PRT
<213> Unknown

<220>
<223> Obtained from an environmental sample

<400> 340
Met Ser Gly Ser Phe Lys Val Ala Ala Val Gln Ala Ala Pro Ala Phe
1               5                   10                  15
Leu Asn Leu Asp Ala Gly Ile Asp Gly Gly Gly Ala Asp Arg Ala Ser
            20                  25                  30
Arg Arg Ala Gly Arg Ser Ala His Arg Leu Ser Arg Asp Leu Val Ala
        35                  40                  45
Gly Tyr Pro Trp Trp Ile Trp Leu Asp Ala Pro Ala Val Thr Met Gly
    50                  55                  60
Tyr Val Val Pro Tyr Asn Leu Asn Ser Arg Gly Gly Gln Pro Ala Gly
65                  70                  75                  80
Gln Ala Ser Gly Lys Cys Arg Ala Glu Gln His Pro Gly Gly Asp Gly
                85                  90                  95
Leu Ser Glu Arg His Asp Gly Thr Leu Tyr Ile Ala Gln Trp His Tyr
            100                 105                 110
Gly Glu Asp Gly Glu Val Ile Ala Thr Ala Gln Ala Gln Ala Asp Pro
        115                 120                 125
Cys Arg Thr His Gly Val Arg Gly Arg Arg Arg Gln Arg His Val Val
    130                 135                 140
Lys Asp Thr Ser Leu Gly Arg Val Gly Ala Leu Cys Cys Trp Glu His
145                 150                 155                 160
Leu Gln Pro Leu Asn Lys Thr Arg Cys Thr Pro Arg Thr Ser Arg Ser
                165                 170                 175
Thr Ser Val Pro Gly Pro Ala Ser Ala Ser Thr Arg Ala Ala Leu Cys

180                 185                 190
Ala Trp Gly Arg Pro Gln His Gly Gly Lys Pro Asp Val Cys Gly Arg
        195                 200                 205
Gly Pro Val Leu Cys Ser Leu Pro Ala Pro Arg Ser Val Arg Thr Cys
    210                 215                 220
Ser Thr Cys Ser Ala Thr Arg Lys Ser Ser Ser Ser Thr Gly Gly Gly
225                 230                 235                 240
Phe Ala Arg Ile Phe Gly Pro Asp Gly Ser Pro Met Gly Asn Val Leu
                245                 250                 255
Glu Glu His Glu Arg Ala Gly Asp Arg Arg Asn Arg Ser His His Asp
            260                 265                 270
Cys Asp Arg Gln Gly Gly Gly Pro Val Arg Ala Leu Phe Pro Ala Arg
        275                 280                 285
Cys Val Pro Thr Asp Val Gln Pro Glu Ala Lys Pro Gly Gly Asp Ala
    290                 295                 300
Ile Arg Lys Thr Trp Pro Ala Arg Leu Ser Arg Pro Pro Arg Ile Arg
305                 310                 315                 320
Ser Ala Gln Thr Gly Ser Gln Arg Asn
                325

<210> 341
<211> 1056
<212> DNA
<213> Unknown

<220>
<223> Obtained from an environmental sample

<400> 341
atggcactga ccaacccaaa atataaagtc gccgccgtcc aggccgcgcc agcgttcctc      60
gatctggatg cgtccgtcga aaaagcggtc cggctgatcg acgaggccgg cgccaagggc     120
gcgcgcctca ttgcgttccc ggaaacctgg atccccggct atccctggtg gatctggctc     180
ggcgcgccgg cctgggcgat catgaaaggt tttgtctcgg cctatttcga caattcgctc     240
acctatgaca gtccggccgc ggacaaattg cgccaggccg ccaagcgcaa cgatatcgtc     300
gtggtgctcg gcctgtcgga gcgcgacggc ggcagtctct atatcgcgca atggatcatc     360
ggcccggacg gcgaaactgt cgcccagcgc cgcaagctca agccgaccca tgtcgagcgt     420
tcggtgttcg gcgagggcga tggcagcgac cttgccgtgc atgagctcgc gatcgggcgc     480
gtcggcgcgc tgtgctgctg ggagcatctg caaccgctgt cgaaatacgc catgtatgcg     540
cagaacgagc aggttcacgt ggcggcctgg ccgagctttt cgctgtacga tccgttcgcc     600
catgccctcg gtgcggaggt caacaacgcc gccagcaaga tctacgcggt cgagggatcg     660
tgcttcgtcg tggcaccctg cgccaccgtt tcaaaggaaa tgatcgacct gttgtgcgac     720
acgccggaca agcacggtct gctgcacgcc ggcggcgggt ttgccgcgat ctacggccct     780
gacggctcgc cgatcggcga ccgcctggcg cccgaccagg aaggtctgat ctatgccgat     840
gtcgatctcg gcatgatctc ggtcgcgaag gccgccgccg atccggccgg acattatgcg     900
cggccggatg tcacaaggtt gctgctcaac aaacgcccgg gcaatcgtgt cgaggcgctg     960
gcacttccgg tggatcaggt tgcggcaggt gaggagatcc cctcgatatc gcgatcggcc    1020
agaggggttg ccgaactgcc aaacgcggcc gaatag                              1056

<210> 342
<211> 342
<212> PRT
<213> Unknown

<220>
<223> Obtained from an environmental sample

<400> 342

Met Ala Leu Thr Asn Pro Lys Tyr Lys Val Ala Ala Val Gln Ala Ala
1               5                   10                  15
Pro Ala Phe Leu Asp Leu Asp Ala Pro Ser Lys Lys Arg Ser Gly Ser
            20                  25                  30
Thr Arg Pro Ala Pro Arg Ala Arg Ala Ser Leu Arg Ser Arg Thr Trp
        35                  40                  45
Ile Pro Gly Tyr Pro Trp Trp Ile Trp Leu Gly Ala Pro Ala Trp Ala
    50                  55                  60
Ile Met Lys Gly Phe Val Ser Pro Ile Ser Thr Ile Arg Ser Pro Met
65                  70                  75                  80
Thr Val Arg Pro Arg Thr Asn Cys Ala Arg Pro Pro Ser Ala Thr Tyr
                85                  90                  95
Arg Arg Gly Ala Arg Pro Val Gly Ala Arg Arg Arg Gln Ser Leu Tyr
            100                 105                 110
Arg Ala Met Asp His Arg Pro Thr Ala Lys Leu Ser Pro Ser Ala Ala
        115                 120                 125
Ser Ser Ser Arg Pro Met Ser Ser Val Arg Cys Ser Ala Arg Ala Met
    130                 135                 140
Ala Ala Thr Leu Pro Cys Met Ser Ser Arg Ser Gly Ala Ser Ala Arg
145                 150                 155                 160
Cys Ala Ala Gly Ser Ile Cys Thr Ala Val Glu Ile Arg His Val Cys
                165                 170                 175
Ala Glu Arg Ala Gly Ser Arg Gly Gly Leu Ala Glu Leu Phe Ala Thr
            180                 185                 190
Ile Arg Ser Pro Met Pro Ser Val Arg Arg Ser Thr Thr Pro Pro Ala
        195                 200                 205
Arg Ser Thr Arg Ser Arg Asp Arg Ala Ser Ser Trp His Pro Ala Pro
    210                 215                 220
Pro Phe Gln Arg Lys Ser Thr Cys Cys Ala Thr Arg Arg Thr His Gly
225                 230                 235                 240
Leu Leu His Ala Gly Gly Gly Phe Ala Ala Ile Tyr Gly Pro Asp Gly
                245                 250                 255
Ser Pro Ile Gly Asp Arg Trp Arg Pro Thr Arg Lys Val Ser Met Pro
            260                 265                 270
Met Ser Ile Ser Ala Ser Arg Ser Arg Arg Pro Pro Asp Pro Ala Gly
        275                 280                 285
His Tyr Ala Arg Pro Asp Val Thr Arg Leu Leu Leu Asn Lys Arg Pro
    290                 295                 300
Gly Asn Arg Val Arg Arg Trp His Phe Arg Trp Ile Arg Leu Arg Gln
305                 310                 315                 320
Val Arg Arg Ser Pro Arg Tyr Arg Asp Arg Pro Glu Gly Cys Arg Thr
                325                 330                 335
Ala Lys Arg Gly Arg Ile
            340

<210> 343
<211> 942
<212> DNA
<213> Unknown

<220>
<223> Obtained from an environmental sample

<400> 343
atgaagtcaa aaattgcggt tattcagcga cctcctgtat tgttagacct tcaggcttca      60
atcgcccgtg ccattacttc tgttgaggaa gcggcaggca agggatctga gctccttgtt     120
tttcccgaga catttctgcc tggttatccg tcctggatct ggcgtctcaa gccgggcgga     180

gacatggtgc tgacatctga aatccacgca aaatatcgcg cgaactctgt tgatgttgag 240
cgcggggatc tggccccttt atgcgaagcg gcggcgaaac acggcgtcac aattgtcatg 300
gggctcagtg aaattgatgg gcgctacagc gggactacac tctttaatac agtggtgacc 360
attggcgcgg aaggagagct ccttaataga caccgcaagc tcatgccgac aaacccagag 420
cgtatggtct gggggcaagg ggatgcctct ggtctgcggg ttgtcgacac gcccgtgggc 480
cgcgtcggca cgctgatctg ctgggaaaac tacatgccgc tatcgcgcta tgcgctttat 540
tctcaaaaca ttgacatcta tgtggcgccg acctgggacg cgggcgagag ctggatcgcc 600
tccatgcagc atatcgccaa agaaggtggc tgctgggtga tcggcacggc cacggcgatg 660
gagggctctg atgtcccagc cgacttccct cagcgggagg tgcttttccc tgatagcagc 720
gaatggatca atgacggtga cgctgtagtg gttaaaccca tgggggcgat tgtcgcgggt 780
ccgcatcacc gggataagag tattctctat gctgagattg acgtcgaagt ggcacgcaat 840
gcgcggcgct cgctcgatgt ggcggggcat tactcccggc cggatatttt ttcctttggc 900
gtggatcgcc ggcctttgcc gccggttacg tttgaggatt ga 942

<210> 344
<211> 303
<212> PRT
<213> Unknown

<220>
<223> Obtained from an environmental sample

<400> 344
Met Lys Ser Lys Ile Ala Val Ile Gln Arg Pro Pro Val Leu Leu Asp
1               5                   10                  15
Leu Gln Ala Ser Ile Ala Arg Ala Ile Thr Leu Leu Arg Lys Arg Gln
            20                  25                  30
Ala Arg Asp Leu Ser Ser Leu Phe Phe Pro Arg His Phe Cys Leu Val
        35                  40                  45
Ile Arg Pro Gly Ser Gly Val Ser Ser Arg Ala Glu Thr Trp Cys His
    50                  55                  60
Leu Lys Ser Thr Gln Asn Ile Ala Arg Thr Leu Asp Val Glu Arg Gly
65                  70                  75                  80
Asp Leu Ala Pro Leu Cys Glu Ala Ala Ala Lys His Gly Val Thr Ile
                85                  90                  95
Val Met Gly Gln Asn Trp Ala Leu Gln Arg Asp Tyr Thr Leu Tyr Ser
            100                 105                 110
Gly Asp His Trp Arg Gly Arg Arg Leu Leu Asn Arg His Arg Lys Leu
        115                 120                 125
Met Pro Thr Asn Pro Glu Arg Met Val Trp Gly Gln Gly Asp Ala Ser
    130                 135                 140
Val Cys Gly Leu Ser Thr Arg Pro Trp Ala Ala Ser Ala Arg Ser Ala
145                 150                 155                 160
Gly Lys Thr Thr Cys Arg Tyr Arg Tyr Ala Leu Tyr Ser Gln Asn Ile
                165                 170                 175
Asp Ile Tyr Val Ala Pro Thr Trp Asp Ala Gly Glu Ser Trp Ile Ala
            180                 185                 190
Ser Met Gln His Ile Ala Lys Glu Gly Gly Cys Trp Val Ile Gly Thr
        195                 200                 205
Ala Thr Ala Met Glu Gly Ser Asp Ser Gln Pro Thr Ser Leu Ser Gly
    210                 215                 220
Arg Cys Phe Ser Leu Ile Ala Ala Asn Gly Ser Met Thr Val Thr Leu
225                 230                 235                 240
Trp Leu Asn Pro Trp Gly Arg Leu Ser Arg Val Arg Ile Thr Gly Ile
                245                 250                 255
Arg Val Phe Ser Met Leu Arg Leu Thr Arg Ser Gly Thr Gln Cys Ala
            260                 265                 270

Ala Leu Ala Arg Cys Gly Gly Ala Leu Leu Pro Ala Gly Tyr Phe Phe
        275                 280                 285
Leu Trp Val Asp Arg Arg Pro Leu Pro Pro Val Thr Phe Glu Asp
    290                 295                 300

<210> 345
<211> 1011
<212> DNA
<213> Unknown

<220>
<223> Obtained from an environmental sample

<400> 345
atgaaagcga tcaaggccgc tgccgtgcag gcagcaccgg tattcctgaa cctcgacgca      60
tcgatcacca aggcggaaac attcgtcgcc gaggccgccg cgaatggtgc caagctggtg     120
gcgtttccgg aaacctggct gccgggctat ccctggttca tctggctcgg tgcgcccgcc     180
gaaggcatgc agttcatccc gcgctatcac gaaaacagca tggagctccg ctcgcccgag     240
atgcgccgct tgcaggcgat cgcgcgcaag tatgaagtga cgctcgtcat gggctattcc     300
gagcgcgatg gtggcagccg ctacatgtcc caggtcatta ttggcgatca gggcgacatc     360
cttctcaatc gccgtaaatt gaagccaacc catgtcgagc ggacggtctt cggcgaaggc     420
gacggttcgg acctggtggt ggtcgaaacg gcattcggca ggctcggtgc gctcaattgc     480
tgggaacata tccagccgct cgtcaagatg tcgatgtatg cccagcatga ggaaatccat     540
gtcgcgggtt ggccgagctt ctgcgtctac cgcgatctcg cctatgccct gggaccggaa     600
gtcaacaatg ccgtcagtca ggtctatgcc gtggagggta gcgcctatgt tctggcaccc     660
tgtgcgatcg taagccagga gatgttcgac attctggccg acaagcctga aaaggccttt     720
ctcctcaatc cccgcacatc caagcccggc ggtggcttca cgcagatcta tgcgccggat     780
ggtcgaccgc tttgcgagcc gcttgccgac gatgtggaag gcatcctcta tgccgatctc     840
gatccggcaa cgatcgccgt cgcgaaggcg gccgccgatc ctgcggggca ctattcgcgg     900
ccggacgcac tctcgctggt gatcaatcgc gaaaagcgcg cggtgatggc tgaaatcaac     960
gcgccggcga cgccgacctt cacccccatc tccctggacg ctgcggagta g             1011

<210> 346
<211> 329
<212> PRT
<213> Unknown

<220>
<223> Obtained from an environmental sample

<400> 346
Met Lys Ala Ile Lys Ala Ala Ala Val Gln Ala Ala Pro Val Phe Leu
1               5                   10                  15
Asn Leu Asp Ala Ser Ile Thr Lys Ala Glu Thr Phe Val Ala Glu Ala
            20                  25                  30
Ala Ala Asn Gly Ala Lys Leu Val Ala Phe Pro Glu Thr Trp Leu Pro
        35                  40                  45
Gly Tyr Pro Trp Phe Ile Trp Leu Gly Ala Pro Ala Glu Gly Met Gln
    50                  55                  60
Phe Ile Pro Arg Tyr His Glu Asn His Gly Ala Pro Leu Ala Arg Asp
65                  70                  75                  80
Ala Pro Leu Ala Gly Asp Arg Ala Gln Val Ser Asp Ala Arg His Gly
                85                  90                  95
Tyr Ser Glu Arg Asp Gly Gly Ser Arg Tyr Met Ser Gln Val Ile Ile
            100                 105                 110
Gly Asp Gln Gly Asp Ile Leu Leu Asn Arg Arg Lys Leu Lys Pro Thr
        115                 120                 125

His Val Glu Arg Thr Val Phe Gly Glu Gly Asp Gly Ser Asp Leu Val
    130                 135                 140
Trp Ser Lys Arg His Ser Ala Gly Ser Val Arg Ser Ile Ala Gly Asn
145                 150                 155                 160
Ile Ser Ser Arg Ser Ser Arg Cys Met Tyr Ala Gln His Glu Glu Ile
                165                 170                 175
His Val Ala Gly Trp Pro Ser Phe Cys Val Tyr Arg Asp Leu Ala Tyr
            180                 185                 190
Ala Trp Asp Arg Lys Ser Thr Met Pro Ser Val Arg Ser Met Pro Trp
        195                 200                 205
Arg Val Ala Pro Met Phe Trp His Pro Val Arg Ser Ala Arg Arg Cys
    210                 215                 220
Ser Thr Phe Trp Pro Thr Ser Leu Lys Arg Pro Phe Ser Ser Ile Pro
225                 230                 235                 240
Ala His Pro Ser Pro Ala Val Ala Ser Arg Arg Ser Met Arg Arg Met
                245                 250                 255
Val Asp Arg Phe Ala Ser Arg Leu Pro Thr Val Glu Gly Ile Leu Tyr
            260                 265                 270
Ala Asp Leu Asp Pro Ala Thr Ile Ala Val Ala Lys Ala Ala Ala Asp
        275                 280                 285
Pro Ala Gly Thr Ile Arg Gly Arg Thr His Ser Arg Trp Ser Ile Ala
    290                 295                 300
Lys Ser Ala Arg Trp Leu Lys Ser Arg Ala Gly Asp Ala Asp Leu His
305                 310                 315                 320
Pro His Leu Pro Gly Arg Cys Gly Val
                325

<210> 347
<211> 909
<212> DNA
<213> Unknown

<220>
<223> Obtained from an environmental sample

<400> 347
atgaaagtcg cggcgattca agcggcgccg gtctacttgg accggcaagc cacgctggaa      60
aaggcgcttt ctctaatgga tgaagcggcc gcaaacggcg cccaagtctg cgccttccct     120
gagaccttcc tcgcgggcta tcccgtctgg atggacttga cggacggcgc caagtggaat     180
gacgacaaac aaaaagcggc ctacgcatgt tatgtagatg cggccgtcga agccgacgga     240
cctgagttgc aagccattgc caagaaatcg aaggcgctcg gcctgttcac ctatctgggc     300
atggtcgaac gcgcggcgtc ggccgggtca gtatattgtt ccctcgctgc cttcgatccc     360
gacaagggtc tcgtcagcct acaccgaaaa ctcatgccca cctacaccga gcgcctcgtg     420
tggagccaag gcgacggaca cggactccag gtgcatgaat tcgccggctt caaaatcggc     480
gcgctaaact gctgggaaaa ttggatgccc ctcgcccgtt acgcaatgta cgcccagggc     540
gaacagctcc acgtcgcgac ctggcccggg tccccctggc tcaccaagga catcacccgt     600
ttcatcgccc ttgagggacg catctacgtc atgtccgttg gcggcgtcct tagcgcaaac     660
gatatccccg actccttccc gcttaaaacc gacctcctca agatccgaga ccgctacctc     720
agcggcggca ccatgatagt cgccccagac ggcaccaccc tcgaaggccc cgccaaaaac     780
gaagagacca tactctacgc ggagctcgac ctcaacaccg tcctccaaga acgccaaaat     840
ttcgatcccg cgggacacta cgcgcgccca gacgtcttcc aattggaaat cgacaaaaat     900
cgacgatag                                                             909

<210> 348
<211> 297
<212> PRT
<213> Unknown

<220>
<223> Obtained from an environmental sample

<400> 348
Met Lys Val Ala Ala Ile Gln Ala Ala Pro Val Tyr Leu Asp Arg Gln
1               5                   10                  15
Ala Thr Leu Glu Lys Ala Leu Ser Trp Met Lys Arg Pro Gln Thr Ala
            20                  25                  30
Pro Lys Ser Ala Pro Ser Leu Arg Pro Ser Ser Arg Ala Ile Pro Trp
        35                  40                  45
Met Asp Leu Thr Asp Gly Ala Lys Trp Asn Asp Asp Lys Gln Lys Ala
    50                  55                  60
Ala Tyr Ala Cys Tyr Val Asp Ala Ala Val Glu Ala Asp Gly Pro Glu
65                  70                  75                  80
Leu Gln Ala Ile Ala Lys Lys Ser Lys Ala Leu Gly Leu Phe Thr Ile
                85                  90                  95
Trp Ala Trp Ser Asn Ala Arg Arg Arg Pro Gly Gln Tyr Ile Val Pro
            100                 105                 110
Ser Leu Pro Ser Ile Pro Thr Arg Val Ser Ser Ala Tyr Thr Glu Asn
        115                 120                 125
Ser Cys Pro Pro Thr Pro Ser Ala Ser Cys Gly Ala Lys Ala Thr Asp
    130                 135                 140
Thr Asp Ser Arg Cys Met Asn Ser Pro Ala Ser Lys Ser Ala Arg Thr
145                 150                 155                 160
Ala Gly Lys Ile Gly Cys Pro Ala Arg Tyr Ala Met Tyr Ala Gln Gly
                165                 170                 175
Glu Gln Leu His Val Ala Thr Trp Pro Gly Ser Pro Trp Leu Thr Lys
            180                 185                 190
Asp Ile Thr Arg Phe Ile Ala Leu Glu Gly Arg Ile Tyr Val Met Ser
        195                 200                 205
Val Gly Gly Val Leu Ser Ala Asn Asp Ile Pro Asp Ser Phe Pro Leu
    210                 215                 220
Lys Thr Asp Leu Leu Lys Ile Arg Asp Arg Tyr Leu Ser Gly Gly Thr
225                 230                 235                 240
Asp Ser Arg Pro Arg Arg His His Pro Arg Arg Pro Arg Gln Lys Arg
                245                 250                 255
Arg Asp His Thr Leu Arg Gly Leu Asp Leu Asn Thr Val Leu Gln Glu
            260                 265                 270
Arg Gln Asn Phe Asp Pro Ala Gly His Tyr Ala Arg Pro Asp Val Ser
        275                 280                 285
Asn Trp Lys Ser Thr Lys Ile Asp Asp
    290                 295

<210> 349
<211> 1002
<212> DNA
<213> Unknown

<220>
<223> Obtained from an environmental sample

<400> 349
atgacgtccc ctgttcaaac caagtacaaa gtcgcctgtg ttcaggcggc gcccgagttt      60
ctcgatctcg acaaaggcgt tgccaaagcg gtgcgcctga tcgaagaagc cgccacccaa     120
aaggcctcgc tgatcgcgtt tcccgaagtc tggctccccg gctatccgtg gtggatctgg     180
ctcgactcgc cggcctgggg cttgcagttc gtccagcgct acttcgaaaa cgctctggtc     240

gtcggcagcc cccaatggga gcgcttgtgc aaggccgccg ccgacaacaa tatccatgtt 300
gtgctcggat tctccgaacg ggacggcagc acgctgtacc tcgcacaggc catcatcgat 360
aacaccggga aggtgatcgc cacgcggcgc aaactcaagc caacacacgc cgaacgcacg 420
gttttcggcg aaggcgacgg gagccacatc gcggtgcatg aaaccacttt gggccgcatg 480
ggtgcactct gctgcgccga gcacatccag ccactgacca agtacgccat gtactcgcag 540
cacgagcaga ttcacattgc cgcatggccc agcttctcgg tctaccgcgg agcagcgttc 600
cagctgagcg ccgaagccaa caacgccgcg agccaggtct atgccctgga gggcagttgc 660
tacgtggtgg ccccttgcgc gacggtgtcc aaggagatgt tggacatgct ggctgattcg 720
ccgcaaaaga agcagctcct gctggaaggc ggtggctacg ccatgattta tgggcccgac 780
gccaagcccc tgtgcgagcc cattccagag acagaagaag gcattcttta cgcagatgtg 840
gacctgggct tcatcggtgt caccaaggca gcgtatgacc ccgccggtca ctattcacgc 900
cccgacgtgc tgcgcctttt gttcaatcgg aagcctgccc ctcgggttca cgatttcgat 960
cctgaataca cggccaccga gcagaagaca gacgcggcct ga 1002

<210> 350
<211> 333
<212> PRT
<213> Unknown

<220>
<223> Obtained from an environmental sample

<400> 350
Met Thr Ser Pro Val Gln Thr Lys Tyr Lys Val Ala Cys Val Gln Ala
1               5                   10                  15
Ala Pro Glu Phe Leu Asp Leu Asp Lys Gly Val Ala Lys Ala Val Arg
            20                  25                  30
Leu Ile Glu Glu Ala Ala Thr Gln Lys Ala Ser Leu Ile Ala Phe Pro
        35                  40                  45
Glu Val Trp Leu Pro Gly Tyr Pro Trp Trp Ile Trp Leu Asp Ser Pro
    50                  55                  60
Ala Trp Gly Leu Gln Phe Val Gln Arg Tyr Phe Glu Asn Ala Leu Val
65                  70                  75                  80
Val Gly Ser Pro Gln Trp Glu Arg Leu Cys Lys Ala Ala Ala Asp Asn
                85                  90                  95
Asn Ile His Val Val Leu Gly Phe Ser Glu Arg Asp Gly Ser Thr Leu
            100                 105                 110
Tyr Leu Ala Gln Ala Ile Ile Asp Asn Thr Gly Lys Val Ile Ala Thr
        115                 120                 125
Arg Arg Lys Leu Lys Pro Thr His Ala Glu Arg Thr Val Phe Gly Glu
    130                 135                 140
Gly Asp Gly Ser His Ile Ala Val His Glu Thr Thr Leu Gly Arg Met
145                 150                 155                 160
Gly Ala Leu Cys Cys Ala Glu His Ile Gln Pro Leu Thr Lys Tyr Ala
                165                 170                 175
Met Tyr Ser Gln His Glu Gln Ile His Ile Ala Ala Trp Pro Ser Phe
            180                 185                 190
Ser Val Tyr Arg Gly Ala Ala Phe Gln Leu Ser Ala Glu Ala Asn Asn
        195                 200                 205
Ala Ala Ser Gln Val Tyr Ala Leu Glu Gly Ser Cys Tyr Val Val Ala
    210                 215                 220
Pro Cys Ala Thr Val Ser Lys Glu Met Leu Asp Met Leu Ala Asp Ser
225                 230                 235                 240
Pro Gln Lys Lys Gln Leu Leu Leu Glu Gly Gly Gly Tyr Ala Met Ile
                245                 250                 255
Tyr Gly Pro Asp Ala Lys Pro Leu Cys Glu Pro Ile Pro Glu Thr Glu
            260                 265                 270

Glu Gly Ile Leu Tyr Ala Asp Val Asp Leu Gly Phe Ile Gly Val Thr
        275                 280                 285
Lys Ala Ala Tyr Asp Pro Ala Gly His Tyr Ser Arg Pro Asp Val Leu
    290                 295                 300
Arg Leu Leu Phe Asn Arg Lys Pro Ala Pro Arg Val His Asp Phe Asp
305                 310                 315                 320
Pro Glu Tyr Thr Ala Thr Glu Gln Lys Thr Asp Ala Ala
                325                 330

<210> 351
<211> 936
<212> DNA
<213> Unknown

<220>
<223> Obtained from an environmental sample

<400> 351
atgattacag caggcatcgc agtcgccgct ccggtggtgt tggacaaaac aaaaaccatt      60
gagaaagccg ttggcattat tcacgaggca gcgggtaagg gtgtgaacct gcttgtgttt     120
cccgaggcat ttattccctc ctatcccgcc tggggttggc gcctgcgtcc cggtggagat     180
ttcgggttgt gcgaggagtt gcacgccctg ttgcttgata attcggtaaa tttgcaaggt     240
gatgacctgg accctgtccg gggcgctgca gccgagcatt caatgaccgt ggtgatggga     300
ttgaatgagc gcgaaggcca gttcggtcgg gctaccctgt ttaacgccat ggtatttatc     360
ggtccggacg gcagcatcct gaaccatcat cggaaactta tgccaaccaa tcatgagcgt     420
acgattcatg gcttcggcga tgcgcgggga ttgaaagtgg tggatacccc gtgcggtcgc     480
gtgggtggtc tgatttgctg ggagaatttc atgcccctgg ctcgctacgg cctgtatgcc     540
cagggcgtag aagtgtatgt tgcgcccacc tacgaccagg gtgatgggtg gataggatcc     600
atgcagcata ttgcccggga aggacggtgc tgggtgttat cggccggaac accgctacgc     660
ggcagtgatt ttcccgcgga catgccgggc aaggctcaac tgtttcccga tgacgatgaa     720
tgggtgaatc ccggtgggtc agtggttatc gcaccgggtg gggaattagt ggctggaccg     780
cttttccgtg aggagggcat ccttgtctgt gaattggatc cggcgaaaag tgctcatgcc     840
aagcggtcct ttgacgtggc cggtcattac gccaggccag atattttcga gttggaaata     900
gaccgtgatc cacaggatcc cgtcgagtgg gactga                               936

<210> 352
<211> 301
<212> PRT
<213> Unknown

<220>
<223> Obtained from an environmental sample

<400> 352
Met Ile Thr Ala Gly Ile Ala Val Ala Ala Pro Val Val Leu Asp Lys
1               5                   10                  15
Thr Lys Thr Ile Glu Lys Ala Val Ala Leu Phe Thr Arg Gln Arg Val
            20                  25                  30
Arg Val Thr Cys Leu Cys Phe Pro Arg His Leu Phe Pro Pro Ile Pro
        35                  40                  45
Pro Gly Val Gly Ala Cys Val Pro Val Glu Ile Ser Gly Cys Ala Arg
    50                  55                  60
Ser Cys Thr Pro Cys Cys Leu Ile Ile Arg Asn Leu Gln Gly Asp Asp
65                  70                  75                  80
Leu Asp Pro Val Arg Gly Ala Ala Ala Glu His Ser Met Thr Val Val
                85                  90                  95
Met Gly Glu Ala Arg Arg Pro Val Arg Ser Gly Tyr Pro Val Arg His

                  100                 105                 110
Gly Ile Tyr Arg Ser Gly Arg Gln His Pro Glu Pro Ser Ser Glu Thr
        115                 120                 125
Tyr Ala Asn Gln Ser Ala Tyr Asp Ser Trp Leu Arg Arg Cys Ala Gly
    130                 135                 140
Ile Glu Ser Gly Gly Tyr Pro Val Arg Ser Arg Gly Trp Ser Asp Leu
145                 150                 155                 160
Leu Gly Glu Phe His Ala Pro Gly Ser Leu Gly Leu Tyr Ala Gln Gly
                165                 170                 175
Val Glu Val Tyr Val Ala Pro Thr Tyr Asp Gln Gly Asp Gly Trp Ile
            180                 185                 190
Gly Ser Ala Ala Tyr Cys Pro Gly Arg Thr Val Leu Gly Val Ile Gly
        195                 200                 205
Arg Asn Thr Ala Thr Arg Gln Phe Ser Arg Thr Cys Arg Ala Arg Leu
    210                 215                 220
Asn Cys Phe Pro Met Thr Met Asn Gly Ile Pro Val Gly Gln Trp Leu
225                 230                 235                 240
Ala Pro Gly Gly Glu Leu Val Ala Gly Pro Leu Phe Arg Glu Glu Gly
                245                 250                 255
Ile Leu Val Cys Glu Leu Asp Pro Ala Lys Ser Ala His Ala Lys Arg
            260                 265                 270
Ser Phe Asp Val Ala Gly His Tyr Ala Arg Pro Asp Ile Phe Glu Leu
        275                 280                 285
Glu Ile Asp Arg Asp Pro Gln Asp Pro Val Glu Trp Asp
    290                 295                 300

<210> 353
<211> 1035
<212> DNA
<213> Psuedomonas putida ATCC 700801

<400> 353
atgaatgctt caaaaacaca ttataaagtt gcagctgtgc aagccgctcc tgaattcctg      60
gatttggaca aaggggttga taaggctata cgcctcatta aagaagcagc tgataacgga     120
gcatcgctgg ttgcctttcc ggaagtttat cttccagggt atccgtggtg gatctggctt     180
ggctctcccg catggggaat gcagtttgtt cagcgctacg tagaaaactc gcttgattta     240
aaaagcgagc agttcgagcg actgtgcaaa gcggctgcta cttaccgtat tcacgttgta     300
atgggttaca gcgaacgctc gtttggcacc ctctacctcg gtcaagcaat tatcgatgac     360
agcgggaaag taattggtac gcgccgtaag ctcaagccca cccatgctga gcgtactgtt     420
tacggtgagg gtaacggcag tgacctcagg gttttcaatt cgcaacttgg aagggtaggt     480
gcactctgct gcgcagagca cgtacaacca ctctcgaagt ttgcgatgta cagccagcat     540
gagcagttgc acattgcctc ttggccgagc ttctcggtat atcgcggtgg cgcatatcaa     600
ctgagcgctg aggctaactg cgcggccacc caagtctatg ctcttgaagg ccagtgcttc     660
gtaatttcag catgcgcaat cgtatctaaa gacatgctaa acgttctcat cgacacccct     720
gacaagggta acctgttgca ggatggcggt ggcttcgcga tgatttatgg ccccgatggc     780
gcaccgctgt gtgaacccct gggcgaacat gaagaaggca tcctttatgc cgacgtcgat     840
ttgggcgcca tttccgtagc taaagcggca ctcgacccgg ttggccatta ctcgcggcca     900
gatgttttgc gtctgctttt caacgatcaa ccgacacctt gcgtggaggc gttcaatccg     960
gctcctgtcg gtactgatgc tccaggtaca gaccttcagg gtgatgagcc cgatgcgcaa    1020
ccgatatctg aataa                                                     1035

<210> 354
<211> 312
<212> PRT
<213> Psuedomonas putida ATCC 700801

<400> 354

Met Asn Ala Ser Lys Thr His Tyr Lys Val Ala Ala Val Gln Ala Ala
1               5                   10                  15
Pro Glu Phe Leu Asp Leu Asp Lys Gly Val Asp Lys Ala Ile Arg Leu
            20                  25                  30
Ile Lys Glu Ala Ala Asp Asn Gly Ala Ser Leu Val Ala Phe Pro Glu
        35                  40                  45
Val Tyr Ser Arg Val Ser Val Val Asp Leu Ala Trp Leu Ser Arg Met
    50                  55                  60
Gly Asn Ala Val Cys Ser Ala Leu Arg Arg Lys Arg Leu Ile Lys Ala
65                  70                  75                  80
Ser Ser Ser Ser Asp Cys Ala Lys Arg Leu Leu Leu Thr Val Phe Thr
                85                  90                  95
Leu Trp Leu Gln Arg Thr Leu Val Trp His Pro Leu Pro Arg Ser Ser
            100                 105                 110
Asn Tyr Arg Gln Arg Glu Ser Asn Trp Tyr Ala Pro Ala Gln Ala His
        115                 120                 125
Pro Cys Ala Tyr Cys Leu Arg Gly Arg Gln Pro Gln Gly Phe Gln Phe
    130                 135                 140
Ala Thr Trp Lys Gly Arg Cys Thr Leu Leu Arg Arg Ala Arg Thr Thr
145                 150                 155                 160
Thr Leu Glu Val Cys Asp Val Gln Pro Ala Ala Val Ala His Cys Leu
                165                 170                 175
Leu Ala Glu Leu Leu Gly Ile Ser Arg Trp Arg Ile Ser Thr Glu Arg
            180                 185                 190
Gly Leu Arg Gly His Pro Ser Leu Cys Ser Arg Pro Val Leu Arg Asn
        195                 200                 205
Phe Ser Met Arg Asn Val Ser Lys Asp Met Leu Asn Val Leu Ile Asp
    210                 215                 220
Thr Pro Asp Lys Gly Asn Leu Leu Gln Asp Gly Gly Gly Phe Ala Met
225                 230                 235                 240
Ile Tyr Gly Pro Asp Gly Ala Pro Leu Cys Glu Pro Leu Gly Glu His
                245                 250                 255
Glu Glu Gly Ile Leu Tyr Arg Arg Arg Phe Gly Arg His Phe Arg Ser
            260                 265                 270
Ser Gly Thr Arg Pro Gly Trp Pro Leu Leu Ala Ala Arg Cys Leu Arg
        275                 280                 285
Leu Leu Phe Asn Asp Gln Pro Thr Pro Cys Val Glu Ala Phe Asn Pro
    290                 295                 300
Ala Pro Val Gly Thr Asp Ala Pro
305                 310

<210> 355
<211> 1014
<212> DNA
<213> Unknown

<220>
<223> Obtained from an environmental sample

<400> 355
atggccgtct ctaaagacgg tactgtttca ggaaagtcgc ctatccgatt gcatgtcgcc      60
gcgatacaga tggtcccaaa gctgggtgac gcgcaggcga acgtgaatca ggcagaagcc     120
cttattcgga aggctcttgg gctgggtgcg cgttggatcg tgttaccaga gatgtttacc     180
tccggtgcgg cgtttcatcc cgacatgctc aaagccattc agccattcga tggcgcccca     240
ctccagttgc tgaaagacct ttctcgcaag ggcaatgctg tcatcggcgg ctcgtttctc     300
gccaagcgtg ggcaacaagt attcaatacc ttcgttttgg tttctccgga cgggtcagtc     360
gtaacgcatg acaaggattc accgacctat tgggaaaatt gctattaccg gggcggtacc      420

gatgatggcg tgttgtctac gcccattggc ccggtcggct ccgtcctctg ttgggaattt 480
atccgctcaa gaaccgcgag acggctggcg aacaaggtca agatggtcgt gggaggctcc 540
tgttggtgga cgctccccga tgatgctgat ccagacagcc cgcgcagagc cgtgaacctc 600
aagatgctgc aagaagcgcc ggttcgcatg gcgcggatgc tgggtgttcc ggtaatacat 660
ggctcccacg cgggcagctt cgaaggattc ttcagtccgg aacttgcgga tgttccctat 720
aactcgacgt acctgggcga gacaatgatt gtcgacgcgg gtggccgggt acttgcccgt 780
agagcgcaag atgcaggcga aggcgtggta acggcagaag tggttttgcc cgacaagtcc 840
gtaccaagcg aacccatccc ggagactttc tggattccca aggaaatgcc ggatgattgg 900
aaagaagcct gggagcgttg gttcgatacc ggtgcggatt actacgagat ggtgaccgcg 960
ccctttatca agacgggtgt gataaacgag tacacaccgg aatatcttag gtag 1014

<210> 356
<211> 325
<212> PRT
<213> Unknown

<220>
<223> Obtained from an environmental sample

<400> 356
Met Ala Val Ser Lys Asp Gly Thr Val Ser Gly Lys Ser Pro Ile Arg
1               5                   10                  15
Leu His Val Ala Ala Ile Gln Met Val Gln Ser Trp Val Thr Arg Arg
            20                  25                  30
Arg Thr Ile Arg Gln Lys Pro Leu Phe Gly Arg Leu Leu Gly Trp Val
        35                  40                  45
Arg Val Gly Ser Cys Tyr Gln Arg Cys Leu Pro Pro Val Arg Arg Phe
    50                  55                  60
Ile Pro Thr Cys Ser Lys Pro Phe Ser Phe Asp Gly Ala Pro Leu Gln
65                  70                  75                  80
Leu Leu Lys Asp Leu Ser Arg Lys Gly Asn Ala Val Ile Gly Gly Ser
                85                  90                  95
Phe Leu Gln Ala Trp Ala Thr Ser Ile Gln Tyr Leu Arg Phe Gly Phe
            100                 105                 110
Ser Gly Arg Val Ser Arg Asn Ala Gln Gly Ser Pro Thr Tyr Trp Glu
        115                 120                 125
Asn Cys Tyr Tyr Arg Gly Gly Thr Asp Asp Gly Val Leu Ser Thr Pro
    130                 135                 140
Ile Gly Pro Ser Ala Pro Ser Ser Val Gly Asn Leu Ser Ala Gln Glu
145                 150                 155                 160
Pro Arg Asp Gly Trp Arg Thr Arg Ser Arg Trp Val Gly Gly Ser Cys
                165                 170                 175
Trp Trp Thr Leu Pro Asp Asp Ala Asp Pro Asp Ser Pro Arg Arg Ala
            180                 185                 190
Val Asn Leu Arg Cys Cys Lys Lys Arg Arg Phe Ala Trp Arg Gly Cys
        195                 200                 205
Trp Val Phe Arg Tyr Met Ala Pro Thr Arg Gln Leu Arg Arg Ile Leu
    210                 215                 220
Gln Ser Gly Thr Cys Gly Cys Ser Leu Leu Asp Val Pro Gly Arg Asp
225                 230                 235                 240
Asn Asp Val Asp Ala Gly Gly Arg Val Leu Ala Arg Arg Ala Gln Asp
                245                 250                 255
Ala Gly Glu Gly Val Val Thr Ala Glu Gly Phe Ala Arg Gln Val Arg
            260                 265                 270
Thr Lys Arg Thr His Pro Gly Asp Phe Leu Asp Ser Gln Gly Asn Ala
        275                 280                 285
Gly Ile Gly Lys Lys Pro Gly Ser Val Gly Ser Ile Pro Val Arg Ile

290                 295                 300
Thr Thr Arg Trp Pro Arg Pro Leu Ser Arg Arg Val Thr Ser Thr His
305                 310                 315                 320
Arg Asn Ile Leu Gly
                325

<210> 357
<211> 951
<212> DNA
<213> Unknown

<220>
<223> Obtained from an environmental sample

<400> 357
atgacaaaac tagccatcgt acaaaaaccg ccagtctttc tggataagca aaaaaccatt      60
gagctggccg tcgccaacat tgaagaggcc gccgccaagg gtgccgatct cgtggtgttt     120
tctgaagctt tcattcccgg ctatcctgcc tggatctggc gtctacgccc cggcggtgac     180
tgggggcttt cagaagagtt gcaccagcgt ttgctgcgca atgccgtcaa tgtggactcc     240
gatgatctgg ctccgttgtt tgaggtcgcc cgcaagcacg aactcaccat cgtttgcggt     300
atcgaggagc gtgacaacaa actaagtcaa acaaccttat ataacaccgt catcacgatt     360
ggtcccgatg gatcgttact gaacaaacat cgcaagctta tgcccaccaa cccggagcga     420
atggtgtggg ggtttggtga cgcatccggt ttaaaagtcg ttgataccaa tgctggtcga     480
attggctcat taatgtgctg ggaaaattac atgccgctgg ctcgctatgc cctatatgca     540
caaggtgtcg agatctatat cgcaccgacc tacgacagcg gtgatggctg gataggcagc     600
atgcagcaca tcgcacgtga agggggctgt tgggtggtgg gatgtgggtg tctcatgaaa     660
ggcagtgata ttccagatga tttcccggag aaatccacgt tgtatccaga tgcagatgaa     720
tgggtgaacc cgggtgattc tgtagtgata gcacccggcg gtgaaattat ggccggccca     780
atgaacagag agtccggtat tttgtatcac gagctagaca gagaaaaagt cagcaacgct     840
aaacgagcat tcgatgttgc cgggcattat tcacgtcccg atatctttca gctgcatgta     900
aatacacagg agcagtcacc ctgcgtattc gaaaataatt ccataactta a              951

<210> 358
<211> 300
<212> PRT
<213> Unknown

<220>
<223> Obtained from an environmental sample

<400> 358
Met Thr Lys Leu Ala Ile Val Gln Lys Pro Pro Val Phe Leu Asp Lys
1               5                   10                  15
Gln Lys Thr Ile Glu Leu Ala Val Pro Thr Leu Lys Arg Pro Pro Pro
            20                  25                  30
Arg Val Pro Ile Ser Trp Cys Phe Leu Lys Leu Ser Phe Pro Ala Ile
        35                  40                  45
Leu Trp Ile Trp Arg Leu Arg Pro Gly Gly Asp Trp Gly Leu Ser Glu
    50                  55                  60
Glu Leu His Gln Arg Leu Leu Arg Asn Ala Val Asn Val Asp Ser Asp
65                  70                  75                  80
Asp Leu Ala Pro Leu Phe Glu Val Ala Arg Lys His Glu Leu Thr Ile
                85                  90                  95
Val Cys Gly Arg Gly Ala Gln Gln Thr Lys Ser Asn Asn Leu Ile His
            100                 105                 110
Arg His His Asp Trp Ser Arg Trp Ile Tyr Thr Asn Ile Ala Ser Leu
        115                 120                 125

Cys Pro Pro Thr Arg Ser Glu Trp Cys Gly Gly Leu Val Thr His Pro
    130                 135                 140
Leu Lys Val Val Asp Thr Asn Ala Gly Arg Ile Gly Ser Leu Met Cys
145                 150                 155                 160
Trp Glu Asn Tyr Met Pro Leu Ala Arg Tyr Ala Leu Tyr Ala Gln Gly
                165                 170                 175
Val Glu Ile Tyr Ile Ala Pro Thr Tyr Asp Ser Gly Asp Gly Trp Ile
            180                 185                 190
Gly Ser Ala Ala His Arg Thr Arg Gly Leu Leu Gly Gly Gly Met Trp
        195                 200                 205
Val Ser His Glu Arg Gln Tyr Ser Met Ile Ser Arg Arg Asn Pro Arg
    210                 215                 220
Cys Ile Gln Met Gln Met Asn Gly Thr Arg Val Ile Leu Thr Arg Arg
225                 230                 235                 240
Asn Tyr Gly Arg Pro Asn Glu Gln Arg Val Arg Tyr Phe Val Ser Arg
                245                 250                 255
Ala Arg Gln Arg Lys Val Ser Asn Ala Lys Arg Ala Phe Asp Val Ala
            260                 265                 270
Gly His Tyr Ser Arg Pro Asp Ile Phe Gln Leu His Val Ile His Arg
        275                 280                 285
Ser Ser His Pro Ala Tyr Ser Lys Ile Ile Pro Leu
    290                 295                 300

<210> 359
<211> 1029
<212> DNA
<213> Unknown

<220>
<223> Obtained from an environmental sample

<400> 359
atggcaaacg tcgttcgtgc tgcggccgta cagttgagcc ctgttttggg tagtcgcgag      60
ggtacggtag agaaggtagt tgctgcgatc cgtgacgccg cctcgcaggg cgcacagctg     120
tgcgttttcc cggagacggt tgttccctat tatccgtatt tctcgttcat tcggccgccc     180
gcggccatgg gcaaagacca catgcagctg tacgagcaag ctgtggtcgt gccttctccc     240
agcacgaacg cgattgccgc ggcggccaaa caacactcga tcgtcgtttc aatcggcgtc     300
aatgaacgcg atcacggtac gatatacaac acgcagttgt tgttcgatgc cgacgggaca     360
ctcgtgcaac ggcgtcgcaa gataaccccc acgttccacg agcgtatggt gtggggtcaa     420
ggtgatgggt cgggtttgcg ctgtgtcgac acacaaatcg ggcgcatcgg tagcctggct     480
tgttgggaac attacaatcc cttggcgcgc tacgcattga tggccgatca cgaagagatc     540
cacgtcgcca tgtttccggg ttcgatggtg ggtcagatct tcgccgatca aattcaggta     600
accattcgcc accacgcgct cgaaagcggc tgtttcgtcg tcaacgctac ggggtatctg     660
agcaaggaac aggtcgccca gttgtcacaa ggcacgtcgc tcgacgcggc gttgaccggt     720
ggttgttaca ccgcgattgt atcgcctgaa ggcgtcgtac tgggcgaacc gctcaccgac     780
ggcgaaggca tggtcgtggc ggatatggat ctcagcctca tcaccaaacg caaacgcatg     840
atggatagcg tcgggcacta cagtcgcccg gaattgctgt ctctgctgat caatcgaacg     900
ccaacccaca cggcggtcga cgtcgaattc aactccaatt ccgagtctca tcatgtcagc     960
aatacacgaa caccaaagcg cacaactggc ccacgttcga accttcaagt tgccgctgat    1020
cgcgagtaa                                                            1029

<210> 360
<211> 335
<212> PRT
<213> Unknown

<220>

<223> Obtained from an environmental sample

<400> 360
Met Ala Asn Val Val Arg Ala Ala Ala Val Gln Leu Ser Pro Val Leu
1               5                   10                  15
Gly Ser Arg Glu Gly Thr Val Glu Lys Val Val Ala Ala Ile Arg Asp
            20                  25                  30
Ala Ala Ser Gln Gly Ala Gln Leu Cys Val Phe Pro Glu Thr Val Val
        35                  40                  45
Pro Tyr Ser Val Phe Leu Val His Ser Ala Ala Arg Gly His Gly Gln
    50                  55                  60
Arg Pro His Ala Ala Val Arg Ala Ser Cys Gly Arg Ala Phe Ser Gln
65                  70                  75                  80
His Glu Arg Asp Cys Arg Gly Gly Gln Thr Thr Leu Asp Arg Arg Phe
                85                  90                  95
Asn Arg Arg Met Asn Ala Ile Thr Val Arg Tyr Thr Thr Arg Ser Cys
            100                 105                 110
Cys Ser Met Pro Thr Gly His Ser Cys Asn Gly Arg Lys Ile Thr Pro
        115                 120                 125
Thr Phe His Glu Arg Met Val Trp Gly Gln Gly Asp Gly Ser Gly Leu
    130                 135                 140
Arg Cys Val Asp Thr Gln Ile Gly Arg Ile Gly Ser Leu Ala Cys Trp
145                 150                 155                 160
Glu His Tyr Asn Pro Leu Ala Arg Tyr Ala Leu Met Ala Asp His Glu
                165                 170                 175
Glu Ile His Val Ala Met Phe Pro Gly Ser Met Val Gly Gln Ile Phe
            180                 185                 190
Ala Asp Gln Ile Gln Val Pro Phe Ala Thr Thr Arg Ser Lys Ala Ala
        195                 200                 205
Val Ser Ser Ser Thr Leu Arg Gly Ile Ala Arg Asn Arg Ala Gln Leu
    210                 215                 220
Ser Gln Gly Thr Ser Leu Asp Ala Ala Leu Thr Gly Gly Cys Tyr Thr
225                 230                 235                 240
Ala Ile Val Ser Pro Glu Gly Val Val Leu Gly Glu Pro Leu Thr Asp
                245                 250                 255
Gly Glu Gly Met Val Val Ala Asp Met Asp Leu Ser Leu Ile Pro Asn
            260                 265                 270
Ala Asn Ala Trp Ile Ala Ser Gly Thr Thr Val Ala Arg Asn Cys Cys
        275                 280                 285
Leu Cys Ser Ile Thr Pro Thr His Thr Ala Val Asp Val Glu Phe Asn
    290                 295                 300
Ser Asn Ser Glu Ser His His Val Ser Asn Thr Arg His Gln Ser Ala
305                 310                 315                 320
Gln Leu Ala His Val Arg Thr Phe Lys Leu Pro Leu Ile Ala Ser
                325                 330                 335

<210> 361
<211> 951
<212> DNA
<213> Unknown

<220>
<223> Obtained from an environmental sample

<400> 361
atgctaaacg agaacaatgg cgctactttc aaggttgctg ccgtgcaggc ttcaccggta      60
tttcttgatc gcgctgctac aatcgacaag gcttgcgatt taattgctac ggccggacgc     120

gagggggctc gcctgatcgt ctttccagaa gcgttcgtcc cggcctatcc tgattgggta 180
tgggcgattc ccgcgggtga tgagggcatg ctcaatgagc tgtatgcaga attacttgcc 240
aatgctgtca ccattcccag cgatgcgacc gagaggttgt gtcgcgcggc gcggcttgct 300
aatgcttacg tggtgatggg gatgagcgaa cgcaatgccg aagcgagtgg cgccagcctg 360
tataatacgc tgttgtatat tgatgcacag ggacaaatcc tgggaaagca ccggaagctg 420
gttccaacgg gcggcgagcg cctggtatgg gcacagggag atggcagcac cctggaggtt 480
tacgatactc ctttgggaaa actcggtggc ttaatctgct gggagaatta tatgccgctg 540
gcacgctata ctatgtatgc ctggggcacg caaatctaca ttgcagcgac gtgggatcgc 600
gggcagccat ggctatccac tttgcgacac attgctaaag agggcagagt atatgtgatc 660
ggctgttgta ttgctatgcg caaagatgat atccccgacc attacgcgat gaaggagaag 720
tattacgcgg aagaagacga gtggatcaat attggcgata gcgcaatcgt caatccagaa 780
ggggtattta ttgccgggcc agtgcgtaag caagaagaaa tcctctacgc cgaggttgac 840
ccgcgaatga tgcaggggcc aaagtggatg ctcgacgtgg caggacatta cgcgcgcccg 900
gatgtattcc agttgacggt gcacacggag aggcggcaga tgatccacta g 951

<210> 362
<211> 302
<212> PRT
<213> Unknown

<220>
<223> Obtained from an environmental sample

<400> 362
Met Leu Asn Glu Asn Asn Gly Ala Thr Phe Lys Val Ala Ala Val Gln
1               5                   10                  15
Ala Ser Pro Val Phe Leu Asp Arg Ala Tyr Asn Arg Gln Gly Leu Arg
            20                  25                  30
Phe Asn Cys Tyr Gly Arg Thr Arg Gly Gly Ser Pro Asp Arg Leu Ser
        35                  40                  45
Arg Ser Val Arg Pro Gly Leu Ser Leu Gly Met Gly Asp Ser Arg Gly
    50                  55                  60
Gly His Ala Gln Ala Val Cys Glu Leu Leu Ala Asn Ala Val Thr Ile
65                  70                  75                  80
Pro Ser Asp Ala Thr Glu Arg Leu Cys Arg Ala Ala Arg Leu Ala Asn
                85                  90                  95
Ala Tyr Val Val Met Gly Met Ser Glu Arg Asn Ala Glu Ala Ser Gly
            100                 105                 110
Ala Ser Leu Tyr Asn Thr Leu Leu Tyr Cys Thr Gly Thr Asn Pro Gly
        115                 120                 125
Lys Ala Pro Glu Ala Gly Ser Asn Gly Arg Arg Ala Pro Gly Met Gly
    130                 135                 140
Thr Gly Arg Trp Gln His Pro Gly Gly Leu Arg Tyr Ser Phe Gly Lys
145                 150                 155                 160
Thr Arg Trp Leu Asn Leu Leu Arg Ile Ile Cys Arg Trp His Ala Ile
                165                 170                 175
Leu Cys Met Pro Gly Ala Arg Lys Ser Thr Leu Gln Arg Arg Gly Ile
            180                 185                 190
Ala Gly Ser His Gly Tyr Pro Leu Cys Asp Thr Leu Leu Lys Arg Ala
        195                 200                 205
Glu Tyr Met Ser Ala Val Val Leu Tyr Ala Gln Arg Tyr Pro Arg Pro
    210                 215                 220
Leu Arg Asp Glu Gly Glu Val Leu Arg Gly Arg Arg Arg Val Asp Gln
225                 230                 235                 240
Tyr Trp Arg Arg Asn Arg Gln Ser Arg Arg Gly Ile Tyr Cys Arg Ala
                245                 250                 255
Ser Ala Ala Arg Arg Ile Leu Tyr Ala Glu Val Asp Pro Arg Met Met

260                 265                 270
Gln Gly Pro Lys Trp Met Leu Asp Val Ala Gly His Tyr Arg Ala Arg
        275                 280                 285
Met Tyr Ser Ser Arg Cys Thr Arg Arg Gly Gly Arg Ser Thr
    290                 295                 300

<210> 363
<211> 1053
<212> DNA
<213> Unknown

<220>
<223> Obtained from an environmental sample

<400> 363
atgccgaaaa agtcgaccgt ccgggtcgca gccgtccaga ttgcgccgga tctgacatcg      60
cgggaaaaga cggtggcacg cgtgatcgag gcgatcgccc aggcatccgc caaaggtgcg     120
gagcttgtgg tttttcccga gacctttgtg ccgtggtatc cttatttctc gttcgtgttg     180
cccccggtct tgtcgggcaa ggagcacctg cggctctacg aagaggcggt tgcggtgcca     240
agtgccgcca caagaagcgt agcggctgcc gctcgcgaac atggcatcgt cgtggcgctt     300
ggcgtcaacg agcgcgacta tggcacgctc tacaatacgc aactgctttt cgatgccgat     360
ggcagtctga tcctgaagcg gcgcaagatc accccgactt tccacgagcg gatgatctgg     420
ggccagggcg atgcctcagg cctgaaggtt gtcgacagcg ccattggccg catcggcgcg     480
ctggcctgct gggaacacta caatccgcta gcccgctatg cgctgatggc gcagcacgag     540
gaaatccaca ttgcgcagtt tcccggctcc atggtcgggc cgatctttgc cgatcagatg     600
gaggtgacga tccgccatca cgcgctggaa agcggctgct tcgtcgtcaa tgccacggga     660
tggctgacgg atgatcagat cgtctcgatc acaccggata ccggcctgca aaaagcgctg     720
cggggtggct gcatgacggc gatcatttcc cccgaaggca agcatctcgt gccgccgctc     780
accgaaggtg agggtatcct cgtcgccgat ctcgacatga gcctcattct caagcgcaag     840
cgcatgatgg attcggtcgg ccactatgcc cggcccgagt tgctgcacct cgtcatggac     900
gcccggccgg ctgcgccgat gagggaatcg tccatgccca ctgccttccc cggcgaaaca     960
ttgacaaccg acatgaccga tggagaacag gatgcgtctt tcgacggaaa cgctgatcaa    1020
cgaattgcag tccttcggag cccggctggt tga                                 1053

<210> 364
<211> 335
<212> PRT
<213> Unknown

<220>
<223> Obtained from an environmental sample

<400> 364
Met Pro Lys Lys Ser Thr Val Arg Val Ala Ala Val Gln Ile Ala Pro
1               5                   10                  15
Asp Leu Thr Ser Arg Glu Lys Gly Gly Thr Arg Asp Arg Gly Asp Arg
            20                  25                  30
Pro Gly Ile Arg Gln Arg Cys Gly Ala Cys Gly Phe Ser Arg Asp Leu
        35                  40                  45
Cys Arg Gly Ile Leu Ile Ser Arg Ser Cys Cys Pro Arg Ser Cys Arg
    50                  55                  60
Ala Arg Ser Thr Cys Gly Ser Thr Lys Arg Arg Leu Arg Cys Gln Val
65                  70                  75                  80
Pro Pro Gln Glu Ala Arg Leu Pro Leu Ala Asn Met Ala Ser Ser Trp
                85                  90                  95
Arg Leu Ala Ser Thr Ser Ala Thr Met Ala Arg Ser Thr Ile Arg Asn
            100                 105                 110

Cys Phe Ser Met Pro Met Ala Val Ser Ser Gly Ala Arg Ser Pro Arg
        115                 120                 125
Leu Ser Thr Ser Gly Ser Gly Ala Arg Ala Met Pro Gln Ala Glu Gly
    130                 135                 140
Cys Arg Gln Arg His Trp Pro His Arg Arg Ala Gly Leu Leu Gly Thr
145                 150                 155                 160
Leu Gln Ser Ala Ser Pro Tyr Ala Leu Met Ala Gln His Glu Glu Ile
                165                 170                 175
His Ile Ala Gln Phe Pro Gly Ser Met Val Gly Pro Ile Phe Ala Asp
            180                 185                 190
Gln Met Glu Val Thr Ile Arg His His Ala Leu Glu Ser Gly Cys Phe
        195                 200                 205
Val Val Asn Ala Thr Gly Trp Arg Met Ile Arg Ser Ser Arg Ser His
    210                 215                 220
Arg Ile Pro Ala Cys Lys Lys Arg Cys Gly Val Ala Ala Gly Asp His
225                 230                 235                 240
Phe Pro Arg Arg Gln Ala Ser Arg Ala Ala Ala His Arg Arg Gly Tyr
                245                 250                 255
Pro Arg Arg Arg Ser Thr Ala Ser Phe Ser Ser Ala Ser Ala Trp Ile
            260                 265                 270
Arg Ser Ala Thr Met Pro Gly Pro Ser Cys Cys Thr Ser Ser Trp Thr
        275                 280                 285
Pro Gly Arg Leu Arg Arg Gly Asn Arg Pro Cys Pro Leu Pro Ser Pro
    290                 295                 300
Ala Lys Ile Asp Asn Arg His Asp Arg Trp Arg Thr Gly Cys Val Phe
305                 310                 315                 320
Arg Arg Lys Arg Ser Thr Asn Cys Ser Leu Arg Ser Pro Ala Gly
                325                 330                 335

<210> 365
<211> 975
<212> DNA
<213> Unknown

<220>
<223> Obtained from an environmental sample

<400> 365
atgacaaagc tagccatcgt tcaaaaaccc cccgtctttc tggataaaga aaaaaccata      60
gcgaagacgg ttgattccat aaaagaggcc gcgacacaaa atgccgactt ggtcatcttc     120
accgaagcct tcatcccggg ctaccccacc tggatatggc gacttaggcc aggcgctgat     180
tggggcctct cagaagagct gcacgagcag ttattgcgta acgcggtgag tatgggatcg     240
accgacctgg atccgcttta tgaagccgcc caacagcata acgtcactat tgtttgcggc     300
atcgtagaaa gagaccacca actcagccaa tcaaccctct acaacagcat ggtcgtcatt     360
gacacagacg gaacccttct caacaagcac cgcaaactca tgccaactaa tcccgaacgc     420
atggtgtggg gctttggcga cgcctcagga ctcaaagccg ttgcaacacc tgcaggccgc     480
atcagcacgt tgttgtgttg ggagaactac atgccattgg cccgatatgc tctgtatgca     540
caaggcgtgg aaatctatat cgcgccaact tacgacagtg gtgcgggttg gataggaagc     600
ttgcaacaca tagcacgcga aggtcgatgc tgggtcgtgg gctgtggcaa cctgattcag     660
gccagtgatc tgcctgaaga cttcccggac aaggacaacc tctacccgga cgcagaagag     720
tgggtgaacc cgggtgactc catagtcatt gcaccagacg gtgagattgt ggccggtcca     780
atgcacaaag agacaggaat tttgtactgc gagatagatc tggagaaagt cagaattgca     840
aaacgagcat tagacgtgac cgggcattat tcgcgaccgg acgttttcaa actgcatgtg     900
aatacccgac ctcaatcacc tgtggaattt gaaggtcagg agaccaacaa tccaacaaca     960
ggagaaagct catga                                                      975

<210> 366

<211> 315
<212> PRT
<213> Unknown

<220>
<223> Obtained from an environmental sample

<400> 366
Met Thr Lys Leu Ala Ile Val Gln Lys Pro Pro Val Phe Leu Asp Lys
1               5                   10                  15
Glu Lys Thr Ile Ala Lys Thr Val Ile Pro Lys Arg Pro Arg His Lys
            20                  25                  30
Met Pro Thr Trp Ser Ser Ser Pro Lys Pro Ser Ser Arg Ala Thr Thr
        35                  40                  45
Trp Ile Trp Arg Leu Arg Pro Gly Ala Asp Trp Gly Leu Ser Glu Glu
    50                  55                  60
Leu His Glu Gln Leu Leu Arg Arg Gly Glu Tyr Gly Ile Asp Arg Pro
65                  70                  75                  80
Gly Ser Ala Leu Ser Arg Pro Thr Ala Arg His Tyr Cys Leu Arg His
                85                  90                  95
Arg Arg Lys Arg Pro Pro Thr Gln Pro Ile Asn Pro Leu Gln Gln His
            100                 105                 110
Gly Arg His His Arg Arg Asn Pro Ser Gln Gln Ala Pro Gln Thr His
        115                 120                 125
Ala Asn Ser Arg Thr His Gly Val Gly Leu Trp Arg Ala Ser Gly Leu
    130                 135                 140
Lys Ala Val Ala Thr Pro Ala Gly Arg Ile Ser Thr Leu Leu Cys Trp
145                 150                 155                 160
Glu Asn Tyr Met Ile Gly Pro Ile Cys Ser Val Cys Thr Arg Arg Gly
                165                 170                 175
Asn Leu Tyr Arg Ala Asn Leu Arg Gln Trp Cys Gly Leu Asp Arg Lys
            180                 185                 190
Leu Ala Thr His Ser Thr Arg Arg Ser Met Leu Gly Arg Gly Leu Trp
        195                 200                 205
Gln Pro Asp Ser Gly Ser Asp Leu Pro Glu Asp Phe Pro Asp Lys Asp
    210                 215                 220
Asn Leu Tyr Pro Asp Ala Glu Glu Trp Val Asn Pro Gly Asp Ser Ile
225                 230                 235                 240
Val Ile Ala Pro Asp Gly Glu Ile Val Ala Gly Pro Met His Lys Glu
                245                 250                 255
Thr Gly Ile Leu Tyr Arg Asp Arg Ser Gly Glu Ser Gln Asn Cys Lys
            260                 265                 270
Thr Ser Ile Arg Arg Asp Arg Ala Leu Phe Ala Thr Gly Arg Phe Gln
        275                 280                 285
Thr Ala Cys Glu Tyr Pro Thr Ser Ile Thr Cys Gly Ile Arg Ser Gly
    290                 295                 300
Asp Gln Gln Ser Asn Asn Arg Arg Lys Leu Met
305                 310                 315

<210> 367
<211> 981
<212> DNA
<213> Unknown

<220>
<223> Obtained from an environmental sample

<400> 367
atgactgccc actttgccga taccctgacc gttgccgtgg cccagatcgc gccggtgtgg 60
ctacagcgcg aagctaccct gcaaaagatg ctggtctggg tggagcgcgc cgcgcgcgaa 120
ggcgcgggcc tggtcgcctt cagtgagggc ctgctgcctg gctacccctt ctggattgaa 180
cacacggacg gcgcccgctt cgagtcgccg ttacagaagc gcctgtacgc ccactactgc 240
gatcagtcgg tccagatcaa tgctgggcac ctcgcaccac tctgcgctgc cgcggccaga 300
caccagattt gggtggtctg cggcgtcatc gagcgcgaca gcgcacgcgg actcagcgtg 360
tttgcatcaa tggtcaccat cgatgccgaa ggcgcgatcc gcagtgtgca ccgcaagctg 420
atgccgacct acgaagaacg cctggtgtgg tcgcccggcg acgcgcacgg actgcgctgc 480
catccgctcg gccagttccg cctcggcagc ctcaattgct gggagaactg gatgccgctg 540
gcgcgcgccg ccctgtacgc ccagggcgag tctttgcatg ttgcatcctg gcccggcagt 600
cgccgcaaca ccgagaccat tactcccttc atcgcccgcg aaggccgcag ttacgcgctg 660
tccgccagtt ccgtgctgca ccgggatgat ctgcccgact ccgttccggc gctgtcggtg 720
ctgcgcgact gcctgccgga cgtgatggcc gacggcggct cctgcgtcgc cggccccgac 780
ggacatttcc tgatcgagcc ggtcgtcggc cgggaagagc tgctgctcgc gcagatcgat 840
catgcccggg tacgcgagga acgtcagaac ttcgacccct tcggccacta ctcgcggccg 900
gaactcctgt cgctggtggt ggatacgcgc cgggcgagcg gagtgcagat agtgaatgct 960
gaccatggct ttaagccctg a 981

<210> 368
<211> 317
<212> PRT
<213> Unknown

<220>
<223> Obtained from an environmental sample

<400> 368
Met Thr Ala His Phe Ala Asp Thr Leu Thr Val Ala Val Ala Gln Ile
1               5                   10                  15
Ala Pro Val Trp Leu Gln Arg Glu Tyr Pro Ala Lys Asp Ala Gly Leu
            20                  25                  30
Gly Gly Ala Arg Arg Ala Arg Arg Arg Gly Pro Gly Arg Leu Gln Gly
        35                  40                  45
Pro Ala Ala Trp Leu Pro Leu Leu Asp Thr His Gly Arg Arg Pro Leu
    50                  55                  60
Arg Val Ala Val Thr Glu Ala Cys Thr Pro Thr Thr Ala Ile Ser Arg
65                  70                  75                  80
Ser Arg Ser Met Leu Gly Thr Ser His His Ser Ala Leu Pro Ala Arg
                85                  90                  95
His Gln Ile Trp Val Val Cys Gly Val Ile Glu Arg Asp Ser Ala Arg
            100                 105                 110
Gly Leu Ser Val Phe Ala Gln Trp Ser Pro Ser Met Pro Lys Ala Arg
        115                 120                 125
Ser Ala Val Cys Thr Ala Ser Cys Arg Pro Thr Lys Asn Ala Trp Cys
    130                 135                 140
Gly Arg Pro Ala Thr Arg Thr Asp Cys Ala Ala Ile Arg Ser Ala Ser
145                 150                 155                 160
Ser Ala Ser Ala Ala Gln Leu Leu Gly Glu Leu Asp Ala Ala Gly Ala
                165                 170                 175
Arg Arg Pro Val Arg Pro Gly Arg Val Phe Ala Cys Cys Ile Leu Ala
            180                 185                 190
Arg Gln Ser Pro Gln His Arg Asp His Tyr Ser Leu His Arg Pro Arg
        195                 200                 205
Arg Pro Gln Leu Arg Ala Ser Ala Ser Ser Val Leu His Arg Asp Asp
    210                 215                 220
Leu Pro Asp Ser Val Pro Ala Leu Ser Val Leu Arg Asp Cys Leu Arg

225                 230                 235                 240
Thr Trp Pro Thr Ala Ala Pro Ala Ser Pro Ala Pro Thr Asp Ile Ser
                245                 250                 255
Ser Ser Arg Ser Ser Pro Gly Arg Ala Ala Ala Arg Ala Asp Arg Ser
            260                 265                 270
Cys Pro Gly Thr Arg Gly Thr Ser Glu Leu Arg Pro Leu Gly His Tyr
        275                 280                 285
Ser Arg Pro Glu Leu Leu Ser Leu Val Val Asp Thr Arg Arg Ala Ser
    290                 295                 300
Gly Val Gln Ile Val Asn Ala Asp His Gly Phe Lys Pro
305                 310                 315

<210> 369
<211> 1074
<212> DNA
<213> Unknown

<220>
<223> Obtained from an environmental sample

<400> 369
atgtccgaag agaacaccac caccgaatca agctcggacg cgatctggac cgttgcggcc      60
gtccaggcgg cgccggtgtt cttgaatcgg gatgcgacgg tgcgtaaggc cgtctcgctg     120
attgccgagg ccgcggcgca cggcgcgcgt ctcattgttt ttcccgaggc gttcattcca     180
tcgtacccgg attgggcgtg ggcggtcccg cccggacagg gcggaaccaa ctcgagactg     240
tatgccaaac tgctcgacaa ttcggttacg gtgcccagcc cagccaccga tgccctggcc     300
agggcggctc gcgacgcggg cgcctacgtc gtcatgggca taaacgagcg gaacacggcg     360
gcgagcggcg gaagtctcta caacagcttg ctctatattg gtccggacgg tcgcatcctg     420
ggcattcatc ggaagttggt gccgacctcg gcggagcggc tgatctgggc acaaggcgat     480
ggaagcacgc ttggcgtgtt cgatacgccg ggcggccggc ttggcggctt gatctgctgg     540
gaaaactata tgccgctggc ccgatattcg atgtacgcgc gcggcgtgca aatatatgtt     600
gcggcgacct gggacagggg cgagccatgg ctttcgacgc tgcgacacat agccaaggaa     660
ggccagacct acgtgatagg ctgttgcatc gccatgcgga cggccgacat cgacgacgcc     720
gagctcgtcg agaagtatta cgcggacgcc ggtgagtgga tcaatgaagg cgacagcgcg     780
attgtcgatc cgagcggaac aatcattgcc ggtccggccc atcagaccaa tgaaatcctc     840
tacgccgcga tcgatcgcca gaaggtgctg gaatcaaaat ggatgttgga cgtggccggg     900
cactacgcgc gtccggacgt gttttcattt ggcgttcgaa ccgatgccaa cccgataatg     960
accatgaacg aaccaagcgc gacggccgag ccgaggcaca atagcgcggg agccgagggt    1020
cgcgacggcc tacgcgggcg tcgtgaccct cgctcgagaa ttcggcagat gtag          1074

<210> 370
<211> 346
<212> PRT
<213> Unknown

<220>
<223> Obtained from an environmental sample

<400> 370
Met Ser Glu Glu Asn Thr Thr Thr Glu Ser Ser Ser Asp Ala Ile Trp
 1               5                  10                  15
Thr Val Ala Ala Val Gln Ala Ala Gly Val Leu Glu Ser Gly Cys Asp
            20                  25                  30
Gly Ala Gly Arg Leu Ala Asp Cys Arg Gly Arg Gly Ala Arg Ala Arg
        35                  40                  45
Leu Ile Val Phe Pro Glu Ala Phe Ile Pro Ser Tyr Pro Asp Trp Ala
    50                  55                  60

Trp Ala Val Pro Pro Gly Gln Ala Glu Pro Thr Arg Asp Cys Met Pro
65                  70                  75                  80
Asn Cys Ser Thr Ile Arg Leu Arg Cys Pro Ala Gln Pro Pro Met Pro
                85                  90                  95
Gly Gln Gly Gly Ser Arg Arg Gly Arg Leu Arg Arg His Gly His Lys
            100                 105                 110
Arg Ala Glu His Gly Gly Ser Gly Gly Ser Leu Tyr Asn Ser Leu Leu
        115                 120                 125
Tyr Ile Gly Pro Asp Gly Arg Ile Leu Gly Ile His Arg Lys Leu Cys
    130                 135                 140
Arg Pro Arg Arg Ser Gly Ser Gly His Lys Ala Met Glu Ala Arg Leu
145                 150                 155                 160
Ala Cys Ser Ile Arg Arg Ala Ala Gly Leu Ala Ala Ser Ala Gly Lys
                165                 170                 175
Thr Ile Cys Arg Trp Pro Asp Ile Arg Cys Thr Arg Ala Val Gln Ile
            180                 185                 190
Tyr Val Ala Ala Thr Trp Asp Arg Gly Glu Pro Trp Leu Ser Thr Leu
        195                 200                 205
Arg His Ile Ala Lys Lys Ala Arg Pro Thr Ala Val Ala Ser Pro Cys
    210                 215                 220
Gly Arg Pro Thr Ser Thr Thr Pro Ser Ser Ser Arg Ser Ile Thr Arg
225                 230                 235                 240
Thr Pro Val Ser Gly Ser Met Lys Ala Thr Ala Arg Leu Ser Ile Arg
                245                 250                 255
Ala Glu Ile Ile Ala Gly Pro Ala His Gln Thr Asn Glu Ile Leu Tyr
            260                 265                 270
Ala Ala Ile Asp Arg Gln Lys Val Leu Glu Lys Met Asp Val Gly Arg
        275                 280                 285
Gly Arg Ala Leu Arg Ala Ser Gly Arg Val Phe Ile Trp Arg Ser Asn
    290                 295                 300
Arg Cys Gln Pro Asp Asn Asp His Glu Arg Thr Lys Arg Asp Gly Arg
305                 310                 315                 320
Ala Glu Ala Gln Arg Gly Ser Arg Val Ala Thr Ala Tyr Ala Gly Val
                325                 330                 335
Val Thr Leu Ala Arg Glu Phe Gly Arg Cys
            340                 345

<210> 371
<211> 1014
<212> DNA
<213> Unknown

<220>
<223> Obtained from an environmental sample

<400> 371
atgggtatcg aacatccgaa gtacaaggtt gcggtggtac aggcggcgcc ggcctggctc      60
gatctcgacg catcgatcgc caagaccatc gcgctgatcg aggaggcggc cgccaagggc     120
gccaagctga tcgcattccc tgaggccttc attcccggct atccttggca tatctggatg     180
gactcgccgg cctgggcgat cgggcgcgga tttgtgcagc gctatttcga caattcgctt     240
tcctacgaca gcccgcaggc cgaacggctg cggctcgcgg tgaagaaggc cggcatcacc     300
gccgtgctcg gcctgtccga gcgggaaggc ggcagccttt atctcgcgca atggctgatc     360
ggtcccgacg gcgagaccat cgccaagcgg cgcaagctgc ggccgacgca tgccgagcgc     420
accgtctacg gcgaaggcga cggcagtgac cttgcggtcc atgaccgcgc tgacattggc     480
cgtctcggcg cgctgtgctg ctgggaacat ctgcagccgc tgtcgaaata cgccatgtat     540
gcccagaacg agcaggtgca tgtcgcggcc tggccgagtt tttcgctgta cgacccgttc     600
gcgccggcgc tgggctggga ggtcaacaac gcggcatccc gcgtctatgc ggtcgaaggc     660

tcctgcttcg tgctggcgcc gtgtgccacc gtctcgcagg cgatggtgga cgaactctgc 720
gaccgcgacg acaagcatgc gctgctgcat gtcggcggcg gccacgccgc gatctacgga 780
ccggacggca gctcgatggc gaacaagctc gatcccgagc aggagggcct gctgttcgcc 840
gacatcgatc tcggggcgat cggggtggca aagaacgccg ccgatccggc cgggcactat 900
tcgcggccgg atgtgacccg tctgctcttg aacagaaaac cctcaaagcg cgtcgagcac 960
tttgcgctgc cgctcgacca tctcgcggac gagggcgttg ctccggtgac ctga 1014

<210> 372
<211> 327
<212> PRT
<213> Unknown

<220>
<223> Obtained from an environmental sample

<400> 372
Met Gly Ile Glu His Pro Lys Tyr Lys Val Ala Val Val Gln Ala Ala
1               5                   10                  15
Pro Ala Trp Leu Asp Leu Asp Ala Arg Ser Pro Arg Pro Ser Arg Ser
            20                  25                  30
Arg Arg Arg Pro Pro Arg Ala Pro Ser Ser His Ser Leu Ala Phe Ile
        35                  40                  45
Pro Gly Tyr Pro Trp His Ile Trp Met Asp Ser Pro Ala Trp Ala Ile
    50                  55                  60
Gly Arg Gly Phe Val Gln Ala Ile Ser Thr Ile Arg Phe Pro Thr Thr
65                  70                  75                  80
Ala Arg Arg Pro Asn Gly Cys Gly Ser Arg Arg Arg Pro His His Arg
                85                  90                  95
Arg Ala Arg Pro Val Arg Ala Gly Arg Arg Gln Pro Leu Ser Arg Ala
            100                 105                 110
Met Ala Asp Arg Ser Thr Ala Arg Pro Ser Pro Ser Gly Ala Ser Cys
        115                 120                 125
Gly Arg Arg Met Pro Ser Ala Pro Ser Thr Ala Lys Ala Thr Ala Val
    130                 135                 140
Thr Leu Arg Ser Met Thr Ala Leu Thr Leu Ala Val Ser Ala Arg Cys
145                 150                 155                 160
Ala Ala Gly Asn Leu Gln Pro Leu Ser Lys Tyr Ala Met Tyr Ala Gln
                165                 170                 175
Asn Glu Gln Val His Val Ala Ala Trp Pro Ser Phe Ala Val Arg Pro
            180                 185                 190
Val Arg Ala Gly Ala Gly Leu Gly Gly Gln Gln Arg Gly Ile Pro Arg
        195                 200                 205
Leu Cys Gly Arg Gly Ser Cys Phe Val Leu Ala Pro Cys Ala Thr Val
    210                 215                 220
Ser Gln Ala Met Val Asp Glu Leu Cys Asp Arg Asp Gln Ala Cys Ala
225                 230                 235                 240
Ala Ala Cys Arg Arg Arg Pro Arg Arg Asp Leu Arg Thr Gly Arg Gln
                245                 250                 255
Leu Asp Gly Thr Ser Ser Ile Pro Ser Arg Arg Ala Cys Cys Ser Pro
            260                 265                 270
Thr Ser Ile Ser Gly Arg Ser Gly Trp Gln Arg Thr Pro Pro Ile Arg
        275                 280                 285
Pro Gly Thr Ile Arg Gly Arg Met Pro Val Cys Ser Thr Glu Asn Pro
    290                 295                 300
Gln Ala Arg Arg Ala Leu Cys Ala Ala Ala Arg Pro Ser Arg Gly Arg
305                 310                 315                 320
Gly Arg Cys Ser Gly Asp Leu

                325

<210> 373
<211> 1056
<212> DNA
<213> Unknown

<220>
<223> Obtained from an environmental sample

<400> 373
atgacgaatt ttctggacgt gacggtggca gcggttcagg cggctcccgt ctatttcgat 60
cgggaggcat cgacggagaa agcgtgccgg ttgatccacg aagcggcagg gctcggcgcg 120
acgctcgcag cgttcggcga aacctggttg ccagggtatc cgttcttcgt ctgggggttc 180
gcgcacaacc ggagcctgtt ctggcaggcc gccgccgagt acatcgccaa tgcggtggag 240
attccgagtc ccacaacgga ccgtctctgt gcggcggcga aggctgccgg ggtcgacgtc 300
gtcattggcg tcgttgaact ggatgaacga acacgagctt cggtttacag tacgctgctt 360
ttcatcggtc gcgacgggac gatcctgggc cgccaccgca agctgaagcc aacacacatg 420
gagcggacga tctggggcga aggggacgca tatggactcc gcgtctacga acgttcgtac 480
gggcggctga gcggcctgaa ttgctgggaa cacaatatga tgctgcccgg ctacgtgctt 540
gccgcacagg gcacgcagtt tcacgtcgcc gcatggcccg gaaaggagag gctcaccgtc 600
ccgccgaacg aagcggctta tacgcgccag cttctcctct ctcgcgcgta tgcatcccag 660
gccggcgcgt acgtgatcag cgtcgccggg ctgctcgcac cagactccat gcccgagcgt 720
tatcgcgagt tagggcggtc atatgagttg accggcgaca gcgtcatcgt cgacccgcgc 780
ggcgaggtca ttgccgggcc tgcaaaaggc gagaccatcc tgctcgcgca gtgcagtcag 840
gaagctctcc tcgcggccaa gtccgccatc gacctcggcg gccattactc acgcccggat 900
atctttcagc tgcgtgtcaa cgatcaactg cagcatcggg tccggagagt tgagccacac 960
ttcacggcgg cgatcggaca tatcggagcc gagcgccgat cccaggagga tggtactggt 1020
cccttcgacc tggcggaatc tctcacgaac tcctag 1056

<210> 374
<211> 351
<212> PRT
<213> Unknown

<220>
<223> Obtained from an environmental sample

<400> 374
Met Thr Asn Phe Leu Asp Val Thr Val Ala Ala Val Gln Ala Ala Pro
1               5                   10                  15
Val Tyr Phe Asp Arg Glu Ala Ser Thr Glu Lys Ala Cys Arg Leu Ile
            20                  25                  30
His Glu Ala Ala Gly Leu Gly Ala Thr Leu Ala Ala Phe Gly Glu Thr
        35                  40                  45
Trp Leu Pro Gly Tyr Pro Phe Phe Val Trp Gly Phe Ala His Asn Arg
    50                  55                  60
Ser Leu Phe Trp Gln Ala Ala Ala Glu Tyr Ile Ala Asn Ala Val Glu
65                  70                  75                  80
Ile Pro Ser Pro Thr Thr Asp Arg Leu Cys Ala Ala Ala Lys Ala Ala
                85                  90                  95
Gly Val Asp Val Val Ile Gly Val Val Glu Leu Asp Glu Arg Thr Arg
            100                 105                 110
Ala Ser Val Tyr Ser Thr Leu Leu Phe Ile Gly Arg Asp Gly Thr Ile
        115                 120                 125
Leu Gly Arg His Arg Lys Leu Lys Pro Thr His Met Glu Arg Thr Ile
    130                 135                 140

Trp Gly Glu Gly Asp Ala Tyr Gly Leu Arg Val Tyr Glu Arg Ser Tyr
145                 150                 155                 160
Gly Arg Leu Ser Gly Leu Asn Cys Trp Glu His Asn Met Met Leu Pro
                165                 170                 175
Gly Tyr Val Leu Ala Ala Gln Gly Thr Gln Phe His Val Ala Ala Trp
            180                 185                 190
Pro Gly Lys Glu Arg Leu Thr Val Pro Pro Asn Glu Ala Ala Tyr Thr
        195                 200                 205
Arg Gln Leu Leu Leu Ser Arg Ala Tyr Ala Ser Gln Ala Gly Ala Tyr
    210                 215                 220
Val Ile Ser Val Ala Gly Leu Leu Ala Pro Asp Ser Met Pro Glu Arg
225                 230                 235                 240
Tyr Arg Glu Leu Gly Arg Ser Tyr Glu Leu Thr Gly Asp Ser Val Ile
                245                 250                 255
Val Asp Pro Arg Gly Glu Val Ile Ala Gly Pro Ala Lys Gly Glu Thr
            260                 265                 270
Ile Leu Leu Ala Gln Cys Ser Gln Glu Ala Leu Leu Ala Ala Lys Ser
        275                 280                 285
Ala Ile Asp Leu Gly Gly His Tyr Ser Arg Pro Asp Ile Phe Gln Leu
    290                 295                 300
Arg Val Asn Asp Gln Leu Gln His Arg Val Arg Arg Val Glu Pro His
305                 310                 315                 320
Phe Thr Ala Ala Ile Gly His Ile Gly Ala Glu Arg Arg Ser Gln Glu
                325                 330                 335
Asp Gly Thr Gly Pro Phe Asp Leu Ala Glu Ser Leu Thr Asn Ser
            340                 345                 350

<210> 375
<211> 939
<212> DNA
<213> Unknown

<220>
<223> Obtained from an environmental sample

<400> 375
atgaccaaaa tcgctgtcat tcaagaaccc ccggtctatc tgaatctgag caaatcgatg      60
gaccgagcgg tcgacttgat tgccgatgcc gcgagccagg ggtgtcagtt gattgtgttt     120
cccgaagcct ggcttgcagg ttaccccacc ttcgtctggc gtcttgcgcc gggcagcgga     180
atgggaaaaa ccgatgagct ttacgcgcgt ttgctcgcca actcggtcga ccgcagcaaa     240
gaggggcttc ggcctttgca ggaggctgca aaagagcatg gcgttgtcat tgttctgggt     300
tatcaagagg tggacggctc gggcagcagc agcacaatct tcaacagctg cgcgattatt     360
gatgccgacg ggcgactggc caacaaccat cgcaagttga tgcccaccaa tgcggagcgg     420
atggtgtggg ggtttggcga cggttcgggc ctgaacgttg ttgacaccgc ggtgggcagg     480
atcggcacgc tgatttgctg ggaaaactac atgcccttgg cgcgctacgc gctgtatgcc     540
caaaacatcg aaatctatgt ggcgccgacc tgggacagcg gtgccatgtg gcaagccacc     600
ctgcaacata tcgcacgtga aggtggctgc tgggtcatcg gatgtgcaac ctcgctgcaa     660
gcctctgaca tcccggacga ccttccccat cgggatgagt tattcccgaa caaagacgaa     720
tgggtgaacc ctggcgatgc ggtggtttac aaacctttgg gcggccttgt ggccggcccc     780
atgcatcagg aaaaggggct tctcatcgca gagttggacg tcgccgctgt tcaggtctca     840
cgtcggaagt tcgatgcgac cgggcattac gctcgccccg atgtcttcca actgcacgtg     900
aatcgcagcg cgatgcggcc ggttgagttc acgaattag                            939

<210> 376
<211> 312
<212> PRT
<213> Unknown

<220>
<223> Obtained from an environmental sample

<400> 376
Met Thr Lys Ile Ala Val Ile Gln Glu Pro Pro Val Tyr Leu Asn Leu
1               5                   10                  15
Ser Lys Ser Met Asp Arg Ala Val Asp Leu Ile Ala Asp Ala Ala Ser
            20                  25                  30
Gln Gly Cys Gln Leu Ile Val Phe Pro Glu Ala Trp Leu Ala Gly Tyr
        35                  40                  45
Pro Thr Phe Val Trp Arg Leu Ala Pro Gly Ser Gly Met Gly Lys Thr
    50                  55                  60
Asp Glu Leu Tyr Ala Arg Leu Leu Ala Asn Ser Val Asp Arg Ser Lys
65                  70                  75                  80
Glu Gly Leu Arg Pro Leu Gln Glu Ala Ala Lys Glu His Gly Val Val
                85                  90                  95
Ile Val Leu Gly Tyr Gln Glu Val Asp Gly Ser Gly Ser Ser Ser Thr
            100                 105                 110
Ile Phe Asn Ser Cys Ala Ile Ile Asp Ala Asp Gly Arg Leu Ala Asn
        115                 120                 125
Asn His Arg Lys Leu Met Pro Thr Asn Ala Glu Arg Met Val Trp Gly
    130                 135                 140
Phe Gly Asp Gly Ser Gly Leu Asn Val Val Asp Thr Ala Val Gly Arg
145                 150                 155                 160
Ile Gly Thr Leu Ile Cys Trp Glu Asn Tyr Met Pro Leu Ala Arg Tyr
                165                 170                 175
Ala Leu Tyr Ala Gln Asn Ile Glu Ile Tyr Val Ala Pro Thr Trp Asp
            180                 185                 190
Ser Gly Ala Met Trp Gln Ala Thr Leu Gln His Ile Ala Arg Glu Gly
        195                 200                 205
Gly Cys Trp Val Ile Gly Cys Ala Thr Ser Leu Gln Ala Ser Asp Ile
    210                 215                 220
Pro Asp Asp Leu Pro His Arg Asp Glu Leu Phe Pro Asn Lys Asp Glu
225                 230                 235                 240
Trp Val Asn Pro Gly Asp Ala Val Val Tyr Lys Pro Phe Gly Gly Leu
                245                 250                 255
Val Ala Gly Pro Met His Gln Glu Lys Gly Leu Leu Ile Ala Glu Leu
            260                 265                 270
Asp Val Ala Ala Val Gln Val Ser Arg Arg Lys Phe Asp Ala Thr Gly
        275                 280                 285
His Tyr Ala Arg Pro Asp Val Phe Gln Leu His Val Asn Arg Ser Ala
    290                 295                 300
Met Arg Pro Val Glu Phe Thr Asn
305                 310

<210> 377
<211> 1050
<212> DNA
<213> Unknown

<220>
<223> Obtained from an environmental sample

<400> 377
atgtccagca ccacccatcc ccgcctgcgc gtcgccgcgg tgcaagccgc ccccgtcttc      60
ctcgacctcg acggcaccat cgacaagacc atcgacctga tggcccgggc ggccggccag     120

ggcgtgcagc tgatcgcctt tcccgagacc tgggtgcccg gctatccgtg gtggatctgg 180
ctcgattcgc cggcctgggg catgcagttc gtgcagcgct accacgacaa cgccctggtc 240
gtcggctcgc ccgagttcga ccgcattcgc gaggccgcgc gcaagcaccg catctgggtc 300
tcgctcggct acagcgagaa ggccgccggc agcctctaca tcgcccaggc gctgatcgac 360
gaccagggca acacgctgca gactcggcgc aagctcaagc cgacgcacgt ggagcgcacc 420
gtgttcggcg agggcgacgg atcggacctg agcgtggtcg agacggctat cggcaacatc 480
ggctcgctgt cgtgctggga gcacctgcag ccgctcagca agtacgcgat gtactcgcag 540
aacgagcaga tccattgcgg cgcctggccc agcttctcgc tctaccgcgg cggcgcctac 600
gcgctcggcg ccgaagtgaa caacgccgcc agccaggtgt acgcggccga gggccagtgc 660
ttcgtgatcg cgccctgcgc cacggtctcg aaggcgatgc acgaactgct gtgcaccgac 720
cctggcaagc agcagatgct gctggtcggc ggcggcttcg cgcgcatcta cggacccgac 780
ggatcgccgc tcggcaagaa cctggcagag gacgaggaag ggctggtggt ggccgacatc 840
gacctcggca tgatctccct ggccaaggcg gccggcgacc cggccggcca ctattcgcgg 900
cccgacgtga cgcagttgct gttcaacagg aagcggcgcg agccggtggt gctgcaaggc 960
ccggccgagc ccgagaaggc ggtcgccgag ccggtgtcca cgccgagcga agcggcggcg 1020
gccgccgccc gccagccggt cgtggcctga 1050

<210> 378
<211> 349
<212> PRT
<213> Unknown

<220>
<223> Obtained from an environmental sample

<400> 378
Met Ser Ser Thr Thr His Pro Arg Leu Arg Val Ala Ala Val Gln Ala
1               5                   10                  15
Ala Pro Val Phe Leu Asp Leu Asp Gly Thr Ile Asp Lys Thr Ile Asp
            20                  25                  30
Leu Met Ala Arg Ala Ala Gly Gln Gly Val Gln Leu Ile Ala Phe Pro
        35                  40                  45
Glu Thr Trp Val Pro Gly Tyr Pro Trp Trp Ile Trp Leu Asp Ser Pro
    50                  55                  60
Ala Trp Gly Met Gln Phe Val Gln Arg Tyr His Asp Asn Ala Leu Val
65                  70                  75                  80
Val Gly Ser Pro Glu Phe Asp Arg Ile Arg Glu Ala Ala Arg Lys His
                85                  90                  95
Arg Ile Trp Val Ser Leu Gly Tyr Ser Glu Lys Ala Ala Gly Ser Leu
            100                 105                 110
Tyr Ile Ala Gln Ala Leu Ile Asp Asp Gln Gly Asn Thr Leu Gln Thr
        115                 120                 125
Arg Arg Lys Leu Lys Pro Thr His Val Glu Arg Thr Val Phe Gly Glu
    130                 135                 140
Gly Asp Gly Ser Asp Leu Ser Val Val Glu Thr Ala Ile Gly Asn Ile
145                 150                 155                 160
Gly Ser Leu Ser Cys Trp Glu His Leu Gln Pro Leu Ser Lys Tyr Ala
                165                 170                 175
Met Tyr Ser Gln Asn Glu Gln Ile His Cys Gly Ala Trp Pro Ser Phe
            180                 185                 190
Ser Leu Tyr Arg Gly Gly Ala Tyr Ala Leu Gly Ala Glu Val Asn Asn
        195                 200                 205
Ala Ala Ser Gln Val Tyr Ala Ala Glu Gly Gln Cys Phe Val Ile Ala
    210                 215                 220
Pro Cys Ala Thr Val Ser Lys Ala Met His Glu Leu Leu Cys Thr Asp
225                 230                 235                 240
Pro Gly Lys Gln Gln Met Leu Leu Val Gly Gly Gly Phe Ala Arg Ile

                    245                     250                     255
Tyr Gly Pro Asp Gly Ser Pro Leu Gly Lys Asn Leu Ala Glu Asp Glu
                260                     265                     270
Glu Gly Leu Val Val Ala Asp Ile Asp Leu Gly Met Ile Ser Leu Ala
            275                     280                     285
Lys Ala Ala Gly Asp Pro Ala Gly His Tyr Ser Arg Pro Asp Val Thr
    290                     295                     300
Gln Leu Leu Phe Asn Arg Lys Arg Arg Glu Pro Val Val Leu Gln Gly
305                     310                     315                     320
Pro Ala Glu Pro Glu Lys Ala Val Ala Glu Pro Val Ser Thr Pro Ser
                    325                     330                     335
Glu Ala Ala Ala Ala Ala Ala Arg Gln Pro Val Val Ala
                340                     345

<210> 379
<211> 936
<212> DNA
<213> Unknown

<220>
<223> Obtained from an environmental sample

<400> 379
atggctaaaa tagcgattgt acaaaaggcg tctgttacct tgaataaaca agaaactgtc      60
gccagtgctg taagagaggt agagctggca gcggcggaag gtgccgagct ggtagtgttt     120
actgaggcat ttattgctgg gtatccggcc tggatctggc gtttgcggcc aggtggtgac     180
tgggggctat ctgaagatct tcattcccgt ttgttgacaa gcgccgtaga cctgggtggt     240
gatgacctgg atccacttta tgccgcagct aaagaaaata acgtgacgat agtgtgcggt     300
attaatgaac gtgataaccg gctcagtaag gcaacgctat ataattctat cgttattatt     360
ggttccgatg gttcattgtt aaatcgacat cgtaagttga tgccgacgaa tccggagaga     420
atggtatggg gctttggtga tgcctctggt ctgaaggtcg ttgatacccc cgttggtcgt     480
gttggtacgc ttgtctgttg ggaaaactat atgcccttgg ccagatatgc gttgtattcg     540
cagggggtag aggtttatat tgcgccgacc tacgatagcg gtgatgactg gatttctaca     600
ttacagcata ttgccaggga gggtcgttgt tgggttgttg gctgtggcaa tctattgcgt     660
ggcagcgata taccggatga cttccctgag aagttggcgt tatacccaga tgaggatgag     720
tggataaatc ctggggattc cgttgtgatt gcacctgggg gtaaaatcat ggccgggcca     780
ttgcgccagg aggcggggat tgtctattgt gatattgcgt ctgaaagtgc cagtcaggca     840
aaacgtgcgc tggatgtggc tggacattat tcccggcctg atatctttga gttgcatgtg     900
aatacgaagg tgcagacccc ggttgtatat gattag                               936

<210> 380
<211> 311
<212> PRT
<213> Unknown

<220>
<223> Obtained from an environmental sample

<400> 380
Met Ala Lys Ile Ala Ile Val Gln Lys Ala Ser Val Thr Leu Asn Lys
1               5                   10                  15
Gln Glu Thr Val Ala Ser Ala Val Arg Glu Val Glu Leu Ala Ala Ala
            20                  25                  30
Glu Gly Ala Glu Leu Val Val Phe Thr Glu Ala Phe Ile Ala Gly Tyr
        35                  40                  45
Pro Ala Trp Ile Trp Arg Leu Arg Pro Gly Gly Asp Trp Gly Leu Ser
    50                  55                  60

Glu Asp Leu His Ser Arg Leu Leu Thr Ser Ala Val Asp Leu Gly Gly
65                  70                  75                  80
Asp Asp Leu Asp Pro Leu Tyr Ala Ala Ala Lys Glu Asn Asn Val Thr
                85                  90                  95
Ile Val Cys Gly Ile Asn Glu Arg Asp Asn Arg Leu Ser Lys Ala Thr
            100                 105                 110
Leu Tyr Asn Ser Ile Val Ile Ile Gly Ser Asp Gly Ser Leu Leu Asn
        115                 120                 125
Arg His Arg Lys Leu Met Pro Thr Asn Pro Glu Arg Met Val Trp Gly
    130                 135                 140
Phe Gly Asp Ala Ser Gly Leu Lys Val Val Asp Thr Pro Val Gly Arg
145                 150                 155                 160
Val Gly Thr Leu Val Cys Trp Glu Asn Tyr Met Pro Leu Ala Arg Tyr
                165                 170                 175
Ala Leu Tyr Ser Gln Gly Val Glu Val Tyr Ile Ala Pro Thr Tyr Asp
            180                 185                 190
Ser Gly Asp Asp Trp Ile Ser Thr Leu Gln His Ile Ala Arg Glu Gly
        195                 200                 205
Arg Cys Trp Val Val Gly Cys Gly Asn Leu Leu Arg Gly Ser Asp Ile
    210                 215                 220
Pro Asp Asp Phe Pro Glu Lys Leu Ala Leu Tyr Pro Asp Glu Asp Glu
225                 230                 235                 240
Trp Ile Asn Pro Gly Asp Ser Val Val Ile Ala Pro Gly Gly Lys Ile
                245                 250                 255
Met Ala Gly Pro Leu Arg Gln Glu Ala Gly Ile Val Tyr Cys Asp Ile
            260                 265                 270
Ala Ser Glu Ser Ala Ser Gln Ala Lys Arg Ala Leu Asp Val Ala Gly
        275                 280                 285
His Tyr Ser Arg Pro Asp Ile Phe Glu Leu His Val Asn Thr Lys Val
    290                 295                 300
Gln Thr Pro Val Val Tyr Asp
305                 310

<210> 381
<211> 945
<212> DNA
<213> Clostridium acetobutylicum ATCC 3625

<400> 381
atgggcgaat tcggtgaagt taccctgggc gtggctcagg ctgctcccgt gtactttgac      60
cgcgaggcct cgaccgagaa agcctgtggc ctgatccgcg aggcgggcga aaagggtgta     120
gatcttctgg cgttcggtga aacgtggtta accggctatc catactggaa agatgcgcct     180
tggtctcgcg aatacaacga cttgcgtgca cgttatgttg cgaacggtgt gatgattcct     240
ggtccggaaa cggacgctct gtgccaagca gccgcggaag caggtgtgga tgtggcgatc     300
ggagtagtag aactggagcc gggctctctt tcctcggttt attgcactct gttatttata     360
agccgcgagg gcgagatcct gggtcgtcac cgcaaactga aaccgaccga tagcgaacgt     420
cgttactggt ctgaaggcga cgcgactggt ctgcgcgttt atgagcgccc atatggtcgg     480
cttagcggcc tgaattgctg ggagcacact atgatgctgc cggggtacgc cctggcggcg     540
cagggcaccc agttccatgt ggccgcttgg ccaaacatgg catcctcgaa ttctgaactt     600
ctgtctcgtg cctacgctat gcaggcgggc tgctacgttt tatgcgcggg tggcctgggc     660
ccggccccag gtgaactgcc ggatggtatc gcggcggaaa gtttagatca cttgactgga     720
gagtcatgta tcatcgaccc gtgggggaaa gtaattgctg gtccggtgtc ttgcgaggaa     780
acccttatca cggctcgcgt tagcaccgca tctatttatc gccgcaaaag tttgacggac     840
gtgggcggtc attatagccg cccggatgtt ttccgttttg aagtcgatcg ctctgagcgt     900
ccccgtgtcg tgtttcgcga tggtgacgtg gatgaccgag gttaa                     945

<210> 382

<211> 314
<212> PRT
<213> Clostridium acetobutylicum ATCC 3625

<400> 382
Met Gly Glu Phe Gly Glu Val Thr Leu Gly Val Ala Gln Ala Ala Pro
1               5                   10                  15
Val Tyr Phe Asp Arg Glu Ala Ser Thr Glu Lys Ala Cys Gly Leu Ile
            20                  25                  30
Arg Glu Ala Gly Glu Lys Gly Val Asp Leu Leu Ala Phe Gly Glu Thr
        35                  40                  45
Trp Leu Thr Gly Tyr Pro Tyr Trp Lys Asp Ala Pro Trp Ser Arg Glu
    50                  55                  60
Tyr Asn Asp Leu Arg Ala Arg Tyr Val Ala Asn Gly Val Met Ile Pro
65                  70                  75                  80
Gly Pro Glu Thr Asp Ala Leu Cys Gln Ala Ala Ala Glu Ala Gly Val
                85                  90                  95
Asp Val Ala Ile Gly Val Val Glu Leu Glu Pro Gly Ser Leu Ser Ser
            100                 105                 110
Val Tyr Cys Thr Leu Leu Phe Ile Ser Arg Glu Gly Glu Ile Leu Gly
        115                 120                 125
Arg His Arg Lys Leu Lys Pro Thr Asp Ser Glu Arg Arg Tyr Trp Ser
    130                 135                 140
Glu Gly Asp Ala Thr Gly Leu Arg Val Tyr Glu Arg Pro Tyr Gly Arg
145                 150                 155                 160
Leu Ser Gly Leu Asn Cys Trp Glu His Thr Met Met Leu Pro Gly Tyr
                165                 170                 175
Ala Leu Ala Ala Gln Gly Thr Gln Phe His Val Ala Ala Trp Pro Asn
            180                 185                 190
Met Ala Ser Ser Asn Ser Glu Leu Leu Ser Arg Ala Tyr Ala Met Gln
        195                 200                 205
Ala Gly Cys Tyr Val Leu Cys Ala Gly Gly Leu Gly Pro Ala Pro Gly
    210                 215                 220
Glu Leu Pro Asp Gly Ile Ala Ala Glu Ser Leu Asp His Leu Thr Gly
225                 230                 235                 240
Glu Ser Cys Ile Ile Asp Pro Trp Gly Lys Val Ile Ala Gly Pro Val
                245                 250                 255
Ser Cys Glu Glu Thr Leu Ile Thr Ala Arg Val Ser Thr Ala Ser Ile
            260                 265                 270
Tyr Arg Arg Lys Ser Leu Thr Asp Val Gly Gly His Tyr Ser Arg Pro
        275                 280                 285
Asp Val Phe Arg Phe Glu Val Asp Arg Ser Glu Arg Pro Arg Val Val
    290                 295                 300
Phe Arg Asp Gly Asp Val Asp Asp Arg Gly
305                 310

<210> 383
<211> 1041
<212> DNA
<213> Unknown

<220>
<223> Obtained from an environmental sample

<400> 383
atgtcggagc ccatgacgaa gtatcgcggc gcggcggtgc aggccgcgcc ggtgttcctc      60
gatctcgacc gcacagtcga gaaagcgatc ggcctgatcg agcaggcggc caagcaggac     120

gtgcgcctga tcgcattccc agagacttgg attcccggct atcccttttg gatatggctg      180
ggcgcgccgg cttggggcat gcgcttcgtc cagcgctatt tcgagaattc gctcgtgcgc      240
ggcagcaagc agtggcaggc cctggcggat gcggcccgcc gccacggcat gcatgtcgtg      300
gccggctata gcgagcgcgc gggcggcagc ctctatatgg gccaggcgat cttcggcccc      360
gatggcgatc tgatcgccgc gcgccgcaag ctcaagccta cccatgcgga gcgcaccgtg      420
ttcggcgagg gagacggcag ccatctcgcg gtgcacgata ccgccatcgg gcgcctcggc      480
gcgctctgtt gctgggagca catccagcca ttgtcgaaat acgccatgta cgccgccgac      540
gaacaggtcc acgtcgcgtc gtggccgagc ttcagcctct atcgcggcat ggcctatgcg      600
ctcggaccgg aggtcaatac cgccgcaagc cagatctacg cggtcgaggg cggctgctac      660
gtgctggcgt cgtgcgcgac cgtttcgccg gagatgatca aggtattggt ggatacgccc      720
gacaaggaga tgttcctcaa ggccggcggc ggttttgcca tgattttcgg gcccgacggc      780
cgcgccctgg ccgagccgct cccggagacc gaagagggac tgctggtcgc cgatatcgac      840
ctcggcatga tcgcgttggc caaggcggcg gccgatccgg cgggccacta ttcacggccc      900
gacgtaacgc ggctgctgct ggatcgacgt ccggcccaac gcgtcgtcac gcttgatgcc      960
gcattcgaac cgcaaaacga ggacaagggc gacgcgcccg cgctgcgcgt ggtggcggaa     1020
agcgccgccg ccgcgcagta g                                               1041

<210> 384
<211> 346
<212> PRT
<213> Unknown

<220>
<223> Obtained from an environmental sample

<400> 384
Met Ser Glu Pro Met Thr Lys Tyr Arg Gly Ala Ala Val Gln Ala Ala
1               5                   10                  15
Pro Val Phe Leu Asp Leu Asp Arg Thr Val Glu Lys Ala Ile Gly Leu
            20                  25                  30
Ile Glu Gln Ala Ala Lys Gln Asp Val Arg Leu Ile Ala Phe Pro Glu
        35                  40                  45
Thr Trp Ile Pro Gly Tyr Pro Phe Trp Ile Trp Leu Gly Ala Pro Ala
    50                  55                  60
Trp Gly Met Arg Phe Val Gln Arg Tyr Phe Glu Asn Ser Leu Val Arg
65                  70                  75                  80
Gly Ser Lys Gln Trp Gln Ala Leu Ala Asp Ala Ala Arg Arg His Gly
                85                  90                  95
Met His Val Val Ala Gly Tyr Ser Glu Arg Ala Gly Gly Ser Leu Tyr
            100                 105                 110
Met Gly Gln Ala Ile Phe Gly Pro Asp Gly Asp Leu Ile Ala Ala Arg
        115                 120                 125
Arg Lys Leu Lys Pro Thr His Ala Glu Arg Thr Val Phe Gly Glu Gly
    130                 135                 140
Asp Gly Ser His Leu Ala Val His Asp Thr Ala Ile Gly Arg Leu Gly
145                 150                 155                 160
Ala Leu Cys Cys Trp Glu His Ile Gln Pro Leu Ser Lys Tyr Ala Met
                165                 170                 175
Tyr Ala Ala Asp Glu Gln Val His Val Ala Ser Trp Pro Ser Phe Ser
            180                 185                 190
Leu Tyr Arg Gly Met Ala Tyr Ala Leu Gly Pro Glu Val Asn Thr Ala
        195                 200                 205
Ala Ser Gln Ile Tyr Ala Val Glu Gly Gly Cys Tyr Val Leu Ala Ser
    210                 215                 220
Cys Ala Thr Val Ser Pro Glu Met Ile Lys Val Leu Val Asp Thr Pro
225                 230                 235                 240
Asp Lys Glu Met Phe Leu Lys Ala Gly Gly Gly Phe Ala Met Ile Phe

                 245                 250                 255
Gly Pro Asp Gly Arg Ala Leu Ala Glu Pro Leu Pro Glu Thr Glu Glu
            260                 265                 270
Gly Leu Leu Val Ala Asp Ile Asp Leu Gly Met Ile Ala Leu Ala Lys
        275                 280                 285
Ala Ala Ala Asp Pro Ala Gly His Tyr Ser Arg Pro Asp Val Thr Arg
    290                 295                 300
Leu Leu Leu Asp Arg Arg Pro Ala Gln Arg Val Val Thr Leu Asp Ala
305                 310                 315                 320
Ala Phe Glu Pro Gln Asn Glu Asp Lys Gly Asp Ala Pro Ala Leu Arg
                325                 330                 335
Val Val Ala Glu Ser Ala Ala Ala Ala Gln
            340                 345

<210> 385
<211> 1014
<212> DNA
<213> Unknown

<220>
<223> Obtained from an environmental sample

<400> 385
atgaaagaag ctatcaaggt cgcctgcgtg caagccgccc cgatctacat ggatttgaag      60
gcgacggtgg acaaaaccat tgagttgatg gaagaagcag cacgtaataa tgctcgtctg     120
atcgcctttc cggaaacttg gattccaggc tacccatggt ttctttggct tgactcacca     180
gcatgggcaa tgcaatttgt acgccaatac catgagaact cattggagtt ggatggccct     240
caagctaagc gcatttcaga tgcagccaag cggttgggaa tcatggtcac cctggggatg     300
agtgaacggg tcggtggcac cctttacatc agtcagtggt tcataggcga taatggtgac     360
accattgggg cccggcgaaa gttgaaacct acttttgttg aacgtacttt gttcggcgaa     420
ggggatggtt catcgctagc ggttttcgag acgtctgttg gaaggctggg tggcttatgc     480
tgttgggagc accttcaacc gctaacaaaa tacgctttgt atgcacaaaa tgaagagatt     540
cattgtgcgg cttggccgag ctttagcctt tatcctaatg cggcgaaagc cctggggcct     600
gatgtcaatg tagcggcctc tcgaatctat gccgttgaag ggcaatgctt cgtactagcg     660
tcgtgtgcgc tcgtttcaca atccatgatc gatatgcttt gtacagatga cgaaaagcat     720
gcgttgcttc tggctggtgg tggacactca cgtatcatag ggcctgatgg tggtgacttg     780
gtcgcgcctc ttgccgaaaa tgaagagggt attctctacg caaaccttga tcctggagta     840
cgcatccttg ctaaaatggc ggcagaccct gctggtcatt attcccgtcc cgacattact     900
cgcttgctaa tagatcgcag ccctaaatta ccggtagttg aaattgaagg tgatcttcgt     960
ccttacgctt tgggtaaagc gtctgagacg ggtgcgcaac tcgaagaaat ttga          1014

<210> 386
<211> 337
<212> PRT
<213> Unknown

<220>
<223> Obtained from an environmental sample

<400> 386
Met Lys Glu Ala Ile Lys Val Ala Cys Val Gln Ala Ala Pro Ile Tyr
1               5                   10                  15
Met Asp Leu Lys Ala Thr Val Asp Lys Thr Ile Glu Leu Met Glu Glu
            20                  25                  30
Ala Ala Arg Asn Asn Ala Arg Leu Ile Ala Phe Pro Glu Thr Trp Ile
        35                  40                  45
Pro Gly Tyr Pro Trp Phe Leu Trp Leu Asp Ser Pro Ala Trp Ala Met

50                  55                  60
Gln Phe Val Arg Gln Tyr His Glu Asn Ser Leu Glu Leu Asp Gly Pro
65                  70                  75                  80
Gln Ala Lys Arg Ile Ser Asp Ala Ala Lys Arg Leu Gly Ile Met Val
                85                  90                  95
Thr Leu Gly Met Ser Glu Arg Val Gly Gly Thr Leu Tyr Ile Ser Gln
            100                 105                 110
Trp Phe Ile Gly Asp Asn Gly Asp Thr Ile Gly Ala Arg Arg Lys Leu
        115                 120                 125
Lys Pro Thr Phe Val Glu Arg Thr Leu Phe Gly Glu Gly Asp Gly Ser
    130                 135                 140
Ser Leu Ala Val Phe Glu Thr Ser Val Gly Arg Leu Gly Gly Leu Cys
145                 150                 155                 160
Cys Trp Glu His Leu Gln Pro Leu Thr Lys Tyr Ala Leu Tyr Ala Gln
                165                 170                 175
Asn Glu Glu Ile His Cys Ala Ala Trp Pro Ser Phe Ser Leu Tyr Pro
            180                 185                 190
Asn Ala Ala Lys Ala Leu Gly Pro Asp Val Asn Val Ala Ala Ser Arg
        195                 200                 205
Ile Tyr Ala Val Glu Gly Gln Cys Phe Val Leu Ala Ser Cys Ala Leu
    210                 215                 220
Val Ser Gln Ser Met Ile Asp Met Leu Cys Thr Asp Asp Glu Lys His
225                 230                 235                 240
Ala Leu Leu Leu Ala Gly Gly Gly His Ser Arg Ile Ile Gly Pro Asp
                245                 250                 255
Gly Gly Asp Leu Val Ala Pro Leu Ala Glu Asn Glu Glu Gly Ile Leu
            260                 265                 270
Tyr Ala Asn Leu Asp Pro Gly Val Arg Ile Leu Ala Lys Met Ala Ala
        275                 280                 285
Asp Pro Ala Gly His Tyr Ser Arg Pro Asp Ile Thr Arg Leu Leu Ile
    290                 295                 300
Asp Arg Ser Pro Lys Leu Pro Val Val Glu Ile Glu Gly Asp Leu Arg
305                 310                 315                 320
Pro Tyr Ala Leu Gly Lys Ala Ser Glu Thr Gly Ala Gln Leu Glu Glu
                325                 330                 335
Ile

SEQUENCE LISTING

<110> VERENIUM CORPORATION

<120> NITRILASES

<130> P/46800.ep01

<140> EP 02780877.3
<141> 2002-05-15

<150> PCT/US2002/015983
<151> 2002-05-15

<150> US 60/309,006
<151> 2001-07-30

<150> US 60/351,336
<151> 2002-01-22

<150> US 60/300,189
<151> 2001-06-21

<150> US 09/751,299
<151> 2000-12-28

<150> US 60/254,414
<151> 2000-12-07

<150> US 60/173,609
<151> 1999-12-29

<160> 392

<170> FastSEQ for Windows Version 4.0

<210> 1
<211> 939
<212> DNA
<213> Unknown

<220>
<223> Obtained from an environmental sample


<400> 1
atggaaaagt atattaaagt cgccgcaatt cagatagcta caaaaatagc agattcaccc 60
gtgaatatag aaaattgcga acgtttggca ttatcggcgg tcaatgaggg tgcgcgttgg 120
attgctttgc cggagttctt caatacgggc gttagttgga acaaaaaaat tgccttggct 180
attcagacgc ctgacggcaa ggctgcgatg ttcttacgcg acttttctgc aagacatcat 240
gtattgatag gaggctcatt tctgtgcagg ttgccggatg gcagtgtgcg caaccgctat 300
atgtgttatg ccaacggcgc tctcgtgggc aaacatgaca aagacctacc cacgatgtgg 360
gaaaatgctt tttatgaagg tggggattcc agcgatattg gggtgctggg aacatttgaa 420
aatacgcgcg ttggtgcagc cgtctgttgg gagttcatgc ggacgatgac tgcccggcgt 480
cttcgcaatc aggtggatgt catcatgggt ggttcctgct ggtggagcat accgaccaat 540
ttccccggtt ttgtgcaaaa gctgtgggaa cctggaaata gccgcaacgc gcttgctgcc 600
atacaggata atgcgcgtct cattggcgtg ccggttgttc atgccgctca ttgcggtgaa 660
attgagtgtc cgatgccagg attgccgata ggttacaggg ggttctttga gggtaacgcg 720
gccattgtga atgcagaagg tcaggtgctt gcgcatcggg gtgctggcga gggcgaagga 780
attgtttgcg cggagatttt accggtagcc aaatcaaaca ggtcggaaat tcccaatcgt 840
tactggttgc gctgcagagg ctttctacct atttttgcct ggcatcagca acgttggttg 900
ggaaggcatt ggtatttgcg caatgtgcgc aggacttaa 939

<210> 2
<211> 312
<212> PRT
<213> Unknown

<220>
<223> Obtained from an environmental sample

<400> 2
Met Glu Lys Tyr Ile Lys Val Ala Ala Ile Gln Ile Ala Thr Lys Ile
1               5                   10                  15
Ala Asp Ser Pro Val Asn Ile Glu Asn Cys Glu Arg Leu Ala Leu Ser
            20                  25                  30
Ala Val Asn Glu Gly Ala Arg Trp Ile Ala Leu Pro Glu Phe Phe Asn
        35                  40                  45
Thr Gly Val Ser Trp Asn Lys Lys Ile Ala Leu Ala Ile Gln Thr Pro
    50                  55                  60
Asp Gly Lys Ala Ala Met Phe Leu Arg Asp Phe Ser Ala Arg His His
65                  70                  75                  80
Val Leu Ile Gly Gly Ser Phe Leu Cys Arg Leu Pro Asp Gly Ser Val
                85                  90                  95
Arg Asn Arg Tyr Met Cys Tyr Ala Asn Gly Ala Leu Val Gly Lys His
            100                 105                 110
Asp Lys Asp Leu Pro Thr Met Trp Glu Asn Ala Phe Tyr Glu Gly Gly
        115                 120                 125
Asp Ser Ser Asp Ile Gly Val Leu Gly Thr Phe Glu Asn Thr Arg Val
    130                 135                 140
Gly Ala Ala Val Cys Trp Glu Phe Met Arg Thr Met Thr Ala Arg Arg
145                 150                 155                 160
Leu Arg Asn Gln Val Asp Val Ile Met Gly Gly Ser Cys Trp Trp Ser
                165                 170                 175
Ile Pro Thr Asn Phe Pro Gly Phe Val Gln Lys Leu Trp Glu Pro Gly
            180                 185                 190
Asn Ser Arg Asn Ala Leu Ala Ala Ile Gln Asp Asn Ala Arg Leu Ile
        195                 200                 205
Gly Val Pro Val Val His Ala Ala His Cys Gly Glu Ile Glu Cys Pro
    210                 215                 220
Met Pro Gly Leu Pro Ile Gly Tyr Arg Gly Phe Phe Glu Gly Asn Ala
225                 230                 235                 240
Ala Ile Val Asn Ala Glu Gly Gln Val Leu Ala His Arg Gly Ala Gly
                245                 250                 255
Glu Gly Glu Gly Ile Val Cys Ala Glu Ile Leu Pro Val Ala Lys Ser
            260                 265                 270
Asn Arg Ser Glu Ile Pro Asn Arg Tyr Trp Leu Arg Cys Arg Gly Phe
        275                 280                 285
Leu Pro Ile Phe Ala Trp His Gln Gln Arg Trp Leu Gly Arg His Trp
    290                 295                 300
Tyr Leu Arg Asn Val Arg Arg Thr
305                 310

<210> 3
<211> 981
<212> DNA
<213> Unknown

<220>
<223> Obtained from an environmental sample

<400> 3
atggggaatt cgttcaagat cgcggtggta caagcctgtc cggtctttct ggatcgtggc      60
gcgacagtcg ccaaggcatg ccgcctgatc gcggaggcag ccgcggcggg cgccagcctg     120
gtggtctttc cggaggcgtt tgtgcccgga tacccactgt gggtctggtt cattccggca     180
gggcattcgc aaccactgcg ggagttatac gccgaactgg tggggaacgc cgtggcggta     240
ccgggcgatg ccaccgatcg gctttgcgcg gcagccagag aagccggcgt ggtagtggcg     300
atcggcatca atgaagtgaa cagcgaagcc agcggcacga cgatttacaa tacgctgctg     360
tacatcggag cggacggcgc gattctgggc aaacaccgca aagtaatgcc gacgggcgga     420
gagcgcctgg tctgggcgct tggcgatggg agcgacctgg aggtctacga cctgcctttc     480
ggccgattgg gtggcctgtt gtgctgggag aactacatgc ccctggcccg gtacgcgatg     540
tcggcatggg gaaccgagat ctacgtggct ccaacttggg atcgcggaga accgtggctg     600
tccacaatgc ggcatatcgc gaaagaaggg cgatgctacg tagtgggatg ctgcagttgc     660

atgaaaattg acgatgtacc cgaccggctg gcgttcaaag ggaagtatct gtcgacggcc 720
gagggctggc tcaaccccgg cgatagcgta atcgtcgatc cggacggcaa gctgatcgcg 780
ggcccggcaa gcgagcagga gacgattctg tatgccgatg ccgaccggtc taagatcacc 840
gggcccaggt ggcagttgga tgtggccggc cactacgcgc ggccggatat cttcgaactg 900
atcgtgcacc gcgaacctaa gcgatttttg acgatagctc cgcggacgaa ggaggagcgg 960
gagcctgggc cggaggcctg a 981

<210> 4
<211> 326
<212> PRT
<213> Unknown

<220>
<223> Obtained from an environmental sample

<400> 4
Met Gly Asn Ser Phe Lys Ile Ala Val Val Gln Ala Cys Pro Val Phe
1               5                   10                  15
Leu Asp Arg Gly Ala Thr Val Ala Lys Ala Cys Arg Leu Ile Ala Glu
            20                  25                  30
Ala Ala Ala Ala Gly Ala Ser Leu Val Val Phe Pro Glu Ala Phe Val
        35                  40                  45
Pro Gly Tyr Pro Leu Trp Val Trp Phe Ile Pro Ala Gly His Ser Gln
    50                  55                  60
Pro Leu Arg Glu Leu Tyr Ala Glu Leu Val Gly Asn Ala Val Ala Val
65                  70                  75                  80
Pro Gly Asp Ala Thr Asp Arg Leu Cys Ala Ala Ala Arg Glu Ala Gly
                85                  90                  95
Val Val Val Ala Ile Gly Ile Asn Glu Val Asn Ser Glu Ala Ser Gly
            100                 105                 110
Thr Thr Ile Tyr Asn Thr Leu Leu Tyr Ile Gly Ala Asp Gly Ala Ile
        115                 120                 125
Leu Gly Lys His Arg Lys Val Met Pro Thr Gly Gly Glu Arg Leu Val
    130                 135                 140
Trp Ala Leu Gly Asp Gly Ser Asp Leu Glu Val Tyr Asp Leu Pro Phe
145                 150                 155                 160
Gly Arg Leu Gly Gly Leu Leu Cys Trp Glu Asn Tyr Met Pro Leu Ala
                165                 170                 175
Arg Tyr Ala Met Ser Ala Trp Gly Thr Glu Ile Tyr Val Ala Pro Thr
            180                 185                 190
Trp Asp Arg Gly Glu Pro Trp Leu Ser Thr Met Arg His Ile Ala Lys
        195                 200                 205
Glu Gly Arg Cys Tyr Val Val Gly Cys Cys Ser Cys Met Lys Ile Asp
    210                 215                 220
Asp Val Pro Asp Arg Leu Ala Phe Lys Gly Lys Tyr Leu Ser Thr Ala
225                 230                 235                 240
Glu Gly Trp Leu Asn Pro Gly Asp Ser Val Ile Val Asp Pro Asp Gly
                245                 250                 255
Lys Leu Ile Ala Gly Pro Ala Ser Glu Gln Glu Thr Ile Leu Tyr Ala
            260                 265                 270
Asp Ala Asp Arg Ser Lys Ile Thr Gly Pro Arg Trp Gln Leu Asp Val
        275                 280                 285
Ala Gly His Tyr Ala Arg Pro Asp Ile Phe Glu Leu Ile Val His Arg
    290                 295                 300
Glu Pro Lys Arg Phe Leu Thr Ile Ala Pro Arg Thr Lys Glu Glu Arg
305                 310                 315                 320
Glu Pro Gly Pro Glu Ala
                325

<210> 5
<211> 1005
<212> DNA
<213> Unknown

<220>
<223> Obtained from an environmental sample

<400> 5
atgggtacca agcacccggc cttcaaggcc gcagtggtcc aggccgcgcc ggaatggctc      60
gatctcgacc gcaccgtcga caagaccatc gcgctgatcg aggaggccgc cggcgccggc     120
gcgaagctca ttgcgttccc ggaaacctgg attcccggct atccgtggca catctgggtc     180
ggcacgccgg cgtgggcgat cagccgcggc ttcgtgcagc gctacttcga caattcactg     240
gcctacgaca gcccgcaggc ccagcgcatc gcggacgccg cgaagaagaa caagatcacc     300
gtggtgctcg gcctgtcgga gcgcgagggt ggcagccttt atatctcgca gtggctgatt     360
gggccggacg gcgagaccat tgccaagcgc cgcaaactgc gccccaccca cgtcgagcgc     420
accgtgttcg gcgatggcga cggcagccac atcgcggtgc acgagcgtgc tgacatcggc     480
cgcctcggcg cgctgtgctg ctgggagcac atccagccgc tgaccaaata cgccatgtat     540
gcccagaacg agcaggtgca cgtcgccgcc tggccgagct tctcgatgta cgagccgttc     600
gcccacgcgc tcggctggga agtcaacaat gcggcgagca agatctacgc cgtcgaaggc     660
tcgtgtttcg tgctcggcgc atgcgcggtg atctcgcagg cgatggtcga cgaaatgtgc     720
gacaccgagg acaagcgggc gctggtccat gccggcggcg gccacgcggt gatcttcggg     780
ccggacggca gatcgctggc ggacaagatt ccggagaccc aggaaggcct gctctatgcc     840
gacatcgacc tcggcgcaat tggcgtggcc aagaacgcgg ccgatccggc ggggcactac     900
tcgcgcccgg acgtgacgcg gctcctgttc aacaacaagc cggcgcgccg ggtcgagtat     960
ttctcgctgc cggtcgacgc ggtcgagacg ccgccgcagc cctga                    1005

<210> 6
<211> 334
<212> PRT
<213> Unknown

<220>
<223> Obtained from an environmental sample


<400> 6
Met Gly Thr Lys His Pro Ala Phe Lys Ala Ala Val Val Gln Ala Ala
1               5                   10                  15
Pro Glu Trp Leu Asp Leu Asp Arg Thr Val Asp Lys Thr Ile Ala Leu
            20                  25                  30
Ile Glu Glu Ala Ala Gly Ala Gly Ala Lys Leu Ile Ala Phe Pro Glu
        35                  40                  45
Thr Trp Ile Pro Gly Tyr Pro Trp His Ile Trp Val Gly Thr Pro Ala
    50                  55                  60
Trp Ala Ile Ser Arg Gly Phe Val Gln Arg Tyr Phe Asp Asn Ser Leu
65                  70                  75                  80
Ala Tyr Asp Ser Pro Gln Ala Gln Arg Ile Ala Asp Ala Ala Lys Lys
                85                  90                  95
Asn Lys Ile Thr Val Val Leu Gly Leu Ser Glu Arg Glu Gly Gly Ser
            100                 105                 110
Leu Tyr Ile Ser Gln Trp Leu Ile Gly Pro Asp Gly Glu Thr Ile Ala
        115                 120                 125
Lys Arg Arg Lys Leu Arg Pro Thr His Val Glu Arg Thr Val Phe Gly
    130                 135                 140
Asp Gly Asp Gly Ser His Ile Ala Val His Glu Arg Ala Asp Ile Gly
145                 150                 155                 160
Arg Leu Gly Ala Leu Cys Cys Trp Glu His Ile Gln Pro Leu Thr Lys
                165                 170                 175
Tyr Ala Met Tyr Ala Gln Asn Glu Gln Val His Val Ala Ala Trp Pro
            180                 185                 190
Ser Phe Ser Met Tyr Glu Pro Phe Ala His Ala Leu Gly Trp Glu Val
        195                 200                 205
Asn Asn Ala Ala Ser Lys Ile Tyr Ala Val Glu Gly Ser Cys Phe Val
    210                 215                 220
Leu Gly Ala Cys Ala Val Ile Ser Gln Ala Met Val Asp Glu Met Cys
225                 230                 235                 240
Asp Thr Glu Asp Lys Arg Ala Leu Val His Ala Gly Gly Gly His Ala
                245                 250                 255
Val Ile Phe Gly Pro Asp Gly Arg Ser Leu Ala Asp Lys Ile Pro Glu
            260                 265                 270
Thr Gln Glu Gly Leu Leu Tyr Ala Asp Ile Asp Leu Gly Ala Ile Gly
        275                 280                 285
Val Ala Lys Asn Ala Ala Asp Pro Ala Gly His Tyr Ser Arg Pro Asp
    290                 295                 300

Val Thr Arg Leu Leu Phe Asn Asn Lys Pro Ala Arg Arg Val Glu Tyr
305                 310                 315                 320
Phe Ser Leu Pro Val Asp Ala Val Glu Thr Pro Pro Gln Pro
                325                 330

<210> 7
<211> 999
<212> DNA
<213> Unknown

<220>
<223> Obtained from an environmental sample


<400> 7
atgccgagtg attatcatgc tccattcaaa gtagcagttg tccaggcaac tcccgtcttt      60
ctcgatcgca gcgcgacgat tgagaaggca tgtgagctaa ttgcctgtgc tggacgtgag     120
ggcgcacgtc tgatcgtgtt tcctgaagcg ttcattccca cctatcccga ttgggtctgg     180
accattccac ctggggagat gcggctgctt ggcgaactct acacagagtt gcttgccaat     240
gcggtcacga tccccagtaa tgcaacggat aggctctgcc aggctgcgaa acgagctgct     300
gcgtatgtgg tcatgggaat gaacgaacgc aatatcgagg cgagtggaag gagtctctat     360
aacaccctgt tatacatcga tgctcagggc cagatcatgg gcaaacaccg caagttgata     420
cccacagccg gtgagcggct catatgggcg caaggagatg ggagtacatt ccaggtctac     480
gatactcctc tgggcaaact gggagggctc atctgctggg aaaactacat gcctctggct     540
cgctatgcga tgtatgcctg gggcacgcag atttatgtcg ccccgacatg ggatcgtggc     600
aacctctggc tctctactct gcggcatatc gctaaggagg gaggcgtcta tgttcttggt     660
tgtagtatgg tcatgcgcaa gaatgacatt cccgatcact ttgctttcaa agagcagttt     720
tatgctactg tggacgaatg gatcaacgtt ggtgacagcg ccattgtcca tcccgagggg     780
aactttcttg cgggaccggt gcgccacaaa gaagagattc tctatgcaga acttgatcca     840
cgccaatcgt gcggtccggg atggatgctc gatgtggctg ggcactatgc acgccctgat     900
gtgtttgaat tgattgtcca cacagagatg cgacccatga tgaagcaaga agaggtagga     960
ggagaaaata catctgaggg aggtgtacga ttcttgtaa                            999

<210> 8
<211> 332
<212> PRT
<213> Unknown

<220>
<223> Obtained from an environmental sample


<400> 8
Met Pro Ser Asp Tyr His Ala Pro Phe Lys Val Ala Val Val Gln Ala
1               5                   10                  15
Thr Pro Val Phe Leu Asp Arg Ser Ala Thr Ile Glu Lys Ala Cys Glu
            20                  25                  30
Leu Ile Ala Cys Ala Gly Arg Glu Gly Ala Arg Leu Ile Val Phe Pro
        35                  40                  45
Glu Ala Phe Ile Pro Thr Tyr Pro Asp Trp Val Trp Thr Ile Pro Pro
    50                  55                  60
Gly Glu Met Arg Leu Leu Gly Glu Leu Tyr Thr Glu Leu Leu Ala Asn
65                  70                  75                  80
Ala Val Thr Ile Pro Ser Asn Ala Thr Asp Arg Leu Cys Gln Ala Ala
                85                  90                  95
Lys Arg Ala Ala Ala Tyr Val Val Met Gly Met Asn Glu Arg Asn Ile
            100                 105                 110
Glu Ala Ser Gly Arg Ser Leu Tyr Asn Thr Leu Leu Tyr Ile Asp Ala
        115                 120                 125
Gln Gly Gln Ile Met Gly Lys His Arg Lys Leu Ile Pro Thr Ala Gly
    130                 135                 140
Glu Arg Leu Ile Trp Ala Gln Gly Asp Gly Ser Thr Phe Gln Val Tyr
145                 150                 155                 160
Asp Thr Pro Leu Gly Lys Leu Gly Gly Leu Ile Cys Trp Glu Asn Tyr
                165                 170                 175
Met Pro Leu Ala Arg Tyr Ala Met Tyr Ala Trp Gly Thr Gln Ile Tyr
            180                 185                 190
Val Ala Pro Thr Trp Asp Arg Gly Asn Leu Trp Leu Ser Thr Leu Arg

195                 200                 205
His Ile Ala Lys Glu Gly Gly Val Tyr Val Leu Gly Cys Ser Met Val
    210                 215                 220
Met Arg Lys Asn Asp Ile Pro Asp His Phe Ala Phe Lys Glu Gln Phe
225                 230                 235                 240
Tyr Ala Thr Val Asp Glu Trp Ile Asn Val Gly Asp Ser Ala Ile Val
                245                 250                 255
His Pro Glu Gly Asn Phe Leu Ala Gly Pro Val Arg His Lys Glu Glu
            260                 265                 270
Ile Leu Tyr Ala Glu Leu Asp Pro Arg Gln Ser Cys Gly Pro Gly Trp
        275                 280                 285
Met Leu Asp Val Ala Gly His Tyr Ala Arg Pro Asp Val Phe Glu Leu
    290                 295                 300
Ile Val His Thr Glu Met Arg Pro Met Met Lys Gln Glu Glu Val Gly
305                 310                 315                 320
Gly Glu Asn Thr Ser Glu Gly Gly Val Arg Phe Leu
                325                 330

<210> 9
<211> 945
<212> DNA
<213> Unknown

<220>
<223> Obtained from an environmental sample


<400> 9
atggcggctc acaagatcgc ggtggttcag gcgcccagcg ttctcctcga tcgcgagggc      60
tcggtcgcgc gcgcggtcac gctgctcgac gaggcggcgg cggccggcgc ccgcctggtc     120
gtgtttccgg aggcctacat ccccggctac ccggactgga tctggcgcct gcgcccctac     180
ccggacgtca agctggccgc cgagctgcac gaacggctgc tcgccaacgc ggtggatctc     240
tccaccgacg tgctggcgcc ggtgctggcg gcggcggcgc gtcacgggct caccgtggtc     300
atgtgcgtgc aggagcgcga cgccggattc agccgcgcca cactttacaa caccgcgctg     360
gtcatcgacg ccgccggcaa gatcgcgaac cggcaccgca agctcatgcc caccaacccc     420
gagcgaatgg tgtggggatt cggtgacgcc tcggggctgc gggtggtgag cacgcccgtc     480
gggcgggtgg gcacgctcct gtgctgggag agctacatgc ccctggcgcg ctgcgcgctc     540
tacgccgagg gggtcgagat ctacgtgacc ccgacctggg actacggcga aggctggcgc     600
gccagcatgc agcacatcgc ccgcgagggg cgctgctggg tggtgaccgc ttgcatgtgc     660
gtgcaggcgc gcgacgtgcc ggccgacttc cccgggcgcg cccagctcta ccccgacgag     720
gaggagtggt tgaaccccgg cgattcgctg gtggtcgacc ccggcggcaa gatcgtggcc     780
ggtccgatgt cgcgcgagaa ggggatcttg tacgcggaga tcgatccgga tcgcgtggcg     840
ggggcgcacc gctcgttcga cgtcgtgggc cactactcgc gtcccgacgt gttccggctg     900
gaggtcgatc ggacaccggc ggcgccggtg agcttcaaaa aatga                     945

<210> 10
<211> 314
<212> PRT
<213> Unknown

<220>
<223> Obtained from an environmental sample


<400> 10
Met Ala Ala His Lys Ile Ala Val Val Gln Ala Pro Ser Val Leu Leu
1               5                   10                  15
Asp Arg Glu Gly Ser Val Ala Arg Ala Val Thr Leu Leu Asp Glu Ala
            20                  25                  30
Ala Ala Ala Gly Ala Arg Leu Val Val Phe Pro Glu Ala Tyr Ile Pro
        35                  40                  45
Gly Tyr Pro Asp Trp Ile Trp Arg Leu Arg Pro Tyr Pro Asp Val Lys
    50                  55                  60
Leu Ala Ala Glu Leu His Glu Arg Leu Leu Ala Asn Ala Val Asp Leu
65                  70                  75                  80
Ser Thr Asp Val Leu Ala Pro Val Leu Ala Ala Ala Ala Arg His Gly
                85                  90                  95
Leu Thr Val Val Met Cys Val Gln Glu Arg Asp Ala Gly Phe Ser Arg

                100                 105                 110
Ala Thr Leu Tyr Asn Thr Ala Leu Val Ile Asp Ala Ala Gly Lys Ile
            115                 120                 125
Ala Asn Arg His Arg Lys Leu Met Pro Thr Asn Pro Glu Arg Met Val
    130                 135                 140
Trp Gly Phe Gly Asp Ala Ser Gly Leu Arg Val Val Ser Thr Pro Val
145                 150                 155                 160
Gly Arg Val Gly Thr Leu Leu Cys Trp Glu Ser Tyr Met Pro Leu Ala
                165                 170                 175
Arg Cys Ala Leu Tyr Ala Glu Gly Val Glu Ile Tyr Val Thr Pro Thr
            180                 185                 190
Trp Asp Tyr Gly Glu Gly Trp Arg Ala Ser Met Gln His Ile Ala Arg
        195                 200                 205
Glu Gly Arg Cys Trp Val Val Thr Ala Cys Met Cys Val Gln Ala Arg
    210                 215                 220
Asp Val Pro Ala Asp Phe Pro Gly Arg Ala Gln Leu Tyr Pro Asp Glu
225                 230                 235                 240
Glu Glu Trp Leu Asn Pro Gly Asp Ser Leu Val Val Asp Pro Gly Gly
                245                 250                 255
Lys Ile Val Ala Gly Pro Met Ser Arg Glu Lys Gly Ile Leu Tyr Ala
            260                 265                 270
Glu Ile Asp Pro Asp Arg Val Ala Gly Ala His Arg Ser Phe Asp Val
        275                 280                 285
Val Gly His Tyr Ser Arg Pro Asp Val Phe Arg Leu Glu Val Asp Arg
    290                 295                 300
Thr Pro Ala Ala Pro Val Ser Phe Lys Lys
305                 310

<210> 11
<211> 966
<212> DNA
<213> Unknown

<220>
<223> Obtained from an environmental sample


<400> 11
atgactggat cttatcctaa agacacactg atcgttgggc tagctcaaat cgctcctgtc      60
tggctggatc gggcggggac actgtcaaag atactggctc aagtccatgc ggcaaatcaa     120
gcgggttgtc atctcgtagc atttggcgaa gggctgcttc ctggatatcc gttttggatt     180
gagcgaacaa atggcgcgct gttcaactcg actgtacaaa aggaaatcca cgcgcattat     240
atggatcagg cggtgcagat cgaagccggt catctcgatc cgctttgtgc aacagccaag     300
aaatttggaa tcaccgttgt actcggatgc atcgaacgcc cactcgatcg gggcggtcac      360
agcttgtatg caagtctggt atatattgat tccgagggca gcattcaatc cgtgcatcgc     420
aaactaatgc caacctacga agaacgactt acctggtcgt caggcgatgg gcacggttta     480
cgagtgcata ccttaggtgc gtttacggtg ggtggtctca actgttggga aaattggatg     540
cccttggcgc gcgcagcgat gtatggtcag ggtgaagatt tacatgttgc gatctggcca     600
ggcggttctc atctcacgca ggatattacc cgctttattg cgctcgaatc acgttcgtac     660
gtattatctg tctccggtct gatgcgcgca accgatttcc caaaagatac tccccatctt      720
gcctccatcc tagctaaagg tgaagagatt cttgcgaatg gtggttcttg tattgcaggt     780
cctgacggca agtgggtcgt tgggcctctt gtaggagaag agaagttaat tgtcgcaacc     840
attgatcact gccgcgtgcg cgaagaacgt cagaatttcg atccttccgg gcattacagc     900
cggcccgatg tactgcaatt aaaaatcaac agggaacgcc agagcacaat ttcatttagc     960
gagtag                                                                966

<210> 12
<211> 321
<212> PRT
<213> Unknown

<220>
<223> Obtained from an environmental sample


<400> 12
Met Thr Gly Ser Tyr Pro Lys Asp Thr Leu Ile Val Gly Leu Ala Gln
1               5                   10                  15

Ile Ala Pro Val Trp Leu Asp Arg Ala Gly Thr Leu Ser Lys Ile Leu
            20                  25                  30
Ala Gln Val His Ala Ala Asn Gln Ala Gly Cys His Leu Val Ala Phe
        35                  40                  45
Gly Glu Gly Leu Leu Pro Gly Tyr Pro Phe Trp Ile Glu Arg Thr Asn
    50                  55                  60
Gly Ala Leu Phe Asn Ser Thr Val Gln Lys Glu Ile His Ala His Tyr
65                  70                  75                  80
Met Asp Gln Ala Val Gln Ile Glu Ala Gly His Leu Asp Pro Leu Cys
                85                  90                  95
Ala Thr Ala Lys Lys Phe Gly Ile Thr Val Val Leu Gly Cys Ile Glu
            100                 105                 110
Arg Pro Leu Asp Arg Gly Gly His Ser Leu Tyr Ala Ser Leu Val Tyr
        115                 120                 125
Ile Asp Ser Glu Gly Ser Ile Gln Ser Val His Arg Lys Leu Met Pro
    130                 135                 140
Thr Tyr Glu Glu Arg Leu Thr Trp Ser Ser Gly Asp Gly His Gly Leu
145                 150                 155                 160
Arg Val His Thr Leu Gly Ala Phe Thr Val Gly Gly Leu Asn Cys Trp
                165                 170                 175
Glu Asn Trp Met Pro Leu Ala Arg Ala Ala Met Tyr Gly Gln Gly Glu
            180                 185                 190
Asp Leu His Val Ala Ile Trp Pro Gly Gly Ser His Leu Thr Gln Asp
        195                 200                 205
Ile Thr Arg Phe Ile Ala Leu Glu Ser Arg Ser Tyr Val Leu Ser Val
    210                 215                 220
Ser Gly Leu Met Arg Ala Thr Asp Phe Pro Lys Asp Thr Pro His Leu
225                 230                 235                 240
Ala Ser Ile Leu Ala Lys Gly Glu Glu Ile Leu Ala Asn Gly Gly Ser
                245                 250                 255
Cys Ile Ala Gly Pro Asp Gly Lys Trp Val Val Gly Pro Leu Val Gly
            260                 265                 270
Glu Glu Lys Leu Ile Val Ala Thr Ile Asp His Cys Arg Val Arg Glu
        275                 280                 285
Glu Arg Gln Asn Phe Asp Pro Ser Gly His Tyr Ser Arg Pro Asp Val
    290                 295                 300
Leu Gln Leu Lys Ile Asn Arg Glu Arg Gln Ser Thr Ile Ser Phe Ser
305                 310                 315                 320
Glu

<210> 13
<211> 1014
<212> DNA
<213> Unknown

<220>
<223> Obtained from an environmental sample

<400> 13
   atgggcattc aacatccgaa atataaggtt gcggtggtgc aggcggcgcc ggcctggctc      60
   gatctcgatg cgtcgatcgc caaatcgatc gcgttgatcg aggaggcggc tgccaatggc     120
   gccaagctga tcgccttccc ggaggcgttc atccctggct atccctggta tatctggctg     180
   gactcgccgg cctgggcgat cggccgcggt tttgtgcagc gctatttcga caactcgctg     240
   gcctatgaca gcccgcaggc cgagaagctg cggctggcgg tgaagaaggc cggcctcacc     300
   gccgtgatcg gcctctccga gcgcgagggc ggcagccttt atctcgcgca atggctgatc     360
   gggcccgatg gcgagaccat cgcaaagcgc cgcaagctgc ggccgaccca tgccgagcgc     420
   accgtctatg gcgaaggcga tggcagcgat ctcgcggtgc atgaccgccc cggcatcggc     480
   cggctcggcg cgctgtgctg ctgggagcat ctgcagccgc tgtcgaaata cgcgatgtat     540
   gcccagaacg agcaggttca tgtcgcggcc tggccgagct tctcgctcta cgacccgttc     600
   gcgccggcgc tcggctggga ggtcaacaat gcggcctcac gcgtctatgc ggtggaaggc     660
   tcgtgcttcg tgctggcgcc ctgcgcgacg gtgtcgaagg cgatgatcga cgagctctgc     720
   gaccgcgacg acaagcacgg gctgctgcat gtcggcgggg gacacgccgc gatctatggg     780
   ccggacggct cttcgattgc ggagaaattg ccgccggagc aggagggcct gctctatgcc     840
   gacatcgatc tcggcgccat cgggattgcc aagaacgccg ccgatccggc cggacattac     900
   tcgcggcccg acgtgacgcg gctgttgctc aacaagaagc cgtcgaagcg tgtcgagcat     960
   tttcgctgcc cggtcgacaa tgtcgagccg gagatcgacg ccgccgccag ctga           1014

<210> 14
<211> 337
<212> PRT
<213> Unknown

<220>
<223> Obtained from an environmental sample

<400> 14
Met Gly Ile Gln His Pro Lys Tyr Lys Val Ala Val Val Gln Ala Ala
1               5                   10                  15
Pro Ala Trp Leu Asp Leu Asp Ala Ser Ile Ala Lys Ser Ile Ala Leu
            20                  25                  30
Ile Glu Glu Ala Ala Ala Asn Gly Ala Lys Leu Ile Ala Phe Pro Glu
        35                  40                  45
Ala Phe Ile Pro Gly Tyr Pro Trp Tyr Ile Trp Leu Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Lys Leu Arg Leu Ala Val Lys Lys
                85                  90                  95
Ala Gly Leu Thr Ala Val Ile Gly Leu Ser Glu Arg Glu Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asp Arg Pro Gly Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Pro Ala Leu Gly Trp Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Lys Ala Met Ile Asp Glu Leu Cys
225                 230                 235                 240
Asp Arg Asp Asp Lys His Gly Leu Leu His Val Gly Gly Gly His Ala
                245                 250                 255
Ala Ile Tyr Gly Pro Asp Gly Ser Ser Ile Ala Glu Lys Leu Pro Pro
            260                 265                 270
Glu Gln Glu Gly Leu Leu Tyr Ala Asp Ile Asp Leu Gly Ala Ile Gly
        275                 280                 285
Ile Ala Lys Asn Ala Ala Asp Pro Ala Gly His Tyr Ser Arg Pro Asp
    290                 295                 300
Val Thr Arg Leu Leu Leu Asn Lys Lys Pro Ser Lys Arg Val Glu His
305                 310                 315                 320
Phe Ser Leu Pro Val Asp Asn Val Glu Pro Glu Ile Asp Ala Ala Ala
                325                 330                 335
Ser

<210> 15
<211> 1047
<212> DNA
<213> Unknown

<220>
<223> Obtained from an environmental sample

<400> 15
   atgccaacat caaaacaatt tagagtcgct gcagttcaag ccgccccggt atttcttgac      60
   ctggagggcg caataagcaa aggcatctcc ctcattgagg aggccgcttc caatggagcc     120
   aagctcattg ccttcccgga aacgtggatt cccggctacc cctggtggat ctggctggac     180
   tcacccgctt ggggcatgcg ctttgtccag cgctattttg acaactcgct catgctgggt     240
   agtgagcaag ccaagcgcat gaaccaggct gccgccaata acaagattta cgtggtgatg     300
   ggttatagcg aacgcagtgg cggcagcctc tacatgggcc aatccattat caacgacaag 360
   ggtgaaacga tttttacccg ccgcaaactc aagccaactc atgtcgagcg taccgtgttt 420
   ggggagggag acggcagcca tctttgcgta atggataccg agattggccg cgtcggcgcg 480
   atgtgctgtt gggaacattt gcagccgctc agcaaatatg caatgtattc tcaggatgaa 540
   caaattcaca ttgcctcctg gccgagcttt tcgttatatc ggggggcagc ctatgcactc 600
   ggccccgaac tgaacaacgc cgccagccaa atgtatgcag ccgaaggcca gtgctttgtc 660
   cttgcccctt gcgccaccgt ctcaaaggag atgatcgaaa tgctgataga tgatcccagg 720
   aaagagccgc ttctgctgga aggtggcggg ttcaccatga tttacggccc cgatgggcga 780
   cctctggcta aaccgttgcc tgaaaacgag gaagggctgc tatatgccga tattgacctg 840
   ggcatgattt caatggccaa ggctgccgcc gacccggcag gtcactacgc acgcccggat 900
   gtcactcgcc tactattcaa ttccgcgccc gccaatcgcg tcgagtatat caacccagcg 960
   tcaggcccaa ccgaatcctt aaaagatatg ggaaagatgc aaatggaggc cgaacagcaa 1020
   aaggcggccc tgcgagagat gatctaa 1047

<210> 16
<211> 348
<212> PRT
<213> Unknown

<220>
<223> Obtained from an environmental sample

<400> 16
   Met Pro Thr Ser Lys Gln Phe Arg Val Ala Ala Val Gln Ala Ala Pro
   1 5 10 15
   Val Phe Leu Asp Leu Glu Gly Ala Ile Ser Lys Gly Ile Ser Leu Ile
   20 25 30
   Glu Glu Ala Ala Ser Asn Gly Ala Lys Leu Ile Ala Phe Pro Glu Thr
   35 40 45
   Trp Ile Pro Gly Tyr Pro Trp Trp Ile Trp Leu Asp Ser Pro Ala Trp
   50 55 60
   Gly Met Arg Phe Val Gln Arg Tyr Phe Asp Asn Ser Leu Met Leu Gly
   65 70 75 80
   Ser Glu Gln Ala Lys Arg Met Asn Gln Ala Ala Ala Asn Asn Lys Ile
   85 90 95
   Tyr Val Val Met Gly Tyr Ser Glu Arg Ser Gly Gly Ser Leu Tyr Met
   100 105 110
   Gly Gln Ser Ile Ile Asn Asp Lys Gly Glu Thr Ile Phe Thr Arg Arg
   115 120 125
   Lys Leu Lys Pro Thr His Val Glu Arg Thr Val Phe Gly Glu Gly Asp
   130 135 140
   Gly Ser His Leu Cys Val Met Asp Thr Glu Ile Gly Arg Val Gly Ala
   145 150 155 160
   Met Cys Cys Trp Glu His Leu Gln Pro Leu Ser Lys Tyr Ala Met Tyr
   165 170 175
   Ser Gln Asp Glu Gln Ile His Ile Ala Ser Trp Pro Ser Phe Ser Leu
   180 185 190
   Tyr Arg Gly Ala Ala Tyr Ala Leu Gly Pro Glu Leu Asn Asn Ala Ala
   195 200 205
   Ser Gln Met Tyr Ala Ala Glu Gly Gln Cys Phe Val Leu Ala Pro Cys
   210 215 220
   Ala Thr Val Ser Lys Glu Met Ile Glu Met Leu Ile Asp Asp Pro Arg
   225 230 235 240
   Lys Glu Pro Leu Leu Leu Glu Gly Gly Gly Phe Thr Met Ile Tyr Gly
   245 250 255
   Pro Asp Gly Arg Pro Leu Ala Lys Pro Leu Pro Glu Asn Glu Glu Gly
   260 265 270
   Leu Leu Tyr Ala Asp Ile Asp Leu Gly Met Ile Ser Met Ala Lys Ala
   275 280 285
   Ala Ala Asp Pro Ala Gly His Tyr Ala Arg Pro Asp Val Thr Arg Leu
   290 295 300
   Leu Phe Asn Ser Ala Pro Ala Asn Arg Val Glu Tyr Ile Asn Pro Ala
   305 310 315 320
   Ser Gly Pro Thr Glu Ser Leu Lys Asp Met Gly Lys Met Gln Met Glu
   325 330 335
   Ala Glu Gln Gln Lys Ala Ala Leu Arg Glu Met Ile
   340 345

<210> 17
<211> 993
<212> DNA
<213> Unknown

<220>
<223> Obtained from an environmental sample

<400> 17
atgagagttg ttaaagccgc agctgtccaa ctgagtcccg tcctctatag ccgcgaggga 60
acggtcgaga aggtcgtgcg gaagatccat gaacttgccg aagagggagt cgagttcgtc 120
acctttcctg agaccgtggt gccttattac ccgtactttt cgttcgttca gacgcccttg 180
cagcaaatct tcggaacaga gtatctgagg ctgctcgacc aggcagtcac cgtgccatcc 240
gccgccaccg acgcgatcgg cgaggctgcc aggttcgctg gagttgttgt ctcgatcggc 300
gtcaacgagc gagacggggg aactctgtac aacactcagc ttctcttcga tgccgacgga 360
agcttaattc agcggcgccg caagatcacg cccacccatt acgagcgcat gatctggggc 420
cagggtgacg gctcaggtct gcgggccgtt gatagcaagg ccggccgcat tggtcagctg 480
gcatgctggg agcacaacaa tccactggcg cgctacgcgc tgatagccga cggcgagcag 540
atccattcgg ccatgtatcc gggctccatg ttcggcgact cgtttgccaa aaagaccgaa 600
atcaatatcc ggcagcatgc gctggagtct gcgtgcttcg tcgtgaacgc aacggcctgg 660
ctggacggcg atcaacaggc gcaaatcatg aaggacaccg gctgcagcat cggcccgatc 720
tccggcggtt gcttcaccac tatcgtggcg ccggacggtt ccctgatcgg cgagcccctc 780
cgctcgggtg agggcgtggt catcgccgac ctcgacttca cgttaatcga caggcgtaag 840
caggtgatgg actcgcgagg ccactacagc cggccggagt tgctcagcct cttaatagac 900
cgcaccccta ccgcgcactt tcacgaacgc gcttcgcacc ccacgacagg agctgagcaa 960
ggctccgagg atgtgttcga ggctaacatt taa 993

<210> 18
<211> 330
<212> PRT
<213> Unknown

<220>
<223> Obtained from an environmental sample

<400> 18
Met Arg Val Val Lys Ala Ala Ala Val Gln Leu Ser Pro Val Leu Tyr
1               5                   10                  15
Ser Arg Glu Gly Thr Val Glu Lys Val Val Arg Lys Ile His Glu Leu
            20                  25                  30
Ala Glu Glu Gly Val Glu Phe Val Thr Phe Pro Glu Thr Val Val Pro
        35                  40                  45
Tyr Tyr Pro Tyr Phe Ser Phe Val Gln Thr Pro Leu Gln Gln Ile Phe
    50                  55                  60
Gly Thr Glu Tyr Leu Arg Leu Leu Asp Gln Ala Val Thr Val Pro Ser
65                  70                  75                  80
Ala Ala Thr Asp Ala Ile Gly Glu Ala Ala Arg Phe Ala Gly Val Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Ser Leu Ile Gln Arg Arg Arg Lys
        115                 120                 125
Ile Thr Pro Thr His Tyr Glu Arg Met Ile Trp Gly Gln Gly Asp Gly
    130                 135                 140
Ser Gly Leu Arg Ala Val Asp Ser Lys Ala Gly Arg Ile Gly Gln Leu
145                 150                 155                 160
Ala Cys Trp Glu His Asn Asn Pro Leu Ala Arg Tyr Ala Leu Ile Ala
                165                 170                 175
Asp Gly Glu Gln Ile His Ser Ala Met Tyr Pro Gly Ser Met Phe Gly
            180                 185                 190
Asp Ser Phe Ala Lys Lys Thr Glu Ile Asn Ile Arg Gln His Ala Leu
        195                 200                 205
Glu Ser Ala Cys Phe Val Val Asn Ala Thr Ala Trp Leu Asp Gly Asp
    210                 215                 220
Gln Gln Ala Gln Ile Met Lys Asp Thr Gly Cys Ser Ile Gly Pro Ile
225                 230                 235                 240
Ser Gly Gly Cys Phe Thr Thr Ile Val Ala Pro Asp Gly Ser Leu Ile
                245                 250                 255

Gly Glu Pro Leu Arg Ser Gly Glu Gly Val Val Ile Ala Asp Leu Asp
            260                 265                 270
Phe Thr Leu Ile Asp Arg Arg Lys Gln Val Met Asp Ser Arg Gly His
        275                 280                 285
Tyr Ser Arg Pro Glu Leu Leu Ser Leu Leu Ile Asp Arg Thr Pro Thr
    290                 295                 300
Ala His Phe His Glu Arg Ala Ser His Pro Thr Thr Gly Ala Glu Gln
305                 310                 315                 320
Gly Ser Glu Asp Val Phe Glu Ala Asn Ile
                325                 330

<210> 19
<211> 1050
<212> DNA
<213> Unknown

<220>
<223> Obtained from an environmental sample

<400> 19
atggaaagca acttccttgc cgcagcagtg caagcagaac cggtttactt caatgctttt      60
cagacggccg aaaaggccgc gtcattgatt gacgatgccg gtcggcaggg ggctcgctta     120
gtgacatttc ccgaaacgtg gctgcccggt tacccgtact ggatctggct tggtgccccc     180
gcctggggaa tgcatcattt catcctaaag taccatcaaa actcgccggt tgcaggagga     240
ccagaggaac agatcctttg tcaggcggcc cgccgcaacg ggatttttgt cgtcatggga     300
ctcagcgaga aaatcggggc aagcctctac atggcgcagt ggttcatcag tccagacggc     360
aaagtggtcg ctcgccgacg caaattgaag cctactcacg tcgaacgttc ggtcttcggg     420
gaaggggatg gttccgacat tgtcgttctt gatacacccc ttggaaaggt cgggggcctt     480
tgctgctggg agcacatgca gccactttcg aagtacgcca tgtactcgca aggcgagcag     540
atccatgctg cttcttggcc gagtgttagc gtctatcgcg ataaaattta cgttctgggg     600
ccggagctga acggtgccgc caatcagatg tatgcggcag aaggtcagtg tttcgtcctg     660
gcatcctggg caacggtttc acaagcggct atcgatcttt tttgcgacac gcccgacaag     720
gccgcgctca tgaaaattgg tggtggtttt tcccagatct atgggccaga cgggtgcccc     780
ctggcgaagc cgttgccgga ggacgtcgaa ggattggtga ccgctgagat tgacttcaat     840
gccatcacgc gcgtgaaagc agcggcggac cccgtagggc actatagccg gcccgatgta     900
ttccgcctgt tgttcaatcg tacgcgccaa gaacgcgtgg tttctgtcaa cacgtttgtg     960
ccaggtgtca cccagcgaac cgccaagaat gggtcggcgg acgaattggt cggtcacccg    1020
gagaacgctg tcgcccgggc tgcagagtaa                                     1050

<210> 20
<211> 349
<212> PRT
<213> Unknown

<220>
<223> Obtained from an environmental sample

<400> 20
Met Glu Ser Asn Phe Leu Ala Ala Ala Val Gln Ala Glu Pro Val Tyr
 1               5                  10                  15
Phe Asn Ala Phe Gln Thr Ala Glu Lys Ala Ala Ser Leu Ile Asp Asp
            20                  25                  30
Ala Gly Arg Gln Gly Ala Arg Leu Val Thr Phe Pro Glu Thr Trp Leu
        35                  40                  45
Pro Gly Tyr Pro Tyr Trp Ile Trp Leu Gly Ala Pro Ala Trp Gly Met
    50                  55                  60
His His Phe Ile Leu Lys Tyr His Gln Asn Ser Pro Val Ala Gly Gly
65                  70                  75                  80
Pro Glu Glu Gln Ile Leu Cys Gln Ala Ala Arg Arg Asn Gly Ile Phe
                85                  90                  95
Val Val Met Gly Leu Ser Glu Lys Ile Gly Ala Ser Leu Tyr Met Ala
            100                 105                 110
Gln Trp Phe Ile Ser Pro Asp Gly Lys Val Val Ala Arg Arg Arg Lys
        115                 120                 125
Leu Lys Pro Thr His Val Glu Arg Ser Val Phe Gly Glu Gly Asp Gly
    130                 135                 140
Ser Asp Ile Val Val Leu Asp Thr Pro Leu Gly Lys Val Gly Gly Leu
145                 150                 155                 160

Cys Cys Trp Glu His Met Gln Pro Leu Ser Lys Tyr Ala Met Tyr Ser
                165                 170                 175
Gln Gly Glu Gln Ile His Ala Ala Ser Trp Pro Ser Val Ser Val Tyr
            180                 185                 190
Arg Asp Lys Ile Tyr Val Leu Gly Pro Glu Leu Asn Gly Ala Ala Asn
        195                 200                 205
Gln Met Tyr Ala Ala Glu Gly Gln Cys Phe Val Leu Ala Ser Trp Ala
    210                 215                 220
Thr Val Ser Gln Ala Ala Ile Asp Leu Phe Cys Asp Thr Pro Asp Lys
225                 230                 235                 240
Ala Ala Leu Met Lys Ile Gly Gly Gly Phe Ser Gln Ile Tyr Gly Pro
                245                 250                 255
Asp Gly Cys Pro Leu Ala Lys Pro Leu Pro Glu Asp Val Glu Gly Leu
            260                 265                 270
Val Thr Ala Glu Ile Asp Phe Asn Ala Ile Thr Arg Val Lys Ala Ala
        275                 280                 285
Ala Asp Pro Val Gly His Tyr Ser Arg Pro Asp Val Phe Arg Leu Leu
    290                 295                 300
Phe Asn Arg Thr Arg Gln Glu Arg Val Val Ser Val Asn Thr Phe Val
305                 310                 315                 320
Pro Gly Val Thr Gln Arg Thr Ala Lys Asn Gly Ser Ala Asp Glu Leu
                325                 330                 335
Val Gly His Pro Glu Asn Ala Val Ala Arg Ala Ala Glu
            340                 345

<210> 21
<211> 1065
<212> DNA
<213> Unknown

<220>
<223> Obtained from an environmental sample

<400> 21
atggcactag aacatccgaa gtacgtggcg gccgtggttc aggccgcgcc cgaattcctg      60
aatctagaca gagggatcga aaagacgatc gcattgatcg acgaagcggg acagaaaggg     120
gcggccctga ttgcatttcc ggaaacctgg ctgccgggct atccgtttca tgtctggctc     180
ggtcctcccg catgggcgct tggctcagga ttcgtccagc gctatttcga caactcgatg     240
acgtacgata gtcctcaggc cgctgcactg agggacgctg ccgcgcgcaa cgggatcacg     300
gtggtattgg gcttgtcgga gcgatgcggc ggcagcctct atatcgcgca atggatcatc     360
ggcccggatg gcgcgacggt cgccacgcgc cgcaaattgc ggccgactca tatcgagcgc     420
accgttttcg gcgatggcga cggcagcgat ctggcagtac acgatctcaa catcggccgc     480
cttggcgcac tgtgctgctg ggagcacatt cagccgctga ccaagtacgc gatgtatgcg     540
cagcacgaac aggtgcacgt cgcggcctgg ccgagcttct ccatgtatga attcgcgccc     600
gcgctcggtc acgaggtgaa caacgcagtc agccgcgtct atgccgttga gggatcgtgc     660
ttcgtgctcg cgccgtgcgc ggtcatcagc gagcaaatgg tcgacatgtt gtgcgacacg     720
gcagacaagc gcgcgatgat acgtgccggc ggcgggcacg cagtggcgtt cgggccggac      780
ggcgaagctc tggtcgagaa actgccggaa aatgaggaag gcttgctgct ggtcgatatc     840
gatctcggtc gcatctcgct tgcgaaggct gcggccgacc ccgtcggtca ctacgcgcgc     900
cccgatgtct tgcggctctg gttcgacaag caaccgcggc ggtgcgtcga acatgccggc     960
gagaacgacg cgtcgcgcag gtcgcacggg tcgtccgggt cacaatcgcc ggcgcaggat    1020
gggccggcga acgacatggt agaccgtcag gaaaacgtcg attga                    1065

<210> 22
<211> 354
<212> PRT
<213> Unknown

<220>
<223> Obtained from an environmental sample

<400> 22
Met Ala Leu Glu His Pro Lys Tyr Val Ala Ala Val Val Gln Ala Ala
1               5                   10                  15
Pro Glu Phe Leu Asn Leu Asp Arg Gly Ile Glu Lys Thr Ile Ala Leu
            20                  25                  30
Ile Asp Glu Ala Gly Gln Lys Gly Ala Ala Leu Ile Ala Phe Pro Glu
        35                  40                  45

Thr Trp Leu Pro Gly Tyr Pro Phe His Val Trp Leu Gly Pro Pro Ala
    50                  55                  60
Trp Ala Leu Gly Ser Gly Phe Val Gln Arg Tyr Phe Asp Asn Ser Met
65                  70                  75                  80
Thr Tyr Asp Ser Pro Gln Ala Ala Ala Leu Arg Asp Ala Ala Ala Arg
                85                  90                  95
Asn Gly Ile Thr Val Val Leu Gly Leu Ser Glu Arg Cys Gly Gly Ser
            100                 105                 110
Leu Tyr Ile Ala Gln Trp Ile Ile Gly Pro Asp Gly Ala Thr Val Ala
        115                 120                 125
Thr Arg Arg Lys Leu Arg Pro Thr His Ile Glu Arg Thr Val Phe Gly
    130                 135                 140
Asp Gly Asp Gly Ser Asp Leu Ala Val His Asp Leu Asn Ile Gly Arg
145                 150                 155                 160
Leu Gly Ala Leu Cys Cys Trp Glu His Ile Gln Pro Leu Thr Lys Tyr
                165                 170                 175
Ala Met Tyr Ala Gln His Glu Gln Val His Val Ala Ala Trp Pro Ser
            180                 185                 190
Phe Ser Met Tyr Glu Phe Ala Pro Ala Leu Gly His Glu Val Asn Asn
        195                 200                 205
Ala Val Ser Arg Val Tyr Ala Val Glu Gly Ser Cys Phe Val Leu Ala
    210                 215                 220
Pro Cys Ala Val Ile Ser Glu Gln Met Val Asp Met Leu Cys Asp Thr
225                 230                 235                 240
Ala Asp Lys Arg Ala Met Ile Arg Ala Gly Gly Gly His Ala Val Ala
                245                 250                 255
Phe Gly Pro Asp Gly Glu Ala Leu Val Glu Lys Leu Pro Glu Asn Glu
            260                 265                 270
Glu Gly Leu Leu Leu Val Asp Ile Asp Leu Gly Arg Ile Ser Leu Ala
        275                 280                 285
Lys Ala Ala Ala Asp Pro Val Gly His Tyr Ala Arg Pro Asp Val Leu
    290                 295                 300
Arg Leu Trp Phe Asp Lys Gln Pro Arg Arg Cys Val Glu His Ala Gly
305                 310                 315                 320
Glu Asn Asp Ala Ser Arg Arg Ser His Gly Ser Ser Gly Ser Gln Ser
                325                 330                 335
Pro Ala Gln Asp Gly Pro Ala Asn Asp Met Val Asp Arg Gln Glu Asn
            340                 345                 350
Val Asp


<210> 23
<211> 1005
<212> DNA
<213> Unknown

<220>
<223> Obtained from an environmental sample

<400> 23
atgaccgtta tcaaagcagc cgccattcaa atcagccccg tgctctacag ccgggcgggg      60
acagtcgaga aagttgttag gaaggttaga gagctcgggg ccaaaggtgt ccgattcgct     120
acctttcccg aaaccatcat accgtactac ccgtacttct cgttcgttca gtcggcgttc     180
gacatgaagc ttgggagtga acatcagcgg ctgctcgacg aatcagtcac aattccttcg     240
tccgagacgg acgcgatcgc ccaggccgcc aaggaagcgg gcatggtggt gtccgtcggg     300
gtcaatgagc gcgatgggcg atccatctac aacactcaac ttctgttcga cgctgatggc     360
acgctcattc agcgtaggcg aaagatcacc ccgacctatc acgagcgcat gatttggggt     420
caaggcgatg gatccggcct acgcgcggtc gatagcgccg tgggccggat cggccagctt     480
gcctgctggg agcactacct tcccctggcg cggtacgccc tcatcgcgga cggagagcaa     540
atccactcgg caatgtatcc aggctcgttc gctggtccgc tatttgccga gcagatagag     600
gttagtatcc gccagcacgc gcttgagtca gcctgcttcg tcgtcaacgc gaccggatgg     660
cttagcgccg agcagcaagc tcaaatagtg aaggataccg gatgcgtcgt tggaccaatc     720
tccggtggct gctttacggc gattgttgat ccggagggtc ggatcatggg ggcgccactc     780
aaggcaggtg agggggaggt catcgcagat ctcgattttg cgcagattga tttccgcaag     840
cgtgtgatgg atacgcgagg gcactacagc cgccccgaac ttctaagcct cacgatcgac     900
cgcagtcagc accatcacat gactgagcga ggcgccgatc accgtgtaga ccacgcaaag     960
ccaacggtca ccgcagagca gtcggccgtc gagccggcgg aatga                    1005

<210> 24
<211> 334
<212> PRT
<213> Unknown

<220>
<223> Obtained from an environmental sample

<400> 24
Met Thr Val Ile Lys Ala Ala Ala Ile Gln Ile Ser Pro Val Leu Tyr
1               5                   10                  15
Ser Arg Ala Gly Thr Val Glu Lys Val Val Arg Lys Val Arg Glu Leu
            20                  25                  30
Gly Ala Lys Gly Val Arg Phe Ala Thr Phe Pro Glu Thr Ile Ile Pro
        35                  40                  45
Tyr Tyr Pro Tyr Phe Ser Phe Val Gln Ser Ala Phe Asp Met Lys Leu
    50                  55                  60
Gly Ser Glu His Gln Arg Leu Leu Asp Glu Ser Val Thr Ile Pro Ser
65                  70                  75                  80
Ser Glu Thr Asp Ala Ile Ala Gln Ala Ala Lys Glu Ala Gly Met Val
                85                  90                  95
Val Ser Val Gly Val Asn Glu Arg Asp Gly Arg Ser Ile Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr Tyr His Glu Arg Met Ile Trp Gly Gln Gly Asp Gly
    130                 135                 140
Ser Gly Leu Arg Ala Val Asp Ser Ala Val Gly Arg Ile Gly Gln Leu
145                 150                 155                 160
Ala Cys Trp Glu His Tyr Leu Pro Leu Ala Arg Tyr Ala Leu Ile Ala
                165                 170                 175
Asp Gly Glu Gln Ile His Ser Ala Met Tyr Pro Gly Ser Phe Ala Gly
            180                 185                 190
Pro Leu Phe Ala Glu Gln Ile Glu Val Ser Ile Arg Gln His Ala Leu
        195                 200                 205
Glu Ser Ala Cys Phe Val Val Asn Ala Thr Gly Trp Leu Ser Ala Glu
    210                 215                 220
Gln Gln Ala Gln Ile Val Lys Asp Thr Gly Cys Val Val Gly Pro Ile
225                 230                 235                 240
Ser Gly Gly Cys Phe Thr Ala Ile Val Asp Pro Glu Gly Arg Ile Met
                245                 250                 255
Gly Ala Pro Leu Lys Ala Gly Glu Gly Glu Val Ile Ala Asp Leu Asp
            260                 265                 270
Phe Ala Gln Ile Asp Phe Arg Lys Arg Val Met Asp Thr Arg Gly His
        275                 280                 285
Tyr Ser Arg Pro Glu Leu Leu Ser Leu Thr Ile Asp Arg Ser Gln His
    290                 295                 300
His His Met Thr Glu Arg Gly Ala Asp His Arg Val Asp His Ala Lys
305                 310                 315                 320
Pro Thr Val Thr Ala Glu Gln Ser Ala Val Glu Pro Ala Glu
                325                 330

<210> 25
<211> 939
<212> DNA
<213> Unknown

<220>
<223> Obtained from an environmental sample

<400> 25
gtgtcatcaa ctatcaaagt cgccattatt caggccgctc ctgcttacta cgacctgcag      60
gcgtcgctgg caaaggccgc cagtctgatc cgcgaggcgg cacgcggcgg cgcgcaattc     120
gtcgcgttcg gggagacatg gctgccgggc tatccgatgt ggctggattg gtgtcctggc     180
gcgatcatct gggataaccc cgccaccaaa accgtcttcg cgcgcctcca tgaaaacagc     240
gtcgccgttc ccggcaggga aacggcattt ctcgccgacc ttgcgatgtc gttaagcatc     300
gtattatgca tcggcgtcaa tgagaaggtc atgaatgggc cgggacacgg cacgctctac     360
aacacgctcc tgacgtttga tgcaacgggt gaaatcatca atcatcatcg caagttgatg     420

ccaacctatg gcgagagatt ggtatggggg ccgggcgacg cagttggcgt gcaagcggtt 480
gatagtacgg tcgggcgcat cggcgggctg atctgttggg agcactggat gccgctgcca 540
cgccaactca tgcacaacag cggcgagcag attcacgtct gcgcatggcc gggcgtgcac 600
gaaatgcacc agatcgcgag ccgtcattat gcattcgagg gccgctgctt tgtgctggcc 660
gccggattga tcatgcccgc gttcgacctg cccagcgaac tcgaatttcc gcccgaactg 720
gccgacaagc gcgactatct cctaatgaac ggcggcagcg ccatcatcaa gcccaatggc 780
aaatatctcg ccgggccggt ttatgacgaa gagactattc tctgcgccga ccttgacctg 840
actgagaaca tcaaggagca gatgacgctg gacgtgacag ggcattatgc gcgagcggaa 900
ctgtttgact tgaatgtggt gcggcggcgg aatgcgtag 939

<210> 26
<211> 312
<212> PRT
<213> Unknown

<220>
<223> Obtained from an environmental sample

<400> 26
Val Ser Ser Thr Ile Lys Val Ala Ile Ile Gln Ala Ala Pro Ala Tyr
1               5                   10                  15
Tyr Asp Leu Gln Ala Ser Leu Ala Lys Ala Ala Ser Leu Ile Arg Glu
            20                  25                  30
Ala Ala Arg Gly Gly Ala Gln Phe Val Ala Phe Gly Glu Thr Trp Leu
        35                  40                  45
Pro Gly Tyr Pro Met Trp Leu Asp Trp Cys Pro Gly Ala Ile Ile Trp
    50                  55                  60
Asp Asn Pro Ala Thr Lys Thr Val Phe Ala Arg Leu His Glu Asn Ser
65                  70                  75                  80
Val Ala Val Pro Gly Arg Glu Thr Ala Phe Leu Ala Asp Leu Ala Met
                85                  90                  95
Ser Leu Ser Ile Val Leu Cys Ile Gly Val Asn Glu Lys Val Met Asn
            100                 105                 110
Gly Pro Gly His Gly Thr Leu Tyr Asn Thr Leu Leu Thr Phe Asp Ala
        115                 120                 125
Thr Gly Glu Ile Ile Asn His His Arg Lys Leu Met Pro Thr Tyr Gly
    130                 135                 140
Glu Arg Leu Val Trp Gly Pro Gly Asp Ala Val Gly Val Gln Ala Val
145                 150                 155                 160
Asp Ser Thr Val Gly Arg Ile Gly Gly Leu Ile Cys Trp Glu His Trp
                165                 170                 175
Met Pro Leu Pro Arg Gln Leu Met His Asn Ser Gly Glu Gln Ile His
            180                 185                 190
Val Cys Ala Trp Pro Gly Val His Glu Met His Gln Ile Ala Ser Arg
        195                 200                 205
His Tyr Ala Phe Glu Gly Arg Cys Phe Val Leu Ala Ala Gly Leu Ile
    210                 215                 220
Met Pro Ala Phe Asp Leu Pro Ser Glu Leu Glu Phe Pro Pro Glu Leu
225                 230                 235                 240
Ala Asp Lys Arg Asp Tyr Leu Leu Met Asn Gly Gly Ser Ala Ile Ile
                245                 250                 255
Lys Pro Asn Gly Lys Tyr Leu Ala Gly Pro Val Tyr Asp Glu Glu Thr
            260                 265                 270
Ile Leu Cys Ala Asp Leu Asp Leu Thr Glu Asn Ile Lys Glu Gln Met
        275                 280                 285
Thr Leu Asp Val Thr Gly His Tyr Ala Arg Ala Glu Leu Phe Asp Leu
    290                 295                 300
Asn Val Val Arg Arg Arg Asn Ala
305                 310

<210> 27
<211> 1056
<212> DNA
<213> Unknown

<220>
<223> Obtained from an environmental sample

<400> 27
atgccaaccc ccagcgatca tttcaaaatc gccgctgttc aggcctcgcc cgtgtttctg      60
gaccgggagg ccactgtgga aaaggcctgc cggttgatcg ccgaagccgc aaagcagggc     120
gcccgcctca tcgtctttcc ggaatctttc atcccgacct acccggattg ggtgtgggcc     180
gttcccccgg gaagggaaag aatcctgaac cagctgtatt ctgaattcct ggccaatgcc     240
gtcgatgttc ccggcgcggc gaccgaacaa cttgcccagg ctgcacgaat ggccggcgcc     300
tatgtgatta tgggcgtcac cgaaagagac acctcggcca gcggggccag cctctacaac     360
accctgctct acttcagccc cgaaggcatc ctaatgggca aacaccggaa gctggttccc     420
acggggggcg aacggctggt ctgggcctac ggagacggca gcacgctgga ggtctacgac     480
actccgctgg gaaagatcgg cgggctgatc tgctgggaga actacatgcc cctggcccgg     540
tacacgatgt acgcctgggg cacccagatt tacatcgccg ccacctggga ccgcggggaa     600
ccgtggctct ccaccctgcg gcatatcgcc aaggaaggaa gggtctacgt catcgggtgc     660
tgcatcgccc tgcgccaggg ggatatcccg gaccggttcg agtacaaggg aaaattttat     720
tccgggtccc gggagtggat caatgagggc gacagcgcca tcgtgaaccc ggacggggaa     780
ttcatcgccg ggccggtgcg gatgaaggag gagatcctgt atgccgagat agacccccgg     840
cagatgcggg gccccaagtg gatgctcgat gtggccggtc attacgcccg gccggatatc     900
ttcgagctca tcgtccaccg gaatccccac ccgatgatca aaatcgccga agacaggggc     960
acggggatcg cctcaagttt gattcgcccc cgccctaacc ttcccccatc aagggggagg    1020
aaatcggcaa gaagcaaacg caagcccaaa aaatga                              1056

<210> 28
<211> 351
<212> PRT
<213> Unknown

<220>
<223> Obtained from an environmental sample

<400> 28
Met Pro Thr Pro Ser Asp His Phe Lys Ile Ala Ala Val Gln Ala Ser
1               5                   10                  15
Pro Val Phe Leu Asp Arg Glu Ala Thr Val Glu Lys Ala Cys Arg Leu
            20                  25                  30
Ile Ala Glu Ala Ala Lys Gln Gly Ala Arg Leu Ile Val Phe Pro Glu
        35                  40                  45
Ser Phe Ile Pro Thr Tyr Pro Asp Trp Val Trp Ala Val Pro Pro Gly
    50                  55                  60
Arg Glu Arg Ile Leu Asn Gln Leu Tyr Ser Glu Phe Leu Ala Asn Ala
65                  70                  75                  80
Val Asp Val Pro Gly Ala Ala Thr Glu Gln Leu Ala Gln Ala Ala Arg
                85                  90                  95
Met Ala Gly Ala Tyr Val Ile Met Gly Val Thr Glu Arg Asp Thr Ser
            100                 105                 110
Ala Ser Gly Ala Ser Leu Tyr Asn Thr Leu Leu Tyr Phe Ser Pro Glu
        115                 120                 125
Gly Ile Leu Met Gly Lys His Arg Lys Leu Val Pro Thr Gly Gly Glu
    130                 135                 140
Arg Leu Val Trp Ala Tyr Gly Asp Gly Ser Thr Leu Glu Val Tyr Asp
145                 150                 155                 160
Thr Pro Leu Gly Lys Ile Gly Gly Leu Ile Cys Trp Glu Asn Tyr Met
                165                 170                 175
Pro Leu Ala Arg Tyr Thr Met Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Lys Glu Gly Arg Val Tyr Val Ile Gly Cys Cys Ile Ala Leu
    210                 215                 220
Arg Gln Gly Asp Ile Pro Asp Arg Phe Glu Tyr Lys Gly Lys Phe Tyr
225                 230                 235                 240
Ser Gly Ser Arg Glu Trp Ile Asn Glu Gly Asp Ser Ala Ile Val Asn
                245                 250                 255
Pro Asp Gly Glu Phe Ile Ala Gly Pro Val Arg Met Lys Glu Glu Ile
            260                 265                 270
Leu Tyr Ala Glu Ile Asp Pro Arg Gln Met Arg Gly Pro Lys Trp Met
        275                 280                 285
Leu Asp Val Ala Gly His Tyr Ala Arg Pro Asp Ile Phe Glu Leu Ile
    290                 295                 300
Val His Arg Asn Pro His Pro Met Ile Lys Ile Ala Glu Asp Arg Gly

305                 310                 315                 320
Thr Gly Ile Ala Ser Ser Leu Ile Arg Pro Arg Pro Asn Leu Pro Pro
                325                 330                 335
Ser Arg Gly Arg Lys Ser Ala Arg Ser Lys Arg Lys Pro Lys Lys
            340                 345                 350

<210> 29
<211> 1017
<212> DNA
<213> Unknown

<220>
<223> Obtained from an environmental sample

<400> 29
atgggcatcg aacatacgaa atacaaggtg gcggtggtgc aggcggcgcc ggcctggctc      60
gacctcgagg cctcgatcgg caagtccatc ggcctaatca aggaggccgc ggacaagggc     120
gccaagctga tcgcctttcc ggaggccttc atccccggtt acccctggta tatctggatg     180
gactcgccgg cctgggcgat cggccgcggc ttcgtccagc gctatttcga caattcgctc     240
tcctacgaca gtccccaggc cgagcggctg cgtgatgccg tgcgccaggc caagctcacc     300
gccgtgatcg gcctgtccga acgcgacggc ggcagccttt acctggcgca atggttgatc     360
gggcccgacg gcgaaaccat tgccaagcgc cgcaagctgc ggccgaccca tgccgagcgc     420
accgtctatg gcgaaggcga cggcagcgat ctggccgtac atgcccggcc cgacatcggt     480
cgcttgggcg cgctgtgctg ctgggagcat cttcagccgt tgtcgaagta cgcaatgtac     540
gcccagaacg agcaggtcca cgtcgctgcc tggccgagct tctcgctcta cgatcccttc     600
gccccggcgc tcggcgccga ggtcaacaac gctgcctcgc gcgtctatgc ggtggagggc     660
tcctgcttcg tgctcgcgcc ttgcgcgacg gtgtcgcagg ccatgatcga cgaactctgc     720
gatcggcccg ataagcatgc gctgctgcat gccggcggag gctttgccgc gatctacggc     780
cccgacggca gccagatcgg cgagaagctg gcgccggatc aggagggtct gctgatcgcc     840
gagattgatc tgggcgccat cggtgttgcc aagaacgcgg cagatcccgc cggtcattat     900
tcacggccgg atgtgacgcg gttgctgctc aacaagaagc ggtaccagcg cgtcgagcaa     960
tttgccttgc ccgccgacat ggtcgagccc gcggacatag gcgcggcggc gagctga       1017

<210> 30
<211> 338
<212> PRT
<213> Unknown

<220>
<223> Obtained from an environmental sample

<400> 30
Met Gly Ile Glu His Thr Lys Tyr Lys Val Ala Val Val Gln Ala Ala
1               5                   10                  15
Pro Ala Trp Leu Asp Leu Glu Ala Ser Ile Gly Lys Ser Ile Gly Leu
            20                  25                  30
Ile Lys Glu Ala Ala Asp Lys Gly Ala Lys Leu Ile Ala Phe Pro Glu
        35                  40                  45
Ala Phe Ile Pro Gly Tyr Pro Trp Tyr Ile Trp Met Asp Ser Pro Ala
    50                  55                  60
Trp Ala Ile Gly Arg Gly Phe Val Gln Arg Tyr Phe Asp Asn Ser Leu
65                  70                  75                  80
Ser Tyr Asp Ser Pro Gln Ala Glu Arg Leu Arg Asp Ala Val Arg Gln
                85                  90                  95
Ala Lys Leu Thr Ala Val Ile Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Ala Arg Pro Asp Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Pro Ala Leu Gly Ala Glu Val
        195                 200                 205

Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Ala Gly Gly Gly Phe Ala
                245                 250                 255
Ala Ile Tyr Gly Pro Asp Gly Ser Gln Ile Gly Glu Lys Leu Ala Pro
            260                 265                 270
Asp Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Lys Arg Tyr Gln Arg Val Glu Gln
305                 310                 315                 320
Phe Ala Leu Pro Ala Asp Met Val Glu Pro Ala Asp Ile Gly Ala Ala
                325                 330                 335
Ala Ser

<210> 31
<211> 933
<212> DNA
<213> Unknown

<220>
<223> Obtained from an environmental sample

<400> 31
atgaccagaa tagccattat tcagcgaccg cccgtgctgc tcgatcgaag cgccaccatt      60
gcccgggccg tgcaatcggt cgccgaagcg gcagcgcaag gcgcgaccct gattgtcttg     120
cccgaatcgt acatccctgg ctatccctca tggatctggc ggctcgcgcc tggcaaagac     180
ggcgcgatcg tgggccagtt gcatgcgcgc ttgctggcca atgcggtcga cctgagcagc     240
actgacctcg atgcgcttct tgaagcggcc cgtcagcacg gcgtgaccat tgtttgcggc     300
atgaacgagt gcgaacggcg tcgcggcggc ggcaccttgt acaacacggt ggtcgtgatc     360
ggaccggacg gcgtcatgct caaccggcat cgcaaattga tgccgaccaa tcccgagcgc     420
atggtgcatg gctttggcga tgcatccgga ctgaaagcag ttgatacgcc tgccggccgg     480
ctgggcacgc tgatctgctg ggagagctac atgccgctgg cacgctatgc cctgtacgag     540
caaggcatcg agatctacat cgcaccaact tatgacagtg gtgacggctg gatcagcacc     600
atgcgccaca ttgcactcga agggcgctgc tgggtgattg gcagcggcac ggtcctgaaa     660
ggcagtgata ttccggacga tttcccggaa cggcacgcct tgttccctga tccggatgag     720
tggatcaacg atggtgattc ggtagttatc gatccgcagg gaaagatcgt tgccggtccg     780
atgcgtaggg aagcaggcat tctatacgcc gatatcgacg tcgcgcgcgt agcaccatca     840
cgccgcacgc tggatgtcgc ggggcattac gcgcgtccgg acgtcttcga gcttcgggta     900
caccaggcac cgggggcacg agtaagtaat tga                                  933

<210> 32
<211> 310
<212> PRT
<213> Unknown

<220>
<223> Obtained from an environmental sample

<400> 32
Met Thr Arg Ile Ala Ile Ile Gln Arg Pro Pro Val Leu Leu Asp Arg
 1               5                  10                  15
Ser Ala Thr Ile Ala Arg Ala Val Gln Ser Val Ala Glu Ala Ala Ala
            20                  25                  30
Gln Gly Ala Thr Leu Ile Val Leu Pro Glu Ser Tyr Ile Pro Gly Tyr
        35                  40                  45
Pro Ser Trp Ile Trp Arg Leu Ala Pro Gly Lys Asp Gly Ala Ile Val
    50                  55                  60
Gly Gln Leu His Ala Arg Leu Leu Ala Asn Ala Val Asp Leu Ser Ser
65                  70                  75                  80
Thr Asp Leu Asp Ala Leu Leu Glu Ala Ala Arg Gln His Gly Val Thr
                85                  90                  95
Ile Val Cys Gly Met Asn Glu Cys Glu Arg Arg Arg Gly Gly Gly Thr
            100                 105                 110

Leu Tyr Asn Thr Val Val Val Ile Gly Pro Asp Gly Val Met Leu Asn
        115                 120                 125
Arg His Arg Lys Leu Met Pro Thr Asn Pro Glu Arg Met Val His Gly
    130                 135                 140
Phe Gly Asp Ala Ser Gly Leu Lys Ala Val Asp Thr Pro Ala Gly Arg
145                 150                 155                 160
Leu Gly Thr Leu Ile Cys Trp Glu Ser Tyr Met Pro Leu Ala Arg Tyr
                165                 170                 175
Ala Leu Tyr Glu Gln Gly Ile Glu Ile Tyr Ile Ala Pro Thr Tyr Asp
            180                 185                 190
Ser Gly Asp Gly Trp Ile Ser Thr Met Arg His Ile Ala Leu Glu Gly
        195                 200                 205
Arg Cys Trp Val Ile Gly Ser Gly Thr Val Leu Lys Gly Ser Asp Ile
    210                 215                 220
Pro Asp Asp Phe Pro Glu Arg Ala Arg Leu Phe Pro Asp Pro Asp Glu
225                 230                 235                 240
Trp Ile Asn Asp Gly Asp Ser Val Val Ile Asp Pro Gln Gly Lys Ile
                245                 250                 255
Val Ala Gly Pro Met Arg Arg Glu Ala Gly Ile Leu Tyr Ala Asp Ile
            260                 265                 270
Asp Val Ala Arg Val Ala Pro Ser Arg Arg Thr Leu Asp Val Ala Gly
        275                 280                 285
His Tyr Ala Arg Pro Asp Val Phe Glu Leu Arg Val His Gln Ala Pro
    290                 295                 300
Gly Ala Arg Val Ser Asn
305                 310

<210> 33
<211> 1026
<212> DNA
<213> Unknown

<220>
<223> Obtained from an environmental sample

<400> 33
atgttaagtc ccgtgacgca gtatcgcgcc gccgcggtgc aggcggcgcc atcttttctc      60
gatctcgacc gcaccgtcga gaagacgatc gcgatcatcg agcaggcggc cgagcaggat     120
gtgcgcctga tcgcgtttcc ggaaacctgg attcccggct atccgctctg gatctggctc     180
ggctcgccgg cctggggcat gcgcttcgtg cagcgctatt tcgagaactc gctggtgcgc     240
ggcagcaaac agtggaacgc gatcgccgat gcggcgcggc gccaccgcat gaccgtcgtc     300
gtcggcttca gcgagcgcgc gggaggcagc ctctacatgg gccaggcgat cttcggcccc     360
gaaggcgagc tcatcgcggc gcgccggaag ctcaagccga cacacgccga gcgaacggtg     420
ttcggcgagg gcgacggcag ccacttggcc gtttacgaga cgggcgttgg tcgcatcggc     480
gccctctgct gctgggagca catccagccg ctctcgaaat acgcgatgta tgcggccaac     540
gaacaggtgc atgtggcctc gtggccgtgc ttcagccttt atcgcggcat ggcctatgcg     600
ctcgggccgg aggtgaacac cgccgcgagc caggtctacg cggtcgaggg cggctgctac     660
gtgctggcct cctgtctcgt cgtgacaccc gagatcctga aggtgctgat cgacacgccc     720
gacaaggagc cgttgctgct cgccggcggg gggttctcga tgatcttcgg ccccgacggc     780
cgcgcgctcg cccagccgct gccggagacc gaagaggggc tcgtcacggc cgagatcgat     840
ctcggcgcga tcgcgctcgc caaggccgcg gccgatcccg ccggccatta cgcgcggccc     900
gacgtgacgc ggttgttgct gaacccgcgc cccgcggcgc gcgtcgaagc gctgggtccg     960
cgcttcgagg tcgtgcagag cgagcaggcc gagccgccca cgcaaccggc cgaagcggcg    1020
gattga                                                               1026

<210> 34
<211> 341
<212> PRT
<213> Unknown

<220>
<223> Obtained from an environmental sample

<400> 34
Met Leu Ser Pro Val Thr Gln Tyr Arg Ala Ala Ala Val Gln Ala Ala
1               5                   10                  15
Pro Ser Phe Leu Asp Leu Asp Arg Thr Val Glu Lys Thr Ile Ala Ile
            20                  25                  30

Ile Glu Gln Ala Ala Glu Gln Asp Val Arg Leu Ile Ala Phe Pro Glu
        35                  40                  45
Thr Trp Ile Pro Gly Tyr Pro Leu Trp Ile Trp Leu Gly Ser Pro Ala
    50                  55                  60
Trp Gly Met Arg Phe Val Gln Arg Tyr Phe Glu Asn Ser Leu Val Arg
65                  70                  75                  80
Gly Ser Lys Gln Trp Asn Ala Ile Ala Asp Ala Ala Arg Arg His Arg
                85                  90                  95
Met Thr Val Val Val Gly Phe Ser Glu Arg Ala Gly Gly Ser Leu Tyr
            100                 105                 110
Met Gly Gln Ala Ile Phe Gly Pro Glu Gly Glu Leu Ile Ala Ala Arg
        115                 120                 125
Arg Lys Leu Lys Pro Thr His Ala Glu Arg Thr Val Phe Gly Glu Gly
    130                 135                 140
Asp Gly Ser His Leu Ala Val Tyr Glu Thr Gly Val Gly Arg Ile Gly
145                 150                 155                 160
Ala Leu Cys Cys Trp Glu His Ile Gln Pro Leu Ser Lys Tyr Ala Met
                165                 170                 175
Tyr Ala Ala Asn Glu Gln Val His Val Ala Ser Trp Pro Cys Phe Ser
            180                 185                 190
Leu Tyr Arg Gly Met Ala Tyr Ala Leu Gly Pro Glu Val Asn Thr Ala
        195                 200                 205
Ala Ser Gln Val Tyr Ala Val Glu Gly Gly Cys Tyr Val Leu Ala Ser
    210                 215                 220
Cys Leu Val Val Thr Pro Glu Ile Leu Lys Val Leu Ile Asp Thr Pro
225                 230                 235                 240
Asp Lys Glu Pro Leu Leu Leu Ala Gly Gly Gly Phe Ser Met Ile Phe
                245                 250                 255
Gly Pro Asp Gly Arg Ala Leu Ala Gln Pro Leu Pro Glu Thr Glu Glu
            260                 265                 270
Gly Leu Val Thr Ala Glu Ile Asp Leu Gly Ala Ile Ala Leu Ala Lys
        275                 280                 285
Ala Ala Ala Asp Pro Ala Gly His Tyr Ala Arg Pro Asp Val Thr Arg
    290                 295                 300
Leu Leu Leu Asn Pro Arg Pro Ala Ala Arg Val Glu Ala Leu Gly Pro
305                 310                 315                 320
Arg Phe Glu Val Val Gln Ser Glu Gln Ala Glu Pro Pro Thr Gln Pro
                325                 330                 335
Ala Glu Ala Ala Asp
            340

<210> 35
<211> 942
<212> DNA
<213> Unknown

<220>
<223> Obtained from an environmental sample

<400> 35
atgacagctc taaaaattgc tgctgttcaa atgtgcgccg aattgggcgc tacagatcga      60
aacctgagtg cagctggatc attcgtgcgc gacgcatttc gcgaaggtgc ccagtgggta     120
atcctcccag agttttttac ctcggcaatg gcattcgcac cttcgatggc gcaagcttgg     180
ttgccactgg aaggaaaggc gctagcgatg atgcgcagcc ttgcgcgtca attcgatggg     240
gttgttggag gctcatatgt tgccagagag gggaacgact gcgtaaatgc ctttcttctc     300
gtctttccgg atggaagcta ctaccggcat gacaaagata ttccaacaat gtgggagaac     360
tgttactaca tcggcggcgt cgacgatggg gtgctggaaa caccaattgg tgcggtggga     420
gttgcactgt gttgggagtt catccgaaca caaaccgccc gaagactgaa ggatcgcgtt     480
caattagtgg ttggcggtac ttgctggtgg gattttccga tgcctgtacc tgaacgatat     540
ctgaggctga ccaggcatat ctccaggaac tttgagcgcg atgctccggc gcggttggcc     600
agtatgttgg gtgtgcctgt tgtacacgct tcccatgctg gggattttac tgctgtcacc     660
ccaggcaatg aaacgaagaa ttaccgatcc aactatctgg gagagaccca gatcgtcgat     720
gccaatggaa atgtgttgaa gcgaatgaca gtggctgatg gtgagggtta cgtcattgct     780
gacgttcaat tgggggccat atcaaccggt cgaacttcga tccccgacac cttctggacc     840
tgcaagctaa cgccaggggc acaacaggct tgggatgaac aaaatgcttt tgggtgtggc     900
tactatgaga acgtcacacg caaacaccta atcggtcgat ga                        942

<210> 36

<211> 313
<212> PRT
<213> Unknown

<220>
<223> Obtained from an environmental sample

<400> 36
Met Thr Ala Leu Lys Ile Ala Ala Val Gln Met Cys Ala Glu Leu Gly
1               5                   10                  15
Ala Thr Asp Arg Asn Leu Ser Ala Ala Gly Ser Phe Val Arg Asp Ala
            20                  25                  30
Phe Arg Glu Gly Ala Gln Trp Val Ile Leu Pro Glu Phe Phe Thr Ser
        35                  40                  45
Ala Met Ala Phe Ala Pro Ser Met Ala Gln Ala Trp Leu Pro Leu Glu
    50                  55                  60
Gly Lys Ala Leu Ala Met Met Arg Ser Leu Ala Arg Gln Phe Asp Gly
65                  70                  75                  80
Val Val Gly Gly Ser Tyr Val Ala Arg Glu Gly Asn Asp Cys Val Asn
                85                  90                  95
Ala Phe Leu Leu Val Phe Pro Asp Gly Ser Tyr Tyr Arg His Asp Lys
            100                 105                 110
Asp Ile Pro Thr Met Trp Glu Asn Cys Tyr Tyr Ile Gly Gly Val Asp
        115                 120                 125
Asp Gly Val Leu Glu Thr Pro Ile Gly Ala Val Gly Val Ala Leu Cys
    130                 135                 140
Trp Glu Phe Ile Arg Thr Gln Thr Ala Arg Arg Leu Lys Asp Arg Val
145                 150                 155                 160
Gln Leu Val Val Gly Gly Thr Cys Trp Trp Asp Phe Pro Met Pro Val
                165                 170                 175
Pro Glu Arg Tyr Leu Arg Leu Thr Arg His Ile Ser Arg Asn Phe Glu
            180                 185                 190
Arg Asp Ala Pro Ala Arg Leu Ala Ser Met Leu Gly Val Pro Val Val
        195                 200                 205
His Ala Ser His Ala Gly Asp Phe Thr Ala Val Thr Pro Gly Asn Glu
    210                 215                 220
Thr Lys Asn Tyr Arg Ser Asn Tyr Leu Gly Glu Thr Gln Ile Val Asp
225                 230                 235                 240
Ala Asn Gly Asn Val Leu Lys Arg Met Thr Val Ala Asp Gly Glu Gly
                245                 250                 255
Tyr Val Ile Ala Asp Val Gln Leu Gly Ala Ile Ser Thr Gly Arg Thr
            260                 265                 270
Ser Ile Pro Asp Thr Phe Trp Thr Cys Lys Leu Thr Pro Gly Ala Gln
        275                 280                 285
Gln Ala Trp Asp Glu Gln Asn Ala Phe Gly Cys Gly Tyr Tyr Glu Asn
    290                 295                 300
Val Thr Arg Lys His Leu Ile Gly Arg
305                 310

<210> 37
<211> 993
<212> DNA
<213> Unknown

<220>
<223> Obtained from an environmental sample

<400> 37
atggattcag atatgacgga tacttttaag gcagccatta ttcagcacgc gcctgttttt      60
ttaaatctcg aagagagtct ggacaaagcc ggcagcctta tagaaaaggc tgccgatcaa     120
ggcgcgaaag tgatcgcctt tcctgaaaca tggctgcccg gttatcccgt atggctcgac     180
tactctccaa aagcgggtct gtgggactat cagcctgcaa aatctctcta tcgtctgcta     240
gtcgataatt cagtcacctt acccggcaaa cacctcgatc aactcctctc catagcgcaa     300
aagaccggcg catatgttgt aatgggggca cacgaacgag tgggtggaac actctataac     360
acgacgatct atgttgggat tgatgggaag gagtacaaac ttcatagaaa gctggtgccg     420
acctataccg aaagattgat ctgggggcgg ggagacggca gcacattgag tgtgttgatg     480
acggattatg gcgttcttgg aggattgatc tgctgggagc actggatgcc tctggcaaga     540
gccgcaatgc atgccagata tgaaaccctt catgtggcgc aatggccggc tgtaaaagat     600

atccatcaga tagcaagcag acattatgct tttgaaggcc ggtgtttcgt gctcgcggca 660
ggctctgttc tgactcgaag agatataata gaaggattca actcactggc tcgcgccgat 720
agtgatgcat tggaacttct gaaagctatt tcgggagaag atagtgatct tattttgaat 780
gggggaagcg cgataattgc gccgaatgga gagtatcttg cgggcccggt ctttaatgaa 840
ccctccatta tttatgctga aattgatcct gcactgataa gtgagggcca tcttacactg 900
gatacaagcg gacactactc gcgccctgac atttttcgtc tggagataaa cgatcaacct 960
caacatgatg taactttcag atcggggcat tag 993

<210> 38
<211> 330
<212> PRT
<213> Unknown

<220>
<223> Obtained from an environmental sample

<400> 38
Met Asp Ser Asp Met Thr Asp Thr Phe Lys Ala Ala Ile Ile Gln His
1               5                   10                  15
Ala Pro Val Phe Leu Asn Leu Glu Glu Ser Leu Asp Lys Ala Gly Ser
            20                  25                  30
Leu Ile Glu Lys Ala Ala Asp Gln Gly Ala Lys Val Ile Ala Phe Pro
        35                  40                  45
Glu Thr Trp Leu Pro Gly Tyr Pro Val Trp Leu Asp Tyr Ser Pro Lys
    50                  55                  60
Ala Gly Leu Trp Asp Tyr Gln Pro Ala Lys Ser Leu Tyr Arg Leu Leu
65                  70                  75                  80
Val Asp Asn Ser Val Thr Leu Pro Gly Lys His Leu Asp Gln Leu Leu
                85                  90                  95
Ser Ile Ala Gln Lys Thr Gly Ala Tyr Val Val Met Gly Ala His Glu
            100                 105                 110
Arg Val Gly Gly Thr Leu Tyr Asn Thr Thr Ile Tyr Val Gly Ile Asp
        115                 120                 125
Gly Lys Glu Tyr Lys Leu His Arg Lys Leu Val Pro Thr Tyr Thr Glu
    130                 135                 140
Arg Leu Ile Trp Gly Arg Gly Asp Gly Ser Thr Leu Ser Val Leu Met
145                 150                 155                 160
Thr Asp Tyr Gly Val Leu Gly Gly Leu Ile Cys Trp Glu His Trp Met
                165                 170                 175
Pro Leu Ala Arg Ala Ala Met His Ala Arg Tyr Glu Thr Leu His Val
            180                 185                 190
Ala Gln Trp Pro Ala Val Lys Asp Ile His Gln Ile Ala Ser Arg His
        195                 200                 205
Tyr Ala Phe Glu Gly Arg Cys Phe Val Leu Ala Ala Gly Ser Val Leu
    210                 215                 220
Thr Arg Arg Asp Ile Ile Glu Gly Phe Asn Ser Leu Ala Arg Ala Asp
225                 230                 235                 240
Ser Asp Ala Leu Glu Leu Leu Lys Ala Ile Ser Gly Glu Asp Ser Asp
                245                 250                 255
Leu Ile Leu Asn Gly Gly Ser Ala Ile Ile Ala Pro Asn Gly Glu Tyr
            260                 265                 270
Leu Ala Gly Pro Val Phe Asn Glu Pro Ser Ile Ile Tyr Ala Glu Ile
        275                 280                 285
Asp Pro Ala Leu Ile Ser Glu Gly His Leu Thr Leu Asp Thr Ser Gly
    290                 295                 300
His Tyr Ser Arg Pro Asp Ile Phe Arg Leu Glu Ile Asn Asp Gln Pro
305                 310                 315                 320
Gln His Asp Val Thr Phe Arg Ser Gly His
                325                 330

<210> 39
<211> 1008
<212> DNA
<213> Unknown

<220>
<223> Obtained from an environmental sample

<400> 39
atgaaaaata tcaaaaactc agaaaaaagc agcacagtaa gagtcgctgc ggtacaaatc      60
agtccggtgt tgtacaaccg cgaagctacc gttcaaaaag tagtcaacaa aatccttgaa     120
ctaggaaaac aaggggtaca attcgccact tttccggaaa cgatagtgcc ttattatcct     180
tatttctctt ttattcaggc gccttatgcc atgggcaaag aacacctgcg cttgcttgaa     240
caatcagtta ctgttccgtc agccgcgacc gatgccataa gtgaggcggc aaaggaagcc     300
aatatggtag tgtctattgg tgtcaatgaa cgagacggtg gtaccattta caatacgcaa     360
ctcctttttg atgctgacgg aacattaatt cagcgcagac gtaaacttac accaacgtat     420
catgaaagaa tgatttgggg acaaggtgac gcttcaggtc ttcgtgccac agacagcgct     480
gttgggcgta tcgggcagtt ggcttgttgg gaacattaca atccattgtt ccgttatgct     540
ttgattgctg atggagaaca aatccattct gccatgtatc ccggatcatt tttaggtgcg     600
ttgcacggtg aacaaaccga aatcaatgta cgccaacacg ctttagaatc ggccagcttc     660
gtcgtagtgg ctaccggttg gttggatgcc gatcaacaag cacaaattgc gaaagacacc     720
ggtggaccaa tcggaccaat ttcgggaggt tgttttacag ccgttatagg ccctgacgga     780
caactaatcg gggaagccct tacatcaggt gaaggggaag tgattgccga tattgatttg     840
gcacaaattg atgcccgcaa aagattaatg gatgccagtg gtcactacaa ccgtcctgaa     900
ttgttgagct tgcatatcga tcacactccg actgctccta tgcatgaaag agtagtttac     960
actgagccgg gattagcaaa aagacaaaat gaaaattcat caaattaa                 1008

<210> 40
<211> 335
<212> PRT
<213> Unknown

<220>
<223> Obtained from an environmental sample

<400> 40
Met Lys Asn Ile Lys Asn Ser Glu Lys Ser Ser Thr Val Arg Val Ala
1               5                   10                  15
Ala Val Gln Ile Ser Pro Val Leu Tyr Asn Arg Glu Ala Thr Val Gln
            20                  25                  30
Lys Val Val Asn Lys Ile Leu Glu Leu Gly Lys Gln Gly Val Gln Phe
        35                  40                  45
Ala Thr Phe Pro Glu Thr Ile Val Pro Tyr Tyr Pro Tyr Phe Ser Phe
    50                  55                  60
Ile Gln Ala Pro Tyr Ala Met Gly Lys Glu His Leu Arg Leu Leu Glu
65                  70                  75                  80
Gln Ser Val Thr Val Pro Ser Ala Ala Thr Asp Ala Ile Ser Glu Ala
                85                  90                  95
Ala Lys Glu Ala Asn Met Val Val Ser Ile Gly Val Asn Glu Arg Asp
            100                 105                 110
Gly Gly Thr Ile Tyr Asn Thr Gln Leu Leu Phe Asp Ala Asp Gly Thr
        115                 120                 125
Leu Ile Gln Arg Arg Arg Lys Leu Thr Pro Thr Tyr His Glu Arg Met
    130                 135                 140
Ile Trp Gly Gln Gly Asp Ala Ser Gly Leu Arg Ala Thr Asp Ser Ala
145                 150                 155                 160
Val Gly Arg Ile Gly Gln Leu Ala Cys Trp Glu His Tyr Asn Pro Leu
                165                 170                 175
Phe Arg Tyr Ala Leu Ile Ala Asp Gly Glu Gln Ile His Ser Ala Met
            180                 185                 190
Tyr Pro Gly Ser Phe Leu Gly Ala Leu His Gly Glu Gln Thr Glu Ile
        195                 200                 205
Asn Val Arg Gln His Ala Leu Glu Ser Ala Ser Phe Val Val Val Ala
    210                 215                 220
Thr Gly Trp Leu Asp Ala Asp Gln Gln Ala Gln Ile Ala Lys Asp Thr
225                 230                 235                 240
Gly Gly Pro Ile Gly Pro Ile Ser Gly Gly Cys Phe Thr Ala Val Ile
                245                 250                 255
Gly Pro Asp Gly Gln Leu Ile Gly Glu Ala Leu Thr Ser Gly Glu Gly
            260                 265                 270
Glu Val Ile Ala Asp Ile Asp Leu Ala Gln Ile Asp Ala Arg Lys Arg
        275                 280                 285
Leu Met Asp Ala Ser Gly His Tyr Asn Arg Pro Glu Leu Leu Ser Leu
    290                 295                 300
His Ile Asp His Thr Pro Thr Ala Pro Met His Glu Arg Val Val Tyr
305                 310                 315                 320

Thr Glu Pro Gly Leu Ala Lys Arg Gln Asn Glu Asn Ser Ser Asn
                325                 330                 335

<210> 41
<211> 966
<212> DNA
<213> Unknown

<220>
<223> Obtained from an environmental sample

<400> 41
atgcccaatg agaataacat cgctacattc aaagttgccg cagtccaggc cacaccggtg      60
tttcttgatc gtgaagcaac catcgacaaa gcttgcgggt tgattgccac tgccggcaat     120
gaaggagcgc gcctgattgt gtttccagaa gcgttcatcc caacctatcc tgaatgggtt     180
tggggtattc cttccggtga gcaaggttta ctcaatgaac tctatgcaga gctgctcacc     240
aatgcggtca ctattcccag tgacgcgact gacaggctgt gcgaggccgc gcagcttgcg     300
aatgcctacg tagtgatggg aatgagcgaa cggaatgtcg aggcgagtgg cgcaagccta     360
tataatacgc tgttgtacat caatgcgcag ggggagattt tagggaaaca tcgaaagctg     420
gtgccaacgg gcggcgaacg cctggtatgg gcgcagggtg atggcagcac gctgcaggtc     480
tacgatacgc cattgggaaa actcggtggt ctcatttgct gggaaaatta tatgccgctg     540
gcacgctatg ctatgtatgc ctgggggaca caaatctatg tcgcggcaac gtgggatcga     600
ggccaaccct ggctttctac attacggcat atcgccaaag aaggcagggt atacgtgatt     660
ggttgctgta tcgcgatgcg aaaagacgat attccggata gttactccat gaagcagaaa     720
taccatgctg aaatggatga atggattaat gttggcgaca gtgtgattgt caatcccgaa     780
ggacacttta tcgcagggcc tgtgcgcaag caagaagaaa ttctctacgc ggagatcgat     840
ccacgtatgg tgcaaggccc gaagtggatg ctcgatgtgg cggggcatta tgcgagacca     900
gatgtgttcc agttgacggt gcatacgaat gtgagagaga tgatgcgggt ggaagatgat     960
tcataa                                                                966

<210> 42
<211> 321
<212> PRT
<213> Unknown

<220>
<223> Obtained from an environmental sample

<400> 42
Met Pro Asn Glu Asn Asn Ile Ala Thr Phe Lys Val Ala Ala Val Gln
1               5                   10                  15
Ala Thr Pro Val Phe Leu Asp Arg Glu Ala Thr Ile Asp Lys Ala Cys
            20                  25                  30
Gly Leu Ile Ala Thr Ala Gly Asn Glu Gly Ala Arg Leu Ile Val Phe
        35                  40                  45
Pro Glu Ala Phe Ile Pro Thr Tyr Pro Glu Trp Val Trp Gly Ile Pro
    50                  55                  60
Ser Gly Glu Gln Gly Leu Leu Asn Glu Leu Tyr Ala Glu Leu Leu Thr
65                  70                  75                  80
Asn Ala Val Thr Ile Pro Ser Asp Ala Thr Asp Arg Leu Cys Glu Ala
                85                  90                  95
Ala Gln Leu Ala Asn Ala Tyr Val Val Met Gly Met Ser Glu Arg Asn
            100                 105                 110
Val Glu Ala Ser Gly Ala Ser Leu Tyr Asn Thr Leu Leu Tyr Ile Asn
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Ala Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Asp Ser Tyr Ser Met Lys Gln Lys

225                 230                 235                 240
Tyr His Ala Glu Met Asp Glu Trp Ile Asn Val Gly Asp Ser Val Ile
                245                 250                 255
Val Asn Pro Glu Gly His Phe Ile Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asn Val Arg Glu Met Met Arg Val Glu Asp Asp
305                 310                 315                 320
Ser

<210> 43
<211> 993
<212> DNA
<213> Unknown

<220>
<223> Obtained from an environmental sample

<400> 43
atgagagtcg ttaaagccgc ggcggtccaa ctgaaacctg tcctctatag ccgagaggga      60
actgtcgaaa acgtcgtccg taaaatccac gagcttggac agcaaggggt acagttcgcg     120
acgtttccag agactgtggt gccttactac ccgtactttt cgatcgtgca gtccggctat     180
caaattcttg gcggtggtga gttcctgaag ctgcttgatc agtcagtaac cgtgccatct     240
ctcgctacgg aagcgatcgg cgaggcttgc aggcaggcgg gcgtcgttgt ctccatcggc     300
gtcaacgagc gtgatggagg aactctatac aacacgcaac ttctctttga tgccgacgga     360
acattgattc aaagacgacg caagatcaca cccacccatt acgagcgcat ggtctggggc     420
cagggcgatg gctcaggttt acgggccatt gacagcaagg tcgcgcgcat tggtcaactg     480
gcgtgttttg agcactacaa ccctctcgca cgttacgcga tgatggccga tggcgagcag     540
atccattctg cgatgttccc cggctccatg ttcggcgata atttttcaga gaaggtggaa     600
atcaacataa ggcagcatgc aatggagtct gggtgctttg tcgtttgcgc tactgcctgg     660
ttggatgctg accagcaggc tcaaatcatg aaagacacgg gatgtgagat cggaccgatc     720
tcaggaggtt gcttcacagc gatcgcggca ccagatggaa gccttatagg tgaacccatc     780
cgctcaggtg aaggcgtttg tattgccgac ctcgatttca aacttatcga caagcggaag     840
cacgtagtag acacacgcgg ccattatagc cggccagaat tgctcagcct cctgattgat     900
cggacgccga cggcccacat acacgaaagg accgagcaac cgagggcggc catcgagaaa     960
gagtcgcagg atgttttcac cgctgttgct taa                                  993

<210> 44
<211> 330
<212> PRT
<213> Unknown

<220>
<223> Obtained from an environmental sample

<400> 44
Met Arg Val Val Lys Ala Ala Ala Val Gln Leu Lys Pro Val Leu Tyr
1               5                   10                  15
Ser Arg Glu Gly Thr Val Glu Asn Val Val Arg Lys Ile His Glu Leu
            20                  25                  30
Gly Gln Gln Gly Val Gln Phe Ala Thr Phe Pro Glu Thr Val Val Pro
        35                  40                  45
Tyr Tyr Pro Tyr Phe Ser Ile Val Gln Ser Gly Tyr Gln Ile Leu Gly
    50                  55                  60
Gly Gly Glu Phe Leu Lys Leu Leu Asp Gln Ser Val Thr Val Pro Ser
65                  70                  75                  80
Leu Ala Thr Glu Ala Ile Gly Glu Ala Cys Arg Gln Ala Gly Val Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Tyr Glu Arg Met Val Trp Gly Gln Gly Asp Gly
    130                 135                 140

Ser Gly Leu Arg Ala Ile Asp Ser Lys Val Ala Arg Ile Gly Gln Leu
145                 150                 155                 160
Ala Cys Phe Glu His Tyr Asn Pro Leu Ala Arg Tyr Ala Met Met Ala
                165                 170                 175
Asp Gly Glu Gln Ile His Ser Ala Met Phe Pro Gly Ser Met Phe Gly
            180                 185                 190
Asp Asn Phe Ser Glu Lys Val Glu Ile Asn Ile Arg Gln His Ala Met
        195                 200                 205
Glu Ser Gly Cys Phe Val Val Cys Ala Thr Ala Trp Leu Asp Ala Asp
    210                 215                 220
Gln Gln Ala Gln Ile Met Lys Asp Thr Gly Cys Glu Ile Gly Pro Ile
225                 230                 235                 240
Ser Gly Gly Cys Phe Thr Ala Ile Ala Ala Pro Asp Gly Ser Leu Ile
                245                 250                 255
Gly Glu Pro Ile Arg Ser Gly Glu Gly Val Cys Ile Ala Asp Leu Asp
            260                 265                 270
Phe Lys Leu Ile Asp Lys Arg Lys His Val Val Asp Thr Arg Gly His
        275                 280                 285
Tyr Ser Arg Pro Glu Leu Leu Ser Leu Leu Ile Asp Arg Thr Pro Thr
    290                 295                 300
Ala His Ile His Glu Arg Thr Glu Gln Pro Arg Ala Ala Ile Glu Lys
305                 310                 315                 320
Glu Ser Gln Asp Val Phe Thr Ala Val Ala
                325                 330

<210> 45
<211> 996
<212> DNA
<213> Unknown

<220>
<223> Obtained from an environmental sample

<400> 45
gtgaaaccgc cgacgtcatt ccgcgtggcc gccgttcagg cctgtcccgt ctacctcgat      60
cgcgacctga cgatcggcaa ggcagaaggg ttgatcgccg aggcggctgg aaacggcgcg     120
aacctcatcg tgttccccga agcgttcgtg cctggctatc cggtgtgggt gtggttcatc     180
ccgcccggtc gcacggcgga tcttcgcgaa gcgtacagcg tcctccacgc caactcgatc     240
gcggtcccca gccagtcgac cgagcgtctg tgcgcggcgg cgcggcgcgc tggcgtcgcc     300
gtggcgattg gtgtcaacga aagaaacagc gaggccagcg gcggcagcct cttcaacacg     360
ctgctgtaca tcggaccgga cggcacgctg ctcggtaaac accgaaagct ggtgccaaca     420
ggcggagagc gtcttgtctg ggccagcggc gacggcagcg accttgccgt gttcacactg     480
cctttcgcgc gagtcggcgg actgatctgc tgggagaact acatgccgct cgcccgctac     540
gcgctggcgg cctggggtgc gcaaatccac gtggcgccga cctgggaccg cggcgagccg     600
tggctctcaa cactgcgtca tgtcgcgaag gaaggtagag ccgtgacgat cggctgctgt     660
caggccgtcc gcaaggaaga cattccggac gggctggcat tcaagtcccg atacctggcc     720
gacgtgggcg cctgggtcaa cccaggcggg agcgtcatcg tcgatcccga cggaaaaatt     780
cttgccggac ctgcgaacga aaccgaaggc atcttgtacg ctgacatcag ggccgatcag     840
ctcgtcgggc cgagatggca actcgacatt gccggacact acgcgcggcc ggacgtcttc     900
gagctgatcg tgcatcggcg ttcgacgccg atgattcgcg aggtctcggc gcctcgtcgt     960
cgcgcaagaa cgggaaagcg accgcgacgc cgctga                               996

<210> 46
<211> 331
<212> PRT
<213> Unknown

<220>
<223> Obtained from an environmental sample

<400> 46
Val Lys Pro Pro Thr Ser Phe Arg Val Ala Ala Val Gln Ala Cys Pro
1               5                   10                  15
Val Tyr Leu Asp Arg Asp Leu Thr Ile Gly Lys Ala Glu Gly Leu Ile
            20                  25                  30
Ala Glu Ala Ala Gly Asn Gly Ala Asn Leu Ile Val Phe Pro Glu Ala
        35                  40                  45
Phe Val Pro Gly Tyr Pro Val Trp Val Trp Phe Ile Pro Pro Gly Arg

    50                  55                  60
Thr Ala Asp Leu Arg Glu Ala Tyr Ser Val Leu His Ala Asn Ser Ile
65                  70                  75                  80
Ala Val Pro Ser Gln Ser Thr Glu Arg Leu Cys Ala Ala Ala Arg Arg
                85                  90                  95
Ala Gly Val Ala Val Ala Ile Gly Val Asn Glu Arg Asn Ser Glu Ala
            100                 105                 110
Ser Gly Gly Ser Leu Phe Asn Thr Leu Leu Tyr Ile Gly Pro Asp Gly
        115                 120                 125
Thr Leu Leu Gly Lys His Arg Lys Leu Val Pro Thr Gly Gly Glu Arg
    130                 135                 140
Leu Val Trp Ala Ser Gly Asp Gly Ser Asp Leu Ala Val Phe Thr Leu
145                 150                 155                 160
Pro Phe Ala Arg Val Gly Gly Leu Ile Cys Trp Glu Asn Tyr Met Pro
                165                 170                 175
Leu Ala Arg Tyr Ala Leu Ala Ala Trp Gly Ala Gln Ile His Val Ala
            180                 185                 190
Pro Thr Trp Asp Arg Gly Glu Pro Trp Leu Ser Thr Leu Arg His Val
        195                 200                 205
Ala Lys Glu Gly Arg Ala Val Thr Ile Gly Cys Cys Gln Ala Val Arg
    210                 215                 220
Lys Glu Asp Ile Pro Asp Gly Leu Ala Phe Lys Ser Arg Tyr Leu Ala
225                 230                 235                 240
Asp Val Gly Ala Trp Val Asn Pro Gly Gly Ser Val Ile Val Asp Pro
                245                 250                 255
Asp Gly Lys Ile Leu Ala Gly Pro Ala Asn Glu Thr Glu Gly Ile Leu
            260                 265                 270
Tyr Ala Asp Ile Arg Ala Asp Gln Leu Val Gly Pro Arg Trp Gln Leu
        275                 280                 285
Asp Ile Ala Gly His Tyr Ala Arg Pro Asp Val Phe Glu Leu Ile Val
    290                 295                 300
His Arg Arg Ser Thr Pro Met Ile Arg Glu Val Ser Ala Pro Arg Arg
305                 310                 315                 320
Arg Ala Arg Thr Gly Lys Arg Pro Arg Arg Arg
                325                 330

<210> 47
<211> 1014
<212> DNA
<213> Unknown

<220>
<223> Obtained from an environmental sample

<400> 47
gtgaaagaag caatcaaagt agcctgtgtg caagcagctc cagtctttct cgacctggac      60
gccacagtgg acaagaccgt cgccctgatt gaggaggcag cccgtaacgg cgcacgccta     120
atcgcctttc cagagacctg gattccaggc tacccatggt tcctttggct ggactcacca     180
gcctggggga tgcaattcgt gcgccgatac cacgagaact cactggtcct cgacagccct     240
caggccaagc gcatcagtga ggccgcccag cgcgccggta tatacgtcgc gctagggtac     300
agcgaacgcg tgagcggaac cctctacatg gggcagtggc tcattgacga taagggcgaa     360
acagctgggc tgcgccgaaa gctgaaacca acccatgtag agcgaaccct cttcggtgaa     420
ggcgacggat catccctttc cactttcgac acaccgttgg gggtgctggg cggactctgc     480
tgttgggaac acttacaacc tctttcgaaa tatgcgctct acgcacagaa cgaggaaata     540
cacttcgccg cctggcctag cttcagcatc taccgtcaag cgacagaagt ccttggacca     600
gaagtaaatg tcgcagcttc tcggatctac gccgtggaag ggcagtgttt tgttctcgct     660
tcctgcgcgc tcgtctcgcc agagatgatc gaaatgctct gcactgacga aagcaagcac     720
agccttcttc aggccggcgg cgggtactcc cgcattatcg gtcccgatgg cagcgaccta     780
gcgcgcccct tgggcgaaaa cgaggaaggt attctctatg ccactctgga ccctgccgct     840
cgaatctatg caaagaccgc agctgatcca gccgggcact actccagacc agacgtcact     900
cggctgctga tcaatcgcag tgccaatcag ccagtcgtag aggttggaag ggaaatacct     960
gcatcggccc aaggctttga agttgaggcg gcccccgggt acgaaggcga ttga          1014

<210> 48
<211> 337
<212> PRT
<213> Unknown

<220>
<223> Obtained from an environmental sample

<400> 48
Val Lys Glu Ala Ile Lys Val Ala Cys Val Gln Ala Ala Pro Val Phe
1               5                   10                  15
Leu Asp Leu Asp Ala Thr Val Asp Lys Thr Val Ala Leu Ile Glu Glu
            20                  25                  30
Ala Ala Arg Asn Gly Ala Arg Leu Ile Ala Phe Pro Glu Thr Trp Ile
        35                  40                  45
Pro Gly Tyr Pro Trp Phe Leu Trp Leu Asp Ser Pro Ala Trp Gly Met
    50                  55                  60
Gln Phe Val Arg Arg Tyr His Glu Asn Ser Leu Val Leu Asp Ser Pro
65                  70                  75                  80
Gln Ala Lys Arg Ile Ser Glu Ala Ala Gln Arg Ala Gly Ile Tyr Val
                85                  90                  95
Ala Leu Gly Tyr Ser Glu Arg Val Ser Gly Thr Leu Tyr Met Gly Gln
            100                 105                 110
Trp Leu Ile Asp Asp Lys Gly Glu Thr Ala Gly Leu Arg Arg Lys Leu
        115                 120                 125
Lys Pro Thr His Val Glu Arg Thr Leu Phe Gly Glu Gly Asp Gly Ser
    130                 135                 140
Ser Leu Ser Thr Phe Asp Thr Pro Leu Gly Val Leu Gly Gly Leu Cys
145                 150                 155                 160
Cys Trp Glu His Leu Gln Pro Leu Ser Lys Tyr Ala Leu Tyr Ala Gln
                165                 170                 175
Asn Glu Glu Ile His Phe Ala Ala Trp Pro Ser Phe Ser Ile Tyr Arg
            180                 185                 190
Gln Ala Thr Glu Val Leu Gly Pro Glu Val Asn Val Ala Ala Ser Arg
        195                 200                 205
Ile Tyr Ala Val Glu Gly Gln Cys Phe Val Leu Ala Ser Cys Ala Leu
    210                 215                 220
Val Ser Pro Glu Met Ile Glu Met Leu Cys Thr Asp Glu Ser Lys His
225                 230                 235                 240
Ser Leu Leu Gln Ala Gly Gly Gly Tyr Ser Arg Ile Ile Gly Pro Asp
                245                 250                 255
Gly Ser Asp Leu Ala Arg Pro Leu Gly Glu Asn Glu Glu Gly Ile Leu
            260                 265                 270
Tyr Ala Thr Leu Asp Pro Ala Ala Arg Ile Tyr Ala Lys Thr Ala Ala
        275                 280                 285
Asp Pro Ala Gly His Tyr Ser Arg Pro Asp Val Thr Arg Leu Leu Ile
    290                 295                 300
Asn Arg Ser Ala Asn Gln Pro Val Val Glu Val Gly Arg Glu Ile Pro
305                 310                 315                 320
Ala Ser Ala Gln Gly Phe Glu Val Glu Ala Ala Pro Gly Tyr Glu Gly
                325                 330                 335
Asp


<210> 49
<211> 1038
<212> DNA
<213> Unknown

<220>
<223> Obtained from an environmental sample

<400> 49
atgaacaaag tcgtggctgc tgccgttcag tgcagcccgg tgctttactc ttgcgccgga      60
actgtaaata aaatttgcga gtggattgca gatttgggca aacaaggggt tgagctggcg     120
gtgttcgcgg aaaccctggt gccttactac ccgtattttt cttttatcca ggctccttgt     180
gcgatgggcg cgcaacattt gttgttgatg caagaatcag tagaggttcc ttccatctac     240
acgcaacaaa ttgccgctgc agcaaaagca gcgaagatgg tggtgtcagt tggtattaac     300
gaacgcgacg gcggttctat ttataacgcg caattattat ttgatgcggg cggtcagctt     360
gttcagcacc gccgaaaaat tacgccgaca tttcatgagc gcatggtgtg ggggcagggc      420
gatggctccg gtttgtgcgc agtggatacg gcagttggtc gtgttggttc gctcgcttgc     480
tgggaacatt acaacccact cgcgcgttac gcattgatgg cagatcgcga acaaattcac     540
gtgagtatgt ttcccggttc tttggtcggc gaaatttttg ccgagcaaat tgaagcaact      600

attcgtcacc acgcattgga gtccggttgc tttgtggtaa atgcgacggg ctggttaacg 660
ccggaacagc aagctcaaat cgtaaaagat actggtggtc ctatcgctgc cattagcggt 720
ggttgtttca ccgccattgt ttcaccggaa ggaaaattgc tcggcacgcc attgcgcagt 780
gattccgggg agggtgcctg tatcgccgaa ctggatttta atctcatcaa taagcgtaag 840
cgcatgatgg attctgtcgg ccattacagt cgtcctgaat tgctcagttt gctgattgat 900
aaaacaccga caagtcatac acatccgctt aaaaaacctt tggctcccag tgaaaaaaat 960
acgccagagg atatcgccac tggtttaaca ctggtcactc ccgtttcaaa tgcaaacctt 1020
ttcagcgcaa gcaactag 1038

<210> 50
<211> 345
<212> PRT
<213> Unknown

<220>
<223> Obtained from an environmental sample

<400> 50
Met Asn Lys Val Val Ala Ala Ala Val Gln Cys Ser Pro Val Leu Tyr
1               5                   10                  15
Ser Cys Ala Gly Thr Val Asn Lys Ile Cys Glu Trp Ile Ala Asp Leu
            20                  25                  30
Gly Lys Gln Gly Val Glu Leu Ala Val Phe Ala Glu Thr Leu Val Pro
        35                  40                  45
Tyr Tyr Pro Tyr Phe Ser Phe Ile Gln Ala Pro Cys Ala Met Gly Ala
    50                  55                  60
Gln His Leu Leu Leu Met Gln Glu Ser Val Glu Val Pro Ser Ile Tyr
65                  70                  75                  80
Thr Gln Gln Ile Ala Ala Ala Ala Lys Ala Ala Lys Met Val Val Ser
                85                  90                  95
Val Gly Ile Asn Glu Arg Asp Gly Gly Ser Ile Tyr Asn Ala Gln Leu
            100                 105                 110
Leu Phe Asp Ala Gly Gly Gln Leu Val Gln His Arg Arg Lys Ile Thr
        115                 120                 125
Pro Thr Phe His Glu Arg Met Val Trp Gly Gln Gly Asp Gly Ser Gly
    130                 135                 140
Leu Cys Ala Val Asp Thr Ala Val Gly Arg Val Gly Ser Leu Ala Cys
145                 150                 155                 160
Trp Glu His Tyr Asn Pro Leu Ala Arg Tyr Ala Leu Met Ala Asp Arg
                165                 170                 175
Glu Gln Ile His Val Ser Met Phe Pro Gly Ser Leu Val Gly Glu Ile
            180                 185                 190
Phe Ala Glu Gln Ile Glu Ala Thr Ile Arg His His Ala Leu Glu Ser
        195                 200                 205
Gly Cys Phe Val Val Asn Ala Thr Gly Trp Leu Thr Pro Glu Gln Gln
    210                 215                 220
Ala Gln Ile Val Lys Asp Thr Gly Gly Pro Ile Ala Ala Ile Ser Gly
225                 230                 235                 240
Gly Cys Phe Thr Ala Ile Val Ser Pro Glu Gly Lys Leu Leu Gly Thr
                245                 250                 255
Pro Leu Arg Ser Asp Ser Gly Glu Gly Ala Cys Ile Ala Glu Leu Asp
            260                 265                 270
Phe Asn Leu Ile Asn Lys Arg Lys Arg Met Met Asp Ser Val Gly His
        275                 280                 285
Tyr Ser Arg Pro Glu Leu Leu Ser Leu Leu Ile Asp Lys Thr Pro Thr
    290                 295                 300
Ser His Thr His Pro Leu Lys Lys Pro Leu Ala Pro Ser Glu Lys Asn
305                 310                 315                 320
Thr Pro Glu Asp Ile Ala Thr Gly Leu Thr Leu Val Thr Pro Val Ser
                325                 330                 335
Asn Ala Asn Leu Phe Ser Ala Ser Asn
            340                 345

<210> 51
<211> 897
<212> DNA
<213> Unknown

<220>
<223> Obtained from an environmental sample

<400> 51
gtgaacgtcc gcgtcgcggt ggtgcaggcc acgccggccg tgctcgacgg gccggcgtcg 60
gtgcggaagg cctgccgcct gatcggcgag gccgcggccg gcggcgcccg cctgatcgcc 120
ctgcccgagg gcttcgtgcc catcatgccg cgctcctgct gggggcacca cttcgcgctg 180
atcgcctcgc cgaagtcggc ggccctgcac cggcgcatct gggagaacgc cgtcgacgtc 240
ggcggcccgc tggcccgcga gctcggcgac gccgcgcgcc gcgcggacgc ctgggtggcc 300
atcggggtga acgagcgcga cgcccgccgg ccgggcacgc tctggaacac gctgctctgg 360
ttcgcgcccg acgggagcct ggcccggcgc caccgcaagc tcgtgcccac catgcacgag 420
cgcacgttct gggggcaggg cgcgggcgac gacctcgagg cgctggccgc ggacttcggc 480
cgcctgggcg gcctgatctg ctgggagaac ttcatgcccg ccgcgcgccg gcgcctgcac 540
cgggacgggg tcgacttcta cctggccccc acggcggacg accgggacat ctgggtcgcc 600
gcgatgcgca cgttcgcctt cgaggccggc gccttcgtcc tctcgccggt gcagtacctg 660
cggaccgccg acttcccgga ggacttcccg ctgcgcgagg agctcgccga ctgccccgag 720
gtccagttca ccgggggcag cgtgatctgc gacccgtggg gcaacctcct ggcggggccg 780
gtccacgggg gcgaggagat cctctacgcc gactgcgatc tcgacctcgt cctcgaggcc 840
cgacgggtgc tcgacacggc cggccactac gaccgcccgg acctcgcctc ggcctga 897

<210> 52
<211> 298
<212> PRT
<213> Unknown

<220>
<223> Obtained from an environmental sample

<400> 52
Val Asn Val Arg Val Ala Val Val Gln Ala Thr Pro Ala Val Leu Asp
1               5                   10                  15
Gly Pro Ala Ser Val Arg Lys Ala Cys Arg Leu Ile Gly Glu Ala Ala
            20                  25                  30
Ala Gly Gly Ala Arg Leu Ile Ala Leu Pro Glu Gly Phe Val Pro Ile
        35                  40                  45
Met Pro Arg Ser Cys Trp Gly His His Phe Ala Leu Ile Ala Ser Pro
    50                  55                  60
Lys Ser Ala Ala Leu His Arg Arg Ile Trp Glu Asn Ala Val Asp Val
65                  70                  75                  80
Gly Gly Pro Leu Ala Arg Glu Leu Gly Asp Ala Ala Arg Arg Ala Asp
                85                  90                  95
Ala Trp Val Ala Ile Gly Val Asn Glu Arg Asp Ala Arg Arg Pro Gly
            100                 105                 110
Thr Leu Trp Asn Thr Leu Leu Trp Phe Ala Pro Asp Gly Ser Leu Ala
        115                 120                 125
Arg Arg His Arg Lys Leu Val Pro Thr Met His Glu Arg Thr Phe Trp
    130                 135                 140
Gly Gln Gly Ala Gly Asp Asp Leu Glu Ala Leu Ala Ala Asp Phe Gly
145                 150                 155                 160
Arg Leu Gly Gly Leu Ile Cys Trp Glu Asn Phe Met Pro Ala Ala Arg
                165                 170                 175
Arg Arg Leu His Arg Asp Gly Val Asp Phe Tyr Leu Ala Pro Thr Ala
            180                 185                 190
Asp Asp Arg Asp Ile Trp Val Ala Ala Met Arg Thr Phe Ala Phe Glu
        195                 200                 205
Ala Gly Ala Phe Val Leu Ser Pro Val Gln Tyr Leu Arg Thr Ala Asp
    210                 215                 220
Phe Pro Glu Asp Phe Pro Leu Arg Glu Glu Leu Ala Asp Cys Pro Glu
225                 230                 235                 240
Val Gln Phe Thr Gly Gly Ser Val Ile Cys Asp Pro Trp Gly Asn Leu
                245                 250                 255
Leu Ala Gly Pro Val His Gly Gly Glu Glu Ile Leu Tyr Ala Asp Cys
            260                 265                 270
Asp Leu Asp Leu Val Leu Glu Ala Arg Arg Val Leu Asp Thr Ala Gly
        275                 280                 285
His Tyr Asp Arg Pro Asp Leu Ala Ser Ala
    290                 295

<210> 53
<211> 954
<212> DNA
<213> Unknown

<220>
<223> Obtained from an environmental sample

<400> 53
atggtcaatt caaagagtca gattaaaatc gcggcggtgc aggctgcccc ggtctttctg      60
gaacgggagg cgacgattga caaagcttgc cggttgattg cggaggcagg cgagcagggc     120
gcgaatctgg tggtcttccc tgagtcattc gtcccggctt atccggattg ggtctgggcc     180
gttccggcag gtgaaacaac gctcctgaac acgctctatg ccgaactgct ggccaatgcc     240
gttgaaattc cgggtccggc gacagagcgg ctgagccagg cagccaacct ggccggggtt     300
tatgtcgcga ttggcttgac cgagcggaac atcgaggcca gtggggcgag cctgtacaat     360
actttgctct ttctcgactc agccggcggc atgttaggca agcatcgcaa actgatcccc     420
accggcggcg agcgcctggt ctgggctcag ggtgatggca gcactctggc ggtgtacgag     480
actaggtttg gaaaaatggg agggttgatt tgctgggaga attacatgcc cctggcccgt     540
tatgccttgt atgcctgggg gacgcagatt tacatcgcgg ccacctggga tcgaggcgag     600
ccgtggctgt caacgctgcg gcatatcgcc gcggaaggcc gggttgttgt cgtcggctgt     660
ggcatggccc tgcgcaaagc cgacctgccc gaccgctttg aactcaagca gcgattttac     720
cagaacgccg atgagtggat caatgtcggc gacagcgcga ttgttaaccc tgatggtgaa     780
ttcatcgccg ggccgctgcg cgagcaggaa ggcatcctct atgctgagat tgatctggcc     840
cagatgcgcg gccccaaatg gatgctcgac gtggccggcc attacgctcg cccggatgtg     900
tttgaactca tcgttcatcg ggaggcgcgg cccatgattg cgctaatttc atga           954

<210> 54
<211> 317
<212> PRT
<213> Unknown

<220>
<223> Obtained from an environmental sample

<400> 54
Met Val Asn Ser Lys Ser Gln Ile Lys Ile Ala Ala Val Gln Ala Ala
1               5                   10                  15
Pro Val Phe Leu Glu Arg Glu Ala Thr Ile Asp Lys Ala Cys Arg Leu
            20                  25                  30
Ile Ala Glu Ala Gly Glu Gln Gly Ala Asn Leu Val Val Phe Pro Glu
        35                  40                  45
Ser Phe Val Pro Ala Tyr Pro Asp Trp Val Trp Ala Val Pro Ala Gly
    50                  55                  60
Glu Thr Thr Leu Leu Asn Thr Leu Tyr Ala Glu Leu Leu Ala Asn Ala
65                  70                  75                  80
Val Glu Ile Pro Gly Pro Ala Thr Glu Arg Leu Ser Gln Ala Ala Asn
                85                  90                  95
Leu Ala Gly Val Tyr Val Ala Ile Gly Leu Thr Glu Arg Asn Ile Glu
            100                 105                 110
Ala Ser Gly Ala Ser Leu Tyr Asn Thr Leu Leu Phe Leu Asp Ser Ala
        115                 120                 125
Gly Gly Met Leu Gly Lys His Arg Lys Leu Ile Pro Thr Gly Gly Glu
    130                 135                 140
Arg Leu Val Trp Ala Gln Gly Asp Gly Ser Thr Leu Ala Val Tyr Glu
145                 150                 155                 160
Thr Arg Phe Gly Lys Met Gly Gly Leu Ile Cys Trp Glu Asn Tyr Met
                165                 170                 175
Pro Leu Ala Arg Tyr Ala Leu Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Ala Glu Gly Arg Val Val Val Val Gly Cys Gly Met Ala Leu
    210                 215                 220
Arg Lys Ala Asp Leu Pro Asp Arg Phe Glu Leu Lys Gln Arg Phe Tyr
225                 230                 235                 240
Gln Asn Ala Asp Glu Trp Ile Asn Val Gly Asp Ser Ala Ile Val Asn
                245                 250                 255
Pro Asp Gly Glu Phe Ile Ala Gly Pro Leu Arg Glu Gln Glu Gly Ile

260                 265                 270
Leu Tyr Ala Glu Ile Asp Leu Ala Gln Met Arg Gly Pro Lys Trp Met
        275                 280                 285
Leu Asp Val Ala Gly His Tyr Ala Arg Pro Asp Val Phe Glu Leu Ile
    290                 295                 300
Val His Arg Glu Ala Arg Pro Met Ile Ala Leu Ile Ser
305                 310                 315

<210> 55
<211> 1017
<212> DNA
<213> Unknown

<220>
<223> Obtained from an environmental sample

<400> 55
atgggtatcg aacatccgaa gtacaaggtc gccgtggtgc aggcagctcc cgcctggctc      60
gatctcgacg cgtcgatcga caagtcgatc gcgctgatcg aggaggcggc ccaaaaaggc     120
gccaagctga tcgcattccc cgaggccttc attcccggct atccctggca catctggatg     180
gactcgccgg cctgggcgat cggccgcggc tttgtgcagc gctattttga caattcgctc     240
gcctatgaca gcccgcaggc cgagaagctg cgcgcggcgg tgcgcaaggc aaagctcacc     300
gccgtgctcg ggctgtccga gcgcgacggc ggcagtctct atctggcgca atggttgatc     360
gggcccgatg gcgagaccat cgcaaaacgc cgcaagctgc ggccgacaca tgccgagcgc     420
acggtgtacg gcgagggcga cggcagcgat ctcgcagtcc acaaccgtcc cgatatcggc     480
cgcctcggcg cgctctgctg ctgggagcat ttgcagccac tgtcgaaata cgcgatgtac     540
gcgcagaacg agcaggtgca tgtcgcggcc tggccgagct tttcgctcta cgatcccttt      600
gcggtggcgc tcggcgccga ggtgaacaac gcggcctcgc gcgtctatgc agtcgaaggc     660
tcctgcttcg tgctggcgcc atgcgccacc gtctcgcagg ccatgatcga cgagctctgc     720
gaccgaccgg acaagcatac gctgctgcat gtcggcggcg gttttgccgc gatctatggt     780
cctgacggca gccagatcgg cgacaagctc gcgcccgacc aggaagggct gttgatcgcg     840
gagatcgacc ttggggccat tggcgtcgcc aagaacgcgg ccgatcccgc cgggcattat     900
tcgcggcccg acgtgacgcg gctcctgctc aacaagaaac cgtacaagcg cgtcgagcag     960
ttctcgccac cggccgaggc ggtcgagccc acagatatcg cagcggcggc aagctga       1017

<210> 56
<211> 338
<212> PRT
<213> Unknown

<220>
<223> Obtained from an environmental sample

<400> 56
Met Gly Ile Glu His Pro Lys Tyr Lys Val Ala Val Val Gln Ala Ala
1               5                   10                  15
Pro Ala Trp Leu Asp Leu Asp Ala Ser Ile Asp Lys Ser Ile Ala Leu
            20                  25                  30
Ile Glu Glu Ala Ala Gln Lys Gly Ala Lys Leu Ile Ala Phe Pro Glu
        35                  40                  45
Ala Phe Ile Pro Gly Tyr Pro Trp His Ile Trp Met Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Lys Leu Arg Ala Ala Val Arg Lys
                85                  90                  95
Ala Lys Leu Thr Ala Val Leu Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asn Arg Pro Asp Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190

Ser Phe Ser Leu Tyr Asp Pro Phe Ala Val Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Thr Leu Leu His Val Gly Gly Gly Phe Ala
                245                 250                 255
Ala Ile Tyr Gly Pro Asp Gly Ser Gln Ile Gly Asp Lys Leu Ala Pro
            260                 265                 270
Asp Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Lys Pro Tyr Lys Arg Val Glu Gln
305                 310                 315                 320
Phe Ser Pro Pro Ala Glu Ala Val Glu Pro Thr Asp Ile Ala Ala Ala
                325                 330                 335
Ala Ser

<210> 57
<211> 1014
<212> DNA
<213> Unknown

<220>
<223> Obtained from an environmental sample

<400> 57
gtgaaagaag caatcaaagt agcctgtgtg caagcagctc cagtctttct cgacctggac      60
gccacagtgg acaagaccgt cgccctgatt gaggaggcag cccgtaacgg cgcacgccta     120
atcgcctttc cagagacctg gattccaggc tacccatggt tcctttggct ggactcacca     180
gcctggggga tgcaattcgt gcgccgatac cacgagaact cactggtcct cgacagccct     240
caggccaagc gcatcagtga ggccgcccag cgcgccggta tatacgtcgc gctagggtac     300
agcgaacgcg tgagcggaac cctctacatg gggcagtggc tcattgacga taagggcgaa     360
acagctgggc tgcgccgaaa gctgaaacca acccatgtag agcgaaccct cttcggtgaa     420
ggcgacggat catccctttc cactttcgac acaccgttgg gggtgctggg cggactctgc     480
tgttgggaac acttacaacc tctttcgaaa tatgcgctct acgcacagaa cgaggaaata     540
cacttcgccg cctggcctag cttcagcatc taccgtcaag cgacagaagt ccttggacca     600
gaagtaaatg tcgcagcttc tcggatctac gccgtggaag ggcagtgttt tgttctcgct     660
tcctgcgcgc tcgtctcgcc agagatgatc gaaatgctct gcactgacga aagcaagcac     720
agccttcttc aggccggcgg cgggtactcc cgcattatcg gtcccgatgg cagcgaccta     780
gcgcgcccct tgggcgaaaa cgaggaaggt attctctatg ccactctgga ccctgccgct     840
cgaatctatg caaagaccgc agctgatcca gccgggcact actccagacc agacgtcact     900
cggctgctga tcaatcgcag tgccaatcag ccagtcgtag aggttggacg ggaaatacct     960
gcatcggccc aaggctttga agttgaggcg gcccccgggt acggaggcga ttga          1014

<210> 58
<211> 337
<212> PRT
<213> Unknown

<220>
<223> Obtained from an environmental sample

<400> 58
Val Lys Glu Ala Ile Lys Val Ala Cys Val Gln Ala Ala Pro Val Phe
 1               5                  10                  15
Leu Asp Leu Asp Ala Thr Val Asp Lys Thr Val Ala Leu Ile Glu Glu
            20                  25                  30
Ala Ala Arg Asn Gly Ala Arg Leu Ile Ala Phe Pro Glu Thr Trp Ile
        35                  40                  45
Pro Gly Tyr Pro Trp Phe Leu Trp Leu Asp Ser Pro Ala Trp Gly Met
    50                  55                  60
Gln Phe Val Arg Arg Tyr His Glu Asn Ser Leu Val Leu Asp Ser Pro
65                  70                  75                  80
Gln Ala Lys Arg Ile Ser Glu Ala Ala Gln Arg Ala Gly Ile Tyr Val

85                  90                  95
Ala Leu Gly Tyr Ser Glu Arg Val Ser Gly Thr Leu Tyr Met Gly Gln
            100                 105                 110
Trp Leu Ile Asp Asp Lys Gly Glu Thr Ala Gly Leu Arg Arg Lys Leu
        115                 120                 125
Lys Pro Thr His Val Glu Arg Thr Leu Phe Gly Glu Gly Asp Gly Ser
    130                 135                 140
Ser Leu Ser Thr Phe Asp Thr Pro Leu Gly Val Leu Gly Gly Leu Cys
145                 150                 155                 160
Cys Trp Glu His Leu Gln Pro Leu Ser Lys Tyr Ala Leu Tyr Ala Gln
                165                 170                 175
Asn Glu Glu Ile His Phe Ala Ala Trp Pro Ser Phe Ser Ile Tyr Arg
            180                 185                 190
Gln Ala Thr Glu Val Leu Gly Pro Glu Val Asn Val Ala Ala Ser Arg
        195                 200                 205
Ile Tyr Ala Val Glu Gly Gln Cys Phe Val Leu Ala Ser Cys Ala Leu
    210                 215                 220
Val Ser Pro Glu Met Ile Glu Met Leu Cys Thr Asp Glu Ser Lys His
225                 230                 235                 240
Ser Leu Leu Gln Ala Gly Gly Gly Tyr Ser Arg Ile Ile Gly Pro Asp
                245                 250                 255
Gly Ser Asp Leu Ala Arg Pro Leu Gly Glu Asn Glu Glu Gly Ile Leu
            260                 265                 270
Tyr Ala Thr Leu Asp Pro Ala Ala Arg Ile Tyr Ala Lys Thr Ala Ala
        275                 280                 285
Asp Pro Ala Gly His Tyr Ser Arg Pro Asp Val Thr Arg Leu Leu Ile
    290                 295                 300
Asn Arg Ser Ala Asn Gln Pro Val Val Glu Val Gly Arg Glu Ile Pro
305                 310                 315                 320
Ala Ser Ala Gln Gly Phe Glu Val Glu Ala Ala Pro Gly Tyr Gly Gly
                325                 330                 335
Asp


<210> 59
<211> 987
<212> DNA
<213> Unknown

<220>
<223> Obtained from an environmental sample

<400> 59
atgcgagata ggaatttcaa actggcggcc attcaggcgg agccggtttt ctttaatcgc      60
cgggcctcga cggaaaaggc ctgcagattg atcaaagaag cgggcgcgat gggcgccgat     120
atcgcgggat tcagcgagac ctggcttccc gggtatccct tttttatctg gggcgcaagc      180
gccgatccat ccctgctctg gaaggcttct gcggaatacc tggccaatgc cgttcaaata     240
cccggtcccg agacggatca attatgcgag gcggcgaaaa aggccggcat cgatgtggcg     300
atcggagtgg ttgaactcga cgagtttacg aagggaacgg cttactgcac gctgctcttc     360
atcggcaaag aagggaagat cctgggaaag caccgcaaac tcaagccgac gcaccgggag     420
cgcacggtat ggggagaggg cgatgcgacg ggactcagtg tccatgagcg tccttacggg     480
cggatcagcg gcctgaactg ctgggagcat aatatggtcc tgcccggcta tgtcctgatg     540
tctcagggca cgcacattca tatcgcggcc tggccgggtt cggaagggaa agcacctccc     600
gcgccgtctc cgatgtggga gcgccagctt ctgctctccc gcgctttcgc ttcgcaatcc     660
gccgcatacg tgattctggt cggaggactc ctgaacccgc agaatattcc ggcgccctac     720
gatgaacttg ccgtcaagta ccggggagac agtttcatca tcgatccgcg cggggagatc     780
atcgccgggc cggccaaggg ggaaaccatt ctcatcgccg aaggctcgat ggaacaggtc     840
ctcgcggcaa agtccgcctt cgatgtcgcg ggacattatt cccgccccga cgtctttcaa     900
ctctgcgtca accgcaaacc gtaccggcgt gtaagggaaa cttcggagca ggaccaaccc     960
gcttctgaaa gagaatcgga atcgtaa                                         987

<210> 60
<211> 328
<212> PRT
<213> Unknown

<220>
<223> Obtained from an environmental sample

<400> 60
Met Arg Asp Arg Asn Phe Lys Leu Ala Ala Ile Gln Ala Glu Pro Val
1               5                   10                  15
Phe Phe Asn Arg Arg Ala Ser Thr Glu Lys Ala Cys Arg Leu Ile Lys
            20                  25                  30
Glu Ala Gly Ala Met Gly Ala Asp Ile Ala Gly Phe Ser Glu Thr Trp
        35                  40                  45
Leu Pro Gly Tyr Pro Phe Phe Ile Trp Gly Ala Ser Ala Asp Pro Ser
    50                  55                  60
Leu Leu Trp Lys Ala Ser Ala Glu Tyr Leu Ala Asn Ala Val Gln Ile
65                  70                  75                  80
Pro Gly Pro Glu Thr Asp Gln Leu Cys Glu Ala Ala Lys Lys Ala Gly
                85                  90                  95
Ile Asp Val Ala Ile Gly Val Val Glu Leu Asp Glu Phe Thr Lys Gly
            100                 105                 110
Thr Ala Tyr Cys Thr Leu Leu Phe Ile Gly Lys Glu Gly Lys Ile Leu
        115                 120                 125
Gly Lys His Arg Lys Leu Lys Pro Thr His Arg Glu Arg Thr Val Trp
    130                 135                 140
Gly Glu Gly Asp Ala Thr Gly Leu Ser Val His Glu Arg Pro Tyr Gly
145                 150                 155                 160
Arg Ile Ser Gly Leu Asn Cys Trp Glu His Asn Met Val Leu Pro Gly
                165                 170                 175
Tyr Val Leu Met Ser Gln Gly Thr His Ile His Ile Ala Ala Trp Pro
            180                 185                 190
Gly Ser Glu Gly Lys Ala Pro Pro Ala Pro Ser Pro Met Trp Glu Arg
        195                 200                 205
Gln Leu Leu Leu Ser Arg Ala Phe Ala Ser Gln Ser Ala Ala Tyr Val
    210                 215                 220
Ile Leu Val Gly Gly Leu Leu Asn Pro Gln Asn Ile Pro Ala Pro Tyr
225                 230                 235                 240
Asp Glu Leu Ala Val Lys Tyr Arg Gly Asp Ser Phe Ile Ile Asp Pro
                245                 250                 255
Arg Gly Glu Ile Ile Ala Gly Pro Ala Lys Gly Glu Thr Ile Leu Ile
            260                 265                 270
Ala Glu Gly Ser Met Glu Gln Val Leu Ala Ala Lys Ser Ala Phe Asp
        275                 280                 285
Val Ala Gly His Tyr Ser Arg Pro Asp Val Phe Gln Leu Cys Val Asn
    290                 295                 300
Arg Lys Pro Tyr Arg Arg Val Arg Glu Thr Ser Glu Gln Asp Gln Pro
305                 310                 315                 320
Ala Ser Glu Arg Glu Ser Glu Ser
                325

<210> 61
<211> 966
<212> DNA
<213> Unknown

<220>
<223> Obtained from an environmental sample

<400> 61
atgactcgat cttacccgaa tgacacactc acggttgggc ttgcgcaaat tgctccagtc      60
tggttggatc gtacagggac aatttcaaag atattagctc aagtccatgc ggcaaatgaa     120
gcgggctgtc atcttgtcgc gtttggcgaa ggtctcctcc ccggatatcc gttttggatt     180
gagcgaacaa atggtgcagt cttcaactcg cccacgcaga aagaaattca cgcgcattat     240
ctggatcagg ctgtccagat cgaagcaggt catcttgagg cgctttgcga agcagccaag     300
gaatatgaga tcgcaattgt cctgggatgc attgaacgtc cgcaagatcg tggagggcac     360
agtctgtatg caagccttgt atatattgat tcagacggca tcatccaatc tgtgcatcga     420
aagttaatgc caacatatga agaacggctc acctggtcgc caggtgacgg acatggatta     480
cgggtgcaca aattaggtgc ctttacggtt ggcggcctca actgttggga aaactggatg     540
cctttggcac gcgcggccat gtatggtcaa ggcgaggatt tgcatattgc catttggccc     600
ggcggctccc acaatacgca agacattaca cgctttattg cactagaatc gcgttcctat     660
gttttatctg tgtcaggttt aatgcgctca ggcgatttcc caaaagagac cccacatctt     720
gcatccatcc tggctaaagg tgaggatatt cttgccaacg gtggttcatg tatcgccggt     780
cctgacggca aatggatcgt tgagccgctt gtaggagaag agaagttaat tgttgcaacg     840

attgatcatt gtcgtgtgcg cgaagagcgt caaaattttg atccttcagg acattacagc 900
aggccagatg tattgcaact gaaaataaac aggcaacgcc agagtacaat ctcgtttgga 960
gagtaa 966

<210> 62
<211> 321
<212> PRT
<213> Unknown

<220>
<223> Obtained from an environmental sample

<400> 62
Met Thr Arg Ser Tyr Pro Asn Asp Thr Leu Thr Val Gly Leu Ala Gln
1               5                   10                  15
Ile Ala Pro Val Trp Leu Asp Arg Thr Gly Thr Ile Ser Lys Ile Leu
            20                  25                  30
Ala Gln Val His Ala Ala Asn Glu Ala Gly Cys His Leu Val Ala Phe
        35                  40                  45
Gly Glu Gly Leu Leu Pro Gly Tyr Pro Phe Trp Ile Glu Arg Thr Asn
    50                  55                  60
Gly Ala Val Phe Asn Ser Pro Thr Gln Lys Glu Ile His Ala His Tyr
65                  70                  75                  80
Leu Asp Gln Ala Val Gln Ile Glu Ala Gly His Leu Glu Ala Leu Cys
                85                  90                  95
Glu Ala Ala Lys Glu Tyr Glu Ile Ala Ile Val Leu Gly Cys Ile Glu
            100                 105                 110
Arg Pro Gln Asp Arg Gly Gly His Ser Leu Tyr Ala Ser Leu Val Tyr
        115                 120                 125
Ile Asp Ser Asp Gly Ile Ile Gln Ser Val His Arg Lys Leu Met Pro
    130                 135                 140
Thr Tyr Glu Glu Arg Leu Thr Trp Ser Pro Gly Asp Gly His Gly Leu
145                 150                 155                 160
Arg Val His Lys Leu Gly Ala Phe Thr Val Gly Gly Leu Asn Cys Trp
                165                 170                 175
Glu Asn Trp Met Pro Leu Ala Arg Ala Ala Met Tyr Gly Gln Gly Glu
            180                 185                 190
Asp Leu His Ile Ala Ile Trp Pro Gly Gly Ser His Asn Thr Gln Asp
        195                 200                 205
Ile Thr Arg Phe Ile Ala Leu Glu Ser Arg Ser Tyr Val Leu Ser Val
    210                 215                 220
Ser Gly Leu Met Arg Ser Gly Asp Phe Pro Lys Glu Thr Pro His Leu
225                 230                 235                 240
Ala Ser Ile Leu Ala Lys Gly Glu Asp Ile Leu Ala Asn Gly Gly Ser
                245                 250                 255
Cys Ile Ala Gly Pro Asp Gly Lys Trp Ile Val Glu Pro Leu Val Gly
            260                 265                 270
Glu Glu Lys Leu Ile Val Ala Thr Ile Asp His Cys Arg Val Arg Glu
        275                 280                 285
Glu Arg Gln Asn Phe Asp Pro Ser Gly His Tyr Ser Arg Pro Asp Val
    290                 295                 300
Leu Gln Leu Lys Ile Asn Arg Gln Arg Gln Ser Thr Ile Ser Phe Gly
305                 310                 315                 320
Glu

<210> 63
<211> 978
<212> DNA
<213> Unknown

<220>
<223> Obtained from an environmental sample

<400> 63
atgcaagata gagtaccgat tgtacgagct gcggctatcc aggctgaacc catagtcctt 60
gattgtgacg cgaccgtgga aaaagcctgc cgattgatcg gtgaagcagc agaaaatggt 120
gcaaacctga tcgtgtttcc cgaagccttc attcccgttt atcccaatgc ggcgatctgg 180

ggtcgaggtc tggccacttt tggcggacag cgccagaaat acgtatggac gcgactatgg 240
aacaattcgg tggaaatccc tggtccggcc accgacaggc tggcaaaggc agcacacgag 300
gctcgagcca ccgttgtcat gggattgaat gagcgcgcgg tcgataacaa cacgctttac 360
aacaccctgc tatttattgg gccagacggt cgcttgctgg gcaagcaccg taagctcatg 420
cccaccaatc acgaacggat gatctggggt atgggagatg ggagcaccct gcgggttttt 480
gatacaccct gtggaaaagt aggcggtctc atctgctggg aaaactacat gcctctggcg 540
cgttatgcac tctatggaca gggcgaacaa atccatgtcg cgccgactgc gcacgatggt 600
gagatcactc tggtcaatgc acgcaatacc gcctatgagg gacgcttatt cgtcatctcc 660
gtgtgcatga tccttcgcaa gtccagcttt ccccatgatt ttgagctggg cgaggaattg 720
gcggaggcag atgacttcat aaaatcaggc ggcagcgcga tcgttgggcc agatggcgag 780
gtgctggcgg gtccattgtg gaatgaagag aatatactgt atgccgatct tgacttgaat 840
cgaattgtgg atgagagacg agtatttgat gtgacgggcc attattcacg tccagatgtt 900
ctacgactgc actttaatgc ttcccctcag aaaactattg aaagatatga gcaacctctc 960
gatccgtctg agggttaa 978

<210> 64
<211> 325
<212> PRT
<213> Unknown

<220>
<223> Obtained from an environmental sample

<400> 64
Met Gln Asp Arg Val Pro Ile Val Arg Ala Ala Ala Ile Gln Ala Glu
1               5                   10                  15
Pro Ile Val Leu Asp Cys Asp Ala Thr Val Glu Lys Ala Cys Arg Leu
            20                  25                  30
Ile Gly Glu Ala Ala Glu Asn Gly Ala Asn Leu Ile Val Phe Pro Glu
        35                  40                  45
Ala Phe Ile Pro Val Tyr Pro Asn Ala Ala Ile Trp Gly Arg Gly Leu
    50                  55                  60
Ala Thr Phe Gly Gly Gln Arg Gln Lys Tyr Val Trp Thr Arg Leu Trp
65                  70                  75                  80
Asn Asn Ser Val Glu Ile Pro Gly Pro Ala Thr Asp Arg Leu Ala Lys
                85                  90                  95
Ala Ala His Glu Ala Arg Ala Thr Val Val Met Gly Leu Asn Glu Arg
            100                 105                 110
Ala Val Asp Asn Asn Thr Leu Tyr Asn Thr Leu Leu Phe Ile Gly Pro
        115                 120                 125
Asp Gly Arg Leu Leu Gly Lys His Arg Lys Leu Met Pro Thr Asn His
    130                 135                 140
Glu Arg Met Ile Trp Gly Met Gly Asp Gly Ser Thr Leu Arg Val Phe
145                 150                 155                 160
Asp Thr Pro Cys Gly Lys Val Gly Gly Leu Ile Cys Trp Glu Asn Tyr
                165                 170                 175
Met Pro Leu Ala Arg Tyr Ala Leu Tyr Gly Gln Gly Glu Gln Ile His
            180                 185                 190
Val Ala Pro Thr Ala His Asp Gly Glu Ile Thr Leu Val Asn Ala Arg
        195                 200                 205
Asn Thr Ala Tyr Glu Gly Arg Leu Phe Val Ile Ser Val Cys Met Ile
    210                 215                 220
Leu Arg Lys Ser Ser Phe Pro His Asp Phe Glu Leu Gly Glu Glu Leu
225                 230                 235                 240
Ala Glu Ala Asp Asp Phe Ile Lys Ser Gly Gly Ser Ala Ile Val Gly
                245                 250                 255
Pro Asp Gly Glu Val Leu Ala Gly Pro Leu Trp Asn Glu Glu Asn Ile
            260                 265                 270
Leu Tyr Ala Asp Leu Asp Leu Asn Arg Ile Val Asp Glu Arg Arg Val
        275                 280                 285
Phe Asp Val Thr Gly His Tyr Ser Arg Pro Asp Val Leu Arg Leu His
    290                 295                 300
Phe Asn Ala Ser Pro Gln Lys Thr Ile Glu Arg Tyr Glu Gln Pro Leu
305                 310                 315                 320
Asp Pro Ser Glu Gly
                325

<210> 65

<211> 1002
<212> DNA
<213> Unknown

<220>
<223> Obtained from an environmental sample

<400> 65
atgccgaccc ccacttcaaa attcaaaatc ggcgccgtgc aggcatcgcc ggtttttctg      60
gaccgggaag ccactgcgca aaaagcctgc aaattgattg ccgaagcggg agggcagggc     120
gcgcggctga tcgttttccc ggagtctttc attcccacct atcctgattg ggtctgggcg     180
gtcccgccgg gaagaggaaa agtgttaagc gaactttacg ccgagctgct ggccaatgcc     240
gtggaagtcc ccgggccggt caccgatcag ctgggtgaag cagcccaaaa aacgggcgcc     300
tatgtcgtca tgggcgtcac ggaaaaggac accgacgcaa gcggcgcgag cctttacaac     360
acgctcctct atttcaaccc cgcgggggac ctcctgggaa aacaccggaa gcttgttcct     420
accggcgggg agcggctggt ctgggcgcag ggcgacggca gcaccctgga agtgtacgac     480
actcccctgg gaaaaatcgg aggcctcatc tgctgggaaa actacatgcc cctcgcccgg     540
tacacgatgt atgcctgggg gacccagatt tatatcgcgg ccacatggga ccagggggag     600
acgtggcttg ccaccctgcg gcatatcgct aaggaaggac gggtgtacgt catcggctgc     660
tgcatcgcgc tgcggcggga cgacatcccc gaccggctgg aatacaagaa gaagttctac     720
tcggggtcgc gggaatggat caatatgggg gacagcgcca tcgtgaaccc ggaaggcgaa     780
ttcattgccg gccccgtgcg gatgaaggag gagatcctgt atgccgaggt ggaccccctc     840
ctgatggcgg gatcgaaatg gatgctcgac gtcgcggggc attacgcgcg ccccgacgtc     900
tttgaactca tcgtccaccg ccagccccac ccgatgatcc gggtaatcga gaaagaggga     960
ggggccggaa gaaccgggga cgagaagaag gaaaatgagt ga                       1002

<210> 66
<211> 333
<212> PRT
<213> Unknown

<220>
<223> Obtained from an environmental sample

<400> 66
Met Pro Thr Pro Thr Ser Lys Phe Lys Ile Gly Ala Val Gln Ala Ser
1               5                   10                  15
Pro Val Phe Leu Asp Arg Glu Ala Thr Ala Gln Lys Ala Cys Lys Leu
            20                  25                  30
Ile Ala Glu Ala Gly Gly Gln Gly Ala Arg Leu Ile Val Phe Pro Glu
        35                  40                  45
Ser Phe Ile Pro Thr Tyr Pro Asp Trp Val Trp Ala Val Pro Pro Gly
    50                  55                  60
Arg Gly Lys Val Leu Ser Glu Leu Tyr Ala Glu Leu Leu Ala Asn Ala
65                  70                  75                  80
Val Glu Val Pro Gly Pro Val Thr Asp Gln Leu Gly Glu Ala Ala Gln
                85                  90                  95
Lys Thr Gly Ala Tyr Val Val Met Gly Val Thr Glu Lys Asp Thr Asp
            100                 105                 110
Ala Ser Gly Ala Ser Leu Tyr Asn Thr Leu Leu Tyr Phe Asn Pro Ala
        115                 120                 125
Gly Asp Leu Leu Gly Lys His Arg Lys Leu Val Pro Thr Gly Gly Glu
    130                 135                 140
Arg Leu Val Trp Ala Gln Gly Asp Gly Ser Thr Leu Glu Val Tyr Asp
145                 150                 155                 160
Thr Pro Leu Gly Lys Ile Gly Gly Leu Ile Cys Trp Glu Asn Tyr Met
                165                 170                 175
Pro Leu Ala Arg Tyr Thr Met Tyr Ala Trp Gly Thr Gln Ile Tyr Ile
            180                 185                 190
Ala Ala Thr Trp Asp Gln Gly Glu Thr Trp Leu Ala Thr Leu Arg His
        195                 200                 205
Ile Ala Lys Glu Gly Arg Val Tyr Val Ile Gly Cys Cys Ile Ala Leu
    210                 215                 220
Arg Arg Asp Asp Ile Pro Asp Arg Leu Glu Tyr Lys Lys Lys Phe Tyr
225                 230                 235                 240
Ser Gly Ser Arg Glu Trp Ile Asn Met Gly Asp Ser Ala Ile Val Asn
                245                 250                 255
Pro Glu Gly Glu Phe Ile Ala Gly Pro Val Arg Met Lys Glu Glu Ile

260                 265                 270
Leu Tyr Ala Glu Val Asp Pro Leu Leu Met Ala Gly Ser Lys Trp Met
        275                 280                 285
Leu Asp Val Ala Gly His Tyr Ala Arg Pro Asp Val Phe Glu Leu Ile
    290                 295                 300
Val His Arg Gln Pro His Pro Met Ile Arg Val Ile Glu Lys Glu Gly
305                 310                 315                 320
Gly Ala Gly Arg Thr Gly Asp Glu Lys Lys Glu Asn Glu
                325                 330

<210> 67
<211> 936
<212> DNA
<213> Unknown

<220>
<223> Obtained from an environmental sample

<400> 67
atgccccgtg tggcggtggt ccagcgcccg ccggtgtttc tcgaccgcgc cgcgaccctc      60
gagaacgccg tggcttcgct cgccgaggcc gcgtcgaacg gggctcgcct cgcggtcttt     120
ccggaagccc tggttcccgg ctatccggcg tggatgtggc ggctgcggcc cgggcccgac     180
atggcgctca ccgagcggat tcacgcgcgc ttgcgggcga actcggtgag cctcgccgcc     240
gacgagctcg cgccgctgcg cgaggcggcc cggcgccacg agctcaccgt agtgtgcggc     300
ctgcacgagc gcgacgaggc gctcggcggc ggcacgctct ataacaccgt cgtcacgatc     360
ggcgccgacg gcgcggtgct caaccgccac cggaagctga tgcccaccaa ccccgagcgc     420
atggtctggg gctgcggcga tgccagcggg ctcaggacgg tccccaccca gtgcgggcgc     480
gtcggcgccc tgatctgctg ggaaagctac atgccgcttg cacgctacgc gctgtacgcc     540
cagggaatcg acctctacgt cacgccgacc tacgacagcg gcgagcgggc ggttgcgacc     600
atgcagcaca ttgcccgcga aggcggctgc tgggtggtga gctgcggctc ggcgtttcag     660
gcgcgcgacg tcccggacgc gtttccgggg aagagcgagc ttttccgcga caacgacgag     720
tggatcaacc cgggcgactc ggtcgtggtc gcgccgggcg gcaaggtcgt cgccgggccg     780
ctgcacaaag aacgcgcgat cctgtacgcc gagatcgacc tcgagcgggt cggcgtggcg     840
cgccgcagcc tggacgtggt cggccattat gcgcggcccg acctcttcga cctgcacgtg     900
aacgcccgcc cgcaaagcgt ggttgaattg cgctga                               936

<210> 68
<211> 311
<212> PRT
<213> Unknown

<220>
<223> Obtained from an environmental sample

<400> 68
Met Pro Arg Val Ala Val Val Gln Arg Pro Pro Val Phe Leu Asp Arg
1               5                   10                  15
Ala Ala Thr Leu Glu Asn Ala Val Ala Ser Leu Ala Glu Ala Ala Ser
            20                  25                  30
Asn Gly Ala Arg Leu Ala Val Phe Pro Glu Ala Leu Val Pro Gly Tyr
        35                  40                  45
Pro Ala Trp Met Trp Arg Leu Arg Pro Gly Pro Asp Met Ala Leu Thr
    50                  55                  60
Glu Arg Ile His Ala Arg Leu Arg Ala Asn Ser Val Ser Leu Ala Ala
65                  70                  75                  80
Asp Glu Leu Ala Pro Leu Arg Glu Ala Ala Arg Arg His Glu Leu Thr
                85                  90                  95
Val Val Cys Gly Leu His Glu Arg Asp Glu Ala Leu Gly Gly Gly Thr
            100                 105                 110
Leu Tyr Asn Thr Val Val Thr Ile Gly Ala Asp Gly Ala Val Leu Asn
        115                 120                 125
Arg His Arg Lys Leu Met Pro Thr Asn Pro Glu Arg Met Val Trp Gly
    130                 135                 140
Cys Gly Asp Ala Ser Gly Leu Arg Thr Val Pro Thr Gln Cys Gly Arg
145                 150                 155                 160
Val Gly Ala Leu Ile Cys Trp Glu Ser Tyr Met Pro Leu Ala Arg Tyr
                165                 170                 175
Ala Leu Tyr Ala Gln Gly Ile Asp Leu Tyr Val Thr Pro Thr Tyr Asp

                180                 185                 190
Ser Gly Glu Arg Ala Val Ala Thr Met Gln His Ile Ala Arg Glu Gly
        195                 200                 205
Gly Cys Trp Val Val Ser Cys Gly Ser Ala Phe Gln Ala Arg Asp Val
    210                 215                 220
Pro Asp Ala Phe Pro Gly Lys Ser Glu Leu Phe Arg Asp Asn Asp Glu
225                 230                 235                 240
Trp Ile Asn Pro Gly Asp Ser Val Val Val Ala Pro Gly Gly Lys Val
                245                 250                 255
Val Ala Gly Pro Leu His Lys Glu Arg Ala Ile Leu Tyr Ala Glu Ile
            260                 265                 270
Asp Leu Glu Arg Val Gly Val Ala Arg Arg Ser Leu Asp Val Val Gly
        275                 280                 285
His Tyr Ala Arg Pro Asp Leu Phe Asp Leu His Val Asn Ala Arg Pro
    290                 295                 300
Gln Ser Val Val Glu Leu Arg
305                 310

<210> 69
<211> 939
<212> DNA
<213> Unknown

<220>
<223> Obtained from an environmental sample

<400> 69
gtgaccgagt ttcggacggt gcgggtcgca gcggtgcagg cgacgccggt gaccctcgac      60
gccgatgcct cggtcgagaa ggcgatcggg ctgatcggcg aggcggtggc cggtggagcg     120
cagctcgtcg tgctgcccga ggccttcgtg tcgctctacc cgtcgaacgc gtgggcgcga     180
gcggccgccg gattcggcgg cttcgacgag ctctgggagc ggatgtgggc cagctcgctc     240
gacgtcccgg gcccgctggt cgaccggctg gtcgatgcgt gccgcaggca tgacgtggta     300
tgcgtgatcg gcgtgaacga gcgcgaaagc gaaaggccgg ggtcgcttta caacacgatg     360
ctgaccctcg gcccgtcggg cctcctgcac cggcaccgca agctcatgcc gacgcaccac     420
gagcggctgt tccatgggat cggcgacggt caagacctcg gcgttgtgga gaccgacgcg     480
ggacggatcg ggggactgat ctgctgggag aaccgaatgc cgctcgcgcg ctacgcggtc     540
taccagggtg gaccgcagat ctgggtcgcg ccgacggccg atgactccga cggctggctc     600
gcgagcatgc gccacatcgc gatcgagtcg ggcgcgttcg tcgtgtcggt gccgcagttc     660
atcccggcgt ccgcgttccc cgacgatttc cccgtcgagc taccgccggg caaggaggtg     720
ttcggccgcg gcggtgcggc gatcgtcgag ccgacctggg gcgaggtaat cgccgggccg     780
ctctacgatc gggaggggat cgtgttcgcc gactgtgacc tgcgacgcgg cttgcatgcc     840
aagcgctggt tcgactccgt cggccattac agccgcgcgg aggtgctcga tggcggcgtc     900
gagcgcgtcc cggcgccggt ggacggcgaa tcgccgtga                            939

<210> 70
<211> 312
<212> PRT
<213> Unknown

<220>
<223> Obtained from an environmental sample

<400> 70
Val Thr Glu Phe Arg Thr Val Arg Val Ala Ala Val Gln Ala Thr Pro
 1               5                  10                  15
Val Thr Leu Asp Ala Asp Ala Ser Val Glu Lys Ala Ile Gly Leu Ile
            20                  25                  30
Gly Glu Ala Val Ala Gly Gly Ala Gln Leu Val Val Leu Pro Glu Ala
        35                  40                  45
Phe Val Ser Leu Tyr Pro Ser Asn Ala Trp Ala Arg Ala Ala Ala Gly
    50                  55                  60
Phe Gly Gly Phe Asp Glu Leu Trp Glu Arg Met Trp Ala Ser Ser Leu
65                  70                  75                  80
Asp Val Pro Gly Pro Leu Val Asp Arg Leu Val Asp Ala Cys Arg Arg
                85                  90                  95
His Asp Val Val Cys Val Ile Gly Val Asn Glu Arg Glu Ser Glu Arg
            100                 105                 110
Pro Gly Ser Leu Tyr Asn Thr Met Leu Thr Leu Gly Pro Ser Gly Leu

Leu His Arg His Arg Lys Leu Met Pro Thr His His Glu Arg Leu Phe
        115                 120                 125
His Gly Ile Gly Asp Gly Gln Asp Leu Gly Val Val Glu Thr Asp Ala
    130                 135                 140
Gly Arg Ile Gly Gly Leu Ile Cys Trp Glu Asn Arg Met Pro Leu Ala
145                 150                 155                 160
Arg Tyr Ala Val Tyr Gln Gly Gly Pro Gln Ile Trp Val Ala Pro Thr
                165                 170                 175
Ala Asp Asp Ser Asp Gly Trp Leu Ala Ser Met Arg His Ile Ala Ile
            180                 185                 190
Glu Ser Gly Ala Phe Val Val Ser Val Pro Gln Phe Ile Pro Ala Ser
        195                 200                 205
Ala Phe Pro Asp Asp Phe Pro Val Glu Leu Pro Pro Gly Lys Glu Val
    210                 215                 220
Phe Gly Arg Gly Gly Ala Ala Ile Val Glu Pro Thr Trp Gly Glu Val
225                 230                 235                 240
Ile Ala Gly Pro Leu Tyr Asp Arg Glu Gly Ile Val Phe Ala Asp Cys
                245                 250                 255
Asp Leu Arg Arg Gly Leu His Ala Lys Arg Trp Phe Asp Ser Val Gly
            260                 265                 270
His Tyr Ser Arg Ala Glu Val Leu Asp Gly Gly Val Glu Arg Val Pro
        275                 280                 285
Ala Pro Val Asp Gly Glu Ser Pro
    290                 295                 300
305                 310

<210> 71
<211> 966
<212> DNA
<213> Unknown

<220>
<223> Obtained from an environmental sample

<400> 71
atgccaaacg agaacaccaa cgccacattc aaagttgccg ctgtgcaggc ttcgcctgtg      60
tttcttgatc gtgccgcaac aatcgacaag gcttgcgatt tgatcgccgc tgctggcggt     120
gaaggggcac gcttgattgt ctttccagaa gcattcatcc cgtcttatcc tgattgggta     180
tgggcaattc cttcgggtga agagggcgta ctcaatgagt tgtacgcaga tctgctatcc     240
aactcggtca cgattcccag tgactcgacg gacaaactgt gcagagcagc caggcttgct     300
aatgcctacg tggtgatggg tatgagcgaa cgcaatgctg aggcaagcgg cgcgagcatg     360
tataacacgc tattgtatat tgatgcacag ggggagattc tgggcaagca tcggaagttg     420
gtgccaacgg gcggcgagcg gctagtctgg gcgcagggcg atggcagtac actgcaggtc     480
tatgatactc ccttagggaa actcggtggc ttaatttgct gggagaatta tatgccactg     540
gcccgctata ccatgtatgc ctggggcaca caaatctatg tcgcggcaac gtgggatcgg     600
ggtcagccct ggctctctac tttacgccac attgccaaag aaggcagggt gtatgtgatt     660
ggttgttgta tcgcgatgcg taaagacgat atcccagacc attatacaat gaaacagaag     720
ttttactcag atgcagatga gtggattaat attggcgata gtgcgattgt taatcccgaa     780
gggcaattta tcgctggacc ggtgcgcaag caggaagaga ttctctatgc ggagattgat     840
ccgcgcatgg tccaagggcc gaagtggatg ctcgacgtgg cgggacatta tgccaggccg     900
gatgtgttcg aactgattgt ccacacggat attcgaagga tgatcaaatc ggaaaagaat     960
tcataa                                                                966

<210> 72
<211> 321
<212> PRT
<213> Unknown

<220>
<223> Obtained from an environmental sample

<400> 72
Met Pro Asn Glu Asn Thr Asn Ala Thr Phe Lys Val Ala Ala Val Gln
1               5                   10                  15
Ala Ser Pro Val Phe Leu Asp Arg Ala Ala Thr Ile Asp Lys Ala Cys
            20                  25                  30
Asp Leu Ile Ala Ala Ala Gly Gly Glu Gly Ala Arg Leu Ile Val Phe
        35                  40                  45

Pro Glu Ala Phe Ile Pro Ser Tyr Pro Asp Trp Val Trp Ala Ile Pro
    50                  55                  60
Ser Gly Glu Glu Gly Val Leu Asn Glu Leu Tyr Ala Asp Leu Leu Ser
65                  70                  75                  80
Asn Ser Val Thr Ile Pro Ser Asp Ser Thr Asp Lys Leu Cys Arg Ala
                85                  90                  95
Ala Arg Leu Ala Asn Ala Tyr Val Val Met Gly Met Ser Glu Arg Asn
            100                 105                 110
Ala Glu Ala Ser Gly Ala Ser Met Tyr Asn Thr Leu Leu Tyr Ile Asp
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Thr Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Asp His Tyr Thr Met Lys Gln Lys
225                 230                 235                 240
Phe Tyr Ser Asp Ala Asp Glu Trp Ile Asn Ile Gly Asp Ser Ala Ile
                245                 250                 255
Val Asn Pro Glu Gly Gln Phe Ile Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Glu
    290                 295                 300
Leu Ile Val His Thr Asp Ile Arg Arg Met Ile Lys Ser Glu Lys Asn
305                 310                 315                 320
Ser

<210> 73
<211> 1035
<212> DNA
<213> Unknown

<220>
<223> Obtained from an environmental sample

<400> 73
atgacagcaa tagactcaac gtttaaagtc gccgccgttc aggctgcgcc ggtcttcctc      60
aatcgcgacg caaccgtgga gaaggcgtgc cggctgatca agtccgcggc agagggaggc     120
gcgcgtctga tcgttttccc ggaagcgttc ataccggcct acccggactg ggtgtggacg     180
gtccctgccg gtgagcaagg cctgctcaac gacctctacg gccaactcgt cgaccagtcc     240
gtgacgattc ccagcgacat caccaccgag ttatgtaacg cggcacgggc agcaaacgcc     300
tatgtcgtga ttggtgtcaa cgagcgcaac gcggaggcaa gcaatggaag cctctacaac     360
tcgctcctct acatcgacgc aaacggcaaa attctcggta agcaccgcaa gctcgttccc     420
acaggcggag aacggctcgt gtgggcgcag ggcgatggca gcacgctcga agcctacgac     480
acggagctgg gcaaactcgg cggtctcatt tgctgggaga actatatgcc gctggcacgc     540
tacgcgatgt acgcatgggg agtgcagctc tatgtcgccg cgacctggga ccgtggcggc     600
ccctggactg ccacgctgcg tcatgtcgcc aaggaaggtc agatgtacgt catcgggtgc     660
tgccaggccc tgcacaagga tgacctgccg gagctagacg ggctgaagga gaagtactac     720
gccaacgcac gagagtggat caatgttggc gacagcgcta ttgtcggccc ggacggacaa     780
ttccttgtcg agcccgtccg aatgcgggaa gacatcctct acgccgaggt ggacactcgc     840
aacttccgcg gcccgaagtg gatgttcgac gcggctggac actacgcgcg tcccgacatt     900
ttccaactca cagtgaaccg cgagcagcgg ccgatggtcc gcgtcgtcgg tgacagcagt     960
gaccagaagg agcggccgct cccggacgac ggacggctct ggtacgccta cagcaccaat    1020
cagcaccacg actga                                                     1035

<210> 74
<211> 344
<212> PRT

<213> Unknown

<220>
<223> Obtained from an environmental sample

<400> 74
Met Thr Ala Ile Asp Ser Thr Phe Lys Val Ala Ala Val Gln Ala Ala
1               5                   10                  15
Pro Val Phe Leu Asn Arg Asp Ala Thr Val Glu Lys Ala Cys Arg Leu
            20                  25                  30
Ile Lys Ser Ala Ala Glu Gly Gly Ala Arg Leu Ile Val Phe Pro Glu
        35                  40                  45
Ala Phe Ile Pro Ala Tyr Pro Asp Trp Val Trp Thr Val Pro Ala Gly
    50                  55                  60
Glu Gln Gly Leu Leu Asn Asp Leu Tyr Gly Gln Leu Val Asp Gln Ser
65                  70                  75                  80
Val Thr Ile Pro Ser Asp Ile Thr Thr Glu Leu Cys Asn Ala Ala Arg
                85                  90                  95
Ala Ala Asn Ala Tyr Val Val Ile Gly Val Asn Glu Arg Asn Ala Glu
            100                 105                 110
Ala Ser Asn Gly Ser Leu Tyr Asn Ser Leu Leu Tyr Ile Asp Ala Asn
        115                 120                 125
Gly Lys Ile Leu Gly Lys His Arg Lys Leu Val Pro Thr Gly Gly Glu
    130                 135                 140
Arg Leu Val Trp Ala Gln Gly Asp Gly Ser Thr Leu Glu Ala Tyr Asp
145                 150                 155                 160
Thr Glu Leu Gly Lys Leu Gly Gly Leu Ile Cys Trp Glu Asn Tyr Met
                165                 170                 175
Pro Leu Ala Arg Tyr Ala Met Tyr Ala Trp Gly Val Gln Leu Tyr Val
            180                 185                 190
Ala Ala Thr Trp Asp Arg Gly Gly Pro Trp Thr Ala Thr Leu Arg His
        195                 200                 205
Val Ala Lys Glu Gly Gln Met Tyr Val Ile Gly Cys Cys Gln Ala Leu
    210                 215                 220
His Lys Asp Asp Leu Pro Glu Leu Asp Gly Leu Lys Glu Lys Tyr Tyr
225                 230                 235                 240
Ala Asn Ala Arg Glu Trp Ile Asn Val Gly Asp Ser Ala Ile Val Gly
                245                 250                 255
Pro Asp Gly Gln Phe Leu Val Glu Pro Val Arg Met Arg Glu Asp Ile
            260                 265                 270
Leu Tyr Ala Glu Val Asp Thr Arg Asn Phe Arg Gly Pro Lys Trp Met
        275                 280                 285
Phe Asp Ala Ala Gly His Tyr Ala Arg Pro Asp Ile Phe Gln Leu Thr
    290                 295                 300
Val Asn Arg Glu Gln Arg Pro Met Val Arg Val Val Gly Asp Ser Ser
305                 310                 315                 320
Asp Gln Lys Glu Arg Pro Leu Pro Asp Asp Gly Arg Leu Trp Tyr Ala
                325                 330                 335
Tyr Ser Thr Asn Gln His His Asp
            340

<210> 75
<211> 1125
<212> DNA
<213> Unknown

<220>
<223> Obtained from an environmental sample

<400> 75
atgagcacca ttgttaaagc cgctgcggtt caaatcagcc cagtcctcta cagccgcgag      60
gggacagtcg caaaagttgt gcggaagatc cacgaacttg gccaaaaggg ggtgcggttc     120
gccacgttcc cggagaccgt ggttccctac tatccatatt tttccgccgt ccagaccccc     180
attcaactat tgtccggaac cgagtacctg aagttgctcg accaaggcgt gaccgtgccg     240
tccacgacta ccgacgcaat cggggaggct gcccggaacg ccggcatggt tgtatctatc     300
ggcgtgaatg agcgtgacgg cgggaccctg tacaacgcgc agttgctctt cgatgcggat     360
gggaccttga ttcagcgtcg ccgcaagatc actcctacgc attacgagcg catgatctgg     420
ggccagggag atggttcggg tttgcgggcc gtcaagagcc aggttggtcg tattggccaa     480

cttgcatgct ttgagcacaa caacccactg gcgcgttacg cgatgatggc cgatggcgag 540
caaatccatt cggccatgta tccaggttcc gcgttcggcg aggggttcgc ggaaaagatg 600
gaaatcaata tccgccagca tgcgttggag tccgggtgct tcgttgtgaa tgcaacggcc 660
tggcttgacg ccagccagca ggcacaaatc atgaatgaca cgggttgcca aatcggtccg 720
atctcgggcg gttgctttac cacgatcgta acacccgacg gcacgtttct gggcgaacct 780
ctccggtcgg gtgagggcga ggtcatcgcc gatctcgatt tcaagctgat cgacaaacgc 840
aagatgttga tggactcgcg cggccactac agtcgcccgg aattgctcag tctgctgatc 900
gaccgcaccc ccaccgcgca cattcatgag cgaggtgcgc cgcagacgtc aggcgctgtg 960
caagaggcga cgaaagtggg ttcacacgcg ccgctcctgc gtgacggaca atgggatcag 1020
ctcaatgcgg gagcgggccg acatacaggg aatggagaag cacagataga aatcatggcc 1080
gcggcccact cgggcacccg tggaattgaa gcgaagggag cctaa 1125

<210> 76
<211> 374
<212> PRT
<213> Unknown

<220>
<223> Obtained from an environmental sample

<400> 76
Met Ser Thr Ile Val Lys Ala Ala Ala Val Gln Ile Ser Pro Val Leu
1               5                   10                  15
Tyr Ser Arg Glu Gly Thr Val Ala Lys Val Val Arg Lys Ile His Glu
            20                  25                  30
Leu Gly Gln Lys Gly Val Arg Phe Ala Thr Phe Pro Glu Thr Val Val
        35                  40                  45
Pro Tyr Tyr Pro Tyr Phe Ser Ala Val Gln Thr Pro Ile Gln Leu Leu
    50                  55                  60
Ser Gly Thr Glu Tyr Leu Lys Leu Leu Asp Gln Gly Val Thr Val Pro
65                  70                  75                  80
Ser Thr Thr Thr Asp Ala Ile Gly Glu Ala Ala Arg Asn Ala Gly Met
                85                  90                  95
Val Val Ser Ile Gly Val Asn Glu Arg Asp Gly Gly Thr Leu Tyr Asn
            100                 105                 110
Ala Gln Leu Leu Phe Asp Ala Asp Gly Thr Leu Ile Gln Arg Arg Arg
        115                 120                 125
Lys Ile Thr Pro Thr His Tyr Glu Arg Met Ile Trp Gly Gln Gly Asp
    130                 135                 140
Gly Ser Gly Leu Arg Ala Val Lys Ser Gln Val Gly Arg Ile Gly Gln
145                 150                 155                 160
Leu Ala Cys Phe Glu His Asn Asn Pro Leu Ala Arg Tyr Ala Met Met
                165                 170                 175
Ala Asp Gly Glu Gln Ile His Ser Ala Met Tyr Pro Gly Ser Ala Phe
            180                 185                 190
Gly Glu Gly Phe Ala Glu Lys Met Glu Ile Asn Ile Arg Gln His Ala
        195                 200                 205
Leu Glu Ser Gly Cys Phe Val Val Asn Ala Thr Ala Trp Leu Asp Ala
    210                 215                 220
Ser Gln Gln Ala Gln Ile Met Asn Asp Thr Gly Cys Gln Ile Gly Pro
225                 230                 235                 240
Ile Ser Gly Gly Cys Phe Thr Thr Ile Val Thr Pro Asp Gly Thr Phe
                245                 250                 255
Leu Gly Glu Pro Leu Arg Ser Gly Glu Gly Glu Val Ile Ala Asp Leu
            260                 265                 270
Asp Phe Lys Leu Ile Asp Lys Arg Lys Met Leu Met Asp Ser Arg Gly
        275                 280                 285
His Tyr Ser Arg Pro Glu Leu Leu Ser Leu Leu Ile Asp Arg Thr Pro
    290                 295                 300
Thr Ala His Ile His Glu Arg Gly Ala Pro Gln Thr Ser Gly Ala Val
305                 310                 315                 320
Gln Glu Ala Thr Lys Val Gly Ser His Ala Pro Leu Leu Arg Asp Gly
                325                 330                 335
Gln Trp Asp Gln Leu Asn Ala Gly Ala Gly Arg His Thr Gly Asn Gly
            340                 345                 350
Glu Ala Gln Ile Glu Ile Met Ala Ala Ala His Ser Gly Thr Arg Gly
        355                 360                 365
Ile Glu Ala Lys Gly Ala

    370

<210> 77
<211> 1056
<212> DNA
<213> Unknown

<220>
<223> Obtained from an environmental sample

<400> 77
atgccaaccc ccagcgatca tttcaaaatc gccgctgttc aggcctcgcc cgtgtttctg 60
gaccgggagg ccactgtgga aaaggcctgc cggttgatcg ccgaagccgc aaagcagggc 120
gtccgcctca tcgtctttcc ggaatcgttc atcccgacct acccggactg ggtatgggcc 180
gttcccccgg gaagggaaag aatcctgaac cagctgtatt ctgaattcct ggccaatgcc 240
gtcgatgttc ccggcgcggc gaccgaacaa cttgcccagg ctgcacgaat ggccggcgcc 300
tatgtgatta tgggcgtcac cgaaagagac acctcggcca gcggggccag cctctacaac 360
accctgctct acttcagccc cgaaggcatc ctaatgggca aacaccggaa gctggttccc 420
acggggggcg aacggctggt ctgggcctac ggagacggca gcacgctgga ggtctacgac 480
actccgctgg gaaagatcgg cgggctgatc tgctgggaga actacatgcc cctggcccgg 540
tacacgatgt acgcctgggg cacccagatt tacatcgccg ccacctggga ccgcggggaa 600
ccgtggctct ccaccctgcg gcatatcgca aaggaaggaa gggtctacgt catcgggtgc 660
tgcatcgccc tgcgccaggg ggatatcccg gaccggttcg agtacaaggg aaaattttat 720
tccgggtccc gggagtggat caatgagggc gacagcgcca tcgtgaaccc ggacggggaa 780
ttcatcgccg ggccggtgcg gacgaaggag gagatcctgt atgccgagat agacccccgg 840
cagatgcggg gccccaagtg gatgctcgat gtggccggtc attacgcccg gccggatatc 900
ttcgagctca tcgtccaccg gaatccccac ccgatgatca aaatcgccga agacaggggc 960
acggggatcg cctcaagttt gattcgcccc cgccctaacc ttcccccatc aagggggagg 1020
aaatcggcaa gaagcaaacg caagcccaaa aaatga 1056

<210> 78
<211> 351
<212> PRT
<213> Unknown

<220>
<223> Obtained from an environmental sample

<400> 78
Met Pro Thr Pro Ser Asp His Phe Lys Ile Ala Ala Val Gln Ala Ser
1               5                   10                  15
Pro Val Phe Leu Asp Arg Glu Ala Thr Val Glu Lys Ala Cys Arg Leu
            20                  25                  30
Ile Ala Glu Ala Ala Lys Gln Gly Val Arg Leu Ile Val Phe Pro Glu
        35                  40                  45
Ser Phe Ile Pro Thr Tyr Pro Asp Trp Val Trp Ala Val Pro Pro Gly
    50                  55                  60
Arg Glu Arg Ile Leu Asn Gln Leu Tyr Ser Glu Phe Leu Ala Asn Ala
65                  70                  75                  80
Val Asp Val Pro Gly Ala Ala Thr Glu Gln Leu Ala Gln Ala Ala Arg
                85                  90                  95
Met Ala Gly Ala Tyr Val Ile Met Gly Val Thr Glu Arg Asp Thr Ser
            100                 105                 110
Ala Ser Gly Ala Ser Leu Tyr Asn Thr Leu Leu Tyr Phe Ser Pro Glu
        115                 120                 125
Gly Ile Leu Met Gly Lys His Arg Lys Leu Val Pro Thr Gly Gly Glu
    130                 135                 140
Arg Leu Val Trp Ala Tyr Gly Asp Gly Ser Thr Leu Glu Val Tyr Asp
145                 150                 155                 160
Thr Pro Leu Gly Lys Ile Gly Gly Leu Ile Cys Trp Glu Asn Tyr Met
                165                 170                 175
Pro Leu Ala Arg Tyr Thr Met Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Lys Glu Gly Arg Val Tyr Val Ile Gly Cys Cys Ile Ala Leu
    210                 215                 220
Arg Gln Gly Asp Ile Pro Asp Arg Phe Glu Tyr Lys Gly Lys Phe Tyr

225                 230                 235                 240
Ser Gly Ser Arg Glu Trp Ile Asn Glu Gly Asp Ser Ala Ile Val Asn
                245                 250                 255
Pro Asp Gly Glu Phe Ile Ala Gly Pro Val Arg Thr Lys Glu Glu Ile
            260                 265                 270
Leu Tyr Ala Glu Ile Asp Pro Arg Gln Met Arg Gly Pro Lys Trp Met
        275                 280                 285
Leu Asp Val Ala Gly His Tyr Ala Arg Pro Asp Ile Phe Glu Leu Ile
    290                 295                 300
Val His Arg Asn Pro His Pro Met Ile Lys Ile Ala Glu Asp Arg Gly
305                 310                 315                 320
Thr Gly Ile Ala Ser Ser Leu Ile Arg Pro Arg Pro Asn Leu Pro Pro
                325                 330                 335
Ser Arg Gly Arg Lys Ser Ala Arg Ser Lys Arg Lys Pro Lys Lys
            340                 345                 350

<210> 79
<211> 990
<212> DNA
<213> Unknown

<220>
<223> Obtained from an environmental sample

<400> 79
atgacgaaga aaagcggccg cgattcgttt cgggtcgctg cggtccaggc ctcgtccgtc      60
tacctggatc gggaacggag catcgagaaa gcgtgccggc tgatcgacga cgcgggacga     120
aacgacgccg acctcgtcgt gttccccgaa gccttcgtgc ccggataccc actgtgggtg     180
tggctcgttc cgccggggcg caccgcagac ttgcgctccg cttatgcgac gctccacgcc     240
aacgcgatca gcattccgga cgactccacc gatcggctgt gcgccgccgc aaaagacgcc     300
ggcgtcgccg tcgcgatcgg cgtcaacgaa cgcaacaccg aagcgagcgg catgagcctg     360
ttcaacacgc tgctctatat cggagcggac ggccggattc tcggaaaaca ccggaagctg     420
gtaccgaccg gcggcgaacg gctcgtctgg gcatctggcg acggcagcga cctcgaggtc     480
tactcgctgc cgttcggtcg cgtaagcgga ctgatctgct gggagcacta catgccgctc     540
gcccggtatg cgctcgccgc gtggggcgaa caggtgcacg tcgctccaac ctgggatcgt     600
ggcgagccgt ggctgtccac gctaaggcac atcgcgaagg aaggccgcgt tctcgtcgtc     660
ggctgctgtc aagccgtgcg caaggacgac atccctgaca cgctcgcgtt caagtccaaa     720
tacctcgcag acgtggacgg ctggatcaac ccaggtggca gcgtcatcat caatcctgac     780
ggcaaggtcg tcgcgggacc ggcgatggaa accgaaactg tactgtacgc ggaccttcgc     840
accgagcagc tcgtcggacc gcgctggcag ctcgacgtcg gcggacatta cgctcgtccg     900
gacgtcttcg agctcgtcgt ccatcggcat ccgaagccgt tgattcggac agcgaccggt     960
gtcaggcgcc gcaagcgtgc acgtcgctaa                                      990

<210> 80
<211> 329
<212> PRT
<213> Unknown

<220>
<223> Obtained from an environmental sample

<400> 80
Met Thr Lys Lys Ser Gly Arg Asp Ser Phe Arg Val Ala Ala Val Gln
1               5                   10                  15
Ala Ser Ser Val Tyr Leu Asp Arg Glu Arg Ser Ile Glu Lys Ala Cys
            20                  25                  30
Arg Leu Ile Asp Asp Ala Gly Arg Asn Asp Ala Asp Leu Val Val Phe
        35                  40                  45
Pro Glu Ala Phe Val Pro Gly Tyr Pro Leu Trp Val Trp Leu Val Pro
    50                  55                  60
Pro Gly Arg Thr Ala Asp Leu Arg Ser Ala Tyr Ala Thr Leu His Ala
65                  70                  75                  80
Asn Ala Ile Ser Ile Pro Asp Asp Ser Thr Asp Arg Leu Cys Ala Ala
                85                  90                  95
Ala Lys Asp Ala Gly Val Ala Val Ala Ile Gly Val Asn Glu Arg Asn
            100                 105                 110
Thr Glu Ala Ser Gly Met Ser Leu Phe Asn Thr Leu Leu Tyr Ile Gly
        115                 120                 125

Ala Asp Gly Arg Ile Leu Gly Lys His Arg Lys Leu Val Pro Thr Gly
    130                 135                 140
Gly Glu Arg Leu Val Trp Ala Ser Gly Asp Gly Ser Asp Leu Glu Val
145                 150                 155                 160
Tyr Ser Leu Pro Phe Gly Arg Val Ser Gly Leu Ile Cys Trp Glu His
                165                 170                 175
Tyr Met Pro Leu Ala Arg Tyr Ala Leu Ala Ala Trp Gly Glu Gln Val
            180                 185                 190
His Val Ala Pro Thr Trp Asp Arg Gly Glu Pro Trp Leu Ser Thr Leu
        195                 200                 205
Arg His Ile Ala Lys Glu Gly Arg Val Leu Val Val Gly Cys Cys Gln
    210                 215                 220
Ala Val Arg Lys Asp Asp Ile Pro Asp Thr Leu Ala Phe Lys Ser Lys
225                 230                 235                 240
Tyr Leu Ala Asp Val Asp Gly Trp Ile Asn Pro Gly Gly Ser Val Ile
                245                 250                 255
Ile Asn Pro Asp Gly Lys Val Val Ala Gly Pro Ala Met Glu Thr Glu
            260                 265                 270
Thr Val Leu Tyr Ala Asp Leu Arg Thr Glu Gln Leu Val Gly Pro Arg
        275                 280                 285
Trp Gln Leu Asp Val Gly Gly His Tyr Ala Arg Pro Asp Val Phe Glu
    290                 295                 300
Leu Val Val His Arg His Pro Lys Pro Leu Ile Arg Thr Ala Thr Gly
305                 310                 315                 320
Val Arg Arg Arg Lys Arg Ala Arg Arg
                325

<210> 81
<211> 993
<212> DNA
<213> Unknown

<220>
<223> Obtained from an environmental sample

<400> 81
atgaaagtcg tcaaagccgc cgctgtccag ttcagcccgg tgctctatag ccgcgaagcg      60
accgtcgcca aggtcgtccg gaaaatccac gagctcggtc agaaaggcgt gcagttcgcc     120
acctttcctg aaacggtcgt gccttattac ccttacttcg cggccgtcca gacgggcatc     180
gagctcttgt cgggcaccga acatctgcgc ctgctcgaac aggccgtgac tgtgccctcc     240
gctgcgaccg atgcaatcgg cgaagccgcg cgacaggccg gcatggtcgt gtccatcggc     300
gtcaatgagc gtgacggcgg cacgctttac aacacgcaac tgctcttcga tgccgacggt     360
acgctgatcc agcgccgccg caagatcacg ccgacccatt tcgaacgcat gatctggggg     420
cagggagatg gctcgggctt gcgtgcagtc gacagcgcag tcggccgcat cggccagctc     480
gcatgcttcg agcacaacaa cccgcttgca cgttacgcaa tgatcgccga cggcgagcag     540
atccattcag cgatgtaccc tggctcggcc tttggcgagg gcttcgccca gcgtatggag     600
atcaacatcc gccagcatgc gctcgagtcc gccgctttcg tcgtcaacgc aacggcgtgg     660
cttgacgccg accagcaggc gcaaatcatg aaggacaccg gttgtggaat cggtccgatc     720
tcgggcggct gcttcaccac gatcgtttct cctgacggta tgctgatggc cgatccgctt     780
cgctcgggcg aaggcgaagt gattgtcgat ctcgacttca cgcagatcga ccgccgcaag     840
atgctgatgg actcggccgg ccactacaac cgccctgaac tgctgagtct gatgatcgac     900
cgtacgccgg ctgcgcatgt tcacgaacgc gcttcgcgcc cgatgaccgt cgacgaccag     960
agttccggcg atctgcgcac ccaggttgca tga                                  993

<210> 82
<211> 330
<212> PRT
<213> Unknown

<220>
<223> Obtained from an environmental sample

<400> 82
Met Lys Val Val Lys Ala Ala Ala Val Gln Phe Ser Pro Val Leu Tyr
1               5                   10                  15
Ser Arg Glu Ala Thr Val Ala Lys Val Val Arg Lys Ile His Glu Leu
            20                  25                  30
Gly Gln Lys Gly Val Gln Phe Ala Thr Phe Pro Glu Thr Val Val Pro

Tyr Tyr Pro Tyr Phe Ala Ala Val Gln Thr Gly Ile Glu Leu Leu Ser
    50                  55                  60
Gly Thr Glu His Leu Arg Leu Leu Glu Gln Ala Val Thr Val Pro Ser
65                  70                  75                  80
Ala Ala Thr Asp Ala Ile Gly Glu Ala Ala Arg Gln Ala Gly Met Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Ala Val Gly Arg Ile Gly Gln Leu
145                 150                 155                 160
Ala Cys Phe Glu His Asn Asn Pro Leu Ala Arg Tyr Ala Met Ile Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Ala Ala Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Ser Gly Gly Cys Phe Thr Thr Ile Val Ser Pro Asp Gly Met Leu Met
                245                 250                 255
Ala Asp Pro Leu Arg Ser Gly Glu Gly Glu Val Ile Val Asp Leu Asp
            260                 265                 270
Phe Thr Gln Ile Asp Arg Arg Lys Met Leu Met Asp Ser Ala Gly His
        275                 280                 285
Tyr Asn Arg Pro Glu Leu Leu Ser Leu Met Ile Asp Arg Thr Pro Ala
    290                 295                 300
Ala His Val His Glu Arg Ala Ser Arg Pro Met Thr Val Asp Asp Gln
305                 310                 315                 320
Ser Ser Gly Asp Leu Arg Thr Gln Val Ala
                325                 330

<210> 83
<211> 1071
<212> DNA
<213> Unknown

<220>
<223> Obtained from an environmental sample

<400> 83
   atgatgagtt cagcccgtgt aataaaactc gccgcagcac agctttcacc tgtgctgccg      60
   ggggagtcca caaatagccg cgacggcacc attgccaaag tcgtcgcggc gattgcggag     120
   gctgcgcgcg ccggcgcgca gctgatcgtg tttcccgaaa cggtggtgcc gtattacccg     180
   tatttctcgt tcattacgcc ggcggtgacg atgggggcgg agcatttgcg cttgtacgat     240
   cagtctgtcg tggtgccgag cgccgccact gatactgttg ccgccgctgc aaaaaaacac     300
   agcatggtgg tcgtgctcgg tattaacgaa cgcgatcacg gcacgctcta caacgcgcaa     360
   ttaattttcg atgcgagcgg cgaattatta ttaaaacgcc gaaaaattac cccgacctat     420
   cacgagcgca tggtgtgggg tcagggcgac ggcagcggtt tgaaaaccgt cgacaccgcg     480
   atcggccgtg tcggtgcgct cgcctgctgg gaacattaca acccattggc gcgttacagc     540
   ctgatggccc agcacgaaga aattcattgc agtcaatttc cggggtcatt ggtcggccca     600
   attttcgccg agcaaatgga agtgacaatg cgccaccacg cgctcgaatc cggttgcttc     660
   gtcgttaatg caacggcgtg gttatcggaa gcgcaaattc aatcgatcag cagcgatccc     720
   gcgatgcaaa aagcactgcg cggcggttgc tacaccgcaa ttatttcgcc cgaaggcaaa     780
   catctgtgcg agccgctacg cgaaggtgaa ggtttgattt ttgccgaagc cgatatggcg     840
   ctcattacca aacgcaaacg catgatggat tcggttggtc attacgcgcg acccgaattg     900
   ctgtcgctgt taatcgacca tcgcgccacc acaccattgc atagcgtcac cgcgagtgat     960
   gccgccgccg taaaaaatac tcggagttcc gctcatgaat cagccgatag tgaaaccatc    1020
   cgcgagtcag ttaataacgg aactccaatc gcacggcttg cgcctagttg a             1071

<210> 84
<211> 356

<212> PRT
<213> Unknown

<220>
<223> Obtained from an environmental sample

<400> 84
Met Met Ser Ser Ala Arg Val Ile Lys Leu Ala Ala Ala Gln Leu Ser
1               5                   10                  15
Pro Val Leu Pro Gly Glu Ser Thr Asn Ser Arg Asp Gly Thr Ile Ala
            20                  25                  30
Lys Val Val Ala Ala Ile Ala Glu Ala Ala Arg Ala Gly Ala Gln Leu
        35                  40                  45
Ile Val Phe Pro Glu Thr Val Val Pro Tyr Tyr Pro Tyr Phe Ser Phe
    50                  55                  60
Ile Thr Pro Ala Val Thr Met Gly Ala Glu His Leu Arg Leu Tyr Asp
65                  70                  75                  80
Gln Ser Val Val Val Pro Ser Ala Ala Thr Asp Thr Val Ala Ala Ala
                85                  90                  95
Ala Lys Lys His Ser Met Val Val Val Leu Gly Ile Asn Glu Arg Asp
            100                 105                 110
His Gly Thr Leu Tyr Asn Ala Gln Leu Ile Phe Asp Ala Ser Gly Glu
        115                 120                 125
Leu Leu Leu Lys Arg Arg Lys Ile Thr Pro Thr Tyr His Glu Arg Met
    130                 135                 140
Val Trp Gly Gln Gly Asp Gly Ser Gly Leu Lys Thr Val Asp Thr Ala
145                 150                 155                 160
Ile Gly Arg Val Gly Ala Leu Ala Cys Trp Glu His Tyr Asn Pro Leu
                165                 170                 175
Ala Arg Tyr Ser Leu Met Ala Gln His Glu Glu Ile His Cys Ser Gln
            180                 185                 190
Phe Pro Gly Ser Leu Val Gly Pro Ile Phe Ala Glu Gln Met Glu Val
        195                 200                 205
Thr Met Arg His His Ala Leu Glu Ser Gly Cys Phe Val Val Asn Ala
    210                 215                 220
Thr Ala Trp Leu Ser Glu Ala Gln Ile Gln Ser Ile Ser Ser Asp Pro
225                 230                 235                 240
Ala Met Gln Lys Ala Leu Arg Gly Gly Cys Tyr Thr Ala Ile Ile Ser
                245                 250                 255
Pro Glu Gly Lys His Leu Cys Glu Pro Leu Arg Glu Gly Glu Gly Leu
            260                 265                 270
Ile Phe Ala Glu Ala Asp Met Ala Leu Ile Thr Lys Arg Lys Arg Met
        275                 280                 285
Met Asp Ser Val Gly His Tyr Ala Arg Pro Glu Leu Leu Ser Leu Leu
    290                 295                 300
Ile Asp His Arg Ala Thr Thr Pro Leu His Ser Val Thr Ala Ser Asp
305                 310                 315                 320
Ala Ala Ala Val Lys Asn Thr Arg Ser Ser Ala His Glu Ser Ala Asp
                325                 330                 335
Ser Glu Thr Ile Arg Glu Ser Val Asn Asn Gly Thr Pro Ile Ala Arg
            340                 345                 350
Leu Ala Pro Ser
        355

<210> 85
<211> 1014
<212> DNA
<213> Unknown

<220>
<223> Obtained from an environmental sample

<400> 85
atgggtctgg ttcatcagaa atacaaggtt gcggtggttc aggcggcgcc ggtctttctc      60
gacctcgatg cgacggtgga caagacgatc gccctgatcg agcaggccgc agcacagggc     120
gcgaagctga tcgcgtttcc cgagaccttc attcccggat atccgtggca gatctggctt     180
ggggcgcccg cctgggcgat cggccgtggc ttcgtgcagc gctatttcga taactcgttg     240
tcatttgaca gcccgcaggc cgaaaaaatt cgcaaggccg tcaagcgcgc caagctgacc     300

gcggtgatcg gcgtctccga acgcgacggc ggcagcctct atatcggcca atggctgatc 360
ggtcccgacg gcgagaccat tgcgaagcgc cgcaagctgc ggccgaccca tgccgaacgc 420
accgtgttcg gcgagggcga cggcagcgac ctcgccgtcc atgatcgcgc cgacgtggga 480
cggctcggtg caatgtgctg ctgggagcat ctgcagccgc tgtcgaaata cgcgatgtac 540
gcccagaacg agcaggttca cgtcggcgcc tggccgagct tctcattgta cgacccattc 600
gcccatgcgc ttggctggga agtaaacaac gcggcgagca aggtttatgc tgtcgagggc 660
tcatgtttct tcctcggccc gtgcgcggtg gtctcgcagg ccatgatcga cgagctctgc 720
gattcccccg aaaagcacgc cttcctgcac gctggcggcg gccacgcggt aatctatggg 780
ccggacggga gttcgcttgc cgacaaactt ccacccgatc aggagggcat tctgtatgcc 840
gatatcgatc tcggcatgat cggcgtggca aagaacgccg ccgaccccgc aggacactat 900
tccaggccgg acgtcacgcg gctgctgctc aacacttccc gcgccaatcg cgtcgagcat 960
tttcattgcc cgatcgatgc cgaggtcatg agcgaaatca gacttcaggc ctga 1014

<210> 86
<211> 337
<212> PRT
<213> Unknown

<220>
<223> Obtained from an environmental sample

<400> 86
Met Gly Leu Val His Gln Lys Tyr Lys Val Ala Val Val Gln Ala Ala
1               5                   10                  15
Pro Val Phe Leu Asp Leu Asp Ala Thr Val Asp Lys Thr Ile Ala Leu
            20                  25                  30
Ile Glu Gln Ala Ala Ala Gln Gly Ala Lys Leu Ile Ala Phe Pro Glu
        35                  40                  45
Thr Phe Ile Pro Gly Tyr Pro Trp Gln Ile Trp Leu Gly Ala Pro Ala
    50                  55                  60
Trp Ala Ile Gly Arg Gly Phe Val Gln Arg Tyr Phe Asp Asn Ser Leu
65                  70                  75                  80
Ser Phe Asp Ser Pro Gln Ala Glu Lys Ile Arg Lys Ala Val Lys Arg
                85                  90                  95
Ala Lys Leu Thr Ala Val Ile Gly Val Ser Glu Arg Asp Gly Gly Ser
            100                 105                 110
Leu Tyr Ile Gly Gln Trp Leu Ile Gly Pro Asp Gly Glu Thr Ile Ala
        115                 120                 125
Lys Arg Arg Lys Leu Arg Pro Thr His Ala Glu Arg Thr Val Phe Gly
    130                 135                 140
Glu Gly Asp Gly Ser Asp Leu Ala Val His Asp Arg Ala Asp Val Gly
145                 150                 155                 160
Arg Leu Gly Ala Met Cys Cys Trp Glu His Leu Gln Pro Leu Ser Lys
                165                 170                 175
Tyr Ala Met Tyr Ala Gln Asn Glu Gln Val His Val Gly Ala Trp Pro
            180                 185                 190
Ser Phe Ser Leu Tyr Asp Pro Phe Ala His Ala Leu Gly Trp Glu Val
        195                 200                 205
Asn Asn Ala Ala Ser Lys Val Tyr Ala Val Glu Gly Ser Cys Phe Phe
    210                 215                 220
Leu Gly Pro Cys Ala Val Val Ser Gln Ala Met Ile Asp Glu Leu Cys
225                 230                 235                 240
Asp Ser Pro Glu Lys His Ala Phe Leu His Ala Gly Gly Gly His Ala
                245                 250                 255
Val Ile Tyr Gly Pro Asp Gly Ser Ser Leu Ala Asp Lys Leu Pro Pro
            260                 265                 270
Asp Gln Glu Gly Ile Leu Tyr Ala Asp Ile Asp Leu Gly Met Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Thr Ser Arg Ala Asn Arg Val Glu His
305                 310                 315                 320
Phe Ser Leu Pro Ile Asp Ala Glu Val Met Ser Glu Ile Arg Leu Gln
                325                 330                 335
Ala

<210> 87

<211> 1062
<212> DNA
<213> Unknown

<220>
<223> Obtained from an environmental sample

<400> 87
atggcggaat cgaagctgaa ggtcgccgca attcaagttg cgcccgtgtt catggatcgc      60
gatgccacga tcgcccgcgc ctgcgagcgg atcgccgaag ccgcccgcgc cggcgcggag     120
ttggtggtct ttcccgaggc attcgtgccc gggtatcccg actggatctg ggtggcgcgg     180
ccaagccaac gcaaactgct caatgatctt tacgcgcacc tcgtctcgca gtcggtcgac     240
gtgccgtcgg cctccgtgga tcgtttgcgc gacgcggctc gcgacggcgg ggtcacggtg     300
gtgatcggcg tcaacgagcg caacaccgaa gcgagcggcg cgagcctcta caacaccgcg     360
ctcgtgatcg gtccactggg gcagctgatc ggccgccacc gcaagcttgt gccgaccggg     420
ccggagcgca tggtgtgggc gcagggcgac ggcagcacgc tcgacgtcta cgacacaccc     480
gtcggcaagc tttcgacgtt gatctgctgg gagaactaca tgccgctcgc gcgctacgcc     540
atggcggcgt ggggcgcgcg catccacgtc gccggcacgt gggaccgcgg cgagccgtgg     600
atctcgacca tgcgtcatgt ggcgacggag ggccgcgtat tcgtgattag ctgttgcatg     660
gcgctgcgca aacgagacat tcccgccgag ctcgagttcg cgatgctcta tcccgacggg     720
cgcgaatgga tcaacgccgg tgattcgctg gtcgtgaatc ccgctggcca gatcatcgct     780
gggccgttgc acgagcagga aggaatcctc tacgccgagc tcgagcgcaa tcagatgacc     840
ggtccgcgtt ggatgttcga cgccgccggc cattacgcgc gaccggacgt cttccaactc     900
acggtaaacc gctccccgcg cccgatgctg cgggaggcgg gggcaaagac gagtgaggca     960
aacacgagag atgccgtacc catggacagc acgccctcga gatcgcggcc ccgcgcggtg    1020
gcgcgaaagg ccgcacgcac cggtcgctcc aagcggcggt ga                       1062

<210> 88
<211> 353
<212> PRT
<213> Unknown

<220>
<223> Obtained from an environmental sample

<400> 88
Met Ala Glu Ser Lys Leu Lys Val Ala Ala Ile Gln Val Ala Pro Val
1               5                   10                  15
Phe Met Asp Arg Asp Ala Thr Ile Ala Arg Ala Cys Glu Arg Ile Ala
            20                  25                  30
Glu Ala Ala Arg Ala Gly Ala Glu Leu Val Val Phe Pro Glu Ala Phe
        35                  40                  45
Val Pro Gly Tyr Pro Asp Trp Ile Trp Val Ala Arg Pro Ser Gln Arg
    50                  55                  60
Lys Leu Leu Asn Asp Leu Tyr Ala His Leu Val Ser Gln Ser Val Asp
65                  70                  75                  80
Val Pro Ser Ala Ser Val Asp Arg Leu Arg Asp Ala Ala Arg Asp Gly
                85                  90                  95
Gly Val Thr Val Val Ile Gly Val Asn Glu Arg Asn Thr Glu Ala Ser
            100                 105                 110
Gly Ala Ser Leu Tyr Asn Thr Ala Leu Val Ile Gly Pro Leu Gly Gln
        115                 120                 125
Leu Ile Gly Arg His Arg Lys Leu Val Pro Thr Gly Pro Glu Arg Met
    130                 135                 140
Val Trp Ala Gln Gly Asp Gly Ser Thr Leu Asp Val Tyr Asp Thr Pro
145                 150                 155                 160
Val Gly Lys Leu Ser Thr Leu Ile Cys Trp Glu Asn Tyr Met Pro Leu
                165                 170                 175
Ala Arg Tyr Ala Met Ala Ala Trp Gly Ala Arg Ile His Val Ala Gly
            180                 185                 190
Thr Trp Asp Arg Gly Glu Pro Trp Ile Ser Thr Met Arg His Val Ala
        195                 200                 205
Thr Glu Gly Arg Val Phe Val Ile Ser Cys Cys Met Ala Leu Arg Lys
    210                 215                 220
Arg Asp Ile Pro Ala Glu Leu Glu Phe Ala Met Leu Tyr Pro Asp Gly
225                 230                 235                 240
Arg Glu Trp Ile Asn Ala Gly Asp Ser Leu Val Val Asn Pro Ala Gly
                245                 250                 255

Gln Ile Ile Ala Gly Pro Leu His Glu Gln Glu Gly Ile Leu Tyr Ala
                260                 265                 270
Glu Leu Glu Arg Asn Gln Met Thr Gly Pro Arg Trp Met Phe Asp Ala
        275                 280                 285
Ala Gly His Tyr Ala Arg Pro Asp Val Phe Gln Leu Thr Val Asn Arg
    290                 295                 300
Ser Pro Arg Pro Met Leu Arg Glu Ala Gly Ala Lys Thr Ser Glu Ala
305                 310                 315                 320
Asn Thr Arg Asp Ala Val Pro Met Asp Ser Thr Pro Ser Arg Ser Arg
                325                 330                 335
Pro Arg Ala Val Ala Arg Lys Ala Ala Arg Thr Gly Arg Ser Lys Arg
                340                 345                 350
Arg

<210> 89
<211> 918
<212> DNA
<213> Unknown

<220>
<223> Obtained from an environmental sample

<400> 89
atgaatacca aagaagtaaa ggtcgcagcc gctcaatttg ccccacattt tctgaatttg      60
agcaaaacgg tggaaaaaac ctgcaacttg atttccgaag caggcaaaaa tggagcaaag     120
ctcattgtat ttccggaagc cttcctctct ggttatcccg attgggtctg gttaattccc     180
aatggaaatt caacaatgct ggatgattta tatcaggaat tggttgagaa cgctgtaaca     240
atccctgatt caacaacaca gaaactctgt caggcagcaa aagatgccgg ggtatatgtc     300
gcagtcggta tccatgaaag aaatgcagaa gcaagtggct tcacactttt caataccctt     360
ctatacatta atgatcaagg cagcatcatt ggaaaacacc gaaaactgat cccaacaggg     420
ggcgaacgcc tggtctgggg gcagggtaat ggggatacgc ttgctgcatt cgatacacac     480
tttggcaaat tgggaggatt gctttgctgg gaaaactaca tgcccctggc tcggcaagct     540
atgtacgcag ttgggactga agtttatgtt gccccaacct gggactccag tgagaattgg     600
ttgctgagta tgcgccatat agccagagag ggcggcatgt ttgtgatcaa tgtttgccag     660
gctgtccgaa aagacgatat tcctgaccgc tatgcattca agcaactcta ttctggtaat     720
tcagaatgga tcaatagcgg caacagttgc atcatcaatc cgcgcggtga aatcattgcc     780
ggaccatcct caaacaggca agaaatactc tacgcagatt tagatctgag tttgattaca     840
aaatctaaac gcatgttcga tgttaccggg cattatgccc ggccggatgt gtttagatat     900
gaaatcaaaa aaagctag                                                   918

<210> 90
<211> 305
<212> PRT
<213> Unknown

<220>
<223> Obtained from an environmental sample

<400> 90
Met Asn Thr Lys Glu Val Lys Val Ala Ala Ala Gln Phe Ala Pro His
1               5                   10                  15
Phe Leu Asn Leu Ser Lys Thr Val Glu Lys Thr Cys Asn Leu Ile Ser
            20                  25                  30
Glu Ala Gly Lys Asn Gly Ala Lys Leu Ile Val Phe Pro Glu Ala Phe
        35                  40                  45
Leu Ser Gly Tyr Pro Asp Trp Val Trp Leu Ile Pro Asn Gly Asn Ser
    50                  55                  60
Thr Met Leu Asp Asp Leu Tyr Gln Glu Leu Val Glu Asn Ala Val Thr
65                  70                  75                  80
Ile Pro Asp Ser Thr Thr Gln Lys Leu Cys Gln Ala Ala Lys Asp Ala
                85                  90                  95
Gly Val Tyr Val Ala Val Gly Ile His Glu Arg Asn Ala Glu Ala Ser
            100                 105                 110
Gly Phe Thr Leu Phe Asn Thr Leu Leu Tyr Ile Asn Asp Gln Gly Ser
        115                 120                 125
Ile Ile Gly Lys His Arg Lys Leu Ile Pro Thr Gly Gly Glu Arg Leu
    130                 135                 140

Val Trp Gly Gln Gly Asn Gly Asp Thr Leu Ala Ala Phe Asp Thr His
145                 150                 155                 160
Phe Gly Lys Leu Gly Gly Leu Leu Cys Trp Glu Asn Tyr Met Pro Leu
                165                 170                 175
Ala Arg Gln Ala Met Tyr Ala Val Gly Thr Glu Val Tyr Val Ala Pro
            180                 185                 190
Thr Trp Asp Ser Ser Glu Asn Trp Leu Leu Ser Met Arg His Ile Ala
        195                 200                 205
Arg Glu Gly Gly Met Phe Val Ile Asn Val Cys Gln Ala Val Arg Lys
    210                 215                 220
Asp Asp Ile Pro Asp Arg Tyr Ala Phe Lys Gln Leu Tyr Ser Gly Asn
225                 230                 235                 240
Ser Glu Trp Ile Asn Ser Gly Asn Ser Cys Ile Ile Asn Pro Arg Gly
                245                 250                 255
Glu Ile Ile Ala Gly Pro Ser Ser Asn Arg Gln Glu Ile Leu Tyr Ala
            260                 265                 270
Asp Leu Asp Leu Ser Leu Ile Thr Lys Ser Lys Arg Met Phe Asp Val
        275                 280                 285
Thr Gly His Tyr Ala Arg Pro Asp Val Phe Arg Tyr Glu Ile Lys Lys
    290                 295                 300
Ser
305

<210> 91
<211> 939
<212> DNA
<213> Unknown

<220>
<223> Obtained from an environmental sample

<400> 91
atgaccaaaa tcgctgtcat tcaagaacct ccggtctatc tgaatctgag taaatcgatg      60
gacagagcgg tcgacttgat tgccaatgct gcaagcaagg ggtgtgagtt gattgtgttt     120
cccgaagcct ggcttgcagg ttaccccacc ttcgtctggc gtcttgcgcc gggcagcgga     180
atgggaaaaa ctgatgagct ttacgcgcgt ttgctcgcca actcggtcga ccgtagcaaa     240
gaggggctta gaccattgca ggaggccgca aaggagcatg gcgttgtcat tgtgctgggt     300
tatcaagagg tggatggcgc gggaagcagc agcacgatct tcaacagctg tgcgattatt     360
gatgcggacg ggcgactggc caacaatcat cgcaagttga tgcccaccaa tccggagagg     420
atggtttggg gttttggcga cggttcaggc ctgaacgtcg ttgacaccgc ggtgggcagg     480
atcggcacgc tgatttgctg ggaaaactac atgccgttag cgcgctacgc gctgtatgtc     540
caaaacatcg aaatctatgt tgccccgact tgggacagtg gtgccatgtg gcaggcgacc     600
ctgcagcata tcgcgcgcga aggtggctgc tgggtcatcg gatgtgcaac gtcgctggaa     660
gcctctgaca tcccggacga cgttccccat cgggatgagc tattcccgaa caaagacgaa     720
tgggtaaacc ctggcgatgc ggtggtttat aagccatttg gcggcattgt ggccggcccc     780
atgcatcagg aaaaggggct tctcatcgca gagttggacg tcgccgctgt tcagtcgtca     840
cgtcggaagt tcgatgcgag cgggcactac gctcgccccg atgtcttcaa actgcatgtg     900
aatcgcaccg cgatgcggcc agttgatttc acgaattag                            939

<210> 92
<211> 312
<212> PRT
<213> Unknown

<220>
<223> Obtained from an environmental sample

<400> 92
Met Thr Lys Ile Ala Val Ile Gln Glu Pro Pro Val Tyr Leu Asn Leu
1               5                   10                  15
Ser Lys Ser Met Asp Arg Ala Val Asp Leu Ile Ala Asn Ala Ala Ser
            20                  25                  30
Lys Gly Cys Glu Leu Ile Val Phe Pro Glu Ala Trp Leu Ala Gly Tyr
        35                  40                  45
Pro Thr Phe Val Trp Arg Leu Ala Pro Gly Ser Gly Met Gly Lys Thr
    50                  55                  60
Asp Glu Leu Tyr Ala Arg Leu Leu Ala Asn Ser Val Asp Arg Ser Lys
65                  70                  75                  80

Glu Gly Leu Arg Pro Leu Gln Glu Ala Ala Lys Glu His Gly Val Val
                85                  90                  95
Ile Val Leu Gly Tyr Gln Glu Val Asp Gly Ala Gly Ser Ser Ser Thr
            100                 105                 110
Ile Phe Asn Ser Cys Ala Ile Ile Asp Ala Asp Gly Arg Leu Ala Asn
        115                 120                 125
Asn His Arg Lys Leu Met Pro Thr Asn Pro Glu Arg Met Val Trp Gly
    130                 135                 140
Phe Gly Asp Gly Ser Gly Leu Asn Val Val Asp Thr Ala Val Gly Arg
145                 150                 155                 160
Ile Gly Thr Leu Ile Cys Trp Glu Asn Tyr Met Pro Leu Ala Arg Tyr
                165                 170                 175
Ala Leu Tyr Val Gln Asn Ile Glu Ile Tyr Val Ala Pro Thr Trp Asp
            180                 185                 190
Ser Gly Ala Met Trp Gln Ala Thr Leu Gln His Ile Ala Arg Glu Gly
        195                 200                 205
Gly Cys Trp Val Ile Gly Cys Ala Thr Ser Leu Glu Ala Ser Asp Ile
    210                 215                 220
Pro Asp Asp Val Pro His Arg Asp Glu Leu Phe Pro Asn Lys Asp Glu
225                 230                 235                 240
Trp Val Asn Pro Gly Asp Ala Val Val Tyr Lys Pro Phe Gly Gly Ile
                245                 250                 255
Val Ala Gly Pro Met His Gln Glu Lys Gly Leu Leu Ile Ala Glu Leu
            260                 265                 270
Asp Val Ala Ala Val Gln Ser Ser Arg Arg Lys Phe Asp Ala Ser Gly
        275                 280                 285
His Tyr Ala Arg Pro Asp Val Phe Lys Leu His Val Asn Arg Thr Ala
    290                 295                 300
Met Arg Pro Val Asp Phe Thr Asn
305                 310

<210> 93
<211> 978
<212> DNA
<213> Unknown

<220>
<223> Obtained from an environmental sample

<400> 93
atgcccatca tcaaagccgc tgccgtgcaa atcagcccgg tgctttacag tcgcgaaggc      60
accgtggaca aggtctgtca acagatcatc gacctcggtc ggcaaggcgt gcagttcgcc     120
gtctttccgg aaacggtggt gccttactac ccgtactttt cgtttgtgca gccggccttt     180
gccatgggcg cacagcacct caagttgctg gatcaatcgg tgacagtgcc gtcggccgcc     240
accttggcca tcggtgaagc ttgcaagcaa gcagggatag tggtgtccat cggcgtcaac     300
gaacgcgatg gcggtacgat ctacaacgcg caattactct tcgatgccga cggcagcctg     360
attcagcatc gccgcaaaat caccccgacc tatcacgaac gcatggtctg gggcaaggcc      420
gatggttccg gcctgcgcgc catcgacagt gcagtggggc gcattggctc cctggcctgt     480
tgggagcatt acaacccgct ggctcgttat gccttgatgg ccgatggcga gcagatccac     540
gccgcgatgt ttcccggctc gctggtgggc gacatttttg ccgagcagat cgaagtcacc     600
atccgccatc acgccttgga gtccggctgt ttcgtggtca acgccaccgc ctggctggac     660
gccgatcagc agggccaaat catgcaagac accggttgca gcctcggccc gatctcgggt     720
ggctgcttca ccgccatcgt ttcccctgaa ggcaagttgc tcggtgagcc gctgcgttcc     780
ggcgaagggg tggtgatcgc cgatctcgat ctggcactga tcgataagcg taaacggatg     840
atggattcgg tcgggcatta cagtcgcccg gaactgctca gcctgttgat cgaccgcacg     900
cccacagcgc atgtgcatga acgcagcgcg cacctggtgg ctgtcgctac cgaggagttc     960
gatcatgcaa accaatga                                                   978

<210> 94
<211> 325
<212> PRT
<213> Unknown

<220>
<223> Obtained from an environmental sample

<400> 94
Met Pro Ile Ile Lys Ala Ala Ala Val Gln Ile Ser Pro Val Leu Tyr

1               5                   10                  15
Ser Arg Glu Gly Thr Val Asp Lys Val Cys Gln Gln Ile Ile Asp Leu
            20                  25                  30
Gly Arg Gln Gly Val Gln Phe Ala Val Phe Pro Glu Thr Val Val Pro
        35                  40                  45
Tyr Tyr Pro Tyr Phe Ser Phe Val Gln Pro Ala Phe Ala Met Gly Ala
    50                  55                  60
Gln His Leu Lys Leu Leu Asp Gln Ser Val Thr Val Pro Ser Ala Ala
65                  70                  75                  80
Thr Leu Ala Ile Gly Glu Ala Cys Lys Gln Ala Gly Ile Val Val Ser
                85                  90                  95
Ile Gly Val Asn Glu Arg Asp Gly Gly Thr Ile Tyr Asn Ala Gln Leu
            100                 105                 110
Leu Phe Asp Ala Asp Gly Ser Leu Ile Gln His Arg Arg Lys Ile Thr
        115                 120                 125
Pro Thr Tyr His Glu Arg Met Val Trp Gly Gln Gly Asp Gly Ser Gly
    130                 135                 140
Leu Arg Ala Ile Asp Ser Ala Val Gly Arg Ile Gly Ser Leu Ala Cys
145                 150                 155                 160
Trp Glu His Tyr Asn Pro Leu Ala Arg Tyr Ala Leu Met Ala Asp Gly
                165                 170                 175
Glu Gln Ile His Ala Ala Met Phe Pro Gly Ser Leu Val Gly Asp Ile
            180                 185                 190
Phe Ala Glu Gln Ile Glu Val Thr Ile Arg His His Ala Leu Glu Ser
        195                 200                 205
Gly Cys Phe Val Val Asn Ala Thr Ala Trp Leu Asp Ala Asp Gln Gln
    210                 215                 220
Gly Gln Ile Met Gln Asp Thr Gly Cys Ser Leu Gly Pro Ile Ser Gly
225                 230                 235                 240
Gly Cys Phe Thr Ala Ile Val Ser Pro Glu Gly Lys Leu Leu Gly Glu
                245                 250                 255
Pro Leu Arg Ser Gly Glu Gly Val Val Ile Ala Asp Leu Asp Leu Ala
            260                 265                 270
Leu Ile Asp Lys Arg Lys Arg Met Met Asp Ser Val Gly His Tyr Ser
        275                 280                 285
Arg Pro Glu Leu Leu Ser Leu Leu Ile Asp Arg Thr Pro Thr Ala His
    290                 295                 300
Val His Glu Arg Ser Ala His Leu Val Ala Val Ala Thr Glu Glu Phe
305                 310                 315                 320
Asp His Ala Asn Gln
                325

<210>  95
<211>  966
<212>  DNA
<213>  Unknown

<220>
<223>  Obtained from an environmental sample

<400>  95
atgtccaacg agaataacat tgctacattc aaagttgccg cagtccaggc cacacctgtg      60
tttcttgatc gtgaagcaac catcgacaaa gcttgcgcgt tgattgccac tgctggcagt     120
gaaggagcgc gcctgattgt gtttccagaa gcattcatcc caacttatcc tgaatgggta     180
tggggtattc cctccggtga gcaaggttta ctcaacgaac tctatgcaga gttgctcacc     240
aatgcggtca ccattcccag cgatgcgact gacaggctgt gcgaggctgc gcagcttgcg     300
aatgcctacg tagtgatggg catgagcgaa cggaacgtcg aggcgagtgg cgcaagcctg     360
tataatacgc tgttgtacat aaatgcgcag ggggagattt tagggaaaca tcgaaagctg      420
gtgccaacgg gcggcgaacg cctggtatgg gcgcagggtg atggcagtac gctgcaggtc     480
tacgatactc cattgggaaa actcggtggc ttaatttgct gggaaaatta tatgccgctg     540
gcacggtatg ctatgtatgc ctggggaaca caaatctatg tcgcggcaac gtgggatcgc     600
ggtcaaccct ggctttctac attaaggcat atcgccaaag aaggcagggt atacgtgatt     660
ggttgctgta tcgcgatgcg taaagacgat attccagatc gttacaccat gaagcaaaaa     720
tattatgctg aaatggatga atggatgaat gttggtgaca gtgtgattgt caatcccgag     780
ggcactttta ttgccgggcc tgtgcgcaag caggaagaaa ttctctacgc ggagattgat     840
cctcgcatgg tgcaaggccc gaagtggatg ctcgatgtgg cagggcatta tgcgagaccg     900
gatgtgttcc agttgacggt gcatacggat gtgaggcgga tgatgcgggt ggaagatgat     960
tcataa                                                                966

<210> 96
<211> 321
<212> PRT
<213> Unknown

<220>
<223> Obtained from an environmental sample

<400> 96
Met Ser Asn Glu Asn Asn Ile Ala Thr Phe Lys Val Ala Ala Val Gln
1               5                   10                  15
Ala Thr Pro Val Phe Leu Asp Arg Glu Ala Thr Ile Asp Lys Ala Cys
            20                  25                  30
Ala Leu Ile Ala Thr Ala Gly Ser Glu Gly Ala Arg Leu Ile Val Phe
        35                  40                  45
Pro Glu Ala Phe Ile Pro Thr Tyr Pro Glu Trp Val Trp Gly Ile Pro
    50                  55                  60
Ser Gly Glu Gln Gly Leu Leu Asn Glu Leu Tyr Ala Glu Leu Leu Thr
65                  70                  75                  80
Asn Ala Val Thr Ile Pro Ser Asp Ala Thr Asp Arg Leu Cys Glu Ala
                85                  90                  95
Ala Gln Leu Ala Asn Ala Tyr Val Val Met Gly Met Ser Glu Arg Asn
            100                 105                 110
Val Glu Ala Ser Gly Ala Ser Leu Tyr Asn Thr Leu Leu Tyr Ile Asn
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Ala Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Asp Arg Tyr Thr Met Lys Gln Lys
225                 230                 235                 240
Tyr Tyr Ala Glu Met Asp Glu Trp Met Asn Val Gly Asp Ser Val Ile
                245                 250                 255
Val Asn Pro Glu Gly His Phe Ile Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asp Val Arg Arg Met Met Arg Val Glu Asp Asp
305                 310                 315                 320
Ser


<210> 97
<211> 1017
<212> DNA
<213> Unknown

<220>
<223> Obtained from an environmental sample

<400> 97
atgggcatcg aacatccgaa atacaaggtc gccgtggtgc aagctgcgcc cgcctggctc      60
gacctcgacg cgtcgatcga caagacgatc gggctgatcg aggaggcggc gaagaaaggc     120
gccaagctga tcgctttccc cgaagccttc attcccggct acccttggca catctggctc     180
gactcacccg cctgggcgat cggccgcggt ttcgtgcagc gctatttcga caattcgctc     240
gcctacgaca gcccacaggc ggaaaggctg cgacaggccg tgcggaaggc caagctcacc     300
gccgtgatcg gcctgtccga gcgcgacggc ggcagcctct atctcgcgca gtggctgatc     360

gggcccgacg gtgagaccat cgcaaagcgc cgcaagctgc ggccgaccca tgccgagcgc 420
accgtctatg gcgaaggcga cggcagcgat ctcgccgtcc atgagcgggc cgacatcggc 480
cggctcggcg cgctgtgctg ctgggagcat ctgcagccgc tgtcgaaatt cgccatgtac 540
gcccagaacg agcaggtaca tgtcgcggcc tggccgagct tctcgctcta cgatcccttc 600
gcgcctgcgc tgggcgcgga ggtgaacaac gccgcctccc gcatctatgc ggtggaaggc 660
tcctgcttcg tgctcgcacc gtgcgcgacg gtctcgcagg ccatgatcga cgagctctgc 720
gatcggccgg acaagcacgc gctgctgcat gccggcggcg gcttcgccgc gatctacggg 780
cccgacggca gccagatcgg cgacaagctg ccgcccgagc aggagggcct gctgatcgcc 840
gagatcgatc tgggcgcgat cggcgtcgcc aagaacgcgg ccgatcccgc cgggcattat 900
tcgcggcccg acgtcacgcg gctcctgctc aacaggaagc cgaacaagcg cgtggagcag 960
ttcgcgctgc ccgtcgacac ggtcgagccc gtcgacgtcg cggcggcagc aagctga 1017

<210> 98
<211> 338
<212> PRT
<213> Unknown

<220>
<223> Obtained from an environmental sample

<400> 98
Met Gly Ile Glu His Pro Lys Tyr Lys Val Ala Val Val Gln Ala Ala
1               5                   10                  15
Pro Ala Trp Leu Asp Leu Asp Ala Ser Ile Asp Lys Thr Ile Gly Leu
            20                  25                  30
Ile Glu Glu Ala Ala Lys Lys Gly Ala Lys Leu Ile Ala Phe Pro Glu
        35                  40                  45
Ala Phe Ile Pro Gly Tyr Pro Trp His Ile Trp Leu Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Arg Leu Arg Gln Ala Val Arg Lys
                85                  90                  95
Ala Lys Leu Thr Ala Val Ile Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Glu Arg Ala Asp Ile Gly
145                 150                 155                 160
Arg Leu Gly Ala Leu Cys Cys Trp Glu His Leu Gln Pro Leu Ser Lys
                165                 170                 175
Phe Ala Met Tyr Ala Gln Asn Glu Gln Val His Val Ala Ala Trp Pro
            180                 185                 190
Ser Phe Ser Leu Tyr Asp Pro Phe Ala Pro Ala Leu Gly Ala Glu Val
        195                 200                 205
Asn Asn Ala Ala Ser Arg Ile Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Ala Gly Gly Gly Phe Ala
                245                 250                 255
Ala Ile Tyr Gly Pro Asp Gly Ser Gln Ile Gly Asp Lys Leu Pro Pro
            260                 265                 270
Glu Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Arg Lys Pro Asn Lys Arg Val Glu Gln
305                 310                 315                 320
Phe Ala Leu Pro Val Asp Thr Val Glu Pro Val Asp Val Ala Ala Ala
                325                 330                 335
Ala Ser

<210> 99
<211> 1014

<212> DNA
<213> Unknown

<220>
<223> Obtained from an environmental sample

<400> 99
atgcctgaca agagaatcgt ccgcgccgcc gcggtccaga tagcaccgga cctcgaacgg 60
cccggtggca cgctcgagaa ggtcctcgag acgatcgacg acgccgcacg ccagggcgtg 120
cagctcatcg tcttccccga gaccttcctg ccctactacc cgtacttttc gttcgtgcgg 180
gcgccggtgg catcgggtgc agagcacatg cggctctatg acgaagcggt ggtcgtgccc 240
gggccggtga cgcatgcggt ggccgagcgg gcacggcggc acggcatggt cgtcgtgctc 300
ggcgtgaacg agcgcgatca cggcagctta tacaacgcac aactgatctt cgataccgac 360
ggcgagctgc tgctcaagcg ccgcaagatc acgccgacgt ttcacgaacg gatgatctgg 420
ggcatgggcg acgcagccgg cctgaaggta gcggaaacgc gtatcggccg ggtgggtgca 480
ctcgcttgct gggaacacta caacccgctt gcacgttatg cactgatgac ccagcacgaa 540
gagattcatt gcagccagtt tcccggctcg ctggtcggac ccatcttcgg tgaacagatc 600
gaagtgacca tccggcatca cgcactggaa tccggctgct tcgtgatcaa ttccaccggc 660
tggctgaccg agccgcagat cgagtcgatc acgaaagatc cgggcctgca gaaggcgctt 720
cgcggcggct gcaacacggc gatcatctcg cccgaaggcc agcatctcgc cccgccgctg 780
cgtgagggcg agggcatggt catcgctgac ctggacatgt cgctgatcac caaacgcaaa 840
cgcatgatgg attctgtcgg ccactacgcg cggcccgaac tgctgagcct cgccatcaac 900
gaccggccgg cggtcacgtc ggcacccatg aacagcttct catcttcaac cgggggattg 960
caccttgaac gcgaacgaga ccttgtcggc cgtgagccgg caattgatga ctga 1014

<210> 100
<211> 337
<212> PRT
<213> Unknown

<220>
<223> Obtained from an environmental sample

<400> 100
Met Pro Asp Lys Arg Ile Val Arg Ala Ala Ala Val Gln Ile Ala Pro
1               5                   10                  15
Asp Leu Glu Arg Pro Gly Gly Thr Leu Glu Lys Val Leu Glu Thr Ile
            20                  25                  30
Asp Asp Ala Ala Arg Gln Gly Val Gln Leu Ile Val Phe Pro Glu Thr
        35                  40                  45
Phe Leu Pro Tyr Tyr Pro Tyr Phe Ser Phe Val Arg Ala Pro Val Ala
    50                  55                  60
Ser Gly Ala Glu His Met Arg Leu Tyr Asp Glu Ala Val Val Val Pro
65                  70                  75                  80
Gly Pro Val Thr His Ala Val Ala Glu Arg Ala Arg Arg His Gly Met
                85                  90                  95
Val Val Val Leu Gly Val Asn Glu Arg Asp His Gly Ser Leu Tyr Asn
            100                 105                 110
Ala Gln Leu Ile Phe Asp Thr Asp Gly Glu Leu Leu Leu Lys Arg Arg
        115                 120                 125
Lys Ile Thr Pro Thr Phe His Glu Arg Met Ile Trp Gly Met Gly Asp
    130                 135                 140
Ala Ala Gly Leu Lys Val Ala Glu Thr Arg Ile Gly Arg Val Gly Ala
145                 150                 155                 160
Leu Ala Cys Trp Glu His Tyr Asn Pro Leu Ala Arg Tyr Ala Leu Met
                165                 170                 175
Thr Gln His Glu Glu Ile His Cys Ser Gln Phe Pro Gly Ser Leu Val
            180                 185                 190
Gly Pro Ile Phe Gly Glu Gln Ile Glu Val Thr Ile Arg His His Ala
        195                 200                 205
Leu Glu Ser Gly Cys Phe Val Ile Asn Ser Thr Gly Trp Leu Thr Glu
    210                 215                 220
Pro Gln Ile Glu Ser Ile Thr Lys Asp Pro Gly Leu Gln Lys Ala Leu
225                 230                 235                 240
Arg Gly Gly Cys Asn Thr Ala Ile Ile Ser Pro Glu Gly Gln His Leu
                245                 250                 255
Ala Pro Pro Leu Arg Glu Gly Glu Gly Met Val Ile Ala Asp Leu Asp
            260                 265                 270

Met Ser Leu Ile Thr Lys Arg Lys Arg Met Met Asp Ser Val Gly His
        275                 280                 285
Tyr Ala Arg Pro Glu Leu Leu Ser Leu Ala Ile Asn Asp Arg Pro Ala
    290                 295                 300
Val Thr Ser Ala Pro Met Asn Ser Phe Ser Ser Ser Thr Gly Gly Leu
305                 310                 315                 320
His Leu Glu Arg Glu Arg Asp Leu Val Gly Arg Glu Pro Ala Ile Asp
                325                 330                 335
Asp

<210> 101
<211> 1065
<212> DNA
<213> Unknown

<220>
<223> Obtained from an environmental sample

<400> 101
atggcgacag tccatccgaa atttaaagta gccgccgtcc aggcggcccc ggcctttctc      60
gacctcgacg cgtcggtgga aaaagcggtg cgcctgattg atgaagccgg cgccgctggt     120
gcccggctca tcgcgtttcc agagactttt atccccggtt atccgtggtg gatctggctc     180
ggtgctccgg cctgggcgat catgcgcggc ttcgtctccc gctatttcga caactcgctg     240
cagtacggca ccccggaagc cgaccggctg cgggcagccg ccaaacgcaa caaaatgttc     300
gtcgcgctcg gactgtcaga gcgcgacggc ggcagtctct acatcgccca atggattatc     360
ggacccgacg gcgagacggt cgcaacgcgc cgcaagctca agcctactca cgccgagcgg     420
acggtgttcg gcgaaggcga tggctcgcac cttgcggtcc acgaacttga tatcgggcgg      480
gtcggtgcgc tgtgctgttg ggagcacctg cagccactgt cgaagtacgc gatgtatgcg     540
cagaacgagc aagttcatat cgcggcgtgg ccgagctttt cgctttacga tccgttcgcg     600
catgcgcttg gcgccgaggt caacaacgcg gcgagcaaga tctacgcggt cgaaggctca     660
tgctttgtga ttgcgccatg cgcgaccgtt tcccaggcga tgatcgacga attgtgtgac     720
tcgcccgaga agcatcagtt cctgcacgtc ggcggcggtt tcgccgtgat ctatggtccc     780
gacggcgcgc cactcgccaa gccactggcg cccgatcagg agggtctcct ttacgcggat     840
atcgacctcg gcatgatttc ggtcgcgaaa gcggcggccg atccggctgg acattacgcg     900
cgcccggacg tgacccgtct gttgttcaac aatcgtcctg ggaaccgggt ggagacactc     960
gcgctgccgg tcgaccagga ggcagaggcg ggagcaggcg gcaaacctgc gcccaagtca    1020
ccgagtgtcg ctgcgttcac actgacgcag gcggcagccg agtag                    1065

<210> 102
<211> 354
<212> PRT
<213> Unknown

<220>
<223> Obtained from an environmental sample

<400> 102
Met Ala Thr Val His Pro Lys Phe Lys Val Ala Ala Val Gln Ala Ala
1               5                   10                  15
Pro Ala Phe Leu Asp Leu Asp Ala Ser Val Glu Lys Ala Val Arg Leu
            20                  25                  30
Ile Asp Glu Ala Gly Ala Ala Gly Ala Arg Leu Ile Ala Phe Pro Glu
        35                  40                  45
Thr Phe Ile Pro Gly Tyr Pro Trp Trp Ile Trp Leu Gly Ala Pro Ala
    50                  55                  60
Trp Ala Ile Met Arg Gly Phe Val Ser Arg Tyr Phe Asp Asn Ser Leu
65                  70                  75                  80
Gln Tyr Gly Thr Pro Glu Ala Asp Arg Leu Arg Ala Ala Ala Lys Arg
                85                  90                  95
Asn Lys Met Phe Val Ala Leu Gly Leu Ser Glu Arg Asp Gly Gly Ser
            100                 105                 110
Leu Tyr Ile Ala Gln Trp Ile Ile Gly Pro Asp Gly Glu Thr Val Ala
        115                 120                 125
Thr Arg Arg Lys Leu Lys Pro Thr His Ala Glu Arg Thr Val Phe Gly
    130                 135                 140
Glu Gly Asp Gly Ser His Leu Ala Val His Glu Leu Asp Ile Gly Arg
145                 150                 155                 160

Val Gly Ala Leu Cys Cys Trp Glu His Leu Gln Pro Leu Ser Lys Tyr
                165                 170                 175
Ala Met Tyr Ala Gln Asn Glu Gln Val His Ile Ala Ala Trp Pro Ser
            180                 185                 190
Phe Ser Leu Tyr Asp Pro Phe Ala His Ala Leu Gly Ala Glu Val Asn
        195                 200                 205
Asn Ala Ala Ser Lys Ile Tyr Ala Val Glu Gly Ser Cys Phe Val Ile
    210                 215                 220
Ala Pro Cys Ala Thr Val Ser Gln Ala Met Ile Asp Glu Leu Cys Asp
225                 230                 235                 240
Ser Pro Glu Lys His Gln Phe Leu His Val Gly Gly Gly Phe Ala Val
                245                 250                 255
Ile Tyr Gly Pro Asp Gly Ala Pro Leu Ala Lys Pro Leu Ala Pro Asp
            260                 265                 270
Gln Glu Gly Leu Leu Tyr Ala Asp Ile Asp Leu Gly Met Ile Ser Val
        275                 280                 285
Ala Lys Ala Ala Ala Asp Pro Ala Gly His Tyr Ala Arg Pro Asp Val
    290                 295                 300
Thr Arg Leu Leu Phe Asn Asn Arg Pro Gly Asn Arg Val Glu Thr Leu
305                 310                 315                 320
Ala Leu Pro Val Asp Gln Glu Ala Glu Ala Gly Ala Gly Gly Lys Pro
                325                 330                 335
Ala Pro Lys Ser Pro Ser Val Ala Ala Phe Thr Leu Thr Gln Ala Ala
            340                 345                 350
Ala Glu

<210> 103
<211> 945
<212> DNA
<213> Unknown

<220>
<223> Obtained from an environmental sample

<400> 103
atgggcgagt tcggcgaggt gacgctgggg gtggcgcagg cggcgccggt gtacttcgac      60
cgggaggcgt cgacggagaa ggctcgcggc ctgatccggg aggcggggga gaagggcgtc     120
gacctgttgg cgttcgggga gacgtggctg acggggtacc cgtactggaa ggatgcgccg     180
tggtctcggg agtacaacga cctgcgcgcg cggtacgtgg cgaatggcgt gatgataccg     240
gggccggaga cggacgcgct atgccaggca gcggcggaag cgggggtgga cgtggcaatc     300
ggcgtggtgg agctggagcc ggggagcctt tcgagcgtgt attgcacgtt gctgttcatc     360
tcgcgcgagg gcgagatcct ggggcggcac cggaagctga agccgacgga ttcggaacgg     420
cggtactggt cagagggtga tgcgacgggg ctgcgggtgt acgagcggcc atatggccgg     480
ttgagcggat tgaactgctg ggaacacctt atgatgttgc cggggtacgc gctggcggca     540
caggggacgc agtttcatgt ggcagcgtgg ccgaacatgg cgagctcggc gagcgagctg     600
ctgtcgcggg cgtatgcgta ccaggccgga tgctacgtgt tgtgcgcggg cgggctcggg     660
cctgcgccgg gagagctacc ggacggcatc gcggcggagt cgctggacca cctgacgggc     720
gagagctgca tcatcgaccc gtggggaaaa gtgatcgcgg ggccggtgtc gtgcgaggag     780
acgctgatta cggcgcgggt atcgaccgcg tcaatctacc ggcgcaagtc gctgacggac     840
gtgggtggcc actactcgcg accggacgtg ttccggttcg aggtggatag gtcggagcgc     900
ccgcgagtgg tgtttcggga tggggatgtg gacgaccggg ggtaa                     945

<210> 104
<211> 314
<212> PRT
<213> Unknown

<220>
<223> Obtained from an environmental sample

<400> 104
Met Gly Glu Phe Gly Glu Val Thr Leu Gly Val Ala Gln Ala Ala Pro
1               5                   10                  15
Val Tyr Phe Asp Arg Glu Ala Ser Thr Glu Lys Ala Arg Gly Leu Ile
            20                  25                  30
Arg Glu Ala Gly Glu Lys Gly Val Asp Leu Leu Ala Phe Gly Glu Thr
        35                  40                  45

Trp Leu Thr Gly Tyr Pro Tyr Trp Lys Asp Ala Pro Trp Ser Arg Glu
    50                  55                  60
Tyr Asn Asp Leu Arg Ala Arg Tyr Val Ala Asn Gly Val Met Ile Pro
65                  70                  75                  80
Gly Pro Glu Thr Asp Ala Leu Cys Gln Ala Ala Ala Glu Ala Gly Val
                85                  90                  95
Asp Val Ala Ile Gly Val Val Glu Leu Glu Pro Gly Ser Leu Ser Ser
            100                 105                 110
Val Tyr Cys Thr Leu Leu Phe Ile Ser Arg Glu Gly Glu Ile Leu Gly
        115                 120                 125
Arg His Arg Lys Leu Lys Pro Thr Asp Ser Glu Arg Arg Tyr Trp Ser
    130                 135                 140
Glu Gly Asp Ala Thr Gly Leu Arg Val Tyr Glu Arg Pro Tyr Gly Arg
145                 150                 155                 160
Leu Ser Gly Leu Asn Cys Trp Glu His Leu Met Met Leu Pro Gly Tyr
                165                 170                 175
Ala Leu Ala Ala Gln Gly Thr Gln Phe His Val Ala Ala Trp Pro Asn
            180                 185                 190
Met Ala Ser Ser Ala Ser Glu Leu Leu Ser Arg Ala Tyr Ala Tyr Gln
        195                 200                 205
Ala Gly Cys Tyr Val Leu Cys Ala Gly Gly Leu Gly Pro Ala Pro Gly
    210                 215                 220
Glu Leu Pro Asp Gly Ile Ala Ala Glu Ser Leu Asp His Leu Thr Gly
225                 230                 235                 240
Glu Ser Cys Ile Ile Asp Pro Trp Gly Lys Val Ile Ala Gly Pro Val
                245                 250                 255
Ser Cys Glu Glu Thr Leu Ile Thr Ala Arg Val Ser Thr Ala Ser Ile
            260                 265                 270
Tyr Arg Arg Lys Ser Leu Thr Asp Val Gly Gly His Tyr Ser Arg Pro
        275                 280                 285
Asp Val Phe Arg Phe Glu Val Asp Arg Ser Glu Arg Pro Arg Val Val
    290                 295                 300
Phe Arg Asp Gly Asp Val Asp Asp Arg Gly
305                 310

<210> 105
<211> 975
<212> DNA
<213> Unknown

<220>
<223> Obtained from an environmental sample

<400> 105
atgaccattg tcaaagccgc tgccgtccag attgcccccg ttctctacag ccgtgaaggc      60
actgtagaaa aggtcgttaa caagattcgc gaactcggcg agaagggcgt gcagttcgcc     120
gttttccctg aaaccgtcgt accgtactac ccgtactttt cctttgtgca gagccctttc     180
aaaatgggtt ccgagcacta caaattgctc gaccaggccg ttgtcgtgcc gtcggcgacc     240
accgatgcca tcggcaaagc ggccaaggaa gccaacatgg tggtgtccat cggcgtcaac     300
gaacgcgatg gcagcaccct ctacaacacg cagttgctgt ttgatgccga cggcactttg     360
attcaggccc gtcgcaagat ttcaccgacc taccacgaac gcatgatctg gggcatgggc      420
gacggttccg gcctgcgcgc caccgacagc gcggtcgggc gcatcggaca attggcctgc     480
tgggaacatt acaatccgct ggcgcgttac gccttgatcg aagacggcga acagatccac     540
gcctcgatgt acccgggctc gttcgcaggt cctttattca ctcgccagat ggaagtcagc     600
atccgcatgc atgccctgga atcggcgtgc ttcgtggtca actcgaccgc gtggttgtac     660
ccggaacagc aagcccagat catggccgac accggttgcg agatcggccc gatctccggc     720
ggctgctaca ccgcgatcat cgacccacag ggtgaagtcg tcggcgcact gaccgaaggc     780
gagggcgaag tgattgccga catcgatctg ttccagatcg aaatccgtaa acgtcagatg     840
gacggccgtg gtcactacag ccgtccggaa atcctgagcc tgaacatcga ccgtacgccg     900
catcgccatg ttcacgaacg caacgaccag cagaaaccgg gtgtgatcga cactgctgaa     960
gaaaccgggc gttga                                                      975

<210> 106
<211> 324
<212> PRT
<213> Unknown

<220>

<223> Obtained from an environmental sample

<400> 106
Met Thr Ile Val Lys Ala Ala Ala Val Gln Ile Ala Pro Val Leu Tyr
1               5                   10                  15
Ser Arg Glu Gly Thr Val Glu Lys Val Val Asn Lys Ile Arg Glu Leu
            20                  25                  30
Gly Glu Lys Gly Val Gln Phe Ala Val Phe Pro Glu Thr Val Val Pro
        35                  40                  45
Tyr Tyr Pro Tyr Phe Ser Phe Val Gln Ser Pro Phe Lys Met Gly Ser
    50                  55                  60
Glu His Tyr Lys Leu Leu Asp Gln Ala Val Val Val Pro Ser Ala Thr
65                  70                  75                  80
Thr Asp Ala Ile Gly Lys Ala Ala Lys Glu Ala Asn Met Val Val Ser
                85                  90                  95
Ile Gly Val Asn Glu Arg Asp Gly Ser Thr Leu Tyr Asn Thr Gln Leu
            100                 105                 110
Leu Phe Asp Ala Asp Gly Thr Leu Ile Gln Ala Arg Arg Lys Ile Ser
        115                 120                 125
Pro Thr Tyr His Glu Arg Met Ile Trp Gly Met Gly Asp Gly Ser Gly
    130                 135                 140
Leu Arg Ala Thr Asp Ser Ala Val Gly Arg Ile Gly Gln Leu Ala Cys
145                 150                 155                 160
Trp Glu His Tyr Asn Pro Leu Ala Arg Tyr Ala Leu Ile Glu Asp Gly
                165                 170                 175
Glu Gln Ile His Ala Ser Met Tyr Pro Gly Ser Phe Ala Gly Pro Leu
            180                 185                 190
Phe Thr Arg Gln Met Glu Val Ser Ile Arg Met His Ala Leu Glu Ser
        195                 200                 205
Ala Cys Phe Val Val Asn Ser Thr Ala Trp Leu Tyr Pro Glu Gln Gln
    210                 215                 220
Ala Gln Ile Met Ala Asp Thr Gly Cys Glu Ile Gly Pro Ile Ser Gly
225                 230                 235                 240
Gly Cys Tyr Thr Ala Ile Ile Asp Pro Gln Gly Glu Val Val Gly Ala
                245                 250                 255
Leu Thr Glu Gly Glu Gly Glu Val Ile Ala Asp Ile Asp Leu Phe Gln
            260                 265                 270
Ile Glu Ile Arg Lys Arg Gln Met Asp Gly Arg Gly His Tyr Ser Arg
        275                 280                 285
Pro Glu Ile Leu Ser Leu Asn Ile Asp Arg Thr Pro His Arg His Val
    290                 295                 300
His Glu Arg Asn Asp Gln Gln Lys Pro Gly Val Ile Asp Thr Ala Glu
305                 310                 315                 320
Glu Thr Gly Arg


<210> 107
<211> 981
<212> DNA
<213> Unknown

<220>
<223> Obtained from an environmental sample

<400> 107
atggccatca ttcgcgcagc agccgtacag atcagcccgg ttctttacag ccgcgaaggc      60
accgtggaca aggtctgcca gcagatcatc acccttggca aacagggtgt gcagttcgcc     120
gtgttcccgg aaacggtggt gccgtactac ccctattttt cctttgtgca gccggcgttc     180
gccatgggtg cgcaacacct caaattgcta gatcaatctg taaccgtgcc atcggccgcc     240
accctggcga ttggcgaagc gtgcaagcaa gcaggaatgg tcgtttccat cggagtcaat     300
gaacgcgatg gcggtacgat ttacaacgcg caattactct tcgatgctga cggcacgctg     360
attcagcatc ggcgcaaaat caccccgacc taccacgagc gcatggtctg ggggcagggc      420
gatggttccg gtctgcgcgc catcgacagc gcggtcggcc gcatcggctc cctggcatgc     480
tgggaacatt acaacccgct ggcccgttac gccttgatgg cagacggcga acagatccac     540
gccgcgatgt ttcccggttc cctggtgggt gacatcttcg ccgagcagat cgaggtcacc     600
atccgccatc acgcattgga gtcaggatgc ttcgtggtca atgcaacagc ctggctggat     660
gcggatcagc agggccaaat aatgcaggac acaggttgcg gccttggtcc catctcgggc     720
ggctgcttca ccgcgatcgt atcgccggaa gggaagctac ttggagagcc gcttcgctcc     780

ggggaaggcg tagtgattgc cgacctcgat acggccttga tcgacaagcg caaacggatg 840
atggattcag taggtcatta cagtcgtccc gagctgctca gcctattgat cgatcgatcg 900
ccgactgcgc atgttcatga acgcgccggc tttgtttcga gcaacgccgg tttgcaggag 960
gtcgcccatg cagaccaatg a 981

<210> 108
<211> 326
<212> PRT
<213> Unknown

<220>
<223> Obtained from an environmental sample

<400> 108
Met Ala Ile Ile Arg Ala Ala Ala Val Gln Ile Ser Pro Val Leu Tyr
1               5                   10                  15
Ser Arg Glu Gly Thr Val Asp Lys Val Cys Gln Gln Ile Ile Thr Leu
            20                  25                  30
Gly Lys Gln Gly Val Gln Phe Ala Val Phe Pro Glu Thr Val Val Pro
        35                  40                  45
Tyr Tyr Pro Tyr Phe Ser Phe Val Gln Pro Ala Phe Ala Met Gly Ala
    50                  55                  60
Gln His Leu Lys Leu Leu Asp Gln Ser Val Thr Val Pro Ser Ala Ala
65                  70                  75                  80
Thr Leu Ala Ile Gly Glu Ala Cys Lys Gln Ala Gly Met Val Val Ser
                85                  90                  95
Ile Gly Val Asn Glu Arg Asp Gly Gly Thr Ile Tyr Asn Ala Gln Leu
            100                 105                 110
Leu Phe Asp Ala Asp Gly Thr Leu Ile Gln His Arg Arg Lys Ile Thr
        115                 120                 125
Pro Thr Tyr His Glu Arg Met Val Trp Gly Gln Gly Asp Gly Ser Gly
    130                 135                 140
Leu Arg Ala Ile Asp Ser Ala Val Gly Arg Ile Gly Ser Leu Ala Cys
145                 150                 155                 160
Trp Glu His Tyr Asn Pro Leu Ala Arg Tyr Ala Leu Met Ala Asp Gly
                165                 170                 175
Glu Gln Ile His Ala Ala Met Phe Pro Gly Ser Leu Val Gly Asp Ile
            180                 185                 190
Phe Ala Glu Gln Ile Glu Val Thr Ile Arg His His Ala Leu Glu Ser
        195                 200                 205
Gly Cys Phe Val Val Asn Ala Thr Ala Trp Leu Asp Ala Asp Gln Gln
    210                 215                 220
Gly Gln Ile Met Gln Asp Thr Gly Cys Gly Leu Gly Pro Ile Ser Gly
225                 230                 235                 240
Gly Cys Phe Thr Ala Ile Val Ser Pro Glu Gly Lys Leu Leu Gly Glu
                245                 250                 255
Pro Leu Arg Ser Gly Glu Gly Val Val Ile Ala Asp Leu Asp Thr Ala
            260                 265                 270
Leu Ile Asp Lys Arg Lys Arg Met Met Asp Ser Val Gly His Tyr Ser
        275                 280                 285
Arg Pro Glu Leu Leu Ser Leu Leu Ile Asp Arg Ser Pro Thr Ala His
    290                 295                 300
Val His Glu Arg Ala Gly Phe Val Ser Ser Asn Ala Gly Leu Gln Glu
305                 310                 315                 320
Val Ala His Ala Asp Gln
                325

<210> 109
<211> 1092
<212> DNA
<213> Unknown

<220>
<223> Obtained from an environmental sample

<400> 109
atggccatca ttcgcgcagc agccgtacag atcagcccgg ttctttacag ccgcgaaggc 60
accgtggaca gggtctgcca gcagatcatc acccttggca aacaaggtgt gcagttcgcc 120

gtgttcccgg aaacggtggt gccgtactac ccctattttt cctttgtgca gccggcattt 180
gcgatgggtg cacaacacct caaattgctc gatcaatctg taaccgtgcc atcggccgcc 240
accctggcga ttggcgaagc gtgcaagcaa gcaggaatgg tcgtttccat cggcgtcaat 300
gaacgcgatg gcggtacgat ttacaacgcg caattactct tcgatgctga cggcactctg 360
attcagcatc ggcgcaaaat caccccgacc taccacgagc gcatggtctg ggggcagggc 420
gatggttccg gtctgcgcgc catcgacagc gcggtcgggc gcatcggctc cctggcatgc 480
tgggaacatt acaacccgct ggcccgttac gccttgatgg cagacggcga acagatccac 540
gccgcgatgt ttcccggttc cctggtgggt gacatcttcg ccgagcagat cgaggtcacc 600
atccgccatc acgcattgga atcaggatgc ttcgtggtca atgcaacagc ttggctggat 660
gcggatcagc agggccaaat aatgcaggac acaggttgcg gccttggtcc catctcgggc 720
ggctgcttca ccgcgatcgt atcgccggaa gggaagctac ttggagagcc gcttcgctca 780
ggggaaggcg tagtgattgc cgacctcgat atggccttga tcgacaagcg caaacggatg 840
atggattcag taggtcatta cagtcgtccc gagctgctca gcctattgat cgatcgatcg 900
ccgactgcgc attttcatga acgcgccggg ctttgttccg agcgacgccg gtttgcagga 960
ggtcgcgcat gcagaccaat gaattgctcg ctgacctgca aatccaaggc ctgcgttggc 1020
cggccgcgca aatggcttgt cgcgccaagg cggcgccggt ccttcagacc acaaggcgct 1080
gagcctaggt aa 1092

<210> 110
<211> 363
<212> PRT
<213> Unknown

<220>
<223> Obtained from an environmental sample

<400> 110
Met Ala Ile Ile Arg Ala Ala Ala Val Gln Ile Ser Pro Val Leu Tyr
1               5                   10                  15
Ser Arg Glu Gly Thr Val Asp Arg Val Cys Gln Gln Ile Ile Thr Leu
            20                  25                  30
Gly Lys Gln Gly Val Gln Phe Ala Val Phe Pro Glu Thr Val Val Pro
        35                  40                  45
Tyr Tyr Pro Tyr Phe Ser Phe Val Gln Pro Ala Phe Ala Met Gly Ala
    50                  55                  60
Gln His Leu Lys Leu Leu Asp Gln Ser Val Thr Val Pro Ser Ala Ala
65                  70                  75                  80
Thr Leu Ala Ile Gly Glu Ala Cys Lys Gln Ala Gly Met Val Val Ser
                85                  90                  95
Ile Gly Val Asn Glu Arg Asp Gly Gly Thr Ile Tyr Asn Ala Gln Leu
            100                 105                 110
Leu Phe Asp Ala Asp Gly Thr Leu Ile Gln His Arg Arg Lys Ile Thr
        115                 120                 125
Pro Thr Tyr His Glu Arg Met Val Trp Gly Gln Gly Asp Gly Ser Gly
    130                 135                 140
Leu Arg Ala Ile Asp Ser Ala Val Gly Arg Ile Gly Ser Leu Ala Cys
145                 150                 155                 160
Trp Glu His Tyr Asn Pro Leu Ala Arg Tyr Ala Leu Met Ala Asp Gly
                165                 170                 175
Glu Gln Ile His Ala Ala Met Phe Pro Gly Ser Leu Val Gly Asp Ile
            180                 185                 190
Phe Ala Glu Gln Ile Glu Val Thr Ile Arg His His Ala Leu Glu Ser
        195                 200                 205
Gly Cys Phe Val Val Asn Ala Thr Ala Trp Leu Asp Ala Asp Gln Gln
    210                 215                 220
Gly Gln Ile Met Gln Asp Thr Gly Cys Gly Leu Gly Pro Ile Ser Gly
225                 230                 235                 240
Gly Cys Phe Thr Ala Ile Val Ser Pro Glu Gly Lys Leu Leu Gly Glu
                245                 250                 255
Pro Leu Arg Ser Gly Glu Gly Val Val Ile Ala Asp Leu Asp Met Ala
            260                 265                 270
Leu Ile Asp Lys Arg Lys Arg Met Met Asp Ser Val Gly His Tyr Ser
        275                 280                 285
Arg Pro Glu Leu Leu Ser Leu Leu Ile Asp Arg Ser Pro Thr Ala His
    290                 295                 300
Phe His Glu Arg Ala Gly Leu Cys Ser Glu Arg Arg Arg Phe Ala Gly
305                 310                 315                 320
Gly Arg Ala Cys Arg Pro Met Asn Cys Ser Leu Thr Cys Lys Ser Lys

325                 330                 335
Ala Cys Val Gly Arg Pro Arg Lys Trp Leu Val Ala Pro Arg Arg Arg
            340                 345                 350
Arg Ser Phe Arg Pro Gln Gly Ala Glu Pro Arg
        355                 360

<210> 111
<211> 990
<212> DNA
<213> Unknown

<220>
<223> Obtained from an environmental sample

<400> 111
atgcccaaaa cagtacgtgc cgcagcagtc cagatcgcgc ccgacctgac gtcacgcgcc      60
ggcaccgtcg agcgggtcct caatgcaatc gccgaagctg ctgacaaagg cgccgagctg     120
atcgtatttc ccgagacctt cgtgccctgg tatccctatt tcagtttcgt tctgccacct     180
gtccagcaag gccctgagca tcttcgtctt tatgaggaag cagtcacggt accatcagca     240
gaaacacggg ccgtcgcgga cgccgcgcgc aaacgcaatg cggttatcgt ccttggcgtc     300
aatgagcgcg accacggctc gctctataac actcagctga tcttcgacgc ggatggcagc     360
ctgaaactca agcgtcgcaa gatcacgccg acctatcacg aacggatgat ctggggccaa     420
ggcgatggcg ccggcctgaa ggttgtcgac actgccgtcg gtcgcgtggg tgccctggca     480
tgctgggagc attacaatcc tctggcccgc tatactttga tggcccagca tgaggaaatt     540
cacgcctctc atttcccggg ctcactggtc ggcccgatat tcggcgagca aatcgaagtc     600
accatgcgcc accacgcgtt ggaatcgggc tgtttcgtgg tcaatgccac cggctggctg     660
agcgaggagc agatcgcatc tattcatccg gaccccgcct tgcaaaaggg cctgcgcgat     720
ggctgcatga cctgcatcat cacgccggaa ggacgccatg tcgtaccgcc gctgacctcg     780
ggcgaaggca tcctgatcgg cgatctggac atgcggctca ttaccaagcg caagcggatg     840
atggattcgg tcggacacta tgctcggcct gaactgctgc accttgtcca tgacacgacg     900
cccgcacgcg cacgcgagca ggtcggcctt tcaggcgatt ttcccgatgc ggagcaagac     960
aagctatttg aggaggttca taatgcgtga                                      990

<210> 112
<211> 329
<212> PRT
<213> Unknown

<220>
<223> Obtained from an environmental sample

<400> 112
Met Pro Lys Thr Val Arg Ala Ala Ala Val Gln Ile Ala Pro Asp Leu
1               5                   10                  15
Thr Ser Arg Ala Gly Thr Val Glu Arg Val Leu Asn Ala Ile Ala Glu
            20                  25                  30
Ala Ala Asp Lys Gly Ala Glu Leu Ile Val Phe Pro Glu Thr Phe Val
        35                  40                  45
Pro Trp Tyr Pro Tyr Phe Ser Phe Val Leu Pro Pro Val Gln Gln Gly
    50                  55                  60
Pro Glu His Leu Arg Leu Tyr Glu Glu Ala Val Thr Val Pro Ser Ala
65                  70                  75                  80
Glu Thr Arg Ala Val Ala Asp Ala Ala Arg Lys Arg Asn Ala Val Ile
                85                  90                  95
Val Leu Gly Val Asn Glu Arg Asp His Gly Ser Leu Tyr Asn Thr Gln
            100                 105                 110
Leu Ile Phe Asp Ala Asp Gly Ser Leu Lys Leu Lys Arg Arg Lys Ile
        115                 120                 125
Thr Pro Thr Tyr His Glu Arg Met Ile Trp Gly Gln Gly Asp Gly Ala
    130                 135                 140
Gly Leu Lys Val Val Asp Thr Ala Val Gly Arg Val Gly Ala Leu Ala
145                 150                 155                 160
Cys Trp Glu His Tyr Asn Pro Leu Ala Arg Tyr Thr Leu Met Ala Gln
                165                 170                 175
His Glu Glu Ile His Ala Ser His Phe Pro Gly Ser Leu Val Gly Pro
            180                 185                 190
Ile Phe Gly Glu Gln Ile Glu Val Thr Met Arg His His Ala Leu Glu
        195                 200                 205

Ser Gly Cys Phe Val Val Asn Ala Thr Gly Trp Leu Ser Glu Glu Gln
    210                 215                 220
Ile Ala Ser Ile His Pro Asp Pro Ala Leu Gln Lys Gly Leu Arg Asp
225                 230                 235                 240
Gly Cys Met Thr Cys Ile Ile Thr Pro Glu Gly Arg His Val Val Pro
                245                 250                 255
Pro Leu Thr Ser Gly Glu Gly Ile Leu Ile Gly Asp Leu Asp Met Arg
            260                 265                 270
Leu Ile Thr Lys Arg Lys Arg Met Met Asp Ser Val Gly His Tyr Ala
        275                 280                 285
Arg Pro Glu Leu Leu His Leu Val His Asp Thr Thr Pro Ala Arg Ala
    290                 295                 300
Arg Glu Gln Val Gly Leu Ser Gly Asp Phe Pro Asp Ala Glu Gln Asp
305                 310                 315                 320
Lys Leu Phe Glu Glu Val His Asn Ala
                325

<210> 113
<211> 993
<212> DNA
<213> Unknown

<220>
<223> Obtained from an environmental sample

<400> 113
atgacgaagg aacgcgccgc gcgcagcctg cgcgcagctg ccatacagct tgaagccgaa      60
gtcggcgaca tcgccgccaa tctcgcacgc atcgaggcga tggtcgagga ggctgcgggc     120
aagggcgccg aactgatcgc cattccggag ttctgcacct cccgcatgcc cttcgatgca     180
cgcgtgcacg acgccgtgct gccgccggac aacttcgtgg tcgatgcctt tcgccgcatg     240
gcagcgacgc acaactgccg gctcggcggc tccatgctca ttgccgacgg tggcgagatc     300
tacaaccgct accacttcgt cgaacccgac ggcagcgtgc atctgcacga caaggatctg     360
ccgacgatgt gggagaacgc cttctacacc ggcggctccg acgacggcgt cttcgacacc     420
ggcatcggcg gcgtcggcgc cgcggtgtgc tgggaactgg tacgcaccgg caccgtgcga     480
cgcatgctcg gtcgcgtcga cgtcgccatg accggcacgc attggtggac gatgccgcac     540
aactggggca gcgccgtcgc gcgcacgctg gccgcgatga cgcagtacaa ccgctacatg     600
tccgagaatg cacccaccga attcgcccgc cgcctgggtg tgccggtgct gcaggcctcg     660
cactgcggaa gcttccgcac cggtttcttg ctgctgccag gcagcgggcg tgcactgccc     720
tatgacaccg agtacgtcgg cgccacacag atcgtcgatg ccgatggcca catcctcgcc     780
caccgtcgca cgcaggaagg ccccggtgtc gtcgtcgccg acatcacgct cggtgcccgc     840
acgcccgagc tgccactgga agaccgcttc tggattcccg agctgccgct cttcctcaag     900
gcctactggc accaccagaa cctgtgcggc aagtcctact accgtcgcgt cggccgcgat     960
gccggcctgg cggcggcgga gcgttcggca tga                                  993

<210> 114
<211> 330
<212> PRT
<213> Unknown

<220>
<223> Obtained from an environmental sample

<400> 114
Met Thr Lys Glu Arg Ala Ala Arg Ser Leu Arg Ala Ala Ala Ile Gln
1               5                   10                  15
Leu Glu Ala Glu Val Gly Asp Ile Ala Ala Asn Leu Ala Arg Ile Glu
            20                  25                  30
Ala Met Val Glu Glu Ala Ala Gly Lys Gly Ala Glu Leu Ile Ala Ile
        35                  40                  45
Pro Glu Phe Cys Thr Ser Arg Met Pro Phe Asp Ala Arg Val His Asp
    50                  55                  60
Ala Val Leu Pro Pro Asp Asn Phe Val Val Asp Ala Phe Arg Arg Met
65                  70                  75                  80
Ala Ala Thr His Asn Cys Arg Leu Gly Gly Ser Met Leu Ile Ala Asp
                85                  90                  95
Gly Gly Glu Ile Tyr Asn Arg Tyr His Phe Val Glu Pro Asp Gly Ser
            100                 105                 110
Val His Leu His Asp Lys Asp Leu Pro Thr Met Trp Glu Asn Ala Phe

115                 120                 125
Tyr Thr Gly Gly Ser Asp Asp Gly Val Phe Asp Thr Gly Ile Gly Gly
    130                 135                 140
Val Gly Ala Ala Val Cys Trp Glu Leu Val Arg Thr Gly Thr Val Arg
145                 150                 155                 160
Arg Met Leu Gly Arg Val Asp Val Ala Met Thr Gly Thr His Trp Trp
                165                 170                 175
Thr Met Pro His Asn Trp Gly Ser Ala Val Ala Arg Thr Leu Ala Ala
            180                 185                 190
Met Thr Gln Tyr Asn Arg Tyr Met Ser Glu Asn Ala Pro Thr Glu Phe
        195                 200                 205
Ala Arg Arg Leu Gly Val Pro Val Leu Gln Ala Ser His Cys Gly Ser
    210                 215                 220
Phe Arg Thr Gly Phe Leu Leu Leu Pro Gly Ser Gly Arg Ala Leu Pro
225                 230                 235                 240
Tyr Asp Thr Glu Tyr Val Gly Ala Thr Gln Ile Val Asp Ala Asp Gly
                245                 250                 255
His Ile Leu Ala His Arg Arg Thr Gln Glu Gly Pro Gly Val Val Val
            260                 265                 270
Ala Asp Ile Thr Leu Gly Ala Arg Thr Pro Glu Leu Pro Leu Glu Asp
        275                 280                 285
Arg Phe Trp Ile Pro Glu Leu Pro Leu Phe Leu Lys Ala Tyr Trp His
    290                 295                 300
His Gln Asn Leu Cys Gly Lys Ser Tyr Tyr Arg Arg Val Gly Arg Asp
305                 310                 315                 320
Ala Gly Leu Ala Ala Ala Glu Arg Ser Ala
                325                 330

<210> 115
<211> 993
<212> DNA
<213> Unknown

<220>
<223> Obtained from an environmental sample

<400> 115
atgaaccaaa tcattaaagc ggcggcagtt caatgtagcc ctgtgttgta tagccaagcg      60
ggtacagtca agaaaatctg tgacacgatt ttggagttgg ggcagcaagg tgtgcaattt     120
gccgtatttc ctgaaactgt tgtgccttat tacccttatt tttcttttgt gcaaccaccg     180
tttgccatgg gtaaagaaca tttaaagcta ttgcatgaat cggttgtcgt gccatcggca     240
gcaacaactt taattggaca ggcatgcaaa gaagcgaaca tggtggtttc tattggtatt     300
aatgagcgtg caggcggcac gatttataac gctcaattgt tgtttgatgc ggatggttcg     360
attattcagc atcgccgtaa aattacccca acgtatcatg aacgtatggt gtgggggcaa     420
ggcgatggca gtggtttacg tgcgatagat tctgctgtag gacgtattgg gtcgctggca     480
tgttgggagc attacaaccc tttggctcgg tttgctttga tggcggatgg tgagcaaatt     540
catgcggcga tgtttccggg atcactcgtg gggcagattt ttgcagatca gatcagtgcc     600
accattcagc accatgcttt agagtcgggc tgttttgtgg tgaatgccac agcatggctt     660
gacccagagc aacaacaaca aattatgcaa gatacaggct gtgaactcgg tccaatttcg     720
gggggatgtt ttacggccat cgtttctcca gaaggcaaat ttttgtctga accgatcaca     780
caaggcgaag gttatgtgat tgccgattta gacttttcct taatcgaaaa acgtaaacgg     840
atgatggatt ctgttgggca ttatagtcgt ccagaattac tcagtttgtt gattgatcgt     900
cgtcctacct cagttttgca tgagttaaaa ctagagaatc catcgaataa cagcatcgaa     960
aaagtgtctg aatttgccga ggtacacgca tag                                  993

<210> 116
<211> 330
<212> PRT
<213> Unknown

<220>
<223> Obtained from an environmental sample

<400> 116
Met Asn Gln Ile Ile Lys Ala Ala Ala Val Gln Cys Ser Pro Val Leu
1               5                   10                  15
Tyr Ser Gln Ala Gly Thr Val Lys Lys Ile Cys Asp Thr Ile Leu Glu
            20                  25                  30

Leu Gly Gln Gln Gly Val Gln Phe Ala Val Phe Pro Glu Thr Val Val
        35                  40                  45
Pro Tyr Tyr Pro Tyr Phe Ser Phe Val Gln Pro Pro Phe Ala Met Gly
    50                  55                  60
Lys Glu His Leu Lys Leu Leu His Glu Ser Val Val Val Pro Ser Ala
65                  70                  75                  80
Ala Thr Thr Leu Ile Gly Gln Ala Cys Lys Glu Ala Asn Met Val Val
                85                  90                  95
Ser Ile Gly Ile Asn Glu Arg Ala Gly Gly Thr Ile Tyr Asn Ala Gln
            100                 105                 110
Leu Leu Phe Asp Ala Asp Gly Ser Ile Ile Gln His Arg Arg Lys Ile
        115                 120                 125
Thr Pro Thr Tyr His Glu Arg Met Val Trp Gly Gln Gly Asp Gly Ser
    130                 135                 140
Gly Leu Arg Ala Ile Asp Ser Ala Val Gly Arg Ile Gly Ser Leu Ala
145                 150                 155                 160
Cys Trp Glu His Tyr Asn Pro Leu Ala Arg Phe Ala Leu Met Ala Asp
                165                 170                 175
Gly Glu Gln Ile His Ala Ala Met Phe Pro Gly Ser Leu Val Gly Gln
            180                 185                 190
Ile Phe Ala Asp Gln Ile Ser Ala Thr Ile Gln His His Ala Leu Glu
        195                 200                 205
Ser Gly Cys Phe Val Val Asn Ala Thr Ala Trp Leu Asp Pro Glu Gln
    210                 215                 220
Gln Gln Gln Ile Met Gln Asp Thr Gly Cys Glu Leu Gly Pro Ile Ser
225                 230                 235                 240
Gly Gly Cys Phe Thr Ala Ile Val Ser Pro Glu Gly Lys Phe Leu Ser
                245                 250                 255
Glu Pro Ile Thr Gln Gly Glu Gly Tyr Val Ile Ala Asp Leu Asp Phe
            260                 265                 270
Ser Leu Ile Glu Lys Arg Lys Arg Met Met Asp Ser Val Gly His Tyr
        275                 280                 285
Ser Arg Pro Glu Leu Leu Ser Leu Leu Ile Asp Arg Arg Pro Thr Ser
    290                 295                 300
Val Leu His Glu Leu Lys Leu Glu Asn Pro Ser Asn Asn Ser Ile Glu
305                 310                 315                 320
Lys Val Ser Glu Phe Ala Glu Val His Ala
                325                 330

<210> 117
<211> 957
<212> DNA
<213> Unknown

<220>
<223> Obtained from an environmental sample

<400> 117
atgactcaat ccaggataat tcgtgctgcg gcagcgcaga tcgctccgga tttgcaggtt      60
ccaggtaaca cgatcgacaa agtttgccgc accatcagcg aggcggccgc aaaaggcgta     120
cagattattg ttttccctga aaccttggtg ccttattacc cttacttctc ttacatttca     180
ccgcccattc aacagggcaa agaacatttg cggctgtatg accatgcagt ggttgtgccc     240
ggctcggaaa ccgaggcaat ttcagctctt gccgcccaac acaatatggt ggtggttttg     300
ggtgtgaacg agcgcgatca cggcacactt tacaacgcac aaattatttt caacagcgac     360
ggaaagattc tgttgaagcg ccgaaaaatt acaccaactt atcacgagcg gatggtgtgg     420
gggcagggtg acgcttcagg cttgaaggtg gttgattccg cagtgggccg tgtgggtgca     480
ttggcctgtt gggaacacta caaccccttg gctcgctatt gtttgatggc ccagcacgaa     540
gaaattcact gtgcgcagtt tcccggttca ttggtggggc aagtttttgc cgaccaaatg      600
gaagtgacca ttcgtcacca cgcacttgag tcgggctgtt ttgtcatcaa cagcaccgct     660
tggctttctg aagaacaggt tcaaagtatt tcatccgaca gcgcattgca gaaaggcctt     720
agaggcggtt gtttcacggc cattgtcagc cctgagggaa agctgttggc tgagccgctc     780
accgagggtg agggcatggt gatcgccgac ctcgacatgg cgttggttac gaaacgcaaa     840
cgcatgatgg attcagtggg ccattatgcg cgccccgagt tgttgagttt gctggttcgg     900
gatgaggctt caagccccat gaaaaaaatt cagggagttc aacatgctga gtactga        957

<210> 118
<211> 318
<212> PRT

<213> Unknown

<220>
<223> Obtained from an environmental sample

<400> 118
   Met Thr Gln Ser Arg Ile Ile Arg Ala Ala Ala Ala Gln Ile Ala Pro
   1               5                   10                  15
   Asp Leu Gln Val Pro Gly Asn Thr Ile Asp Lys Val Cys Arg Thr Ile
               20                  25                  30
   Ser Glu Ala Ala Ala Lys Gly Val Gln Ile Ile Val Phe Pro Glu Thr
           35                  40                  45
   Leu Val Pro Tyr Tyr Pro Tyr Phe Ser Tyr Ile Ser Pro Pro Ile Gln
       50                  55                  60
   Gln Gly Lys Glu His Leu Arg Leu Tyr Asp His Ala Val Val Val Pro
   65                  70                  75                  80
   Gly Ser Glu Thr Glu Ala Ile Ser Ala Leu Ala Ala Gln His Asn Met
                   85                  90                  95
   Val Val Val Leu Gly Val Asn Glu Arg Asp His Gly Thr Leu Tyr Asn
               100                 105                 110
   Ala Gln Ile Ile Phe Asn Ser Asp Gly Lys Ile Leu Leu Lys Arg Arg
           115                 120                 125
   Lys Ile Thr Pro Thr Tyr His Glu Arg Met Val Trp Gly Gln Gly Asp
       130                 135                 140
   Ala Ser Gly Leu Lys Val Val Asp Ser Ala Val Gly Arg Val Gly Ala
   145                 150                 155                 160
   Leu Ala Cys Trp Glu His Tyr Asn Pro Leu Ala Arg Tyr Cys Leu Met
                   165                 170                 175
   Ala Gln His Glu Glu Ile His Cys Ala Gln Phe Pro Gly Ser Leu Val
               180                 185                 190
   Gly Gln Val Phe Ala Asp Gln Met Glu Val Thr Ile Arg His His Ala
           195                 200                 205
   Leu Glu Ser Gly Cys Phe Val Ile Asn Ser Thr Ala Trp Leu Ser Glu
       210                 215                 220
   Glu Gln Val Gln Ser Ile Ser Ser Asp Ser Ala Leu Gln Lys Gly Leu
   225                 230                 235                 240
   Arg Gly Gly Cys Phe Thr Ala Ile Val Ser Pro Glu Gly Lys Leu Leu
                   245                 250                 255
   Ala Glu Pro Leu Thr Glu Gly Glu Gly Met Val Ile Ala Asp Leu Asp
               260                 265                 270
   Met Ala Leu Val Thr Lys Arg Lys Arg Met Met Asp Ser Val Gly His
           275                 280                 285
   Tyr Ala Arg Pro Glu Leu Leu Ser Leu Leu Val Arg Asp Glu Ala Ser
       290                 295                 300
   Ser Pro Met Lys Lys Ile Gln Gly Val Gln His Ala Glu Tyr
   305                 310                 315

<210> 119
<211> 984
<212> DNA
<213> Unknown

<220>
<223> Obtained from an environmental sample

<400> 119
atggatacac tcaaagttgg attggttcag atggccccca tctggttgaa ccgggataaa      60
accctgatca aagttgagga atacatgcag aaagcaggca aacagggctg caacctggta     120
gcttttggtg aagcgctggt tcccggctac cccttctggg tggaacgcac agagggcgcc     180
agattcaatt ccaaagtcca gaaagaactc tttgcacatt accttgatca ggcggtgcag     240
atcgaagccg gccaccttga tcctctccag gcattagccc aacaatacaa gatggctgtg     300
tacgtgggga cgattgaacg cccgcctgag cggagcggcc acagcctgta ctgctcccta     360
atatttatag acccagaagg cgagatcggc tcggttcacc gcaagttgat gcccacccat     420
gaggaacgcc tggtctggtc aactggcgat gggcacggcc tgcgaacaca ttctctgggc     480
gcctttaccg ttggcggact caactgctgg gaaaactgga tgccgctctc ccgcacagct     540
ctttatgcca tgggagagga tcttcatgtt gctgcctggc ccgggagtca gcgcaatact     600
tatgatataa ccaaattcat tgccaaggaa tctcgctctt atgtgatctc cgtatccggg     660
atgatgaaaa aagaaaatat cctctctgaa attccccaca gccaattgat gctggaaaat     720

agcgaggata ttatggctga tggcggatcc tgtctggctg gaccagatgg agaatggatc 780
atcgagccca tcgtcggaga ggaaaccctg gtaactgctg aactatcaca tcagcgggtc 840
agagaagaaa gacagaattt cgacccaaca ggtcactaca gtcggcctga tgtgacccgc 900
ctggtagtcg accgcaggcg ccagcagatc ctggagatca ccccggacga aaaaggaaga 960
tcggatgaaa atcaatccct ttaa 984

<210> 120
<211> 327
<212> PRT
<213> Unknown

<220>
<223> Obtained from an environmental sample

<400> 120
Met Asp Thr Leu Lys Val Gly Leu Val Gln Met Ala Pro Ile Trp Leu
1               5                   10                  15
Asn Arg Asp Lys Thr Leu Ile Lys Val Glu Glu Tyr Met Gln Lys Ala
            20                  25                  30
Gly Lys Gln Gly Cys Asn Leu Val Ala Phe Gly Glu Ala Leu Val Pro
        35                  40                  45
Gly Tyr Pro Phe Trp Val Glu Arg Thr Glu Gly Ala Arg Phe Asn Ser
    50                  55                  60
Lys Val Gln Lys Glu Leu Phe Ala His Tyr Leu Asp Gln Ala Val Gln
65                  70                  75                  80
Ile Glu Ala Gly His Leu Asp Pro Leu Gln Ala Leu Ala Gln Gln Tyr
                85                  90                  95
Lys Met Ala Val Tyr Val Gly Thr Ile Glu Arg Pro Pro Glu Arg Ser
            100                 105                 110
Gly His Ser Leu Tyr Cys Ser Leu Ile Phe Ile Asp Pro Glu Gly Glu
        115                 120                 125
Ile Gly Ser Val His Arg Lys Leu Met Pro Thr His Glu Glu Arg Leu
    130                 135                 140
Val Trp Ser Thr Gly Asp Gly His Gly Leu Arg Thr His Ser Leu Gly
145                 150                 155                 160
Ala Phe Thr Val Gly Gly Leu Asn Cys Trp Glu Asn Trp Met Pro Leu
                165                 170                 175
Ser Arg Thr Ala Leu Tyr Ala Met Gly Glu Asp Leu His Val Ala Ala
            180                 185                 190
Trp Pro Gly Ser Gln Arg Asn Thr Tyr Asp Ile Thr Lys Phe Ile Ala
        195                 200                 205
Lys Glu Ser Arg Ser Tyr Val Ile Ser Val Ser Gly Met Met Lys Lys
    210                 215                 220
Glu Asn Ile Leu Ser Glu Ile Pro His Ser Gln Leu Met Leu Glu Asn
225                 230                 235                 240
Ser Glu Asp Ile Met Ala Asp Gly Gly Ser Cys Leu Ala Gly Pro Asp
                245                 250                 255
Gly Glu Trp Ile Ile Glu Pro Ile Val Gly Glu Glu Thr Leu Val Thr
            260                 265                 270
Ala Glu Leu Ser His Gln Arg Val Arg Glu Glu Arg Gln Asn Phe Asp
        275                 280                 285
Pro Thr Gly His Tyr Ser Arg Pro Asp Val Thr Arg Leu Val Val Asp
    290                 295                 300
Arg Arg Arg Gln Gln Ile Leu Glu Ile Thr Pro Asp Glu Lys Gly Arg
305                 310                 315                 320
Ser Asp Glu Asn Gln Ser Leu
                325

<210> 121
<211> 1158
<212> DNA
<213> Unknown

<220>
<223> Obtained from an environmental sample

<400> 121
atgagcaaaa aagttctagg cggcagagaa aaagtaaaag ttgcagtagt tcaggctgcg 60

cccgttttca tggacaagga gaagacgatt gaaaaggctt gcaagctaat aaaagaagcg     120
gggagaaatc gagccgagct catagcgttc tcagagtcat tcatccccgt ctatcctgca     180
tactataccg tcggctatga aaccccttct caagaatgga gagattacgt gattgcgcta     240
caggataact ccgtgctgat tccgagcgag gataccgagg tactcggaca ggctgcaaag     300
gaggcagggg cttatgcagt aataggatgc agcgagatgg acgaccgtcc gggaagccga     360
acagtttaca acacgctcct cttcatcggc aaagacggca aggtcatggg aaggcataga     420
aaactcaaac ccacgttcac ggagagaata tactggggag agggagatgc tggagacata     480
aaggtttttg ataccgagat cggcaggatc ggaggcctcg tatgctggga gaaccatatg     540
actctagtca gggccgcgat gatacacagg ggagaggagt ttcatatcgc ggtctggccg     600
ggaaactgga agggtgcgga aaacaagctt ctccaagcag ataatagccc aggaggcgcc     660
ctctgcaacc ttcaatctct cattaaagta cacgcctttg aggccggggc gtttgtgctg     720
agcgcttgcg gctttttgac gccagaggat ttcccggaaa ggtggcatta tataagggat     780
ggtaaccata ttaactgcga ctgggcactg ggcggaagct caatcgtcaa tcccgccggc     840
cgttatctcg tcgagcctaa ctttgagaag gatgcaatcc tctatgcgga ttgttatgca     900
aaccagataa aagcagtaaa agcggttttt gattcccttg gccactattc ccgctgggat     960
attgcccaac tggcgataag gcaggaagcc tggaatccag aggtttcttt gatcgattcc    1020
tcttcgactg aagttgagct tccggcagac gagcttcgaa ggatttcgga gaagtttgaa    1080
gtaactgcgg ataagttgga atctttgctt gaggaaattg gaaagattaa aaagcccagg    1140
aaacaagccg gttcctaa                                                  1158

<210> 122
<211> 385
<212> PRT
<213> Unknown

<220>
<223> Obtained from an environmental sample

<400> 122
Met Ser Lys Lys Val Leu Gly Gly Arg Glu Lys Val Lys Val Ala Val
1               5                   10                  15
Val Gln Ala Ala Pro Val Phe Met Asp Lys Glu Lys Thr Ile Glu Lys
            20                  25                  30
Ala Cys Lys Leu Ile Lys Glu Ala Gly Arg Asn Arg Ala Glu Leu Ile
        35                  40                  45
Ala Phe Ser Glu Ser Phe Ile Pro Val Tyr Pro Ala Tyr Tyr Thr Val
    50                  55                  60
Gly Tyr Glu Thr Pro Ser Gln Glu Trp Arg Asp Tyr Val Ile Ala Leu
65                  70                  75                  80
Gln Asp Asn Ser Val Leu Ile Pro Ser Glu Asp Thr Glu Val Leu Gly
                85                  90                  95
Gln Ala Ala Lys Glu Ala Gly Ala Tyr Ala Val Ile Gly Cys Ser Glu
            100                 105                 110
Met Asp Asp Arg Pro Gly Ser Arg Thr Val Tyr Asn Thr Leu Leu Phe
        115                 120                 125
Ile Gly Lys Asp Gly Lys Val Met Gly Arg His Arg Lys Leu Lys Pro
    130                 135                 140
Thr Phe Thr Glu Arg Ile Tyr Trp Gly Glu Gly Asp Ala Gly Asp Ile
145                 150                 155                 160
Lys Val Phe Asp Thr Glu Ile Gly Arg Ile Gly Gly Leu Val Cys Trp
                165                 170                 175
Glu Asn His Met Thr Leu Val Arg Ala Ala Met Ile His Arg Gly Glu
            180                 185                 190
Glu Phe His Ile Ala Val Trp Pro Gly Asn Trp Lys Gly Ala Glu Asn
        195                 200                 205
Lys Leu Leu Gln Ala Asp Asn Ser Pro Gly Gly Ala Leu Cys Asn Leu
    210                 215                 220
Gln Ser Leu Ile Lys Val His Ala Phe Glu Ala Gly Ala Phe Val Leu
225                 230                 235                 240
Ser Ala Cys Gly Phe Leu Thr Pro Glu Asp Phe Pro Glu Arg Trp His
                245                 250                 255
Tyr Ile Arg Asp Gly Asn His Ile Asn Cys Asp Trp Ala Leu Gly Gly
            260                 265                 270
Ser Ser Ile Val Asn Pro Ala Gly Arg Tyr Leu Val Glu Pro Asn Phe
        275                 280                 285
Glu Lys Asp Ala Ile Leu Tyr Ala Asp Cys Tyr Ala Asn Gln Ile Lys
    290                 295                 300
Ala Val Lys Ala Val Phe Asp Ser Leu Gly His Tyr Ser Arg Trp Asp

305                 310                 315                 320
Ile Ala Gln Leu Ala Ile Arg Gln Glu Ala Trp Asn Pro Glu Val Ser
                325                 330                 335
Leu Ile Asp Ser Ser Ser Thr Glu Val Glu Leu Pro Ala Asp Glu Leu
            340                 345                 350
Arg Arg Ile Ser Glu Lys Phe Glu Val Thr Ala Asp Lys Leu Glu Ser
        355                 360                 365
Leu Leu Glu Glu Ile Gly Lys Ile Lys Lys Pro Arg Lys Gln Ala Gly
    370                 375                 380
Ser
385

<210> 123
<211> 990
<212> DNA
<213> Unknown

<220>
<223> Obtained from an environmental sample

<400> 123
atgtcaactt tcaagatcgc taccgtgcag agtgcaccag tatttatgga ccgcgaagct      60
accattgaca agacttgcga gctgatcgcc gaagcagcac aagatgacga cgttcgccta     120
gtggtcttcc ccgaggcctt tatccccacc tatccggact gggtatggcg tatccctccc     180
ggacagcacc agatgcttgc cgacctgtac ggggagttgc tcgagcagtc ggtgacgata     240
cccagtctgg ctaccgagcg gctctgtcag gctgcaaaga aagcgggcgt ttatgtagct     300
gtgggcctta acgaacgcaa tacagaggcc agcaacgcta ccctgtacaa caccctgctc     360
tacattgacg ccgagggcaa cttgctaggt aagcaccgaa agctggtacc gaccgctccc     420
gaacgcatgg tctgggcaca gggagatggc agtacccttg aggtctacga gacctccttc     480
ggaaaactca gcggactaat ctgttgggag aactacatgc ctctcgctcg ttatgccctg     540
tatgcctggg gagtacagct ctatttggct cctacttggg atcgaggcga gccctggctt     600
tccactctgc ggcacattgc caaggaagga cgagtatacg tggtcggctg ctctatcgcc     660
ttacgtaagg aagacatccc cgaccgattc gaattcaagg cgaagtacta cgcagaggca     720
ggagagtgga taaacaaagg tgacagcgtc atcgtcggtc ccgatggcga gctcatcgcc     780
gggcctctac ataaggaaca ggggatactc tatgctgagc tggacacaag gcagatgcac     840
gcccccaagt ggaacctgga tgtagccgga cactacgcgc gcccggacgt gtttcggctg     900
accgtgagca aggatggcca tccgatgctc ggcgttgccc aagggcccaa gcatgagccg     960
caagataaga ccgaagtatt agagggctag                                      990

<210> 124
<211> 329
<212> PRT
<213> Unknown

<220>
<223> Obtained from an environmental sample

<400> 124
Met Ser Thr Phe Lys Ile Ala Thr Val Gln Ser Ala Pro Val Phe Met
1               5                   10                  15
Asp Arg Glu Ala Thr Ile Asp Lys Thr Cys Glu Leu Ile Ala Glu Ala
            20                  25                  30
Ala Gln Asp Asp Asp Val Arg Leu Val Val Phe Pro Glu Ala Phe Ile
        35                  40                  45
Pro Thr Tyr Pro Asp Trp Val Trp Arg Ile Pro Pro Gly Gln His Gln
    50                  55                  60
Met Leu Ala Asp Leu Tyr Gly Glu Leu Leu Glu Gln Ser Val Thr Ile
65                  70                  75                  80
Pro Ser Leu Ala Thr Glu Arg Leu Cys Gln Ala Ala Lys Lys Ala Gly
                85                  90                  95
Val Tyr Val Ala Val Gly Leu Asn Glu Arg Asn Thr Glu Ala Ser Asn
            100                 105                 110
Ala Thr Leu Tyr Asn Thr Leu Leu Tyr Ile Asp Ala Glu Gly Asn Leu
        115                 120                 125
Leu Gly Lys His Arg Lys Leu Val Pro Thr Ala Pro Glu Arg Met Val
    130                 135                 140
Trp Ala Gln Gly Asp Gly Ser Thr Leu Glu Val Tyr Glu Thr Ser Phe
145                 150                 155                 160

Gly Lys Leu Ser Gly Leu Ile Cys Trp Glu Asn Tyr Met Pro Leu Ala
                165                 170                 175
Arg Tyr Ala Leu Tyr Ala Trp Gly Val Gln Leu Tyr Leu Ala Pro Thr
            180                 185                 190
Trp Asp Arg Gly Glu Pro Trp Leu Ser Thr Leu Arg His Ile Ala Lys
        195                 200                 205
Glu Gly Arg Val Tyr Val Val Gly Cys Ser Ile Ala Leu Arg Lys Glu
    210                 215                 220
Asp Ile Pro Asp Arg Phe Glu Phe Lys Ala Lys Tyr Tyr Ala Glu Ala
225                 230                 235                 240
Gly Glu Trp Ile Asn Lys Gly Asp Ser Val Ile Val Gly Pro Asp Gly
                245                 250                 255
Glu Leu Ile Ala Gly Pro Leu His Lys Glu Gln Gly Ile Leu Tyr Ala
            260                 265                 270
Glu Leu Asp Thr Arg Gln Met His Ala Pro Lys Trp Asn Leu Asp Val
        275                 280                 285
Ala Gly His Tyr Ala Arg Pro Asp Val Phe Arg Leu Thr Val Ser Lys
    290                 295                 300
Asp Gly His Pro Met Leu Gly Val Ala Gln Gly Pro Lys His Glu Pro
305                 310                 315                 320
Gln Asp Lys Thr Glu Val Leu Glu Gly
                325

<210> 125
<211> 1050
<212> DNA
<213> Unknown

<220>
<223> Obtained from an environmental sample

<400> 125
atgacaactg taaaaaagac ggtacgcgca gcagcgatcc agatcgcacc tgacctcgac      60
agtgcaggcg gtacgctgga caaggtttgc acggccattc aaaaggcggc ggcacaaggc     120
gcggagctgg tggtttttcc cgaaaccttc ttgccctact atccttactt ttcattcgtg     180
cggccgccct tcgcatccgg cccggaacac ttgctgctat atgaacgcgc agtggcggtg     240
ccaggcccgg tgaccgatgc cgtctctgcc gtcgcgcgca gccacggcgt ggtggtggta     300
ctcggcgtca atgaacgcga ccatggcacg ctgtacaaca cccaactggt gttcgacgcg     360
aatggcgaac tggtgttgaa acgcagaaaa atcacgccga cttatcacga gcggatgatc     420
tggggtcaag gcgacggcag cggactcaaa gtagtgcaaa cggcggtcgg ccggctaggc     480
gcgctagcct gttgggaaca ctacaaccca ctggcccgtt atgcattgat ggcgcaacac     540
gaagaaatcc attgcgccca gtttcccggg tccatggtcg ggcaaatatt cgccgaccag     600
atggaagtga cgatacgcca tcacgctctc gagtcggctt gcttcgtggt gaatgccaca     660
ggctggctga ccgatgcgca aatcacatcg atcacgccgg accccgcgct acaaaaggca     720
ttacgtggcg gttgctgcac cgccatcgtc tcgccggaag gtgtgctcct ggcagagccg     780
ctacgcagcg gcgaaggcat ggtgatcgcc gatctcgata tggcactcat caccaaacgc     840
aaacggatga tggattcggt cggccactat gcgcggcccg aattgttaag cctgcttgtc     900
gacgaccggc gcaaggtacc ggtatccgcg ctatttgccg acagcaaccc tgccaacggg     960
cacacagttt tcaccccatc cgacatacca acccttggga gcgcacatca tgcaaacagt    1020
taccaaaccg aaccagcaac tgatcactga                                    1050

<210> 126
<211> 349
<212> PRT
<213> Unknown

<220>
<223> Obtained from an environmental sample

<400> 126
Met Thr Thr Val Lys Lys Thr Val Arg Ala Ala Ala Ile Gln Ile Ala
1               5                   10                  15
Pro Asp Leu Asp Ser Ala Gly Gly Thr Leu Asp Lys Val Cys Thr Ala
            20                  25                  30
Ile Gln Lys Ala Ala Ala Gln Gly Ala Glu Leu Val Val Phe Pro Glu
        35                  40                  45
Thr Phe Leu Pro Tyr Tyr Pro Tyr Phe Ser Phe Val Arg Pro Pro Phe
    50                  55                  60

Ala Ser Gly Pro Glu His Leu Leu Leu Tyr Glu Arg Ala Val Ala Val
65                  70                  75                  80
Pro Gly Pro Val Thr Asp Ala Val Ser Ala Val Ala Arg Ser His Gly
                85                  90                  95
Val Val Val Val Leu Gly Val Asn Glu Arg Asp His Gly Thr Leu Tyr
            100                 105                 110
Asn Thr Gln Leu Val Phe Asp Ala Asn Gly Glu Leu Val Leu Lys Arg
        115                 120                 125
Arg Lys Ile Thr Pro Thr Tyr His Glu Arg Met Ile Trp Gly Gln Gly
    130                 135                 140
Asp Gly Ser Gly Leu Lys Val Val Gln Thr Ala Val Gly Arg Leu Gly
145                 150                 155                 160
Ala Leu Ala Cys Trp Glu His Tyr Asn Pro Leu Ala Arg Tyr Ala Leu
                165                 170                 175
Met Ala Gln His Glu Glu Ile His Cys Ala Gln Phe Pro Gly Ser Met
            180                 185                 190
Val Gly Gln Ile Phe Ala Asp Gln Met Glu Val Thr Ile Arg His His
        195                 200                 205
Ala Leu Glu Ser Ala Cys Phe Val Val Asn Ala Thr Gly Trp Leu Thr
    210                 215                 220
Asp Ala Gln Ile Thr Ser Ile Thr Pro Asp Pro Ala Leu Gln Lys Ala
225                 230                 235                 240
Leu Arg Gly Gly Cys Cys Thr Ala Ile Val Ser Pro Glu Gly Val Leu
                245                 250                 255
Leu Ala Glu Pro Leu Arg Ser Gly Glu Gly Met Val Ile Ala Asp Leu
            260                 265                 270
Asp Met Ala Leu Ile Thr Lys Arg Lys Arg Met Met Asp Ser Val Gly
        275                 280                 285
His Tyr Ala Arg Pro Glu Leu Leu Ser Leu Leu Val Asp Asp Arg Arg
    290                 295                 300
Lys Val Pro Val Ser Ala Leu Phe Ala Asp Ser Asn Pro Ala Asn Gly
305                 310                 315                 320
His Thr Val Phe Thr Pro Ser Asp Ile Pro Thr Leu Gly Ser Ala His
                325                 330                 335
His Ala Asn Ser Tyr Gln Thr Glu Pro Ala Thr Asp His
            340                 345

<210> 127
<211> 1005
<212> DNA
<213> Unknown

<220>
<223> Obtained from an environmental sample

<400> 127
atgatagcac ggaagacaat aagggcggcg gcggtgcaga tagcgcctgt gatggaagat      60
cggaaggcga cgaccgacaa ggtgtgcgcc tacattcagg aagcaggcga gaatggagcc     120
gaaattgtgg tgtttcctga aaccttcatt cccaattatc cctatttctc ttttgtaaaa     180
cctcccgtgt tggcaggtaa ggatcacctt accttgtatg accaagcggt ggaaatccct     240
agccctacta ccgaccaagt ggggtctatg gccaaaaaat ggggaatcgt agtggtgttg     300
ggcgtgaacg aaagaagcca cggcactttg tacaatgccc aaattgtctt tgacgctact     360
ggtgatattg tattggtgag acgcaaaatc acccctacct atcatgaacg gatgatctgg     420
ggacagggag atggcagtgg attaaaagca gtagacacag ctgtgggaag agtgggcgct     480
ttggcgtgtt gggaacacta taatccactt gcgcgctacg cccttatggt agaccatgag     540
gaaattcatt gcagccaatt ccctggctct atggtcggcc ccattttcgg tgaccagata     600
gaagtgacga ttcgccacca tgcgttggaa tcgggttgtt ttgtcatcaa ttccacaggt     660
tggctgtttg aagagcaaat ccaagccatc accgatgatc cgaaactgca caaagcattg     720
aaagacggct gtatgaccgc cattatttct cccgaaggcg tgcatttgac caaaccctta     780
acagaaggcg aaggcatcat ctacgcctat ctggacatga aactcataga caagcggaaa     840
cggatgatgg actcggtagg acactatgca cgtccagagt tgctctcttt gcatatcaac     900
aatgcagagc aaaaaccagc cgtttacacc tctcctctta ccaaaacgga aaccaaagaa     960
gacgtaaaaa gctatgatcg caacaaagaa cagcttatcg tctga                    1005

<210> 128
<211> 334
<212> PRT
<213> Unknown

<220>
<223> Obtained from an environmental sample

<400> 128
Met Ile Ala Arg Lys Thr Ile Arg Ala Ala Ala Val Gln Ile Ala Pro
1               5                   10                  15
Val Met Glu Asp Arg Lys Ala Thr Thr Asp Lys Val Cys Ala Tyr Ile
            20                  25                  30
Gln Glu Ala Gly Glu Asn Gly Ala Glu Ile Val Val Phe Pro Glu Thr
        35                  40                  45
Phe Ile Pro Asn Tyr Pro Tyr Phe Ser Phe Val Lys Pro Pro Val Leu
    50                  55                  60
Ala Gly Lys Asp His Leu Thr Leu Tyr Asp Gln Ala Val Glu Ile Pro
65                  70                  75                  80
Ser Pro Thr Thr Asp Gln Val Gly Ser Met Ala Lys Lys Trp Gly Ile
                85                  90                  95
Val Val Val Leu Gly Val Asn Glu Arg Ser His Gly Thr Leu Tyr Asn
            100                 105                 110
Ala Gln Ile Val Phe Asp Ala Thr Gly Asp Ile Val Leu Val Arg Arg
        115                 120                 125
Lys Ile Thr Pro Thr Tyr His Glu Arg Met Ile Trp Gly Gln Gly Asp
    130                 135                 140
Gly Ser Gly Leu Lys Ala Val Asp Thr Ala Val Gly Arg Val Gly Ala
145                 150                 155                 160
Leu Ala Cys Trp Glu His Tyr Asn Pro Leu Ala Arg Tyr Ala Leu Met
                165                 170                 175
Val Asp His Glu Glu Ile His Cys Ser Gln Phe Pro Gly Ser Met Val
            180                 185                 190
Gly Pro Ile Phe Gly Asp Gln Ile Glu Val Thr Ile Arg His His Ala
        195                 200                 205
Leu Glu Ser Gly Cys Phe Val Ile Asn Ser Thr Gly Trp Leu Phe Glu
    210                 215                 220
Glu Gln Ile Gln Ala Ile Thr Asp Asp Pro Lys Leu His Lys Ala Leu
225                 230                 235                 240
Lys Asp Gly Cys Met Thr Ala Ile Ile Ser Pro Glu Gly Val His Leu
                245                 250                 255
Thr Lys Pro Leu Thr Glu Gly Glu Gly Ile Ile Tyr Ala Tyr Leu Asp
            260                 265                 270
Met Lys Leu Ile Asp Lys Arg Lys Arg Met Met Asp Ser Val Gly His
        275                 280                 285
Tyr Ala Arg Pro Glu Leu Leu Ser Leu His Ile Asn Asn Ala Glu Gln
    290                 295                 300
Lys Pro Ala Val Tyr Thr Ser Pro Leu Thr Lys Thr Glu Thr Lys Glu
305                 310                 315                 320
Asp Val Lys Ser Tyr Asp Arg Asn Lys Glu Gln Leu Ile Val
                325                 330

<210> 129
<211> 1011
<212> DNA
<213> Unknown

<220>
<223> Obtained from an environmental sample

<400> 129
atgtcagaaa agcgaattat taaagcggct gcagttcaaa tcacaccaga ttttgaatcg      60
catgatggaa ccgtaaagaa ggtttgtaat gtaattgatg aagcgggtgc taaaggtgta     120
cagatcattg tattccctga aacctttatt ccatattacc catatttttc tttcatcact     180
ccaccagtga ctgctggcgc ggagcatttg cggctctatg aaaaaagtgt cgtgatacct     240
ggtcccgtta ctcaagccat ttccgaacgt gcacgcatga ataatatggt tgttgtactt     300
ggtgtaaatg agcgtgataa cggcagtcta tataacaccc agattatttt tgatgctacc     360
ggtgagatgc ttctgaagag aagaaaaatc acacctacct atcatgagcg catgatttgg     420
gggcaaggag atgcttcagg cctgaaggtc gtcgatacgg ctattgggcg agtcggagca     480
ttggcatgct gggagcacta taaccctttg gctagataca gcctcatgac acagcatgaa     540
gaaattcact gtgctcaatt tccaggctcc atggttggtc agatcttcgc agatcaaatg     600
gatgtcacga ttcgtcatca tgccttggag tcaggttgct tcgtcatcaa ctccactggc     660

tggttaactg atgatcagat caaatctatc accgacgatc ccaaaatgca gaaagcttta 720
agaggtggtt gcaacacggc cattatttct ccagaaggga atcatttaac cgagcctttg 780
cgagaaggtg aaggcatggt gattgctgat cttgatatgg cactcatcac caaacgaaaa 840
agaatgatgg actcagttgg ccactacgcc agaccagaac tgttgagctt agcgatcaat 900
gatgctccgg ctactccttc attccagatg aacgaacatc gtcttaaatc agtgcaatta 960
cctatcgcag aggagcttaa aaatgacaac aagcttagca gtggacagta a 1011

<210> 130
<211> 336
<212> PRT
<213> Unknown

<220>
<223> Obtained from an environmental sample

<400> 130
Met Ser Glu Lys Arg Ile Ile Lys Ala Ala Ala Val Gln Ile Thr Pro
1               5                   10                  15
Asp Phe Glu Ser His Asp Gly Thr Val Lys Lys Val Cys Asn Val Ile
            20                  25                  30
Asp Glu Ala Gly Ala Lys Gly Val Gln Ile Ile Val Phe Pro Glu Thr
        35                  40                  45
Phe Ile Pro Tyr Tyr Pro Tyr Phe Ser Phe Ile Thr Pro Pro Val Thr
    50                  55                  60
Ala Gly Ala Glu His Leu Arg Leu Tyr Glu Lys Ser Val Val Ile Pro
65                  70                  75                  80
Gly Pro Val Thr Gln Ala Ile Ser Glu Arg Ala Arg Met Asn Asn Met
                85                  90                  95
Val Val Val Leu Gly Val Asn Glu Arg Asp Asn Gly Ser Leu Tyr Asn
            100                 105                 110
Thr Gln Ile Ile Phe Asp Ala Thr Gly Glu Met Leu Leu Lys Arg Arg
        115                 120                 125
Lys Ile Thr Pro Thr Tyr His Glu Arg Met Ile Trp Gly Gln Gly Asp
    130                 135                 140
Ala Ser Gly Leu Lys Val Val Asp Thr Ala Ile Gly Arg Val Gly Ala
145                 150                 155                 160
Leu Ala Cys Trp Glu His Tyr Asn Pro Leu Ala Arg Tyr Ser Leu Met
                165                 170                 175
Thr Gln His Glu Glu Ile His Cys Ala Gln Phe Pro Gly Ser Met Val
            180                 185                 190
Gly Gln Ile Phe Ala Asp Gln Met Asp Val Thr Ile Arg His His Ala
        195                 200                 205
Leu Glu Ser Gly Cys Phe Val Ile Asn Ser Thr Gly Trp Leu Thr Asp
    210                 215                 220
Asp Gln Ile Lys Ser Ile Thr Asp Asp Pro Lys Met Gln Lys Ala Leu
225                 230                 235                 240
Arg Gly Gly Cys Asn Thr Ala Ile Ile Ser Pro Glu Gly Asn His Leu
                245                 250                 255
Thr Glu Pro Leu Arg Glu Gly Glu Gly Met Val Ile Ala Asp Leu Asp
            260                 265                 270
Met Ala Leu Ile Thr Lys Arg Lys Arg Met Met Asp Ser Val Gly His
        275                 280                 285
Tyr Ala Arg Pro Glu Leu Leu Ser Leu Ala Ile Asn Asp Ala Pro Ala
    290                 295                 300
Thr Pro Ser Phe Gln Met Asn Glu His Arg Leu Lys Ser Val Gln Leu
305                 310                 315                 320
Pro Ile Ala Glu Glu Leu Lys Asn Asp Asn Lys Leu Ser Ser Gly Gln
                325                 330                 335

<210> 131
<211> 1011
<212> DNA
<213> Unknown

<220>
<223> Obtained from an environmental sample

<400> 131

atgtcagaaa agcgaattat taaagcggct gcagttcaaa tcacaccaga ttttgaatcg 60
catgatggaa ccgtaaagaa ggtttgtaat gtaattgatg aagcgggtgc taaaggtgta 120
cagatcattg tattccctga aacctttatt ccatattacc catatttttc tttcatcact 180
ccaccagtga ctgctggcgc ggagcatttg cggctctatg aaaaaagtgt cgtgatacct 240
ggtcccgtta ctcaagacat ttccgaacgt gcacgcatga ataatatggt tgttgtactt 300
ggtgtaaatg agcgtgataa cggcagtcta tataacaccc agattatttt tgatgctacc 360
ggtgagatgc ttctgaagag aagaaaaatc acacctacct atcatgagcg catgatttgg 420
gggcaaggag atgcttcagg cctgaaggtc gtcgatacgg ctattgggcg agtcggagca 480
ttggcatgct gggagcacta taaccctttg gctagataca gcctcatgac acagcatgaa 540
gaaattcact gtgctcaatt tccaggctcc atggttggtc agatcttcgc agatcaaatg 600
gatgtcacga ttcgtcatca tgccttggag tcaggttgct tcgtcatcaa ctccactggc 660
tggttaactg atgatcagat caaatctatc accgacgatc ccaaaatgca gaaagcttta 720
agaggtggtt gcaacacggc cattatttct ccagaaggga atcatttaac cgagcctttg 780
cgagaaggtg aaggcatggt gattgctgat cttgatatgg cactcatcac caaacgaaaa 840
agaatgatgg actcagttgg ccactacgcc agaccagaac tgttgagctt agcgatcaat 900
gatgctccgg ctactccttc attccagatg aacgaacatc gtcttaaatc agtgcaatta 960
cctatcgcag aggagcttaa aaatgacaac aagcttagca gtggacagta a 1011

<210> 132
<211> 336
<212> PRT
<213> Unknown

<220>
<223> Obtained from an environmental sample

<400> 132
Met Ser Glu Lys Arg Ile Ile Lys Ala Ala Ala Val Gln Ile Thr Pro
1               5                   10                  15
Asp Phe Glu Ser His Asp Gly Thr Val Lys Lys Val Cys Asn Val Ile
            20                  25                  30
Asp Glu Ala Gly Ala Lys Gly Val Gln Ile Ile Val Phe Pro Glu Thr
        35                  40                  45
Phe Ile Pro Tyr Tyr Pro Tyr Phe Ser Phe Ile Thr Pro Pro Val Thr
    50                  55                  60
Ala Gly Ala Glu His Leu Arg Leu Tyr Glu Lys Ser Val Val Ile Pro
65                  70                  75                  80
Gly Pro Val Thr Gln Asp Ile Ser Glu Arg Ala Arg Met Asn Asn Met
                85                  90                  95
Val Val Val Leu Gly Val Asn Glu Arg Asp Asn Gly Ser Leu Tyr Asn
            100                 105                 110
Thr Gln Ile Ile Phe Asp Ala Thr Gly Glu Met Leu Leu Lys Arg Arg
        115                 120                 125
Lys Ile Thr Pro Thr Tyr His Glu Arg Met Ile Trp Gly Gln Gly Asp
    130                 135                 140
Ala Ser Gly Leu Lys Val Val Asp Thr Ala Ile Gly Arg Val Gly Ala
145                 150                 155                 160
Leu Ala Cys Trp Glu His Tyr Asn Pro Leu Ala Arg Tyr Ser Leu Met
                165                 170                 175
Thr Gln His Glu Glu Ile His Cys Ala Gln Phe Pro Gly Ser Met Val
            180                 185                 190
Gly Gln Ile Phe Ala Asp Gln Met Asp Val Thr Ile Arg His His Ala
        195                 200                 205
Leu Glu Ser Gly Cys Phe Val Ile Asn Ser Thr Gly Trp Leu Thr Asp
    210                 215                 220
Asp Gln Ile Lys Ser Ile Thr Asp Asp Pro Lys Met Gln Lys Ala Leu
225                 230                 235                 240
Arg Gly Gly Cys Asn Thr Ala Ile Ile Ser Pro Glu Gly Asn His Leu
                245                 250                 255
Thr Glu Pro Leu Arg Glu Gly Glu Gly Met Val Ile Ala Asp Leu Asp
            260                 265                 270
Met Ala Leu Ile Thr Lys Arg Lys Arg Met Met Asp Ser Val Gly His
        275                 280                 285
Tyr Ala Arg Pro Glu Leu Leu Ser Leu Ala Ile Asn Asp Ala Pro Ala
    290                 295                 300
Thr Pro Ser Phe Gln Met Asn Glu His Arg Leu Lys Ser Val Gln Leu
305                 310                 315                 320
Pro Ile Ala Glu Glu Leu Lys Asn Asp Asn Lys Leu Ser Ser Gly Gln

325                    330                    335

<210> 133
<211> 1026
<212> DNA
<213> Unknown

<220>
<223> Obtained from an environmental sample

<400> 133
atgtcgacca agcggatcgt acgcgccgct gccgttcagc tggcaccgga tctggagcgg      60
ccggagggca cactggagaa ggtttgcgcg gccatcgaca aggcggcggg ggacggtgtg     120
cagctcatcg tcttccccga gaccttcgta ccgtactacc cgtacttctc tttcgtgcgt     180
gcgccggtcg cgatgggtgc cgagcacatg cggttatacg agcgcgcggt agcggtgccc     240
ggtccagtaa cggccaccgt ggcggagcgg gcaaaagcgc acgcgatggt cgtcgtgctg     300
ggtgtaaacg agcgcgatca cggctcactg tataacgcgc aactgatctt cgacgagacc     360
ggccgtctcg tcctcaaacg ccgcaagatc actccgacct atcacgagcg catggtgtgg     420
gggcagggcg acggcagcgg ccttaaggtt gtagacaccg gtatcggcag gatcggagcc     480
ctcgcctgct gggagcacta caacccgctc gcgcgctatg cgctcatggc gcagcacgaa     540
gagattcatt gcgcgcagtt tccgggctcg atggtggggc cgatcttcgc ggatcagatc     600
gaggtcacga tccgccatca cgcgctggag tcgggctgct tcgtcgtcaa tgcgaccggc     660
tggctgacac ccgaacagat cgcgtcgatc acaccggacg cgggtctgca aaaggcaatc     720
agcgggggct gcaacaccgc gatcatctcg ccggagggcg tgcacctggc cccgccgttg     780
cgagaaggtg agggcatggt cgtggccgac ctcgacatgg cgctcatcac caaacgcaaa     840
cgcatgatgg attcggtggg tcactacgct cgcccggagt tgctcagcct gcgcatcgat     900
agccgcgccg cttcgccgat gtcgtcacaa atggaaatac ccgggagctt gcatgaaatc     960
accagccacg atgtccagcc agcaactgat gaccgagctc cagtcctccg gcttgaggtt    1020
ggctga                                                               1026

<210> 134
<211> 341
<212> PRT
<213> Unknown

<220>
<223> Obtained from an environmental sample

<400> 134
Met Ser Thr Lys Arg Ile Val Arg Ala Ala Ala Val Gln Leu Ala Pro
1               5                   10                  15
Asp Leu Glu Arg Pro Glu Gly Thr Leu Glu Lys Val Cys Ala Ala Ile
            20                  25                  30
Asp Lys Ala Ala Gly Asp Gly Val Gln Leu Ile Val Phe Pro Glu Thr
        35                  40                  45
Phe Val Pro Tyr Tyr Pro Tyr Phe Ser Phe Val Arg Ala Pro Val Ala
    50                  55                  60
Met Gly Ala Glu His Met Arg Leu Tyr Glu Arg Ala Val Ala Val Pro
65                  70                  75                  80
Gly Pro Val Thr Ala Thr Val Ala Glu Arg Ala Lys Ala His Ala Met
                85                  90                  95
Val Val Val Leu Gly Val Asn Glu Arg Asp His Gly Ser Leu Tyr Asn
            100                 105                 110
Ala Gln Leu Ile Phe Asp Glu Thr Gly Arg Leu Val Leu Lys Arg Arg
        115                 120                 125
Lys Ile Thr Pro Thr Tyr His Glu Arg Met Val Trp Gly Gln Gly Asp
    130                 135                 140
Gly Ser Gly Leu Lys Val Val Asp Thr Gly Ile Gly Arg Ile Gly Ala
145                 150                 155                 160
Leu Ala Cys Trp Glu His Tyr Asn Pro Leu Ala Arg Tyr Ala Leu Met
                165                 170                 175
Ala Gln His Glu Glu Ile His Cys Ala Gln Phe Pro Gly Ser Met Val
            180                 185                 190
Gly Pro Ile Phe Ala Asp Gln Ile Glu Val Thr Ile Arg His His Ala
        195                 200                 205
Leu Glu Ser Gly Cys Phe Val Val Asn Ala Thr Gly Trp Leu Thr Pro
    210                 215                 220
Glu Gln Ile Ala Ser Ile Thr Pro Asp Ala Gly Leu Gln Lys Ala Ile

225                 230                 235                 240
Ser Gly Gly Cys Asn Thr Ala Ile Ile Ser Pro Glu Gly Val His Leu
                245                 250                 255
Ala Pro Pro Leu Arg Glu Gly Glu Gly Met Val Val Ala Asp Leu Asp
            260                 265                 270
Met Ala Leu Ile Thr Lys Arg Lys Arg Met Met Asp Ser Val Gly His
        275                 280                 285
Tyr Ala Arg Pro Glu Leu Leu Ser Leu Arg Ile Asp Ser Arg Ala Ala
    290                 295                 300
Ser Pro Met Ser Ser Gln Met Glu Ile Pro Gly Ser Leu His Glu Ile
305                 310                 315                 320
Thr Ser His Asp Val Gln Pro Ala Thr Asp Asp Arg Ala Pro Val Leu
                325                 330                 335
Arg Leu Glu Val Gly
            340

<210> 135
<211> 1011
<212> DNA
<213> Unknown

<220>
<223> Obtained from an environmental sample

<400> 135
atgtcagaca agcgaatcat taaagcggct gcagttcaaa tcactcctga ctttgactca      60
gcagatggaa ccgttaagaa agtgtgcaag gtaatcgatg aagcaggtgc aaagggagtt     120
caaattattg tattcccgga aaccttcatc ccttactacc catacttttc attcattaca     180
cctccagtca ctgctggcgc tgagcattta aagctttatg agaaaagtgt cgtgatacct     240
ggcccggtta cccaagcgat tgccgagcga gccagggtta atcagatggt tgtcgtgctt     300
ggtgtcaacg agcgagataa cggtagcctc tacaacacac aattgatctt tgataccaac     360
ggcgaactgc tacttaaaag aagaaaaatc acccctacct accatgaacg tatgatctgg     420
gggcaaggtg atgcatcagg tctcaaagta gttgaaacag agatcgcccg agtaggtgcc     480
ttggcttgtt gggaacacta caacccactg gccagatatg cactcatgac acagcatgaa     540
gaaattcact gtgcgcaatt cccaggctct atggttggcc agatatttgc cgatcagatg     600
gatgtcacta tccgacatca cgccttagag tcaggctgct tcgtcatcaa cgccactggc     660
tggctcaccg acgcgcaaat ccaatcgatt actgatgacc caaaaatgca aaaagcatta     720
cgtggcggct gcaacacagc catcatctcc cccgaagggg tgcacttaac agagccacta     780
cgtgaaggag aaggcatggt gattgccaat cttgatatgg cactcatcac aaaacgaaaa     840
agaatgatgg attcggtagg ccattattca agaccagaat tattaagcct ggcaattaac     900
gacaaaccag caactacaac attttcaatg actgaggggc gtactcaaac agagccattt     960
cgaatcgcag aggagttgaa aaatgacgac aagcttagca ctggaaacta a             1011

<210> 136
<211> 336
<212> PRT
<213> Unknown

<220>
<223> Obtained from an environmental sample

<400> 136
Met Ser Asp Lys Arg Ile Ile Lys Ala Ala Ala Val Gln Ile Thr Pro
1               5                   10                  15
Asp Phe Asp Ser Ala Asp Gly Thr Val Lys Lys Val Cys Lys Val Ile
            20                  25                  30
Asp Glu Ala Gly Ala Lys Gly Val Gln Ile Ile Val Phe Pro Glu Thr
        35                  40                  45
Phe Ile Pro Tyr Tyr Pro Tyr Phe Ser Phe Ile Thr Pro Pro Val Thr
    50                  55                  60
Ala Gly Ala Glu His Leu Lys Leu Tyr Glu Lys Ser Val Val Ile Pro
65                  70                  75                  80
Gly Pro Val Thr Gln Ala Ile Ala Glu Arg Ala Arg Val Asn Gln Met
                85                  90                  95
Val Val Val Leu Gly Val Asn Glu Arg Asp Asn Gly Ser Leu Tyr Asn
            100                 105                 110
Thr Gln Leu Ile Phe Asp Thr Asn Gly Glu Leu Leu Leu Lys Arg Arg
        115                 120                 125

Lys Ile Thr Pro Thr Tyr His Glu Arg Met Ile Trp Gly Gln Gly Asp
    130                 135                 140
Ala Ser Gly Leu Lys Val Val Glu Thr Glu Ile Ala Arg Val Gly Ala
145                 150                 155                 160
Leu Ala Cys Trp Glu His Tyr Asn Pro Leu Ala Arg Tyr Ala Leu Met
                165                 170                 175
Thr Gln His Glu Glu Ile His Cys Ala Gln Phe Pro Gly Ser Met Val
            180                 185                 190
Gly Gln Ile Phe Ala Asp Gln Met Asp Val Thr Ile Arg His His Ala
        195                 200                 205
Leu Glu Ser Gly Cys Phe Val Ile Asn Ala Thr Gly Trp Leu Thr Asp
    210                 215                 220
Ala Gln Ile Gln Ser Ile Thr Asp Asp Pro Lys Met Gln Lys Ala Leu
225                 230                 235                 240
Arg Gly Gly Cys Asn Thr Ala Ile Ile Ser Pro Glu Gly Val His Leu
                245                 250                 255
Thr Glu Pro Leu Arg Glu Gly Glu Gly Met Val Ile Ala Asn Leu Asp
            260                 265                 270
Met Ala Leu Ile Thr Lys Arg Lys Arg Met Met Asp Ser Val Gly His
        275                 280                 285
Tyr Ser Arg Pro Glu Leu Leu Ser Leu Ala Ile Asn Asp Lys Pro Ala
    290                 295                 300
Thr Thr Thr Phe Ser Met Thr Glu Gly Arg Thr Gln Thr Glu Pro Phe
305                 310                 315                 320
Arg Ile Ala Glu Glu Leu Lys Asn Asp Asp Lys Leu Ser Thr Gly Asn
                325                 330                 335

<210> 137
<211> 978
<212> DNA
<213> Unknown

<220>
<223> Obtained from an environmental sample

<400> 137
atggctattg tcaaggccgc ggcggtgcag atcagtccgg tgctctacag tcgcgccggc      60
acagtggaca aggtcgtcgc gaagatccgc gagctgggcc gacgaggggt cgagttcgcc     120
gtcttccccg agaccgtcat tccctactat ccctacttct ctttcgtgca gcccccctac     180
acccaggcca ccgaacacct gcgcctgctc gaggaatcgg tgaccgtgcc ctccgccgaa     240
accgacgcga tcgccaaggc cgctcgcgag gcgggcatgg tcgtctccat cggcgtcaac     300
gagcgcgacg gcggaaccat ctacaacacc caactcctct tcgacgccga cggcactctc     360
atccagcgcc gccgcaagat cacccccacc tatcacgaac gcatggtctg gggcagggga     420
gacggctcag gtctgcgcgc cgtcgacagt gcggtcggcc gcatcggcca gctcgcctgc     480
tgggagcact accagccact ggcccggtac gccctcatcg ctgacggcga gcagatccac     540
gccgcgatgt accccggcgc cttcggcggc gatctgttcg ccgagcagat cgaggtcaac     600
atccgccagc acgccctgga atccgccagc ttcgtcgtca acgccaccgc ctggctcgac     660
gccgatcagc aggcccagat cgccaaggac accggaggcc cggtcccggc cttctccggt     720
ggcttcttca ccgccatcgt cgaccccgaa ggccgtatca tcggcgaccc cctcaccagc     780
ggcgaaggcg aagtgatcgc cgacctcgat ctcgctctca tcaaccgccg caagcgcctc     840
atggacgcca gtggacacta ccagccgccc gaaattctta gcttcacatt gaccggtgca     900
ccggcgcctt atgtcaagag cgcggcgtgc cggggaaccc cgggtacgac cgtggccgag     960
gagggacggt ccgcttag                                                   978

<210> 138
<211> 325
<212> PRT
<213> Unknown

<220>
<223> Obtained from an environmental sample

<400> 138
Met Ala Ile Val Lys Ala Ala Ala Val Gln Ile Ser Pro Val Leu Tyr
1               5                   10                  15
Ser Arg Ala Gly Thr Val Asp Lys Val Val Ala Lys Ile Arg Glu Leu
            20                  25                  30
Gly Arg Arg Gly Val Glu Phe Ala Val Phe Pro Glu Thr Val Ile Pro

            35                  40                  45
Tyr Tyr Pro Tyr Phe Ser Phe Val Gln Pro Pro Tyr Thr Gln Ala Thr
    50                  55                  60
Glu His Leu Arg Leu Leu Glu Glu Ser Val Thr Val Pro Ser Ala Glu
65                  70                  75                  80
Thr Asp Ala Ile Ala Lys Ala Ala Arg Glu Ala Gly Met Val Val Ser
                85                  90                  95
Ile Gly Val Asn Glu Arg Asp Gly Gly Thr Ile Tyr Asn Thr Gln Leu
            100                 105                 110
Leu Phe Asp Ala Asp Gly Thr Leu Ile Gln Arg Arg Arg Lys Ile Thr
        115                 120                 125
Pro Thr Tyr His Glu Arg Met Val Trp Gly Gln Gly Asp Gly Ser Gly
    130                 135                 140
Leu Arg Ala Val Asp Ser Ala Val Gly Arg Ile Gly Gln Leu Ala Cys
145                 150                 155                 160
Trp Glu His Tyr Gln Pro Leu Ala Arg Tyr Ala Leu Ile Ala Asp Gly
                165                 170                 175
Glu Gln Ile His Ala Ala Met Tyr Pro Gly Ala Phe Gly Gly Asp Leu
            180                 185                 190
Phe Ala Glu Gln Ile Glu Val Asn Ile Arg Gln His Ala Leu Glu Ser
        195                 200                 205
Ala Ser Phe Val Val Asn Ala Thr Ala Trp Leu Asp Ala Asp Gln Gln
    210                 215                 220
Ala Gln Ile Ala Lys Asp Thr Gly Gly Pro Val Pro Ala Phe Ser Gly
225                 230                 235                 240
Gly Phe Phe Thr Ala Ile Val Asp Pro Glu Gly Arg Ile Ile Gly Asp
                245                 250                 255
Pro Leu Thr Ser Gly Glu Gly Glu Val Ile Ala Asp Leu Asp Leu Ala
            260                 265                 270
Leu Ile Asn Arg Arg Lys Arg Leu Met Asp Ala Ser Gly His Tyr Gln
        275                 280                 285
Pro Pro Glu Ile Leu Ser Phe Thr Leu Thr Gly Ala Pro Ala Pro Tyr
    290                 295                 300
Val Lys Ser Ala Ala Cys Arg Gly Thr Pro Gly Thr Thr Val Ala Glu
305                 310                 315                 320
Glu Gly Arg Ser Ala
                325

<210> 139
<211> 999
<212> DNA
<213> Unknown

<220>
<223> Obtained from an environmental sample

<400> 139
atgaaaacaa cggttaccgt tgcctgcgtt caggccgccc ccgtatttat ggatttagaa      60
ggcaccgtag ataaaacaat caccctcatc tctgaagccg cacagaaagg cgcggagctc     120
atcgcttttc cggagacctg gatacccggt tacccgtggt tcttatggct gaactcgccc     180
gccacaaata tgcccctggt ttatcagtat catcagaact ctctggtgct ggacagtacc     240
caggcgaagc gaattgcgga tgcggcacgg cagaataaca tcactgtcgc tctgggcttc     300
agcgaacgcg atcatggaag cctctatatc gcacagtggc tgattggcag cgacggggag     360
accattggca tccggcgcaa gctcaaggcc acgcacgtgg agcgtacgct gttcggcgaa     420
agcgacggct cctccctgac cacctgggag acacctctgg gtaacgtcgg ggccctctgc     480
tgctgggagc acctgcagcc gctgtcccgc tatgcaatgt attcccagca tgaggagatc     540
cacatcgctg cctggcccag tttcagtctc tacaccagtg caacggccgc actgggtcct     600
gacgtcaata cggcggcttc acgcctctat gccgcggagg ggcagtgctt cgtgatagcc     660
ccgtgtgccg tggtttctga tgaaatgatt gatttactct gtcctgatga tgaccggaga     720
gcgttactca gtgccggagg gggacatgcc cgtatttacg gcccggacgg aagagaactc     780
gtcacccctc tcggggaaaa tgaggaagga ctgcttatcg ctgagctcga ctctgctgcg     840
attacctttg ccaaactggc ggcagacccg gttggccact attcccgtcc tgacgtgacc     900
cgcctccttt ttaatccttc agccaacaag actgtgatta aacgacattc gcctcctgag     960
ttaattgccg agcagactgc agaagaagag gaggagtag                            999

<210> 140
<211> 332
<212> PRT

<213> Unknown

<220>
<223> Obtained from an environmental sample

<400> 140
Met Lys Thr Thr Val Thr Val Ala Cys Val Gln Ala Ala Pro Val Phe
1               5                   10                  15
Met Asp Leu Glu Gly Thr Val Asp Lys Thr Ile Thr Leu Ile Ser Glu
            20                  25                  30
Ala Ala Gln Lys Gly Ala Glu Leu Ile Ala Phe Pro Glu Thr Trp Ile
        35                  40                  45
Pro Gly Tyr Pro Trp Phe Leu Trp Leu Asn Ser Pro Ala Thr Asn Met
    50                  55                  60
Pro Leu Val Tyr Gln Tyr His Gln Asn Ser Leu Val Leu Asp Ser Thr
65                  70                  75                  80
Gln Ala Lys Arg Ile Ala Asp Ala Ala Arg Gln Asn Asn Ile Thr Val
                85                  90                  95
Ala Leu Gly Phe Ser Glu Arg Asp His Gly Ser Leu Tyr Ile Ala Gln
            100                 105                 110
Trp Leu Ile Gly Ser Asp Gly Glu Thr Ile Gly Ile Arg Arg Lys Leu
        115                 120                 125
Lys Ala Thr His Val Glu Arg Thr Leu Phe Gly Glu Ser Asp Gly Ser
    130                 135                 140
Ser Leu Thr Thr Trp Glu Thr Pro Leu Gly Asn Val Gly Ala Leu Cys
145                 150                 155                 160
Cys Trp Glu His Leu Gln Pro Leu Ser Arg Tyr Ala Met Tyr Ser Gln
                165                 170                 175
His Glu Glu Ile His Ile Ala Ala Trp Pro Ser Phe Ser Leu Tyr Thr
            180                 185                 190
Ser Ala Thr Ala Ala Leu Gly Pro Asp Val Asn Thr Ala Ala Ser Arg
        195                 200                 205
Leu Tyr Ala Ala Glu Gly Gln Cys Phe Val Ile Ala Pro Cys Ala Val
    210                 215                 220
Val Ser Asp Glu Met Ile Asp Leu Leu Cys Pro Asp Asp Asp Arg Arg
225                 230                 235                 240
Ala Leu Leu Ser Ala Gly Gly Gly His Ala Arg Ile Tyr Gly Pro Asp
                245                 250                 255
Gly Arg Glu Leu Val Thr Pro Leu Gly Glu Asn Glu Glu Gly Leu Leu
            260                 265                 270
Ile Ala Glu Leu Asp Ser Ala Ala Ile Thr Phe Ala Lys Leu Ala Ala
        275                 280                 285
Asp Pro Val Gly His Tyr Ser Arg Pro Asp Val Thr Arg Leu Leu Phe
    290                 295                 300
Asn Pro Ser Ala Asn Lys Thr Val Ile Lys Arg His Ser Pro Pro Glu
305                 310                 315                 320
Leu Ile Ala Glu Gln Thr Ala Glu Glu Glu Glu Glu
                325                 330

<210> 141
<211> 1026
<212> DNA
<213> Unknown

<220>
<223> Obtained from an environmental sample

<400> 141
atggtgttca aggcagcgac tgttcatgca gctccggtat tcatggacaa ggaagcgtcg      60
atagataagg ctatcgacct catcaagaag gccggtcagg aagggattaa gcttctggtt     120
tttccggaaa cgtttattcc gggctatccg tattttatcg aatgctatcc gccgcttgcg     180
caggtggaag cgctcgccca gtacactgac gcttccgtgg agatcgacgg cccggaagtc     240
acccggcttc agcaggtagc caaggcggca ggcgttgcag tcgtcatggg catcagcgaa     300
cgaatggctg agacccgaac ctgcttcaac tcgcaggtgt tcattgacgt cgacggcacg     360
ctgctcggcg tgcatcgcaa gctgcagccg acttatgccg agcgcaaggt atgggcacag     420
ggcggtggtt atacgctgag gacctacaag agctcgcttg gcgtgctcgg cggtcttgcc     480
tgctgggagc acacgatgaa cctcgcgcgg caggccctga tcatgcagag cgagcagatc     540
catgcggctg catggcccgg actatcgacg atgcgaggtt tcgagcccgt ggccgatatc     600

cagatcgacg ccatgatgaa gactcacgcg cttaccgcac agtgctgggt gctttcggcc 660
ggcaatcccg tcgaccggac ctgcctcgac tggatggaaa agaacatcgg accgcaggat 720
tacgtcaccg agggcggcgg atggagcgcc gttatccatc cgttcaacag ctatctcggc 780
ggccctcaca cgggccttga ggaaaagctg gtcgtcggcg agatcaatct ggacgatctc 840
aagttcgtca aagtctggct cgacagcaaa gggcactatg ctcggccgga aatcctgaaa 900
cttggcgtca accaaaagca gatttggcct gatgaacatt tgctggcgcg gcaggatgtg 960
accgagttgc tggaggcgga tatcatcgaa taccccttgc aactgttgca agaccgcgcg 1020
caatag 1026

<210> 142
<211> 341
<212> PRT
<213> Unknown

<220>
<223> Obtained from an environmental sample

<400> 142
Met Val Phe Lys Ala Ala Thr Val His Ala Ala Pro Val Phe Met Asp
1               5                   10                  15
Lys Glu Ala Ser Ile Asp Lys Ala Ile Asp Leu Ile Lys Lys Ala Gly
            20                  25                  30
Gln Glu Gly Ile Lys Leu Leu Val Phe Pro Glu Thr Phe Ile Pro Gly
        35                  40                  45
Tyr Pro Tyr Phe Ile Glu Cys Tyr Pro Pro Leu Ala Gln Val Glu Ala
    50                  55                  60
Leu Ala Gln Tyr Thr Asp Ala Ser Val Glu Ile Asp Gly Pro Glu Val
65                  70                  75                  80
Thr Arg Leu Gln Gln Val Ala Lys Ala Ala Gly Val Ala Val Val Met
                85                  90                  95
Gly Ile Ser Glu Arg Met Ala Glu Thr Arg Thr Cys Phe Asn Ser Gln
            100                 105                 110
Val Phe Ile Asp Val Asp Gly Thr Leu Leu Gly Val His Arg Lys Leu
        115                 120                 125
Gln Pro Thr Tyr Ala Glu Arg Lys Val Trp Ala Gln Gly Gly Gly Tyr
    130                 135                 140
Thr Leu Arg Thr Tyr Lys Ser Ser Leu Gly Val Leu Gly Gly Leu Ala
145                 150                 155                 160
Cys Trp Glu His Thr Met Asn Leu Ala Arg Gln Ala Leu Ile Met Gln
                165                 170                 175
Ser Glu Gln Ile His Ala Ala Ala Trp Pro Gly Leu Ser Thr Met Arg
            180                 185                 190
Gly Phe Glu Pro Val Ala Asp Ile Gln Ile Asp Ala Met Met Lys Thr
        195                 200                 205
His Ala Leu Thr Ala Gln Cys Trp Val Leu Ser Ala Gly Asn Pro Val
    210                 215                 220
Asp Arg Thr Cys Leu Asp Trp Met Glu Lys Asn Ile Gly Pro Gln Asp
225                 230                 235                 240
Tyr Val Thr Glu Gly Gly Gly Trp Ser Ala Val Ile His Pro Phe Asn
                245                 250                 255
Ser Tyr Leu Gly Gly Pro His Thr Gly Leu Glu Glu Lys Leu Val Val
            260                 265                 270
Gly Glu Ile Asn Leu Asp Asp Leu Lys Phe Val Lys Val Trp Leu Asp
        275                 280                 285
Ser Lys Gly His Tyr Ala Arg Pro Glu Ile Leu Lys Leu Gly Val Asn
    290                 295                 300
Gln Lys Gln Ile Trp Pro Asp Glu His Leu Leu Ala Arg Gln Asp Val
305                 310                 315                 320
Thr Glu Leu Leu Glu Ala Asp Ile Ile Glu Tyr Pro Leu Gln Leu Leu
                325                 330                 335
Gln Asp Arg Ala Gln
            340

<210> 143
<211> 1122
<212> DNA
<213> Unknown

<220>
<223> Obtained from an environmental sample

<400> 143
atgacgatca ttgcaggcgc ggttcatgcg gcgccggtat tcatggatgt cgatgccact 60
atcgacaagg catgcgaaat cattcgcaag gcaggcaaag acggaatcga gcttctcgtc 120
ttccctgagg ttttcgtacc cggctacccc tacttcatcg agtgctatcc gaccttgaac 180
caaaccgctg cgctggccgc ctatacggat gcctcgatcg aggttccagg cccggaagtc 240
cggcgcttgc aggtggccgc acatcaggcc ggcgtgatgg ttgtgatggg cgtgagcgag 300
cgtctgcgcg gatctcgcac ctgcttcaac agccaggtgt tcatcgaccg tgacggcacc 360
ttgctgggcg tgcaccgcaa actccagccg acctatgtcg agcgcatcgt ctggggccag 420
ggcggcggac acaccctcaa ggtattcgac agcacactgg gcaaggtggg cggactggcc 480
tgctgggagc acacgatgaa cctcgcgcgc catgcgttga tcgcccaggg tatccagatc 540
catgccgccg cctggcctgg gctttcgaca atggccgggt tcgaagcggt ggctgacgtc 600
cagatcgacg cgatgatgaa aactcatgcg ttgagcgcgc aatgctttgt cgtatcggcc 660
gcaaaccctg tggatcagac ctgcctggag tggatggaga aacacctcgg cccgcagcaa 720
ctcgttaccg ccggcggagg ctggtcggca atcgtccatc ctttctgtgg ttatatcgcc 780
gcccctcaca ccggtgccga ggagaaggtt ctggtaggcg aaatcaatct ggacgacctc 840
aagcaggtca aggtatgggt tgattccgca ggtcattatg cgcgcccgga agtcgtgcaa 900
ttgcgcgacg ccctggagag ccgtggcaat tatcgcgttg cgctgacccg cgacgccgac 960
accttcgtgc cgctggaaga ccgcgtgcgc tttgcgcgcc agcagaacgc cgacctcttc 1020
atctcgatcc acgccgacgc caacgccaac cacgatgcgc gcggggctgg cttcacttcg 1080
aaggttgaaa acctttccac gggcatttta ccaggcgatt ga 1122

<210> 144
<211> 373
<212> PRT
<213> Unknown

<220>
<223> Obtained from an environmental sample

<400> 144
Met Thr Ile Ile Ala Gly Ala Val His Ala Ala Pro Val Phe Met Asp
1               5                   10                  15
Val Asp Ala Thr Ile Asp Lys Ala Cys Glu Ile Ile Arg Lys Ala Gly
            20                  25                  30
Lys Asp Gly Ile Glu Leu Leu Val Phe Pro Glu Val Phe Val Pro Gly
        35                  40                  45
Tyr Pro Tyr Phe Ile Glu Cys Tyr Pro Thr Leu Asn Gln Thr Ala Ala
    50                  55                  60
Leu Ala Ala Tyr Thr Asp Ala Ser Ile Glu Val Pro Gly Pro Glu Val
65                  70                  75                  80
Arg Arg Leu Gln Val Ala Ala His Gln Ala Gly Val Met Val Val Met
                85                  90                  95
Gly Val Ser Glu Arg Leu Arg Gly Ser Arg Thr Cys Phe Asn Ser Gln
            100                 105                 110
Val Phe Ile Asp Arg Asp Gly Thr Leu Leu Gly Val His Arg Lys Leu
        115                 120                 125
Gln Pro Thr Tyr Val Glu Arg Ile Val Trp Gly Gln Gly Gly Gly His
    130                 135                 140
Thr Leu Lys Val Phe Asp Ser Thr Leu Gly Lys Val Gly Gly Leu Ala
145                 150                 155                 160
Cys Trp Glu His Thr Met Asn Leu Ala Arg His Ala Leu Ile Ala Gln
                165                 170                 175
Gly Ile Gln Ile His Ala Ala Ala Trp Pro Gly Leu Ser Thr Met Ala
            180                 185                 190
Gly Phe Glu Ala Val Ala Asp Val Gln Ile Asp Ala Met Met Lys Thr
        195                 200                 205
His Ala Leu Ser Ala Gln Cys Phe Val Val Ser Ala Ala Asn Pro Val
    210                 215                 220
Asp Gln Thr Cys Leu Glu Trp Met Glu Lys His Leu Gly Pro Gln Gln
225                 230                 235                 240
Leu Val Thr Ala Gly Gly Gly Trp Ser Ala Ile Val His Pro Phe Cys
                245                 250                 255
Gly Tyr Ile Ala Ala Pro His Thr Gly Ala Glu Glu Lys Val Leu Val
            260                 265                 270
Gly Glu Ile Asn Leu Asp Asp Leu Lys Gln Val Lys Val Trp Val Asp

275                 280                 285
Ser Ala Gly His Tyr Ala Arg Pro Glu Val Val Gln Leu Arg Asp Ala
    290                 295                 300
Leu Glu Ser Arg Gly Asn Tyr Arg Val Ala Leu Thr Arg Asp Ala Asp
305                 310                 315                 320
Thr Phe Val Pro Leu Glu Asp Arg Val Arg Phe Ala Arg Gln Gln Asn
                325                 330                 335
Ala Asp Leu Phe Ile Ser Ile His Ala Asp Ala Asn Ala Asn His Asp
            340                 345                 350
Ala Arg Gly Ala Gly Phe Thr Ser Lys Val Glu Asn Leu Ser Thr Gly
        355                 360                 365
Ile Leu Pro Gly Asp
    370

<210> 145
<211> 1014
<212> DNA
<213> Unknown

<220>
<223> Obtained from an environmental sample

<400> 145
atgggcatca cccacccgaa ctacaaggtc gcagtggtcc aggctgcgcc ggtctggttg      60
aacctcgagg caacggtcga gaagacaatc aggtatattg aagaggcggc caaggctgga     120
gcgaagctga tagcgtttcc ggaaacctgg attccgggct atccatggca catttggatc     180
ggaacgcccg catgggcaat cggtaagggc ttcgtccagc gctatttcga caactcgctc     240
agctatgaca gcccgctcgc gcggcagatc gctgacgccg cagcaaagag caagatcacg     300
gttgttctcg gcctctccga gcgcgacggt ggaagcctat acatcgcgca atggctgatc     360
ggaccagatg gcgagaccat cgcgaagcgg cgcaagctgc gtccgaccca cgtcgagcgc     420
acggtgttcg gtgacggtga cggcagccac atcgccgtgc atgaccgatc cgatctgggc     480
cggctcgggg cgttgtgctg ctgggagcac gtgcagccgt tgacgaaatt cgcgatgtac     540
gcgcagaacg agcaggttca cgtggcagca tggccgagct tctcgatgta cgaacccttt     600
gcgcatgcgc tgggttggga gacgaacaac gcggtcagca aggtctacgc ggtcgaggga     660
tcgtgcttcg tgctcgctcc ctgtgccgtt atttcgcaag cgatggtgga cgagatgtgc     720
gacactcccg acaagcgcga gcttgttcac gccggcggcg gccacgcggt gatttacggc     780
cctgacggaa gcccgctcgc agaaaagctc ggggaaaacg aagaggggct tctctacgcg     840
acggtcaatc ttgctgcgat cggggttgcc aagaatgccg cggatccggc cgggcactat     900
tcgcgtccgg acgttctaag gctgctattc aacaagagcc cggcccgaag agtggagcat     960
tttgcgctgc cgcacgagca gctcgagatc ggggcaggcc cgtctggcga ctga          1014

<210> 146
<211> 337
<212> PRT
<213> Unknown

<220>
<223> Obtained from an environmental sample

<400> 146
Met Gly Ile Thr His Pro Asn Tyr Lys Val Ala Val Val Gln Ala Ala
1               5                   10                  15
Pro Val Trp Leu Asn Leu Glu Ala Thr Val Glu Lys Thr Ile Arg Tyr
            20                  25                  30
Ile Glu Glu Ala Ala Lys Ala Gly Ala Lys Leu Ile Ala Phe Pro Glu
        35                  40                  45
Thr Trp Ile Pro Gly Tyr Pro Trp His Ile Trp Ile Gly Thr Pro Ala
    50                  55                  60
Trp Ala Ile Gly Lys Gly Phe Val Gln Arg Tyr Phe Asp Asn Ser Leu
65                  70                  75                  80
Ser Tyr Asp Ser Pro Leu Ala Arg Gln Ile Ala Asp Ala Ala Ala Lys
                85                  90                  95
Ser Lys Ile Thr Val Val Leu Gly Leu Ser Glu Arg Asp Gly Gly Ser
            100                 105                 110
Leu Tyr Ile Ala Gln Trp Leu Ile Gly Pro Asp Gly Glu Thr Ile Ala
        115                 120                 125
Lys Arg Arg Lys Leu Arg Pro Thr His Val Glu Arg Thr Val Phe Gly
    130                 135                 140

Asp Gly Asp Gly Ser His Ile Ala Val His Asp Arg Ser Asp Leu Gly
145                 150                 155                 160
Arg Leu Gly Ala Leu Cys Cys Trp Glu His Val Gln Pro Leu Thr Lys
                165                 170                 175
Phe Ala Met Tyr Ala Gln Asn Glu Gln Val His Val Ala Ala Trp Pro
            180                 185                 190
Ser Phe Ser Met Tyr Glu Pro Phe Ala His Ala Leu Gly Trp Glu Thr
        195                 200                 205
Asn Asn Ala Val Ser Lys Val Tyr Ala Val Glu Gly Ser Cys Phe Val
    210                 215                 220
Leu Ala Pro Cys Ala Val Ile Ser Gln Ala Met Val Asp Glu Met Cys
225                 230                 235                 240
Asp Thr Pro Asp Lys Arg Glu Leu Val His Ala Gly Gly Gly His Ala
                245                 250                 255
Val Ile Tyr Gly Pro Asp Gly Ser Pro Leu Ala Glu Lys Leu Gly Glu
            260                 265                 270
Asn Glu Glu Gly Leu Leu Tyr Ala Thr Val Asn Leu Ala Ala Ile Gly
        275                 280                 285
Val Ala Lys Asn Ala Ala Asp Pro Ala Gly His Tyr Ser Arg Pro Asp
    290                 295                 300
Val Leu Arg Leu Leu Phe Asn Lys Ser Pro Ala Arg Arg Val Glu His
305                 310                 315                 320
Phe Ala Leu Pro His Glu Gln Leu Glu Ile Gly Ala Gly Pro Ser Gly
                325                 330                 335
Asp

<210> 147
<211> 1098
<212> DNA
<213> Unknown

<220>
<223> Obtained from an environmental sample

<400> 147
atgacccagc acgagaccac tgcccggagg ctggcagctg tgcatgccgc gcctgtgttc      60
atggacaccg acgcgaccat cgacaaggtg atcggcttcg tcgaacaggc cggccgcgaa     120
ggcatcgaac tcctggtgtt ccccgagacc ttcgtgcctg gttaccccta ctggatcgag     180
tgctatccgc cgctgcagca ggtggccgcc aacgcgcagt acacggacgc ctccgtcgag     240
gtgcctggtc cggagatcaa gcgggtgcag gcggcctgtg cccgcgctgg cgtcgaagtc     300
gtcctcggcg tcagcgagcg actcaggggt accaggacat gcttcaactc ccaggtgttc     360
atcgacgccg acgggagcct gctcggcgtg caccgcaagc tgcagccgac gtacgtggag     420
cgcatcgtgt gggcccaggg cggaggcgcg accctgtcgg tgttcggctc ccgctccggc     480
cggatcggcg gtctggcctg ctgggagcac acgatgaacc tggctcgtca ggcactgctt     540
gagcaggagc agcagatcca cgcggcggcg tggcctgccc tgtcgacgat ggcggggttc     600
gagaccgtcg cggacgccca gatcgaggcc atgatgaaga cccatgcgct cacggcacag     660
gtgttcgtca tctgcgcgtc caacccggtc gacggcactt gcctggaatg gatgcgggac     720
aacctcggtg aacagaagtt cgtgaccgcc ggagggggct ggtccgcggt catccacccc     780
ttcaactcct tcctcggcgg gccgcatacc ggtttggagg agaagctcgt cagcgcgacg     840
atcgacttct ccgacatccg cttggtcaag gcctgggttg attcgaaggg gcactacgcg     900
cggcccgagg tcctgcgact cgcggtcgac cgcaagccac tgtggcacga cgagtgcgag     960
gtgccgggac aggcgcaggt acgcacccgc gctgcttctc tggcagtgca ggagcacccg    1020
gtggtgctgc ctcagggggc ggcgcggccc gctccgcaag actgggacac ctctgcggcg    1080
caggagctga cttcctga                                                  1098

<210> 148
<211> 365
<212> PRT
<213> Unknown

<220>
<223> Obtained from an environmental sample

<400> 148
Met Thr Gln His Glu Thr Thr Ala Arg Arg Leu Ala Ala Val His Ala
1               5                   10                  15
Ala Pro Val Phe Met Asp Thr Asp Ala Thr Ile Asp Lys Val Ile Gly

                20                  25                  30
Phe Val Glu Gln Ala Gly Arg Glu Gly Ile Glu Leu Leu Val Phe Pro
        35                  40                  45
Glu Thr Phe Val Pro Gly Tyr Pro Tyr Trp Ile Glu Cys Tyr Pro Pro
    50                  55                  60
Leu Gln Gln Val Ala Ala Asn Ala Gln Tyr Thr Asp Ala Ser Val Glu
65                  70                  75                  80
Val Pro Gly Pro Glu Ile Lys Arg Val Gln Ala Ala Cys Ala Arg Ala
                85                  90                  95
Gly Val Glu Val Val Leu Gly Val Ser Glu Arg Leu Arg Gly Thr Arg
            100                 105                 110
Thr Cys Phe Asn Ser Gln Val Phe Ile Asp Ala Asp Gly Ser Leu Leu
        115                 120                 125
Gly Val His Arg Lys Leu Gln Pro Thr Tyr Val Glu Arg Ile Val Trp
    130                 135                 140
Ala Gln Gly Gly Gly Ala Thr Leu Ser Val Phe Gly Ser Arg Ser Gly
145                 150                 155                 160
Arg Ile Gly Gly Leu Ala Cys Trp Glu His Thr Met Asn Leu Ala Arg
                165                 170                 175
Gln Ala Leu Leu Glu Gln Glu Gln Gln Ile His Ala Ala Ala Trp Pro
            180                 185                 190
Ala Leu Ser Thr Met Ala Gly Phe Glu Thr Val Ala Asp Ala Gln Ile
        195                 200                 205
Glu Ala Met Met Lys Thr His Ala Leu Thr Ala Gln Val Phe Val Ile
    210                 215                 220
Cys Ala Ser Asn Pro Val Asp Gly Thr Cys Leu Glu Trp Met Arg Asp
225                 230                 235                 240
Asn Leu Gly Glu Gln Lys Phe Val Thr Ala Gly Gly Gly Trp Ser Ala
                245                 250                 255
Val Ile His Pro Phe Asn Ser Phe Leu Gly Gly Pro His Thr Gly Leu
            260                 265                 270
Glu Glu Lys Leu Val Ser Ala Thr Ile Asp Phe Ser Asp Ile Arg Leu
        275                 280                 285
Val Lys Ala Trp Val Asp Ser Lys Gly His Tyr Ala Arg Pro Glu Val
    290                 295                 300
Leu Arg Leu Ala Val Asp Arg Lys Pro Leu Trp His Asp Glu Cys Glu
305                 310                 315                 320
Val Pro Gly Gln Ala Gln Val Arg Thr Arg Ala Ala Ser Leu Ala Val
                325                 330                 335
Gln Glu His Pro Val Val Leu Pro Gln Gly Ala Ala Arg Pro Ala Pro
            340                 345                 350
Gln Asp Trp Asp Thr Ser Ala Ala Gln Glu Leu Thr Ser
        355                 360                 365

<210> 149
<211> 942
<212> DNA
<213> Unknown

<220>
<223> Obtained from an environmental sample

<400> 149
atgacgaagc ttgagaaggt ggtcgcggcg gcggtccagg cgacgccgga gttcctcgac      60
cgcgaggcga ccgtcgagaa ggccgtgcgg ctgatcaagg aagcggccgg ggagggcgcc     120
ggcctgatcg tgttccccga gacgttcatc ccgacgtacc cggactgggt ctggcgcgcg     180
ccggcctggg acggcccatc cgcggacctg tacgcaatgc tgctggagaa cgcggtggag     240
atccccgggc cggtgacgga gaccctgggg aaggcggcga agcaggccaa ggccttcgtg     300
tcgatgggcg tcaacgagcg cgagccgggc ggcgggacga tctacaacac gcaggtcacg     360
ttcggacccg acgggagcgt gctcggcaag caccgcaagc tgatgccgac cggcggcgag     420
cgcctggtgt gggggatggg cgacgggtcg atgctccagg tctatgacac gccgttcggc     480
cgcctgggcg ggctgatctg ctgggagaac tacatgccgc tcgcgcgcta ctcgatgtac     540
gccaagggcg tggacgtcta cgttgcgccg acgtgggaca acagcgacat gtgggtggcg      600
acgctccgcc acatcgccaa ggaggggcgg ctgtacgtga tcggcgtggc gccgctgctg      660
cgcgggtcgg acgtccccga cgacgtgccg gggaaggccg agctgtgggg cggcgatgac      720
gactggatgt cgcgcggctt ctccaccatc gtcgcgccgg gcggcgaggt gctggccggt     780
ccgctgacgg aggaggaagg catcctctac gcggagatcg acccggcgag agcccgttcg     840
tcacggcacc agttcgatcc ggtggggcac tactcgcgcc ccgacgtgtt tcggctcgtc     900

gtggacgagt cgcccaagcc ccagacgtcc ggcccgggct ag                    942

<210> 150
<211> 313
<212> PRT
<213> Unknown

<220>
<223> Obtained from an environmental sample

<400> 150
Met Thr Lys Leu Glu Lys Val Val Ala Ala Ala Val Gln Ala Thr Pro
1               5                   10                  15
Glu Phe Leu Asp Arg Glu Ala Thr Val Glu Lys Ala Val Arg Leu Ile
            20                  25                  30
Lys Glu Ala Ala Gly Glu Gly Ala Gly Leu Ile Val Phe Pro Glu Thr
        35                  40                  45
Phe Ile Pro Thr Tyr Pro Asp Trp Val Trp Arg Ala Pro Ala Trp Asp
    50                  55                  60
Gly Pro Ser Ala Asp Leu Tyr Ala Met Leu Leu Glu Asn Ala Val Glu
65                  70                  75                  80
Ile Pro Gly Pro Val Thr Glu Thr Leu Gly Lys Ala Ala Lys Gln Ala
                85                  90                  95
Lys Ala Phe Val Ser Met Gly Val Asn Glu Arg Glu Pro Gly Gly Gly
            100                 105                 110
Thr Ile Tyr Asn Thr Gln Val Thr Phe Gly Pro Asp Gly Ser Val Leu
        115                 120                 125
Gly Lys His Arg Lys Leu Met Pro Thr Gly Gly Glu Arg Leu Val Trp
    130                 135                 140
Gly Met Gly Asp Gly Ser Met Leu Gln Val Tyr Asp Thr Pro Phe Gly
145                 150                 155                 160
Arg Leu Gly Gly Leu Ile Cys Trp Glu Asn Tyr Met Pro Leu Ala Arg
                165                 170                 175
Tyr Ser Met Tyr Ala Lys Gly Val Asp Val Tyr Val Ala Pro Thr Trp
            180                 185                 190
Asp Asn Ser Asp Met Trp Val Ala Thr Leu Arg His Ile Ala Lys Glu
        195                 200                 205
Gly Arg Leu Tyr Val Ile Gly Val Ala Pro Leu Leu Arg Gly Ser Asp
    210                 215                 220
Val Pro Asp Asp Val Pro Gly Lys Ala Glu Leu Trp Gly Gly Asp Asp
225                 230                 235                 240
Asp Trp Met Ser Arg Gly Phe Ser Thr Ile Val Ala Pro Gly Gly Glu
                245                 250                 255
Val Leu Ala Gly Pro Leu Thr Glu Glu Glu Gly Ile Leu Tyr Ala Glu
            260                 265                 270
Ile Asp Pro Ala Arg Ala Arg Ser Ser Arg His Gln Phe Asp Pro Val
        275                 280                 285
Gly His Tyr Ser Arg Pro Asp Val Phe Arg Leu Val Val Asp Glu Ser
    290                 295                 300
Pro Lys Pro Gln Thr Ser Gly Pro Gly
305                 310

<210> 151
<211> 993
<212> DNA
<213> Unknown

<220>
<223> Obtained from an environmental sample

<400> 151
atgagagtcg ttaaagccgc cgcggtccaa ctgaaaccag tcctttatag ccgtgaggga      60
acagtcgata acgtcgtcaa gaagatccac gagctgggcc aacaaggagt gcagttcgca     120
acgttcccgg aaaccgtggt gccttactat ccgtactttt cgatcgtgca gtccggctat     180
caaatccttg ccggcggtga gttcctaaag ctgcttgatc agtcagtgac cgtgccatct     240
cttgccaccg aagcgatcgg cgaggcctgc aggcaagcgg gcgtcgttgt ctccatcggc     300
gtcaacgagc gtgacggggg aactctgtac aatacgcaac ttctctttga tgccgacggc     360
acgttgattc aaagacgacg caagatcacg cccacccatt acgagcgcat ggtctggggc     420

cagggcgatg gctcaggttt acgggcggtt gacagcaagg tcgcgcgcat tggtcaactg 480
gcttgttttg agcactacaa cccgcttgcg cgttacgcca tgatggccga tggcgagcaa 540
atccactctg cgatgttccc gggctccatg ttcggcgatg cgttttcaga gaaggtggaa 600
atcaacgtaa ggcagcatgc aatggagtct ggatgctttg tcgtctgcgc tacggcctgg 660
ctggatgccg accaacaggc acaaatcatg aaggacacag gctgcgagat cggtccgatc 720
tcgggcggtt gcttcaccgc tatcgtgaca cccgacggga cgctgatagg cgaacccatc 780
cactcgggcg aaggcgtttg tattgccgac ctcgatttca agctcatcga caagcggaag 840
cacgtggtgg acacgcgcgg ccactacagc cggccagaat tgctcagcct cctaattgat 900
cggactccca cggcacacat acacgaacgg aacgagcaac cgaagtcggc cgttgagcaa 960
gactcgcaga atgtattcac cgctattgct taa 993

<210> 152
<211> 330
<212> PRT
<213> Unknown

<220>
<223> Obtained from an environmental sample

<400> 152
Met Arg Val Val Lys Ala Ala Ala Val Gln Leu Lys Pro Val Leu Tyr
1               5                   10                  15
Ser Arg Glu Gly Thr Val Asp Asn Val Val Lys Lys Ile His Glu Leu
            20                  25                  30
Gly Gln Gln Gly Val Gln Phe Ala Thr Phe Pro Glu Thr Val Val Pro
        35                  40                  45
Tyr Tyr Pro Tyr Phe Ser Ile Val Gln Ser Gly Tyr Gln Ile Leu Ala
    50                  55                  60
Gly Gly Glu Phe Leu Lys Leu Leu Asp Gln Ser Val Thr Val Pro Ser
65                  70                  75                  80
Leu Ala Thr Glu Ala Ile Gly Glu Ala Cys Arg Gln Ala Gly Val Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Tyr Glu Arg Met Val Trp Gly Gln Gly Asp Gly
    130                 135                 140
Ser Gly Leu Arg Ala Val Asp Ser Lys Val Ala Arg Ile Gly Gln Leu
145                 150                 155                 160
Ala Cys Phe Glu His Tyr Asn Pro Leu Ala Arg Tyr Ala Met Met Ala
                165                 170                 175
Asp Gly Glu Gln Ile His Ser Ala Met Phe Pro Gly Ser Met Phe Gly
            180                 185                 190
Asp Ala Phe Ser Glu Lys Val Glu Ile Asn Val Arg Gln His Ala Met
        195                 200                 205
Glu Ser Gly Cys Phe Val Val Cys Ala Thr Ala Trp Leu Asp Ala Asp
    210                 215                 220
Gln Gln Ala Gln Ile Met Lys Asp Thr Gly Cys Glu Ile Gly Pro Ile
225                 230                 235                 240
Ser Gly Gly Cys Phe Thr Ala Ile Val Thr Pro Asp Gly Thr Leu Ile
                245                 250                 255
Gly Glu Pro Ile His Ser Gly Glu Gly Val Cys Ile Ala Asp Leu Asp
            260                 265                 270
Phe Lys Leu Ile Asp Lys Arg Lys His Val Val Asp Thr Arg Gly His
        275                 280                 285
Tyr Ser Arg Pro Glu Leu Leu Ser Leu Leu Ile Asp Arg Thr Pro Thr
    290                 295                 300
Ala His Ile His Glu Arg Asn Glu Gln Pro Lys Ser Ala Val Glu Gln
305                 310                 315                 320
Asp Ser Gln Asn Val Phe Thr Ala Ile Ala
                325                 330

<210> 153
<211> 1074
<212> DNA
<213> Unknown

<220>
<223> Obtained from an environmental sample

<400> 153
atgccaaacg caagaaagat tgttggagcc gtggcccaag ttgcacagga attcttcgac 60
actgaagcga atctcggtaa agcgatagcg gcgattcaca atgctgcgaa gcaaggcgca 120
gatatcgtcg tcttcgccga atgctatttg ggccaatatc catattgggc gcaattttac 180
gacaactctg ccaagaacta ttccaaggtt tggacggccc tgtacgacgg tgcgatcact 240
gtgggtggcg atgaatgccg ggctattgct gctgcggcta gacagtccaa gattcatgtc 300
gtcatgggtt gcaatgagct atccgaccga gccggcggcg caacgttata caacagcctc 360
ttgtttttcg accgaaaggg cgagttgatc ggtcgacacc ggaaattgat gccgtcgatg 420
cacgagcggt tgatccatgg cacaggcgac ggaagagact tgaatgttta cgataccgat 480
atcggtatgt tgggtgggtt gatttgctgg gagcaccata tgtcgctctc gaagtatgcc 540
atggcgacta tgggtgaaga agttcatgtt gcaagctggc ctgggatgtg gcgcggagga 600
gacgcggcaa tcggtgagag gatggtcgaa gcggatcttg ggcgccgttt tgtttgtgac 660
gccgaatttg cgatccgaga atatgcggca gagacaggaa atttcgttct aagcgcgtct 720
ggatattttc cgaaggacaa tatatccgat gagtggcgcg aagcgattcc aaaccttcaa 780
gcgcagtggg ctgtgggcgg gagttctatc gtggcaccgg ggggctccta tctggtccca 840
ccactcatta atgaggagaa gatcctctgc gccgaactcg atttcaatct caggcgtctt 900
tggaaagcct ggatcgatcc gattggtcac tattcgcgtc ccgatgttta tagcctgcaa 960
ctgcataacg ttgctgggcg tgagtattcc tatcaggccg tagatttgaa gcgcacgcca 1020
aagccccaat cgctgtgggt agatgcgtcc gaggaagacg gtgcgctgaa ttga 1074

<210> 154
<211> 357
<212> PRT
<213> Unknown

<220>
<223> Obtained from an environmental sample

<400> 154
   Met Pro Asn Ala Arg Lys Ile Val Gly Ala Val Ala Gln Val Ala Gln
   1 5 10 15
   Glu Phe Phe Asp Thr Glu Ala Asn Leu Gly Lys Ala Ile Ala Ala Ile
   20 25 30
   His Asn Ala Ala Lys Gln Gly Ala Asp Ile Val Val Phe Ala Glu Cys
   35 40 45
   Tyr Leu Gly Gln Tyr Pro Tyr Trp Ala Gln Phe Tyr Asp Asn Ser Ala
   50 55 60
   Lys Asn Tyr Ser Lys Val Trp Thr Ala Leu Tyr Asp Gly Ala Ile Thr
   65 70 75 80
   Val Gly Gly Asp Glu Cys Arg Ala Ile Ala Ala Ala Ala Arg Gln Ser
   85 90 95
   Lys Ile His Val Val Met Gly Cys Asn Glu Leu Ser Asp Arg Ala Gly
   100 105 110
   Gly Ala Thr Leu Tyr Asn Ser Leu Leu Phe Phe Asp Arg Lys Gly Glu
   115 120 125
   Leu Ile Gly Arg His Arg Lys Leu Met Pro Ser Met His Glu Arg Leu
   130 135 140
   Ile His Gly Thr Gly Asp Gly Arg Asp Leu Asn Val Tyr Asp Thr Asp
   145 150 155 160
   Ile Gly Met Leu Gly Gly Leu Ile Cys Trp Glu His His Met Ser Leu
   165 170 175
   Ser Lys Tyr Ala Met Ala Thr Met Gly Glu Glu Val His Val Ala Ser
   180 185 190
   Trp Pro Gly Met Trp Arg Gly Gly Asp Ala Ala Ile Gly Glu Arg Met
   195 200 205
   Val Glu Ala Asp Leu Gly Ala Pro Phe Val Cys Asp Ala Glu Phe Ala
   210 215 220
   Ile Arg Glu Tyr Ala Ala Glu Thr Gly Asn Phe Val Leu Ser Ala Ser
   225 230 235 240
   Gly Tyr Phe Pro Lys Asp Asn Ile Ser Asp Glu Trp Arg Glu Ala Ile
   245 250 255
   Pro Asn Leu Gln Ala Gln Trp Ala Val Gly Gly Ser Ser Ile Val Ala
   260 265 270
   Pro Gly Gly Ser Tyr Leu Val Pro Pro Leu Ile Asn Glu Glu Lys Ile
   275 280 285
   Leu Cys Ala Glu Leu Asp Phe Asn Leu Arg Arg Leu Trp Lys Ala Trp
       290                 295                 300
   Ile Asp Pro Ile Gly His Tyr Ser Arg Pro Asp Val Tyr Ser Leu Gln
   305                 310                 315                 320
   Leu His Asn Val Ala Gly Arg Glu Tyr Ser Tyr Gln Ala Val Asp Leu
                   325                 330                 335
   Lys Arg Thr Pro Lys Pro Gln Ser Leu Trp Val Asp Ala Ser Glu Glu
               340                 345                 350
   Asp Gly Ala Leu Asn
           355

<210> 155
<211> 1041
<212> DNA
<213> Unknown

<220>
<223> Obtained from an environmental sample

<400> 155
atgggcatcg aacatccgaa atacaaggtg gccgtggtgc aggccgcgcc ggcctggctc      60
gatctcgacg gctcgatcaa gaaggcgatt gcgctgatcg aggaagcggc cgccaagggc     120
gctaagctga tcgctttccc cgaaaccttc attcccggct atccctggca catctggctg     180
gactcgccgg cctgggcgat cggccgcggc tttgtgcagc gctacttcga taactcgctg     240
gcctacgaca gcccgcaagc cgaaaagctg cgcgccgcgg tcaagaaggc caagctcact     300
gccgtgattg gcctgtcgga gcgcgacggc ggcagcctct atatagcgca atggctgatt     360
ggccctgatg gcgagaccat cgcaaaacgc agaaagctgc ggccaacgca cgcggaacgc     420
accgtttttg gcgagggtga cggcagcgac cttgccgtgc acgaccggcc cggaatcggg     480
cggctgggag cgctgtgctg ctgggagcac ctgcaaccgc tttcgaaata cgcgatgtat     540
gcgcagaacg aacaggtcca tgtcgcgtca tggccgagct tctcgctcta cgaccccttc     600
gcgccggcgc tcggcgccga ggtcaacaat gcggcttccc gcgtctacgc ggtcgagggc     660
tcgtgcttcg tgctggcgcc gtgcgccacg gtttcgcaag ccatgatcga cgagctgtgt     720
gaccggccgg acaagcatgc gctgttgcac gccggtggcg gacacgccgc gatttacggc     780
ccggacggca gctcgatcgc ggagaagctg ccgcaggacg cggagggcct gttgatcgcc     840
gagatcgatc tcggggcgat cggggttgcc aagaatgcag ccgacccggc cggtcattat     900
tcgcggccgg acgtgacgcg actcctgctg aacaagaacc ggatgcgaag ggtcgaggag     960
tttgcgctgc cggtcgatcc ggtcgcaacg accgaggagg agcaagtcgc gacgccgtcg    1020
aggcccagcc aggccgcgta a                                              1041

<210> 156
<211> 346
<212> PRT
<213> Unknown

<220>
<223> Obtained from an environmental sample

<400> 156
Met Gly Ile Glu His Pro Lys Tyr Lys Val Ala Val Val Gln Ala Ala
1               5                   10                  15
Pro Ala Trp Leu Asp Leu Asp Gly Ser Ile Lys Lys Ala Ile Ala Leu
            20                  25                  30
Ile Glu Glu Ala Ala Ala Lys Gly Ala Lys Leu Ile Ala Phe Pro Glu
        35                  40                  45
Thr Phe Ile Pro Gly Tyr Pro Trp His Ile Trp Leu Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Lys Leu Arg Ala Ala Val Lys Lys
                85                  90                  95
Ala Lys Leu Thr Ala Val Ile Gly Leu Ser Glu Arg Asp Gly Gly Ser
            100                 105                 110
Leu Tyr Ile Ala Gln Trp Leu Ile Gly Pro Asp Gly Glu Thr Ile Ala
        115                 120                 125
Lys Arg Arg Lys Leu Arg Pro Thr His Ala Glu Arg Thr Val Phe Gly
    130                 135                 140
Glu Gly Asp Gly Ser Asp Leu Ala Val His Asp Arg Pro Gly Ile Gly
145                 150                 155                 160

Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ser Trp Pro
            180                 185                 190
Ser Phe Ser Leu Tyr Asp Pro Phe Ala Pro Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Ala Gly Gly Gly His Ala
                245                 250                 255
Ala Ile Tyr Gly Pro Asp Gly Ser Ser Ile Ala Glu Lys Leu Pro Gln
            260                 265                 270
Asp Ala Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Asn Arg Met Arg Arg Val Glu Glu
305                 310                 315                 320
Phe Ala Leu Pro Val Asp Pro Val Ala Thr Thr Glu Glu Glu Gln Val
                325                 330                 335
Ala Thr Pro Ser Arg Pro Ser Gln Ala Ala
                340                 345

<210> 157
<211> 1011
<212> DNA
<213> Unknown

<220>
<223> Obtained from an environmental sample

<400> 157
atgagagtcg ttaaagctgc tgcggtccaa ctgagtcccg tgctgtatag ccgtgaggga      60
acagtagaaa aggtcgttcg gaagatccac gagcttggcg atcaaggagt cgagttcgcc     120
acgttcccgg agaccgtagt gccctactat ccgtacttct cggccgtcca gacgccgatt     180
cagaacatgc acggcccgga gcacctgaag ttgctcgagc aatcggtgac cgtcccgtcg     240
cccgccaccg acgcgatcgg cgacgcctgc cgccacgccg gcgtcgtcgt ctcgatcggc     300
gtcaacgaac gcgatggcgg cacgatctac aacacgcagc tcctgttcga cgccgacggc     360
accttgatcc agcgccggcg aaagatcacg ccgaccttct acgaacgaat ggtctgggga     420
cagggtgacg gttcggggct gcgcgccgtc gacagccgcg taggacgcat cggccagctc     480
gcctgtttcg agcactacaa cccgctggcg cgctacgcca tgatggccga cggcgagcag     540
attcactccg cgatgtaccc cggctccatc tttggagacg cattcgcgca gaaaatcgag     600
atcaacatcc gccagcacgc gctcgagtcc ggtgcgttcg tcgtcaacgc caccgcctgg     660
ctcgatgccg accagcaggc gcggatcatg aaggataccg gctgcaccat cgaaccgatc     720
tcgggcggtt gcttcaccgc catcgtcacc ccggacggga ccctgctggg cgaagcgata     780
cgttcggggg agggagtggt ggtcgccgat ctcgacttca cgctgatcga caggcgcaag     840
caagtgatgg actctcgtgg tcactacagt cggccggagt tgctcagcct tctgatcgac     900
cgcacaccca ccgcacacct acacgaacgc gaagcgcacc ccagagcaag tgaggactgg     960
caaggttccg agagtctgcg cgccatgcag gcctcggcac cgaaggtctg a             1011

<210> 158
<211> 336
<212> PRT
<213> Unknown

<220>
<223> Obtained from an environmental sample

<400> 158
Met Arg Val Val Lys Ala Ala Ala Val Gln Leu Ser Pro Val Leu Tyr
1               5                   10                  15
Ser Arg Glu Gly Thr Val Glu Lys Val Val Arg Lys Ile His Glu Leu
            20                  25                  30
Gly Asp Gln Gly Val Glu Phe Ala Thr Phe Pro Glu Thr Val Val Pro
        35                  40                  45
Tyr Tyr Pro Tyr Phe Ser Ala Val Gln Thr Pro Ile Gln Asn Met His

50                  55                  60
Gly Pro Glu His Leu Lys Leu Leu Glu Gln Ser Val Thr Val Pro Ser
65                  70                  75                  80
Pro Ala Thr Asp Ala Ile Gly Asp Ala Cys Arg His Ala Gly Val Val
                85                  90                  95
Val Ser Ile Gly Val Asn Glu Arg Asp Gly Gly Thr Ile Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr Phe Tyr Glu Arg Met Val Trp Gly Gln Gly Asp Gly
    130                 135                 140
Ser Gly Leu Arg Ala Val Asp Ser Arg Val Gly Arg Ile Gly Gln Leu
145                 150                 155                 160
Ala Cys Phe Glu His Tyr Asn Pro Leu Ala Arg Tyr Ala Met Met Ala
                165                 170                 175
Asp Gly Glu Gln Ile His Ser Ala Met Tyr Pro Gly Ser Ile Phe Gly
            180                 185                 190
Asp Ala Phe Ala Gln Lys Ile Glu Ile Asn Ile Arg Gln His Ala Leu
        195                 200                 205
Glu Ser Gly Ala Phe Val Val Asn Ala Thr Ala Trp Leu Asp Ala Asp
    210                 215                 220
Gln Gln Ala Arg Ile Met Lys Asp Thr Gly Cys Thr Ile Glu Pro Ile
225                 230                 235                 240
Ser Gly Gly Cys Phe Thr Ala Ile Val Thr Pro Asp Gly Thr Leu Leu
                245                 250                 255
Gly Glu Ala Ile Arg Ser Gly Glu Gly Val Val Val Ala Asp Leu Asp
            260                 265                 270
Phe Thr Leu Ile Asp Arg Arg Lys Gln Val Met Asp Ser Arg Gly His
        275                 280                 285
Tyr Ser Arg Pro Glu Leu Leu Ser Leu Leu Ile Asp Arg Thr Pro Thr
    290                 295                 300
Ala His Leu His Glu Arg Glu Ala His Pro Arg Ala Ser Glu Asp Trp
305                 310                 315                 320
Gln Gly Ser Glu Ser Leu Arg Ala Met Gln Ala Ser Ala Pro Lys Val
                325                 330                 335

<210> 159
<211> 930
<212> DNA
<213> Unknown

<220>
<223> Obtained from an environmental sample

<400> 159
atgtcatcaa ccgtgacggt tgccattatt caggcagcac ccgtgtatta tgacctgcct      60
gccacgctgg acaaagccgc caaactggtg gcggatgcgg cggcacaggg cgcaacgctg     120
attgtcttcg gcgagacatg gtttccgggg tatccggcat ggctggatta ctgccccaat     180
gtcgcgctgt ggaatcatcc cccgaccaag caggtatttg agcgcctgca tcgcaacagc     240
atcgctgtgc caagcaagga actcgatttt ctgggggcgc tggcacgcaa gcatcaggtg     300
gtgctggtgt tgagcattaa tgaacgtgtg gagcagggcg cggggcatgg cacgctgtat     360
aacacgctgc tcacgattga cgccgatggc acgctggcaa atcatcatcg caaactgatg     420
ccgacctata ccgagcgcat ggtgtggggc atgggcgacg gggtggggtt gcaagcggtg     480
gatactgccg tcgggcgcgt aggcggctta atctgctggg aacactggat gccgttggca     540
cgccagacca tgcacatcag cggcgaacag attcatattt ccgtcttccc aaccgtccat     600
gagatgcacc agattgccag ccgccagtat gcctttgaag ggcggacgtt tgtgctgacc     660
gttggcggca ttcttgcggc acaggacttg cccgccgaac tggaacgccc cgccgatttg     720
ccgcccacgc agcttgtcca gcgcggcggc agcgccatta tcgcgccgga tggtcgttat     780
ctggcgggtc cagtctataa tgaggaaacc atcctgaccg caacgctgga tttgggcgag     840
atcatccgcg agagcatgac gctggatgtc accggacatt atgcccgccc ggatgttttt     900
gacctgaccg tgaagcgcag ccgaccatga                                      930

<210> 160
<211> 309
<212> PRT
<213> Unknown

<220>

<223> Obtained from an environmental sample

<400> 160
Met Ser Ser Thr Val Thr Val Ala Ile Ile Gln Ala Ala Pro Val Tyr
1               5                   10                  15
Tyr Asp Leu Pro Ala Thr Leu Asp Lys Ala Ala Lys Leu Val Ala Asp
            20                  25                  30
Ala Ala Ala Gln Gly Ala Thr Leu Ile Val Phe Gly Glu Thr Trp Phe
        35                  40                  45
Pro Gly Tyr Pro Ala Trp Leu Asp Tyr Cys Pro Asn Val Ala Leu Trp
    50                  55                  60
Asn His Pro Pro Thr Lys Gln Val Phe Glu Arg Leu His Arg Asn Ser
65                  70                  75                  80
Ile Ala Val Pro Ser Lys Glu Leu Asp Phe Leu Gly Ala Leu Ala Arg
                85                  90                  95
Lys His Gln Val Val Leu Val Leu Ser Ile Asn Glu Arg Val Glu Gln
            100                 105                 110
Gly Ala Gly His Gly Thr Leu Tyr Asn Thr Leu Leu Thr Ile Asp Ala
        115                 120                 125
Asp Gly Thr Leu Ala Asn His His Arg Lys Leu Met Pro Thr Tyr Thr
    130                 135                 140
Glu Arg Met Val Trp Gly Met Gly Asp Gly Val Gly Leu Gln Ala Val
145                 150                 155                 160
Asp Thr Ala Val Gly Arg Val Gly Gly Leu Ile Cys Trp Glu His Trp
                165                 170                 175
Met Pro Leu Ala Arg Gln Thr Met His Ile Ser Gly Glu Gln Ile His
            180                 185                 190
Ile Ser Val Phe Pro Thr Val His Glu Met His Gln Ile Ala Ser Arg
        195                 200                 205
Gln Tyr Ala Phe Glu Gly Arg Thr Phe Val Leu Thr Val Gly Gly Ile
    210                 215                 220
Leu Ala Ala Gln Asp Leu Pro Ala Glu Leu Glu Arg Pro Ala Asp Leu
225                 230                 235                 240
Pro Pro Thr Gln Leu Val Gln Arg Gly Gly Ser Ala Ile Ile Ala Pro
                245                 250                 255
Asp Gly Arg Tyr Leu Ala Gly Pro Val Tyr Asn Glu Glu Thr Ile Leu
            260                 265                 270
Thr Ala Thr Leu Asp Leu Gly Glu Ile Ile Arg Glu Ser Met Thr Leu
        275                 280                 285
Asp Val Thr Gly His Tyr Ala Arg Pro Asp Val Phe Asp Leu Thr Val
    290                 295                 300
Lys Arg Ser Arg Pro
305

<210> 161
<211> 1008
<212> DNA
<213> Unknown

<220>
<223> Obtained from an environmental sample

<400> 161
atgaccacca tccgcgccgc cgccgtgcag tttagcccgg tgctgtactc gcgccaggcc      60
accgtcgaca agctgtgccg caccctgctg gaactgggcc gcgaaggggt gcagttcgcg     120
gtattcccgg aaaccgtggt gccgtactac ccatattttt ccttcgtgca gccaccgttc     180
gccatgggca aacaacacct gttgctgctc gagcaatccg tcactgtgcc ctctgacgtc     240
acccggcaga tcggtgaggc ctgccgggaa gcggggatcg tcgccagcat cggcgtcaac     300
gaacgcgacg gcggcactat ttataacgcg cagttgctgt tcgatgccga cggcagcctg     360
attcagcagc ggcgcaagat caccccgacc tatcacgaac gcatggtctg ggggcagggc      420
gatggttccg gcctgcgcgc cgtggacagt gcggtggggc gtatcggttc cctggcctgc     480
tgggaacatt acaaccccct ggcgcgctac gcgctgatgg ccgatggcga acagattcat     540
gtggcgatgt ttcccggctc cctggtcggc gacatctttg ccgagcagat cgaagtcacc     600
atccgccacc acgccctgga aagcggctgc ttcgtggtca acgccacggc ttggctggat     660
gccgaccagc agggccggat catgcaggac accggctgcg agttggggcc gatttccggc     720
ggctgtttta ccgcgatcat ttccccggag ggcaaggttc tcggcgagcc gctgcgcagc     780
ggcgaagggg tggtcattgc tgacctcgac ctggccctga tcgacaagcg caaacgcatg     840
atggattcgg tcggtcacta cagccgcccg gaactgctca gcctgcttat cgaccgcagc     900

ccgaccgccc acgtgcatga acttgccgcc gcgcttaatc ctgccaggga gtctgatcca 960
ctagtgtcga cctgcaggcg cgcgagctcc agcttttgtt ccctttag 1008

<210> 162
<211> 335
<212> PRT
<213> Unknown

<220>
<223> Obtained from an environmental sample

<400> 162
Met Thr Thr Ile Arg Ala Ala Ala Val Gln Phe Ser Pro Val Leu Tyr
1               5                   10                  15
Ser Arg Gln Ala Thr Val Asp Lys Leu Cys Arg Thr Leu Leu Glu Leu
            20                  25                  30
Gly Arg Glu Gly Val Gln Phe Ala Val Phe Pro Glu Thr Val Val Pro
        35                  40                  45
Tyr Tyr Pro Tyr Phe Ser Phe Val Gln Pro Pro Phe Ala Met Gly Lys
    50                  55                  60
Gln His Leu Leu Leu Leu Glu Gln Ser Val Thr Val Pro Ser Asp Val
65                  70                  75                  80
Thr Arg Gln Ile Gly Glu Ala Cys Arg Glu Ala Gly Ile Val Ala Ser
                85                  90                  95
Ile Gly Val Asn Glu Arg Asp Gly Gly Thr Ile Tyr Asn Ala Gln Leu
            100                 105                 110
Leu Phe Asp Ala Asp Gly Ser Leu Ile Gln Gln Arg Arg Lys Ile Thr
        115                 120                 125
Pro Thr Tyr His Glu Arg Met Val Trp Gly Gln Gly Asp Gly Ser Gly
    130                 135                 140
Leu Arg Ala Val Asp Ser Ala Val Gly Arg Ile Gly Ser Leu Ala Cys
145                 150                 155                 160
Trp Glu His Tyr Asn Pro Leu Ala Arg Tyr Ala Leu Met Ala Asp Gly
                165                 170                 175
Glu Gln Ile His Val Ala Met Phe Pro Gly Ser Leu Val Gly Asp Ile
            180                 185                 190
Phe Ala Glu Gln Ile Glu Val Thr Ile Arg His His Ala Leu Glu Ser
        195                 200                 205
Gly Cys Phe Val Val Asn Ala Thr Ala Trp Leu Asp Ala Asp Gln Gln
    210                 215                 220
Gly Arg Ile Met Gln Asp Thr Gly Cys Glu Leu Gly Pro Ile Ser Gly
225                 230                 235                 240
Gly Cys Phe Thr Ala Ile Ile Ser Pro Glu Gly Lys Val Leu Gly Glu
                245                 250                 255
Pro Leu Arg Ser Gly Glu Gly Val Val Ile Ala Asp Leu Asp Leu Ala
            260                 265                 270
Leu Ile Asp Lys Arg Lys Arg Met Met Asp Ser Val Gly His Tyr Ser
        275                 280                 285
Arg Pro Glu Leu Leu Ser Leu Leu Ile Asp Arg Ser Pro Thr Ala His
    290                 295                 300
Val His Glu Leu Ala Ala Ala Leu Asn Pro Ala Arg Glu Ser Asp Pro
305                 310                 315                 320
Leu Val Ser Thr Cys Arg Arg Ala Ser Ser Ser Phe Cys Ser Leu
                325                 330                 335

<210> 163
<211> 978
<212> DNA
<213> Unknown

<220>
<223> Obtained from an environmental sample

<400> 163
gtgaccatca tcaaagccgc cgcagtgcag atcagccccg tgctttacag ccgggaagcc 60
accgtcgaaa aggtcgttcg cgagacccgc gaactcggcc agaagggcgt gcagttcgca 120
acgtttccgg aaaccgtggt gccgtactac ccatacttct ccgccgtcca gacgggcatc 180
gaactgctgt ccggcaaaga gcacctgcga ctgctggagc aggccgtgac tgttccttcc 240

cccgccactg atgcgattgc ccaggcggca cgcgaggccg gcatggtggt gtcgatcggc 300
gtcaacgagc gtgacggcgg caccatctac aacacgcagc tgctctttga tgccgacggc 360
acgctggtgc agcgccgccg caagatcacg ccgacgcatt tcgagcgcat ggtgtggggc 420
cagggcgacg gttcgggcct gcgcgcagtg gataccaagg tcggccgcat tggccagctg 480
gcctgcttcg agcacaacaa cccgctcgcg cgctacgcaa tgatggccga tggcgagcag 540
atccattcct ccatgtaccc gggctccgcc ttcggcgacg gattcgcgca gcgcatggag 600
atcaacattc gccaacacgc cctggagtcg ggttgcttcg tggtgaatgc caccgcgtgg 660
ctcgacgccg accagcaggc gcagatcatg aaggacacgg gctgcgccat cgggccgatc 720
tctggcggct gcttcacgac catcgtcacg ccggacggca tgctgatcgg cgaacccctc 780
cgcgagggcg agggcgagat catcgccgac ctcgatttca ccctgatcga ccgccgcaag 840
ctgctgatgg actcggtcgg ccactacaac cgtccggagc tgctgagcct gctgatcgac 900
cgcacacccg cggcgaactt ccatgagcgc agtacgcatc cggccgtcga tgccgccagc 960
ggcctcgaaa tcctctaa 978

<210> 164
<211> 325
<212> PRT
<213> Unknown

<220>
<223> Obtained from an environmental sample

<400> 164
Val Thr Ile Ile Lys Ala Ala Ala Val Gln Ile Ser Pro Val Leu Tyr
1               5                   10                  15
Ser Arg Glu Ala Thr Val Glu Lys Val Val Arg Glu Thr Arg Glu Leu
            20                  25                  30
Gly Gln Lys Gly Val Gln Phe Ala Thr Phe Pro Glu Thr Val Val Pro
        35                  40                  45
Tyr Tyr Pro Tyr Phe Ser Ala Val Gln Thr Gly Ile Glu Leu Leu Ser
    50                  55                  60
Gly Lys Glu His Leu Arg Leu Leu Glu Gln Ala Val Thr Val Pro Ser
65                  70                  75                  80
Pro Ala Thr Asp Ala Ile Ala Gln Ala Ala Arg Glu Ala Gly Met Val
                85                  90                  95
Val Ser Ile Gly Val Asn Glu Arg Asp Gly Gly Thr Ile Tyr Asn Thr
            100                 105                 110
Gln Leu Leu Phe Asp Ala Asp Gly Thr Leu Val Gln Arg Arg Arg Lys
        115                 120                 125
Ile Thr Pro Thr His Phe Glu Arg Met Val Trp Gly Gln Gly Asp Gly
    130                 135                 140
Ser Gly Leu Arg Ala Val Asp Thr Lys Val Gly Arg Ile Gly Gln Leu
145                 150                 155                 160
Ala Cys Phe Glu His Asn Asn Pro Leu Ala Arg Tyr Ala Met Met Ala
                165                 170                 175
Asp Gly Glu Gln Ile His Ser Ser Met Tyr Pro Gly Ser Ala Phe Gly
            180                 185                 190
Asp Gly Phe Ala Gln Arg Met Glu Ile Asn Ile Arg Gln His Ala Leu
        195                 200                 205
Glu Ser Gly Cys Phe Val Val Asn Ala Thr Ala Trp Leu Asp Ala Asp
    210                 215                 220
Gln Gln Ala Gln Ile Met Lys Asp Thr Gly Cys Ala Ile Gly Pro Ile
225                 230                 235                 240
Ser Gly Gly Cys Phe Thr Thr Ile Val Thr Pro Asp Gly Met Leu Ile
                245                 250                 255
Gly Glu Pro Leu Arg Glu Gly Glu Gly Glu Ile Ile Ala Asp Leu Asp
            260                 265                 270
Phe Thr Leu Ile Asp Arg Arg Lys Leu Leu Met Asp Ser Val Gly His
        275                 280                 285
Tyr Asn Arg Pro Glu Leu Leu Ser Leu Leu Ile Asp Arg Thr Pro Ala
    290                 295                 300
Ala Asn Phe His Glu Arg Ser Thr His Pro Ala Val Asp Ala Ala Ser
305                 310                 315                 320
Gly Leu Glu Ile Leu
                325

<210> 165
<211> 1008

<212> DNA
<213> Unknown

<220>
<223> Obtained from an environmental sample

<400> 165
atggccaatt tcaaattcaa ggcggcggcg gtgcaggccg cgcccgcttt cctcgatctc 60
gaggctagca tcgccaagtc gatcgccctg atcgaacaag ccgccgccaa cggcgccaag 120
ctgatcgcct ttcccgaagt cttcattccc ggctacccct ggcacatctg gctcgacagt 180
cccgcctggg cgatcgggcg cggcttcgtc tcgcgctatt tcgagaactc gctggactac 240
aacagccccg aggccgagcg cctcaggctc gccgtcaaga aggcgggcct gacggcggtg 300
atcggcctct ccgagcgcga cggcggcagc ctctacatcg cgcaatggat catcggccct 360
gacggcgaga ccgttgcgaa acggcgtaag ctccggccga cccattgcga gcgcacggtc 420
tatggagaag gcgacggcag cgacctcgcg gttcacgacg tatctggcat cggccgtctc 480
ggcgcgctct gctgctggga gcatatccag ccgctgtcga aattcgcgat gtattcgcaa 540
aatgagcaag tgcacgtcgc gtcctggccg agcttctcgc tctacgaccc gttcgcgccg 600
gcgctgggcg ccgaggtcaa caacgcagcc tcgcggatct atgcggtcga aggctcatgc 660
ttcgtcattg cgccctgcgc gaccgtttcg cctgcaatga tcgaggaact gtgcgacgcg 720
ccaaacaaac atgcgcttct gcacgcgggc ggcggcttcg cgcgcatcta tggcccggac 780
ggcgcttcga tcgccgagac gctgccgcca gatcaggaag gcttgatcta cgccgacatc 840
gacctcaccg cgatcggcgt cgccaaggcc gccgccgatc ccgccggcca ttattcgcgc 900
cccgacgtca cgcgcctgct cttcaacaag aagcccgctc ggcgagtcga aacttttgct 960
ttgcccgtcg atgcgccggc gccggagacg cagaccgccg cgagctga 1008

<210> 166
<211> 335
<212> PRT
<213> Unknown

<220>
<223> Obtained from an environmental sample

<400> 166
Met Ala Asn Phe Lys Phe Lys Ala Ala Ala Val Gln Ala Ala Pro Ala
1               5                   10                  15
Phe Leu Asp Leu Glu Ala Ser Ile Ala Lys Ser Ile Ala Leu Ile Glu
            20                  25                  30
Gln Ala Ala Ala Asn Gly Ala Lys Leu Ile Ala Phe Pro Glu Val Phe
        35                  40                  45
Ile Pro Gly Tyr Pro Trp His Ile Trp Leu Asp Ser Pro Ala Trp Ala
    50                  55                  60
Ile Gly Arg Gly Phe Val Ser Arg Tyr Phe Glu Asn Ser Leu Asp Tyr
65                  70                  75                  80
Asn Ser Pro Glu Ala Glu Arg Leu Arg Leu Ala Val Lys Lys Ala Gly
                85                  90                  95
Leu Thr Ala Val Ile Gly Leu Ser Glu Arg Asp Gly Gly Ser Leu Tyr
            100                 105                 110
Ile Ala Gln Trp Ile Ile Gly Pro Asp Gly Glu Thr Val Ala Lys Arg
        115                 120                 125
Arg Lys Leu Arg Pro Thr His Cys Glu Arg Thr Val Tyr Gly Glu Gly
    130                 135                 140
Asp Gly Ser Asp Leu Ala Val His Asp Val Ser Gly Ile Gly Arg Leu
145                 150                 155                 160
Gly Ala Leu Cys Cys Trp Glu His Ile Gln Pro Leu Ser Lys Phe Ala
                165                 170                 175
Met Tyr Ser Gln Asn Glu Gln Val His Val Ala Ser Trp Pro Ser Phe
            180                 185                 190
Ser Leu Tyr Asp Pro Phe Ala Pro Ala Leu Gly Ala Glu Val Asn Asn
        195                 200                 205
Ala Ala Ser Arg Ile Tyr Ala Val Glu Gly Ser Cys Phe Val Ile Ala
    210                 215                 220
Pro Cys Ala Thr Val Ser Pro Ala Met Ile Glu Glu Leu Cys Asp Ala
225                 230                 235                 240
Pro Asn Lys His Ala Leu Leu His Ala Gly Gly Gly Phe Ala Arg Ile
                245                 250                 255
Tyr Gly Pro Asp Gly Ala Ser Ile Ala Glu Thr Leu Pro Pro Asp Gln
            260                 265                 270

Glu Gly Leu Ile Tyr Ala Asp Ile Asp Leu Thr Ala Ile Gly Val Ala
        275                 280                 285
Lys Ala Ala Ala Asp Pro Ala Gly His Tyr Ser Arg Pro Asp Val Thr
    290                 295                 300
Arg Leu Leu Phe Asn Lys Lys Pro Ala Arg Arg Val Glu Thr Phe Ala
305                 310                 315                 320
Leu Pro Val Asp Ala Pro Ala Pro Glu Thr Gln Thr Ala Ala Ser
                325                 330                 335

<210> 167
<211> 1017
<212> DNA
<213> Unknown

<220>
<223> Obtained from an environmental sample

<400> 167
atgggtattg aacatccgaa gtacagggtt gccgtggtgc aggccgcacc ggcctggctc      60
gatcttgacg cgtcgatcga caagtcgatc gcgctgatcg aggaggctgc ccagaaaggc     120
gccaagctga tcgcattccc cgaggccttc atccccggct acccctggca tatctggatg     180
gactcgcccg cctgggcgat tggccgcggt tttgtgcagc gctacttcga caattcgctg     240
gcctatgaca gcccgcaggc cgagaagctg cgcgcggccg tgcgcaaggc aaaactcacg     300
gccgtgatcg gcttgtcgga gcgtgacggc ggcagccttt atctcgcaca atggctgatc     360
ggccccgacg gcgagaccat cgcaaaacgg cgcaagctgc ggccgacaca tgccgagcgc     420
actgtgtacg gcgagggcga cggcagcgac cttgcggtcc acaatcgtcc ggacatcggc     480
aggctcggtg cgctctgctg ctgggagcat cttcagccac tgtcgaaata cgcgatgtac     540
gcgcagaacg agcaggtgca cgtcgcggcc tggccgagct tttcgctcta cgatcccttc     600
gccgtggcgc tcggcgccga ggtgaacaac gcggcctccc gcgtctatgc ggtcgaaggc     660
tcctgcttcg tgctggcgcc gtgcgcgaca gtctcgcaag ccatgatcga cgagctctgc     720
gatcggccgg acaagcacgc gctgctgcat gtcggcggcg gctttgccgc gatctacggg     780
cccgacggca gccagatcgg cgacaagctc gcccccgacc aggagggcct gttgatcgcc     840
gagatcgatc tcggcgccat aggtgtcgcc aagaacgccg cggatcccgc cgggcactat     900
tcgcggcccg acgtgacgcg gctgttgctc aacaagaaac cgtacaagcg cgtcgaacag     960
ttctcgccgc cgtcggaggc ggttgaaccc acggatatcg cggcggcggc aagctga       1017

<210> 168
<211> 338
<212> PRT
<213> Unknown

<220>
<223> Obtained from an environmental sample

<400> 168
Met Gly Ile Glu His Pro Lys Tyr Arg Val Ala Val Val Gln Ala Ala
1               5                   10                  15
Pro Ala Trp Leu Asp Leu Asp Ala Ser Ile Asp Lys Ser Ile Ala Leu
            20                  25                  30
Ile Glu Glu Ala Ala Gln Lys Gly Ala Lys Leu Ile Ala Phe Pro Glu
        35                  40                  45
Ala Phe Ile Pro Gly Tyr Pro Trp His Ile Trp Met Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Lys Leu Arg Ala Ala Val Arg Lys
                85                  90                  95
Ala Lys Leu Thr Ala Val Ile Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asn Arg Pro Asp Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro

                180                 185                 190
Ser Phe Ser Leu Tyr Asp Pro Phe Ala Val Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Val Gly Gly Gly Phe Ala
                245                 250                 255
Ala Ile Tyr Gly Pro Asp Gly Ser Gln Ile Gly Asp Lys Leu Ala Pro
                260                 265                 270
Asp Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Lys Pro Tyr Lys Arg Val Glu Gln
305                 310                 315                 320
Phe Ser Pro Pro Ser Glu Ala Val Glu Pro Thr Asp Ile Ala Ala Ala
                325                 330                 335
Ala Ser


<210> 169
<211> 1077
<212> DNA
<213> Unknown

<220>
<223> Obtained from an environmental sample

<400> 169
atggccctca cccatccgaa attgaaagtc gccgccgtgc aggcagctcc cgcgttcctc      60
gatgtcgatg ccgcagtgga caaagcggtg cggctaatcg acgaagcggc agcaaacggc     120
tccagtctgg tggcattccc cgagacctgg atccccggct atccgttttg gatctggctt     180
ggctcgccgg cctgggcaat catgcgcggg tttgtgtctc gctatttcga taattcgctc     240
agctatgaca gccggcaggc agagcgcctg cgcgacgccg cgaagcgcca caaactgacc     300
gtcgtcatgg gcctgtccga gcgcgccggc ggtagccttt acatcgcgca gtggatcatt     360
ggtcccaatg gcgagaccgt cgcacagcgg cgcaagctca agcccaccca tgcggagcgc     420
accgtcttcg gcgagggtga cggcagccac ctggcggtac acaatcttcc aatcggacgg     480
ctcggtgcgc tgtgctgctg ggagcacctc cagccgctct ccaaatacgc gatgtacgcc     540
cagaacgaag agatccacgt ggcggcatgg ccgtccttct cgctctacga cccgtttgcg     600
cacgcgctcg gcgccgaagt caacaacgca gcgagccaga tctacgcggt tgaaggttcc     660
tgctttgtcg tcgcgccatg tgcggtgatc tcgcaggaaa tgatcgatct tatgtgcgat     720
acccccgaca agcatcagct tattcacgtc ggtggcggct tcaccgtgat ctatggcccg     780
gacggtgcgc gcatcggcga caagctcgcg ccagatcagg aaggcattgt ctatgccgac     840
atcgatctcg gcatgatccc gatcgcgaaa gctgccgccg atcctgccgg ccactatgcg     900
cgacccgacg ttacccgcct tctgttcaac aatcgtcccg ccaatcgggt ggaaaccctc     960
gtgctccccg ttgatcaggt ccgtgacatc gatgcacgtg tggaggccgc ggcacctcag    1020
gcgcgaccag caaccgggaa cgaggatccc gccgcaaagc ctatggccgc cgaatga       1077

<210> 170
<211> 358
<212> PRT
<213> Unknown

<220>
<223> Obtained from an environmental sample

<400> 170
Met Ala Leu Thr His Pro Lys Leu Lys Val Ala Ala Val Gln Ala Ala
1               5                   10                  15
Pro Ala Phe Leu Asp Val Asp Ala Ala Val Asp Lys Ala Val Arg Leu
            20                  25                  30
Ile Asp Glu Ala Ala Ala Asn Gly Ser Ser Leu Val Ala Phe Pro Glu
        35                  40                  45
Thr Trp Ile Pro Gly Tyr Pro Phe Trp Ile Trp Leu Gly Ser Pro Ala
    50                  55                  60
Trp Ala Ile Met Arg Gly Phe Val Ser Arg Tyr Phe Asp Asn Ser Leu

65                  70                  75                  80
Ser Tyr Asp Ser Arg Gln Ala Glu Arg Leu Arg Asp Ala Ala Lys Arg
                85                  90                  95
His Lys Leu Thr Val Val Met Gly Leu Ser Glu Arg Ala Gly Gly Ser
            100                 105                 110
Leu Tyr Ile Ala Gln Trp Ile Ile Gly Pro Asn Gly Glu Thr Val Ala
        115                 120                 125
Gln Arg Arg Lys Leu Lys Pro Thr His Ala Glu Arg Thr Val Phe Gly
    130                 135                 140
Glu Gly Asp Gly Ser His Leu Ala Val His Asn Leu Pro Ile Gly Arg
145                 150                 155                 160
Leu Gly Ala Leu Cys Cys Trp Glu His Leu Gln Pro Leu Ser Lys Tyr
                165                 170                 175
Ala Met Tyr Ala Gln Asn Glu Glu Ile His Val Ala Ala Trp Pro Ser
            180                 185                 190
Phe Ser Leu Tyr Asp Pro Phe Ala His Ala Leu Gly Ala Glu Val Asn
        195                 200                 205
Asn Ala Ala Ser Gln Ile Tyr Ala Val Glu Gly Ser Cys Phe Val Val
    210                 215                 220
Ala Pro Cys Ala Val Ile Ser Gln Glu Met Ile Asp Leu Met Cys Asp
225                 230                 235                 240
Thr Pro Asp Lys His Gln Leu Ile His Val Gly Gly Gly Phe Thr Val
                245                 250                 255
Ile Tyr Gly Pro Asp Gly Ala Arg Ile Gly Asp Lys Leu Ala Pro Asp
            260                 265                 270
Gln Glu Gly Ile Val Tyr Ala Asp Ile Asp Leu Gly Met Ile Pro Ile
        275                 280                 285
Ala Lys Ala Ala Ala Asp Pro Ala Gly His Tyr Ala Arg Pro Asp Val
    290                 295                 300
Thr Arg Leu Leu Phe Asn Asn Arg Pro Ala Asn Arg Val Glu Thr Leu
305                 310                 315                 320
Val Leu Pro Val Asp Gln Val Arg Asp Ile Asp Ala Arg Val Glu Ala
                325                 330                 335
Ala Ala Pro Gln Ala Arg Pro Ala Thr Gly Asn Glu Asp Pro Ala Ala
            340                 345                 350
Lys Pro Met Ala Ala Glu
        355

<210> 171
<211> 1011
<212> DNA
<213> Unknown

<220>
<223> Obtained from an environmental sample

<400> 171
atgactaagg gaacagtgaa agtcgcggcg gcgcaggtca cccccgtgtt catggatcgt      60
aaagccacga tcgtcaaggc ctgcgacacg atcgccgagg cgggcaagaa cggcgcgcgg     120
ctggtggtgt tcccggagac gtttgttcct ggctacccgg actgggtctg gacggcgacg     180
gctgggacgc atcgcgatat ccaccaggcg atgtacgcgg aactgctgga ccaggctgtc     240
tcgattccga gcccggcgac ggacgccctc tgccgtgctg caaagaaggc gggcgtctac     300
gtcgtcatcg gcgtcaatga gctgagtggg ccgggcggaa gcctgtacaa cacgctgatc     360
tacatcgatg acgaaggcga gatcatgggc cgccaccgca agctggtccc cacgatgggc     420
gagcgcctgg tctgggcacc cggcgacggc agcacgctgg aggcgtacga gacatcgatc     480
ggcaggctgg gcggactgat ctgctgggag aactacatgc cgctggcccg ctacgccatg     540
tacgcctggg gcgtgcagat ctacgtcgcg ccgacgtggg acagctcgga cgggtgggtt     600
ggcagcatgc agcacatcgc ccgcgaaggg cggacggcgg tgatcggctg ctgcatggcg     660
atccgtcgca gcgacatccc ggacaagtac gagttcaaga agctgtaccc gccgagcaag     720
agcaaagacg aagaatgggt gaacgatggc aacagcgtca tcgtcgcacc cggtggacga     780
atactcgccg ggccggtcgc caaagaggag acgatcctct acgccgatct ggacccggca     840
gccgagcgcg gttcaaagtt ctcgttagat gtggcagggc actacgcgcg gccggacgtc     900
ttccagctga cggtgaatcg cggtccggca gaactggtga atgtggccgg tgatatcgca     960
ccggcaacca acggcaaagt caaaacaccg gcgaaattac gccgcaagta a             1011

<210> 172
<211> 336
<212> PRT

<213> Unknown

<220>
<223> Obtained from an environmental sample

<400> 172
Met Thr Lys Gly Thr Val Lys Val Ala Ala Ala Gln Val Thr Pro Val
1               5                   10                  15
Phe Met Asp Arg Lys Ala Thr Ile Val Lys Ala Cys Asp Thr Ile Ala
            20                  25                  30
Glu Ala Gly Lys Asn Gly Ala Arg Leu Val Val Phe Pro Glu Thr Phe
        35                  40                  45
Val Pro Gly Tyr Pro Asp Trp Val Trp Thr Ala Thr Ala Gly Thr His
    50                  55                  60
Arg Asp Ile His Gln Ala Met Tyr Ala Glu Leu Leu Asp Gln Ala Val
65                  70                  75                  80
Ser Ile Pro Ser Pro Ala Thr Asp Ala Leu Cys Arg Ala Ala Lys Lys
                85                  90                  95
Ala Gly Val Tyr Val Val Ile Gly Val Asn Glu Leu Ser Gly Pro Gly
            100                 105                 110
Gly Ser Leu Tyr Asn Thr Leu Ile Tyr Ile Asp Asp Glu Gly Glu Ile
        115                 120                 125
Met Gly Arg His Arg Lys Leu Val Pro Thr Met Gly Glu Arg Leu Val
    130                 135                 140
Trp Ala Pro Gly Asp Gly Ser Thr Leu Glu Ala Tyr Glu Thr Ser Ile
145                 150                 155                 160
Gly Arg Leu Gly Gly Leu Ile Cys Trp Glu Asn Tyr Met Pro Leu Ala
                165                 170                 175
Arg Tyr Ala Met Tyr Ala Trp Gly Val Gln Ile Tyr Val Ala Pro Thr
            180                 185                 190
Trp Asp Ser Ser Asp Gly Trp Val Gly Ser Met Gln His Ile Ala Arg
        195                 200                 205
Glu Gly Arg Thr Ala Val Ile Gly Cys Cys Met Ala Ile Arg Arg Ser
    210                 215                 220
Asp Ile Pro Asp Lys Tyr Glu Phe Lys Lys Leu Tyr Pro Pro Ser Lys
225                 230                 235                 240
Ser Lys Asp Glu Glu Trp Val Asn Asp Gly Asn Ser Val Ile Val Ala
                245                 250                 255
Pro Gly Gly Arg Ile Leu Ala Gly Pro Val Ala Lys Glu Glu Thr Ile
            260                 265                 270
Leu Tyr Ala Asp Leu Asp Pro Ala Ala Glu Arg Gly Ser Lys Phe Ser
        275                 280                 285
Leu Asp Val Ala Gly His Tyr Ala Arg Pro Asp Val Phe Gln Leu Thr
    290                 295                 300
Val Asn Arg Gly Pro Ala Glu Leu Val Asn Val Ala Gly Asp Ile Ala
305                 310                 315                 320
Pro Ala Thr Asn Gly Lys Val Lys Thr Pro Ala Lys Leu Arg Arg Lys
                325                 330                 335

<210> 173
<211> 993
<212> DNA
<213> Unknown

<220>
<223> Obtained from an environmental sample

<400> 173
atgaaagttg ttaaagccgc cgcagtccaa ctgagtcccg tcctctatag ccgcgaggga      60
acggtcgaga gggtcgttcg gaagatccac gagcttggcc ggcagggggt acagttcgcc     120
accttcccgg agaccgtagt gccttactac ccgtactttt ccttcgtcca gacgccctta     180
cagattatag ccggacctga gcatctaaag ctgctcgacc aggcagtgac cgtgccgtcc     240
cctgccaccg acgctatcag cgaggctgcc aggcaggcgg gagttgtggt gtccataggc     300
gtcaacgagc gtgacggcgg aaccctgtac aacacgcagc tgctcttcga tgccgatggc     360
gccttgatcc agcgccgccg caagattacg cccactcatt tcgagcgcat gatctggggc     420
cagggcgacg ggtcgggcct gcgcgctgtc gacagcaagg tcggtcgcat tggccagctc     480
gcatgctggg agcacaacaa ccccctggcg cgctacgcga tgatagccga cggcgagcag     540
atccattcgg caatgtatcc gggctccatg ttcggcgacc cgtttgccca gaagacggaa     600

atcaatatcc ggcagcatgc attggagtct gcgtgcttcg tcgtgtgcgc cacggcctgg 660
ctggacgccg atcagcaggc gcaaatctgc aaggacactg gctgcgacat cggcccgatc 720
tccggcggtt gcttcaccgc gatcgtggcg cctgatggaa ccttgctggg cgagcccatc 780
cgctcgggcg aaggcatggt catcgtcgac ctcgacttca cgctcatcga caagcgcaag 840
caggtgatgg actcgcgcgg ccactacaac cggccggaat tgctcagtct cctgatcgac 900
cgcacaccca ctgcgcatgt tcacgaccgc gctgtgcgcc ccgagtcagc cgcggagcaa 960
cgttcggagg aacttctcgc tacggctgtc taa 993

<210> 174
<211> 330
<212> PRT
<213> Unknown

<220>
<223> Obtained from an environmental sample

<400> 174
Met Lys Val Val Lys Ala Ala Ala Val Gln Leu Ser Pro Val Leu Tyr
1               5                   10                  15
Ser Arg Glu Gly Thr Val Glu Arg Val Val Arg Lys Ile His Glu Leu
            20                  25                  30
Gly Arg Gln Gly Val Gln Phe Ala Thr Phe Pro Glu Thr Val Val Pro
        35                  40                  45
Tyr Tyr Pro Tyr Phe Ser Phe Val Gln Thr Pro Leu Gln Ile Ile Ala
    50                  55                  60
Gly Pro Glu His Leu Lys Leu Leu Asp Gln Ala Val Thr Val Pro Ser
65                  70                  75                  80
Pro Ala Thr Asp Ala Ile Ser Glu Ala Ala Arg Gln Ala Gly Val Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Ala Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Lys Val Gly Arg Ile Gly Gln Leu
145                 150                 155                 160
Ala Cys Trp Glu His Asn Asn Pro Leu Ala Arg Tyr Ala Met Ile Ala
                165                 170                 175
Asp Gly Glu Gln Ile His Ser Ala Met Tyr Pro Gly Ser Met Phe Gly
            180                 185                 190
Asp Pro Phe Ala Gln Lys Thr Glu Ile Asn Ile Arg Gln His Ala Leu
        195                 200                 205
Glu Ser Ala Cys Phe Val Val Cys Ala Thr Ala Trp Leu Asp Ala Asp
    210                 215                 220
Gln Gln Ala Gln Ile Cys Lys Asp Thr Gly Cys Asp Ile Gly Pro Ile
225                 230                 235                 240
Ser Gly Gly Cys Phe Thr Ala Ile Val Ala Pro Asp Gly Thr Leu Leu
                245                 250                 255
Gly Glu Pro Ile Arg Ser Gly Glu Gly Met Val Ile Val Asp Leu Asp
            260                 265                 270
Phe Thr Leu Ile Asp Lys Arg Lys Gln Val Met Asp Ser Arg Gly His
        275                 280                 285
Tyr Asn Arg Pro Glu Leu Leu Ser Leu Leu Ile Asp Arg Thr Pro Thr
    290                 295                 300
Ala His Val His Asp Arg Ala Val Arg Pro Glu Ser Ala Ala Glu Gln
305                 310                 315                 320
Arg Ser Glu Glu Leu Leu Ala Thr Ala Val
                325                 330

<210> 175
<211> 945
<212> DNA
<213> Unknown

<220>
<223> Obtained from an environmental sample

<400> 175
atgaccacct acgggcagtt tagacttgcc gcgatcaatg ccgcgcctgt ctacttcgac 60
agggaagcat ccaccgaaaa agcttgccgg ctcattctcg aagcgggggc atcaggggcg 120
acgctggcgg cgtttggcga gacgtggctt cccggctatc cattccacat ctggagggaa 180
gttccgactg ctctccgagt ggaatacatc gctaatgccg tcgagattcc cagcccaacg 240
accgaccgat tgtgcgcggc ggctcgtcag gcgaacatcg atgttgtgat cggcgttgtc 300
gaactggatg cgcagacaca cgggacggtc tactgtacgc tcttgttcat tggcagcgat 360
ggctcaattc tgggacgtca tcgaaagatt aaaccgactt tcgtggagcg aaccgcatgg 420
ggggaaggtg acggcagcag cctgatcgtc tacgagcgcc cgtatggcaa gatcagtggt 480
ctgtgttgct gggaacacaa tatggttctg ccgggctacg cgctgatggc gcaggggacg 540
cagattcata tcgccgcatg gcccggctgg gaaagcactc gccatctgct cttatcaaga 600
gcattcgctt ctcaggcagc ggcgtatgtg attgatgtag gcgctatcgt caatcgtgac 660
gaccttcggg aagattacca ggctttgatt gctggaagct actggggcgg aagttgcatc 720
atcaacccag aaggcgaggt catcgctggt ccagcgaaat cggagaccat tctggttgca 780
gattgctcaa ccgagcagat ctttagctca aaagtgctct gtgatgtggg cgggcattat 840
tctcgcccgg atattttca gctccatgtc aatcgaaagc catatcaacg tatcgtcgag 900
acgaacaacc cacaccccgc tccgattgag ttcgattacc gttga 945

<210> 176
<211> 314
<212> PRT
<213> Unknown

<220>
<223> Obtained from an environmental sample

<400> 176
Met Thr Thr Tyr Gly Gln Phe Arg Leu Ala Ala Ile Asn Ala Ala Pro
1               5                   10                  15
Val Tyr Phe Asp Arg Glu Ala Ser Thr Glu Lys Ala Cys Arg Leu Ile
            20                  25                  30
Leu Glu Ala Gly Ala Ser Gly Ala Thr Leu Ala Ala Phe Gly Glu Thr
        35                  40                  45
Trp Leu Pro Gly Tyr Pro Phe His Ile Trp Arg Glu Val Pro Thr Ala
    50                  55                  60
Leu Arg Val Glu Tyr Ile Ala Asn Ala Val Glu Ile Pro Ser Pro Thr
65                  70                  75                  80
Thr Asp Arg Leu Cys Ala Ala Ala Arg Gln Ala Asn Ile Asp Val Val
                85                  90                  95
Ile Gly Val Val Glu Leu Asp Ala Gln Thr His Gly Thr Val Tyr Cys
            100                 105                 110
Thr Leu Leu Phe Ile Gly Ser Asp Gly Ser Ile Leu Gly Arg His Arg
        115                 120                 125
Lys Ile Lys Pro Thr Phe Val Glu Arg Thr Ala Trp Gly Glu Gly Asp
    130                 135                 140
Gly Ser Ser Leu Ile Val Tyr Glu Arg Pro Tyr Gly Lys Ile Ser Gly
145                 150                 155                 160
Leu Cys Cys Trp Glu His Asn Met Val Leu Pro Gly Tyr Ala Leu Met
                165                 170                 175
Ala Gln Gly Thr Gln Ile His Ile Ala Ala Trp Pro Gly Trp Glu Ser
            180                 185                 190
Thr Arg His Leu Leu Leu Ser Arg Ala Phe Ala Ser Gln Ala Ala Ala
        195                 200                 205
Tyr Val Ile Asp Val Gly Ala Ile Val Asn Arg Asp Asp Leu Arg Glu
    210                 215                 220
Asp Tyr Gln Ala Leu Ile Ala Gly Ser Tyr Trp Gly Gly Ser Cys Ile
225                 230                 235                 240
Ile Asn Pro Glu Gly Glu Val Ile Ala Gly Pro Ala Lys Ser Glu Thr
                245                 250                 255
Ile Leu Val Ala Asp Cys Ser Thr Glu Gln Ile Phe Ser Ser Lys Val
            260                 265                 270
Leu Cys Asp Val Gly Gly His Tyr Ser Arg Pro Asp Ile Phe Gln Leu
        275                 280                 285
His Val Asn Arg Lys Pro Tyr Gln Arg Ile Val Glu Thr Asn Asn Pro
    290                 295                 300
His Pro Ala Pro Ile Glu Phe Asp Tyr Arg
305                 310

<210> 177
<211> 948
<212> DNA
<213> Unknown

<220>
<223> Obtained from an environmental sample

<400> 177
atggactcac ttaccgttgg tctcgcgcaa atcgcgcccg tttggttgaa tcgggcgggg 60
acgttgtcaa agatgttgga acaggttcga gcggcgaaag aggcgggctg tcagcttgtt 120
gtgtttggtg aggcgttgct ccccggttat ccattttgga tcgaactgac gaacggcgca 180
gtcttcaatt cgccgatgca aaaggaaatc cacgcgcact acatggatca agctgtgcag 240
atcgaagcag ggcatcttga tccattgtgc ggcgcggcaa aagcgcacgg catcaccgtg 300
gtcgcgggca tcatcgagcg tccgttggat cgcggcggac atagtttata tgcgagtctg 360
gtgtatatcg atttgaacgg tgtcatccaa tcggtgcatc gcaaactgat gcccacctat 420
gaagaacgac tcacctggtc gcctggcgat ggtcatgggt tacgcgtgca tacactgggc 480
gcttttacgg ttggcaaact caattgttgg gaaaactgga tgccgctgcc gcgcgcggct 540
ctgtatgcgc aaggcgaaga tctgcacgtt gctgtctggc ccgggtccgt gcgcaacaca 600
caggatatta cgcgctttat cgcaatggag tcgcgatcgt ttgtcgtttc ggtttcgagt 660
ttgatgcgca agagtgactt cccacaagat acgcctcatc tctccgccat tcttgaatct 720
gcacccgatc cactcgccaa cggaggttcg tgtctggctg gacctgacgg taaatggatc 780
gttgaaccgg ttgcggatga agagaagttg atcgtcgcca ccattgacca tgcccgtgta 840
cgtgaagaac gccagaactt tgatccatcc ggtcattaca gccgaccaga tgtgacacaa 900
ttgagagtca accgccagcg acaaagcgtt atcgcttttg atgagtag 948

<210> 178
<211> 315
<212> PRT
<213> Unknown

<220>
<223> Obtained from an environmental sample

<400> 178
Met Asp Ser Leu Thr Val Gly Leu Ala Gln Ile Ala Pro Val Trp Leu
1               5                   10                  15
Asn Arg Ala Gly Thr Leu Ser Lys Met Leu Glu Gln Val Arg Ala Ala
            20                  25                  30
Lys Glu Ala Gly Cys Gln Leu Val Val Phe Gly Glu Ala Leu Leu Pro
        35                  40                  45
Gly Tyr Pro Phe Trp Ile Glu Leu Thr Asn Gly Ala Val Phe Asn Ser
    50                  55                  60
Pro Met Gln Lys Glu Ile His Ala His Tyr Met Asp Gln Ala Val Gln
65                  70                  75                  80
Ile Glu Ala Gly His Leu Asp Pro Leu Cys Gly Ala Ala Lys Ala His
                85                  90                  95
Gly Ile Thr Val Val Ala Gly Ile Ile Glu Arg Pro Leu Asp Arg Gly
            100                 105                 110
Gly His Ser Leu Tyr Ala Ser Leu Val Tyr Ile Asp Leu Asn Gly Val
        115                 120                 125
Ile Gln Ser Val His Arg Lys Leu Met Pro Thr Tyr Glu Glu Arg Leu
    130                 135                 140
Thr Trp Ser Pro Gly Asp Gly His Gly Leu Arg Val His Thr Leu Gly
145                 150                 155                 160
Ala Phe Thr Val Gly Lys Leu Asn Cys Trp Glu Asn Trp Met Pro Leu
                165                 170                 175
Pro Arg Ala Ala Leu Tyr Ala Gln Gly Glu Asp Leu His Val Ala Val
            180                 185                 190
Trp Pro Gly Ser Val Arg Asn Thr Gln Asp Ile Thr Arg Phe Ile Ala
        195                 200                 205
Met Glu Ser Arg Ser Phe Val Val Ser Val Ser Ser Leu Met Arg Lys
    210                 215                 220
Ser Asp Phe Pro Gln Asp Thr Pro His Leu Ser Ala Ile Leu Glu Ser
225                 230                 235                 240
Ala Pro Asp Pro Leu Ala Asn Gly Gly Ser Cys Leu Ala Gly Pro Asp
                245                 250                 255
Gly Lys Trp Ile Val Glu Pro Val Ala Asp Glu Glu Lys Leu Ile Val

260                 265˙                270
Ala Thr Ile Asp His Ala Arg Val Arg Glu Glu Arg Gln Asn Phe Asp
        275                 280                 285
Pro Ser Gly His Tyr Ser Arg Pro Asp Val Thr Gln Leu Arg Val Asn
    290                 295                 300
Arg Gln Arg Gln Ser Val Ile Ala Phe Asp Glu
305                 310                 315

<210> 179
<211> 915
<212> DNA
<213> Unknown

<220>
<223> Obtained from an environmental sample

<400> 179
atgaccacca aagcagccat cattcaggcg cgccccatat actacgatct ggcggcgtgt      60
gtcgataagg cgcttgccct catcaccgag gcggcggcac gcggcgcgaa catcgtcacg     120
ctcggcgaga cgtggctgcc gggctatccc gcgtggctgg atgtgtgcgt cgagatgggg     180
ctgtgggatc acgcgccgac caaagccgtc tttcagcggc tccatgccaa cagcgtcacc     240
atccccggcg cggagatcag ccagttctgc gacatcgccc gccgccttag catcgtgctg     300
gtgctcagcg tcaacgagcg cgtccgcaac accttgttca acaccctgct cacgattgac     360
gagcgcggcg acatccgcaa ccaccaccgc aagctgatgc cgacctacac tgagcgcatc     420
gtctgggggc agggcgacgg cgcgggctta caggcggtcg agacggcaac cgggcgcgtc     480
ggcgggctga tctgctggga acactggatg ccgctggcac ggcaggcgct gcacaacgcc     540
ggggagcaaa ttcacgtttc ggtcttcccg accgtcaacg acccgcgcca ccaagtcgcc     600
agccgccagt acgctttcga ggggcgctgc ttcgtgctga ccgccggcag catccagcgc     660
gccgacgacc taccgccgga actgaccgtc aaggcgggca tcgcgccgga tgatctggtg     720
cagggcggcg gcagcgccat catcgcgccg gacatgcgct acctcgccgg accctgcttc     780
gacgaggaaa ccatcctcta cgccgacctc gacctgagcg agacgatccg cgagagcatg     840
acgctggacg tgagcgggca ttactcgcgc cccgacgtgt tcaccttcga ggttaatcgg     900
cagcggaaaa tttag                                                      915

<210> 180
<211> 304
<212> PRT
<213> Unknown

<220>
<223> Obtained from an environmental sample

<400> 180
Met Thr Thr Lys Ala Ala Ile Ile Gln Ala Arg Pro Ile Tyr Tyr Asp
1               5                   10                  15
Leu Ala Ala Cys Val Asp Lys Ala Leu Ala Leu Ile Thr Glu Ala Ala
            20                  25                  30
Ala Arg Gly Ala Asn Ile Val Thr Leu Gly Glu Thr Trp Leu Pro Gly
        35                  40                  45
Tyr Pro Ala Trp Leu Asp Val Cys Val Glu Met Gly Leu Trp Asp His
    50                  55                  60
Ala Pro Thr Lys Ala Val Phe Gln Arg Leu His Ala Asn Ser Val Thr
65                  70                  75                  80
Ile Pro Gly Ala Glu Ile Ser Gln Phe Cys Asp Ile Ala Arg Arg Leu
                85                  90                  95
Ser Ile Val Leu Val Leu Ser Val Asn Glu Arg Val Arg Asn Thr Leu
            100                 105                 110
Phe Asn Thr Leu Leu Thr Ile Asp Glu Arg Gly Asp Ile Arg Asn His
        115                 120                 125
His Arg Lys Leu Met Pro Thr Tyr Thr Glu Arg Ile Val Trp Gly Gln
    130                 135                 140
Gly Asp Gly Ala Gly Leu Gln Ala Val Glu Thr Ala Thr Gly Arg Val
145                 150                 155                 160
Gly Gly Leu Ile Cys Trp Glu His Trp Met Pro Leu Ala Arg Gln Ala
                165                 170                 175
Leu His Asn Ala Gly Glu Gln Ile His Val Ser Val Phe Pro Thr Val
            180                 185                 190
Asn Asp Pro Arg His Gln Val Ala Ser Arg Gln Tyr Ala Phe Glu Gly

195                 200                 205
Arg Cys Phe Val Leu Thr Ala Gly Ser Ile Gln Arg Ala Asp Asp Leu
    210                 215                 220
Pro Pro Glu Leu Thr Val Lys Ala Gly Ile Ala Pro Asp Asp Leu Val
225                 230                 235                 240
Gln Gly Gly Gly Ser Ala Ile Ile Ala Pro Asp Met Arg Tyr Leu Ala
                245                 250                 255
Gly Pro Cys Phe Asp Glu Glu Thr Ile Leu Tyr Ala Asp Leu Asp Leu
            260                 265                 270
Ser Glu Thr Ile Arg Glu Ser Met Thr Leu Asp Val Ser Gly His Tyr
        275                 280                 285
Ser Arg Pro Asp Val Phe Thr Phe Glu Val Asn Arg Gln Arg Lys Ile
    290                 295                 300

<210> 181
<211> 990
<212> DNA
<213> Unknown

<220>
<223> Obtained from an environmental sample

<400> 181
atgcccaaga ccgtacgcgc agccgcagtc cagatcgcgc ccgacctgac gtcacgcgct      60
ggcaccgtcg agcgggtcct gaatgcgatt gccgaagcct ctgacaaagg cgcggagctg     120
attgtatttc cagaaacctt tgtgccttgg tatccgtatt tcagcttcgt tctgccgcct     180
gtccagcagg gacccgagca cctgcggctt tatgaagaag cggtgacggt accatcagca     240
gaaacacggg ccgtcgccga cgccgcgcgc aaacgcaatg cggtcatcgt ccttggcgtc     300
aatgagcgcg accacggctc gctctacaac acccagctga tcttcgacgc ggatggcagc     360
ctgaaactca agcgccgcaa gatcacgccc acctatcacg agcggatgat ctggggacag     420
ggcgatggcg ccggtctaaa agtggtcgaa actgccatcg gccgcatggg cgcattggcg     480
tgctgggagc actacaaccc cctcgcccga tacgcgctga tggctcagca tgaggaaatt     540
cacgcctctc attttccggg ctcactggtc ggcccgatat tcggcgagca gatcgaagtc     600
acgatgcgcc accacgcgtt ggaatcgggc tgtttcgtgg tcaatgccac cggctggtta     660
agcgaggagc agatcgcgtc cattcaccca gatcccagcc tgcagaaggg tcttcgagat     720
ggctgcatga cctgcatcat aaccccggaa ggccgccacg tcgttcctcc tctgacatcg     780
ggtgaaggaa tcctgattgg cgacctggac atgcggctca tcaccaagcg caagcgaatg     840
atggattccg tcggacacta tgcacgtcct gagctgctgc accttgtcca tgacacgacg     900
cccgcacgcg cacgcgagca ggtgggcctt tcaggcgatt tttccgatgc agggcaagac     960
aagctatttg aggaggttca agatgcgtga                                      990

<210> 182
<211> 329
<212> PRT
<213> Unknown

<220>
<223> Obtained from an environmental sample

<400> 182
Met Pro Lys Thr Val Arg Ala Ala Ala Val Gln Ile Ala Pro Asp Leu
1               5                   10                  15
Thr Ser Arg Ala Gly Thr Val Glu Arg Val Leu Asn Ala Ile Ala Glu
            20                  25                  30
Ala Ser Asp Lys Gly Ala Glu Leu Ile Val Phe Pro Glu Thr Phe Val
        35                  40                  45
Pro Trp Tyr Pro Tyr Phe Ser Phe Val Leu Pro Pro Val Gln Gln Gly
    50                  55                  60
Pro Glu His Leu Arg Leu Tyr Glu Glu Ala Val Thr Val Pro Ser Ala
65                  70                  75                  80
Glu Thr Arg Ala Val Ala Asp Ala Ala Arg Lys Arg Asn Ala Val Ile
                85                  90                  95
Val Leu Gly Val Asn Glu Arg Asp His Gly Ser Leu Tyr Asn Thr Gln
            100                 105                 110
Leu Ile Phe Asp Ala Asp Gly Ser Leu Lys Leu Lys Arg Arg Lys Ile
        115                 120                 125
Thr Pro Thr Tyr His Glu Arg Met Ile Trp Gly Gln Gly Asp Gly Ala
    130                 135                 140

Gly Leu Lys Val Val Glu Thr Ala Ile Gly Arg Met Gly Ala Leu Ala
145                 150                 155                 160
Cys Trp Glu His Tyr Asn Pro Leu Ala Arg Tyr Ala Leu Met Ala Gln
                165                 170                 175
His Glu Glu Ile His Ala Ser His Phe Pro Gly Ser Leu Val Gly Pro
            180                 185                 190
Ile Phe Gly Glu Gln Ile Glu Val Thr Met Arg His His Ala Leu Glu
        195                 200                 205
Ser Gly Cys Phe Val Val Asn Ala Thr Gly Trp Leu Ser Glu Glu Gln
    210                 215                 220
Ile Ala Ser Ile His Pro Asp Pro Ser Leu Gln Lys Gly Leu Arg Asp
225                 230                 235                 240
Gly Cys Met Thr Cys Ile Ile Thr Pro Glu Gly Arg His Val Val Pro
                245                 250                 255
Pro Leu Thr Ser Gly Glu Gly Ile Leu Ile Gly Asp Leu Asp Met Arg
            260                 265                 270
Leu Ile Thr Lys Arg Lys Arg Met Met Asp Ser Val Gly His Tyr Ala
        275                 280                 285
Arg Pro Glu Leu Leu His Leu Val His Asp Thr Thr Pro Ala Arg Ala
    290                 295                 300
Arg Glu Gln Val Gly Leu Ser Gly Asp Phe Ser Asp Ala Gly Gln Asp
305                 310                 315                 320
Lys Leu Phe Glu Glu Val Gln Asp Ala
                325

<210> 183
<211> 1002
<212> DNA
<213> Unknown

<220>
<223> Obtained from an environmental sample

<400> 183
atggctgagt cacgcattat tcgtgccgcc gccgcccaga ttgcgccaga tctccatgag      60
gccagcaaaa cgctggcccg ggtgctggac gcgatcgatc aggcagccgc acagggggca     120
gagatcatcg tctttcccga gacctttgtg ccttattacc cctacttctc gtttatcacg     180
cccgcgatga ccgccggagc ggcccatctg aaattgtatg accaggcggt ggtggtgccc     240
ggcccgatca cccatgcggt gggcgaacgc gcccgcctgc gcaacatcgt cgtggtgctg     300
ggggtgaatg aacgtgacca cggcacgctc tacaacaccc aactggtatt tgatgccagc     360
ggggaactgg tgctgaaacg ccgcaaaatc accccgacct atcacgaacg gatgatctgg     420
ggacagggag acggtgccgg attaaaggtg gtggactcgg cggttgggcg catcggggct     480
ttagcctgct gggagcacta caacccactg gcgcgctaca gcctgatgac tcagcacgag     540
gagatccatt gcagccagtt ccctggttca ctggtggggc cgatttttgc cgagcagatg     600
gacgtcacca ttcgccatca tgcactggag tccggttgct ttgtcatcaa tgccaccggc     660
tggctgaccg aggagcagat caacgagctg accagcgacc cggcgttaca aaggggggctg     720
cgtggtggct gcaacaccgc catcatctcg ccggaaggcc gccatctggt gccgccactg     780
accgaaggtg aggggatttt gattgccgat ctggacatgg ccctgatcac caaacgcaaa     840
cgcatgatgg attctgtcgg ccactatgcc cgaccggaat tactcagcct gcgcctcgat     900
gcgacgcctg cccgttatgt ggtggcgcgt gataatgagt ccgaaaccgg aggaggcaac     960
gatgcagaac gtaccgtcta cgcgccagca gctgatcact ga                       1002

<210> 184
<211> 333
<212> PRT
<213> Unknown

<220>
<223> Obtained from an environmental sample

<400> 184
Met Ala Glu Ser Arg Ile Ile Arg Ala Ala Ala Ala Gln Ile Ala Pro
1               5                   10                  15
Asp Leu His Glu Ala Ser Lys Thr Leu Ala Arg Val Leu Asp Ala Ile
            20                  25                  30
Asp Gln Ala Ala Ala Gln Gly Ala Glu Ile Ile Val Phe Pro Glu Thr
        35                  40                  45
Phe Val Pro Tyr Tyr Pro Tyr Phe Ser Phe Ile Thr Pro Ala Met Thr

50                  55                  60
Ala Gly Ala Ala His Leu Lys Leu Tyr Asp Gln Ala Val Val Val Pro
65                  70                  75                  80
Gly Pro Ile Thr His Ala Val Gly Glu Arg Ala Arg Leu Arg Asn Ile
                85                  90                  95
Val Val Val Leu Gly Val Asn Glu Arg Asp His Gly Thr Leu Tyr Asn
            100                 105                 110
Thr Gln Leu Val Phe Asp Ala Ser Gly Glu Leu Val Leu Lys Arg Arg
        115                 120                 125
Lys Ile Thr Pro Thr Tyr His Glu Arg Met Ile Trp Gly Gln Gly Asp
    130                 135                 140
Gly Ala Gly Leu Lys Val Val Asp Ser Ala Val Gly Arg Ile Gly Ala
145                 150                 155                 160
Leu Ala Cys Trp Glu His Tyr Asn Pro Leu Ala Arg Tyr Ser Leu Met
                165                 170                 175
Thr Gln His Glu Glu Ile His Cys Ser Gln Phe Pro Gly Ser Leu Val
            180                 185                 190
Gly Pro Ile Phe Ala Glu Gln Met Asp Val Thr Ile Arg His His Ala
        195                 200                 205
Leu Glu Ser Gly Cys Phe Val Ile Asn Ala Thr Gly Trp Leu Thr Glu
    210                 215                 220
Glu Gln Ile Asn Glu Leu Thr Ser Asp Pro Ala Leu Gln Lys Gly Leu
225                 230                 235                 240
Arg Gly Gly Cys Asn Thr Ala Ile Ile Ser Pro Glu Gly Arg His Leu
                245                 250                 255
Val Pro Pro Leu Thr Glu Gly Glu Gly Ile Leu Ile Ala Asp Leu Asp
            260                 265                 270
Met Ala Leu Ile Thr Lys Arg Lys Arg Met Met Asp Ser Val Gly His
        275                 280                 285
Tyr Ala Arg Pro Glu Leu Leu Ser Leu Arg Leu Asp Ala Thr Pro Ala
    290                 295                 300
Arg Tyr Val Val Ala Arg Asp Asn Glu Ser Glu Thr Gly Gly Gly Asn
305                 310                 315                 320
Asp Ala Glu Arg Thr Val Tyr Ala Pro Ala Ala Asp His
                325                 330

<210> 185
<211> 1017
<212> DNA
<213> Unknown

<220>
<223> Obtained from an environmental sample

<400> 185
atgggcattg aacatccgaa atacaaggtc gcggtggtgc aggcggcccc cgcctggctc      60
gatctcgacg gctcggtcga taagtcgatc gcgctgatca aggaggcggc cgagaagggg     120
gcgaagctga tcgcctttcc cgaggccttc atccccggtt acccctggca tatctggatg     180
gactcgccgg cctgggcgat cggccgcggc ttcgtgcagc gttatttcga caattcgctg     240
tcctatgaca gtccgcaggc cgagcggctg cgcgatgcgg tgaagaaggc gaagctcacc     300
gccgtgttcg gactgtccga gcgcgacggc ggcagcctct acctcgcgca atggctgatc     360
gggcccgatg gcgagaccat cgccaagcgc cgcaagctgc ggccgaccca cgccgaacgt     420
accgtctatg gcgaaggcga cggcagcgat cttgccgtgc atgcgcgcgc cgacatcggc     480
cggatcggcg cgctctgctg ctgggagcat ctgcagccac tgtcgaaata cgcgatgtac     540
gcccagaacg aacaggtcca tgtcgcagcc tggcccagct tctcgctgta cgaccccttc     600
gcgccggcgt taggggccga ggtcaacaac gcggcctccc gcgtctatgc ggtggaaggc     660
tcctgcttcg tgctcgcgcc gtgcgcgacg gtgtcgcagg cgatgatcga cgagctctgc     720
gaccggcccg acaagaacgc gctgctgcac gtcggcggcg gctttgccgc gatctatggc     780
cccgacggca gccagatcgg cgacaagctg gcgccggacc aggaggggct gctgatcgcc     840
gagatcgacc ttggcgccat cggtgtcgcc aagaacgccg ccgatcccgc cgggcactat     900
tcgcgtcccg acgtgacgcg gttgctgctc aacaagaagc gataccagcg cgtcgagcag     960
ttcgcgctgc cggtcgacac cgtcgagccg gcggatatcg gcgcagcggc gagctga       1017

<210> 186
<211> 338
<212> PRT
<213> Unknown

<220>
<223> Obtained from an environmental sample

<400> 186
Met Gly Ile Glu His Pro Lys Tyr Lys Val Ala Val Val Gln Ala Ala
1               5                   10                  15
Pro Ala Trp Leu Asp Leu Asp Gly Ser Val Asp Lys Ser Ile Ala Leu
            20                  25                  30
Ile Lys Glu Ala Ala Glu Lys Gly Ala Lys Leu Ile Ala Phe Pro Glu
        35                  40                  45
Ala Phe Ile Pro Gly Tyr Pro Trp His Ile Trp Met Asp Ser Pro Ala
    50                  55                  60
Trp Ala Ile Gly Arg Gly Phe Val Gln Arg Tyr Phe Asp Asn Ser Leu
65                  70                  75                  80
Ser Tyr Asp Ser Pro Gln Ala Glu Arg Leu Arg Asp Ala Val Lys Lys
                85                  90                  95
Ala Lys Leu Thr Ala Val Phe Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Ala Arg Ala Asp Ile Gly
145                 150                 155                 160
Arg Ile Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Pro Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys Asn Ala Leu Leu His Val Gly Gly Gly Phe Ala
                245                 250                 255
Ala Ile Tyr Gly Pro Asp Gly Ser Gln Ile Gly Asp Lys Leu Ala Pro
            260                 265                 270
Asp Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Lys Arg Tyr Gln Arg Val Glu Gln
305                 310                 315                 320
Phe Ala Leu Pro Val Asp Thr Val Glu Pro Ala Asp Ile Gly Ala Ala
                325                 330                 335
Ala Ser


<210> 187
<211> 1059
<212> DNA
<213> Unknown

<220>
<223> Obtained from an environmental sample

<400> 187
atgggcatca atcatcccaa gtacaaagtg gccgtcgtgc aagcggcacc tgtctggctc      60
gacctggatg gaacagtcga caagtgcatt cggctgatag gcgaggccgc tgagaaggga     120
tgcaagctca ttgcatttcc cgagacgttc atcccggggt acccctggca catctggatg     180
ggagctccgg cctggacgat cgggcgcgga ttcgtgcagc gatacttcga caattcgctt     240
gcgtacgaca gtccgcaggc aaacaagctt cgcgccgcgg tgaagcgcgc cggagtgacg     300
gcagttctcg gcttgtcgga gcgccgcgga ggctccctgt acatcgccca gtggctcatc     360
ggacctgatg gcgagaccat cgctcaacgg cgaaagctgc gccccaccca tgcggagcgc     420
accgtcttcg gcgagggcga tggcagcgat ttggcggtgc acagccgccc cgacatcggc     480
cgactgggtg ccctttgctg ctgggaacat ctccagcctt tgaccaagta cgcgatgtac     540
gcgcaagacg agcaagtgca cgtcgctgca tggccgagct tctcgatgta cgagcctttc     600

gcgcacgccc tggggtggga gacgaacaac gcggtgagca aggtgtacgc ggtcgaaggt 660
tcgtgctacg tcctggcccc ctgcgccatc atctctcagg cgatggtgga cgaactcgtc 720
gacagcgagg acaagaagcc gctggttcat gccggcgggg ggcatgcggt gatctatggt 780
cccgatggca ccctgcttac tcccaagctt gcagaagacg aggagggcct actgatcgcg 840
gagatcgatc tgggggcaat cggggtcgcc aagaacgcgg cagaccccgc cggccactac 900
tcgcggcccg atgtcacccg cctgctcttc aacaaccggc cggccaagcg cgtggagacg 960
atgctgctcc cggtcgacgc ggcagaagtc gtggagccgg cggacggagc gctcaatgcg 1020
tccgagggac gccagcgaca gttcaagctg cccgcctag 1059

<210> 188
<211> 352
<212> PRT
<213> Unknown

<220>
<223> Obtained from an environmental sample

<400> 188
Met Gly Ile Asn His Pro Lys Tyr Lys Val Ala Val Val Gln Ala Ala
1               5                   10                  15
Pro Val Trp Leu Asp Leu Asp Gly Thr Val Asp Lys Cys Ile Arg Leu
            20                  25                  30
Ile Gly Glu Ala Ala Glu Lys Gly Cys Lys Leu Ile Ala Phe Pro Glu
        35                  40                  45
Thr Phe Ile Pro Gly Tyr Pro Trp His Ile Trp Met Gly Ala Pro Ala
    50                  55                  60
Trp Thr Ile Gly Arg Gly Phe Val Gln Arg Tyr Phe Asp Asn Ser Leu
65                  70                  75                  80
Ala Tyr Asp Ser Pro Gln Ala Asn Lys Leu Arg Ala Ala Val Lys Arg
                85                  90                  95
Ala Gly Val Thr Ala Val Leu Gly Leu Ser Glu Arg Arg Gly Gly Ser
            100                 105                 110
Leu Tyr Ile Ala Gln Trp Leu Ile Gly Pro Asp Gly Glu Thr Ile Ala
        115                 120                 125
Gln Arg Arg Lys Leu Arg Pro Thr His Ala Glu Arg Thr Val Phe Gly
    130                 135                 140
Glu Gly Asp Gly Ser Asp Leu Ala Val His Ser Arg Pro Asp Ile Gly
145                 150                 155                 160
Arg Leu Gly Ala Leu Cys Cys Trp Glu His Leu Gln Pro Leu Thr Lys
                165                 170                 175
Tyr Ala Met Tyr Ala Gln Asp Glu Gln Val His Val Ala Ala Trp Pro
            180                 185                 190
Ser Phe Ser Met Tyr Glu Pro Phe Ala His Ala Leu Gly Trp Glu Thr
        195                 200                 205
Asn Asn Ala Val Ser Lys Val Tyr Ala Val Glu Gly Ser Cys Tyr Val
    210                 215                 220
Leu Ala Pro Cys Ala Ile Ile Ser Gln Ala Met Val Asp Glu Leu Val
225                 230                 235                 240
Asp Ser Glu Asp Lys Lys Pro Leu Val His Ala Gly Gly Gly His Ala
                245                 250                 255
Val Ile Tyr Gly Pro Asp Gly Thr Leu Leu Thr Pro Lys Leu Ala Glu
            260                 265                 270
Asp Glu Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Phe Asn Asn Arg Pro Ala Lys Arg Val Glu Thr
305                 310                 315                 320
Met Leu Leu Pro Val Asp Ala Ala Glu Val Val Glu Pro Ala Asp Gly
                325                 330                 335
Ala Leu Asn Ala Ser Glu Gly Arg Gln Arg Gln Phe Lys Leu Pro Ala
            340                 345                 350

<210> 189
<211> 1005
<212> DNA
<213> Unknown

<220>
<223> Obtained from an environmental sample

<400> 189
atgcaagaca cgaaattcaa agttgcagtc gtccaggccg cgccggtatt catggatgcg 60
ccagcctccg tggccaaggc gatcggtttc atccaggagg cgggcgcagc cggggcgaag 120
ctgctggcgt tcccggaggt ctggattccg ggctaccctt ggtggctttg gctcgggacg 180
ccggcgtggg gaatgcagtt tgtgccgcgc tatcacgcca attcgctgcg tgctgatgga 240
cccgaaatcc tcgctctttg tgcggccgcc gccgaagcga agatcaacgt cgtgatgggc 300
ttctccgaaa tcgacggagg aacgctctac ctaagtcagg ttttcatcag cgatgcgggc 360
aagatcatct tcaagcgccg aaagctcaag ccgacccacg tcgaacgtac gctatttggt 420
gaaggagatg ggtctgattt ccgagtcgtc gacagcagcg tcgggcgcct cggagccctg 480
tgctgtgccg aacacattca gccgttgtcg aaatacgcca tgtacgcgat gaacgagcaa 540
attcatgtgg cgtcgtggcc atctttcacg ctctatcgcg gcaaagccta cgctttgggt 600
catgaggtga atcttgccgc cagccaaatc tacgcgctcg aaggaggttg cttcgtcttg 660
catgccacgg caattaccgg tcaggatatg ttcgacatgc tttgcgacac tccggaaagg 720
gcggatttgc tgaatgcgga gggagcaaag ccgggtggag gctattcgat gatttttggt 780
cccgatggtc agccgatgtg cgagcatctg ccgcaggaca aggaaggcat cctctatgcc 840
ggcgtagacc tgtcgatgat tgcgatcgcc aaagcggcct acgatcctac ggggcactac 900
gcccgcggtg atgtcgtccg tctcatggtc aaccgcagcc cccgtcgcac gagcgtcagc 960
ttcagcgaag acgagaacgc ggcggtcact ttcaccgaga cctga 1005

<210> 190
<211> 334
<212> PRT
<213> Unknown

<220>
<223> Obtained from an environmental sample

<400> 190
Met Gln Asp Thr Lys Phe Lys Val Ala Val Val Gln Ala Ala Pro Val
1               5                   10                  15
Phe Met Asp Ala Pro Ala Ser Val Ala Lys Ala Ile Gly Phe Ile Gln
            20                  25                  30
Glu Ala Gly Ala Ala Gly Ala Lys Leu Leu Ala Phe Pro Glu Val Trp
        35                  40                  45
Ile Pro Gly Tyr Pro Trp Trp Leu Trp Leu Gly Thr Pro Ala Trp Gly
    50                  55                  60
Met Gln Phe Val Pro Arg Tyr His Ala Asn Ser Leu Arg Ala Asp Gly
65                  70                  75                  80
Pro Glu Ile Leu Ala Leu Cys Ala Ala Ala Ala Glu Ala Lys Ile Asn
                85                  90                  95
Val Val Met Gly Phe Ser Glu Ile Asp Gly Gly Thr Leu Tyr Leu Ser
            100                 105                 110
Gln Val Phe Ile Ser Asp Ala Gly Lys Ile Ile Phe Lys Arg Arg Lys
        115                 120                 125
Leu Lys Pro Thr His Val Glu Arg Thr Leu Phe Gly Glu Gly Asp Gly
    130                 135                 140
Ser Asp Phe Arg Val Val Asp Ser Ser Val Gly Arg Leu Gly Ala Leu
145                 150                 155                 160
Cys Cys Ala Glu His Ile Gln Pro Leu Ser Lys Tyr Ala Met Tyr Ala
                165                 170                 175
Met Asn Glu Gln Ile His Val Ala Ser Trp Pro Ser Phe Thr Leu Tyr
            180                 185                 190
Arg Gly Lys Ala Tyr Ala Leu Gly His Glu Val Asn Leu Ala Ala Ser
        195                 200                 205
Gln Ile Tyr Ala Leu Glu Gly Gly Cys Phe Val Leu His Ala Thr Ala
    210                 215                 220
Ile Thr Gly Gln Asp Met Phe Asp Met Leu Cys Asp Thr Pro Glu Arg
225                 230                 235                 240
Ala Asp Leu Leu Asn Ala Glu Gly Ala Lys Pro Gly Gly Gly Tyr Ser
                245                 250                 255
Met Ile Phe Gly Pro Asp Gly Gln Pro Met Cys Glu His Leu Pro Gln
            260                 265                 270
Asp Lys Glu Gly Ile Leu Tyr Ala Gly Val Asp Leu Ser Met Ile Ala
        275                 280                 285
Ile Ala Lys Ala Ala Tyr Asp Pro Thr Gly His Tyr Ala Arg Gly Asp

290                 295                 300
Val Val Arg Leu Met Val Asn Arg Ser Pro Arg Arg Thr Ser Val Ser
305                 310                 315                 320
Phe Ser Glu Asp Glu Asn Ala Ala Val Thr Phe Thr Glu Thr
                325                 330

<210> 191
<211> 945
<212> DNA
<213> Unknown

<220>
<223> Obtained from an environmental sample

<400> 191
atgaaaaagt tagctgtggt tcaacgtgcg tcaggatttt tagataagca gcagagcatc      60
gcgttggcgg tggaaagtat tcagtctgct gcgaataatg gcgcagagct tgttgttttt     120
acggaagcct ttattcctgg ttatcctgtc tggttatggc gtctgcgccc tggcaaagac     180
tgggggacaa cagacagtct ttatcaacgc ttaataagca acgcggttga tttaagctca     240
tcggatttgg atccgattta tgaagcggca aaacgtcatc acgtcacggt tgtatgcggc     300
attaatgaac gcgactccag cgtcagccga acaacgctat acaacactta catcacggtt     360
tgtcatgagg gcaatctcat caatgttcat cgaaaactga tgccgaccaa cccagagaga     420
atggtgtggg gctttggtga tgcgactgga ttaagggtag tagacactcc tgtcggaagg     480
attggctcac tcgtttgctg ggagaactac atgccgttgg cacgctatgc actttatgct     540
cagggcgtcg aaatttacat tgcgcctact tatgacagtg gctcggactg gactgaaagc     600
ttgcgccata tcgccagaga gggcagatgc tacgttgtcg gcagcggtaa cttgttgaga     660
gccagcgacc tgcctgatga ttttccagaa aaagaaaccc tctatcctga taaagacgag     720
tggattaacg gcggagactc taccgttatc gctcccggcg gtgaaacatt agttgctccg     780
ctgcatgcag aggaaggcat actgtattgc gatattgata ctgataaagt ggcggcggct     840
cggcgttctt tcgacgttgc aggccattac tctcgcccag acatatttac actcaacgta     900
aatcgagcgc cgcaaacatc tctgcgtatc agggaagccg agtaa                     945

<210> 192
<211> 314
<212> PRT
<213> Unknown

<220>
<223> Obtained from an environmental sample

<400> 192
Met Lys Lys Leu Ala Val Val Gln Arg Ala Ser Gly Phe Leu Asp Lys
1               5                   10                  15
Gln Gln Ser Ile Ala Leu Ala Val Glu Ser Ile Gln Ser Ala Ala Asn
            20                  25                  30
Asn Gly Ala Glu Leu Val Val Phe Thr Glu Ala Phe Ile Pro Gly Tyr
        35                  40                  45
Pro Val Trp Leu Trp Arg Leu Arg Pro Gly Lys Asp Trp Gly Thr Thr
    50                  55                  60
Asp Ser Leu Tyr Gln Arg Leu Ile Ser Asn Ala Val Asp Leu Ser Ser
65                  70                  75                  80
Ser Asp Leu Asp Pro Ile Tyr Glu Ala Ala Lys Arg His His Val Thr
                85                  90                  95
Val Val Cys Gly Ile Asn Glu Arg Asp Ser Ser Val Ser Arg Thr Thr
            100                 105                 110
Leu Tyr Asn Thr Tyr Ile Thr Val Cys His Glu Gly Asn Leu Ile Asn
        115                 120                 125
Val His Arg Lys Leu Met Pro Thr Asn Pro Glu Arg Met Val Trp Gly
    130                 135                 140
Phe Gly Asp Ala Thr Gly Leu Arg Val Val Asp Thr Pro Val Gly Arg
145                 150                 155                 160
Ile Gly Ser Leu Val Cys Trp Glu Asn Tyr Met Pro Leu Ala Arg Tyr
                165                 170                 175
Ala Leu Tyr Ala Gln Gly Val Glu Ile Tyr Ile Ala Pro Thr Tyr Asp
            180                 185                 190
Ser Gly Ser Asp Trp Thr Glu Ser Leu Arg His Ile Ala Arg Glu Gly
        195                 200                 205
Arg Cys Tyr Val Val Gly Ser Gly Asn Leu Leu Arg Ala Ser Asp Leu

210                 215                 220
Pro Asp Asp Phe Pro Glu Lys Glu Thr Leu Tyr Pro Asp Lys Asp Glu
225                 230                 235                 240
Trp Ile Asn Gly Gly Asp Ser Thr Val Ile Ala Pro Gly Gly Glu Thr
                245                 250                 255
Leu Val Ala Pro Leu His Ala Glu Glu Gly Ile Leu Tyr Cys Asp Ile
            260                 265                 270
Asp Thr Asp Lys Val Ala Ala Ala Arg Arg Ser Phe Asp Val Ala Gly
        275                 280                 285
His Tyr Ser Arg Pro Asp Ile Phe Thr Leu Asn Val Asn Arg Ala Pro
    290                 295                 300
Gln Thr Ser Leu Arg Ile Arg Glu Ala Glu
305                 310

<210> 193
<211> 966
<212> DNA
<213> Unknown

<220>
<223> Obtained from an environmental sample

<400> 193
atgtcaaacg agaaccacaa ccaaacattc aaagttgccg cggtgcaggc cacacctgta      60
ttcctcgatc gtgaagcgac catcgacaaa gcttgtgagt tgattgctgc agccggcaat     120
gaaggagcgc ggctggttgt cttcccggag gcattcatcc catcctaccc agattgggta     180
tgggcaatcc caccgggcga agaaggcgtg ctcaatgagt tgtacgcgga actgctctcc     240
aattcggtca cgattcccag tgatgtgacg gatagactgt gccgagccgc gagacttgcc     300
aatgcctacg tagtgatggg gatgagcgaa cgcaatgccg aggccagtgg cgcaagcctg     360
tataacacgc tgttgtacat cgatgcgcag ggcgagattc tgggcaaaca tcgaaagctg     420
gtgcccacag gcggcgaacg gctggtgtgg gcgcagggcg atggcagcac gctgcaggtc     480
tacgatactc cactgggtaa actcggcggt ttgatttgct gggagaatta tatgccgctg     540
gcccgctaca ccatgtacgc atggggcaca caaatctatg ttgcggcgac atgggatcgc     600
gggcaaccct ggctctccac tttacggcat atcgccaaag aaggcagggt gtacgtgatc     660
ggctgctgta tcgtgatgcg caaagacgat atcccagatc gttacccgat gaagcagaag     720
ttttacgcgg aggccgatga gtggatcaac ataggggaca gcgcaatcgt caatcctgaa     780
gggcagttta gcgccgggcc ggtacgcaaa caggaagaga ttctctacgc ggaaattgat     840
ccgcgcatgg tgcaaggccc gaagtggatg ctcgacgtag cagggcacta cgcgaggccg     900
gacgtattcc agttgacggt gcatacggat gcgaggcaga tgatcaggtt ggaacacgat     960
gtttaa                                                                966

<210> 194
<211> 321
<212> PRT
<213> Unknown

<220>
<223> Obtained from an environmental sample

<400> 194
Met Ser Asn Glu Asn His Asn Gln Thr Phe Lys Val Ala Ala Val Gln
1               5                   10                  15
Ala Thr Pro Val Phe Leu Asp Arg Glu Ala Thr Ile Asp Lys Ala Cys
            20                  25                  30
Glu Leu Ile Ala Ala Ala Gly Asn Glu Gly Ala Arg Leu Val Val Phe
        35                  40                  45
Pro Glu Ala Phe Ile Pro Ser Tyr Pro Asp Trp Val Trp Ala Ile Pro
    50                  55                  60
Pro Gly Glu Glu Gly Val Leu Asn Glu Leu Tyr Ala Glu Leu Leu Ser
65                  70                  75                  80
Asn Ser Val Thr Ile Pro Ser Asp Val Thr Asp Arg Leu Cys Arg Ala
                85                  90                  95
Ala Arg Leu Ala Asn Ala Tyr Val Val Met Gly Met Ser Glu Arg Asn
            100                 105                 110
Ala Glu Ala Ser Gly Ala Ser Leu Tyr Asn Thr Leu Leu Tyr Ile Asp
        115                 120                 125
Ala Gln Gly Glu Ile Leu Gly Lys His Arg Lys Leu Val Pro Thr Gly
    130                 135                 140

Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Thr Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Val Met Arg Lys Asp Asp Ile Pro Asp Arg Tyr Pro Met Lys Gln Lys
225                 230                 235                 240
Phe Tyr Ala Glu Ala Asp Glu Trp Ile Asn Ile Gly Asp Ser Ala Ile
                245                 250                 255
Val Asn Pro Glu Gly Gln Phe Ser Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asp Ala Arg Gln Met Ile Arg Leu Glu His Asp
305                 310                 315                 320
Val


<210> 195
<211> 993
<212> DNA
<213> Unknown

<220>
<223> Obtained from an environmental sample

<400> 195
atgaaagtcg tcaaagccgc cgcggtacag ttcagcccgg tgctctatag ccgtgaggca      60
accgtcgcca aggtcgtgca gaagatccac gaactcggcc agaaaggcgt gcagttcgcc     120
accttccccg aaacggtcgt gccttattac ccttactttg cggccgtcca gacgggcatc     180
gagcttctct cgggcaccga acatctgcgc ctgctcgaac aggctgtgac tgtcccgtcc     240
gctgctaccg acgcgatcgg cgaagccgcg cgaaaggccg gcatggtcgt gtccattggc     300
gtcaatgagc gcgatggcgg cacgctgtac aacgcacaac tgctcttcga tgccgacggt     360
acgctgatcc agcgccgccg caagatcacg ccgacgcatt tcgaacgcat gatctggggc     420
cagggagatg gctcgggctt gcgtgcagtc gacagcgccg tcggccgcgt cggccagctc     480
gcatgtttcg agcacaacaa cccgctcgcc cgctacgcaa tgatcgccga cggcgagcag     540
atccattcgg cgatgtaccc tggctcggcc tttggcgagg gcttcgccca gcgtatggaa     600
atcaacatcc gccagcatgc gctcgagtcc gccgctttcg tcgtcaacgc aacagcgtgg     660
ctggacgccg accagcaggc gcaaatcatg aaggacaccg gttgtggaat cggtccgatc     720
acgggcggct gcttcaccac gatcgtctct cctgacggca tgctgatggc cgagccgctt     780
cgctcgggtg aaggcgaagt gatcgtcgat ctcgacttca cgcagatcga ccgccgcaag     840
atgctgatgg actcggccgg ccactacaac cgccctgaac tgctgagtct gatgatcgac     900
cgtacgccga ccgcgcatgt tcacgaacgc gcttcgcacc cgatgatcgt caacgaccag     960
ggttccgacg atctgcgcac ccaggctgca tga                                  993

<210> 196
<211> 330
<212> PRT
<213> Unknown

<220>
<223> Obtained from an environmental sample

<400> 196
Met Lys Val Val Lys Ala Ala Ala Val Gln Phe Ser Pro Val Leu Tyr
1               5                   10                  15
Ser Arg Glu Ala Thr Val Ala Lys Val Val Gln Lys Ile His Glu Leu
            20                  25                  30
Gly Gln Lys Gly Val Gln Phe Ala Thr Phe Pro Glu Thr Val Val Pro
        35                  40                  45
Tyr Tyr Pro Tyr Phe Ala Ala Val Gln Thr Gly Ile Glu Leu Leu Ser

    50                  55                  60
Gly Thr Glu His Leu Arg Leu Leu Glu Gln Ala Val Thr Val Pro Ser
65                  70                  75                  80
Ala Ala Thr Asp Ala Ile Gly Glu Ala Ala Arg Lys Ala Gly Met Val
                85                  90                  95
Val Ser Ile Gly Val Asn Glu Arg Asp Gly Gly Thr Leu Tyr Asn Ala
            100                 105                 110
Gln Leu Leu Phe Asp Ala Asp Gly Thr Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Ala Val Gly Arg Val Gly Gln Leu
145                 150                 155                 160
Ala Cys Phe Glu His Asn Asn Pro Leu Ala Arg Tyr Ala Met Ile Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Ala Ala Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Thr Gly Gly Cys Phe Thr Thr Ile Val Ser Pro Asp Gly Met Leu Met
                245                 250                 255
Ala Glu Pro Leu Arg Ser Gly Glu Gly Glu Val Ile Val Asp Leu Asp
            260                 265                 270
Phe Thr Gln Ile Asp Arg Arg Lys Met Leu Met Asp Ser Ala Gly His
        275                 280                 285
Tyr Asn Arg Pro Glu Leu Leu Ser Leu Met Ile Asp Arg Thr Pro Thr
    290                 295                 300
Ala His Val His Glu Arg Ala Ser His Pro Met Ile Val Asn Asp Gln
305                 310                 315                 320
Gly Ser Asp Asp Leu Arg Thr Gln Ala Ala
                325                 330

<210> 197
<211> 1017
<212> DNA
<213> Unknown

<220>
<223> Obtained from an environmental sample

<400> 197
atgggcatcg aacatccaaa ataccgcgtt gccgccgtgc aggctgcgcc ggcctggctc      60
gacctcgatc gctcgatcga caaggctatt gcactgatcg aggaggccgc cgcgaacggc     120
gccagattga tcgcattccc ggaggtcttc atccccggct acccctggca tatctggctc     180
gactcgccgg cctgggcgat cggccgcggc ttcgtgcagc gttatttcga caattcgctc     240
gcttatgata gtccgcaggc cgagcggctc cgcgcagcgg tccgcaaggc gcgcctgacc     300
gccgtgatcg gcctttcgga gcggagcggc ggcagcctct acatcgcgca atggctcgtt     360
ggccccgacg gcgagaccat cgcgaagcgc cgcaagctcc gtccgacgca tgccgagcgc     420
acggtctatg gcgagggcga cggcagcgat ctggcggtcc atgaccggcc cgatatcgga     480
cggctcggcg cgctgtgctg ctgggaacat ctgcaaccgt tgtcgaaata tgcgatgtat     540
gcccagaacg agcaggtcca tgtggcgtca tggccgagtt tttcgctcta cgatcccttt     600
gccccggcgc tcggcgcgga ggtgaacaat gcggcctccc gggtctatgc ggtcgaaggc     660
tcctgcttcg tgctggcgcc gtgcgcgacc gtctcgcagg ccatgatcga tgagctgtgc     720
gaccggcccg acaagcacgc gctgctccat gccggcggtg gctttgccgc gatctacggg     780
cccgacggca gttcgctggc cgaaaagctc gcgccggacc aggagggcct gctttacgcc     840
gacatcgatc tcggcgcgat cggcgtcgcg aagaacgccg ccgacccggc agggcattat     900
tcgcggcccg atgtcacgcg gctgctgctg aacaacaagc cctacaagcg cgtggagcat     960
tttgctttgc ccggcgatac cgtggcgcct gccgatgtgg atgcggcggc gagctga       1017

<210> 198
<211> 338
<212> PRT
<213> Unknown

<220>
<223> Obtained from an environmental sample

<400> 198
Met Gly Ile Glu His Pro Lys Tyr Arg Val Ala Ala Val Gln Ala Ala
1               5                   10                  15
Pro Ala Trp Leu Asp Leu Asp Arg Ser Ile Asp Lys Ala Ile Ala Leu
            20                  25                  30
Ile Glu Glu Ala Ala Ala Asn Gly Ala Arg Leu Ile Ala Phe Pro Glu
        35                  40                  45
Val Phe Ile Pro Gly Tyr Pro Trp His Ile Trp Leu Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Arg Leu Arg Ala Ala Val Arg Lys
                85                  90                  95
Ala Arg Leu Thr Ala Val Ile Gly Leu Ser Glu Arg Ser Gly Gly Ser
            100                 105                 110
Leu Tyr Ile Ala Gln Trp Leu Val Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asp Arg Pro Asp Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ser Trp Pro
            180                 185                 190
Ser Phe Ser Leu Tyr Asp Pro Phe Ala Pro Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Ala Gly Gly Gly Phe Ala
                245                 250                 255
Ala Ile Tyr Gly Pro Asp Gly Ser Ser Leu Ala Glu Lys Leu Ala Pro
            260                 265                 270
Asp Gln Glu Gly Leu Leu Tyr Ala Asp Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Asn Lys Pro Tyr Lys Arg Val Glu His
305                 310                 315                 320
Phe Ala Leu Pro Gly Asp Thr Val Ala Pro Ala Asp Val Asp Ala Ala
                325                 330                 335
Ala Ser


<210> 199
<211> 993
<212> DNA
<213> Unknown

<220>
<223> Obtained from an environmental sample

<400> 199
atgtcaaacg cactgaagcc gttcaaaatc gccgccgttc aggcgacgcc ggtttttctg      60
aaccgggaag ccacggcgga gaaggccgcg gctttgatcc gcgaagcggg aagcgccgga     120
gccaagctca tcgttttccc ggaatcgttt attccggcct atccggactg ggtctgggtg     180
gtcccctcgg ggagggatcg ccttctcagc ggcctctacg gggagatgct cgaaaacgcc     240
gtggaaatcc ccggcccggc cacggggcat atcggccggg cggcgaagga atcgggcgct     300
tatgtcgtca tgggcgtgac cgagcgggac acggaggcga gcggagccag tttgttcaac     360
accttgattt atttcggtcc gaccggggaa attttgggca aacaccggaa gctggttccc     420
accgggggcg aacggatcgt ctgggcccag ggggacggaa gcaccctgga ggtctacgat     480
acgcccctgg gaaaactggg cgggctgatc tgctgggaaa actacatgcc cctggcccgg     540
tacgccatgt acgcctgggg aacccagctt tacgtggccg ccacctggga ccgaggcgaa     600

ccctggcttt cgacgcttcg gcatatcgcc aaggaagggc gggtgtatgt catcgggtgc 660
tgcatcgcca tgcggaaagg ggatatcccg gatcggttcg aacacaaggg gctctacgcc 720
cccgaccggg actggatcaa ccccggcgac agcgcgatcg tcaacccccca gggggagatg 780
atcgccgggc ccgcttccaa taaggaagag atcctttatg cggaagtcga cccgcagatg 840
atgcgcgggc ccaaatggat gctcgatgtg gccggccatt acgcgcggcc cgatgtcttc 900
gagctcaccg tccgccggga accgcggccg atgatccgcg tggcgggagg cgcgggcggg 960
accgaaccca aagagaagaa gaccgccggc tga 993

<210> 200
<211> 330
<212> PRT
<213> Unknown

<220>
<223> Obtained from an environmental sample

<400> 200
Met Ser Asn Ala Leu Lys Pro Phe Lys Ile Ala Ala Val Gln Ala Thr
1               5                   10                  15
Pro Val Phe Leu Asn Arg Glu Ala Thr Ala Glu Lys Ala Ala Ala Leu
            20                  25                  30
Ile Arg Glu Ala Gly Ser Ala Gly Ala Lys Leu Ile Val Phe Pro Glu
        35                  40                  45
Ser Phe Ile Pro Ala Tyr Pro Asp Trp Val Trp Val Val Pro Ser Gly
    50                  55                  60
Arg Asp Arg Leu Leu Ser Gly Leu Tyr Gly Glu Met Leu Glu Asn Ala
65                  70                  75                  80
Val Glu Ile Pro Gly Pro Ala Thr Gly His Ile Gly Arg Ala Ala Lys
                85                  90                  95
Glu Ser Gly Ala Tyr Val Val Met Gly Val Thr Glu Arg Asp Thr Glu
            100                 105                 110
Ala Ser Gly Ala Ser Leu Phe Asn Thr Leu Ile Tyr Phe Gly Pro Thr
        115                 120                 125
Gly Glu Ile Leu Gly Lys His Arg Lys Leu Val Pro Thr Gly Gly Glu
    130                 135                 140
Arg Ile Val Trp Ala Gln Gly Asp Gly Ser Thr Leu Glu Val Tyr Asp
145                 150                 155                 160
Thr Pro Leu Gly Lys Leu Gly Gly Leu Ile Cys Trp Glu Asn Tyr Met
                165                 170                 175
Pro Leu Ala Arg Tyr Ala Met Tyr Ala Trp Gly Thr Gln Leu Tyr Val
            180                 185                 190
Ala Ala Thr Trp Asp Arg Gly Glu Pro Trp Leu Ser Thr Leu Arg His
        195                 200                 205
Ile Ala Lys Glu Gly Arg Val Tyr Val Ile Gly Cys Cys Ile Ala Met
    210                 215                 220
Arg Lys Gly Asp Ile Pro Asp Arg Phe Glu His Lys Gly Leu Tyr Ala
225                 230                 235                 240
Pro Asp Arg Asp Trp Ile Asn Pro Gly Asp Ser Ala Ile Val Asn Pro
                245                 250                 255
Gln Gly Glu Met Ile Ala Gly Pro Ala Ser Asn Lys Glu Glu Ile Leu
            260                 265                 270
Tyr Ala Glu Val Asp Pro Gln Met Met Arg Gly Pro Lys Trp Met Leu
        275                 280                 285
Asp Val Ala Gly His Tyr Ala Arg Pro Asp Val Phe Glu Leu Thr Val
    290                 295                 300
Arg Arg Glu Pro Arg Pro Met Ile Arg Val Ala Gly Gly Ala Gly Gly
305                 310                 315                 320
Thr Glu Pro Lys Glu Lys Lys Thr Ala Gly
                325                 330

<210> 201
<211> 930
<212> DNA
<213> Unknown

<220>
<223> Obtained from an environmental sample

<400> 201
atgaccacga agagcatccg catcgcggcc gtacaggcgg ctcccgcgtt tctcgacctg      60
gctggtacgc tggaccggct cgaggcctgg gcccgcaagg ccgccgccac cggtgcccgc     120
gtcatcgcgt tccccgagac ctggctgccg ggctacccgg cgtggatcga ctcgtcgccg     180
gaggccgcga tctggggcca tcccggctcg cgcgacctgc accagcgcct gatggagaat     240
gccgtcgagg tcccgggccc cgcgaccgcg cgcatcgcga agctcgccgg cgagctcggc     300
gtgacgatcg tggtcggcgc gcacgagcgg gcggggaaca ccctctacaa cacggcgctg     360
acgttcgggc ccgagggcag gctgctcaat caccaccgga agctggtgcc gacctacagc     420
gaacggctgc tgtggggcta cggcgacggc gctggactgg tggcgccggc ggtggacggt     480
gtgaaggtcg gggcgctggt gtgctgggag cactggatgc cgctcacccg ccaggcgatg     540
cacgacgtcg gcgagcacgt gcacgtcgcc ctgtggcccg gcgtccacga gatgcaccag     600
gtggcctcgc ggcactatgc gttcgagggc cgctgtttcg tgatcgcggt cgggagcatc     660
ctgcgcgtgg accagatgcc gaagcagctg ccgccgctgg agaagtacgc gaagagcgcc     720
aaggggctga tgatcgcggg cggcagcgcc atcatcgcgc cgaacggccg ctacgtcgcg     780
gcgccggtgt acgacgagga gacgatcgtc accgccgact gcgacctcgg cgagatcccg     840
cgcgaggcgc agacgctcga tgtctcgggc cactacagcc ggccggacgt gttcagcttc     900
ggggtggtca gacaccggcc gcgtgcgtaa                                      930

<210> 202
<211> 309
<212> PRT
<213> Unknown

<220>
<223> Obtained from an environmental sample

<400> 202
Met Thr Thr Lys Ser Ile Arg Ile Ala Ala Val Gln Ala Ala Pro Ala
1               5                   10                  15
Phe Leu Asp Leu Ala Gly Thr Leu Asp Arg Leu Glu Ala Trp Ala Arg
            20                  25                  30
Lys Ala Ala Ala Thr Gly Ala Arg Val Ile Ala Phe Pro Glu Thr Trp
        35                  40                  45
Leu Pro Gly Tyr Pro Ala Trp Ile Asp Ser Ser Pro Glu Ala Ala Ile
    50                  55                  60
Trp Gly His Pro Gly Ser Arg Asp Leu His Gln Arg Leu Met Glu Asn
65                  70                  75                  80
Ala Val Glu Val Pro Gly Pro Ala Thr Ala Arg Ile Ala Lys Leu Ala
                85                  90                  95
Gly Glu Leu Gly Val Thr Ile Val Val Gly Ala His Glu Arg Ala Gly
            100                 105                 110
Asn Thr Leu Tyr Asn Thr Ala Leu Thr Phe Gly Pro Glu Gly Arg Leu
        115                 120                 125
Leu Asn His His Arg Lys Leu Val Pro Thr Tyr Ser Glu Arg Leu Leu
    130                 135                 140
Trp Gly Tyr Gly Asp Gly Ala Gly Leu Val Ala Pro Ala Val Asp Gly
145                 150                 155                 160
Val Lys Val Gly Ala Leu Val Cys Trp Glu His Trp Met Pro Leu Thr
                165                 170                 175
Arg Gln Ala Met His Asp Val Gly Glu His Val His Val Ala Leu Trp
            180                 185                 190
Pro Gly Val His Glu Met His Gln Val Ala Ser Arg His Tyr Ala Phe
        195                 200                 205
Glu Gly Arg Cys Phe Val Ile Ala Val Gly Ser Ile Leu Arg Val Asp
    210                 215                 220
Gln Met Pro Lys Gln Leu Pro Pro Leu Glu Lys Tyr Ala Lys Ser Ala
225                 230                 235                 240
Lys Gly Leu Met Ile Ala Gly Gly Ser Ala Ile Ile Ala Pro Asn Gly
                245                 250                 255
Arg Tyr Val Ala Ala Pro Val Tyr Asp Glu Glu Thr Ile Val Thr Ala
            260                 265                 270
Asp Cys Asp Leu Gly Glu Ile Pro Arg Glu Ala Gln Thr Leu Asp Val
        275                 280                 285
Ser Gly His Tyr Ser Arg Pro Asp Val Phe Ser Phe Gly Val Val Arg
    290                 295                 300
His Arg Pro Arg Ala
305

<210> 203
<211> 966
<212> DNA
<213> Unknown

<220>
<223> Obtained from an environmental sample

<400> 203
atgtcaagct tacccacatc cgcgttcacc gtcgccgccg cgcaagcgtc gccagtgttc 60
ctcgaccgcg acgcgacgct gcagaaggct tgcgggctga tcgccgacgc cgggcgcgcg 120
ggcgcgcgcc tgatcgtctt ccccgaagcc ttcatcccgg cctaccccga ttgggtgtgg 180
gcggtcccag ctggcgaaga ggggatgctg agcgagctct acgccgagct ggtcgcgaat 240
tcgctggcta ttccgagcga cgcgaccgat cggctatgtc gcgcggcgca ggccgcgcat 300
atcaatgtgg tcgtggggtt gagcgagcgc aatgtcgagg ccagcggcgc cagcctctac 360
aacacgctgc tgtatatcga cgcggcggga acgatcctgg gtaaacaccg caagcttgtg 420
ccgaccggcg gggagcgcct ggtctgggcg cagggcgacg gcagcacgct cgatgtgtac 480
gacaccgcgc tcggcaagct cggcggcctg atctgttggg aaaactacat gccgctggca 540
cgctacgcgc tgtacgcctg gggtgtgcaa atctatgtcg cggccacctg ggatcgcggc 600
gagccctggc tttctactct gcgacatatc gccaaggaag gccgtgtcta cgtgatcggc 660
tgtggcatgg cgctgcgcag agatgatatt cccgatcgct tcgctttcaa gcagcgcttc 720
tatgcccagg ccggcgaatg gatcaacgtc ggcgacagcg cgatcgtcaa cccgagcggc 780
gagtttattg ccggacctgt gcgcgaacgc gaggagattc tgtacgcgga ggtcgacccg 840
gagcagatga gcgggccaaa gtggatgctc gacgtggccg ggcactacgg gcggccggat 900
gtcttccggc tcagcgtcaa ccgggcgccg caccagatga tccagacgga gaaccgggag 960
acctga 966

<210> 204
<211> 321
<212> PRT
<213> Unknown

<220>
<223> Obtained from an environmental sample

<400> 204
Met Ser Ser Leu Pro Thr Ser Ala Phe Thr Val Ala Ala Ala Gln Ala
1               5                   10                  15
Ser Pro Val Phe Leu Asp Arg Asp Ala Thr Leu Gln Lys Ala Cys Gly
            20                  25                  30
Leu Ile Ala Asp Ala Gly Arg Ala Gly Ala Arg Leu Ile Val Phe Pro
        35                  40                  45
Glu Ala Phe Ile Pro Ala Tyr Pro Asp Trp Val Trp Ala Val Pro Ala
    50                  55                  60
Gly Glu Glu Gly Met Leu Ser Glu Leu Tyr Ala Glu Leu Val Ala Asn
65                  70                  75                  80
Ser Leu Ala Ile Pro Ser Asp Ala Thr Asp Arg Leu Cys Arg Ala Ala
                85                  90                  95
Gln Ala Ala His Ile Asn Val Val Val Gly Leu Ser Glu Arg Asn Val
            100                 105                 110
Glu Ala Ser Gly Ala Ser Leu Tyr Asn Thr Leu Leu Tyr Ile Asp Ala
        115                 120                 125
Ala Gly Thr Ile Leu Gly Lys His Arg Lys Leu Val Pro Thr Gly Gly
    130                 135                 140
Glu Arg Leu Val Trp Ala Gln Gly Asp Gly Ser Thr Leu Asp Val Tyr
145                 150                 155                 160
Asp Thr Ala Leu Gly Lys Leu Gly Gly Leu Ile Cys Trp Glu Asn Tyr
                165                 170                 175
Met Pro Leu Ala Arg Tyr Ala Leu Tyr Ala Trp Gly Val Gln Ile Tyr
            180                 185                 190
Val Ala Ala Thr Trp Asp Arg Gly Glu Pro Trp Leu Ser Thr Leu Arg
        195                 200                 205
His Ile Ala Lys Glu Gly Arg Val Tyr Val Ile Gly Cys Gly Met Ala
    210                 215                 220
Leu Arg Arg Asp Asp Ile Pro Asp Arg Phe Ala Phe Lys Gln Arg Phe
225                 230                 235                 240
Tyr Ala Gln Ala Gly Glu Trp Ile Asn Val Gly Asp Ser Ala Ile Val
                245                 250                 255

Asn Pro Ser Gly Glu Phe Ile Ala Gly Pro Val Arg Glu Arg Glu Glu
            260                 265                 270
Ile Leu Tyr Ala Glu Val Asp Pro Glu Gln Met Ser Gly Pro Lys Trp
        275                 280                 285
Met Leu Asp Val Ala Gly His Tyr Gly Arg Pro Asp Val Phe Arg Leu
    290                 295                 300
Ser Val Asn Arg Ala Pro His Gln Met Ile Gln Thr Glu Asn Arg Glu
305                 310                 315                 320
Thr

<210> 205
<211> 969
<212> DNA
<213> Unknown

<220>
<223> Obtained from an environmental sample

<400> 205
atgaccaccg taaaagccgc cgcagtacag atcagccccg tgctctatag ccgggaggcc      60
accgtagaca aggtcgttcg caagatccgc gagctcggcc aaaagggagt gcagttcgcc     120
accttcccgg aaaccgtagt gccgtactac ccctacttcg ctgcagtcca gacaggcatc     180
gaactgttgt ccggcaagga acacatgcgc ctgctggagc aggccgttac cgtcccctcg     240
cccgccacgg atgcgattgc tcaggcggcg cgcgaagcca atatggtggt gtccatcggc     300
gtcaacgagc gcgacggcgg caccatctac aacacgcagc tgctcttcga tgccgacggc     360
acgctcgtgc agcgccgccg caagataacg ccaacgcact tcgagcgcat ggtctggggc     420
cagggcgatg gttcgggatt gcgcgccgcg gacaccaagg ttggccgcat cggccagttg     480
gcctgcttcg agcacaacaa cccgctcgcc cgttacgcca tgatggccga tggcgagcag     540
atccactccg ccatgtaccc gggctcggcc ttcggcgagg gcttcgcgca gcgcatggag     600
atcaacatcc gccagcatgc cctggagtct ggctgcttcg tggtgaatgc gaccgcctgg     660
ctcgatgccg accaacaggc gcagatcatg aaggacaccg gttgctcgat cggcccgatc     720
tccggcggct gcttcacgac catcgtcacg cctgagggca tgctgattgg cgagccgctc     780
cgcgagggcg aaggcgaaat catcgccgac ctcgatttct cgatgatcga tcgccgcaag     840
ctgctgatgg actcggtcgg tcactacaac cgtccggagc ttctgagcct cctgatcgat     900
cgcacgcctg ccgcgaactt ccatgaacgt accgcgagcc aggcgaacgc cggcgtcgaa     960
atcctctga                                                             969

<210> 206
<211> 322
<212> PRT
<213> Unknown

<220>
<223> Obtained from an environmental sample

<400> 206
Met Thr Thr Val Lys Ala Ala Ala Val Gln Ile Ser Pro Val Leu Tyr
 1               5                  10                  15
Ser Arg Glu Ala Thr Val Asp Lys Val Val Arg Lys Ile Arg Glu Leu
            20                  25                  30
Gly Gln Lys Gly Val Gln Phe Ala Thr Phe Pro Glu Thr Val Val Pro
        35                  40                  45
Tyr Tyr Pro Tyr Phe Ala Ala Val Gln Thr Gly Ile Glu Leu Leu Ser
    50                  55                  60
Gly Lys Glu His Met Arg Leu Leu Glu Gln Ala Val Thr Val Pro Ser
65                  70                  75                  80
Pro Ala Thr Asp Ala Ile Ala Gln Ala Ala Arg Glu Ala Asn Met Val
                85                  90                  95
Val Ser Ile Gly Val Asn Glu Arg Asp Gly Gly Thr Ile Tyr Asn Thr
            100                 105                 110
Gln Leu Leu Phe Asp Ala Asp Gly Thr Leu Val Gln Arg Arg Arg Lys
        115                 120                 125
Ile Thr Pro Thr His Phe Glu Arg Met Val Trp Gly Gln Gly Asp Gly
    130                 135                 140
Ser Gly Leu Arg Ala Ala Asp Thr Lys Val Gly Arg Ile Gly Gln Leu
145                 150                 155                 160
Ala Cys Phe Glu His Asn Asn Pro Leu Ala Arg Tyr Ala Met Met Ala

                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Gly Cys Phe Val Val Asn Ala Thr Ala Trp Leu Asp Ala Asp
    210                 215                 220
Gln Gln Ala Gln Ile Met Lys Asp Thr Gly Cys Ser Ile Gly Pro Ile
225                 230                 235                 240
Ser Gly Gly Cys Phe Thr Thr Ile Val Thr Pro Glu Gly Met Leu Ile
                245                 250                 255
Gly Glu Pro Leu Arg Glu Gly Glu Gly Glu Ile Ile Ala Asp Leu Asp
            260                 265                 270
Phe Ser Met Ile Asp Arg Arg Lys Leu Leu Met Asp Ser Val Gly His
        275                 280                 285
Tyr Asn Arg Pro Glu Leu Leu Ser Leu Leu Ile Asp Arg Thr Pro Ala
    290                 295                 300
Ala Asn Phe His Glu Arg Thr Ala Ser Gln Ala Asn Ala Gly Val Glu
305                 310                 315                 320
Ile Leu


<210> 207
<211> 966
<212> DNA
<213> Unknown

<220>
<223> Obtained from an environmental sample

<400> 207
atgtcaaacg agaacaacaa cgctacattc aaagttgccg cagtacaggc tacacctgtt      60
tttctcgatc gtgaagcgac tctcgacaag gcttgcgatt tgatcgccgc cgccggaggt     120
gaaggggcac gattggttgt ctttccagaa gccttcatac cggcctatcc ggattgggta     180
tgggcaatcc caccgggtga agagggcgta cttaatgagt tgtacgcaga gctgctctcc     240
aactcggtca cgattcccag tgacgcgacg gacagactgt gccgggccgc gaggcttgct     300
aatgcttacg tggtgatggg gataagcgaa cgcaatgtcg aggcgagtgg agcaagcctg     360
tataacacgc tgttgtacat cgatgcgcag ggtgagattc taggcaaaca tcgaaagcta     420
gtgccaacgg gcggcgagcg gctggtgtgg gcgcagggcg atggcagcac actgcaggtc     480
tacgatactc cactgggaaa actcggcggt ttaatttgct gggagaatta tatgccgctg     540
gcccgctata ccatgtatgc ctggggcaca caaatctatg tcgccgctac gtgggatcgc     600
gggcaaccct ggctctccac tttgcggcat atcgccaaag aaggcagggt gtacgtgatt     660
ggttgttgta tcgcgatgcg caaagacgat atccctgatc gttacgcaat gaagcagaag     720
ttttacgcgg aggcagatga gtggatcaat ataggtgaca gcgcgattgt caatcctgaa     780
gggcaattta tcgcagggcc agtacgcaag caggaagaga ttctctacgc agagattgat     840
ccgcgcatgg tacaagggcc gaagtggatg ctcgacgtgg cggggcacta tgccaggccg     900
gatgtgttcc agttgacggt gcatacggat gtgcgacaga tgattcggat ggaacacgat     960
tcttaa                                                                966

<210> 208
<211> 321
<212> PRT
<213> Unknown

<220>
<223> Obtained from an environmental sample

<400> 208
Met Ser Asn Glu Asn Asn Asn Ala Thr Phe Lys Val Ala Ala Val Gln
1               5                   10                  15
Ala Thr Pro Val Phe Leu Asp Arg Glu Ala Thr Leu Asp Lys Ala Cys
            20                  25                  30
Asp Leu Ile Ala Ala Ala Gly Gly Glu Gly Ala Arg Leu Val Val Phe
        35                  40                  45
Pro Glu Ala Phe Ile Pro Ala Tyr Pro Asp Trp Val Trp Ala Ile Pro
    50                  55                  60
Pro Gly Glu Glu Gly Val Leu Asn Glu Leu Tyr Ala Glu Leu Leu Ser
65                  70                  75                  80

Asn Ser Val Thr Ile Pro Ser Asp Ala Thr Asp Arg Leu Cys Arg Ala
                85                  90                  95
Ala Arg Leu Ala Asn Ala Tyr Val Val Met Gly Ile Ser Glu Arg Asn
            100                 105                 110
Val Glu Ala Ser Gly Ala Ser Leu Tyr Asn Thr Leu Leu Tyr Ile Asp
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Thr Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Asp Arg Tyr Ala Met Lys Gln Lys
225                 230                 235                 240
Phe Tyr Ala Glu Ala Asp Glu Trp Ile Asn Ile Gly Asp Ser Ala Ile
                245                 250                 255
Val Asn Pro Glu Gly Gln Phe Ile Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asp Val Arg Gln Met Ile Arg Met Glu His Asp
305                 310                 315                 320
Ser

<210> 209
<211> 993
<212> DNA
<213> Unknown

<220>
<223> Obtained from an environmental sample

<400> 209
atgaaagtcg tcaaagccgc cgctgtccag atcagtcccg ttctctacag ccgtgaggca      60
accgtcgaaa aggtcgtgaa gaaaattcac gagcttggcc aactgggcgt gcagttcgcc     120
acctttcccg agaccgtagt gccttactac ccgtactttt ccgccgtcca gacaggcatt     180
gagcttctgt ccggaactga gcatctgcgg ctgctcgatc aggccgtgac ggtaccgtct     240
cccgctaccg atgcgatcgg agaggcggcc cgcaaggcgg gcatggtggt gtccatcggc     300
gtgaatgaac gcgacggcgg caccttgtac aacacacagt tgctcttcga tgccgatggc     360
accttgatcc agcgccgccg caagatcacg cccacccact tcgaacggat gatctggggc     420
caggggggacg gctcgggcct gcgcgccgtc gacagcaagg ttggtcgcat tggtcagctt     480
gcctgcttcg agcacaacaa cccgctggcc cgctacgcgc tgattgccga cggcgagcag     540
atccattccg ccatgtatcc gggttctgct ttcggcgaag gctttgccca aaggatggaa     600
atcaatatcc gccagcatgc gctggagtct ggtgcctttg tcgtcaacgc aacggcctgg     660
ctggatgctg accagcaggc gcaaatcatc aaggacaccg gctgtgggat tggcccgatc     720
tcgggcggct gcttcaccac gatcgtggca cccgacggca tgctgatggc cgaacctctg     780
cgttcgggcg agggtgaggt catcgtggat ctcgacttca cgctgatcga ccgacgcaag     840
atgttgatgg actcggcggg ccactataac cgtccagaac tgctcagtct catgattgac     900
cgtaccgcga cggcgcatgt tcacgaacgc gctgcgcatc cggtgtcggg cgcggagcag     960
ggtccggagg atctgcgcac tccggccgcg tga                                  993

<210> 210
<211> 330
<212> PRT
<213> Unknown

<220>
<223> Obtained from an environmental sample

<400> 210
Met Lys Val Val Lys Ala Ala Ala Val Gln Ile Ser Pro Val Leu Tyr
1               5                   10                  15
Ser Arg Glu Ala Thr Val Glu Lys Val Val Lys Lys Ile His Glu Leu
            20                  25                  30
Gly Gln Leu Gly Val Gln Phe Ala Thr Phe Pro Glu Thr Val Val Pro
        35                  40                  45
Tyr Tyr Pro Tyr Phe Ser Ala Val Gln Thr Gly Ile Glu Leu Leu Ser
    50                  55                  60
Gly Thr Glu His Leu Arg Leu Leu Asp Gln Ala Val Thr Val Pro Ser
65                  70                  75                  80
Pro Ala Thr Asp Ala Ile Gly Glu Ala Ala Arg Lys Ala Gly Met Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Lys Val Gly Arg Ile Gly Gln Leu
145                 150                 155                 160
Ala Cys Phe Glu His Asn Asn Pro Leu Ala Arg Tyr Ala Leu Ile Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Gly Ala Phe Val Val Asn Ala Thr Ala Trp Leu Asp Ala Asp
    210                 215                 220
Gln Gln Ala Gln Ile Ile Lys Asp Thr Gly Cys Gly Ile Gly Pro Ile
225                 230                 235                 240
Ser Gly Gly Cys Phe Thr Thr Ile Val Ala Pro Asp Gly Met Leu Met
                245                 250                 255
Ala Glu Pro Leu Arg Ser Gly Glu Gly Glu Val Ile Val Asp Leu Asp
            260                 265                 270
Phe Thr Leu Ile Asp Arg Arg Lys Met Leu Met Asp Ser Ala Gly His
        275                 280                 285
Tyr Asn Arg Pro Glu Leu Leu Ser Leu Met Ile Asp Arg Thr Ala Thr
    290                 295                 300
Ala His Val His Glu Arg Ala Ala His Pro Val Ser Gly Ala Glu Gln
305                 310                 315                 320
Gly Pro Glu Asp Leu Arg Thr Pro Ala Ala
                325                 330

<210> 211
<211> 1062
<212> DNA
<213> Unknown

<220>
<223> Obtained from an environmental sample

<400> 211
atgggcgtcg cacatccgaa atacaaagtg gccgccgtgc aggcagcgcc cgcttttctc      60
gacctggacg cctcggtcga aaaggccgtc cgtttcatcg acgaagccgg cgccgccggc     120
gcccgcctca tcgcctttcc ggagacctgg atacccggtt acccctggtg gatctggcta     180
ggcgcgccgg cctgggctat catgcgcggc ttcgtctcgc gctatttcga caactcgctc     240
agctacgaca gcccgcaggc cgagaagctc cgcgccgccg ccaagcgcaa caagatggtg     300
gtggtgctcg gcctctccga gcgcgacggc ggcagccttt acatcgcgca atggatcatc     360
ggcccggacg gcgaaaccat cgccaagcgc cgcaagctca agccgaccca cgcggagcgg     420
accgtgttcg gcgaaggcga cggctcgcat cttgcggtgc acgagcttga tgttggccgg     480
ctcggcgcgc tgtgctgctg ggaacacctg cagccgctgt ccaaatacgc catgtatgcg     540
cagaacgaac aggtgcatgt cgcggcctgg ccgagctttt cgctttacga tccgttcgcg     600
cacgcgctcg gcgcggaagt gaacaatgcg gcgagcaaaa tctatgcggt cgagggctcg     660
tgtttcgtca tcgcgccgtg cgcgaccgtt tcgcaggcga tgatcgacga actctgcgat     720
acgccggaga agcatcagtt cctgcatgcc ggcggcggct ttgccgtgat ttacggcccc     780
gacggcgccc cgctcgcggc gccgctgccg cccgacaagg aaggcttgct ctacgccgac     840
atcgatctcg ggatgatttc ggttgccaaa gcggcagccg atccggccgg gcattatgca     900

cgccccgacg tcacccggct tctgttcaac aatcggcctg ggtatcgggt cgagaccatg 960
gcgttgccga tcgatgcgga gaccaaggcg gaagcaccgg ctaagccgga acccaaggca 1020
ccgaacgtgg cgccgttcgc gccggtgcaa gcggccgagt ga 1062

<210> 212
<211> 353
<212> PRT
<213> Unknown

<220>
<223> Obtained from an environmental sample

<400> 212
Met Gly Val Ala His Pro Lys Tyr Lys Val Ala Ala Val Gln Ala Ala
1               5                   10                  15
Pro Ala Phe Leu Asp Leu Asp Ala Ser Val Glu Lys Ala Val Arg Phe
            20                  25                  30
Ile Asp Glu Ala Gly Ala Ala Gly Ala Arg Leu Ile Ala Phe Pro Glu
        35                  40                  45
Thr Trp Ile Pro Gly Tyr Pro Trp Trp Ile Trp Leu Gly Ala Pro Ala
    50                  55                  60
Trp Ala Ile Met Arg Gly Phe Val Ser Arg Tyr Phe Asp Asn Ser Leu
65                  70                  75                  80
Ser Tyr Asp Ser Pro Gln Ala Glu Lys Leu Arg Ala Ala Ala Lys Arg
                85                  90                  95
Asn Lys Met Val Val Val Leu Gly Leu Ser Glu Arg Asp Gly Gly Ser
            100                 105                 110
Leu Tyr Ile Ala Gln Trp Ile Ile Gly Pro Asp Gly Glu Thr Ile Ala
        115                 120                 125
Lys Arg Arg Lys Leu Lys Pro Thr His Ala Glu Arg Thr Val Phe Gly
    130                 135                 140
Glu Gly Asp Gly Ser His Leu Ala Val His Glu Leu Asp Val Gly Arg
145                 150                 155                 160
Leu Gly Ala Leu Cys Cys Trp Glu His Leu Gln Pro Leu Ser Lys Tyr
                165                 170                 175
Ala Met Tyr Ala Gln Asn Glu Gln Val His Val Ala Ala Trp Pro Ser
            180                 185                 190
Phe Ser Leu Tyr Asp Pro Phe Ala His Ala Leu Gly Ala Glu Val Asn
        195                 200                 205
Asn Ala Ala Ser Lys Ile Tyr Ala Val Glu Gly Ser Cys Phe Val Ile
    210                 215                 220
Ala Pro Cys Ala Thr Val Ser Gln Ala Met Ile Asp Glu Leu Cys Asp
225                 230                 235                 240
Thr Pro Glu Lys His Gln Phe Leu His Ala Gly Gly Gly Phe Ala Val
                245                 250                 255
Ile Tyr Gly Pro Asp Gly Ala Pro Leu Ala Ala Pro Leu Pro Pro Asp
            260                 265                 270
Lys Glu Gly Leu Leu Tyr Ala Asp Ile Asp Leu Gly Met Ile Ser Val
        275                 280                 285
Ala Lys Ala Ala Ala Asp Pro Ala Gly His Tyr Ala Arg Pro Asp Val
    290                 295                 300
Thr Arg Leu Leu Phe Asn Asn Arg Pro Gly Tyr Arg Val Glu Thr Met
305                 310                 315                 320
Ala Leu Pro Ile Asp Ala Glu Thr Lys Ala Glu Ala Pro Ala Lys Pro
                325                 330                 335
Glu Pro Lys Ala Pro Asn Val Ala Pro Phe Ala Pro Val Gln Ala Ala
            340                 345                 350
Glu

<210> 213
<211> 993
<212> DNA
<213> Unknown

<220>
<223> Obtained from an environmental sample

<400> 213
atgagagttg ttaaagccgc tgctgtccaa ctgagtcccg tcctctatag tcgcgaggga 60
acggtcgaaa aggtcgtgcg gaagatccat gaacttgccg aagagggagt cgagttcgcc 120
acctttcctg agaccgtggt gccttactac ccgtactttt ccttcgttca gacgcccttg 180
gagcaaatct tcggaacaga gtatctgagg ctgctcgacc aggcagtcac cgtgccatcc 240
cctgccaccg acgcgatcgg cgaggcagcc aggttcgctg gagttgttgt ctcgatcggc 300
gtcaacgagc gagacggggg aactctatac aacactcagc ttctcttcga tgccgacggc 360
aggataattc agcggcgccg caagatcacg cccacccatt acgagcgcat gatctggggc 420
cagggcgacg gctcaggtct gcgggccgtt gatagcaagg ccggccgtat tggtcagctg 480
gcatgctggg agcacaacaa tccactggcg cgctacgcgc tgatggccga cggcgagcag 540
atccattccg ccatgtatcc gggctccatg ttcggcgact cgtttgccca gaagaccgaa 600
atcaatatcc ggcagcatgc cctagagtct gggtgcttcg tcgtgaacgc aacggcctgg 660
ctggacggcg atcagcaggc gcatatcatg aaggacaccg gctgcagcat cggcccgatc 720
tccggcggtt gcttcactgc gatcgtcgca cccgatggta gcctgctggg cgaacccatc 780
cgttccggtg agggcgtggt catcgccgac ctcgacttca cgttgatcga caggcgtaag 840
caggtgatgg actcgcgagg ccattacagc cggccggagt tgctcagcct cttaatagac 900
cgcaccccta ccgcgcactt tcacgaacgc gcttcgcccc ccacgacaga agctgagcaa 960
ggctccgagg atgtgttcga ggctcgcatt taa 993

<210> 214
<211> 330
<212> PRT
<213> Unknown

<220>
<223> Obtained from an environmental sample

<400> 214
Met Arg Val Val Lys Ala Ala Ala Val Gln Leu Ser Pro Val Leu Tyr
1               5                   10                  15
Ser Arg Glu Gly Thr Val Glu Lys Val Val Arg Lys Ile His Glu Leu
            20                  25                  30
Ala Glu Glu Gly Val Glu Phe Ala Thr Phe Pro Glu Thr Val Val Pro
        35                  40                  45
Tyr Tyr Pro Tyr Phe Ser Phe Val Gln Thr Pro Leu Glu Gln Ile Phe
    50                  55                  60
Gly Thr Glu Tyr Leu Arg Leu Leu Asp Gln Ala Val Thr Val Pro Ser
65                  70                  75                  80
Pro Ala Thr Asp Ala Ile Gly Glu Ala Ala Arg Phe Ala Gly Val Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Arg Ile Ile Gln Arg Arg Arg Lys
        115                 120                 125
Ile Thr Pro Thr His Tyr Glu Arg Met Ile Trp Gly Gln Gly Asp Gly
    130                 135                 140
Ser Gly Leu Arg Ala Val Asp Ser Lys Ala Gly Arg Ile Gly Gln Leu
145                 150                 155                 160
Ala Cys Trp Glu His Asn Asn Pro Leu Ala Arg Tyr Ala Leu Met Ala
                165                 170                 175
Asp Gly Glu Gln Ile His Ser Ala Met Tyr Pro Gly Ser Met Phe Gly
            180                 185                 190
Asp Ser Phe Ala Gln Lys Thr Glu Ile Asn Ile Arg Gln His Ala Leu
        195                 200                 205
Glu Ser Gly Cys Phe Val Val Asn Ala Thr Ala Trp Leu Asp Gly Asp
    210                 215                 220
Gln Gln Ala His Ile Met Lys Asp Thr Gly Cys Ser Ile Gly Pro Ile
225                 230                 235                 240
Ser Gly Gly Cys Phe Thr Ala Ile Val Ala Pro Asp Gly Ser Leu Leu
                245                 250                 255
Gly Glu Pro Ile Arg Ser Gly Glu Gly Val Val Ile Ala Asp Leu Asp
            260                 265                 270
Phe Thr Leu Ile Asp Arg Arg Lys Gln Val Met Asp Ser Arg Gly His
        275                 280                 285
Tyr Ser Arg Pro Glu Leu Leu Ser Leu Leu Ile Asp Arg Thr Pro Thr
    290                 295                 300
Ala His Phe His Glu Arg Ala Ser Pro Pro Thr Thr Glu Ala Glu Gln
305                 310                 315                 320

Gly Ser Glu Asp Val Phe Glu Ala Arg Ile
                325                 330

<210> 215
<211> 1008
<212> DNA
<213> Unknown

<220>
<223> Obtained from an environmental sample

<400> 215
atgagtatta cccaccccaa atttaaagct gctgttgttc aagctgcccc agtatttcta      60
gacctagatg ggtctgttaa taaggcgatt aatctcattg atgaagctgc cgctgccgga     120
gccaagctca ttgccttccc tgaaaccttc attccaggct atccatggtg gatttggctg     180
ggatcgccgg cgtgggctct gggccggggg ttcgttcagc gttacttcga caattccctg     240
cagtacgaca gcccgcaggc ggatcgctta cgcgaggcgg cacgacgcaa cagcattacg     300
gtcgtgctgg gcttgtccga gcgtgatggc ggttctctct atatcgcaca gtggctgatc     360
ggcccggatg gcgaaaccat cgcgcagcgg cgcaagcttc gtcctactca tggggagcgc     420
acggtattcg gtgaagggga tggcagcgat ctggtggttc atcaaaccga actgggccgt     480
cttggcgcgc ttaactgctg ggagaacatc ctgtctctga acaaatatgt gatgtactcc     540
cagcatgaac aggtccatgt agcatcctgg cccagtttct cgacgtatga accgttcgcg     600
catgcgctcg gctatgaggt aaacaacgca attagccagg tctatgcggt ggaaggcggg     660
tgcttcgtgt tggccccgtg ctctaccatc tctgaagaaa tgattgccga actgtgcgat     720
acacccgata aattcgagct gacgcatgct ggtggcggcc acgcaatcat ctatggtccg     780
gacggtcgtg ctctgtgcga aaagctgccc gagaaccagg agggcctgct gtacgcggaa     840
atcgatctgg gggtgatttc tatggcaaaa agtgccatgg atcctgtcgg ccattactct     900
cgccccgatg tctaccgtgt gctgttcaat aagatcccgg caaagcgtat cgagcacttc     960
aatttgccgt tggatgagca agcaggggaa gagccaccag ctgattaa                 1008

<210> 216
<211> 335
<212> PRT
<213> Unknown

<220>
<223> Obtained from an environmental sample

<400> 216
Met Ser Ile Thr His Pro Lys Phe Lys Ala Ala Val Val Gln Ala Ala
1               5                   10                  15
Pro Val Phe Leu Asp Leu Asp Gly Ser Val Asn Lys Ala Ile Asn Leu
            20                  25                  30
Ile Asp Glu Ala Ala Ala Ala Gly Ala Lys Leu Ile Ala Phe Pro Glu
        35                  40                  45
Thr Phe Ile Pro Gly Tyr Pro Trp Trp Ile Trp Leu Gly Ser Pro Ala
    50                  55                  60
Trp Ala Leu Gly Arg Gly Phe Val Gln Arg Tyr Phe Asp Asn Ser Leu
65                  70                  75                  80
Gln Tyr Asp Ser Pro Gln Ala Asp Arg Leu Arg Glu Ala Ala Arg Arg
                85                  90                  95
Asn Ser Ile Thr Val Val Leu Gly Leu Ser Glu Arg Asp Gly Gly Ser
            100                 105                 110
Leu Tyr Ile Ala Gln Trp Leu Ile Gly Pro Asp Gly Glu Thr Ile Ala
        115                 120                 125
Gln Arg Arg Lys Leu Arg Pro Thr His Gly Glu Arg Thr Val Phe Gly
    130                 135                 140
Glu Gly Asp Gly Ser Asp Leu Val Val His Gln Thr Glu Leu Gly Arg
145                 150                 155                 160
Leu Gly Ala Leu Asn Cys Trp Glu Asn Ile Leu Ser Leu Asn Lys Tyr
                165                 170                 175
Val Met Tyr Ser Gln His Glu Gln Val His Val Ala Ser Trp Pro Ser
            180                 185                 190
Phe Ser Thr Tyr Glu Pro Phe Ala His Ala Leu Gly Tyr Glu Val Asn
        195                 200                 205
Asn Ala Ile Ser Gln Val Tyr Ala Val Glu Gly Gly Cys Phe Val Leu
    210                 215                 220
Ala Pro Cys Ser Thr Ile Ser Glu Glu Met Ile Ala Glu Leu Cys Asp

225                 230                 235                 240
Thr Pro Asp Lys Phe Glu Leu Thr His Ala Gly Gly Gly His Ala Ile
                245                 250                 255
Ile Tyr Gly Pro Asp Gly Arg Ala Leu Cys Glu Lys Leu Pro Glu Asn
            260                 265                 270
Gln Glu Gly Leu Leu Tyr Ala Glu Ile Asp Leu Gly Val Ile Ser Met
        275                 280                 285
Ala Lys Ser Ala Met Asp Pro Val Gly His Tyr Ser Arg Pro Asp Val
    290                 295                 300
Tyr Arg Val Leu Phe Asn Lys Ile Pro Ala Lys Arg Ile Glu His Phe
305                 310                 315                 320
Asn Leu Pro Leu Asp Glu Gln Ala Gly Glu Glu Pro Pro Ala Asp
                325                 330                 335

<210> 217
<211> 1011
<212> DNA
<213> Unknown

<220>
<223> Obtained from an environmental sample

<400> 217
gtgggtatca gccatccgaa attcaaggct gcggtcgtac aggccgggcc tgccttcctc      60
gacctcgacg gcggcgtcga acgagccgtg tcgctcatcg gccaagcagc ggccgaaggg     120
gcacagctga ttgcctttcc tgaaacgtgg attcccggtt acccgtggca cacctggctt     180
ggcagcccgg cgtgggcaat ggaaaaaggc tttgtccaac gatatttcga caacgcgttg     240
cggcatggtt ctccgcaagc cgagcgaatc tccggggctg cggcggagca caagattatg     300
gtgtcgcttg ggtttgcgga acgcgatgga ggcacgcttt atatcgcgca gtggctcatc     360
ggacccgacg gccaaactat ctcacgacgg cggaagctta agccgactca cgtcgagcgc     420
actgtatttg gcgagggaga cggaagcgat ctctccgtgc atgatacggc gcttggacgt     480
atcggctcac tttgttgctg ggagcatttg caaccgttgt cgaaatacgc aatgtacgcc     540
cagaatgaac agattcacat tggcgcatgg cccagctttt cgctatacca gccatttgcg     600
aatgcgctga gtcccgaagt caatatcgca gtaagccgcg tgtacgccgt ggaaggccag     660
tgtttcttcc tcgcgccgtg cgcgacggtt tcggacgcca tgatcgaaac actgtgcgat     720
acgcccgaaa agcagggact gattcgggcg ggtggcgggc acgccgcgat cttcggccca     780
gatggaagtc tgctgacgcc tacggtagcg gatacttacg agggcctgct gtatgcagaa     840
ctcgacctcg gcgtcatttc gatcgccaag agtgcagcgg accccgccgg ccactattcg     900
cggccagatg tcacacgcct tctattgaat cagacgcctt cgaagcgcgt tcagaatatg     960
gtgttaccac tggagacggt cacggagccc gaaggcccgg ttcagcccta g             1011

<210> 218
<211> 336
<212> PRT
<213> Unknown

<220>
<223> Obtained from an environmental sample

<400> 218
Val Gly Ile Ser His Pro Lys Phe Lys Ala Ala Val Val Gln Ala Gly
1               5                   10                  15
Pro Ala Phe Leu Asp Leu Asp Gly Gly Val Glu Arg Ala Val Ser Leu
            20                  25                  30
Ile Gly Gln Ala Ala Ala Glu Gly Ala Gln Leu Ile Ala Phe Pro Glu
        35                  40                  45
Thr Trp Ile Pro Gly Tyr Pro Trp His Thr Trp Leu Gly Ser Pro Ala
    50                  55                  60
Trp Ala Met Glu Lys Gly Phe Val Gln Arg Tyr Phe Asp Asn Ala Leu
65                  70                  75                  80
Arg His Gly Ser Pro Gln Ala Glu Arg Ile Ser Gly Ala Ala Ala Glu
                85                  90                  95
His Lys Ile Met Val Ser Leu Gly Phe Ala Glu Arg Asp Gly Gly Thr
            100                 105                 110
Leu Tyr Ile Ala Gln Trp Leu Ile Gly Pro Asp Gly Gln Thr Ile Ser
        115                 120                 125
Arg Arg Arg Lys Leu Lys Pro Thr His Val Glu Arg Thr Val Phe Gly
    130                 135                 140

Glu Gly Asp Gly Ser Asp Leu Ser Val His Asp Thr Ala Leu Gly Arg
145                 150                 155                 160
Ile Gly Ser Leu Cys Cys Trp Glu His Leu Gln Pro Leu Ser Lys Tyr
                165                 170                 175
Ala Met Tyr Ala Gln Asn Glu Gln Ile His Ile Gly Ala Trp Pro Ser
            180                 185                 190
Phe Ser Leu Tyr Gln Pro Phe Ala Asn Ala Leu Ser Pro Glu Val Asn
        195                 200                 205
Ile Ala Val Ser Arg Val Tyr Ala Val Glu Gly Gln Cys Phe Phe Leu
    210                 215                 220
Ala Pro Cys Ala Thr Val Ser Asp Ala Met Ile Glu Thr Leu Cys Asp
225                 230                 235                 240
Thr Pro Glu Lys Gln Gly Leu Ile Arg Ala Gly Gly Gly His Ala Ala
                245                 250                 255
Ile Phe Gly Pro Asp Gly Ser Leu Leu Thr Pro Thr Val Ala Asp Thr
            260                 265                 270
Tyr Glu Gly Leu Leu Tyr Ala Glu Leu Asp Leu Gly Val Ile Ser Ile
        275                 280                 285
Ala Lys Ser Ala Ala Asp Pro Ala Gly His Tyr Ser Arg Pro Asp Val
    290                 295                 300
Thr Arg Leu Leu Leu Asn Gln Thr Pro Ser Lys Arg Val Gln Asn Met
305                 310                 315                 320
Val Leu Pro Leu Glu Thr Val Thr Glu Pro Glu Gly Pro Val Gln Pro
                325                 330                 335

<210> 219
<211> 996
<212> DNA
<213> Unknown

<220>
<223> Obtained from an environmental sample

<400> 219
atgaaaatcg tcaaagctgc cgccgttcaa attagtcccg tgctttatag ccgcgatgga      60
acggtggaaa aagttgtgcg gaagatccat gagctcggcc aacagggagt gcagttcgca     120
accttccccg agaccgtgat tccgtactat ccctattttt cctttctgca acccgcctac     180
cagatcgcgg ccgggcagga gcacctcaag ttgctcgacc aggcggtgac ggtgccgtcc     240
gctgccacgc acgcgatcgg ccaggcatgc aaacaggcag gagtggttgt ttcgatcggc     300
atcaatgagc gggacaatgg aacgatctac aacacccagc ttctcttcga ctccgacgga     360
accctgctgc agcggcgccg caagatctca cccacttacc acgaacggat gatctggggc     420
cagggcgacg gttccggcct gcgcgccgtg gatagcaagg ccggccggat tgggcagctc     480
gcctgttggg agcactacaa ccctttggcg cgctacgcga tgatggccga cggcgagcag     540
atccattccg ccatgtaccc tggctccatc ttcggcgatc tgtttgccga acagacccag     600
atcaacgtcc ggcagcacgc gttggagtcc ggctgtttcg tagtctgctc caccgcctgg     660
ttggatcccg atcaacaagc gcaaatcgtc aaggacacag gcggcgccat tggtgcgatc     720
tcgggcggct gtttcacggc gattgtggcc cccgatagca ctctgctcgg agagccgatc     780
cgctccggcg aaggcgtggt cattgcggat ctcgacttca cccggatcga caagcgtaaa     840
cagttgatgg attcccgagg tcattacagc cggccggaat tgctgagtct gctgattgat     900
cgcacttcca ccgcacatgt gcaaaaccgc gtgccggccg atccattcgg cgccggctgt     960
gttgcagaac caggagtaga aacatgccca cgatag                               996

<210> 220
<211> 331
<212> PRT
<213> Unknown

<220>
<223> Obtained from an environmental sample

<400> 220
Met Lys Ile Val Lys Ala Ala Ala Val Gln Ile Ser Pro Val Leu Tyr
1               5                   10                  15
Ser Arg Asp Gly Thr Val Glu Lys Val Val Arg Lys Ile His Glu Leu
            20                  25                  30
Gly Gln Gln Gly Val Gln Phe Ala Thr Phe Pro Glu Thr Val Ile Pro
        35                  40                  45
Tyr Tyr Pro Tyr Phe Ser Phe Leu Gln Pro Ala Tyr Gln Ile Ala Ala

50                  55                  60
Gly Gln Glu His Leu Lys Leu Leu Asp Gln Ala Val Thr Val Pro Ser
65                  70                  75                  80
Ala Ala Thr His Ala Ile Gly Gln Ala Cys Lys Gln Ala Gly Val Val
                85                  90                  95
Val Ser Ile Gly Ile Asn Glu Arg Asp Asn Gly Thr Ile Tyr Asn Thr
            100                 105                 110
Gln Leu Leu Phe Asp Ser Asp Gly Thr Leu Leu Gln Arg Arg Arg Lys
        115                 120                 125
Ile Ser Pro Thr Tyr His Glu Arg Met Ile Trp Gly Gln Gly Asp Gly
    130                 135                 140
Ser Gly Leu Arg Ala Val Asp Ser Lys Ala Gly Arg Ile Gly Gln Leu
145                 150                 155                 160
Ala Cys Trp Glu His Tyr Asn Pro Leu Ala Arg Tyr Ala Met Met Ala
                165                 170                 175
Asp Gly Glu Gln Ile His Ser Ala Met Tyr Pro Gly Ser Ile Phe Gly
            180                 185                 190
Asp Leu Phe Ala Glu Gln Thr Gln Ile Asn Val Arg Gln His Ala Leu
        195                 200                 205
Glu Ser Gly Cys Phe Val Val Cys Ser Thr Ala Trp Leu Asp Pro Asp
    210                 215                 220
Gln Gln Ala Gln Ile Val Lys Asp Thr Gly Gly Ala Ile Gly Ala Ile
225                 230                 235                 240
Ser Gly Gly Cys Phe Thr Ala Ile Val Ala Pro Asp Ser Thr Leu Leu
                245                 250                 255
Gly Glu Pro Ile Arg Ser Gly Glu Gly Val Val Ile Ala Asp Leu Asp
            260                 265                 270
Phe Thr Arg Ile Asp Lys Arg Lys Gln Leu Met Asp Ser Arg Gly His
        275                 280                 285
Tyr Ser Arg Pro Glu Leu Leu Ser Leu Leu Ile Asp Arg Thr Ser Thr
    290                 295                 300
Ala His Val Gln Asn Arg Val Pro Ala Asp Pro Phe Gly Ala Gly Cys
305                 310                 315                 320
Val Ala Glu Pro Gly Val Glu Thr Cys Pro Arg
                325                 330

<210> 221
<211> 1146
<212> DNA
<213> Unknown

<220>
<223> Obtained from an environmental sample

<400> 221
atgttgacct acaaaggcgt gttcaaagca gcgacagttc aggcggagcc ggtgtggatg      60
gacgctgacg ccaccattac caaagccatc cggatcatcg aagaggcggc tgataacggg     120
gcgaagtttg tcgcattccc ggaggtgttc atccctggat acccttggtg gatctggctg     180
ggcactgcga tgtggggagc caaattcgtc gtgcccttcc acgaaaattg ccttgagctt     240
ggcgacaagc ggatgcagcg cattcaagct gcggcaaaac aaaatggcat tgcactcgtc     300
atgggttacg gtgaacgtga cggaggtagc cgctacatga gccaggtatt catcgacgat     360
agcggaaaaa ttgtggcgaa ccgccgcaag ctcaaaccga cgcacgaaga gcgaacgatt     420
ttcggcgagg gaaacggatc cgatttcatc acccatgact tcccatttgc gagagtcgga     480
ggcttcaact gctgggaaca ccttcagccc ctgagcaaat acatgatgta cagcctgcag     540
gaacaggtgc atgtcgcgtc gtggccggcc atgtgcacct atcagcctga cgtgccccag     600
ttaggtgcag gcgctaacga ggcggtcacc cgctcttacg cgatcgaagg cgcatgttat     660
gtgctgggag cgacactcgt tatcggcaag gcagcccatg atgcattctg cgacactgaa     720
gagcatcaca agctgcttgg catgggcgga gggtgggccc gaattttcgg cccggacggt     780
gagtatctcg cagaatcgct cgctcatgac gcggagggca tcctgtacgc tgacattgat     840
ctgagcaaga tcctgctcgc caaagcaaac acggacacag ttggccacta cgcgcgacct     900
gacgtcttgt cattgctcgt caacacacac aatcccggcc ctgtacgcta tcttgacgaa     960
gagggccggc aggtttcaac gtcaattcgc aggcacgaaa aactggaagg ccagagcctc    1020
gacctcgagg ttacgccagc gacaccagcg accctggaca tcgcgagcct ggtacaacag    1080
gctaagccat cgaccgttaa gagcgagagt aacgcgagca caaaacaacc ggatctcgcg    1140
gtctga                                                               1146

<210> 222
<211> 381

<212> PRT
<213> Unknown

<220>
<223> Obtained from an environmental sample

<400> 222
Met Leu Thr Tyr Lys Gly Val Phe Lys Ala Ala Thr Val Gln Ala Glu
1               5                   10                  15
Pro Val Trp Met Asp Ala Asp Ala Thr Ile Thr Lys Ala Ile Arg Ile
            20                  25                  30
Ile Glu Glu Ala Ala Asp Asn Gly Ala Lys Phe Val Ala Phe Pro Glu
        35                  40                  45
Val Phe Ile Pro Gly Tyr Pro Trp Trp Ile Trp Leu Gly Thr Ala Met
    50                  55                  60
Trp Gly Ala Lys Phe Val Val Pro Phe His Glu Asn Cys Leu Glu Leu
65                  70                  75                  80
Gly Asp Lys Arg Met Gln Arg Ile Gln Ala Ala Ala Lys Gln Asn Gly
                85                  90                  95
Ile Ala Leu Val Met Gly Tyr Gly Glu Arg Asp Gly Gly Ser Arg Tyr
            100                 105                 110
Met Ser Gln Val Phe Ile Asp Asp Ser Gly Lys Ile Val Ala Asn Arg
        115                 120                 125
Arg Lys Leu Lys Pro Thr His Glu Glu Arg Thr Ile Phe Gly Glu Gly
    130                 135                 140
Asn Gly Ser Asp Phe Ile Thr His Asp Phe Pro Phe Ala Arg Val Gly
145                 150                 155                 160
Gly Phe Asn Cys Trp Glu His Leu Gln Pro Leu Ser Lys Tyr Met Met
                165                 170                 175
Tyr Ser Leu Gln Glu Gln Val His Val Ala Ser Trp Pro Ala Met Cys
            180                 185                 190
Thr Tyr Gln Pro Asp Val Pro Gln Leu Gly Ala Gly Ala Asn Glu Ala
        195                 200                 205
Val Thr Arg Ser Tyr Ala Ile Glu Gly Ala Cys Tyr Val Leu Gly Ala
    210                 215                 220
Thr Leu Val Ile Gly Lys Ala Ala His Asp Ala Phe Cys Asp Thr Glu
225                 230                 235                 240
Glu His His Lys Leu Leu Gly Met Gly Gly Gly Trp Ala Arg Ile Phe
                245                 250                 255
Gly Pro Asp Gly Glu Tyr Leu Ala Glu Ser Leu Ala His Asp Ala Glu
            260                 265                 270
Gly Ile Leu Tyr Ala Asp Ile Asp Leu Ser Lys Ile Leu Leu Ala Lys
        275                 280                 285
Ala Asn Thr Asp Thr Val Gly His Tyr Ala Arg Pro Asp Val Leu Ser
    290                 295                 300
Leu Leu Val Asn Thr His Asn Pro Gly Pro Val Arg Tyr Leu Asp Glu
305                 310                 315                 320
Glu Gly Arg Gln Val Ser Thr Ser Ile Arg Arg His Glu Lys Leu Glu
                325                 330                 335
Gly Gln Ser Leu Asp Leu Glu Val Thr Pro Ala Thr Pro Ala Thr Leu
            340                 345                 350
Asp Ile Ala Ser Leu Val Gln Gln Ala Lys Pro Ser Thr Val Lys Ser
        355                 360                 365
Glu Ser Asn Ala Ser Thr Lys Gln Pro Asp Leu Ala Val
    370                 375                 380

<210> 223
<211> 996
<212> DNA
<213> Unknown

<220>
<223> Obtained from an environmental sample

<400> 223
atgagagttg ttaaagccag cgcagttcaa ctgaagcctg tactttatag ccgcgagggg      60
acggtcgaaa aggtcgtagc gaagattcat gagctagggc agcagggtgt gcagttcgcc     120
gcgttccctg agaccgtggt gccttattac ccgtactttt cgatcgtgca gtccggctat     180

caaatccttc gcggcggtga gttcgtcaag ctcctcgatc agtccgtgac ggtgccatct 240
tatagcactg aagccatcgg cgaagcctgc aggcaggagg agatggttgt ctccataggc 300
gtcaacgagc gtgatggcgg aacgatctac aacgcgcagt tactctttga ttccgacggc 360
acgttgatcc aaagacgacg caagatcacc cccacccatt acgaacgcat gatctggggc 420
cagggcgatg gctcaggtct gcgcgctgtt gacagcaatg tcgcacgcat tggtcaactg 480
gcatgctttg agcactacaa ccctcttgcg cggtacgcga tgatggccga tggcgagcag 540
atccattccg ccatgttccc cggttccatg ttcggcgatg gttttgcgga gaggacggaa 600
attgccgtaa ggcaacatgc gatggagtcc gggtgctttg tcgtttgcgc tacggcctgg 660
ctcgatcccg gccagcaggc tcagatcgcc aacgacaccg gtatcaccga catcggcccg 720
atctccgggg gttgcttcac tgcgatcatc gcacccgatg ggagcctgct gggccaacct 780
atccgctcgg gtgaaggcga agtcatcgtc gacctcgatt tcacgttaat tgacaagcgg 840
aaacatattg tcgactcgag aggacattac agccggccag aattgctgag cctgctgatc 900
gatcgcactc ccaccgcgca ccttcacgac cgcgctgtgc agcacaatgc cggatcggaa 960
ggagcgtcgg aacatcttcg cgaagacgcc gcctga 996

<210> 224
<211> 331
<212> PRT
<213> Unknown

<220>
<223> Obtained from an environmental sample

<400> 224
Met Arg Val Val Lys Ala Ser Ala Val Gln Leu Lys Pro Val Leu Tyr
1               5                   10                  15
Ser Arg Glu Gly Thr Val Glu Lys Val Val Ala Lys Ile His Glu Leu
            20                  25                  30
Gly Gln Gln Gly Val Gln Phe Ala Ala Phe Pro Glu Thr Val Val Pro
        35                  40                  45
Tyr Tyr Pro Tyr Phe Ser Ile Val Gln Ser Gly Tyr Gln Ile Leu Arg
    50                  55                  60
Gly Gly Glu Phe Val Lys Leu Leu Asp Gln Ser Val Thr Val Pro Ser
65                  70                  75                  80
Tyr Ser Thr Glu Ala Ile Gly Glu Ala Cys Arg Gln Glu Glu Met Val
                85                  90                  95
Val Ser Ile Gly Val Asn Glu Arg Asp Gly Gly Thr Ile Tyr Asn Ala
            100                 105                 110
Gln Leu Leu Phe Asp Ser Asp Gly Thr Leu Ile Gln Arg Arg Arg Lys
        115                 120                 125
Ile Thr Pro Thr His Tyr Glu Arg Met Ile Trp Gly Gln Gly Asp Gly
    130                 135                 140
Ser Gly Leu Arg Ala Val Asp Ser Asn Val Ala Arg Ile Gly Gln Leu
145                 150                 155                 160
Ala Cys Phe Glu His Tyr Asn Pro Leu Ala Arg Tyr Ala Met Met Ala
                165                 170                 175
Asp Gly Glu Gln Ile His Ser Ala Met Phe Pro Gly Ser Met Phe Gly
            180                 185                 190
Asp Gly Phe Ala Glu Arg Thr Glu Ile Ala Val Arg Gln His Ala Met
        195                 200                 205
Glu Ser Gly Cys Phe Val Val Cys Ala Thr Ala Trp Leu Asp Pro Gly
    210                 215                 220
Gln Gln Ala Gln Ile Ala Asn Asp Thr Gly Ile Thr Asp Ile Gly Pro
225                 230                 235                 240
Ile Ser Gly Gly Cys Phe Thr Ala Ile Ile Ala Pro Asp Gly Ser Leu
                245                 250                 255
Leu Gly Gln Pro Ile Arg Ser Gly Glu Gly Glu Val Ile Val Asp Leu
            260                 265                 270
Asp Phe Thr Leu Ile Asp Lys Arg Lys His Ile Val Asp Ser Arg Gly
        275                 280                 285
His Tyr Ser Arg Pro Glu Leu Leu Ser Leu Leu Ile Asp Arg Thr Pro
    290                 295                 300
Thr Ala His Leu His Asp Arg Ala Val Gln His Asn Ala Gly Ser Glu
305                 310                 315                 320
Gly Ala Ser Glu His Leu Arg Glu Asp Ala Ala
                325                 330

<210> 225

<211> 951
<212> DNA
<213> Unknown

<220>
<223> Obtained from an environmental sample

<400> 225
atgaccaccg tcaaagccgc cgctgtccag atgagtcccg tgctttacag tcgcgacgat 60
accatcgaga aaatttgtcg acagatcatc gagcttggcc gacagggagt gcagttcgcc 120
acgtttccag agacagttat tccgtattat ccatatttcg ccttcgtgca gcggccttac 180
gaaatgtcgg cccaatatca tcagttgctt gatcaagcgg tgaccgtgcc ttccggttca 240
acgcacgcca ttggggccgc ctgtaaacaa gccggaattg ttgtctcaat cggcgtcaac 300
gagcgggagg gcggcacgct ctatggcaca cagttgctgt ttgatgcaga cggcctcttg 360
atccagcgtc gccgcaagat cactccgacc taccacgagc ggatgatttg gggacagggt 420
gacggttccg ggctgcgagc cgtagatagc gcggtcggtc gaatcggaca gttggcgtgt 480
tgggaacacc acaatccgct ggctcgttat gctttagcgg ccgatggcga acaaattcac 540
gcggcgatgt accctggctc gatcttgggt gaactatttg ccgagcagat tcaggtcaac 600
atccggcagc acgccatgga atctggttgc ttcgtcgtga acgccacggc ctggctaagc 660
gaggaacagc aagcccgaat catgaaggac accggatcat tcgatagccc aatcaccggt 720
ggttgcttta ccgccattgt cgcgcccaac gggcagataa tcggtgaacc gctgcgcatc 780
ggcgaaggcg tcgtgattgc cgatttggac ttcgctttga ttgatgagag gaagcggctg 840
atggactcac gcggcctcta tagccgccct gagttgctaa gcttgttaat cgacagaatg 900
cctacatccc atgtgcatga acgggttgag cgtagcatgg cgatggcatg a 951

<210> 226
<211> 316
<212> PRT
<213> Unknown

<220>
<223> Obtained from an environmental sample

<400> 226
Met Thr Thr Val Lys Ala Ala Ala Val Gln Met Ser Pro Val Leu Tyr
1               5                   10                  15
Ser Arg Asp Asp Thr Ile Glu Lys Ile Cys Arg Gln Ile Ile Glu Leu
            20                  25                  30
Gly Arg Gln Gly Val Gln Phe Ala Thr Phe Pro Glu Thr Val Ile Pro
        35                  40                  45
Tyr Tyr Pro Tyr Phe Ala Phe Val Gln Arg Pro Tyr Glu Met Ser Ala
    50                  55                  60
Gln Tyr His Gln Leu Leu Asp Gln Ala Val Thr Val Pro Ser Gly Ser
65                  70                  75                  80
Thr His Ala Ile Gly Ala Ala Cys Lys Gln Ala Gly Ile Val Val Ser
                85                  90                  95
Ile Gly Val Asn Glu Arg Glu Gly Gly Thr Leu Tyr Gly Thr Gln Leu
            100                 105                 110
Leu Phe Asp Ala Asp Gly Leu Leu Ile Gln Arg Arg Arg Lys Ile Thr
        115                 120                 125
Pro Thr Tyr His Glu Arg Met Ile Trp Gly Gln Gly Asp Gly Ser Gly
    130                 135                 140
Leu Arg Ala Val Asp Ser Ala Val Gly Arg Ile Gly Gln Leu Ala Cys
145                 150                 155                 160
Trp Glu His His Asn Pro Leu Ala Arg Tyr Ala Leu Ala Ala Asp Gly
                165                 170                 175
Glu Gln Ile His Ala Ala Met Tyr Pro Gly Ser Ile Leu Gly Glu Leu
            180                 185                 190
Phe Ala Glu Gln Ile Gln Val Asn Ile Arg Gln His Ala Met Glu Ser
        195                 200                 205
Gly Cys Phe Val Val Asn Ala Thr Ala Trp Leu Ser Glu Glu Gln Gln
    210                 215                 220
Ala Arg Ile Met Lys Asp Thr Gly Ser Phe Asp Ser Pro Ile Thr Gly
225                 230                 235                 240
Gly Cys Phe Thr Ala Ile Val Ala Pro Asn Gly Gln Ile Ile Gly Glu
                245                 250                 255
Pro Leu Arg Ile Gly Glu Gly Val Val Ile Ala Asp Leu Asp Phe Ala
            260                 265                 270

Leu Ile Asp Glu Arg Lys Arg Leu Met Asp Ser Arg Gly Leu Tyr Ser
        275                 280                 285
Arg Pro Glu Leu Leu Ser Leu Leu Ile Asp Arg Met Pro Thr Ser His
    290                 295                 300
Val His Glu Arg Val Glu Arg Ser Met Ala Met Ala
305                 310                 315

<210> 227
<211> 1035
<212> DNA
<213> Unknown

<220>
<223> Obtained from an environmental sample

<400> 227
atgtccgatc gacgcatcgt tcgcgcggca gccattcaga tcgccccgga tctcgagcgc      60
tgcagtgtaa cgctcgagaa ggtttgctcg gcgatcgacg aagccgcggg caaaggcgct     120
caactgagcg tctttcccga gaccttcgtc ccgtactacc cctatttctc gtttgtccgc     180
cctccggtcg catcgggggc cgatcacatg cggctatacg aggaagcggt ggtggtgccg     240
ggtcccgtga cgcaggctgt ttccgaacgt gcgcgcatgc atgggatggt ggtggtgctc     300
ggcgtcaacg agcgcgacca cgggagcctc tacaacaccc aactgatttt cgattgcgat     360
ggtcggctcg ctctgaaacg ccgcaagatc acgccgacgt ttcacgagcg catgatctgg     420
ggccagggcg acgccagtgg actgaaagtc gcgcgcacgg gtatcgggcg ggtcggcgcg     480
ctcgcgtgct gggagcatta caacccgctc gcgcgctacg cgctgatgac ccagcacgaa     540
gagatccatt gcagtcaatt tccaggctcg ctcgtcggtc cgatcttctc cgagcagatg     600
gacgtgacga tccgccatca cgccctcgaa tcggggtgct tcgtcgtgaa cgcaaccggt     660
tggctcacgg acgcgcagat cgcctcgatc accgatgacc cgaagcttca acgggcgctg     720
cgcggtggct gcaacacggc gatcgtttca ccggaaggcc agcatctggc ggagccgttg     780
cgcgaaggcg aggggatggt ggtcgcggac ctcgacatgt cgctcatcac caagcgcaag     840
cgaatgatgg attcggtcgg ccactatgcg cggccggaac tgttgagcct cgcgatcaac     900
gatcgtcccg cagccccttt cggccggatg tgcgctgccg aagcaatgcg gggagccgac     960
gacgtcgtca cagtaggagc atttcatgag cgccagcgag aacgtgtcgg cgaagagccg    1020
gcaattgatg actga                                                     1035

<210> 228
<211> 344
<212> PRT
<213> Unknown

<220>
<223> Obtained from an environmental sample

<400> 228
Met Ser Asp Arg Arg Ile Val Arg Ala Ala Ala Ile Gln Ile Ala Pro
 1               5                  10                  15
Asp Leu Glu Arg Cys Ser Val Thr Leu Glu Lys Val Cys Ser Ala Ile
            20                  25                  30
Asp Glu Ala Ala Gly Lys Gly Ala Gln Leu Ser Val Phe Pro Glu Thr
        35                  40                  45
Phe Val Pro Tyr Tyr Pro Tyr Phe Ser Phe Val Arg Pro Pro Val Ala
    50                  55                  60
Ser Gly Ala Asp His Met Arg Leu Tyr Glu Glu Ala Val Val Val Pro
65                  70                  75                  80
Gly Pro Val Thr Gln Ala Val Ser Glu Arg Ala Arg Met His Gly Met
                85                  90                  95
Val Val Val Leu Gly Val Asn Glu Arg Asp His Gly Ser Leu Tyr Asn
            100                 105                 110
Thr Gln Leu Ile Phe Asp Cys Asp Gly Arg Leu Ala Leu Lys Arg Arg
        115                 120                 125
Lys Ile Thr Pro Thr Phe His Glu Arg Met Ile Trp Gly Gln Gly Asp
    130                 135                 140
Ala Ser Gly Leu Lys Val Ala Arg Thr Gly Ile Gly Arg Val Gly Ala
145                 150                 155                 160
Leu Ala Cys Trp Glu His Tyr Asn Pro Leu Ala Arg Tyr Ala Leu Met
                165                 170                 175
Thr Gln His Glu Glu Ile His Cys Ser Gln Phe Pro Gly Ser Leu Val
            180                 185                 190

Gly Pro Ile Phe Ser Glu Gln Met Asp Val Thr Ile Arg His His Ala
        195                 200                 205
Leu Glu Ser Gly Cys Phe Val Val Asn Ala Thr Gly Trp Leu Thr Asp
    210                 215                 220
Ala Gln Ile Ala Ser Ile Thr Asp Asp Pro Lys Leu Gln Arg Ala Leu
225                 230                 235                 240
Arg Gly Gly Cys Asn Thr Ala Ile Val Ser Pro Glu Gly Gln His Leu
                245                 250                 255
Ala Glu Pro Leu Arg Glu Gly Glu Gly Met Val Val Ala Asp Leu Asp
            260                 265                 270
Met Ser Leu Ile Thr Lys Arg Lys Arg Met Met Asp Ser Val Gly His
        275                 280                 285
Tyr Ala Arg Pro Glu Leu Leu Ser Leu Ala Ile Asn Asp Arg Pro Ala
    290                 295                 300
Ala Pro Phe Gly Arg Met Cys Ala Ala Glu Ala Met Arg Gly Ala Asp
305                 310                 315                 320
Asp Val Val Thr Val Gly Ala Phe His Glu Arg Gln Arg Glu Arg Val
                325                 330                 335
Gly Glu Glu Pro Ala Ile Asp Asp
            340

<210> 229
<211> 975
<212> DNA
<213> Unknown

<220>
<223> Obtained from an environmental sample

<400> 229
atgcctgaaa acaaagtcaa agttgccctc gttcagcatc cgcctgtttt tctgaatttg      60
ccgaaaacgt tggaaaaagt agaaggtttg gcgcgagagt gcgccgccaa tgaagcgaaa     120
atcgttgtct ttcctgaaac ctggctgacc ggctatccgg tctggcttga tgaagcgccg     180
agagccgcgc tgtgggatta tccgcccgcc aagcgtttgt atcaatacct cacggaaaat     240
tcattgcaga ttccgagcgc tgagtttgaa tctttgcgcg aaatcgctaa gaaaaattcc     300
ctttatttag tcgttggagt tcacgaacga agcggcggaa cgctctacaa tacgataatt     360
tatctcacgc ccgacggcag ttataaaact caccgcaaat tggttccaac ttacacggaa     420
agacttgtct ggggcgcagg cgacggaagc ggtctaaatg ttgtggaaac gccttacggg     480
attctcggag gtttgatttg ctgggaacat tggatgcctc tggctagggc ggcaatgcat     540
tcaaaaaatg aagcgattca cgtttgccaa tttcccacgg ttcacgagcg acatcaaatc     600
gccagccgtc attacgcctt cgaagggcag tgttttgtct tgacttccgg ttgcgcgatg     660
acgaaaacgg atgttttgga aggttttgaa tcgctcgaaa caaacgacca cgaagttttc     720
gggcttttgg attcgataga aaaggaagaa ctgatgcgtg gcggaagcgc gattattgcg     780
cccgatttga gctattcggt cgagccggtt tttgacgaaa aaacgattgt ttacggcgaa     840
ttaaatctcg atttaaccaa gcaggacatc ctgtttttgg ataccgacgg acattattcg     900
cgtcccgatg ttttcgagtt gcgcgtcaac gataaagcga accgaaacgt ccgttttgca     960
tccgaaacag tatag                                                      975

<210> 230
<211> 324
<212> PRT
<213> Unknown

<220>
<223> Obtained from an environmental sample

<400> 230
Met Pro Glu Asn Lys Val Lys Val Ala Leu Val Gln His Pro Pro Val
1               5                   10                  15
Phe Leu Asn Leu Pro Lys Thr Leu Glu Lys Val Glu Gly Leu Ala Arg
            20                  25                  30
Glu Cys Ala Ala Asn Glu Ala Lys Ile Val Val Phe Pro Glu Thr Trp
        35                  40                  45
Leu Thr Gly Tyr Pro Val Trp Leu Asp Glu Ala Pro Arg Ala Ala Leu
    50                  55                  60
Trp Asp Tyr Pro Pro Ala Lys Arg Leu Tyr Gln Tyr Leu Thr Glu Asn
65                  70                  75                  80
Ser Leu Gln Ile Pro Ser Ala Glu Phe Glu Ser Leu Arg Glu Ile Ala

                85                  90                  95
Lys Lys Asn Ser Leu Tyr Leu Val Val Gly Val His Glu Arg Ser Gly
            100                 105                 110
Gly Thr Leu Tyr Asn Thr Ile Ile Tyr Leu Thr Pro Asp Gly Ser Tyr
        115                 120                 125
Lys Thr His Arg Lys Leu Val Pro Thr Tyr Thr Glu Arg Leu Val Trp
    130                 135                 140
Gly Ala Gly Asp Gly Ser Gly Leu Asn Val Val Glu Thr Pro Tyr Gly
145                 150                 155                 160
Ile Leu Gly Gly Leu Ile Cys Trp Glu His Trp Met Pro Leu Ala Arg
                165                 170                 175
Ala Ala Met His Ser Lys Asn Glu Ala Ile His Val Cys Gln Phe Pro
            180                 185                 190
Thr Val His Glu Arg His Gln Ile Ala Ser Arg His Tyr Ala Phe Glu
        195                 200                 205
Gly Gln Cys Phe Val Leu Thr Ser Gly Cys Ala Met Thr Lys Thr Asp
    210                 215                 220
Val Leu Glu Gly Phe Glu Ser Leu Glu Thr Asn Asp His Glu Val Phe
225                 230                 235                 240
Gly Leu Leu Asp Ser Ile Glu Lys Glu Glu Leu Met Arg Gly Gly Ser
                245                 250                 255
Ala Ile Ile Ala Pro Asp Leu Ser Tyr Ser Val Glu Pro Val Phe Asp
            260                 265                 270
Glu Lys Thr Ile Val Tyr Gly Glu Leu Asn Leu Asp Leu Thr Lys Gln
        275                 280                 285
Gly His Leu Phe Leu Asp Thr Asp Gly His Tyr Ser Arg Pro Asp Val
    290                 295                 300
Phe Glu Leu Arg Val Asn Asp Lys Ala Asn Arg Asn Val Arg Phe Ala
305                 310                 315                 320
Ser Glu Thr Val

<210> 231
<211> 1062
<212> DNA
<213> Unknown

<220>
<223> Obtained from an environmental sample

<400> 231
atgagtcaga tccatccaaa acttaaggtc gcggccgtgc aggctgcccc cgcctttctc 60
gatctcgatg catcgatcga aaagacaata cgctatgtcg acgaagcggc tgcggccggg 120
gcgaagttga ttgcgtttcc ggaaacctgg attcccggct acccatggtg gatctggctc 180
ggcgctccgg cctgggcgat catgcgtggc ttcgtctcgc gctatttcga caactcgctg 240
caatatggca gtccggaagc tgaacggctg cgggacgccg ccaggcgcaa caagatatac 300
atcgccctcg gcctgtccga gcgcgacggg ggcagtcttt atatcgcgca atggatcatc 360
gggcctggcg gcgaaacggt tgcacaacgc cgcaagctca agcccacgca cgccgagcgc 420
actgtattcg gcgaaggcga tggttcacat ctggccgtgc atgatctcga tattggaaga 480
ttgggcgcgc tttgttgctg ggaacatctg caaccgttgt cgaaatatgc aatgtacgcc 540
cagaacgagc aaattcacgt cgccgcctgg ccgagcttct cgctatacga tccctttgca 600
cacgcactcg gcgccgaggt caataacgct gcgagcaaga tctatgcggt cgagggatcg 660
tgcttcgtca ttgcgccgtg cgcaacggtt tcgcaggtga tgatcgatga gctctgcgat 720
acccccgaaa agcatcaatt ccttcacgtc ggcggcggct tcgccgtcat ttacggtccc 780
gacggctcgc cactggccaa acctcttccg ccagaccagg agggacttct ctatgccgac 840
atcgatctcg gcatgatctc ggtcgccaag gccgcagccg atcccgccgg acattatgca 900
cgtcccgatg taactcgcct gctgttcaac aatcgcccgg caaaccgcgt cgagaagctg 960
gcgttgccgg tcgatcagga ggccgaagtg gatagtccgc tgaaggctcc cgacgcatct 1020
cccaaagtga cggcgctcaa gccgtcgcag gctgcggagt ag 1062

<210> 232
<211> 353
<212> PRT
<213> Unknown

<220>
<223> Obtained from an environmental sample

<400> 232
Met Ser Gln Ile His Pro Lys Leu Lys Val Ala Ala Val Gln Ala Ala
1               5                   10                  15
Pro Ala Phe Leu Asp Leu Asp Ala Ser Ile Glu Lys Thr Ile Arg Tyr
            20                  25                  30
Val Asp Glu Ala Ala Ala Ala Gly Ala Lys Leu Ile Ala Phe Pro Glu
        35                  40                  45
Thr Trp Ile Pro Gly Tyr Pro Trp Trp Ile Trp Leu Gly Ala Pro Ala
    50                  55                  60
Trp Ala Ile Met Arg Gly Phe Val Ser Arg Tyr Phe Asp Asn Ser Leu
65                  70                  75                  80
Gln Tyr Gly Ser Pro Glu Ala Glu Arg Leu Arg Asp Ala Ala Arg Arg
                85                  90                  95
Asn Lys Ile Tyr Ile Ala Leu Gly Leu Ser Glu Arg Asp Gly Gly Ser
            100                 105                 110
Leu Tyr Ile Ala Gln Trp Ile Ile Gly Pro Gly Gly Glu Thr Val Ala
        115                 120                 125
Gln Arg Arg Lys Leu Lys Pro Thr His Ala Glu Arg Thr Val Phe Gly
    130                 135                 140
Glu Gly Asp Gly Ser His Leu Ala Val His Asp Leu Asp Ile Gly Arg
145                 150                 155                 160
Leu Gly Ala Leu Cys Cys Trp Glu His Leu Gln Pro Leu Ser Lys Tyr
                165                 170                 175
Ala Met Tyr Ala Gln Asn Glu Gln Ile His Val Ala Ala Trp Pro Ser
            180                 185                 190
Phe Ser Leu Tyr Asp Pro Phe Ala His Ala Leu Gly Ala Glu Val Asn
        195                 200                 205
Asn Ala Ala Ser Lys Ile Tyr Ala Val Glu Gly Ser Cys Phe Val Ile
    210                 215                 220
Ala Pro Cys Ala Thr Val Ser Gln Val Met Ile Asp Glu Leu Cys Asp
225                 230                 235                 240
Thr Pro Glu Lys His Gln Phe Leu His Val Gly Gly Gly Phe Ala Val
                245                 250                 255
Ile Tyr Gly Pro Asp Gly Ser Pro Leu Ala Lys Pro Leu Pro Pro Asp
            260                 265                 270
Gln Glu Gly Leu Leu Tyr Ala Asp Ile Asp Leu Gly Met Ile Ser Val
        275                 280                 285
Ala Lys Ala Ala Ala Asp Pro Ala Gly His Tyr Ala Arg Pro Asp Val
    290                 295                 300
Thr Arg Leu Leu Phe Asn Asn Arg Pro Ala Asn Arg Val Glu Lys Leu
305                 310                 315                 320
Ala Leu Pro Val Asp Gln Glu Ala Glu Val Asp Ser Pro Leu Lys Ala
                325                 330                 335
Pro Asp Ala Ser Pro Lys Val Thr Ala Leu Lys Pro Ser Gln Ala Ala
            340                 345                 350
Glu

<210> 233
<211> 1002
<212> DNA
<213> Unknown

<220>
<223> Obtained from an environmental sample

<400> 233
gtgagcacca tcgtcaaagc cgcggccgtg cagatcagcc ccgtgctgta cagccgcgag      60
ggcaccgtcg agcgggtcgt gaagaagatc cgggagctgg gcgaaaaggg cgtccagttc     120
gccaccttcc ccgagaccgt catcccttac tacccgtact tttccttcgt tcagacgccc     180
ttgcagatcc tcgccggccc cgagcatctg aagctgctcg accagtcggt gaccgtgccg     240
tcccccgcca cggacgcgat cggccaggcc gcccggcagg caggaatggt ggtgtccatc     300
ggcgtcaacg agcgtgacgg cggcaccctg tacaacacgc agctgctctt cgacgcggac     360
ggcgcgctga tccagcgtcg ccgcaagatc aagcccaccc actacgagcg catgatctgg     420
ggcgagggcg acggctccgg cctgcgcgcc gtcgacagcc aggtcggtcg tatcggccag     480
ctggcctgct gggagcacaa caaccccctg gcgcgctacg ccatgatggc cgacggcgag     540
cagatccatt cggccatgta tccgggctcg atgttcggcg acccgttcgc ccagaagacg     600
gaaatcaaca tccggcagca tgcgctggaa tccggatgct tcgtcgtctg ctcgacggcc     660

tggttggacg ccgatcagca ggcgcaaatc atgcaggaca cgggctgcgc catcggcccg 720
atctcgggcg gctgcctcac ggcgatcgtg gcgcccgacg gcacgttcct gggcgaaccg 780
ctcacgtcgg gcgagggcga ggtcatcgcc gacctcgatt tcaagctgat cgacaagcgc 840
aagcagacga tggactcgcg cggccactac aaccgccccg aactgctcag cctgctgatc 900
gatcgaacgc cgacgtcgaa cgtccatgag cgcgccgcgc acccgaaggt cgaggcgtca 960
caaacggctg gcgacacgga gcggacccgc gaggtcctgt aa 1002

<210> 234
<211> 333
<212> PRT
<213> Unknown

<220>
<223> Obtained from an environmental sample

<400> 234
Val Ser Thr Ile Val Lys Ala Ala Ala Val Gln Ile Ser Pro Val Leu
1               5                   10                  15
Tyr Ser Arg Glu Gly Thr Val Glu Arg Val Val Lys Lys Ile Arg Glu
            20                  25                  30
Leu Gly Glu Lys Gly Val Gln Phe Ala Thr Phe Pro Glu Thr Val Ile
        35                  40                  45
Pro Tyr Tyr Pro Tyr Phe Ser Phe Val Gln Thr Pro Leu Gln Ile Leu
    50                  55                  60
Ala Gly Pro Glu His Leu Lys Leu Leu Asp Gln Ser Val Thr Val Pro
65                  70                  75                  80
Ser Pro Ala Thr Asp Ala Ile Gly Gln Ala Ala Arg Gln Ala Gly Met
                85                  90                  95
Val Val Ser Ile Gly Val Asn Glu Arg Asp Gly Gly Thr Leu Tyr Asn
            100                 105                 110
Thr Gln Leu Leu Phe Asp Ala Asp Gly Ala Leu Ile Gln Arg Arg Arg
        115                 120                 125
Lys Ile Lys Pro Thr His Tyr Glu Arg Met Ile Trp Gly Glu Gly Asp
    130                 135                 140
Gly Ser Gly Leu Arg Ala Val Asp Ser Gln Val Gly Arg Ile Gly Gln
145                 150                 155                 160
Leu Ala Cys Trp Glu His Asn Asn Pro Leu Ala Arg Tyr Ala Met Met
                165                 170                 175
Ala Asp Gly Glu Gln Ile His Ser Ala Met Tyr Pro Gly Ser Met Phe
            180                 185                 190
Gly Asp Pro Phe Ala Gln Lys Thr Glu Ile Asn Ile Arg Gln His Ala
        195                 200                 205
Leu Glu Ser Gly Cys Phe Val Val Cys Ser Thr Ala Trp Leu Asp Ala
    210                 215                 220
Asp Gln Gln Ala Gln Ile Met Gln Asp Thr Gly Cys Ala Ile Gly Pro
225                 230                 235                 240
Ile Ser Gly Gly Cys Leu Thr Ala Ile Val Ala Pro Asp Gly Thr Phe
                245                 250                 255
Leu Gly Glu Pro Leu Thr Ser Gly Glu Gly Glu Val Ile Ala Asp Leu
            260                 265                 270
Asp Phe Lys Leu Ile Asp Lys Arg Lys Gln Thr Met Asp Ser Arg Gly
        275                 280                 285
His Tyr Asn Arg Pro Glu Leu Leu Ser Leu Leu Ile Asp Arg Thr Pro
    290                 295                 300
Thr Ser Asn Val His Glu Arg Ala Ala His Pro Lys Val Glu Ala Ser
305                 310                 315                 320
Gln Thr Ala Gly Asp Thr Glu Arg Thr Arg Glu Val Leu
                325                 330

<210> 235
<211> 993
<212> DNA
<213> Unknown

<220>
<223> Obtained from an environmental sample

<400> 235

atgaaaattg ttaaagcggc agcagttcag ctgagccccg tcctctatag ccgcgagggg 60
acggtcgaaa gagtagtgcg gaagattcat caacttggtc aacagggagt gcagtttgcc 120
accttcccgg aaacagtggt gccttactac ccgtattttt cgatcgtgca gtccgggtat 180
caaatccttc gcggcggtga gttcgtgaag ctgctcgatc agtcagtgac ggtgccatct 240
cttgccaccg aagcgatcgc cgaggcctgc aggcaggcgg gcgtcgttgt ctccatcggc 300
gtcaatgagc gtgacggcgg aactatatac aatgcgcagc ttctgtttga ttcggacggc 360
acattgattc agaggcgacg caagatcacg cccacccact acgagcgcat gatctggggc 420
cagggcgatg gctcgggtct gcgggctgtg gacagcaagg tggcacgtat tggtcaactg 480
gcgtgctttg agcattacaa ccccctcgca cgatacgcga tgatcgccga tggcgagcag 540
atccactctg caatgtttcc cggttccatg ttcggcgatg gtttcgcgga gaggaccgag 600
atcgcggtca ggcagcatgc gcaggagtcc ggatgctttg tagtttgtgc tacggcgtgg 660
ctggatgccg accagcaggc tcaaattgcc gcggacacag gcatcaccga cctgggaccg 720
atctccggcg gttgcttcac tgcgatcatt gcacctgatg ggagcctgct gggtcaacca 780
atccgctcgg gcgaaggtga cgtcattgtc gatctcgatt tcactctgat cgacaggcgg 840
aagcatgttg tggactcgag aggtcactac agccggccgg aattgctaag cctgctgatc 900
gaccgtactc ccacagcgca cgttcacgaa cgggccgcgc actctcactt ggccgccgag 960
caatgcttgg aggatcttaa cgcgcttgct taa 993

<210> 236
<211> 330
<212> PRT
<213> Unknown

<220>
<223> Obtained from an environmental sample

<400> 236
Met Lys Ile Val Lys Ala Ala Ala Val Gln Leu Ser Pro Val Leu Tyr
1               5                   10                  15
Ser Arg Glu Gly Thr Val Glu Arg Val Val Arg Lys Ile His Gln Leu
            20                  25                  30
Gly Gln Gln Gly Val Gln Phe Ala Thr Phe Pro Glu Thr Val Val Pro
        35                  40                  45
Tyr Tyr Pro Tyr Phe Ser Ile Val Gln Ser Gly Tyr Gln Ile Leu Arg
    50                  55                  60
Gly Gly Glu Phe Val Lys Leu Leu Asp Gln Ser Val Thr Val Pro Ser
65                  70                  75                  80
Leu Ala Thr Glu Ala Ile Ala Glu Ala Cys Arg Gln Ala Gly Val Val
                85                  90                  95
Val Ser Ile Gly Val Asn Glu Arg Asp Gly Gly Thr Ile Tyr Asn Ala
            100                 105                 110
Gln Leu Leu Phe Asp Ser Asp Gly Thr Leu Ile Gln Arg Arg Arg Lys
        115                 120                 125
Ile Thr Pro Thr His Tyr Glu Arg Met Ile Trp Gly Gln Gly Asp Gly
    130                 135                 140
Ser Gly Leu Arg Ala Val Asp Ser Lys Val Ala Arg Ile Gly Gln Leu
145                 150                 155                 160
Ala Cys Phe Glu His Tyr Asn Pro Leu Ala Arg Tyr Ala Met Ile Ala
                165                 170                 175
Asp Gly Glu Gln Ile His Ser Ala Met Phe Pro Gly Ser Met Phe Gly
            180                 185                 190
Asp Gly Phe Ala Glu Arg Thr Glu Ile Ala Val Arg Gln His Ala Gln
        195                 200                 205
Glu Ser Gly Cys Phe Val Val Cys Ala Thr Ala Trp Leu Asp Ala Asp
    210                 215                 220
Gln Gln Ala Gln Ile Ala Ala Asp Thr Gly Ile Thr Asp Leu Gly Pro
225                 230                 235                 240
Ile Ser Gly Gly Cys Phe Thr Ala Ile Ile Ala Pro Asp Gly Ser Leu
                245                 250                 255
Leu Gly Gln Pro Ile Arg Ser Gly Glu Gly Asp Val Ile Val Asp Leu
            260                 265                 270
Asp Phe Thr Leu Ile Asp Arg Arg Lys His Val Val Asp Ser Arg Gly
        275                 280                 285
His Tyr Ser Arg Pro Glu Leu Leu Ser Leu Leu Ile Asp Arg Thr Pro
    290                 295                 300
Thr Ala His Val His Glu Arg Ala Ala His Ser His Leu Ala Ala Glu
305                 310                 315                 320
Gln Cys Leu Glu Asp Leu Asn Ala Leu Ala

325                    330

<210> 237
<211> 993
<212> DNA
<213> Unknown

<220>
<223> Obtained from an environmental sample

<400> 237
atgaaagtcg tcaaagccgc cgcagtgcag atcagtcctg ttctctacag ccgagaggca      60
accgtcgcca aggtcgtgca gaagatccac gaactcggcc agaaaggcgt gcagttcgcc     120
acctttccag agaccgtagt gccttactac ccgtactttt ccgccgtcca gacaggcatc     180
gagcttctgt ccggcacgga gcatctccgg ctgctcgatc aggccgtgac ggtgccgtct     240
gccgctaccg acgcgatcgg agaggcagcc cggaaggcag gcatggtggt gtcgatcggc     300
gtcaatgaac gcgatggcgg caccttgtac aacacacagt tgctcttcga tgccgatggc     360
accttgatcc agcgccgccg caagatcacg ccgacccact tcgaacgcat gatctggggc     420
cagggggacg gttcgggcct gcgcgctgtc gacagcaagg tcggtcgcat tggccagttg     480
gcctgcttcg agcacaacaa cccgctggcc cgctacgcgt tgattgccga cggcgagcag     540
atccattccg ccatgtatcc gggttctgct tttggcgaag gatttgccca aaggatggaa     600
atcaatatcc gccagcatgc gctggagtcg ggtgcgttcg tcgtcaacgc aacggcctgg     660
ctggatgctg accagcaggc gcagatcatg aaggacaccg gctgcgggat tggcccgatc     720
tcgggcggtt gcttcaccac gatcgtgtca cccgacggca tgctgatggc cgaacccctg     780
cgctcgggcg agggtgaggt catcgtcgat ctcgacttca cgctgatcga ccgtcgcaag     840
atgttgatgg actcggcggg ccactataac cgcccggaac tgctcagtct catgatcgac     900
cgcaccccga ccgcgcacgt tcatgaacgc gctgcgcgtc cggtgtcggg cgttgagcag     960
aacccggagg aacttcgcat cccggccgcg tga                                  993

<210> 238
<211> 330
<212> PRT
<213> Unknown

<220>
<223> Obtained from an environmental sample

<400> 238
Met Lys Val Val Lys Ala Ala Ala Val Gln Ile Ser Pro Val Leu Tyr
1               5                   10                  15
Ser Arg Glu Ala Thr Val Ala Lys Val Val Gln Lys Ile His Glu Leu
            20                  25                  30
Gly Gln Lys Gly Val Gln Phe Ala Thr Phe Pro Glu Thr Val Val Pro
        35                  40                  45
Tyr Tyr Pro Tyr Phe Ser Ala Val Gln Thr Gly Ile Glu Leu Leu Ser
    50                  55                  60
Gly Thr Glu His Leu Arg Leu Leu Asp Gln Ala Val Thr Val Pro Ser
65                  70                  75                  80
Ala Ala Thr Asp Ala Ile Gly Glu Ala Ala Arg Lys Ala Gly Met Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Lys Val Gly Arg Ile Gly Gln Leu
145                 150                 155                 160
Ala Cys Phe Glu His Asn Asn Pro Leu Ala Arg Tyr Ala Leu Ile Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Gly Ala Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240

Ser Gly Gly Cys Phe Thr Thr Ile Val Ser Pro Asp Gly Met Leu Met
                245                 250                 255
Ala Glu Pro Leu Arg Ser Gly Glu Gly Glu Val Ile Val Asp Leu Asp
            260                 265                 270
Phe Thr Leu Ile Asp Arg Arg Lys Met Leu Met Asp Ser Ala Gly His
        275                 280                 285
Tyr Asn Arg Pro Glu Leu Leu Ser Leu Met Ile Asp Arg Thr Pro Thr
    290                 295                 300
Ala His Val His Glu Arg Ala Ala Arg Pro Val Ser Gly Val Glu Gln
305                 310                 315                 320
Asn Pro Glu Glu Leu Arg Ile Pro Ala Ala
                325                 330

<210> 239
<211> 969
<212> DNA
<213> Unknown

<220>
<223> Obtained from an environmental sample

<400> 239
atgtccaatg agaataagta tgacacattt aaagttgctg cagtccaggc cacacctgtg      60
tttcttgatc gtgaagcaac catcgacaaa gcttgcgagt tgattgctac tgcaggtcgt     120
gaaggtgctc gcctgattgt ctttccagaa gcgttcatcc catcctatcc cgagtgggta     180
tggggtattc cctctggtga gcaaggttta ctcaacgaac tttattcaga gttgctcacc     240
aatgcggtta ccatacccag cgacgcgact gacagattgt gcgaggcggc aaagcttgcg     300
aatgcctatg tagtgatggg aatgagcgaa cggaatgtcg aagcgagtgg tgcaagcctg     360
tataacacga tgttgtatat agatgcacag ggggagattt tagggaaaca tcggaagctg      420
gtgccaacgg gtggtgaacg cctggtatgg gcgcaaggtg atggcagcac gctgcaggtc     480
tacgatactc cattgggaaa acttggtggg ttaatttgct gggaaaatta tatgccgctg     540
gcacgctata cgatgtatgc ctggggaaca caaatctatg ttgcagcaac gtgggattgc     600
ggccaaccct ggctctcaac gatacggcat attgctaaag aaggcagggt atacgtggtt     660
ggttgctgta tcgcgatgcg taaagatgat attccagatc gttactctat gaagcagaaa     720
tattatgctg aaatggatga atggataaat gttggggata gcgcgattgt caatcccgaa     780
ggacatttta ttgcagggcc tgtgcgcaag caagaagaaa ttctctatgc ggagattgat     840
ccacgtatga tgcaaggccc gaagtggatg cttgacgtgg cgggacatta tgcaagacca     900
gatgtgttcc agttgacggt gcatacggat gtgaggcaga tgatacgggt ggaagatgat     960
tctcaatga                                                             969

<210> 240
<211> 322
<212> PRT
<213> Unknown

<220>
<223> Obtained from an environmental sample

<400> 240
Met Ser Asn Glu Asn Lys Tyr Asp Thr Phe Lys Val Ala Ala Val Gln
 1               5                  10                  15
Ala Thr Pro Val Phe Leu Asp Arg Glu Ala Thr Ile Asp Lys Ala Cys
            20                  25                  30
Glu Leu Ile Ala Thr Ala Gly Arg Glu Gly Ala Arg Leu Ile Val Phe
        35                  40                  45
Pro Glu Ala Phe Ile Pro Ser Tyr Pro Glu Trp Val Trp Gly Ile Pro
    50                  55                  60
Ser Gly Glu Gln Gly Leu Leu Asn Glu Leu Tyr Ser Glu Leu Leu Thr
65                  70                  75                  80
Asn Ala Val Thr Ile Pro Ser Asp Ala Thr Asp Arg Leu Cys Glu Ala
                85                  90                  95
Ala Lys Leu Ala Asn Ala Tyr Val Val Met Gly Met Ser Glu Arg Asn
            100                 105                 110
Val Glu Ala Ser Gly Ala Ser Leu Tyr Asn Thr Met Leu Tyr Ile Asp
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val

145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Thr Met Tyr Ala Trp Gly Thr Gln Ile
            180                 185                 190
Tyr Val Ala Ala Thr Trp Asp Cys Gly Gln Pro Trp Leu Ser Thr Ile
        195                 200                 205
Arg His Ile Ala Lys Glu Gly Arg Val Tyr Val Val Gly Cys Cys Ile
    210                 215                 220
Ala Met Arg Lys Asp Asp Ile Pro Asp Arg Tyr Ser Met Lys Gln Lys
225                 230                 235                 240
Tyr Tyr Ala Glu Met Asp Glu Trp Ile Asn Val Gly Asp Ser Ala Ile
                245                 250                 255
Val Asn Pro Glu Gly His Phe Ile Ala Gly Pro Val Arg Lys Gln Glu
            260                 265                 270
Glu Ile Leu Tyr Ala Glu Ile Asp Pro Arg Met Met Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asp Val Arg Gln Met Ile Arg Val Glu Asp Asp
305                 310                 315                 320
Ser Gln

<210> 241
<211> 972
<212> DNA
<213> Unknown

<220>
<223> Obtained from an environmental sample

<400> 241
atgatatcca acgagcataa caatactcca ttcaaggttg ctgctgtgca ggctacacct      60
gtgtttcttg atcgcgaagc aacgatcgat aaagcgtgtg aactgatcgc tactgccggt     120
catgaaggcg ctcgtttgat tgtttttcca gaagcgttca tcccatccta tcccgagtgg     180
gtatggggaa ttccctctgg cgagcaaggt ttgctcaacg atctgtatgc agagttactc     240
accaattcag ttacgatacc cagcaacgca actgacaggc tttgtagagc cgcgaagctt     300
gctaatgcct acgtggtgat ggggatgagc gaacggaata tcgaagcgag cggcgcaagc     360
ctgtacaata cgatgttata tatagatgca cagggtgaga ttttgggcaa acatcgaaag     420
ctggtgccaa cgggcggaga acgcctggta tgggcacaag gagatggaag cacgctgcag     480
gtttacgata cacctctagg aaagcttggt ggtttaattt gctgggaaaa ttatatgccg     540
ctggcacgct acgctatgta tgcctgggga actcaaatct acgtcgcggc aacgtgggat     600
cgcggccaac cctggctctc aacgatacgg catatcgcta aagagggcag ggtatacgta     660
atcggttgct gtatcgcgat gcgtaaagac gatattccag ataggtactc catgaagcag     720
aagtattatg cggagatgga tgaatggatc aacgtaggtg acagcgcgat tgtcaatcct     780
gagggggact tcattgcggg gcctgtgagc aagcaggagg aaattctcta tgcggagatt     840
gatccgcgga tggtgcaagg tccgaagtgg atgctggatg tggcggggca ttacgcgagg     900
cctgatgtgt tcgagttgac ggtgcatacg gatgtgaggg agatgatgcg ggtggagcat     960
gattatcaat ga                                                         972

<210> 242
<211> 323
<212> PRT
<213> Unknown

<220>
<223> Obtained from an environmental sample

<400> 242
Met Ile Ser Asn Glu His Asn Asn Thr Pro Phe Lys Val Ala Ala Val
1               5                   10                  15
Gln Ala Thr Pro Val Phe Leu Asp Arg Glu Ala Thr Ile Asp Lys Ala
            20                  25                  30
Cys Glu Leu Ile Ala Thr Ala Gly His Glu Gly Ala Arg Leu Ile Val
        35                  40                  45
Phe Pro Glu Ala Phe Ile Pro Ser Tyr Pro Glu Trp Val Trp Gly Ile
    50                  55                  60

Pro Ser Gly Glu Gln Gly Leu Leu Asn Asp Leu Tyr Ala Glu Leu Leu
65                  70                  75                  80
Thr Asn Ser Val Thr Ile Pro Ser Asn Ala Thr Asp Arg Leu Cys Arg
                85                  90                  95
Ala Ala Lys Leu Ala Asn Ala Tyr Val Val Met Gly Met Ser Glu Arg
            100                 105                 110
Asn Ile Glu Ala Ser Gly Ala Ser Leu Tyr Asn Thr Met Leu Tyr Ile
        115                 120                 125
Asp Ala Gln Gly Glu Ile Leu Gly Lys His Arg Lys Leu Val Pro Thr
    130                 135                 140
Gly Gly Glu Arg Leu Val Trp Ala Gln Gly Asp Gly Ser Thr Leu Gln
145                 150                 155                 160
Val Tyr Asp Thr Pro Leu Gly Lys Leu Gly Gly Leu Ile Cys Trp Glu
                165                 170                 175
Asn Tyr Met Pro Leu Ala Arg Tyr Ala Met Tyr Ala Trp Gly Thr Gln
            180                 185                 190
Ile Tyr Val Ala Ala Thr Trp Asp Arg Gly Gln Pro Trp Leu Ser Thr
        195                 200                 205
Ile Arg His Ile Ala Lys Glu Gly Arg Val Tyr Val Ile Gly Cys Cys
    210                 215                 220
Ile Ala Met Arg Lys Asp Asp Ile Pro Asp Arg Tyr Ser Met Lys Gln
225                 230                 235                 240
Lys Tyr Tyr Ala Glu Met Asp Glu Trp Ile Asn Val Gly Asp Ser Ala
                245                 250                 255
Ile Val Asn Pro Glu Gly Asp Phe Ile Ala Gly Pro Val Ser Lys Gln
            260                 265                 270
Glu Glu Ile Leu Tyr Ala Glu Ile Asp Pro Arg Met Val Gln Gly Pro
        275                 280                 285
Lys Trp Met Leu Asp Val Ala Gly His Tyr Ala Arg Pro Asp Val Phe
    290                 295                 300
Glu Leu Thr Val His Thr Asp Val Arg Glu Met Met Arg Val Glu His
305                 310                 315                 320
Asp Tyr Gln

<210> 243
<211> 999
<212> DNA
<213> Unknown

<220>
<223> Obtained from an environmental sample

<400> 243
atgaagcaaa ctcgagtagc gatcattcag gcggaacctg tatatctcaa tttgcaagcg      60
agtgttgcga gggctatcga tcttgccgga cgcgccgcga agcaaggagc gcgtctgata     120
gtgtttggag agacctggtt gccgggttat cccgcgtggc tggattactg tcccggcatg     180
gcgttctggg atcaccggcc gacaaaagaa gtgtttgccc ggacccgcga gaacagtgtt     240
gtaattccgg gaaaggaaat cgaacagctc tgtaaaactg cggcggagct gggagttgta     300
atttcgatcg gtgtaaacga aaaaattctg gaaggccccg gaaacggcac gctctacaat     360
tctcttttgc tgattgatga atcaggaaaa ctggccggcc atcaccgcaa actggttccg     420
acttatacgg aacggatggt gtggggaatg ggtgatggag gtggaatgga agccatatcg     480
actgcagccg gcagggttgg cggattgatt tgctgggagc actggatgcc attgagccgg     540
caggtgctgc acatgtcggg tgaggaaatt catgtggcag tgtggcccac ggttcatgag     600
gtgcaccagc ttgcatcacg ccattatgca tttgaagggc gttgttttgt gctcgcagcc     660
ggattgttga tgaaggtccg ggatattcct ccggagctgg aattgccttc tcagatgtcg     720
cgtgaatccg aagactggct tctgcgcggc gggagcgccg tcattggtcc ggatggaaag     780
tacattgtgg agccgttgtt tgatcgagag gcgattctca cagccgatct tgaattagcc     840
gcatgcgatc gtgaaaaaat gacgctggac gtaacgggac attattcccg ccccgatctt     900
tttcacctgg aattcaggaa acagcaatcc ggccatattg cgggagcagg aacgatcagc     960
cggcaaaaat cagcgccgga ccgcgcggac gatcactaa                            999

<210> 244
<211> 332
<212> PRT
<213> Unknown

<220>

<223> Obtained from an environmental sample

<400> 244
Met Lys Gln Thr Arg Val Ala Ile Ile Gln Ala Glu Pro Val Tyr Leu
1               5                   10                  15
Asn Leu Gln Ala Ser Val Ala Arg Ala Ile Asp Leu Ala Gly Arg Ala
            20                  25                  30
Ala Lys Gln Gly Ala Arg Leu Ile Val Phe Gly Glu Thr Trp Leu Pro
        35                  40                  45
Gly Tyr Pro Ala Trp Leu Asp Tyr Cys Pro Gly Met Ala Phe Trp Asp
    50                  55                  60
His Arg Pro Thr Lys Glu Val Phe Ala Arg Thr Arg Glu Asn Ser Val
65                  70                  75                  80
Val Ile Pro Gly Lys Glu Ile Glu Gln Leu Cys Lys Thr Ala Ala Glu
                85                  90                  95
Leu Gly Val Val Ile Ser Ile Gly Val Asn Glu Lys Ile Leu Glu Gly
            100                 105                 110
Pro Gly Asn Gly Thr Leu Tyr Asn Ser Leu Leu Leu Ile Asp Glu Ser
        115                 120                 125
Gly Lys Leu Ala Gly His His Arg Lys Leu Val Pro Thr Tyr Thr Glu
    130                 135                 140
Arg Met Val Trp Gly Met Gly Asp Gly Gly Gly Met Glu Ala Ile Ser
145                 150                 155                 160
Thr Ala Ala Gly Arg Val Gly Gly Leu Ile Cys Trp Glu His Trp Met
                165                 170                 175
Pro Leu Ser Arg Gln Val Leu His Met Ser Gly Glu Glu Ile His Val
            180                 185                 190
Ala Val Trp Pro Thr Val His Glu Val His Gln Leu Ala Ser Arg His
        195                 200                 205
Tyr Ala Phe Glu Gly Arg Cys Phe Val Leu Ala Ala Gly Leu Leu Met
    210                 215                 220
Lys Val Arg Asp Ile Pro Pro Glu Leu Glu Leu Pro Ser Gln Met Ser
225                 230                 235                 240
Arg Glu Ser Glu Asp Trp Leu Leu Arg Gly Gly Ser Ala Val Ile Gly
                245                 250                 255
Pro Asp Gly Lys Tyr Ile Val Glu Pro Leu Phe Asp Arg Glu Ala Ile
            260                 265                 270
Leu Thr Ala Asp Leu Glu Leu Ala Ala Cys Asp Arg Glu Lys Met Thr
        275                 280                 285
Leu Asp Val Thr Gly His Tyr Ser Arg Pro Asp Leu Phe His Leu Glu
    290                 295                 300
Phe Arg Lys Gln Gln Ser Gly His Ile Ala Gly Ala Gly Thr Ile Ser
305                 310                 315                 320
Arg Gln Lys Ser Ala Pro Asp Arg Ala Asp Asp His
                325                 330

<210> 245
<211> 999
<212> DNA
<213> Unknown

<220>
<223> Obtained from an environmental sample

<400> 245
   atgggtgaga atgccaactt caccgtcgca gctgtccagg caacgccggt cttcttagac      60
   cgggatgcga cggtcgagaa ggcttgcgag ctcatcgctg aagccgggcg aaacggagcg     120
   cgcctggccg tttttcccga ggcgtttgtg ccggcttacc cggactgggt ctgggctgtt     180
   ccgccgggcg attcaaggct gctgcacgag ctctacggtg agctgatcca gaactctgtc     240
   acgattccca gcgagtcgac ggagaaactc tgccgggccg cccgcggggc caaagtctgc     300
   gtggcgatcg gcatcaacga gaggaatgcg gaggcaagcg ggggtagcct ctacaacagc      360
   ctcctgtaca tcagcccgga cggccaggtc ctcgggaagc accgcaagct cgttcccacc     420
   ggagcggagc ggcttgtctg ggcgcagggc gacggcagca ctatcgacgt gtttgagttg     480
   cctttctgtc gtttgggtgg cctcatctgt tgggagaact acatgccgct ggcccgttat     540
   gcgatgtacg cctggggcac gcaggtctac gtcgcggcaa cgtgggacca cggcgaacct     600
   tggctctcaa ccttgaggca tatcgccagg gaggggcgtg catatgtcat tggcgtttgc     660
   atgccgatgc gcatgagcga catcccggac cgatacgagt tcaagcgcaa gtactatggc     720
   gggcgcgact ggatcaatac tggtgacagc gccatcgtgg gtccggacgg aaacttcatc     780
   gccggccccc tgagcgagcg cgaagagatc ctgtacgccg atatagacct gaatcggctt 840
   gcgaactcga agtggatgct ggacgtcgcc gggcactatg cacggccgga cgtcttccag 900
   ttgaccgtta accgcgagcc gaacccgatg atctctgagg atgggcacaa gacggttccc 960
   acgctaccga aacgtgcggg gaagagtagg acgagatga 999

<210> 246
<211> 332
<212> PRT
<213> Unknown

<220>
<223> Obtained from an environmental sample

<400> 246
Met Gly Glu Asn Ala Asn Phe Thr Val Ala Ala Val Gln Ala Thr Pro
1               5                   10                  15
Val Phe Leu Asp Arg Asp Ala Thr Val Glu Lys Ala Cys Glu Leu Ile
            20                  25                  30
Ala Glu Ala Gly Arg Asn Gly Ala Arg Leu Ala Val Phe Pro Glu Ala
        35                  40                  45
Phe Val Pro Ala Tyr Pro Asp Trp Val Trp Ala Val Pro Pro Gly Asp
    50                  55                  60
Ser Arg Leu Leu His Glu Leu Tyr Gly Glu Leu Ile Gln Asn Ser Val
65                  70                  75                  80
Thr Ile Pro Ser Glu Ser Thr Glu Lys Leu Cys Arg Ala Ala Arg Gly
                85                  90                  95
Ala Lys Val Cys Val Ala Ile Gly Ile Asn Glu Arg Asn Ala Glu Ala
            100                 105                 110
Ser Gly Gly Ser Leu Tyr Asn Ser Leu Leu Tyr Ile Ser Pro Asp Gly
        115                 120                 125
Gln Val Leu Gly Lys His Arg Lys Leu Val Pro Thr Gly Ala Glu Arg
    130                 135                 140
Leu Val Trp Ala Gln Gly Asp Gly Ser Thr Ile Asp Val Phe Glu Leu
145                 150                 155                 160
Pro Phe Cys Arg Leu Gly Gly Leu Ile Cys Trp Glu Asn Tyr Met Pro
                165                 170                 175
Leu Ala Arg Tyr Ala Met Tyr Ala Trp Gly Thr Gln Val Tyr Val Ala
            180                 185                 190
Ala Thr Trp Asp His Gly Glu Pro Trp Leu Ser Thr Leu Arg His Ile
        195                 200                 205
Ala Arg Glu Gly Arg Ala Tyr Val Ile Gly Val Cys Met Pro Met Arg
    210                 215                 220
Met Ser Asp Ile Pro Asp Arg Tyr Glu Phe Lys Arg Lys Tyr Tyr Gly
225                 230                 235                 240
Gly Arg Asp Trp Ile Asn Thr Gly Asp Ser Ala Ile Val Gly Pro Asp
                245                 250                 255
Gly Asn Phe Ile Ala Gly Pro Leu Ser Glu Arg Glu Glu Ile Leu Tyr
            260                 265                 270
Ala Asp Ile Asp Leu Asn Arg Leu Ala Asn Ser Lys Trp Met Leu Asp
        275                 280                 285
Val Ala Gly His Tyr Ala Arg Pro Asp Val Phe Gln Leu Thr Val Asn
    290                 295                 300
Arg Glu Pro Asn Pro Met Ile Ser Glu Asp Gly His Lys Thr Val Pro
305                 310                 315                 320
Thr Leu Pro Lys Arg Ala Gly Lys Ser Arg Thr Arg
                325                 330

<210> 247
<211> 990
<212> DNA
<213> Unknown

<220>
<223> Obtained from an environmental sample

<400> 247
atgccgaccc ccaaggaaaa gtttagaatc gccgccgttc aggcttgccc cgtttttctg 60
gaccgggggg agacggtaaa gaaggcctgc cggctggcgg ccgaggccgg gggccagggc 120

gcccggctga tcgtgtttcc ggagtccttc atccccgctt acccggactg ggtgtgggcc 180
gttccgccgg ggagggagaa gctcctgaat gaaatgtacg ccgaattcct ggccggcgcg 240
gtggaagtcc cggggccggt gacggaggaa ttgggccggg cggcggaaag ggccggcgct 300
tacctggtca tgggggtcac cgagcgggac accgaggcca gcggggcaag cctgtacaac 360
accctcctct atttcggtcc ccagggaagc ctgctgggaa aacaccgcaa actggtgccc 420
acgggagggg aacggaccgt ctgggcccgg ggggacggca gcacgctgca ggtgtacgat 480
acccccctgg gaaagatcgg cggcctgatc tgctgggaga actacatgcc cctggcccgc 540
tacgccatgt atgcgtgggg cactcagatt tacctggccc ccacctggga ccggggggaa 600
ccctggcttt caaccctgcg gcacatcgcc aaggaagggc gggtttacgt ggtggggtgc 660
tgcatggcta tgcaaaaggg ggacatcccg gatcgcttcg aatacaagca aaaatactat 720
cccgcagccc gggagtggat caacacgggc gacagcgcca tcctgaaccc ggagggggaa 780
ttcatcgccg ggccggcggg aaagaaggaa gagatcctgt acgctgaaat agacccccgg 840
cagatggggg ggcccaagtg gatgctggac gtggccggcc attacgcccg gccggatgtc 900
ttcgaactga tcgttcaccg ggaggcccgg ccgatgatcc gggtgacgga agcgccgtct 960
ccgggagaga aagaaacggg tgaaggatag 990

<210> 248
<211> 329
<212> PRT
<213> Unknown

<220>
<223> Obtained from an environmental sample

<400> 248
Met Pro Thr Pro Lys Glu Lys Phe Arg Ile Ala Ala Val Gln Ala Cys
1               5                   10                  15
Pro Val Phe Leu Asp Arg Gly Glu Thr Val Lys Lys Ala Cys Arg Leu
            20                  25                  30
Ala Ala Glu Ala Gly Gly Gln Gly Ala Arg Leu Ile Val Phe Pro Glu
        35                  40                  45
Ser Phe Ile Pro Ala Tyr Pro Asp Trp Val Trp Ala Val Pro Pro Gly
    50                  55                  60
Arg Glu Lys Leu Leu Asn Glu Met Tyr Ala Glu Phe Leu Ala Gly Ala
65                  70                  75                  80
Val Glu Val Pro Gly Pro Val Thr Glu Glu Leu Gly Arg Ala Ala Glu
                85                  90                  95
Arg Ala Gly Ala Tyr Leu Val Met Gly Val Thr Glu Arg Asp Thr Glu
            100                 105                 110
Ala Ser Gly Ala Ser Leu Tyr Asn Thr Leu Leu Tyr Phe Gly Pro Gln
        115                 120                 125
Gly Ser Leu Leu Gly Lys His Arg Lys Leu Val Pro Thr Gly Gly Glu
    130                 135                 140
Arg Thr Val Trp Ala Arg Gly Asp Gly Ser Thr Leu Gln Val Tyr Asp
145                 150                 155                 160
Thr Pro Leu Gly Lys Ile Gly Gly Leu Ile Cys Trp Glu Asn Tyr Met
                165                 170                 175
Pro Leu Ala Arg Tyr Ala Met Tyr Ala Trp Gly Thr Gln Ile Tyr Leu
            180                 185                 190
Ala Pro Thr Trp Asp Arg Gly Glu Pro Trp Leu Ser Thr Leu Arg His
        195                 200                 205
Ile Ala Lys Glu Gly Arg Val Tyr Val Val Gly Cys Cys Met Ala Met
    210                 215                 220
Gln Lys Gly Asp Ile Pro Asp Arg Phe Glu Tyr Lys Gln Lys Tyr Tyr
225                 230                 235                 240
Pro Ala Ala Arg Glu Trp Ile Asn Thr Gly Asp Ser Ala Ile Leu Asn
                245                 250                 255
Pro Glu Gly Glu Phe Ile Ala Gly Pro Ala Gly Lys Lys Glu Glu Ile
            260                 265                 270
Leu Tyr Ala Glu Ile Asp Pro Arg Gln Met Gly Gly Pro Lys Trp Met
        275                 280                 285
Leu Asp Val Ala Gly His Tyr Ala Arg Pro Asp Val Phe Glu Leu Ile
    290                 295                 300
Val His Arg Glu Ala Arg Pro Met Ile Arg Val Thr Glu Ala Pro Ser
305                 310                 315                 320
Pro Gly Glu Lys Glu Thr Gly Glu Gly
                325

<210> 249
<211> 1017
<212> DNA
<213> Unknown

<220>
<223> Obtained from an environmental sample

<400> 249
atgggcattc aacatccgaa atatcgcgtc gcggtggtgc aggcggcacc ggcctggctc 60
gacctcgagg cgtcggtcag caagagcatc gcgctgatag aggaggccgc cgccaagggc 120
gccaagctga tcgcgttccc cgaggccttc atccccggct atccctggta catctggctg 180
gactcgccgg cctgggcgat cggccgcggc ttcgtgcagc gctatttcga caattcgctc 240
agctatgaca gcccgcaggc ggagcgcctg aggctcgcag tgaagaaggc cggcatgacc 300
gcagtgctcg gcctgtccga gcgcgacggc ggcagcctct atctcgcgca atggttgatc 360
ggacccgacg gcgagaccat cgcaaagcgg cgcaagctgc ggccgaccca tgccgagcgc 420
accgtctacg gcgagggcga cggcagcgac cttgcggtgc atgaccgccc cggcatcggc 480
cggctcggtg cgctgtgctg ctgggagcat ctgcagccgc tgtcgaaata cgcgatgtac 540
gcccagaacg agcaggtgca tgtcgcggcc tggccgagct tctcgctgta cgatccgttc 600
gcgccggcgc tcggctggga ggtcaacaat gcggcctcgc gcgtctatgc cgtcgagggc 660
tcctgcttcg tgctggcgcc ctgcgccacc gtctcgcagg cgatgatcga cgagctctgc 720
gaccgcgacg acaagcatgc gctgctgcat gttggcggcg gccatgccgc gatcttcggc 780
cccgacggca gcgcgatcgc ggacaagctt ccgtccgacc aggagggcct cctgttcgcc 840
gacatcgatc tcggcgcgat cgggatcgcg aagaatgccg ctgatccggc cgggcactat 900
tcgcgcccgg acgtgacgcg gctgctgctc aacaagaagc cctcgaagcg cgtcgagcac 960
ttcgcgctgc cgctcgacac gctcgcgggc gaggagatcg acgcggccgc aagctaa 1017

<210> 250
<211> 338
<212> PRT
<213> Unknown

<220>
<223> Obtained from an environmental sample

<400> 250
Met Gly Ile Gln His Pro Lys Tyr Arg Val Ala Val Val Gln Ala Ala
1               5                   10                  15
Pro Ala Trp Leu Asp Leu Glu Ala Ser Val Ser Lys Ser Ile Ala Leu
            20                  25                  30
Ile Glu Glu Ala Ala Ala Lys Gly Ala Lys Leu Ile Ala Phe Pro Glu
        35                  40                  45
Ala Phe Ile Pro Gly Tyr Pro Trp Tyr Ile Trp Leu Asp Ser Pro Ala
    50                  55                  60
Trp Ala Ile Gly Arg Gly Phe Val Gln Arg Tyr Phe Asp Asn Ser Leu
65                  70                  75                  80
Ser Tyr Asp Ser Pro Gln Ala Glu Arg Leu Arg Leu Ala Val Lys Lys
                85                  90                  95
Ala Gly Met Thr Ala Val Leu Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asp Arg Pro Gly Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Pro Ala Leu Gly Trp Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Asp Asp Lys His Ala Leu Leu His Val Gly Gly Gly His Ala
                245                 250                 255

Ala Ile Phe Gly Pro Asp Gly Ser Ala Ile Ala Asp Lys Leu Pro Ser
            260                 265                 270
Asp Gln Glu Gly Leu Leu Phe Ala Asp Ile Asp Leu Gly Ala Ile Gly
        275                 280                 285
Ile Ala Lys Asn Ala Ala Asp Pro Ala Gly His Tyr Ser Arg Pro Asp
    290                 295                 300
Val Thr Arg Leu Leu Leu Asn Lys Lys Pro Ser Lys Arg Val Glu His
305                 310                 315                 320
Phe Ala Leu Pro Leu Asp Thr Leu Ala Gly Glu Glu Ile Asp Ala Ala
                325                 330                 335
Ala Ser

<210> 251
<211> 978
<212> DNA
<213> Unknown

<220>
<223> Obtained from an environmental sample

<400> 251
gtgaccatcg tgagagctgc cgccgtgcag atcagtcccg tgctctacag ccgggaagcc      60
accgtagaaa aagtcgttcg caagatccgc gaactgggaa gaaacggcgt gcagttcgcc     120
accttcccgg aaaccctggt gccctactac ccgtacttcg cggccgtgca gacgggcatc     180
gaactgctgt ccggcaagga gcacctgcga ctgctggaac aatccgtaac ggttccctcg     240
cccgccaccg atgccattgc ccaggcggca cgcgaagccg gcatggtggt gtccatcggt     300
gtcaacgagc gcgacggagg caccatctac aacacgcagc tgctgtttga cgccgacggc     360
acgctggtac agcgccgccg caagatcacg ccgacgcatt tcgagcgcat ggtctggggc     420
cagggcgacg gctcgggcct gcgagccgtg gacaccaagg ccggccgcat cggtcagctc     480
gcctgcttcg agcacaacaa cccgctggcg cgctacgcca tgatcgccga cggtgagcag     540
atccattcgg ccatgtaccc gggctctgcc ttcggcgagg gcttcgcgca gcgcatggaa     600
atcaacatac gccagcacgc cctggagtct ggctgcttcg tggtgaatgc gaccgcgtgg     660
ctggatgccg accagcaggc gcagatcatg aaggatacgg gctgcggcat cggcccgatc     720
tccggcggct gcttcacgac catcgtcacg ccggacggca tgctgatcgg tgaacccctc     780
cgcgaaggcg aaggcgaagt catcgccgac ctcgatttca ccctgatcga ccggcgcaag     840
ctgctggtgg actcggtggg ccactacaac cgtccggagc tgctgagcct gctgatcgat     900
cgcacccctg cggcgaactt ccatgagcgc aatgcgcttc cgtccgtcaa caccgccagc     960
agcctcgaaa tcgtctga                                                  978

<210> 252
<211> 325
<212> PRT
<213> Unknown

<220>
<223> Obtained from an environmental sample

<400> 252
Val Thr Ile Val Arg Ala Ala Ala Val Gln Ile Ser Pro Val Leu Tyr
1               5                   10                  15
Ser Arg Glu Ala Thr Val Glu Lys Val Val Arg Lys Ile Arg Glu Leu
            20                  25                  30
Gly Arg Asn Gly Val Gln Phe Ala Thr Phe Pro Glu Thr Leu Val Pro
        35                  40                  45
Tyr Tyr Pro Tyr Phe Ala Ala Val Gln Thr Gly Ile Glu Leu Leu Ser
    50                  55                  60
Gly Lys Glu His Leu Arg Leu Leu Glu Gln Ser Val Thr Val Pro Ser
65                  70                  75                  80
Pro Ala Thr Asp Ala Ile Ala Gln Ala Ala Arg Glu Ala Gly Met Val
                85                  90                  95
Val Ser Ile Gly Val Asn Glu Arg Asp Gly Gly Thr Ile Tyr Asn Thr
            100                 105                 110
Gln Leu Leu Phe Asp Ala Asp Gly Thr Leu Val Gln Arg Arg Arg Lys
        115                 120                 125
Ile Thr Pro Thr His Phe Glu Arg Met Val Trp Gly Gln Gly Asp Gly
    130                 135                 140
Ser Gly Leu Arg Ala Val Asp Thr Lys Ala Gly Arg Ile Gly Gln Leu

145                 150                 155                 160
Ala Cys Phe Glu His Asn Asn Pro Leu Ala Arg Tyr Ala Met Ile Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu
        195                 200                 205
Glu Ser Gly Cys Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Ser Gly Gly Cys Phe Thr Thr Ile Val Thr Pro Asp Gly Met Leu Ile
                245                 250                 255
Gly Glu Pro Leu Arg Glu Gly Glu Gly Glu Val Ile Ala Asp Leu Asp
            260                 265                 270
Phe Thr Leu Ile Asp Arg Arg Lys Leu Leu Val Asp Ser Val Gly His
        275                 280                 285
Tyr Asn Arg Pro Glu Leu Leu Ser Leu Leu Ile Asp Arg Thr Pro Ala
    290                 295                 300
Ala Asn Phe His Glu Arg Asn Ala Leu Pro Ser Val Asn Thr Ala Ser
305                 310                 315                 320
Ser Leu Glu Ile Val
                325

<210> 253
<211> 924
<212> DNA
<213> Unknown

<220>
<223> Obtained from an environmental sample

<400> 253
atgtcaaacg agaaccacaa ccaaacattc aaagttgccg cggtgcaggc cacacctgta      60
ttcctcgatc gtgaagcgac catcgacaaa gcttgcgagt tgattgctgc agccggcaat     120
gaaggggcga ggctggttgt cttcccggag gcattcatcc cgtcctatcc agattgggta     180
tgggcaatcc caccgggcga agaaggcgtg ctcaatgagt tgtacgcgga actgctctcc     240
aattcggtca cgattcccag tgacgtgacg gatagactgt gccgggccgc gaggcttgcc     300
aatgcctacg tagtgatggg gatgagcgaa tgcaatgccg aggccagtgg cgcaagcctg     360
tataacacgc tattgtacat cgatgcgaag ggtgaaatcc tgggtaaaca tcgaaagttg     420
gtgccaactg gcggcgagcg actggtgtgg gcacagggcg atggcagcac gctgcaggtc     480
tacgatactc cactgggtaa actcggcggt ttaatttgct gggagaatta tatgccgctg     540
gcccgctaca ccatgtacgc ctggggcaca caaatctata tcgcagcgac atgggatcgc     600
gggcaaccct ggctctccac cttgcggcat atcgccaaag aaggcagggt gtacgtgatc     660
ggctgttgta tcgcgatgcg caaagatgat atcccagagc gttacccaat gaagcagaag     720
ttttacgcgg aggccgatga gtggatcaat ataggcgaca gcgcgatcgt caatcctgaa     780
gggcagttta tcgcggggcc ggtacgcaaa caggaagaga ttctctacgc ggagattaat     840
ccgcgcatgg tgcaaggccc gaagtggatg ctcgacgtgg cagggcacta cgccaggccg     900
gacgtattcc agttgacagt gtaa                                            924

<210> 254
<211> 307
<212> PRT
<213> Unknown

<220>
<223> Obtained from an environmental sample

<400> 254
Met Ser Asn Glu Asn His Asn Gln Thr Phe Lys Val Ala Ala Val Gln
1               5                   10                  15
Ala Thr Pro Val Phe Leu Asp Arg Glu Ala Thr Ile Asp Lys Ala Cys
            20                  25                  30
Glu Leu Ile Ala Ala Ala Gly Asn Glu Gly Ala Arg Leu Val Val Phe
        35                  40                  45
Pro Glu Ala Phe Ile Pro Ser Tyr Pro Asp Trp Val Trp Ala Ile Pro
    50                  55                  60
Pro Gly Glu Glu Gly Val Leu Asn Glu Leu Tyr Ala Glu Leu Leu Ser

65                  70                  75                  80
Asn Ser Val Thr Ile Pro Ser Asp Val Thr Asp Arg Leu Cys Arg Ala
                85                  90                  95
Ala Arg Leu Ala Asn Ala Tyr Val Val Met Gly Met Ser Glu Cys Asn
            100                 105                 110
Ala Glu Ala Ser Gly Ala Ser Leu Tyr Asn Thr Leu Leu Tyr Ile Asp
        115                 120                 125
Ala Lys Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Thr Met Tyr Ala Trp Gly Thr Gln Ile
            180                 185                 190
Tyr Ile Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Glu Arg Tyr Pro Met Lys Gln Lys
225                 230                 235                 240
Phe Tyr Ala Glu Ala Asp Glu Trp Ile Asn Ile Gly Asp Ser Ala Ile
                245                 250                 255
Val Asn Pro Glu Gly Gln Phe Ile Ala Gly Pro Val Arg Lys Gln Glu
            260                 265                 270
Glu Ile Leu Tyr Ala Glu Ile Asn Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val
305

<210> 255
<211> 1005
<212> DNA
<213> Unknown

<220>
<223> Obtained from an environmental sample

<400> 255
atgacaatgt ctaagaccaa atttagggtt gcagctgtgc aggcggcgcc ggttttcctt      60
gatcgggaag cgacgttgga taaagcttgt ggattgattg aggaggcggg ccgcaacggc     120
gccagcctcg tcgtcttccc tgagtcattc attccggcct accccgattg ggtttgggct     180
gtgccggcgg gcgaagaagc tttactcaat gaactgtacg cacaactgtt ggccaacgcc     240
gttgaaattc ccggcccggc cactcaacgt ttgagccagg cggctaaaaa ggctaaggtt     300
cacctggcta tgggcctgac cgaacgcaac agcgaggcca gcggcggcag cctttacaac     360
accttgctct atcttgaccc gcagggccac attctgggca agcatcgtaa gctggtgccc     420
accggcggtg agcggctggt ttgggcccag ggcgacggca gcactttgca agtttacgat     480
acgcctctgg gtaaactcag cggcctgatt tgctgggaaa attatatgcc gctggcgcgc     540
tacgcgctgt atgcctgggg tacgcaaatt tatattgcgg ccacctggga tcggggtgag     600
ccgtggcttt cgacgttgcg gcatattgcc aaagagggcc gggtgttggt catcggttgc     660
ggtatggcct tgcgcaaggc tgatattcct gatcattttg aattcaagca gcgcttttat     720
caaaacgccg ccgagtggat caacgggggc gacagcgcca ttgtcaaccc tgatggtgaa     780
tttattgctg gccccttaag cgagcaggaa ggcattttgt acgccgagat tgatccggcc     840
cagatgggcg ggccaaagtg gatgctcgac gtggccgggc attacgctcg cccggatgtg     900
tttgaactga cggtccatac cgccgcccga cccatgatca cctcgaaaaa ggatggccta     960
acacccgccg aggccgttac gcaagtaacg aaagcattat tgtaa                    1005

<210> 256
<211> 334
<212> PRT
<213> Unknown

<220>
<223> Obtained from an environmental sample

<400> 256

Met Thr Met Ser Lys Thr Lys Phe Arg Val Ala Ala Val Gln Ala Ala
1               5                   10                  15
Pro Val Phe Leu Asp Arg Glu Ala Thr Leu Asp Lys Ala Cys Gly Leu
            20                  25                  30
Ile Glu Glu Ala Gly Arg Asn Gly Ala Ser Leu Val Val Phe Pro Glu
        35                  40                  45
Ser Phe Ile Pro Ala Tyr Pro Asp Trp Val Trp Ala Val Pro Ala Gly
    50                  55                  60
Glu Glu Ala Leu Leu Asn Glu Leu Tyr Ala Gln Leu Leu Ala Asn Ala
65                  70                  75                  80
Val Glu Ile Pro Gly Pro Ala Thr Gln Arg Leu Ser Gln Ala Ala Lys
                85                  90                  95
Lys Ala Lys Val His Leu Ala Met Gly Leu Thr Glu Arg Asn Ser Glu
            100                 105                 110
Ala Ser Gly Gly Ser Leu Tyr Asn Thr Leu Leu Tyr Leu Asp Pro Gln
        115                 120                 125
Gly His Ile Leu Gly Lys His Arg Lys Leu Val Pro Thr Gly Gly Glu
    130                 135                 140
Arg Leu Val Trp Ala Gln Gly Asp Gly Ser Thr Leu Gln Val Tyr Asp
145                 150                 155                 160
Thr Pro Leu Gly Lys Leu Ser Gly Leu Ile Cys Trp Glu Asn Tyr Met
                165                 170                 175
Pro Leu Ala Arg Tyr Ala Leu Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Lys Glu Gly Arg Val Leu Val Ile Gly Cys Gly Met Ala Leu
    210                 215                 220
Arg Lys Ala Asp Ile Pro Asp His Phe Glu Phe Lys Gln Arg Phe Tyr
225                 230                 235                 240
Gln Asn Ala Ala Glu Trp Ile Asn Gly Gly Asp Ser Ala Ile Val Asn
                245                 250                 255
Pro Asp Gly Glu Phe Ile Ala Gly Pro Leu Ser Glu Gln Glu Gly Ile
            260                 265                 270
Leu Tyr Ala Glu Ile Asp Pro Ala Gln Met Gly Gly Pro Lys Trp Met
        275                 280                 285
Leu Asp Val Ala Gly His Tyr Ala Arg Pro Asp Val Phe Glu Leu Thr
    290                 295                 300
Val His Thr Ala Ala Arg Pro Met Ile Thr Ser Lys Lys Asp Gly Leu
305                 310                 315                 320
Thr Pro Ala Glu Ala Val Thr Gln Val Thr Lys Ala Leu Leu
                325                 330

<210> 257
<211> 942
<212> DNA
<213> Unknown

<220>
<223> Obtained from an environmental sample

<400> 257
atgaacaaga tcgcgatcat tcagcgccca cccgtgctac tcgaccgcat cgccacgctg      60
gcggttgcgg tggagtcgat cgacgaagct gccgcagccg gtgcctcact gatcgttctt     120
ccagaaacct tcatccccgg ctacccgtcc tggatctggc gtctcgcgcc ggggagggat     180
ggtgcgctca ttgcccagtt gcatgcccga ctgctcgcca acgcggtcga tcttgcggct     240
ggagatctgg atgccctgtg tgaagttgcg cacggccacc gggtgaccgt ggtgtgcggc     300
ctcaacgaat gcgagcgcag tcgcggcggg ggcactctct acaacacggt cgtcgtgatc     360
gaccccgacg gcaagctgtg caatcgccac cgcaagctga tgccgaccaa cccggaacgc     420
atggtgcacg gtctgggtga tgcatcgggc ctgcgcgccg tcgacacccc ggtgggtcga     480
gtgggcgcac tcatctgctg ggaaaactat atgccgctgg cacgctacgc actttacgcc     540
gagggggtgg aagtctacgt ggcgcccacc tatgacagcg gcgatggctg gatcagtacg     600
atgcgtcata ttgcgcttga gggacgctgc tgggtgctgg gtagcggaac cgtactgcgt     660
ggcagcgacg tcccagaaga ctttccgtca cacctggacc tgtttcccga cgcggaggaa     720
tggatcaatc cgggcgactc ggtggtcgtc gatcctcagg gcaagatcgt cgcaggcccg     780
atgcgacgtg agacaggcat tctctacgca gaaatcgacg ccgaacgggt cgcgccttcg     840
cgccgcacgc tcgatgtcgc cggacactac gcccgcccgg atattttcga gctccatgtc      900
cgacgtacgc cggcgatgcc ggtccacgcc gttgatgcat ga                        942

<210> 258
<211> 313
<212> PRT
<213> Unknown

<220>
<223> Obtained from an environmental sample

<400> 258
Met Asn Lys Ile Ala Ile Ile Gln Arg Pro Pro Val Leu Leu Asp Arg
1               5                   10                  15
Ile Ala Thr Leu Ala Val Ala Val Glu Ser Ile Asp Glu Ala Ala Ala
            20                  25                  30
Ala Gly Ala Ser Leu Ile Val Leu Pro Glu Thr Phe Ile Pro Gly Tyr
        35                  40                  45
Pro Ser Trp Ile Trp Arg Leu Ala Pro Gly Arg Asp Gly Ala Leu Ile
    50                  55                  60
Ala Gln Leu His Ala Arg Leu Leu Ala Asn Ala Val Asp Leu Ala Ala
65                  70                  75                  80
Gly Asp Leu Asp Ala Leu Cys Glu Val Ala His Gly His Arg Val Thr
                85                  90                  95
Val Val Cys Gly Leu Asn Glu Cys Glu Arg Ser Arg Gly Gly Gly Thr
            100                 105                 110
Leu Tyr Asn Thr Val Val Val Ile Asp Pro Asp Gly Lys Leu Cys Asn
        115                 120                 125
Arg His Arg Lys Leu Met Pro Thr Asn Pro Glu Arg Met Val His Gly
    130                 135                 140
Leu Gly Asp Ala Ser Gly Leu Arg Ala Val Asp Thr Pro Val Gly Arg
145                 150                 155                 160
Val Gly Ala Leu Ile Cys Trp Glu Asn Tyr Met Pro Leu Ala Arg Tyr
                165                 170                 175
Ala Leu Tyr Ala Glu Gly Val Glu Val Tyr Val Ala Pro Thr Tyr Asp
            180                 185                 190
Ser Gly Asp Gly Trp Ile Ser Thr Met Arg His Ile Ala Leu Glu Gly
        195                 200                 205
Arg Cys Trp Val Leu Gly Ser Gly Thr Val Leu Arg Gly Ser Asp Val
    210                 215                 220
Pro Glu Asp Phe Pro Ser His Leu Asp Leu Phe Pro Asp Ala Glu Glu
225                 230                 235                 240
Trp Ile Asn Pro Gly Asp Ser Val Val Val Asp Pro Gln Gly Lys Ile
                245                 250                 255
Val Ala Gly Pro Met Arg Arg Glu Thr Gly Ile Leu Tyr Ala Glu Ile
            260                 265                 270
Asp Ala Glu Arg Val Ala Pro Ser Arg Arg Thr Leu Asp Val Ala Gly
        275                 280                 285
His Tyr Ala Arg Pro Asp Ile Phe Glu Leu His Val Arg Arg Thr Pro
    290                 295                 300
Ala Met Pro Val His Ala Val Asp Ala
305                 310

<210> 259
<211> 981
<212> DNA
<213> Unknown

<220>
<223> Obtained from an environmental sample

<400> 259
atggccatca tcaaagccgc cgccgtccag atcagtccgg tgctgtatag ccgcgaggga      60
accgtggaca aggtctgcca gcagatcatc gccctcggtc agcaaggcgt gcaatttgcg     120
gtctttccgg aaacggtggt gccgtactac ccctatttct ccttcgtcca gccgccgttc     180
gccatgggca aggaacacct gaaactgttg gaacaatcgg tgattgtgcc gtcggctgcc     240
accttggcga tcggcgaagc gtgcaaacaa gcgggaatgg tggtgtctat cggcgtcaat     300
gagcgcgatg gcggcacgat ctacaacgcc cagttgctgt ttgacgctga tggcagcttg     360
attcagcacc gtcgcaaaat aaccccgacg taccacgagc ggatgatctg gggtcaaggc      420
gacggctccg ggttgcgcgc catcgacagc gcggtcgggc gtattggctc gctggcctgc     480

tgggaacatt acaacccctt ggcccgctac gccctgatgg ccgacggcga gcagattcac 540
gcggctatgt ttcccggctc tctggtgggt gacatttttg ccgatcagat agaggtcact 600
attcgtcatc acgccttgga gtccggctgc ttcgtggtca actccaccgc gtggcttgat 660
gctgatcagc aaggccaaat catgcaggac accggttgca gcattggccc aatctcgggt 720
ggctgcttca cggccatcgt ttccccggaa ggcaaattac tcggcgaacc gctgcgttca 780
ggtgagggcg cagtcatcgc cgacctggac atggcattga tcgacaagcg caaacggatg 840
atggattccg tcggccatta cagccgccca gaactgctca gtttattgat cgaccgcacg 900
cccaccgctc atgtgcatga gcgcggcgcc catcaccttg ccgtagcctc tatcggggag 960
cttgaccatg caaaccaatg a 981

<210> 260
<211> 326
<212> PRT
<213> Unknown

<220>
<223> Obtained from an environmental sample

<400> 260
Met Ala Ile Ile Lys Ala Ala Ala Val Gln Ile Ser Pro Val Leu Tyr
1               5                   10                  15
Ser Arg Glu Gly Thr Val Asp Lys Val Cys Gln Gln Ile Ile Ala Leu
            20                  25                  30
Gly Gln Gln Gly Val Gln Phe Ala Val Phe Pro Glu Thr Val Val Pro
        35                  40                  45
Tyr Tyr Pro Tyr Phe Ser Phe Val Gln Pro Pro Phe Ala Met Gly Lys
    50                  55                  60
Glu His Leu Lys Leu Leu Glu Gln Ser Val Ile Val Pro Ser Ala Ala
65                  70                  75                  80
Thr Leu Ala Ile Gly Glu Ala Cys Lys Gln Ala Gly Met Val Val Ser
                85                  90                  95
Ile Gly Val Asn Glu Arg Asp Gly Gly Thr Ile Tyr Asn Ala Gln Leu
            100                 105                 110
Leu Phe Asp Ala Asp Gly Ser Leu Ile Gln His Arg Arg Lys Ile Thr
        115                 120                 125
Pro Thr Tyr His Glu Arg Met Ile Trp Gly Gln Gly Asp Gly Ser Gly
    130                 135                 140
Leu Arg Ala Ile Asp Ser Ala Val Gly Arg Ile Gly Ser Leu Ala Cys
145                 150                 155                 160
Trp Glu His Tyr Asn Pro Leu Ala Arg Tyr Ala Leu Met Ala Asp Gly
                165                 170                 175
Glu Gln Ile His Ala Ala Met Phe Pro Gly Ser Leu Val Gly Asp Ile
            180                 185                 190
Phe Ala Asp Gln Ile Glu Val Thr Ile Arg His His Ala Leu Glu Ser
        195                 200                 205
Gly Cys Phe Val Val Asn Ser Thr Ala Trp Leu Asp Ala Asp Gln Gln
    210                 215                 220
Gly Gln Ile Met Gln Asp Thr Gly Cys Ser Ile Gly Pro Ile Ser Gly
225                 230                 235                 240
Gly Cys Phe Thr Ala Ile Val Ser Pro Glu Gly Lys Leu Leu Gly Glu
                245                 250                 255
Pro Leu Arg Ser Gly Glu Gly Ala Val Ile Ala Asp Leu Asp Met Ala
            260                 265                 270
Leu Ile Asp Lys Arg Lys Arg Met Met Asp Ser Val Gly His Tyr Ser
        275                 280                 285
Arg Pro Glu Leu Leu Ser Leu Leu Ile Asp Arg Thr Pro Thr Ala His
    290                 295                 300
Val His Glu Arg Gly Ala His His Leu Ala Val Ala Ser Ile Gly Glu
305                 310                 315                 320
Leu Asp His Ala Asn Gln
                325

<210> 261
<211> 1014
<212> DNA
<213> Unknown

<220>

<223> Obtained from an environmental sample

<400> 261
atgggtctgg ttcatcagaa atacaaggta gcggtggttc aggcggcacc ggtttttctc 60
gatctcgatg caaccgtgga caagaccatt gcgctgatcg aggaagcttc cgcgcaaggc 120
gcaaaactgg tcgcgtttcc cgagaccttc attcccggat atccgtggca gatctggctc 180
ggcgcgccgg cctgggcgat cggccgcggc tttgtgcagc gctacttcga caattcgctg 240
ggcttcgaca gcccgcaggc ggaaaaaatc cgccaggccg tgaagcgcgc caagctgacc 300
gcggtgcttg ggctatccga acgcgacggc ggcagcctct atatcgcgca gtggctgatc 360
ggccctgacg gcgagaccat cgccaagcgc cggaaactgc gtccgaccca tgccgaacgc 420
accgtgttcg gcgaaggcga cggcagcgat cttgcggtcc acgatcgcgc cgacgtcggg 480
cgcttgggcg cactgtgctg ctgggagcac ctgcagccgc tatcgaaata cgcgatgtac 540
gcccagaacg aacaggtgca tgtcggcgcc tggccgagct tttcgctgta cgatccgttc 600
gcacatgcgc tcggccatga ggtcaacaac gccgccagca aggtctatgc ggtcgaaggc 660
tcctgcttct tcctgggtcc ctgcgctgtc gtctcgcagg cgatgatcga cgagctctgc 720
gactctcccg agaaacatgc cttcctgcat gtcggcggcg gccacgcggt gatctacggc 780
ccggacggca gttcgctggc cgagaaactc ccgcccgacc aggaaggcat tctgtacgcc 840
gatatcgatc tcggcatgat cggggtcgcg aagaacgccg ccgatccggc cgggcattat 900
tcgcggcccg acgtcacgcg gctgttgctc aacaccacgc gcgccaaccg cgtcgagcat 960
ttctcgcttc ccgtcgatgc cgaggtcatg agcgaaatca ggctgcaggc ctga 1014

<210> 262
<211> 337
<212> PRT
<213> Unknown

<220>
<223> Obtained from an environmental sample

<400> 262
Met Gly Leu Val His Gln Lys Tyr Lys Val Ala Val Val Gln Ala Ala
1               5                   10                  15
Pro Val Phe Leu Asp Leu Asp Ala Thr Val Asp Lys Thr Ile Ala Leu
            20                  25                  30
Ile Glu Glu Ala Ser Ala Gln Gly Ala Lys Leu Val Ala Phe Pro Glu
        35                  40                  45
Thr Phe Ile Pro Gly Tyr Pro Trp Gln Ile Trp Leu Gly Ala Pro Ala
    50                  55                  60
Trp Ala Ile Gly Arg Gly Phe Val Gln Arg Tyr Phe Asp Asn Ser Leu
65                  70                  75                  80
Gly Phe Asp Ser Pro Gln Ala Glu Lys Ile Arg Gln Ala Val Lys Arg
                85                  90                  95
Ala Lys Leu Thr Ala Val Leu Gly Leu Ser Glu Arg Asp Gly Gly Ser
            100                 105                 110
Leu Tyr Ile Ala Gln Trp Leu Ile Gly Pro Asp Gly Glu Thr Ile Ala
        115                 120                 125
Lys Arg Arg Lys Leu Arg Pro Thr His Ala Glu Arg Thr Val Phe Gly
    130                 135                 140
Glu Gly Asp Gly Ser Asp Leu Ala Val His Asp Arg Ala Asp Val Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Gly Ala Trp Pro
            180                 185                 190
Ser Phe Ser Leu Tyr Asp Pro Phe Ala His Ala Leu Gly His Glu Val
        195                 200                 205
Asn Asn Ala Ala Ser Lys Val Tyr Ala Val Glu Gly Ser Cys Phe Phe
    210                 215                 220
Leu Gly Pro Cys Ala Val Val Ser Gln Ala Met Ile Asp Glu Leu Cys
225                 230                 235                 240
Asp Ser Pro Glu Lys His Ala Phe Leu His Val Gly Gly Gly His Ala
                245                 250                 255
Val Ile Tyr Gly Pro Asp Gly Ser Ser Leu Ala Glu Lys Leu Pro Pro
            260                 265                 270
Asp Gln Glu Gly Ile Leu Tyr Ala Asp Ile Asp Leu Gly Met Ile Gly
        275                 280                 285
Val Ala Lys Asn Ala Ala Asp Pro Ala Gly His Tyr Ser Arg Pro Asp
    290                 295                 300

Val Thr Arg Leu Leu Leu Asn Thr Thr Arg Ala Asn Arg Val Glu His
305                 310                 315                 320
Phe Ser Leu Pro Val Asp Ala Glu Val Met Ser Glu Ile Arg Leu Gln
                325                 330                 335
Ala

<210> 263
<211> 1014
<212> DNA
<213> Unknown

<220>
<223> Obtained from an environmental sample

<400> 263
atgaaagtcg tcaaagcggc agcggttcag ttgagccctg tcctctacag ccgcgaggca      60
accgtcgcga aggtcgtgcg gaagatccac gagcttggcc agcagggcgt gcagttcgcc     120
accttcccgg aaaccgttgt gccgtactac ccgtatttct ccgcggtcca gacgccgatg     180
cagcttctgg ctggaaccga gcatctgaaa ttgctcgacc aggccgtgac ggtgccgtct     240
cccgcgaccg acgcgatcgg cgaggcagcc cggaaggcgg gcatggtggt gtccatcggc     300
gtcaacgagc gtgatggtgg aaccctgtac aacacccaat tgctcttcga cgccgatggc     360
accctgatcc agcgccgccg caagatcacg cccacccatt tcgagcgcat gatctggggc     420
cagggtgacg ggtcgggcct gcgtgccgtc gacagcaagg tcggccgcat tggccagctg     480
gcatgcttcg agcacaacaa tcctctggcg cgctacgcga tgatggccga cggcgagcag     540
atccattcgg ccatgtatcc gggttctgcc ttcggcgagg gctttgccca gaggatggaa     600
atcaatatcc gccagcacgc actggagtcc gggtgcttcg tcgtgaacgc gacggcctgg     660
ctggatgccg accagcaggc gcaaatcatg aaagacacgg gctgcgggat cggtccgatc     720
tcgggcggtt gcttcaccac gatcgtggca cccgacggca cgctgctggg ggaacctctg     780
cgctcgggcg agggcgaggt catcgccgat ctcgatttca cggagatcga ccggcgcaag     840
atgctgatgg actcggcagg ccactacaac cgtccggaac tgctcagtct gctgatcgac     900
cgcacgccga ccgcaaacgt gcacgaacgg atggcgcatc cccaagcgag cacgaagcag     960
ccgcgctccg gcgatctgcc cgctgcgctg gctggcgcgc aggagatcct gtga          1014

<210> 264
<211> 337
<212> PRT
<213> Unknown

<220>
<223> Obtained from an environmental sample

<400> 264
Met Lys Val Val Lys Ala Ala Ala Val Gln Leu Ser Pro Val Leu Tyr
1               5                   10                  15
Ser Arg Glu Ala Thr Val Ala Lys Val Val Arg Lys Ile His Glu Leu
            20                  25                  30
Gly Gln Gln Gly Val Gln Phe Ala Thr Phe Pro Glu Thr Val Val Pro
        35                  40                  45
Tyr Tyr Pro Tyr Phe Ser Ala Val Gln Thr Pro Met Gln Leu Leu Ala
    50                  55                  60
Gly Thr Glu His Leu Lys Leu Leu Asp Gln Ala Val Thr Val Pro Ser
65                  70                  75                  80
Pro Ala Thr Asp Ala Ile Gly Glu Ala Ala Arg Lys Ala Gly Met Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Thr Leu Ile Gln Arg Arg Arg Lys
        115                 120                 125
Ile Thr Pro Thr His Phe Glu Arg Met Ile Trp Gly Gln Gly Asp Gly
    130                 135                 140
Ser Gly Leu Arg Ala Val Asp Ser Lys Val Gly Arg Ile Gly Gln Leu
145                 150                 155                 160
Ala Cys Phe Glu His Asn Asn Pro Leu Ala Arg Tyr Ala Met Met Ala
                165                 170                 175
Asp Gly Glu Gln Ile His Ser Ala Met Tyr Pro Gly Ser Ala Phe Gly
            180                 185                 190
Glu Gly Phe Ala Gln Arg Met Glu Ile Asn Ile Arg Gln His Ala Leu

195                 200                 205
Glu Ser Gly Cys Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Ser Gly Gly Cys Phe Thr Thr Ile Val Ala Pro Asp Gly Thr Leu Leu
                245                 250                 255
Gly Glu Pro Leu Arg Ser Gly Glu Gly Glu Val Ile Ala Asp Leu Asp
            260                 265                 270
Phe Thr Glu Ile Asp Arg Arg Lys Met Leu Met Asp Ser Ala Gly His
        275                 280                 285
Tyr Asn Arg Pro Glu Leu Leu Ser Leu Leu Ile Asp Arg Thr Pro Thr
    290                 295                 300
Ala Asn Val His Glu Arg Met Ala His Pro Gln Ala Ser Thr Lys Gln
305                 310                 315                 320
Pro Arg Ser Gly Asp Leu Pro Ala Ala Leu Ala Gly Ala Gln Glu Ile
                325                 330                 335
Leu

<210> 265
<211> 999
<212> DNA
<213> Unknown

<220>
<223> Obtained from an environmental sample

<400> 265
atgcttaatc taggcatagt ccagatgaac gcagagccgc tcaacgtgga aggcaacctg      60
ctcaaggcgg agcgctatgt cgcgaagtgc gccgcggacg gcgcccaact cgtggtgctg     120
ccggagatgt tcaacgtcgg cttccacctc ggcgagtccc tgatgatggt cgccgagccc     180
ctggacggca agaccgtgca gtggctgcaa cggcaggcgt ccacccataa catatatatc     240
accgggagct tatacgagcg ttacgacgag catttctaca acaccatggt catggtggga     300
tacgacggca gcgtgcagta ctaccgcaag cgcaatccta cctggtccga gtcggcggtg     360
tggcgccgca gcgaggtgcc aggccccggt atattcgata ccccgttcgg gcgcatcggg     420
ggcgtcatct gcttcgattc cttcgcgcgc gagacccacg agggcttcaa gcagagcggg     480
gtcgaggcgg tggtaatcat cgccctgtgg ggcgccaacc gtgcgcgggc attcttctgg     540
cgcccggacc tcctgctaag ccgggaaggg ctggtccgtt ggtcccggct ggcctcggag     600
gacgtccccc gaaatcacgc gaaagagctc ggggtcccgg tcgccttcgt caaccagagt     660
ggcaccatcc gcatgaccag ccccatccct ttccccgact ggccggtgca gagctccttc     720
tacgacttca tcggcaagtc ccacgtccgg gacgcatccg gagaggtgat cgcgagggtg     780
gacgaggggg agatcgactc ctgcctggtg gtcccggtag aggtcgagca ggcgcagagc     840
aggccggaga tcaggaagtc aaatatatcg cccggctacc tcggcaagga ttactatttc     900
gtggagccgc cgcttatctg caagctcttc caggtctggt tcctcagcgg cctggtgccc     960
accgaatacg aggcgcggcg tctgcgccac ctgttctga                            999

<210> 266
<211> 332
<212> PRT
<213> Unknown

<220>
<223> Obtained from an environmental sample

<400> 266
Met Leu Asn Leu Gly Ile Val Gln Met Asn Ala Glu Pro Leu Asn Val
1               5                   10                  15
Glu Gly Asn Leu Leu Lys Ala Glu Arg Tyr Val Ala Lys Cys Ala Ala
            20                  25                  30
Asp Gly Ala Gln Leu Val Val Leu Pro Glu Met Phe Asn Val Gly Phe
        35                  40                  45
His Leu Gly Glu Ser Leu Met Met Val Ala Glu Pro Leu Asp Gly Lys
    50                  55                  60
Thr Val Gln Trp Leu Gln Arg Gln Ala Ser Thr His Asn Ile Tyr Ile
65                  70                  75                  80
Thr Gly Ser Leu Tyr Glu Arg Tyr Asp Glu His Phe Tyr Asn Thr Met
                85                  90                  95

Val Met Val Gly Tyr Asp Gly Ser Val Gln Tyr Tyr Arg Lys Arg Asn
            100                 105                 110
Pro Thr Trp Ser Glu Ser Ala Val Trp Arg Arg Ser Glu Val Pro Gly
        115                 120                 125
Pro Gly Ile Phe Asp Thr Pro Phe Gly Arg Ile Gly Gly Val Ile Cys
    130                 135                 140
Phe Asp Ser Phe Ala Arg Glu Thr His Glu Gly Phe Lys Gln Ser Gly
145                 150                 155                 160
Val Glu Ala Val Val Ile Ile Ala Leu Trp Gly Ala Asn Arg Ala Arg
                165                 170                 175
Ala Phe Phe Trp Arg Pro Asp Leu Leu Leu Ser Arg Glu Gly Leu Val
            180                 185                 190
Arg Trp Ser Arg Leu Ala Ser Glu Asp Val Pro Arg Asn His Ala Lys
        195                 200                 205
Glu Leu Gly Val Pro Val Ala Phe Val Asn Gln Ser Gly Thr Ile Arg
    210                 215                 220
Met Thr Ser Pro Ile Pro Phe Pro Asp Trp Pro Val Gln Ser Ser Phe
225                 230                 235                 240
Tyr Asp Phe Ile Gly Lys Ser His Val Arg Asp Ala Ser Gly Glu Val
                245                 250                 255
Ile Ala Arg Val Asp Glu Gly Glu Ile Asp Ser Cys Leu Val Val Pro
            260                 265                 270
Val Glu Val Glu Gln Ala Gln Ser Arg Pro Glu Ile Arg Lys Ser Asn
        275                 280                 285
Ile Ser Pro Gly Tyr Leu Gly Lys Asp Tyr Tyr Phe Val Glu Pro Pro
    290                 295                 300
Leu Ile Cys Lys Leu Phe Gln Val Trp Phe Leu Ser Gly Leu Val Pro
305                 310                 315                 320
Thr Glu Tyr Glu Ala Arg Arg Leu Arg His Leu Phe
                325                 330

<210> 267
<211> 1038
<212> DNA
<213> Unknown

<220>
<223> Obtained from an environmental sample

<400> 267
atgggcatta cgcatccgaa gttcaaggcc gcggcggtac aggcggcgcc gggctttctc      60
gacagcgagg ccaccgtcga caagacgatc cgcctgatgc aggaagcggc ggaccacggc     120
gcctcgctga tcgtctttcc ggaagcctgg ctgcccggtt atccgtggtg gatctggctc     180
ggtccgcccg cctggggcat gcagttcgtg cagcgctact tcgacaattc gccgagcgtc     240
ggcgatgatc ttttccgccg gatcgagcgc gcggccgcca aggcgaagat cgaagtggtc     300
ctcggtctca gcgagcgcgc tgccggctcg ctgtacctcg cgcaggcgtt catctcctca     360
acgggcgaga cgcgcgcagt gcgccgcaag ttgcgaccaa cgcacgtcga gcgaaccgtt     420
ttcggcgagg gcgatggcag cgacttcaag gtgttcgaca ctccgctggg ccgcgtcggt     480
ggtctcttgt gctgggaaca cctgcaaccg ctgtcgcgct acgcgatgtt ctcgatgaac     540
gagcaggtgc acgccgccgc ctggccgacg ttcagcctct acacggattt tgcccatgcc     600
ctcggccacg aactgaatct cgcagccagc gctacttacg cggctgaagg gcagtgctac     660
gtgattgccg cctgtggcgt ggtcacgcag gagatgctgg atctgatgaa ggcgccgtgc     720
cccccggaat atctgcgggt cggcggcgga tacgccatga tctttgcgcc cgacggacgg     780
cgcattgcgg cggcgctgcc gccggaacaa gaagggctga tttacgccga catcgatctt     840
tcgatgatct ctctcgccaa ggcggctgcc gatcccaccg gtcactactc gcggccggat     900
gtcgtgcggc ttatgctgaa taccgaaccg atgcagcggg tcgaaaagct gcagccgccg     960
ctggactcag ccgcgcgccg tgagaatgaa ccggcacgcg agaccgcagc ggcgaccgag    1020
agccgccagc cccagtaa                                                  1038

<210> 268
<211> 344
<212> PRT
<213> Unknown

<220>
<223> Obtained from an environmental sample

<400> 268

Met Gly Ile Thr His Pro Lys Phe Lys Ala Ala Ala Val Gln Ala Ala
1               5                   10                  15
Pro Gly Phe Leu Asp Ser Glu Ala Thr Val Asp Lys Thr Ile Arg Leu
            20                  25                  30
Met Gln Glu Ala Ala Asp His Gly Ala Ser Leu Ile Val Phe Pro Glu
        35                  40                  45
Ala Trp Leu Pro Gly Tyr Pro Trp Trp Ile Trp Leu Gly Pro Pro Ala
    50                  55                  60
Trp Gly Met Gln Phe Val Gln Arg Tyr Phe Asp Asn Ser Pro Ser Val
65                  70                  75                  80
Gly Asp Asp Leu Phe Arg Arg Ile Glu Arg Ala Ala Ala Lys Ala Lys
                85                  90                  95
Ile Glu Val Val Leu Gly Leu Ser Glu Arg Ala Ala Gly Ser Leu Tyr
            100                 105                 110
Leu Ala Gln Ala Phe Ile Ser Ser Thr Gly Glu Thr Arg Ala Val Arg
        115                 120                 125
Arg Lys Leu Arg Pro Thr His Val Glu Arg Thr Val Phe Gly Glu Gly
    130                 135                 140
Asp Gly Ser Asp Phe Lys Val Phe Asp Thr Pro Leu Gly Arg Val Gly
145                 150                 155                 160
Gly Leu Leu Cys Trp Glu His Leu Gln Pro Leu Ser Arg Tyr Ala Met
                165                 170                 175
Phe Ser Met Asn Glu Gln Val His Ala Ala Ala Trp Pro Thr Phe Ser
            180                 185                 190
Leu Tyr Thr Asp Phe Ala His Ala Leu Gly His Glu Leu Asn Leu Ala
        195                 200                 205
Ala Ser Ala Thr Tyr Ala Ala Glu Gly Gln Cys Tyr Val Ile Ala Ala
    210                 215                 220
Cys Gly Val Val Thr Gln Glu Met Leu Asp Leu Met Lys Ala Pro Cys
225                 230                 235                 240
Pro Pro Glu Tyr Leu Arg Val Gly Gly Gly Tyr Ala Met Ile Phe Ala
                245                 250                 255
Pro Asp Gly Arg Arg Ile Ala Ala Ala Leu Pro Pro Glu Gln Glu Gly
            260                 265                 270
Leu Ile Tyr Ala Asp Ile Asp Leu Ser Met Ile Ser Leu Ala Lys Ala
        275                 280                 285
Ala Ala Asp Pro Thr Gly His Tyr Ser Arg Pro Asp Val Val Arg Leu
    290                 295                 300
Met Leu Asn Thr Glu Pro Met Gln Arg Val Glu Lys Leu Gln Pro Pro
305                 310                 315                 320
Leu Asp Ser Ala Ala Arg Arg Glu Asn Glu Pro Ala Arg Glu Thr Ala
                325                 330                 335
Ala Ala Thr Glu Ser Arg Gln Pro
            340

<210> 269
<211> 1014
<212> DNA
<213> Unknown

<220>
<223> Obtained from an environmental sample

<400> 269
atgtctgaaa cagccttcaa gatcgcggtc gtacaggcgg ctccggtttt tctcgacgca      60
aaggcgacgg tggacaaggc gatcggtctg atggccgaag ccggcgccaa gggcgccaag     120
ctgctcgcat tcccggaagt attcatcccc ggctaccctt ggtggctgtg gctgggcaca     180
ccggcatggg gcatgcagtt tgttgccaag tatcacgcga actcgcttcg tgcagacggg     240
cctgaattgg cagccctcgc ggcggcggcg gcgaagtccg atatcaatgc cgtcatcggc     300
ttctcggaga tcgacggcgg ttccctctac atcagccagg cgctcatcag cgacaagggc     360
gagataatgt tcaaacggcg caagctgaag ccgacgcacg tcgaacgcac gttgttcggc     420
gaaggggacg ggtccgactt ccaggtcgtg gacacgagcg tcggcaggct cggtgccttg     480
tgttgcgccg aacacataca gccgctgtcg aagtacgcga tgtactccat gcacgaacag     540
gtgcacgtcg cctcctggcc gtcatttact ttgtaccgcg gcacggcata tgccttgggc     600
cacgaggtca atctggccgc gagccagatt tatgcgctcg agggaggctg tttcgtcctt     660
catgcgagcg ccatcaccgg ccaggacatg tttgacgtgc tgtgcgacac tccggagagg     720
acgcaactgc tgaactccga cggcggcaag gtcggcggcg gctactcgat gatcttcggt     780
cccgatggcc agccccttgt tgggcatctg cctcaagaca ccgagggaat actctacgca     840

gatattgacc tggcgaacat ttccgttgcc aaagcggcct acgacccgtc cggacactat 900
gcgcgcggag acgtggtgcg cttgatggtc aatcgcaacc cgcggcatac gagtgttgcg 960
ttcggcgggg gcgccggcga ggcagcaacc tggacggaag caaaagcgga gtga 1014

<210> 270
<211> 337
<212> PRT
<213> Unknown

<220>
<223> Obtained from an environmental sample

<400> 270
Met Ser Glu Thr Ala Phe Lys Ile Ala Val Val Gln Ala Ala Pro Val
1               5                   10                  15
Phe Leu Asp Ala Lys Ala Thr Val Asp Lys Ala Ile Gly Leu Met Ala
            20                  25                  30
Glu Ala Gly Ala Lys Gly Ala Lys Leu Leu Ala Phe Pro Glu Val Phe
        35                  40                  45
Ile Pro Gly Tyr Pro Trp Trp Leu Trp Leu Gly Thr Pro Ala Trp Gly
    50                  55                  60
Met Gln Phe Val Ala Lys Tyr His Ala Asn Ser Leu Arg Ala Asp Gly
65                  70                  75                  80
Pro Glu Leu Ala Ala Leu Ala Ala Ala Ala Ala Lys Ser Asp Ile Asn
                85                  90                  95
Ala Val Ile Gly Phe Ser Glu Ile Asp Gly Gly Ser Leu Tyr Ile Ser
            100                 105                 110
Gln Ala Leu Ile Ser Asp Lys Gly Glu Ile Met Phe Lys Arg Arg Lys
        115                 120                 125
Leu Lys Pro Thr His Val Glu Arg Thr Leu Phe Gly Glu Gly Asp Gly
    130                 135                 140
Ser Asp Phe Gln Val Val Asp Thr Ser Val Gly Arg Leu Gly Ala Leu
145                 150                 155                 160
Cys Cys Ala Glu His Ile Gln Pro Leu Ser Lys Tyr Ala Met Tyr Ser
                165                 170                 175
Met His Glu Gln Val His Val Ala Ser Trp Pro Ser Phe Thr Leu Tyr
            180                 185                 190
Arg Gly Thr Ala Tyr Ala Leu Gly His Glu Val Asn Leu Ala Ala Ser
        195                 200                 205
Gln Ile Tyr Ala Leu Glu Gly Gly Cys Phe Val Leu His Ala Ser Ala
    210                 215                 220
Ile Thr Gly Gln Asp Met Phe Asp Val Leu Cys Asp Thr Pro Glu Arg
225                 230                 235                 240
Thr Gln Leu Leu Asn Ser Asp Gly Gly Lys Val Gly Gly Gly Tyr Ser
                245                 250                 255
Met Ile Phe Gly Pro Asp Gly Gln Pro Leu Val Gly His Leu Pro Gln
            260                 265                 270
Asp Thr Glu Gly Ile Leu Tyr Ala Asp Ile Asp Leu Ala Asn Ile Ser
        275                 280                 285
Val Ala Lys Ala Ala Tyr Asp Pro Ser Gly His Tyr Ala Arg Gly Asp
    290                 295                 300
Val Val Arg Leu Met Val Asn Arg Asn Pro Arg His Thr Ser Val Ala
305                 310                 315                 320
Phe Gly Gly Gly Ala Gly Glu Ala Ala Thr Trp Thr Glu Ala Lys Ala
                325                 330                 335
Glu

<210> 271
<211> 966
<212> DNA
<213> Unknown

<220>
<223> Obtained from an environmental sample

<400> 271
atgtccagcg agaataacaa cgctacattc aaagttgccg cagttcaggc cacacctgtg 60

tatcttgatc gtgaagcaac catcgacaag gcttgcgagt tgatcgctac tgctggcagc 120
gaaggagctc gcctgattat ctttccagaa gcgttcatcc caacctatcc tgagtgggta 180
tggggtattc cttctggtga gcaaggttta ctcaacgagc tctattcaga gttgctcacc 240
aattcggtca cgattcccag cgacgcgact gacagactgt gcgaggccgc gaagcttgct 300
aatgcctacg tggtgatggg aatgagtgaa cggaatgtcg aagcgagtgg tgcaagcctg 360
tataatacgc tcttgtacat agatgcgcag ggggagattt tagggaaaca tcgaaagttg 420
gtaccaacgg gcggtgagcg cctggtatgg gcgcaaggtg atggcagcac gctgcaggtc 480
tacgatactc cattgggaaa actcggtggt ttaatttgct gggaaaatta tatgccactg 540
gcacgctacg ctatgtatgc ctggggtaca caaatctatg tcgcagcaac gtgggatcgc 600
ggccaaccct ggctctcaac gttacggcat attgccaaag aaggcagggt atacgtaatt 660
ggttgctgta ttgcgatgcg taaagatgat attccagatc gttactccat gaagcagaag 720
tattacgctg aaatggagga atggattaat attggtgaca gcgcgattgt caatcccgaa 780
ggacacttta ttgcagggcc tgtgcgcaag caagaagaaa ttctttacgc ggagatcgat 840
ccacgcatgg tgcaaggccc gaagtggatg ctcgatgtgg ctgggcacta tgcgagacca 900
gatgtgttcc agttgacggt gcatacggat gtgaggcaga tgattcgggt ggaacatgat 960
tcataa 966

<210> 272
<211> 321
<212> PRT
<213> Unknown

<220>
<223> Obtained from an environmental sample

<400> 272
Met Ser Ser Glu Asn Asn Asn Ala Thr Phe Lys Val Ala Ala Val Gln
1               5                   10                  15
Ala Thr Pro Val Tyr Leu Asp Arg Glu Ala Thr Ile Asp Lys Ala Cys
            20                  25                  30
Glu Leu Ile Ala Thr Ala Gly Ser Glu Gly Ala Arg Leu Ile Ile Phe
        35                  40                  45
Pro Glu Ala Phe Ile Pro Thr Tyr Pro Glu Trp Val Trp Gly Ile Pro
    50                  55                  60
Ser Gly Glu Gln Gly Leu Leu Asn Glu Leu Tyr Ser Glu Leu Leu Thr
65                  70                  75                  80
Asn Ser Val Thr Ile Pro Ser Asp Ala Thr Asp Arg Leu Cys Glu Ala
                85                  90                  95
Ala Lys Leu Ala Asn Ala Tyr Val Val Met Gly Met Ser Glu Arg Asn
            100                 105                 110
Val Glu Ala Ser Gly Ala Ser Leu Tyr Asn Thr Leu Leu Tyr Ile Asp
        115                 120                 125
Ala Gln Gly Glu Ile Leu Gly Lys His Arg Lys Leu Val Pro Thr Gly
    130                 135                 140
Gly Glu Arg Leu Val Trp Ala Gln Gly Asp Gly Ser Thr Leu Gln Val
145                 150                 155                 160
Tyr Asp Thr Pro Leu Gly Lys Leu Gly Gly Leu Ile Cys Trp Glu Asn
                165                 170                 175
Tyr Met Pro Leu Ala Arg Tyr Ala Met Tyr Ala Trp Gly Thr Gln Ile
            180                 185                 190
Tyr Val Ala Ala Thr Trp Asp Arg Gly Gln Pro Trp Leu Ser Thr Leu
        195                 200                 205
Arg His Ile Ala Lys Glu Gly Arg Val Tyr Val Ile Gly Cys Cys Ile
    210                 215                 220
Ala Met Arg Lys Asp Asp Ile Pro Asp Arg Tyr Ser Met Lys Gln Lys
225                 230                 235                 240
Tyr Tyr Ala Glu Met Glu Glu Trp Ile Asn Ile Gly Asp Ser Ala Ile
                245                 250                 255
Val Asn Pro Glu Gly His Phe Ile Ala Gly Pro Val Arg Lys Gln Glu
            260                 265                 270
Glu Ile Leu Tyr Ala Glu Ile Asp Pro Arg Met Val Gln Gly Pro Lys
        275                 280                 285
Trp Met Leu Asp Val Ala Gly His Tyr Ala Arg Pro Asp Val Phe Gln
    290                 295                 300
Leu Thr Val His Thr Asp Val Arg Gln Met Ile Arg Val Glu His Asp
305                 310                 315                 320
Ser

<210> 273
<211> 1023
<212> DNA
<213> Unknown

<220>
<223> Obtained from an environmental sample

<400> 273
atgaataact caccaccaac catccgcgct gctgccattc agctcagtcc agttctgttt 60
agtcgggatg ggactaccga gaaggtgttg caggcgatcg ctagtgctgc caaggaaggg 120
gcacaactgg ttgtttttcc agaaactttc atcccctact acccctattt ctcattcatt 180
caaccccctg tgttgatggg caaagagcac atgcggctct atgaggaagc cgtgacggtc 240
ccgggtccgg tgacagatgc ggtcagtcga gcagcccgtt cttacggcat ggtggtagtg 300
ctgggggtga atgagcgaga tggtggctca atttacaata cacagttgat tttcgatgct 360
gacggcacat tgttgctgaa gcgacgcaaa atcaccccta cctatcatga gcgcatggtc 420
tgggggcagg gagacggtgc tggattgaag gtattggata cagcagtcgg taaggtgggt 480
gcgctggcat gttgggaaca ttacaatccc ctggcacgat ttgcgctgat ggcacagcat 540
gagcagattc actgcgctca gttccccggt tctctggtgg gacaaatttt cactgatcag 600
attgaggtaa cgattcggca tcatgcgttg gaatcgggtt gttttgtggt gaatgctact 660
ggctggctct ctccagaaca ggtggcacaa atcaccacgg atgaaaagtt gcaacgggtg 720
ctgagtggcg ggtgtaatac cgccattatt ggacctgaag gcaatcatct ctgtcctccc 780
attaccgatg gtgagggcat agcgatcgcc gatctcgact tctcactaat caccaaacgc 840
aaacgcatga tggattgcgt cggtcactac tcccgccctg acttgttgaa gctgcaactc 900
aatgcaacgg catggtcggt gctggctggg gagcaggggg caggtgccag ggagcagggg 960
ctaggtgtgc cggatgccat gctgtctacg cctaagccag aatactcaac actggatcag 1020
tag 1023

<210> 274
<211> 340
<212> PRT
<213> Unknown

<220>
<223> Obtained from an environmental sample

<400> 274
Met Asn Asn Ser Pro Pro Thr Ile Arg Ala Ala Ala Ile Gln Leu Ser
1               5                   10                  15
Pro Val Leu Phe Ser Arg Asp Gly Thr Thr Glu Lys Val Leu Gln Ala
            20                  25                  30
Ile Ala Ser Ala Ala Lys Glu Gly Ala Gln Leu Val Val Phe Pro Glu
        35                  40                  45
Thr Phe Ile Pro Tyr Tyr Pro Tyr Phe Ser Phe Ile Gln Pro Pro Val
    50                  55                  60
Leu Met Gly Lys Glu His Met Arg Leu Tyr Glu Glu Ala Val Thr Val
65                  70                  75                  80
Pro Gly Pro Val Thr Asp Ala Val Ser Arg Ala Ala Arg Ser Tyr Gly
                85                  90                  95
Met Val Val Val Leu Gly Val Asn Glu Arg Asp Gly Gly Ser Ile Tyr
            100                 105                 110
Asn Thr Gln Leu Ile Phe Asp Ala Asp Gly Thr Leu Leu Leu Lys Arg
        115                 120                 125
Arg Lys Ile Thr Pro Thr Tyr His Glu Arg Met Val Trp Gly Gln Gly
    130                 135                 140
Asp Gly Ala Gly Leu Lys Val Leu Asp Thr Ala Val Gly Lys Val Gly
145                 150                 155                 160
Ala Leu Ala Cys Trp Glu His Tyr Asn Pro Leu Ala Arg Phe Ala Leu
                165                 170                 175
Met Ala Gln His Glu Gln Ile His Cys Ala Gln Phe Pro Gly Ser Leu
            180                 185                 190
Val Gly Gln Ile Phe Thr Asp Gln Ile Glu Val Thr Ile Arg His His
        195                 200                 205
Ala Leu Glu Ser Gly Cys Phe Val Val Asn Ala Thr Gly Trp Leu Ser
    210                 215                 220
Pro Glu Gln Val Ala Gln Ile Thr Thr Asp Glu Lys Leu Gln Arg Val
225                 230                 235                 240

Leu Ser Gly Gly Cys Asn Thr Ala Ile Ile Gly Pro Glu Gly Asn His
                245                 250                 255
Leu Cys Pro Pro Ile Thr Asp Gly Glu Gly Ile Ala Ile Ala Asp Leu
            260                 265                 270
Asp Phe Ser Leu Ile Thr Lys Arg Lys Arg Met Met Asp Cys Val Gly
        275                 280                 285
His Tyr Ser Arg Pro Asp Leu Leu Lys Leu Gln Leu Asn Ala Thr Ala
    290                 295                 300
Trp Ser Val Leu Ala Gly Glu Gln Gly Ala Gly Ala Arg Glu Gln Gly
305                 310                 315                 320
Leu Gly Val Pro Asp Ala Met Leu Ser Thr Pro Lys Pro Glu Tyr Ser
                325                 330                 335
Thr Leu Asp Gln
            340

<210> 275
<211> 849
<212> DNA
<213> Unknown

<220>
<223> Obtained from an environmental sample

<400> 275
atggagacgg ctcacaaagc aaaggtcgat ttccttgtgc tgggtgagac gtggctctca      60
ggctacccgg cttggctgga ccactgcccc gatgttggcc ggtgggatta tgaaccgatg     120
aaaaaagtgt atttgagatt tcgacaaagt gctatttctg ttcctggcaa agaatttgat     180
ttccttactg gcctctgtaa aaaatattca caaacgcttg ccatcggtgt taatgagaaa     240
gtagatcatg gggtaggtaa tggtaccatt tataattcat ttctactgat tgattctgat     300
ggaacactgt tgaatcatca tcgcaagtta gttcccactt ttactgagaa attattatac     360
ggccatggag atggccatgg gctgaagtcg atggatactt cggtgggaag aatcggaggg     420
agcatttgtt gggaacattg gatgccacta tgcagacaag cacttcatga tgcaggtgag     480
caaatccatg ttgccctttg gccgactgtt catgacatcc atcaagtggc aagtagaagc     540
tatgcatttg aagggcgctg ctttgtattg gctgccgggc agatttttgc tgctaaagat     600
tttccaaagg aacttgtctt accagactat ctaaagcaaa atccggatca gctcattttg      660
aatgggggga gctgcgtgat cggccctgat gggaaatatt tgattgagcc cgtgtttgat     720
cgggaagaac tgattgtgtg tgaacttgac cttgacgaag cttataaaga aagaatgacg     780
atggacgttt caggtcacta ccaaagacga gacgttttca gttttgacgt gaaccaacat     840
cgacattga                                                             849

<210> 276
<211> 310
<212> PRT
<213> Unknown

<220>
<223> Obtained from an environmental sample

<400> 276
Met Thr Lys Leu Lys Ile Ala Ile Gly Gln Phe Ser Ser Asn His Leu
 1               5                  10                  15
Asp Leu Lys Cys Ser Leu Glu Lys Leu Glu Lys Ile Met Glu Thr Ala
            20                  25                  30
His Lys Ala Lys Val Asp Phe Leu Val Leu Gly Glu Thr Trp Leu Ser
        35                  40                  45
Gly Tyr Pro Ala Trp Leu Asp His Cys Pro Asp Val Gly Arg Trp Asp
    50                  55                  60
Tyr Glu Pro Met Lys Lys Val Tyr Leu Arg Phe Arg Gln Ser Ala Ile
65                  70                  75                  80
Ser Val Pro Gly Lys Glu Phe Asp Phe Leu Thr Gly Leu Cys Lys Lys
                85                  90                  95
Tyr Ser Gln Thr Leu Ala Ile Gly Val Asn Glu Lys Val Asp His Gly
            100                 105                 110
Val Gly Asn Gly Thr Ile Tyr Asn Ser Phe Leu Leu Ile Asp Ser Asp
        115                 120                 125
Gly Thr Leu Leu Asn His His Arg Lys Leu Val Pro Thr Phe Thr Glu
    130                 135                 140
Lys Leu Leu Tyr Gly His Gly Asp Gly His Gly Leu Lys Ser Met Asp

145                 150                 155                 160
Thr Ser Val Gly Arg Ile Gly Gly Ser Ile Cys Trp Glu His Trp Met
                165                 170                 175
Pro Leu Cys Arg Gln Ala Leu His Asp Ala Gly Glu Gln Ile His Val
            180                 185                 190
Ala Leu Trp Pro Thr Val His Asp Ile His Gln Val Ala Ser Arg Ser
        195                 200                 205
Tyr Ala Phe Glu Gly Arg Cys Phe Val Leu Ala Ala Gly Gln Ile Phe
    210                 215                 220
Ala Ala Lys Asp Phe Pro Lys Glu Leu Val Leu Pro Asp Tyr Leu Lys
225                 230                 235                 240
Gln Asn Pro Asp Gln Leu Ile Leu Asn Gly Gly Ser Cys Val Ile Gly
                245                 250                 255
Pro Asp Gly Lys Tyr Leu Ile Glu Pro Val Phe Asp Arg Glu Glu Leu
            260                 265                 270
Ile Val Cys Glu Leu Asp Leu Asp Glu Ala Tyr Lys Glu Arg Met Thr
        275                 280                 285
Met Asp Val Ser Gly His Tyr Gln Arg Arg Asp Val Phe Ser Phe Asp
    290                 295                 300
Val Asn Gln His Arg His
305                 310

<210> 277
<211> 1056
<212> DNA
<213> Unknown

<220>
<223> Obtained from an environmental sample

<400> 277
atgccaaccc ccagcgatca tttcaaaatc gccgctgttc aggcctcgcc cgtgtttctg      60
gaccgggagg ccactgtgga aaaggcctgc cggttgatcg ccgaagccgc aaagcagggc     120
gcccgcctca tcgtctttcc ggaatctttc atcccgacct acccggactg ggtatgggcc     180
gttcccccgg gaagggaaag aatcctgaac cagctgtatt ctgaattcct ggccaatgcc     240
gtcgatgttc ccggcgcggc gaccgaacaa cttgcccagg ctgcacgaat ggccggcgcc     300
tatgtgatta tgggcgtcac cgaaagagac acctcggcca gcggggccag cctctacaac     360
accctgctct acttcagccc cgaaggcatc ctaatgggca aacaccggaa gctggttccc     420
acggggggcg aacggctggt ctgggcctac ggagacggca gcacgctgga ggtctacgac     480
actccgctgg gaaagatcgg cgggctgatc tgctgggaga actacatgcc cctggcccgg     540
tacacgatgt acgcctgggg cacccagatt tacatcgccg ccacctggga ccgcggggaa     600
ccgtggctct ccaccctgcg gcatatcgcc aaggaaggaa gggtctacgt catcgggtgc     660
tgcatcgccc tgcgccaggg ggatatcccg gaccggttcg agtacaaggg aaaattttat     720
tccgggtccc gggagtggat caatgagggc gacagcgcca tcgtgaaccc ggacggggaa     780
ttcatcgccg ggccggtgcg gatgaaggag gagatcctgt atgccgagat agacccccgg     840
cagatgcggg gccccaagtg gatgctcgat gtggccggtc attacgcccg gccggatatc     900
ttcgagctca tcgtccaccg gaatccccac ccgatgatca aaatcgccga agacaggggc     960
gcggggatcg cctcaagttt gattcgcccc cgccctaacc ttcccccatc aagggggagg    1020
aaatcggcaa gaagcaaacg caagcccaaa aaatga                              1056

<210> 278
<211> 351
<212> PRT
<213> Unknown

<220>
<223> Obtained from an environmental sample

<400> 278
Met Pro Thr Pro Ser Asp His Phe Lys Ile Ala Ala Val Gln Ala Ser
1               5                   10                  15
Pro Val Phe Leu Asp Arg Glu Ala Thr Val Glu Lys Ala Cys Arg Leu
            20                  25                  30
Ile Ala Glu Ala Ala Lys Gln Gly Ala Arg Leu Ile Val Phe Pro Glu
        35                  40                  45
Ser Phe Ile Pro Thr Tyr Pro Asp Trp Val Trp Ala Val Pro Pro Gly
    50                  55                  60
Arg Glu Arg Ile Leu Asn Gln Leu Tyr Ser Glu Phe Leu Ala Asn Ala

65                  70                  75                  80
Val Asp Val Pro Gly Ala Ala Thr Glu Gln Leu Ala Gln Ala Ala Arg
                85                  90                  95
Met Ala Gly Ala Tyr Val Ile Met Gly Val Thr Glu Arg Asp Thr Ser
            100                 105                 110
Ala Ser Gly Ala Ser Leu Tyr Asn Thr Leu Leu Tyr Phe Ser Pro Glu
        115                 120                 125
Gly Ile Leu Met Gly Lys His Arg Lys Leu Val Pro Thr Gly Gly Glu
    130                 135                 140
Arg Leu Val Trp Ala Tyr Gly Asp Gly Ser Thr Leu Glu Val Tyr Asp
145                 150                 155                 160
Thr Pro Leu Gly Lys Ile Gly Gly Leu Ile Cys Trp Glu Asn Tyr Met
                165                 170                 175
Pro Leu Ala Arg Tyr Thr Met Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Lys Glu Gly Arg Val Tyr Val Ile Gly Cys Cys Ile Ala Leu
    210                 215                 220
Arg Gln Gly Asp Ile Pro Asp Arg Phe Glu Tyr Lys Gly Lys Phe Tyr
225                 230                 235                 240
Ser Gly Ser Arg Glu Trp Ile Asn Glu Gly Asp Ser Ala Ile Val Asn
                245                 250                 255
Pro Asp Gly Glu Phe Ile Ala Gly Pro Val Arg Met Lys Glu Glu Ile
            260                 265                 270
Leu Tyr Ala Glu Ile Asp Pro Arg Gln Met Arg Gly Pro Lys Trp Met
        275                 280                 285
Leu Asp Val Ala Gly His Tyr Ala Arg Pro Asp Ile Phe Glu Leu Ile
    290                 295                 300
Val His Arg Asn Pro His Pro Met Ile Lys Ile Ala Glu Asp Arg Gly
305                 310                 315                 320
Ala Gly Ile Ala Ser Ser Leu Ile Arg Pro Arg Pro Asn Leu Pro Pro
                325                 330                 335
Ser Arg Gly Arg Lys Ser Ala Arg Ser Lys Arg Lys Pro Lys Lys
            340                 345                 350

<210> 279
<211> 1014
<212> DNA
<213> Unknown

<220>
<223> Obtained from an environmental sample

<400> 279
atgggcatta cccatcccaa atacaaagtc gctgcagtcc aggccgcgcc ggtctggctg      60
gacctggatg ccacggtgga caagtgcatc cgcctgatcc aggaggccgc tgacaagggc     120
tgcaagctga tcgcgtttcc ggagacgttc attcccggct atccctggca catctggatg     180
ggtgcgccgg cctgggccat cggccggggc tttgtgcagc ggtatttcga caactccctg     240
tcctatgaca gcccgcaggc cgaaaagctg cgccaggccg tcaaggccgc aggcatcacc     300
gcgtcgctgg gactgtcgga gcgctcgggc ggcagtctct acatcgcgca gtggctcatt     360
ggccccgacg gcgaaacgat ctcgcagcgg cgtaagctgc ggcccacgca ttccgaacgc     420
accgtcttcg gcgacggcga tgggagcgac ctcaaggtgc acgacacgcc gctgggccgt     480
gtgggtgagc tggcgtgctg ggagaacatc ctgtcgctga acaagtacgc catgttctcg     540
cagcacgagc aggtgcacat tgcagcctgg cctagcttct ccacctacga gccctttgca     600
catgccctgg gctgggaagt gaacaacgca gtcagcaagg tgtacgcagt ggaaggcggg     660
tgtttcgtgg tggcaccctg cgccatcatt tccaaggaga tggtcgatga actgtgcgac     720
accccggaca agcacaccct gacccatgtg ggaggcggcc acgcggtgat ctacgggccg     780
gatggcgccc ccctggcgga caagctgcct gaagacgccg agggcctgct gatcgcggag     840
atcgacctcg ggatgattgg cgtggccaag aacgccatgg acccggtagg gcactactcg     900
cgccccgacg tgcaccgcct gcttctcaac cgcaagaagg cagcgcaggt ggagcacttt     960
gcgctgccgg tggacgccat cgattccgag ccccaagcca ccacggcgca ctga          1014

<210> 280
<211> 337
<212> PRT
<213> Unknown

<220>
<223> Obtained from an environmental sample

<400> 280
Met Gly Ile Thr His Pro Lys Tyr Lys Val Ala Ala Val Gln Ala Ala
1               5                   10                  15
Pro Val Trp Leu Asp Leu Asp Ala Thr Val Asp Lys Cys Ile Arg Leu
            20                  25                  30
Ile Gln Glu Ala Ala Asp Lys Gly Cys Lys Leu Ile Ala Phe Pro Glu
        35                  40                  45
Thr Phe Ile Pro Gly Tyr Pro Trp His Ile Trp Met Gly Ala Pro Ala
    50                  55                  60
Trp Ala Ile Gly Arg Gly Phe Val Gln Arg Tyr Phe Asp Asn Ser Leu
65                  70                  75                  80
Ser Tyr Asp Ser Pro Gln Ala Glu Lys Leu Arg Gln Ala Val Lys Ala
                85                  90                  95
Ala Gly Ile Thr Ala Ser Leu Gly Leu Ser Glu Arg Ser Gly Gly Ser
            100                 105                 110
Leu Tyr Ile Ala Gln Trp Leu Ile Gly Pro Asp Gly Glu Thr Ile Ser
        115                 120                 125
Gln Arg Arg Lys Leu Arg Pro Thr His Ser Glu Arg Thr Val Phe Gly
    130                 135                 140
Asp Gly Asp Gly Ser Asp Leu Lys Val His Asp Thr Pro Leu Gly Arg
145                 150                 155                 160
Val Gly Glu Leu Ala Cys Trp Glu Asn Ile Leu Ser Leu Asn Lys Tyr
                165                 170                 175
Ala Met Phe Ser Gln His Glu Gln Val His Ile Ala Ala Trp Pro Ser
            180                 185                 190
Phe Ser Thr Tyr Glu Pro Phe Ala His Ala Leu Gly Trp Glu Val Asn
        195                 200                 205
Asn Ala Val Ser Lys Val Tyr Ala Val Glu Gly Gly Cys Phe Val Val
    210                 215                 220
Ala Pro Cys Ala Ile Ile Ser Lys Glu Met Val Asp Glu Leu Cys Asp
225                 230                 235                 240
Thr Pro Asp Lys His Thr Leu Thr His Val Gly Gly Gly His Ala Val
                245                 250                 255
Ile Tyr Gly Pro Asp Gly Ala Pro Leu Ala Asp Lys Leu Pro Glu Asp
            260                 265                 270
Ala Glu Gly Leu Leu Ile Ala Glu Ile Asp Leu Gly Met Ile Gly Val
        275                 280                 285
Ala Lys Asn Ala Met Asp Pro Val Gly His Tyr Ser Arg Pro Asp Val
    290                 295                 300
His Arg Leu Leu Leu Asn Arg Lys Lys Ala Ala Gln Val Glu His Phe
305                 310                 315                 320
Ala Leu Pro Val Asp Ala Ile Asp Ser Glu Pro Gln Ala Thr Thr Ala
                325                 330                 335
His


<210> 281
<211> 936
<212> DNA
<213> Unknown

<220>
<223> Obtained from an environmental sample

<400> 281
atgtccaaga tcgccgtcgt ccaagagcct ccggtgctgc tcgatcgcgc cgccaccctc      60
gagcgcgccg tctcggccat cgagcgggcg gccgacgtgg gggcgcacct cgtcgtgttc     120
cccgagacgt acgtcccggg gtaccccgac tgggtctggc ggacgcgccc cgacgacttc     180
aagctcgcgg gcgcgctgca cgagcgcctc ctcgcgaacg cggtcgacct cgaaaaggac     240
cagctcgcgc cgctccgcga ggccgcgcgc cggcgaggcg tcaccatcgc gtgcggcgtg     300
aacgagcgcg aggggagcca cggccgcgcg accctctaca acaccgtcgt cgtcgtcgga     360
cccgacggcg cgatcctcaa ccgccaccgc aagctcgtcc ccacgaaccc cgagcgcatg     420
gtctggggcc cgggtgacgc gagcgggctg cgcgtcgtcg acacgccggc cggccgcgtg     480
ggggcgctca tctgctggga gaactacatg ccgctcgcgc gcttcgcgct ctacgcgcag     540
ggcgtcgagg tctacctcgc gccgacgtgg gatcacggcg acacttggct cgcctccatg     600

cggcacatcg cgcgcgaggc gcgcgcctgg gtcgtctcgg gggccatctg catgcaggcg 660
aaggacgtcc ccgccgactt cccgcagcgc gcggcgatct accccgacga ggaggagtgg 720
ctcaaccccg gcgatgccgt cgtcgtcgat cccaccggcg ccgtcgccgc cggcccgctg 780
caccgcgagc gcggcatcct ctacgccgag tgcgatcccg cgcgggcgtc gctcgcccgc 840
cgcacgctcg acgtctccgg gcactacgga cggcccgacg tctttcacct gcagatcgac 900
cgcacaccgc gcgtgccggc gtcgttccgg gactga 936

<210> 282
<211> 311
<212> PRT
<213> Unknown

<220>
<223> Obtained from an environmental sample

<400> 282
Met Ser Lys Ile Ala Val Val Gln Glu Pro Pro Val Leu Leu Asp Arg
1               5                   10                  15
Ala Ala Thr Leu Glu Arg Ala Val Ser Ala Ile Glu Arg Ala Ala Asp
            20                  25                  30
Val Gly Ala His Leu Val Val Phe Pro Glu Thr Tyr Val Pro Gly Tyr
        35                  40                  45
Pro Asp Trp Val Trp Arg Thr Arg Pro Asp Asp Phe Lys Leu Ala Gly
    50                  55                  60
Ala Leu His Glu Arg Leu Leu Ala Asn Ala Val Asp Leu Glu Lys Asp
65                  70                  75                  80
Gln Leu Ala Pro Leu Arg Glu Ala Ala Arg Arg Arg Gly Val Thr Ile
                85                  90                  95
Ala Cys Gly Val Asn Glu Arg Glu Gly Ser His Gly Arg Ala Thr Leu
            100                 105                 110
Tyr Asn Thr Val Val Val Val Gly Pro Asp Gly Ala Ile Leu Asn Arg
        115                 120                 125
His Arg Lys Leu Val Pro Thr Asn Pro Glu Arg Met Val Trp Gly Pro
    130                 135                 140
Gly Asp Ala Ser Gly Leu Arg Val Val Asp Thr Pro Ala Gly Arg Val
145                 150                 155                 160
Gly Ala Leu Ile Cys Trp Glu Asn Tyr Met Pro Leu Ala Arg Phe Ala
                165                 170                 175
Leu Tyr Ala Gln Gly Val Glu Val Tyr Leu Ala Pro Thr Trp Asp His
            180                 185                 190
Gly Asp Thr Trp Leu Ala Ser Met Arg His Ile Ala Arg Glu Ala Arg
        195                 200                 205
Ala Trp Val Val Ser Gly Ala Ile Cys Met Gln Ala Lys Asp Val Pro
    210                 215                 220
Ala Asp Phe Pro Gln Arg Ala Ala Ile Tyr Pro Asp Glu Glu Glu Trp
225                 230                 235                 240
Leu Asn Pro Gly Asp Ala Val Val Val Asp Pro Thr Gly Ala Val Ala
                245                 250                 255
Ala Gly Pro Leu His Arg Glu Arg Gly Ile Leu Tyr Ala Glu Cys Asp
            260                 265                 270
Pro Ala Arg Ala Ser Leu Ala Arg Arg Thr Leu Asp Val Ser Gly His
        275                 280                 285
Tyr Gly Arg Pro Asp Val Phe His Leu Gln Ile Asp Arg Thr Pro Arg
    290                 295                 300
Val Pro Ala Ser Phe Arg Asp
305                 310

<210> 283
<211> 1017
<212> DNA
<213> Unknown

<220>
<223> Obtained from an environmental sample

<400> 283
atgggcatcg aacatccgaa gtacaaggtc gcggtggtgc aggcggcacc ggcctggctc 60
gatctcgacg cgtcgatcga caagaccatc gggctgatcg aggaggccgc ccaaaaaggc 120

gccaagctga ttgcattccc cgaggccttc atccccggtt acccctggca catctggatg     180
gactcgccgg cctgggcgat cggccgcggt ttcgtgcagc gctattttga caattcgctc     240
gcctatgaca gcccgcaggc cgagaaactg cgcgcggcgg ttcgcaaggc aaagctcacg     300
gccgtgatcg ggctgtccga gcgcgacggc ggcagtcttt acctcgcgca atggctgatc     360
gggcccgacg gtgagaccat cgccaagcgc cgcaagctgc ggccgacaca tgcggagcgc     420
acggtatacg gcgagggcga cggcagcgac ctcgcggttc acaaccgtcc ggacattggc     480
cgccttggcg cgctctgctg ctgggagcat ctccagccgc tgtcgaaata cgcgatgtac     540
gcgcagaacg agcaggtgca tgtcgcggcc tggccgagct tttcgctgta cgatcccttc     600
gcggtggcgc tcggcgccga agtgaacaac gcggcctcgc gcgtctatgc ggtcgaaggc     660
tcctgcttcg tgctggcgcc gtgcgcgacg gtctcgcagg ccatgatcga cgaactctgc     720
gaccggccgg acaagcacgc gctgttgcat gtcggcggcg gttttgccgc gatctacggt     780
cctgacggca gccagatcgg cgacaaactc gctcccgacc aggaagggtt gctgatcgcg     840
gagatcgatc tcggcgccat cggcgtcgcc aagaatgcgg cggatcccgc cgggcattat     900
tcgcggcctg acgtgacgcg actgttgctc aacaagaagc cgtacaagcg cgtcgagcag     960
ttttcgccgc cggccgaggc gctcgagccg acggatatcg cagcagcagc aagctaa      1017

<210> 284
<211> 338
<212> PRT
<213> Unknown

<220>
<223> Obtained from an environmental sample

<400> 284
Met Gly Ile Glu His Pro Lys Tyr Lys Val Ala Val Val Gln Ala Ala
1               5                   10                  15
Pro Ala Trp Leu Asp Leu Asp Ala Ser Ile Asp Lys Thr Ile Gly Leu
            20                  25                  30
Ile Glu Glu Ala Ala Gln Lys Gly Ala Lys Leu Ile Ala Phe Pro Glu
        35                  40                  45
Ala Phe Ile Pro Gly Tyr Pro Trp His Ile Trp Met Asp Ser Pro Ala
    50                  55                  60
Trp Ala Ile Gly Arg Gly Phe Val Gln Arg Tyr Phe Asp Asn Ser Leu
65                  70                  75                  80
Ala Tyr Asp Ser Pro Gln Ala Glu Lys Leu Arg Ala Ala Val Arg Lys
                85                  90                  95
Ala Lys Leu Thr Ala Val Ile Gly Leu Ser Glu Arg Asp Gly Gly Ser
            100                 105                 110
Leu Tyr Leu Ala Gln Trp Leu Ile Gly Pro Asp Gly Glu Thr Ile Ala
        115                 120                 125
Lys Arg Arg Lys Leu Arg Pro Thr His Ala Glu Arg Thr Val Tyr Gly
    130                 135                 140
Glu Gly Asp Gly Ser Asp Leu Ala Val His Asn Arg Pro Asp Ile Gly
145                 150                 155                 160
Arg Leu Gly Ala Leu Cys Cys Trp Glu His Leu Gln Pro Leu Ser Lys
                165                 170                 175
Tyr Ala Met Tyr Ala Gln Asn Glu Gln Val His Val Ala Ala Trp Pro
            180                 185                 190
Ser Phe Ser Leu Tyr Asp Pro Phe Ala Val Ala Leu Gly Ala Glu Val
        195                 200                 205
Asn Asn Ala Ala Ser Arg Val Tyr Ala Val Glu Gly Ser Cys Phe Val
    210                 215                 220
Leu Ala Pro Cys Ala Thr Val Ser Gln Ala Met Ile Asp Glu Leu Cys
225                 230                 235                 240
Asp Arg Pro Asp Lys His Ala Leu Leu His Val Gly Gly Gly Phe Ala
                245                 250                 255
Ala Ile Tyr Gly Pro Asp Gly Ser Gln Ile Gly Asp Lys Leu Ala Pro
            260                 265                 270
Asp Gln Glu Gly Leu Leu Ile Ala Glu Ile Asp Leu Gly Ala Ile Gly
        275                 280                 285
Val Ala Lys Asn Ala Ala Asp Pro Ala Gly His Tyr Ser Arg Pro Asp
    290                 295                 300
Val Thr Arg Leu Leu Leu Asn Lys Lys Pro Tyr Lys Arg Val Glu Gln
305                 310                 315                 320
Phe Ser Pro Pro Ala Glu Ala Leu Glu Pro Thr Asp Ile Ala Ala Ala
                325                 330                 335
Ala Ser

<210> 285
<211> 918
<212> DNA
<213> Unknown

<220>
<223> Obtained from an environmental sample

<400> 285
atgaatacta atctagtaaa ggtcgcggcg gctcaagttg ctccccattt tctcaatttg 60
agcaatacgg tggaaaaaac ctgcaactta atttctgaag caggcaaaaa tggagcaaag 120
ctaatcgtat ttccagaagc cttcatctct ggttatcccg attgggtctg gcttattccc 180
aatgcgaatt ctgcaatgct ggatgattta taccaggaat tggttgagaa cgcagtaacg 240
atccctgata caacaacaca taaactatgt caggctgcaa aagatgcagg ggtatatgtt 300
gcggtaggta tacatgagag aaattcagaa gcaagtggct tcacgctttt caatacgctt 360
ctatacatca acgatcaagg cgtaatcatc ggaaaacacc gaaaattaat ccctacaggg 420
ggcgaacggc tggtctgggg gcagggtaat ggggatacac tttctgcatt cgatacagac 480
ttcggcaaat taggaggatt gctttgttgg gaaaattata tgccactcgc gcgtcaagct 540
atgtattccg ttggaactga agtatatgta gccccaacct gggactccag cgagaattgg 600
ttgttaagca tgcgccatat tgcccgagag ggcggtatgt ttgtaattag tgtttgccag 660
gctctccgaa aagacgacat ccctgatcag tatgaattta agaaactcta tcctgataat 720
tcagaatgga tcaatagcgg taacagttgc atcatcaacc cgcgcggtga gattattgct 780
ggaccaatct caaaccaaca agaaatactt tatgcagatt tagacctgag tttaattgca 840
aaatctaaac gtatgttcga tgttactggg cattattccc ggccggatgt gtttagctat 900
gaaatcaaca aaagctag 918

<210> 286
<211> 305
<212> PRT
<213> Unknown

<220>
<223> Obtained from an environmental sample

<400> 286
Met Asn Thr Asn Leu Val Lys Val Ala Ala Ala Gln Val Ala Pro His
1               5                   10                  15
Phe Leu Asn Leu Ser Asn Thr Val Glu Lys Thr Cys Asn Leu Ile Ser
            20                  25                  30
Glu Ala Gly Lys Asn Gly Ala Lys Leu Ile Val Phe Pro Glu Ala Phe
        35                  40                  45
Ile Ser Gly Tyr Pro Asp Trp Val Trp Leu Ile Pro Asn Ala Asn Ser
    50                  55                  60
Ala Met Leu Asp Asp Leu Tyr Gln Glu Leu Val Glu Asn Ala Val Thr
65                  70                  75                  80
Ile Pro Asp Thr Thr Thr His Lys Leu Cys Gln Ala Ala Lys Asp Ala
                85                  90                  95
Gly Val Tyr Val Ala Val Gly Ile His Glu Arg Asn Ser Glu Ala Ser
            100                 105                 110
Gly Phe Thr Leu Phe Asn Thr Leu Leu Tyr Ile Asn Asp Gln Gly Val
        115                 120                 125
Ile Ile Gly Lys His Arg Lys Leu Ile Pro Thr Gly Gly Glu Arg Leu
    130                 135                 140
Val Trp Gly Gln Gly Asn Gly Asp Thr Leu Ser Ala Phe Asp Thr Asp
145                 150                 155                 160
Phe Gly Lys Leu Gly Gly Leu Leu Cys Trp Glu Asn Tyr Met Pro Leu
                165                 170                 175
Ala Arg Gln Ala Met Tyr Ser Val Gly Thr Glu Val Tyr Val Ala Pro
            180                 185                 190
Thr Trp Asp Ser Ser Glu Asn Trp Leu Leu Ser Met Arg His Ile Ala
        195                 200                 205
Arg Glu Gly Gly Met Phe Val Ile Ser Val Cys Gln Ala Leu Arg Lys
    210                 215                 220
Asp Asp Ile Pro Asp Gln Tyr Glu Phe Lys Lys Leu Tyr Pro Asp Asn
225                 230                 235                 240
Ser Glu Trp Ile Asn Ser Gly Asn Ser Cys Ile Ile Asn Pro Arg Gly

Glu Ile Ile Ala Gly Pro Ile Ser Asn Gln Gln Glu Ile Leu Tyr Ala
            245                 250                 255
Asp Leu Asp Leu Ser Leu Ile Ala Lys Ser Lys Arg Met Phe Asp Val
        260                 265                 270
Thr Gly His Tyr Ser Arg Pro Asp Val Phe Ser Tyr Glu Ile Asn Lys
    275                 280                 285
Ser
    290                 295                 300
305

<210> 287
<211> 936
<212> DNA
<213> Unknown

<220>
<223> Obtained from an environmental sample

<400> 287
gtgatcaagg tagcaatcgc ccaggtggca ccggtggttc tggacaaggc gcgcaccatt      60
gagaaagcgg taggaattat tcgcgctgcc gcgcaagagg gcattgagct cctggttttc     120
ccggagacgt ttatcccgac ctatccagcc tgggtatggc gcttgcgtcc gggtactgat     180
tacggcctga gcgaggaact gcacgcgctc ctgctggata attcggtaga tatggagagc     240
aaggacctgg agccattgca agctgttgct gcagagacca gcatgaccgt ggtaataggt     300
atgaacgagc gagacggccg attcagccgg ggtacaatct acaatgccct ggttgtgatc     360
ggtccaggtg gcacgatcct gaacaggcac cgcaagctta tgcccaccaa ccccgagcgt     420
atggtttggg gtatgggcga tgccagcggg ctgaaggtag tggaaatgtc ttacgggcgc      480
ctgggtgggc tgatttgctg ggagaatttc atgcctctcg cgcgctatgg cttgtatgcc     540
cagggtgtgg agatttacgt ggcgcccacc tatgaccagg gcgacggctg ggtcggcagc     600
atgcagcata tagcccggga gggtcgttgc tgggtactct cggccgggac ccttttgcgt    660
ggcagtgatt ttctgccgga ttttccgggc aagaccgagt tatatcccga tgaccaggag     720
tgggtgaatc cgggtggctc ggtgatcgtg gcaccggggg gagagattgt ggccggcccc     780
atgtatcgcg acgaaggtct gctggtctgc gagttggatg cgacgcttag tgtccgcggc     840
aagcgctcgc tggatgtggc cggccattac tcccggccgg atttgtttga actggaaata     900
gatggcgacc cgctggaacc catagagtgg gattga                               936

<210> 288
<211> 311
<212> PRT
<213> Unknown

<220>
<223> Obtained from an environmental sample

<400> 288
Val Ile Lys Val Ala Ile Ala Gln Val Ala Pro Val Val Leu Asp Lys
1               5                   10                  15
Ala Arg Thr Ile Glu Lys Ala Val Gly Ile Ile Arg Ala Ala Ala Gln
            20                  25                  30
Glu Gly Ile Glu Leu Leu Val Phe Pro Glu Thr Phe Ile Pro Thr Tyr
        35                  40                  45
Pro Ala Trp Val Trp Arg Leu Arg Pro Gly Thr Asp Tyr Gly Leu Ser
    50                  55                  60
Glu Glu Leu His Ala Leu Leu Leu Asp Asn Ser Val Asp Met Glu Ser
65                  70                  75                  80
Lys Asp Leu Glu Pro Leu Gln Ala Val Ala Ala Glu Thr Ser Met Thr
                85                  90                  95
Val Val Ile Gly Met Asn Glu Arg Asp Gly Arg Phe Ser Arg Gly Thr
            100                 105                 110
Ile Tyr Asn Ala Leu Val Val Ile Gly Pro Gly Gly Thr Ile Leu Asn
        115                 120                 125
Arg His Arg Lys Leu Met Pro Thr Asn Pro Glu Arg Met Val Trp Gly
    130                 135                 140
Met Gly Asp Ala Ser Gly Leu Lys Val Val Glu Met Ser Tyr Gly Arg
145                 150                 155                 160
Leu Gly Gly Leu Ile Cys Trp Glu Asn Phe Met Pro Leu Ala Arg Tyr
                165                 170                 175
Gly Leu Tyr Ala Gln Gly Val Glu Ile Tyr Val Ala Pro Thr Tyr Asp

                180                 185                 190
Gln Gly Asp Gly Trp Val Gly Ser Met Gln His Ile Ala Arg Glu Gly
        195                 200                 205
Arg Cys Trp Val Leu Ser Ala Gly Thr Leu Leu Arg Gly Ser Asp Phe
    210                 215                 220
Leu Pro Asp Phe Pro Gly Lys Thr Glu Leu Tyr Pro Asp Asp Gln Glu
225                 230                 235                 240
Trp Val Asn Pro Gly Gly Ser Val Ile Val Ala Pro Gly Gly Glu Ile
                245                 250                 255
Val Ala Gly Pro Met Tyr Arg Asp Glu Gly Leu Leu Val Cys Glu Leu
            260                 265                 270
Asp Ala Thr Leu Ser Val Arg Gly Lys Arg Ser Leu Asp Val Ala Gly
        275                 280                 285
His Tyr Ser Arg Pro Asp Leu Phe Glu Leu Glu Ile Asp Gly Asp Pro
    290                 295                 300
Leu Glu Pro Ile Glu Trp Asp
305                 310

<210> 289
<211> 921
<212> DNA
<213> Unknown

<220>
<223> Obtained from an environmental sample

<400> 289
atggtgacgg tggccgccgt acaggcaacg ccggtgttcc tcgaccgcga ggcgacctcg      60
gacaaggtct gcgccttggt caaggaggcg gccggccacg gggcagaact gatcgtcttc     120
cccgagtcct tcgtccctgc ctatccggac tgggtgtggc gcacccctgc ctggagtgac     180
accgagttcg tgaagcgctt ctacgcgaac gcggtgaccg tccccggcgc gaccctcgag     240
cgcatcggcg cagcggcggc ggaggcggag gcgtacgtcg tgatcggcgt gaccgagatc     300
gacggcggaa ctctctacaa caccctcttc tacctgggcc cggacggaca gctgttgcaa     360
cggcatcgca agctcatgcc caccggtggg gagcggaccg tgtggggaat gggagacggc     420
tctgagctcg acgtcgtgag cacgccgttc ggcgtcgtcg gtgggttgtt gtgctgggag     480
aactacatgc cgctcgcccg ggcggcgatc tacgcccagc actgtgacat ctacctggct     540
ccgacatggg acaacagcga cacgtgggta gccacgttgc gtcacatcgc caaggagggg     600
cggcagttcg tcatcggcgt cgccccgctg ctgcgcggct ccgacgtacc ggaggacctc     660
cgcggcacgc tctacgggct gtcggacgac tggatgtcgc gcggctacac caccatcgtc     720
gcaccaagcg gcgaggtgat cgccggcccg gtcctggagc gtgaggagat cctcttcgcg     780
gacctcgacc tggccgacgt gcaggagcag agaaggatgt tcgaccctgt cggccactac     840
tcacgacccg acgtcttcac gctccacgtc gacgcacgac cgaagagccc ggtcgtcttc     900
gagagggatg caccgacctg a                                               921

<210> 290
<211> 306
<212> PRT
<213> Unknown

<220>
<223> Obtained from an environmental sample

<400> 290
Met Val Thr Val Ala Ala Val Gln Ala Thr Pro Val Phe Leu Asp Arg
1               5                   10                  15
Glu Ala Thr Ser Asp Lys Val Cys Ala Leu Val Lys Glu Ala Ala Gly
            20                  25                  30
His Gly Ala Glu Leu Ile Val Phe Pro Glu Ser Phe Val Pro Ala Tyr
        35                  40                  45
Pro Asp Trp Val Trp Arg Thr Pro Ala Trp Ser Asp Thr Glu Phe Val
    50                  55                  60
Lys Arg Phe Tyr Ala Asn Ala Val Thr Val Pro Gly Ala Thr Leu Glu
65                  70                  75                  80
Arg Ile Gly Ala Ala Ala Ala Glu Ala Glu Ala Tyr Val Val Ile Gly
                85                  90                  95
Val Thr Glu Ile Asp Gly Gly Thr Leu Tyr Asn Thr Leu Leu Tyr Leu
            100                 105                 110
Gly Pro Asp Gly Gln Leu Leu Gln Arg His Arg Lys Leu Met Pro Thr

115                 120                 125
Gly Gly Glu Arg Thr Val Trp Gly Met Gly Asp Gly Ser Glu Leu Asp
    130                 135                 140
Val Val Ser Thr Pro Phe Gly Val Val Gly Gly Leu Leu Cys Trp Glu
145                 150                 155                 160
Asn Tyr Met Pro Leu Ala Arg Ala Ala Ile Tyr Ala Gln His Cys Asp
                165                 170                 175
Ile Tyr Leu Ala Pro Thr Trp Asp Asn Ser Asp Thr Trp Val Ala Thr
            180                 185                 190
Leu Arg His Ile Ala Lys Glu Gly Arg Gln Phe Val Ile Gly Val Ala
        195                 200                 205
Pro Leu Leu Arg Gly Ser Asp Val Pro Glu Asp Leu Arg Gly Thr Leu
    210                 215                 220
Tyr Gly Leu Ser Asp Asp Trp Met Ser Arg Gly Tyr Thr Thr Ile Val
225                 230                 235                 240
Ala Pro Ser Gly Glu Val Ile Ala Gly Pro Val Leu Glu Arg Glu Glu
                245                 250                 255
Ile Leu Phe Ala Asp Leu Asp Leu Ala Asp Val Gln Glu Gln Arg Arg
            260                 265                 270
Met Phe Asp Pro Val Gly His Tyr Ser Arg Pro Asp Val Phe Thr Leu
        275                 280                 285
His Val Asp Ala Arg Pro Lys Ser Pro Val Val Phe Glu Arg Asp Ala
    290                 295                 300
Pro Thr
305

<210> 291
<211> 1002
<212> DNA
<213> Unknown

<220>
<223> Obtained from an environmental sample

<400> 291
atgatgaaaa caactgttac cgttgcctgc gttcaggccg cccccgtatt tatggattta      60
gaaggcacca tagataaaac gatcaccctc atctctgaag ccgcacagaa aggcgcggag     120
ctcatcgctt ttccggagac ctggataccc ggttacccgt ggttcttatg gctgaactcg     180
cccgcgacaa atatgcccct ggtttatcaa tatcatcaga actctctggt gctggacagt     240
gcccaggcga agcgaattgc ggatgctgca cagcagaata acatcactgt cgttctggga     300
ttcagcgagc gcgatcatgg aagcctctat atctcacagt ggctgattgg cagcgacggg     360
gaaactattg gcatccggcg caagctcaag gccacacacg tggagcgtac gctgttcggc     420
gaaagcgacg gctcctccct gaccacctgg gagacacctc tgggtaacgt cggggccctc     480
tgctgctggg agcacctgca gccgctgtct cgctatgcga tgtattccca gcatgaagag     540
atccatatcg ctgcgtggcc cagcttcagt ctttacacca gcgcaactgc cgcgctcgga     600
cctgacgtca acacggcggc ttcacgcctc tatgccgcgg aggggcagtg cttcgtgtta     660
gccccatgtg ccgtggtttc tgatgagatg attgatttac tctgtcctga tgatgaccgg     720
agagcgttac tcagtgccgg aggggacatt gcccgtattt acggacctga tggaagagaa     780
ctcgtcaccc ctctcgggga aaatgaggaa ggactgctta tcgctgagct cgactctgct     840
gcgatcacct ttgccaaact ggcggcagat cccgtaggcc actattcccg ccctgacgtg     900
acccgccttc tttttaatcc ttcagccaac aagactgtta ttaaacggca ttcgcctcct     960
gagttaattg ccgaacaggc tccggaagaa gaggaggagt ag                       1002

<210> 292
<211> 333
<212> PRT
<213> Unknown

<220>
<223> Obtained from an environmental sample

<400> 292
Met Met Lys Thr Thr Val Thr Val Ala Cys Val Gln Ala Ala Pro Val
1               5                   10                  15
Phe Met Asp Leu Glu Gly Thr Ile Asp Lys Thr Ile Thr Leu Ile Ser
            20                  25                  30
Glu Ala Ala Gln Lys Gly Ala Glu Leu Ile Ala Phe Pro Glu Thr Trp
        35                  40                  45

Ile Pro Gly Tyr Pro Trp Phe Leu Trp Leu Asn Ser Pro Ala Thr Asn
    50                  55                  60
Met Pro Leu Val Tyr Gln Tyr His Gln Asn Ser Leu Val Leu Asp Ser
65                  70                  75                  80
Ala Gln Ala Lys Arg Ile Ala Asp Ala Ala Gln Gln Asn Asn Ile Thr
                85                  90                  95
Val Val Leu Gly Phe Ser Glu Arg Asp His Gly Ser Leu Tyr Ile Ser
            100                 105                 110
Gln Trp Leu Ile Gly Ser Asp Gly Glu Thr Ile Gly Ile Arg Arg Lys
        115                 120                 125
Leu Lys Ala Thr His Val Glu Arg Thr Leu Phe Gly Glu Ser Asp Gly
    130                 135                 140
Ser Ser Leu Thr Thr Trp Glu Thr Pro Leu Gly Asn Val Gly Ala Leu
145                 150                 155                 160
Cys Cys Trp Glu His Leu Gln Pro Leu Ser Arg Tyr Ala Met Tyr Ser
                165                 170                 175
Gln His Glu Glu Ile His Ile Ala Ala Trp Pro Ser Phe Ser Leu Tyr
            180                 185                 190
Thr Ser Ala Thr Ala Ala Leu Gly Pro Asp Val Asn Thr Ala Ala Ser
        195                 200                 205
Arg Leu Tyr Ala Ala Glu Gly Gln Cys Phe Val Leu Ala Pro Cys Ala
    210                 215                 220
Val Val Ser Asp Glu Met Ile Asp Leu Leu Cys Pro Asp Asp Asp Arg
225                 230                 235                 240
Arg Ala Leu Leu Ser Ala Gly Gly Gly His Ala Arg Ile Tyr Gly Pro
                245                 250                 255
Asp Gly Arg Glu Leu Val Thr Pro Leu Gly Glu Asn Glu Glu Gly Leu
            260                 265                 270
Leu Ile Ala Glu Leu Asp Ser Ala Ala Ile Thr Phe Ala Lys Leu Ala
        275                 280                 285
Ala Asp Pro Val Gly His Tyr Ser Arg Pro Asp Val Thr Arg Leu Leu
    290                 295                 300
Phe Asn Pro Ser Ala Asn Lys Thr Val Ile Lys Arg His Ser Pro Pro
305                 310                 315                 320
Glu Leu Ile Ala Glu Gln Ala Pro Glu Glu Glu Glu Glu
                325                 330

<210> 293
<211> 1008
<212> DNA
<213> Unknown

<220>
<223> Obtained from an environmental sample

<400> 293
atgaaaaata tcaaaaactc agaaaaaagc agcacagtaa gagtcgctgc ggtacaaatc      60
agtccggtgt tgtacaaccg cgaagctacc gttcaaaaag tagtcaacaa aatccttgaa     120
ctaggaaaac aaggggtaca attcgccact tttccggaaa cgatagtgcc ttattatcct     180
tatttctctt ttattcaggc gccttatgcc atgggcaaag aacacctgcg cttgcttgaa     240
caatcagtta ctgttccgtc agccgcgacc gatgccataa gtgaggcggc aaaggaagcc     300
aatatggtag tgtctattgg tgtcaatgaa cgagacggtg gtaccattta caatacgcaa     360
ctcctttttg atgctgacgg aacattaatt cagcgcagac gtaaacttac accaacgtat     420
catgaaagaa tgatttgggg acaaggtgac gcttcaggtc ttcgtgccac agacagcgct     480
gttgggcgta tcgggcagtt ggcttgttgg gaacattaca atccattgtt ccgttatgct     540
ttgattgctg atggagaaca aatccattct gccatgtatc ccggatcatt tttaggtgcg     600
ttgcacggtg aacaaaccga aatcaatgta cgccaacacg ctttagaatc ggccagcttc     660
gtcgtagtgg ctaccggttg gttggatgcc gatcaacaag cacaaattgc gaaagacacc     720
ggtggaccaa tcggaccaat ttcgggaggt tgttttacag ccgttatagg ccctgacgga     780
caactaatcg gggaagccct tacatcaggt gaaggggaag tgattgccga tattgatttg     840
gcacaaattg atgcccgcaa aagattaatg gatgccagtg gtcactacaa ccgtcctgaa     900
ttgttgagct tgcatatcga tcacactccg actgctccta tgcatgaaag agtagtttac     960
actgagccgg gattagcaaa aagacaaaat gaaaattcat caaattaa                 1008

<210> 294
<211> 335
<212> PRT
<213> Unknown

<220>
<223> Obtained from an environmental sample

<400> 294
Met Lys Asn Ile Lys Asn Ser Glu Lys Ser Ser Thr Val Arg Val Ala
1               5                   10                  15
Ala Val Gln Ile Ser Pro Val Leu Tyr Asn Arg Glu Ala Thr Val Gln
            20                  25                  30
Lys Val Val Asn Lys Ile Leu Glu Leu Gly Lys Gln Gly Val Gln Phe
        35                  40                  45
Ala Thr Phe Pro Glu Thr Ile Val Pro Tyr Tyr Pro Tyr Phe Ser Phe
    50                  55                  60
Ile Gln Ala Pro Tyr Ala Met Gly Lys Glu His Leu Arg Leu Leu Glu
65                  70                  75                  80
Gln Ser Val Thr Val Pro Ser Ala Ala Thr Asp Ala Ile Ser Glu Ala
                85                  90                  95
Ala Lys Glu Ala Asn Met Val Val Ser Ile Gly Val Asn Glu Arg Asp
            100                 105                 110
Gly Gly Thr Ile Tyr Asn Thr Gln Leu Leu Phe Asp Ala Asp Gly Thr
        115                 120                 125
Leu Ile Gln Arg Arg Arg Lys Leu Thr Pro Thr Tyr His Glu Arg Met
    130                 135                 140
Ile Trp Gly Gln Gly Asp Ala Ser Gly Leu Arg Ala Thr Asp Ser Ala
145                 150                 155                 160
Val Gly Arg Ile Gly Gln Leu Ala Cys Trp Glu His Tyr Asn Pro Leu
                165                 170                 175
Phe Arg Tyr Ala Leu Ile Ala Asp Gly Glu Gln Ile His Ser Ala Met
            180                 185                 190
Tyr Pro Gly Ser Phe Leu Gly Ala Leu His Gly Glu Gln Thr Glu Ile
        195                 200                 205
Asn Val Arg Gln His Ala Leu Glu Ser Ala Ser Phe Val Val Val Ala
    210                 215                 220
Thr Gly Trp Leu Asp Ala Asp Gln Gln Ala Gln Ile Ala Lys Asp Thr
225                 230                 235                 240
Gly Gly Pro Ile Gly Pro Ile Ser Gly Gly Cys Phe Thr Ala Val Ile
                245                 250                 255
Gly Pro Asp Gly Gln Leu Ile Gly Glu Ala Leu Thr Ser Gly Glu Gly
            260                 265                 270
Glu Val Ile Ala Asp Ile Asp Leu Ala Gln Ile Asp Ala Arg Lys Arg
        275                 280                 285
Leu Met Asp Ala Ser Gly His Tyr Asn Arg Pro Glu Leu Leu Ser Leu
    290                 295                 300
His Ile Asp His Thr Pro Thr Ala Pro Met His Glu Arg Val Val Tyr
305                 310                 315                 320
Thr Glu Pro Gly Leu Ala Lys Arg Gln Asn Glu Asn Ser Ser Asn
                325                 330                 335

<210> 295
<211> 1134
<212> DNA
<213> Unknown

<220>
<223> Obtained from an environmental sample

<400> 295
atggccgcaa aagtacttgg aggacgtgac acagtaaaag tagcagttgt tcaaacacca      60
tcagtcttta tggacaagaa agcctgtctc gaacttgcct gcgataagat tatcgaagcc     120
ggcaaagagg gcgcggagct tgttgttttt cctgaaacct ggattccgac atatccttat     180
tggaccatgg gatgggatac catcgcccat ggcttccatg atgtgatggc ggacctgcag     240
gacaattccg tggtcgtcgg gagcgaagac accgacatat tgggcaaagc cgcccgggaa     300
gctggcgcct acgtcgtcat gggctgtaat gagctcgatg accgggtcgg cagcaggacc     360
ttgttcaact cgctggtcta tatggacaaa tatggcggcg tgctcggccg tcaccgtaaa     420
ttaatcccgt cctttatcga acgcatctgg tggggcaatg gggacagccg cgatctcaaa     480
gtttacgaca cggaaattgg gcgcatcggc ggtcaaatct gctgggaaaa tcacattgtg     540
aacatcaccg catggtacat tgcccaaggc gtggatattc atgtctcggt ttggccggga     600
atgtggaact gtggcgcaga agaaggagaa tccttcatat acgccggtca cgacatcaac     660

aaatgtgacc tcatccctgc tacacgcgga cgggcattta cgggtcagtg ttatgtcctc 720
tcagccaaca acctactgcg gatggaagac attcctgacg atttcccgtt ccgggatacc 780
atgaactatg gcggtccggg ccaggaggat tttgtcggat gggcttgcgg tggcagccat 840
attgttgcgc caacgtctga atttatggtg ccgccgacgt ttgatataga caccatcatc 900
tatgcagaac ttcaggcgaa atacatcaaa gtggtgaagt cggtcttcga ttccctcggc 960
cactacgcgc ggtgggacct cgtcaacttg accacgccgc caccgccgta tgaacctgaa 1020
accgacgcac cagctctcac ggccgatatc cgtgatcggg tcatcgagag tgtggctaaa 1080
gagttcaagc tcgaaccaga aaaagtggct gaagttgtgc gcaatgccgc ctag 1134

<210> 296
<211> 377
<212> PRT
<213> Unknown

<220>
<223> Obtained from an environmental sample

<400> 296
Met Ala Ala Lys Val Leu Gly Gly Arg Asp Thr Val Lys Val Ala Val
1               5                   10                  15
Val Gln Thr Pro Ser Val Phe Met Asp Lys Lys Ala Cys Leu Glu Leu
            20                  25                  30
Ala Cys Asp Lys Ile Ile Glu Ala Gly Lys Glu Gly Ala Glu Leu Val
        35                  40                  45
Val Phe Pro Glu Thr Trp Ile Pro Thr Tyr Pro Tyr Trp Thr Met Gly
    50                  55                  60
Trp Asp Thr Ile Ala His Gly Phe His Asp Val Met Ala Asp Leu Gln
65                  70                  75                  80
Asp Asn Ser Val Val Val Gly Ser Glu Asp Thr Asp Ile Leu Gly Lys
                85                  90                  95
Ala Ala Arg Glu Ala Gly Ala Tyr Val Val Met Gly Cys Asn Glu Leu
            100                 105                 110
Asp Asp Arg Val Gly Ser Arg Thr Leu Phe Asn Ser Leu Val Tyr Met
        115                 120                 125
Asp Lys Tyr Gly Gly Val Leu Gly Arg His Arg Lys Leu Ile Pro Ser
    130                 135                 140
Phe Ile Glu Arg Ile Trp Trp Gly Asn Gly Asp Ser Arg Asp Leu Lys
145                 150                 155                 160
Val Tyr Asp Thr Glu Ile Gly Arg Ile Gly Gly Gln Ile Cys Trp Glu
                165                 170                 175
Asn His Ile Val Asn Ile Thr Ala Trp Tyr Ile Ala Gln Gly Val Asp
            180                 185                 190
Ile His Val Ser Val Trp Pro Gly Met Trp Asn Cys Gly Ala Glu Glu
        195                 200                 205
Gly Glu Ser Phe Ile Tyr Ala Gly His Asp Ile Asn Lys Cys Asp Leu
    210                 215                 220
Ile Pro Ala Thr Arg Gly Arg Ala Phe Thr Gly Gln Cys Tyr Val Leu
225                 230                 235                 240
Ser Ala Asn Asn Leu Leu Arg Met Glu Asp Ile Pro Asp Asp Phe Pro
                245                 250                 255
Phe Arg Asp Thr Met Asn Tyr Gly Gly Pro Gly Gln Glu Asp Phe Val
            260                 265                 270
Gly Trp Ala Cys Gly Gly Ser His Ile Val Ala Pro Thr Ser Glu Phe
        275                 280                 285
Met Val Pro Pro Thr Phe Asp Ile Asp Thr Ile Ile Tyr Ala Glu Leu
    290                 295                 300
Gln Ala Lys Tyr Ile Lys Val Val Lys Ser Val Phe Asp Ser Leu Gly
305                 310                 315                 320
His Tyr Ala Arg Trp Asp Leu Val Asn Leu Thr Thr Pro Pro Pro Pro
                325                 330                 335
Tyr Glu Pro Glu Thr Asp Ala Pro Ala Leu Thr Ala Asp Ile Arg Asp
            340                 345                 350
Arg Val Ile Glu Ser Val Ala Lys Glu Phe Lys Leu Glu Pro Glu Lys
        355                 360                 365
Val Ala Glu Val Val Arg Asn Ala Ala
    370                 375

<210> 297

<211> 1059
<212> DNA
<213> Unknown

<220>
<223> Obtained from an environmental sample

<400> 297
atgacacgtt ttcgggacgt cacggtggcg gcggttcagg ccgcacccgt ctatttcgat 60
cgggaggcct ccacagataa ggcgtgccaa ttgattcacg aagcggcgaa gaaaggcgca 120
gccctcgcgg cgttcggcga aacgtggttg ccgggatatc cgttctttgc atgggggttc 180
gcgcacaacc ggagcctgtt ctggaatgcg gccgccgagt acatcgccaa tgctgtggag 240
attccgagtc caacgacgga ccgcctctgc gccgcagcga agatcgccgg gattgacgtg 300
gtaatcggcg tcgtagaact ggatggacga acgcgagcgt cggtttacag cacactgctg 360
ttcatcggga gagagggggc gatcctgggg cgccaccgca aattgaagcc aacccacatg 420
gagcgaacgg tgtggggtga aggggacgct cacgggctcc gcgttcacga gcgtccgtac 480
ggccgcctca gcgggctgaa ttgctgggaa cacaacatga tgctgcccgg ctatgtgctt 540
gccgcgcagg gcacgcagtt tcacgtcgcg acatggcctg ggaaagagag gctcacagtt 600
ccgccgaacg aggcggctta tacgcgccag cttctcctct ctcgcgccta tgcatcccag 660
gcgggcgcgt acgtgatcag cgtcgcgggg ctgctcggac ccgactcgat gccggagcgt 720
tatcgcgaac tgggacagtc ctatgagttg accggcgaca gcgtcatcat cgatccgcgc 780
ggcgaggtca tcgcggggcc cgcgaagggc gagaccatcc tgctcgcgca atgcagccag 840
gaagccctct tcgccgcaaa gtccgccatt gacgtcggcg gtcattactc gcgcccggat 900
atttttcagc tgcgtgtcaa cgatcagcta cagcatcagg tccggagact cgaggcgact 960
ctcacgcccc cagtcgccgt agttgtcggg cctgagggta gctcacatga gcaggagacg 1020
gccttcgggc catccagcct cctggccacg acaagctag 1059

<210> 298
<211> 352
<212> PRT
<213> Unknown

<220>
<223> Obtained from an environmental sample

<400> 298
Met Thr Arg Phe Arg Asp Val Thr Val Ala Ala Val Gln Ala Ala Pro
1               5                   10                  15
Val Tyr Phe Asp Arg Glu Ala Ser Thr Asp Lys Ala Cys Gln Leu Ile
            20                  25                  30
His Glu Ala Ala Lys Lys Gly Ala Ala Leu Ala Ala Phe Gly Glu Thr
        35                  40                  45
Trp Leu Pro Gly Tyr Pro Phe Phe Ala Trp Gly Phe Ala His Asn Arg
    50                  55                  60
Ser Leu Phe Trp Asn Ala Ala Ala Glu Tyr Ile Ala Asn Ala Val Glu
65                  70                  75                  80
Ile Pro Ser Pro Thr Thr Asp Arg Leu Cys Ala Ala Ala Lys Ile Ala
                85                  90                  95
Gly Ile Asp Val Val Ile Gly Val Val Glu Leu Asp Gly Arg Thr Arg
            100                 105                 110
Ala Ser Val Tyr Ser Thr Leu Leu Phe Ile Gly Arg Glu Gly Ala Ile
        115                 120                 125
Leu Gly Arg His Arg Lys Leu Lys Pro Thr His Met Glu Arg Thr Val
    130                 135                 140
Trp Gly Glu Gly Asp Ala His Gly Leu Arg Val His Glu Arg Pro Tyr
145                 150                 155                 160
Gly Arg Leu Ser Gly Leu Asn Cys Trp Glu His Asn Met Met Leu Pro
                165                 170                 175
Gly Tyr Val Leu Ala Ala Gln Gly Thr Gln Phe His Val Ala Thr Trp
            180                 185                 190
Pro Gly Lys Glu Arg Leu Thr Val Pro Pro Asn Glu Ala Ala Tyr Thr
        195                 200                 205
Arg Gln Leu Leu Leu Ser Arg Ala Tyr Ala Ser Gln Ala Gly Ala Tyr
    210                 215                 220
Val Ile Ser Val Ala Gly Leu Leu Gly Pro Asp Ser Met Pro Glu Arg
225                 230                 235                 240
Tyr Arg Glu Leu Gly Gln Ser Tyr Glu Leu Thr Gly Asp Ser Val Ile
                245                 250                 255

Ile Asp Pro Arg Gly Glu Val Ile Ala Gly Pro Ala Lys Gly Glu Thr
            260                 265                 270
Ile Leu Leu Ala Gln Cys Ser Gln Glu Ala Leu Phe Ala Ala Lys Ser
        275                 280                 285
Ala Ile Asp Val Gly Gly His Tyr Ser Arg Pro Asp Ile Phe Gln Leu
    290                 295                 300
Arg Val Asn Asp Gln Leu Gln His Gln Val Arg Arg Leu Glu Ala Thr
305                 310                 315                 320
Leu Thr Pro Pro Val Ala Val Val Val Gly Pro Glu Gly Ser Ser His
                325                 330                 335
Glu Gln Glu Thr Ala Phe Gly Pro Ser Ser Leu Leu Ala Thr Thr Ser
            340                 345                 350

<210> 299
<211> 987
<212> DNA
<213> Unknown

<220>
<223> Obtained from an environmental sample

<400> 299
atgactgttg ttaaggccgc cgcagtgcag atcagcccgg tgctctacag ccgtgcagga      60
accgtcgaga aggtcgtgaa gaagatcgac gagctgggcc agaagggtgt cgagtttgcc     120
gtcttccctg aaaccgttgt cccctactac ccctacttct ccttcgtgca gcccccctac     180
aaactggcca cggagcacct gcgcctgctt gaggagtcgg tgaccgtgcc ctctgccgag     240
acggacgcca tcggcgacgc cgcccgcaag gccaacatgg tcgtctcgat cggtgtcaac     300
gaacgtgatg gcggcaccat ttacaacacc caactcctgt tcgacgccga cggaaccctg     360
atccagcgcc gccgcaagat cacgccgacc taccacgagc ggatgatctg gggacaggga     420
gacggatcag gcttgcgcgc ggtcgacagc gtcgtcggcc gcatcggcca gctcgcctgc     480
tgggagcact accagccgct ggcccgttac gctctcatcg ccgacggcga gcagatccac     540
gccgcgatgt accccggcgc cttcgggggc gatctgttcg cagagcagat cgaagtcaat     600
gtccgtcagc acgctctgga atcggccagt ttcgtcgtca gcgccaccgc ctggctcgac     660
gccgaccagc aggcccagat tgcgaaggac accggcggcc ccgtacaggc gatctccggc     720
ggcttcttca cagccatcat cgaccccgac ggccgcatca tcggcgaacc gatcacctcc     780
ggcgaaggcg aagtcatcgc tgacctcgac tttgcgctca tcgaccgccg caagcgcctg     840
atggacgcca gcggccacta cagccgcccc gaactgctca gcctgcagat cgaccggacg     900
ccggcacccg ccgtccacga tcgcaatcgc caggggtcct caagcgctcc ggcaactgaa     960
aagggccgct cagccgaggc caagtga                                         987

<210> 300
<211> 328
<212> PRT
<213> Unknown

<220>
<223> Obtained from an environmental sample

<400> 300
Met Thr Val Val Lys Ala Ala Ala Val Gln Ile Ser Pro Val Leu Tyr
1               5                   10                  15
Ser Arg Ala Gly Thr Val Glu Lys Val Val Lys Lys Ile Asp Glu Leu
            20                  25                  30
Gly Gln Lys Gly Val Glu Phe Ala Val Phe Pro Glu Thr Val Val Pro
        35                  40                  45
Tyr Tyr Pro Tyr Phe Ser Phe Val Gln Pro Pro Tyr Lys Leu Ala Thr
    50                  55                  60
Glu His Leu Arg Leu Leu Glu Glu Ser Val Thr Val Pro Ser Ala Glu
65                  70                  75                  80
Thr Asp Ala Ile Gly Asp Ala Ala Arg Lys Ala Asn Met Val Val Ser
                85                  90                  95
Ile Gly Val Asn Glu Arg Asp Gly Gly Thr Ile Tyr Asn Thr Gln Leu
            100                 105                 110
Leu Phe Asp Ala Asp Gly Thr Leu Ile Gln Arg Arg Arg Lys Ile Thr
        115                 120                 125
Pro Thr Tyr His Glu Arg Met Ile Trp Gly Gln Gly Asp Gly Ser Gly
    130                 135                 140
Leu Arg Ala Val Asp Ser Val Val Gly Arg Ile Gly Gln Leu Ala Cys

145                 150                 155                 160
Trp Glu His Tyr Gln Pro Leu Ala Arg Tyr Ala Leu Ile Ala Asp Gly
                165                 170                 175
Glu Gln Ile His Ala Ala Met Tyr Pro Gly Ala Phe Gly Gly Asp Leu
            180                 185                 190
Phe Ala Glu Gln Ile Glu Val Asn Val Arg Gln His Ala Leu Glu Ser
        195                 200                 205
Ala Ser Phe Val Val Ser Ala Thr Ala Trp Leu Asp Ala Asp Gln Gln
    210                 215                 220
Ala Gln Ile Ala Lys Asp Thr Gly Gly Pro Val Gln Ala Ile Ser Gly
225                 230                 235                 240
Gly Phe Phe Thr Ala Ile Ile Asp Pro Asp Gly Arg Ile Ile Gly Glu
                245                 250                 255
Pro Ile Thr Ser Gly Glu Gly Glu Val Ile Ala Asp Leu Asp Phe Ala
            260                 265                 270
Leu Ile Asp Arg Arg Lys Arg Leu Met Asp Ala Ser Gly His Tyr Ser
        275                 280                 285
Arg Pro Glu Leu Leu Ser Leu Gln Ile Asp Arg Thr Pro Ala Pro Ala
    290                 295                 300
Val His Asp Arg Asn Arg Gln Gly Ser Ser Ser Ala Pro Ala Thr Glu
305                 310                 315                 320
Lys Gly Arg Ser Ala Glu Ala Lys
                325

<210> 301
<211> 1032
<212> DNA
<213> Unknown

<220>
<223> Obtained from an environmental sample

<400> 301
atgaccacgc ccgcgccgtt caccgccgcc gtcgtccagg cgtcgcccgt gttcctcgac      60
cgcgatgcca cggtcgataa ggcgtgcgcc ctgatcaccg cggccggagc gcgcggcgcg     120
aagctcgtcg tgctgcccga gaccttcgtg cccgcatacc ccgcgtgggt ctggtacctg     180
ccgctcacgc gtcggcccga cgtcgcgaag ctctaccgcg cgctcgtcga gaacgcgatc     240
gacgtgccgg gtccggaaac cgagcgcctc gggcgcgcgg cgcgtgacgc gggcgcgtgg     300
gtggcgatcg gtgtcaacga gcgcaacgcg aaggcgagcc gcacgtcgct ctacaacacc     360
gtgctcctgt tcgacgacca gggcacgctg gtcgagtcgc ggcgcaagct gatgccgacc     420
ggcggcgagc gcctggtgtg gacgccgggc gagccggtgc cgttgcgggt tcacgacacg     480
ccgctcggcc gcgtcggcgc gctgatctgc tgggagaact acatgccgct cgcgcgcttc     540
gcgctctacg agcagggcgt ccagatctat ctcgcgccga cctgggacta cagcgaggcg     600
tggctcgcgt ccatgcggca cgtcgcgcgt gaaggtcgca cgtgggtgat cgggtgcagc     660
caggcggtgc ggcgtgacga gatcccggac cacctgccgt tcaaggacgc gatccccgag     720
agcctcgagt ggatcaacgc cgggaacagc atcgtcgtcg atccggacgg cgcggtggtc     780
gcgggaccgc tggcgcgggc gcacgacacg ctctacgtcg agatcgatcc gggccgcgcc     840
gccggctccc gatggatctt cgacgccgcc ggtcactatc accgtcccga cctgttccat     900
ttcgcgatgc gcgcgccggt ggcgccagag accgcgccgg ttgtgcggaa gcgcggccgg     960
cggctagcct cggccccggc gcggaagcca cggaaacggc cggcgcgtcc cacccgaagg    1020
aggactcgat ga                                                        1032

<210> 302
<211> 335
<212> PRT
<213> Unknown

<220>
<223> Obtained from an environmental sample

<400> 302
Met Thr Thr Pro Ala Pro Phe Thr Ala Ala Val Val Gln Ala Ser Pro
1               5                   10                  15
Val Phe Leu Asp Arg Asp Ala Thr Val Asp Lys Ala Cys Ala Leu Ile
            20                  25                  30
Thr Ala Ala Gly Ala Arg Gly Ala Lys Leu Val Val Leu Pro Glu Thr
        35                  40                  45
Phe Val Pro Ala Tyr Pro Ala Trp Val Trp Tyr Leu Pro Leu Thr Arg

50                  55                  60
Arg Pro Asp Val Ala Lys Leu Tyr Arg Ala Leu Val Glu Asn Ala Ile
65                  70                  75                  80
Asp Val Pro Gly Pro Glu Thr Glu Arg Leu Gly Arg Ala Ala Arg Asp
                85                  90                  95
Ala Gly Ala Trp Val Ala Ile Gly Val Asn Glu Arg Asn Ala Lys Ala
            100                 105                 110
Ser Arg Thr Ser Leu Tyr Asn Thr Val Leu Leu Phe Asp Asp Gln Gly
        115                 120                 125
Thr Leu Val Glu Ser Arg Arg Lys Leu Met Pro Thr Gly Gly Glu Arg
    130                 135                 140
Leu Val Trp Thr Pro Gly Glu Pro Val Pro Leu Arg Val His Asp Thr
145                 150                 155                 160
Pro Leu Gly Arg Val Gly Ala Leu Ile Cys Trp Glu Asn Tyr Met Pro
                165                 170                 175
Leu Ala Arg Phe Ala Leu Tyr Glu Gln Gly Val Gln Ile Tyr Leu Ala
            180                 185                 190
Pro Thr Trp Asp Tyr Ser Glu Ala Trp Leu Ala Ser Met Arg His Val
        195                 200                 205
Ala Arg Glu Gly Arg Thr Trp Val Ile Gly Cys Ser Gln Ala Val Arg
    210                 215                 220
Arg Asp Glu Ile Pro Asp His Leu Pro Phe Lys Asp Ala Ile Pro Glu
225                 230                 235                 240
Ser Leu Glu Trp Ile Asn Ala Gly Asn Ser Ile Val Val Asp Pro Asp
                245                 250                 255
Gly Ala Val Val Ala Gly Pro Leu Ala Arg Ala His Asp Thr Leu Tyr
            260                 265                 270
Val Glu Ile Asp Pro Gly Arg Ala Ala Gly Ser Arg Trp Ile Phe Asp
        275                 280                 285
Ala Ala Gly His Tyr His Arg Pro Asp Leu Phe His Phe Ala Met Arg
    290                 295                 300
Ala Pro Val Ala Pro Glu Thr Ala Pro Val Val Arg Lys Arg Gly Arg
305                 310                 315                 320
Arg Leu Ala Ser Ala Pro Ala Arg Lys Pro Arg Lys Arg Pro Ala
                325                 330                 335

<210> 303
<211> 1011
<212> DNA
<213> Unknown

<220>
<223> Obtained from an environmental sample

<400> 303
atgggcatcg ttcaccccaa gtacaaagtc gctgtcgttc aggctgcccc tgtatggcta      60
gacctggagg ccactgttga taaatgcatt cagttgattg aagaagcagc cagcaagggc     120
tgcaagctca tcgccttccc tgagaccttc attcccggat acccttggta tatctggatg     180
ggaacgcctg cctggactat tcagcgcggc tttgtacagc gctattttga taattctctg     240
tcttatgaca gtccgcaagc ggagaagcta aggcaggcag tcaaaaaggc tgagatcacg     300
gccgtactag gtctttctga gcgcagcggc ggtagcttgt acattgcaca atggaccatt     360
ggccctgacg gagaaaccat acacaaacgc agaaaagtgc gtccaacgca tggtgagcgt     420
acggtatttg gcgacggtga cggtagtgat cttgcggtgc acgatacccc cctggggcgg     480
ctgggcgcgc ttgcgtgctg ggagaacata ctgtcactga acaagtatgc gatgtattca     540
cagaatgagc aggtgcacgt agccgcttgg cctagcttct cggtctacga gcctttcgcc     600
catgcattgg gttgggaggt caataacgca gtcagcaagg tttacgcggt agaaggcggc     660
tgttttgtat tggcgccttg cgcagtggtc tccgaggaaa tgatcgaagc actgtgcgat     720
acacccgata agcaccaact ggctcatgcg ggtggagggc atgctgtcat ttacggacca     780
gatggcagtc ctctggcaga taagttaccc gaaggagagg aggggctatt aattgcagaa     840
attgatctcg gtctcatcag cttggcgaaa aatgccatgg acccggtggg gcattactct     900
cgacctgacg tacatcgctt gctattgaat cgcaatccag caaagcgggt tgaggaattt     960
tctctgccca ttgatttggc agagacaact ccgccaatat taggcacgta g             1011

<210> 304
<211> 336
<212> PRT
<213> Unknown

<220>
<223> Obtained from an environmental sample

<400> 304
Met Gly Ile Val His Pro Lys Tyr Lys Val Ala Val Val Gln Ala Ala
1               5                   10                  15
Pro Val Trp Leu Asp Leu Glu Ala Thr Val Asp Lys Cys Ile Gln Leu
            20                  25                  30
Ile Glu Glu Ala Ala Ser Lys Gly Cys Lys Leu Ile Ala Phe Pro Glu
        35                  40                  45
Thr Phe Ile Pro Gly Tyr Pro Trp Tyr Ile Trp Met Gly Thr Pro Ala
    50                  55                  60
Trp Thr Ile Gln Arg Gly Phe Val Gln Arg Tyr Phe Asp Asn Ser Leu
65                  70                  75                  80
Ser Tyr Asp Ser Pro Gln Ala Glu Lys Leu Arg Gln Ala Val Lys Lys
                85                  90                  95
Ala Glu Ile Thr Ala Val Leu Gly Leu Ser Glu Arg Ser Gly Gly Ser
            100                 105                 110
Leu Tyr Ile Ala Gln Trp Thr Ile Gly Pro Asp Gly Glu Thr Ile His
        115                 120                 125
Lys Arg Arg Lys Val Arg Pro Thr His Gly Glu Arg Thr Val Phe Gly
    130                 135                 140
Asp Gly Asp Gly Ser Asp Leu Ala Val His Asp Thr Pro Leu Gly Arg
145                 150                 155                 160
Leu Gly Ala Leu Ala Cys Trp Glu Asn Ile Leu Ser Leu Asn Lys Tyr
                165                 170                 175
Ala Met Tyr Ser Gln Asn Glu Gln Val His Val Ala Ala Trp Pro Ser
            180                 185                 190
Phe Ser Val Tyr Glu Pro Phe Ala His Ala Leu Gly Trp Glu Val Asn
        195                 200                 205
Asn Ala Val Ser Lys Val Tyr Ala Val Glu Gly Gly Cys Phe Val Leu
    210                 215                 220
Ala Pro Cys Ala Val Val Ser Glu Glu Met Ile Glu Ala Leu Cys Asp
225                 230                 235                 240
Thr Pro Asp Lys His Gln Leu Ala His Ala Gly Gly Gly His Ala Val
                245                 250                 255
Ile Tyr Gly Pro Asp Gly Ser Pro Leu Ala Asp Lys Leu Pro Glu Gly
            260                 265                 270
Glu Glu Gly Leu Leu Ile Ala Glu Ile Asp Leu Gly Leu Ile Ser Leu
        275                 280                 285
Ala Lys Asn Ala Met Asp Pro Val Gly His Tyr Ser Arg Pro Asp Val
    290                 295                 300
His Arg Leu Leu Leu Asn Arg Asn Pro Ala Lys Arg Val Glu Glu Phe
305                 310                 315                 320
Ser Leu Pro Ile Asp Leu Ala Glu Thr Thr Pro Pro Ile Leu Gly Thr
                325                 330                 335

<210> 305
<211> 1068
<212> DNA
<213> Unknown

<220>
<223> Obtained from an environmental sample

<400> 305
atgacgatgc aacatcccaa gtttcgcgct gctgccgtgc aggcggcacc ggtcttcctt      60
gaccttgatg cgtcgataga taaagccatc gacctgatcg cgcaagccgc caaaggcggc     120
gcgcaattga ttgcctttcc ggaaacctgg ctgcccggct accccttttt catctggctc     180
gattcgccgg cctggggcat gcaattcatc cagcgctacc atgacaattc cctggtctac     240
ggcacaccgc aggccgagcg catcgcgcag gctgcgaaaa agcatcgcat catggtcgtc     300
atggggcaca gcgagcggga tcatggaagt ctgtacatcg ctcagtggat catcggtgcc     360
gatggggaaa cggttgcgac acgtcgtaag ctcaaaccga ctcatgccga gcgcaccttg     420
tttggggaag gcgatggcag tgacctgagc gtgttcgata caccgctggg aagggttggc     480
gcactatgct gctgggagca cctccagccc ctgtcgaaat acgcgctata cgcacagaat     540
gagcaggtcc acattgcttc ctggccaagc ttttccctgt atcgcggggg cgcctacgcg     600
ctcggcgccg aagtgaacaa tgcggccagc cagatttatg ctgtcgaagg ccagtgtttt     660
gtgatcgcgc cgtgcggggt cgtcacgaaa gaaatgctgg acgtgctgtg caccgacgaa     720

atgaaaaagc agttgttggt tgaaggcggc gggttcgcgc gaatttacgc gcccgatgga 780
cagatgatgc acgcgccgct ggcggaaaac gaagagggcc tggtgtatgc cgatctcgac 840
ctgggcatga tctcgctggc caaagtagtc gccgacccgg ccgggcatta tgcgcggccc 900
gacgtaaccc ggttactgct ggacaagact ccgggagacc gcgtcatgct ggcgagccgt 960
cgcggcaagg aggtcagccg cgctggtaac gacgagccgc aagtgctggt ctcgcgcaac 1020
gacacgctga cctcgccgaa gccagcgtct tcgcgcaaag caagctga 1068

<210> 306
<211> 355
<212> PRT
<213> Unknown

<220>
<223> Obtained from an environmental sample

<400> 306
Met Thr Met Gln His Pro Lys Phe Arg Ala Ala Ala Val Gln Ala Ala
1               5                   10                  15
Pro Val Phe Leu Asp Leu Asp Ala Ser Ile Asp Lys Ala Ile Asp Leu
            20                  25                  30
Ile Ala Gln Ala Ala Lys Gly Gly Ala Gln Leu Ile Ala Phe Pro Glu
        35                  40                  45
Thr Trp Leu Pro Gly Tyr Pro Phe Phe Ile Trp Leu Asp Ser Pro Ala
    50                  55                  60
Trp Gly Met Gln Phe Ile Gln Arg Tyr His Asp Asn Ser Leu Val Tyr
65                  70                  75                  80
Gly Thr Pro Gln Ala Glu Arg Ile Ala Gln Ala Ala Lys Lys His Arg
                85                  90                  95
Ile Met Val Val Met Gly His Ser Glu Arg Asp His Gly Ser Leu Tyr
            100                 105                 110
Ile Ala Gln Trp Ile Ile Gly Ala Asp Gly Glu Thr Val Ala Thr Arg
        115                 120                 125
Arg Lys Leu Lys Pro Thr His Ala Glu Arg Thr Leu Phe Gly Glu Gly
    130                 135                 140
Asp Gly Ser Asp Leu Ser Val Phe Asp Thr Pro Leu Gly Arg Val Gly
145                 150                 155                 160
Ala Leu Cys Cys Trp Glu His Leu Gln Pro Leu Ser Lys Tyr Ala Leu
                165                 170                 175
Tyr Ala Gln Asn Glu Gln Val His Ile Ala Ser Trp Pro Ser Phe Ser
            180                 185                 190
Leu Tyr Arg Gly Gly Ala Tyr Ala Leu Gly Ala Glu Val Asn Asn Ala
        195                 200                 205
Ala Ser Gln Ile Tyr Ala Val Glu Gly Gln Cys Phe Val Ile Ala Pro
    210                 215                 220
Cys Gly Val Val Thr Lys Glu Met Leu Asp Val Leu Cys Thr Asp Glu
225                 230                 235                 240
Met Lys Lys Gln Leu Leu Val Glu Gly Gly Gly Phe Ala Arg Ile Tyr
                245                 250                 255
Ala Pro Asp Gly Gln Met Met His Ala Pro Leu Ala Glu Asn Glu Glu
            260                 265                 270
Gly Leu Val Tyr Ala Asp Leu Asp Leu Gly Met Ile Ser Leu Ala Lys
        275                 280                 285
Val Val Ala Asp Pro Ala Gly His Tyr Ala Arg Pro Asp Val Thr Arg
    290                 295                 300
Leu Leu Leu Asp Lys Thr Pro Gly Asp Arg Val Met Leu Ala Ser Arg
305                 310                 315                 320
Arg Gly Lys Glu Val Ser Arg Ala Gly Asn Asp Glu Pro Gln Val Leu
                325                 330                 335
Val Ser Arg Asn Asp Thr Leu Thr Ser Pro Lys Pro Ala Ser Ser Arg
            340                 345                 350
Lys Ala Ser
        355

<210> 307
<211> 942
<212> DNA
<213> Unknown

<220>
<223> Obtained from an environmental sample

<400> 307
atggctgaac cggaatcctt tatcgtcgct gctgtacagg ctacaccgat ctttcttgac 60
cgccaggcaa cccttgagaa agcgtgcgac ctgattgccg aagctggcag caatggcgca 120
aaactcgttc tttttcccga agcctttatc cccacctatc ctgattggat atgggcggtg 180
acaggctcac aatctgcgct gctcgacgaa ctttatgtgg aactactgga aaactccgtg 240
accatccccg acgcgaccac tgagcaactt tgtgaagcag cacgtaacgc cggtctctac 300
gtcgtcatgg gagtgaatga gcgcaacgcc gaggcgagca acgccacact ctataacacc 360
ctgctctata ttgacgatca gggcaaaatt ctcggcaagc atcgcaaatt ggtcccgacc 420
gccctggagc gaatcgtctg gggctatggc gatggcagca cgcttgacgc ctttgaaacg 480
ccgctgggca agattggcgg gctgatctgt tgggaaaatt acatgccact ggcgcgccaa 540
acactttatg cctggggggt gcaaatttac ttggccgcaa cgtgggatcg cggcgaagtt 600
tggcaggcga ccatgcgcca tattgccagg gaaggcggcg tctatgtagt cgcctcctgt 660
attccatttc acatcaaaga cattcctgac cacatgcctg aaatccgcaa tctctatgca 720
ccggggacag actggatcaa cgtcggccaa agctgcatca tcaaccccag cggcgactat 780
attgcaggcc ctgtcgagtg tcgcgaggag attctttatg ccgaggtaaa tctgcgccag 840
agtgcggcgg caaaacgtat gttggatgtg gcgggccatt atggacgccc tgatgtcttt 900
cacctcaccg tcaaccgcac gcccaatccg catattcgat aa 942

<210> 308
<211> 313
<212> PRT
<213> Unknown

<220>
<223> Obtained from an environmental sample

<400> 308
Met Ala Glu Pro Glu Ser Phe Ile Val Ala Ala Val Gln Ala Thr Pro
1               5                   10                  15
Ile Phe Leu Asp Arg Gln Ala Thr Leu Glu Lys Ala Cys Asp Leu Ile
            20                  25                  30
Ala Glu Ala Gly Ser Asn Gly Ala Lys Leu Val Leu Phe Pro Glu Ala
        35                  40                  45
Phe Ile Pro Thr Tyr Pro Asp Trp Ile Trp Ala Val Thr Gly Ser Gln
    50                  55                  60
Ser Ala Leu Leu Asp Glu Leu Tyr Val Glu Leu Leu Glu Asn Ser Val
65                  70                  75                  80
Thr Ile Pro Asp Ala Thr Thr Glu Gln Leu Cys Glu Ala Ala Arg Asn
                85                  90                  95
Ala Gly Leu Tyr Val Val Met Gly Val Asn Glu Arg Asn Ala Glu Ala
            100                 105                 110
Ser Asn Ala Thr Leu Tyr Asn Thr Leu Leu Tyr Ile Asp Asp Gln Gly
        115                 120                 125
Lys Ile Leu Gly Lys His Arg Lys Leu Val Pro Thr Ala Leu Glu Arg
    130                 135                 140
Ile Val Trp Gly Tyr Gly Asp Gly Ser Thr Leu Asp Ala Phe Glu Thr
145                 150                 155                 160
Pro Leu Gly Lys Ile Gly Gly Leu Ile Cys Trp Glu Asn Tyr Met Pro
                165                 170                 175
Leu Ala Arg Gln Thr Leu Tyr Ala Trp Gly Val Gln Ile Tyr Leu Ala
            180                 185                 190
Ala Thr Trp Asp Arg Gly Glu Val Trp Gln Ala Thr Met Arg His Ile
        195                 200                 205
Ala Arg Glu Gly Gly Val Tyr Val Val Ala Ser Cys Ile Pro Phe His
    210                 215                 220
Ile Lys Asp Ile Pro Asp His Met Pro Glu Ile Arg Asn Leu Tyr Ala
225                 230                 235                 240
Pro Gly Thr Asp Trp Ile Asn Val Gly Gln Ser Cys Ile Ile Asn Pro
                245                 250                 255
Ser Gly Asp Tyr Ile Ala Gly Pro Val Glu Cys Arg Glu Glu Ile Leu
            260                 265                 270
Tyr Ala Glu Val Asn Leu Arg Gln Ser Ala Ala Ala Lys Arg Met Leu
        275                 280                 285
Asp Val Ala Gly His Tyr Gly Arg Pro Asp Val Phe His Leu Thr Val
    290                 295                 300

Asn Arg Thr Pro Asn Pro His Ile Arg
305                 310

<210> 309
<211> 951
<212> DNA
<213> Unknown

<220>
<223> Obtained from an environmental sample

<400> 309
ttgaccgact gtctcaaaat agccgccgcc caaatcactc cggtcttcct cgaccgcgtt      60
gcgaccacga agaaggtcgt cgaaaccatc gaaaaagcgg ccgccggcgg tgcgcggctg     120
gtcgcattcg gcgaagcgct gttgcccgcc tatccattgt ggctgacgcg caccgacgcc     180
gcgcggttca attccgacgt gcaaaaaaac ttgcacgcga tctatctcaa gcaatccgtc     240
tcgatagcag gcggtcacct atctccgatt tgcaaaatcg caagcgaacg caagattgcc     300
gtcatcctcg gcatcgccga gcgcgcgacc gaccggggcg accacaccat ttactgctcg     360
tgcgtgttca tcgatgccga cggccgaatc gcgtcggtcc atcgcaagct gatgccgaca     420
tacgaagaac gcctcagttg gggattcggc gacggtgcgg gactcgtcac gcatccggtc     480
gggccgttca cggtgggcgc gttgaactgc tgggaaaact ggatgcccct cgcgcgcacc     540
gcgctgtatg ccggcggaga agatttgcac gttgcgatct ggcccggcgg atcggtgctc     600
acggaagaca tcacgcgctt catcgcacgc gagtcgcgct cgttcgtcct gtccgtcagc     660
ggcatcattc gcgaaagcga catccccagc ggggtcccct atcgcgatga aatgtgtgcg     720
aaaggcgaaa ccatctacaa cggcggaagc tgcatcgccg gacccgacgg tcagtggatc     780
atcgcgcccg taaccgaccg tgaagagttg atcttcgcgg agatcgacca cgaacacgtc     840
cgccgcgagc ggcagaattt cgacccggcc gggcattacg cgcggcccga tgtgttgcaa     900
ataaccgttg atcgtcgacg acaaacagcg gcgaatttta ttgatgacta a              951

<210> 310
<211> 316
<212> PRT
<213> Unknown

<220>
<223> Obtained from an environmental sample

<400> 310
Leu Thr Asp Cys Leu Lys Ile Ala Ala Ala Gln Ile Thr Pro Val Phe
1               5                   10                  15
Leu Asp Arg Val Ala Thr Thr Lys Lys Val Val Glu Thr Ile Glu Lys
            20                  25                  30
Ala Ala Ala Gly Gly Ala Arg Leu Val Ala Phe Gly Glu Ala Leu Leu
        35                  40                  45
Pro Ala Tyr Pro Leu Trp Leu Thr Arg Thr Asp Ala Ala Arg Phe Asn
    50                  55                  60
Ser Asp Val Gln Lys Asn Leu His Ala Ile Tyr Leu Lys Gln Ser Val
65                  70                  75                  80
Ser Ile Ala Gly Gly His Leu Ser Pro Ile Cys Lys Ile Ala Ser Glu
                85                  90                  95
Arg Lys Ile Ala Val Ile Leu Gly Ile Ala Glu Arg Ala Thr Asp Arg
            100                 105                 110
Gly Asp His Thr Ile Tyr Cys Ser Cys Val Phe Ile Asp Ala Asp Gly
        115                 120                 125
Arg Ile Ala Ser Val His Arg Lys Leu Met Pro Thr Tyr Glu Glu Arg
    130                 135                 140
Leu Ser Trp Gly Phe Gly Asp Gly Ala Gly Leu Val Thr His Pro Val
145                 150                 155                 160
Gly Pro Phe Thr Val Gly Ala Leu Asn Cys Trp Glu Asn Trp Met Pro
                165                 170                 175
Leu Ala Arg Thr Ala Leu Tyr Ala Gly Gly Glu Asp Leu His Val Ala
            180                 185                 190
Ile Trp Pro Gly Gly Ser Val Leu Thr Glu Asp Ile Thr Arg Phe Ile
        195                 200                 205
Ala Arg Glu Ser Arg Ser Phe Val Leu Ser Val Ser Gly Ile Ile Arg
    210                 215                 220
Glu Ser Asp Ile Pro Ser Gly Val Pro Tyr Arg Asp Glu Met Cys Ala
225                 230                 235                 240

Lys Gly Glu Thr Ile Tyr Asn Gly Gly Ser Cys Ile Ala Gly Pro Asp
                245                 250                 255
Gly Gln Trp Ile Ile Ala Pro Val Thr Asp Arg Glu Glu Leu Ile Phe
            260                 265                 270
Ala Glu Ile Asp His Glu His Val Arg Arg Glu Arg Gln Asn Phe Asp
        275                 280                 285
Pro Ala Gly His Tyr Ala Arg Pro Asp Val Leu Gln Ile Thr Val Asp
    290                 295                 300
Arg Arg Arg Gln Thr Ala Ala Asn Phe Ile Asp Asp
305                 310                 315

<210> 311
<211> 1011
<212> DNA
<213> Unknown

<220>
<223> Obtained from an environmental sample

<400> 311
atgtcagaaa agcgaataat cagagcagct gcagttcaga tcacacctga atttgactca      60
gcagatggaa cagttaagaa ggtatgcaag gtaatcgatg aagcaggcgc aaagggtgta     120
caaattatag tattccccga aaccttcatt ccgtattacc catacttctc attcattacc     180
cccccagttt ctgctggcgc tgagcatttg aagctttatg aaaaaagtgt cgtgatacct     240
ggtcctgtca ctcaagcgat cgccgagaga gctagagtga atcaaatggt cgtcgtactc     300
ggtgtaaacg agagagataa cggtagcctc tataacactc aactgatctt cgataccaat     360
ggtgagttga tgttgaagag aagaaaaatc actcctacat atcatgagcg catgatctgg     420
ggacaaggtg atgcttcagg cttaaaagta gttgaaacga gcattgcccg ggtaggtgct     480
ctagcttgct gggaacatta caacccgctg gccagatatt ctctcatgac acagcatgaa     540
gaaattcact gcgcacaatt cccaggttct atggttggcc aaatatttgc cgaccaaatg     600
gatgtcacta tcagacatca cgcattggaa tctggctgtt tcgtcattaa tgccaccggc     660
tggctcacag acgagcaaat ccagtccatt acagatgacc caaaaatgca gaaagcatta     720
cgtggcggct gcaacacagc aatcatttct cccgaaggtg tgcacttaac agagccctta     780
cgtgaaggtg aaggcatttt gattgctgac ctggacatgt cactcatcac aaaacgaaaa     840
agaatgatgg attcagtagg tcattattca agacctgaac tattaagtct ggcgatcaat     900
gacaagccag caacaacaaa attttcaatg actgaggggt gtactcaaac tgagcaattt     960
cgaatcgcag aggagttgaa aaatgacgac aagcttagca ccggaaacta a             1011

<210> 312
<211> 336
<212> PRT
<213> Unknown

<220>
<223> Obtained from an environmental sample

<400> 312
Met Ser Glu Lys Arg Ile Ile Arg Ala Ala Ala Val Gln Ile Thr Pro
1               5                   10                  15
Glu Phe Asp Ser Ala Asp Gly Thr Val Lys Lys Val Cys Lys Val Ile
            20                  25                  30
Asp Glu Ala Gly Ala Lys Gly Val Gln Ile Ile Val Phe Pro Glu Thr
        35                  40                  45
Phe Ile Pro Tyr Tyr Pro Tyr Phe Ser Phe Ile Thr Pro Pro Val Ser
    50                  55                  60
Ala Gly Ala Glu His Leu Lys Leu Tyr Glu Lys Ser Val Val Ile Pro
65                  70                  75                  80
Gly Pro Val Thr Gln Ala Ile Ala Glu Arg Ala Arg Val Asn Gln Met
                85                  90                  95
Val Val Val Leu Gly Val Asn Glu Arg Asp Asn Gly Ser Leu Tyr Asn
            100                 105                 110
Thr Gln Leu Ile Phe Asp Thr Asn Gly Glu Leu Met Leu Lys Arg Arg
        115                 120                 125
Lys Ile Thr Pro Thr Tyr His Glu Arg Met Ile Trp Gly Gln Gly Asp
    130                 135                 140
Ala Ser Gly Leu Lys Val Val Glu Thr Ser Ile Ala Arg Val Gly Ala
145                 150                 155                 160
Leu Ala Cys Trp Glu His Tyr Asn Pro Leu Ala Arg Tyr Ser Leu Met

                    165                     170                     175
Thr Gln His Glu Glu Ile His Cys Ala Gln Phe Pro Gly Ser Met Val
                180                     185                     190
Gly Gln Ile Phe Ala Asp Gln Met Asp Val Thr Ile Arg His His Ala
            195                     200                     205
Leu Glu Ser Gly Cys Phe Val Ile Asn Ala Thr Gly Trp Leu Thr Asp
    210                     215                     220
Glu Gln Ile Gln Ser Ile Thr Asp Asp Pro Lys Met Gln Lys Ala Leu
225                     230                     235                     240
Arg Gly Gly Cys Asn Thr Ala Ile Ile Ser Pro Glu Gly Val His Leu
                245                     250                     255
Thr Glu Pro Leu Arg Glu Gly Glu Gly Ile Leu Ile Ala Asp Leu Asp
                260                     265                     270
Met Ser Leu Ile Thr Lys Arg Lys Arg Met Met Asp Ser Val Gly His
            275                     280                     285
Tyr Ser Arg Pro Glu Leu Leu Ser Leu Ala Ile Asn Asp Lys Pro Ala
    290                     295                     300
Thr Thr Lys Phe Ser Met Thr Glu Gly Cys Thr Gln Thr Glu Gln Phe
305                     310                     315                     320
Arg Ile Ala Glu Glu Leu Lys Asn Asp Asp Lys Leu Ser Thr Gly Asn
                325                     330                     335

<210> 313
<211> 987
<212> DNA
<213> Unknown

<220>
<223> Obtained from an environmental sample

<400> 313
atggcggccg ttcaggctgc gccggttccg tttgatgctg aggcttcggt ggataaggcc      60
tgtcgcttaa ttcaagaagc tgcagccaaa ggcgcagata tagttgcttt cggtgaggca     120
tggctacccg gctaccccta ttttgcctgg ttaccccaag taacaccaga gtggtatagt     180
gcggctgccg attatcttgc cagctctgtt gatatccctg gcccgatcac cgataaactt     240
tgccaggctg cccgtcgtgc atcggttgaa ctcatcatgg gtgtggtaga acgcagcaag     300
tctcagggaa ccacctattg cacgcttctt tttattagca aggatggcga aataattggc     360
aagcaccgca agctgaagcc cacactcgcc gaacgaaccg tctggggtga aggcgatgcc     420
agcggactga gggttcacga tcggcctatt gccagaatca gtgggctctc ctgctgggag     480
aacaaaatga tgctgccagg ttacgcactg atggcgcagg gtacgcaggt gcatgtctcc     540
gcctggccag ggatcccaga ggattcaccc atggaggtgc ctgcacaccc ccgtcaaaag     600
ctgctttccc aagcctttgc actgcaaggc gggtgctatg ttatttctcc ctccattgtc     660
cttagggcag aagatgtgcc cgagaaacac gccgctctac tgatgggaga ccaagtgggc     720
ggtagctata tcattgaccc ctgcggaaaa gtgatcgccg aggccggtgc gggtgagact     780
atcctgattg ccaaaggcaa ccttgacctc gtaagggccg ccaaaatggc cagtgatgta     840
ggggggtctt attcacgccc ggatcttttg cagttgatga tcaataaccg accactcgaa     900
cagctgattg agttcagtgc tgaaggtgca ggtaggggga atctagtatc caactcaccc     960
gaggtgtcag aacaagaagg tgagtaa                                         987

<210> 314
<211> 328
<212> PRT
<213> Unknown

<220>
<223> Obtained from an environmental sample

<400> 314
Met Ala Ala Val Gln Ala Ala Pro Val Pro Phe Asp Ala Glu Ala Ser
1               5                   10                  15
Val Asp Lys Ala Cys Arg Leu Ile Gln Glu Ala Ala Ala Lys Gly Ala
            20                  25                  30
Asp Ile Val Ala Phe Gly Glu Ala Trp Leu Pro Gly Tyr Pro Tyr Phe
        35                  40                  45
Ala Trp Leu Pro Gln Val Thr Pro Glu Trp Tyr Ser Ala Ala Ala Asp
    50                  55                  60
Tyr Leu Ala Ser Ser Val Asp Ile Pro Gly Pro Ile Thr Asp Lys Leu
65                  70                  75                  80

Cys Gln Ala Ala Arg Arg Ala Ser Val Glu Leu Ile Met Gly Val Val
                85                  90                  95
Glu Arg Ser Lys Ser Gln Gly Thr Thr Tyr Cys Thr Leu Leu Phe Ile
            100                 105                 110
Ser Lys Asp Gly Glu Ile Ile Gly Lys His Arg Lys Leu Lys Pro Thr
        115                 120                 125
Leu Ala Glu Arg Thr Val Trp Gly Glu Gly Asp Ala Ser Gly Leu Arg
    130                 135                 140
Val His Asp Arg Pro Ile Ala Arg Ile Ser Gly Leu Ser Cys Trp Glu
145                 150                 155                 160
Asn Lys Met Met Leu Pro Gly Tyr Ala Leu Met Ala Gln Gly Thr Gln
                165                 170                 175
Val His Val Ser Ala Trp Pro Gly Ile Pro Glu Asp Ser Pro Met Glu
            180                 185                 190
Val Pro Ala His Pro Arg Gln Lys Leu Leu Ser Gln Ala Phe Ala Leu
        195                 200                 205
Gln Gly Gly Cys Tyr Val Ile Ser Pro Ser Ile Val Leu Arg Ala Glu
    210                 215                 220
Asp Val Pro Glu Lys His Ala Ala Leu Leu Met Gly Asp Gln Val Gly
225                 230                 235                 240
Gly Ser Tyr Ile Ile Asp Pro Cys Gly Lys Val Ile Ala Glu Ala Gly
                245                 250                 255
Ala Gly Glu Thr Ile Leu Ile Ala Lys Gly Asn Leu Asp Leu Val Arg
            260                 265                 270
Ala Ala Lys Met Ala Ser Asp Val Gly Gly Ser Tyr Ser Arg Pro Asp
        275                 280                 285
Leu Leu Gln Leu Met Ile Asn Asn Arg Pro Leu Glu Gln Leu Ile Glu
    290                 295                 300
Phe Ser Ala Glu Gly Ala Gly Arg Gly Asn Leu Val Ser Asn Ser Pro
305                 310                 315                 320
Glu Val Ser Glu Gln Glu Gly Glu
                325

<210> 315
<211> 960
<212> DNA
<213> Unknown

<220>
<223> Obtained from an environmental sample

<400> 315
atgacaatgc ttaagaccaa attccgggtt gctgcggtgc aggcagcgcc tgtattcctg      60
gatcgagagg ctacgctgga gaaagcctgc ggactgattg aggaggcggg tcgcaacggg     120
gccagcctgg tggtctttcc tgagtcgttc attccggcct atcccgattg ggtctgggct     180
gtgccggcgg gtgaagaagc tttacttaat gaactgtatg ctcaactgct ggccaacgcc     240
gttgaaattc ccagcccggc caccgaacgc ttgagccagg cagcgaaaaa ggctaaagtc     300
catgtggtta tgggcctgac cgaacgcaac agcgaggcca gcggcggcag cctctacaat     360
accttgctct atcttgaccc acagggccaa attctgggca aacatcgcaa gctggtgccc     420
accggcggcg agcggctggt ttgggcccag ggcgacggca gtaccctgca agtctacgag     480
acccccttgg gtaaactcag cggcttgatt tgctgggaaa attatatgcc gctggcccgc     540
tacgcgctct atgcctgggg tacgcaaatc tatatcgccg ccacctggga tcgaggcgag     600
ccgtggcttt cgacgctgcg gcatatcgcc aaagagggcc gggtgtttgt catcggctgt     660
ggcatggcct tgcgtaaggc tgatattccc gaccatttgg aattcaagca gcgcttttat     720
caaaatgccg gcgagtggat caatggaggc gacagcgcca ttgtcaatcc tgagggtgaa     780
tttattgctg gacccctgag cgagcaggaa ggtattttgt acgccgagat tgatcctggc     840
cagatggccg gaccaaaatg gatgctcgat gtggccgggc actatgctcg cccggatgtc     900
tttgaactga ccgtccagac cgtagctcgg cccatgatta cctctactca gccgcgatag     960

<210> 316
<211> 319
<212> PRT
<213> Unknown

<220>
<223> Obtained from an environmental sample

<400> 316

Met Thr Met Leu Lys Thr Lys Phe Arg Val Ala Ala Val Gln Ala Ala
1               5                   10                  15
Pro Val Phe Leu Asp Arg Glu Ala Thr Leu Glu Lys Ala Cys Gly Leu
            20                  25                  30
Ile Glu Glu Ala Gly Arg Asn Gly Ala Ser Leu Val Val Phe Pro Glu
        35                  40                  45
Ser Phe Ile Pro Ala Tyr Pro Asp Trp Val Trp Ala Val Pro Ala Gly
    50                  55                  60
Glu Glu Ala Leu Leu Asn Glu Leu Tyr Ala Gln Leu Leu Ala Asn Ala
65                  70                  75                  80
Val Glu Ile Pro Ser Pro Ala Thr Glu Arg Leu Ser Gln Ala Ala Lys
                85                  90                  95
Lys Ala Lys Val His Val Val Met Gly Leu Thr Glu Arg Asn Ser Glu
            100                 105                 110
Ala Ser Gly Gly Ser Leu Tyr Asn Thr Leu Leu Tyr Leu Asp Pro Gln
        115                 120                 125
Gly Gln Ile Leu Gly Lys His Arg Lys Leu Val Pro Thr Gly Gly Glu
    130                 135                 140
Arg Leu Val Trp Ala Gln Gly Asp Gly Ser Thr Leu Gln Val Tyr Glu
145                 150                 155                 160
Thr Pro Leu Gly Lys Leu Ser Gly Leu Ile Cys Trp Glu Asn Tyr Met
                165                 170                 175
Pro Leu Ala Arg Tyr Ala Leu Tyr Ala Trp Gly Thr Gln Ile Tyr Ile
            180                 185                 190
Ala Ala Thr Trp Asp Arg Gly Glu Pro Trp Leu Ser Thr Leu Arg His
        195                 200                 205
Ile Ala Lys Glu Gly Arg Val Phe Val Ile Gly Cys Gly Met Ala Leu
    210                 215                 220
Arg Lys Ala Asp Ile Pro Asp His Phe Glu Phe Lys Gln Arg Phe Tyr
225                 230                 235                 240
Gln Asn Ala Gly Glu Trp Ile Asn Gly Gly Asp Ser Ala Ile Val Asn
                245                 250                 255
Pro Glu Gly Glu Phe Ile Ala Gly Pro Leu Ser Glu Gln Glu Gly Ile
            260                 265                 270
Leu Tyr Ala Glu Ile Asp Pro Gly Gln Met Ala Gly Pro Lys Trp Met
        275                 280                 285
Leu Asp Val Ala Gly His Tyr Ala Arg Pro Asp Val Phe Glu Leu Thr
    290                 295                 300
Val Gln Thr Val Ala Arg Pro Met Ile Thr Ser Thr Gln Pro Arg
305                 310                 315

<210> 317
<211> 993
<212> DNA
<213> Unknown

<220>
<223> Obtained from an environmental sample

<400> 317
atgaccattg taaaagccgc tgccgtccaa ataagccctg tcctttacag ccgcgaaggc      60
accgtggaca aggttgtcca gaagatcctc gaactcggca agcaaggcgt ccagttcgcc     120
actttcccgg agacggtggt cccctactac ccctacttct ccttcgtcca gtcgggctac     180
gccctcaagg tgggcaagga acatctgcgc ttgctcgaac agtcggtcac cgtgccatcg     240
gccaccacgc tcgccatcgg cgaagcctgc aagcaggcgg ggatggtggt gtccatcggc     300
gtcaacgaac gcgacggcag cacgatctac aacacgcagc tgctcttcga tgccgacggc     360
accttgattc agcgccgccg aaagatcagc ccgaccttcc atgaacgcat ggtctggggc     420
cagggcgacg gctccgggct gcgcgcggtc gacagcgcgg tcgggcgcat cggccagttg     480
gcgtgctggg agcactacaa cccgctggcc cgctacgcca tgatggccga cggcgagcag     540
atccactcgg cgatgtaccc cggttccttc gcaggcgacg ccttctccga acagatccag     600
gtcaacatcc gccagcacgc attggaagcc ggctgcttcg tcgtgaacgc caccgcgtgg     660
ctggacgccg atcagcaggc gcagatcatg caggacaccg gttgcgccat cggcccgatc     720
tccagtggtt gcttcaccgc catcgtttcg ccggacggcg tgttgctggg cgagcctctg     780
cggtccggtg agggcgaggt gattgccgat ctcgacttca cgctgatcga caagcgcaag     840
cagatgatgg attcacgcgg gcactatgcg cgcccggaat tgctcagcct gttgatcgac     900
cgcacggcga ccgcgcatgt gcatgagcgc agcgcgcatc cgaaggcgac tgccgagcag     960
gcggacggtc catccgccgt gaatgcgcag taa                                  993

<210> 318
<211> 330
<212> PRT
<213> Unknown

<220>
<223> Obtained from an environmental sample

<400> 318
Met Thr Ile Val Lys Ala Ala Ala Val Gln Ile Ser Pro Val Leu Tyr
1               5                   10                  15
Ser Arg Glu Gly Thr Val Asp Lys Val Val Gln Lys Ile Leu Glu Leu
            20                  25                  30
Gly Lys Gln Gly Val Gln Phe Ala Thr Phe Pro Glu Thr Val Val Pro
        35                  40                  45
Tyr Tyr Pro Tyr Phe Ser Phe Val Gln Ser Gly Tyr Ala Leu Lys Val
    50                  55                  60
Gly Lys Glu His Leu Arg Leu Leu Glu Gln Ser Val Thr Val Pro Ser
65                  70                  75                  80
Ala Thr Thr Leu Ala Ile Gly Glu Ala Cys Lys Gln Ala Gly Met Val
                85                  90                  95
Val Ser Ile Gly Val Asn Glu Arg Asp Gly Ser Thr Ile Tyr Asn Thr
            100                 105                 110
Gln Leu Leu Phe Asp Ala Asp Gly Thr Leu Ile Gln Arg Arg Arg Lys
        115                 120                 125
Ile Ser Pro Thr Phe His Glu Arg Met Val Trp Gly Gln Gly Asp Gly
    130                 135                 140
Ser Gly Leu Arg Ala Val Asp Ser Ala Val Gly Arg Ile Gly Gln Leu
145                 150                 155                 160
Ala Cys Trp Glu His Tyr Asn Pro Leu Ala Arg Tyr Ala Met Met Ala
                165                 170                 175
Asp Gly Glu Gln Ile His Ser Ala Met Tyr Pro Gly Ser Phe Ala Gly
            180                 185                 190
Asp Ala Phe Ser Glu Gln Ile Gln Val Asn Ile Arg Gln His Ala Leu
        195                 200                 205
Glu Ala Gly Cys Phe Val Val Asn Ala Thr Ala Trp Leu Asp Ala Asp
    210                 215                 220
Gln Gln Ala Gln Ile Met Gln Asp Thr Gly Cys Ala Ile Gly Pro Ile
225                 230                 235                 240
Ser Ser Gly Cys Phe Thr Ala Ile Val Ser Pro Asp Gly Val Leu Leu
                245                 250                 255
Gly Glu Pro Leu Arg Ser Gly Glu Gly Glu Val Ile Ala Asp Leu Asp
            260                 265                 270
Phe Thr Leu Ile Asp Lys Arg Lys Gln Met Met Asp Ser Arg Gly His
        275                 280                 285
Tyr Ala Arg Pro Glu Leu Leu Ser Leu Leu Ile Asp Arg Thr Ala Thr
    290                 295                 300
Ala His Val His Glu Arg Ser Ala His Pro Lys Ala Thr Ala Glu Gln
305                 310                 315                 320
Ala Asp Gly Pro Ser Ala Val Asn Ala Gln
                325                 330

<210> 319
<211> 1017
<212> DNA
<213> Unknown

<220>
<223> Obtained from an environmental sample

<400> 319
atgacgaccc cgcgtatcgt ccgcgtcgcc gcggtgcaga tggcgcccga cctggaatcc      60
gcacacggca cggtagacaa ggtctgccgc gccattctgg aggccggcga aaaaggcgcg     120
cgcatggtgg tctttcccga gaccttcgtg ccctactacc cctacttctc cttcatccag     180
ccggcggtga cgatgggcgc cgcccacctc gagctctacg agcgcgcggt gacggtgccc     240
ggcccggtca ccctggccgt gggcgaagcg gcgcggcggg cgggcgcggt cgtcgtgctc     300
ggcgtcaatg aacgcgacca cggctcgctc tacaacaccc agctgatctt cgacgagacc     360
ggcgcgctgg tgctcaagcg ccgcaagctc accccgacct atcacgagcg catggtgtgg     420

ggccagggcg atggcagcgg tctcaaggtg gtggacaccg gcatcggccg catcggcgcg 480
ctggcctgct gggagcacta caacccgctc gcccgctaca ccctgatggc gcagcatgaa 540
gagatccacg ccgcgcagtt ccccggttcc atggtcggcc agatctttgc cgaccagatg 600
gcggtgacca tccgccacca cgcgctggaa tccggctgct tcgtggtgaa cgccaccggc 660
tggctcaccg atgcgcagat caccgccatc acccccgacc cggccatgca gcgcgcgctg 720
cgcggcggct gccacaccgc catcgtgtcg ccggaaggca gctacgtctg cgaaccgctc 780
accgagggcg aaggcatgct ggtggccgac ctcgacatgc ggctggtcac caagcgcaag 840
cggatgatgg attcggtcgg ccactacgcc cggccggagc tgctttcgct caacgccgat 900
cttgccccca agccggcgct gcacacacag ccggctgcgt ccctccctct ctcccttcag 960
gcaggagccg accatgtcga cgacgaccgc accgcgtccg caacagctga tctctga 1017

<210> 320
<211> 338
<212> PRT
<213> Unknown

<220>
<223> Obtained from an environmental sample

<400> 320
Met Thr Thr Pro Arg Ile Val Arg Val Ala Ala Val Gln Met Ala Pro
1               5                   10                  15
Asp Leu Glu Ser Ala His Gly Thr Val Asp Lys Val Cys Arg Ala Ile
            20                  25                  30
Leu Glu Ala Gly Glu Lys Gly Ala Arg Met Val Val Phe Pro Glu Thr
        35                  40                  45
Phe Val Pro Tyr Tyr Pro Tyr Phe Ser Phe Ile Gln Pro Ala Val Thr
    50                  55                  60
Met Gly Ala Ala His Leu Glu Leu Tyr Glu Arg Ala Val Thr Val Pro
65                  70                  75                  80
Gly Pro Val Thr Leu Ala Val Gly Glu Ala Ala Arg Arg Ala Gly Ala
                85                  90                  95
Val Val Val Leu Gly Val Asn Glu Arg Asp His Gly Ser Leu Tyr Asn
            100                 105                 110
Thr Gln Leu Ile Phe Asp Glu Thr Gly Ala Leu Val Leu Lys Arg Arg
        115                 120                 125
Lys Leu Thr Pro Thr Tyr His Glu Arg Met Val Trp Gly Gln Gly Asp
    130                 135                 140
Gly Ser Gly Leu Lys Val Val Asp Thr Gly Ile Gly Arg Ile Gly Ala
145                 150                 155                 160
Leu Ala Cys Trp Glu His Tyr Asn Pro Leu Ala Arg Tyr Thr Leu Met
                165                 170                 175
Ala Gln His Glu Glu Ile His Ala Ala Gln Phe Pro Gly Ser Met Val
            180                 185                 190
Gly Gln Ile Phe Ala Asp Gln Met Ala Val Thr Ile Arg His His Ala
        195                 200                 205
Leu Glu Ser Gly Cys Phe Val Val Asn Ala Thr Gly Trp Leu Thr Asp
    210                 215                 220
Ala Gln Ile Thr Ala Ile Thr Pro Asp Pro Ala Met Gln Arg Ala Leu
225                 230                 235                 240
Arg Gly Gly Cys His Thr Ala Ile Val Ser Pro Glu Gly Ser Tyr Val
                245                 250                 255
Cys Glu Pro Leu Thr Glu Gly Glu Gly Met Leu Val Ala Asp Leu Asp
            260                 265                 270
Met Arg Leu Val Thr Lys Arg Lys Arg Met Met Asp Ser Val Gly His
        275                 280                 285
Tyr Ala Arg Pro Glu Leu Leu Ser Leu Asn Ala Asp Leu Ala Pro Lys
    290                 295                 300
Pro Ala Leu His Thr Gln Pro Ala Ala Ser Leu Pro Leu Ser Leu Gln
305                 310                 315                 320
Ala Gly Ala Asp His Val Asp Asp Asp Arg Thr Ala Ser Ala Thr Ala
                325                 330                 335
Asp Leu

<210> 321
<211> 993
<212> DNA

<213> Unknown

<220>
<223> Obtained from an environmental sample

<400> 321
atgagagttg tcaaagccgc tgctgtccaa ctgagtcctg tcctccatag ccgcgacgga 60
acggtcgaaa aggtcgtgcg gaagatccat gaactcgccg aagagggagt cgagttcgcc 120
acctttcctg agaccgtggt gccttactac ccgtactttt ccttcgttca gacgcccttg 180
cagcaaatct tcggaactga gtatctgagg ctgctcgacc aggcagtcac cgtgccatcc 240
gctgccaccg acgcgatcgg cgaggctgcc aggtgggctg gacttgttgt ctcgatcggc 300
gtcaacgagc gagacggggg aactctctac aacactcagc ttctcttcga tgccgacggc 360
agcttaattc agcggcgtcg caagatcaca cccacccatt acgagcgcat gatctggggc 420
cagggcgacg gctcaggtct gcgggccgtt gatagcaagg ccggccgcat tggtcagctg 480
gcatgctggg aacacaacaa ccccctggcg cgctacgcgc tgatggccga cggcgagcag 540
atccattcgg ccatgtatcc gggctccatg ttcggcgact cgttttccca aaagaccgaa 600
atcaatatcc ggcagcatgc gctggaatct gcgtgcttcg tcgtgaacgc aacggcctgg 660
ctggacgccg atcagcaggc gcaaatcatg aaggacaccg gctgcggcat cggcccgatc 720
tccggcggtt gcttcactgc gatcgttgca cccgatggta gcctgctggg cgaacccatc 780
cgttccggtg agggcgtcgt cgtcgccaac ctcgacttca cgctgatcga caggcgtaag 840
caggtgatgg actcgcgagg ccactacagc cggccggagt tgctcagcct cttaatagac 900
cgcactccta ccgcgcacgt tcacgaacgc gctacgcacc ccacgacagg agctgagcaa 960
ggctccgagg atgtgttcga ggctcgcatt taa 993

<210> 322
<211> 330
<212> PRT
<213> Unknown

<220>
<223> Obtained from an environmental sample

<400> 322
Met Arg Val Val Lys Ala Ala Ala Val Gln Leu Ser Pro Val Leu His
1               5                   10                  15
Ser Arg Asp Gly Thr Val Glu Lys Val Val Arg Lys Ile His Glu Leu
            20                  25                  30
Ala Glu Glu Gly Val Glu Phe Ala Thr Phe Pro Glu Thr Val Val Pro
        35                  40                  45
Tyr Tyr Pro Tyr Phe Ser Phe Val Gln Thr Pro Leu Gln Gln Ile Phe
    50                  55                  60
Gly Thr Glu Tyr Leu Arg Leu Leu Asp Gln Ala Val Thr Val Pro Ser
65                  70                  75                  80
Ala Ala Thr Asp Ala Ile Gly Glu Ala Ala Arg Trp Ala Gly Leu Val
                85                  90                  95
Val Ser Ile Gly Val Asn Glu Arg Asp Gly Gly Thr Leu Tyr Asn Thr
            100                 105                 110
Gln Leu Leu Phe Asp Ala Asp Gly Ser Leu Ile Gln Arg Arg Arg Lys
        115                 120                 125
Ile Thr Pro Thr His Tyr Glu Arg Met Ile Trp Gly Gln Gly Asp Gly
    130                 135                 140
Ser Gly Leu Arg Ala Val Asp Ser Lys Ala Gly Arg Ile Gly Gln Leu
145                 150                 155                 160
Ala Cys Trp Glu His Asn Asn Pro Leu Ala Arg Tyr Ala Leu Met Ala
                165                 170                 175
Asp Gly Glu Gln Ile His Ser Ala Met Tyr Pro Gly Ser Met Phe Gly
            180                 185                 190
Asp Ser Phe Ser Gln Lys Thr Glu Ile Asn Ile Arg Gln His Ala Leu
        195                 200                 205
Glu Ser Ala Cys Phe Val Val Asn Ala Thr Ala Trp Leu Asp Ala Asp
    210                 215                 220
Gln Gln Ala Gln Ile Met Lys Asp Thr Gly Cys Gly Ile Gly Pro Ile
225                 230                 235                 240
Ser Gly Gly Cys Phe Thr Ala Ile Val Ala Pro Asp Gly Ser Leu Leu
                245                 250                 255
Gly Glu Pro Ile Arg Ser Gly Glu Gly Val Val Val Ala Asn Leu Asp
            260                 265                 270
Phe Thr Leu Ile Asp Arg Arg Lys Gln Val Met Asp Ser Arg Gly His

275                 280                 285
Tyr Ser Arg Pro Glu Leu Leu Ser Leu Leu Ile Asp Arg Thr Pro Thr
    290                 295                 300
Ala His Val His Glu Arg Ala Thr His Pro Thr Thr Gly Ala Glu Gln
305                 310                 315                 320
Gly Ser Glu Asp Val Phe Glu Ala Arg Ile
                325                 330

<210> 323
<211> 951
<212> DNA
<213> Unknown

<220>
<223> Obtained from an environmental sample

<400> 323
atgagcaacg tgaaagtcgc ggtcgtgcaa cgcgcgccgg tgttcggcaa ccgcgccgcc      60
accctcgatc gcgccgtcgc cgcgctggcc gaagcggcgc agcagggggc gaagctggtc     120
gtgatgcccg agcacttcat ccccggctac ccggcctgga tctggcgcct gcgtccgggc     180
accgacctgc gattgtgcga acagctgcac gcgatgctgc gcgccaacgc cgtgaggctg     240
gatgacggtg acctggcccc gttgaccgag gccgcgcagc ggcatgcgct caccgtggtc     300
tgcggcgtct gcgagatcga caccgaattc agtcgcggca ccctgtacaa caccgtggtc     360
gtgatcgggc ccgacggcac gctgctcaac cggcatcgca agctgatgcc caccaacccc     420
gagcgcatgg tctggggcat gggcgacgcc acggggctga aggtggtcga cacgccctgc     480
gggcgcatcg gcacgctgat ttgctgggag aactacatgc cattcgcacg cgccgcgctg     540
tacgcgcagg gggtcgaggt cctggttgca ccgacctacg acgaaggccc ggtatggctg     600
gcgtcgatgc agcacatcgc ccgcgaaggc ggctgctggg tggtgggcaa cggctgcgca     660
ttccagggcc gcgacatgcc ggacaccttg ccgggcaagg cccagctgtt tcccgaggcc     720
gacgcctggg tcaacgccgg ggactcggtc atcgtcgcgc caggcggccg gacagtggcg     780
ggtccgttgc acgaggcgtt cgggctgttc accgccgaga tcgacctctc ccgggtcgga     840
atggcccggc gcagcctgga tgtggccggg cactatggac ggcccgacat cttctgcctg     900
caggtcaacg cccgggcgca gccgccggtt gaggtgacgc accatggctg a              951

<210> 324
<211> 316
<212> PRT
<213> Unknown

<220>
<223> Obtained from an environmental sample

<400> 324
Met Ser Asn Val Lys Val Ala Val Val Gln Arg Ala Pro Val Phe Gly
1               5                   10                  15
Asn Arg Ala Ala Thr Leu Asp Arg Ala Val Ala Ala Leu Ala Glu Ala
            20                  25                  30
Ala Gln Gln Gly Ala Lys Leu Val Val Met Pro Glu His Phe Ile Pro
        35                  40                  45
Gly Tyr Pro Ala Trp Ile Trp Arg Leu Arg Pro Gly Thr Asp Leu Arg
    50                  55                  60
Leu Cys Glu Gln Leu His Ala Met Leu Arg Ala Asn Ala Val Arg Leu
65                  70                  75                  80
Asp Asp Gly Asp Leu Ala Pro Leu Thr Glu Ala Ala Gln Arg His Ala
                85                  90                  95
Leu Thr Val Val Cys Gly Val Cys Glu Ile Asp Thr Glu Phe Ser Arg
            100                 105                 110
Gly Thr Leu Tyr Asn Thr Val Val Val Ile Gly Pro Asp Gly Thr Leu
        115                 120                 125
Leu Asn Arg His Arg Lys Leu Met Pro Thr Asn Pro Glu Arg Met Val
    130                 135                 140
Trp Gly Met Gly Asp Ala Thr Gly Leu Lys Val Val Asp Thr Pro Cys
145                 150                 155                 160
Gly Arg Ile Gly Thr Leu Ile Cys Trp Glu Asn Tyr Met Pro Phe Ala
                165                 170                 175
Arg Ala Ala Leu Tyr Ala Gln Gly Val Glu Val Leu Val Ala Pro Thr
            180                 185                 190
Tyr Asp Glu Gly Pro Val Trp Leu Ala Ser Met Gln His Ile Ala Arg

195                 200                 205
Glu Gly Gly Cys Trp Val Val Gly Asn Gly Cys Ala Phe Gln Gly Arg
    210                 215                 220
Asp Met Pro Asp Thr Leu Pro Gly Lys Ala Gln Leu Phe Pro Glu Ala
225                 230                 235                 240
Asp Ala Trp Val Asn Ala Gly Asp Ser Val Ile Val Ala Pro Gly Gly
                245                 250                 255
Arg Thr Val Ala Gly Pro Leu His Glu Ala Phe Gly Leu Phe Thr Ala
            260                 265                 270
Glu Ile Asp Leu Ser Arg Val Gly Met Ala Arg Arg Ser Leu Asp Val
        275                 280                 285
Ala Gly His Tyr Gly Arg Pro Asp Ile Phe Cys Leu Gln Val Asn Ala
    290                 295                 300
Arg Ala Gln Pro Pro Val Glu Val Thr His His Gly
305                 310                 315

<210> 325
<211> 1077
<212> DNA
<213> Unknown

<220>
<223> Obtained from an environmental sample

<400> 325
atgactgcaa aaaagattgt ccgcgccgcc gctgtccagc tcaatcccgt gctggacagc      60
gccgacggca cccttgtgaa agtgttgcag gcgattgccg acgccgcagc gcagggcgtg     120
caactgatcg tctttcccga gacggtggtg ccctactatc cttacttttc cttcgtcacg     180
cctgcggttt cgatgggcgc ggcgcatctg aaactgtacg aacaatcgcc cacggtgcca     240
ggtccactga ccgacgccgt cgccgcagcc gcgcgggcac atcagatggt ggttgtgctc     300
ggcgtcaacg agcgcgatca cggcacgctc tacaacacgc aactgatctt cgacgccgac     360
ggcacgctcc cactgaagcg tcgcaagatc acgccgacct atcacgagcg catggtctgg     420
ggcatgggcg atggctccgg cctgcgcacg gtgaagaccg aggtcggaac cgttggcgcg     480
ctggcctgct gggaacacta caacccgctg gcacgctacg cgctgatggc gcagcacgaa     540
gagatccatt gcagccagtt ccccggctcg ctggtcggcc cgatcttttc cgagcagatg     600
gaaatcacca tgcgtcatca cgcgctggaa tccggctgct tcgtcgtcaa cgccactgca     660
tggctgacgc ctgagcaggt gcgatcacag gcgccaacac cggcaatgga aaaagccttc     720
tccggtggtt gctacaccgc gatcatttcg ccggaaggaa aacatctggg cgaacctctt     780
cgcgacggcg aaggcatggt catcgccgat cttgatttgg atctcatcac caagcgcaag     840
cgaatgatgg attcggttgg ccactacgca cggccggaat tgttgagcct gcagctcgac     900
aaccgatcaa ctgcaccgct gacaacgtcg ccggtggccg ccgcagcgcc gtcgcttgca     960
gagatggaag cacagcgcct gtcacgttat ctcgatgcca gctccggcag cgccgcacaa    1020
ggcatcgaag ccgcctacat caatgccctc agctcttttt caggaaaacc ctcatga       1077

<210> 326
<211> 358
<212> PRT
<213> Unknown

<220>
<223> Obtained from an environmental sample

<400> 326
Met Thr Ala Lys Lys Ile Val Arg Ala Ala Ala Val Gln Leu Asn Pro
1               5                   10                  15
Val Leu Asp Ser Ala Asp Gly Thr Leu Val Lys Val Leu Gln Ala Ile
            20                  25                  30
Ala Asp Ala Ala Ala Gln Gly Val Gln Leu Ile Val Phe Pro Glu Thr
        35                  40                  45
Val Val Pro Tyr Tyr Pro Tyr Phe Ser Phe Val Thr Pro Ala Val Ser
    50                  55                  60
Met Gly Ala Ala His Leu Lys Leu Tyr Glu Gln Ser Pro Thr Val Pro
65                  70                  75                  80
Gly Pro Leu Thr Asp Ala Val Ala Ala Ala Ala Arg Ala His Gln Met
                85                  90                  95
Val Val Val Leu Gly Val Asn Glu Arg Asp His Gly Thr Leu Tyr Asn
            100                 105                 110
Thr Gln Leu Ile Phe Asp Ala Asp Gly Thr Leu Pro Leu Lys Arg Arg

115                 120                 125
Lys Ile Thr Pro Thr Tyr His Glu Arg Met Val Trp Gly Met Gly Asp
    130                 135                 140
Gly Ser Gly Leu Arg Thr Val Lys Thr Glu Val Gly Thr Val Gly Ala
145                 150                 155                 160
Leu Ala Cys Trp Glu His Tyr Asn Pro Leu Ala Arg Tyr Ala Leu Met
                165                 170                 175
Ala Gln His Glu Glu Ile His Cys Ser Gln Phe Pro Gly Ser Leu Val
            180                 185                 190
Gly Pro Ile Phe Ser Glu Gln Met Glu Ile Thr Met Arg His His Ala
        195                 200                 205
Leu Glu Ser Gly Cys Phe Val Val Asn Ala Thr Ala Trp Leu Thr Pro
    210                 215                 220
Glu Gln Val Arg Ser Gln Ala Pro Thr Pro Ala Met Glu Lys Ala Phe
225                 230                 235                 240
Ser Gly Gly Cys Tyr Thr Ala Ile Ile Ser Pro Glu Gly Lys His Leu
                245                 250                 255
Gly Glu Pro Leu Arg Asp Gly Glu Gly Met Val Ile Ala Asp Leu Asp
            260                 265                 270
Phe Asp Leu Ile Thr Lys Arg Lys Arg Met Met Asp Ser Val Gly His
        275                 280                 285
Tyr Ala Arg Pro Glu Leu Leu Ser Leu Gln Leu Asp Asn Arg Ser Thr
    290                 295                 300
Ala Pro Leu Thr Thr Ser Pro Val Ala Ala Ala Ala Pro Ser Leu Ala
305                 310                 315                 320
Glu Met Glu Ala Gln Arg Leu Ser Arg Tyr Leu Asp Ala Ser Ser Gly
                325                 330                 335
Ser Ala Ala Gln Gly Ile Glu Ala Ala Tyr Ile Asn Ala Leu Ser Ser
            340                 345                 350
Phe Ser Gly Lys Pro Ser
        355

<210> 327
<211> 975
<212> DNA
<213> Unknown

<220>
<223> Obtained from an environmental sample

<400> 327
atggttgatc aaatacttaa cgatcgttct gaattattaa cagttggttt ggcgcagatt      60
gctccaatct ggctgaaccg cgaaaaaaca cttgcgaaag tggttgagaa agtaaatcaa     120
gctgcaaagc aagattgtca tcttgttgct tttggtgaag ctttggtccc cggatatccc     180
ttttggattg aacttacaga tggcgcgcga ttcaactcca atgttcaaaa agaaattcac     240
gcgcactata tggatcaggc agtacagata gagaatggtc atttaaaagc gctttgtgaa     300
acatcggccg caaacaagat tgccgtgatc gttgggtgca ttgaacgcgc agccgatcgc     360
ggcgggcaca gcttatatgc ttcattggtt tttattaatc ctcaagggca aatcggatcg     420
gtgcatcgca aacttatgcc aacttatgaa gaacgtttaa cctggtcacc tggcgatgga     480
catggtttgc gaacgcatca actaggtgct ttcactgttg gcggcttgaa ttgttgggaa     540
aactggatgc cattgccacg cgccgcattg tacgcacaag gtgaagactt tcatgtcgca     600
atctggccgg gaagcattca caatacgcaa gatattacgc gctatattgc gaaggaatcc     660
agatcgtttg taatgtctac ttccgggttc atgcgtaaag aagactttcc atctgatacg     720
cctcacttag acaaaatact tgcaaactct ccgaatgtac ttgcaaatgg tggatcctgt     780
ctcgccggac cggacggtca gtggatcgtt gaaccatttg tgaatgagga aaaattagtt     840
gttgcgactg tgaaccacaa gcaggtccgt gaagaaagac aaaattttga tccggtcgga     900
cactattccc gtcctgatgt cacgcagttg attgtgaaca gacaaagaca atcaacaatc     960
aagctcaacg attaa                                                      975

<210> 328
<211> 324
<212> PRT
<213> Unknown

<220>
<223> Obtained from an environmental sample

<400> 328

Met Val Asp Gln Ile Leu Asn Asp Arg Ser Glu Leu Leu Thr Val Gly
1               5                   10                  15
Leu Ala Gln Ile Ala Pro Ile Trp Leu Asn Arg Glu Lys Thr Leu Ala
            20                  25                  30
Lys Val Val Glu Lys Val Asn Gln Ala Ala Lys Gln Asp Cys His Leu
        35                  40                  45
Val Ala Phe Gly Glu Ala Leu Val Pro Gly Tyr Pro Phe Trp Ile Glu
    50                  55                  60
Leu Thr Asp Gly Ala Arg Phe Asn Ser Asn Val Gln Lys Glu Ile His
65                  70                  75                  80
Ala His Tyr Met Asp Gln Ala Val Gln Ile Glu Asn Gly His Leu Lys
                85                  90                  95
Ala Leu Cys Glu Thr Ser Ala Ala Asn Lys Ile Ala Val Ile Val Gly
            100                 105                 110
Cys Ile Glu Arg Ala Ala Asp Arg Gly Gly His Ser Leu Tyr Ala Ser
        115                 120                 125
Leu Val Phe Ile Asn Pro Gln Gly Gln Ile Gly Ser Val His Arg Lys
    130                 135                 140
Leu Met Pro Thr Tyr Glu Glu Arg Leu Thr Trp Ser Pro Gly Asp Gly
145                 150                 155                 160
His Gly Leu Arg Thr His Gln Leu Gly Ala Phe Thr Val Gly Gly Leu
                165                 170                 175
Asn Cys Trp Glu Asn Trp Met Pro Leu Pro Arg Ala Ala Leu Tyr Ala
            180                 185                 190
Gln Gly Glu Asp Phe His Val Ala Ile Trp Pro Gly Ser Ile His Asn
        195                 200                 205
Thr Gln Asp Ile Thr Arg Tyr Ile Ala Lys Glu Ser Arg Ser Phe Val
    210                 215                 220
Met Ser Thr Ser Gly Phe Met Arg Lys Glu Asp Phe Pro Ser Asp Thr
225                 230                 235                 240
Pro His Leu Asp Lys Ile Leu Ala Asn Ser Pro Asn Val Leu Ala Asn
                245                 250                 255
Gly Gly Ser Cys Leu Ala Gly Pro Asp Gly Gln Trp Ile Val Glu Pro
            260                 265                 270
Phe Val Asn Glu Glu Lys Leu Val Val Ala Thr Val Asn His Lys Gln
        275                 280                 285
Val Arg Glu Glu Arg Gln Asn Phe Asp Pro Val Gly His Tyr Ser Arg
    290                 295                 300
Pro Asp Val Thr Gln Leu Ile Val Asn Arg Gln Arg Gln Ser Thr Ile
305                 310                 315                 320
Lys Leu Asn Asp

<210> 329
<211> 1023
<212> DNA
<213> Unknown

<220>
<223> Obtained from an environmental sample

<400> 329
gtgccggcca aagtacgcgc cgccgccgtg cagctcagcc ccgtgctctt cagccgtgaa      60
ggcacgacca gcaaggtctg cgacaagatt gccgaggcgg cggcgcaggg cgccgagctg     120
gtggtgtttc ccgaaaccgt agtgccgtat tacccgtatt tctcgttcat caaggctccg     180
gccgtgatcg gcgccgagca cttactcttg ctcgaacaag ccgtcacggt gcccgggccc     240
agcgtcgaag ccatcgcaga agccgctcgc aaggcgggcg cggtggtttc gatcggcgtc     300
aacgaacgcg atcacggcac gctgtacaac acccagctgt tgttcgacgc cgacggtcgg     360
ttggcgcaag cccgccgcaa gatcaccccc acgtatcacg agcggatgat ctgggggcag     420
ggcgatggct cgggcttggt ggcggtggat acgcgagtcg gcaggattgg ctccctggcg     480
tgctgggagc actacaaccc gctggctcgc tatgcgctga tggccgacca cgagcaaatt     540
cacgtggcca tgttccctgg ctcgctcgtg ggcgacatct tccgcgagca aatcgaggtc     600
acgattcggc accacgcgct cgagtcgggc tgcttcgtcg tcaacgcgac gggctacctg     660
agcgacgcgc aggtgacgca gatcgcgggc gacaccaagc tcgaccgcgc cctgcgcggc     720
ggttgcttca ccgccatcgt atcgcccgag ggcacgctgc tggcgccacc gctcaccgac     780
ggtgagggca tggtcattgc cgacctcgat ctgtcgctca tcgccaaacg caaacgcatg     840
atggacagcg tcggccatta cagccggccc gagctgctca gcgtgctgat cgaccgctcg     900
ccgcagccgc atttgcgcga gaaaactgcc tctttacccg aacctcggat gtcccatgaa     960

tcgctcgctc ccgatagtaa cggcctccgc gacgctgacg cgaaagcctc gaccctctcc 1020
tga 1023

<210> 330
<211> 340
<212> PRT
<213> Unknown

<220>
<223> Obtained from an environmental sample

<400> 330
Val Pro Ala Lys Val Arg Ala Ala Ala Val Gln Leu Ser Pro Val Leu
1               5                   10                  15
Phe Ser Arg Glu Gly Thr Thr Ser Lys Val Cys Asp Lys Ile Ala Glu
            20                  25                  30
Ala Ala Ala Gln Gly Ala Glu Leu Val Val Phe Pro Glu Thr Val Val
        35                  40                  45
Pro Tyr Tyr Pro Tyr Phe Ser Phe Ile Lys Ala Pro Ala Val Ile Gly
    50                  55                  60
Ala Glu His Leu Leu Leu Leu Glu Gln Ala Val Thr Val Pro Gly Pro
65                  70                  75                  80
Ser Val Glu Ala Ile Ala Glu Ala Ala Arg Lys Ala Gly Ala Val Val
                85                  90                  95
Ser Ile Gly Val Asn Glu Arg Asp His Gly Thr Leu Tyr Asn Thr Gln
            100                 105                 110
Leu Leu Phe Asp Ala Asp Gly Arg Leu Ala Gln Ala Arg Arg Lys Ile
        115                 120                 125
Thr Pro Thr Tyr His Glu Arg Met Ile Trp Gly Gln Gly Asp Gly Ser
    130                 135                 140
Gly Leu Val Ala Val Asp Thr Arg Val Gly Arg Ile Gly Ser Leu Ala
145                 150                 155                 160
Cys Trp Glu His Tyr Asn Pro Leu Ala Arg Tyr Ala Leu Met Ala Asp
                165                 170                 175
His Glu Gln Ile His Val Ala Met Phe Pro Gly Ser Leu Val Gly Asp
            180                 185                 190
Ile Phe Arg Glu Gln Ile Glu Val Thr Ile Arg His His Ala Leu Glu
        195                 200                 205
Ser Gly Cys Phe Val Val Asn Ala Thr Gly Tyr Leu Ser Asp Ala Gln
    210                 215                 220
Val Thr Gln Ile Ala Gly Asp Thr Lys Leu Asp Arg Ala Leu Arg Gly
225                 230                 235                 240
Gly Cys Phe Thr Ala Ile Val Ser Pro Glu Gly Thr Leu Leu Ala Pro
                245                 250                 255
Pro Leu Thr Asp Gly Glu Gly Met Val Ile Ala Asp Leu Asp Leu Ser
            260                 265                 270
Leu Ile Ala Lys Arg Lys Arg Met Met Asp Ser Val Gly His Tyr Ser
        275                 280                 285
Arg Pro Glu Leu Leu Ser Val Leu Ile Asp Arg Ser Pro Gln Pro His
    290                 295                 300
Leu Arg Glu Lys Thr Ala Ser Leu Pro Glu Pro Arg Met Ser His Glu
305                 310                 315                 320
Ser Leu Ala Pro Asp Ser Asn Gly Leu Arg Asp Ala Asp Ala Lys Ala
                325                 330                 335
Ser Thr Leu Ser
            340

<210> 331
<211> 1041
<212> DNA
<213> Unknown

<220>
<223> Obtained from an environmental sample

<400> 331
atgtccggaa caggacctat cggaccggtc gtgaaggtcg ccgtcgtaca agccgcgcca 60
tgtctgctcg atcttgatgc gggcgtcgag aaggcgatcg ccttgatcga ccaagcgggc 120

aaggctggcg cgcggctcat caattttccc gaaatttggc tgccgggtta tccttggtgg 180
atctggctga atccacccgc catcaacatg cagtatgtcg cgccttacat gaacaactcc 240
attgtcgcag gcagcaagca tgaccacgca cttcgggccg ccgcgcgccg caacaatatt 300
cacgtcgtga tcggtgtctc cgagcgcgcc ggcggcagtc tgtacatggc tcagtggcac 360
tatgggcccg agggcgaggt gatctcgcgt cgtcgtaagc taaaacccac ccatgtcgaa 420
cgcagcgtct ttggcgaagg cgacggcagc gacatgatcg ttactcagac cgatttcggg 480
cgcgtcggcg cgctatgctg ttgggaacac ctgcaaccgc tgtcgaaata cgcgctcttt 540
tcccaggacg agcagattca ttgcgcggcg tggcccgctt tcagcctcta tgcaaaactc 600
tcgaaggcct tcagccccga agtcagcgtc aacgtgaacc aaatctacgc cgtagaaggg 660
caatgcttcg tcctgtcgtc gtgctcggtt atcgatcagg cgatctacga cacgttggtg 720
cagaacgaat tgcaccagaa gttcctcgag gtgggcgggg gctacagccg aatcttcggg 780
ccgaacggtg cggaattcgg tgagaatctc ccacccgata gggaaggcct ggtggttgcc 840
gatatcgatc tcggcctgat ctcgcactcc aagagtgccg ctgatccggc tggtcactac 900
gcgagggccg acgctctggc gctcatgcat aaccgtaatc cccgccgtcc ggttatcggt 960
ttcggcgagg cgacgcgcaa ggttgccgac gcactgccta aaggcgcgga acccgcggaa 1020
gcgctcgaag cggccgagtg a 1041

<210> 332
<211> 346
<212> PRT
<213> Unknown

<220>
<223> Obtained from an environmental sample

<400> 332
Met Ser Gly Thr Gly Pro Ile Gly Pro Val Val Lys Val Ala Val Val
1               5                   10                  15
Gln Ala Ala Pro Cys Leu Leu Asp Leu Asp Ala Gly Val Glu Lys Ala
            20                  25                  30
Ile Ala Leu Ile Asp Gln Ala Gly Lys Ala Gly Ala Arg Leu Ile Asn
        35                  40                  45
Phe Pro Glu Ile Trp Leu Pro Gly Tyr Pro Trp Trp Ile Trp Leu Asn
    50                  55                  60
Pro Pro Ala Ile Asn Met Gln Tyr Val Ala Pro Tyr Met Asn Asn Ser
65                  70                  75                  80
Ile Val Ala Gly Ser Lys His Asp His Ala Leu Arg Ala Ala Ala Arg
                85                  90                  95
Arg Asn Asn Ile His Val Val Ile Gly Val Ser Glu Arg Ala Gly Gly
            100                 105                 110
Ser Leu Tyr Met Ala Gln Trp His Tyr Gly Pro Glu Gly Glu Val Ile
        115                 120                 125
Ser Arg Arg Arg Lys Leu Lys Pro Thr His Val Glu Arg Ser Val Phe
    130                 135                 140
Gly Glu Gly Asp Gly Ser Asp Met Ile Val Thr Gln Thr Asp Phe Gly
145                 150                 155                 160
Arg Val Gly Ala Leu Cys Cys Trp Glu His Leu Gln Pro Leu Ser Lys
                165                 170                 175
Tyr Ala Leu Phe Ser Gln Asp Glu Gln Ile His Cys Ala Ala Trp Pro
            180                 185                 190
Ala Phe Ser Leu Tyr Ala Lys Leu Ser Lys Ala Phe Ser Pro Glu Val
        195                 200                 205
Ser Val Asn Val Asn Gln Ile Tyr Ala Val Glu Gly Gln Cys Phe Val
    210                 215                 220
Leu Ser Ser Cys Ser Val Ile Asp Gln Ala Ile Tyr Asp Thr Leu Val
225                 230                 235                 240
Gln Asn Glu Leu His Gln Lys Phe Leu Glu Val Gly Gly Gly Tyr Ser
                245                 250                 255
Arg Ile Phe Gly Pro Asn Gly Ala Glu Phe Gly Glu Asn Leu Pro Pro
            260                 265                 270
Asp Arg Glu Gly Leu Val Val Ala Asp Ile Asp Leu Gly Leu Ile Ser
        275                 280                 285
His Ser Lys Ser Ala Ala Asp Pro Ala Gly His Tyr Ala Arg Ala Asp
    290                 295                 300
Ala Leu Ala Leu Met His Asn Arg Asn Pro Arg Arg Pro Val Ile Gly
305                 310                 315                 320
Phe Gly Glu Ala Thr Arg Lys Val Ala Asp Ala Leu Pro Lys Gly Ala
                325                 330                 335

Glu Pro Ala Glu Ala Leu Glu Ala Ala Glu
            340                 345

<210> 333
<211> 1038
<212> DNA
<213> Unknown

<220>
<223> Obtained from an environmental sample

<400> 333
atggccattg aacatccccg ctatcgggtc gctgccgttc aggcggcgcc agagttcctt      60
aatctcgagg caaccgttga caagacgatt gcgcttatcg aggaggccgc ccgcggaggc     120
gcaagtctca ttgctttccc tgaaacttgg atacctggat atccatggtt tgcctggctt     180
ggtgcgccga tctggggcat gaaattcatc caggcgtacc atgacaactc gatggtgatc     240
gacggcgccc agtttgagcg tattgcgcaa gcagcatcgc gttgcaacat cactgttgtg     300
cttggcttta gcgagaagga cgcaggaagc ctgtatattg cccaggccat cctgagccct     360
gaagggaaga ccatcgccac gcgtcgcaag ctgaaaccca ctcatgtcga acgcgcgatc     420
ttcggcgaag gcgacggcag cgacctggca gttcacgaca ccaagctcgg cagggtgggc     480
gccctttgct gctgggagca tcttcagcca ctttccaaat acgcgatgta tgctcagaac     540
gagcaggtcc atatcgccgc ctggcccagt tttccctttt acgtcgacgc ggcttacgcg     600
cttgggccag aggtgaacaa cgccgcgagt cggttgtatg cggtcgaggg ccagtgcttc     660
gtggttgcgc cgtgtgcaac ggtttcgcaa aagatgatcg atatgctttg cgagacaccc     720
gagcaacaag cgctcttgaa gccggggggt ggtcacgcgc aaatctacgg tcccgacgga     780
cgatcgctgg ccgatccgct gcctccggac gcggaggggc tgttgtatgc agacattgac     840
cttgcagcca tcaccctcgc gaaagcagct gcagatcctg ctggccatta ctctcgccct     900
gacgtgacac aactgctgct tgaccgcaat ccaaagcccc gtgtcgtgca tgctaagccc     960
ggccaaagcg ccaacaacag ctcacccggc atgcgggccg tcgagcatac cgagctcgaa    1020
gaaggtgaac aggcctga                                                  1038

<210> 334
<211> 345
<212> PRT
<213> Unknown

<220>
<223> Obtained from an environmental sample

<400> 334
Met Ala Ile Glu His Pro Arg Tyr Arg Val Ala Ala Val Gln Ala Ala
1               5                   10                  15
Pro Glu Phe Leu Asn Leu Glu Ala Thr Val Asp Lys Thr Ile Ala Leu
            20                  25                  30
Ile Glu Glu Ala Ala Arg Gly Gly Ala Ser Leu Ile Ala Phe Pro Glu
        35                  40                  45
Thr Trp Ile Pro Gly Tyr Pro Trp Phe Ala Trp Leu Gly Ala Pro Ile
    50                  55                  60
Trp Gly Met Lys Phe Ile Gln Ala Tyr His Asp Asn Ser Met Val Ile
65                  70                  75                  80
Asp Gly Ala Gln Phe Glu Arg Ile Ala Gln Ala Ala Ser Arg Cys Asn
                85                  90                  95
Ile Thr Val Val Leu Gly Phe Ser Glu Lys Asp Ala Gly Ser Leu Tyr
            100                 105                 110
Ile Ala Gln Ala Ile Leu Ser Pro Glu Gly Lys Thr Ile Ala Thr Arg
        115                 120                 125
Arg Lys Leu Lys Pro Thr His Val Glu Arg Ala Ile Phe Gly Glu Gly
    130                 135                 140
Asp Gly Ser Asp Leu Ala Val His Asp Thr Lys Leu Gly Arg Val Gly
145                 150                 155                 160
Ala Leu Cys Cys Trp Glu His Leu Gln Pro Leu Ser Lys Tyr Ala Met
                165                 170                 175
Tyr Ala Gln Asn Glu Gln Val His Ile Ala Ala Trp Pro Ser Phe Ser
            180                 185                 190
Leu Tyr Val Asp Ala Ala Tyr Ala Leu Gly Pro Glu Val Asn Asn Ala
        195                 200                 205
Ala Ser Arg Leu Tyr Ala Val Glu Gly Gln Cys Phe Val Val Ala Pro
    210                 215                 220

Cys Ala Thr Val Ser Gln Lys Met Ile Asp Met Leu Cys Glu Thr Pro
225                 230                 235                 240
Glu Gln Gln Ala Leu Leu Lys Pro Gly Gly Gly His Ala Gln Ile Tyr
                245                 250                 255
Gly Pro Asp Gly Arg Ser Leu Ala Asp Pro Leu Pro Pro Asp Ala Glu
            260                 265                 270
Gly Leu Leu Tyr Ala Asp Ile Asp Leu Ala Ala Ile Thr Leu Ala Lys
        275                 280                 285
Ala Ala Ala Asp Pro Ala Gly His Tyr Ser Arg Pro Asp Val Thr Gln
    290                 295                 300
Leu Leu Leu Asp Arg Asn Pro Lys Pro Arg Val Val His Ala Lys Pro
305                 310                 315                 320
Gly Gln Ser Ala Asn Asn Ser Ser Pro Gly Met Arg Ala Val Glu His
                325                 330                 335
Thr Glu Leu Glu Glu Gly Glu Gln Ala
            340                 345

<210> 335
<211> 1053
<212> DNA
<213> Unknown

<220>
<223> Obtained from an environmental sample

<400> 335
atgactacgc gcgtgattaa agtcgctgcc gcgcagctgt ctccggtgct ggcaactgca      60
tcggagcaca gccgcgagga cacgattgcg aaagtgatcg acgccatcgc tgcggcgtcg     120
caacagggcg cgcaactgat tgtgtttccg gaaacagtcg ttccgtatta cccgtatttt     180
tcatttatca cgcccgcggt aacgatgggt gccgaacatt tgaaattgta cgaacaggca     240
gtgacggtac ccagcgcagc gacagatgct gtcgctgcgg cggcaaaaaa ttatggcatg     300
gtagtggtgc tcggaattaa tgaacgcgat cacggctcgc tgtacaacgc gcaattaatt     360
ttcgacgccg atggtgagct gctattaaag cgtcgaaaaa ttacgccgac ttatcacgaa     420
cgcatggtgt ggggacaagg cgacggcagc ggtttaaaag ttgtcgatac tgctgccggc     480
cgtgtcggcg cgctcgcgtg ctgggaacat tacaacccgc tcgcgcgtta cagcctgatg     540
gcacaacacg aagaaattca ttgcagtcaa tttccgggat cgttggtcgg ccctattttc      600
gccgaacaaa tggaaatcac catgcgtcat cacgcactcg aatccggctg ttttgtggtg     660
aatgcaacgg cctggcttag cgatacacaa atccaatcaa ttacccccga taaagccatg     720
cagaaagcac tgcgcggcgg ttgctacacc gcaatcatct cgcccgaagg caaacatctg     780
tgcccaccgc tgtatgacgg agaaggaata attgtggcgg aattggactt cgcgttaatc     840
accaaacgta aacgcatgat ggattccgtc ggccattacg cgcgaccaga actactttct     900
ttgctcctcg atgatcgcgt aactgcgccg ctcaaaaact tacagacgac gatggcctct     960
gccaaatccg ctgaagatgg ttttccttta tttgcagacg ttttatatcc agacagttct    1020
ttcattgaga cgtcgaaatt cgcggagtca tga                                 1053

<210> 336
<211> 350
<212> PRT
<213> Unknown

<220>
<223> Obtained from an environmental sample

<400> 336
Met Thr Thr Arg Val Ile Lys Val Ala Ala Ala Gln Leu Ser Pro Val
1               5                   10                  15
Leu Ala Thr Ala Ser Glu His Ser Arg Glu Asp Thr Ile Ala Lys Val
            20                  25                  30
Ile Asp Ala Ile Ala Ala Ala Ser Gln Gln Gly Ala Gln Leu Ile Val
        35                  40                  45
Phe Pro Glu Thr Val Val Pro Tyr Tyr Pro Tyr Phe Ser Phe Ile Thr
    50                  55                  60
Pro Ala Val Thr Met Gly Ala Glu His Leu Lys Leu Tyr Glu Gln Ala
65                  70                  75                  80
Val Thr Val Pro Ser Ala Ala Thr Asp Ala Val Ala Ala Ala Ala Lys
                85                  90                  95
Asn Tyr Gly Met Val Val Val Leu Gly Ile Asn Glu Arg Asp His Gly
            100                 105                 110

Ser Leu Tyr Asn Ala Gln Leu Ile Phe Asp Ala Asp Gly Glu Leu Leu
        115                 120                 125
Leu Lys Arg Arg Lys Ile Thr Pro Thr Tyr His Glu Arg Met Val Trp
    130                 135                 140
Gly Gln Gly Asp Gly Ser Gly Leu Lys Val Val Asp Thr Ala Ala Gly
145                 150                 155                 160
Arg Val Gly Ala Leu Ala Cys Trp Glu His Tyr Asn Pro Leu Ala Arg
                165                 170                 175
Tyr Ser Leu Met Ala Gln His Glu Glu Ile His Cys Ser Gln Phe Pro
            180                 185                 190
Gly Ser Leu Val Gly Pro Ile Phe Ala Glu Gln Met Glu Ile Thr Met
        195                 200                 205
Arg His His Ala Leu Glu Ser Gly Cys Phe Val Val Asn Ala Thr Ala
    210                 215                 220
Trp Leu Ser Asp Thr Gln Ile Gln Ser Ile Thr Pro Asp Lys Ala Met
225                 230                 235                 240
Gln Lys Ala Leu Arg Gly Gly Cys Tyr Thr Ala Ile Ile Ser Pro Glu
                245                 250                 255
Gly Lys His Leu Cys Pro Pro Leu Tyr Asp Gly Glu Gly Ile Ile Val
            260                 265                 270
Ala Glu Leu Asp Phe Ala Leu Ile Thr Lys Arg Lys Arg Met Met Asp
        275                 280                 285
Ser Val Gly His Tyr Ala Arg Pro Glu Leu Leu Ser Leu Leu Leu Asp
    290                 295                 300
Asp Arg Val Thr Ala Pro Leu Lys Asn Leu Gln Thr Thr Met Ala Ser
305                 310                 315                 320
Ala Lys Ser Ala Glu Asp Gly Phe Pro Leu Phe Ala Asp Val Leu Tyr
                325                 330                 335
Pro Asp Ser Ser Phe Ile Glu Thr Ser Lys Phe Ala Glu Ser
            340                 345                 350

<210> 337
<211> 957
<212> DNA
<213> Unknown

<220>
<223> Obtained from an environmental sample

<400> 337
atgaagggag tgtgtgccat gtcgacgaaa gttgccatcg tccaggcgcc gccggtcctg      60
ctgcatcgcg acaggacgat tgcgaaggtg cgttcgtcga tcgaggatgc cgccaatgcg     120
ggcgcctcgc tgatcgtatt tcccgaagct tatgtacccg gctatccgag ttggatctgg     180
cgtctcaggc ccggaggcga catgggactg tcgtctgaga ttcacgcaag attgcgggaa     240
aatgccgttg atctcgcgaa cggaggcctg gcgcatgtcc agggggctgc agcaaaattc     300
ggcgcgactg tcgttatcgg catcaatgaa ctcgacagcg agttcagcgg aacgacattg     360
ttcaacaccg tggtggtcat cggccccgac ggaacgcgcc tcaacaggca tcgaaaatta     420
atgccgacca acccggagcg catggtgtgg ggcacgggcg atgcctcggg tctgcgtgtc     480
atcgatacgc cggcgggacg gctgggaacc atgatctgct gggagagcta catgccgctg     540
gcgcgctatg ctctctatgc gcaaggcatc gagatatacg tcgctcccac gtgggacgca     600
ggcgagagct ggattgctac gatgcgccac atcgccaagg aggccggctg ctgggtgatc     660
ggcacggcaa ccgtcatcca gggcagcgat gttccggacg attttcccga acgcgacaag     720
ctcttcaagc cggaggagtg gatcaacgac ggcgatgcgg tcgtggtcaa gcccatgggc     780
gcgattgctg ccggaccgca caatcgacag aaaagcatac tctacgccga catcgaccgg     840
gaggccgcgc ggcgagcccg ccggtcgctc gatgtctgtg gccactattc ccgcccagac     900
gttttctctt tctcggtcaa ccgaaagcca ttccgccctg ccgactttgt gggttga        957

<210> 338
<211> 313
<212> PRT
<213> Unknown

<220>
<223> Obtained from an environmental sample

<400> 338
Met Lys Gly Val Cys Ala Met Ser Thr Lys Val Ala Ile Val Gln Ala
1               5                   10                  15

Pro Pro Val Leu Leu His Arg Asp Gly Arg Leu Arg Arg Cys Val Arg
            20                  25                  30
Arg Ser Arg Met Pro Pro Met Arg Ala Pro Arg Ser Tyr Phe Pro Ala
        35                  40                  45
Tyr Val Pro Gly Tyr Pro Ser Trp Ile Trp Arg Leu Arg Pro Gly Gly
    50                  55                  60
Asp Met Gly Leu Ser Ser Glu Ile Thr Gln Asp Cys Gly Lys Met Pro
65                  70                  75                  80
Leu Ile Ser Arg Thr Glu Ala Trp Arg Met Ser Arg Gly Leu Gln Gln
                85                  90                  95
Asn Ser Ala Arg Leu Ser Leu Ser Ala Ser Met Asn Ser Thr Ala Ser
            100                 105                 110
Ser Ala Glu Arg His Cys Ser Thr Val Val Val Ile Gly Pro Asp Gly
        115                 120                 125
Thr Arg Leu Asn Arg His Arg Lys Leu Met Pro Thr Asn Pro Glu His
    130                 135                 140
Gly Val Gly His Gly Arg Cys Leu Gly Ser Ala Cys His Arg Tyr Ala
145                 150                 155                 160
Gly Gly Thr Ala Gly Asn His Ile Cys Trp Glu Ser Tyr Met Pro Leu
                165                 170                 175
Ala Arg Tyr Ala Leu Tyr Ala Gln Gly Ile Glu Ile Tyr Val Ala Pro
            180                 185                 190
Thr Trp Asp Ala Gly Glu Ser Trp Ile Ala Thr Met Arg His Ile Ala
        195                 200                 205
Lys Glu Ala Gly Cys Trp Val Ile Gly Thr Ala Thr Val Ile Gln Gly
    210                 215                 220
Ser Asp Val Pro Asp Asp Phe Pro Glu Arg Asp Lys Leu Phe Lys Pro
225                 230                 235                 240
Arg Ser Gly Ser Thr Thr Ala Met Arg Ser Trp Ser Ser Pro Trp Ala
                245                 250                 255
Arg Leu Leu Pro Asp Arg Thr Ile Asp Arg Lys Ala Tyr Ser Thr Pro
            260                 265                 270
Thr Ser Thr Gly Arg Pro Arg Gly Glu Pro Ala Gly Arg Ser Met Cys
        275                 280                 285
Gly His Tyr Ser Arg Pro Asp Val Phe Ser Phe Ser Val Asn Arg Lys
    290                 295                 300
Pro Phe Arg Pro Ala Asp Phe Val Gly
305                 310

<210> 339
<211> 1020
<212> DNA
<213> Unknown

<220>
<223> Obtained from an environmental sample

<400> 339
atgtcaggat cattcaaagt tgcagcagtg caggcggctc cggccttcct caatctcgat 60
gcgggcatcg acaaggcggt ggcgctgatc gagcaagccg ccgcgcagga cgttcagctc 120
atcgcctttc ccgagacctg gttgcccgga tacccctggt ggatctggct cgatgcaccc 180
gccgttacca tgggctatgt cgttccctac aatctcaatt cactagaggc gggcagcccg 240
caggacaagc gtctggcaaa tgccgcgcgt gagaacaaca tccaggtggt gatgggtctg 300
tctgaacgcc atgacggcac gctctacatc gcgcagtggc actatggtga agatggcgag 360
gtgatctcgc gacggcgcaa gctcaagccg acccatgtcg aacgcacggt gttcggggaa 420
ggcgacggca gcgacatggt ggtcaaggac acaagtctgg gacgggtcgg cgctctgtgc 480
tgttgggaac acctgcagcc gctcaacaaa tacgcgatgt actcccagaa cgagcagatc 540
cacatcggtt cctggcccag cttcagcctc tacaagggcg gcgcctatgc gcttggggca 600
gacctcaaca cggcggcaag ccagatgtat gcggccgagg gccagtgctt tgttctggct 660
gcctgcgcca cggtcagtca ggacatgttc gacatgctct gcgacacgga aatgaagcag 720
cagttcctga ccaccggtgg cggattcgcc cgcattttcg gacctgacgg ctcgcccatg 780
ggcaatgtgc ttgaagaaca tgaagaaggg ctggtgatcg ccgaaatcga tctcaccatg 840
attgcgatcg ccaaggcggc ggctgacccg tgcgggcact attcccggcc cgatgtgttc 900
cgactgatgt tcaaccagaa gccaagcccg gtggtgatgc cattcgaaaa cgacgtggcc 960
cgcgagattg tcgaggccgc cgaggatcag gtcagcgcaa accggctcgc agcggaatga 1020

<210> 340
<211> 329

<212> PRT
<213> Unknown

<220>
<223> Obtained from an environmental sample

<400> 340
Met Ser Gly Ser Phe Lys Val Ala Ala Val Gln Ala Ala Pro Ala Phe
1               5                   10                  15
Leu Asn Leu Asp Ala Gly Ile Asp Gly Gly Gly Ala Asp Arg Ala Ser
            20                  25                  30
Arg Arg Ala Gly Arg Ser Ala His Arg Leu Ser Arg Asp Leu Val Ala
        35                  40                  45
Gly Tyr Pro Trp Trp Ile Trp Leu Asp Ala Pro Ala Val Thr Met Gly
    50                  55                  60
Tyr Val Val Pro Tyr Asn Leu Asn Ser Arg Gly Gly Gln Pro Ala Gly
65                  70                  75                  80
Gln Ala Ser Gly Lys Cys Arg Ala Glu Gln His Pro Gly Gly Asp Gly
                85                  90                  95
Leu Ser Glu Arg His Asp Gly Thr Leu Tyr Ile Ala Gln Trp His Tyr
            100                 105                 110
Gly Glu Asp Gly Glu Val Ile Ala Thr Ala Gln Ala Gln Ala Asp Pro
        115                 120                 125
Cys Arg Thr His Gly Val Arg Gly Arg Arg Arg Gln Arg His Val Val
    130                 135                 140
Lys Asp Thr Ser Leu Gly Arg Val Gly Ala Leu Cys Cys Trp Glu His
145                 150                 155                 160
Leu Gln Pro Leu Asn Lys Thr Arg Cys Thr Pro Arg Thr Ser Arg Ser
                165                 170                 175
Thr Ser Val Pro Gly Pro Ala Ser Ala Ser Thr Arg Ala Ala Leu Cys
            180                 185                 190
Ala Trp Gly Arg Pro Gln His Gly Gly Lys Pro Asp Val Cys Gly Arg
        195                 200                 205
Gly Pro Val Leu Cys Ser Leu Pro Ala Pro Arg Ser Val Arg Thr Cys
    210                 215                 220
Ser Thr Cys Ser Ala Thr Arg Lys Ser Ser Ser Ser Thr Gly Gly Gly
225                 230                 235                 240
Phe Ala Arg Ile Phe Gly Pro Asp Gly Ser Pro Met Gly Asn Val Leu
                245                 250                 255
Glu Glu His Glu Arg Ala Gly Asp Arg Arg Asn Arg Ser His His Asp
            260                 265                 270
Cys Asp Arg Gln Gly Gly Gly Pro Val Arg Ala Leu Phe Pro Ala Arg
        275                 280                 285
Cys Val Pro Thr Asp Val Gln Pro Glu Ala Lys Pro Gly Gly Asp Ala
    290                 295                 300
Ile Arg Lys Thr Trp Pro Ala Arg Leu Ser Arg Pro Pro Arg Ile Arg
305                 310                 315                 320
Ser Ala Gln Thr Gly Ser Gln Arg Asn
                325

<210> 341
<211> 1056
<212> DNA
<213> Unknown

<220>
<223> Obtained from an environmental sample

<400> 341
atggcactga ccaacccaaa atataaagtc gccgccgtcc aggccgcgcc agcgttcctc      60
gatctggatg cgtccgtcga aaaagcggtc cggctgatcg acgaggccgg cgccaagggc     120
gcgcgcctca ttgcgttccc ggaaacctgg atccccggct atccctggtg gatctggctc     180
ggcgcgccgg cctgggcgat catgaaaggt tttgtctcgg cctatttcga caattcgctc     240
acctatgaca gtccggccgc ggacaaattg cgccaggccg ccaagcgcaa cgatatcgtc     300
gtggtgctcg gcctgtcgga gcgcgacggc ggcagtctct atatcgcgca atggatcatc     360
ggcccggacg gcgaaactgt cgcccagcgc cgcaagctca agccgaccca tgtcgagcgt     420
tcggtgttcg gcgagggcga tggcagcgac cttgccgtgc atgagctcgc gatcgggcgc     480
gtcggcgcgc tgtgctgctg ggagcatctg caaccgctgt cgaaatacgc catgtatgcg     540

cagaacgagc aggttcacgt ggcggcctgg ccgagctttt cgctgtacga tccgttcgcc 600
catgccctcg gtgcggaggt caacaacgcc gccagcaaga tctacgcggt cgagggatcg 660
tgcttcgtcg tggcaccctg cgccaccgtt tcaaaggaaa tgatcgacct gttgtgcgac 720
acgccggaca agcacggtct gctgcacgcc ggcggcgggt ttgccgcgat ctacggccct 780
gacggctcgc cgatcggcga ccgcctggcg cccgaccagg aaggtctgat ctatgccgat 840
gtcgatctcg gcatgatctc ggtcgcgaag gccgccgccg atccggccgg acattatgcg 900
cggccggatg tcacaaggtt gctgctcaac aaacgcccgg gcaatcgtgt cgaggcgctg 960
gcacttccgg tggatcaggt tgcggcaggt gaggagatcc cctcgatatc gcgatcggcc 1020
agaggggttg ccgaactgcc aaacgcggcc gaatag 1056

<210> 342
<211> 342
<212> PRT
<213> Unknown

<220>
<223> Obtained from an environmental sample

<400> 342
Met Ala Leu Thr Asn Pro Lys Tyr Lys Val Ala Ala Val Gln Ala Ala
1               5                   10                  15
Pro Ala Phe Leu Asp Leu Asp Ala Pro Ser Lys Lys Arg Ser Gly Ser
            20                  25                  30
Thr Arg Pro Ala Pro Arg Ala Arg Ala Ser Leu Arg Ser Arg Thr Trp
        35                  40                  45
Ile Pro Gly Tyr Pro Trp Trp Ile Trp Leu Gly Ala Pro Ala Trp Ala
    50                  55                  60
Ile Met Lys Gly Phe Val Ser Pro Ile Ser Thr Ile Arg Ser Pro Met
65                  70                  75                  80
Thr Val Arg Pro Arg Thr Asn Cys Ala Arg Pro Pro Ser Ala Thr Tyr
                85                  90                  95
Arg Arg Gly Ala Arg Pro Val Gly Ala Arg Arg Arg Gln Ser Leu Tyr
            100                 105                 110
Arg Ala Met Asp His Arg Pro Thr Ala Lys Leu Ser Pro Ser Ala Ala
        115                 120                 125
Ser Ser Ser Arg Pro Met Ser Ser Val Arg Cys Ser Ala Arg Ala Met
    130                 135                 140
Ala Ala Thr Leu Pro Cys Met Ser Ser Arg Ser Gly Ala Ser Ala Arg
145                 150                 155                 160
Cys Ala Ala Gly Ser Ile Cys Thr Ala Val Glu Ile Arg His Val Cys
                165                 170                 175
Ala Glu Arg Ala Gly Ser Arg Gly Gly Leu Ala Glu Leu Phe Ala Thr
            180                 185                 190
Ile Arg Ser Pro Met Pro Ser Val Arg Arg Ser Thr Thr Pro Pro Ala
        195                 200                 205
Arg Ser Thr Arg Ser Arg Asp Arg Ala Ser Ser Trp His Pro Ala Pro
    210                 215                 220
Pro Phe Gln Arg Lys Ser Thr Cys Cys Ala Thr Arg Arg Thr His Gly
225                 230                 235                 240
Leu Leu His Ala Gly Gly Gly Phe Ala Ala Ile Tyr Gly Pro Asp Gly
                245                 250                 255
Ser Pro Ile Gly Asp Arg Trp Arg Pro Thr Arg Lys Val Ser Met Pro
            260                 265                 270
Met Ser Ile Ser Ala Ser Arg Ser Arg Arg Pro Pro Asp Pro Ala Gly
        275                 280                 285
His Tyr Ala Arg Pro Asp Val Thr Arg Leu Leu Leu Asn Lys Arg Pro
    290                 295                 300
Gly Asn Arg Val Arg Arg Trp His Phe Arg Trp Ile Arg Leu Arg Gln
305                 310                 315                 320
Val Arg Arg Ser Pro Arg Tyr Arg Asp Arg Pro Glu Gly Cys Arg Thr
                325                 330                 335
Ala Lys Arg Gly Arg Ile
            340

<210> 343
<211> 942
<212> DNA
<213> Unknown

<220>
<223> Obtained from an environmental sample

<400> 343
atgaagtcaa aaattgcggt tattcagcga cctcctgtat tgttagacct tcaggcttca      60
atcgcccgtg ccattacttc tgttgaggaa gcggcaggca agggatctga gctccttgtt     120
tttcccgaga catttctgcc tggttatccg tcctggatct ggcgtctcaa gccgggcgga     180
gacatggtgc tgacatctga aatccacgca aaatatcgcg cgaactctgt tgatgttgag     240
cgcggggatc tggccccttt atgcgaagcg gcggcgaaac acggcgtcac aattgtcatg     300
gggctcagtg aaattgatgg gcgctacagc gggactacac tctttaatac agtggtgacc     360
attggcgcgg aaggagagct ccttaataga caccgcaagc tcatgccgac aaacccagag     420
cgtatggtct gggggcaagg ggatgcctct ggtctgcggg ttgtcgacac gcccgtgggc     480
cgcgtcggca cgctgatctg ctgggaaaac tacatgccgc tatcgcgcta tgcgctttat     540
tctcaaaaca ttgacatcta tgtggcgccg acctgggacg cgggcgagag ctggatcgcc     600
tccatgcagc atatcgccaa agaaggtggc tgctgggtga tcggcacggc cacggcgatg     660
gagggctctg atgtcccagc cgacttccct cagcgggagg tgcttttccc tgatagcagc     720
gaatggatca atgacggtga cgctgtagtg gttaaaccca tgggggcgat tgtcgcgggt     780
ccgcatcacc gggataagag tattctctat gctgagattg acgtcgaagt ggcacgcaat     840
gcgcggcgct cgctcgatgt ggcggggcat tactcccggc cggatatttt ttcctttggc     900
gtggatcgcc ggcctttgcc gccggttacg tttgaggatt ga                        942

<210> 344
<211> 303
<212> PRT
<213> Unknown

<220>
<223> Obtained from an environmental sample

<400> 344
Met Lys Ser Lys Ile Ala Val Ile Gln Arg Pro Pro Val Leu Leu Asp
1               5                   10                  15
Leu Gln Ala Ser Ile Ala Arg Ala Ile Thr Leu Leu Arg Lys Arg Gln
            20                  25                  30
Ala Arg Asp Leu Ser Ser Leu Phe Phe Pro Arg His Phe Cys Leu Val
        35                  40                  45
Ile Arg Pro Gly Ser Gly Val Ser Ser Arg Ala Glu Thr Trp Cys His
    50                  55                  60
Leu Lys Ser Thr Gln Asn Ile Ala Arg Thr Leu Asp Val Glu Arg Gly
65                  70                  75                  80
Asp Leu Ala Pro Leu Cys Glu Ala Ala Ala Lys His Gly Val Thr Ile
                85                  90                  95
Val Met Gly Gln Asn Trp Ala Leu Gln Arg Asp Tyr Thr Leu Tyr Ser
            100                 105                 110
Gly Asp His Trp Arg Gly Arg Arg Leu Leu Asn Arg His Arg Lys Leu
        115                 120                 125
Met Pro Thr Asn Pro Glu Arg Met Val Trp Gly Gln Gly Asp Ala Ser
    130                 135                 140
Val Cys Gly Leu Ser Thr Arg Pro Trp Ala Ala Ser Ala Arg Ser Ala
145                 150                 155                 160
Gly Lys Thr Thr Cys Arg Tyr Arg Tyr Ala Leu Tyr Ser Gln Asn Ile
                165                 170                 175
Asp Ile Tyr Val Ala Pro Thr Trp Asp Ala Gly Glu Ser Trp Ile Ala
            180                 185                 190
Ser Met Gln His Ile Ala Lys Glu Gly Gly Cys Trp Val Ile Gly Thr
        195                 200                 205
Ala Thr Ala Met Glu Gly Ser Asp Ser Gln Pro Thr Ser Leu Ser Gly
    210                 215                 220
Arg Cys Phe Ser Leu Ile Ala Ala Asn Gly Ser Met Thr Val Thr Leu
225                 230                 235                 240
Trp Leu Asn Pro Trp Gly Arg Leu Ser Arg Val Arg Ile Thr Gly Ile
                245                 250                 255
Arg Val Phe Ser Met Leu Arg Leu Thr Arg Ser Gly Thr Gln Cys Ala
            260                 265                 270
Ala Leu Ala Arg Cys Gly Gly Ala Leu Leu Pro Ala Gly Tyr Phe Phe
        275                 280                 285
Leu Trp Val Asp Arg Arg Pro Leu Pro Pro Val Thr Phe Glu Asp

290                 295                 300

<210> 345
<211> 1011
<212> DNA
<213> Unknown

<220>
<223> Obtained from an environmental sample

<400> 345
atgaaagcga tcaaggccgc tgccgtgcag gcagcaccgg tattcctgaa cctcgacgca      60
tcgatcacca aggcggaaac attcgtcgcc gaggccgccg cgaatggtgc caagctggtg     120
gcgtttccgg aaacctggct gccgggctat ccctggttca tctggctcgg tgcgcccgcc     180
gaaggcatgc agttcatccc gcgctatcac gaaaacagca tggagctccg ctcgcccgag     240
atgcgccgct tgcaggcgat cgcgcgcaag tatgaagtga cgctcgtcat gggctattcc     300
gagcgcgatg gtggcagccg ctacatgtcc caggtcatta ttggcgatca gggcgacatc     360
cttctcaatc gccgtaaatt gaagccaacc catgtcgagc ggacggtctt cggcgaaggc     420
gacggttcgg acctggtggt ggtcgaaacg gcattcggca ggctcggtgc gctcaattgc     480
tgggaacata tccagccgct cgtcaagatg tcgatgtatg cccagcatga ggaaatccat     540
gtcgcgggtt ggccgagctt ctgcgtctac cgcgatctcg cctatgccct gggaccggaa     600
gtcaacaatg ccgtcagtca ggtctatgcc gtggagggta gcgcctatgt tctggcaccc     660
tgtgcgatcg taagccagga gatgttcgac attctggccg acaagcctga aaaggccttt     720
ctcctcaatc cccgcacatc caagcccggc ggtggcttca cgcagatcta tgcgccggat     780
ggtcgaccgc tttgcgagcc gcttgccgac gatgtggaag gcatcctcta tgccgatctc     840
gatccggcaa cgatcgccgt cgcgaaggcg gccgccgatc ctgcggggca ctattcgcgg     900
ccggacgcac tctcgctggt gatcaatcgc gaaaagcgcg cggtgatggc tgaaatcaac     960
gcgccggcga cgccgacctt cacccccatc tccctggacg ctgcggagta g             1011

<210> 346
<211> 329
<212> PRT
<213> Unknown

<220>
<223> Obtained from an environmental sample

<400> 346
Met Lys Ala Ile Lys Ala Ala Ala Val Gln Ala Ala Pro Val Phe Leu
1               5                   10                  15
Asn Leu Asp Ala Ser Ile Thr Lys Ala Glu Thr Phe Val Ala Glu Ala
            20                  25                  30
Ala Ala Asn Gly Ala Lys Leu Val Ala Phe Pro Glu Thr Trp Leu Pro
        35                  40                  45
Gly Tyr Pro Trp Phe Ile Trp Leu Gly Ala Pro Ala Glu Gly Met Gln
    50                  55                  60
Phe Ile Pro Arg Tyr His Glu Asn His Gly Ala Pro Leu Ala Arg Asp
65                  70                  75                  80
Ala Pro Leu Ala Gly Asp Arg Ala Gln Val Ser Asp Ala Arg His Gly
                85                  90                  95
Tyr Ser Glu Arg Asp Gly Gly Ser Arg Tyr Met Ser Gln Val Ile Ile
            100                 105                 110
Gly Asp Gln Gly Asp Ile Leu Leu Asn Arg Arg Lys Leu Lys Pro Thr
        115                 120                 125
His Val Glu Arg Thr Val Phe Gly Glu Gly Asp Gly Ser Asp Leu Val
    130                 135                 140
Trp Ser Lys Arg His Ser Ala Gly Ser Val Arg Ser Ile Ala Gly Asn
145                 150                 155                 160
Ile Ser Ser Arg Ser Ser Arg Cys Met Tyr Ala Gln His Glu Glu Ile
                165                 170                 175
His Val Ala Gly Trp Pro Ser Phe Cys Val Tyr Arg Asp Leu Ala Tyr
            180                 185                 190
Ala Trp Asp Arg Lys Ser Thr Met Pro Ser Val Arg Ser Met Pro Trp
        195                 200                 205
Arg Val Ala Pro Met Phe Trp His Pro Val Arg Ser Ala Arg Arg Cys
    210                 215                 220
Ser Thr Phe Trp Pro Thr Ser Leu Lys Arg Pro Phe Ser Ser Ile Pro
225                 230                 235                 240

Ala His Pro Ser Pro Ala Val Ala Ser Arg Arg Ser Met Arg Arg Met
                245                 250                 255
Val Asp Arg Phe Ala Ser Arg Leu Pro Thr Val Glu Gly Ile Leu Tyr
            260                 265                 270
Ala Asp Leu Asp Pro Ala Thr Ile Ala Val Ala Lys Ala Ala Ala Asp
        275                 280                 285
Pro Ala Gly Thr Ile Arg Gly Arg Thr His Ser Arg Trp Ser Ile Ala
    290                 295                 300
Lys Ser Ala Arg Trp Leu Lys Ser Arg Ala Gly Asp Ala Asp Leu His
305                 310                 315                 320
Pro His Leu Pro Gly Arg Cys Gly Val
                325

<210> 347
<211> 909
<212> DNA
<213> Unknown

<220>
<223> Obtained from an environmental sample

<400> 347
atgaaagtcg cggcgattca agcggcgccg gtctacttgg accggcaagc cacgctggaa      60
aaggcgcttt ctctaatgga tgaagcggcc gcaaacggcg cccaagtctg cgccttccct     120
gagaccttcc tcgcgggcta tcccgtctgg atggacttga cggacggcgc caagtggaat     180
gacgacaaac aaaaagcggc ctacgcatgt tatgtagatg cggccgtcga agccgacgga     240
cctgagttgc aagccattgc caagaaatcg aaggcgctcg gcctgttcac ctatctgggc     300
atggtcgaac gcgcggcgtc ggccgggtca gtatattgtt ccctcgctgc cttcgatccc     360
gacaagggtc tcgtcagcct acaccgaaaa ctcatgccca cctacaccga gcgcctcgtg     420
tggagccaag gcgacggaca cggactccag gtgcatgaat tcgccggctt caaaatcggc     480
gcgctaaact gctgggaaaa ttggatgccc ctcgcccgtt acgcaatgta cgcccagggc     540
gaacagctcc acgtcgcgac ctggcccggg tccccctggc tcaccaagga catcacccgt     600
ttcatcgccc ttgagggacg catctacgtc atgtccgttg gcggcgtcct tagcgcaaac     660
gatatccccg actccttccc gcttaaaacc gacctcctca agatccgaga ccgctacctc     720
agcggcggca ccatgatagt cgccccagac ggcaccaccc tcgaaggccc cgccaaaaac     780
gaagagacca tactctacgc ggagctcgac ctcaacaccg tcctccaaga acgccaaaat     840
ttcgatcccg cgggacacta cgcgcgccca gacgtcttcc aattggaaat cgacaaaaat     900
cgacgatag                                                             909

<210> 348
<211> 297
<212> PRT
<213> Unknown

<220>
<223> Obtained from an environmental sample

<400> 348
Met Lys Val Ala Ala Ile Gln Ala Ala Pro Val Tyr Leu Asp Arg Gln
 1               5                  10                  15
Ala Thr Leu Glu Lys Ala Leu Ser Trp Met Lys Arg Pro Gln Thr Ala
            20                  25                  30
Pro Lys Ser Ala Pro Ser Leu Arg Pro Ser Ser Arg Ala Ile Pro Trp
        35                  40                  45
Met Asp Leu Thr Asp Gly Ala Lys Trp Asn Asp Asp Lys Gln Lys Ala
    50                  55                  60
Ala Tyr Ala Cys Tyr Val Asp Ala Ala Val Glu Ala Asp Gly Pro Glu
65                  70                  75                  80
Leu Gln Ala Ile Ala Lys Lys Ser Lys Ala Leu Gly Leu Phe Thr Ile
                85                  90                  95
Trp Ala Trp Ser Asn Ala Arg Arg Arg Pro Gly Gln Tyr Ile Val Pro
            100                 105                 110
Ser Leu Pro Ser Ile Pro Thr Arg Val Ser Ser Ala Tyr Thr Glu Asn
        115                 120                 125
Ser Cys Pro Pro Thr Pro Ser Ala Ser Cys Gly Ala Lys Ala Thr Asp
    130                 135                 140
Thr Asp Ser Arg Cys Met Asn Ser Pro Ala Ser Lys Ser Ala Arg Thr
145                 150                 155                 160

Ala Gly Lys Ile Gly Cys Pro Ala Arg Tyr Ala Met Tyr Ala Gln Gly
                165                 170                 175
Glu Gln Leu His Val Ala Thr Trp Pro Gly Ser Pro Trp Leu Thr Lys
            180                 185                 190
Asp Ile Thr Arg Phe Ile Ala Leu Glu Gly Arg Ile Tyr Val Met Ser
        195                 200                 205
Val Gly Gly Val Leu Ser Ala Asn Asp Ile Pro Asp Ser Phe Pro Leu
    210                 215                 220
Lys Thr Asp Leu Leu Lys Ile Arg Asp Arg Tyr Leu Ser Gly Gly Thr
225                 230                 235                 240
Asp Ser Arg Pro Arg Arg His His Pro Arg Arg Pro Arg Gln Lys Arg
                245                 250                 255
Arg Asp His Thr Leu Arg Gly Leu Asp Leu Asn Thr Val Leu Gln Glu
            260                 265                 270
Arg Gln Asn Phe Asp Pro Ala Gly His Tyr Ala Arg Pro Asp Val Ser
        275                 280                 285
Asn Trp Lys Ser Thr Lys Ile Asp Asp
    290                 295

<210> 349
<211> 1002
<212> DNA
<213> Unknown

<220>
<223> Obtained from an environmental sample

<400> 349
atgacgtccc ctgttcaaac caagtacaaa gtcgcctgtg ttcaggcggc gcccgagttt      60
ctcgatctcg acaaaggcgt tgccaaagcg gtgcgcctga tcgaagaagc cgccacccaa     120
aaggcctcgc tgatcgcgtt tcccgaagtc tggctccccg gctatccgtg gtggatctgg     180
ctcgactcgc cggcctgggg cttgcagttc gtccagcgct acttcgaaaa cgctctggtc     240
gtcggcagcc cccaatggga gcgcttgtgc aaggccgccg ccgacaacaa tatccatgtt     300
gtgctcggat tctccgaacg ggacggcagc acgctgtacc tcgcacaggc catcatcgat     360
aacaccggga aggtgatcgc cacgcggcgc aaactcaagc caacacacgc cgaacgcacg     420
gttttcggcg aaggcgacgg gagccacatc gcggtgcatg aaaccacttt gggccgcatg     480
ggtgcactct gctgcgccga gcacatccag ccactgacca agtacgccat gtactcgcag     540
cacgagcaga ttcacattgc cgcatggccc agcttctcgg tctaccgcgg agcagcgttc     600
cagctgagcg ccgaagccaa caacgccgcg agccaggtct atgccctgga gggcagttgc     660
tacgtggtgg ccccttgcgc gacggtgtcc aaggagatgt tggacatgct ggctgattcg     720
ccgcaaaaga agcagctcct gctggaaggc ggtggctacg ccatgattta tgggcccgac     780
gccaagcccc tgtgcgagcc cattccagag acagaagaag gcattcttta cgcagatgtg     840
gacctgggct tcatcggtgt caccaaggca gcgtatgacc ccgccggtca ctattcacgc     900
cccgacgtgc tgcgcctttt gttcaatcgg aagcctgccc ctcgggttca cgatttcgat     960
cctgaataca cggccaccga gcagaagaca gacgcggcct ga                       1002

<210> 350
<211> 333
<212> PRT
<213> Unknown

<220>
<223> Obtained from an environmental sample

<400> 350
Met Thr Ser Pro Val Gln Thr Lys Tyr Lys Val Ala Cys Val Gln Ala
1               5                   10                  15
Ala Pro Glu Phe Leu Asp Leu Asp Lys Gly Val Ala Lys Ala Val Arg
            20                  25                  30
Leu Ile Glu Glu Ala Ala Thr Gln Lys Ala Ser Leu Ile Ala Phe Pro
        35                  40                  45
Glu Val Trp Leu Pro Gly Tyr Pro Trp Trp Ile Trp Leu Asp Ser Pro
    50                  55                  60
Ala Trp Gly Leu Gln Phe Val Gln Arg Tyr Phe Glu Asn Ala Leu Val
65                  70                  75                  80
Val Gly Ser Pro Gln Trp Glu Arg Leu Cys Lys Ala Ala Ala Asp Asn
                85                  90                  95
Asn Ile His Val Val Leu Gly Phe Ser Glu Arg Asp Gly Ser Thr Leu

                100                 105                 110
Tyr Leu Ala Gln Ala Ile Ile Asp Asn Thr Gly Lys Val Ile Ala Thr
        115                 120                 125
Arg Arg Lys Leu Lys Pro Thr His Ala Glu Arg Thr Val Phe Gly Glu
    130                 135                 140
Gly Asp Gly Ser His Ile Ala Val His Glu Thr Thr Leu Gly Arg Met
145                 150                 155                 160
Gly Ala Leu Cys Cys Ala Glu His Ile Gln Pro Leu Thr Lys Tyr Ala
                165                 170                 175
Met Tyr Ser Gln His Glu Gln Ile His Ile Ala Ala Trp Pro Ser Phe
                180                 185                 190
Ser Val Tyr Arg Gly Ala Ala Phe Gln Leu Ser Ala Glu Ala Asn Asn
        195                 200                 205
Ala Ala Ser Gln Val Tyr Ala Leu Glu Gly Ser Cys Tyr Val Val Ala
    210                 215                 220
Pro Cys Ala Thr Val Ser Lys Glu Met Leu Asp Met Leu Ala Asp Ser
225                 230                 235                 240
Pro Gln Lys Lys Gln Leu Leu Leu Glu Gly Gly Gly Tyr Ala Met Ile
                245                 250                 255
Tyr Gly Pro Asp Ala Lys Pro Leu Cys Glu Pro Ile Pro Glu Thr Glu
                260                 265                 270
Glu Gly Ile Leu Tyr Ala Asp Val Asp Leu Gly Phe Ile Gly Val Thr
        275                 280                 285
Lys Ala Ala Tyr Asp Pro Ala Gly His Tyr Ser Arg Pro Asp Val Leu
    290                 295                 300
Arg Leu Leu Phe Asn Arg Lys Pro Ala Pro Arg Val His Asp Phe Asp
305                 310                 315                 320
Pro Glu Tyr Thr Ala Thr Glu Gln Lys Thr Asp Ala Ala
                325                 330

<210> 351
<211> 936
<212> DNA
<213> Unknown

<220>
<223> Obtained from an environmental sample

<400> 351
atgattacag caggcatcgc agtcgccgct ccggtggtgt tggacaaaac aaaaaccatt      60
gagaaagccg ttggcattat tcacgaggca gcgggtaagg gtgtgaacct gcttgtgttt     120
cccgaggcat ttattccctc ctatcccgcc tggggttggc gcctgcgtcc cggtggagat     180
ttcgggttgt gcgaggagtt gcacgccctg ttgcttgata attcggtaaa tttgcaaggt     240
gatgacctgg accctgtccg gggcgctgca gccgagcatt caatgaccgt ggtgatggga     300
ttgaatgagc gcgaaggcca gttcggtcgg gctaccctgt ttaacgccat ggtatttatc     360
ggtccggacg gcagcatcct gaaccatcat cggaaactta tgccaaccaa tcatgagcgt     420
acgattcatg gcttcggcga tgcgcgggga ttgaaagtgg tggatacccc gtgcggtcgc     480
gtgggtggtc tgatttgctg ggagaatttc atgcccctgg ctcgctacgg cctgtatgcc     540
cagggcgtag aagtgtatgt tgcgcccacc tacgaccagg gtgatgggtg gataggatcc     600
atgcagcata ttgcccggga aggacggtgc tgggtgttat cggccggaac accgctacgc     660
ggcagtgatt ttcccgcgga catgccgggc aaggctcaac tgtttcccga tgacgatgaa     720
tgggtgaatc ccggtgggtc agtggttatc gcaccgggtg gggaattagt ggctggaccg     780
cttttccgtg aggagggcat ccttgtctgt gaattggatc cggcgaaaag tgctcatgcc     840
aagcggtcct ttgacgtggc cggtcattac gccaggccag atattttcga gttggaaata     900
gaccgtgatc cacaggatcc cgtcgagtgg gactga                               936

<210> 352
<211> 301
<212> PRT
<213> Unknown

<220>
<223> Obtained from an environmental sample

<400> 352
Met Ile Thr Ala Gly Ile Ala Val Ala Ala Pro Val Val Leu Asp Lys
1               5                   10                  15
Thr Lys Thr Ile Glu Lys Ala Val Ala Leu Phe Thr Arg Gln Arg Val

                    20                  25                  30
Arg Val Thr Cys Leu Cys Phe Pro Arg His Leu Phe Pro Pro Ile Pro
        35                  40                  45
Pro Gly Val Gly Ala Cys Val Pro Val Glu Ile Ser Gly Cys Ala Arg
    50                  55                  60
Ser Cys Thr Pro Cys Cys Leu Ile Ile Arg Asn Leu Gln Gly Asp Asp
65                  70                  75                  80
Leu Asp Pro Val Arg Gly Ala Ala Ala Glu His Ser Met Thr Val Val
                85                  90                  95
Met Gly Glu Ala Arg Arg Pro Val Arg Ser Gly Tyr Pro Val Arg His
            100                 105                 110
Gly Ile Tyr Arg Ser Gly Arg Gln His Pro Glu Pro Ser Ser Glu Thr
        115                 120                 125
Tyr Ala Asn Gln Ser Ala Tyr Asp Ser Trp Leu Arg Arg Cys Ala Gly
    130                 135                 140
Ile Glu Ser Gly Gly Tyr Pro Val Arg Ser Arg Gly Trp Ser Asp Leu
145                 150                 155                 160
Leu Gly Glu Phe His Ala Pro Gly Ser Leu Gly Leu Tyr Ala Gln Gly
                165                 170                 175
Val Glu Val Tyr Val Ala Pro Thr Tyr Asp Gln Gly Asp Gly Trp Ile
            180                 185                 190
Gly Ser Ala Ala Tyr Cys Pro Gly Arg Thr Val Leu Gly Val Ile Gly
        195                 200                 205
Arg Asn Thr Ala Thr Arg Gln Phe Ser Arg Thr Cys Arg Ala Arg Leu
    210                 215                 220
Asn Cys Phe Pro Met Thr Met Asn Gly Ile Pro Val Gly Gln Trp Leu
225                 230                 235                 240
Ala Pro Gly Gly Glu Leu Val Ala Gly Pro Leu Phe Arg Glu Glu Gly
                245                 250                 255
Ile Leu Val Cys Glu Leu Asp Pro Ala Lys Ser Ala His Ala Lys Arg
            260                 265                 270
Ser Phe Asp Val Ala Gly His Tyr Ala Arg Pro Asp Ile Phe Glu Leu
        275                 280                 285
Glu Ile Asp Arg Asp Pro Gln Asp Pro Val Glu Trp Asp
    290                 295                 300

<210> 353
<211> 1035
<212> DNA
<213> Psuedomonas putida ATCC 700801

<400> 353
atgaatgctt caaaaacaca ttataaagtt gcagctgtgc aagccgctcc tgaattcctg      60
gatttggaca aaggggttga taaggctata cgcctcatta aagaagcagc tgataacgga     120
gcatcgctgg ttgcctttcc ggaagtttat cttccagggt atccgtggtg gatctggctt     180
ggctctcccg catggggaat gcagtttgtt cagcgctacg tagaaaactc gcttgattta     240
aaaagcgagc agttcgagcg actgtgcaaa gcggctgcta cttaccgtat tcacgttgta     300
atgggttaca gcgaacgctc gtttggcacc ctctacctcg gtcaagcaat tatcgatgac     360
agcgggaaag taattggtac gcgccgtaag ctcaagccca cccatgctga gcgtactgtt     420
tacggtgagg gtaacggcag tgacctcagg gttttcaatt cgcaacttgg aagggtaggt     480
gcactctgct gcgcagagca cgtacaacca ctctcgaagt ttgcgatgta cagccagcat     540
gagcagttgc acattgcctc ttggccgagc ttctcggtat atcgcggtgg cgcatatcaa     600
ctgagcgctg aggctaactg cgcggccacc caagtctatg ctcttgaagg ccagtgcttc     660
gtaatttcag catgcgcaat cgtatctaaa gacatgctaa acgttctcat cgacacccct     720
gacaagggta acctgttgca ggatggcggt ggcttcgcga tgatttatgg ccccgatggc     780
gcaccgctgt gtgaacccct gggcgaacat gaagaaggca tcctttatgc cgacgtcgat     840
ttgggcgcca tttccgtagc taaagcggca ctcgacccgg ttggccatta ctcgcggcca     900
gatgttttgc gtctgctttt caacgatcaa ccgacacctt gcgtggaggc gttcaatccg     960
gctcctgtcg gtactgatgc tccaggtaca gaccttcagg gtgatgagcc cgatgcgcaa    1020
ccgatatctg aataa                                                     1035

<210> 354
<211> 312
<212> PRT
<213> Psuedomonas putida ATCC 700801

<400> 354
Met Asn Ala Ser Lys Thr His Tyr Lys Val Ala Ala Val Gln Ala Ala

1                   5                   10                  15
Pro Glu Phe Leu Asp Leu Asp Lys Gly Val Asp Lys Ala Ile Arg Leu
                20                  25                  30
Ile Lys Glu Ala Ala Asp Asn Gly Ala Ser Leu Val Ala Phe Pro Glu
            35                  40                  45
Val Tyr Ser Arg Val Ser Val Val Asp Leu Ala Trp Leu Ser Arg Met
    50                  55                  60
Gly Asn Ala Val Cys Ser Ala Leu Arg Arg Lys Arg Leu Ile Lys Ala
65                  70                  75                  80
Ser Ser Ser Ser Asp Cys Ala Lys Arg Leu Leu Leu Thr Val Phe Thr
                85                  90                  95
Leu Trp Leu Gln Arg Thr Leu Val Trp His Pro Leu Pro Arg Ser Ser
            100                 105                 110
Asn Tyr Arg Gln Arg Glu Ser Asn Trp Tyr Ala Pro Ala Gln Ala His
        115                 120                 125
Pro Cys Ala Tyr Cys Leu Arg Gly Arg Gln Pro Gln Gly Phe Gln Phe
    130                 135                 140
Ala Thr Trp Lys Gly Arg Cys Thr Leu Leu Arg Arg Ala Arg Thr Thr
145                 150                 155                 160
Thr Leu Glu Val Cys Asp Val Gln Pro Ala Ala Val Ala His Cys Leu
                165                 170                 175
Leu Ala Glu Leu Leu Gly Ile Ser Arg Trp Arg Ile Ser Thr Glu Arg
            180                 185                 190
Gly Leu Arg Gly His Pro Ser Leu Cys Ser Arg Pro Val Leu Arg Asn
        195                 200                 205
Phe Ser Met Arg Asn Val Ser Lys Asp Met Leu Asn Val Leu Ile Asp
    210                 215                 220
Thr Pro Asp Lys Gly Asn Leu Leu Gln Asp Gly Gly Gly Phe Ala Met
225                 230                 235                 240
Ile Tyr Gly Pro Asp Gly Ala Pro Leu Cys Glu Pro Leu Gly Glu His
                245                 250                 255
Glu Glu Gly Ile Leu Tyr Arg Arg Arg Phe Gly Arg His Phe Arg Ser
            260                 265                 270
Ser Gly Thr Arg Pro Gly Trp Pro Leu Leu Ala Ala Arg Cys Leu Arg
        275                 280                 285
Leu Leu Phe Asn Asp Gln Pro Thr Pro Cys Val Glu Ala Phe Asn Pro
    290                 295                 300
Ala Pro Val Gly Thr Asp Ala Pro
305                 310

<210> 355
<211> 1014
<212> DNA
<213> Unknown

<220>
<223> Obtained from an environmental sample

<400> 355
atggccgtct ctaaagacgg tactgtttca ggaaagtcgc ctatccgatt gcatgtcgcc      60
gcgatacaga tggtcccaaa gctgggtgac gcgcaggcga acgtgaatca ggcagaagcc     120
cttattcgga aggctcttgg gctgggtgcg cgttggatcg tgttaccaga gatgtttacc     180
tccggtgcgg cgtttcatcc cgacatgctc aaagccattc agccattcga tggcgcccca     240
ctccagttgc tgaaagacct ttctcgcaag ggcaatgctg tcatcggcgg ctcgtttctc     300
gccaagcgtg ggcaacaagt attcaatacc ttcgttttgg tttctccgga cgggtcagtc     360
gtaacgcatg acaaggattc accgacctat tgggaaaatt gctattaccg gggcggtacc      420
gatgatggcg tgttgtctac gcccattggc ccggtcggct ccgtcctctg ttgggaattt     480
atccgctcaa gaaccgcgag acggctggcg aacaaggtca agatggtcgt gggaggctcc     540
tgttggtgga cgctccccga tgatgctgat ccagacagcc cgcgcagagc cgtgaacctc     600
aagatgctgc aagaagcgcc ggttcgcatg gcgcggatgc tgggtgttcc ggtaatacat     660
ggctcccacg cgggcagctt cgaaggattc ttcagtccgg aacttgcgga tgttccctat     720
aactcgacgt acctgggcga gacaatgatt gtcgacgcgg gtggccgggt acttgcccgt     780
agagcgcaag atgcaggcga aggcgtggta acggcagaag tggttttgcc cgacaagtcc     840
gtaccaagcg aacccatccc ggagactttc tggattccca aggaaatgcc ggatgattgg     900
aaagaagcct gggagcgttg gttcgatacc ggtgcggatt actacgagat ggtgaccgcg     960
ccctttatca agacgggtgt gataaacgag tacacaccgg aatatcttag gtag          1014

<210> 356

<211> 325
<212> PRT
<213> Unknown

<220>
<223> Obtained from an environmental sample

<400> 356
Met Ala Val Ser Lys Asp Gly Thr Val Ser Gly Lys Ser Pro Ile Arg
1               5                   10                  15
Leu His Val Ala Ala Ile Gln Met Val Gln Ser Trp Val Thr Arg Arg
            20                  25                  30
Arg Thr Ile Arg Gln Lys Pro Leu Phe Gly Arg Leu Leu Gly Trp Val
        35                  40                  45
Arg Val Gly Ser Cys Tyr Gln Arg Cys Leu Pro Pro Val Arg Arg Phe
    50                  55                  60
Ile Pro Thr Cys Ser Lys Pro Phe Ser Phe Asp Gly Ala Pro Leu Gln
65                  70                  75                  80
Leu Leu Lys Asp Leu Ser Arg Lys Gly Asn Ala Val Ile Gly Gly Ser
                85                  90                  95
Phe Leu Gln Ala Trp Ala Thr Ser Ile Gln Tyr Leu Arg Phe Gly Phe
            100                 105                 110
Ser Gly Arg Val Ser Arg Asn Ala Gln Gly Ser Pro Thr Tyr Trp Glu
        115                 120                 125
Asn Cys Tyr Tyr Arg Gly Gly Thr Asp Asp Gly Val Leu Ser Thr Pro
    130                 135                 140
Ile Gly Pro Ser Ala Pro Ser Ser Val Gly Asn Leu Ser Ala Gln Glu
145                 150                 155                 160
Pro Arg Asp Gly Trp Arg Thr Arg Ser Arg Trp Val Gly Gly Ser Cys
                165                 170                 175
Trp Trp Thr Leu Pro Asp Asp Ala Asp Pro Asp Ser Pro Arg Arg Ala
            180                 185                 190
Val Asn Leu Arg Cys Cys Lys Lys Arg Arg Phe Ala Trp Arg Gly Cys
        195                 200                 205
Trp Val Phe Arg Tyr Met Ala Pro Thr Arg Gln Leu Arg Arg Ile Leu
    210                 215                 220
Gln Ser Gly Thr Cys Gly Cys Ser Leu Leu Asp Val Pro Gly Arg Asp
225                 230                 235                 240
Asn Asp Val Asp Ala Gly Gly Arg Val Leu Ala Arg Arg Ala Gln Asp
                245                 250                 255
Ala Gly Glu Gly Val Val Thr Ala Glu Gly Phe Ala Arg Gln Val Arg
            260                 265                 270
Thr Lys Arg Thr His Pro Gly Asp Phe Leu Asp Ser Gln Gly Asn Ala
        275                 280                 285
Gly Ile Gly Lys Lys Pro Gly Ser Val Gly Ser Ile Pro Val Arg Ile
    290                 295                 300
Thr Thr Arg Trp Pro Arg Pro Leu Ser Arg Arg Val Thr Ser Thr His
305                 310                 315                 320
Arg Asn Ile Leu Gly
                325

<210> 357
<211> 951
<212> DNA
<213> Unknown

<220>
<223> Obtained from an environmental sample

<400> 357
atgacaaaac tagccatcgt acaaaaaccg ccagtctttc tggataagca aaaaaccatt      60
gagctggccg tcgccaacat tgaagaggcc gccgccaagg gtgccgatct cgtggtgttt     120
tctgaagctt tcattcccgg ctatcctgcc tggatctggc gtctacgccc cggcggtgac     180
tgggggcttt cagaagagtt gcaccagcgt ttgctgcgca atgccgtcaa tgtggactcc     240
gatgatctgg ctccgttgtt tgaggtcgcc cgcaagcacg aactcaccat cgtttgcggt     300
atcgaggagc gtgacaacaa actaagtcaa acaaccttat ataacaccgt catcacgatt     360
ggtcccgatg gatcgttact gaacaaacat cgcaagctta tgcccaccaa cccggagcga     420
atggtgtggg ggtttggtga cgcatccggt ttaaaagtcg ttgataccaa tgctggtcga     480

attggctcat taatgtgctg ggaaaattac atgccgctgg ctcgctatgc cctatatgca 540
caaggtgtcg agatctatat cgcaccgacc tacgacagcg gtgatggctg gataggcagc 600
atgcagcaca tcgcacgtga agggggctgt tgggtggtgg gatgtgggtg tctcatgaaa 660
ggcagtgata ttccagatga tttcccggag aaatccacgt tgtatccaga tgcagatgaa 720
tgggtgaacc cgggtgattc tgtagtgata gcacccggcg gtgaaattat ggccggccca 780
atgaacagag agtccggtat tttgtatcac gagctagaca gagaaaaagt cagcaacgct 840
aaacgagcat tcgatgttgc cgggcattat tcacgtcccg atatctttca gctgcatgta 900
aatacacagg agcagtcacc ctgcgtattc gaaaataatt ccataactta a 951

<210> 358
<211> 300
<212> PRT
<213> Unknown

<220>
<223> Obtained from an environmental sample

<400> 358
Met Thr Lys Leu Ala Ile Val Gln Lys Pro Pro Val Phe Leu Asp Lys
1               5                   10                  15
Gln Lys Thr Ile Glu Leu Ala Val Pro Thr Leu Lys Arg Pro Pro Pro
            20                  25                  30
Arg Val Pro Ile Ser Trp Cys Phe Leu Lys Leu Ser Phe Pro Ala Ile
        35                  40                  45
Leu Trp Ile Trp Arg Leu Arg Pro Gly Gly Asp Trp Gly Leu Ser Glu
    50                  55                  60
Glu Leu His Gln Arg Leu Leu Arg Asn Ala Val Asn Val Asp Ser Asp
65                  70                  75                  80
Asp Leu Ala Pro Leu Phe Glu Val Ala Arg Lys His Glu Leu Thr Ile
                85                  90                  95
Val Cys Gly Arg Gly Ala Gln Gln Thr Lys Ser Asn Asn Leu Ile His
            100                 105                 110
Arg His His Asp Trp Ser Arg Trp Ile Tyr Thr Asn Ile Ala Ser Leu
        115                 120                 125
Cys Pro Pro Thr Arg Ser Glu Trp Cys Gly Gly Leu Val Thr His Pro
    130                 135                 140
Leu Lys Val Val Asp Thr Asn Ala Gly Arg Ile Gly Ser Leu Met Cys
145                 150                 155                 160
Trp Glu Asn Tyr Met Pro Leu Ala Arg Tyr Ala Leu Tyr Ala Gln Gly
                165                 170                 175
Val Glu Ile Tyr Ile Ala Pro Thr Tyr Asp Ser Gly Asp Gly Trp Ile
            180                 185                 190
Gly Ser Ala Ala His Arg Thr Arg Gly Leu Leu Gly Gly Gly Met Trp
        195                 200                 205
Val Ser His Glu Arg Gln Tyr Ser Met Ile Ser Arg Arg Asn Pro Arg
    210                 215                 220
Cys Ile Gln Met Gln Met Asn Gly Thr Arg Val Ile Leu Thr Arg Arg
225                 230                 235                 240
Asn Tyr Gly Arg Pro Asn Glu Gln Arg Val Arg Tyr Phe Val Ser Arg
                245                 250                 255
Ala Arg Gln Arg Lys Val Ser Asn Ala Lys Arg Ala Phe Asp Val Ala
            260                 265                 270
Gly His Tyr Ser Arg Pro Asp Ile Phe Gln Leu His Val Ile His Arg
        275                 280                 285
Ser Ser His Pro Ala Tyr Ser Lys Ile Ile Pro Leu
    290                 295                 300

<210> 359
<211> 1029
<212> DNA
<213> Unknown

<220>
<223> Obtained from an environmental sample

<400> 359
atggcaaacg tcgttcgtgc tgcggccgta cagttgagcc ctgttttggg tagtcgcgag 60
ggtacggtag agaaggtagt tgctgcgatc cgtgacgccg cctcgcaggg cgcacagctg 120

tgcgttttcc cggagacggt tgttccctat tatccgtatt tctcgttcat tcggccgccc 180
gcggccatgg gcaaagacca catgcagctg tacgagcaag ctgtggtcgt gccttctccc 240
agcacgaacg cgattgccgc ggcggccaaa caacactcga tcgtcgtttc aatcggcgtc 300
aatgaacgcg atcacggtac gatatacaac acgcagttgt tgttcgatgc cgacgggaca 360
ctcgtgcaac ggcgtcgcaa gataaccccc acgttccacg agcgtatggt gtggggtcaa 420
ggtgatgggt cgggtttgcg ctgtgtcgac acacaaatcg ggcgcatcgg tagcctggct 480
tgttgggaac attacaatcc cttggcgcgc tacgcattga tggccgatca cgaagagatc 540
cacgtcgcca tgtttccggg ttcgatggtg ggtcagatct tcgccgatca aattcaggta 600
accattcgcc accacgcgct cgaaagcggc tgtttcgtcg tcaacgctac ggggtatctg 660
agcaaggaac aggtcgccca gttgtcacaa ggcacgtcgc tcgacgcggc gttgaccggt 720
ggttgttaca ccgcgattgt atcgcctgaa ggcgtcgtac tgggcgaacc gctcaccgac 780
ggcgaaggca tggtcgtggc ggatatggat ctcagcctca tcaccaaacg caaacgcatg 840
atggatagcg tcgggcacta cagtcgcccg gaattgctgt ctctgctgat caatcgaacg 900
ccaacccaca cggcggtcga cgtcgaattc aactccaatt ccgagtctca tcatgtcagc 960
aatacacgaa caccaaagcg cacaactggc ccacgttcga accttcaagt tgccgctgat 1020
cgcgagtaa 1029

<210> 360
<211> 335
<212> PRT
<213> Unknown

<220>
<223> Obtained from an environmental sample

<400> 360
Met Ala Asn Val Val Arg Ala Ala Ala Val Gln Leu Ser Pro Val Leu
1               5                   10                  15
Gly Ser Arg Glu Gly Thr Val Glu Lys Val Val Ala Ala Ile Arg Asp
            20                  25                  30
Ala Ala Ser Gln Gly Ala Gln Leu Cys Val Phe Pro Glu Thr Val Val
        35                  40                  45
Pro Tyr Ser Val Phe Leu Val His Ser Ala Ala Arg Gly His Gly Gln
    50                  55                  60
Arg Pro His Ala Ala Val Arg Ala Ser Cys Gly Arg Ala Phe Ser Gln
65                  70                  75                  80
His Glu Arg Asp Cys Arg Gly Gly Gln Thr Thr Leu Asp Arg Arg Phe
                85                  90                  95
Asn Arg Arg Met Asn Ala Ile Thr Val Arg Tyr Thr Thr Arg Ser Cys
            100                 105                 110
Cys Ser Met Pro Thr Gly His Ser Cys Asn Gly Arg Lys Ile Thr Pro
        115                 120                 125
Thr Phe His Glu Arg Met Val Trp Gly Gln Gly Asp Gly Ser Gly Leu
    130                 135                 140
Arg Cys Val Asp Thr Gln Ile Gly Arg Ile Gly Ser Leu Ala Cys Trp
145                 150                 155                 160
Glu His Tyr Asn Pro Leu Ala Arg Tyr Ala Leu Met Ala Asp His Glu
                165                 170                 175
Glu Ile His Val Ala Met Phe Pro Gly Ser Met Val Gly Gln Ile Phe
            180                 185                 190
Ala Asp Gln Ile Gln Val Pro Phe Ala Thr Thr Arg Ser Lys Ala Ala
        195                 200                 205
Val Ser Ser Ser Thr Leu Arg Gly Ile Ala Arg Asn Arg Ala Gln Leu
    210                 215                 220
Ser Gln Gly Thr Ser Leu Asp Ala Ala Leu Thr Gly Gly Cys Tyr Thr
225                 230                 235                 240
Ala Ile Val Ser Pro Glu Gly Val Val Leu Gly Glu Pro Leu Thr Asp
                245                 250                 255
Gly Glu Gly Met Val Val Ala Asp Met Asp Leu Ser Leu Ile Pro Asn
            260                 265                 270
Ala Asn Ala Trp Ile Ala Ser Gly Thr Thr Val Ala Arg Asn Cys Cys
        275                 280                 285
Leu Cys Ser Ile Thr Pro Thr His Thr Ala Val Asp Val Glu Phe Asn
    290                 295                 300
Ser Asn Ser Glu Ser His His Val Ser Asn Thr Arg His Gln Ser Ala
305                 310                 315                 320
Gln Leu Ala His Val Arg Thr Phe Lys Leu Pro Leu Ile Ala Ser
                325                 330                 335

<210> 361
<211> 951
<212> DNA
<213> Unknown

<220>
<223> Obtained from an environmental sample

<400> 361
atgctaaacg agaacaatgg cgctactttc aaggttgctg ccgtgcaggc ttcaccggta 60
tttcttgatc gcgctgctac aatcgacaag gcttgcgatt taattgctac ggccggacgc 120
gagggggctc gcctgatcgt ctttccagaa gcgttcgtcc cggcctatcc tgattgggta 180
tgggcgattc ccgcgggtga tgagggcatg ctcaatgagc tgtatgcaga attacttgcc 240
aatgctgtca ccattcccag cgatgcgacc gagaggttgt gtcgcgcggc gcggcttgct 300
aatgcttacg tggtgatggg gatgagcgaa cgcaatgccg aagcgagtgg cgccagcctg 360
tataatacgc tgttgtatat tgatgcacag ggacaaatcc tgggaaagca ccggaagctg 420
gttccaacgg gcggcgagcg cctggtatgg gcacagggag atggcagcac cctggaggtt 480
tacgatactc ctttgggaaa actcggtggc ttaatctgct gggagaatta tatgccgctg 540
gcacgctata ctatgtatgc ctggggcacg caaatctaca ttgcagcgac gtgggatcgc 600
gggcagccat ggctatccac tttgcgacac attgctaaag agggcagagt atatgtgatc 660
ggctgttgta ttgctatgcg caaagatgat atccccgacc attacgcgat gaaggagaag 720
tattacgcgg aagaagacga gtggatcaat attggcgata gcgcaatcgt caatccagaa 780
ggggtattta ttgccgggcc agtgcgtaag caagaagaaa tcctctacgc cgaggttgac 840
ccgcgaatga tgcaggggcc aaagtggatg ctcgacgtgg caggacatta cgcgcgcccg 900
gatgtattcc agttgacggt gcacacggag aggcggcaga tgatccacta g 951

<210> 362
<211> 302
<212> PRT
<213> Unknown

<220>
<223> Obtained from an environmental sample

<400> 362
Met Leu Asn Glu Asn Asn Gly Ala Thr Phe Lys Val Ala Ala Val Gln
1               5                   10                  15
Ala Ser Pro Val Phe Leu Asp Arg Ala Tyr Asn Arg Gln Gly Leu Arg
            20                  25                  30
Phe Asn Cys Tyr Gly Arg Thr Arg Gly Gly Ser Pro Asp Arg Leu Ser
        35                  40                  45
Arg Ser Val Arg Pro Gly Leu Ser Leu Gly Met Gly Asp Ser Arg Gly
    50                  55                  60
Gly His Ala Gln Ala Val Cys Glu Leu Leu Ala Asn Ala Val Thr Ile
65                  70                  75                  80
Pro Ser Asp Ala Thr Glu Arg Leu Cys Arg Ala Ala Arg Leu Ala Asn
                85                  90                  95
Ala Tyr Val Val Met Gly Met Ser Glu Arg Asn Ala Glu Ala Ser Gly
            100                 105                 110
Ala Ser Leu Tyr Asn Thr Leu Leu Tyr Cys Thr Gly Thr Asn Pro Gly
        115                 120                 125
Lys Ala Pro Glu Ala Gly Ser Asn Gly Arg Arg Ala Pro Gly Met Gly
    130                 135                 140
Thr Gly Arg Trp Gln His Pro Gly Gly Leu Arg Tyr Ser Phe Gly Lys
145                 150                 155                 160
Thr Arg Trp Leu Asn Leu Leu Arg Ile Ile Cys Arg Trp His Ala Ile
                165                 170                 175
Leu Cys Met Pro Gly Ala Arg Lys Ser Thr Leu Gln Arg Arg Gly Ile
            180                 185                 190
Ala Gly Ser His Gly Tyr Pro Leu Cys Asp Thr Leu Leu Lys Arg Ala
        195                 200                 205
Glu Tyr Met Ser Ala Val Val Leu Tyr Ala Gln Arg Tyr Pro Arg Pro
    210                 215                 220
Leu Arg Asp Glu Gly Glu Val Leu Arg Gly Arg Arg Arg Val Asp Gln
225                 230                 235                 240
Tyr Trp Arg Arg Asn Arg Gln Ser Arg Arg Gly Ile Tyr Cys Arg Ala
                245                 250                 255

Ser Ala Ala Arg Arg Ile Leu Tyr Ala Glu Val Asp Pro Arg Met Met
            260                 265                 270
Gln Gly Pro Lys Trp Met Leu Asp Val Ala Gly His Tyr Arg Ala Arg
        275                 280                 285
Met Tyr Ser Ser Arg Cys Thr Arg Arg Gly Gly Arg Ser Thr
    290                 295                 300

<210> 363
<211> 1053
<212> DNA
<213> Unknown

<220>
<223> Obtained from an environmental sample

<400> 363
atgccgaaaa agtcgaccgt ccgggtcgca gccgtccaga ttgcgccgga tctgacatcg      60
cgggaaaaga cggtggcacg cgtgatcgag gcgatcgccc aggcatccgc caaaggtgcg     120
gagcttgtgg tttttcccga gacctttgtg ccgtggtatc cttatttctc gttcgtgttg     180
cccccggtct tgtcgggcaa ggagcacctg cggctctacg aagaggcggt tgcggtgcca     240
agtgccgcca caagaagcgt agcggctgcc gctcgcgaac atggcatcgt cgtggcgctt     300
ggcgtcaacg agcgcgacta tggcacgctc tacaatacgc aactgctttt cgatgccgat     360
ggcagtctga tcctgaagcg gcgcaagatc accccgactt tccacgagcg gatgatctgg     420
ggccagggcg atgcctcagg cctgaaggtt gtcgacagcg ccattggccg catcggcgcg     480
ctggcctgct gggaacacta caatccgcta gcccgctatg cgctgatggc gcagcacgag     540
gaaatccaca ttgcgcagtt tcccggctcc atggtcgggc cgatctttgc cgatcagatg     600
gaggtgacga tccgccatca cgcgctggaa agcggctgct tcgtcgtcaa tgccacggga     660
tggctgacgg atgatcagat cgtctcgatc acaccggata ccggcctgca aaaagcgctg     720
cggggtggct gcatgacggc gatcatttcc cccgaaggca agcatctcgt gccgccgctc     780
accgaaggtg agggtatcct cgtcgccgat ctcgacatga gcctcattct caagcgcaag     840
cgcatgatgg attcggtcgg ccactatgcc cggcccgagt tgctgcacct cgtcatggac     900
gcccggccgg ctgcgccgat gagggaatcg tccatgccca ctgccttccc cggcgaaaca     960
ttgacaaccg acatgaccga tggagaacag gatgcgtctt tcgacggaaa cgctgatcaa    1020
cgaattgcag tccttcggag cccggctggt tga                                 1053

<210> 364
<211> 335
<212> PRT
<213> Unknown

<220>
<223> Obtained from an environmental sample

<400> 364
Met Pro Lys Lys Ser Thr Val Arg Val Ala Ala Val Gln Ile Ala Pro
1               5                   10                  15
Asp Leu Thr Ser Arg Glu Lys Gly Gly Thr Arg Asp Arg Gly Asp Arg
            20                  25                  30
Pro Gly Ile Arg Gln Arg Cys Gly Ala Cys Gly Phe Ser Arg Asp Leu
        35                  40                  45
Cys Arg Gly Ile Leu Ile Ser Arg Ser Cys Cys Pro Arg Ser Cys Arg
    50                  55                  60
Ala Arg Ser Thr Cys Gly Ser Thr Lys Arg Arg Leu Arg Cys Gln Val
65                  70                  75                  80
Pro Pro Gln Glu Ala Arg Leu Pro Leu Ala Asn Met Ala Ser Ser Trp
                85                  90                  95
Arg Leu Ala Ser Thr Ser Ala Thr Met Ala Arg Ser Thr Ile Arg Asn
            100                 105                 110
Cys Phe Ser Met Pro Met Ala Val Ser Ser Gly Ala Arg Ser Pro Arg
        115                 120                 125
Leu Ser Thr Ser Gly Ser Gly Ala Arg Ala Met Pro Gln Ala Glu Gly
    130                 135                 140
Cys Arg Gln Arg His Trp Pro His Arg Arg Ala Gly Leu Leu Gly Thr
145                 150                 155                 160
Leu Gln Ser Ala Ser Pro Tyr Ala Leu Met Ala Gln His Glu Glu Ile
                165                 170                 175
His Ile Ala Gln Phe Pro Gly Ser Met Val Gly Pro Ile Phe Ala Asp
            180                 185                 190

Gln Met Glu Val Thr Ile Arg His His Ala Leu Glu Ser Gly Cys Phe
        195                 200                 205
Val Val Asn Ala Thr Gly Trp Arg Met Ile Arg Ser Ser Arg Ser His
    210                 215                 220
Arg Ile Pro Ala Cys Lys Lys Arg Cys Gly Val Ala Ala Gly Asp His
225                 230                 235                 240
Phe Pro Arg Arg Gln Ala Ser Arg Ala Ala Ala His Arg Arg Gly Tyr
                245                 250                 255
Pro Arg Arg Arg Ser Thr Ala Ser Phe Ser Ser Ala Ser Ala Trp Ile
            260                 265                 270
Arg Ser Ala Thr Met Pro Gly Pro Ser Cys Cys Thr Ser Ser Trp Thr
        275                 280                 285
Pro Gly Arg Leu Arg Arg Gly Asn Arg Pro Cys Pro Leu Pro Ser Pro
    290                 295                 300
Ala Lys Ile Asp Asn Arg His Asp Arg Trp Arg Thr Gly Cys Val Phe
305                 310                 315                 320
Arg Arg Lys Arg Ser Thr Asn Cys Ser Leu Arg Ser Pro Ala Gly
                325                 330                 335

<210> 365
<211> 975
<212> DNA
<213> Unknown

<220>
<223> Obtained from an environmental sample

<400> 365
atgacaaagc tagccatcgt tcaaaaaccc cccgtctttc tggataaaga aaaaaccata      60
gcgaagacgg ttgattccat aaaagaggcc gcgacacaaa atgccgactt ggtcatcttc     120
accgaagcct tcatcccggg ctaccccacc tggatatggc gacttaggcc aggcgctgat     180
tggggcctct cagaagagct gcacgagcag ttattgcgta acgcggtgag tatgggatcg     240
accgacctgg atccgcttta tgaagccgcc caacagcata acgtcactat tgtttgcggc     300
atcgtagaaa gagaccacca actcagccaa tcaaccctct acaacagcat ggtcgtcatt     360
gacacagacg gaacccttct caacaagcac cgcaaactca tgccaactaa tcccgaacgc     420
atggtgtggg gctttggcga cgcctcagga ctcaaagccg ttgcaacacc tgcaggccgc     480
atcagcacgt tgttgtgttg ggagaactac atgccattgg cccgatatgc tctgtatgca     540
caaggcgtgg aaatctatat cgcgccaact tacgacagtg gtgcgggttg gataggaagc     600
ttgcaacaca tagcacgcga aggtcgatgc tgggtcgtgg gctgtggcaa cctgattcag     660
gccagtgatc tgcctgaaga cttcccggac aaggacaacc tctacccgga cgcagaagag     720
tgggtgaacc cgggtgactc catagtcatt gcaccagacg gtgagattgt ggccggtcca     780
atgcacaaag agacaggaat tttgtactgc gagatagatc tggagaaagt cagaattgca     840
aaacgagcat tagacgtgac cgggcattat tcgcgaccgg acgttttcaa actgcatgtg     900
aatacccgac ctcaatcacc tgtggaattt gaaggtcagg agaccaacaa tccaacaaca     960
ggagaaagct catga                                                      975

<210> 366
<211> 315
<212> PRT
<213> Unknown

<220>
<223> Obtained from an environmental sample

<400> 366
Met Thr Lys Leu Ala Ile Val Gln Lys Pro Pro Val Phe Leu Asp Lys
1               5                   10                  15
Glu Lys Thr Ile Ala Lys Thr Val Ile Pro Lys Arg Pro Arg His Lys
            20                  25                  30
Met Pro Thr Trp Ser Ser Ser Pro Lys Pro Ser Ser Arg Ala Thr Thr
        35                  40                  45
Trp Ile Trp Arg Leu Arg Pro Gly Ala Asp Trp Gly Leu Ser Glu Glu
    50                  55                  60
Leu His Glu Gln Leu Leu Arg Arg Gly Glu Tyr Gly Ile Asp Arg Pro
65                  70                  75                  80
Gly Ser Ala Leu Ser Arg Pro Thr Ala Arg His Tyr Cys Leu Arg His
                85                  90                  95
Arg Arg Lys Arg Pro Pro Thr Gln Pro Ile Asn Pro Leu Gln Gln His

            100                 105                 110
Gly Arg His His Arg Arg Asn Pro Ser Gln Gln Ala Pro Gln Thr His
        115                 120                 125
Ala Asn Ser Arg Thr His Gly Val Gly Leu Trp Arg Ala Ser Gly Leu
    130                 135                 140
Lys Ala Val Ala Thr Pro Ala Gly Arg Ile Ser Thr Leu Leu Cys Trp
145                 150                 155                 160
Glu Asn Tyr Met Ile Gly Pro Ile Cys Ser Val Cys Thr Arg Arg Gly
                165                 170                 175
Asn Leu Tyr Arg Ala Asn Leu Arg Gln Trp Cys Gly Leu Asp Arg Lys
            180                 185                 190
Leu Ala Thr His Ser Thr Arg Arg Ser Met Leu Gly Arg Gly Leu Trp
        195                 200                 205
Gln Pro Asp Ser Gly Ser Asp Leu Pro Glu Asp Phe Pro Asp Lys Asp
    210                 215                 220
Asn Leu Tyr Pro Asp Ala Glu Glu Trp Val Asn Pro Gly Asp Ser Ile
225                 230                 235                 240
Val Ile Ala Pro Asp Gly Glu Ile Val Ala Gly Pro Met His Lys Glu
                245                 250                 255
Thr Gly Ile Leu Tyr Arg Asp Arg Ser Gly Glu Ser Gln Asn Cys Lys
            260                 265                 270
Thr Ser Ile Arg Arg Asp Arg Ala Leu Phe Ala Thr Gly Arg Phe Gln
        275                 280                 285
Thr Ala Cys Glu Tyr Pro Thr Ser Ile Thr Cys Gly Ile Arg Ser Gly
    290                 295                 300
Asp Gln Gln Ser Asn Asn Arg Arg Lys Leu Met
305                 310                 315

<210> 367
<211> 981
<212> DNA
<213> Unknown

<220>
<223> Obtained from an environmental sample

<400> 367
atgactgccc actttgccga taccctgacc gttgccgtgg cccagatcgc gccggtgtgg 60
ctacagcgcg aagctaccct gcaaaagatg ctggtctggg tggagcgcgc cgcgcgcgaa 120
ggcgcgggcc tggtcgcctt cagtgagggc ctgctgcctg gctacccctt ctggattgaa 180
cacacggacg gcgcccgctt cgagtcgccg ttacagaagc gcctgtacgc ccactactgc 240
gatcagtcgg tccagatcaa tgctgggcac ctcgcaccac tctgcgctgc cgcggccaga 300
caccagattt gggtggtctg cggcgtcatc gagcgcgaca gcgcacgcgg actcagcgtg 360
tttgcatcaa tggtcaccat cgatgccgaa ggcgcgatcc gcagtgtgca ccgcaagctg 420
atgccgacct acgaagaacg cctggtgtgg tcgcccggcg acgcgcacgg actgcgctgc 480
catccgctcg gccagttccg cctcggcagc ctcaattgct gggagaactg gatgccgctg 540
gcgcgcgccg ccctgtacgc ccagggcgag tctttgcatg ttgcatcctg gcccggcagt 600
cgccgcaaca ccgagaccat tactcccttc atcgcccgcg aaggccgcag ttacgcgctg 660
tccgccagtt ccgtgctgca ccgggatgat ctgcccgact ccgttccggc gctgtcggtg 720
ctgcgcgact gcctgccgga cgtgatggcc gacggcggct cctgcgtcgc cggccccgac 780
ggacatttcc tgatcgagcc ggtcgtcggc cgggaagagc tgctgctcgc gcagatcgat 840
catgcccggg tacgcgagga acgtcagaac ttcgacccct tcggccacta ctcgcggccg 900
gaactcctgt cgctggtggt ggatacgcgc cgggcgagcg gagtgcagat agtgaatgct 960
gaccatggct ttaagccctg a 981

<210> 368
<211> 317
<212> PRT
<213> Unknown

<220>
<223> Obtained from an environmental sample

<400> 368
Met Thr Ala His Phe Ala Asp Thr Leu Thr Val Ala Val Ala Gln Ile
1               5                   10                  15
Ala Pro Val Trp Leu Gln Arg Glu Tyr Pro Ala Lys Asp Ala Gly Leu
            20                  25                  30

Gly Gly Ala Arg Arg Ala Arg Arg Arg Gly Pro Gly Arg Leu Gln Gly
        35                  40                  45
Pro Ala Ala Trp Leu Pro Leu Leu Asp Thr His Gly Arg Arg Pro Leu
    50                  55                  60
Arg Val Ala Val Thr Glu Ala Cys Thr Pro Thr Thr Ala Ile Ser Arg
65                  70                  75                  80
Ser Arg Ser Met Leu Gly Thr Ser His His Ser Ala Leu Pro Ala Arg
                85                  90                  95
His Gln Ile Trp Val Val Cys Gly Val Ile Glu Arg Asp Ser Ala Arg
            100                 105                 110
Gly Leu Ser Val Phe Ala Gln Trp Ser Pro Ser Met Pro Lys Ala Arg
        115                 120                 125
Ser Ala Val Cys Thr Ala Ser Cys Arg Pro Thr Lys Asn Ala Trp Cys
    130                 135                 140
Gly Arg Pro Ala Thr Arg Thr Asp Cys Ala Ala Ile Arg Ser Ala Ser
145                 150                 155                 160
Ser Ala Ser Ala Ala Gln Leu Leu Gly Glu Leu Asp Ala Ala Gly Ala
                165                 170                 175
Arg Arg Pro Val Arg Pro Gly Arg Val Phe Ala Cys Cys Ile Leu Ala
            180                 185                 190
Arg Gln Ser Pro Gln His Arg Asp His Tyr Ser Leu His Arg Pro Arg
        195                 200                 205
Arg Pro Gln Leu Arg Ala Ser Ala Ser Ser Val Leu His Arg Asp Asp
    210                 215                 220
Leu Pro Asp Ser Val Pro Ala Leu Ser Val Leu Arg Asp Cys Leu Arg
225                 230                 235                 240
Thr Trp Pro Thr Ala Ala Pro Ala Ser Pro Ala Pro Thr Asp Ile Ser
                245                 250                 255
Ser Ser Arg Ser Ser Pro Gly Arg Ala Ala Ala Arg Ala Asp Arg Ser
            260                 265                 270
Cys Pro Gly Thr Arg Gly Thr Ser Glu Leu Arg Pro Leu Gly His Tyr
        275                 280                 285
Ser Arg Pro Glu Leu Leu Ser Leu Val Val Asp Thr Arg Arg Ala Ser
    290                 295                 300
Gly Val Gln Ile Val Asn Ala Asp His Gly Phe Lys Pro
305                 310                 315

<210> 369
<211> 1074
<212> DNA
<213> Unknown

<220>
<223> Obtained from an environmental sample

<400> 369
atgtccgaag agaacaccac caccgaatca agctcggacg cgatctggac cgttgcggcc      60
gtccaggcgg cgccggtgtt cttgaatcgg gatgcgacgg tgcgtaaggc cgtctcgctg     120
attgccgagg ccgcggcgca cggcgcgcgt ctcattgttt ttcccgaggc gttcattcca     180
tcgtacccgg attgggcgtg ggcggtcccg cccggacagg gcggaaccaa ctcgagactg     240
tatgccaaac tgctcgacaa ttcggttacg gtgcccagcc cagccaccga tgccctggcc     300
agggcggctc gcgacgcggg cgcctacgtc gtcatgggca taaacgagcg gaacacggcg     360
gcgagcggcg gaagtctcta caacagcttg ctctatattg gtccggacgg tcgcatcctg     420
ggcattcatc ggaagttggt gccgacctcg gcggagcggc tgatctgggc acaaggcgat     480
ggaagcacgc ttggcgtgtt cgatacgccg ggcggccggc ttggcggctt gatctgctgg     540
gaaaactata tgccgctggc ccgatattcg atgtacgcgc gcggcgtgca aatatatgtt     600
gcggcgacct gggacagggg cgagccatgg ctttcgacgc tgcgacacat agccaaggaa     660
ggccagacct acgtgatagg ctgttgcatc gccatgcgga cggccgacat cgacgacgcc     720
gagctcgtcg agaagtatta cgcggacgcc ggtgagtgga tcaatgaagg cgacagcgcg     780
attgtcgatc cgagcggaac aatcattgcc ggtccggccc atcagaccaa tgaaatcctc     840
tacgccgcga tcgatcgcca gaaggtgctg gaatcaaaat ggatgttgga cgtggccggg     900
cactacgcgc gtccggacgt gttttcattt ggcgttcgaa ccgatgccaa cccgataatg     960
accatgaacg aaccaagcgc gacggccgag ccgaggcaca atagcgcggg agccgagggt    1020
cgcgacggcc tacgcgggcg tcgtgaccct cgctcgagaa ttcggcagat gtag          1074

<210> 370
<211> 346
<212> PRT

<213> Unknown

<220>
<223> Obtained from an environmental sample

<400> 370
Met Ser Glu Glu Asn Thr Thr Thr Glu Ser Ser Ser Asp Ala Ile Trp
1               5                   10                  15
Thr Val Ala Ala Val Gln Ala Ala Gly Val Leu Glu Ser Gly Cys Asp
            20                  25                  30
Gly Ala Gly Arg Leu Ala Asp Cys Arg Gly Arg Gly Ala Arg Ala Arg
        35                  40                  45
Leu Ile Val Phe Pro Glu Ala Phe Ile Pro Ser Tyr Pro Asp Trp Ala
    50                  55                  60
Trp Ala Val Pro Pro Gly Gln Ala Glu Pro Thr Arg Asp Cys Met Pro
65                  70                  75                  80
Asn Cys Ser Thr Ile Arg Leu Arg Cys Pro Ala Gln Pro Pro Met Pro
                85                  90                  95
Gly Gln Gly Gly Ser Arg Arg Gly Arg Leu Arg Arg His Gly His Lys
            100                 105                 110
Arg Ala Glu His Gly Gly Ser Gly Gly Ser Leu Tyr Asn Ser Leu Leu
        115                 120                 125
Tyr Ile Gly Pro Asp Gly Arg Ile Leu Gly Ile His Arg Lys Leu Cys
    130                 135                 140
Arg Pro Arg Arg Ser Gly Ser Gly His Lys Ala Met Glu Ala Arg Leu
145                 150                 155                 160
Ala Cys Ser Ile Arg Arg Ala Ala Gly Leu Ala Ala Ser Ala Gly Lys
                165                 170                 175
Thr Ile Cys Arg Trp Pro Asp Ile Arg Cys Thr Arg Ala Val Gln Ile
            180                 185                 190
Tyr Val Ala Ala Thr Trp Asp Arg Gly Glu Pro Trp Leu Ser Thr Leu
        195                 200                 205
Arg His Ile Ala Lys Lys Ala Arg Pro Thr Ala Val Ala Ser Pro Cys
    210                 215                 220
Gly Arg Pro Thr Ser Thr Thr Pro Ser Ser Ser Arg Ser Ile Thr Arg
225                 230                 235                 240
Thr Pro Val Ser Gly Ser Met Lys Ala Thr Ala Arg Leu Ser Ile Arg
                245                 250                 255
Ala Glu Ile Ile Ala Gly Pro Ala His Gln Thr Asn Glu Ile Leu Tyr
            260                 265                 270
Ala Ala Ile Asp Arg Gln Lys Val Leu Glu Lys Met Asp Val Gly Arg
        275                 280                 285
Gly Arg Ala Leu Arg Ala Ser Gly Arg Val Phe Ile Trp Arg Ser Asn
    290                 295                 300
Arg Cys Gln Pro Asp Asn Asp His Glu Arg Thr Lys Arg Asp Gly Arg
305                 310                 315                 320
Ala Glu Ala Gln Arg Gly Ser Arg Val Ala Thr Ala Tyr Ala Gly Val
                325                 330                 335
Val Thr Leu Ala Arg Glu Phe Gly Arg Cys
            340                 345

<210> 371
<211> 1014
<212> DNA
<213> Unknown

<220>
<223> Obtained from an environmental sample

<400> 371
atgggtatcg aacatccgaa gtacaaggtt gcggtggtac aggcggcgcc ggcctggctc      60
gatctcgacg catcgatcgc caagaccatc gcgctgatcg aggaggcggc cgccaagggc     120
gccaagctga tcgcattccc tgaggccttc attcccggct atccttggca tatctggatg     180
gactcgccgg cctgggcgat cgggcgcgga tttgtgcagc gctatttcga caattcgctt     240
tcctacgaca gcccgcaggc cgaacggctg cggctcgcgg tgaagaaggc cggcatcacc     300
gccgtgctcg gcctgtccga gcgggaaggc ggcagccttt atctcgcgca atggctgatc     360
ggtcccgacg gcgagaccat cgccaagcgg cgcaagctgc ggccgacgca tgccgagcgc     420
accgtctacg gcgaaggcga cggcagtgac cttgcggtcc atgaccgcgc tgacattggc     480

cgtctcggcg cgctgtgctg ctgggaacat ctgcagccgc tgtcgaaata cgccatgtat 540
gcccagaacg agcaggtgca tgtcgcggcc tggccgagtt tttcgctgta cgacccgttc 600
gcgccggcgc tgggctggga ggtcaacaac gcggcatccc gcgtctatgc ggtcgaaggc 660
tcctgcttcg tgctggcgcc gtgtgccacc gtctcgcagg cgatggtgga cgaactctgc 720
gaccgcgacg acaagcatgc gctgctgcat gtcggcggcg gccacgccgc gatctacgga 780
ccggacggca gctcgatggc gaacaagctc gatcccgagc aggagggcct gctgttcgcc 840
gacatcgatc tcggggcgat cggggtggca aagaacgccg ccgatccggc cgggcactat 900
tcgcggccgg atgtgacccg tctgctcttg aacagaaaac cctcaaagcg cgtcgagcac 960
tttgcgctgc cgctcgacca tctcgcggac gagggcgttg ctccggtgac ctga 1014

<210> 372
<211> 327
<212> PRT
<213> Unknown

<220>
<223> Obtained from an environmental sample

<400> 372
Met Gly Ile Glu His Pro Lys Tyr Lys Val Ala Val Val Gln Ala Ala
1               5                   10                  15
Pro Ala Trp Leu Asp Leu Asp Ala Arg Ser Pro Arg Pro Ser Arg Ser
            20                  25                  30
Arg Arg Arg Pro Pro Arg Ala Pro Ser Ser His Ser Leu Ala Phe Ile
        35                  40                  45
Pro Gly Tyr Pro Trp His Ile Trp Met Asp Ser Pro Ala Trp Ala Ile
    50                  55                  60
Gly Arg Gly Phe Val Gln Ala Ile Ser Thr Ile Arg Phe Pro Thr Thr
65                  70                  75                  80
Ala Arg Arg Pro Asn Gly Cys Gly Ser Arg Arg Arg Pro His His Arg
                85                  90                  95
Arg Ala Arg Pro Val Arg Ala Gly Arg Arg Gln Pro Leu Ser Arg Ala
            100                 105                 110
Met Ala Asp Arg Ser Thr Ala Arg Pro Ser Pro Ser Gly Ala Ser Cys
        115                 120                 125
Gly Arg Arg Met Pro Ser Ala Pro Ser Thr Ala Lys Ala Thr Ala Val
    130                 135                 140
Thr Leu Arg Ser Met Thr Ala Leu Thr Leu Ala Val Ser Ala Arg Cys
145                 150                 155                 160
Ala Ala Gly Asn Leu Gln Pro Leu Ser Lys Tyr Ala Met Tyr Ala Gln
                165                 170                 175
Asn Glu Gln Val His Val Ala Ala Trp Pro Ser Phe Ala Val Arg Pro
            180                 185                 190
Val Arg Ala Gly Ala Gly Leu Gly Gly Gln Gln Arg Gly Ile Pro Arg
        195                 200                 205
Leu Cys Gly Arg Gly Ser Cys Phe Val Leu Ala Pro Cys Ala Thr Val
    210                 215                 220
Ser Gln Ala Met Val Asp Glu Leu Cys Asp Arg Asp Gln Ala Cys Ala
225                 230                 235                 240
Ala Ala Cys Arg Arg Arg Pro Arg Arg Asp Leu Arg Thr Gly Arg Gln
                245                 250                 255
Leu Asp Gly Thr Ser Ser Ile Pro Ser Arg Arg Ala Cys Cys Ser Pro
            260                 265                 270
Thr Ser Ile Ser Gly Arg Ser Gly Trp Gln Arg Thr Pro Pro Ile Arg
        275                 280                 285
Pro Gly Thr Ile Arg Gly Arg Met Pro Val Cys Ser Thr Glu Asn Pro
    290                 295                 300
Gln Ala Arg Arg Ala Leu Cys Ala Ala Ala Arg Pro Ser Arg Gly Arg
305                 310                 315                 320
Gly Arg Cys Ser Gly Asp Leu
                325

<210> 373
<211> 1056
<212> DNA
<213> Unknown

<220>

<223> Obtained from an environmental sample

<400> 373
atgacgaatt ttctggacgt gacggtggca gcggttcagg cggctcccgt ctatttcgat 60
cgggaggcat cgacggagaa agcgtgccgg ttgatccacg aagcggcagg gctcggcgcg 120
acgctcgcag cgttcggcga aacctggttg ccagggtatc cgttcttcgt ctgggggttc 180
gcgcacaacc ggagcctgtt ctggcaggcc gccgccgagt acatcgccaa tgcggtggag 240
attccgagtc ccacaacgga ccgtctctgt gcggcggcga aggctgccgg ggtcgacgtc 300
gtcattggcg tcgttgaact ggatgaacga acacgagctt cggtttacag tacgctgctt 360
ttcatcggtc gcgacgggac gatcctgggc cgccaccgca agctgaagcc aacacacatg 420
gagcggacga tctggggcga aggggacgca tatggactcc gcgtctacga acgttcgtac 480
gggcggctga gcggcctgaa ttgctgggaa cacaatatga tgctgcccgg ctacgtgctt 540
gccgcacagg gcacgcagtt tcacgtcgcc gcatggcccg gaaaggagag gctcaccgtc 600
ccgccgaacg aagcggctta tacgcgccag cttctcctct ctcgcgcgta tgcatcccag 660
gccggcgcgt acgtgatcag cgtcgccggg ctgctcgcac cagactccat gcccgagcgt 720
tatcgcgagt tagggcggtc atatgagttg accggcgaca gcgtcatcgt cgacccgcgc 780
ggcgaggtca ttgccgggcc tgcaaaaggc gagaccatcc tgctcgcgca gtgcagtcag 840
gaagctctcc tcgcggccaa gtccgccatc gacctcggcg gccattactc acgcccggat 900
atctttcagc tgcgtgtcaa cgatcaactg cagcatcggg tccggagagt tgagccacac 960
ttcacggcgg cgatcggaca tatcggagcc gagcgccgat cccaggagga tggtactggt 1020
cccttcgacc tggcggaatc tctcacgaac tcctag 1056

<210> 374
<211> 351
<212> PRT
<213> Unknown

<220>
<223> Obtained from an environmental sample

<400> 374
Met Thr Asn Phe Leu Asp Val Thr Val Ala Ala Val Gln Ala Ala Pro
1               5                   10                  15
Val Tyr Phe Asp Arg Glu Ala Ser Thr Glu Lys Ala Cys Arg Leu Ile
            20                  25                  30
His Glu Ala Ala Gly Leu Gly Ala Thr Leu Ala Ala Phe Gly Glu Thr
        35                  40                  45
Trp Leu Pro Gly Tyr Pro Phe Phe Val Trp Gly Phe Ala His Asn Arg
    50                  55                  60
Ser Leu Phe Trp Gln Ala Ala Ala Glu Tyr Ile Ala Asn Ala Val Glu
65                  70                  75                  80
Ile Pro Ser Pro Thr Thr Asp Arg Leu Cys Ala Ala Ala Lys Ala Ala
                85                  90                  95
Gly Val Asp Val Val Ile Gly Val Val Glu Leu Asp Glu Arg Thr Arg
            100                 105                 110
Ala Ser Val Tyr Ser Thr Leu Leu Phe Ile Gly Arg Asp Gly Thr Ile
        115                 120                 125
Leu Gly Arg His Arg Lys Leu Lys Pro Thr His Met Glu Arg Thr Ile
    130                 135                 140
Trp Gly Glu Gly Asp Ala Tyr Gly Leu Arg Val Tyr Glu Arg Ser Tyr
145                 150                 155                 160
Gly Arg Leu Ser Gly Leu Asn Cys Trp Glu His Asn Met Met Leu Pro
                165                 170                 175
Gly Tyr Val Leu Ala Ala Gln Gly Thr Gln Phe His Val Ala Ala Trp
            180                 185                 190
Pro Gly Lys Glu Arg Leu Thr Val Pro Pro Asn Glu Ala Ala Tyr Thr
        195                 200                 205
Arg Gln Leu Leu Leu Ser Arg Ala Tyr Ala Ser Gln Ala Gly Ala Tyr
    210                 215                 220
Val Ile Ser Val Ala Gly Leu Leu Ala Pro Asp Ser Met Pro Glu Arg
225                 230                 235                 240
Tyr Arg Glu Leu Gly Arg Ser Tyr Glu Leu Thr Gly Asp Ser Val Ile
                245                 250                 255
Val Asp Pro Arg Gly Glu Val Ile Ala Gly Pro Ala Lys Gly Glu Thr
            260                 265                 270
Ile Leu Leu Ala Gln Cys Ser Gln Glu Ala Leu Leu Ala Ala Lys Ser
        275                 280                 285
Ala Ile Asp Leu Gly Gly His Tyr Ser Arg Pro Asp Ile Phe Gln Leu

290                 295                 300
Arg Val Asn Asp Gln Leu Gln His Arg Val Arg Arg Val Glu Pro His
305                 310                 315                 320
Phe Thr Ala Ala Ile Gly His Ile Gly Ala Glu Arg Arg Ser Gln Glu
                325                 330                 335
Asp Gly Thr Gly Pro Phe Asp Leu Ala Glu Ser Leu Thr Asn Ser
            340                 345                 350

<210> 375
<211> 939
<212> DNA
<213> Unknown

<220>
<223> Obtained from an environmental sample

<400> 375
atgaccaaaa tcgctgtcat tcaagaaccc ccggtctatc tgaatctgag caaatcgatg      60
gaccgagcgg tcgacttgat tgccgatgcc gcgagccagg ggtgtcagtt gattgtgttt     120
cccgaagcct ggcttgcagg ttaccccacc ttcgtctggc gtcttgcgcc gggcagcgga     180
atgggaaaaa ccgatgagct ttacgcgcgt ttgctcgcca actcggtcga ccgcagcaaa     240
gaggggcttc ggcctttgca ggaggctgca aaagagcatg gcgttgtcat tgttctgggt     300
tatcaagagg tggacggctc gggcagcagc agcacaatct tcaacagctg cgcgattatt     360
gatgccgacg ggcgactggc caacaaccat cgcaagttga tgcccaccaa tgcggagcgg     420
atggtgtggg ggtttggcga cggttcgggc ctgaacgttg ttgacaccgc ggtgggcagg     480
atcggcacgc tgatttgctg ggaaaactac atgcccttgg cgcgctacgc gctgtatgcc     540
caaaacatcg aaatctatgt ggcgccgacc tgggacagcg gtgccatgtg gcaagccacc     600
ctgcaacata tcgcacgtga aggtggctgc tgggtcatcg gatgtgcaac ctcgctgcaa     660
gcctctgaca tcccggacga ccttccccat cgggatgagt tattcccgaa caaagacgaa     720
tgggtgaacc ctggcgatgc ggtggtttac aaaccttttg gcggccttgt ggccggcccc     780
atgcatcagg aaaaggggct tctcatcgca gagttggacg tcgccgctgt tcaggtctca     840
cgtcggaagt tcgatgcgac cgggcattac gctcgccccg atgtcttcca actgcacgtg     900
aatcgcagcg cgatgcggcc ggttgagttc acgaattag                            939

<210> 376
<211> 312
<212> PRT
<213> Unknown

<220>
<223> Obtained from an environmental sample

<400> 376
Met Thr Lys Ile Ala Val Ile Gln Glu Pro Pro Val Tyr Leu Asn Leu
1               5                   10                  15
Ser Lys Ser Met Asp Arg Ala Val Asp Leu Ile Ala Asp Ala Ala Ser
            20                  25                  30
Gln Gly Cys Gln Leu Ile Val Phe Pro Glu Ala Trp Leu Ala Gly Tyr
        35                  40                  45
Pro Thr Phe Val Trp Arg Leu Ala Pro Gly Ser Gly Met Gly Lys Thr
    50                  55                  60
Asp Glu Leu Tyr Ala Arg Leu Leu Ala Asn Ser Val Asp Arg Ser Lys
65                  70                  75                  80
Glu Gly Leu Arg Pro Leu Gln Glu Ala Ala Lys Glu His Gly Val Val
                85                  90                  95
Ile Val Leu Gly Tyr Gln Glu Val Asp Gly Ser Gly Ser Ser Ser Thr
            100                 105                 110
Ile Phe Asn Ser Cys Ala Ile Ile Asp Ala Asp Gly Arg Leu Ala Asn
        115                 120                 125
Asn His Arg Lys Leu Met Pro Thr Asn Ala Glu Arg Met Val Trp Gly
    130                 135                 140
Phe Gly Asp Gly Ser Gly Leu Asn Val Val Asp Thr Ala Val Gly Arg
145                 150                 155                 160
Ile Gly Thr Leu Ile Cys Trp Glu Asn Tyr Met Pro Leu Ala Arg Tyr
                165                 170                 175
Ala Leu Tyr Ala Gln Asn Ile Glu Ile Tyr Val Ala Pro Thr Trp Asp
            180                 185                 190
Ser Gly Ala Met Trp Gln Ala Thr Leu Gln His Ile Ala Arg Glu Gly

195                 200                 205
Gly Cys Trp Val Ile Gly Cys Ala Thr Ser Leu Gln Ala Ser Asp Ile
    210                 215                 220
Pro Asp Asp Leu Pro His Arg Asp Glu Leu Phe Pro Asn Lys Asp Glu
225                 230                 235                 240
Trp Val Asn Pro Gly Asp Ala Val Val Tyr Lys Pro Phe Gly Gly Leu
                245                 250                 255
Val Ala Gly Pro Met His Gln Glu Lys Gly Leu Leu Ile Ala Glu Leu
            260                 265                 270
Asp Val Ala Ala Val Gln Val Ser Arg Arg Lys Phe Asp Ala Thr Gly
        275                 280                 285
His Tyr Ala Arg Pro Asp Val Phe Gln Leu His Val Asn Arg Ser Ala
    290                 295                 300
Met Arg Pro Val Glu Phe Thr Asn
305                 310

<210> 377
<211> 1050
<212> DNA
<213> Unknown

<220>
<223> Obtained from an environmental sample

<400> 377
atgtccagca ccacccatcc ccgcctgcgc gtcgccgcgg tgcaagccgc ccccgtcttc      60
ctcgacctcg acggcaccat cgacaagacc atcgacctga tggcccgggc ggccggccag     120
ggcgtgcagc tgatcgcctt tcccgagacc tgggtgcccg gctatccgtg gtggatctgg     180
ctcgattcgc cggcctgggg catgcagttc gtgcagcgct accacgacaa cgccctggtc     240
gtcggctcgc ccgagttcga ccgcattcgc gaggccgcgc gcaagcaccg catctgggtc     300
tcgctcggct acagcgagaa ggccgccggc agcctctaca tcgcccaggc gctgatcgac     360
gaccagggca acacgctgca gactcggcgc aagctcaagc cgacgcacgt ggagcgcacc     420
gtgttcggcg agggcgacgg atcggacctg agcgtggtcg agacggctat cggcaacatc     480
ggctcgctgt cgtgctggga gcacctgcag ccgctcagca agtacgcgat gtactcgcag     540
aacgagcaga tccattgcgg cgcctggccc agcttctcgc tctaccgcgg cggcgcctac     600
gcgctcggcg ccgaagtgaa caacgccgcc agccaggtgt acgcggccga gggccagtgc     660
ttcgtgatcg cgccctgcgc cacggtctcg aaggcgatgc acgaactgct gtgcaccgac     720
cctggcaagc agcagatgct gctggtcggc ggcggcttcg cgcgcatcta cggacccgac     780
ggatcgccgc tcggcaagaa cctggcagag gacgaggaag ggctggtggt ggccgacatc     840
gacctcggca tgatctccct ggccaaggcg gccggcgacc cggccggcca ctattcgcgg     900
cccgacgtga cgcagttgct gttcaacagg aagcggcgcg agccggtggt gctgcaaggc     960
ccggccgagc ccgagaaggc ggtcgccgag ccggtgtcca cgccgagcga agcggcggcg    1020
gccgccgccc gccagccggt cgtggcctga                                     1050

<210> 378
<211> 349
<212> PRT
<213> Unknown

<220>
<223> Obtained from an environmental sample

<400> 378
Met Ser Ser Thr Thr His Pro Arg Leu Arg Val Ala Ala Val Gln Ala
1               5                   10                  15
Ala Pro Val Phe Leu Asp Leu Asp Gly Thr Ile Asp Lys Thr Ile Asp
            20                  25                  30
Leu Met Ala Arg Ala Ala Gly Gln Gly Val Gln Leu Ile Ala Phe Pro
        35                  40                  45
Glu Thr Trp Val Pro Gly Tyr Pro Trp Trp Ile Trp Leu Asp Ser Pro
    50                  55                  60
Ala Trp Gly Met Gln Phe Val Gln Arg Tyr His Asp Asn Ala Leu Val
65                  70                  75                  80
Val Gly Ser Pro Glu Phe Asp Arg Ile Arg Glu Ala Ala Arg Lys His
                85                  90                  95
Arg Ile Trp Val Ser Leu Gly Tyr Ser Glu Lys Ala Ala Gly Ser Leu
            100                 105                 110
Tyr Ile Ala Gln Ala Leu Ile Asp Asp Gln Gly Asn Thr Leu Gln Thr

115                 120                 125
Arg Arg Lys Leu Lys Pro Thr His Val Glu Arg Thr Val Phe Gly Glu
    130                 135                 140
Gly Asp Gly Ser Asp Leu Ser Val Val Glu Thr Ala Ile Gly Asn Ile
145                 150                 155                 160
Gly Ser Leu Ser Cys Trp Glu His Leu Gln Pro Leu Ser Lys Tyr Ala
                165                 170                 175
Met Tyr Ser Gln Asn Glu Gln Ile His Cys Gly Ala Trp Pro Ser Phe
            180                 185                 190
Ser Leu Tyr Arg Gly Gly Ala Tyr Ala Leu Gly Ala Glu Val Asn Asn
        195                 200                 205
Ala Ala Ser Gln Val Tyr Ala Ala Glu Gly Gln Cys Phe Val Ile Ala
    210                 215                 220
Pro Cys Ala Thr Val Ser Lys Ala Met His Glu Leu Leu Cys Thr Asp
225                 230                 235                 240
Pro Gly Lys Gln Gln Met Leu Leu Val Gly Gly Gly Phe Ala Arg Ile
                245                 250                 255
Tyr Gly Pro Asp Gly Ser Pro Leu Gly Lys Asn Leu Ala Glu Asp Glu
            260                 265                 270
Glu Gly Leu Val Val Ala Asp Ile Asp Leu Gly Met Ile Ser Leu Ala
        275                 280                 285
Lys Ala Ala Gly Asp Pro Ala Gly His Tyr Ser Arg Pro Asp Val Thr
    290                 295                 300
Gln Leu Leu Phe Asn Arg Lys Arg Arg Glu Pro Val Val Leu Gln Gly
305                 310                 315                 320
Pro Ala Glu Pro Glu Lys Ala Val Ala Glu Pro Val Ser Thr Pro Ser
                325                 330                 335
Glu Ala Ala Ala Ala Ala Ala Arg Gln Pro Val Val Ala
            340                 345

<210> 379
<211> 936
<212> DNA
<213> Unknown

<220>
<223> Obtained from an environmental sample

<400> 379
atggctaaaa tagcgattgt acaaaaggcg tctgttacct tgaataaaca agaaactgtc      60
gccagtgctg taagagaggt agagctggca gcggcggaag gtgccgagct ggtagtgttt     120
actgaggcat ttattgctgg gtatccggcc tggatctggc gtttgcggcc aggtggtgac     180
tgggggctat ctgaagatct tcattcccgt ttgttgacaa gcgccgtaga cctgggtggt     240
gatgacctgg atccacttta tgccgcagct aaagaaaata acgtgacgat agtgtgcggt     300
attaatgaac gtgataaccg gctcagtaag gcaacgctat ataattctat cgttattatt     360
ggttccgatg gttcattgtt aaatcgacat cgtaagttga tgccgacgaa tccggagaga     420
atggtatggg gctttggtga tgcctctggt ctgaaggtcg ttgatacccc cgttggtcgt     480
gttggtacgc ttgtctgttg ggaaaactat atgcccttgg ccagatatgc gttgtattcg     540
caggggggtag aggtttatat tgcgccgacc tacgatagcg gtgatgactg gatttctaca     600
ttacagcata ttgccaggga gggtcgttgt tgggttgttg gctgtggcaa tctattgcgt     660
ggcagcgata taccggatga cttccctgag aagttggcgt tatacccaga tgaggatgag     720
tggataaatc ctggggattc cgttgtgatt gcacctgggg gtaaaatcat ggccgggcca     780
ttgcgccagg aggcggggat tgtctattgt gatattgcgt ctgaaagtgc cagtcaggca     840
aaacgtgcgc tggatgtggc tggacattat tcccggcctg atatctttga gttgcatgtg     900
aatacgaagg tgcagacccc ggttgtatat gattag                               936

<210> 380
<211> 311
<212> PRT
<213> Unknown

<220>
<223> Obtained from an environmental sample

<400> 380
Met Ala Lys Ile Ala Ile Val Gln Lys Ala Ser Val Thr Leu Asn Lys
1               5                   10                  15
Gln Glu Thr Val Ala Ser Ala Val Arg Glu Val Glu Leu Ala Ala Ala

                20                  25                  30
Glu Gly Ala Glu Leu Val Val Phe Thr Glu Ala Phe Ile Ala Gly Tyr
        35                  40                  45
Pro Ala Trp Ile Trp Arg Leu Arg Pro Gly Gly Asp Trp Gly Leu Ser
    50                  55                  60
Glu Asp Leu His Ser Arg Leu Leu Thr Ser Ala Val Asp Leu Gly Gly
65                  70                  75                  80
Asp Asp Leu Asp Pro Leu Tyr Ala Ala Ala Lys Glu Asn Asn Val Thr
                85                  90                  95
Ile Val Cys Gly Ile Asn Glu Arg Asp Asn Arg Leu Ser Lys Ala Thr
            100                 105                 110
Leu Tyr Asn Ser Ile Val Ile Ile Gly Ser Asp Gly Ser Leu Leu Asn
        115                 120                 125
Arg His Arg Lys Leu Met Pro Thr Asn Pro Glu Arg Met Val Trp Gly
    130                 135                 140
Phe Gly Asp Ala Ser Gly Leu Lys Val Val Asp Thr Pro Val Gly Arg
145                 150                 155                 160
Val Gly Thr Leu Val Cys Trp Glu Asn Tyr Met Pro Leu Ala Arg Tyr
                165                 170                 175
Ala Leu Tyr Ser Gln Gly Val Glu Val Tyr Ile Ala Pro Thr Tyr Asp
            180                 185                 190
Ser Gly Asp Asp Trp Ile Ser Thr Leu Gln His Ile Ala Arg Glu Gly
        195                 200                 205
Arg Cys Trp Val Val Gly Cys Gly Asn Leu Leu Arg Gly Ser Asp Ile
    210                 215                 220
Pro Asp Asp Phe Pro Glu Lys Leu Ala Leu Tyr Pro Asp Glu Asp Glu
225                 230                 235                 240
Trp Ile Asn Pro Gly Asp Ser Val Val Ile Ala Pro Gly Gly Lys Ile
                245                 250                 255
Met Ala Gly Pro Leu Arg Gln Glu Ala Gly Ile Val Tyr Cys Asp Ile
            260                 265                 270
Ala Ser Glu Ser Ala Ser Gln Ala Lys Arg Ala Leu Asp Val Ala Gly
        275                 280                 285
His Tyr Ser Arg Pro Asp Ile Phe Glu Leu His Val Asn Thr Lys Val
    290                 295                 300
Gln Thr Pro Val Val Tyr Asp
305                 310

<210> 381
<211> 945
<212> DNA
<213> Clostridium acetobutylicum ATCC 3625

<400> 381
atgggcgaat tcggtgaagt taccctgggc gtggctcagg ctgctcccgt gtactttgac      60
cgcgaggcct cgaccgagaa agcctgtggc ctgatccgcg aggcgggcga aaagggtgta     120
gatcttctgg cgttcggtga aacgtggtta accggctatc catactggaa agatgcgcct     180
tggtctcgcg aatacaacga cttgcgtgca cgttatgttg cgaacggtgt gatgattcct     240
ggtccggaaa cggacgctct gtgccaagca gccgcggaag caggtgtgga tgtggcgatc     300
ggagtagtag aactggagcc gggctctctt tcctcggttt attgcactct gttatttata     360
agccgcgagg gcgagatcct gggtcgtcac cgcaaactga aaccgaccga tagcgaacgt     420
cgttactggt ctgaaggcga cgcgactggt ctgcgcgttt atgagcgccc atatggtcgg     480
cttagcggcc tgaattgctg ggagcacact atgatgctgc cggggtacgc cctggcggcg     540
cagggcaccc agttccatgt ggccgcttgg ccaaacatgg catcctcgaa ttctgaactt     600
ctgtctcgtg cctacgctat gcaggcgggc tgctacgttt tatgcgcggg tggcctgggc     660
ccggccccag gtgaactgcc ggatggtatc gcggcggaaa gtttagatca cttgactgga     720
gagtcatgta tcatcgaccc gtgggggaaa gtaattgctg gtccggtgtc ttgcgaggaa     780
acccttatca cggctcgcgt tagcaccgca tctatttatc gccgcaaaag tttgacggac     840
gtgggcggtc attatagccg cccggatgtt ttccgttttg aagtcgatcg ctctgagcgt     900
ccccgtgtcg tgtttcgcga tggtgacgtg gatgaccgag gttaa                     945

<210> 382
<211> 314
<212> PRT
<213> Clostridium acetobutylicum ATCC 3625

<400> 382
Met Gly Glu Phe Gly Glu Val Thr Leu Gly Val Ala Gln Ala Ala Pro

1               5                   10                  15
Val Tyr Phe Asp Arg Glu Ala Ser Thr Glu Lys Ala Cys Gly Leu Ile
            20                  25                  30
Arg Glu Ala Gly Glu Lys Gly Val Asp Leu Leu Ala Phe Gly Glu Thr
        35                  40                  45
Trp Leu Thr Gly Tyr Pro Tyr Trp Lys Asp Ala Pro Trp Ser Arg Glu
    50                  55                  60
Tyr Asn Asp Leu Arg Ala Arg Tyr Val Ala Asn Gly Val Met Ile Pro
65                  70                  75                  80
Gly Pro Glu Thr Asp Ala Leu Cys Gln Ala Ala Ala Glu Ala Gly Val
                85                  90                  95
Asp Val Ala Ile Gly Val Val Glu Leu Glu Pro Gly Ser Leu Ser Ser
            100                 105                 110
Val Tyr Cys Thr Leu Leu Phe Ile Ser Arg Glu Gly Glu Ile Leu Gly
        115                 120                 125
Arg His Arg Lys Leu Lys Pro Thr Asp Ser Glu Arg Arg Tyr Trp Ser
    130                 135                 140
Glu Gly Asp Ala Thr Gly Leu Arg Val Tyr Glu Arg Pro Tyr Gly Arg
145                 150                 155                 160
Leu Ser Gly Leu Asn Cys Trp Glu His Thr Met Met Leu Pro Gly Tyr
                165                 170                 175
Ala Leu Ala Ala Gln Gly Thr Gln Phe His Val Ala Ala Trp Pro Asn
            180                 185                 190
Met Ala Ser Ser Asn Ser Glu Leu Leu Ser Arg Ala Tyr Ala Met Gln
        195                 200                 205
Ala Gly Cys Tyr Val Leu Cys Ala Gly Gly Leu Gly Pro Ala Pro Gly
    210                 215                 220
Glu Leu Pro Asp Gly Ile Ala Ala Glu Ser Leu Asp His Leu Thr Gly
225                 230                 235                 240
Glu Ser Cys Ile Ile Asp Pro Trp Gly Lys Val Ile Ala Gly Pro Val
                245                 250                 255
Ser Cys Glu Glu Thr Leu Ile Thr Ala Arg Val Ser Thr Ala Ser Ile
            260                 265                 270
Tyr Arg Arg Lys Ser Leu Thr Asp Val Gly Gly His Tyr Ser Arg Pro
        275                 280                 285
Asp Val Phe Arg Phe Glu Val Asp Arg Ser Glu Arg Pro Arg Val Val
    290                 295                 300
Phe Arg Asp Gly Asp Val Asp Asp Arg Gly
305                 310

<210> 383
<211> 1041
<212> DNA
<213> Unknown

<220>
<223> Obtained from an environmental sample

<400> 383
atgtcggagc ccatgacgaa gtatcgcggc gcggcggtgc aggccgcgcc ggtgttcctc      60
gatctcgacc gcacagtcga gaaagcgatc ggcctgatcg agcaggcggc caagcaggac     120
gtgcgcctga tcgcattccc agagacttgg attcccggct atcccttttg gatatggctg      180
ggcgcgccgg cttggggcat gcgcttcgtc cagcgctatt tcgagaattc gctcgtgcgc     240
ggcagcaagc agtggcaggc cctggcggat gcggcccgcc gccacggcat gcatgtcgtg     300
gccggctata gcgagcgcgc gggcggcagc ctctatatgg gccaggcgat cttcggcccc     360
gatggcgatc tgatcgccgc gcgccgcaag ctcaagccta cccatgcgga gcgcaccgtg     420
ttcggcgagg gagacggcag ccatctcgcg gtgcacgata ccgccatcgg gcgcctcggc     480
gcgctctgtt gctgggagca catccagcca ttgtcgaaat acgccatgta cgccgccgac     540
gaacaggtcc acgtcgcgtc gtggccgagc ttcagcctct atcgcggcat ggcctatgcg     600
ctcggaccgg aggtcaatac cgccgcaagc cagatctacg cggtcgaggg cggctgctac     660
gtgctggcgt cgtgcgcgac cgtttcgccg gagatgatca aggtattggt ggatacgccc     720
gacaaggaga tgttcctcaa ggccggcggc ggttttgcca tgattttcgg gcccgacggc     780
cgcgccctgg ccgagccgct cccggagacc gaagagggac tgctggtcgc cgatatcgac     840
ctcggcatga tcgcgttggc caaggcggcg gccgatccgg cgggccacta ttcacggccc     900
gacgtaacgc ggctgctgct ggatcgacgt ccggcccaac gcgtcgtcac gcttgatgcc     960
gcattcgaac cgcaaaacga ggacaagggc gacgcgcccg cgctgcgcgt ggtggcggaa    1020
agcgccgccg ccgcgcagta g                                              1041

<210> 384
<211> 346
<212> PRT
<213> Unknown

<220>
<223> Obtained from an environmental sample

<400> 384
Met Ser Glu Pro Met Thr Lys Tyr Arg Gly Ala Ala Val Gln Ala Ala
1               5                   10                  15
Pro Val Phe Leu Asp Leu Asp Arg Thr Val Glu Lys Ala Ile Gly Leu
            20                  25                  30
Ile Glu Gln Ala Ala Lys Gln Asp Val Arg Leu Ile Ala Phe Pro Glu
        35                  40                  45
Thr Trp Ile Pro Gly Tyr Pro Phe Trp Ile Trp Leu Gly Ala Pro Ala
    50                  55                  60
Trp Gly Met Arg Phe Val Gln Arg Tyr Phe Glu Asn Ser Leu Val Arg
65                  70                  75                  80
Gly Ser Lys Gln Trp Gln Ala Leu Ala Asp Ala Ala Arg Arg His Gly
                85                  90                  95
Met His Val Val Ala Gly Tyr Ser Glu Arg Ala Gly Gly Ser Leu Tyr
            100                 105                 110
Met Gly Gln Ala Ile Phe Gly Pro Asp Gly Asp Leu Ile Ala Ala Arg
        115                 120                 125
Arg Lys Leu Lys Pro Thr His Ala Glu Arg Thr Val Phe Gly Glu Gly
    130                 135                 140
Asp Gly Ser His Leu Ala Val His Asp Thr Ala Ile Gly Arg Leu Gly
145                 150                 155                 160
Ala Leu Cys Cys Trp Glu His Ile Gln Pro Leu Ser Lys Tyr Ala Met
                165                 170                 175
Tyr Ala Ala Asp Glu Gln Val His Val Ala Ser Trp Pro Ser Phe Ser
            180                 185                 190
Leu Tyr Arg Gly Met Ala Tyr Ala Leu Gly Pro Glu Val Asn Thr Ala
        195                 200                 205
Ala Ser Gln Ile Tyr Ala Val Glu Gly Gly Cys Tyr Val Leu Ala Ser
    210                 215                 220
Cys Ala Thr Val Ser Pro Glu Met Ile Lys Val Leu Val Asp Thr Pro
225                 230                 235                 240
Asp Lys Glu Met Phe Leu Lys Ala Gly Gly Gly Phe Ala Met Ile Phe
                245                 250                 255
Gly Pro Asp Gly Arg Ala Leu Ala Glu Pro Leu Pro Glu Thr Glu Glu
            260                 265                 270
Gly Leu Leu Val Ala Asp Ile Asp Leu Gly Met Ile Ala Leu Ala Lys
        275                 280                 285
Ala Ala Ala Asp Pro Ala Gly His Tyr Ser Arg Pro Asp Val Thr Arg
    290                 295                 300
Leu Leu Leu Asp Arg Arg Pro Ala Gln Arg Val Val Thr Leu Asp Ala
305                 310                 315                 320
Ala Phe Glu Pro Gln Asn Glu Asp Lys Gly Asp Ala Pro Ala Leu Arg
                325                 330                 335
Val Val Ala Glu Ser Ala Ala Ala Ala Gln
            340                 345

<210> 385
<211> 1014
<212> DNA
<213> Unknown

<220>
<223> Obtained from an environmental sample

<400> 385
atgaaagaag ctatcaaggt cgcctgcgtg caagccgccc cgatctacat ggatttgaag      60
gcgacggtgg acaaaaccat tgagttgatg gaagaagcag cacgtaataa tgctcgtctg     120
atcgcctttc cggaaacttg gattccaggc tacccatggt ttctttggct tgactcacca     180
gcatgggcaa tgcaatttgt acgccaatac catgagaact cattggagtt ggatggccct     240
caagctaagc gcatttcaga tgcagccaag cggttgggaa tcatggtcac cctggggatg     300

agtgaacggg tcggtggcac cctttacatc agtcagtggt tcataggcga taatggtgac 360
accattgggg cccggcgaaa gttgaaacct acttttgttg aacgtacttt gttcggcgaa 420
ggggatggtt catcgctagc ggttttcgag acgtctgttg gaaggctggg tggcttatgc 480
tgttgggagc accttcaacc gctaacaaaa tacgctttgt atgcacaaaa tgaagagatt 540
cattgtgcgg cttggccgag ctttagcctt tatcctaatg cggcgaaagc cctggggcct 600
gatgtcaatg tagcggcctc tcgaatctat gccgttgaag ggcaatgctt cgtactagcg 660
tcgtgtgcgc tcgtttcaca atccatgatc gatatgcttt gtacagatga cgaaaagcat 720
gcgttgcttc tggctggtgg tggacactca cgtatcatag ggcctgatgg tggtgacttg 780
gtcgcgcctc ttgccgaaaa tgaagagggt attctctacg caaaccttga tcctggagta 840
cgcatccttg ctaaaatggc ggcagaccct gctggtcatt attcccgtcc cgacattact 900
cgcttgctaa tagatcgcag ccctaaatta ccggtagttg aaattgaagg tgatcttcgt 960
ccttacgctt tgggtaaagc gtctgagacg ggtgcgcaac tcgaagaaat ttga 1014

<210> 386
<211> 337
<212> PRT
<213> Unknown

<220>
<223> Obtained from an environmental sample

<400> 386
Met Lys Glu Ala Ile Lys Val Ala Cys Val Gln Ala Ala Pro Ile Tyr
1               5                   10                  15
Met Asp Leu Lys Ala Thr Val Asp Lys Thr Ile Glu Leu Met Glu Glu
            20                  25                  30
Ala Ala Arg Asn Asn Ala Arg Leu Ile Ala Phe Pro Glu Thr Trp Ile
        35                  40                  45
Pro Gly Tyr Pro Trp Phe Leu Trp Leu Asp Ser Pro Ala Trp Ala Met
    50                  55                  60
Gln Phe Val Arg Gln Tyr His Glu Asn Ser Leu Glu Leu Asp Gly Pro
65                  70                  75                  80
Gln Ala Lys Arg Ile Ser Asp Ala Ala Lys Arg Leu Gly Ile Met Val
                85                  90                  95
Thr Leu Gly Met Ser Glu Arg Val Gly Gly Thr Leu Tyr Ile Ser Gln
            100                 105                 110
Trp Phe Ile Gly Asp Asn Gly Asp Thr Ile Gly Ala Arg Arg Lys Leu
        115                 120                 125
Lys Pro Thr Phe Val Glu Arg Thr Leu Phe Gly Glu Gly Asp Gly Ser
    130                 135                 140
Ser Leu Ala Val Phe Glu Thr Ser Val Gly Arg Leu Gly Gly Leu Cys
145                 150                 155                 160
Cys Trp Glu His Leu Gln Pro Leu Thr Lys Tyr Ala Leu Tyr Ala Gln
                165                 170                 175
Asn Glu Glu Ile His Cys Ala Ala Trp Pro Ser Phe Ser Leu Tyr Pro
            180                 185                 190
Asn Ala Ala Lys Ala Leu Gly Pro Asp Val Asn Val Ala Ala Ser Arg
        195                 200                 205
Ile Tyr Ala Val Glu Gly Gln Cys Phe Val Leu Ala Ser Cys Ala Leu
    210                 215                 220
Val Ser Gln Ser Met Ile Asp Met Leu Cys Thr Asp Asp Glu Lys His
225                 230                 235                 240
Ala Leu Leu Leu Ala Gly Gly Gly His Ser Arg Ile Ile Gly Pro Asp
                245                 250                 255
Gly Gly Asp Leu Val Ala Pro Leu Ala Glu Asn Glu Glu Gly Ile Leu
            260                 265                 270
Tyr Ala Asn Leu Asp Pro Gly Val Arg Ile Leu Ala Lys Met Ala Ala
        275                 280                 285
Asp Pro Ala Gly His Tyr Ser Arg Pro Asp Ile Thr Arg Leu Leu Ile
    290                 295                 300
Asp Arg Ser Pro Lys Leu Pro Val Val Glu Ile Glu Gly Asp Leu Arg
305                 310                 315                 320
Pro Tyr Ala Leu Gly Lys Ala Ser Glu Thr Gly Ala Gln Leu Glu Glu
                325                 330                 335
Ile

<210> 387
<211> 5

<212> PRT
<213> Unknown

<220>
<223> Obtained from an environmental sample

<400> 387
Phe Pro Glu Thr Phe
1               5

<210> 388
<211> 7
<212> PRT
<213> Unknown

<220>
<223> Obtained from an environmental sample

<220>
<221>
<222>
<223> Xaa = a residue not conserved

<400> 388
Arg Arg Lys Leu Xaa Pro Thr
1               5

<210> 389
<211> 9
<212> PRT
<213> Unknown

<220>
<223> Obtained from an environmental sample

<220>
<221>
<222>
<223> Xaa = a residue not conserved

<400> 389
Leu Xaa Cys Trp Glu His Xaa Xaa Pro
1               5

<210> 390
<211> 5
<212> PRT
<213> Unknown

<220>
<223> Obtained from an environmental sample

<220>
<221>
<222>
<223> Xaa = a residue not conserved

<400> 390
Phe Pro Glu Xaa Xaa
1               5

<210> 391
<211> 7
<212> PRT
<213> Unknown

<220>
<223> Obtained from an environmental sample

<220>
<221>
<222>
<223> Xaa = a residue not conserved

<400> 391
Xaa Arg Lys Leu Xaa Pro Thr
1               5

<210> 392
<211> 9
<212> PRT
<213> Unknown

<220>
<223> Obtained from an environmental sample

<220>
<221>
<222>
<223> Xaa = a residue not conserved

<400> 392
Leu Xaa Cys Trp Glu Xaa Xaa Xaa Pro
1               5